(19) Europäisches Patentamt
European Patent Office
Office européen des brevets

(11) EP 3 636 807 A1

(12) # EUROPEAN PATENT APPLICATION
published in accordance with Art. 153(4) EPC

(43) Date of publication:
**15.04.2020 Bulletin 2020/16**

(21) Application number: **18813910.9**

(22) Date of filing: **08.06.2018**

(51) Int Cl.:
*C40B 40/10* (2006.01) *C07K 1/13* (2006.01)
*C07K 2/00* (2006.01) *C12P 21/02* (2006.01)
*C40B 30/04* (2006.01) *G01N 33/566* (2006.01)
*C07K 7/64* (2006.01) *C12N 15/09* (2006.01)

(86) International application number:
**PCT/JP2018/022097**

(87) International publication number:
**WO 2018/225864 (13.12.2018 Gazette 2018/50)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **09.06.2017 JP 2017114074**

(71) Applicant: **Chugai Seiyaku Kabushiki Kaisha**
**Kita-ku**
**Tokyo 115-8543 (JP)**

(72) Inventors:
- **MURAOKA, Terushige**
**Gotemba-shi**
**Shizuoka 412-8513 (JP)**
- **IIDA, Takeo**
**Kamakura-shi**
**Kanagawa 247-8530 (JP)**
- **MATSUO, Atsushi**
**Gotemba-shi**
**Shizuoka 412-8513 (JP)**
- **TAKEYAMA, Ryuichi**
**Gotemba-shi**
**Shizuoka 412-8513 (JP)**
- **TANADA, Mikimasa**
**Gotemba-shi**
**Shizuoka 412-8513 (JP)**
- **TANAKA, Shota**
**Kamakura-shi**
**Kanagawa 247-8530 (JP)**
- **HAYASHI, Ryuji**
**Gotemba-shi**
**Shizuoka 412-8513 (JP)**
- **FUJINO, Machiko**
**Kamakura-shi**
**Kanagawa 247-8530 (JP)**
- **IIKURA, Hitoshi**
**Gotemba-shi**
**Shizuoka 412-8513 (JP)**

(74) Representative: **Vossius & Partner**
**Patentanwälte Rechtsanwälte mbB**
**Siebertstrasse 3**
**81675 München (DE)**

(54) **CYCLIC PEPTIDE COMPOUND HAVING HIGH MEMBRANE PERMEABILITY, AND LIBRARY CONTAINING SAME**

(57) The present inventors have found that when screening for cyclic peptide compounds that can specifically bind to a target molecule, the use of a library including cyclic peptide compounds having a long side chain in the cyclic portion can improve the hit rate for cyclic peptide compounds that can specifically bind to the target molecule. Meanwhile, the present inventors have found that tryptophan and tyrosine residues, which have conventionally been used in oral low molecular-weight pharmaceuticals and are amino acid residues having an indole skeleton or a hydroxyphenyl group, are not suitable for peptides intended to attain high membrane permeability.

EP 3 636 807 A1

## Description

Technical Field

The present invention relates to cyclic peptide compounds having high membrane permeability, libraries comprising the same, and methods of preparing and screening for the same.

Background Art

Middle-molecular weight compounds (molecular weight: 500 to 2000) have attracted attention in recent years, because such compounds may have advantages over low-molecular weight compounds due to their accessibility to tough targets and may be superior to antibodies due to their ability to translocate into cells. Cyclosporin A, a natural product, is a representative example of such compounds and is a peptide that inhibits an intracellular target cyclophilin and can be administered orally.

Most of middle-molecular weight compounds developed as new drugs so far are natural products and their derivatives and often need to be chemically synthesized in a complex manner. Therefore, currently, drug-likeness (high metabolic stability and membrane permeability) in drug development using middle-molecular weight compounds has not sufficiently been elucidated, as compared with drug development using low-molecular weight compounds (molecular weight: 500 or less). Peptides are representative molecular species of middle-molecular weight compounds. Peptides are generally considered to have low metabolic stability and membrane permeability. However, conditions required for middle-molecular weight peptides to have drug-likeness have been reported in recent years (e.g., having a cyclic portion; the number of N-substituted amino acids; the range of number of amino acid residues; and lipophilicity) (Patent Document 1). It has also been reported that N-methylpeptide libraries were generated by translational synthesis (ribosomal synthesis), with a focus on N-methylamino acids that are unnatural amino acids (Patent Document 2).

Meanwhile, with regard to library design, guidelines for designing synthetic macrocyclic molecular libraries have been proposed based on the results of analyzing known macrocyclic molecules (Non-patent Document 1). However, it is not clear how such guidelines can be applied to peptides, since the guidelines are derived from macrocyclic molecules including molecules other than peptides.

[Citation List]

[Patent Documents]

[Patent Document 1] WO 2013/100132
[Patent Document 2] WO 2012/033154

[Non-patent Document]

[Non-patent Document 1] Villar et al., Nat Chem Biol. 2014 September; 10(9): 723-731

Summary of the Invention

[Problems to be Solved by the Invention]

The present invention was achieved in view of the above circumstances. In a non-limiting aspect, an objective of the present invention is to provide libraries of cyclic peptide compounds for screening for cyclic peptide compounds having high cell membrane permeability (also called "membrane permeability"). In a non-limiting aspect, an objective of the present invention is to provide libraries of cyclic peptide compounds for efficiently screening for cyclic peptide compounds that can specifically bind to target molecules. In another aspect, an objective of the present invention is to provide cyclic peptide compounds having high membrane permeability, or methods of producing or screening for such compounds.

[Means for Solving the Problems]

The present inventors have found that hit rates for cyclic peptide compounds that can specifically bind to target molecules can be improved, specifically, efficiency in screening for such compounds can be improved, by using a library

including cyclic peptide compounds having a long side chain in the cyclic portion when screening for the cyclic peptide compounds that can specifically bind to the target molecules.

Meanwhile, the present inventors have surprisingly found that tryptophan and tyrosine residues, which have conventionally been used in oral low-molecular weight pharmaceuticals and are amino acid residues having an indole skeleton and a hydroxyphenyl group, are actually not suitable for providing peptides having high membrane permeability, specifically, peptides having the above structures are difficult to show high membrane permeability.

In particular, the present inventors have found that cyclic peptide compounds having low membrane permeability are enriched as hit compounds by repeatedly panning for compounds capable of binding to target molecules using a library including cyclic peptide compounds having amino acid residues with a long side chain such as tryptophan and tyrosine residues.

Without wishing to be bound by any particular theory, presumably, it is less likely that cyclic peptide compounds having both (i) binding affinity to target molecules and (ii) high membrane permeability can be obtained by hit-to-lead strategy from cyclic peptide compounds enriched as described above, because the long side chain itself considered to contribute to binding to target molecules is structurally difficult to show high membrane permeability.

The present inventors have thus found libraries of cyclic peptide compounds having a long side chain in the cyclic portion and not having a side chain difficult to show high membrane permeability, as an embodiment of libraries that can be efficiently screened for cyclic peptide compounds having high membrane permeability and that can specifically bind to target molecules, thereby completing the present invention.

In a non-limiting specific embodiment, the present invention includes the following.

[A1] A cyclic peptide compound, wherein:

(1) the cyclic peptide compound does not have a methylthio group, a thiol group, an indole skeleton, or a substituted or unsubstituted hydroxyphenyl group in the side chains of the cyclic portion;
(2) if the cyclic peptide compound has an acidic side chain in the cyclic portion, the acidic side chain has a pKa of 3.5 to 10;
(3) if the cyclic peptide compound has a basic side chain in the cyclic portion, the basic side chain has a basic pKa of 4.0 to 10;
(4) the cyclic peptide compound has a long side chain having a side chain length of 6.0 to 13 angstroms in the cyclic portion; and
(5) the long side chain comprises $R^1$, wherein $R^1$ is:

(a) a phenyl group, or (b) a 4- to 6-membered heterocyclic group having 1 to 3 heteroatoms independently selected from the group consisting of an oxygen atom and a nitrogen atom in the ring,
wherein (a) and (b) optionally have at least one substituent independently selected from Group A below:
Group A: oxo, a halogen atom, a C1-C4 alkyl group optionally substituted with a halogen atom, and a C1-C4 dialkylamino group (wherein one or two non-adjacent methylene groups in the C1-C4 alkyl group are optionally substituted with an oxygen atom);
or

(c) a group represented by the following general formula (II), (III), or (IV):

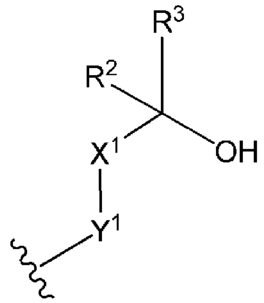
,
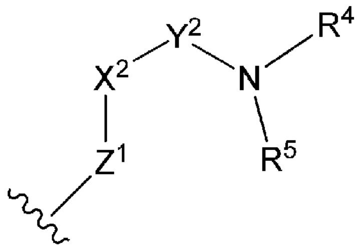
,
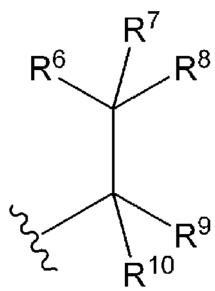

(II) (III) (IV)

wherein

$X^1$ represents a single bond, or a C1-C2 alkylene group optionally having at least one substituent

independently selected from Group B below,
$X^2$ represents a single bond, or a C1-C2 alkylene group optionally having at least one substituent independently selected from Group B below,
$Y^1$ represents a single bond, an oxygen atom, a carbonyl group (-CO-), or a sulfonyl group ($-SO_2-$),
$Y^2$ represents a single bond, a C1-C2 alkylene group optionally substituted with a C1-C4 alkyl group, a carbonyl group (-CO-), or a sulfonyl group ($-SO_2-$),
$Z^1$ represents a single bond, a methylene group optionally having at least one substituent independently selected from Group B below, or an oxygen atom,
$R^2$ and $R^3$ each independently represent a hydrogen atom, a C1-C6 alkyl group, a C2-C4 alkenyl group, or a C2-C4 alkynyl group, wherein the C1-C6 alkyl is optionally substituted with a halogen atom, and one or two non-adjacent methylene groups in the C1-C6 alkyl group are optionally substituted with an oxygen atom,
$R^2$ and $R^3$, or $R^2$ and $X^1$ are optionally joined to form a 4- to 6-membered ring structure, wherein the ring structure is optionally substituted with a C1-C4 alkyl group or a halogen atom, optionally has an oxygen atom in the ring, and is optionally partially unsaturated,
$R^4$ represents a C1-C6 alkyl group, a C2-C6 alkenyl group, a C2-C6 alkynyl group, or a C1-C6 alkanoyl group,
$R^5$ represents a hydrogen atom, a C1-C6 alkyl group, a C2-C6 alkenyl group, or a C2-C6 alkynyl group, wherein one or two non-adjacent methylene groups in the C1-C6 alkyl group of $R^4$ and/or $R^5$ are optionally substituted with an oxygen atom,
or $R^4$ and $R^5$, or $R^5$ and $X^2$ are optionally taken together with the atom(s) to which they are attached to form a 4- to 6-membered ring structure, wherein the ring structure is optionally substituted with a C1-C4 alkyl group or a halogen atom, optionally has an oxygen atom in the ring, and is optionally partially unsaturated,
$R^6$, $R^7$, $R^8$, $R^9$, and $R^{10}$ each independently represent a hydrogen atom, a halogen atom, a C1-C4 alkyl group, a C2-C4 alkenyl group, or a C2-C4 alkynyl group, wherein one or two non-adjacent methylene groups in the C1-C4 alkyl group are optionally substituted with an oxygen atom, and

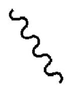

represents the point of attachment;
Group B: a halogen atom, a C1-C4 alkyl group, and a C1-C2 alkoxy group.

[A2] The cyclic peptide compound according to [A1], wherein the long side chain is a side chain represented by the following general formula (1-3):

$$-(CH_2)_n-R^1 \qquad (1\text{-}3)$$

wherein

n is an integer of 1 to 5;
one or two non-adjacent methylene groups in the alkylene group are optionally substituted with an oxygen atom; and
$R^1$ is as defined in [A1].

[A3] The cyclic peptide compound according to [A1] or [A2], wherein the heterocyclic group is a group selected from the group consisting of a pyridyl group, a piperidino group, a morpholino group, and an azetidinyl group, each of which optionally has at least one substituent independently selected from Group A above.
[A4] The cyclic peptide compound according to any one of [A1] to [A3], wherein the cyclic peptide compound does not have a fused-ring structure formed by two or more aromatic rings in the side chains of the cyclic portion.
[A5] The cyclic peptide compound according to any one of [A1] to [A4], wherein the acidic side chain has a pKa of 5.0 to 10.
[A6] The cyclic peptide compound according to any one of [A1] to [A5], wherein the basic side chain has a basic pKa of 4.0 to 7.5.
[A7] The cyclic peptide compound according to any one of [A1] to [A6], wherein the number of amino acids constituting the cyclic portion is 5 to 15.

[A8] The cyclic peptide compound according to any one of [A1] to [A7], wherein the number of amino acids constituting the peptide moiety excluding the nucleic acid-linked portion is 5 to 20.
[A9] The cyclic peptide compound according to any one of [A1] to [A8], wherein the number of N-substituted amino acids constituting the peptide moiety excluding the nucleic acid-linked portion is 3 or more.
[A10] The cyclic peptide compound according to any one of [A1] to [A9], wherein the number of unnatural amino acids constituting the peptide moiety excluding the nucleic acid-linked portion is 4 or more.
[A11] The cyclic peptide compound according to any one of [A1] to [A10], wherein the cyclic peptide compound has a ClogP/total aa of 1.0 to 1.8.
[A12] The cyclic peptide compound according to any one of [A1] to [A11], wherein the cyclic peptide compound has an amide bond at the cyclization site.
[A13] The cyclic peptide compound according to any one of [A1] to [A12], wherein the cyclic peptide compound has a $P_{app}$ of 1.0 x $10^{-6}$ cm/sec or more.
[A14] The cyclic peptide compound according to any one of [A1] to [A13], wherein the number of aromatic rings comprised in the side chains of the cyclic portion is 0 to 3.
[A15] The cyclic peptide compound according to any one of [A1] to [A13], wherein the number of aromatic rings comprised in the side chains of the cyclic portion is 1 to 3.
[A16] The cyclic peptide compound according to any one of [A1] to [A15], wherein the number of aromatic rings comprised in the long side chain is 1 to 3.
[A17] The cyclic peptide compound according to any one of [A1] to [A15], wherein the number of aromatic rings comprised in the long side chain is 2 to 3.
[A18] The library according to any one of [A1] to [A17], wherein the long side chain is a side chain comprising no amide bond or one amide bond in the side chain.
[A19] The cyclic peptide compound according to any one of [A1] to [A18], wherein the long side chain is the longest side chain comprised in at least one amino acid selected from Group C below:
Group C: the amino acids set forth in Tables 2-1 to 2-6.
[A20] A cyclic peptide compound selected from pd100 to pd247 and pd300 to pd504 set forth in Table 26.

In a non-limiting specific embodiment, the present invention also includes the following.

[B1] A library comprising the cyclic peptide compounds according to any one of [A1] to [A20].
[B2] A library of cyclic peptide compounds, wherein the library substantially consists of cyclic peptide compounds which do not have an indole skeleton in the side chains of the cyclic portion.
[B3] A library of cyclic peptide compounds, wherein the library substantially consists of cyclic peptide compounds which do not have a fused-ring structure formed by two or more aromatic rings in the side chains of the cyclic portion.
[B4] The library according to any one of [B1] to [B3], wherein the library substantially consists of cyclic peptide compounds which do not have a substituted or unsubstituted hydroxyphenyl group in the side chains of the cyclic portion.
[B5] The library according to any one of [B1] to [B4], wherein if the library comprises cyclic peptide compounds having an acidic side chain in the cyclic portion, the cyclic peptide compounds substantially consist of cyclic peptide compounds the acidic side chain of which has a pKa of 3.5 to 10.
[B6] The library according to [B5], wherein the pKa is 4.5 to 10.
[B7] The library according to [B5], wherein the pKa is 5.0 to 10.
[B8] The library according to any one of [B1] to [B7], wherein if the library comprises cyclic peptide compounds having a basic side chain in the cyclic portion, the cyclic peptide compounds substantially consist of cyclic peptide compounds the basic side chain of which has a basic pKa of 4.0 to 10.
[B9] The library according to [B8], wherein the basic pKa is 4.0 to 9.5.
[B10] The library according to [B8], wherein the basic pKa is 4.0 to 9.0.
[B11] The library according to [B8], wherein the basic pKa is 4.0 to 8.5.
[B12] The library according to [B8], wherein the basic pKa is 4.0 to 7.5.
[B13] The library according to [B8], wherein the basic pKa is 4.0 to 7.2.
[B14] The library according to any one of [B1] to [B13], wherein the library comprises cyclic peptide compounds having a long side chain of 5.4 to 13 angstroms in length in the cyclic portion.
[B15] The library according to any one of [B1] to [B13], wherein the library comprises cyclic peptide compounds having a long side chain of 6.0 to 13 angstroms in length in the cyclic portion.
[B16] The library according to any one of [B1] to [B13], wherein the library comprises cyclic peptide compounds having a long side chain of 6.0 to 10 angstroms in length in the cyclic portion.
[B17] The library according to any one of [B14] to [B16], wherein the long side chain is a side chain comprising no amide bond or one amide bond in the side chain.

[B18] The library according to any one of [B1] to [B17], wherein the library comprises cyclic peptide compounds having an aromatic ring in the side chains of the cyclic portion.

[B19] The library according to any one of [B14] to [B18], wherein at least one of the long side chains is a side chain comprising an aromatic ring.

[B20] The library according to any one of [B14] to [B18], wherein two or more of the long side chains are side chains comprising an aromatic ring.

[B21] The library according to any one of [B1] to [B20], wherein the library substantially consists of cyclic peptide compounds which do not have a methylthio group in the side chains of the cyclic portion.

[B22] The library according to any one of [B1] to [B21], wherein the library substantially consists of cyclic peptide compounds which do not have a thiol group in the side chains of the cyclic portion.

[B23] The library according to any one of [B1] to [B22], wherein the average of the number of amino acids constituting the cyclic portion of each cyclic peptide compound comprised in the library is 5 to 15.

[B24] The library according to any one of [B1] to [B22], wherein the average of the number of amino acids constituting the cyclic portion of each cyclic peptide compound comprised in the library is 8 to 13.

[B25] The library according to any one of [B1] to [B22], wherein the average of the number of amino acids constituting the cyclic portion of each cyclic peptide compound comprised in the library is 8 to 11.

[B26] The library according to any one of [B1] to [B25], wherein the average of the number of amino acids constituting the peptide moiety excluding the nucleic acid-linked portion of each cyclic peptide compound comprised in the library is 5 to 20.

[B27] The library according to any one of [B1] to [B25], wherein the average of the number of amino acids constituting the peptide moiety excluding the nucleic acid-linked portion of each cyclic peptide compound comprised in the library is 8 to 13.

[B28] The library according to any one of [B1] to [B27], wherein the average of ClogP of each cyclic peptide compound comprised in the library is 4 to 18.

[B29] The library according to any one of [B1] to [B28], wherein the average of ClogP/total aa of each cyclic peptide compound comprised in the library is 1.0 to 1.8.

[B30] The library according to any one of [B1] to [B29], wherein the average of the number of unnatural amino acids comprised in the peptide moiety excluding the nucleic acid-linked portion of each cyclic peptide compound comprised in the library is 4 or more.

[B31] The library according to any one of [B1] to [B30], wherein the average of the percentage of the number of N-substituted amino acids relative to the number of amino acids comprised in the peptide moiety excluding the nucleic acid-linked portion of each cyclic peptide compound comprised in the library is 30% or higher.

[B32] The library according to any one of [B1] to [B30], wherein the average of the number of N-substituted amino acids comprised in the peptide moiety excluding the nucleic acid-linked portion of each cyclic peptide compound comprised in the library is 3 or more.

[B33] The library according to any one of [B1] to [B32], wherein the cyclic peptide compounds comprised in the library are cyclic peptide compounds having an amide bond in the cyclization site.

[B34] The library according to any one of [B1] to [B33], wherein the library is a library for use in identifying a compound that can specifically bind to a target molecule.

[B35] The library according to any one of [B1] to [B34], wherein the library is a library for use in identifying a compound that can be administered orally or a precursor thereof.

[B36] The library according to any one of [B1] to [B35], wherein the library is a library for use in obtaining a cyclic peptide compound having a $P_{app}$ of 1.0 x $10^{-6}$ or more.

[B37] The library according to any one of [B1] to [B36], wherein the library has a diversity of 1 x $10^4$ or more.

[B38] The library according to any one of [B1] to [B36], wherein the library has a diversity of 1 x $10^{10}$ or more.

[B39] The library according to any one of [B1] to [B38], wherein the average of the molecular weight of each cyclic peptide compound comprised in the library is 500 to 2000.

[B40] The library according to any one of [B1] and [B14] to [B39], wherein the long side chain comprises $R^1$, wherein $R^1$ is as defined in [A1].

[B41] The library according to any one of [B1] and [B14] to [B40], wherein the long side chain is a side chain represented by the following general formula (1-3):

$$-(CH_2)_n-R^1 \qquad (1-3)$$

wherein

n is an integer of 1 to 5;

one or two non-adjacent methylene groups in the alkylene group are optionally substituted with an oxygen atom;

and
$R^1$ is as defined in [B40].

[B42] The library according to [B40] or [B41], wherein the heterocyclic group is a group selected from the group consisting of a pyridyl group, a piperidino group, a morpholino group, and an azetidinyl group, each of which optionally has at least one substituent independently selected from Group A above.
[B43] The library according to any one of [B40] to [B42], wherein the $R^1$ is at least one group selected from the group consisting of (a) and (b) above.
[B44] The library according to any one of [B40] to [B42], wherein the $R^1$ is a group selected from (1) to (6) below:

(1) the general formula (II), wherein $Y^1$ is a single bond or an oxygen atom, $X^1$ is a single bond or an optionally substituted C1-C2 alkylene group, and $R^2$ and $R^3$ each independently represent a hydrogen atom or a C1-C6 alkyl group, wherein the C1-C6 alkyl group is optionally substituted with a halogen atom;
(2) the general formula (III), wherein $R^5$ is a hydrogen atom, $Y^2$ is a single bond, a carbonyl group, or a sulfonyl group, $X^2$ is an optionally substituted C1-C2 alkylene group, and Z1 is an oxygen atom;
(3) the general formula (III), wherein $R^5$ is a C1-C6 alkyl group and does not form a ring structure, $Y^2$ is a single bond, an optionally substituted C1-C2 alkylene group, a carbonyl group, or a sulfonyl group, $X^2$ is a single bond or an optionally substituted C1-C2 alkylene group, and $Z^1$ is a single bond or an optionally substituted methylene group;
(4) the general formula (III), wherein $R^4$ and $R^5$ are taken together with the atom(s) to which they are attached to form a 4- to 6-membered ring structure which optionally has an oxygen atom in the ring, wherein the ring structure is optionally substituted with a C1-C4 alkyl group or a halogen atom, $X^2$ is a single bond or an optionally substituted C1-C2 alkylene group, $Y^2$ is a single bond or an optionally substituted C1-C2 alkylene group, and $Z^1$ is a single bond, an optionally substituted methylene group, or an oxygen atom;
(5) the general formula (III), wherein $R^5$ and $X^2$ are taken together with the atom(s) to which they are attached to form a 4- to 6-membered ring structure which optionally has an oxygen atom in the ring, wherein the ring structure is optionally substituted with a C1-C4 alkyl group or a halogen atom, $Y^2$ is a single bond or an optionally substituted C1-C2 alkylene group, $Z^1$ is an oxygen atom, and $R^4$ is a C1-C4 alkyl group; and
(6) the general formula (IV), wherein $R^6$, $R^7$, $R^8$, $R^9$, and $R^{10}$ are each independently a hydrogen atom, a halogen atom, a methyl group, or an ethyl group.

[B45] The library according to any one of [B1] and [B14] to [B44], wherein the long side chain is the longest side chain comprised in at least one amino acid selected from Group C of [A19].
[B46] The library according to any one of [B1] and [B14] to [B44], wherein at least one of the long side chains is a side chain comprising an aromatic ring.
[B47] The library according to [B40] or [B41], wherein the $R^1$ is a phenyl group that optionally has at least one substituent independently selected from Group A above.
[B48] The library according to any one of [B1] and [B14] to [B47], wherein the long side chain is a side chain comprising at least one group selected from the group consisting of a halophenyl group and a C1-C4 haloalkylphenyl group.
[B49] The library according to any one of [B 1] to [B48], wherein the average of the number of aromatic rings comprised in the side chains of the cyclic portion of each cyclic peptide compound comprised in the library is 0 to 3.
[B50] The library according to any one of [B1] to [B49], wherein the average of the percentage of the number of aromatic rings relative to the number of amino acids constituting the cyclic portion of each cyclic peptide compound comprised in the library is 40% or lower.
[B51] The library according to any one of [B1] to [B50], wherein the average of the number of aromatic rings comprised in the long side chains of the cyclic portion of each cyclic peptide compound comprised in the library is 1 to 3.
[B52] The library according to any one of [B1] to [B51], wherein the library is a nucleic acid display library.
[B53] A library of nucleic acids encoding the library of cyclic peptide compounds according to any one of [B1] to [B52].

In a non-limiting specific embodiment, the present invention also includes the following.

[C1] A method of screening for a cyclic peptide compound that can specifically bind to a target molecule, the method comprising the steps of:

(i) contacting a cyclic peptide compound comprised in the library according to any one of [B1] to [B52] with the target molecule; and
(ii) selecting a cyclic peptide compound that can specifically bind to the target molecule.

[C2] The method according to [C1], wherein the target molecule is a protein.

In a non-limiting specific embodiment, the present invention also includes the following.

[D1] A cell-free translation system for producing a peptide compound, wherein the cell-free translation system does not substantially comprise at least one selected from the group consisting of (a) to (c) below:

(a) an amino acid having an indole skeleton in the side chain, or a nucleic acid encoding the amino acid;
(b) an amino acid having a fused-ring structure formed by two or more aromatic rings in the side chain, or a nucleic acid encoding the amino acid; and
(c) an amino acid having a substituted or unsubstituted hydroxyphenyl group in the side chain, or a nucleic acid encoding the amino acid.

[D2] The cell-free translation system according to [D1], wherein the cell-free translation system does not substantially comprise (a), (b), or (c) of [D1].
[D3] The cell-free translation system according to [D1] or [D2], wherein if the translation system comprises a tRNA acylated with an amino acid having an acidic side chain, and a nucleic acid encoding the amino acid, the side chain of the amino acid has a pKa of 3.5 to 10.
[D4] The cell-free translation system according to any one of [D1] to [D3], wherein if the translation system comprises a tRNA acylated with an amino acid having a basic side chain, and a nucleic acid encoding the amino acid, the side chain of the amino acid has a basic pKa of 4.0 to 10.
[D5] The cell-free translation system according to [D3] or [D4], wherein the pKa is 5.0 to 10.
[D6] The cell-free translation system according to [D4] or [D5], wherein the basic pKa is 4.0 to 7.5.
[D7] The cell-free translation system according to any one of [D1] to [D6], wherein the cell-free translation system comprises amino acids having a long side chain of 5.4 to 13 angstroms in length.
[D8] The cell-free translation system according to [D7], wherein the long side chain is 6.0 to 10 angstroms in length.
[D9] The cell-free translation system according to [D7] or [D8], wherein at least one of the long side chains is a side chain having an aromatic ring.
[D10] The cell-free translation system according to [D7] or [D8], wherein two or more of the long side chains are side chains having an aromatic ring.
[D11] The cell-free translation system according to any one of [D1] to [D10], wherein 40% or more of the number of amino acid species comprised in the translation system are N-substituted amino acids.
[D12] The cell-free translation system according to any one of [D1] to [D11], wherein the peptide compound is a cyclic peptide compound.
[D13] The cell-free translation system according to any one of [D1] to [D12], wherein the percentage of the number of amino acid species having an aromatic ring relative to the number of amino acid species comprised in the translation system is 40% or lower.
[D14] A cell-free translation system, wherein the average of the number of aromatic rings comprised in the side chains of the cyclic portion of a cyclic peptide compound having a cyclic portion composed of 8 to 11 amino acids is adjusted to be 0 to 3.

In a non-limiting specific embodiment, the present invention also includes the following.

[E1] A method of producing a library of peptide compounds, the method comprising at least one step selected from the group consisting of (a) and (b) below:

(a) preparing an amino acid pool substantially not comprising an amino acid which has an indole skeleton in the side chain, and synthesizing a peptide compound using a part or all of the amino acids comprised in the pool as constituent amino acids; and
(b) preparing a template pool substantially not comprising a nucleic acid encoding an amino acid having an indole skeleton in the side chain, and synthesizing a peptide compound from the template pool.

[E2] The method according to [E1], wherein the pools of steps (a) and (b) above are pools which do not substantially comprise an amino acid having a fused-ring structure formed by two or more aromatic rings in the side chain or a nucleic acid encoding the amino acid.
[E3] The method according to [E1] or [E2], wherein the pools of steps (a) and (b) above are pools which do not substantially comprise an amino acid having a substituted or unsubstituted hydroxyphenyl group in the side chain and a nucleic acid encoding the amino acid.

[E4] The method according to any one of [E1] to [E3], wherein if the amino acid pool to be used in synthesizing a peptide compound comprises an amino acid having an acidic side chain and the template pool comprises a nucleic acid encoding the amino acid, the side chain of the amino acid has a pKa of 3.5 to 10.
[E5] The method according to [E4], wherein the pKa is 5.0 to 10.
[E6] The method according to any one of [E1] to [E5], wherein if the amino acid pool to be used in synthesizing a peptide compound comprises an amino acid having a basic side chain and the template pool comprises a nucleic acid encoding the amino acid, the side chain of the amino acid has a basic pKa of 4.0 to 10.
[E7] The method according to [E6], wherein the basic pKa is 4.0 to 7.5.
[E8] The method according to any one of [E1] to [E7], wherein the amino acid pool to be used in synthesizing a peptide compound is a pool comprising amino acids which have a long side chain of 5.4 to 13 angstroms in length.
[E9] The method according to any one of [E1] to [E7], wherein the amino acid pool to be used in synthesizing a peptide compound is a pool comprising amino acids which have a long side chain of 6.0 to 10 angstroms in length.
[E10] The method according to [E8] or [E9], wherein at least one of the long side chains is a side chain having an aromatic ring.
[E11] The method according to any one of [E1] to [E10], wherein 50% or more of the number of amino acid species comprised in the amino acid pool to be used in synthesizing a peptide compound are unnatural amino acids.
[E12] The method according to any one of [E1] to [E11], wherein 40% or more of the number of amino acid species comprised in the amino acid pool to be used in synthesizing a peptide compound are N-substituted amino acids.
[E13] The method according to any one of [E1] to [E12], wherein the peptide compound is a cyclic peptide compound.
[E14] The method according to [E13], wherein the method comprises at least one step selected from the group consisting of (c) and (d) below:

(c) preparing an amino acid pool adjusted such that the average of the percentage of the number of amino acids having an aromatic ring relative to the number of amino acids constituting the cyclic portion becomes 40% or lower in a translationally synthesized (ribosomally synthesized) cyclic peptide compound, and synthesizing a peptide compound using a part or all of the amino acids comprised in the pool as constituent amino acids; and
(d) preparing a template pool adjusted such that the average of the percentage of the number of amino acids having an aromatic ring relative to the number of amino acids constituting the cyclic portion becomes 40% or lower in a ribosomally synthesized cyclic peptide compound, and synthesizing a peptide compound from the template pool.

[E15] A method of producing a library of peptide compounds, the method comprising the step of synthesizing a peptide compound by using the cell-free translation system according to any one of [D1] to [D14].
[E16] The method according to any one of [E1] to [E13], wherein the method comprises the step of synthesizing a peptide compound by using the cell-free translation system according to any one of [D1] to [D14].
[E17] The method according to any one of [E1] to [E16], wherein the library has a diversity of 1 x $10^4$ or more.
[E18] The method according to any one of [E1] to [E17], wherein the library is a nucleic acid display library.
[E19] The method according to any one of [E1] to [E18] for producing the library according to any one of [B1] to [B44].
[E20] A library obtained by the method according to any one of [E1] to [E19].
[E21] A cyclic peptide compound constituting the library according to [E20].

In a non-limiting specific embodiment, the present invention also includes the following.

[F1] A method of producing a cyclic peptide compound, the method comprising the steps of:

(i) contacting a cyclic peptide compound comprised in the library according to any one of [B1] to [B52] with a target molecule;
(ii) selecting a cyclic peptide compound that can bind to the target molecule; and
(iii) producing a cyclic peptide compound based on the amino acid sequence of the cyclic peptide compound selected in (ii).

[F2] A cyclic peptide compound produced by the method according to [F1].

In a non-limiting specific embodiment, the present invention also includes the following.

[G1] An amino acid having a long side chain of 5.4 to 13 angstroms in length, wherein the long side chain comprises R1, wherein R1 is as defined in [A1].
[G2] An amino acid having a long side chain of 5.4 to 13 angstroms in length.

[G3] An amino acid selected from the group consisting of amino acids set forth in Tables 2-1 to 2-6.
[G4] The amino acid according to any one of [G1] to [G3], wherein the main chain amino group is protected by a protecting group.
[G5] The amino acid according to any one of [G1] to [G3], wherein the main chain amino group is protected by an Fmoc group.
[G6] The amino acid according to any one of [G1] to [G5] for use in synthesizing a cyclic peptide compound.
[G7] A pdCpA amino acid or pCpA amino acid in which pdCpA or pCpA is linked to the carboxy group of the amino acid according to any one of [G1] to [G5] by an ester bond.
[G8] The amino acid according to any one of [G1] to [G5] and [G7] for use in translating a cyclic peptide compound.
[H1] An amino acid having a group represented by the following general formula (II) or (III) in the side chain:

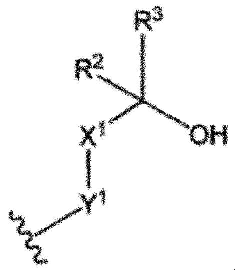
(II)

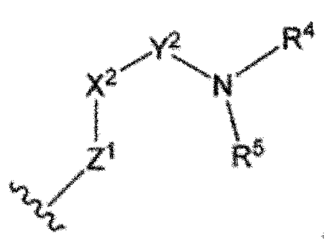
(III)

wherein

$X^1$ represents a single bond, or a C1-C2 alkylene group optionally having at least one substituent independently selected from Group B below,
$X^2$ represents a single bond, or a C1-C2 alkylene group optionally having at least one substituent independently selected from Group B below,
$Y^1$ represents a single bond, an oxygen atom, a carbonyl group (-CO-), or a sulfonyl group ($-SO_2-$),
$Y^2$ represents a single bond, a C1-C2 alkylene group optionally substituted with a C1-C4 alkyl group, a carbonyl group (-CO-), or a sulfonyl group ($-SO_2-$),
$Z^1$ represents an oxygen atom,
$R^2$ and $R^3$ each independently represent a hydrogen atom, a C1-C6 alkyl group, a C2-C4 alkenyl group, or a C2-C4 alkynyl group, wherein the C1-C6 alkyl group is optionally substituted with a halogen atom, and one or two non-adjacent methylene groups in the C1-C6 alkyl group are optionally substituted with an oxygen atom,
$R^2$ and $R^3$, or $R^2$ and $X^1$ are optionally joined to form a 4- to 6-membered ring structure, wherein the ring structure is optionally substituted with a C1-C4 alkyl group or a halogen atom, optionally has an oxygen atom in the ring, and is optionally partially unsaturated,
$R^4$ represents a C1-C6 alkyl group, a C2-C6 alkenyl group, a C2-C6 alkynyl group, or a C1-C6 alkanoyl group,
$R^5$ represents a hydrogen atom, wherein one or two non-adjacent methylene groups in the C1-C6 alkyl group of $R^4$ are optionally substituted with an oxygen atom, and

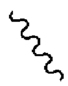

represents the point of attachment;
Group B: a halogen atom, a C1-C4 alkyl group, and a C1-C2 alkoxy group.

[H2] A peptide compound library comprising a peptide compound comprising at least one amino acid selected from the group consisting of the amino acids set forth in Tables 2-1 to 2-6.
[H3] A method of producing a peptide compound library comprising a peptide compound comprising at least one amino acid selected from the group consisting of the amino acids set forth in Tables 2-1 to 2-6, the method comprising the step of ribosomally synthesizing a peptide compound with a cell-free translation system comprising (i) and (ii) below:

(i) a tRNA bound to at least one amino acid selected from the group consisting of the amino acids set forth in

Tables 2-1 to 2-6; and
(ii) a nucleic acid library encoding the peptide compound library,
wherein the nucleic acid library comprises a nucleic acid comprising at least one codon corresponding to an anticodon of the tRNA.

[H4] A cell-free translation system for producing a peptide compound comprising at least one amino acid selected from the group consisting of the amino acids set forth in Tables 2-1 to 2-6, wherein the cell-free translation system comprises (i) and (ii) below:

(i) a tRNA bound to at least one amino acid selected from the group consisting of the amino acids set forth in Tables 2-1 to 2-6; and
(ii) a nucleic acid encoding the peptide compound,
wherein the nucleic acid comprises at least one codon corresponding to an anticodon of the tRNA.

[H5] A method of ribosomally synthesizing a peptide compound comprising at least one amino acid selected from the group consisting of the amino acids set forth in Tables 2-1 to 2-6, the method comprising the steps (i) and (ii):

(i) preparing a tRNA bound to at least one amino acid selected from the group consisting of the amino acids set forth in Tables 2-1 to 2-6; and
(ii) translating a nucleic acid comprising at least one codon corresponding to an anticodon of the tRNA with a cell-free translation system to obtain the peptide compound.

[Effects of the Invention]

An embodiment of the present invention can efficiently screen for cyclic peptide compounds that can specifically bind to target molecules. An embodiment of the present invention can also provide cyclic peptide compounds having high cell membrane permeability.

Brief Description of the Drawings

Fig. 1 is a diagram showing an outline for an embodiment of a method of measuring membrane permeability.
Fig. 2 is a conceptual diagram showing the presupposed and actual cell membrane permeability measurements using Caco-2 cells.
Fig. 3-1 is a set of diagrams showing mass spectra of ribosomally synthesized peptide compounds containing unnatural amino acids.
Fig. 3-2 is a continuation of Fig. 3-1.
Fig. 3-3 is a continuation of Fig. 3-2.
Fig. 4 is a set of diagrams showing mass spectra of ribosomally synthesized peptide compounds containing unnatural amino acids.
Fig. 5 is a set of diagrams showing mass spectra of ribosomally synthesized peptide compounds containing unnatural amino acids.
Fig. 6 is a set of diagrams showing mass spectra of ribosomally synthesized peptide compounds containing unnatural amino acids.
Fig. 7 is a graph showing the change in TEER when Caco-2 cells were incubated up to three hours according to a conventional method.
Fig. 8 a graph showing the change in TEER when Caco-2 cells were incubated up to 24 hours according to an improved method.
Fig. 9 is a graph showing the correlation between $P_{app}$ and Fa measured by a conventional method.
Fig. 10 is a graph showing the correlation between $P_{app}$ and Fa measured by an improved method. Compared to the conventional method, the improved method showed higher correlation between $P_{app}$ and Fa.
Fig. 11 is a graph showing the correlation between ClogP/total AA and $P_{app}$ using a population of cyclic peptide compounds not having a Trp side chain.
Fig. 12 is a graph showing the correlation between ClogP/total AA and $P_{app}$ using a population of cyclic peptide compounds having a Trp side chain.
Fig. 13 is a graph showing the correlation between ClogP/total AA and $P_{app}$ using a population of cyclic peptide compounds having a Phe(4-$CF_3$) side chain.

Fig. 14 is a graph showing the correlation between ARC and cell membrane permeability of cyclic peptide compounds.

Mode for Carrying Out the Invention

Preferred non-limiting embodiments of the present disclosure are described below.

It is intended that all elements described in the Examples set forth later will naturally be deemed as also being equally described in this "Mode for Carrying Out the Invention" without being bound by any limitation of patent practice, custom, law, and the like by which one could attempt to interpret what is described in the Examples in a limited manner in countries where patent protection is sought by the present patent application.

It is intended, and is to be naturally understood by persons with ordinary skill in the art, that the present disclosure includes any combinations of some or all of one or more elements described anywhere in the present disclosure as long as they are not technically contradictory based on the common technical knowledge of the skilled persons.

The following abbreviations are used herein: Ala (alanine), Arg (arginine), Asn (asparagine), Asp (aspartic acid), Cys (cysteine), Glu (glutamic acid), Gln (glutamine), Gly (glycine), His (histidine), Ile (isoleucine), Leu (leucine), Lys (lysine), Met (methionine), Phe (phenylalanine), Pro (proline), Ser (serine), Thr (threonine), Trp (tryptophan), Tyr (tyrosine), and Val (valine). In addition to these, the abbreviations set forth in Tables 2-1 to 2-6 and Tables 4-1 to 4-32 are used.

The term "alkyl" as used herein refers to a monovalent group derived by removing any one hydrogen atom from an aliphatic hydrocarbon, and covers a subset of hydrocarbyl or hydrocarbon group structures that contain hydrogen and carbon atoms, but do not contain a heteroatom (which refers to an atom other than carbon and hydrogen atoms) or an unsaturated carbon-carbon bond in the skeleton. The alkyl groups include linear or branched groups. The alkyl group is an alkyl group having 1 to 20 carbon atoms (C1-C20; hereinafter, "Cp-Cq" means that it has p to q carbon atoms), preferred examples of which include a C1-C6 alkyl group, a C1-C5 alkyl group, a C1-C4 alkyl group, and a C1-C3 alkyl group. Specific examples of the alkyl include methyl, ethyl, propyl, butyl, pentyl, hexyl, isopropyl, tert-butyl, sec-butyl, 1-methylpropyl, 1,1-dimethylpropyl, 2,2-dimethylpropyl, 1,2-dimethylpropyl, 1,1,2-trimethylpropyl, 1,2,2-trimethylpropyl, 1,1,2,2-tetramethylpropyl, 1-methylbutyl, 2-methylbutyl, 3-methylbutyl, 1,1-dimethylbutyl, 1,2-dimethylbutyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, isopentyl, and neopentyl.

The term "alkenyl" as used herein refers to a monovalent group having at least one double bond (two adjacent SP2 carbon atoms). Depending on the configuration of a double bond and a substituent (if present), the geometry of the double bond can be an entgegen (E) or zuzammen (Z) configuration or a cis or trans configuration. Examples of the alkenyl include linear or branched ones, including linear ones including internal olefins. Preferred examples include C2-C10 alkenyl, with C2-C6 alkenyl and C2-C4 alkenyl being more preferred.

Specific examples of such alkenyl include vinyl, allyl, 1-propenyl, 2-propenyl, 1-butenyl, 2-butenyl (including cis and trans), 3-butenyl, pentenyl, and hexenyl.

The term "alkynyl" as used herein refers to a monovalent group having at least one triple bond (two adjacent SP carbon atoms). Examples include linear or branched alkynyl groups, including internal alkylenes. Preferred examples include C2-C10 alkynyl, with C2-C6 alkynyl and C2-C4 alkynyl being more preferred. Specific examples of the alkynyl include ethynyl, 1-propynyl, propargyl, 3-butynyl, pentynyl, hexynyl, 3-phenyl-2-propynyl, 3-(2'-fluorophenyl)-2-propynyl, 2-hydroxy-2-propynyl, 3-(3-fluorophenyl)-2-propynyl, and 3-methyl-(5-phenyl)-4-pentynyl.

The term "cycloalkyl" as used herein refers to a saturated or partially saturated cyclic monovalent aliphatic hydrocarbon group, including single rings, bicyclo rings, and spiro rings. Preferred examples include C3-C10 cycloalkyl. The cycloalkyl group may be partially unsaturated. Specific examples of the cycloalkyl include cyclopropyl, cyclobutyl, cyclopentyl, cyclohexyl, cycloheptyl, cyclooctyl, and bicyclo[2.2.1]heptyl.

The term "aryl" as used herein refers to a monovalent aromatic hydrocarbon ring, preferred examples of which include C6-C10 aryl. Specific examples of the aryl include phenyl and naphthyl (e.g., 1-naphthyl or 2-naphthyl).

The term "heteroaryl" as used herein refers to a monovalent aromatic ring group containing preferably 1 to 5 heteroatoms in the ring-forming atoms (herein also called "in the ring"), which may be partially saturated. The ring may be a single ring or two fused rings (such as bicyclic heteroaryl in which heteroaryl is fused with benzene or monocyclic heteroaryl). The number of the ring-forming atoms is preferably 5 to 10 (5- to 10-membered heteroaryl). Specific examples of the heteroaryl include furyl, thienyl, pyrrolyl, imidazolyl, pyrazolyl, thiazolyl, isothiazolyl, oxazolyl, isoxazolyl, oxadiazolyl, thiadiazolyl, triazolyl, tetrazolyl, pyridyl, pyrimidyl, pyridazinyl, pyrazinyl, triazinyl, benzofuranyl, benzothienyl, benzothiadiazolyl, benzothiazolyl, benzoxazolyl, benzoxadiazolyl, benzimidazolyl, indolyl, isoindolyl, indazolyl, quinolyl, isoquinolyl, cinnolinyl, quinazolinyl, quinoxalinyl, benzodioxolyl, indolizinyl, and imidazopyridyl.

The term "arylalkyl (aralkyl)" as used herein refers to a group containing both aryl and alkyl, for example, a group in which at least one hydrogen atom of the alkyl is replaced with aryl, preferred examples of which include "C5-C10 aryl-C1-C6 alkyl." Examples include benzyl.

The term "alkylene" as used herein refers to a divalent group derived by removing any one hydrogen atom from

the "alkyl." Preferred examples of the alkylene include C1-C2 alkylene, C1-C3 alkylene, C1-C4 alkylene, C1-C5 alkylene, and C1-C6 alkylene. Specific examples of the alkylene include methylene, 1,2-ethylene, 1,1-ethylene, 1,3-propylene, tetramethylene, pentamethylene, and hexamethylene.

The term "arylene" as used herein refers to a divalent group derived by further removing any one hydrogen atom from the aryl. The arylene may be a single ring or fused rings. The number of the ring-forming atoms is not particularly limited, but is preferably 6 to 10 (C6-C10 arylene). Specific examples of the arylene include phenylene and naphtylene.

The term "heteroarylene" as used herein refers to a divalent group derived by further removing any one hydrogen atom from the heteroaryl. The heteroarylene may be a single ring or fused rings. The number of the ring-forming atoms is not particularly limited, but is preferably 5 to 10 (5- to 10-membered heteroarylene). Specific examples of the heteroarylene include pyrrolediyl, imidazolediyl, pyrazolediyl, pyridinediyl, pyridazinediyl, pyrimidinediyl, pyrazinediyl, triazolediyl, triazinediyl, isoxazolediyl, oxazolediyl, oxadiazolediyl, isothiazolediyl, thiazolediyl, thiadiazolediyl, furandiyl, and thiophenediyl.

The term "alkylenearylene" as used herein refers to a divalent group in which the alkylene and the arylene are attached to each other at any position and the alkylene is attached to the basic skeleton. Specific examples of the alkylenearylene include -C1-C6 alkylene-C6-C10 arylene.

The term "arylenealkylene" as used herein refers to a divalent group in which the arylene and the alkylene are attached to each other at any position and the arylene is attached to the basic skeleton. Specific examples of the arylenealkylene include -C6-C10 arylene-C1-C6 alkylene.

The term "alkyleneheteroarylene" as used herein refers to a divalent group in which the alkylene and the heteroarylene are attached to each other at any position and the alkylene is attached to the basic skeleton. Specific examples of the alkyleneheteroarylene include -C1-C6 alkylene-5- to 10-membered heteroarylene.

The term "heteroarylenealkylene" as used herein refers to a divalent group in which the heteroarylene and the alkylene are attached to each other at any position and the heteroarylene is attached to the basic skeleton. Specific examples of the heteroarylenealkylene include -5- to 10-membered heteroarylene-C1-C6 alkylene.

The term "active ester" as used herein refers to a group which contains carbonyl that reacts with an amino group to generate an amide bond, in which for example, OBt, OAt, OSu, OPfp, or SR1 is attached to the carbonyl, and which can promote reaction with amine.

The term "reaction auxiliary group" as used herein refers to a group that is introduced near a functional group to be subjected to bonding and activates the functional group for bonding reaction so that the reaction occurs selectively at the desired position. For example, a reaction auxiliary group can be introduced either on carbonyl side or amine side, or both, in order to cause reaction between carbonyl and amine. Such reaction auxiliary groups can also be eliminated simultaneously with or after attachment reaction.

The term "amino acid" as used herein includes natural and unnatural amino acids. The term "natural amino acid" as used herein refers to Gly, Ala, Ser, Thr, Val, Leu, Ile, Phe, Tyr, Trp, His, Glu, Asp, Gln, Asn, Cys, Met, Lys, Arg, or Pro. Examples of the unnatural amino acid include, but are not particularly limited to, β-amino acids, γ-amino acids, D-amino acids, N-substituted amino acids, α,α-disubstituted amino acids, amino acids having side chains that are different from those of natural amino acids, and hydroxycarboxylic acids. Amino acids herein may have any conformation. There is no particular limitation on the selection of amino acid side chain, but in addition to a hydrogen atom, it can be freely selected from, for example, an alkyl group, an alkenyl group, an alkynyl group, an aryl group, a heteroaryl group, an aralkyl group, and a cycloalkyl group. One or two non-adjacent methylene groups in such a group are optionally substituted with an oxygen atom, a carbonyl group (-CO-), or a sulfonyl group ($-SO_2-$). Each group may have a substituent, and there are no limitations on the substituent. For example, one or more substituents may be freely and independently selected from any substituents including a halogen atom, an O atom, an S atom, an N atom, a B atom, an Si atom, or a P atom. Examples include an optionally substituted alkyl group, alkenyl group, alkynyl group, aryl group, heteroaryl group, aralkyl group, and cycloalkyl group. Amino acids herein may have a "long side chain" described below as the side chain. In a non-limiting embodiment, amino acids herein may be compounds having a carboxy group and an amino group in the same molecule (even in this case, imino acids such as proline and hydroxyproline are also included in amino acids).

The main chain amino group of an amino acid may be unsubstituted (an $NH_2$ group) or substituted (i.e., an -NHR group, where R represents alkyl, alkenyl, alkynyl, aryl, heteroaryl, aralkyl, or cycloalkyl which may have a substituent, one or two non-adjacent methylene groups in such a group may be substituted with an oxygen atom, a carbonyl group (-CO-), or a sulfonyl group ($-SO_2-$), and the carbon chain bonded to the N atom and the carbon atom at the position α may form a ring, as in proline). The R substituent is selected as the substituent in the aforementioned amino acid side chain is selected. When the main chain amino group is substituted, the R is included in the "amino acid side chain" as used herein. The R may be a "long side chain" described below. Such amino acids in which the main chain amino group is substituted are herein called "N-substituted amino acids." Preferred examples of the "N-substituted amino acids" as used herein include, but are not limited to, N-alkylamino acids, N-C1-C6 alkylamino acids, N-C1-C4 alkylamino acids, N-methylamino acids, and N-substituted amino acids having a long side chain.

"Amino acids" as used herein which constitute a peptide compound include all isotopes corresponding to each amino acid. The isotope of the "amino acid" refers to one having at least one atom replaced with an atom of the same atomic number (number of protons) and different mass number (total number of protons and neutrons). Examples of isotopes contained in the "amino acid" constituting the peptide compounds of the present invention include a hydrogen atom, a carbon atom, a nitrogen atom, an oxygen atom, a phosphorus atom, a sulfur atom, a fluorine atom, and a chlorine atom, which respectively include $^{2}H$ and $^{3}H$; $^{13}C$ and $^{14}C$; $^{15}N$; $^{17}O$ and $^{18}O$; $^{31}P$ and $^{32}P$; $^{35}S$; $^{18}F$; and $^{36}Cl$.

Halogen atoms as used herein include F, Cl, Br, and I, preferred examples of which include F or Cl. Substituents containing a halogen atom include, but are not limited to, fluoro (-F), chloro (-Cl), bromo (-Br), and iodo (-I). Further examples include a halogen-substituted alkyl group, cycloalkyl group, alkenyl group, alkynyl group, aryl group, heteroaryl group, or aralkyl group substituted with one or more such substituents. More specific examples include fluoroalkyl, difluoroalkyl, and trifluoroalkyl.

Substituents containing an O atom include groups such as hydroxy (-OH), oxy (-OR), carbonyl (-C=O-R), carboxy ($-CO_2H$), oxycarbonyl (-C=O-OR), carbonyloxy (-O-C=O-R), thiocarbonyl (-C=O-SR), carbonylthio (-S-C=O-R), aminocarbonyl (-C=O-NHR), carbonylamino (-NH-C=O-R), oxycarbonylamino (-NH-C=O-OR), sulfonylamino ($-NH-SO_2-R$), aminosulfonyl ($-SO_2-NHR$), sulfamoylamino ($-NH-SO_2-NHR$), thiocarboxyl (-C(=O)-SH), and carboxylcarbonyl ($-C(=O)-CO_2H$).

Examples of oxy (-OR) include alkoxy, cycloalkoxy, alkenyloxy, alkynyloxy, aryloxy, heteroaryloxy, and aralkyloxy. The alkoxy is preferably C1-C4 alkoxy and C1-C2 alkoxy, and particularly preferably methoxy or ethoxy.

Examples of carbonyl (-C=O-R) include formyl (-C=O-H), alkylcarbonyl, cycloalkylcarbonyl, alkenylcarbonyl, alkynylcarbonyl, arylcarbonyl, heteroarylcarbonyl, and aralkylcarbonyl.

Examples of oxycarbonyl (-C=O-OR) include alkyloxycarbonyl, cycloalkyloxycarbonyl, alkenyloxycarbonyl, alkynyloxycarbonyl, aryloxycarbonyl, heteroaryloxycarbonyl, and aralkyloxycarbonyl.

Examples of carbonyloxy (-O-C=O-R) include alkylcarbonyloxy, cycloalkylcarbonyloxy, alkenylcarbonyloxy, alkynylcarbonyloxy, arylcarbonyloxy, heteroarylcarbonyloxy, and aralkylcarbonyloxy.

Examples of thiocarbonyl (-C=O-SR) include alkylthiocarbonyl, cycloalkylthiocarbonyl, alkenylthiocarbonyl, alkynylthiocarbonyl, arylthiocarbonyl, heteroarylthiocarbonyl, and aralkylthiocarbonyl.

Examples of carbonylthio (-S-C=O-R) include alkylcarbonylthio, cycloalkylcarbonylthio, alkenylcarbonylthio, alkynylcarbonylthio, arylcarbonylthio, heteroarylcarbonylthio, and aralkylcarbonylthio.

Examples of aminocarbonyl (-C=O-NHR) include alkylaminocarbonyl (examples of which include C1-C6 or C1-C4 alkylaminocarbonyl, in particular, ethylaminocarbonyl and methylaminocarbonyl), cycloalkylaminocarbonyl, alkenylaminocarbonyl, alkynylaminocarbonyl, arylaminocarbonyl, heteroarylaminocarbonyl, and aralkylaminocarbonyl. Additional examples include compounds in which the H atom bonded to the N atom in -C=O-NHR is further replaced with alkyl, cycloalkyl, alkenyl, alkynyl, aryl, heteroaryl, or aralkyl.

Examples of carbonylamino (-NH-C=O-R) include alkylcarbonylamino, cycloalkylcarbonylamino, alkenylcarbonylamino, alkynylcarbonylamino, arylcarbonylamino, heteroarylcarbonylamino, and aralkylcarbonylamino. Additional examples include compounds in which the H atom bonded to the N atom in -NH-C=O-R is further replaced with alkyl, cycloalkyl, alkenyl, alkynyl, aryl, heteroaryl, or aralkyl.

Examples of oxycarbonylamino (-NH-C=O-OR) include alkoxycarbonylamino, cycloalkoxycarbonylamino, alkenyloxycarbonylamino, alkynyloxycarbonylamino, aryloxycarbonylamino, heteroaryloxycarbonylamino, and aralkyloxycarbonylamino. Additional examples include compounds in which the H atom bonded to the N atom in -NH-C=O-OR is further replaced with alkyl, cycloalkyl, alkenyl, alkynyl, aryl, heteroaryl, or aralkyl.

Examples of sulfonylamino ($-NH-SO_2-R$) include alkylsulfonylamino, cycloalkylsulfonylamino, alkenylsulfonylamino, alkynylsulfonylamino, arylsulfonylamino, heteroarylsulfonylamino, and aralkylsulfonylamino. Additional examples include compounds in which the H atom attached to the N atom in $-NH-SO_2-R$ is further replaced with alkyl, cycloalkyl, alkenyl, alkynyl, aryl, heteroaryl, or aralkyl.

Examples of aminosulfonyl ($-SO_2-NHR$) include alkylaminosulfonyl, cycloalkylaminosulfonyl, alkenylaminosulfonyl, alkynylaminosulfonyl, arylaminosulfonyl, heteroarylaminosulfonyl, and aralkylaminosulfonyl. Additional examples include compounds in which the H atom attached to the N atom in $-SO_2-NHR$ is further replaced with alkyl, cycloalkyl, alkenyl, alkynyl, aryl, heteroaryl, or aralkyl.

Examples of sulfamoylamino ($-NH-SO_2-NHR$) include alkylsulfamoylamino, cycloalkylsulfamoylamino, alkenylsulfamoylamino, alkynylsulfamoylamino, arylsulfamoylamino, heteroarylsulfamoylamino, and aralkylsulfamoylamino. The two H atoms bonded to the N atoms in $-NH-SO_2-NHR$ may be further replaced with substituents independently selected from the group consisting of alkyl, cycloalkyl, alkenyl, alkynyl, aryl, heteroaryl, and aralkyl, and these two substituents may form a ring.

Substituents containing an S atom include groups such as thiol (-SH), thio (-S-R), sulfinyl (-S=O-R), sulfonyl ($-SO_2-R$), and sulfo ($-SO_3H$).

Examples of thio (-S-R) include alkylthio, cycloalkylthio, alkenylthio, alkynylthio, arylthio, heteroarylthio, and aralkylthio.

Examples of sulfonyl ($-SO_2-R$) include alkylsulfonyl, cycloalkylsulfonyl, alkenylsulfonyl, alkynylsulfonyl, arylsulfonyl, heteroarylsulfonyl, and aralkylsulfonyl.

Substituents containing an N atom include groups such as azido ($-N_3$, also called "azido group"), cyano (-CN), primary amino ($-NH_2$), secondary amino (-NH-R; also called monosubstituted amino), tertiary amino (-NR(R'); also called disubstituted amino), amidino ($-C(=NH)-NH_2$), substituted amidino (-C(=NR)-NR'R"), guanidino ($-NH-C(=NH)-NH_2$), substituted guanidino (-NR-C(=NR"')-NR'R"), aminocarbonylamino (-NR-CO-NR'R"), pyridyl, piperidino, morpholino, and azetidinyl.

Examples of secondary amino (-NH-R; monosubstituted amino) include alkylamino, cycloalkylamino, alkenylamino, alkynylamino, arylamino, heteroarylamino, and aralkylamino.

Examples of tertiary amino (-NR(R'); disubstituted amino) include amino groups having any two substituents each independently selected from alkyl, cycloalkyl, alkenyl, alkynyl, aryl, heteroaryl, and aralkyl, such as alkyl(aralkyl)amino, where any two such substituents may form a ring. Specific examples include dialkylamino, in particular, C1-C6 dialkylamino, C1-C4 dialkylamino, dimethylamino, and diethylamino. The term "$C_p$-$C_q$ dialkylamino group" as used herein refers to an amino group substituted with two $C_p$-$C_q$ alkyl groups, where the two $C_p$-$C_q$ alkyl groups may be the same or different.

Examples of substituted amidino (-C(=NR)-NR'R") include groups in which three substituents R, R', and R" on the N atom are each independently selected from alkyl, cycloalkyl, alkenyl, alkynyl, aryl, heteroaryl, and aralkyl, such as alkyl(aralkyl)(aryl)amidino.

Examples of substituted guanidino (-NR-C(=NR"')-NR'R") include groups in which R, R', R", and R"' are each independently selected from alkyl, cycloalkyl, alkenyl, alkynyl, aryl, heteroaryl, and aralkyl, or groups in which these substituents form a ring.

Examples of aminocarbonylamino (-NR-CO-NR'R") include groups in which R, R', and R" are each independently selected from a hydrogen atom, alkyl, cycloalkyl, alkenyl, alkynyl, aryl, heteroaryl, and aralkyl, or groups in which these substituents form a ring.

The phrase "having a heteroatom in the ring" as used herein refers to containing a heteroatom in the ring-forming atoms. Examples of such a group include a heteroaryl group such as a pyridyl group, a piperidino group, a morpholino group, and an azetidinyl group. When the heteroatom is an oxygen atom, the phrase is expressed as "having an oxygen atom in the ring," for example.

The term "translated amino acid" or "translatable amino acid" as used herein refers to an "amino acid" having a translatable side chain. In a non-limiting embodiment, the amino acid as used herein may be a translatable amino acid.

The term "drug-likeness" or "drug-like" as used herein refers to high metabolic stability and membrane permeability. The term "drug-like amino acid" as used herein refers to $\alpha$-, $\beta$-, and $\gamma$-amino acids, where one of the two hydrogen atoms of the main chain amino group ($NH_2$ group), or one or two of the hydrogen atoms of the main chain methylene group ($-CH_2-$ group), may be substituted with an alkyl group, a cycloalkyl group, an alkenyl group, an alkynyl group, an aryl group, a heteroaryl group, an aralkyl group, or the like. These substituents may be further substituted with a "substituent contributing to drug-likeness." Preferred examples of the drug-like amino acid are amino acids having a "long side chain" described below. Drug-like amino acids may also be L-amino acids, D-amino acids, $\alpha,\alpha$-disubstituted amino acids, N-substituted amino acids, or the like. Drug-like amino acids do not necessarily have to be translatable. Drug-like amino acids include side chain moieties of peptides obtained from "translated amino acids" (e.g., a D-amino acid obtained by chemically modifying D-tyrosine when a hit compound is obtained at D-tyrosine, or a $\beta$-amino acid obtained by chemically modifying $\beta$-alanine when a hit compound is obtained at $\beta$-alanine), or amino acids that can be chemically synthesized by optimizing the structure of the N-substituted moiety by chemical transformation of N-methylamino acid. Such amino acids function as a constituent of a drug-like peptide compound, and therefore are selected from a range of amino acids that can provide a drug-like peptide compound by post-translational chemical modification. For example, as described below, lysines having an aminoalkyl group are not included in drug-like amino acids when amino groups are not involved in post-translational modification. However, lysine units are included as units of drug-like amino acids when amino groups of lysines are utilized as reactive functional groups in post-translational modification (e.g., intersection units). In this manner, whether amino acids are "drug-like amino acids" is determined by the functional group that has been transformed by post-translational modification. Examples of the substituents separately defined above that can be such substituents include an ester group (-CO-OR), a thioester group (-CO-SR), a thiol group (-SH) or a protected thiol group, an amino group ($-NH_2$), a monosubstituted amino group (-NH-R) or a disubstituted amino group (-NRR'), or a protected amino group, a substituted sulfonylamino group ($-NH-SO_2-R$), an alkylborane group (-BRR'), an alkoxyborane group (-B(OR)(OR')), an azido group ($-N_3$), a keto acid group ($-CO-CO_2H$), a thiocarboxylic acid group (-CO-SH), a phosphoryl ester group (-CO-PO(R)(R')), and an acylhydroxyamino group (-NH-O-CO-R). In an embodiment of the present disclosure, an indole skeleton and/or a substituted or unsubstituted hydroxyphenyl group are not contained in a drug-like amino acid in the present disclosure; the details will be described later. One or two non-adjacent methylene groups contained in the side chain of the drug-like amino acid may be substituted with an oxygen atom, a carbonyl group (-CO-), or a sulfonyl group ($-SO_2-$).

Examples of the "substituent contributing to drug-likeness" as used herein include substituents such as halogen (such as F, Cl, Br, or I), hydroxy (-OH), alkoxy (-OR), oxy (-OR), amido (-NR-COR' or -CO-NRR'), sulfonyl (-$SO_2$-R), sulfinyl (-SOR), oxyamino (-NR-OR'), aminooxy (-O-NRR'), oxycarbonyl (-CO-OR), thiocarbonyl (-CO-SR), thiol (-SH), thio (-SR), primary amino (-$NH_2$), secondary amino (-NHR), or tertiary amino (-NRR'), sulfonylamino (-NH-$SO_2$-R), boryl (-BRR'), dioxyboryl (-B(OR)(OR')), azido (-$N_3$), carboxycarbonyl (-$COCO_2H$), phosphorylcarbonyl (-CO-PO(R)(R')), carbonyloxyamino (-NH-O-CO-R), hydroxyamino (-NR-OR'), and aminohydroxy (-O-NRR').

The term "fused-ring structure" as used herein refers to a ring structure in which a plurality of rings share two or more atoms in a cyclic compound having two or more rings. The term "fused-ring structure formed by two or more aromatic rings" refers to a ring structure in which a plurality of aromatic rings share two or more atoms in a cyclic compound having two or more aromatic rings. Without any limitation intended, examples of the fused-ring structure include an indole skeleton, a benzofuran skeleton, a benzimidazole skeleton, a quinoline skeleton, and bicyclo[4.4.0]decane.

The term "substituted hydroxyphenyl group" as used herein refers to a group in which at least one hydrogen atom of the aromatic ring of a hydroxyphenyl group is replaced with a substituent. The substituent is selected as the substituent in the aforementioned amino acid side chain is selected. Preferred examples include halogen, with fluorine being particularly preferred. Without any limitation intended, examples of the substituted hydroxyphenyl group include a 3-fluoro-4-hydroxyphenyl group. Hydrogen atoms of aromatic rings as used herein do not include H of the hydroxy group (-OH) in a hydroxyphenyl group. For example, neither the "substituted hydroxyphenyl group" nor the "unsubstituted hydroxyphenyl group" as used herein includes a methoxyphenyl group.

The term "unsubstituted hydroxyphenyl group" as used herein refers to a hydroxyphenyl group not having a substituent. A substituted hydroxyphenyl group and an unsubstituted hydroxyphenyl group may be collectively called "substituted or unsubstituted hydroxyphenyl group."

The phrase "not having a substituted or unsubstituted XX group/do not have a substituted or unsubstituted XX group " as used herein refers to having neither a substituted XX group nor an unsubstituted XX group.

The term "heterocyclic group" as used herein refers to a group having at least one heteroatom (such as N, O, or S) in the ring. The heteroatom is preferably N or O, the number of heteroatoms is preferably 1 or 2, and the ring is preferably a 4- to 6-membered ring. Preferred examples of the heterocyclic group include a pyridyl group, a piperidino group, a morpholino group, and an azetidinyl group.

The term "translational synthesis (ribosomal synthesis)" as used herein refers to synthesizing a peptide moiety from a nucleic acid (such as DNA or RNA) encoding the peptide moiety by translation. Translation is a process of obtaining a linear peptide by repeating amide bonding and/or ester bonding reaction by action of a ribosome using mRNA as a template.

The term "post-translational modification" as used herein refers to chemical reactions caused automatically by any action other than that of a libosome or caused by an enzyme or a reagent after translation. Without any limitation intended, examples include amino acid elimination reaction, cyclization reaction, desulfurization reaction, deprotection reaction, and reactions for forming a linear portion.

The phrase "substantially consisting of/substantially consist(s) of" as used herein means that main components are those listed herein (examples of which include, but are not limited to, cyclic peptide compounds, amino acids, and nucleic acids), and other components may be contained if they do not have a negative impact on the effects of an embodiment of the present invention or if they are contained in such an amount or embodiment that does not have such a negative impact. For example, components not listed herein (such as reaction by-products and unreacted products) may be contained if they do not have a negative impact on the effects of an embodiment of the present invention or if they are contained in such an amount or embodiment that does not have such a negative impact.

The phrase "substantially not comprising/ do(es) not substantially comprise" as used herein means that components listed herein (examples of which include, but are not limited to, cyclic peptide compounds, amino acids, and nucleic acids) are not contained, or even if such components are contained, they do not have a negative impact on the effects of an embodiment of the present invention or they are contained in such an amount or embodiment that does not have such a negative impact. For example, such components listed herein may be contained if they do not have a negative impact on the effects of an embodiment of the present invention or if they are contained in such an amount or embodiment that does not have such a negative impact.

As used herein, the term "negative impact" used in the context of the effects of the invention refers to an impact that negates the effects of the invention. For example, when the effects of the invention are reduced to 30%, 20%, 10%, or 5% or less based on the effects that should be naturally demonstrated as 100%, it can be said that there is a "negative impact."

For example, when the hit rate that should be naturally achieved by panning is 1% and the hit rate is reduced to 0.3% or less, it can be said that there is a "negative impact." In addition, when the percentage of hit compounds not containing Trp which should be naturally obtained by panning is 100% and the percentage of hit compounds not containing Trp is reduced to 30% or less, it can be said that there is a negative impact. However, the negative effect is not limited

to these cases.

Peptide compounds

Without any limitation intended, "peptide compounds" in the present disclosure include linear peptide compounds and cyclic peptide compounds. Peptide compounds in the present disclosure also include peptides, complexes of peptides and nucleic acids (peptide-nucleic acid complexes), and complexes of peptides, ribosomes, and nucleic acids. Without any limitation intended, peptide compounds in the present disclosure include linear peptides, cyclic peptides, complexes of linear peptide moieties and nucleic acids (linear peptide-nucleic acid complexes), and complexes of cyclic peptide moieties and nucleic acids (cyclic peptide-nucleic acid complexes). In a non-limiting embodiment, the cyclic portion of a cyclic peptide compound is formed by subjecting a ribosomally synthesized peptide compound to cyclization reaction. Herein, peptides themselves, or peptide portions in peptide-nucleic acid complexes, may be described as "peptide moieties." "Nucleic acids" in the present disclosure include DNAs, mRNAs, and tRNAs. Herein, "amino acids" constituting a peptide compound may be called "amino acid residues." Herein, peptide compounds having a cyclic portion may be called "cyclic peptide compounds." Herein, the "cyclic portion" of a peptide compound refers to a cyclic portion formed by linking two or more amino acid residues to each other. Herein, the "linear portion" used to refer to a partial structure of a cyclic peptide compound refers to a portion which is not contained in the main chain structure of a cyclic portion and which has at least one amide bond and/or at least one ester bond on the chain of the portion. "Peptide compounds" as used herein may include their pharmaceutically acceptable salts.

Herein, the "peptide moiety" of a peptide compound refers to a site in which 2 to 100, 3 to 50, or 4 to 30 amino acids are linked to each other by amide bonds and/or ester bonds. However, when the peptide moiety contains a cyclic portion, the mode of bond at the cyclization site is not particularly limited. Preferred examples of the mode of bond at the cyclization site include cyclization via covalent bonds such as amide bonds, carbon-carbon bonds, disulfide bonds, ester bonds, thioester bonds, thioether bonds, lactam bonds, bonds through a triazole structure, and bonds through a fluorophore structure, with amide bonds being particularly preferred due to high metabolic stability. Specifically, in one embodiment, cyclic peptide compounds in the present disclosure preferably have an amide bond at the cyclization site. The "mode of bond at the cyclization site" refers to a mode of bond at the site where a cyclization is occurred by cyclization reaction.

The term "cyclization reaction" as used herein refers to a reaction that forms a cyclic portion in the peptide moiety of a peptide compound. In one embodiment, peptide compounds in the present disclosure also include peptide compounds obtained by further chemically modifying or reacting a peptide compound where the cyclic portion is formed by cyclization reaction. Chemical modification or the like may also be performed before cyclization reaction.

Scheme A is an example of cyclization reaction of the peptide moiety of a peptide compound in the present disclosure. The white circle (○) unit (intersection unit), black circle (●) units (cyclic portion main chain units), square (■) units (linear portion main chain units), and triangle (▲) unit (cyclized N-terminal unit) each represent an amino acid residue constituting the peptide moiety. The respective units may be the same or different amino acids.

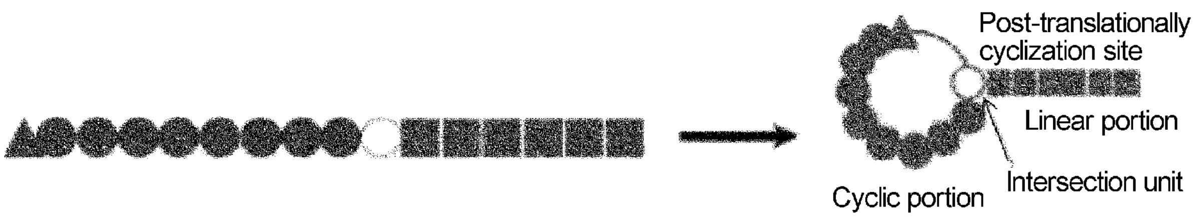


Scheme A

The term "unit" as used herein refers to an amino acid after synthesis of a linear peptide compound in the present disclosure and before cyclization, after cyclization, or at the time of completion of chemical modification after cyclization. Examples of amino acid residues at the time of completion of chemical modification after cyclization include amino acid residues in which amino acid residues translated by one tRNA are chemically or skeletally transformed by post-translational chemical modification.

In Scheme A, the cyclic portion is a portion composed of one triangle unit, eight black circle units, and one white circle unit, and the linear portion is a portion composed of six square units. The curved portion in Scheme A is the site to be cyclized (herein also called "cyclization site" or "post-translationally cyclization site").

In one embodiment, drug-like amino acids are not necessarily selected for the triangle and intersection units described in Scheme A in a peptide compound in the present disclosure before cyclization, since the peptide moiety of the peptide compound requires reactive functional groups for the triangle and intersection units.

In one embodiment, even if the triangle and intersection units are not drug-like amino acids or are naturally unfavorable amino acids in a peptide compound before cyclization, the triangle and intersection units can be transformed into drug-like amino acids and favorable amino acids by performing chemical modification or the like after cyclization.

In one embodiment, an amino acid residue which has a thiol group near an amino group and in which both the thiol group and the amino group are protected by protecting groups can be utilized for the triangle unit in the peptide moiety before cyclization (an N-terminal unit before cyclization) of a peptide compound in the present disclosure.

In one embodiment, an amino acid having in the side chain a functional group (second reaction point) that enables cyclization by reaction with a functional group (first reaction point) possessed by the triangle unit amino acid is preferably used for the intersection unit in the peptide moiety before cyclization of a peptide compound in the present disclosure. The intersection unit preferably has three or more functional groups in total, because the main chain amino group and carboxy group are used for forming covalent bonds with other amino acid residues in translational synthesis or the like and a third functional group is necessary for cyclization. Among these groups, the functional group at the side chain site of the intersection unit is preferably used for cyclization reaction.

The intersection unit can be incorporated at any position that permits cyclization in the peptide moiety before cyclization, but is preferably incorporated at a position which allows the cyclic portion after cyclization or after post-translational modification after cyclization to have 5 to 20 or 7 to 20 amino acids, more preferably 5 to 13 or 8 to 13 amino acids, and still more preferably 8 to 11 amino acids. Specifically, the intersection unit of a peptide compound in the present disclosure is preferably incorporated at a position at least four amino acid residues (e.g., 4, 5, 6, 7, 8, 9, or 10 amino acid residues) closer to the C-terminus from the triangle unit.

The black circle and square units are selected from amino acids, and are preferably selected from drug-like amino acids.

In a non-limiting embodiment, the peptide moiety of a cyclic peptide compound in the present disclosure may have one or more linear portions, and examples of the number of linear portions include 0, 1, or 2. Cyclic peptide compounds in the present disclosure can be described by schemes such as, but not particularly limited to, Scheme A-1 or A-2.

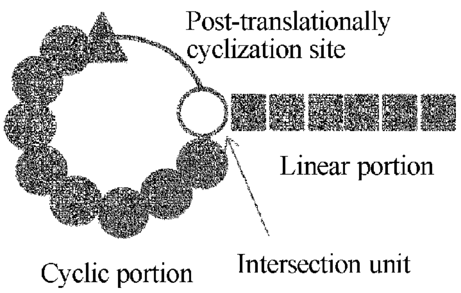


Scheme A-1

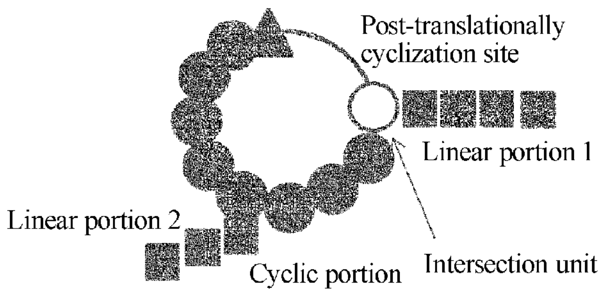


Scheme A-2

The term "number of amino acids" as used herein refers to the number of amino acid residues (amino acid units) constituting a peptide, which means the number of amino acid units that occur when the amide bonds, ester bonds, and bond at the cyclization site which link the amino acids are cleaved. Accordingly, the number of amino acids in a peptide compound or peptide moiety (also called the number of amino acids constituting a peptide compound or peptide moiety) refers to the number of amino acid units that occur when the amide bonds and ester bonds included in the entire peptide moiety excluding the nucleic acid site and including the cyclic portion and the linear portion, and bond at the cyclization site are cleaved.

The term "amino acids constituting the cyclic portion" as used herein (also called "amino acids of the cyclic portion") refers to amino acids present on the main chain of the cyclic portion among amino acids that occur when the amide bonds and ester bonds present in the peptide moiety of a cyclic peptide compound, and the bond at the cyclization site are cleaved, and such amino acids do not include amino acids constituting the linear portion. The term "number of amino acids constituting the cyclic portion" as used herein refers to the number of amino acid units present on the main chain of the cyclic portion.

The number of amino acids constituting the peptide moiety of a cyclic peptide compound in the present disclosure is not particularly limited, but is preferably 30 or less. In order to achieve high membrane permeability, the number of amino acids in the peptide moiety excluding the nucleic acid-linked portion is preferably 20 or less, more preferably 18 or less, 16 or less, 15 or less, or 14 or less, and particularly preferably 13 or less. Specific examples include 9, 10, 11, 12, 13, 14, 15, 16, 17, and 18. In order to achieve high metabolic stability, the number of amino acids in the peptide moiety excluding the nucleic acid-linked portion is preferably 8 or more, more preferably 9 or more, still more preferably

10 or more, and particularly preferably 11 or more. In order to achieve both membrane permeability and metabolic stability, the number of amino acids in the peptide moiety excluding the nucleic acid-linked portion is preferably 5 to 20 or 7 to 20, more preferably 7 to 17, 8 to 16, 9 to 16, or 10 to 16, still more preferably 8 to 13, 10 to 15, 11 to 15, 10 to 14, 10 to 13, or 11 to 14, and particularly preferably 11 to 13.

The term "peptide moiety excluding the nucleic acid-linked portion" as used herein refers to a moiety excluding the linear portion to which a nucleic acid is linked (also called "nucleic acid-linked portion") in a cyclic peptide-nucleic acid complex. For a peptide compound not having a nucleic acid-linked portion, the entire peptide moiety is called "peptide moiety excluding the nucleic acid-linked portion." For example, in Scheme A-2 above, when a nucleic acid is linked to the end of the linear portion 1, the "peptide moiety excluding the nucleic acid-linked portion" is a moiety excluding the linear portion 1, specifically, a portion in which the cyclic portion and the linear portion 2 are combined. Therefore, the "number of amino acids constituting the peptide moiety excluding the nucleic acid-linked portion" or the "number of amino acids in the peptide moiety excluding the nucleic acid-linked portion" in a cyclic peptide-nucleic acid complex refers to the number of amino acid units in a portion in which the amino acids in the linear portion to which a nucleic acid is linked are excluded from the amino acids constituting the peptide moiety. The "peptide moiety excluding the nucleic acid-linked portion" is preferably a portion excluding the nucleic acid-linked portion in a cyclic peptide-nucleic acid complex.

Examples of the number of amino acids constituting the cyclic portion of a cyclic peptide compound in the present disclosure include, but are not limited to, 4 or more, 6 or more, 7 or more, 8 or more, 9 or more, 10 or more, 11 or more, 12 or more, 20 or less, 18 or less, 16 or less, 15 or less, 14 or less, 13 or less, 12 or less, 11 or less, 7, 8, 9, 10, 11, 12, 13, 14, 15, and 16. In order to achieve both membrane permeability and metabolic stability, the number of amino acids constituting the cyclic portion is preferably 5 to 15, more preferably 5 to 14, 7 to 14, or 8 to 14, still more preferably 8 to 13, 9 to 13, 8 to 12, 8 to 11, or 9 to 12, and particularly preferably 9 to 11.

In a non-limiting embodiment, the number of amino acids (the number of units) in the linear portion is preferably 0 to 8, more preferably 0 to 5, and still more preferably 0 to 3. In a non-limiting embodiment, "linear portions" as used herein may include natural amino acids and unnatural amino acids (including chemically modified or skeletally transformed amino acids).

In a non-limiting embodiment, examples of the number of unnatural amino acids contained in the peptide moiety excluding the nucleic acid-linked portion of a cyclic peptide compound in the present disclosure preferably include 2 or more, more preferably 4 or more, 5 or more, or 6 or more, still more preferably 7 or more, and particularly preferably 8 or more, and also preferably include 20 or less, 15 or less, 14 or less, 13 or less, 12 or less, 10 or less, or 9 or less. Examples of the number of unnatural amino acids contained in a cyclic peptide compound in the present disclosure include 30% or more, 40% or more, 50% or more, 60% or more, 70% or more, or 80% or more relative to the number of amino acids constituting the cyclic portion or the number of amino acids contained in the peptide moiety excluding the nucleic acid-linked portion.

In a non-limiting embodiment, examples of the number of N-substituted amino acids contained in the peptide moiety excluding the nucleic acid-linked portion of a cyclic peptide compound in the present disclosure preferably include 3 or more, more preferably 4 or more, 5 or more, or 6 or more, still more preferably 7 or more, and particularly preferably 8 or more, and also preferably include 20 or less, 15 or less, 14 or less, 13 or less, 12 or less, 10 or less, or 9 or less. Examples of the number of N-substituted amino acids contained in a cyclic peptide compound in the present disclosure include 30% or more, 40% or more, 50% or more, 60% or more, 70% or more, or 80% or more relative to the number of amino acids constituting the cyclic portion or the number of amino acids contained in the peptide moiety excluding the nucleic acid-linked portion. N-substituted amino acids as used herein may be preferably N-alkylamino acids, and more preferably N-methylamino acids. Specifically, in a non-limiting embodiment, the number of N-alkylamino or N-methylamino acids is provided as an example of the number of the N-substituted amino acids. Even in this case, it is obviously possible that other N-substituted amino acids may be contained in the peptide moiety excluding the nucleic acid-linked portion.

Without any limitation intended, as shown in the Examples provided below, the number or percentage of aromatic rings contained in the peptide moiety excluding the nucleic acid-linked portion, in the cyclic portion, or in the side chains of the cyclic portion may affect membrane permeability of a cyclic peptide compound. Even in the case of a cyclic peptide compound not containing tryptophan and tyrosine residues, if the number or percentage of aromatic rings contained in the peptide moiety excluding the nucleic acid-linked portion, in the cyclic portion, or in the side chains of the cyclic portion exceeds a certain value, the probability of obtaining a compound having high membrane permeability will be reduced.

In a non-limiting embodiment, in order to achieve high membrane permeability, the number of aromatic rings contained in the peptide moiety excluding the nucleic acid-linked portion, in the cyclic portion, or in the side chains of the cyclic portion of a cyclic peptide compound in the present disclosure is preferably 3 of less. Preferred examples include 0, 1, 2, or 3, and examples of preferred ranges include from 0 to 3 or from 1 to 3. The percentage of the number of aromatic rings contained in the peptide moiety excluding the nucleic acid-linked portion, in the cyclic portion, or in the side chains of the cyclic portion relative to the number of amino acids constituting the peptide moiety excluding the

nucleic acid-linked portion or constituting the cyclic portion is preferably 40% or less. Preferred examples include 35% or less, 30% or less, 27% or less, 25% or less, and 20% or less.

In a non-limiting embodiment, in order to achieve high membrane permeability, a cyclic peptide compound in the present disclosure preferably has an aromatic ring in the long side chains of the cyclic portion which are sites that can contribute to binding to a target molecule, since the number of aromatic rings that may be contained in the compound is limited. Specifically, in one embodiment, examples of the number of aromatic rings contained in the long side chains of the cyclic portion of a cyclic peptide compound in the present disclosure include 1, 2, or 3, and examples of ranges include from 1 to 3 or from 2 to 3. Examples of the percentage of the number of aromatic rings contained in the long side chains of the cyclic portion relative to the number of aromatic rings contained in the peptide moiety excluding the nucleic acid-linked portion, in the cyclic portion, or in the side chains of the cyclic portion of a cyclic peptide compound in the present disclosure include 30% or more, 45% or more, 60% or more, 80% or more, or 100%.

The term "number of aromatic rings" as used herein (also called "aromatic ring count" (ARC)) refers to the number of aromatic rings contained in the peptide moiety excluding the nucleic acid-linked portion, in the cyclic portion, or in the side chains of the cyclic portion of a cyclic peptide compound. For example, the number is 1 for a phenol group, 2 for fused bicyclic rings such as an indole skeleton, and 3 for fused tricyclic rings such as anthracene.

In a non-limiting embodiment, the ClogP of a cyclic peptide compound in the present disclosure is preferably 4 or more, more preferably 5 or more, still more preferably 6 or more, and particularly preferably 8 or more, and preferred examples include 18 or less, 17 or less, or 16 or less. Examples include from 4 to 18, from 5 to 17, and from 6 to 16. The ClogP as used herein is a distribution coefficient calculated by a computer and can be calculated using Daylight Version 4.9 of Daylight Chemical Information Systems, Inc.

In a non-limiting embodiment, the ClogP/total aa of a cyclic peptide compound in the present disclosure is preferably 1.0 or more, more preferably 1.1 or more, and still more preferably 1.2 or more, and preferred examples include 1.8 or less, 1.7 or less, 1.6 or less, or 1.5 or less. Examples include from 1.0 to 1.8, from 1.0 to 1.7, from 1.1 to 1.6, and from 1.1 to 1.5. The term "total aa" as used herein (also expressed as "total AA") refers to the number of amino acids constituting the peptide moiety excluding the nucleic acid-linked portion of a peptide compound. For example, the total aa is 11 for a cyclic peptide compound in which the cyclic portion is composed of 10 amino acids and the linear portion to which a nucleic acid not linked is composed of one amino acid. The ClogP/total aa as used herein is calculated by dividing ClogP by total aa.

In a non-limiting embodiment, the molecular weight of a peptide compound in the present disclosure, or its peptide moiety excluding the nucleic acid-linked portion may be 500 to 2000.

Herein, the term "side chain" is used in the context of side chains of amino acids, side chains of cyclic moieties of cyclic peptide compounds, or such, and refers to a moiety not contained in each main chain structure. However, when a nucleic acid is attached to the peptide moiety of a cyclic peptide compound, the nucleic acid moiety and the nucleic acid-linked portion are not included in the side chains of the cyclic portion.

Amino acid side chains as used herein include chains attached to carbon atoms contained in amino acids (such as α-, β-, or γ-carbon atoms), and chains attached to nitrogen atoms. Herein, the length of an amino acid side chain can be determined by the following method. Specifically, the length can be determined by capping the N-terminus and the C-terminus of an amino acid unit with an acetyl group and a methylamino group, respectively, generating a conformation with Low Mode MD of molecular modeling software MOE (Chemical Computing Group), and measuring the distance from the atom to which the side chain moiety is attached (an α-carbon atom (Cα carbon) in the case of natural amino acids) to the most distal atom of the same side chain (excluding a hydrogen atom). A method of determining the length of the side chain of Phe or nBuGly is illustrated below as an example.

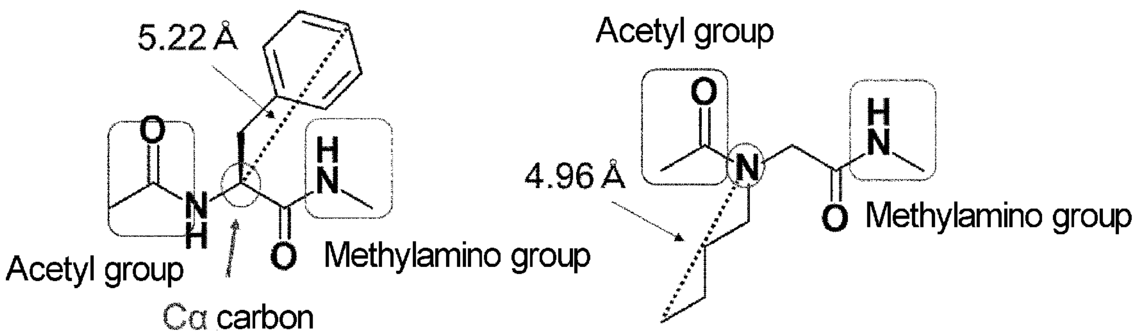


A method of determining the length of an amino acid side chain, Phe (left), nBuGly (right)

When an amino acid side chain forms a ring with part of the main chain structure, there are a plurality of atoms

to which the side chain moiety is attached. In this case, the length of the side chain is the longest distance among the distances determined by calculating as described above for each atom to which the side chain moiety is attached and the most distal atom of the side chain (excluding a hydrogen atom). Taking Hyp(Et) as an example, the length of the Hyp(Et) side chain is 6.09 angstroms, because the length of the side chain determined from the nitrogen atom is 6.04 angstroms and the length of the side chain determined from the Cα carbon is 6.09 angstroms.

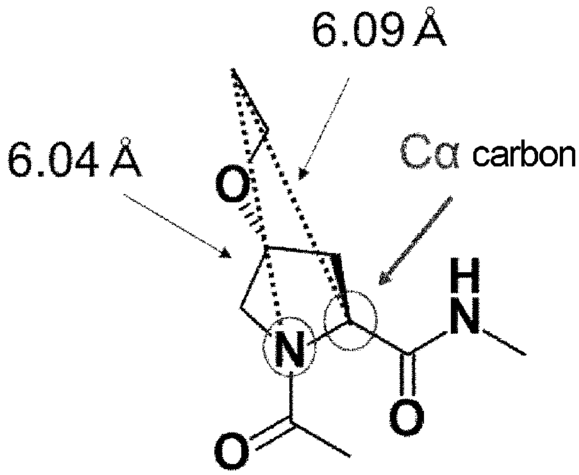


A method of determining the length of an amino acid side chain, Hyp (Et)

The lengths of the amino acid side chains calculated by the above method are shown in Tables 1-1 and 1-2.

[Table 1-1]

| Amino acid | Length of side chain (angstrom) |
|---|---|
| MePhe (4-Cl) | 6.79 |
| Phe (4-$CF_3$) | 7.32 |
| Trp | 6.47 |
| Hyp(Et) | 6.09 |
| Phe (3-Cl) | 6.08 |
| Tyr (3-F) | 6.40 |
| Ph e | 5.22 |
| Met (O2) | 5.35 |
| nBuGly | 4.96 |

[Table 1-2]

| Amino acid | Length of side chain (angstrom) |
|---|---|
| Hph | 6.64 |
| Ser (Ph-2-Cl) | 6.51 |
| Ser (3-F-5-Me-Pyr) | 8.11 |
| Ly s (Ac) | 8.47 |
| Ser (Et-2-Mor) | 8.71 |

The term "side chain of the cyclic portion" as used herein refers to a moiety not contained in the main chain structure of the cyclic portion of a cyclic peptide compound. The origins of side chains of the cyclic portion (the atoms

to which the side chain sites are attached) is not particularly limited. Examples of the origins include carbon and nitrogen atoms constituting the cyclic portion. Specific examples of such carbon atoms include α-, β-, and γ-carbon atoms of amino acids constituting the cyclic portion. The lengths of the side chains of the cyclic portion of a cyclic peptide compound can be calculated using the above-described method of calculating the length of an amino acid side chain for each amino acid constituting the cyclic portion. The "side chain of the cyclic portion" as used herein may have an amide bond and/or an ester bond thereon. For example, when a cyclic peptide compound has a linear portion constituted by three or more amino acids (e.g., a linear portion 2 of Scheme A-2 constituted by three square units) and the linear portion is not linked to a nucleic acid, the linear portion can also be a "side chain of the cyclic portion." The term "side chain of the peptide moiety" as used herein refers to an amino acid side chain constituting the peptide moiety.

In a non-limiting embodiment, amino acids constituting the cyclic portion or peptide moiety of a cyclic peptide compound in the present disclosure may contain at least one, two, or three amino acids having a long side chain. Amino acids having a long side chain may be present continuously or may be present with other amino acids therebetween. Cyclic peptide compounds in the present disclosure may contain at least one, two, or three long side chains in the cyclic portion or peptide moiety. In one embodiment, cyclic peptide compounds in the present disclosure may have a long side chain in the cyclic portion or peptide moiety. This long side chain allows the cyclic peptide compound to have high binding affinity to a target molecule. In one embodiment, appropriate selection of a long side chain can provide a cyclic peptide compound having high membrane permeability with binding affinity to a target molecule maintained.

The term "long side chain" as used herein refers to a side chain 5.4 angstroms or more in length. The length of the side chain is preferably 5.8 angstroms or more or 6.0 angstroms or more, more preferably 6.2 angstroms or more, and particularly preferably 6.4 angstroms or more. Examples of the upper limit of the length of the side chain include, but are not particularly limited to, 20 angstroms or less, 15 angstroms or less, 13 angstroms or less, 12 angstroms or less, 11 angstroms or less, 10 angstroms or less, 9.0 angstroms or less, 8.8 angstroms or less, 8.5 angstroms or less, and 8.0 angstroms or less. Examples of the length of the side chain include 5.4 to 20 angstroms, 5.4 to 15 angstroms, 5.4 to 13 angstroms, 5.4 to 10 angstroms, 5.4 to 9.0 angstroms, 6.0 to 20 angstroms, 6.0 to 15 angstroms, 6.0 to 13 angstroms, 6.0 to 10 angstroms, 6.0 to 9.0 angstroms, 6.0 to 8.5 angstroms, and 6.0 to 8.0 angstroms. In one embodiment, the "long side chain" is more preferably (i) a side chain containing no amide bond or one amide bond in the side chain and may be (ii) a side chain containing no amide bond in the side chain. Specifically, in a non-limiting embodiment, preferred examples include a cyclic peptide compound having a long side chain in the cyclic portion, which side chain does not have two or more amide bonds in the side chain and is 6.0 to 10 angstroms in length. Long side chains in the present disclosure may or may not have an aromatic ring. In a non-limiting embodiment, cyclic peptide compounds in the present disclosure may have an aromatic ring in at least one or more long side chains contained in the cyclic portion.

In the "long side chain" as used herein, the number of atoms on the chain between the atom to which the side chain moiety is attached (an α-carbon atom in the case of natural amino acids) and the most distal atom of the same side chain (excluding a hydrogen atom) may be 6 or more, 7 or more, or 8 or more and may be 15 or less, 13 or less, 11 or less, 10 or less, 9 or less, or 8 or less. Specific examples of the number of atoms include 6, 7, 8, 9, 10, 11, 12, 13, 14, and 15 or less. When counting the "number of atoms on the chain between the atom to which the side chain site is attached and the most distal atom of the same side chain," the atom to which the side chain site is attached (which may be an α-carbon atom in the case of natural amino acids but, for example, may be a nitrogen atom in the case of N-substituted amino acids) is included. Specifically, for example, the number of atoms is 4 for Leu, 6 for Phe, and 7 for Tyr.

In a non-limiting embodiment, the long side chain as used herein may contain $R^1$ defined below.

In a non-limiting embodiment, the long side chain as used herein may be a side chain represented by the following general formula (1-1) or (1-2).

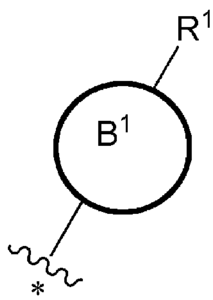

(I − 1)

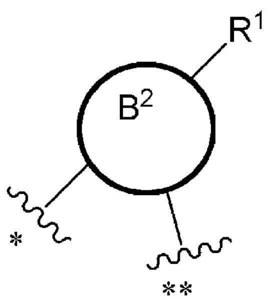

(I − 2)

Herein,

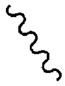

represents the point of attachment,

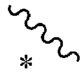

represents the point of attachment to a nitrogen or carbon atom, and

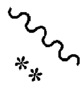

represents the point of attachment to a carbon atom.

The carbon atom to which

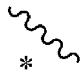

is attached may be the same as or different from the carbon atom to which

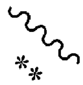

is attached.

The $B^1$ is preferably a single bond, an optionally substituted C1-C5 alkylene group, or a C3-C8 cycloalkylene group, where one or two non-adjacent methylene groups in the alkylene or cycloalkylene group may be substituted with an oxygen atom. When $B^1$ is an alkylene group, the long side chain as used herein is also represented by the following general formula (1-3):

$$-(CH_2)_n-R^1 \qquad (1\text{-}3)$$

where

n is an integer of 1 to 5; and
one or two non-adjacent methylene groups in the alkylene group may be substituted with an oxygen atom.

The $B^2$ is preferably a group represented by the following general formula (1-4) or (1-5):

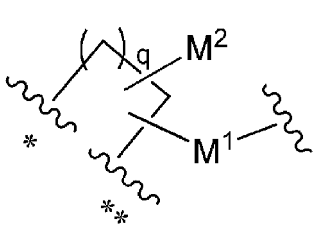

、

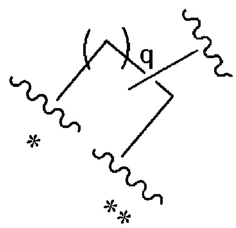

(I－4) (I－5)

wherein

q is an integer of 1 to 3;

$M^1$ represents an optionally substituted C1-C4 alkylene group, wherein one or two non-adjacent methylene groups in the alkylene group are optionally substituted with an oxygen atom;
$M^2$ represents a hydrogen atom or an optionally substituted C1-C4 alkyl group; and
$M^1$ and $M^2$ are optionally taken together with the atom(s) to which they are attached to form a 3-to 6-membered ring structure, wherein the ring structure is optionally substituted with a C1-C4 alkyl group or a halogen atom and optionally has an oxygen atom in the ring.

The following structures are examples of the partial structure of an amino acid having a side chain wherein $M^1$ and $M^2$ are taken together with the atom(s) to which they are attached to form a 3- to 6-membered ring structure in the general formula (1-4).

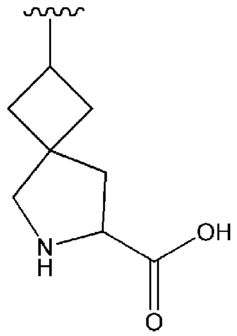
、
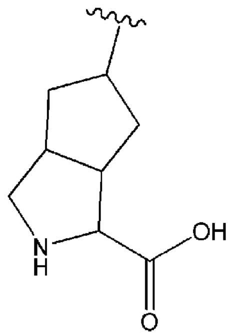

(I−4') (I−4'')

In a non-limiting embodiment, $R^1$ in the present disclosure is optionally:

(a) a phenyl group, or (b) a 4- to 6-membered heterocyclic group having 1 to 3 heteroatoms independently selected from the group consisting of an oxygen atom and a nitrogen atom in the ring,
wherein (a) and (b) optionally have at least one substituent independently selected from Group A below:
Group A: oxo, a halogen atom, a C1-C4 alkyl group optionally substituted with a halogen atom, and a C1-C4 dialkylamino group (wherein one or two non-adjacent methylene groups in the C1-C4 alkyl group are optionally substituted with an oxygen atom);
or
(c) a group represented by the following general formula (II), (III), or (IV):

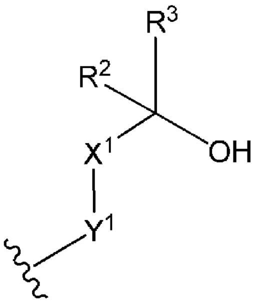
、
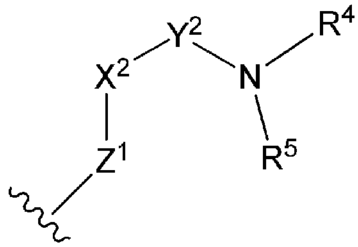
、
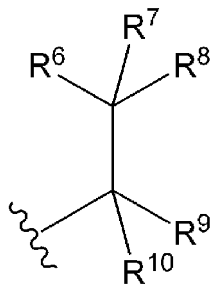

(II) (III) (IV)

wherein

$X^1$ represents a single bond, or a C1-C2 alkylene group optionally having at least one substituent independently selected from Group B below,
$X^2$ represents a single bond, or a C1-C2 alkylene group optionally having at least one substituent independently selected from Group B below,
$Y^1$ represents a single bond, an oxygen atom, a carbonyl group (-CO-), or a sulfonyl group ($-SO_2-$),
$Y^2$ represents a single bond, a C1-C2 alkylene group optionally substituted with a C1-C4 alkyl group, a carbonyl group (-CO-), or a sulfonyl group ($-SO_2-$),

$Z^1$ represents a single bond, a methylene group optionally having at least one substituent independently selected from Group B below, or an oxygen atom,
$R^2$ and $R^3$ each independently represent a hydrogen atom, a C1-C6 alkyl group, a C2-C4 alkenyl group, or a C2-C4 alkynyl group, wherein the C1-C6 alkyl group is optionally substituted with a halogen atom, and one or two non-adjacent methylene groups in the C1-C6 alkyl group are optionally substituted with an oxygen atom,
$R^2$ and $R^3$, or $R^2$ and $X^1$ are optionally taken together with the atom(s) to which they are attached to form a 4- to 6-membered ring structure, wherein the ring structure may be substituted with a C1-C4 alkyl group or a halogen atom, optionally has an oxygen atom in the ring, and is optionally partially unsaturated,
$R^4$ represents a C1-C6 alkyl group, a C2-C6 alkenyl group, a C2-C6 alkynyl group, or a C1-C6 alkanoyl group,
$R^5$ represents a hydrogen atom, a C1-C6 alkyl group, a C2-C6 alkenyl group, or a C2-C6 alkynyl group, wherein one or two non-adjacent methylene groups in the C1-C6 alkyl group of $R^4$ and/or $R^5$ are optionally substituted with an oxygen atom,
$R^4$ and $R^5$, or $R^5$ and $X^2$ are optionally taken together with the atom(s) to which they are attached to form a 4- to 6-membered ring structure, where the ring structure is optionally substituted with a C1-C4 alkyl group or a halogen atom, optionally has an oxygen atom in the ring, and is optionally partially unsaturated, and
$R^6$, $R^7$, $R^8$, $R^9$, and $R^{10}$ each independently represent a hydrogen atom, a halogen atom, a C1-C4 alkyl group, a C2-C4 alkenyl group, or a C2-C4 alkynyl group, wherein one or two non-adjacent methylene groups in the C1-C4 alkyl group are optionally substituted with an oxygen atom;
Group B: a halogen atom, a C1-C4 alkyl group, and a C1-C2 alkoxy group.

In one embodiment, when the $M^1$ and $M^2$ are taken together with the atom(s) to which they are attached to form a ring structure, $R^1$ is optionally a group selected from the group consisting of a halogen atom, an optionally substituted C1-C4 alkyl group, and a C1-C4 alkoxy group, in addition to the above groups.

In a non-limiting embodiment, in the general formula (II), $Y^1$ is preferably an oxygen atom, $X^1$ is preferably an optionally substituted C1-C2 alkylene group, in particular, an unsubstituted C1-C2 alkylene group, and $R^2$ and $R^3$ are each independently preferably a hydrogen atom or a C1-C6 alkyl group, wherein the C1-C6 alkyl group is preferably substituted with a halogen atom.

In a non-limiting embodiment, when $R^5$ of the general formula (III) is a hydrogen atom, $Y^2$ is preferably a single bond, a carbonyl group, or a sulfonyl group, in particular, a carbonyl group, $X^2$ is preferably an optionally substituted C1-C2 alkylene group, in particular, an unsubstituted C1-C2 alkylene group, and $Z^1$ is preferably an oxygen atom.

In a non-limiting embodiment, when $R^5$ of the general formula (III) is a C1-C6 alkyl group and does not form a ring structure, $Y^2$ is preferably a single bond, an optionally substituted C1-C2 alkylene group, a carbonyl group, or a sulfonyl group, in particular, an unsubstituted C1-C2 alkylene group or a carbonyl group, $X^2$ is preferably a single bond, an optionally substituted C1-C2 alkylene group, or an unsubstituted C1-C2 alkylene group, and $Z^1$ is preferably a single bond, an optionally substituted methylene group, or an unsubstituted methylene group.

In a non-limiting embodiment, when $R^4$ and $R^5$ of the general formula (III) are taken together with the atom(s) to which they are attached to form a 4- to 6-membered ring structure, the ring structure optionally has an oxygen atom in the ring, the ring structure is optionally substituted with one or more C1-C4 alkyl groups and/or halogen atoms, $X^2$ is preferably a single bond, an optionally substituted C1-C2 alkylene group, or an unsubstituted C1-C2 alkylene group, $Y^2$ is preferably a single bond or an optionally substituted C1-C2 alkylene group, in particular, an unsubstituted C1-C2 alkylene group, and $Z^1$ is preferably a single bond, an optionally substituted methylene group, an unsubstituted methylene group, or an oxygen atom.

In a non-limiting embodiment, when $R^5$ and $X^2$ of the general formula (III) are taken together with the atom(s) to which they are attached to form a 4- to 6-membered ring structure, the ring structure optionally has an oxygen atom in the ring, the ring structure is optionally substituted with one or more C1-C4 alkyl groups and/or halogen atoms, $Y^2$ is preferably a single bond or an optionally substituted C1-C2 alkylene group, in particular, an unsubstituted C1-C2 alkylene group, $Z^1$ is preferably an oxygen atom, and $R^4$ is preferably a C1-C4 alkyl group.

In a non-limiting embodiment, $R^6$, $R^7$, $R^8$, $R^9$, and $R^{10}$ of the general formula (IV) are each independently preferably a hydrogen atom, a halogen atom, a methyl group, or an ethyl group. The halogen atom is preferably F or Cl, and more preferably F.

In a non-limiting embodiment, when the phenyl group of (a) in the $R^1$ has a substituent, the substituent is preferably a halogen atom, a C1-C4 haloalkyl group, or a C1-C4 dialkylamino group, and particularly preferably F, Cl, a fluoromethyl group (such as a trifluoromethyl group or a difluoromethyl group), or a dimethylamino group. When a plurality of substituents are selected, they are optionally the same or different.

In a non-limiting embodiment, the 4- to 6-membered heterocyclic group having 1 to 3 heteroatoms independently selected from the group consisting of an oxygen atom and a nitrogen atom in the ring of (b) in the $R^1$ is preferably a pyridyl group, a piperidino group, a morpholino group, or an azetidinyl group, and such a group optionally has at least one substituent independently selected from Group A below:

Group A: oxo, a halogen atom, a C1-C4 alkyl group optionally substituted with a halogen atom, and a C1-C4 dialkylamino group (wherein one or two non-adjacent methylene groups in the C1-C4 alkyl group are optionally substituted with an oxygen atom).

The halogen atom is preferably F and Cl. The C1-C4 alkyl group optionally substituted with a halogen atom is more preferably a fluoromethyl group (such as a trifluoromethyl group or a difluoromethyl group), a methyl group, and an ethyl group. A preferred example of the C1-C4 dialkylamino group is a dimethylamino group. When a plurality of substituents are selected, they are optionally the same or different.

In a non-limiting embodiment, examples of the optionally substituted phenyl group in the present disclosure include an unsubstituted phenyl group, a phenyl group substituted with 1 to 5, preferably 1 to 3, halogen atoms (a halophenyl group), a phenyl group substituted with a C1-C4 alkyl group optionally substituted with a halogen atom (a C1-C4 alkyl group or a C1-C4 haloalkyl group), (a C1-C4 alkylphenyl group or a C1-C4 haloalkylphenyl group), and a dialkylaminophenyl group. Specific examples include a chlorophenyl group (such as a 2-, 3-, or 4-chlorophenyl group), a dichlorophenyl group, a trichlorophenyl group, a fluorophenyl group (such as a 2-, 3-, or 4-fluorophenyl group), a difluorophenyl group, a trifluorophenyl group, a fluoromethylphenyl group (such as a trifluoromethylphenyl group or a difluoromethylphenyl group), and a dimethylaminophenyl group.

In a non-limiting embodiment, examples of the optionally substituted pyridyl group in the present disclosure include an unsubstituted pyridyl group, a chloropyridyl group (such as a 2-, 3-, or 4-chloropyridyl group), a fluoropyridyl group (such as a 2-, 3-, or 4-fluoropyridyl group), a methylpyridyl group, an ethylpyridyl group, and a dimethylaminopyridyl group.

In a non-limiting embodiment, examples of the optionally substituted piperidino group in the present disclosure include an unsubstituted piperidino group, a chloropiperidino group (such as a 2-, 3-, or 4-chloropiperidino group), a dichloropiperidino group (such as a 2,2-, 3,3-, or 4,4-dichloropiperidino group), a fluoropiperidino group (such as a 2-, 3-, or 4-fluoropiperidino group), a difluoropiperidino group (such as a 2,2-, 3,3-, or 4,4-difluoropiperidino group), a methylpiperidino group, and an ethylpiperidino group.

In a non-limiting embodiment, examples of the optionally substituted morpholino group in the present disclosure include an unsubstituted morpholino group, a chloromorpholino group (such as a 2- or 3-chloromorpholino group), a fluoromorpholino group (such as a 2- or 3-fluoromorpholino group), a methylmorpholino group, and an ethylmorpholino group.

In a non-limiting embodiment, examples of the optionally substituted azetidinyl group in the present disclosure include an unsubstituted azetidinyl group, a chloroazetidinyl group (such as a 1-, 2-, or 3-chloroazetidinyl group), a fluoroazetidinyl group (such as a 1-, 2-, or 3-fluoroazetidinyl group), a methylazetidinyl group (such as a 1-, 2-, or 3-methylazetidinyl group or a N-methylazetidinyl group), and an ethylazetidinyl group (such as a 1-, 2-, or 3-ethylazetidinyl group).

Although the "long side chain" as used herein is not particularly limited, examples of amino acids having the long side chain are provided in Tables 2-1 to 2-6. Without any limitation intended, side chain moieties of the amino acids (longest side chain moieties when one amino acid has two or more side chains) are provided as examples of preferred embodiments of the long side chain as used herein.

[Table 2-1]

| Chem code | structure |
|---|---|
| MeHnl(7-F2) | 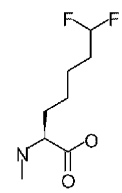 |
| Ser(F(4)nPr) | 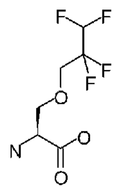 |

(continued)

| Chem code | structure |
|---|---|
| MeSer(F(4)nPr) | 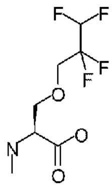 |
| Ser(EtOH) | 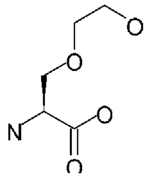 |
| MeSer(EtOH) | 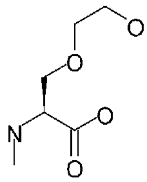 |
| Ser( nPr) | 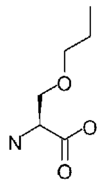 |
| MeSer(nPr) | 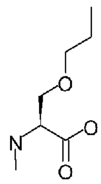 |
| Ser(iPen) | 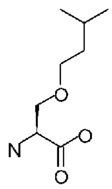 |
| MeSer(iPen) | 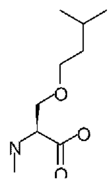 |

(continued)

| Chem code | structure |
|---|---|
| Hnl(7-F2) | 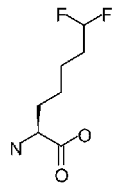 |
| MeSer(tBuOH) | 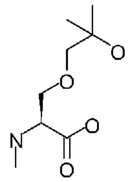 |
| Ser(2-Me-BuOH) | 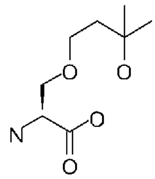 |
| MeSer(2-Me-BuOH) | 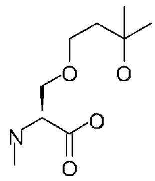 |
| Gln(Me2) | 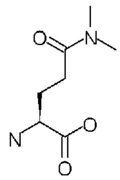 |
| MeGln(Me2) | 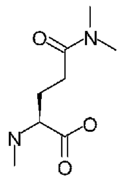 |
| Ser(S-2-PrOH) | 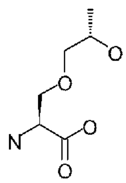 |

(continued)

| Chem code | structure |
|---|---|
| MeSer(S-2-PrOH) | 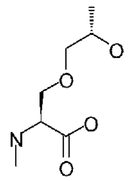 |
| Ser(R-2-PrOH) | 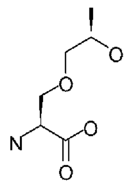 |
| MeSer(R-2-PrOH) | 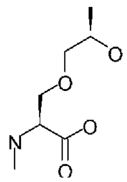 |
| Ser(tBuOH) | 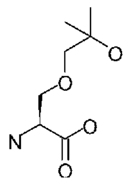 |

[Table 2-2]

| Chem code | structure |
|---|---|
| MeSer(Ph-2-Cl) | 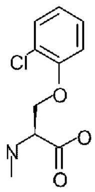 |
| Ser(Ph-3-Cl) | 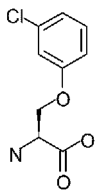 |
| MeSer(Ph-3-Cl) | 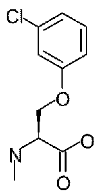 |

(continued)

| Chem code | structure |
|---|---|
| Hph(2-Cl) | 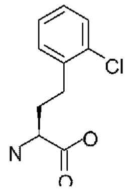 |
| MeHph(2-Cl) | 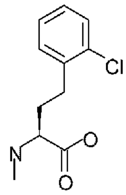 |
| Ser(NtBu-Aca) | 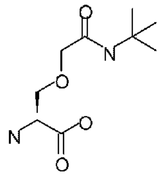 |
| MeSer(NtBu-Aca) | 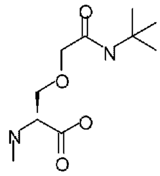 |
| Hph | 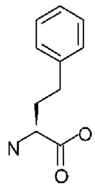 |
| MeHph | 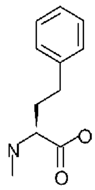 |
| Ser(Ph-2-Cl) | 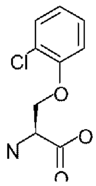 |

(continued)

| Chem code | structure |
|---|---|
| MeSer(3-F-5-Me-Pyr) | 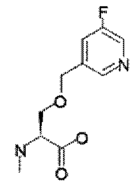 |
| Phe{#(CH2)2} | 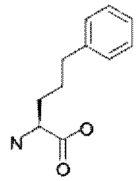 |
| MePhe{#(CH2)2} | 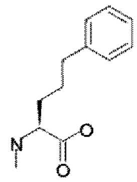 |
| Ser(Bn) | 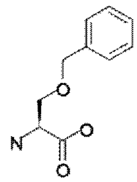 |
| MeSer(Bn) | 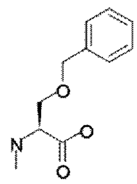 |
| Hph(3-Cl) | 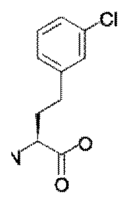 |
| MeHph(3-Cl) | 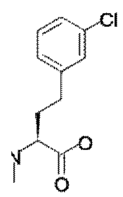 |

(continued)

| Chem code | structure |
|---|---|
| Hph(4-Cl) | 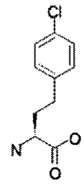 |
| MeHph(4-Cl) | 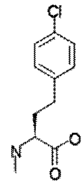 |
| Ser(3-F-5-Me-Pyr) | 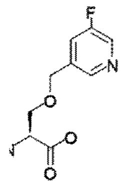 |

[Table 2-3]

| Chem code | structure |
|---|---|
| MeAla(3-Pyr-4-NMe2) | 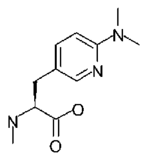 |
| nPenGly | 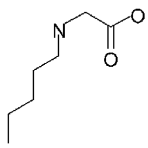 |
| nHexGly | 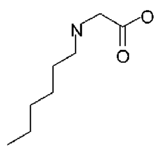 |
| (PhEt)NGly | 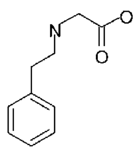 |

(continued)

| Chem code | structure |
|---|---|
| (EtOEt)NGly | 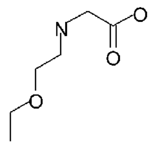 |
| (PhOEt)NGly | 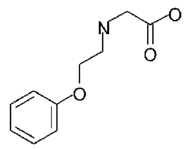 |
| Abu(pip-4-F2) | 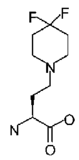 |
| MeAbu(pip-4-F2) | 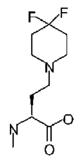 |
| Abu(pip-3-F2) | 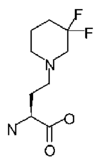 |
| MeAbu(pip-3-F2) | 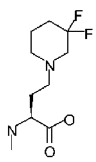 |
| Ala(3-Pyr-4-NMe2) | 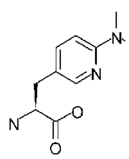 |
| Ser(Ph-4-Cl) | 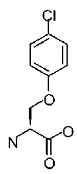 |

(continued)

| Chem code | structure |
|---|---|
| MeSer(Ph-4-Cl) | 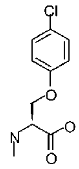 |
| Hyp(Et) | 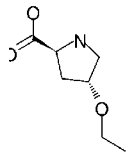 |

[Table 2-4]

| Chem code | structure |
|---|---|
| Ser(Et-2-Mor) | 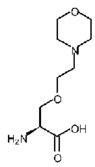 |
| MeSer(Et-2-Mor) | 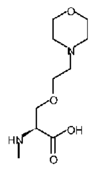 |
| Abu(5-Oxo-Odz) | 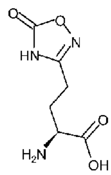 |
| MeAbu(5-Oxo-Odz) | 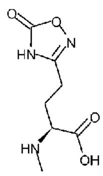 |
| Phe(4-CHF2) | 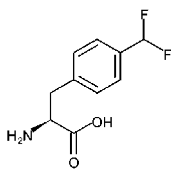 |

(continued)

| Chem code | structure |
|---|---|
| MeGln(Me) | 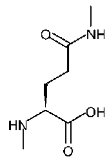 |
| Ahp(2)(3-R-OH) | 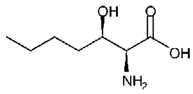 |
| MeAhp(2)(3-R-OH) | 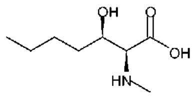 |
| Lys(Ac) | 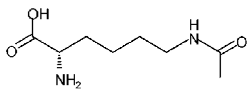 |
| MeLys(Ac) | 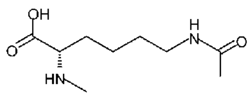 |
| MeAbu(Mor) | 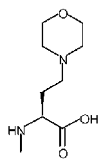 |
| (2-(pip-4-F2)-Et)Gly | 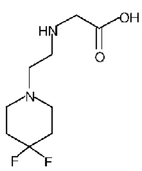 |
| Pro(4-pip-4-F2) | 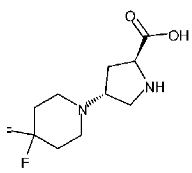 |
| cisPro(4-pip-4-F2) | 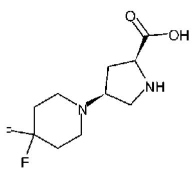 |

(continued)

| Chem code | structure |
|---|---|
| Gln(Me) | 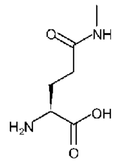 |
| Hse(Et) | 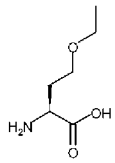 |
| MeHse(Et) | 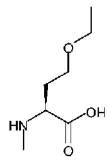 |
| Nle(6-OH) | 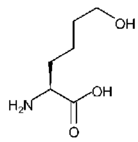 |
| MeNle(6-OH) | 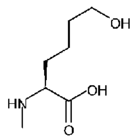 |
| Abu(Mor) | 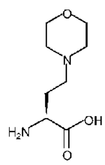 |

[Table 2-5]

| Chem code | structure |
|---|---|
| Phe(4-Cl) | 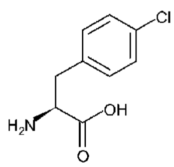 |

(continued)

| Chem code | structure |
|---|---|
| MePhe(4-Cl) | 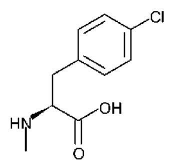 |
| Phe(4-CF3) | 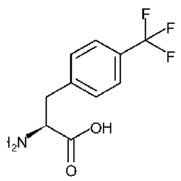 |
| MePhe(4-CF3) | 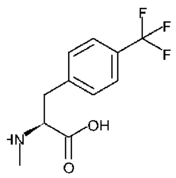 |
| MePhe(4-CHF2) | 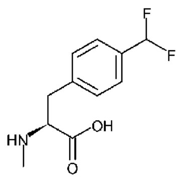 |
| Phe(4-OCHF2) | 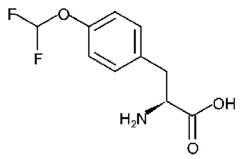 |
| MePhe(4-OCHF2) | 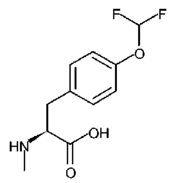 |
| Phe(3-Cl) | 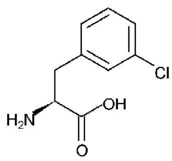 |

(continued)

| Chem code | structure |
| --- | --- |
| MePhe(3-Cl) | 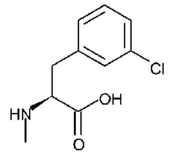 |

[Table 2-6]

| Chem code | structure |
| --- | --- |
| Ser(1-CF3-EtOH) | 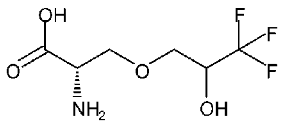 |
| MeSer(1-CF3-EtOH) | 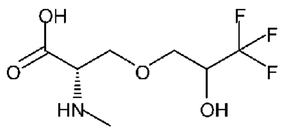 |
| HnI(7-F3-6-OH) | 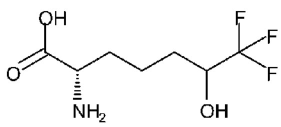 |
| MeHnI(7-F3-6-OH) | 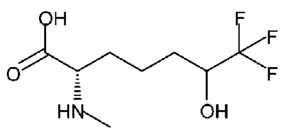 |

In a non-limiting embodiment, in order for cyclic peptide compounds in the present disclosure to have high membrane permeability, it is preferred that the compounds do not have a side chain having a negative impact on membrane permeability. In one embodiment, even when peptide compounds before cyclization include the side chain having a negative impact on membrane permeability, high membrane permeability can be achieved if cyclic peptide compounds after cyclization do not include such a side chain. Accordingly, in one embodiment, cyclic peptide compounds that do not have a side chain having a negative impact on membrane permeability can be obtained, for example by chemical modification after cyclization reaction. In a non-limiting embodiment, in the optimization step after obtaining cyclic peptide compounds that can specifically bind to target molecules, for example, such compounds can be chemically modified to improve membrane permeability. In doing this, side chains having a negative impact on membrane permeability may be modified.

In a non-limiting embodiment, cyclic peptide compounds in the present disclosure may be cyclic peptide compounds which do not have at least one selected from the group consisting of (A) to (D) below in the side chains of the cyclic portion or of the peptide moiety excluding the nucleic acid-linked portion: (A) an indole skeleton; (B) a fused-ring structure formed by two or more aromatic rings; (C) an unsubstituted hydroxyphenyl group; and (D) a substituted hydroxyphenyl group. Among these, cyclic peptide compounds which do not have (A), (C), and (D) above in the side chains of the cyclic portion are preferred, and cyclic peptide compounds which do not have (B), (C), and (D) above in the side chains of the cyclic portion are more preferred. In a non-limiting embodiment, the side chains of the cyclic portion may be long side chains of the cyclic portion. In a non-limiting embodiment, the "fused-ring structure formed by two or more aromatic rings" may be a "fused-ring structure."

In a non-limiting embodiment, cyclic peptide compounds in the present disclosure may be cyclic peptide compounds which do not have at least one or both selected from the group consisting of (A) and (B) below in the side chains of the cyclic portion or of the peptide moiety excluding the nucleic acid-linked portion: (A) a methylthio group; and a (B) thiol group.

In a non-limiting embodiment, it is preferred that cyclic peptide compounds in the present disclosure do not have a functional group that is extremely ionized at a neutral pH (for example, pH = 7.0) in the peptide moiety excluding the nucleic acid-linked portion or in the cyclic portion, in order to have high membrane permeability. The term pKa as used herein refers to an observed pKa unless otherwise indicated. pKa values determined by ADMET Predictor described below are called calculated pKas. The term basic pKa as used herein refers to an observed basic pKa unless otherwise indicated. Basic pKa values determined by ADMET Predictor described below are called calculated basic pKas.

pKas and basic pKas can be measured by a conventional method. For example, they can be measured by a method such as that described in Experimental Chemistry Lecture 5, "Thermal Measurements and Equilibrium", p. 460 (edited by The Chemical Society of Japan, published by Maruzen Co., Ltd.). More specifically, they can be measured by a method described in Examples. When it is difficult to determine the pKa and basic pKa values of the side chain to be measured of an amino acid due to influence of other functional groups, the other functional groups can be appropriately protected by protecting groups or the like so that only the pKa and basic pKa of the functional group of interest can be measured.

In a non-limiting embodiment, when a cyclic peptide compound in the present disclosure has an acidic side chain in the cyclic portion, the acidic side chain has a pKa of preferably 3.5 or more, more preferably 3.9 or more, still more preferably 4.5 or more, yet more preferably 5.0 or more, even more preferably 5.5 or more, still yet more preferably 5.7 or more, and preferably 10 or less. Preferred examples of the pKa include a range from 3.5 to 10, from 3.9 to 10, from 4.5 to 10, from 5.0 to 10, or from 5.5 to 10, from 5.7 to 10. When these pKas are expressed as calculated values, a calculated pKa is preferably 3.5 or more, more preferably 4.5 or more, still more preferably 5.0 or more, yet more preferably 5.4 or more, even more preferably 8.0 or more, still yet more preferably 8.3 or more, and preferably 10 or less. Preferred examples of the calculated pKa include a range from 3.5 to 10, from 4.5 to 10, from 5.0 to 10, from 5.4 to 10, from 8.0 to 10, or from 8.3 to 10.

In a non-limiting embodiment, when a cyclic peptide compound in the present disclosure has a basic side chain in the cyclic portion, the basic side chain has a basic pKa of preferably 10 or less, more preferably 9.5 or less, still more preferably 9.0 or less, yet more preferably 8.5 or less, even more preferably 7.5 or less, still yet more preferably 7.2 or less, particularly preferably 6.5 or less, and preferably 4.0 or more. Preferred examples of the basic pKa include a range from 4.0 to 10, from 4.0 to 9.5, from 4.0 to 9.0, from 4.0 to 8.5, from 4.0 to 7.5, from 4.0 to 7.2, or from 4.0 to 6.5. When these basic pKas are expressed as calculated values, a calculated basic pKa is preferably 10 or less, more preferably 9.5 or less, still more preferably 9.0 or less, yet more preferably 8.8 or less, even more preferably 8.6 or less, still yet more preferably 8.5 or less, still even more preferably 7.5 or less, particularly preferably 6.5 or less, and more preferably 4.0 or more. Preferred examples of the calculated basic pKa include a range from 4.0 to 10, from 4.0 to 9.5, from 4.0 to 9.0, from 4.0 to 8.8, from 4.0 to 8.6, from 4.0 to 8.5, from 4.0 to 7.5, or from 4.0 to 6.5.

Herein, the "acidic side chain" refers to a side chain having a pKa of 10 or less, and the "basic side chain" refers to a side chain having a basic pKa of 4 or more. Herein, side chains having a pKa of more than 10 and side chains having a basic pKa of less than 4 are defined as neutral side chains.

Herein, calculated pKas and calculated basic pKas of amino acid side chains or of side chains of the cyclic portion of a cyclic peptide compound can be determined using ADMET Predictor (Simulations Plus Inc., ver. 8.0). Calculated pKas and calculated basic pKas are calculated using a partial structure obtained by separating the side chain moiety starting from the position β of the side chain (the carbon directly attached to the main chain). The case of Lys is provided below as an example. The calculated basic pKa was calculated to be 10.5 using a partial structure including the position β of the side chain (the carbon directly attached to the main chain). When similarly calculating for acids, the side chain carboxy group of Asp had a calculated pKa of 4.3, the side chain phenolic hydroxyl group of Tyr had a calculated pKa of 9.9, the side chain phenolic hydroxyl group of 3-fluorotyrosine (Tyr(3-F)) had a calculated pKa of 8.7, and tetrazole had a calculated pKa of 3.7. On the other hand, for bases, the side chain guanidino group of Arg had a calculated basic pKa of 12.7, the imidazolyl group of His had a calculated basic pKa of 7.6, and pyridine had a calculated

basic pKa of 5.4. In the present disclosure, at least one side chain selected from the group consisting of amino acid side chains, and side chains, long side chains, and basic side chains of the cyclic portion of a cyclic peptide compound may be a side chain not having a proton donor.

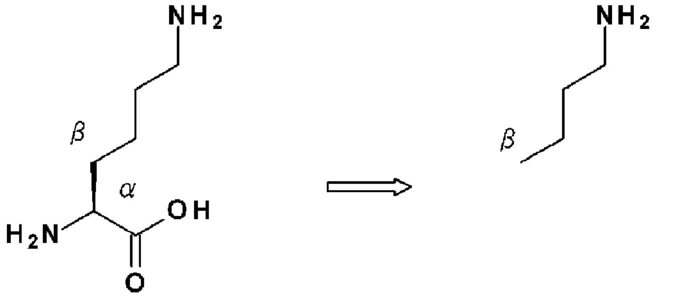

Lys Partial structure

Some amino acids having basic side chains whose basic pKas were calculated by a method described herein are provided in the following table.

[Table 33]

| X | Ser(Et-2-NMe2) | MeAbu(pip-4-F2) | MeAbu(Mor) | Ser(Et-2-Mor) | MeAbu(pip-3-F2) |
|---|---|---|---|---|---|
| Amino acid structure | 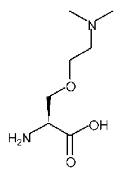 | 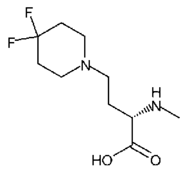 | 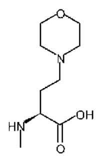 | 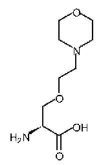 | 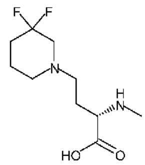 |
| basic pKa | 8.9 | 8.6 | 8.2 | 7.1 | 6.8 |

Some amino acids having acidic side chains whose pKas were calculated by a method described herein are provided in the following table.

[Table 34]

| X | Abu(5-Oxo-Odz) | Gln(Ms) |
|---|---|---|
| Amino acid structure | 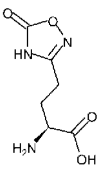 | 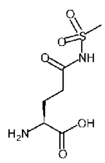 |
| pKa | 8.3 | 5.3 |

In a non-limiting embodiment, peptide compounds in the present disclosure may be subjected to post-translational modification. Post-translational modification may be performed before cyclization and/or after cyclization. Examples of post-translational modification include, but are not particularly limited to, amino acid elimination reaction, cyclization reaction, desulfurization reaction, deprotection reaction, and reactions for forming a linear portion.

Without any limitation intended, amino acid elimination reactions as used herein include reactions of removing N-terminal amino acids. N-terminal amino acids may be translation initiation amino acids, specific examples of which include fMet (formylmethionine), fNle (formylnorleucine), Met, and Nle. In one embodiment, reactions of removing N-terminal amino acids of peptide compounds include reactions of treating with peptide deformylase (PDF) and/or methionine aminopeptidase (MAP).

Peptide-nucleic acid complexes

In one embodiment, the peptide compounds in the present disclosure may be complexes formed between a peptide and a nucleic acid (peptide-nucleic acid complexes). The peptide-nucleic acid complexes may be formed through linkage between the C-terminal side of the peptide moiety and the nucleic acid. The peptide and the nucleic acid may be linked through a linker (spacer), but the mode of linkage is not particularly limited thereto. In one embodiment, the nucleic acid complexed with the peptide moiety may be a DNA or an RNA, preferably an RNA, and particularly preferably an mRNA. In one embodiment, the nucleic acid complexed with the peptide may be a template used for ribosomal synthesis of the peptide moiety, and the template is preferably an mRNA. In a more specific embodiment, a preferred example is a peptide compound in which the C terminus of the peptide moiety is conjugated to the 3'-end of the template mRNA *via* a linker (spacer) containing the antibiotic puromycin, which is an aminoacyl-tRNA analog.

Cell membrane permeability of peptide compounds

Herein, "cell membrane permeability" is sometimes referred to as "membrane permeability". Methods that use human colon cancer cell line Caco-2 cells have been reported as methods for measuring the membrane permeability of drugs and such (this is also referred to as "conventional methods". Artursson, P., 2001. Adv Drug Deliv Rev 46, 27-43; Mason, A.K., 2013. Drug Metab Dispos 41, 1347-1366; Polli, J.W., 2001. J Pharmacol Exp Ther 299, 620-628; and Sun, H. 2008. Expert Opin Drug Metab Toxicol 4, 395-411). These methods are methods of measuring membrane permeability of a substance to be measured (also called a "test substance") based on the membrane permeability coefficient ($P_{app}$) calculated from the amount of test substance which transferred to the basement membrane side (Basal side) after adding the test substance to the luminal side (Apical side) of the cultured Caco-2 cell layer (see Fig. 1). The permeability of Caco-2 cells evaluated by such an experiment has been found to be correlated with oral absorptivity in humans, and oral absorptivity in humans can be predicted from this correlation. Since Caco-2 cells form a monolayer membrane when cultured under specified conditions, and permeability of a drug to such a monolayer membrane shows good correlation with human oral absorptivity, it is widely used as a method for evaluating oral absorptivity *in vitro.* However, as described below, accurately measuring membrane permeability of highly lipid-soluble drugs and such is difficult using such conventional methods.

In a membrane permeability test using Caco-2 cells, as a prerequisite in calculating the membrane permeability coefficient ($P_{app}$) using Equation 1 below, it is assumed that the intracellular distribution of the drug can be ignored, that the drug concentration on the Basal side increases linearly, that a drug once permeates does not go back into the cell (*i.e.,* a sink condition), that change in concentration on the Apical side is small, that intracellular accumulation of the drug is not taken into account, and such (Bhoopathy, S., et al., 2014. Methods Mol Biol 1113, 229-252; Knipp, G.T., et al., 1997. J Pharm Sci 86, 1105-1110; Korzekwa, K.R., et al., 2012. Drug Metab Dispos 40, 865-876; and Sun, H., et al., 2008. Drug Metab Dispos 36, 102-123).

[Equation 1]

$$P_{app} = \frac{1}{C_1(0) \times S} \times \frac{dQ}{dt} \qquad \text{(Equation 1)}$$

dQ/dt is the permeation rate of the drug (the amount of drug that appears on the Basal side per unit time), S is the surface area of whole cells, and $C_1(0)$ is the concentration of the drug added to the Apical side

However, it has been reported that there are compounds for which the prerequisites in calculating $P_{app}$ using the above-mentioned Equation 1 are not applicable. For example, Ozeki *et al.* reported that they conducted membrane permeability tests using Caco-2 cells on drugs that permeate the membrane by passive diffusion (atenolol, metoprolol, and propranolol) and on P-glycoprotein (P-gp) substrates (digoxin, cyclosporine, and verapamil), and as a result of evaluating the changes in concentrations on the Apical side, inside the cell, and on the Basal side, it was found that for metoprolol and propranolol, 120 minutes of incubation caused decrease in the concentrations on the Apical side by approximately 20% and by approximately 40%, respectively (Ozeki K., et al., 2015, Int J Pharm. 495, 963-971).

That is, a large change in concentration on the Apical side is suggested. In addition, regarding the drugs propranolol, cyclosporine, and verapamil, approximately 8% of the added drugs were distributed in the cells after completion of incubation, suggesting intracellular accumulation of the drugs. Furthermore, a lagtime (intersection between the linear approximation of the linear region and the x axis) was present in the change in the concentration of cyclosporine on the Basal side (Ozeki K., et al., 2015, Int J Pharm. 495, 963-971), and the time until the linear portion starts (three

times the lagtime) was over 300 minutes in both the test where a drug was added to the Apical side and sampling was performed from the Basal side (AtoB) and the test where a drug was added to the Basal side and sampling was performed from the Apical side (BtoA); therefore, this revealed that a linear region does not exist in the 120-minute incubation time. More specifically, this suggests that drug concentration on the Basal side does not increase linearly. $P_{app}$ calculated using Equation 1 from only the Basal-side concentration after the 120-minute incubation in the AtoB test gave a five-fold underestimated value compared to $P_{app}$ calculated using an optimized value from a model that adjusts for the intracellular distribution. It is considered that the cause for this is the strong cyclosporine binding to the intracellular matrix, which leads to delayed appearance of cyclosporine on the Basal side (Ozeki K., et al., 2015, Int J Pharm. 495, 963-971). In addition, it has been reported that for a compound whose $P_{app}$ value is 2.5 x $10^{-5}$ cm/sec, the linear region ends within the 120-minute incubation time (also referred to as "plateau"). Furthermore, since a drug that has a large ClogP binds strongly to the intracellular matrix, start of the linear region is delayed, and accordingly, accurately evaluating $P_{app}$ of a highly lipid soluble drug is suggested to be difficult using conventional methods (Fig. 2).

Accordingly, the present inventors developed methods which improve the conventional method to measure the membrane permeability of peptide compounds, particularly cyclic peptide compounds. As shown in the Examples, use of this improved method, more specifically a method for measuring membrane permeability in the present disclosure, has enabled accurate measurement of membrane permeability of peptide compounds. Note, however, that test substances that can be measured by this improved method are not limited to peptide compounds.

In conventional methods, membrane permeability is measured without conducting a pre-incubating step under conditions where the test substance is mixed. Although conventional methods sometimes do involve incubation performed for a short period of time before membrane permeability measurements for buffer conditioning, this is not incubation under conditions where the test substance is mixed, and is different from the pre-incubation in the present disclosure.

In a non-limiting embodiment, the methods for measuring membrane permeability in the present disclosure (also referred to as the "measurement method in the present disclosure") comprise the step of pre-incubating Caco-2 cells in the mixed presence with a test substance. In the measurement methods in the present disclosure, "pre-incubation" and "pre-incubate (pre-incubating)" refer to incubating Caco-2 cells in the mixed presence with a test substance, prior to the step of measuring membrane permeability. Herein, "(pre)incubating Caco-2 cells in the mixed presence with a test substance" is not particularly limited as long as the state in which Caco-2 cells and a test substance co-exist occurs during the (pre)incubation, and for example, the (pre)incubation may be carried out by mixing Caco-2 cells and a test substance in a medium, and then starting the incubation. Alternatively, it may refer to placing a solution containing the test substance so that it contacts with one side of a Caco-2 cell layer, placing a solution not containing the test substance so that it contacts with the other side of the Caco-2 cell layer, and incubating for a predetermined period of time. Herein, at the start of the pre-incubation, the test substance is added to the solution on the Apical side and the test substance is not added to the solution on the Basal side, unless particularly stated otherwise.

In a non-limiting embodiment of the measurement methods in the present disclosure, during the pre-incubation, incubation can be carried out by placing a solution containing the test substance on the Apical side of the Caco-2 cell layer, or on its opposite side. The pre-incubation time in the measurement methods in the present disclosure is not particularly limited, but examples include 2 hours or more, 4 hours or more, 6 hours or more, 8 hours or more, 12 hours or more, 18 hours or more, 20 hours or more, and 24 hours or more. The upper limit of the pre-incubation time is not limited as long as it does not affect the membrane permeability measurements, but a length of time that does not cause damage on the Caco-2 cells is preferred, and examples include 72 hours or less, 48 hours or less, 36 hours or less, 30 hours or less, 26 hours, or 24 hours or less.

Herein, "pre-incubation solution" refers to a solution which is made to contact with Caco-2 cells during the pre-incubation. While the pre-incubation solution of the measurement method in the present disclosure is not particularly limited, use of a medium is preferred from the viewpoint of not causing damage on Caco-2 cells by the pre-incubation. More specifically, pre-incubation in the present disclosure can be performed by contacting Caco-2 cells with a medium. Herein, "not cause(causing) damage on Caco-2 cells" can be checked by measuring the transepithelial electrical resistance (TEER) of Caco-2 cells. Herein, if the TEER value at the time of measurement is 70% or higher, or preferably 80% or higher compared to the initial value before the pre-incubation, it is determined that Caco-2 cells are not damaged at the time of measurement. TEER can be measured by methods known to those skilled in the art.

In a non-limiting embodiment, in the pre-incubation of the measurement methods in the present disclosure, a medium may be used as the pre-incubation solution. In one embodiment, in the pre-incubation in the present disclosure, the pre-incubation solution on the Apical side may be a medium. In one embodiment, as the pre-incubation solution, the medium may be used only on the Apical side or the Basal side, and while the medium may be used on both sides, the medium is preferably used at least on the Apical side. In one embodiment, it is preferred that the test substance is included in the pre-incubation solution on the Apical side of the Caco-2 cells. Herein, the medium is not particularly limited, but media that do not cause damage on Caco-2 cells are preferred, cell culture media are more preferred, and non-essential amino acid-containing media are even more preferred. Specific examples of the media include Dulbecco's modified Eagle's medium (DMEM), MEM, and RPMI 1640 medium, and among them, DMEM is preferred. In one embodiment, other components may be added to the media, and while the components that can be added are not particularly limited, examples include organic solvents and solubilizing agents, and specific examples include fasted state simulated intestinal and stomach fluids (FaSSIF), dimethyl sulfoxide (DMSO), dimethyl acetamide (DMA), methanol, acetonitrile, surfactants, and Tween. Herein, the phrase such as "the medium is DMEM" does not exclude the case where other components are included in the medium. The medium and/or the added components on the Apical side and the Basal side may be the same or different. Preferred examples of the medium on the Apical side are a mixed medium of FaSSIF and DMEM, and preferred examples of the medium on the Basal side are DMEM. In a non-limiting embodiment, while the pH of the pre-incubation solution is not particularly limited, examples include pH 5.0 to pH 8.0. Examples of the pre-incubation temperature include 5°C to 45°C, preferably 20°C to 40°C, and more preferably 37°C.

The measurement methods in the present disclosure include the step of measuring membrane permeability. In this step, membrane permeability of a test substance can be measured by placing a solution containing the test substance so that it contacts with one side of a Caco-2 cell layer, placing a solution not containing the test substance so that it contacts with the other side of the Caco-2 cell layer, and after a predetermined period of time, measuring the amount of the test substance in the solution on said other side and/or the amount of the test substance in the solution on said one side.

The aforementioned "step of measuring membrane permeability" in the measurement methods of the present disclosure can be carried out according to conventional methods. In one embodiment, after the pre-incubation step, the pre-incubation solutions on the Apical side and the Basal side are removed by aspiration, a solution containing the test substance is added to the Apical side and a solution not containing the test substance is added to the Basal side, and membrane permeability can be measured. In the step of measuring membrane permeability, for example, membrane permeability can be measured by adding a mixed solution of FaSSIF and Hanks' Balanced Salt Solutions (HBSS) containing the test substance (containing 1% DMSO) (pH 6.0) to the Apical side and adding HBSS (4% BSA) (pH 7.4) to the Basal side, culturing under conditions of 37°C, measuring the amount of the test substance on the Basal side by LC/MS after a predetermined period of time, and calculating the membrane permeability coefficient from the amount of permeation.

The compounds (test substances) measured by the measurement method in the present disclosure are not particularly limited, but examples include highly lipid-soluble compounds for which accurate measurement of membrane permeability is difficult by conventional methods. Examples of highly lipid-soluble compounds include compounds having ClogP of 3 or higher, 4 or higher, 5 or higher, 6 or higher, or 8 or higher. Furthermore, examples of highly lipid-soluble compounds include compounds having ClogP/total aa of 0.8 or higher, 1.0 or higher, 1.1 or higher, 1.2 or higher, or 1.3 or higher. In a non-limiting embodiment, the test substances may be peptide compounds, and preferably cyclic peptide compounds.

In a non-limiting embodiment, by measuring the membrane permeability of a plurality of test substances by the measurement methods in the present disclosure, test substances having the desired membrane permeability can be selected from the plurality of test substances based on the membrane permeability data obtained from this measurement. More specifically, herein, methods of screening for test substances using the measurement methods in the present disclosure are disclosed.

In a non-limiting embodiment, test substances having membrane permeability at the level required for development as pharmaceuticals can be selected by methods of screening for test substances using the measurement methods in the present disclosure. The criteria used in this case can be selected depending on the purpose of the screening, such as location of the target molecule in a living body or administration route, and examples of the membrane permeability coefficient ($P_{app}$) include 5.0 x $10^{-7}$ cm/sec or greater, 8.0 x $10^{-7}$ cm/sec or greater, 9.0 x $10^{-7}$ cm/sec or greater, 1.0 x $10^{-6}$ cm/sec or greater, and 3.0 x $10^{-6}$ cm/sec or greater. Herein, "$10^{-n}$" may be expressed as "E-n" (for example, 1.0 x $10^{-6}$ may be expressed as 1.0E-6 or 1.0E-06).

The measurement methods (improved methods) in the present disclosure enable appropriate evaluation of membrane permeability of highly lipid-soluble test substances which were difficult to evaluate by conventional methods. Therefore, highly lipid-soluble test substances can also be subjected to test substance screening using criteria similar to that used in conventional methods. For example, by using measurement methods in the present disclosure, $P_{app}$ of 1.0 x $10^{-6}$ cm/sec or greater can be set as a criterion for enabling development as oral drugs as in conventional methods (P. Arturssonand J. et al., 1991, Biochem Biophys Res Commun. 175, 880-885).

In a non-limiting embodiment, the test substances selected by the screening methods in the present disclosure may be peptide compounds, and are particularly preferably cyclic peptide compounds. In a non-limiting embodiment, after selecting a peptide compound having the desired features by the aforementioned screening method, a peptide compound having the desired features can be produced based on the amino acid sequence of the selected peptide compound.

The present disclosure also provides pre-incubation methods which are included in the above-mentioned measurement methods in the present disclosure. More specifically, in one aspect, the present disclosure provides pre-incubation methods for membrane permeability measurements. The description, examples, preferred range, embodiments,

and such of the pre-incubation methods in the present disclosure are as described above.

Non-limiting embodiments of methods of measuring membrane permeability in the present disclosure include the following:

<1> a method of measuring membrane permeability of a test substance using Caco-2 cells, which comprises the step of pre-incubating Caco-2 cells in the mixed presence with the test substance;
<2> a method of measuring membrane permeability of a test substance using Caco-2 cells, which comprises the step of pre-incubating Caco-2 cells in the mixed presence with the test substance for two hours or more;
<3> the method of <1> or <2>, which comprises the step of pre-incubating Caco-2 cells in the mixed presence with the test substance for four hours or more;
<4> the method of any one of <1> to <3>, which comprises the step of pre-incubating Caco-2 cells in the mixed presence with the test substance for six hours or more;
<5> the method of any one of <1> to <4>, which comprises the step of pre-incubating Caco-2 cells in the mixed presence with the test substance for eight hours or more;
<6> the method of any one of <1> to <5>, which comprises the step of pre-incubating Caco-2 cells in the mixed presence with the test substance for twelve hours or more;
<7> the method of any one of <1> to <6>, which comprises the step of pre-incubating Caco-2 cells in the mixed presence with the test substance for 24 hours or more;
<8> the method of any one of <1> to <7>, wherein pre-incubation time in the pre-incubating step is 48 hours or less;
<9> a pre-incubation method for measuring membrane permeability, which comprises incubating Caco-2 cells in the mixed presence with a test substance for four hours or more;
<10> the method of <9>, which comprises the step of incubating Caco-2 cells in the mixed presence with the test substance for 24 hours or more;
<11> the method of any one of <1> to <8>, wherein the pre-incubation is performed by contacting the Caco-2 cells with a medium;
<12> the method of <11>, wherein the medium is a cell culture medium;
<13> the method of <11>, wherein the medium is DMEM;
<14> the method of any one of <1> to <13>, wherein the test substance is a substance with ClogP of 3 or greater;
<15> the method of any one of <1> to <14>, wherein the test substance is a peptide compound;
<16> the method of any one of <1> to <15>, wherein the test substance is a cyclic peptide compound;
<17> the method of any one of <1> to <16>, which further comprises the step of measuring membrane permeability of the test substance by placing a solution containing the test substance so that it contacts with one side of a Caco-2 cell layer, placing a solution not containing the test substance so that it contacts with the other side of the Caco-2 cell layer, and after a predetermined period of time, measuring the amount of the test substance in the solution on said one side and/or the amount of the test substance in the solution on said other side;
<18> a method of screening for a test substance, which comprises the steps of:

(a) measuring membrane permeability of a plurality of test substances by the method of any one of <1> to <17>; and
(b) selecting a desired test substance from the plurality of test substances based on the membrane permeability data obtained in (a);

<19> the method of <18>, wherein the step (b) includes selecting a test substance having a membrane permeability coefficient ($P_{app}$) of 1.0 x $10^{-6}$ cm/second or greater;
<20> a method of producing a peptide compound, which comprises the steps of:

(i) measuring membrane permeability of a plurality of peptide compounds by the method of any one of <1> to <17>; and
(ii) selecting a desired peptide compound from the plurality of peptide compounds based on the membrane permeability data obtained in (i); and
(iii) producing a peptide compound based on the amino acid sequence of the peptide compound selected in (ii); and

<21> a peptide compound produced by the method of <20>.

The membrane permeability of peptide compounds in the present disclosure can be measured by the methods for measuring membrane permeability in the present disclosure. More specifically, membrane permeability can be measured by the following method. A mixed medium of FaSSIF and DMEM containing 1% DMSO is added as the medium

on the Apical side, DMEM is added as the medium on the Basal side, and under conditions of 5% $CO_2$, 37°C, and 80 rpm, Caco-2 cells and the peptide compounds are pre-incubated for 20 hours to 24 hours. Thereafter, the media on the Apical side and the Basal side are removed by aspiration and washed, a peptide compound is added to the FaSSIF/HBSS buffer (pH 6.0) containing 1% DMSO (1% DMSO) on the Apical side, HBSS buffer (pH 7.4) containing 4% BSA is added to the Basal side, and membrane permeability measurement is initiated. Each well is shaken under conditions of 5% $CO_2$, 37°C, and 80 rpm, and approximately 180 minutes after initiation, samples on the Basal side are collected. The amount of the peptide compound in the samples is measured by LC/MS, and the membrane permeability coefficient is calculated from the amount of permeation. When calculating the membrane permeability coefficient ($P_{app}$) from the amount of permeation, the above described Equation 1 can be used.

In a non-limiting embodiment, when measuring the membrane permeability of a peptide compound in the present disclosure, a peptide compound that does not carry a nucleic acid moiety serving as a template for the peptide moiety, is preferably used as the test substance. In one embodiment, as described later, after performing panning on peptide-nucleic acid complexes, peptide-nucleic acid complexes which can bind specifically to the target molecule are selected, and peptide compounds can be synthesized based on the nucleotide sequences of their nucleic acid moiety. In a preferred example, membrane permeability measurements are performed using peptide compounds synthesized as described as the test substances. In one embodiment, derivatives of peptide compounds encoded by the aforementioned nucleotide sequences can be synthesized, and these can be used as test substance to measure membrane permeability, or derivatives can be synthesized from test substances based on membrane permeability results, and membrane permeability can be measured for those derivatives.

In a non-limiting embodiment, $P_{app}$ of the cyclic peptide compound in the present disclosure is preferably 5.0 x $10^{-7}$ cm/sec or greater or 8.0 x $10^{-7}$ cm/sec or greater, more preferably 9.0 x $10^{-7}$ cm/sec or greater, even more preferably 1.0 x $10^{-6}$ cm/sec or greater, and particularly preferably 3.0 x $10^{-6}$ cm/sec or greater.

Herein, unless otherwise stated particularly, "membrane permeability coefficient ($P_{app}$)" means a value measured using the measurement method (improved method) for membrane permeability in the present disclosure, and more specifically means a value measured using the improved method for membrane permeability test described in the Examples.

Metabolic stability of the peptide compounds

In a non-limiting embodiment, the peptide compounds in the present disclosure preferably have good metabolic stability. For good metabolic stability, the number of amino acids included in the peptide compound is preferably 8 or more, more preferably 9 or more, and even more preferably 11 or more. In one embodiment, the peptide compounds preferably do not carry a thioether bond, which may be readily oxidized. Furthermore, in one embodiment, the peptide compounds preferably do not carry a methylthio group, since this is easily oxidized and may interfere with metabolic stability.

Nucleic acids

In a non-limiting embodiment, preferred examples of the nucleic acids in the present disclosure include nucleic acids encoding the peptide compounds in the present disclosure. The nucleic acids may or may not be linked to a peptide moiety. When linked, they may be linked *via* a spacer or a linker. In one embodiment, "a nucleic acid encoding a peptide compound" refers to a template nucleic acid which is used/has been used in ribosomally synthesizing the peptide compound.

Libraries

The libraries in the present disclosure include libraries comprising the peptide compounds in the present disclosure, and libraries comprising nucleic acids encoding the peptide compounds in the present disclosure. The libraries in the present disclosure include libraries of the peptide compounds and libraries of peptide-nucleic acid complexes in the present disclosure. Among them, libraries of cyclic peptide compounds or libraries of cyclic peptide-nucleic acid complexes are preferred, and libraries of cyclic peptide-mRNA complexes are particularly preferred. Preferred libraries are display libraries. Examples of display libraries include display-utilizing libraries, and among them, mRNA display libraries, DNA display libraries, and ribosome display libraries are preferred, and mRNA display libraries are more preferred.

Herein, "a (cyclic) peptide compound encoded by a nucleic acid" is not limited to a peptide compound directly synthesized by translating the nucleic acid as a template, and may include a peptide compound that can be synthesized as a result of posttranslational modification. For example, when a linear peptide compound is synthesized by translating a nucleic acid and this is followed by a cyclization step to synthesize a cyclic peptide compound, this cyclic peptide

compound may be called a cyclic peptide compound encoded by the aforementioned nucleic acid. Furthermore, in one embodiment, complexes between a peptide ribosomally synthesized using a nucleic acid as a template or a peptide that has undergone subsequent posttranslational modification and the nucleic acid (peptide-nucleic acid complexes) are also included in the (cyclic) peptide compound encoded by a nucleic acid.

In a non-limiting embodiment, the average of the number of amino acids constituting the peptide moiety, excluding the nucleic acid-linked portion, of each cyclic peptide compound included in a library or the cyclic peptide compound encoded by each nucleic acid included in a library in the present disclosure is not particularly limited, but is preferably 30 or less. To acquire high membrane permeability, the average of the number of amino acids of the peptide moiety excluding the nucleic acid-linked portion is preferably 20 or less, more preferably 18 or less, 16 or less, 15 or less, or 14 or less, particularly preferably 13 or less, and specific examples include 9, 10, 11, 12, 13, 14, 15, 16, 17, and 18. To acquire high metabolic stability, the average of the number of amino acids of the peptide moiety excluding the nucleic acid-linked portion is preferably 8 or more, more preferably 9 or more, even more preferably 10 or more, and particularly preferably 11 or more. When considering acquisition of both membrane permeability and metabolic stability, the average of the number of amino acids of the peptide moiety excluding the nucleic acid-linked portion is preferably 5 to 20, 7 to 20, 7 to 17, 8 to 16, 9 to 16, 10 to 16, 8 to 13, 9 to 14, 9 to 14, 9 to 13, 10 to 15, 11 to 15, 10 to 14, 10 to 13, or 11 to 14, or more preferably 11 to 13. In one embodiment, the peptide moiety excluding the nucleic acid-linked portion may comprise linear portions. The average of the number of linear portions is not particularly limited, and examples include one or more, or two or more, and specific examples include zero, one, or two.

In a non-limiting embodiment, the average of the number of amino acids constituting the cyclic portion of each cyclic peptide compound included in a library or the cyclic peptide compound encoded by each nucleic acid included in a library in the present disclosure is not limited, but examples include 4 or more, 6 or more, 7 or more, 8 or more, 9 or more, 10 or more, 11 or more, 12 or more, 20 or less, 18 or less, 16 or less, 15 or less, 14 or less, 13 or less, 12 or less, 11 or less, 7, 8, 9, 10, 11, 12, 13, 14, 15, and 16. When considering acquisition of both membrane permeability and metabolic stability, the average of the number of amino acids constituting the above-mentioned cyclic portion is preferably 5 to 15, more preferably 5 to 14, 7 to 14, or 8 to 14, even more preferably 8 to 13, 9 to 13, 8 to 12, 8 to 11, or 9 to 12, and particularly preferably 9 to 11.

In a non-limiting embodiment, the average of the number of amino acids (number of units) in the linear portion(s) of each cyclic peptide compound included in a library or the cyclic peptide compound encoded by each nucleic acid included in a library in the present disclosure is preferably 0 to 8, more preferably 0 to 5, and even more preferably 0 to 3.

In a non-limiting embodiment, the average number of unnatural amino acids included in the peptide moiety, excluding the nucleic acid-linked portion, of each cyclic peptide compound included in a library or the cyclic peptide compound encoded by each nucleic acid included in a library in the present disclosure is preferably 2 or more, more preferably 4 or more, 5 or more, or 6 or more, even more preferably 7 or more, and particularly preferably 8 or more. In the cyclic peptide compounds included in a library or the cyclic peptide compounds encoded by nucleic acids included in a library in the present disclosure, the average of the number of unnatural amino acids to the number of amino acids constituting the cyclic portion, or the average of the proportion of the number of unnatural amino acids to the number of amino acids included in the peptide moiety, excluding the nucleic acid-linked portion, of each cyclic peptide compound is, for example, 30% or higher, 40% or higher, 50% or higher, 60% or higher, 70% or higher, or 80% or higher.

In a non-limiting embodiment, the average of the number of *N*-substituted amino acids included in the peptide moiety, excluding the nucleic acid-linked portion, of each cyclic peptide compound included in a library or the cyclic peptide compound encoded by each nucleic acid included in a library in the present disclosure is preferably 3 or more, more preferably 4 or more, 5 or more, or 6 or more, even more preferably 7 or more, and particularly preferably 8 or more. For the cyclic peptide compounds included in a library or the cyclic peptide compounds encoded by nucleic acids included in a library in the present disclosure, the average of the percentage of the number of *N*-substituted amino acids to the number of amino acids constituting the cyclic portion, or the average of the number of *N*-substituted amino acids to the number of amino acids included in the peptide moiety, excluding the nucleic acid-linked portion, of each cyclic peptide compound is, for example, 30% or higher, 40% or higher, 50% or higher, 60% or higher, 70% or higher, or 80% or higher. Here, *N*-substituted amino acids may be preferably *N*-alkylamino acids, or more preferably *N*-methylamino acids. More specifically, in a non-limiting embodiment, the average of the number of the *N*-substituted amino acids is exemplified as the average of the number of *N*-alkylamino acids or the number of *N*-methylamino acids.

In a non-limiting embodiment, to achieve high membrane permeability, the average of the number of aromatic rings included in the peptide moiety excluding the nucleic acid-linked portion, in the cyclic portion, or in the side chains of the cyclic portion of each cyclic peptide compound included in a library or the cyclic peptide compound encoded by each nucleic acid included in a library in the present disclosure is preferably 3 or less, and the range of 0 to 3, or 1 to 3 is exemplified as the preferred range. Furthermore, the average of the percentage of the number of aromatic rings to the number of amino acids constituting the cyclic portion or the peptide moiety excluding the nucleic acid-linked portion is preferably 40% or less, and preferred examples include 35% or less, 30% or less, 27% or less, 25% or less, and 20% or less.

In a non-limiting embodiment, since the number of aromatic rings that may be included in the cyclic peptide compound in the present disclosure is limited to achieve high membrane permeability, it is preferable that the cyclic peptide compounds have aromatic rings in the long side chain(s) of the cyclic portion, which is the site which may contribute to binding to the target molecule. More specifically, in one embodiment, examples of the average number of aromatic rings included in the long side chain(s) of the cyclic portion of the cyclic peptide compound in the present disclosure include 0 to 3, 1 to 3, or 2 to 3. Furthermore, the average of the percentage of the number of aromatic rings included in the long side chain(s) of the cyclic portion to the number of aromatic rings included in the peptide moiety excluding the nucleic acid-linked portion, in the cyclic portion, or in the side chains of the cyclic portion of the cyclic peptide compound in the present disclosure includes, for example, 30% or higher, 45% or higher, 60% or higher, 80% or higher, or 100%.

In a non-limiting embodiment, the average of the number of unnatural amino acids, *N*-substituted amino acids, or aromatic ring-containing amino acids included in each cyclic peptide compound included in a library or the cyclic peptide compound encoded by each nucleic acid included in a library in the present disclosure can be adjusted by means of the frequency of appearance of a codon assigned to each amino acid in the synthesis of a DNA library. For example, for a library of peptides containing 10 variable amino acid residue sites, when it is supposed that there are 20 amino acid species to choose from, 10 out of the 20 amino acid species can be *N*-substituted amino acids to synthesize the library such that codon units for the *N*-substituted amino acids account for 10/20 (50%) per variable site. In this way, the average of the percentage of the number of unnatural amino acids, the number of *N*-substituted amino acids, the number of aromatic rings, or the number of aromatic ring-containing amino acids can be calculated.

In a non-limiting embodiment, the mean ClogP for the cyclic peptide compounds included in a library or the cyclic peptide compounds encoded by the nucleic acids included in a library in the present disclosure is preferably 4 or higher, more preferably 5 or higher, even more preferably 6 or higher, particularly preferably 8 or higher, and preferred examples include 18 or below, 17 or below, and 16 or below, and examples include 4 to 18, 5 to 17, and 6 to 16.

In a non-limiting embodiment, the mean ClogP/total aa for the cyclic peptide compounds included in a library or the cyclic peptide compounds encoded by the nucleic acids included in a library in the present disclosure is preferably 1.0 or higher, more preferably 1.1 or more, even more preferably 1.2 or higher, particularly preferably 1.3 or higher, and preferred examples include 1.8 or below, 1.7 or below, 1.6 or below, and 1.5 or below, and examples include 1.0 to 1.8, 1.0 to 1.7, 1.1 to 1.6, and 1.1 to 1.5.

In a non-limiting embodiment, the libraries in the present disclosure may contain cyclic peptide compounds carrying at least one or at least two long side chains in their cyclic portion, or nucleic acids encoding these compounds. In one embodiment, the libraries in the present disclosure may contain cyclic peptide compounds carrying at least one or at least two amino acids which have a long side chain as amino acids constituting the cyclic portion, or nucleic acids encoding these compounds. The cyclic peptide compounds included in a library or encoded by the nucleic acids included in a library in the present disclosure may have high binding affinity to a target molecule by carrying a long side chain in their cyclic portion. In one embodiment, by appropriately selecting the long side chain(s), cyclic peptide compounds having high membrane permeability while maintaining binding affinity to a target molecule can be obtained.

In a non-limiting embodiment, the libraries in the present disclosure may include cyclic peptide compounds carrying the above-described long side chain(s) in their cyclic portion, and/or nucleic acids encoding such compounds. For the "long side chain", the above-described examples, preferred ranges, and embodiments can be applied as is.

In a non-limiting embodiment, the cyclic peptide compounds included in a library or the cyclic peptide compounds encoded by the nucleic acids included in a library in the present disclosure may have aromatic rings in side chains of their cyclic portion, and at least one or two or more of the long side chains included in the cyclic portion may be aromatic ring-containing side chains.

In a non-limiting embodiment, the libraries of cyclic peptide compounds or the libraries of nucleic acids encoding these compounds in the present disclosure may be libraries substantially consisting of cyclic peptide compounds which do not have an indole skeleton in the side chains of the cyclic portion or the side chains of the peptide moiety excluding the nucleic acid-linked portion, or nucleic acids encoding such compounds, respectively. In another embodiment, the libraries of cyclic peptide compounds or the libraries of nucleic acids encoding these compounds in the present disclosure may be libraries substantially not comprising cyclic peptide compounds which have an indole skeleton in the side chains of the cyclic portion or in the side chains of the peptide moiety excluding the nucleic acid-linked portion, or nucleic acids encoding such compounds, respectively. In one embodiment, the side chains of the aforementioned cyclic portion may be long side chains of the cyclic portion.

In a non-limiting embodiment, the libraries of cyclic peptide compounds or the libraries of nucleic acids encoding these compounds in the present disclosure may be libraries substantially consisting of cyclic peptide compounds which do not have a fused-ring structure formed by two or more aromatic rings in the side chains of the cyclic portion or the side chains of the peptide moiety excluding the nucleic acid-linked portion, or nucleic acids encoding such compounds, respectively. In another embodiment, the libraries of cyclic peptide compounds or the libraries of nucleic acids encoding these compounds in the present disclosure may be libraries substantially not comprising cyclic peptide compounds

which have a fused-ring structure formed by two or more aromatic rings in the side chains of the cyclic portion or in the side chains of the peptide moiety excluding the nucleic acid-linked portion, or nucleic acids encoding such compounds, respectively. In one embodiment, the side chains of the aforementioned cyclic portion may be long side chains of the cyclic portion. In a non-limiting embodiment, the aforementioned "fused-ring structure formed by two or more aromatic rings" may be a "fused-ring structure".

In a non-limiting embodiment, the libraries of cyclic peptide compounds or the libraries of nucleic acids encoding these compounds in the present disclosure may be libraries substantially consisting of cyclic peptide compounds which do not have a substituted or unsubstituted hydroxyphenyl group in the side chains of the cyclic portion or in the side chains of the peptide moiety excluding the nucleic acid-linked portion, or nucleic acids encoding such compounds, respectively. In another embodiment, the libraries of cyclic peptide compounds or the libraries of nucleic acids encoding these compounds in the present disclosure may be libraries substantially not comprising cyclic peptide compounds which have a substituted or unsubstituted hydroxyphenyl group in the side chains of the cyclic portion or in the side chains of the peptide moiety excluding the nucleic acid-linked portion, or nucleic acids encoding such compounds, respectively. In one embodiment, the side chains of the aforementioned cyclic portion may be long side chains of the cyclic portion.

In a non-limiting embodiment, the libraries of cyclic peptide compounds or the libraries of nucleic acids encoding these compounds in the present disclosure may be libraries substantially consisting of cyclic peptide compounds which do not have a methylthio group in the side chains of the cyclic portion or in the side chains of the peptide moiety excluding the nucleic acid-linked portion, or nucleic acids encoding such compounds, respectively. In another embodiment, the libraries of cyclic peptide compounds or the libraries of nucleic acids encoding these compounds in the present disclosure may be libraries substantially not comprising cyclic peptide compounds which have a substituted or unsubstituted methylthio group in the side chains of the cyclic portion or in the side chains of the peptide moiety excluding the nucleic acid-linked portion, or nucleic acids encoding such compounds, respectively. In one embodiment, the side chains of the aforementioned cyclic portion may be long side chains of the cyclic portion.

In a non-limiting embodiment, the libraries of cyclic peptide compounds or the libraries of nucleic acids encoding these compounds in the present disclosure may be libraries substantially consisting of cyclic peptide compounds which do not have a thiol group in the side chains of the cyclic portion or in the side chains of the peptide moiety excluding the nucleic acid-linked portion, or nucleic acids encoding such compounds, respectively. In another embodiment, the libraries of cyclic peptide compounds or the libraries of nucleic acids encoding these compounds in the present disclosure may be libraries substantially not comprising cyclic peptide compounds which have a substituted or unsubstituted thiol group in the side chains of the cyclic portion or in the side chains of the peptide moiety excluding the nucleic acid-linked portion, or nucleic acids encoding such compounds, respectively. In one embodiment, the side chains of the aforementioned cyclic portion may be long side chains of the cyclic portion. In one embodiment, the side chains of the aforementioned cyclic portion may be long side chains of the cyclic portion.

In a non-limiting embodiment, to achieve high membrane permeability, the libraries in the present disclosure may be libraries substantially consisting of cyclic peptide compounds that do not carry a functional group that becomes extremely ionized at neutral pH (for example, pH = 7.0) and/or nucleic acids encoding such compounds.

In a non-limiting embodiment, when cyclic peptide compounds having an acidic side chain in the cyclic portion and/or nucleic acids encoding such compounds are included in the library in the present disclosure, the library may be a library substantially consisting of cyclic peptide compounds having an acidic side chain whose pKa is 3.5 to 10. The pKa of the aforementioned acidic side chain is preferably 3.5 or higher, more preferably 3.9 or higher, more preferably 4.5 or higher, more preferably 5.0 or higher, more preferably 5.5 or higher, more preferably 5.7 or higher, and preferably 10 or lower. Preferred examples of the range of the aforementioned pKa include 3.5 to 10, 3.9 to 10, 4.5 to 10, 5.0 to 10, 5.5 to 10, or 5.7 to 10. When these pKa are presented as calculated values, the calculated pKa is preferably 3.5 or higher, more preferably 4.5 or higher, preferably 5.0 or higher, preferably 5.4 or higher, preferably 8.0 or higher, preferably 8.3 or higher, and preferably 10 or lower. Preferred examples of the range of the aforementioned calculated pKa include 3.5 to 10, 4.5 to 10, 5.0 to 10, 5.4 to 10, 8.0 to 10, and 8.3 to 10. In a non-limiting embodiment, the aforementioned acidic side chain may be an acidic long side chain.

In a non-limiting embodiment, when cyclic peptide compounds having a basic side chain in the cyclic portion and/or nucleic acids encoding such compounds are included in the library in the present disclosure, the library may be a library substantially consisting of cyclic peptide compounds having a basic side chain whose basic pKa is 4.0 to 10. The basic pKa of the aforementioned basic side chain is preferably 10 or lower, more preferably 9.5 or lower, more preferably 9.0 or lower, more preferably 8.5 or lower, more preferably 7.5 or lower, more preferably 7.2 or lower, particularly preferably 6.5 or lower, and preferably 4.0 or higher. Preferred examples of the range of the aforementioned basic pKa include 4.0 to 10, 4.0 to 9.5, 4.0 to 9.0, 4.0 to 8.5, 4.0 to 7.5, 4.0 to 7.2, and 4.0 to 6.5. When these basic pKa are presented as calculated values, the calculated basic pKa is preferably 10 or lower, more preferably 9.5 or lower, preferably 9.0 or lower, preferably 8.8 or lower, preferably 8.6 or lower, preferably 8.5 or lower, preferably 7.5 or lower, preferably 6.5 or lower, and preferably 4.0 or higher. Preferred examples of the range of the aforementioned calculated basic pKa

include 4.0 to 10, 4.0 to 9.5, 4.0 to 9.0, 4.0 to 8.8, 4.0 to 8.6, 4.0 to 8.5, 4.0 to 7.5, and 4.0 to 6.5. In a non-limiting embodiment, the aforementioned basic side chain may be a basic long side chain.

Regarding the libraries of cyclic peptide compounds and/or the libraries of nucleic acids encoding such compounds in the present disclosure, "substantially consist of' means that the percentage of the theoretical number of variations of the mentioned cyclic peptide compounds and/or nucleic acids encoding these compounds to the theoretical total number of variations of the cyclic peptide compounds and/or the nucleic acids encoding these compounds included in the library may be 90% or higher, 93% or higher, 95% or higher, 97% or higher, 98% or higher, or 99% or higher, but is not limited thereto.

Regarding the libraries of cyclic peptide compounds and/or the libraries of nucleic acids encoding such compounds in the present disclosure, "substantially not comprise" means that the percentage of the theoretical number of variations of the mentioned cyclic peptide compounds and/or nucleic acids encoding these compounds to the theoretical total number of variations of the cyclic peptide compounds and/or the nucleic acids encoding these compounds included in the library may be 10% or below, 7% or below, 5% or below, 3% or below, 2% or below, or 1% or below, but is not limited thereto.

In the libraries in the present disclosure, when determining the "theoretical (total) number of variations" of cyclic peptide compounds, compounds that cannot actually be produced as cyclic peptide compounds are not included in the calculation of the theoretical (total) number. For example, in the following cases (1) and (2), since the corresponding amino acids are not ribosomally synthesized, peptide compounds containing such amino acids are not included in the calculation of the theoretical (total) number: (1) a case where amino acids are added to the translation solution as ingredients for a peptide compound, but nucleic acids encoding those amino acids are not included as templates; and (2) a case where nucleotide sequences are included in nucleic acid templates for a peptide compound, but a translation solution not containing the corresponding amino acids is used. Furthermore, when byproducts or unreacted substances are present in the process of producing cyclic peptide compounds, those compounds are not included in the calculation of the aforementioned theoretical (total) number.

For example, when 11-residue peptides are ribosomally synthesized using 20 natural amino acid species in total from nucleotide sequences with randomly-arranged codon units according to a codon table in which each of the codon units corresponds to one amino acid species, the theoretical number of variations becomes $20^{10}$ if the translation initiation amino acid is fixed to methionine.

In a non-limiting embodiment, the libraries in the present disclosure may comprise, in addition to cyclic peptide compounds and/or nucleic acids encoding these compounds, other components necessary for screening for peptide compounds that can specifically bind to target molecules. Furthermore, it may comprise other components to the extent that they do not give negative influence on the effects of the libraries in the present disclosure.

Libraries in the present disclosure comprise a plurality of cyclic peptide compounds or nucleic acids encoding these compounds. Herein, the number of variations (types) of cyclic peptide compounds included in a library is referred to as "diversity". While the diversity of the cyclic peptide compounds or nucleic acids encoding these compounds included in a library in the present disclosure is not particularly limited, examples include $10^3$ or more, $10^4$ or more, $10^5$ or more, $10^6$ or more, $10^7$ or more, $10^8$ or more, $10^9$ or more, $10^{10}$ or more, $10^{11}$ or more, and $10^{12}$ or more. The diversity is not limited to measured values and may be theoretical values.

In a non-limiting embodiment, the average of the ClogP of each peptide compound included in a library or the peptide compound encoded by each nucleic acid included in a library in the present disclosure is preferably 4 or higher, more preferably 5 or higher, even more preferably 6 or higher, particularly preferably 8 or higher, and preferably 15 or below.

In a non-limiting embodiment, the ClogP/total aa of each peptide compound included in a library or the peptide compound encoded by each nucleic acid included in a library in the present disclosure is preferably 0.8 or higher, more preferably 1.0 or higher, more preferably 1.1 or higher, even more preferably 1.2 or higher, and particularly preferably 1.3 or higher.

In a non-limiting embodiment, the average of the molecular weight of the peptide moiety excluding the nucleic acid-linked portion, or the average of the molecular weight of the cyclic portion, of each cyclic peptide compound included in a library or the cyclic peptide compound encoded by each nucleic acid included in a library in the present disclosure, may be 500 to 2000.

<u>Membrane permeability</u>

In a non-limiting embodiment, the libraries in the present disclosure may be libraries that include cyclic peptide compounds which have high membrane permeability or can easily acquire high membrane permeability by their derivatization, or nucleic acids encoding such compounds. Furthermore, in a non-limiting embodiment, the libraries in the present disclosure may be libraries in which cyclic peptide compounds hit as compounds that can specifically bind to target molecules have high membrane permeability or can easily acquire high membrane permeability by their derivatization. Herein, "high membrane permeability" means that the membrane permeability coefficient ($P_{app}$) measured by the methods of measuring membrane permeability in the present disclosure is 5.0 x $10^{-7}$ cm/sec or higher, preferably 8.0 x $10^{-7}$ cm/sec or higher or 9.0 x $10^{-7}$ cm/sec or higher, more preferably 1.0 x $10^{-6}$ cm/sec or higher, more preferably 3.0 x $10^{-6}$ cm/sec or higher, or even more preferably 5.0 x $10^{-6}$ cm/sec or higher. In a non-limiting embodiment, the libraries in the present disclosure may be libraries for identifying compounds that can be administered orally (orally-administrable compounds), or precursors thereof. Herein, "can be administered orally (orally-administrable)" means that the membrane permeability coefficient ($P_{app}$) is 1.0 x $10^{-6}$ cm/sec or higher, preferably 3.0 x $10^{-6}$ cm/sec or higher, or more preferably 5.0 x $10^{-6}$ cm/sec or higher. Herein, "precursor" means a compound prior to performing chemical modifications and the like for optimization, and examples include hit compounds through panning which have not yet been optimized.

Hit rate of the peptide compounds that can specifically bind to target molecules

In a non-limiting embodiment, the libraries in the present disclosure may be libraries for identifying compounds that can specifically bind to a target molecule. In one embodiment, the libraries in the present disclosure have a higher hit rate for peptide compounds that can specifically bind to a target molecule than conventional libraries. More specifically, by using the libraries in the present disclosure, many peptide compounds that can specifically bind to a target molecule can be obtained. Furthermore, in one embodiment, peptide compounds with high binding affinity to a target molecule can be obtained. Therefore, it is possible to select desired compounds from among many selected peptide compounds taking into consideration additional factors such as metabolic stability and membrane permeability. Herein, "hit rate" means the percentage of the number of variations of hit compounds obtained by panning to the number of variations of compounds included in a library. The number of variations of compounds included in a library may be a theoretical number.

In a non-limiting embodiment, peptide compounds not present in the libraries as the cyclic peptide compounds for obtaining hit compounds (for example, peptide compounds whose lack of an ability to bind to the target molecule is apparent before panning is performed) are not regarded as peptide compounds constituting the libraries in the present disclosure.

By analyzing the frequency of appearance of amino acids using cyclic peptide compounds enriched by panning, a group of amino acids that may contribute to target molecule-binding can be identified.

Display libraries

A display library refers to a library in which correspondences are made between peptides as phenotypes and their peptide-encoding RNAs or DNAs as genotypes. Using this library, peptide compounds that can specifically bind to a target molecule can be identified. For example, the library is contacted with a desired immobilized target, and target-binding peptides can be enriched by washing out molecules not bound to the target (panning method). The gene information corresponding to the peptides selected through such a process can be analyzed to determine the sequences of the peptides bound to the target. For example, methods taking advantage of non-specific linking of the antibiotic puromycin, an aminoacyl-tRNA analog, to a protein undergoing ribosomal mRNA translation elongation have been reported as mRNA display (Proc. Natl. Acad. Sci. USA. 1997; 94: 12297-302. RNA-peptide fusions for the in vitro selection of peptides and proteins. Roberts, RW., Szostak, JW.) or as *in vitro* virus (FEBS Lett. 1997; 414: 405-8. In vitro virus: bonding of mRNA bearing puromycin at the 3'-terminal end to the C-terminal end of its encoded protein on the ribosome in vitro. Nemoto, N, Miyamoto-Sato, E, Husimi, Y, Yanagawa, H.).

A spacer such as puromycin is conjugated to the 3'-ends of an mRNA library obtained by transcription from a DNA library containing a promoter such as T7 promoter. When these mRNAs are translated into proteins in a cell-free translation system, puromycin is mistakenly recognized as an amino acid and incorporated into proteins by ribosomes. This causes the mRNAs to be linked to the proteins encoded thereby, resulting in a library in which each mRNA corresponds to its product. This process, which does not involve the transformation of *E. coli* or the like, attains high efficiency and can construct a large-scale display library. From the mRNAs tagged onto the molecules enriched and selected by panning, which contain their genetic information, cDNAs can be synthesized, amplified by PCR, and sequenced to determine the sequences of the linked proteins.

In addition to the mRNA display, known display libraries using cell-free translation systems include:

cDNA display which is a library comprising peptide-encoding cDNAs to which peptide-puromycin complexes are linked (Nucleic Acids Res. 2009; 37 (16): e108. cDNA display: a novel screening method for functional disulfide-rich peptides by solid-phase synthesis and stabilization of mRNA-protein fusions. Yamaguchi, J., Naimuddin, M, Biyani, M, Sasaki, T, Machida, M, Kubo, T, Funatsu, T, Husimi, Y, Nemoto, N.);
ribosome display which utilizes the relative stability of complexes between ribosomes and translation products during mRNA translation (Proc. Natl. Acad. Sci. USA. 1994; 91: 9022-6. An in vitro polysome display system for identifying

ligands from very large peptide libraries. Mattheakis, LC, Bhatt, RR, Dower, WJ);
covalent display which utilizes the formation of a covalent bond between bacteriophage endonuclease P2A and DNA (Nucleic Acids Res. 2005; 33: e10. Covalent antibody display-an in vitro antibody-DNA library selection system. Reiersen, H, Lobersli, I, Loset, GA, Hvattum, E, Simonsen, B, Stacy, JE, McGregor, D, Fitzgerald, K, Welschof, M, Brekke, OH, Marvik, OJ.); and
CIS display which utilizes the binding of microbial RepA, a plasmid replication initiator protein, to replication origin ori (Proc. Natl. Acad. Sci. USA. 2004; 101: 2806-10. CIS display: In vitro selection of peptides from libraries of protein-DNA complexes. Odegrip, R, Coomber, D, Eldridge, B, Hederer, R, Kuhlman, PA, Ullman, C, FitzGerald, K, McGregor, D). In addition, *in vitro* compartmentalization (Nat. Biotechnol. 1998; 16: 652-6. Man-made cell-like compartments for molecular evolution. Tawfik, DS, Griffiths, AD) is also known in which transcription-translation systems are encapsulated into water-in-oil emulsions or liposomes for every single one of the DNA molecules constituting a DNA library, and subjected to translation reaction. The methods described above can be employed appropriately using known methods.

Nucleic acid libraries

"Nucleic acid" in the present invention may include deoxyribonucleic acids (DNAs), ribonucleic acids (RNAs), or nucleotide derivatives having artificial bases. Peptide nucleic acids (PNAs) may also be included. The nucleic acids of the present invention may be any of these nucleic acids or mixtures thereof as long as the genetic information of interest is retained. More specifically, the nucleic acids according to the present invention also include DNA-RNA hybrid nucleotides, and chimeric nucleic acids in which different nucleic acids, such as DNAs and RNAs, are linked together to make a single strand.

Examples of libraries of nucleic acids serving as templates for the peptide compounds included in the peptide compound library include mRNA libraries and DNA libraries. A nucleic acid library can be obtained by synthesis with a mixture of bases for variable amino acid residue sites on a peptide sequence. For example, it can be synthesized as triplet repeats of a mixture of four bases (N)-A, T, G and C for a DNA library, and A, U, G, and C for an RNA library-or as those in which the first and second letters in each codon are N and the third letter is a mixture of two bases such as W, M, K, or S. Furthermore, if it is intended that the number of amino acid species to be introduced be reduced to 16 or fewer, the third letter may be one base. Alternatively, codon units corresponding to three-letter codons can be prepared and mixed at arbitrary ratios for use in the synthesis to adjust the frequency of appearance of amino acid residues freely.

These nucleic acid libraries can be translated by using a cell-free translation system. When using a cell-free translation system, a spacer-encoding sequence is preferably included downstream of the nucleic acids of interest. The spacer sequence includes, but is not limited to, sequences containing glycine or serine. In addition, a linker formed by RNA, DNA, a polymer of hexaethylene glycol (spc18) (*e.g.,* 5-mer) or the like is preferably included between the nucleic acid libraries and a compound incorporated into peptides during ribosomal translation such as puromycin or derivatives thereof.

Production methods for peptide compounds and libraries

In one embodiment, production methods in the present disclosure include methods of producing peptide compounds and libraries containing peptide compounds. Such production methods are not particularly limited, but for example, chemical synthesis, ribosomal synthesis, or combinations thereof may be used.

Chemical synthesis methods for peptide compounds

Chemical synthesis methods for peptide compounds in the present disclosure include, for example, liquid phase synthesis methods, solid phase synthesis methods using Fmoc synthesis, Boc synthesis, or such, and combinations thereof. In Fmoc synthesis, an amino acid in which the main chain amino group is protected with an Fmoc group, the side-chain functional groups are protected when necessary with protecting groups that are not cleaved by a base such as piperidine, and the main chain carboxylic acid group is not protected, is used as a basic unit. The basic unit is not particularly limited and may be any other combination as long as it has an Fmoc-protected amino group and a carboxylic acid group. For example, a dipeptide may be used as a basic unit. The basic unit to be positioned at the N terminus may be one that is not an Fmoc amino acid. For example, it may be a Boc amino acid, or a carboxylic acid analog that does not have an amino group. The main chain carboxylic acid group is immobilized onto a solid phase by a chemical reaction with a functional group on a solid-phase carrier. Next, the Fmoc group is deprotected by a base such as piperidine or DBU, and the newly generated amino group and a subsequently added basic unit, i.e. a protected amino acid carrying a carboxylic acid, are subjected to a condensation reaction to generate a peptide bond. In the condensation reaction, various combinations such as DIC and HOBt, DIC and HOAt, and HATU and DIPEA are possible. Repeating the Fmoc

group deprotection and the subsequent peptide bond-forming reaction enables generation of the desired peptide sequence. After the desired sequence is obtained, this is cleaved from the solid phase, and the protecting groups introduced as necessary to the side-chain functional groups are deprotected. Furthermore, before cleaving from the solid phase, conformational conversion and cyclization of the peptide can be carried out. Cleavage from the solid phase and deprotection may be performed under the same conditions, for example, 90:10 TFA/$H_2O$, or deprotection may be performed under different conditions as necessary. Cleavage from the solid phase may be possible by using weak acids such as 1% TFA in some cases, or by using Pd or such as a protecting group and thus utilizing the orthogonality of these chemical reactions. Steps such as cyclization can be carried out during or after these steps. For example, a side-chain carboxylic acid and an N-terminal main chain amino group can be condensed, or a side-chain amino group and a C-terminal main chain carboxylic acid can be condensed. In this case, reaction orthogonality is necessary between the C-terminal carboxylic acid and the side-chain carboxylic acid to be cyclized, or between the N-terminal main chain amino group or hydroxy group and the side chain amino group to be cyclized, and protecting groups are selected by considering their orthogonality as described above. Reaction products thus obtained can be purified using a reverse-phase column, molecular sieve column, and such. Details of such methods are described, for example, in the Solid-phase Synthesis Handbook, published on May 1, 2002 by Merck Co.

Ribosomal synthesis methods for peptide compounds

Examples of translational (ribosomal) synthesis methods for peptide compounds in the present disclosure include methods of synthesizing compounds using cell-free translation systems, and the methods include synthesis methods that use reconstituted cell-free translation systems. Use of reconstituted cell-free translation systems is preferred from the view point that factors which one would like to remove can be eliminated.

In one aspect, the present disclosure provides ribosomal synthesis methods for peptide compounds containing at least one, two or more, or three or more amino acids selected from the group consisting of the amino acids set forth in Tables 2-1 to 2-6. Without limitation, such ribosomal synthesis methods may comprise the steps of:

(i) preparing tRNAs to which at least one, two or more, or three or more amino acids selected from the group consisting of the amino acids set forth in Tables 2-1 to 2-6 are linked; and
(ii) obtaining the aforementioned peptide compound by translating a nucleic acid comprising at least one of the codons corresponding to the anticodons of the tRNAs in a cell-free translation system.

In a non-limiting embodiment, the ribosomal synthesis methods in the present disclosure may be ribosomal synthesis methods for cyclic peptide compounds.

Cell-free translation systems

Herein, the "cell-free translation system" refers to a system in which ribosomes extracted from cells are combined with a group of protein factors involved in translation, tRNAs, amino acids, energy sources such as ATPs and regenerating systems thereof, and is not limited as long as it can translate mRNAs into proteins. Furthermore, systems in which the ribosomal synthesis reactions are progressing may also be included in the "cell-free translation system" herein. The cell-free translation systems herein can contain nucleic acids that will serve as templates during peptide translation, and additionally contain initiation factors, elongation factors, release factors, aminoacyl-tRNA synthetases, and such. These factors can be obtained by purification from various cell extracts. Examples of the cells for purifying the factors therefrom may include prokaryotic cells and eukaryotic cells. Examples of the prokaryotic cells may include *E. coli* cells, extreme thermophile cells, and *Bacillus subtilis* cells. Known eukaryotic cells include those prepared using as materials yeast cells, wheat germs, rabbit reticulocytes, plant cells, insect cells, or animal cells. In addition to naturally-occurring tRNAs and aminoacyl tRNA synthetases (ARSs), artificial tRNAs and artificial aminoacyl tRNA synthetases that recognize unnatural amino acids can also be used. Peptides in which unnatural amino acids are introduced in a site-specific manner can be synthesized by using artificial tRNAs and artificial aminoacyl tRNA synthetases. Furthermore, when necessary, transcription can be performed from template DNAs by adding RNA polymerases such as T7 RNA polymerase to the cell-free translation systems.

Herein, "a cell-free translation system comprises a certain substance" includes embodiments where even if the substance is not included at the start of ribosomal synthesis, the substance is synthesized within the system in the process of ribosomal synthesis and becomes comprised in the system. For example, when a tRNA acylated with an amino acid is synthesized in the process of ribosomal synthesis, the cell-free translation system is understood to comprise the aminoacyl-tRNA.

PURESYSTEM (registered trademark) (BioComber, Japan) is a reconstituted cell-free translation system in which protein factors, energy-regenerating enzymes, and ribosomes necessary for translation in *E. coli* are respectively

extracted and purified and then mixed with tRNAs, amino acids, ATP, GTP, and such. Since this system not only has a low content of impurities, but is also a reconstituted system, it is possible to easily prepare a system free from protein factors and amino acids desired to be excluded ((i) Nat. Biotechnol. 2001; 19: 751-5. Cell-free translation reconstituted with purified components. Shimizu, Y., Inoue, A., Tomari, Y., Suzuki, T., Yokogawa, T., Nishikawa, K., Ueda, T.; (ii) Methods Mol. Biol. 2010; 607: 11-21. PURE technology. Shimizu, Y., Ueda, T.).

For example, there have been many reports of methods using a stop codon as a codon for introducing an unnatural amino acid. By using the PURESYSTEM mentioned above, synthesis systems can be constructed excluding natural amino acids and ARSs. This allows assignment of the codons encoding the excluded natural amino acids to unnatural amino acids (J. Am. Chem. Soc. 2005; 127: 11727-35. Ribosomal synthesis of unnatural peptides. Josephson, K., Hartman, MC., Szostak, JW.). Furthermore, unnatural amino acids can be added without the exclusion of natural amino acids by breaking codon degeneracy (Kwon, I., et al., Breaking the degeneracy of the genetic code. J. Am. Chem. Soc. 2003, 125, 7512-3.). Peptides containing N-methylamino acids can be ribosomally synthesized by utilizing the cell-free translation systems such as PURESYSTEM.

More specifically, ribosomal synthesis can be carried out, for example, by the addition of mRNA to a known cell-free translation system such as the PURESYSTEM in which protein factors necessary for translation in *E. coli* (methionyl-tRNA transformylase, EF-G, RF1, RF2, RF3, RRF, IF1, IF2, IF3, EF-Tu, EF-Ts and ARS (necessary ones are selected from AlaRS, ArgRS, AsnRS, AspRS, CysRS, GlnRS, GluRS, GlyRS, HisRS, IleRS, LeuRS, LysRS, MetRS, PheRS, ProRS, SerRS, ThrRS, TrpRS, TyrRS and ValRS)), ribosome, amino acids, creatine kinase, myokinase, inorganic pyrophosphatase, nucleoside diphosphate kinase, *E. coli*-derived tRNAs, creatine phosphate, potassium glutamate, HEPES-KOH (pH 7.6), magnesium acetate, spermidine, dithiothreitol, GTP, ATP, CTP, UTP and the like are appropriately selected and mixed. Also, addition of T7 RNA polymerase enables coupled transcription/translation from template DNAs containing T7 promoter. In addition, a group of desired aminoacyl tRNAs and a group of unnatural amino acids (for example, F-Tyr) acceptable by aminoacyl tRNA synthetases (ARSs) can be added to a system to ribosomally synthesize peptide compounds containing the unnatural amino acids (Kawakami, T., et al. Ribosomal synthesis of polypeptoids and peptoid-peptide hybrids. J. Am. Chem. Soc. 2008, 130, 16861-3; Kawakami, T., et al. Diverse backbone-cyclized peptides via codon reprogramming. Nat. Chem. Biol. 2009, 5, 888-90). Furthermore, peptide compounds containing unnatural amino acids can also be ribosomally synthesized by adding variants of ARSs instead of or in addition to natural ARSs, and also adding a group of unnatural amino acids in the system. Alternatively, the translational incorporation efficiency of unnatural amino acids may be increased by using variants of ribosome, EF-Tu, and the like (Dedkova LM, et al. Construction of modified ribosomes for incorporation of D-amino acids into proteins. Biochemistry. 2006, 45, 15541-51; Doi Y, et al. Elongation factor Tu mutants expand amino acid tolerance of protein biosynthesis system. J Am Chem Soc. 2007, 129, 14458-62; Park HS, et al. Expanding the genetic code of Escherichia coli with phosphoserine. Science. 2011, 333, 1151-4).

In a non-limiting embodiment, the cell-free translation systems in the present disclosure (also referred to as "translation system(s) in the present disclosure") may be translation systems for producing peptide compounds, and are preferably translation systems for producing cyclic peptide compounds.

In a non-limiting embodiment, the translation systems in the present disclosure may be cell-free translation systems substantially not comprising at least one, two, or all selected from the group consisting of the following: (a) an amino acid which has an indole skeleton in its side chain, or a nucleic acid encoding the amino acid; (b) an amino acid which has a fused-ring structure formed by two or more aromatic rings in its side chain, or a nucleic acid encoding the amino acid; and (c) an amino acid which has a substituted or unsubstituted hydroxyphenyl group in its side chain, or a nucleic acid encoding the amino acid. In a non-limiting embodiment, the translation systems in the present disclosure may be cell-free translation systems substantially not comprising at least one, two, or all selected from the group consisting of the following: (d) a nucleic acid encoding an amino acid which has an indole skeleton in its side chain; (e) a nucleic acid encoding an amino acid which has a fused-ring structure formed by two or more aromatic rings in its side chain; and (f) a nucleic acid encoding an amino acid which has a substituted or unsubstituted hydroxyphenyl group in its side chain. In a non-limiting embodiment, the translation systems in the present disclosure may be cell-free translation systems substantially not comprising at least one, two, or all selected from the group consisting of the following: (g) an amino acid which has an indole skeleton in its side chain; (h) an amino acid which has a fused-ring structure formed by two or more aromatic rings in its side chain; and (i) an amino acid which has a substituted or unsubstituted hydroxyphenyl group in its side chain. Even if a certain amino acid or nucleic acid is included in a translation system, when it is included such that it is not used in ribosomal synthesis, the translation system is understood as substantially not comprising that amino acid or nucleic acid. Such cases include, for example, a case where although an amino acid is present, its corresponding ARS is not included and therefore the amino acid is not used in ribosomal synthesis. In a non-limiting embodiment, the aforementioned "fused-ring structure formed by two or more aromatic rings" may also be a "fused-ring structure".

In a non-limiting embodiment, when a translation system in the present disclosure contains tRNAs acylated with amino acids carrying an acidic side chain and nucleic acids encoding such amino acids, the pKa of the amino acid

side chain may be 3.5 to 10. In one embodiment, examples of the range of the aforementioned pKa preferably include 3.5 to 10, 3.9 to 10, 4.5 to 10, 5.0 to 10, 5.5 to 10, and 5.7 to 10. When these pKa are presented as calculated pKa, examples of the range preferably include 3.5 to 10, 4.5 to 10, 5.0 to 10, 5.4 to 10, 8.0 to 10, and 8.3 to 10.

In a non-limiting embodiment, when a translation system in the present disclosure contains tRNAs acylated with amino acids carrying a basic side chain and nucleic acids encoding such amino acids, the basic pKa of the amino acid side chain may be 4.0 to 10. In one embodiment, preferred examples of the range of the aforementioned basic pKa include 4.0 to 10, 4.0 to 9.5, 4.0 to 9.0, 4.0 to 8.5, 4.0 to 7.5, 4.0 to 7.2, and 4.0 to 6.5. When these pKa are presented as calculated pKa, preferred examples of the range include 4.0 to 10, 4.0 to 9.5, 4.0 to 9.0, 4.0 to 8.8, 4.0 to 8.6, 4.0 to 8.5, 4.0 to 7.5, and 4.0 to 6.5.

In a non-limiting embodiment, the translation system in the present disclosure may contain amino acids carrying a long side chain that is, for example, between 5.4 angstroms to 13 angstroms long or between 5.4 angstroms to 10 angstroms long. In one embodiment, the aforementioned term "long side chain" may be the same as that described in the section "Peptide compounds". In one embodiment, at least one, or two or more of the long side chains may be side chains carrying an aromatic ring.

In a non-limiting embodiment, the translation systems in the present disclosure may contain 5 to 32, 5 to 28, or 5 to 20 unnatural amino acids, and preferred examples include 8 to 20, 10 to 20, and 13 to 20 unnatural amino acids. In one embodiment, 50% or more, 60% or more, 70% or more, or 80% or more of the amino acid species included in the translation systems in the present disclosure may be unnatural amino acids.

In a non-limiting embodiment, the translation systems in the present disclosure may contain 5 to 28, or 5 to 20 *N*-substituted amino acids, and preferred examples include 5 to 18 and 5 to 15 *N*-substituted amino acids. In one embodiment, 40% or more, 50% or more, 60% or more, or 70% or more of the amino acid species included in the translation systems in the present disclosure may be *N*-substituted amino acids. Here, the *N*-substituted amino acids may mean N-alkylamino acids or *N*-methylamino acids. However, even if this is the case, it does not exclude cases where the translation systems in the present disclosure contain other *N*-substituted amino acids.

In a non-limiting embodiment, the translation systems in the present disclosure may be adjusted such that the average of the number of aromatic rings included in the cyclic peptide compounds produced using the translation systems will be within a given range. For example, the translation systems in the present disclosure may be adjusted such that in the ribosomally-synthesized cyclic peptide compounds, the average of the percentage of the number of amino acids having an aromatic ring to the number of amino acids constituting the cyclic portion becomes 40% or less, 35% or less, 30% or less, 27% or less, 25% or less, or 20% or less; or the average of the number of aromatic rings included in the side chains of the cyclic portion of each cyclic peptide compound having a cyclic portion composed of 8 to 11 amino acids will be 0 to 3. While the adjusting methods are not particularly limited, examples include adjusting the number of aromatic ring-containing amino acid species accounting for the number of amino acid species included in the translation system.

In a non-limiting embodiment, the translation systems in the present disclosure may include aromatic ring-containing amino acids, and the percentage of the number of aromatic ring-containing amino acid species to the number of amino acid types included in the translation system is preferably 40% or less, and preferred examples include 35% or less, 30% or less, 27% or less, 25% or less, and 20% or less.

In a non-limiting embodiment, the cell-free translation systems in the present disclosure may be cell-free translation systems for producing a peptide compound containing at least one, two or more, or three or more amino acids selected from the group consisting of the amino acids set forth in Tables 2-1 to 2-6. While not being limited thereto, such cell-free translation systems may comprise the following:

(i) tRNAs to which at least one, two or more, or three or more amino acids selected from the group consisting of the amino acids set forth in Tables 2-1 to 2-6 are linked; and
(ii) a nucleic acid encoding the peptide compound;

wherein the nucleic acid may contain at least one of the codons corresponding to the anticodons of the tRNAs.

<u>tRNAs</u>

For translational incorporation of unnatural amino acids into peptides, aminoacylation of tRNAs that are orthogonal and efficiently incorporated into ribosomes is necessary ((i) Biochemistry. 2003; 42: 9598-608. Adaptation of an orthogonal archaeal leucyl-tRNA and synthetase pair for four-base, amber, and opal suppression. Anderson, JC., Schultz, PG.; and (ii) Chem. Biol. 2003; 10: 1077-84. Using a solid-phase ribozyme aminoacylation system to reprogram the genetic code. Murakami, H., Kourouklis, D., Suga, H.). The following five methods can be used as methods for aminoacylating tRNAs.

Within cells, aminoacyl tRNA synthetases (ARS) for respective amino acids are provided as enzymes for

aminoacylating tRNAs. Therefore, the first method includes methods of utilizing the fact that certain ARSs accept unnatural amino acids such as *N*-Me His, or methods of preparing and using mutant aminoacyl tRNA synthetases that accept unnatural amino acids ((i) Proc. Natl. Acad. Sci. U S A. 2002; 99: 9715-20. An engineered Escherichia coli tyrosyl-tRNA synthetase for site-specific incorporation of an unnatural amino acid into proteins in eukaryotic translation and its application in a wheat germ cell-free system. Kiga, D., Sakamoto, K., Kodama, K., Kigawa, T., Matsuda, T., Yabuki, T., Shirouzu, M., Harada, Y., Nakayama, H., Takio, K., Hasegawa, Y., Endo, Y., Hirao, I., Yokoyama, S.; (ii) Science. 2003; 301: 964-7. An expanded eukaryotic genetic code. Chin, JW., Cropp, TA., Anderson, JC., Mukherji, M., Zhang, Z., Schultz, PG., Chin, JW.; and (iii) Proc. Natl. Acad. Sci. U S A. 2006; 103: 4356-61. Enzymatic aminoacylation of tRNA with unnatural amino acids. Hartman, MC., Josephson, K., Szostak, JW.). Second, a method in which tRNAs are aminoacylated *in vitro,* and then the amino acids are chemically modified can also be used (J. Am. Chem. Soc. 2008; 130: 6131-6. Ribosomal synthesis of N-methyl peptides. Subtelny, AO., Hartman, MC., Szostak, JW.). Third, tRNAs in which CA has been removed from the 3'-end CCA sequence can be linked with a separately prepared aminoacylated pdCpA by using RNA ligase to obtain aminoacyl tRNAs (Biochemistry. 1984; 23: 1468-73. T4 RNA ligase mediated preparation of novel "chemically misacylated" tRNAPheS. Heckler, TG., Chang, LH., Zama, Y., Naka, T., Chorghade, MS., Hecht, SM.). There is also aminoacylation by flexizymes, which are ribozymes that allow tRNAs to carry active esters of various unnatural amino acids (J. Am. Chem. Soc. 2002; 124: 6834-5. Aminoacyl-tRNA synthesis by a resin-immobilized ribozyme. Murakami, H., Bonzagni, NJ., Suga, H.). Fourth, a method in which tRNA and the active ester of an amino acid are ultrasonically agitated within cationic micelles can also be used (Chem. Commun. (Camb). 2005; (34): 4321-3. Simple and quick chemical aminoacylation of tRNA in cationic micellar solution under ultrasonic agitation. Hashimoto, N., Ninomiya, K., Endo, T., Sisido, M.). Fifth, aminoacylation is also possible by linking an amino acid active ester to a PNA that is complementary to a sequence close to the 3'-end of a tRNA and adding this to the tRNA (J. Am. Chem. Soc. 2004; 126: 15984-9. In situ chemical aminoacylation with amino acid thioesters linked to a peptide nucleic acid. Ninomiya, K., Minohata, T., Nishimura, M., Sisido, M.).

More specifically, aminoacyl tRNAs can be prepared using methods such as the following. A template DNA encoding a desired tRNA sequence upstream of which a T7, T3 or SP6 promoter is placed is prepared. RNA can be synthesized by transcription of the DNA using an RNA polymerase compatible with the promoter, such as T7 RNA polymerase, or T3 or SP6 RNA polymerase. tRNAs can also be extracted from cells and purified, and a generated tRNA of interest can be extracted therefrom by using a probe having a sequence complementary to the tRNA sequence. In such extraction, cells transformed with an expression vector for the tRNA of interest may be used as a source. RNA with a desired sequence may also be synthesized chemically. For example, the tRNA thus obtained in which CA has been removed from the 3'-end CCA sequence may be linked to a separately prepared aminoacylated pdCpA or pCpA by RNA ligase to obtain an aminoacyl tRNA (pdCpA method, pCpA method). Such tRNAs are useful in the preparation of peptide compounds. Alternatively, aminoacyl tRNAs can also be prepared by preparing full-length tRNAs and aminoacylating them using flexizymes, which are ribozymes that enable tRNAs to carry active esters of various unnatural amino acids. Without any limitation intended, aminoacyl tRNAs can also be prepared using native ARSs or variants thereof. When native ARSs or variants thereof are used, the aminoacyl tRNAs once consumed in the translation system can be regenerated by the native ARSs or variants thereof; therefore, there is no need for aminoacyl tRNAs prepared in advance to exist in a large amount in the translation system. Such ARS variants are described in WO 2016/148044. These methods for preparing aminoacyl tRNAs can also be combined appropriately.

## Methods of cyclizing the peptide moieties

In a non-limiting embodiment, the peptide moieties of the peptide compounds in the present disclosure preferably have a cyclic portion. While the methods of cyclizing the peptide compounds are not particularly limited, a cyclization reaction can be performed, for example, after synthesizing peptide compounds by chemical synthesis, ribosomal synthesis, or such. Examples include cyclization that utilizes amide bonding, carbon-carbon bonding, thioether bonding, disulfide bonding, ester bonding, thioester bonding, lactam bonding, bonding *via* a triazole structure, or bonding *via* a fluorophore structure. The step for synthesizing the peptide compound and the step for the cyclization reaction may proceed separately or sequentially. The cyclization can be performed by methods known to those skilled in the art which are described in, for example, WO 2013/100132, WO 2008/117833, and WO 2012/074129.

The cyclization site may be, without limitation, any one of a bond between the N terminus and C terminus of a peptide, a bond between the N terminus of a peptide and the side chain of another amino acid residue of the peptide, a bond between the C terminus of a peptide and the side chain of another amino acid residue of the peptide, or a bond between the side chains of amino acid residues; or two or more of these can be used in combination. When a peptide-nucleic acid complex in which a nucleic acid is linked to the C-terminal side is cyclized, the cyclization site may be a bond between the N terminus of the peptide and the side chain of another amino acid residue of the peptide or a bond between the side chains of amino acid residues.

In a non-limiting embodiment, the cyclic peptide compounds in the present disclosure can be produced by the

following method. For example, production methods include methods comprising the following steps:

(1) synthesizing a non-cyclic peptide compound composed of amino acid residues or of amino acid residues and an N-terminal carboxylic acid analog by translating a nucleic acid encoding the peptide compound, wherein the non-cyclic peptide compound comprises an amino acid residue at the C-terminal side which carries one reactive site in a side chain, and an amino acid residue or an N-terminal carboxylic acid analog at the N-terminal side which carries another reactive site; and
(2) linking the reactive site of the N-terminal side amino acid residue or of the N-terminal carboxylic acid analog, and the reactive site of the side chain of the amino acid residue at the C-terminal side to form an amide bond, a carbon-carbon bond, or a thioether bond.

Steps (1) and (2) may proceed separately or sequentially.

A non-limiting embodiment for the methods of cyclizing peptide compounds by an amide bond includes the method of translating a peptide compound carrying a cysteine or a cysteine analog at the N terminus and an active ester in the side chain of an amino acid at the C-terminal side, and cyclizing the peptide compound by using native chemical ligation. The method for ribosomally synthesizing the peptide compound carrying a cysteine or a cysteine analog at the N terminus, which is the precursor of this cyclic peptide compound, is not particularly limited.

In one embodiment, to synthesize a peptide compound carrying an amino acid other than Met or Nle at the N terminus of the peptide compound, for example, the following three methods can be used.

The first method is a method of subjecting a peptide having formylmethionine or Met as the initiation amino acid to enzymatic cleavage of its N-terminal amino acid or a peptide at its N-terminal side. As the enzyme, peptide deformylases (PDF) and/or methionine amino peptidases (MAP) can be used.

The second method is a method using a reconstituted cell-free translation system in which Met is removed from the system in advance and an amino acid desired to be placed at the N-terminus is placed at the second residue from the N-terminus, and ribosomal synthesis is performed while skipping Met, the initiation codon (this method is defined as the initiation read-through (iRT) method).

The third method is a method of performing ribosomal synthesis in a reconstituted cell-free translation system devoid of Met or translation initiator methionine tRNA, to which an initiator aminoacyl-tRNA prepared by aminoacylation with an amino acid desired to be placed at the N terminus has been added in advance (this method is defined as the initiation suppression (iSP) method).

In a non-limiting embodiment, the C-terminal portion of the cyclic peptide compounds in the present disclosure does not have to remain to be a carboxylic acid and may be chemically modified. For example, the carboxylic acid portion may be converted to piperidinamide or such by reacting it with piperidine or such.

Methods of preparing libraries

In a non-limiting embodiment, when preparing the libraries in the present disclosure, it is preferred to include constituent amino acids that contribute to desired features and not to include constituent amino acids having unfavorable properties. Furthermore, in one embodiment, the diversity of the libraries is desirably enlarged as much as possible. Specifically, it is preferred that amino acids that may contribute to at least one feature selected from the group consisting of the following (1) to (4) be included: (1) increase in hit rate of peptide compounds that can specifically bind to a target molecule; (2) increase in binding affinity to a target molecule; (3) increase in metabolic stability; and (4) increase in membrane permeability. On the other hand, amino acids that can negatively affect at least one feature selected from the group consisting of (1) to (4) above are preferably not included in the constituent amino acids.

In a non-limiting embodiment, the production of the libraries in the present disclosure can be carried out according to the above-described methods of producing peptide compounds in the present disclosure, and can appropriately be combined with known methods. In one embodiment, the peptide compound libraries in the present disclosure can be produced using the above-described cell-free translation systems in the present disclosure. More specifically, the library production methods in the present disclosure may comprise the step of synthesizing peptide compounds using the cell-free translation systems in the present disclosure. In one embodiment, the examples, preferred ranges, and embodiments described for the cell-free translation systems in the present disclosure can also be applied, as they are, to the library production methods in the present disclosure.

In a non-limiting embodiment, the methods of producing a library of peptide compounds in the present disclosure may include the following step: (a) preparing an amino acid pool substantially not comprising an amino acid which has an indole skeleton in the side chain, and synthesizing peptide compounds using a part or all of the amino acids included in the pool as constituent amino acids. Furthermore, in one embodiment, the methods of producing a library of peptide compounds in the present disclosure may include the following step: (b) preparing a template pool substantially not comprising a nucleic acid encoding an amino acid which has an indole skeleton in the side chain, and synthesizing

peptide compounds from the template pool.

Herein, even if an amino acid pool comprises a certain amino acid, when the amino acid is included in such a manner that it is not available for ribosomal synthesis, or when the amino acid is included only in a very small amount, the amino acid pool is understood to substantially not comprise the amino acid. For example, even if an amino acid pool comprises a certain amino acid, when no ARS corresponding to the amino acid is included and therefore the amino acid is not used in ribosomal synthesis, the amino acid pool is understood to substantially not comprise the amino acid. Meanwhile, even if an ARS is not included, when the corresponding amino acid is included in such a manner that it is available for ribosomal synthesis, the amino acid is understood to be included in the amino acid pool.

Herein, even if a template pool comprises a nucleic acid encoding a certain amino acid, when the nucleic acid is included in such a manner that that amino acid is not available in ribosomal synthesis, or when the nucleic acid is included only in a very small amount, the template pool is understood to substantially not comprise the nucleic acid.

In a non-limiting embodiment, the pools of the aforementioned steps (a) and (b) may be pools substantially not comprising an amino acid which has a fused-ring structure formed by two or more aromatic rings in the side chain, and a nucleic acid encoding the amino acid, respectively. In one embodiment, the pools of the aforementioned steps (a) and (b) may be pools substantially not comprising an amino acid which has a substituted or unsubstituted hydroxyphenyl group in the side chain, and a nucleic acid encoding the amino acid, respectively. In a non-limiting embodiment, the aforementioned "fused-ring structure formed by two or more aromatic rings" may be a "ring-fused structure".

In a non-limiting embodiment, the amino acid pools used in synthesizing the peptide compounds may contain amino acids carrying an acidic side chain, and the template pools may contain nucleic acids encoding such amino acids. In this case, preferred examples of the range of the pKa of the amino acid side chain include 3.5 to 10, 3.9 to 10, 4.5 to 10, 5.0 to 10, 5.5 to 10, and 5.7 to 10. When these pKa are presented as calculated pKa, preferred examples of the range include 3.5 to 10, 4.5 to 10, 5.0 to 10, 5.4 to 10, 8.0 to 10, and 8.3 to 10.

In a non-limiting embodiment, the amino acid pools used in synthesizing the peptide compounds may contain amino acids carrying a basic side chain, and the template pools may contain nucleic acids encoding such amino acids. In this case, preferred examples of the range of the basic pKa of the amino acid side chain include 4.0 to 10, 4.0 to 9.5, 4.0 to 9.0, 4.0 to 8.5, 4.0 to 7.5, 4.0 to 7.2, and 4.0 to 6.5. When these pKa are presented as calculated pKa, preferred examples of the range include 4.0 to 10, 4.0 to 9.5, 4.0 to 9.0, 4.0 to 8.8, 4.0 to 8.6, 4.0 to 8.5, 4.0 to 7.5, and 4.0 to 6.5.

In a non-limiting embodiment, the amino acid pools used in synthesizing the peptide compounds may include amino acids having the above-described long side chain. For the "long side chain", the above-mentioned descriptions for examples, preferred ranges, and embodiments can be applied as they are. In one embodiment, at least one, two, or three or more of the long side chains included in the aforementioned amino acid pools may be aromatic ring-containing side chains.

In a non-limiting embodiment, the amino acid pools used in synthesizing the peptide compounds may include the above-described unnatural amino acids, *N*-substituted amino acids, and aromatic ring-containing amino acids, and the examples, preferred ranges, and embodiments described in the "Cell-free translation systems" section can be applied as they are. The percentage of the number of unnatural amino acid species, *N*-substituted amino acid species, or aromatic ring-containing amino acid species to the number of amino acid species included in the amino acid pool may be selected similarly to their percentages in the translation systems in the present disclosure.

In a non-limiting embodiment, mRNA display libraries can be prepared as follows. First, a library of DNAs in which desired sequences are positioned downstream of a promoter such as T7 promoter is chemically synthesized, and this is used as a template to produce double-stranded DNAs by primer extension reaction. The double-stranded DNAs are used as templates and transcribed into mRNAs by using RNA polymerase such as T7 RNA polymerase. The 3'-end of these RNAs is conjugated to a linker (spacer) to which the aminoacyl-tRNA analog antibiotic puromycin or such is attached. The resulting conjugates are added to a known cell-free translation system such as the above-mentioned PURESYSTEM, and incubated so that the mRNAs are translated, and as a result each mRNA is linked to the peptide encoded thereby through the linker containing puromycin or such. In this way, a display library composed of mRNA-product complexes in which the mRNAs are associated with their products can be constructed. The linker can contain additional spacers well-known to those skilled in the art. Furthermore, when necessary, posttranslational modifications such as cyclization can be performed by the above-described methods or known methods.

In a non-limiting embodiment, the libraries in the present disclosure may be peptide compound libraries comprising peptide compounds each of which contains at least one, two or more, or three or more amino acids selected from the group consisting of the amino acids set forth in Tables 2-1 to 2-6. Methods of producing such libraries may include the step of ribosomally synthesizing peptide compounds using a cell-free translation system comprising the following:

(i) tRNAs to which at least one, two or more, or three or more amino acids selected from the group consisting of the amino acids set forth in Tables 2-1 to 2-6 are linked; and
(ii) a nucleic acid library encoding the peptide compound library;

wherein the nucleic acid library may contain nucleic acids carrying at least one codon corresponding to the anticodon of the tRNA.

Screening methods

In a non-limiting embodiment, peptide compounds that can specifically bind to a target molecule can be selected by screening which uses the libraries in the present disclosure. The screening methods in the present disclosure are not particularly limited, but for example, mRNA display methods may be used.
In a non-limiting embodiment, the screening methods in the present disclosure can enrich peptide compounds that bind to a target molecule by contacting the cyclic peptide compound library in the present disclosure with the target molecule, and washing off peptide compounds unbound with the target molecules (panning). In one embodiment, the amino acid sequences of the bound peptides can be identified by synthesizing cDNAs from the mRNAs tags included in the peptide compounds thus selected, which contain their nucleotide sequence information, then amplifying the cDNAs by PCR, and sequencing their nucleotide sequences. In one embodiment, a library of mRNAs can be obtained by transcribing the above-mentioned amplified cDNAs, and these can be used as templates to produce a library of peptide compounds again. Since this library is enriched for peptide compounds that can bind to a target molecule, this library can be used for panning again to further concentrate peptide compounds that can specifically bind to the target molecule. The peptide compounds of interest can further be concentrated by repeating these steps multiple times. In one embodiment, the amino acid sequences of the peptide compounds concentrated in this manner can be determined based on the nucleotide sequence information contained in the peptide compounds, and then the peptide compounds that can specifically bind to the target molecule can be produced. In one embodiment, the screening methods in the present disclosure can be performed *in vitro.*
In a non-limiting embodiment, the peptide compounds obtained by the screening methods in the present disclosure may be subjected to chemical modifications and such by known methods to optimize the peptide compounds. Herein, "optimize (optimization)" means chemical modification of a ribosomally synthesized compound by transforming the structure of each amino acid within the compound such that the compound becomes a more drug-like peptide compound, a peptide compound having stronger activity to a drug efficacy target, and/or a peptide compound whose toxicity is avoided to a higher extent.
In a non-limiting embodiment, the screening methods in the present disclosure comprise the following steps:

(a) contacting the peptide compounds included in the library in the present disclosure with a target molecule; and
(b) selecting peptide compounds that can bind to the target molecule.

In one embodiment, in the screening methods in the present disclosure, the aforementioned steps (a) and (b) can be repeated two or more times to concentrate peptide compounds that can specifically bind to a target molecule.

Target molecules

Target molecules used in the screening methods in the present disclosure are not particularly limited, and include, for example, proteins, peptides, nucleic acids, sugars, and lipids, but, *inter alia,* proteins are preferably used as targets. In one embodiment, the screening methods in the present disclosure can provide peptide compounds that inhibit protein-protein interactions (PPI). While not intending to be restricted by a particular theory, since the interface of PPI in particular has a site called "hotspot" which is considered to increase the binding force of PPI, PPI may be inhibited through intrusion of a long side chain into this site. Thus, the libraries and screening methods in the present disclosure may provide peptide compounds that inhibit PPI regardless of what the target molecule is.
The locations where the target molecule exists *in vivo* are also not particularly limited. In one embodiment, the libraries and screening methods in the present disclosure can provide cyclic peptide compounds with high membrane permeability or cyclic peptide compounds that can be easily made highly membrane-permeable by derivatization; therefore, intracellular proteins can also become targets.
In a non-limiting embodiment, target molecules used in the screening methods in the present disclosure are used after being immobilized onto carriers. The carriers are not particularly limited as long as they can immobilize target molecules, and examples include beads and resins. Target molecules can be immobilized onto carriers by known methods.
As described above, the target molecules of the libraries and screening methods in the present disclosure are not particularly limited, but examples include GTPase KRas (KRAS), Dual specificity mitogen-activated protein kinase kinase 1 (MEK1), Mitogen-activated protein kinase 3 (ERK1), and interleukin 6 receptor (IL-6R). As described in the Examples, the libraries in the present disclosure include peptide compounds that can specifically bind to various target molecules. Therefore, in one embodiment, they may enable drug development for "tough targets", for which drug discovery

has been considered difficult.

In a non-limiting embodiment, the methods of producing cyclic peptide compounds in the present disclosure may comprise the steps of:

(i) contacting the cyclic peptide compounds included in the cyclic peptide compound library in the present disclosure with a target molecule;
(ii) selecting a cyclic peptide compound that can bind to the target molecule; and
(iii) producing the cyclic peptide compound based on the amino acid sequence of the cyclic peptide compound selected in (ii).

In a non-limiting embodiment, the library production methods, peptide compound production methods, and/or screening methods in the present disclosure may be performed *in vitro.*

In one aspect, the present disclosure provides amino acids having the "long side chain" defined herein as the side chain. Examples of such amino acids include amino acids selected from the group consisting of the amino acids set forth in Tables 2-1 to 2-6.

In one aspect, the present disclosure provides an amino acid having a group represented by the following general formula (II) or (III) in the side chain:

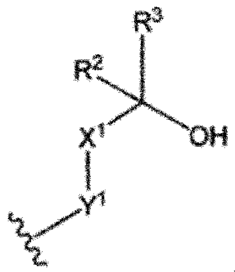

(II)

,

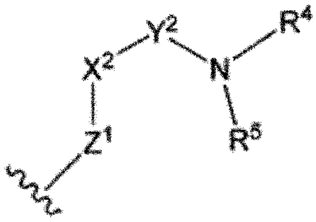

(III)

wherein

$X^1$ represents a single bond, or a C1-C2 alkylene group optionally having at least one substituent independently selected from Group B below,
$X^2$ represents a single bond, or a C1-C2 alkylene group optionally having at least one substituent independently selected from Group B below,
$Y^1$ represents a single bond, an oxygen atom, a carbonyl group (-CO-), or a sulfonyl group ($-SO_2-$),
$Y^2$ represents a single bond, a C1-C2 alkylene group optionally substituted with a C1-C4 alkyl group, a carbonyl group (-CO-), or a sulfonyl group ($-SO_2-$),
$Z^1$ represents an oxygen atom,
$R^2$ and $R^3$ each independently represent a hydrogen atom, a C1-C6 alkyl group, a C2-C4 alkenyl group, or a C2-C4 alkynyl group, wherein the C1-C6 alkyl group is optionally substituted with a halogen atom, and one or two non-adjacent methylene groups in the C1-C6 alkyl group are optionally substituted with an oxygen atom,
$R^2$ and $R^3$, or $R^2$ and $X^1$ are optionally joined to form a 3- to 6-membered ring structure, wherein the ring structure is optionally substituted with a C1-C4 alkyl group or a halogen atom, optionally has an oxygen atom in the ring, and is optionally partially unsaturated,
$R^4$ represents a C1-C6 alkyl group, a C2-C6 alkenyl group, a C2-C6 alkynyl group, or a C1-C6 alkanoyl group,
$R^5$ represents a hydrogen atom, wherein one or two non-adjacent methylene groups in the C1-C6 alkyl group of $R^4$ are optionally substituted with an oxygen atom, and

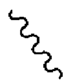

represents the point of attachment;
Group B: a halogen atom, a C1-C4 alkyl group, and a C1-C2 alkoxy group.

In one embodiment, the present disclosure provides an amino acid having a group represented by the following

general formula (II) or (III) in the side chain:

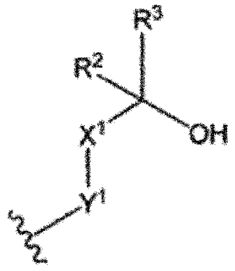

(II)

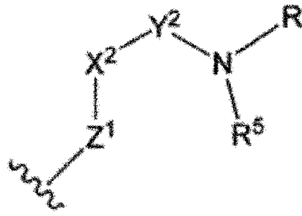

(III)

wherein

$X^1$ represents a C1-C2 alkylene group optionally having at least one substituent independently selected from Group B below,
$X^2$ represents a C1-C2 alkylene group optionally having at least one substituent independently selected from Group B below,
$Y^1$ represents an oxygen atom, a carbonyl group (-CO-), or a sulfonyl group ($-SO_2-$),
$Y^2$ represents a carbonyl group (-CO-) or a sulfonyl group ($-SO_2-$),
$Z^1$ represents an oxygen atom,
$R^2$ and $R^3$ each independently represent a hydrogen atom or a C1-C6 alkyl group, wherein the C1-C6 alkyl group is optionally substituted with a halogen atom, and one or two non-adjacent methylene groups in the C1-C6 alkyl group are optionally substituted with an oxygen atom,
$R^2$ and $R^3$, or $R^2$ and $X^1$ are optionally joined to form a 3- to 6-membered ring structure, wherein the ring structure may be substituted with a C1-C4 alkyl group or a halogen atom, optionally has an oxygen atom in the ring, and is optionally partially unsaturated,
$R^4$ represents a C1-C6 alkyl group,
$R^5$ represents a hydrogen atom, wherein one or two non-adjacent methylene groups in the C1-C6 alkyl group of $R^4$ are optionally substituted with an oxygen atom, and

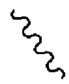

represents the point of attachment;
Group B: a halogen atom, a C1-C4 alkyl group, and a C1-C2 alkoxy group.

The $X^1$ is preferably a C1-C2 alkylene group optionally having at least one substituent independently selected from Group B above,
the $X^2$ is preferably a C1-C2 alkylene group optionally having at least one substituent independently selected from Group B above,
the $Y^1$ is preferably an oxygen atom,
the $Y^2$ is preferably a carbonyl group (-CO-),
the $R^2$ and $R^3$ are each independently preferably a hydrogen atom or a C1-C6 alkyl group, wherein the C1-C6 alkyl group is optionally substituted with a halogen atom, wherein the halogen atom is preferably fluorine,
the $R^4$ is preferably a C1-C6 alkyl group, and
the halogen atom in Group B above is preferably a fluorine atom.

In a non-limiting embodiment, amino acids in the present disclosure may be pdCpA amino acids or pCpA amino acids in which pdCpA or pCpA is linked to the carboxy group by an ester bond. The structures of pdCpA and pCpA and the points of linkage to the carboxy group of an amino acid through an ester bond are provided below:

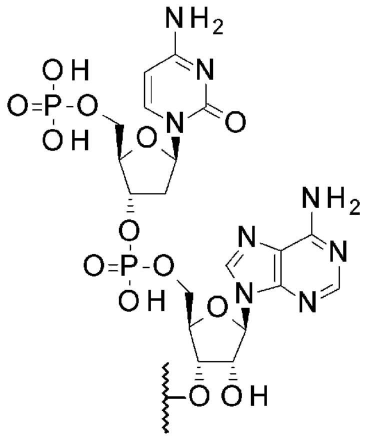

(pdCpA)

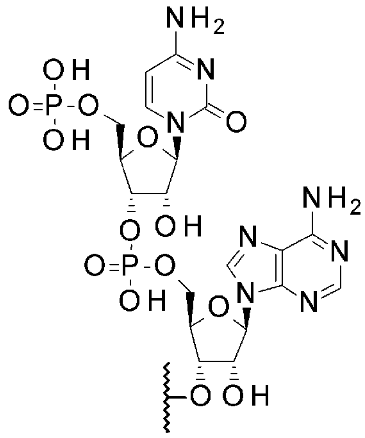

(pCpA)

wherein

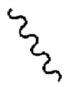

represents the point of linkage to the carboxy group of an amino acid through an ester bond.

Such amino acids can be produced, for example, using a method described in Examples.

In a non-limiting embodiment, main chain amino groups of amino acids, pdCpA amino acids, and/or pCpA amino acids in the present disclosure may be protected by protecting groups. Groups such as, but not limited to, an Fmoc group, a Boc group, and a Cbz group can be used as amino-protecting groups. Methods known in the art or methods described in Examples may be used as methods of protecting an amino group.

In a non-limiting embodiment, amino acids in the present disclosure may be used for chemically and/or ribosomally synthesizing peptide compounds. Without any limitation intended, for example, peptide compounds may be translationally synthesized using pdCpA amino acids and/or pCpA amino acids, or peptide compounds may be chemically synthesized using amino acids protected with an Fmoc group. Peptide compounds here are preferably cyclic peptide compounds, but are not limited thereto.

In one aspect, the present disclosure provides an amino acid having a "long side chain" as the side chain for use in synthesizing a peptide compound. In another aspect, the present disclosure provides an amino acid having a "long side chain" as the side chain for use in translating a peptide compound. Peptide compounds here are preferably cyclic peptide compounds, but are not limited thereto.

Methods of chemically and ribosomally synthesizing peptide compounds using amino acids in the present disclosure can be performed according to methods known in the art or methods described in Examples.

All prior art documents cited in the present specification are incorporated herein by reference.

## Examples

The present invention is further illustrated by the following Examples, but is not limited thereto.

The following abbreviations were used in Examples.

AA Ammonium acetate

Acbz 4-Azidobenzyloxycarbonyl group

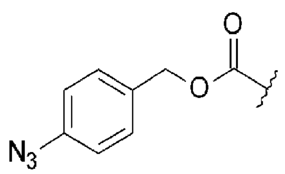

$AcONH_4$ Ammonium acetate

$CH_2CN$ Cyanomethyl group

DBU 1,8-Diazabicyclo[5.4.0]-7-undecene

DCM Dichloromethane

DCE 1,2-Dichloroethane

DMF N,N-Dimethylformamide

DIC N,N'-Diisopropylcarbodiimide

DIPEA N-Ethyl-isopropylpropan-2-amine or N,N-diisopropylethylamine

MeCN Acetonitrile

MeOH Methanol

NMP N-Methyl-2-pyrrolidone

FA Formic acid

TFA Trifluoroacetic acid

TFE 2,2,2-Trifluoroethanol

HFIP 1,1,1,3,3,3-Hexafluoroisopropylalcohol

HOAt 1-Hydroxy-7-azabenzotriazole

HOBt 1-Hydroxybenzotriazole

WSCI·HCl 1-Ethyl-3-(3-dimethylaminopropyl)carbodiimide hydrochloride

TBME t-Butyl methyl ether

TIPS Triisopropylsilane

HATU O-(7-Aza-1H-benzotriazol-1-yl)-N,N,N',N'-tetramethyluronium hexafluorophosphate

Allyl Allyl group

dba Dibenzylideneacetone

DEAD Diethyl azodicarboxylate

DIAD Diisopropyl azodicarboxylate

DAST Dimethylaminosulfur trifluoride

DMAP 4-Dimethylaminopyridine

DMSO Dimethyl sulfoxide

Fmoc-Cl (9H-Fluoren-9-yl)methyl carbonochloridate

Fmoc-OSu (9H-Fluoren-9-yl)methyl (2,5-dioxopyrrolidin-1-yl) carbonate, or N-(9-fluorenylmethoxycarbonyloxy)succinimide, or 9-fluorenylmethyl N-succinimidyl carbonate F-Pnaz 4-(2-(4-Fluorophenyl)acetamido)benzyloxycarbonyl group

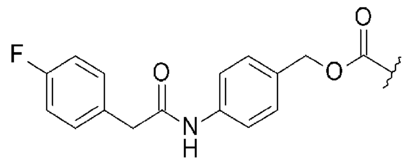

GC Gas chromatography

MeAbu (S)-2-(Methylamino)butanoic acid

Ns Nosyl group

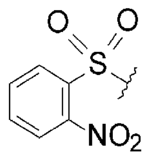

pCpA ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate

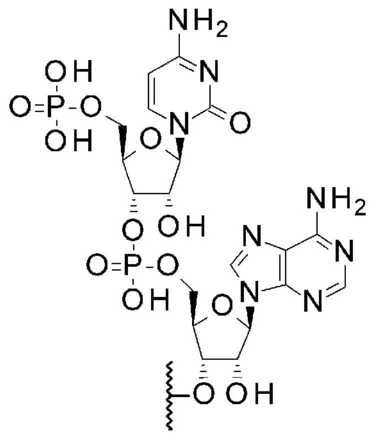

pCpA(THF) ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate

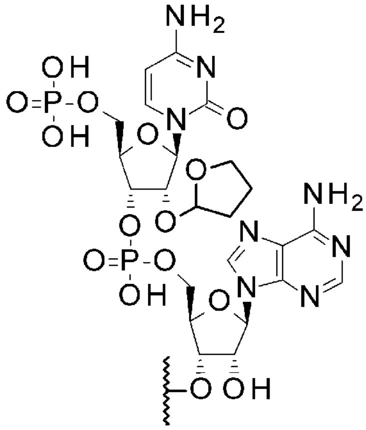

PPTS Pyridinium p-toluenesulfonate

TBAF Tetrabutylammonium fluoride

$Tf_2O$ Trifluoromethanesulfonic anhydride

THF Tetrahydrofuran

TMSCl Chlorotrimethylsilane

T3P Propylphosphonic anhydride

Trt Trityl group

Peptide synthesis grade solvent (purchased from Watanabe Chemical Industries and Wako Pure Chemical Industries) were used for peptide synthesis and solid-phase synthesis. Examples include DCM, DMF, NMP, 2% DBU in DMF, and 20% piperidine in DMF. Dehydrated solvents, ultradehydrated solvents, and anhydrous solvents (purchased from Kanto Chemical, Wako Pure Chemical Industries, and others) were used for reactions in which water was not added as a solvent.

The LC/MS analysis conditions are as follows.

[Table 3]

| Analytical condition | Instrument | Column (I.D. x length) (mm) | Mobile phase | Gradient (A/B) | Flow rate (mL/min) | Column temperature (°C) | Wavelength |
|---|---|---|---|---|---|---|---|
| SQDAA05 | Acquity UPLC/SQD | Ascentis Express C18 (2.1x50) | A)10mM $AcONH_4$,$H_2O$ B)MeOH | 95/5=> 0/100(1.0 min) => 0/100(0.4 min) | 1.0 | 35 | 210-400nm PDA total |
| SQDAA50 | Acquity UPLC/SQD | Ascentis Express C18 (2.1x50) | A)10mM $AcONH_4$,$H_2O$ B)MeOH | 50/50=> 0/100(0.7 min) => 0/100(0.7 min) | 1.0 | 35 | 210-400nm PDA total |
| SQDFA05 | Acquity UPLC/SQD | Ascentis Express C18 (2.1x50) | A)0.1% FA,$H_2O$ B)0.1% FA,MeCN | 95/5=> 0/100(1.0 min) => 0/100(0.4 min) | 1.0 | 35 | 210-400nm PDA total |
| SQDFA50 | Acquity UPLC/SQD | Ascentis Express C18 (2.1x50) | A)0.1% FA,$H_2O$ B)0.1% FA,MeCN | 50/50=> 0/100(0.7 min) => 0/100(0.7 min) | 1.0 | 35 | 210-400nm PDA total |
| SMD method1 | Shimadzu LCMS-2020 LC-20AD | Shim-Pack XR-ODS (3.0x50) | A)0.05% TFA,$H_2O$ B)0.05% TFA,MeCN | 95/5=> 0/100(2.2 min) => 0/100(1.0 min) | 1.0 | 40 | 210-800nm PDA total |
| SMD method2 | Shimadzu LCMS-2020 LC-20AD | Shim-Pack XR-ODS (3.0x50) | A)0.05% TFA,$H_2O$ B)0.05% TFA,MeCN | 95/5=> 0/80(2.2 min) => 0/80 (1.0 min) | 1.0 | 40 | 210-800nm PDA total |
| SMD method3 | Nexera/2020 | Ascentis Express C18 (2.1x50) | A)0.1% FA,$H_2O$ B)0.1% FA,MeCN | 95/5=> 0/100(1.5 min) => 0/100(0.7 min) | 1.0 | 35 | 210-800nm PDA total |
| SMD method4 | Shimadzu LCMS-2020 LC-30AD | ACQUITY UPLC BEH C18 (2.1x50) | A)0.1% FA,$H_2O$ B)0.05% FA,MeCN | 95/5=> 20/80(4.0 min)=> 20/80(1.2 min) =>95/5(0.2 min) =>95/5(0.2 min) | 0.7 | 45 | 190-800nm PDA total |
| SMD method5 | Shimadzu LCMS-2020 LC-20AD | Shim-Pack XR-ODS (3.0x50) | A)0.05% TFA,$H_2O$ B)0.05% TFA,MeCN | 95/5=> 0/100(2.2 min)=> 0/100(1.0 min) =>95/5(0.1 min) =>95/5(0.3 min) | 1.0 | 40 | 190-800nm PDA total |
| SMD method6 | Nexera/2020 | Speed Core C18 (2.1x50) | A)0.1% FA,$H_2O$ B)0.1% FA,MeCN | 95/5=> 0/100(1.5 min) => 0/100(0.5 min) | 1.0 | 35 | 210-400nm PDA total |
| SMD method7 | Shimadzu LCMS-2020 LC-20AD | Shim-Pack XR-ODS (3.0x50) | A)0.05% TFA,$H_2O$ B)0.05% TFA,MeCN | 95/5=> 0/100(1.2 min) => 0/100(1.0 min) =>95/5(0.1 min) | 1.0 | 40 | 190-800nm PDA total |
| SMD method8 | Shimadzu LCMS-2020 LC-30AD | Ascentis Express C18 (2.1x50) | A)0.1% FA,$H_2O$ B)0.1% FA,MeCN | 95/5=> 0/100(1.1 min)=> 0/100(0.5 min) | 1.0 | 40 | 190-400nm PDA total |

(continued)

| Analytical condition | Instrument | Column (I.D. x length) (mm) | Mobile phase | Gradient (A/B) | Flow rate (mL/min) | Column temperature (°C) | Wavelength |
|---|---|---|---|---|---|---|---|
| SMD method9 | Shimadzu LCMS-2020 LC-20ADXR | | A)0.1% FA,$H_2O$ B)0.1% FA,MeCN | 90/10=> 0/100(1.2 min)=> 0/100(0.5 min) => 90/10(0.1 min) | 1.5 | 40 | 190-400nm PDA total |
| SMD method16 | Shimadzu LCMS-2020 LC-30AD | | Poroshell HPH-C18 (3.0x50) A)6.5mM NH4HCO3 pH10 B)MeCN | 90/10=> 5/95(1.1 min)=> 5/95(0.5 min) =>90/10(0.1 min) | 1.2 | 40 | 190-400nm PDA total |
| SMD method 11 | Shimadzu LCMS-2020 LC-30AD | | A)6.5mM NH4HCO3 PH10 B)MeCN | 90/10=> 5/95(1.1 min)=> 5/95(0.5 min) =>90/10(0.1 min) | 1.0 | 40 | 190-400nm PDA total |
| SMD method12 | Shimadzu LCMS-2020 LC-30AD | | A)6.5mM NH4HCO3 PH10 B)MeCN | 90/10=> 5/95(1.1 min)=> 5/95(0.5 min) =>90/10(0.1 min) | 1.5 | 40 | 190-400nm PDA total |
| SMD method13 | Shimadzu LCMS-2020 LC-20AD | | A)0.05% TFA,$H_2O$ B)0.05% TFA,MeCN | 95/5=> 0/100(1.1 min)=> 0/100(0.6 min) =>95/5(0.05 min) | 1.2 | 40 | 190-400nm PDA total |
| SMD method14 | Shimadzu LCMS-2020 LC-20AD | | A)0.05% TFA,$H_2O$ B)0.05% TFA,MeCN | 95/5=> 5/95(3.0 min)=> 5/95 (0.7 min) =>95/5(0.05 min) | 1.2 | 40 | 190-400nm PDA total |
| SMD method15 | Shimadzu LCMS-2020 LC-20AD | | A)0.1% FA,$H_2O$ B)0.1% FA,MeCN | 90/10=> 0/100(3.0 min)=> 0/100(0.7 min) =>95/5(0.05 min) | 1.2 | 40 | 190-400nm PDA total |
| SMD method16 | Shimadzu LCMS-2020 LC-20ADXR | CORTECS C18 (2.1x50) | A)0.1% FA,$H_2O$ B)0.1% FA,MeCN | 90/10=> 0/100(1.2 min)=> 0/100(0.5 min) =>90/10(0.1 min) | 1.0 | 40 | 190-400nm PDA total |
| SMD method 17 | Shimadzu LCMS-2020 LC-20ADXR | kinetex XB-C18 (3.0x50) | A)0.1% FA,$H_2O$ B)0.1% FA,MeCN | 90/10=> 0/100(1.2 min)=> 0/100(0.5 min) =>90/10(0.1 min) | 1.5 | 40 | 190-400nm PDA total |
| SMD method 18 | Shimadzu LCMS-2020 LC-20AD | Shim-Pack XR-ODS (3.0x50) | A)0.05% TFA,$H_2O$ B)0.05% TFA,MeCN | 95/5=> 0/100(2.2 min)=> 0/100(1.0 min) =>95/5(0.1 min) | 1.0 | 40 | 190-400nm PDA total |

(continued)

| Analytical condition | Instrument | Column (I.D. x length) (mm) | Mobile phase | Gradient (A/B) | Flow rate (mL/min) | Column temperature (°C) | Wavelength |
|---|---|---|---|---|---|---|---|
| SMD method19 | Shimadzu LCMS-2020 LC-20ADXR | kinetex XB-C18 (3.0x50) | A)0.1% FA,$H_2O$ B)0.1% FA,MeCN | 90/10=> 5/95(2.0 min)=> 5/95(0.7 min) =>90/10(0.1 min) | 1.5 | 40 | 190-400nm PDA total |
| SMD method20 | Shimadzu LCMS-2020 LC-30AD | Kinetex EVO C18 (2.1x50) | A)6.5mM $NH_4HCO_3$ pH10 B)MeCN | 90/10=> 5/95(2.0 min) => 5/95(0.7 min) =>90/10(0.1 min) | 1.0 | 40 | 190-400nm PDA total |
| SMD method21 | Shimadzu LCMS-2020 LC-20AD | Ascentis Express C18 (4.6x100) | A)0.1% FA,$H_2O$ B)0.1% FA,MeCN | 95/5=> 70/30(15.0 min) => 70/30(3.0 min) =>5/95(2.0 min) | 1.0 | 40 | 190-400nm PDA total |
| SMD method22 | Shimadzu LCMS-2020 LC-30AD | Ascentis Express C18 (2.1x50) | A)0.1% FA,$H_2O$ B)0.05% FA,MeCN | 95/5=> 0/100(1.1 min)=> 0/100(0.5 min) =>95/5 (0.1 min) =>95/5(0.1 min) | 1.0 | 40 | 190-600nm PDA total |
| SMD method23 | Shimadzu LCMS-2020 LC-20ADXR | phenomenex kinetex (3.0x50) | A)0.1% FA,$H_2O$ B)0.1% FA,MeCN | 90/10=> 0/100(1.1 min)=> 0/100(0.7 min) => 90/10(0.1 min) | 1.5 | 40 | 190-400nm PDA total |
| SMD method24 | Nexera/2020 | Ascentis Express C18 (2.1x50) | A)0.05% TFA,$H_2O$ B)0.05% TFA,MeCN | 95/5=> 0/100(1.5 min) => 0/100(0.5 min) | 1.0 | 35 | 210-400nm PDA total |
| SMD method25 | Nexera/2020 | Meteoric Core C18 (2.1x50) | A)0.05% TFA,$H_2O$ B)0.05% TFA,MeCN | 95/5=> 0/100(1.5 min) => 0/100(0.5 min) | 1.0 | 35 | 210-400nm PDA total |
| SMD method26 | Shimadzu LCMS-2020 LC-20AD | Shim-Pack XR-ODS (3.0x50) | A)0.05% TFA,$H_2O$ B)MeCN | 95/5=> 0/100(2.0 min) => 0/100(1.2 min) =>95/5(0.1 min) | 1.0 | 40 | 190-800nm PDA total |
| SMD method27 | Shimadzu LCMS-2020 LC-20AD | Shim-Pack XR-ODS (3.0x50) | A)0.05% TFA,$H_2O$ B)0.05% TFA,MeCN | 80/20=> 20/80(4 min)=> 0/100(0.3 min) =>0/100(0.9 min)=>95/5(0.1 min) | 1.0 | 40 | 190-400 PDA total |
| SMD method28 | Shimadzu LCMS-2020 LC-20AD | Shim-Pack XR-ODS (3.0x50) | A)0.05% TFA,$H_2O$ B)0.05% TFA,MeCN | 95/5=> 30/70(3.8 min)=> 0/100(0.3 min) =>0/100(0.5 min)=>95/5(0.1 min) | 1.2 | 40 | 190-400 PDA total |

(continued)

| Analytical condition | Instrument | Column (I.D. x length) (mm) | Mobile phase | Gradient (A/B) | Flow rate (mL/min) | Column temperature (°C) | Wavelength |
|---|---|---|---|---|---|---|---|
| SMD method29 | Shimadzu LCMS-2010EV | Shim-Pack XR-ODS (3.0x50) | A)0.05% TFA,$H_2O$<br>B)0.05% TFA,MeCN | 95/05=> 0/100(1.1 min)=> 0/100(0.6 min) =>95/5(0.05 min) | 1.2 | 40 | 190-400 PDA total |
| SMD method30 | Shimadzu LCMS-2020 LC-20ADXR | phenomenex kinetex (3.0x50) | A)0.1% FA,$H_2O$<br>B)0.1% FA,MeCN | 90/10=> 0/100(1.2 min)=> 0/100(0.5 min) =>90/10(0.1 min) | 1.5 | 40 | 190-400 PDA total |
| SMD method31 | Shimadzu LCMS-2020 LC-20AD | Shim-Pack XR-ODS (3.0x50) | A)0.05% TFA,$H_2O$<br>B)0.05% TFA,MeCN | 95/5=> 0/100(1.2 min)=> 0/100(1.0 min) =>95/5(0.1 min) | 1.0 | 40 | 190-800 PDA total |
| SMD method32 | Shimadzu LCMS-2020 LC-20AD | Shim-Pack XR-ODS (3.0x50) | A)0.05% TFA,$H_2O$<br>B)0.05% TFA,MeCN | 95/5=> 0/100(1.6 min)=> 0/100(0.6 min) =>95/5(0.1 min) | 1.0 | 40 | 190-800 PDA total |
| SMD method33 | Shimadzu LCMS-2020 LC-20AD | Shim-Pack XR-ODS (3.0x50) | A)0.05% TFA,$H_2O$<br>B)0.05% TFA,MeCN | 95/5=> 0/100(1.2 min)=> 0/100(1.0 min) =>95/5(0.1 min) | 1.0 | 40 | 190-400 PDA total |
| SMD method34 | Shimadzu LCMS-2020 LC-20AD | Shim-Pack XR-ODS (3.0x50) | A)0.05% TFA,$H_2O$<br>B)0.05% TFA,MeCN | 95/5=> 5/95(2.0 min) => 5/95 (0.7 min) =>95/5(0.05 min) | 1.2 | 40 | 190-400 PDA total |
| GC01 | Agilent GCMS 7890A-5975 | Agilent, DB-5ms (0.2x12) | MeCN | 50°C(1min)=> 40°C~300°C (6.25 min) => 300°C (1.75 min) | 1.0 | 50 | - |
| ELSD1 | Shimadzu UFLC-MS 2010EV | Shim-Pack XR-ODS (3.0x50) | A)0.05% TFA,$H_2O$<br>B)0.05% TFA,MeCN | 95/5=> 0/100(1.6 min) => 0/100(0.6 min) =>95/5(0.1 min) | 1.0 | 40 | *ELSD detection |
| ELSD2 | Shimadzu UFLC-MS 2010EV | Shim-Pack XR-ODS (3.0x50) | A)0.05% TFA,$H_2O$<br>B)0.05% TFA,MeCN | 95/5=> 0/100(2.2 min) => 0/100(1.0 min) =>95/5(0.1 min) | 1.0 | 40 | *ELSD detection |
| SMD method35 | Shimadzu LCMS-2020 LC-30AD | Ascentis Express C18 (2.1x50) | A)0.05% TFA,$H_2O$<br>B)0.05% TFA,MeCN | 95/5=> 0/100(1.1 min) => 0/100(0.5 min) =>95/5(0.1 min) | 1.0 | 40 | 190-400nm PDA total |

(continued)

| Analytical condition | Instrument | Column (I.D. x length) (mm) | Mobile phase | Gradient (A/B) | Flow rate (mL/min) | Column temperature (°C) | Wavelength |
|---|---|---|---|---|---|---|---|
| SMD method36 | Shimadzu LCMS-2020 LC-20ADXR | CORTECS C18 (2.1x50) | A)0.1% FA,$H_2O$ B)0.1% FA,MeCN | 90/10=> 0/100(1.0 min) => 0/100(0.5 min) =>90/10(0.1 min) | 1.0 | 40 | 190-400nm PDA total |
| SMD method37 | Shimadzu LCMS-2020 LC-20AD | Shim-Pack XR-ODS (3.0x50) | A)0.05% TFA,$H_2O$ B)0.05% TFA,MeCN | 95/5=> 40/60(3.8 min) => 0/100(0.3 min) =>0/100(0.5 min) =>95/5(0.1 min) | 1.2 | 40 | 190-400 PDA total |
| SMD method38 | Shimadzu LCMS-2020 LC-30AD | Accucore C18 (2.1x50) | A)0.1% FA,$H_2O$ B)0.1% FA,MeCN | 90/10=> 0/100(1.1 min)=> 0/100(0.5 min) =>90/10(0.1 min) | 1.0 | 40 | 190-400nm PDA total |
| SMD method39 | Shimadzu LCMS-2020 LC-20AD | Shim-Pack XR-ODS (3.0x50) | A)0.05% TFA,$H_2O$ B)0.05% TFA,MeCN | 95/5=> 0/100(1.1 min) => 0/100(0.6 min) =>95/5(0.05 min) | 1.2 | 40 | 190-400nm PDA total |
| SMD method40 | Shimadzu LCMS-2020 LC-20ADXR | CORTECS C18 (2.1x50) | A)0.1% FA,$H_2O$ B)0.1% FA,MeCN | 90/10=> 5/95(3.0 min)=> 5/95(0.7 min) =>90/10(0.1 min)) | 1.0 | 40 | 190-400nm PDA total |
| SMD method41 | Shimadzu LCMS-2020 LC-30AD | Kinetex EVO C18 (2.1x50) | A)6.5mM NH4HCO3 PH10 B)MeCN | 90/10=> 5/95(3.0 min)=> 5/95(0.7 min)=> 90/10(0.1 min) | 1.0 | 35 | 190-400nm PDA total |
| SMD method42 | Shimadzu LCMS-2020 LC-30AD | CORTECS C18 (2.1x50) | A)0.05% TFA,$H_2O$ B)0.05% TFA,MeCN | 95/5=> 5/95(3.0 min)=> 5/95 (0.7 min) =>95/5(0.1 min) | 1.0 | 45 | 190-400nm PDA total |
| SMD method43 | Shimadzu LCMS-2020 LC-20ADXR | CORTECS C18 (2.1x50) | A)0.1% FA,$H_2O$ B)0.1% FA,MeCN | 95/5=> 0/100(1.0 min)=> 0/100(0.5 min) =>95/5(0.1 min) | 1.0 | 40 | 190-400nm PDA total |
| SMD method44 | Shimadzu LCMS-2020 LC-30AD | InertCore C18 (2.1x50) | A)0.05% TFA,$H_2O$ B)0.05% TFA,MeCN | 95/5=> 0/100(1.1 min)=> 0/100(0.5 min) =>95/5(0.1 min) | 1.0 | 45 | 190-400nm PDA total |
| SMD method45 | Shimadzu Nexera/ 2020 | Ascentis Express C18 (2.1x50) | A)0.1% FA,$H_2O$ B)0.1% FA,MeCN | 95/5=> 0/100(1.5 min) => 0/100(0.5 min) | 1.0 | 35 | 210-400nm PDA total |
| SMD method46 | Shimadzu Nexera/ 2020 | Ascentis Express C18 (2.1x50) | A)0.05% TFA,$H_2O$ B)0.05% TFA,MeCN | 95/5=> 0/100(4.5 min)=> 0/100(0.5 min) | 1.0 | 35 | 210-400nm PDA total |

(continued)

| Analytical condition | Instrument | Column (I.D. x length) (mm) | Mobile phase | Gradient (A/B) | Flow rate (mL/min) | Column temperature (°C) | Wavelength |
|---|---|---|---|---|---|---|---|
| SMD method47 | Shimadzu Nexera/ 2020 | Ascentis Express C18 (2.1x50) | A)0.1% FA,$H_2O$<br>B)0.1% FA,MeCN | 95/5=> 0/100(4.5 min)=> 0/100(0.5 min) | 1.0 | 35 | 210-400nm PDA total |
| SQDAA05-2 | Acquity UPLC I-Class /SQD | Ascentis Express C18 (2.1x50) | A)10mM $AcONH_4$,$H_2O$<br>B)MeOH | 95/5=> 0/100(1.0 min) => 0/100(0.4 min) | 0.9 | 35 | 210-400nm PDA total |
| SMD method48 | Shimadzu LCMS-2020 LC-20AD | Shim-Pack XR-ODS (3.0x50) | A)0.05% TFA,$H_2O$<br>B)0.05% TFA,MeCN | 95/5=> 5/95(3 min)=> 5/95 (0.7 min) | 1.2 | 40 | 190-400nm PDA total |
| SMD method49 | Shimadzu LCMS-2010EV | Shim-Pack XR-ODS (3.0x50) | A)0.05% TFA,$H_2O$<br>B)0.05% TFA,MeCN | 95/5=> 5/95(3.0 min)=> 5/95 (0.7 min) =>95/5(0.05 min) | 1.2 | 40 | 190-400nm PDA total |

[Example 1] Chemical synthesis of peptide compounds

method by the Fmoc chemistry described in WO 2013/100132, specifically, by the following five steps: 1) peptide elongation reaction by the Fmoc chemistry from the N-terminus of Asp in which the Asp side chain carboxylic acid is loaded onto a 2-chlorotrityl resin, 2) a process of cleavage of a peptide from the 2-chlorotrityl resin, 3) amide cyclization by condensation between the Asp side chain carboxylic acid resulting from removal from the 2-chlorotrityl resin by the cleavage process and the N-terminal (triangle unit) amino group of the peptide chain, 4) deprotection of the protecting group for the side chain functional group contained in the peptide chain, and 5) purification of the compound by preparative HPLC. In the present Examples, unless otherwise stated, peptide compounds were synthesized based on this basic route.

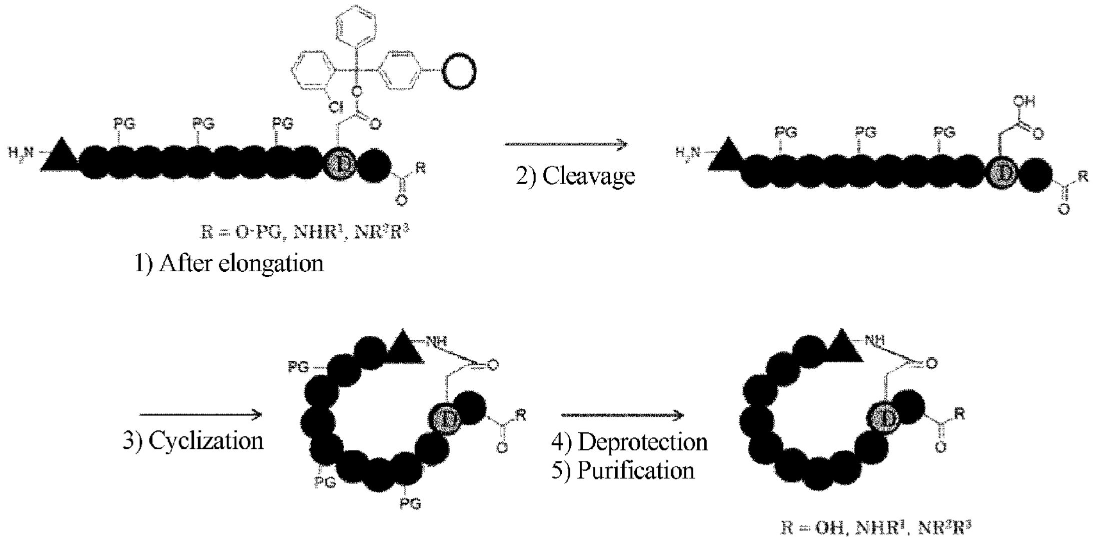


1-1. Fmoc amino acids used in peptide synthesis by a peptide synthesizer

The following Fmoc amino acids were used in peptide synthesis described herein using a peptide synthesizer. The abbreviations for the amino acids are set forth in Tables 4-1 to 4-32.

Fmoc-MeLeu-OH, Fmoc-Leu-OH, Fmoc-MeTrp-OH, Fmoc-MePhe-OH, Fmoc-MeIle-OH, Fmoc-Thr(Trt)-OH, Fmoc-MeGly-OH, Fmoc-g-MeAbu-OH, Fmoc-Ile-OH, Fmoc-Ser(tBu)-OH, Fmoc-Trp-OH, Fmoc-MeAla-OH, Fmoc-Val-OH, Fmoc-D-Ala-OH, Fmoc-MeVal-OH, Fmoc-Ala-OH, Fmoc-D-Val-OH, Fmoc-Tyr(tBu)-OH, Fmoc-Tyr(Clt)-OH, Fmoc-D-Leu-OH, Fmoc-Phe(4-$CF_3$)-OH, Fmoc-D-MeAla-OH, Fmoc-Pro-OH, Fmoc-Pic(2)-OH, Fmoc-EtGly-OH, Fmoc-D-Pro-OH, Fmoc-Abu-OH, Fmoc-Ala(3-Pyr)-OH, FmocAla(4-Pyr)-OH, Fmoc-Phe(3-Cl)-OH, Fmoc-Phg-OH, Fmoc-Met($O_2$)-OH, Fmoc-Ser(Bn)-OH, Fmoc-Hph-OH, Fmoc-Phe{#($CH_2$)2}-OH, Fmoc-b-MeAla-OH, Fmoc-Ala(Thz)-OH, Fmoc-Gly-OH, and others were purchased from Watanabe Chemical Industries, Chempep, Chem-Impex, or Bachem. Fmoc-nPrGly-OH, Fmoc-MePhe(3-Cl)-OH, Fmoc-MeAla(4-Thz)-OH, Fmoc-MeSer(DMT)-OH, and others were synthesized by the method described in WO 2013/100132. Fmoc-Thr(THP)-OH (Compound aa01), Fmoc-Tyr(3-F,tBu)-OH (Compound aa02), Fmoc-Tyr(3-F,Pis)-OH (Compound aa03), Fmoc-MePhe(4-Cl)-OH (Compound aa04), Fmoc-Me-His(Trt)-OH (Compound aa05), Fmoc-MeSer(THP)-OH (Compound aa06), Fmoc-Ser(3-F-5-Me-Pyr)-OH (Compound aa10), Fmoc-MeHph-OH (Compound aa11), Fmoc-Ser(Ph-3-Cl)-OH (Compound aa12), and others were synthesized as described below.

Fmoc-MeAbu-OH, Fmoc-D-Abu-OH, Fmoc-D-MeLeu-OH, Fmoc-MeNva-OH, Fmoc-(PhEt)NGly-OH, Fmoc-Hse(Et)-OH, Fmoc-Lys(Ac)-OH, Fmoc-Phe(4-CHF2)-OH, and Fmoc-Phe(4-OCHF2)-OH were purchased from Amatek, Watanabe Chemical Industries, ChemBioBank, and others.

Fmoc-Ser(nPr)-OH (Compound aa51), Fmoc-MeSer(nPr)-OH (Compound aa52), Fmoc-Ser(iPen)-OH (Compound aa54), Fmoc-MeSer(iPen)-OH (Compound aa55), Fmoc-Hnl(7-F2)-OH (Compound aa58), Fmoc-MeHnl(7-F2)-OH (Compound aa59), Fmoc-Ser(Ph-2-Cl)-OH (Compound aa60), Fmoc-MeSer(Ph-2-Cl)-OH (Compound aa65), Fmoc-MeSer(3-F-5-Me-Pyr)-OH (Compound aa67), Fmoc-Ser(F(4)nPr)-OH (Compound aa72), Fmoc-Hph(2-Cl)-OH (Compound aa73), Fmoc-MeHph(2-Cl)-OH (Compound aa75), Fmoc-Hph(3-Cl)-OH (Compound aa76), Fmoc-MeHph(3-Cl)-OH (Compound aa78), Fmoc-Hph(4-Cl)-OH (Compound aa79), Fmoc-MeHph(4-Cl)-OH (Compound aa81), Fmoc-MePhe{#(CH2)2}-OH (Compound aa82), Fmoc-MeSer(Bn)-OH (Compound aa83), Fmoc-Hyp(Et)-OH (Compound aa84), Fmoc-Ala(3-Pyr-4-NMe2)-OH (Compound aa88), Fmoc-nPenGly-OH (Compound aa89), Fmoc-nHexGly (Compound aa90), Fmoc-(EtOEt)NGly-OH (Compound aa91), Fmoc-(PhOEt)NGly-OH (Compound aa92), Fmoc-Gln(Me2)-OH (Compound aa94), Fmoc-MeGln(Me2)-OH (Compound aa96), Fmoc-Gln(Me)-OH (Compound aa98), Fmoc-MeGln(Me)-OH (Compound aa101), Fmoc-Ser(NtBu-Aca)-OH (Compound aa104), Fmoc-MeSer(NtBu-Aca)-OH (Compound aa105), Fmoc-MeHse(Et)-OH (Compound aa106), Fmoc-Nle(6-OTHP)-OH (Compound aa107), Fmoc-Abu(pip-4-F2)-OH (Compound aa109), Fmoc-MeAbu(pip-4-F2)-OH (Compound aa110), Fmoc-MeAbu(pip-3-F2)-OH (Compound aa116), Fmoc-Abu(Mor)-OH (Compound aa123), Fmoc-MeAbu(Mor)-OH (Compound aa125), Fmoc-(2-(pip-4-F2)-Et)Gly-OH (Compound aa126), Fmoc-Pro(pip-4-F2)-OH (Compound aa130), Fmoc-cisPro(pip-4-F2)-OH (Compound aa133), Fmoc-Ahp(2)(3-R-OTHP)-OH (Compound aa137), Fmoc-Ser(EtOTHP)-OH (Compound aa142), Fmoc-MeSer(EtOTHP)-OH (Compound aa147), Fmoc-Ser(S-2-PrOTHP)-OH (Compound aa154), Fmoc-MeSer(S-2-PrOTHP)-OH (Compound aa159), Fmoc-Ser(R-2-PrOTHP)-OH (Compound aa166), Fmoc-MeSer(R-2-PrOTHP)-OH (Compound aa171), Fmoc-Ser(tBuOH)-OH (Compound aa173), Fmoc-Ser(tBuOTHP)-OH (Compound aa174), Fmoc-MeSer(tBuOTHP)-OH (Compound aa175), Fmoc-Ser(2-Me-BuOH)-OH (Compound aa181), Fmoc-Ser(2-Me-BuOTHP)-OH (Compound aa182), Fmoc-MeSer(2-Me-BuOH)-OH (Compound aa184), Fmoc-MeAla(3-Pyr-4-NMe2)-OH (Compound aa186), Fmoc-MePhe(4-CF3)-OH (Compound aa187), Fmoc-MePhe(4-CHF2)-OH (Compound aa188), Fmoc-MePhe(4-OCHF2)-OH (Compound aa189), Fmoc-Ser(Ph-4-Cl)-OH (Compound aa190), Fmoc-Ser(Et-2-Mor)-OH (Compound aa191), Fmoc-nBuGly-OH (Compound aa192), Fmoc-iPenGly-OH (Compound aa193), Fmoc-Abu(5-Oxo-Odz)-OH (Compound aa194), Fmoc-MeAbu(5-Oxo-Odz)-OH (Compound aa196), Fmoc-MeAla(3-Pyr)-OH (Compound aa197), Fmoc-Ser(Et-2-NMe2)-OH (Compound aa198), Fmoc-Gln(Ms)-OH (Compound aa199), Fmoc-Ser(1-CF3-EtOH)-OH (Compound aa201), Fmoc-MeSer(1-CF3-EtOH)-OH (Compound aa202), Fmoc-Ser(1-CF3-EtOTHP)-OH (Compound aa203), Fmoc-Hnl(7-F3-6-OH)-OH (Compound aa208), and others were synthesized as described below.

[Table 4-1]

Abbreviations for amino acids

| Chem code | structure |
|---|---|
| Met(O2) | 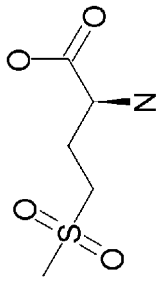 |
| nPrGly | 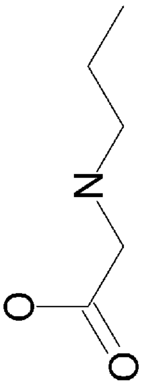 |
| Tyr(3-F) | 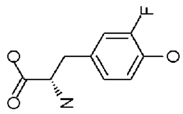 |
| MePhe(3-Cl) | 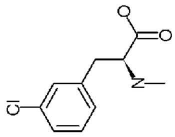 |
| MeAla(4-Thz) | 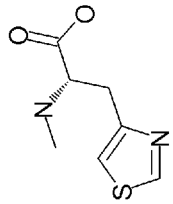 |

| Chem code | structure |
|---|---|
| Phg | 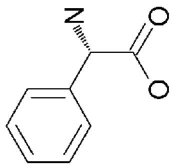 |
| MeHis | 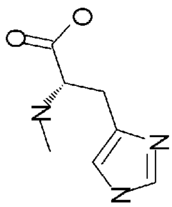 |
| Phe(4-CF3) | 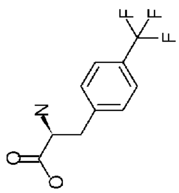 |
| Tyr | 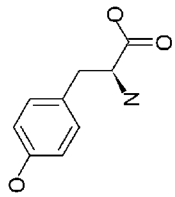 |
| Leu | 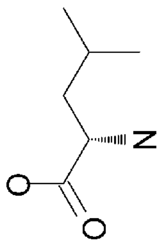 |

[Table 4-2]

| Chem code | structure |
|---|---|
| MeIle | 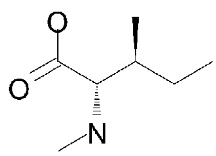 |

(continued)

| Chem code | structure |
|---|---|
| MeSer | 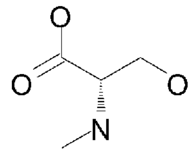 |
| MePhe | 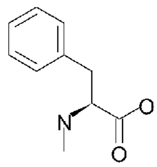 |
| Val | 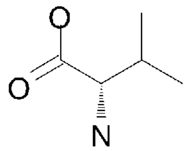 |
| Ile | 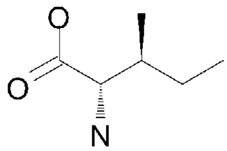 |
| MeGly | 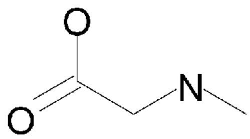 |
| MeTrp | 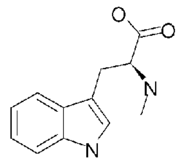 |
| Gly | 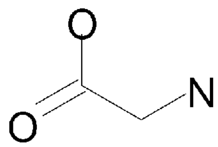 |
| MeAla | 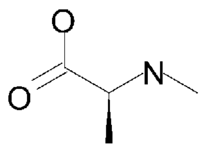 |

(continued)

| Chem code | structure |
|---|---|
| MeVal | 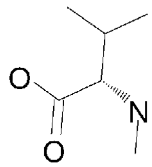 |
| Ala(4-Pyr) | 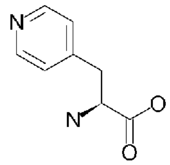 |
| Ala(3-Pyr) | 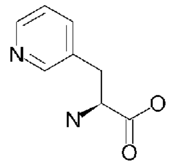 |
| Phe(3-Cl) | 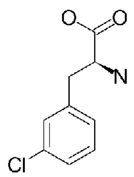 |
| MePhe(4-Cl) | 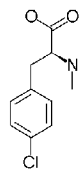 |
| EtGly | 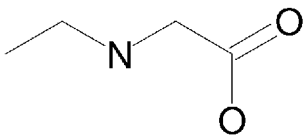 |

[Table 4-3]

| Chem code | structure |
|---|---|
| D-Val | 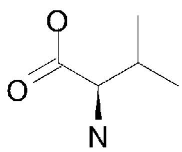 |

(continued)

| Chem code | structure |
|---|---|
| Ser(tBu) | 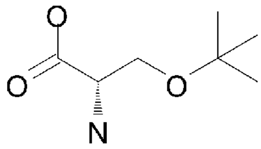 |
| D-MeAla | 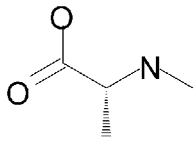 |
| g-MeAbu | 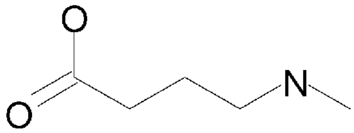 |
| Pic(2) | 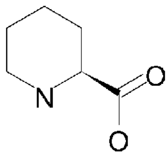 |
| MeLeu | 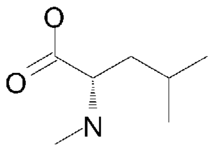 |
| Abu | 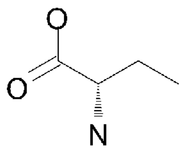 |
| D-Ala | 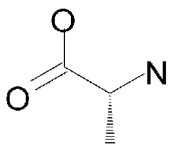 |
| D-Leu | 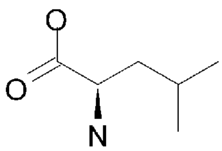 |

(continued)

| Chem code | structure |
|---|---|
| D-Pro | 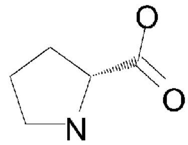 |
| Phe | 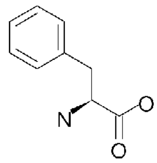 |
| Thr | 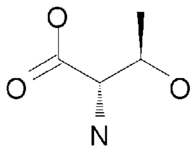 |
| Pro | 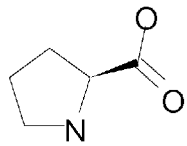 |
| Trp | 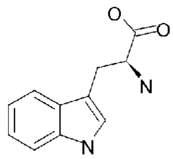 |
| Ala | 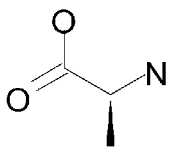 |

[Table 4-4]

| Chem code | structure |
|---|---|
| Ser(Bn) | 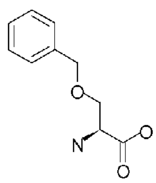 |

(continued)

| Chem code | structure |
|---|---|
| Ser(3-F-5-Me-Pyr) | 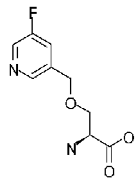 |
| Hph | 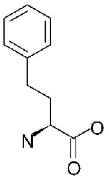 |
| MeHph | 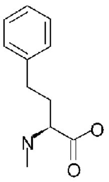 |
| Ser(Ph-3-Cl) | 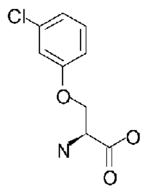 |
| Phe{#(CH2)2} | 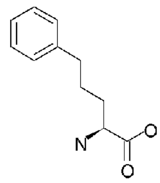 |
| b-MeAla | 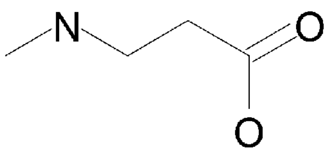 |
| Ala(Thz) | 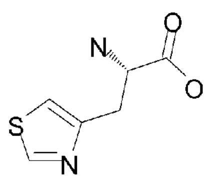 |

[Table 4-5]

| Chem code | structure |
|---|---|
| MeSer(nPr) | 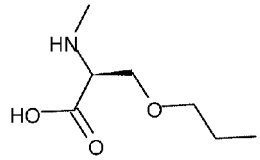 |
| Hnl(7-F2) | 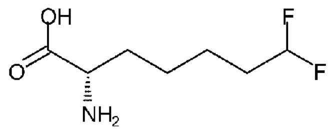 |
| MeHnl(7-F2) | 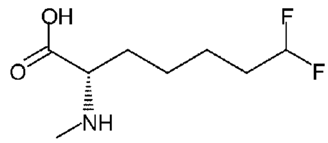 |
| Ser(Ph-2-Cl) | 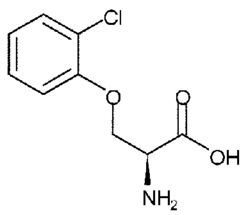 |
| Ser(iPen) | 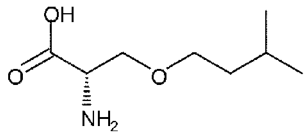 |
| MeSer(iPen) | 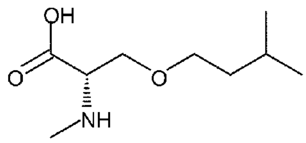 |

(continued)

| Chem code | structure |
|---|---|
| MeAbu | 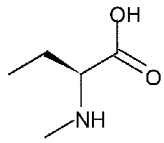 |
| Ser(nPr) | 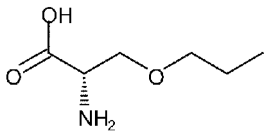 |

[Table 4-6]

| Chem code | structure |
|---|---|
| MeSer(3-F-5-Me-Pyr) | 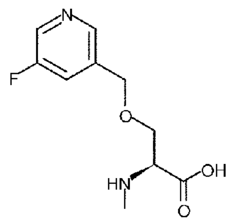 |
| Ser(F(4)nPr) | 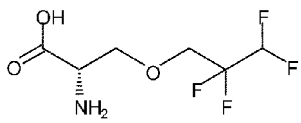 |
| **MeSer(F(4)nPr)** | 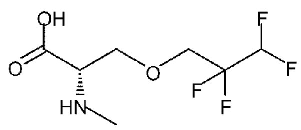 |
| **Hph(2-Cl)** | 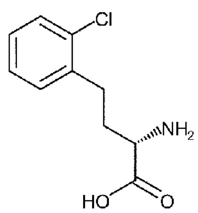 |

(continued)

| Chem code | structure |
|---|---|
| MeSer(Ph-2-Cl) | 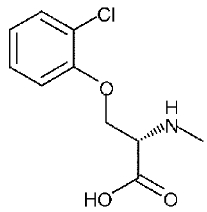 |
| MeSer(Ph-3-Cl) | 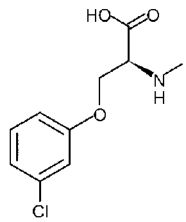 |
| Ser(Ph-4-Cl) | 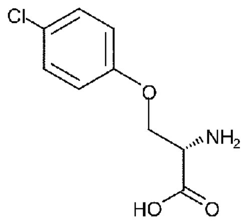 |
| MeSer(Ph-4-Cl) | 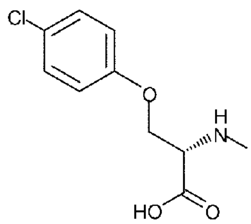 |

[Table 4-7]

| Chem code | structure |
|---|---|
| MeHph(4-Cl) | 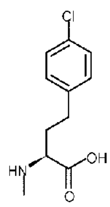 |

(continued)

| Chem code | structure |
|---|---|
| MePhe{#(CH2)2} | 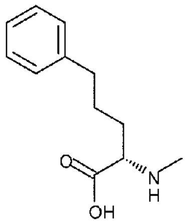 |
| MeSer(Bn) | 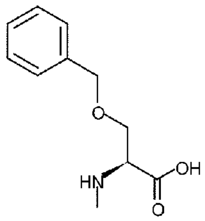 |
| Hyp(Et) | 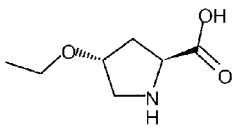 |
| MeHph(2-Cl) | 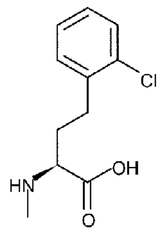 |
| Hph(3-Cl) | 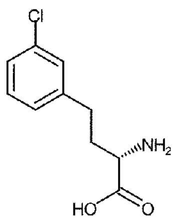 |
| MeHph(3-Cl) | 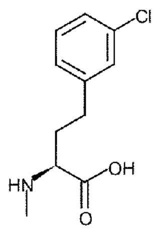 |

(continued)

| Chem code | structure |
|---|---|
| Hph(4-Cl) | 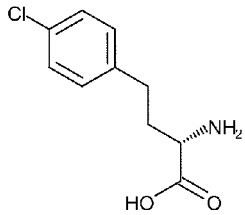 |

[Table 4-8]

| Chem code | structure |
|---|---|
| Abu(pi p-3-F2) | 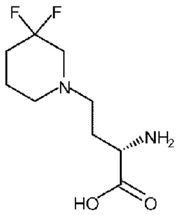 |
| MeAbu(pip-3-F2) | 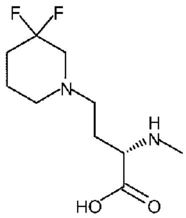 |
| Abu(pip-4-F2) | 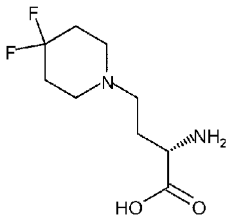 |
| MeAbu(pip-4-F2) | 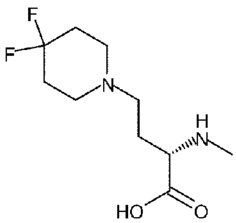 |
| D-Abu | 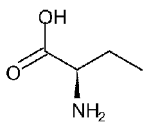 |

(continued)

| Chem code | structure |
|---|---|
| D-MeLeu | 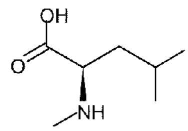 |
| MeNva | 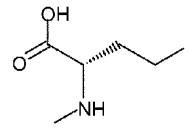 |
| nBuGly | 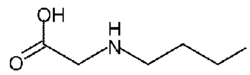 |

[Table 4-9]

| Chem code | structure |
|---|---|
| nHexGly | 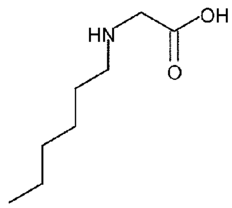 |
| (PhEt)NGly | 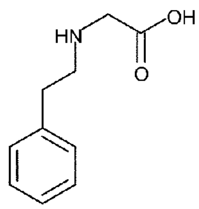 |
| (EtOEt)NGly | 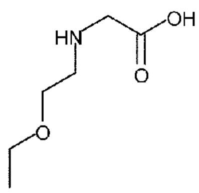 |

(continued)

| Chem code | structure |
|---|---|
| (PhOEt)NGly | 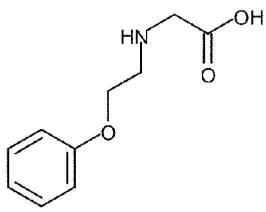 |
| Ala(3-Pyr-4-N Me2) | 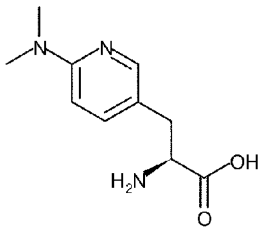 |
| MeAla(3-Pyr-4-NMe2) | 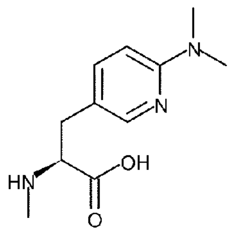 |
| iPenGly | 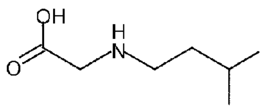 |
| nPenGly | 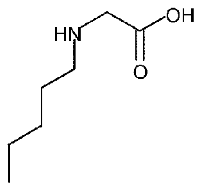 |

[Table 4-10]

| Chem code | structure |
|---|---|
| Ser(R-2-PrOH) | 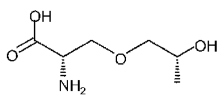 |

(continued)

| Chem code | structure |
|---|---|
| MeSer(R-2-PrOH) | 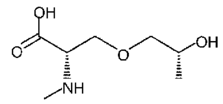 |
| Ser(tBuOH) | 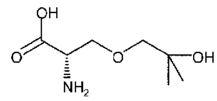 |
| MeSer(tBuOH) | 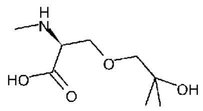 |
| Ser(EtOH) | 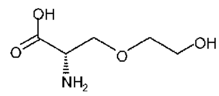 |
| MeSer(EtOH) | 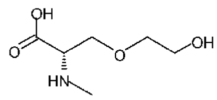 |
| Ser(S-2-PrOH) | 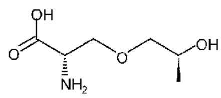 |
| MeSer(S-2-PrOH) | 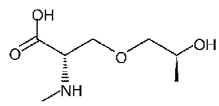 |

[Table 4-11]

| Chem code | structure |
|---|---|
| Ser(NtBu-Aca) | 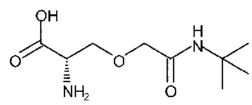 |
| MeSer(NtBu-Aca) | 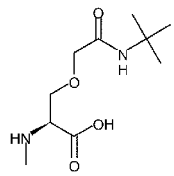 |
| Hse(Et) | 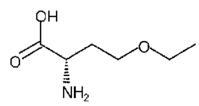 |

(continued)

| Chem code | structure |
|---|---|
| MeHse(Et) | 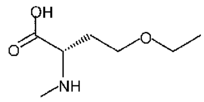 |
| Ser(2-Me-2-BuOH) | 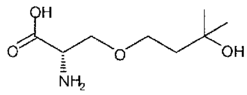 |
| MeSer(2-Me-2-BuOH) | 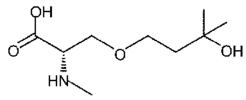 |
| Gln(Me2) | 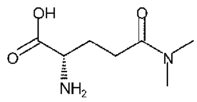 |
| MeGln(Me2) | 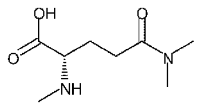 |

[Table 4-12]

| Chem code | structure |
|---|---|
| (2-(pip-4-F2)-Et)Gly | 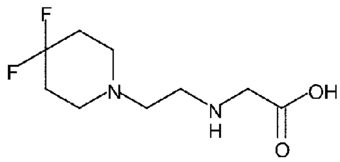 |
| Pro(4-pip-4-F2) | 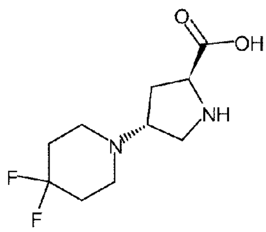 |

(continued)

| Chem code | structure |
|---|---|
| cisPro(4-pip-4-F2) | 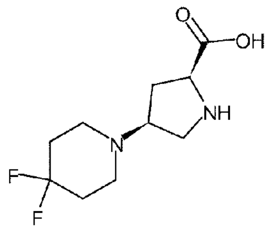 |
| Gln(Me) | 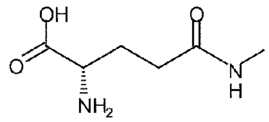 |
| Nle(6-OH) | 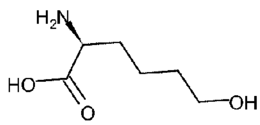 |
| MeNle(6-OH) | 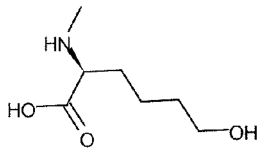 |
| Abu(Mor) | 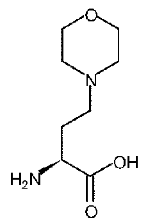 |
| MeAbu(Mor) | 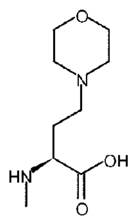 |

[Table 4-13]

| Chem code | structure |
|---|---|
| MeLys(Ac) | 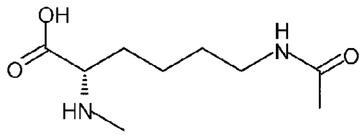 |
| Phe(4-CHF2) | 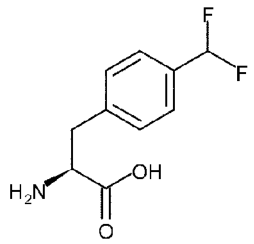 |
| MePhe(4-CHF2) | 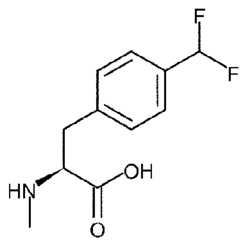 |
| Phe(4-OCHF2) | 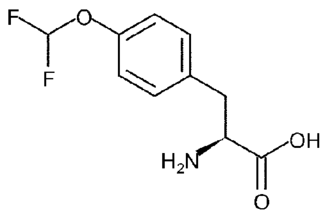 |
| MeGln(Me) | 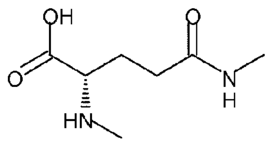 |
| Ahp(2)(3-R-OH) | 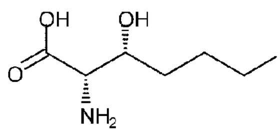 |

(continued)

| Chem code | structure |
|---|---|
| MeAhp(2)(3-R-OH) | 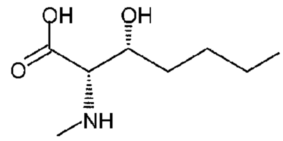 |
| Lys(Ac) | 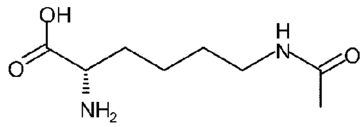 |

[Table 4-14]

| Chem code | structure |
|---|---|
| MeAbu(5-Oxo-Odz) | 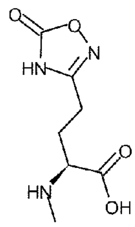 |
| Ser(Et-2-NMe2) | 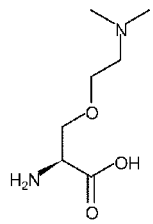 |
| Gln(Ms) | 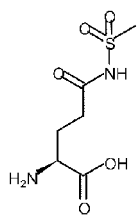 |
| MePhe(4-OCHF2) | 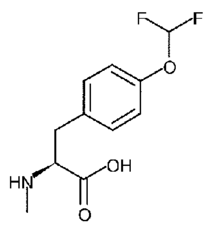 |

(continued)

| Chem code | structure |
|---|---|
| Ser(Et-2-Mor) | 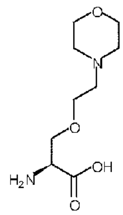 |
| MeSer(Et-2-Mor) | 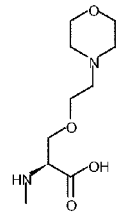 |
| Abu(5-Oxo-Odz) | 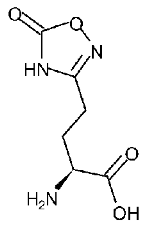 |

[Table 4-15]

| Chem code | structure |
|---|---|
| Ser(1-CF3-EtOH) | 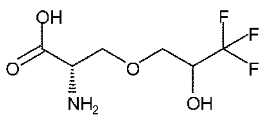 |
| MeSer(1-CF3-EtOH) | 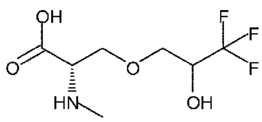 |
| HnI(7-F3-6-OH) | 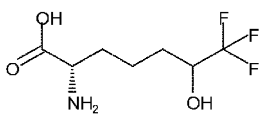 |
| MeHnI(7-F3-6-OH) | 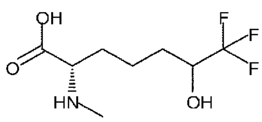 |

[Table 4-16]

| name | structure |
|---|---|
| Fmoc-MeLeu-OH | 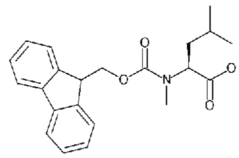 |
| Fmoc-Leu-OH | 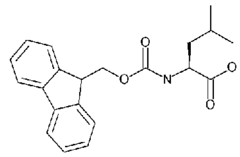 |
| Fmoc-MeTrp-OH | 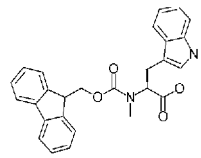 |
| Fmoc-MePhe-OH | 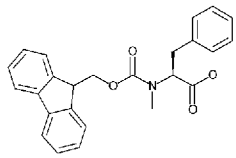 |
| Fmoc-MeIle-OH | 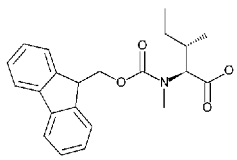 |
| Fmoc-Thr(THP)-OH | 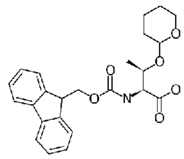 |
| Fmoc-Thr(Trt)-OH | 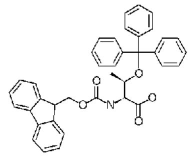 |
| Fmoc-MeGly-OH | 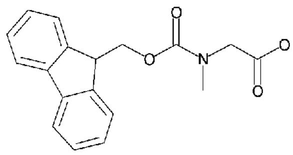 |

(continued)

| name | structure |
|---|---|
| Fmoc-g-MeAbu-OH | 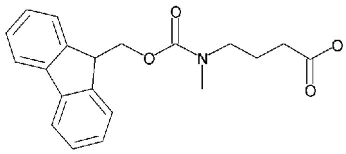 |
| Fmoc-Ile-OH | 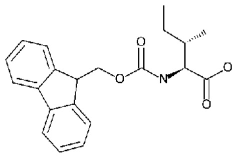 |

[Table 4-17]

| name | structure |
|---|---|
| Fmoc-Ser(tBu)-OH | 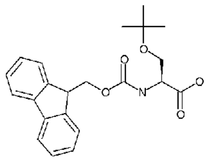 |
| Fmoc-Trp-OH | 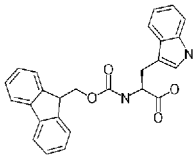 |
| Fmoc-MeAla-OH | 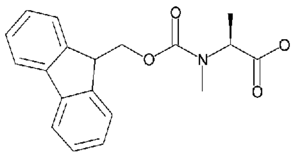 |
| Fmoc-Val-OH | 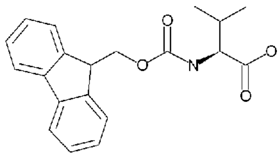 |
| Fmoc-D-Ala-OH | 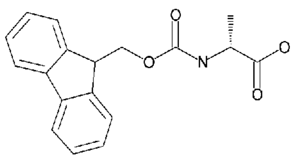 |

(continued)

| name | structure |
|---|---|
| Fmoc-MeVal-OH | 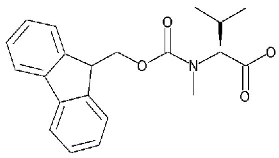 |
| Fmoc-Ala-OH | 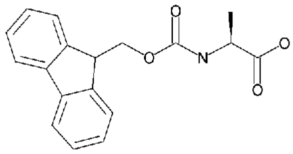 |
| Fmoc-D-Val-OH | 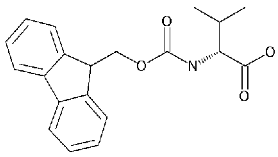 |
| Fmoc- Tyr(tBu)-OH | 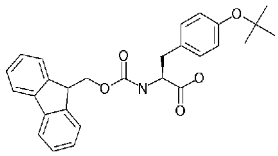 |
| Fmoc-Tyr(Clt)-OH | 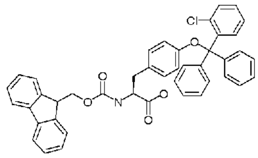 |

[Table 4-18]

| name | structure |
|---|---|
| Fmoc-Tyr(3-F, tBu)-OH | 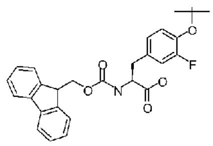 |
| Fmoc-D-Leu-OH | 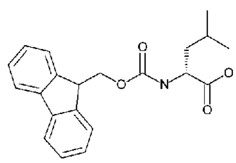 |
| Fmoc-Phe(4-CF3)-OH | 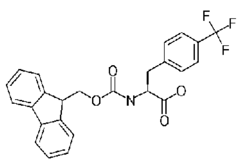 |

(continued)

| name | structure |
|---|---|
| Fmoc-D-MeAla-OH | 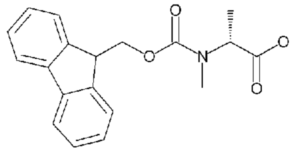 |
| Fmoc-Pro-OH | 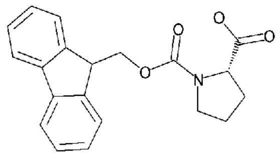 |
| Fmoc-Pic(2)-OH | 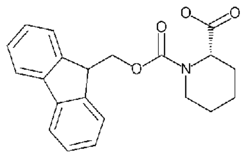 |
| Fmoc-EtGly-OH | 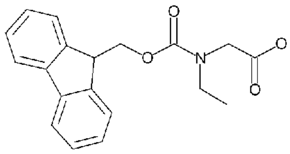 |
| Fmoc-nPrGly-OH | 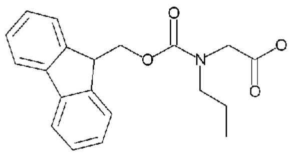 |
| Fmoc-MePhe(3-Cl)-OH | 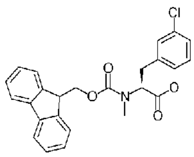 |
| Fmoc-D-Pro-OH | 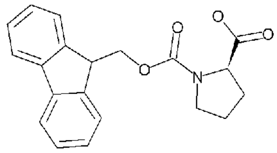 |

[Table 4-19]

| name | structure |
|---|---|
| Fmoc-Abu-OH | 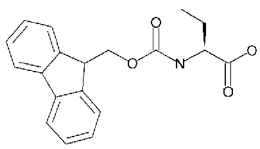 |

(continued)

| name | structure |
|---|---|
| Fmoc-Ala(3-Pyr)-OH | 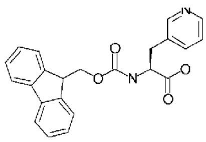 |
| Fmoc-Ala(4-Pyr)-OH | 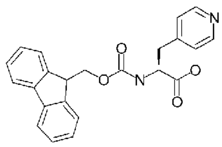 |
| Fmoc-Phe(3-Cl)-OH | 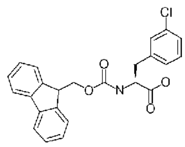 |
| Fmoc-MePhe(4-Cl)-OH | 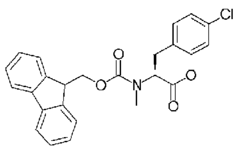 |
| Fmoc-Phg-OH | 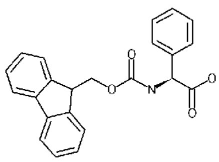 |
| Fmoc-MeHis(Trt)-OH | 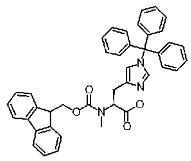 |
| Fmoc-MeAla(4-Thz)-OH | 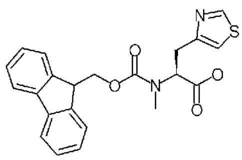 |
| Fmoc-MeSer(DMT)-OH | 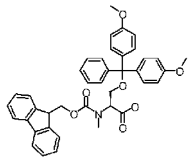 |

(continued)

| name | structure |
|---|---|
| Fmoc-MeSer(THP)-OH | 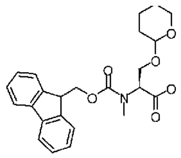 |

[Table 4-20]

| name | structure |
|---|---|
| Fmoc-Met(O2)-OH | 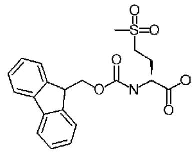 |
| Fmoc-Ser(Bn)-OH | 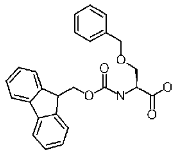 |
| Fmoc-Ser(3-F-5-Me-Pyr)-OH | 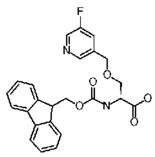 |
| Fmoc-Hph-OH | 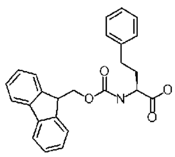 |
| Fmoc-MeHph-OH | 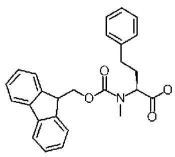 |
| Fmoc-Ser(Ph-3-Cl)-OH | 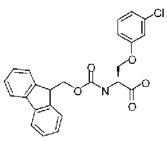 |
| Fmoc-Phe{#(CH2)2}-OH | 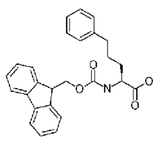 |

(continued)

| name | structure |
|---|---|
| Fmoc-b-MeAla-OH | 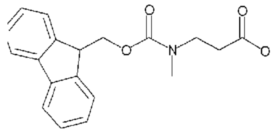 |
| Fmoc-Phe-OH | 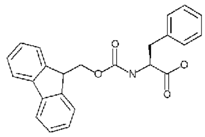 |
| Fmoc-Ala(4-Thz)-OH | 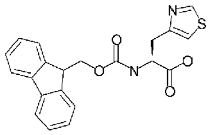 |
| Fmoc-Gly-OH | 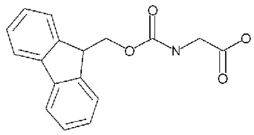 |

[Table 4-21]

| name | structure |
|---|---|
| Fmoc-MeSer(nPr)-OH | 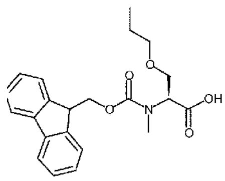 |
| Fmoc-Hnl(7-F2)-OH | 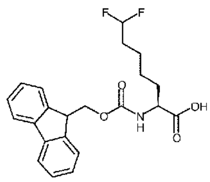 |
| Fmoc-MeHnl(7-F2)-OH | 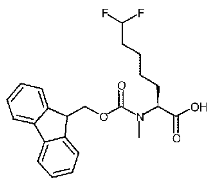 |

(continued)

| name | structure |
|---|---|
| Fmoc-Ser(Ph-2-Cl)-OH | 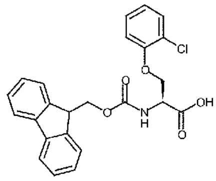 |
| Fmoc-Ser(iPen)-OH | 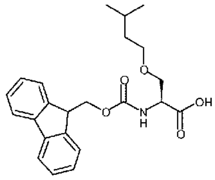 |
| Fmoc-MeSer(iPen)-OH | 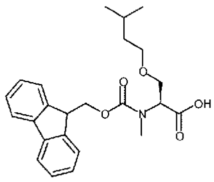 |
| Fmoc-MeAbu-OH | 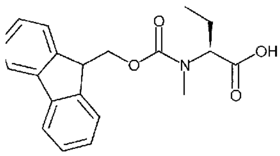 |
| Fmoc-Ser(nPr)-OH | 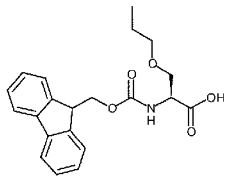 |

[Table 4-22]

| name | structure |
|---|---|
| Fmoc-MeSer(3-F-5-Me-Pyr)-OH | 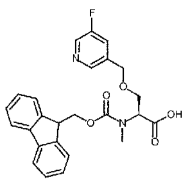 |
| Fmoc-Ser(F(4)nPr)-OH | 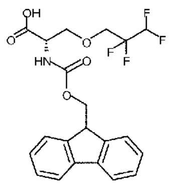 |

(continued)

| name | structure |
|---|---|
| Fmoc-MeSer(F(4)nPr)-OH | 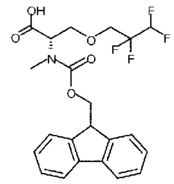 |
| Fmoc-Hph(2-Cl)-OH | 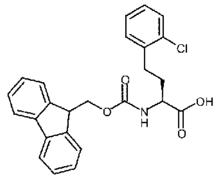 |
| **F**moc-MeSer(Ph-2-Cl)-OH | 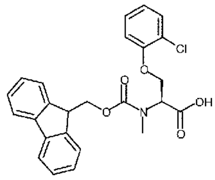 |
| **F**moc-MeSer(Ph-3-Cl)-OH | 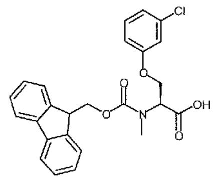 |
| Fmoc-Ser(Ph-4-Cl)-OH | 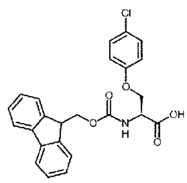 |
| Fmoc-MeSer(Ph-4-Cl)-OH | 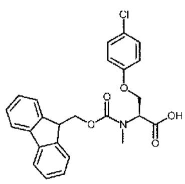 |

[Table 4-23]

| name | structure |
|---|---|
| Fmoc-MeHph(4-Cl)-OH | 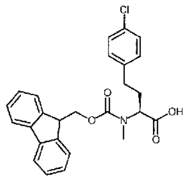 |
| Fmoc-MePhe{#(CH2)2}-OH | 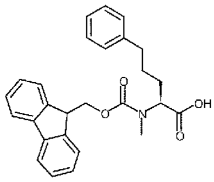 |
| Fmoc-MeSer(Bn)-OH | 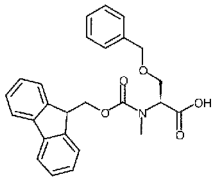 |
| Fmoc-Hyp(Et)-OH | 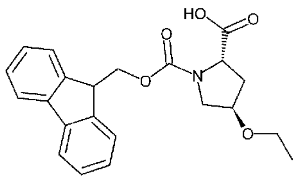 |
| Fmoc-MeHph(2-Cl)-OH | 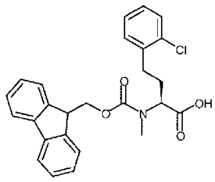 |
| Fmoc-Hph(3-Cl)-OH | 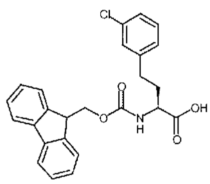 |
| Fmoc-MeHph(3-Cl)-OH | 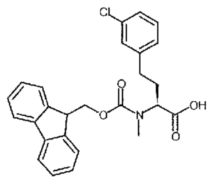 |

(continued)

| name | structure |
|---|---|
| Fmoc-Hph(4-Cl)-OH | 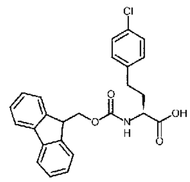 |

[Table 4-24]

| name | structure |
|---|---|
| **Fmoc-Abu(pip-3-F2)-OH** | 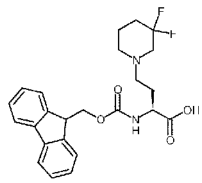 |
| **Fmoc-MeAbu(pip-3-F2)-OH** | 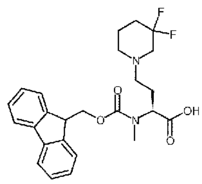 |
| **Fmoc-Abu(pip-4-F2)-OH** | 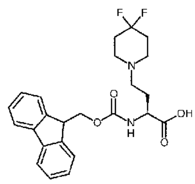 |
| **Fmoc-MeAbu(pip-4-F2)-OH** | 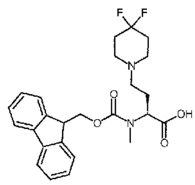 |
| **Fmoc-D-Abu-OH** | 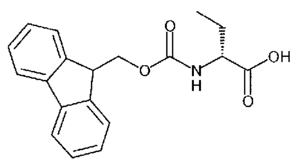 |
| **Fmoc-D-MeLeu-OH** | 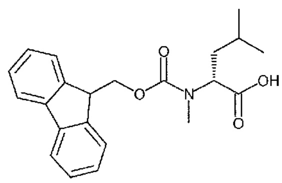 |

(continued)

| name | structure |
|---|---|
| Fmoc-MeNva-OH | 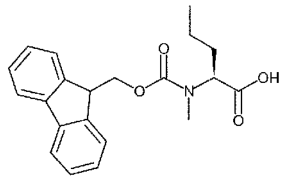 |
| Fmoc-nBuGly-OH | 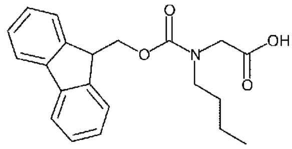 |

[Table 4-25]

| name | structure |
|---|---|
| Fmoc-nHexGly-OH | 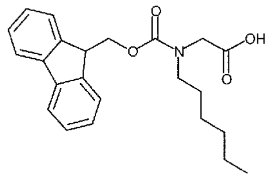 |
| Fmoc-(PhEt)NGly-OH | 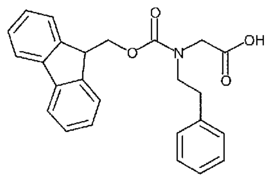 |
| Fmoc-(EtOEt)NGly-OH | 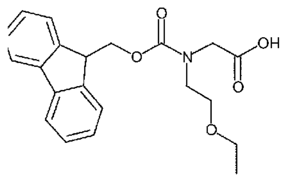 |
| Fmoc-(PhOEt)NGly-OH | 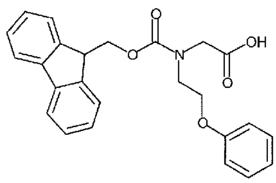 |
| Fmoc-Ala(3-Pyr-4-NMe2)-OH | 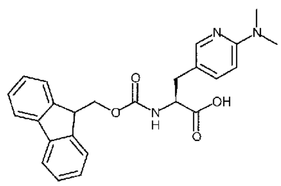 |

(continued)

| name | structure |
|---|---|
| Fmoc-MeAla(3-Pyr-4-NMe2)-OH | 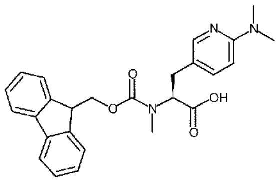 |
| Fmoc-iPenGly-OH | 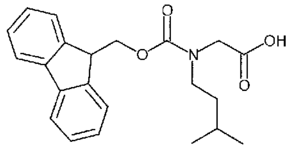 |
| Fmoc-nPenGly-OH | 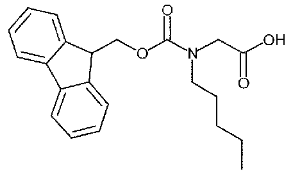 |

[Table 4-26]

| name | structure |
|---|---|
| Fmoc-Ser(R-2-PrOTHP)-OH | 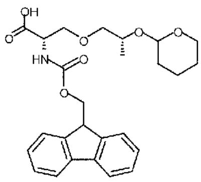 |
| Fmoc-MeSer(R-2-PrOTHP)-OH | 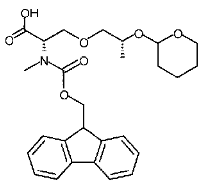 |
| Fmoc-Ser(tBuOH)-OH | 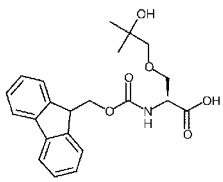 |

(continued)

| name | structure |
|---|---|
| Fmoc-Ser(tBuOTHP)-OH | 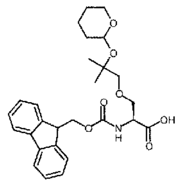 |
| Fmoc-Ser(EtOTHP)-OH | 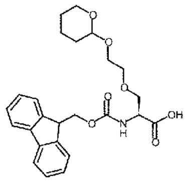 |
| Fmoc-MeSer(EtOTHP)-OH | 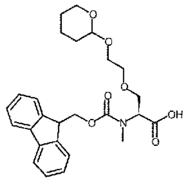 |
| Fmoc-Ser(S-2-PrOTHP)-OH | 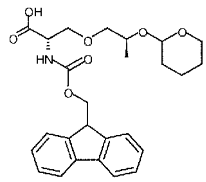 |
| Fmoc-MeSer(S-2-PrOTHP)-OH | 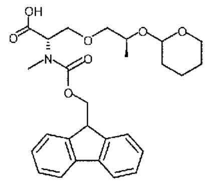 |

[Table 4-27]

| name | structure |
|---|---|
| Fmoc-MeSer(2-Me-2-BuOH)-OH | 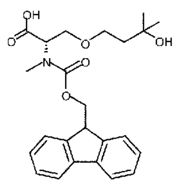 |

(continued)

| name | structure |
|---|---|
| **Fmoc-Gln(Me2)-OH** | 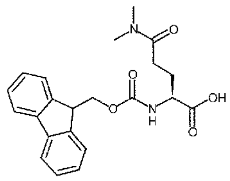 |
| **Fmoc-MeGln(Me2)-OH** | 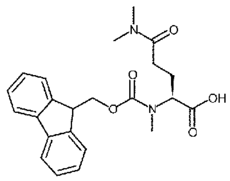 |
| **Fmoc-Ser(NtBu-Aca)-OH** | 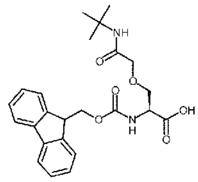 |
| **F**moc-MeSer(tBuOH)-OH | 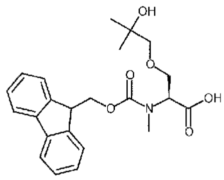 |
| **Fmoc-MeSer(tBuOTHP)-OH** | 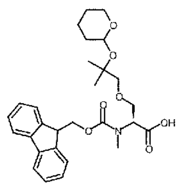 |
| **Fmoc-Ser(2-Me-2-BuOH)-OH** | 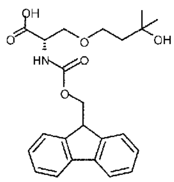 |
| **Fmoc-Ser(2-Me-2-BuOTHP)-OH** | 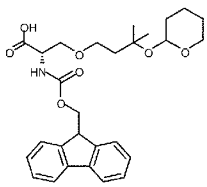 |

[Table 4-28]

| name | structure |
|---|---|
| Fmoc-MeNle(6-OTHP)-OH | 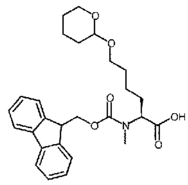 |
| Fmoc-Abu(Mor)-OH | 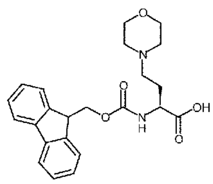 |
| Fmoc-MeAbu(Mor)-OH | 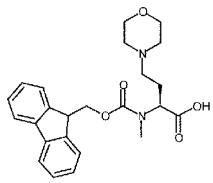 |
| Fmoc-(2-(pip-4-F2)-Et)Gly-OH | 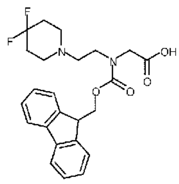 |
| Fmoc-MeSer(NtBu-Aca)-OH | 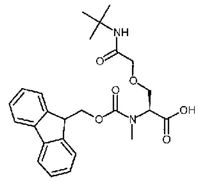 |
| Fmoc-Hse(Et)-OH | 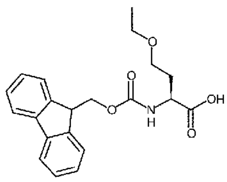 |
| Fmoc-MeHse(Et)-OH | 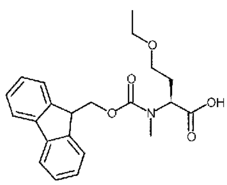 |

(continued)

| name | structure |
|---|---|
| Fmoc-Nle(6-OTHP)-OH | 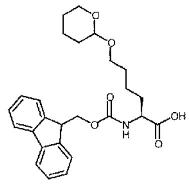 |

[Table 4-29]

| name | structure |
|---|---|
| Fmoc-Ahp(2)(3-R-OTHP) -OH | 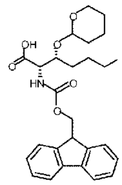 |
| Fmoc-MeAhp(2)(3-R-OTHP)-OH | 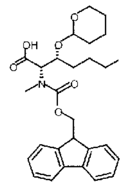 |
| Fmoc-Lys(Ac)-OH | 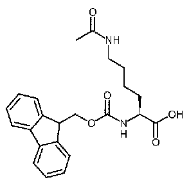 |
| Fmoc-MeLys(Ac)-OH | 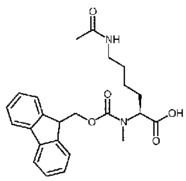 |
| Fmoc-Pro(4-pip-4-F2)-OH | 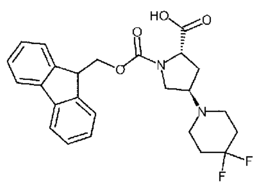 |
| Fmoc-cisPro(4-pip-4-F2)-OH | 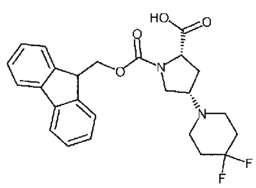 |

(continued)

| name | structure |
|---|---|
| Fmoc-Gln(Me)-OH | 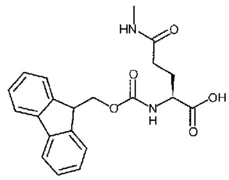 |
| Fmoc-MeGln(Me)-OH | 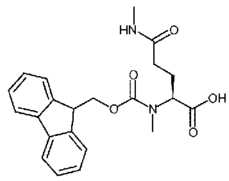 |

[Table 4-30]

| name | structure |
|---|---|
| Fmoc-Ser(Et-2-Mor)-OH | 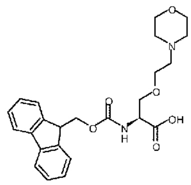 |
| Fmoc-MeSer(Et-2-Mor)-OH | 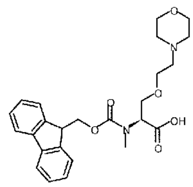 |
| Fmoc-Abu(5-Oxo-Odz)-OH | 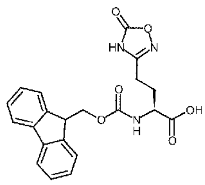 |
| Fmoc-MeAbu(5-Oxo-Odz)-OH | 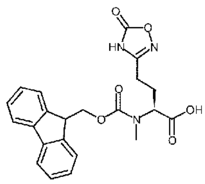 |

(continued)

| name | structure |
|---|---|
| Fmoc-Phe(4-CHF2)-OH | 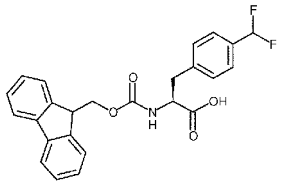 |
| Fmoc-MePhe(4-CHF2)-OH | 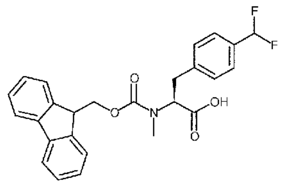 |
| Fmoc-Phe(4-OCHF2)-OH | 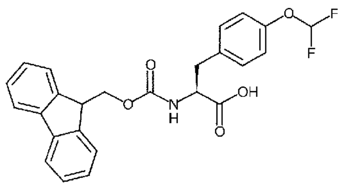 |
| Fmoc-MePhe(4-OCHF2)-OH | 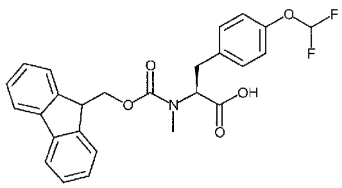 |

[Table 4-31]

| name | structure |
|---|---|
| Fmoc-Ser(Et-2-NMe2)-OH | 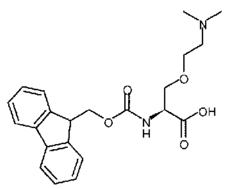 |
| Fmoc-Gln(Ms)-OH | 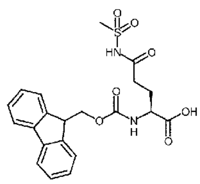 |

[Table 4-32]

| name | structure |
|---|---|
| Fmoc-Ser(1-CF3-EtOH)-OH | 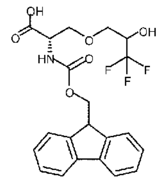 |
| Fmoc-Ser(1-CF3-EtOTHP)-OH | 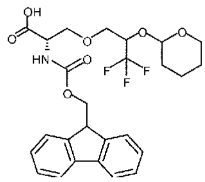 |
| Fmoc-MeSer(1-CF3-EtOH)-OH | 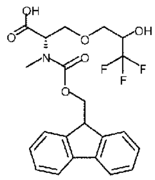 |
| Fmoc-HnI(7-F3-6-OH)-OH | 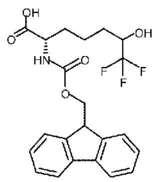 |
| Fmoc-MeHnI(7-F3-6-OH)-OH | 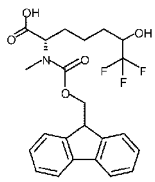 |

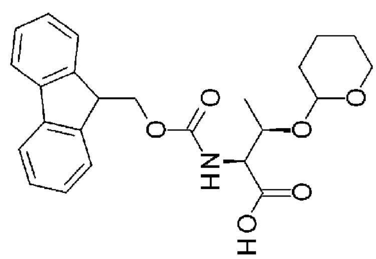

1-2 Synthesis of amino acids used for peptide synthesis by a peptide synthesizer

Synthesis of (2S,3R)-2-((((9H-fluoren-9-yl)methoxy)carbonyl)amino)-3-((tetrahydro-2H-pyran-2-yl)oxy)butonoic acid (Compound aa01, Fmoc-Thr(THP)-OH)

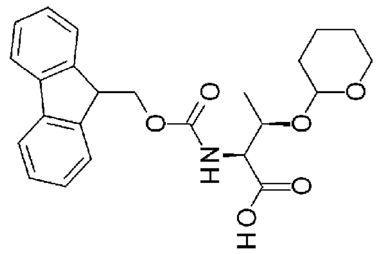

To a mixture of (2S,3R)-2-((((9H-fluoren-9-yl)methoxy)carbonyl)amino)-3-hydroxybutanoic acid monohydrate (Fmoc-Thr-OH monohydrate, purchased from Tokyo Chemical Industry 5.0 g, 13.9 mmol) and pyridium p-toluenesulfonate (PPTS, 0.175 g, 0.70 mmol) was added toluene (50 mL), and the toluene was evaporated under reduced pressure to azeotropically remove water. To the resulting residue were added ultradehydrated tetrahydrofuran (THF, 28 mL) and 3,4-dihydro-2H-pyran (8.8 mL, 97 mmol), and the mixture was stirred at 50°C for 4 h under a nitrogen atmosphere. After the disappearance of the raw materials was confirmed by LCMS (SQDFA05), the mixture was cooled to 25°C and ethyl acetate (30 mL) was added. The organic layer was then washed by adding a saturated aqueous sodium chloride solution (30 mL), and the aqueous layer was extracted with ethyl acetate (30 mL). All the resulting organic layers were combined and further washed with a saturated aqueous sodium chloride solution (30 mL) twice. The organic layers were dried over sodium sulfate and the solvent was evaporated under reduced pressure to give a crude product (9.3 g).

4.65 g of the resulting crude product was dissolved in tetrahydrofuran (THF, 30 mL), followed by addition of 1.0 M phosphate buffer adjusted to pH 8.0 (30 mL). This mixture was stirred at 50°C for 4 h. After cooling to 25°C, ethyl acetate (30 mL) was added and the organic layer and the aqueous layer were separated. The aqueous layer was extracted by adding ethyl acetate (30 mL), and all the resulting organic layers were then combined and washed with a saturated aqueous sodium chloride solution (30 mL) twice. The organic layers were dried over sodium sulfate, the solvent was evaporated under reduced pressure, and the residue was further dried under reduced pressure using a pump at 25°C for 30 min.

The resulting residue was dissolved in diethyl ether (50 mL) and heptane (50 mL) was then added. Only the diethyl ether was evaporated under controlled reduced pressure (~100 hPa) and the resulting mixture was filtered to give a solid. This washing operation with heptane was repeated twice. The resulting solid was dried under reduced pressure using a pump at 25°C for 2 h to afford Fmoc-Thr(THP)-OH sodium salt (2.80 g, 6.26 mmol). Ethyl acetate (50 mL) and 0.05 M aqueous phosphoric acid solution, pH 2.1 (140 mL) were added to the total amount of the resulting Fmoc-Thr(THP)-OH sodium salt. After stirring at 25°C for 5 min, the organic layer and the aqueous layer were separated. The aqueous layer was extracted by adding ethyl acetate (50 mL), and all the resulting organic layers were then combined and washed with a saturated aqueous sodium chloride solution (50 mL) twice. The organic layers were dried over sodium sulfate and the solvent was evaporated under reduced pressure. The residue was dried under reduced pressure using a pump at 25°C for 2 h, after which the resulting solid was dissolved in t-butyl methyl ether (TBME, 50 mL) and the solvent was evaporated under reduced pressure. The residue was further dried under reduced pressure using a pump at 25°C for 1 h to afford (2S,3R)-2-((((9H-fluoren-9-yl)methoxy)carbonyl)amino)-3-((tetrahydro-2H-pyran-2-yl)oxy)butanoic acid (Compound aa01, Fmoc-Thr(THP)-OH, 2.70 g, 30 mol% of t-butyl methyl ether (TBME) remaining) as a THP-protected diastereomer derived from asymmetric carbon. The obtained Fmoc-Thr(THP)-OH was stored in a freezer at -25°C.
LCMS (ESI) m/z = 424.2 $(M-H)^-$
Retention time: 0.84 min, 0.85 min (analytical condition SQDFA05)

Synthesis of (S)-methyl 2-((((9H-fluoren-9-yl)methoxy)carbonyl)amino)-3-(3-fluoro-4-hydroxyphenyl)propanoate (Compound aa07, Fmoc-Tyr(3-F)-OMe)

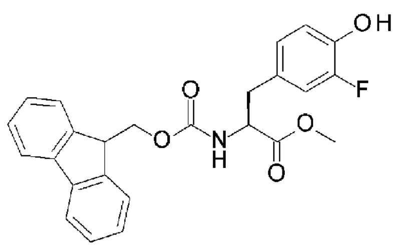

(S)-2-Amino-3-(3-fluoro-4-hydroxyphenyl)propanoic acid ($H_2N$-Tyr(3-F)-OH, purchased from Astatech, 2.0 g, 10.0 mmol) was dissolved in a 10% aqueous sodium carbonate solution, and a solution of (9H-fluoren-9-yl)methyl (2,5-dioxopyrrolidin-1-yl) carbonate (Fmoc-OSu, 3.39 g, 10.0 mmol) in 1,4-dioxane (35 mL) was then added via a dropping funnel at 0°C. The reaction solution was stirred at 25°C for 40 minutes, after which water (35 mL) and diethyl ether (70 mL) were added and the mixture was washed with diethyl ether three times. The aqueous layer was adjusted to pH 2-3 with a 5 N aqueous hydrochloric acid solution and then extracted with ethyl acetate three times (100 mL x 3). The organic layers were dried over magnesium sulfate, the solvent was evaporated under reduced pressure, and the residue was further dried using a pump. The resulting residue (4.08 g) was used as such in the next reaction without further purification.

The above residue (1.04 g) was dissolved in methanol (10 mL) and thionyl chloride ($SOCl_2$, 539 μL, 7.38 mmol) was added dropwise at 0°C. The reaction solution was stirred at 60°C for 1 h and then cooled to room temperature, and the solvent was evaporated using an evaporator. To the resulting residue were added ethyl acetate and water, and the mixture was extracted with ethyl acetate twice. The organic layers were washed with brine and dried over magnesium sulfate, the solvent was evaporated under reduced pressure, and the residue was further dried using a pump. The resulting residue was purified by flash column chromatography (Purif Pack (registered trademark) SIZE 200, hexane/ethyl acetate) to afford (S)-methyl 2-((((9H-fluoren-9-yl)methoxy)carbonyl)amino)-3-(3-fluoro-4-hydroxyphenyl)propanoate (Compound aa07, Fmoc-Tyr(3-F)-OMe, 900 mg, 2.07 mmol) in 84% yield over two steps.
LCMS (ESI) m/z = 436.4 $(M+H)^+$
Retention time: 0.82 min (analytical condition SQDFA05)

Synthesis of (S)-2-((((9H-fluoren-9-yl)methoxy)carbonyl)amino)-3-(4-(tert-butoxy)-3-fluorophenyl)propanoic acid (Compound aa02, Fmoc-Tyr(3-F,tBu)-OH)

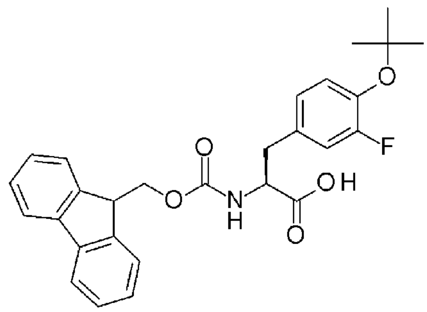

To a solution of (S)-methyl 2-((((9H-fluoren-9-yl)methoxy)carbonyl)amino)-3-(3-fluoro-4-hydroxyphenyl)propanoate (Compound aa07, Fmoc-Tyr(3-F)-OMe, 300 mg, 0.689 mmol) in tetrahydrofuran (THF) (690 μL) were added tert-butyl 2,2,2-trichloroacetimidate (308 μL, 1.72 mmol) and a catalytic amount of boron trifluoride-ethyl ether complex ($BF_3$-$OEt_2$, 13.1 μL, 0.103 mmol) dropwise at 0°C. After stirring the reaction solution at 25°C for 1 h, the same amounts of tert-butyl 2,2,2-trichloroacetimidate (308 μL, 1.72 mmol) and boron trifluoride-ethyl ether complex ($BF_3$-$OEt_2$, 13.1 μL, 0.103 mmol) were added again and the reaction solution was further stirred at 25°C for 1 h. The reaction solution was diluted with dichloromethane (DCM) and a saturated aqueous sodium bicarbonate solution was added. After extraction with dichloromethane, the organic layer was washed with a saturated aqueous sodium bicarbonate solution and brine. The organic layer was dried over sodium sulfate, the solvent was evaporated under reduced pressure, and the residue was further dried using a pump. The resulting residue was purified by flash column chromatography (Purif Pack (registered trademark) SIZE 60, hexane/ethyl acetate) to afford (S)-methyl 2-((((9H-fluoren-9-yl)methoxy)carbonyl)amino)-3-(4-(tert-butoxy)-3-fluorophenyl)propanoate (Fmoc-Tyr(3-F,tBu)-OMe) as a mixture.

The mixture obtained as above (40 mg) was dissolved in dichloroethane (DCE) (810 μL), trimethyltin(IV) hydroxide ($Me_3SnOH$, 29.4 mg, 0.163 mmol) was added, and the mixture was stirred at 60°C for 1 h. Formic acid (15.35 μL, 0.407 mmol) was added to the reaction solution, and the mixture was then purified by reverse phase chromatography (Wakosil 25C18 10 g, 0.1% aqueous formic acid solution/0.1% formic acid-acetonitrile solution) to afford (S)-2-((((9H-fluoren-9-yl)methoxy)carbonyl)amino)-3-(4-(tert-butoxy)-3-fluorophenyl)propanoic acid (Compound aa02, Fmoc-Tyr(3-F,tBu)-OH, 27 mg, 56.5 μmol) in 93% yield over two steps.
LCMS (ESI) m/z = 478.3 $(M+H)^+$
Retention time: 0.94 min (analytical condition SQDFA05)

Synthesis of (S)-methyl 2-((((9H-fluoren-9-yl)methoxy)carbonyl)amino)-3-(3-fluoro-4-((2-phenylpropan-2-yl)oxy)phenyl)propanoate (Compound aa08, Fmoc-Tyr(3-F,Pis)-OMe)

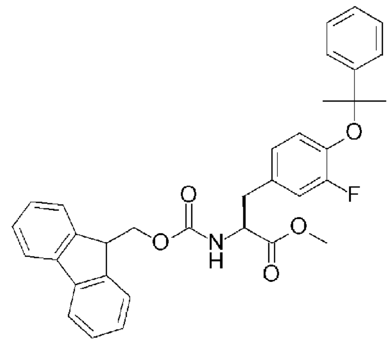

(S)-Methyl 2-((((9H-fluoren-9-yl)methoxy)carbonyl)amino)-3-(3-fluoro-4-hydroxyphenyl)propanoate (Compound aa07, Fmoc-Tyr(3-F)-OMe, 200 mg, 0.459 mmol) was dissolved in THF (460 μL), after which pre-prepared 2-phenylpropan-2-yl 2,2,2-trichloroacetimidate (Compound aa09, 322 mg, 1.15 mmol) and a catalytic amount of boron trifluoride-ethyl ether complex ($BF_3$-$OEt_2$, 8.73 μL, 0.069 mmol) were added dropwise at 0°C. After stirring the reaction solution at room temperature for 30 min, the same amounts of 2-phenylpropan-2-yl 2,2,2-trichloroacetimidate (322 mg, 1.15 mmol) and boron trifluoride-ethyl ether complex ($BF_3$-$OEt_2$, 8.73 μL, 0.069 mmol) were added dropwise at 0°C. After further stirring the reaction solution at room temperature for 30 min, the reaction solution was diluted with dichloromethane and a saturated aqueous sodium bicarbonate solution was added under ice-cooling. After extraction with dichloromethane, the organic layer was washed with brine. The organic layer was dried over sodium sulfate, the solvent was evaporated under reduced pressure, and the residue was further dried using a pump. The resulting residue was washed with dichloromethane/hexane = 1/1 (20 mL, 10 mL) twice and the white solid was removed by filtration. The resulting filtrate was concentrated and the residue was purified by flash column chromatography (Purif Pack SIZE 20, hexane/ethyl acetate, 0.1% diisopropylethylamine (DIPEA)) to afford (S)-methyl 2-((((9H-fluoren-9-yl)methoxy)carbonyl)amino)-3-(3-fluoro-4-((2-phenylpropan-2-yl)oxy)phenyl)propanoate (Compound aa08, Fmoc-Tyr(3-F,Pis)-OMe, 210 mg, 0.379 mmol) in 83% yield.
LCMS (ESI) m/z = 554.4 $(M+H)^+$
Retention time: 1.09 min (analytical condition SQDFA05)

Preparation of 2-phenylpropan-2-yl 2,2,2-trichloroacetimidate (Compound aa09)

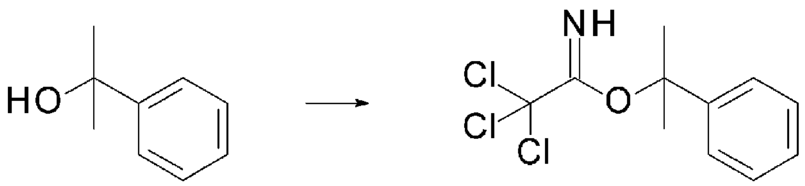

Compound aa09

To a solution of 2-phenylpropan-2-ol (purchased from Wako, 2.0 g, 14.7 mmol) in diethyl ether ($Et_2O$) (4.8 mL) was added a 1.9 M solution of NaHMDS in tetrahydrofuran (THF) (850 μL, 1.62 mmol) dropwise at 22°C. The reaction solution was stirred at the same temperature for 20 min and then cooled to 0°C, and 2,2,2-trichloroacetonitrile (1.47 mL, 14.7 mmol) was added dropwise. The reaction solution was stirred at 0°C for 10 minutes, warmed to 15°C, and further stirred for 1 h. The reaction solution was concentrated by an evaporator, hexane (1.8 mL) and methanol (65 μL) were added to the resulting residue, and the mixture was stirred at 15°C for 15 min. The resulting solid was filtered and washed with hexane (2.0 mL) three times to afford 4.19 g of 2-phenylpropan-2-yl 2,2,2-trichloroacetimidate (Compound aa09). This was used as such for the reaction without further purification.
$^1H$ NMR (Varian 400-MR, 400 MHz, $CDCl_3$) δ 1.89 (6H, s), 7.28 (1H, m), 7.36 (2H, m), 7.43 (2H, m), 8.20 (1H, brs)

Synthesis of (S)-2-((((9H-fluoren-9-yl)methoxy)carbonyl)amino)-3-(3-fluoro-4-((2-phenylpropan-2-yl)oxy)phenyl)propanoic acid (Compound aa03, Fmoc-Tyr(3-F,Pis)-OH)

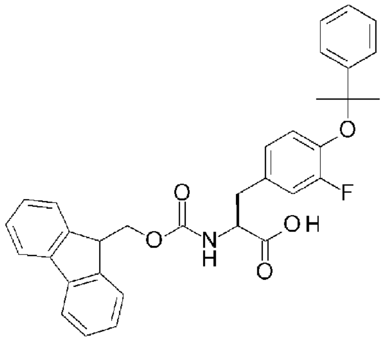

(S)-methyl 2-((((9H-fluoren-9-yl)methoxy)carbonyl)amino)-3-(3-fluoro-4-((2-phenylpropan-2-yl)oxy)phenyl)propanoate (Compound aa08, Fmoc-Tyr(3-F,Pis)-OMe, 210 mg, 0.379 mmol) was dissolved in dichloroethane (DCE) (1.26 mL), trimethyltin(IV) hydroxide ($Me_3SnOH$, 137 mg, 0.379 mmol) was added, and the mixture was stirred at 60°C for 3 h. The reaction solution was concentrated by an evaporator, t-butyl methyl ether (TBME, 2.0 mL) and 0.05 M aqueous phosphoric acid (pH 2.1, 4.0 mL) were added, and the mixture was stirred at 25°C for 15 min. The organic layer was separated and the aqueous layer was then extracted with t-butyl methyl ether (TBME, 1 mL) twice. The organic layers were dried over sodium sulfate, the solvent was evaporated under reduced pressure, and the residue was further dried using a pump. The resulting residue was dissolved in a 0.1% formic acid-acetonitrile solution and stirred for 15 min, and the resulting solution was then purified by reverse phase chromatography (Wakosil 25C18 30 g, 0.1% aqueous formic acid solution/0.1% formic acid-acetonitrile) to afford (S)-2-((((9H-fluoren-9-yl)methoxy)carbonyl)amino)-3-(3-fluoro-4-((2-phenylpropan-2-yl)oxy)phenyl)propanoic acid (Compound aa03, Fmoc-Tyr(3-F,Pis)-OH, 190 mg, 0.352 mmol) in 93% yield.
LCMS (ESI) m/z = 538.2 $(M-H)^-$
Retention time: 1.00 min (analytical condition SQDFA05)

Synthesis of (S)-2-((((9H-fluoren-9-yl)methoxy)carbonyl)(methyl)amino)-3-(4-chlorophenyl)propanoic acid (Compound aa04, Fmoc-MePhe(4-Cl)-OH)

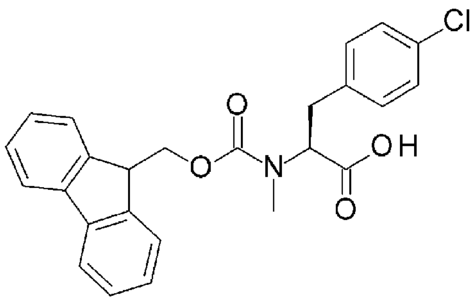

To a solution of (S)-2-((((9H-fluoren-9-yl)methoxy)carbonyl)amino)-3-(4-chlorophenyl)propanoic acid (Fmoc-Phe(4-Cl)-OH, 170 g, 402.96 mmol) in toluene (2.5 L) were added paraformaldehyde (48 g, 1.60 mol) and 10-camphorsulfonic acid (CSA, 4.6 g, 19.83 mmol), and the mixture was stirred at 110°C for 16 h. The reaction solution was then washed with a saturated aqueous sodium bicarbonate solution (1.0 L) twice and with a saturated aqueous sodium chloride solution (1.0 L) twice. The organic layer was dried over sodium sulfate, the solid was removed by filtration, and the solvent was evaporated under reduced pressure to afford 160 g of (S)-(9H-fluoren-9-yl)methyl 4-(4-chlorobenzyl)-5-oxooxazolidine-3-carboxylate.

A solution of (S)-(9H-fluoren-9-yl)methyl 4-(4-chlorobenzyl)-5-oxooxazolidine-3-carboxylate mixed with another lot prepared by the same operation (230 g, 530.10 mmol) in dichloromethane (2.5 L) was mixed with triethylsilane (881 g, 7.58 mol) and trifluoroacetic acid (TFA, 2518 g, 22.28 mol), and the mixture was stirred at 30°C for 12 h. The solvent

was then evaporated under reduced pressure, and the resulting residue was recrystallized with dichloromethane/hexane (1/10, v/v) to afford (S)-2-((((9H-fluoren-9-yl)methoxy)carbonyl)(methyl)amino)-3-(4-chlorophenyl)propanoic acid (Compound aa04, Fmoc-MePhe(4-Cl)-OH, 205 g).
LCMS (ESI) m/z = 436.3 (M+H)+
Retention time: 0.99 min (analytical condition SQDFA05)

Synthesis of (S)-2-((((9H-fluoren-9-yl)methoxy)carbonyl)(methyl)amino)-3-(1-trityl-1H-imidazol-4-yl)propanoic acid (Compound aa05, Fmoc-MeHis(Trt)-OH)

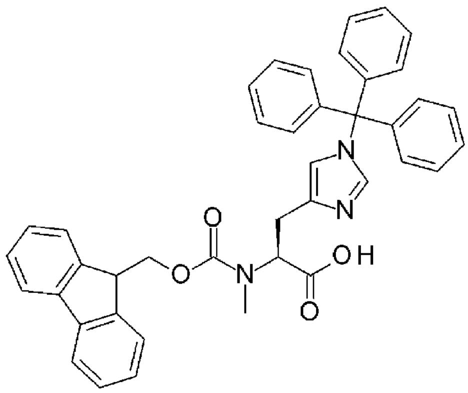

A solution of (S)-3-(1H-imidazol-4-yl)-2-(methylamino)propanoic acid hydrochloride (75 g, 364.71 mmol) in dichloromethane (1.0 L), dichlorodimethylsilane (51 g, 395.16 mmol), and triethylamine (40 g, 395.30 mmol) were put into a 3 L flask. A solution of (chloromethanetriyl)tribenzene (Trt-Cl, 111 g, 398.17 mmol) in dichloromethane (500 mL), and triethylamine (40 g, 395.30 mmol) were then added. The resulting reaction solution was stirred under heat reflux for 4 h and further stirred at 20°C for 2 h. The reaction was quenched by adding methanol to the reaction solution, and the solvent was then evaporated under reduced pressure. The pH was adjusted to 8-8.5 with triethylamine to give 125 g of a solid.

To the resulting solid were added 1,4-dioxane (1.0 L), potassium carbonate (84 g, 603.39 mmol), and water (1.0 L). (9H-Fluoren-9-yl)methyl (2,5-dioxopyrrolidin-1-yl) carbonate (Fmoc-OSu, 102 g, 302.38 mmol) was further added and the mixture was stirred at 0°C for two hours. The resulting reaction solution was washed with diethyl ether (2.0 L), and the solution was adjusted to pH 6-7 with acetic acid. The resulting solid was filtered to afford (S)-2-((((9H-fluoren-9-yl)methoxy)carbonyl)(methyl)amino)-3-(1-trityl-1H-imidazol-4-yl)propanoic acid (Compound aa05, Fmoc-MeHis(Trt)-OH, 155 g).
LCMS (ESI) m/z = 634.4 (M+H)+
Retention time: 1.07 min (analytical condition SQDAA05)

Synthesis of (2S)-2-((((9H-fluoren-9-yl)methoxy)carbonyl)(methyl)amino)-3-((tetrahydro-2H-pyran-2-yl)oxy)propanoic acid (Compound aa06, Fmoc-MeSer(THP)-OH)

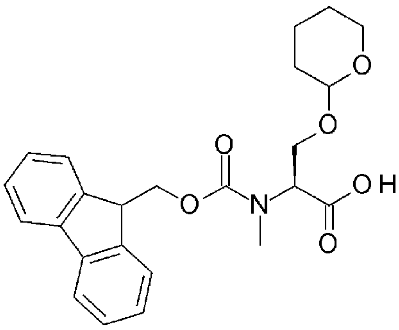

(S)-2-((((9H-Fluoren-9-yl)methoxy)carbonyl)(methyl)amino)-3-hydroxypropanoic acid (Fmoc-MeSer-OH) was synthesized by the method described in WO 2013/100132. To a solution of (S)-2-((((9H-fluoren-9-yl)methoxy)carbonyl)(methyl)amino)-3-hydroxypropanoic acid (Fmoc-MeSer-OH, 15 g, 43.9 mmol) in tetrahydrofuran (88 mL) were added pyridinium p-toluenesulfonate (PPTS, 0.552 g, 2.197 mmol) and 3,4-dihydro-2H-pyran (23.85 mL), and the mixture was stirred at 50°C for 4 h. The mixture was cooled to 25°C and ethyl acetate (90 mL) was added. The organic layer was then washed with a saturated aqueous sodium chloride solution (90 mL) and the aqueous layer was extracted with ethyl acetate (90 mL). All the resulting organic layers were combined and further washed with a saturated aqueous sodium chloride solution (90 mL) twice. The organic layers were dried over sodium sulfate and the solvent was evaporated under reduced pressure.

15.0 g of the resulting residue was dissolved in tetrahydrofuran (175 mL), followed by addition of 1.0 M phosphate buffer adjusted to pH 8.0 (175 mL). This mixture was stirred at 50°C for 3 h. After cooling to 25°C, ethyl acetate (175 mL) was added and the organic layer and the aqueous layer were separated. The aqueous layer was extracted by adding ethyl acetate (175 mL), and all the resulting organic layers were then combined and washed with a saturated aqueous sodium chloride solution (175 mL) twice. The organic layers were dried over sodium sulfate and the solvent was evaporated under reduced pressure.

The resulting residue was dissolved in dichloromethane (100 mL) and heptane (250 mL) was then added. Only the dichloromethane was evaporated under controlled reduced pressure (~100 hPa), and the resulting mixture was filtered to give a solid. This washing operation with heptane was repeated twice. The resulting solid was dried under reduced pressure using a pump at 25°C for 2 h.

To the resulting residue were added t-butyl methyl ether (TBME, 250 mL) and 0.05 M aqueous phosphoric acid, pH 2.1 (700 mL). After stirring at 25°C for 5 min, the organic layer and the aqueous layer were separated. The aqueous layer was extracted by adding t-butyl methyl ether (TBME, 250 mL), and all the resulting organic layers were then combined and washed with a saturated aqueous sodium chloride solution (250 mL) twice. The organic layers were dried over sodium sulfate and the solvent was evaporated under reduced pressure. The residue was dried under reduced pressure using a pump at 25°C for 2 h to afford (2S)-2-((((9H-fluoren-9-yl)methoxy)carbonyl)(methyl)amino)-3-((tetrahydro-2H-pyran-2-yl)oxy)propanoic acid (Compound aa06, Fmoc-MeSer(THP)-OH, 9.0 g, 30 mol% of t-butyl methyl ether (TBME) remaining). The obtained Fmoc-MeSer(THP)-OH was stored in a freezer at -25°C.
LCMS (ESI) m/z = 426.4 $(M+H)^+$
Retention time: 0.86 min (analytical condition SQDFA05)

Synthesis of N-(((9H-fluoren-9-yl)methoxy)carbonyl)-O-((5-fluoropyridin-3-yl)methyl)-L-serine (Compound aa10, Fmoc-Ser(3-F-5-Me-Pyr)-OH)

N-(((9H-Fluoren-9-yl)methoxy)carbonyl)-O-((5-fluoropyridin-3-yl)methyl)-L-serine (Compound aa10, Fmoc-Ser(3-F-5-Me-Pyr)-OH) was synthesized by the following route.

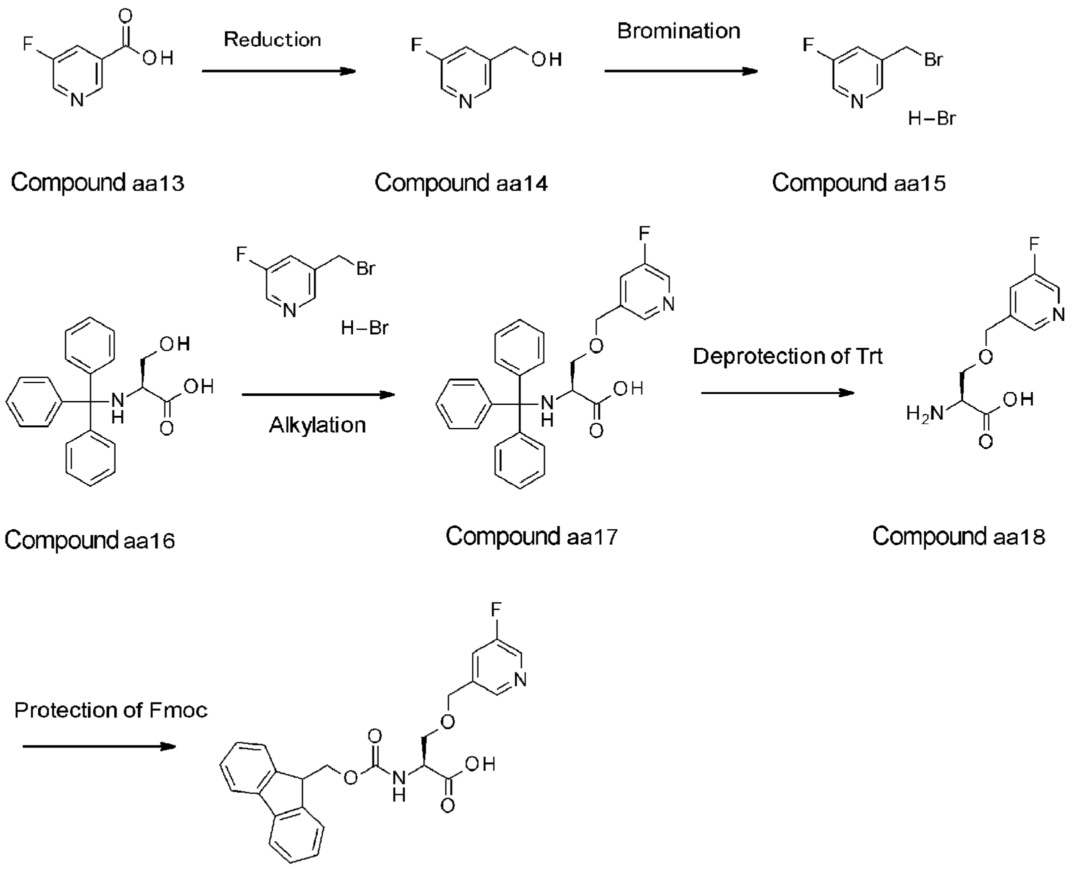


Compound aa13 Compound aa14 Compound aa15

Compound aa16 Compound aa17 Compound aa18

Compound aa10

<u>Synthesis of (5-fluoropyridin-3-yl)methanol (Compound aa14)</u>

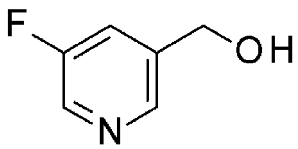

THF (200 mL) was added to commercially available 5-fluoronicotinic acid (Compound aa13) (14.1 g, 100 mmol) under a nitrogen atmosphere, and the mixture was stirred using a mechanical stirrer. Triethylamine (19.51 mL, 140 mmol) was then added at room temperature, and the mixture was stirred until the solid was completely dissolved, after which the reaction solution was cooled using an ice bath. Ethyl chloroformate (11.5 mL, 120 mmol) was then added dropwise and the mixture was stirred in an ice bath for 30 min. The reaction solution was then cooled to -60°C or lower using a dry ice bath, and a solution of $LiAlH_4$ (lithium aluminum hydride) in THF (2.5 M, 40 mL, 100 mmol) was added dropwise over 5 min or more so that the temperature of the reaction solution did not exceed -20°C. After stirring the reaction solution at - 78°C for 3 h, ethyl acetate (69 mL) was added so that the temperature of the reaction solution did not exceed -20°C. The reaction solution was then further stirred using an ice bath for 1 h, water (18 mL) was added, and the mixture was stirred at room temperature for 15 min. The reaction solution was filtered using an NH silica gel and concentrated under reduced pressure to afford (5-fluoropyridin-3-yl)methanol (Compound aa14) (9.28 g, 73%) as an yellow oily component.
LCMS (ESI) m/z = 128 $(M+H)^+$
Retention time: 0.28 min (analytical condition SQDFA05)

Synthesis of 3-(bromomethyl)-5-fluoropyridine hydrobromide (Compound aa15)

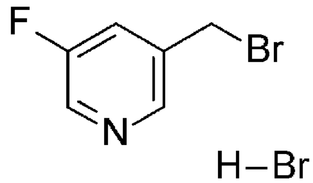

A condenser cooled with acetone/dry ice was connected to a round-bottom flask, and (5-fluoropyridin-3-yl)methanol (Compound aa14) (8.21 g, 64.6 mmol) and a 25% HBr-acetic acid solution (96 mL, 388 mmol) were added under a nitrogen atmosphere. The reaction was carried out in an open system, and a 1 M aqueous sodium hydroxide solution was used to trap the generated HBr. The reaction solution was gradually warmed from room temperature to 100°C with stirring and was further stirred for 3 h. The reaction solution was then cooled to room temperature, slow addition of diisopropyl ether (48 mL) was repeated three times, and the resulting solid was filtered to afford 3-(bromomethyl)-5-fluoropyridine hydrobromide (Compound aa15) (12.43 g, 71%) as a gray solid.
$^1H$ NMR (Varian 400-MR, 400 MHz, d-DMSO) $\delta$ 4.77 (2H, s), 7.85-7.88 (1H, m), 8.55-8.56 (2H, m)

Synthesis of O-((5-fluoropyridin-3-yl)methyl)-N-trityl-L-serine (Compound aa17, Trt-Ser(3-F-5-Me-Pyr)-OH)

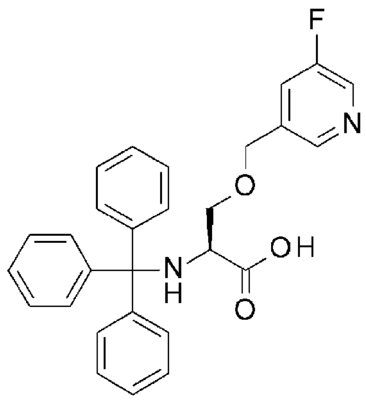

THF (45.8 mL) was added to sodium t-pentoxide (NaOtPen) (13.7 g, 125 mmol) under a nitrogen atmosphere. After stirring, commercially available tritylserine triethylamine salt (Trt-Ser-OH triethylamine salt) (11.24 g, 25 mmol) was added in three portions and the mixture was stirred at room temperature for 30 min. The reaction solution was stirred in an ice bath for 15 min and cooled, after which a solution of 3-(bromomethyl)-5-fluoropyridine hydrobromide (Compound aa15, 8.13 g, 30 mmol) in DMF (16 mL) was added dropwise and DMF (14 mL) was further added. After stirring the reaction solution in an ice bath for 45 min, a solution of 3-(bromomethyl)-5-fluoropyridine hydrobromide (Compound aa15, 2.03 g, 7.5 mmol) in DMF (4.0 mL) was added dropwise and DMF (4.0 mL) was further added. The reaction solution was then further stirred at room temperature for 1 h, and water (125 mL) was added. The resulting mixture was washed with t-butyl methyl ether (TBME), and the organic layer was extracted with water. The resulting aqueous layers were combined, adjusted to pH = 7 with a saturated aqueous sodium dihydrogenphosphate solution (15 mL), and then extracted with ethyl acetate twice. The resulting organic layers were combined, washed with 2-fold diluted brine three times, and then washed with brine twice. The resulting organic layers were dried over anhydrous sodium sulfate, filtered, and concentrated under reduced pressure to afford O-((5-fluoropyridin-3-yl)methyl)-N-trityl-L-serine (Compound aa17, Trt-Ser(3-F-5-Me-Pyr)-OH) (11.07 g, 97%) as a yellow amorphous.
LCMS (ESI) m/z = 455 $(M-H)^-$
Retention time: 0.86 min (analytical condition SQDFA05)

Synthesis of O-((5-fluoropyridin-3-yl)methyl)-L-serine (Compound aa18, H-Ser(3-F-5-Me-Pyr)-OH)

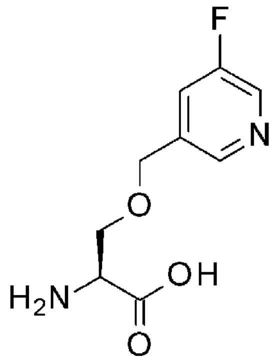

To a solution of O-((5-fluoropyridin-3-yl)methyl)-N-trityl-L-serine (Compound aa17, Trt-Ser(3-F-5-Me-Pyr)-OH) (10.76 g, 23.57 mmol) in 1,4-dioxane (21.5 mL) was added a 5-10% hydrochloric acid/methanol solution (64.2 mL) at room temperature. The reaction solution was stirred at room temperature for 10 min to 20 min, followed by addition of 1,4-dioxane (135 mL). After further adding additional 1,4-dioxane (90 mL), seed crystals previously prepared in a small amount as described below (5 mg) were added and the mixture was further stirred at room temperature for 30 min. The resulting solid was filtered, washed with diisopropyl ether (50 mL) four times, and dried under reduced pressure to afford O-((5-fluoropyridin-3-yl)methyl)-L-serine (Compound aa18, H-Ser(3-F-5-Me-Pyr)-OH) (6.58 g, 95%) as a hydrochloride.
LCMS (ESI) m/z = 213 $(M-H)^-$
Retention time: 0.24 min (analytical condition SQDAA05)

Preparation of seed crystals of O-((5-fluoropyridin-3-yl)methyl)-L-serine (Compound aa18, H-Ser(3-F-5-Me-Pyr)-OH)

To a solution of O-((5-fluoropyridin-3-yl)methyl)-N-trityl-L-serine (Compound aa17, Trt-Ser(3-F-5-Me-Pyr)-OH) (98 mg, 2.69 mmol) in 1,4-dioxane (819 μL) was added a 5-10% hydrochloric acid/methanol solution (2.45 mL) at room temperature, and the mixture was stirred for 5 h. To the reaction solution was added 1,4-dioxane (6.0 mL), and the resulting solid was filtered, washed with diisopropyl ether, and dried under reduced pressure to afford O-((5-fluoropyridin-3-yl)methyl)-L-serine (Compound aa18, H-Ser(3-F-5-Me-Pyr)-OH) (222.5 mg, 86%) as a hydrochloride.
LCMS (ESI) m/z = 213 $(M-H)^-$
Retention time: 0.24 min (analytical condition SQDAA05)

Synthesis of N-(((9H-fluoren-9-yl)methoxy)carbonyl)-O-((5-fluoropyridin-3-yl)methyl)-L-serine (Compound aa10, Fmoc-Ser(3-F-5-Me-Pyr)-OH)

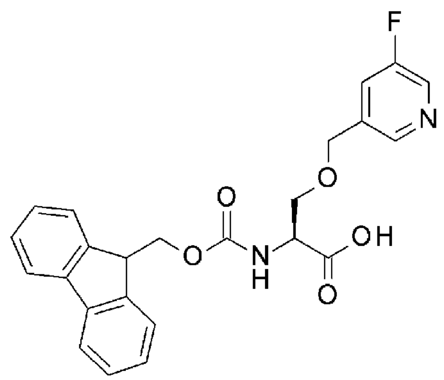

To O-((5-fluoropyridin-3-yl)methyl)-L-serine (Compound aa18, H-Ser(3-F-5-Me-Pyr)-OH) hydrochloride (6.37 g, 22.19 mmol) were added water (42 mL), 1,4-dioxane (115 mL), and diisopropylethylamine (DIPEA) (13.53 mL, 78 mmol) at room temperature, followed by stirring. To the reaction solution was then added 9-fluorenylmethyl N-succinimidyl carbonate (Fmoc-OSu) (7.86 g, 23.3 mmol) at room temperature, followed by stirring at room temperature. After the disappearance of the raw materials was confirmed by LC-MS, water (56.2 mL) was added to the reaction solution at room temperature and the mixture was washed with a 25% t-butyl methyl ether (MTBE)/hexane solution twice. The resulting aqueous layer was adjusted to pH = 6.1 with a saturated aqueous sodium dihydrogenphosphate solution

($NaH_2PO_4$). The aqueous layer was then extracted with ethyl acetate twice, and the resulting organic layers were combined and washed with 2-fold diluted brine and with brine. The resulting organic layers were dried over anhydrous sodium sulfate, filtered, and concentrated under reduced pressure to afford N-(((9H-fluoren-9-yl)methoxy)carbonyl)-O-((5-fluoropyridin-3-yl)methyl)-L-serine (Compound aa10, Fmoc-Ser(3-F-5-Me-Pyr)-OH) (9.47 g, 98%) as a yellow solid.
LCMS (ESI) m/z = 437 (M+H)+
Retention time: 0.86 min (analytical condition SQDAA05)

Synthesis of (S)-2-((((9H-fluoren-9-yl)methoxy)carbonyl)(methyl)amino)-4-phenylbutanoic acid (Compound aa11, Fmoc-MeHph-OH)

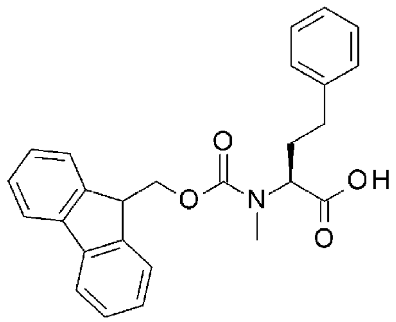

To a solution of commercially available (S)-2-((((9H-fluoren-9-yl)methoxy)carbonyl)amino)-4-phenylbutanoic acid (100 g, 244.11 mmol) in toluene (1.5 L) were added tosyl acid (TsOH) (2.575 g, 14.95 mmol) and paraformaldehyde (15.96 g, 488.77 mmol) at room temperature under a nitrogen atmosphere, and the mixture was stirred at 110°C for 16 h. After adding water to the organic layer and washing it with water three times, the resulting organic layers and the organic layer obtained by extracting the resulting aqueous layer with ethyl acetate were combined, dried over anhydrous sodium sulfate, filtered, and concentrated under reduced pressure to afford 100 g of (9H-fluoren-9-yl)methyl (S)-5-oxo-4-phenethyloxazolidine-3-carboxylate as a crude product.

To a solution of (9H-fluoren-9-yl) (S)-5-oxo-4-phenethyloxazolidine-3-carboxylate obtained as described above (50 g, 118.51 mmol) in dichloromethane (700 mL) were added triethylsilane ($Et_3SiH$) and trifluoroacetic acid (TFA) (700 mL), and the mixture was stirred at 25°C for 16 h. The reaction solution was concentrated under reduced pressure, an aqueous potassium carbonate solution was added to the resulting residue, and the mixture was washed with petroleum ether. The resulting aqueous layer was adjusted to pH = 3 with concentrated hydrochloric acid and extracted with ethyl acetate. The organic layer was dried over anhydrous sodium sulfate, filtered, and concentrated under reduced pressure. To the resulting residue were added methanol and hexane, and the mixture was again concentrated under reduced pressure to afford (S)-2-((((9H-fluoren-9-yl)methoxy)carbonyl)(methyl)amino)-4-phenylbutanoic acid (Compound aa11, Fmoc-MeHph-OH) (29.5 g, 58%, over two steps).
LCMS (ESI) m/z = 416 $(M+H)^+$
Retention time: 0.95 min (analytical condition SQDFA05)

Synthesis of (S)-2-((((9H-fluoren-9-yl)methoxy)carbonyl)amino)-3-(3-chlorophenoxy)propanoic acid (Compound aa12, Fmoc-Ser(Ph-3-Cl)-OH)

(S)-2-((((9H-Fluoren-9-yl)methoxy)carbonyl)amino)-3-(3-chlorophenoxy)propanoic acid (Compound aa12, Fmoc-Ser(Ph-3-Cl)-OH) was synthesized by the following route.

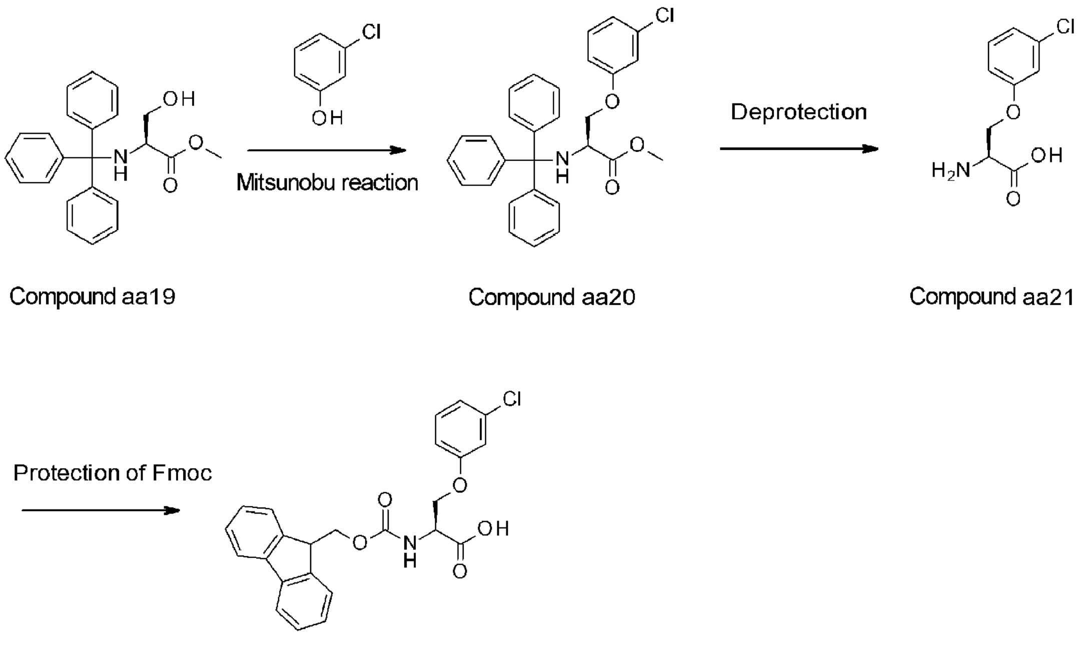


Compound aa19 Compound aa20 Compound aa21

Compound aa12

Synthesis of (S)-methyl 3-(3-chlorophenoxy)-2-(tritylamino)propanoate (Trt-Ser(Ph-3-Cl)-OMe) (Compound aa20)

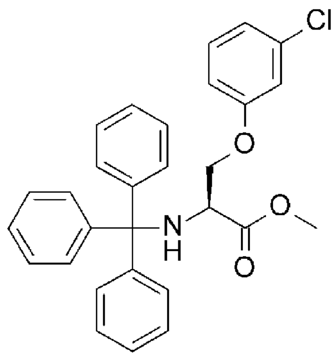

To a solution of commercially available (S)-methyl 3-hydroxy-2-(tritylamino)propanoate (Compound aa19, Trt-Ser-OMe) (6.0 g, 16.6 mmol) and triphenylphosphine ($PPh_3$) (8.71 g, 33.2 mmol) in toluene (35 mL) was added a 2.2 M solution of diethyl azocarboxylate (DEAD) in toluene (15.06 mL, 33.2 mmol) at room temperature under a nitrogen atmosphere, and the reaction solution was stirred at 50°C for 30 min. The reaction solution was concentrated under reduced pressure and the resulting residue was then purified by reverse phase silica gel column chromatography (10 mM aqueous ammonium acetate/methanol) to afford (S)-methyl 3-(3-chlorophenoxy)-2-(tritylamino)propanoate (Trt-Ser(Ph-3-Cl)-OMe)
(Compound aa20) (4.43 g, 57%).
$^1$H NMR (Varian 400-MR, 400 MHz, d-DMSO) $\delta$ 3.17 (3H, s), 3.49-3.51 (1H, m), 4.06-4.08 (1H, m), 4.19-4.22 (1H, m), 6.83-6.85 (1H, m), 6.99-7.01 (2H, m), 7.20-7.21 (3H, m), 7.28-7.30 (7H, m), 7.42-7.44 (6H, m)

Synthesis of (S)-2-amino-3-(3-chlorophenoxy)propanoic acid (Compound aa21, H-Ser(Ph-3-Cl)-OH)

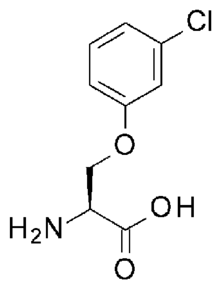

To a solution of (S)-methyl 3-(3-chlorophenoxy)-2-(tritylamino)propanoate (Trt-Ser(Ph-3-Cl)-OMe) (Compound aa20) (3.9 g, 8.26 mmol) in 1,4-dioxane (80 mL) was added a 1.0 M lithium hydroxide/methanol solution (83 mL) at room temperature, and the reaction solution was stirred at 50°C for 3 h. After concentrating the reaction solution under reduced pressure, trifluoroacetic acid (TFA) (30 mL) was added to the resulting residue and the mixture was stirred at 50°C for 10 min. The reaction solution was then concentrated under reduced pressure and the resulting residue was purified by reverse phase silica gel column chromatography (0.1% aqueous formic acid solution/0.1% formic acid-acetonitrile) to afford (S)-2-amino-3-(3-chlorophenoxy)propanoic acid (Compound aa21, H-Ser(Ph-3-Cl)-OH) (850 mg, 48%, over two steps).
LCMS (ESI) m/z = 216 $(M+H)^+$
Retention time: 0.37 min (analytical condition SQDFA05)

Synthesis of (S)-2-((((9H-fluoren-9-yl)methoxy)carbonyl)amino)-3-(3-chlorophenoxy)propanoic acid (Compound aa12, Fmoc-Ser(Ph-3-Cl)-OH)

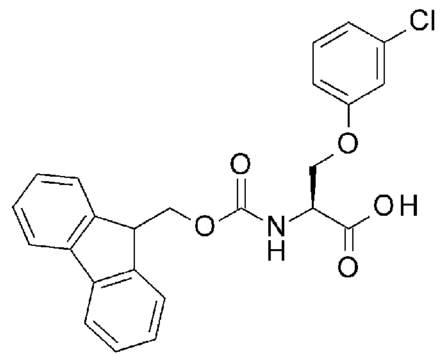

To (S)-2-amino-3-(3-chlorophenoxy)propanoic acid (Compound aa21, H-Ser(Ph-3-Cl)-OH) (850 mg, 3.94 mmol) and 9-fluorenylmethyl N-succinimidyl carbonate (Fmoc-OSu) (1.33 g, 3.94 mmol) were added 1,4-dioxane (20 mL) and water (20 mL) at room temperature, cesium carbonate (2.569 g, 7.88 mmol) was added, and the mixture was stirred at room temperature. After the disappearance of the raw materials was confirmed by LC-MS, water (20 mL) was added and the mixture was washed with diethyl ether (40 mL) twice. The resulting aqueous layer was adjusted to pH = 2 with a 5 N aqueous hydrochloric acid solution and extracted with ethyl acetate twice. The organic layers were dried over anhydrous sodium sulfate, filtered, and concentrated under reduced pressure. The resulting residue was purified by reverse phase silica gel column chromatography (0.1% aqueous formic acid solution/0.1% formic acid-acetonitrile) to afford (S)-2-((((9H-fluoren-9-yl)methoxy)carbonyl)amino)-3-(3-chlorophenoxy)propanoic acid (Compound aa12, Fmoc-Ser(Ph-3-Cl)-OH) (1.42 g, 82%).
LCMS (ESI) m/z = 438 $(M+H)^+$
Retention time: 0.91 min (analytical condition SQDFA05)
Fmoc-Ser(nPr)-OH (Compound aa51) was synthesized according to the following scheme.

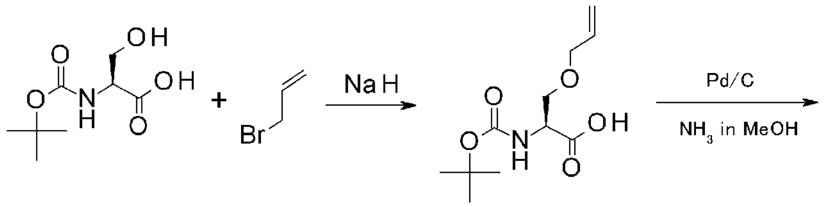


aa50

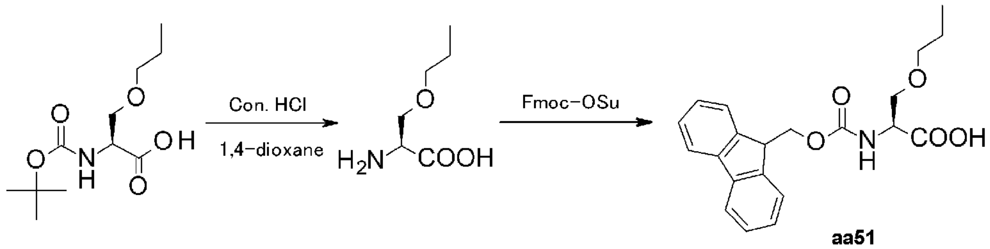


aa51

Synthesis of O-allyl-N-(tert-butoxycarbonyl)-L-serine (Compound aa50, Boc-Ser(Allyl)-OH)

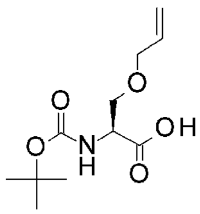

(tert-Butoxycarbonyl)-L-serine (Boc-Ser-OH) (50 g, 234.65 mmol) was dissolved in dehydrated dimethylformamide (DMF) (250 mL), and sodium hydride (22 g, 916.67 mmol, 60% oil dispersion) was added under ice-cooling (10°C or lower). After stirring under ice-cooling for 30 min, 3-bromoprop-1-ene (36.7 g, 303.37 mmol) was added dropwise under ice-cooling (10°C or lower). After the addition, the reaction solution was stirred at 25°C for 16 h, the reaction was then quenched by adding ice water (500 mL), and the mixture was extracted with ethyl acetate (500 mL x 3). The organic layers were washed with brine three times and then dried over anhydrous sodium sulfate. After filtration, the ethyl acetate was removed by concentration under reduced pressure to give a crude product containing the target compound. The same reaction was carried out three times, and the four batches in total of the crude product were combined and purified by column chromatography (petroleum ether/ethyl acetate) to afford O-allyl-N-(tert- butoxycarbonyl)-L-serine (Compound aa50, Boc-Ser(Allyl)-OH) (196.7 g, 82%).
LCMS (ESI) m/z = 268 (M+Na)+
Retention time: 0.90 min (analytical condition SMD method 17)

Synthesis of N-(((9H-fluoren-9-yl)methoxy)carbonyl)-O-propyl-L-serine (Compound aa51, Fmoc-Ser(nPr)-OH)

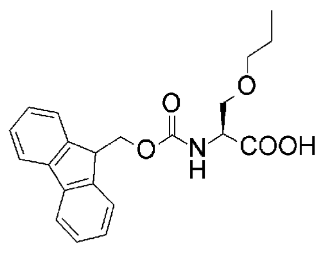

O-Allyl-N-(tert-butoxycarbonyl)-L-serine (Compound aa50, Boc-Ser(Allyl)-OH) (148 g, 603.41 mmol) was dissolved in methanol (1000 mL), a solution of ammonia in methanol (2 M, 453 mL) was added, and the mixture was stirred

at room temperature for 20 min. Pd/C (29.6 g) was then added and the mixture was stirred under a hydrogen atmosphere (5 atm) at 25°C for 16 h. The solid was then removed by filtration through celite and the solvent was removed by concentration under reduced pressure to afford N-(tert-butoxycarbonyl)-O-propyl-L-serine (Boc-Ser(nPr)-OH) (146.7 g, 98%).

The resulting N-(tert-butoxycarbonyl)-O-propyl-L-serine (Boc-Ser(nPr)-OH) (146.7 g) was dissolved in 1,4-dioxane (500 mL), concentrated hydrochloric acid (297 mL) was added dropwise, and the mixture was then stirred at room temperature for 16 h. The reaction solution was adjusted to pH 7-8 by adding an aqueous potassium carbonate solution, N-(9-fluorenylmethoxycarbonyloxy)succinimide (Fmoc-OSu) (240.17 g) and potassium carbonate (165.1 g, 1.19 mol) were added, and the mixture was stirred at room temperature for 16 h. The reaction solution was then washed with hexane three times, and the aqueous layer was adjusted to pH 1-2 by adding a aqueous hydrochloric acid solution (6 M) and extracted with ethyl acetate three times. The organic layers were dried over anhydrous sodium sulfate and filtered, after which the solvent was removed by concentration. The resulting crude product was washed with hexane and ether and purified by reverse phase column chromatography to afford N-(((9H-fluoren-9-yl)methoxy)carbonyl)-O-propyl-L-serine (Compound aa51, Fmoc-Ser(nPr)-OH) (103.2 g, 24%) as a white solid.
LCMS (ESI) m/z = 370 (M+H)+
Retention time: 2.11 min (analytical condition SMD method 18)

Compound aa52 was synthesized according to the following scheme.

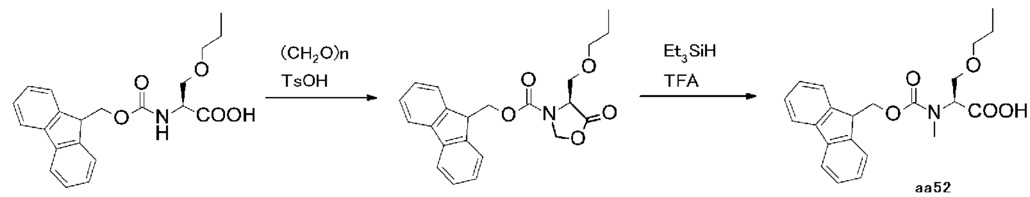


aa52

Synthesis of N-(((9H-fluoren-9-yl)methoxy)carbonyl)-N-methyl-O-propyl-L-serine (Compound aa52, Fmoc-Me-Ser(nPr)-OH)

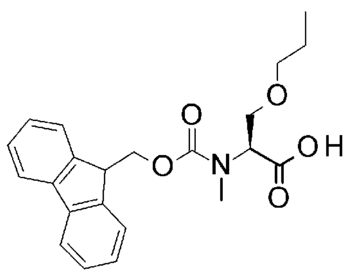

N-(((9H-Fluoren-9-yl)methoxy)carbonyl)-O-propyl-L-serine (Compound aa51, Fmoc-Ser(nPr)-OH) (67 g, 181.37 mmol), p-toluenesulfonic acid (TsOH) (1.86 g, 10.80 mmol), and paraformaldehyde (($CH_2O$)n) (10.86 g) were suspended in toluene (670 mL), and the suspension was stirred at 110°C for 16 h under a nitrogen atmosphere. The reaction solution was then diluted with ethyl acetate and washed with a saturated aqueous sodium bicarbonate solution twice. The organic layer was dried over anhydrous sodium sulfate and then filtered, and the solvent was removed by concentration under reduced pressure to afford (9H-fluoren-9-yl)methyl (S)-5-oxo-4-(propoxymethyl)oxazolidine-3-carboxylate as a crude product.

The resulting crude product (9H-fluoren-9-yl)methyl (S)-5-oxo-4-(propoxymethyl)oxazolidine-3-carboxylate (30 g, 78.65 mmol) was dissolved in dichloromethane (DCM) (480 mL), and triethylsilane ($Et_3SiH$) (28 g, 240.80 mmol) and trifluoroacetic acid (TFA) (480 mL) were added at room temperature. The reaction solution was stirred at room temperature for two days and then concentrated under reduced pressure. The resulting residue was dissolved in an aqueous potassium carbonate solution, and this was washed with hexane twice. The aqueous layer was then adjusted to pH 2-3 by adding concentrated hydrochloric acid and extracted with ethyl acetate twice. The organic layers were washed with brine three times, dried over anhydrous sodium sulfate, and then filtered. The solvent was removed by concentration under reduced pressure to afford N-(((9H-fluoren-9-yl)methoxy)carbonyl)-N-methyl-O-propyl-L-serine (Compound aa52, Fmoc-Me-Ser(nPr)-OH) (24 g, 80%).
LCMS (ESI) m/z = 384 (M+H)+

Retention time: 1.68 min (analytical condition SMD method 19)

Compound aa54 (Fmoc-Ser(iPen)-OH) was synthesized according to the following scheme.

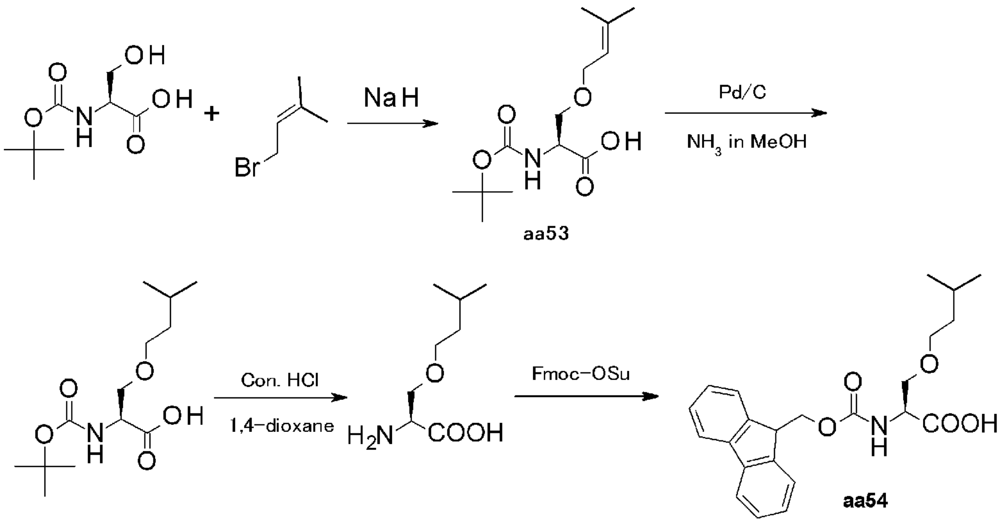


Synthesis of N-(tert-butoxycarbonyl)-O-(3-methylbut-2-en-1-yl)-L-serine (Compound aa53)

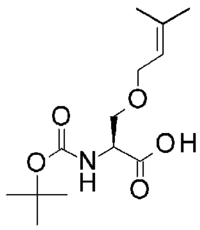

(tert-Butoxycarbonyl)-L-serine (Boc-Ser-OH) (50 g, 234.65 mmol) was dissolved in dimethylformamide (DMF) (250 mL), and sodium hydride (22 g, 916.67 mmol, 60% oil dispersion) was added under ice-cooling (0°C). After stirring under ice-cooling for 30 min, 1-bromo-3-methylbut-2-ene (44 g, 295.24 mmol) was added dropwise. After the addition, the reaction solution was stirred at 25°C for 16 h, the reaction was then quenched by adding ice water, and the pH was adjusted to 2-3 by adding an aqueous hydrochloric acid solution (5 M). The reaction solution was then extracted with ethyl acetate twice, and the organic layers were washed with brine and dried over anhydrous sodium sulfate. After filtration, the ethyl acetate was removed by concentration under reduced pressure to give a crude product containing the target compound. This crude product was purified by normal phase column chromatography (petroleum ether/ethyl acetate = 100/0 -> 80/20) to afford N-(tert-butoxycarbonyl)-O-(3-methylbut-2-en-1-yl)-L-serine (Compound aa53) (32 g, 48%).
LCMS (ESI) m/z = 296 (M+Na)+
Retention time: 1.39 min (analytical condition SMD method 7)

Synthesis of N-(((9H-fluoren-9-yl)methoxy)carbonyl)-O-isopentyl-L-serine (Compound aa54, Fmoc-Ser(iPen)-OH)

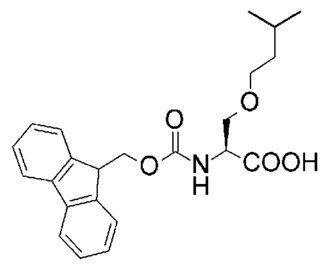

N-(tert-Butoxycarbonyl)-O-(3-methylbut-2-en-1-yl)-L-serine (Compound aa53) (185 g, 676.85 mmol), a solution of ammonia in methanol (2 M, 555 mL), Pd/C (18.5 g), and methanol (1500 mL) were mixed and stirred under a hydrogen atmosphere (5 atm) at room temperature for 16 h. The solid was removed by filtration and the solvent was then removed by concentration under reduced pressure to afford N-(tert-butoxycarbonyl)-O-isopentyl-L-serine (Boc-Ser(iPen)-OH) (183 g).

The obtained N-(tert-butoxycarbonyl)-O-isopentyl-L-serine (Boc-Ser(iPen)-OH) (175 g) was dissolved in 1,4-dioxane (500 mL), concentrated hydrochloric acid (300 mL) was added, and the mixture was then stirred at room temperature for 16 h. The reaction solution was concentrated under reduced pressure, the resulting crude product (122 g) was dissolved in 1,4-dioxane/water (1000 mL/1000 mL), potassium carbonate (239 g, 1.73 mol) and N-(9-fluorenylmethoxycarbonyloxy)succinimide (Fmoc-OSu) (234 g, 694.36 mmol) were added, and the mixture was stirred at room temperature for 16 h. The reaction solution was then washed with hexane three times. After the washing, the aqueous layer was adjusted to pH 2-3 by adding an aqueous hydrochloric acid solution (6 M) and extracted with ethyl acetate twice. The organic layers were dried over anhydrous sodium sulfate and filtered, after which the solvent was removed by concentration under reduced pressure. The resulting crude product was washed with ether, hexane, and ethyl acetate and further purified by reverse phase column chromatography (water/acetonitrile = 100/0 -> 30/70) to afford N-(((9H-fluoren-9-yl)methoxy)carbonyl)-O-isopentyl-L-serine (Compound aa54, Fmoc-Ser(iPen)-OH) (95 g, 41%) as a white solid.

LCMS (ESI) m/z = 398 (M+H)+

Retention time: 2.29 min (analytical condition SMD method 18)

Compound aa55 (Fmoc-MeSer(iPen)-OH) was synthesized according to the following scheme.

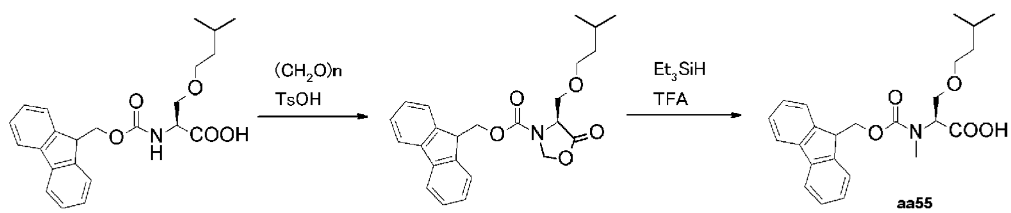


aa55

<u>Synthesis of N-(((9H-fluoren-9-yl)methoxy)carbonyl)-O-isopentyl-N-methyl-L-serine (Fmoc-MeSer(iPen)-OH, Compound aa55)</u>

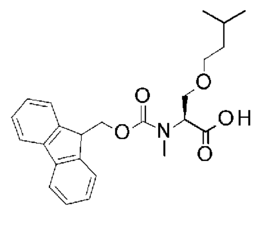

N-(((9H-fluoren-9-yl)methoxy)carbonyl)-O-isopentyl-L-serine (Compound aa54, Fmoc-Ser(iPen)-OH) (75 g, 188.70 mmol), p-toluenesulfonic acid (TsOH) (1.95 g, 11.32 mmol), and paraformaldehyde (11.4 g) were suspended in toluene (750 mL), and the suspension was stirred at 110°C for 16 h under a nitrogen atmosphere. The reaction solution was then washed with a saturated aqueous sodium bicarbonate solution twice. The organic layer was dried over anhydrous sodium sulfate and then filtered, and the solvent was removed by concentration under reduced pressure to afford (9H-fluoren-9-yl)methyl (S)-4-((isopentyloxy)methyl)-5-oxooxazolidine-3-carboxylate as a crude product.

The obtained (9H-fluoren-9-yl)methyl (S)-4-((isopentyloxy)methyl)-5-oxooxazolidine-3-carboxylate (40 g, 97.69 mmol) was dissolved in dichloromethane (670 mL), and triethylsilane ($Et_3SiH$) (34 g, 292.41 mmol) and trifluoroacetic acid (TFA) (670 mL) were added at room temperature. The reaction solution was stirred at room temperature for two days and then concentrated under reduced pressure. The resulting residue was dissolved in an aqueous potassium carbonate solution, and this was washed with petroleum ether three times. The aqueous layer was then adjusted to pH 2-3 by adding concentrated hydrochloric acid and extracted with ethyl acetate three times. The organic layers were washed with brine three times, dried over sodium sulfate, and then filtered. The solvent was removed by concentration under reduced pressure, the resulting residue was dissolved in methanol and washed with hexane three times, and the methanol was removed by concentration under reduced pressure to afford N-(((9H-fluoren-9-yl)methoxy)carbonyl)-O-isopentyl-N-methyl-L-serine (Fmoc-MeSer(iPen)-OH, Compound aa55) (35 g, 87%).
LCMS (ESI) m/z = 412 (M+H)+
Retention time: 0.97 min (analytical condition SQDFA05)

Synthesis of 1,1-difluoro-4-iodobutane (Compound aa56)

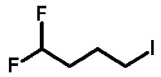

5-Bromo-1,1-difluoropentane (5 g, 26.73 mmol) was dissolved in acetone (20 ml) under a nitrogen atmosphere, and sodium iodide (6 g) was added at room temperature. The reaction solution was warmed to 45°C and stirred for 1 h. The reaction solution was filtered, the resulting filtrate was concentrated under reduced pressure, hexane was added, and the mixture was washed with an aqueous sodium thiosulfate solution and brine. The resulting organic layer was dried over anhydrous sodium sulfate, then filtered, and concentrated under reduced pressure. The resulting crude product was purified by normal phase silica gel chromatography (hexane) to afford 1,1-difluoro-4-iodobutane (Compound aa56) (4.2 g, 67%).
$^1H$ NMR (300 MHz, $CDCl_3$-d): $\delta$ 5.87 (t, J = 27.0 Hz, 1H), 3.26 (t, J = 6.0 Hz, 2H), 2.06-1.99 (m, 4H)

Synthesis of (S)-2-(((9H-fluoren-9-yl)methoxy)carbonylamino)-7,7-difluoroheptanoic acid (Compound aa58, Fmoc-Hnl(7-F2)-OH)

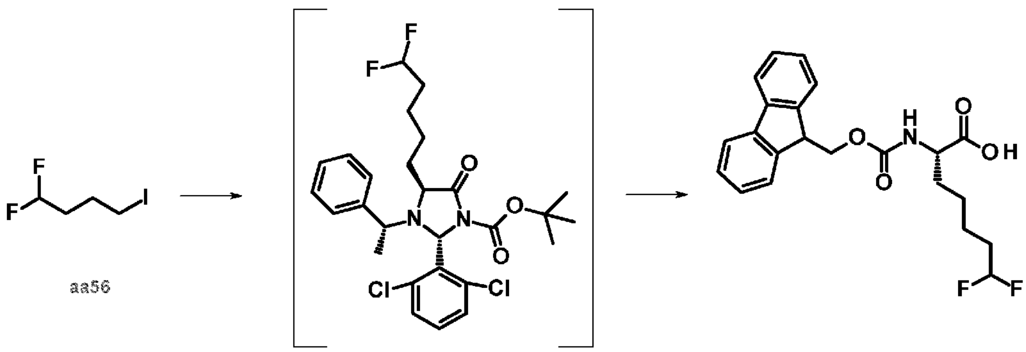

aa56

aa57

aa58

tert-Butyl (R)-2-(2,6-dichlorophenyl)-5-oxo-3-((R)-1-phenylethyl)imidazolidine-1-carboxylate (50 g, 114.85 mmol) was dissolved in tetrahydrofuran (300 ml) under a nitrogen atmosphere, and the solution was cooled to -20°C. Sodium bis(trimethylsilyl)amide (1.9 M tetrahydrofuran solution, 64 ml, 121.6 mmol) was added dropwise and the mixture was stirred for 10 min, after which 1,1-difluoro-4-iodobutane (aa56, 35 g, 149.56 mmol) diluted with 40 ml of tetrahydrofuran was added dropwise and the mixture was stirred at -20°C for 30 min. To the reaction solution was added a 20% aqueous ammonium chloride solution, and the mixture was then extracted with ethyl acetate twice. The organic layers were washed with a 20% aqueous ammonium acetate solution (twice) and 15% saline, after which the resulting organic layers were dried over anhydrous sodium sulfate, filtered, and concentrated under reduced pressure to afford a crude product tert-butyl (2R,4S)-2-(2,6-dichlorophenyl)-4-(5,5-difluoropentyl)-5-oxo-3-((R)-1-phenylethyl)imidazolidine-1-carboxylate

(Compound aa57, 74 g).

The obtained tert-butyl (2R,4S)-2-(2,6-dichlorophenyl)-4-(5,5-difluoropentyl)-5-oxo-3-((R)-1-phenylethyl)imidazolidine-1-carboxylate (Compound aa57, 74 g) was dissolved in chlorobenzene (200 ml). Trifluoromethanesulfonic acid (45 ml) was added dropwise over 30 min while maintaining the internal temperature of the reaction at 10°C or lower. The reaction solution was stirred at room temperature for 2 h, then 100 ml of water was added, and the mixture was warmed to 110°C and stirred for 4 h. The reaction solution was brought to room temperature and the aqueous layer was washed with 1:1 cyclopentyl methyl ether-hexane three times. The aqueous layer was then neutralized (pH = 7) with a 40% aqueous potassium phosphate solution. N,N-Diisopropylethylamine (25.7 ml), acetonitrile (50 ml), and N-(9-fluorenylmethoxycarbonyloxy)succinimide (Fmoc-OSu) (44.8 g) were added thereto under a nitrogen atmosphere, and the mixture was stirred at room temperature for 16 h. N,N-Diisopropylethylamine (19 ml) was added to maintain the reaction solution at pH 8 to 9. N,N-Diisopropylethylamine (9 ml) was further added to allow the reaction to proceed. The reaction solution was then filtered, hexane:ethyl acetate (2:1) (150 ml) was added to the resulting filtrate, and the mixture was vigorously stirred for 40 min. The intermediate layer was recovered from the resulting three layers of the reaction solution and washed with a 2:1 hexane-ethyl acetate solution (150 ml) nine times. Concentrated hydrochloric acid was added to the washed aqueous layer until it became pH 1, and the aqueous layer was then extracted with ethyl acetate twice. The resulting organic layers were washed with water twice and with 10% saline, and the resulting organic layers were then dried over anhydrous sodium sulfate, filtered, and concentrated under reduced pressure to afford (S)-2-(((9H-fluoren-9-yl)methoxy)carbonylamino)-7,7-difluoroheptanoic acid (Compound aa58, Fmoc-Hnl(7-F2)-OH) (33 g).
LCMS (ESI) m/z = 404 (M+H)+
Retention time: 3.29 min (analytical condition SMD method 27)

Synthesis of (S)-2-((((9H-fluoren-9-yl)methoxy)carbonyl)(methyl)amino)-7,7-difluoroheptanoic acid (Compound aa59, Fmoc-MeHnl(7-F2)-OH)

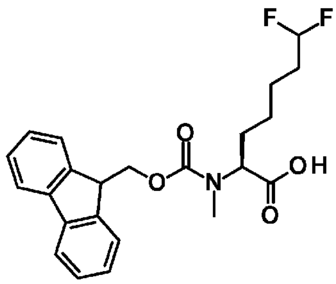

A solution of (S)-2-(((9H-fluoren-9-yl)methoxy)carbonylamino)-7,7-difluoroheptanoic acid (Compound aa58, Fmoc-Hnl(7-F2)-OH) (35 g, 86.76 mmol), paraformaldehyde (8.05 g, 268.33 mmol), and 10-camphorsulfonic acid (CSA) (1.01 g, 4.35 mmol) in toluene (525 mL) was warmed to 95°C under a nitrogen atmosphere, and the mixture was stirred for 2 h. The reaction solution was brought to room temperature and then washed with a saturated aqueous sodium bicarbonate solution three times. The resulting organic layers were dried over sodium sulfate, then filtered, and concentrated under reduced pressure to afford 9H-fluoren-9-ylmethyl (4S)-4-(5,5-difluoropentyl)-5-oxo-1,3-oxazolidine-3-carboxylate as a crude product (33 g, 79.44 mmol).

To a solution of the aforementioned crude product 9H-fluoren-9-ylmethyl (4S)-4-(5,5-difluoropentyl)-5-oxo-1,3-oxazolidine-3-carboxylate (33 g, 79.44 mmol) in dichloromethane (DCM) (330 mL) were added triethylsilane (25.4 mL, 12.42 mmol), water (1.43 ml), and boron trifluoride-diethyl ether complex ($BF_3 \cdot OEt_2$) (20.1 mL) at 0°C, and the mixture was stirred at room temperature for 16 h. A 5% aqueous ammonium chloride solution was added to the reaction solution, the organic layer was washed with brine, and the resulting organic layer was dried over anhydrous sodium sulfate, then filtered, and concentrated under reduced pressure. The resulting residue was purified by reverse phase column chromatography (0.1% aqueous formic acid solution/0.1% formic acid-acetonitrile solution) to afford (S)-2-((((9H-fluoren-9-yl)methoxy)carbonyl)(methyl)amino)-7,7-difluoroheptanoic acid (Compound aa59, Fmoc-MeHnl(7-F2)-OH) (27 g, 80% over two steps).
LCMS (ESI) m/z = 440 (M+Na)+
Retention time: 2.20 min (analytical condition SMD method 18)

N-(((9H-Fluoren-9-yl)methoxy)carbonyl)-O-(2-chlorophenyl)-L-serine (Compound aa60, Fmoc-Ser(Ph-2-Cl)-OH) was synthesized according to the following scheme.

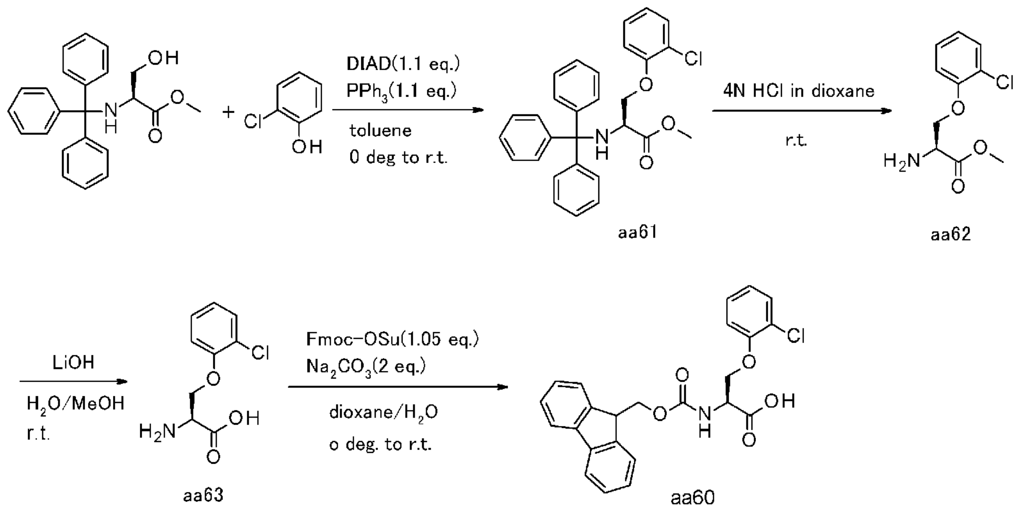


Synthesis of methyl O-(2-chlorophenyl)-N-trityl-L-serinate (Compound aa61, Trt-Ser(Ph-2-Cl)-OMe)

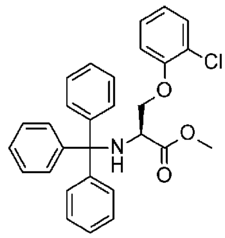

Methyl trityl-L-serinate (Trt-Ser-OMe) (25.0 g, 69.2 mmol), triphenylphosphine ($PPh_3$) (20.0 g, 76.1 mmol), and 2-chlorophenol (10.5 mL, 103.7 mmol) were mixed with toluene (57.2 mL), and diisopropyl azodicarboxylate (DIAD) (40% solution in toluene, 1.9 mol/l, 40.3 mL, 76.1 mmol) was added dropwise under ice-cooling over 40 min. After the dropwise addition was completed, the reaction solution was warmed to room temperature and further stirred for 90 min. The reaction solution was then concentrated under reduced pressure, and ethanol/water (8/2, 180 mL) was added to the resulting residue, followed by stirring at room temperature for 5 min. The resulting white solid was filtered, washed with ethanol/water (8/2, 100 mL) three times, and dried under reduced pressure to afford methyl O-(2-chlorophenyl)-N-trityl-L-serinate (Compound aa61, Trt-Ser(Ph-2-Cl)-OMe) (24.3 g, 75%).
LCMS (ESI) m/z = 494 (M+Na)+
Retention time: 1.16 min (analytical condition SQDFA05)

Synthesis of methyl O-(2-chlorophenyl)-L-serinate (Compound aa62)

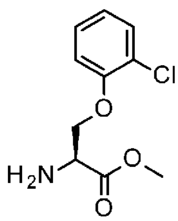

Methyl O-(2-chlorophenyl)-N-trityl-L-serinate (Compound aa61, Trt-Ser(Ph-2-Cl)-OMe) (24.3 g, 51.5 mmol) was dissolved in a 4 N hydrochloric acid/1,4-dioxane solution (38.7 mL, 154.5 mmol), and the mixture was stirred at room temperature for 15 min. The reaction solution was concentrated under reduced pressure, hexane (100 mL) was added to the resulting solid, and the mixture was stirred at room temperature for 10 min and then filtered. The solid was

further washed with hexane (100 mL) three times and dried under reduced pressure to afford methyl O-(2-chlorophenyl)-L-serinate (Compound aa62, H-Ser(Ph-2-Cl)-OMe) (13.5 g, 98%).
LCMS (ESI) m/z = 230 (M+H)+
Retention time: 0.40 min (analytical condition SQDFA05)

Synthesis of O-(2-chlorophenyl)-L-serine (Compound aa63, H-Ser(Ph-2-Cl)-OH)

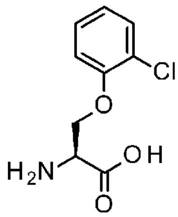

Methyl O-(2-chlorophenyl)-L-serinate (Compound aa62, H-Ser(Ph-2-Cl)-OMe) (13.5 g, 50.7 mmol) was dissolved in water (75 mL), and a solution of lithium hydroxide monohydrate (4.68 g, 111.5 mmol) in water/methanol (30 mL/30 mL) was added dropwise under ice-cooling over 20 min. After the dropwise addition was completed, the mixture was stirred under ice-cooling for 60 min. Acetonitrile (400 mL) cooled in a refrigerator was then added to the reaction solution, resulting in precipitation of a solid. The reaction solution was further stirred under ice-cooling for 20 min and then allowed to stand in a refrigerator for 5 h. After the solid was recovered by filtration, the solid precipitated in the mother liquor was also recovered. The recovered solids were combined and washed with acetonitrile (150 mL) three times and dried under reduced pressure to afford O-(2-chlorophenyl)-L-serine (Compound aa63, H-Ser(Ph-2-Cl)-OH) (Compound aa63, H-Ser(Ph-2-Cl)-OH) (10.3 g, 91%).
LCMS (ESI) m/z = 216 (M+H)+
Retention time: 0.34 min (analytical condition SQDFA05)

Synthesis of N-(((9H-fluoren-9-yl)methoxy)carbonyl)-O-(2-chlorophenyl)-L-serine (Compound aa60, Fmoc-Ser(Ph-2-Cl)-OH)

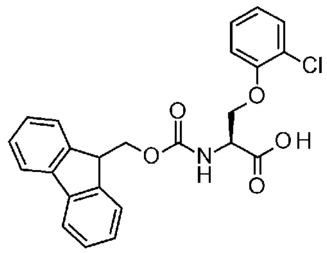

To O-(2-chlorophenyl)-L-serine (Compound aa63, H-Ser(Ph-2-Cl)-OH) (1.0 g, 4.49 mmol) were added a solution of sodium carbonate (0.952 g, 8.99 mmol) in water (3.8 mL), and 1,4-dioxane (2.0 mL), and N-(9-fluorenylmethoxycarbonyloxy)succinimide (Fmoc-OSu) (1.59 g, 4.72 mmol) was slowly added under ice-cooling. After the addition, the mixture was stirred at room temperature for 10 min and the solid was then dissolved by adding water (12 mL). After washing with t-butyl methyl ether (15 mL) three times, the aqueous layer was adjusted to pH 1 by slowly adding a 5 M aqueous hydrochloric acid solution (4.49 mL, 22.46 mmol). The aqueous layer was extracted with t-butyl methyl ether (15 mL) three times and the organic layers were dried over anhydrous magnesium sulfate and then filtered. The solvent was removed by concentration under reduced pressure to give a white solid.
The same operation using O-(2-chlorophenyl)-L-serine (Compound aa63, H-Ser(Ph-2-Cl)-OH) (9.28 g, 41.7 mmol) was also performed. The obtained solids were combined and dried under reduced pressure to afford N-(((9H-fluoren-9-yl)methoxy)carbonyl)-O-(2-chlorophenyl)-L-serine (Compound aa60, Fmoc-Ser(Ph-2-Cl)-OH) (19.6 g, 97%).
LCMS (ESI) m/z = 438 (M+H)+
Retention time: 0.90 min (analytical condition SQDFA05)

Synthesis of (9H-fluoren-9-yl)methyl (S)-4-((2-chlorophenoxy)methyl)-5-oxooxazolidine-3-carboxylate (Compound aa64)

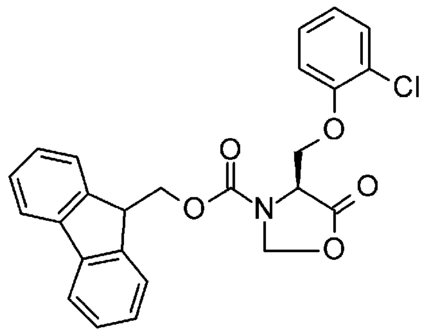

A suspension of N-(((9H-fluoren-9-yl)methoxy)carbonyl)-O-(2-chlorophenyl)-L-serine (Compound aa60, Fmoc-Ser(Ph-2-Cl)-OH) (3.00 g, 6.85 mmol), paraformaldehyde (0.823 g, 27.4 mmol), and (+)-10-camphorsulfonic acid (80.0 mg, 0.343 mmol) in toluene (23 mL) was stirred at 80°C for 3 h. The reaction solution was allowed to cool, then diluted with ethyl acetate (15 mL), and filtered through celite. A saturated aqueous sodium bicarbonate solution/water (1/1, 15 mL) was added to the filtrate, followed by extraction with ethyl acetate. The organic layer was washed with a saturated aqueous sodium bicarbonate solution/water (1/1, 15 mL) twice and washed with brine/water (1/1, 15 mL) once. The organic layer was dried over anhydrous sodium sulfate and the solvent was then evaporated under reduced pressure to afford (9H-fluoren-9-yl)methyl (S)-4-((2-chlorophenoxy)methyl)-5-oxooxazolidine-3-carboxylate (Compound aa64) (3.23 g) as a crude product.
LCMS (ESI) m/z = 450 (M+H)+
Retention time: 0.99 min (analytical condition SQDFA05)

Synthesis of N-(((9H-fluoren-9-yl)methoxy)carbonyl)-O-(2-chlorophenyl)-N-methyl-L-serine (Compound aa65, Fmoc-MeSer(Ph-2-Cl)-OH)

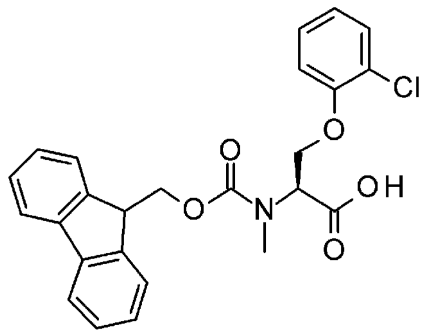

To a solution of (9H-fluoren-9-yl)methyl (S)-4-((2-chlorophenoxy)methyl)-5-oxooxazolidine-3-carboxylate (Compound aa64, 1.50 g, 3.33 mmol) in dichloroethane (DCE) (11 mL) were added triethylsilane (4.79 mL, 30.0 mmol) and trifluoroacetic acid (TFA) (6.94 mL, 90.0 mmol), and the mixture was stirred at 70°C for 2.5 h. The solvent was evaporated under reduced pressure, and the residue was dissolved in acetonitrile (25 mL) and washed with hexane (25 mL) four times. The solvent was evaporated from the acetonitrile layer under reduced pressure, and the residue was purified by reverse phase column chromatography to afford N-(((9H-fluoren-9-yl)methoxy)carbonyl)-O-(2-chlorophenyl)-N-methyl-L-serine (Compound aa65, Fmoc-MeSer(Ph-2-Cl)-OH) (1.1 g, 83%).
LCMS (ESI) m/z = 452 (M+H)+
Retention time: 0.93 min (analytical condition SQDFA05)

Synthesis of (9H-fluoren-9-yl)methyl (S)-4-(((5-fluoropyridin-3-yl)methoxy)methyl)-5-oxooxazolidine-3-carboxylate (Compound aa66)

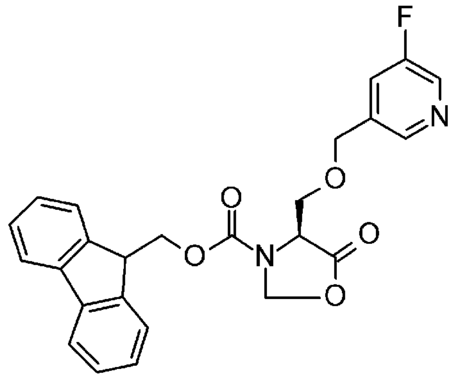

To a solution of N-(((9H-fluoren-9-yl)methoxy)carbonyl)-O-((5-fluoropyridin-3-yl)methyl)-L-serine (Fmoc-Ser(3-F-5-Me-Pyr)-OH) (Compound aa10, 3.00 g, 6.87 mmol) in toluene (13.7 mL) were added paraformaldehyde (0.619 g, 20.6 mmol) and trifluoroacetic acid (TFA) (4.77 mL, 61.9 mmol), and the mixture was stirred at 70°C for 3 h. The solvent was evaporated from the reaction solution under reduced pressure, and the residue was dissolved in dichloromethane (30 ml) and washed with a saturated aqueous sodium bicarbonate solution/water (1:1, 15 mL) three times. The organic layer was dried over sodium sulfate and the solvent was then evaporated under reduced pressure to afford (9H-fluoren-9-yl)methyl (S)-4-(((5-fluoropyridin-3-yl)methoxy)methyl)-5-oxooxazolidine-3-carboxylate (Compound aa66) (3.08 g) as a crude product.
LCMS (ESI) m/z = 449 (M+H)+
Retention time: 0.84 min (analytical condition SQDFA05)

Synthesis of N-(((9H-fluoren-9-yl)methoxy)carbonyl)-O-((5-fluoropyridin-3-yl)methyl)-N-methyl-L-serine (Compound aa67, Fmoc-MeSer(3-F-5-Me-Pyr)-OH)

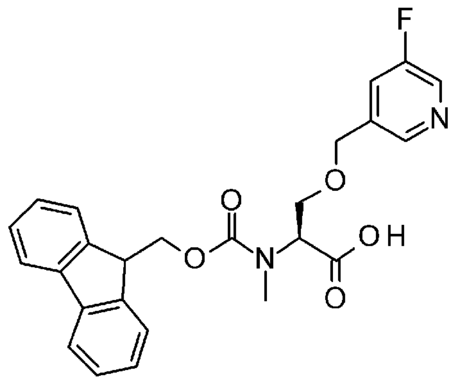

To a solution of (9H-fluoren-9-yl)methyl (S)-4-(((5-fluoropyridin-3-yl)methoxy)methyl)-5-oxooxazolidine-3-carboxylate (Compound aa66) (1.75 g, 3.90 mmol) in dichloroethane (DCE) (13 mL) were added triethylsilane (5.61 mL, 35.1 mmol) and trifluoroacetic acid (8.12 mL, 105 mmol), and the mixture was stirred at 70°C for 2 hours and 30 minutes. The solvent was evaporated under reduced pressure, and the residue was dissolved in acetonitrile (25 mL) and washed with hexane (25 mL). The solvent was evaporated from the acetonitrile layer under reduced pressure, and the resulting crude product was purified by reverse phase column chromatography to afford N-(((9H-fluoren-9-yl)methoxy)carbonyl)-O-((5-fluoropyridin-3-yl)methyl)-N-methyl-L-serine (Compound aa67, Fmoc-MeSer(3-F-5-Me-Pyr)-OH) (1.63 g, 93%).
LCMS (ESI) m/z = 451 (M+H)+

Retention time: 0.77 min (analytical condition SQDFA05)

Synthesis of methyl ((2-nitrophenyl)sulfonyl)-L-serinate (Compound aa68, Ns-Ser-OMe)

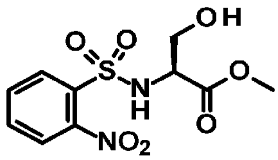

A solution obtained by dissolving sodium carbonate (7.76 g, 73.2 mmol) in water (75 mL) was added to commercially available methyl L-serinate hydrochloride (H-Ser-OMe·HCl) (5.0 g, 32.1 mmol) at room temperature, after which a solution of 2-nitrobenzenesulfonyl chloride (NsCl) (7.05 g, 31.8 mmol) in 1,4-dioxane (75 mL) was added and the mixture was stirred at room temperature for 1 hour and 30 minutes. The reaction solution was diluted with ethyl acetate and washed with a saturated aqueous sodium bicarbonate solution, a 1 N aqueous hydrochloric acid solution, and brine, after which the resulting organic layer was dried over anhydrous magnesium sulfate, then filtered, and concentrated under reduced pressure to afford methyl ((2-nitrophenyl)sulfonyl)-L-serinate (Compound aa68, Ns-Ser-OMe) (7.9 g, 82%).
LCMS (ESI) m/z = 305 (M+H)+
Retention time: 0.50 min (analytical condition SQDFA05)

Synthesis of methyl (S)-1-((2-nitrophenyl)sulfonyl)aziridine-2-carboxylate (Compound aa69, Ns-Azy-OMe)

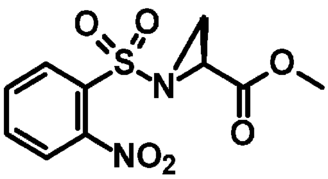

To methyl ((2-nitrophenyl)sulfonyl)-L-serinate (Compound aa68, Ns-Ser-OMe) (2.49 g, 8.18 mmol) was added dichloromethane (DCM) (69 mL) under a nitrogen atmosphere, and the solution was stirred at room temperature until it became transparent, after which triphenylphosphine ($PPh_3$) (2.545 g, 9.82 mmol) was added at -10°C using an ice bath prepared using sodium chloride and ice, and the solution was stirred at -10°C until it became transparent. A 40% diethyl azodicarboxylate (DEAD)-toluene solution (4.46 mL, purchased from Tokyo Chemical Industry) was added at -10°C and the reaction solution was stirred for 30 min, after which toluene (40 mL) was added, and the dichloromethane (DCM) was removed by concentration under reduced pressure using a rotary evaporator while maintaining the water bath at a temperature of 15°C or lower. The solid was removed by filtration, and the resulting filtrate was purified by normal phase silica gel chromatography (toluene/acetone) to afford methyl (S)-1-((2-nitrophenyl)sulfonyl)aziridine-2-carboxylate (Compound aa69, Ns-Azy-OMe) (1.9 g, 81%).
LCMS (ESI) m/z = 287 (M+H)+
Retention time: 0.64 min (analytical condition SQDFA05)

Synthesis of methyl N-((2-nitrophenyl)sulfonyl)-O-(2,2,3,3-tetrafluoropropyl)-L-serinate (Compound aa70, Ns-Ser(F(4)nPr)-OMe)

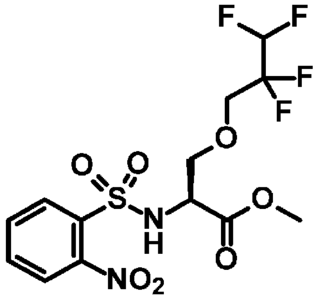

To a mixture of methyl (S)-1-((2-nitrophenyl)sulfonyl)aziridine-2-carboxylate (Compound aa69, Ns-Azy-OMe) (1.9 g, 6.64 mmol) and 2,2,3,3-tetrafluoropropan-1-ol (8.82 mL, 100 mmol) was added boron trifluoride-diethyl ether complex ($BF_3 \cdot OEt_2$) (2.52 mL, 19.91 mmol) at room temperature under a nitrogen atmosphere, and the mixture was stirred at 70°C for 40 min. The reaction solution was cooled to room temperature, then diluted with dimethyl sulfoxide (DMSO), and purified by reverse phase column chromatography (0.1% aqueous formic acid solution/0.1% formic acid-acetonitrile solution) to afford methyl N-((2-nitrophenyl)sulfonyl)-O-(2,2,3,3-tetrafluoropropyl)-L-serinate (Compound aa70, Ns-Ser(F(4)nPr)-OMe) (1.3 g, 47%).
LCMS (ESI) m/z = 419 (M+H)+
Retention time: 0.77 min (analytical condition SQDFA05)

Synthesis of N-((2-nitrophenyl)sulfonyl)-O-(2,2,3,3-tetrafluoropropyl)-L-serine (Compound aa71, Ns-Ser(F(4)nPr)-OH)

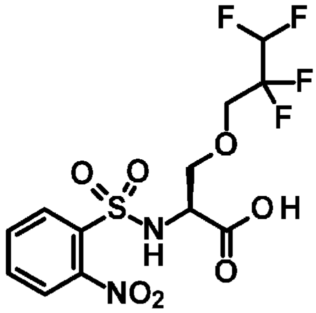

To methyl N-((2-nitrophenyl)sulfonyl)-O-(2,2,3,3-tetrafluoropropyl)-L-serinate (Compound aa70, Ns-Ser(F(4)nPr)-OMe) (142 mg, 0.339 mmol) were added methanol (MeOH) (0.75 mL), water (0.75 mL), and lithium hydroxide monohydrate (24.39 mg, 1.018 mmol), and the mixture was stirred at room temperature for 8 hours and 30 minutes. Formic acid (0.13 mL) was added to the reaction solution, and the mixture was diluted with water. The resulting solution was then purified by reverse phase column chromatography (0.1% aqueous formic acid solution/0.1% formic acid-acetonitrile solution) to afford N-((2-nitrophenyl)sulfonyl)-O-(2,2,3,3-tetrafluoropropyl)-L-serine (Compound aa71, Ns-Ser(F(4)nPr)-OH) (96 mg, 70%).
LCMS (ESI) m/z = 403 (M-H)-
Retention time: 0.68 min (analytical condition SQDFA05)

Synthesis of N-(((9H-fluoren-9-yl)methoxy)carbonyl)-O-(2,2,3,3-tetrafluoropropyl)-L-serine (Compound aa72, Fmoc-Ser(F(4)nPr)-OH)

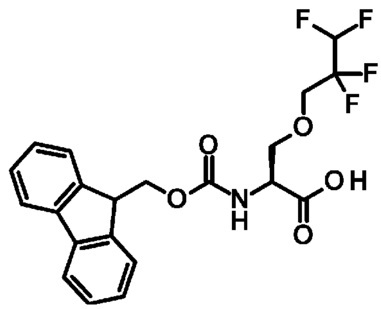

To a solution of N-((2-nitrophenyl)sulfonyl)-O-(2,2,3,3-tetrafluoropropyl)-L-serine (Compound aa71, Ns-Ser(F(4)nPr)-OH) (40.0 mg, 0.099 mmol) in acetonitrile (1.0 mL) were added potassium carbonate (68.4 mg, 0.495 mmol) and thiophenol (PhSH) (0.031 mL, 0.297 mmol) under a nitrogen atmosphere, and the mixture was stirred at room temperature for 4 h. Formic acid (0.038 mL) was added to the reaction solution, and the mixture was diluted with water and then washed with t-butyl methyl ether (TBME)/hexane = 1/4. The resulting aqueous layer was purified by reverse phase column chromatography (0.1% aqueous formic acid solution/0.1% formic acid-acetonitrile solution) to afford O-(2,2,3,3-tetrafluoropropyl)-L-serine (H-Ser(F(4)nPr)-OH) (16 mg, 74%).

The aforementioned O-(2,2,3,3-tetrafluoropropyl)-L-serine (H-Ser(F(4)nPr)-OH) (14 mg, 0.064 mmol) was dissolved in water (160 μL), after which N-ethyl-isopropylpropan-2-amine (DIPEA) (39.1 μL, 0.224 mmol), 1,4-dioxane (480 μL), and N-(9-fluorenylmethoxycarbonyloxy)succinimide (Fmoc-OSu) (22.63 mg, 0.067 mmol) were added and the mixture was stirred at room temperature for 2 h. The reaction solution was diluted with water, washed with t-butyl methyl ether (TBME)/hexane = 4/1, and extracted with a saturated aqueous sodium bicarbonate solution. To the resulting aqueous layer was added a 1 N aqueous hydrochloric acid solution until pH = 1.5. After extraction with ethyl acetate, the resulting organic layer was washed with brine, dried over anhydrous sodium sulfate, then filtered, and concentrated under reduced pressure to afford N-(((9H-fluoren-9-yl)methoxy)carbonyl)-O-(2,2,3,3-tetrafluoropropyl)-L-serine (Compound aa72, Fmoc-Ser(F(4)nPr)-OH) (24 mg, 85%).
LCMS (ESI) m/z = 440 (M-H)-
Retention time: 0.85 min (analytical condition SQDFA05)

<u>Synthesis of (S)-2-((((9H-fluoren-9-yl)methoxy)carbonyl)amino)-4-(2-chlorophenyl)butanoic acid (Compound aa73, Fmoc-Hph(2-Cl)-OH)</u>

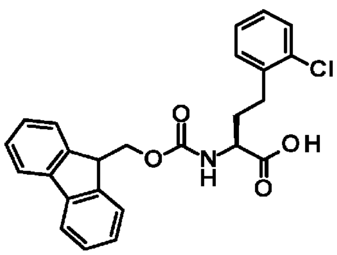

Commercially available (S)-2-amino-4-(2-chlorophenyl)butanoic acid (H-Hph(2-Cl)-OH) (1.0 g, 4.68 mmol) was dissolved in water (10 mL), after which sodium carbonate (1.488 g, 14.04 mmol), 1,4-dioxane (4 mL), and a solution of N-(9-fluorenylmethoxycarbonyloxy)succinimide (Fmoc-OSu) (1.579 g, 4.68 mmol) in 1,4-dioxane (11 mL) were added at room temperature and the mixture was stirred at room temperature overnight. After the disappearance of the raw materials was confirmed by LC-MS, the reaction solution was diluted with water at room temperature and washed with diethyl ether. The resulting aqueous layer was acidified with a 1 N aqueous hydrochloric acid solution and then extracted with ethyl acetate three times. The resulting organic layers were combined, washed with brine, dried over anhydrous sodium sulfate, then filtered, and concentrated under reduced pressure to afford (S)-2-((((9H-fluoren-9-yl)methoxy)carbonyl)amino)-4-(2-chlorophenyl)butanoic acid (Compound aa73, Fmoc-Hph(2-Cl)-OH) (2.0 g, 98%).
LCMS (ESI) m/z = 436 (M+H)+
Retention time: 0.93 min (analytical condition SQDFA05)

(S)-2-((((9H-Fluoren-9-yl)methoxy)carbonyl)(methyl)amino)-4-(2-chlorophenyl)butanoic acid (Compound aa75, Fmoc-MeHph(2-Cl)-OH) was synthesized according to the following scheme.

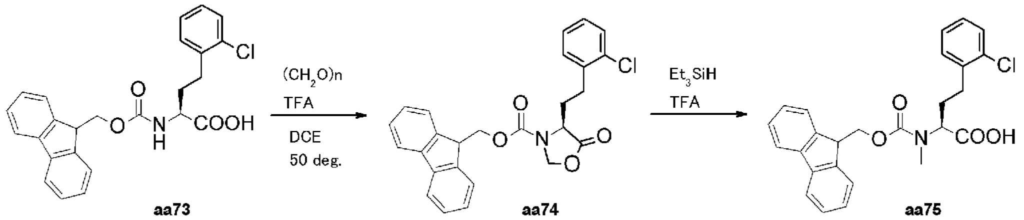


aa73 aa74 aa75

Synthesis of (9H-fluoren-9-yl)methyl (S)-4-(2-chlorophenethyl)-5-oxooxazolidine-3-carboxylate (Compound aa74)

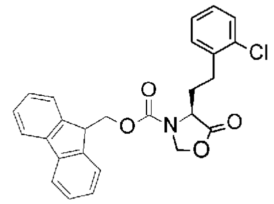

(S)-2-((((9H-Fluoren-9-yl)methoxy)carbonyl)amino)-4-(2-chlorophenyl)butanoic acid (Compound aa73, Fmoc-Hph(2-Cl)-OH) (400 mg, 0.918 mmol) and paraformaldehyde (110 mg, 3.67 mmol) were dissolved in dichloroethane (DCE) (3.06 mL) and trifluoroacetic acid (TFA) (636 μL, 8.26 mmol), and the mixture was stirred at 50°C for 2 h. The reaction solution was diluted with dichloroethane (DCE), and the solvent was then removed by concentration under reduced pressure. The resulting residue was dissolved in dichloromethane and washed with a saturated aqueous sodium bicarbonate solution and brine. The organic layer was dried over anhydrous sodium sulfate and filtered, and the dichloromethane was then removed by concentration under reduced pressure to afford (9H-fluoren-9-yl)methyl (S)-4-(2-chlorophenethyl)-5-oxooxazolidine-3-carboxylate (Compound aa74) (378.4 mg, 92%).
LCMS (ESI) m/z = 448 (M+H)+
Retention time: 1.05 min (analytical condition SQDFA05)

Synthesis of (S)-2-((((9H-fluoren-9-yl)methoxy)carbonyl)(methyl)amino)-4-(2-chlorophenyl)butanoic acid (Compound aa75, Fmoc-MeHph(2-Cl)-OH)

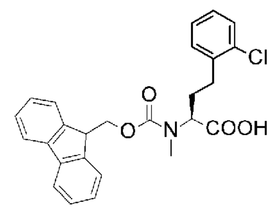

(9H-Fluoren-9-yl)methyl (S)-4-(2-chlorophenethyl)-5-oxooxazolidine-3-carboxylate (Compound aa74) (411 mg, 0.918 mmol) and triethylsilane ($Et_3SiH$) (1.46 mL, 9.18 mmol) were dissolved in dichloromethane (4.59 mL) and trifluoroacetic acid (TFA) (2.12 mL), and the mixture was stirred at room temperature for 5 h. The reaction solution was diluted with dichloromethane, and the solvent was then removed by concentration under reduced pressure. The resulting residue was purified by reverse phase column chromatography (0.1% aqueous formic acid solution/0.1% formic acid-acetonitrile solution) to afford (S)-2-((((9H-fluoren-9-yl)methoxy)carbonyl)(methyl)amino)-4-(2-chlorophenyl)butanoic acid (Compound aa75, Fmoc-MeHph(2-Cl)-OH) (164.6 mg, 40%).
LCMS (ESI) m/z = 450 (M+H)+
Retention time: 0.99 min (analytical condition SQDFA05)

Synthesis of (S)-2-((((9H-fluoren-9-yl)methoxy)carbonyl)amino)-4-(3-chlorophenyl)butanoic acid (Compound aa76, Fmoc-Hph(3-Cl)-OH)

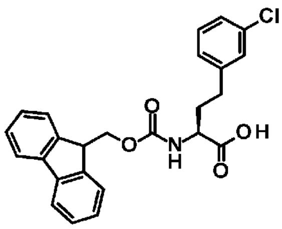

Commercially available (S)-2-amino-4-(3-chlorophenyl)butanoic acid (H-Hph(3-Cl)-OH) (1.0 g, 4.68 mmol) was dissolved in water (10 mL), after which sodium carbonate (1.488 g, 14.04 mmol), 1,4-dioxane (4 mL), and a solution of N-(9-fluorenylmethoxycarbonyloxy)succinimide (Fmoc-OSu) (1.579 g, 4.68 mmol) in 1,4-dioxane (11 mL) were added at room temperature and the mixture was stirred at room temperature overnight. After the disappearance of the raw materials was confirmed by LC-MS, the reaction solution was diluted with water at room temperature and washed with diethyl ether. The resulting aqueous layer was acidified with a 1 N aqueous hydrochloric acid solution and then extracted with ethyl acetate three times. The resulting organic layers were combined, washed with brine, dried over anhydrous sodium sulfate, then filtered, and concentrated under reduced pressure to afford (S)-2-((((9H-fluoren-9-yl)methoxy)carbonyl)amino)-4-(3-chlorophenyl)butanoic acid (Compound aa76, Fmoc-Hph(3-Cl)-OH) (2.0 g, 98%).
LCMS (ESI) m/z = 436 (M+H)+
Retention time: 0.94 min (analytical condition SQDFA05)

(S)-2-((((9H-Fluoren-9-yl)methoxy)carbonyl)(methyl)amino)-4-(3-chlorophenyl)butanoic acid (Compound aa78, Fmoc-MeHph(3-Cl)-OH) was synthesized according to the following scheme.

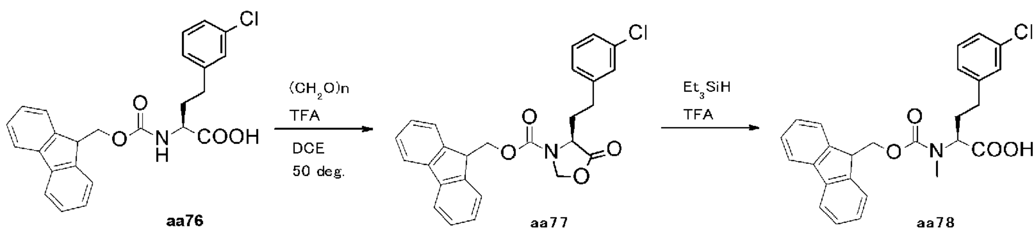


Synthesis of (9H-fluoren-9-yl)methyl (S)-4-(3-chlorophenethyl)-5-oxooxazolidine-3-carboxylate (Compound aa77)

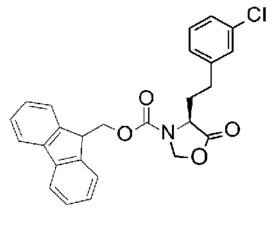

(S)-2-((((9H-Fluoren-9-yl)methoxy)carbonyl)amino)-4-(3-chlorophenyl)butanoic acid (Compound aa76, Fmoc-Hph(3-Cl)-OH) (400 mg, 0.918 mmol) and paraformaldehyde (110 mg, 3.67 mmol) were dissolved in dichloroethane (DCE) (3.06 mL) and trifluoroacetic acid (TFA) (636 μL, 8.26 mmol), and the mixture was stirred at 50°C for 2 h. The reaction solution was diluted with dichloroethane (DCE), and the solvent was then removed by concentration under reduced pressure. The resulting residue was dissolved in dichloromethane and washed with a saturated aqueous sodium bicarbonate solution and brine. The organic layer was dried over anhydrous sodium sulfate and filtered, and the dichloromethane was then removed under reduced pressure to afford (9H-fluoren-9-yl)methyl (S)-4-(3-chlorophenethyl)-5-oxooxazolidine-3-carboxylate (Compound aa77, 426.3 mg) quantitatively.
LCMS (ESI) m/z = 448 (M+H)+

Retention time: 1.06 min (analytical condition SQDFA05)

Synthesis of (S)-2-((((9H-fluoren-9-yl)methoxy)carbonyl)(methyl)amino)-4-(3-chlorophenyl)butanoic acid (Compound aa78, Fmoc-MeHph(3-Cl)-OH)

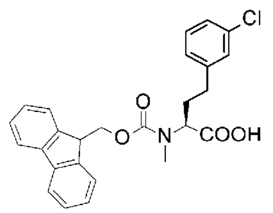

(9H-Fluoren-9-yl)methyl (S)-4-(3-chlorophenethyl)-5-oxooxazolidine-3-carboxylate (Compound aa77) (411 mg, 0.918 mmol) and triethylsilane ($Et_3SiH$) (1.46 mL, 9.18 mmol) were dissolved in dichloromethane (4.59 mL) and trifluoroacetic acid (TFA) (2.12 mL), and the mixture was stirred at room temperature for 6 h. The reaction solution was diluted with dichloromethane, and the solvent was then removed by concentration under reduced pressure. The resulting residue was purified by reverse phase column chromatography (0.1% aqueous formic acid solution/0.1% formic acid-acetonitrile solution) to afford (S)-2-((((9H-fluoren-9-yl)methoxy)carbonyl)(methyl)amino)-4-(3-chlorophenyl)butanoic acid (Compound aa78, Fmoc-MeHph(3-Cl)-OH) (144.3 mg, 35%).
LCMS (ESI) m/z = 450 (M+H)+
Retention time: 0.99 min (analytical condition SQDFA05)

Synthesis of (S)-2-((((9H-fluoren-9-yl)methoxy)carbonyl)amino)-4-(4-chlorophenyl)butanoic acid (Compound aa79, Fmoc-Hph(4-Cl)-OH)

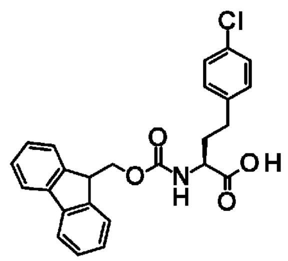

Commercially available (S)-2-amino-4-(4-chlorophenyl)butanoic acid (H-Hph(4-Cl)-OH) (1.0 g, 4.68 mmol) was dissolved in water (10 mL), after which sodium carbonate (1.488 g, 14.04 mmol), 1,4-dioxane (4 mL), and a solution of N-(9-fluorenylmethoxycarbonyloxy)succinimide (Fmoc-OSu) (1.579 g, 4.68 mmol) in 1,4-dioxane (11 mL) were added at room temperature and the mixture was stirred at room temperature overnight. After the disappearance of the raw materials was confirmed by LC-MS, the reaction solution was diluted with water at room temperature and washed with diethyl ether. The resulting aqueous layer was acidified with a 1 N aqueous hydrochloric acid solution and then extracted with ethyl acetate three times. The resulting organic layers were combined, washed with brine, dried over anhydrous sodium sulfate, filtered, and concentrated under reduced pressure, and the resulting mixture was then purified by reverse phase column chromatography (0.1% aqueous formic acid solution/0.1% formic acid-acetonitrile solution) to afford (S)-2-((((9H-fluoren-9-yl)methoxy)carbonyl)amino)-4-(4-chlorophenyl)butanoic acid (Compound aa79, Fmoc-Hph(4-Cl)-OH) (1.2 g, 59%).
LCMS (ESI) m/z = 434 (M-H)-
Retention time: 0.95 min (analytical condition SQDFA05)
(S)-2-((((9H-fluoren-9-yl)methoxy)carbonyl)(methyl)amino)-4-(4-chlorophenyl)butanoic acid (Compound aa81, Fmoc-MeHph(4-Cl)-OH) was synthesized according to the following scheme.

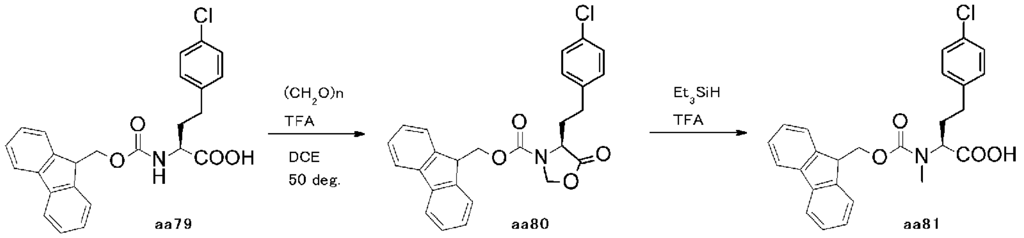


aa79 aa80 aa81

Synthesis of (9H-fluoren-9-yl)methyl (S)-4-(4-chlorophenethyl)-5-oxooxazolidine-3-carboxylate (Compound aa80)

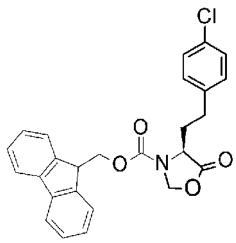

(S)-2-((((9H-fluoren-9-yl)methoxy)carbonyl)amino)-4-(3-chlorophenyl)butanoic acid (Compound aa79, Fmoc-Hph(4-Cl)-OH) (250 mg, 0.574 mmol) and paraformaldehyde (68.9 mg, 2.29 mmol) were dissolved in dichloroethane (DCE) (1.91 mL) and trifluoroacetic acid (TFA) (398 μL, 5.16 mmol), and the mixture was stirred at 50°C for 2 h. The reaction solution was diluted with dichloroethane (DCE), and the solvent was then removed by concentration under reduced pressure. The resulting residue was dissolved in dichloromethane and washed with a saturated aqueous sodium bicarbonate solution and brine. The organic layer was dried over anhydrous sodium sulfate and filtered, and the dichloromethane was then removed to afford (9H-fluoren-9-yl)methyl (S)-4-(4-chlorophenethyl)-5-oxooxazolidine-3-carboxylate (Compound aa80) (297.5 mg) quantitatively.
LCMS (ESI) m/z = 448 (M+H)+
Retention time: 1.06 min (analytical condition SQDFA05)

Synthesis of (S)-2-((((9H-fluoren-9-yl)methoxy)carbonyl)(methyl)amino)-4-(4-chlorophenyl)butanoic acid (Compound aa81, Fmoc-MeHph(4-Cl)-OH)

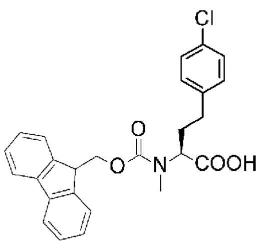

(9H-Fluoren-9-yl)methyl (S)-4-(4-chlorophenethyl)-5-oxooxazolidine-3-carboxylate (Compound aa80) (257 mg, 0.574 mmol) and triethylsilane ($Et_3SiH$) (914 μL, 5.74 mmol) were dissolved in dichloromethane (2.87 mL) and trifluoroacetic acid (TFA) (1.33 mL), and the mixture was stirred at room temperature for 8 h. The reaction solution was diluted with dichloromethane, and the solvent was then removed by concentration under reduced pressure. The resulting residue was purified by reverse phase column chromatography (0.1% aqueous formic acid solution/0.1% formic acid-acetonitrile solution) to afford (S)-2-((((9H-fluoren-9-yl)methoxy)carbonyl)(methyl)amino)-4-(4-chlorophenyl)butanoic acid (Compound aa81, Fmoc-MeHph(4-Cl)-OH) (180.5 mg, 70%).
LCMS (ESI) m/z = 450 (M+H)+
Retention time: 0.99 min (analytical condition SQDFA05)

Synthesis of (S)-2-((((9H-fluoren-9-yl)methoxy)carbonyl)(methyl)amino)-5-phenylpentanoic acid (Compound aa82, Fmoc-MePhe{#(CH2)2}-OH)

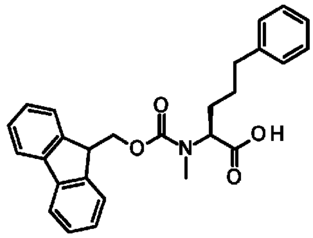

To a solution of commercially available (S)-2-((((9H-fluoren-9-yl)methoxy)carbonyl)amino)-5-phenylpentanoic acid (Fmoc-Phe{#(CH2)2}-OH, purchased from Watanabe Chemical Industries) (500 mg, 1.203 mmol) and paraformaldehyde (108 mg, 3.61 mmol) in toluene (3.0 mL) was added trifluoroacetic acid (0.834 mL, 10.83 mmol) at room temperature under a nitrogen atmosphere, and the mixture was stirred for 4 h. The reaction solution was concentrated, diluted with dichloromethane, and then washed with a saturated aqueous sodium bicarbonate solution. The organic layer was dried over anhydrous magnesium sulfate, then filtered, and concentrated under reduced pressure to afford (9H-fluoren-9-yl)methyl (S)-5-oxo-4-(3-phenylpropyl)oxazolidine-3-carboxylate (590 mg) as a crude product.

To a solution of the above crude product (9H-fluoren-9-yl)methyl (S)-5-oxo-4-(3-phenylpropyl)oxazolidine-3-carboxylate (590 mg) in dichloroethane (DCE) (7.0 mL) were added triethylsilane (1.984 mL, 12.42 mmol) and trifluoroacetic acid (2.87 mL, 37.3 mmol) at room temperature, and the reaction solution was stirred at 60°C for 1 h. The reaction solution was cooled at room temperature and then concentrated under reduced pressure. The resulting residue was purified by reverse phase column chromatography (0.1% aqueous formic acid solution/0.1% formic acid-acetonitrile solution) to afford (S)-2-((((9H-fluoren-9-yl)methoxy)carbonyl)(methyl)amino)-5-phenylpentanoic acid (Compound aa82, Fmoc-MePhe{#(CH2)2}-OH) (488 mg, 82% over two steps).
LCMS (ESI) m/z = 430 (M+H)+
Retention time: 0.97 min (analytical condition SQDFA05)

Synthesis of N-(((9H-fluoren-9-yl)methoxy)carbonyl)-O-benzyl-N-methyl-L-serine (Compound aa83, Fmoc-MeSer(Bn)-OH)

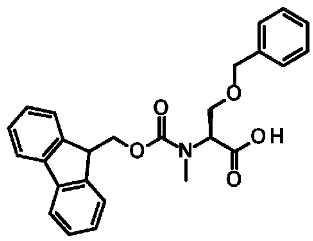

To a solution of commercially available N-(((9H-fluoren-9-yl)methoxy)carbonyl)-O-benzyl-L-serine (Fmoc-Ser(Bn)-OH, purchased from Watanabe Chemical Industries) (500 mg, 1.198 mmol) and paraformaldehyde (108 mg, 3.59 mmol) in toluene (3.0 mL) was added trifluoroacetic acid (0.834 mL, 10.78 mmol) at room temperature under a nitrogen atmosphere, and the mixture was stirred for 3 h. The reaction solution was concentrated, diluted with dichloromethane (DCM), and then washed with a saturated aqueous sodium bicarbonate solution. The organic layer was dried over anhydrous magnesium sulfate, then filtered, and concentrated under reduced pressure to afford (9H-fluoren-9-yl)methyl (S)-4-((benzyloxy)methyl)-5-oxooxazolidine-3-carboxylate (544 mg) as a crude product.

To a solution of the above crude product (9H-fluoren-9-yl)methyl (S)-4-((benzyloxy)methyl)-5-oxooxazolidine-3-carboxylate (544 mg) in dichloroethane (DCE) (7.0 mL) were added triethylsilane (1.821 mL, 11.40 mmol) and trifluoroacetic acid (2.63 mL, 34.2 mmol) at room temperature, and the reaction solution was stirred at 60°C for 6 h. The reaction solution was cooled at room temperature and then concentrated under reduced pressure. The resulting residue was purified by reverse phase column chromatography (0.1% aqueous formic acid solution/0.1% formic acid-acetonitrile solution) to afford N-(((9H-fluoren-9-yl)methoxy)carbonyl)-O-benzyl-N-methyl-L-serine (Compound aa83, Fmoc-Me-

Ser(Bn)-OH) (425 mg, 78% over two steps).
LCMS (ESI) m/z = 432 (M+H)+
Retention time: 0.91 min (analytical condition SQDFA05)

Synthesis of (2S,4R)-1-(((9H-fluoren-9-yl)methoxy)carbonyl)-4-ethoxypyrrolidine-2-carboxylic acid (Compound aa84, Fmoc-Hyp(Et)-OH)

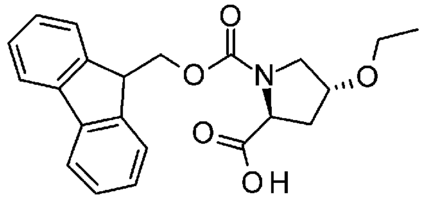

A solution of commercially available (2S,4R)-1-(tert-butoxycarbonyl)-4-ethoxypyrrolidine-2-carboxylic acid (Boc-Hyp(Et)-OH) (CAS No. 146060-18-6, 65 g, 251 mmol) in dichloromethane (DCM) (400 mL) was stirred at room temperature while bubbling with hydrogen chloride gas. After six hours, the solvent was evaporated from the reaction solution under reduced pressure and the residue was washed with diethyl ether to afford (2S,4R)-4-ethoxypyrrolidine-2-carboxylic acid hydrochloride (H-Hyp(Et)-OH·HCl) (45 g) as a crude product.
The above crude product (2S,4R)-4-ethoxypyrrolidine-2-carboxylic acid hydrochloride (H-Hyp(Et)-OH·HCl) was dissolved in a mixture of water (500 mL) and 1,4-dioxane (500 mL), potassium carbonate (79.4 g, 574 mmol) and N-(9-fluorenylmethoxycarbonyloxy)succinimide (Fmoc-OSu) (69.8 g, 207 mmol) were added, and the mixture was stirred at room temperature for 2 h.
The reaction solution was washed with diethyl ether, and the aqueous layer was adjusted to pH 3 with a 5% potassium aqueous bisulfate solution and then extracted with ethyl acetate. The resulting organic layer was washed with brine and dried over anhydrous sodium sulfate, and the solvent was evaporated under reduced pressure to afford (2S,4R)-1-(((9H-fluoren-9-yl)methoxy)carbonyl)-4-ethoxypyrrolidine-2-carboxylic acid (Compound aa84, Fmoc-Hyp(Et)-OH) (90.7 g, 95%).
LCMS (ESI) m/z = 382 (M+H)+
Retention time: 1.97 min (analytical condition SMD method 26)

Synthesis of (S)-2-((((9H-fluoren-9-yl)methoxy)carbonyl)amino)-3-(6-(dimethylamino)pyridin-3-yl)propanoic acid (Compound aa88, Fmoc-Ala(3-Pyr-4-NMe2)-OH)

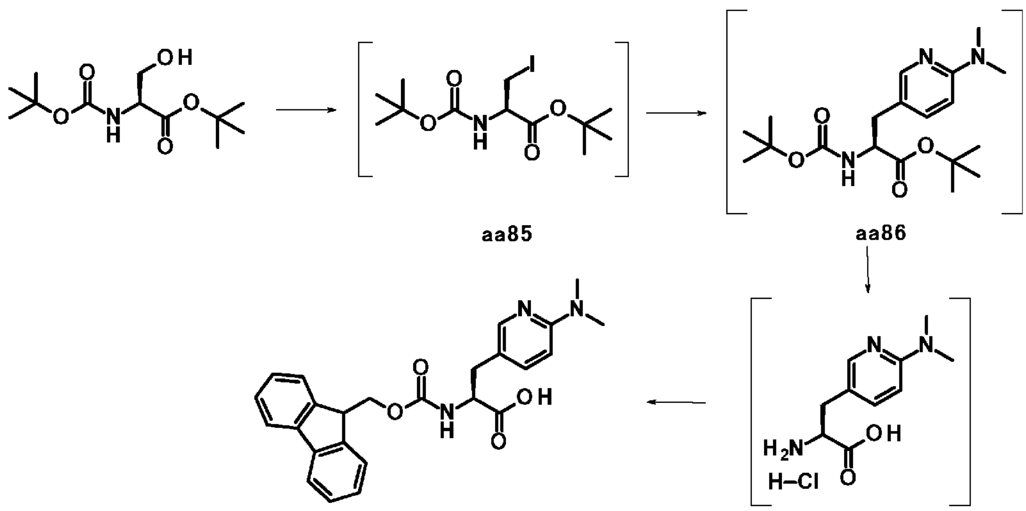

aa85 aa86

aa88 aa87

To a stirring solution of triphenylphosphine ($PPh_3$) (24.09 g, 91.84 mmol) and imidazole (6.25 g, 91.91 mmol) in dichloromethane (200 mL) was added iodine (23.36 g, 91.97 mmol) under shading conditions at 0°C under a nitrogen atmosphere. To the stirring reaction solution at 0°C was added a solution of tert-butyl (tert-butoxycarbonyl)-L-serinate (Boc-Ser-OtBu) (20.0 g, 76.54 mmol) in dichloromethane (100 mL) dropwise, and the mixture was stirred for 1 h. The reaction solution was then further stirred at 20°C for one hour, after which the reaction solution was washed with a saturated aqueous sodium thiosulfate solution. The resulting aqueous layer was extracted with dichloromethane and the organic layer was dried over anhydrous sodium sulfate, filtered, and concentrated under reduced pressure. The resulting residue was purified by normal phase silica gel column chromatography (petroleum ether/ethyl acetate) to afford tert-butyl (R)-2-((tert-butoxycarbonyl)amino)-3-iodopropanoate (Compound aa85) (20.0 g, 70%).

Zinc (2.588 g, 40.44 mmol) was vacuum heated and dried. Dehydrated N,N-dimethylformamide (15 mL) was added thereto under a nitrogen atmosphere, followed by addition of iodine (0.514 g, 2.02 mmol). tert-Butyl (R)-2-((tert-butoxycarbonyl)amino)-3-iodopropanoate (Compound aa85) (5.0 g, 13.47 mmol) and iodine (0.514 g, 2.02 mmol) were added thereto, and the mixture was stirred at 20°C for 2 h. To the reaction solution were added 2-dicyclohexylphosphino-2',4',6'-triisopropylbiphenyl (Xphos) (0.321 g, 0.67 mmol), tris(dibenzylideneacetone)dipalladium(O) ($Pd_2(dba)_3$) (0.349 g, 0.34 mmol), and 5-bromo-N,N-dimethylpyridin-2-amine (3.608 g, 17.94 mmol), and the mixture was stirred at 50°C for 16 h. Ethyl acetate was added to the reaction solution at room temperature, and the mixture was washed with water three times and with brine three times. The resulting organic layer was dried over anhydrous sodium sulfate, filtered, and concentrated under reduced pressure, and the resulting residue was purified by normal phase silica gel column chromatography (petroleum ether/ethyl acetate) to afford tert-butyl (S)-2-((tert-butoxycarbonyl)amino)-3-(6-(dimethylamino)pyridin-3-yl)propanoate (Compound aa86, Boc-Ala(3-Pyr-4-NMe2)-OtBu) (3.5 g, 71%).

Hydrogen chloride gas was injected into a solution of the resulting tert-butyl (S)-2-((tert-butoxycarbonyl)amino)-3-(6-(dimethylamino)pyridin-3-yl)propanoate (Compound aa86, Boc-Ala(3-Pyr-4-NMe2)-OtBu) (10.0 g, 27.36 mmol) in dichloromethane (120 mL), and the mixture was stirred at room temperature for 2 h. The precipitated crude product was recovered by filtration, washed with dichloromethane, and dried to afford (S)-2-amino-3-(6-(dimethylamino)pyridin-3-yl)propanoic acid hydrochloride (Compound aa87, H-Ala(3-Pyr-4-NMe2)-OH·HCl) (6.7 g) quantitatively.

(S)-2-Amino-3-(6-(dimethylamino)pyridin-3-yl)propanoic acid hydrochloride (Compound aa87, H-Ala(3-Pyr-4-NMe2)-OH·HCl) (6.7 g, 27.27 mmol) was dissolved in a 10% aqueous potassium carbonate solution (150 mL), a solution of N-(9-fluorenylmethoxycarbonyloxy)succinimide (Fmoc-OSu) (9.2 g, 27.27 mmol) in 1,4-dioxane (70 mL) was added dropwise thereto, and the mixture was stirred at room temperature for 90 min. Water (100 mL) was added to the reaction solution, and the mixture was washed with diethyl ether three times. The resulting aqueous layer was adjusted to pH 3-5 with an aqueous potassium bisulfate solution and then extracted with ethyl acetate three times. The combined organic layers were dried over anhydrous sodium sulfate, filtered, and concentrated under reduced pressure, and the resulting residue was purified by normal phase silica gel column chromatography (dichloromethane/methanol) to afford (S)-2-((((9H-fluoren-9-yl)methoxy)carbonyl)amino)-3-(6-(dimethylamino)pyridin-3-yl)propanoic acid (Compound aa88, Fmoc-Ala(3-Pyr-4-NMe2)-OH) (3.9 g, 33%).
LCMS (ESI) m/z = 432 (M+H)+
Retention time: 1.55 min (analytical condition ELSD2)

Synthesis of N-(((9H-fluoren-9-yl)methoxy)carbonyl)-N-pentylglycine (Compound aa89, Fmoc-nPenGly-OH)

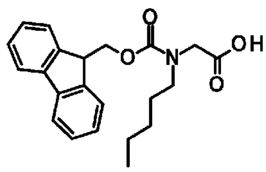

To a solution of pentan-1-amine (18.9 g, 216.95 mmol) in water (450 mL) was added a solution of 2-bromoacetic acid (10.0 g, 71.97 mmol) in water (50 mL) at 0°C, and a 8 N aqueous sodium hydroxide solution (36 mL) was then added dropwise at 0°C. After stirring the reaction solution at room temperature for 16 h, the solvent was evaporated to about 100 mL under reduced pressure. The resulting solution was neutralized to pH = 7 with concentrated hydrochloric acid to give a solution of 2-(pentylamino)acetic acid. To the resulting solution was added a solution of potassium carbonate (38 g, 274.94 mmol) and N-(9-fluorenylmethoxycarbonyloxy)succinimide (Fmoc-OSu) (51 g, 151.34 mmol) in 1,4-dioxane (150 mL) at room temperature, and the mixture was stirred for 16 h. The reaction solution was diluted with t-butyl methyl ether/hexane (1/3) and then filtered, and the resulting filtrate was washed with t-butyl methyl ether/hexane (1/3) three times. Hydrochloric acid was then added to the aqueous layer until pH = 1.0, and the aqueous layer was extracted with

ethyl acetate three times. The organic layers were then dried over anhydrous sodium sulfate, the solvent was evaporated under reduced pressure, and the residue was further dried using a vacuum pump. The resulting residue was purified by reverse phase chromatography (water/acetonitrile) to afford N-(((9H-fluoren-9-yl)methoxy)carbonyl)-N-pentylglycine (Compound aa89, Fmoc-nPenGly-OH) (5.447 g, 11% over two steps).
LCMS (ESI) m/z = 368 (M+H)+
Retention time: 0.92 min (analytical condition SQDFA05)

Synthesis of N-(((9H-fluoren-9-yl)methoxy)carbonyl)-N-hexylglycine (Compound aa90, Fmoc-nHexGly-OH)

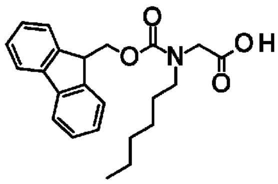

To a solution of hexan-1-amine (21.96 g, 217.02 mmol) in water (500 mL) was added a solution of 2-bromoacetic acid (10.0 g, 71.97 mmol) in water (50 mL) at 0°C, and a 8 N aqueous sodium hydroxide solution (36 mL) was then added dropwise at 0°C. After stirring the reaction solution at room temperature for 16 h, the solvent was evaporated to about 100 mL under reduced pressure. The resulting solution was neutralized to pH = 7 with concentrated hydrochloric acid. To the resulting solution was added a solution of potassium carbonate (32.1 g, 230.58 mmol) and N-(9-fluorenylmethoxycarbonyloxy)succinimide (Fmoc-OSu) (43.1 g, 127.89 mmol) in 1,4-dioxane (125 mL) at room temperature, and the mixture was stirred for 16 h. The reaction solution was diluted with t-butyl methyl ether/hexane (1/3) and then filtered, and the resulting filtrate was washed with t-butyl methyl ether/hexane (1/3) three times. Hydrochloric acid was then added to the aqueous layer until pH = 1.0, and the aqueous layer was extracted with ethyl acetate three times. The organic layers were then dried over anhydrous sodium sulfate, the solvent was evaporated under reduced pressure, and the residue was further dried using a vacuum pump. The resulting residue was purified by reverse phase chromatography (water/acetonitrile) to afford N-(((9H-fluoren-9-yl)methoxy)carbonyl)-N-hexylglycine (Compound aa90, Fmoc-nHexGly-OH) (5.08 g, 19% over two steps).
LCMS (ESI) m/z = 382 (M+H)+
Retention time: 0.98 min (analytical condition SQDFA05)

Synthesis of N-(((9H-fluoren-9-yl)methoxy)carbonyl)-N-(2-ethoxyethyl)glycine (Compound aa91, Fmoc-(EtOEt)NGly-OH)

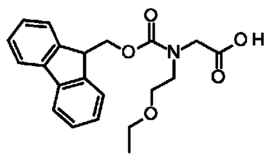

To a solution of 2-ethoxyethan-1-amine (20.0 g, 224.38 mmol) in water (450 mL) was added a solution of 2-bromoacetic acid (10.0 g, 71.97 mmol) in water (50 mL) at 0°C, and a 8 N aqueous sodium hydroxide solution (36 mL) was then added dropwise at 0°C. After stirring the reaction solution at room temperature for 16 h, the solvent was evaporated to about 100 mL under reduced pressure. The resulting solution was neutralized to pH = 7 with concentrated hydrochloric acid. To the resulting solution was added a solution of potassium carbonate (20.0 g, 144.71 mmol) and N-(9-fluorenylmethoxycarbonyloxy)succinimide (Fmoc-OSu) (26.0 g, 77.15 mmol) in 1,4-dioxane (100 mL) at room temperature, and the mixture was stirred for 16 h. The reaction solution was diluted with t-butyl methyl ether/hexane (1/3) and then filtered, and the resulting filtrate was washed with t-butyl methyl ether/hexane (1/3) three times. Hydrochloric acid was then added to the aqueous layer until pH = 1.0, and the aqueous layer was extracted with ethyl acetate three times. The organic layers were then dried over anhydrous sodium sulfate, the solvent was evaporated under reduced pressure, and the residue was further dried using a pump. The resulting residue was purified by reverse phase chromatography (water/acetonitrile) to afford N-(((9H-fluoren-9-yl)methoxy)carbonyl)-N-(2-ethoxyethyl)glycine (Compound aa91, Fmoc-(EtOEt)NGly-OH) (13.0 g, 49% over two steps).

LCMS (ESI) m/z = 370 (M+H)+
Retention time: 0.79 min (analytical condition SQDFA05)

Synthesis of N-(((9H-fluoren-9-yl)methoxy)carbonyl)-N-(2-phenoxyethyl)glycine (Compound aa92, Fmoc-(PhOEt)NGly-OH)

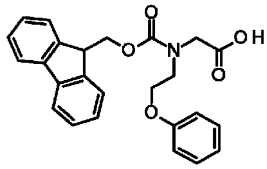

To a solution of 2-phenoxyethan-1-amine (29.8 g, 217.23 mmol) in water (450 mL) was added a solution of 2-bromoacetic acid (10.0 g, 71.97 mmol) in water (50 mL) at 0°C, followed by dropwise addition of a 8 N aqueous sodium hydroxide solution (36 mL) at 0°C. After stirring the reaction solution at room temperature for 16 h, the solvent was evaporated to about 100 mL under reduced pressure. The resulting solution was neutralized to pH = 7 with concentrated hydrochloric acid. To the resulting solution was added a solution of potassium carbonate (20.0 g, 144.71 mmol) and N-(9-fluorenylmethoxycarbonyloxy)succinimide (Fmoc-OSu) (26.0 g, 77.15 mmol) in 1,4-dioxane (100 mL) at room temperature, and the mixture was stirred for 16 h. The reaction solution was diluted with t-butyl methyl ether/hexane (1/3) and then filtered, and the resulting filtrate was washed with t-butyl methyl ether/hexane (1/3) three times. Hydrochloric acid was then added to the aqueous layer until pH = 1.0, and the aqueous layer was extracted with ethyl acetate three times. The organic layers were then dried over anhydrous sodium sulfate, the solvent was evaporated under reduced pressure, and the residue was further dried using a pump. The resulting residue was purified by reverse phase chromatography (water/acetonitrile) to afford N-(((9H-fluoren-9-yl)methoxy)carbonyl)-N-(2-phenoxyethyl)glycine (Compound aa92, Fmoc-(PhOEt) NGly-OH) (5.36 g, 14% over two steps).
LCMS (ESI) m/z = 418 (M+H)+
Retention time: 0.89 min (analytical condition SQDFA05)

Synthesis of tert-butyl N2-(((9H-fluoren-9-yl)methoxy)carbonyl)-N5,N5-dimethyl-L-glutaminate (Compound aa93, Fmoc-Gln(Me2)-OtBu)

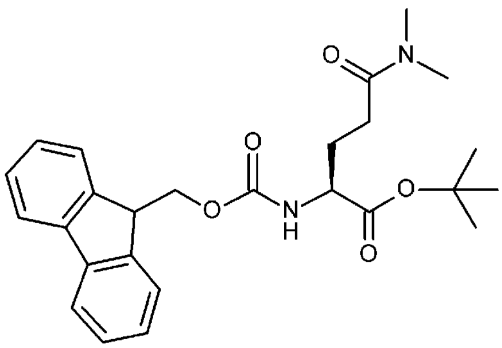

To a solution of (S)-4-((((9H-fluoren-9-yl)methoxy)carbonyl)amino)-5-(tert-butoxy)-5-oxopentanoic acid (Fmoc-Glu-OtBu) (4.00 g, 9.40 mmol) and 1-hydroxybenzotriazole (HOBt) (1.40 g, 10.3 mmol) in dimethylformamide (DMF) (31 mL) were added 1-(3-dimethylaminopropyl)-3-ethylcarbodiimide hydrochloride (WSC·HCl) (2.16 g, 11.3 mmol), dimethylamine hydrochloride (0.767 g, 9.40 mmol), and N,N-diisopropylethylamine (DIPEA) (1.64 mL, 9.40 mmol) under ice-cooling, and the mixture was stirred at room temperature for 90 min. The reaction solution was diluted with a hexane/ethyl acetate (1/1, 100 mL), and the organic layer was washed with brine/water (1:1, 75 mL). The aqueous layer was extracted with hexane/ethyl acetate (1/1), all the organic layers were combined, and the solvent was evaporated under reduced pressure to afford tert-butyl N2-(((9H-fluoren-9-yl)methoxy)carbonyl)-N5,N5-dimethyl-L-glutaminate (Compound aa93, 5.79 g) as a crude product.

LCMS (ESI) m/z = 453 (M+H)+
Retention time: 0.63 min (analytical condition SQDAA50)

Synthesis of N2-(((9H-fluoren-9-yl)methoxy)carbonyl)-N5,N5-dimethyl-L-glutamine (Compound aa94, Fmoc-Gln(Me2)-OH)

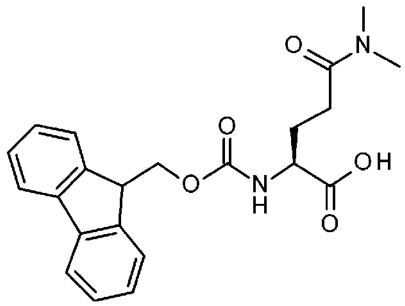

A solution of tert-butyl N2-(((9H-fluoren-9-yl)methoxy)carbonyl)-N5,N5-dimethyl-L-glutaminate (Compound aa93, Fmoc-Gln(Me2)-OtBu) (4.25 g, 9.39 mmol) in dichloromethane (DCM) (4.70 mL) was ice-cooled under a nitrogen atmosphere, trifluoroacetic acid (TFA) (14.5 mL, 188 mmol) was added, and the mixture was stirred at room temperature for 2 h. The solvent was evaporated under reduced pressure, and the resulting crude product was purified by reverse phase column chromatography (0.1% aqueous formic acid solution/0.1% formic acid-acetonitrile solution) to afford N2-(((9H-fluoren-9-yl)methoxy)carbonyl)-N5,N5-dimethyl-L-glutamine (Compound aa94, Fmoc-Gln(Me2)-OH) (3.45 g, 93%).
LCMS (ESI) m/z = 397 (M+H)+
Retention time: 0.40 min (analytical condition SQDAA05)

Synthesis of (9H-fluoren-9-yl)methyl (S)-4-(3-(dimethylamino)-3-oxopropyl)-5-oxooxazolidine-3-carboxylate (Compound aa95)

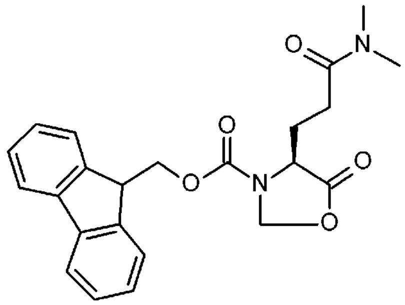

To a solution of N2-(((9H-fluoren-9-yl)methoxy)carbonyl)-N5,N5-dimethyl-L-glutamine (Compound aa94, Fmoc-Gln(Me2)-OH) (13 g, 32.8 mmol) in toluene (325 mL) were added paraformaldehyde (1.98 g, 65.6 mmol) and p-toluenesulfonic acid (338 mg, 1.96 mmol), and the mixture was stirred at 110°C for 16 h. The reaction solution was washed with an aqueous sodium bicarbonate solution and then dried over anhydrous sodium sulfate, and the solvent was evaporated under reduced pressure to afford (9H-fluoren-9-yl)methyl (S)-4-(3-(dimethylamino)-3-oxopropyl)-5-oxooxazolidine-3-carboxylate (Compound aa95) (11.8 g) as a crude product.
LCMS (ESI) m/z = 409 (M+H)+
Retention time: 0.92 min (analytical condition SMD method 22)

Synthesis of N2-(((9H-fluoren-9-yl)methoxy)carbonyl)-N2,N5,N5-trimethyl-L-glutamine (Compound aa96, Fmoc-MeGln(Me2)-OH)

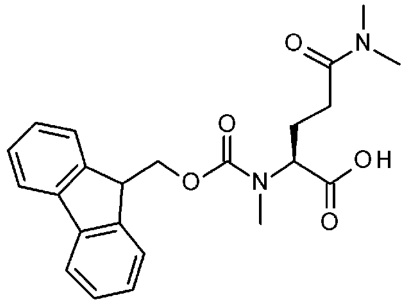

To a solution of (9H-fluoren-9-yl)methyl (S)-4-(3-(dimethylamino)-3-oxopropyl)-5-oxooxazolidine-3-carboxylate (Compound aa95) (13 g, 31.8 mmol) in dichloromethane (DCM) (220 mL) were added triethylsilane (11.1 g, 95.5 mmol) and trifluoroacetic acid (TFA) (220 mL), and the mixture was stirred at room temperature for three days. The solvent was evaporated under reduced pressure, an aqueous potassium carbonate solution was added to the residue, and the mixture was washed with hexane. The aqueous layer was adjusted to a pH range of 2 to 3 with 5% aqueous hydrochloric acid and extracted with ethyl acetate. The organic layer was washed with brine and dried over anhydrous sodium sulfate, and the solvent was then evaporated under reduced pressure. The resulting residue was dissolved in methanol and washed with hexane. The solvent was evaporated from the methanol layer under reduced pressure to afford N2-(((9H-fluoren-9-yl)methoxy)carbonyl)-N2,N5,N5-trimethyl-L-glutamine (Compound aa96, Fmoc-MeGln(Me2)-OH) (12.0 g).
LCMS (ESI) m/z = 411 (M+H)+
Retention time: 1.38 min (analytical condition SMD method 33)

Synthesis of tert-butyl N2-(((9H-fluoren-9-yl)methoxy)carbonyl)-N5-methyl-L-glutaminate (Compound aa97, Fmoc-Gln(Me)-OtBu)

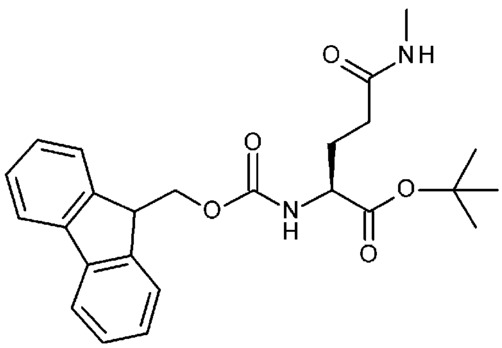

To a solution of (S)-4-((((9H-fluoren-9-yl)methoxy)carbonyl)amino)-5-(tert-butoxy)-5-oxopentanoic acid (Fmoc-Glu-OtBu) (20.0 g, 47.0 mmol) and 1-hydroxy-7-azabenzotriazole (HOAt) (8.8 g) in dimethylformamide (DMF) (300 mL) was added 1-(3-dimethylaminopropyl)-3-ethylcarbodiimide hydrochloride (WSC·HCl) (13.5 g) under ice-cooling, and the mixture was stirred for 10 min. A 2 mol/L solution of methylamine in tetrahydrofuran (29.5 mL) was added while maintaining the temperature of the reaction solution at 5°C or lower, and the mixture was stirred at room temperature for 16 h. The reaction solution was diluted with hexane/ethyl acetate (1/1, 1400 mL) and an aqueous ammonium chloride solution (500 mL), and the organic layer was separated. The organic layer was washed with an aqueous sodium bicarbonate solution and brine and dried over anhydrous sodium sulfate, and the solvent was then evaporated under reduced pressure to afford tert-butyl N2-(((9H-fluoren-9-yl)methoxy)carbonyl)-N5-methyl-L-glutaminate (Compound aa97, Fmoc-Gln(Me)-OtBu) (36.2 g) as a crude product.
LCMS (ESI) m/z = 439 (M+H)+
Retention time: 1.05 min (analytical condition SMD method 23)

Synthesis of N2-(((9H-fluoren-9-yl)methoxy)carbonyl)-N5-methyl-L-glutamine (Compound aa98, Fmoc-Gln(Me)-OH)

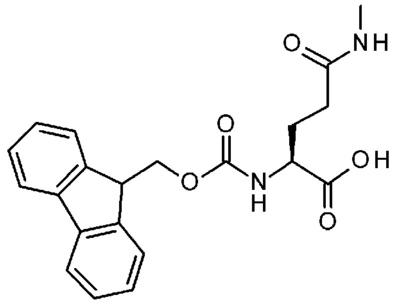

To a solution of tert-butyl N2-(((9H-fluoren-9-yl)methoxy)carbonyl)-N5-methyl-L-glutaminate (Compound aa97, Fmoc-Gln(Me)-OtBu) (43.6 g) in dichloromethane (DCM) (300 mL) was added trifluoroacetic acid (TFA) (300 mL) dropwise under ice-cooling, and the mixture was stirred at room temperature for 16 h. The solvent was evaporated under reduced pressure, diethyl ether and aqueous sodium bicarbonate were added to the resulting residue, and the organic phase was separated. The resulting aqueous layer was adjusted to pH 1-2 with a 5 mol/L aqueous hydrochloric acid solution, and the precipitated solid was collected by filtration to afford N2-(((9H-fluoren-9-yl)methoxy)carbonyl)-N5-methyl-L-glutamine (Compound aa98, Fmoc-Gln(Me)-OH) (26.7 g).
LCMS (ESI) m/z = 383 (M+H)+
Retention time: 1.75 min (analytical condition SMD method 5)

Synthesis of (S)-3-(3-(((9H-fluoren-9-yl)methoxy)carbonyl)-5-oxooxazolidin-4-yl)propanoic acid (Compound aa99)

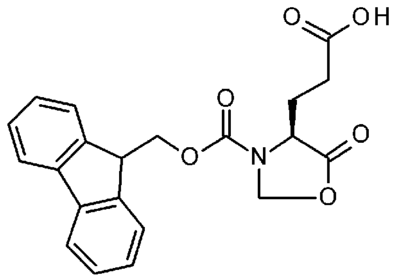

A suspension of N-α-(9-fluorenylmethoxycarbonyl)-L-glutamic acid (Fmoc-Glu-OH, CAS No. 121343-82-6) (2 g, 5.41 mmol), paraformaldehyde (0.65 g, 21.7 mmol), and (+)-10-camphorsulfonic acid (0.69 mg, 0.298 mmol) in toluene (16 ml) was stirred at 75°C for 6 hours and 15 minutes under a nitrogen atmosphere. The reaction solution was cooled to room temperature, sodium bicarbonate (25 mg, 0.298 mmol) was added, and the mixture was stirred at room temperature for 20 h. Ethyl acetate (15 ml) was added, the mixture was filtered through celite, and brine/water (1/1, 5 ml) was added to the filtrate, which was then extracted with ethyl acetate twice. The organic layers were washed with brine/water (1/1, 5 ml) twice and dried over anhydrous sodium sulfate, and the solvent was then evaporated under reduced pressure to afford (S)-3-(3-(((9H-fluoren-9-yl)methoxy)carbonyl)-5-oxooxazolidin-4-yl)propanoic acid (Compound aa99) (2.31 g) as a crude product.
LCMS (ESI) m/z = 382 (M+H)+
Retention time: 0.74 min (analytical condition SQDFA05)

Synthesis of (9H-fluoren-9-yl)methyl (S)-4-(3-(methylamino)-3-oxopropyl)-5-oxooxazolidine-3-carboxylate (Compound aa100)

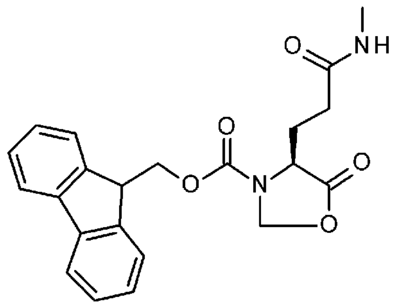

To a solution of (S)-3-(3-(((9H-fluoren-9-yl)methoxy)carbonyl)-5-oxooxazolidin-4-yl)propanoic acid (Compound aa99) (2.30 g, 6.03 mmol) in dimethylformamide (7.0 mL) were added O-(7-aza-1H-benzotriazol-1-yl)-N,N,N',N'-tetramethyluronium hexafluorophosphate (HATU) (2.52 g, 6.63 mmol), methylamine (2 mol/L solution in tetrahydrofuran, 3.02 mL, 6.03 mmol), and N,N-diisopropylethylamine (DIPEA) (0.527 mL, 3.02 mmol) under ice-cooling, and the mixture was stirred for 3 h under a nitrogen atmosphere. The reaction solution was diluted with ethyl acetate and hexane and adjusted to pH 1 with a 5 mol/L aqueous hydrochloric acid solution. A saturated aqueous ammonium chloride solution was added and the mixture was extracted with ethyl acetate twice. The resulting organic layers were washed with saturated aqueous ammonium chloride/water (1/1) twice and dried over anhydrous sodium sulfate, and the solvent was then evaporated under reduced pressure. The resulting crude product was purified by normal phase silica gel column chromatography (hexane/ethyl acetate) to afford (9H-fluoren-9-yl)methyl (S)-4-(3-(methylamino)-3-oxopropyl)-5-oxooxazolidine-3-carboxylate (Compound aa100) (2.40 g) quantitatively.
LCMS (ESI) m/z = 395 (M+H)+
Retention time: 0.74 min (analytical condition SQDFA05)

Synthesis of N2-(((9H-fluoren-9-yl)methoxy)carbonyl)-N2,N5-dimethyl-L-glutamine (Fmoc-MeGln(Me)-OH) (Compound aa101)

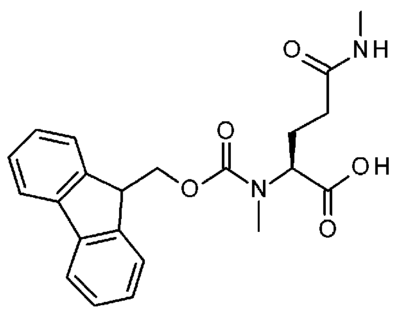

To a solution of (S)-(9H-fluoren-9-yl)methyl 4-(3-(methylamino)-3-oxopropyl)-5-oxooxazolidine-3-carboxylate (Compound aa100) (2.40 g, 6.08 mmol) in dichloromethane (DCM) (15 mL) were added triethylsilane ($Et_3SiH$) (2.92 mL, 18.3 mmol) and boron trifluoride-diethyl ether complex ($BF_3 \cdot OEt_2$) (1.54 mL, 12.2 mmol) under ice-cooling under a nitrogen atmosphere, and the mixture was stirred at room temperature for 24 h. A saturated aqueous ammonium chloride solution/water (1/1, 5 mL) was added and the mixture was extracted with dichloromethane. The organic layer was washed with brine/water (1/1, 5 mL) twice and dried over anhydrous sodium sulfate, and the solvent was then evaporated under reduced pressure. The resulting crude product was purified by reverse phase column chromatography to afford N2-(((9H-fluoren-9-yl)methoxy)carbonyl)-N2,N5-dimethyl-L-glutamine (Compound aa101, Fmoc-MeGln(Me)-OH) (491 mg, 20%).
LCMS (ESI) m/z = 397 (M+H)+
Retention time: 0.65 min (analytical condition SQDFA05)

Synthesis of 2-bromo-N-tert-butylacetamide (Compound aa102)

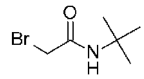

To a solution of 2-bromoacetic acid (50.0 g, 360 mmol) in N,N-dimethylformamide (80.0 mL) were added 2-methylpropan-2-amine (26.7 g, 365 mmol), N-ethyl-isopropylpropan-2-amine (DIPEA) (139 g, 1.08 mol), and propylphosphonic acid anhydride (T3P) (229 g, 720 mmol) under a nitrogen atmosphere, and the mixture was stirred at room temperature for 2 h. Water was added to the reaction solution, and the mixture was extracted with ethyl acetate twice. The resulting organic layers were combined, washed with brine, and then dried over anhydrous sodium sulfate. The solvent was evaporated under reduced pressure, and the resulting residue was washed with hexane to afford 2-bromo-N-tert-butylacetamide (Compound aa102, 36.5 g) as a crude product. This was used in the next step without purification.

Synthesis of O-(2-(tert-butylamino)-2-oxoethyl)-N-trityl-L-serine (Compound aa103, Trt-Ser(NtBu-Aca)-OH)

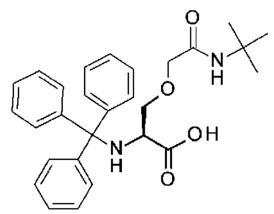

To a solution of trityl-L-serine (Trt-Ser-OH) triethylamine salt (1.50 g, 4.32 mmol) in dimethylformamide (DMF) (2.00 mL) was added sodium hydride (0.52 g, 13.0 mmol, 60% oil dispersion) under a nitrogen atmosphere, and the mixture was stirred at room temperature for 2 h. A solution of 2-bromo-N-tert-butylacetamide (Compound aa102) (0.96 g) in DMF (1.00 mL) was added thereto, and the mixture was stirred at room temperature for 1 h. Water was added to the reaction solution, and the mixture was extracted with ethyl acetate twice. The resulting organic layers were combined, washed with brine, and then dried over anhydrous sodium sulfate. The solvent was evaporated under reduced pressure, and the residue was further dried with a pump to afford O-(2-(tert-butylamino)-2-oxoethyl)-N-trityl-L-serine (Compound aa103, Trt-Ser(NtBu-Aca)-OH) (1.60 g) as a crude product. This was used in the next step without purification.

Synthesis of N-(((9H-fluoren-9-yl)methoxy)carbonyl)-O-(2-(tert-butylamino)-2-oxoethyl)-L-serine (Compound aa104, Fmoc-Ser(NtBu-Aca)-OH)

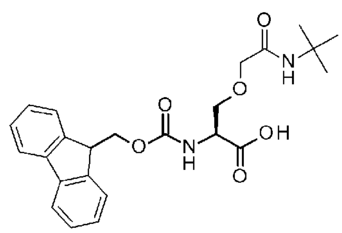

To a solution of O-(2-(tert-butylamino)-2-oxoethyl)-N-trityl-L-serine (Compound aa103, Trt-Ser(NtBu-Aca)-OH) (15.3 g, 33.2 mmol) in dichloromethane (150 mL)/water (150 mL) was added trifluoroacetic acid (11.2 g, 99.1 mmol) at 4°C under a nitrogen atmosphere, and the mixture was stirred at the same temperature for 2 h. The aqueous layer was separated and N-ethyl-isopropylpropan-2-amine (DIPEA) was added until pH 7. After adding 1,4-dioxane (150 mL) thereto, N-ethyl-isopropylpropan-2-amine (DIPEA) (14.9 g, 115 mmol) and N-(9-fluorenylmethoxycarbonyloxy)succinimide (Fmoc-OSu) (22.3 g, 231 mmol) were added and the mixture was stirred at 25°C for 16 h. Water was added to the reaction solution, and the mixture was washed with hexane twice. Concentrated hydrochloric acid was then added to the aqueous layer until pH 2, and the aqueous layer was extracted with ethyl acetate twice. The resulting organic layers were combined, washed with brine, and then dried over anhydrous sodium sulfate. The solvent was evaporated under reduced pressure, and the resulting residue was purified by reverse phase column chromatography (0.1% aqueous formic acid solution/0.1 % formic acid-acetonitrile solution) to afford N-(((9H-fluoren-9-yl)methoxy)carbonyl)-O-(2-(tert-butylamino)-2-oxoethyl)-L-serine (Compound aa104, Fmoc-Ser(NtBu-Aca)-OH) (14.2 g, 98%).
LCMS (ESI) m/z = 441 (M+H)+
Retention time: 1.08 min (analytical condition SMD method 9)

Synthesis of N-(((9H-fluoren-9-yl)methoxy)carbonyl)-O-(2-(tert-butylamino)-2-oxoethyl)-N-methyl-L-serine (Compound aa105, Fmoc-MeSer(NtBu-Aca)-OH)

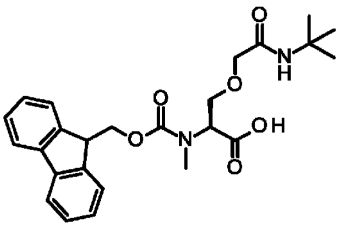

A solution of N-(((9H-fluoren-9-yl)methoxy)carbonyl)-O-(2-(tert-butylamino)-2-oxoethyl)-L-serine (Compound aa104, Fmoc-Ser(NtBu-Aca)-OH) (1.0 g, 2.270 mmol), paraformaldehyde (203 mg, 6.81 mmol), and ((1S,4R)-7,7-dimethyl-2-oxobicyclo[2.2.1]heptan-1-yl)methanesulfonic acid (26 mg, 0.114 mmol) in toluene (1.2 mL) was stirred at 80°C for one hour under a nitrogen atmosphere. A saturated aqueous sodium bicarbonate solution was added to the reaction solution, and the mixture was extracted with ethyl acetate three times and washed with brine. The resulting organic layers were dried over anhydrous sodium sulfate, then filtered, and concentrated under reduced pressure to afford (9H-fluoren-9-yl)methyl (S)-4-((2-(tert-butylamino)-2-oxoethoxy)methyl)-5-oxooxazolidine-3-carboxylate (1.035 g) as a crude product.

To a solution of the above crude product (9H-fluoren-9-yl)methyl (S)-4-((2-(tert-butylamino)-2-oxoethoxy)methyl)-5-oxooxazolidine-3-carboxylate (1.035 g) in dichloromethane (DCM) (2.86 mL) were added triethylsilane ($Et_3SiH$) (1.096 mL, 6.86 mmol) and water (40 μL) under a nitrogen atmosphere, and the mixture was stirred at room temperature for 2 min, after which boron trifluoride-diethyl ether complex ($BF_3{\cdot}OEt_2$) (0.580 mL, 4.57 mmol) was added and the mixture was stirred at room temperature for 24 h. A saturated aqueous ammonium chloride solution was added to the reaction solution, and the mixture was stirred for 40 min at room temperature and then extracted with dichloromethane (DCM) twice. The resulting organic layers were dried over anhydrous sodium sulfate and concentrated under reduced pressure, acetonitrile was added to the resulting residue, and the mixture was washed with hexane. The acetonitrile was evaporated under reduced pressure to afford N-(((9H-fluoren-9-yl)methoxy)carbonyl)-O-(2-(tert-butylamino)-2-oxoethyl)-N-methyl-L-serine (Compound aa105, Fmoc-MeSer(NtBu-Aca)-OH) (0.9449 g, 92% over two steps).
LCMS (ESI) m/z = 455 (M+H)+
Retention time: 0.79 min (analytical condition SQDFA05)

Synthesis of N-(((9H-fluoren-9-yl)methoxy)carbonyl)-O-ethyl-N-methyl-L-homoserine (Compound aa106, Fmoc-MeHse(Et)-OH)

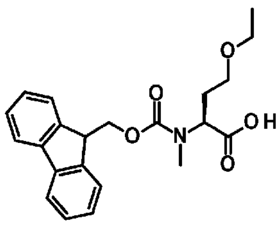

To a solution of commercially available N-(((9H-fluoren-9-yl)methoxy)carbonyl)-O-ethyl-L-homoserine (Fmoc-Hse(Et)-OH, purchased from Watanabe Chemical Industries) (300 mg, 0.812 mmol) and paraformaldehyde (73.2 mg, 2.436 mmol) in toluene (2.5 mL) was added trifluoroacetic acid (0.563 mL, 7.31 mmol) at room temperature under a nitrogen atmosphere, and the mixture was stirred for 6 h. The reaction solution was concentrated, diluted with dichloromethane, and then washed with a saturated aqueous sodium bicarbonate solution. The organic layer was dried over

anhydrous magnesium sulfate, then filtered, and concentrated under reduced pressure to afford (9H-fluoren-9-yl)methyl (S)-4-(2-ethoxyethyl)-5-oxooxazolidine-3-carboxylate (323 mg) as a crude product.

To a solution of the above crude product (9H-fluoren-9-yl) (S)-4-(2-ethoxyethyl)-5-oxooxazolidine-3-carboxylate (310 mg) in dichloroethane (DCE) (4.0 mL) were added triethylsilane ($Et_3SiH$) (1.168 mL, 7.31 mmol) and trifluoroacetic acid (1.691 mL, 21.94 mmol) at room temperature, and the reaction solution was stirred at 60°C for 8 hours and 30 minutes. The reaction solution was cooled to room temperature and then concentrated under reduced pressure. The resulting residue was diluted with hexane/ethyl acetate = 4/1, and the mixture was extracted with a saturated aqueous sodium bicarbonate solution twice. The resulting aqueous layer was acidified with a 1 N aqueous hydrochloric acid solution and then extracted with ethyl acetate three times. The organic layers were dried over anhydrous magnesium sulfate and concentrated under reduced pressure to afford N-(((9H-fluoren-9-yl)methoxy)carbonyl)-O-ethyl-N-methyl-L-homoserine (Compound aa106, Fmoc-MeHse(Et)-OH) (101 mg, 33% over two steps).
LCMS (ESI) m/z = 384 (M+H)+
Retention time: 0.82 min (analytical condition SQDFA05)

<u>Synthesis of (2S)-2-((((9H-fluoren-9-yl)methoxy)carbonyl)amino)-6-((tetrahydro-2H-pyran-2-yl)oxy)hexanoic acid (Compound aa107, Fmoc-Nle(6-OTHP)-OH)</u>

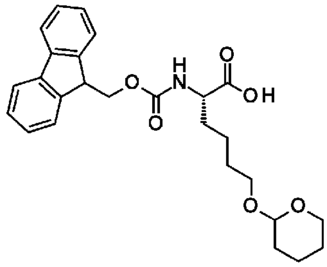

A solution of commercially purchased (S)-2-((((9H-fluoren-9-yl)methoxy)carbonyl)amino)-6-hydroxyhexanoic acid (Fmoc-Nle(6-OH)-OH) (3 g, 8.12 mmol) and pyridinium p-toluenesulfonate (PPTS) (408 mg, 1.62 mmol) in toluene (10 mL) was concentrated under reduced pressure and azeotropically dehydrated. The resulting residue was dissolved in tetrahydrofuran (15 mL), dihydropyran (5.41 mL, 56.8 mmol) was added, and the mixture was stirred at 50°C for 1 h. Ethyl acetate was added to the reaction solution at room temperature, and the organic layer was washed with brine twice, after which the resulting organic layer was dried over anhydrous sodium sulfate, filtered, and concentrated under reduced pressure. The resulting residue was dissolved in tetrahydrofuran (30 mL), phosphate buffer adjusted to pH 8 (30 mL) was added, and the mixture was stirred at 50°C for 3 h. The reaction solution was extracted with ethyl acetate twice at room temperature, and the resulting organic layers were washed with brine, dried over anhydrous sodium sulfate, filtered, and concentrated under reduced pressure. The resulting residue was dissolved in diethyl ether (50 mL), heptane (50 mL) was added thereto to allow the crude product to precipitate, the diethyl ether was removed by concentration under reduced pressure, and the remaining supernatant solution was removed by decantation. This operation was repeated twice to give a crude product. The resulting crude product was dissolved in tert-butyl methyl ether (150 mL), a 0.05 M phosphoric acid solution (150 mL) was added, and the mixture was stirred at room temperature for 1 h. The organic layer was recovered and the aqueous layer was extracted with ethyl acetate. The resulting organic layers were combined and then washed with brine twice. The resulting organic layers were dried over anhydrous sodium sulfate, filtered, and concentrated under reduced pressure to afford (2S)-2-((((9H-fluoren-9-yl)methoxy)carbonyl)amino)-6-((tetrahydro-2H-pyran-2-yl)oxy)hexanoic acid (Compound aa107, Fmoc-Nle(6-OTHP)-OH) (2.925 g, 79%).
LCMS (ESI) m/z = 454 (M+H)+
Retention time: 0.86 min (analytical condition SQDFA05)

<u>Synthesis of tert-butyl (S)-2-((((9H-fluoren-9-yl)methoxy)carbonyl)amino)-4-(4,4-difluoropiperidin-1-yl)-4-oxobutanoate (Compound aa108, Fmoc-Asp(pip-4-F2)-OtBu)</u>

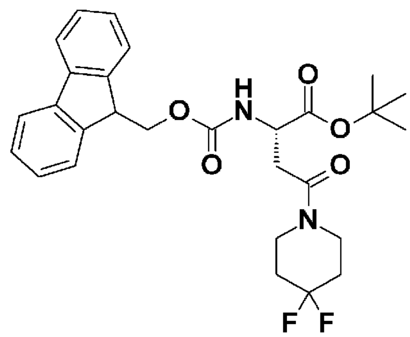

To a solution of 1-(3-dimethylaminopropyl)-3-ethylcarbodiimide hydrochloride (WSC·HCl) in N,N-dimethylformamide (12 mL) were added 1-hydroxybenzotriazole (HOBt) (723 mg, 5.35 mmol) and commercially purchased (S)-3-((((9H-fluoren-9-yl)methoxy)carbonyl)amino)-4-(tert-butoxy)-4-oxobutanoic acid (Fmoc-Asp-OtBu) (2 g, 4.86 mmol) at 0°C under a nitrogen atmosphere, and the mixture was stirred for 1 h. To the reaction solution was added a solution of 4,4-difluoropiperidine hydrochloride (843 mg, 5.35 mmol) and N-ethyl-N-isopropylpropan-2-amine (DIPEA) (931 μL, 5.35 mmol) in N,N-dimethylformamide (2 mL), and the mixture was stirred at 0°C for 3 h. Water was added to the reaction solution, and the mixture was extracted with ethyl acetate twice. The combined organic layers were washed with 0.5 M aqueous hydrochloric acid, water, 50% aqueous sodium bicarbonate, and 50% saline, and the resulting organic layers were concentrated under reduced pressure to afford tert-butyl (S)-2-((((9H-fluoren-9-yl)methoxy)carbonyl)amino)-4-(4,4-difluoropiperidin-1-yl)-4-oxobutanoate (Compound aa108, Fmoc-Asp(pip-4-F2)-OtBu) (2.635 g).
LCMS (ESI) m/z = 515 (M+H)+
Retention time: 0.97 min (analytical condition SQDFA05)

Synthesis of (S)-2-((((9H-fluoren-9-yl)methoxy)carbonyl)amino)-4-(4,4-difluoropiperidin-1-vl)butanoic acid (Compound aa109, Fmoc-Abu(pip-4-F2)-OH)

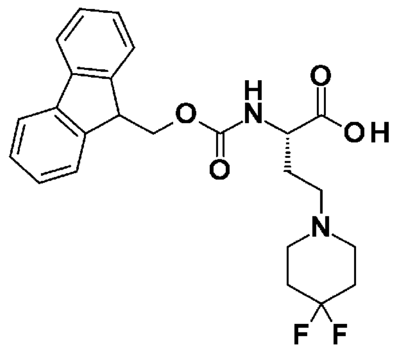

To a solution of triruthenium dodecacarbonyl (62 mg, 0.097 mmol) in tetrahydrofuran (5 mL) was added 1,1,3,3-tetramethyldisiloxane (2.76 mL, 15.55 mmol) under a nitrogen atmosphere, and the mixture was stirred at room temperature for 10 min. To the reaction solution was added tert-butyl (S)-2-((((9H-fluoren-9-yl)methoxy)carbonyl)amino)-4-(4,4-difluoropiperidin-1-yl)-4-oxobutanoate (Compound aa108, Fmoc-Asp(pip-4-F2)-OtBu) (1.0 g, 1.943 mmol) dissolved in tetrahydrofuran (7 mL). After stirring at 50°C for 3 h, the reaction solution was concentrated under reduced pressure. The resulting residue was dissolved in 2,2,2-trifluoroethanol (10 mL), trimethylchlorosilane (745 μL, 5.83 mmol) was added, and the mixture was stirred at room temperature for 1 h. After concentrating the reaction solution under reduced pressure, the resulting residue was dissolved in 1,4-dioxane (5 mL) and a 2 N aqueous hydrochloric acid solution (10 mL) and the mixture was stirred at room temperature for 1 h. The reaction solution was extracted with tert-butyl methyl ether twice, and the organic layers were concentrated under reduced pressure. The resulting residue was purified by reverse phase column chromatography (0.1% aqueous formic acid solution/0.1 % formic acid-acetonitrile solution) to afford (S)-2-((((9H-fluoren-9-yl)methoxy)carbonyl)amino)-4-(4,4-difluoropiperidin-1-yl)butanoic acid (Compound aa109, Fmoc-Abu(pip-4-F2)-OH) (540 mg, 63%).
LCMS (ESI) m/z = 445 (M+H)+
Retention time: 0.56 min (analytical condition SQDFA05)

Synthesis of (S)-2-((((9H-fluoren-9-yl)methoxy)carbonyl)(methyl)amino)-4-(4,4-difluoropiperidin-1-yl)butanoic acid (Compound aa110, Fmoc-MeAbu(pip-4-F2)-OH)

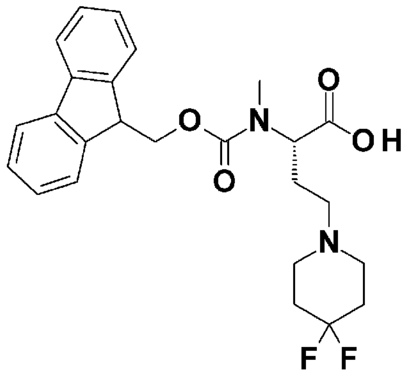

A solution of (S)-2-((((9H-fluoren-9-yl)methoxy)carbonyl)amino)-4-(4,4-difluoropiperidin-1-yl)butanoic acid (Compound aa109, Fmoc-Abu(pip-4-F2)-OH) (13 g, 9.25 mmol), paraformaldehyde (2.6 g, 28.86 mmol), and trifluoroacetic acid (30.59 g, 263.71 mmol) in toluene (60 mL) was stirred at room temperature for 16 h under a nitrogen atmosphere. The reaction solution was concentrated under reduced pressure, then dissolved in dichloromethane, and washed with a saturated aqueous sodium bicarbonate solution, water, and brine. The resulting organic layer was then dried over anhydrous sodium sulfate, filtered, and concentrated under reduced pressure. The resulting residue was purified by normal phase silica gel column chromatography (petroleum ether/ethyl acetate) to afford an intermediate (9H-fluoren-9-yl)methyl (S)-4-(2-(4,4-difluoropiperidin-1-yl)ethyl)-5-oxooxazolidine-3-carboxylate (10.9 g, 82%). The resulting intermediate (6 g, 13.14 mmol) was dissolved in trifluoroacetic acid (65 mL) and dichloroethane (65 mL), triethylsilane (13.5 g, 116.1 mmol) was added at room temperature, and the mixture was stirred at 70°C for 4 h. The reaction solution was brought back to room temperature and then concentrated under reduced pressure, and the resulting residue was dissolved in dichloromethane. The organic layer was washed with water, then dried over anhydrous sodium sulfate, filtered, and concentrated under reduced pressure. The resulting residue was purified by reverse phase column chromatography (0.5% aqueous hydrochloric acid solution/0.5% hydrochloric acid-acetonitrile solution) to afford (S)-2-((((9H-fluoren-9-yl)methoxy)carbonyl)(methyl)amino)-4-(4,4-difluoropiperidin-1-yl)butanoic acid (Compound aa110, Fmoc-MeAbu(pip-4-F2)-OH) (4.0 g, 66%).
LCMS (ESI) m/z = 459 (M+H)+
Retention time: 2.71 min (analytical condition SMD method 28)

Synthesis of 1-benzyl-3,3-difluoropiperidine (Compound aa111)

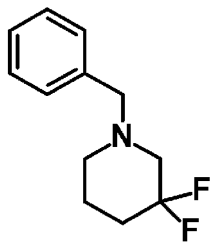

To a solution of 1-benzylpiperidin-3-one (60 g, 317.03 mmol) in dichloromethane was added dimethylaminosulfur trifluoride (DAST) (150 g, 4.04 mol) at 0°C under a nitrogen atmosphere, and the mixture was stirred for 2 h. To the reaction solution was then added 300 mL of a saturated aqueous sodium bicarbonate solution, the mixture was extracted with ethyl acetate three times, and the combined organic layers were washed with brine. The resulting organic layers were dried over anhydrous sodium sulfate, then filtered, and concentrated under reduced pressure to give a crude product 1-benzyl-3,3-difluoropiperidine (Compound aa111) (31 g, 46%).
LCMS (ESI) m/z = 212 (M+H)+
Retention time: 0.94 min (analytical condition SMD method 31)

Synthesis of benzyl (S)-2-(((benzyloxy)carbonyl)(methyl)amino)-4-(3,3-difluoropiperidin-1-yl)butanoate (Compound aa113, Cbz-MeAbu(pip-3-F2)-OBn)

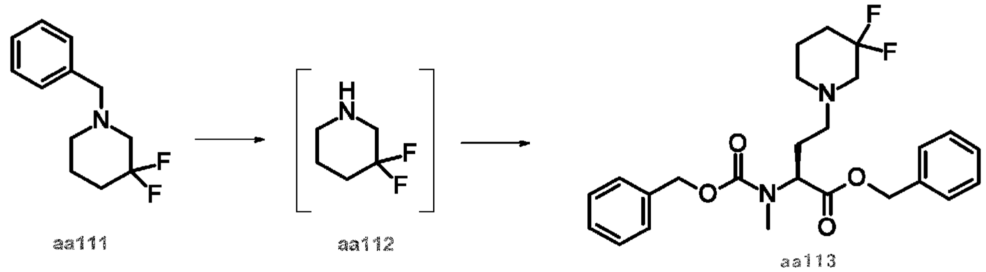

aa111 aa112 aa113

The resulting crude product 1-benzyl-3,3-difluoropiperidine (Compound aa111) was dissolved in 200 mL of methanol, 10% palladium/carbon (1 g) was added, and the mixture was stirred at room temperature for 48 h under a hydrogen atmosphere. The reaction solution was filtered and the resulting filtrate was concentrated under reduced pressure to afford 3,3-difluoropiperidine (Compound aa112) (19 g) as a crude product.

To a solution of separately synthesized benzyl (S)-2-(((benzyloxy)carbonyl)(methyl)amino)-4-(ethylthio)-4-oxobutanoate (Compound aa114, Cbz-MeAsp(SEt)-OBn) (20 g, 48.13 mmol), 3,3-difluoropiperidine (Compound aa112) (8.26 g, 68.19 mmol), triethylsilane (40 mL), and 10% palladium/carbon (2.6 g) in N,N-dimethylformamide (20 mL) was added sodium triacetoxyborohydride ($NaBH(OAc)_3$) (20.4 g, 204 mmol) at 0°C, and the mixture was stirred at room temperature for 30 min. The reaction solution was filtered and concentrated under reduced pressure, and the resulting crude product was purified by normal phase silica gel chromatography (hexane/ethyl acetate) to afford benzyl (S)-2-(((benzyloxy)carbonyl)(methyl)amino)-4-(3,3-difluoropiperidin-1-yl)butanoate (Compound aa113, Cbz-MeAbu(pip-3-F2)-OBn) (11 g, 50%).
LCMS (ESI) m/z = 461 (M+H)+
Retention time: 1.42 min (analytical condition SMD method 32)

Synthesis of (S)-2-((((9H-fluoren-9-yl)methoxy)carbonyl)(methyl)amino)-4-(3,3-difluoropiperidin-1-yl)butanoic acid (Compound aa116, Fmoc-MeAbu(pip-3-F2)-OH)

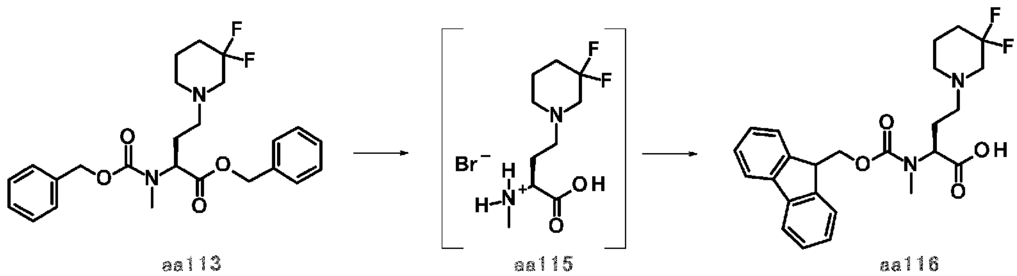

aa113 aa115 aa116

Benzyl (S)-2-(((benzyloxy)carbonyl)(methyl)amino)-4-(3,3-difluoropiperidin-1-yl)butanoate (Compound aa113, Cbz-MeAbu(pip-3-F2)-OBn) (20 g, 43.43 mmol) was dissolved in a 33% hydrogen bromide-acetic acid solution, and the reaction solution was stirred at room temperature for 2 h and then stirred at 50°C for a further 1 h. The reaction solution was concentrated under reduced pressure, and the resulting residue was precipitated by diethyl ether to afford (2S)-4-(3,3-difluoropiperidin-1-yl)-2-(methylamino)butanoic acid bromide (Compound aa115) (29 g) as a crude product.

The above crude product (2S)-4-(3,3-difluoropiperidin-1-yl)-2-(methylamino)butanoic acid bromide (Compound aa115) (7.08 g, 29.97 mmol) and N-(9-fluorenylmethoxycarbonyloxy)succinimide (Fmoc-OSu) (16 g, 47.48 mmol) were dissolved in a solution of potassium carbonate in water/1,4-dioxane = 1/1 (300 mL), and the reaction solution was stirred at room temperature for 4 h. The reaction solution was then washed with diethyl ether three times, and the aqueous layer was adjusted to pH 3 with hydrochloric acid and extracted with ethyl acetate twice. The resulting organic layers were combined, dried over anhydrous sodium sulfate, filtered, and concentrated under reduced pressure, and the resulting residue was purified by reverse phase column chromatography (10 mmol aqueous ammonium bicarbonate/acetonitrile) to afford (S)-2-((((9H-fluoren-9-yl)methoxy)carbonyl)(methyl)amino)-4-(3,3-difluoropiperidin-1-yl)butanoic acid

(Compound aa116, Fmoc-MeAbu(pip-3-F2)-OH) (7.2g, 71%).
LCMS (ESI) m/z = 459 (M+H)+
Retention time: 1.61 min (analytical condition SMD method 5)

Synthesis of (S)-2-(((benzyloxy)carbonyl)(methyl)amino)-4-(ethylthio)-4-oxobutanoic acid (Compound aa119, Cbz-MeAsp(SEt)-OH)

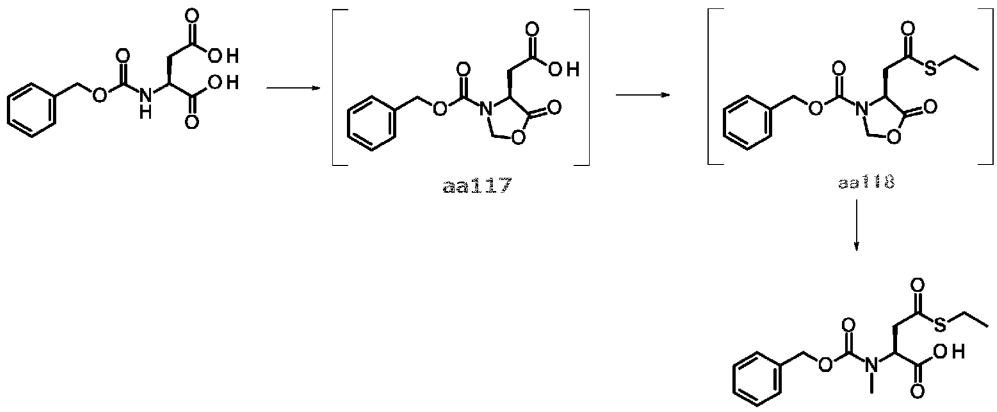

aa117 aa118 aa119

A solution of ((benzyloxy)carbonyl)-L-aspartic acid (Cbz-Asp-OH) (200 g, 748.41 mmol), paraformaldehyde (67.5 g, 749.35 mmol), and p-toluenesulfonic acid (7.74 g, 44.95 mmol) in toluene (2000 mL) was warmed to 110°C under a nitrogen atmosphere and stirred for three days. The reaction solution was brought to room temperature, water was then added, and the mixture was extracted with ethyl acetate three times. The combined organic layers were washed with brine, and the resulting organic layers were dried over anhydrous sodium sulfate, then filtered, and concentrated under reduced pressure to afford (S)-2-(3-((benzyloxy)carbonyl)-5-oxooxazolidin-4-yl)acetic acid (Compound aa117) as a crude product (200 g).

To a solution of a crude product of (S)-2-(3-((benzyloxy)carbonyl)-5-oxooxazolidin-4-yl)acetic acid (Compound aa117) synthesized by the same method as described above (265 g, 948.99 mmol), ethanethiol (88.3 g, 1.42 mol), and 4-dimethylaminopyridine (DMAP) (11.59 g, 95 mmol) in dichloromethane was added N,N'-dicyclohexylcarbodiimide (DCC) (214 g, 1.04 mol) at 0°C, and the mixture was stirred at room temperature for 5 h. The reaction solution was filtered, the resulting filtrate was washed with brine, and the resulting organic layer was dried over anhydrous sodium sulfate, then filtered, and concentrated under reduced pressure. The resulting crude product was purified by normal phase silica gel chromatography (petroleum ether/ethyl acetate) to afford a mixture of benzyl (S)-4-(2-(ethylthio)-2-oxoethyl)-5-oxooxazolidine-3-carboxylate (Compound aa118) (167 g).

To a solution of the obtained benzyl (S)-4-(2-(ethylthio)-2-oxoethyl)-5-oxooxazolidine-3-carboxylate (Compound aa118) (139 g, 1.20 mol) in dichloromethane (DCM) and trifluoroacetic acid (1500 mL/1500 mL) was added triethylsilane (139 g, 1.2 mol), and the mixture was stirred at room temperature for three days. The reaction solution was concentrated under reduced pressure, an aqueous potassium carbonate solution was added to the resulting residue, and the mixture was washed with diethyl ether three times. The resulting aqueous layer was adjusted to pH 3 with a 2 N aqueous hydrochloric acid solution and extracted with ethyl acetate twice. The combined organic layers were washed with brine, dried over anhydrous sodium sulfate, then filtered, and concentrated under reduced pressure to afford a crude product of (S)-2-(((benzyloxy)carbonyl)(methyl)amino)-4-(ethylthio)-4-oxobutanoic acid (Compound aa119, Cbz-MeAsp(SEt)-OH) (75 g).
LCMS (ESI) m/z = 326 (M+H)+
Retention time: 1.00 min (analytical condition SMD method 16)

Synthesis of benzyl (S)-2-(((benzyloxy)carbonyl)(methyl)amino)-4-(ethylthio)-4-oxobutanoate (Compound aa114, Cbz-MeAsp(SEt)-OBn)

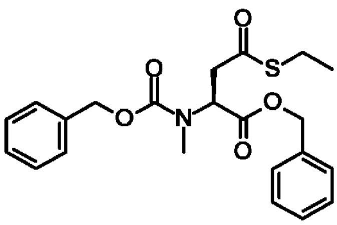

To a solution of the crude product of (S)-2-(((benzyloxy)carbonyl)(methyl)amino)-4-(ethylthio)-4-oxobutanoic acid (Compound aa119, Cbz-MeAsp(SEt)-OH) (3 g, 9.22 mmol) and potassium carbonate (1.9 g, 13.65 mmol) in N,N-dimethylformamide (46 mL) was added benzyl bromide (1.73 g, 10.11 mmol) at room temperature, and the mixture was stirred for 16 hours and then filtered. The resulting filtrate was concentrated under reduced pressure, and the resulting residue was purified by normal phase silica gel chromatography (petroleum ether/ethyl acetate) to afford benzyl (S)-2-(((benzyloxy)carbonyl)(methyl)amino)-4-(ethylthio)-4-oxobutanoate (Compound aa114, Cbz-MeAsp(SEt)-OBn) (1.5 g).

$^1$H NMR (300 MHz, $CDCl_3$): δ 7.36-7.31 (m, 10H), 5.19-4.89 (m, 5H), 3.34-2.85 (m, 7H), 1.30-1.21 (m, 3H)

(S)-2-((((9H-Fluoren-9-yl)methoxy)carbonyl)amino)-4-morpholinobutanoic acid (Compound aa123, Fmoc-Abu(Mor)-OH) was synthesized according to the following scheme.

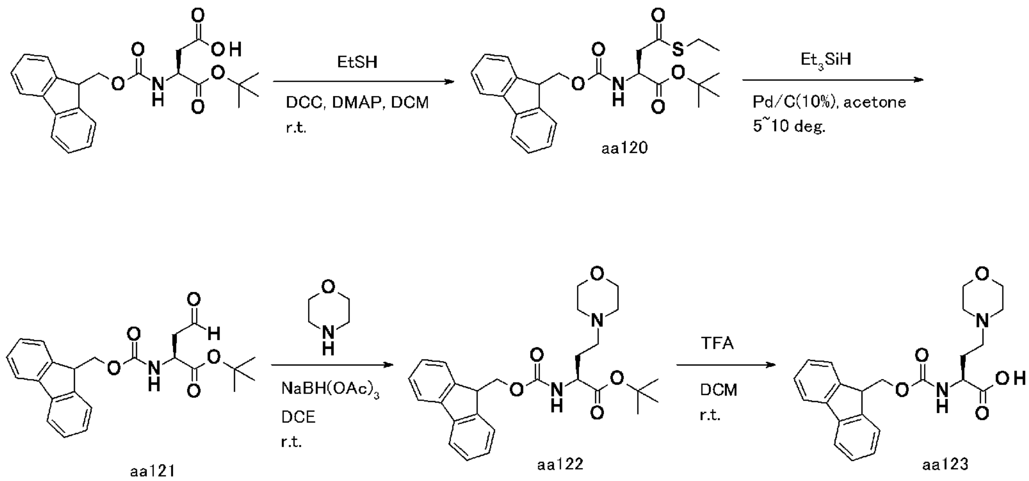


Synthesis of tert-butyl (S)-2-((((9H-fluoren-9-yl)methoxy)carbonyl)amino)-4-(ethylthio)-4-oxobutanoate (Compound aa120, Fmoc-Asp(SEt)-OtBu)

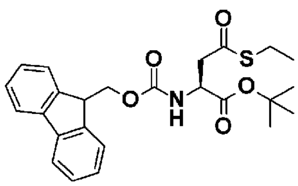

(S)-3-((((9H-Fluoren-9-yl)methoxy)carbonyl)amino)-4-(tert-butoxy)-4-oxobutanoic acid (Fmoc-Asp-OtBu) (5 g, 12.15 mmol), 4-dimethylaminopyridine (DMAP) (148 mg, 1.22 mmol), and ethanethiol (EtSH) (1.13 g, 18.19 mmol) were dissolved in dichloromethane (DCM) (50 mL), and N,N'-dicyclohexylcarbodiimide (DCC) (2.756 g, 1.337 mmol) was added under ice-cooling. After stirring at room temperature for 5 h, the solid was removed by filtration. The filtrate was diluted with dichloromethane (DCM), then washed with brine, dried over anhydrous sodium sulfate, then filtered, and concentrated under reduced pressure. The resulting residue was purified by normal phase column chromatography (0 to 40% ethyl acetate/petroleum ether) to afford tert-butyl (S)-2-((((9H-fluoren-9-yl)methoxy)carbonyl)amino)-4-(ethylthio)-4-oxobutanoate (Compound aa120, Fmoc-Asp(SEt)-OtBu) (3.9 g, 70%).

LCMS (ESI) m/z = 478 (M+Na)+

Retention time: 3.13 min (analytical condition SMD method 14)

Synthesis of tert-butyl (S)-2-((((9H-fluoren-9-yl)methoxy)carbonyl)amino)-4-oxobutanoate (Compound aa121)

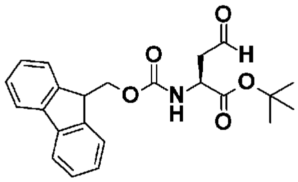

tert-Butyl (S)-2-((((9H-fluoren-9-yl)methoxy)carbonyl)amino)-4-(ethylthio)-4-oxobutanoate (Compound aa120, Fmoc-Asp(SEt)-OtBu) (1 g, 2.20 mmol) was dissolved in acetone (4.4 mL), and Pd/C (wet, 10%, 44 mg) was added. Triethylsilane (1.276 g, 10.97 mmol) was added under ice-cooling, and the mixture was stirred for 1 h. Pd/C was then removed by filtration and the filtrate was concentrated under reduced pressure. The resulting residue was purified by normal phase column chromatography (0 to 60% ethyl acetate/petroleum ether) to afford tert-butyl (S)-2-((((9H-fluoren-9-yl)methoxy)carbonyl)amino)-4-oxobutanoate (Compound aa121) (680 mg, 78%).
LCMS (ESI) m/z = 418 (M+Na)+
Retention time: 2.07 min (analytical condition SMD method 40)

Synthesis of tert-butyl (S)-2-((((9H-fluoren-9-yl)methoxy)carbonyl)amino)-4-morpholinobutanoate (Compound aa122, Fmoc-Abu(Mor)-OtBu)

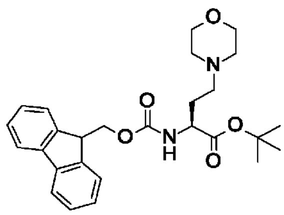

tert-Butyl (S)-2-((((9H-fluoren-9-yl)methoxy)carbonyl)amino)-4-oxobutanoate (Compound aa121) (1.6 g, 4.05 mmol) and morpholine (387 mg, 4.44 mmol) were dissolved in dichloroethane (DCE) (8 mL), and the reaction solution was stirred at room temperature for 1 h. A solution of sodium triacetoxyborohydride ($NaBH(OAc)_3$) (1.717 g, 8.10 mmol) in dichloroethane (DCE) (8 mL) was then added under ice-cooling, and the mixture was stirred for 4 h. The reaction solution was then diluted with dichloromethane and washed with a saturated aqueous sodium bicarbonate solution and brine, after which the organic layer was dried over anhydrous sodium sulfate, filtered, and then concentrated under reduced pressure. The resulting residue was purified by normal phase column chromatography (petroleum ether/ethyl acetate) to afford tert-butyl (S)-2-((((9H-fluoren-9-yl)methoxy)carbonyl)amino)-4-morpholinobutanoate (Compound aa122, Fmoc-Abu(Mor)-OtBu) (1.7 g, 98%).
LCMS (ESI) m/z = 467 (M+H)+
Retention time: 2.02 min (analytical condition SMD method 41)

Synthesis of (S)-2-((((9H-Fluoren-9-yl)methoxy)carbonyl)amino)-4-morpholinobutanoic acid (Compound aa123, Fmoc-Abu(Mor)-OH)

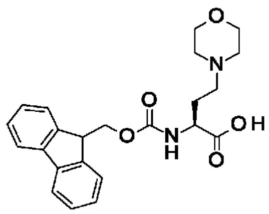

tert-Butyl (S)-2-((((9H-fluoren-9-yl)methoxy)carbonyl)amino)-4-morpholinobutanoate (Compound aa122, Fmoc-Abu(Mor)-OtBu) (9 g, 19.29 mmol) was dissolved in trifluoroacetic acid (TFA)/dichloroethane (DCE) (25 mL/25 mL), and the reaction solution was stirred at 40°C for 2 h. After cooling to room temperature, the reaction solution was diluted with dichloromethane and washed with water. The organic layer was dried over anhydrous sodium sulfate, then filtered, and concentrated under reduced pressure to afford (S)-2-((((9H-fluoren-9-yl)methoxy)carbonyl)amino)-4-morpholinobutanoic acid (Compound aa123, Fmoc-Abu(Mor)-OH) (7 g, 90%).
LCMS (ESI) m/z = 411 (M+H)+
Retention time: 1.20 min (analytical condition SMD method 42)

(S)-2-((((9H-Fluoren-9-yl)methoxy)carbonyl)(methyl)amino)-4-morpholinobutanoic acid (Compound aa125, Fmoc-MeAbu(Mor)-OH) was synthesized according to the following scheme.

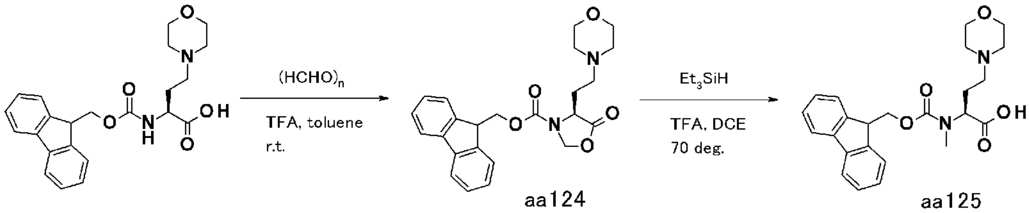


Synthesis of (9H-fluoren-9-yl)methyl(S)-4-(2-morpholinoethyl)-5-oxooxazolidine-3-carboxylate (Compound aa124)

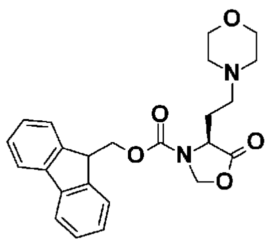

(S)-2-((((9H-Fluoren-9-yl)methoxy)carbonyl)amino)-4-morpholinobutanoic acid (Compound aa123, Fmoc-Abu(Mor)-OH) (600 mg, 1.46 mmol) and paraformaldehyde (131.4 mg, 1.46 mmol) were suspended in toluene (3 mL) and trifluoroacetic acid (TFA) (1.527 g, 13.16 mmol), and the suspension was stirred at room temperature for 16 h. The reaction solution was then concentrated under reduced pressure, and the resulting residue was diluted with dichloromethane and washed with a saturated aqueous sodium bicarbonate solution, water, and brine. The organic layer was dried over anhydrous sodium sulfate and then filtered, the filtrate was concentrated under reduced pressure, and the resulting residue was purified by normal phase column chromatography (petroleum ether/ethyl acetate) to afford (9H-fluoren-9-yl)methyl (S)-4-(2-morpholinoethyl)-5-oxooxazolidine-3-carboxylate (Compound aa124) (486 mg, 79%).
LCMS (ESI) m/z = 423 (M+H)+
Retention time: 1.14 min (analytical condition SMD method 40)

Synthesis of (S)-2-((((9H-fluoren-9-yl)methoxy)carbonyl)(methyl)amino)-4-morpholinobutanoic acid (Compound aa125, Fmoc-MeAbu(Mor)-OH)

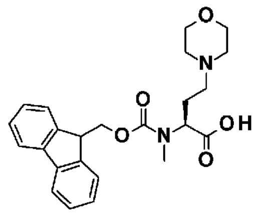

(9H-Fluoren-9-yl)methyl (S)-4-(2-morpholinoethyl)-5-oxooxazolidine-3-carboxylate (Compound aa124) (486 mg, 1.15 mmol) and triethylsilane (1.5 mL, 10.35 mmol) were dissolved in trifluoroacetic acid/dichloroethane (1/1, 12 mL), and the reaction solution was stirred at 70°C for 4 h. After cooling to room temperature, the reaction solution was concentrated under reduced pressure, the resulting residue was diluted with dichloromethane and washed with water, the organic layer was then dried over anhydrous sodium sulfate and filtered, and the filtrate was then concentrated under reduced pressure. The resulting residue was purified by reverse phase column chromatography (5 to 70% 0.5% aqueous hydrochloric acid solution/0.5% hydrochloric acid-acetonitrile solution) to afford (S)-2-((((9H-fluoren-9-yl)methoxy)carbonyl)(methyl)amino)-4-morpholinobutanoic acid (Compound aa125, Fmoc-MeAbu(Mor)-OH) (400 mg, 82%).
LCMS (ESI) m/z = 425 (M+H)+
Retention time: 1.40 min (analytical condition SMD method 34)

Synthesis of N-(((9H-fluoren-9-yl)methoxy)carbonyl)-N-(2-(4,4-difluoropiperidin-1-yl)ethyl)glycine (Compound aa126, Fmoc-(2-(pip-4-F2)-Et)Gly-OH)

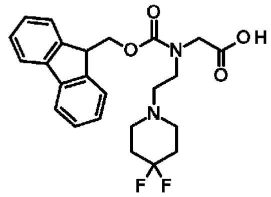

To a solution of 2-(4,4-difluoropiperidin-1-yl)ethan-1-amine (0.842 g, 5.13 mmol) in water (8.5 mL) was added a solution of tert-butyl 2-bromoacetate (0.378 mL, 2.56 mmol) in tetrahydrofuran (THF) (1.7 mL) at 0°C, and the mixture was then stirred at room temperature overnight. Water (8.5 mL) was added to the reaction solution, N-ethyl-N-isopropylpropan-2-amine (DIPEA) (1.365 mL, 7.69 mmol) and N-(9-fluorenylmethoxycarbonyloxy)succinimide (Fmoc-OSu) (1.902 g, 5.64 mmol) were added, and the mixture was stirred at room temperature for 1 h. To the reaction solution were added dimethyl sulfoxide (DMSO) and a 20% aqueous acetonitrile solution containing 0.1% formic acid, the tetrahydrofuran was removed under reduced pressure, and the residue was then purified by reverse phase chromatography (0.1% aqueous formic acid solution/ 0.1% formic acid-acetonitrile solution) to afford tert-butyl N-(((9H-fluoren-9-yl)methoxy)carbonyl)-N-(2-(4,4-difluoropiperidin-1-yl)ethyl)glycinate (Fmoc-(2-(pip-4-F2)-Et)Gly-OtBu) (875 mg).

To a solution of the above tert-butyl N-(((9H-fluoren-9-yl)methoxy)carbonyl)-N-(2-(4,4-difluoropiperidin-1-yl)ethyl)glycinate (Fmoc-(2-(pip-4-F2)-Et)Gly-OtBu) (870 mg) in 2,2,2-trifluoroethanol (TFE) (7.0 mL) was added chlorotrimethylsilane (TMSCl) (0.666 mL, 5.21 mmol), and the mixture was stirred at room temperature for 1 h. The reaction solution was concentrated under reduced pressure, and the resulting residue was then purified by reverse phase chromatography (0.1% aqueous formic acid solution/0.1 % formic acid-acetonitrile solution) to give a mixture of the title compound and formic acid. To remove the formic acid, 4 N hydrochloric acid/1,4-dioxane was added to the resulting mixture, which was then concentrated using a rotary evaporator. This operation was repeated six times to afford N-(((9H-fluoren-9-yl)methoxy)carbonyl)-N-(2-(4,4-difluoropiperidin-1-yl)ethyl)glycine (Compound aa126, Fmoc-(2-(pip-4-F2)-Et)Gly-OH) (798.7 mg, 70% over three steps) as a hydrochloride.
LCMS (ESI) m/z = 445 (M+H)+
Retention time: 0.58 min (analytical condition SQDFA05)

(2S,4R)-4-(4,4-difluoro-1-piperidyl)-1-(9H-fluoren-9-ylmethoxycarbonyl)pyrrolidine-2-carboxylic acid (Compound aa130, Fmoc-Pro(pip-4-F2)-OH) was synthesized according to the following scheme.

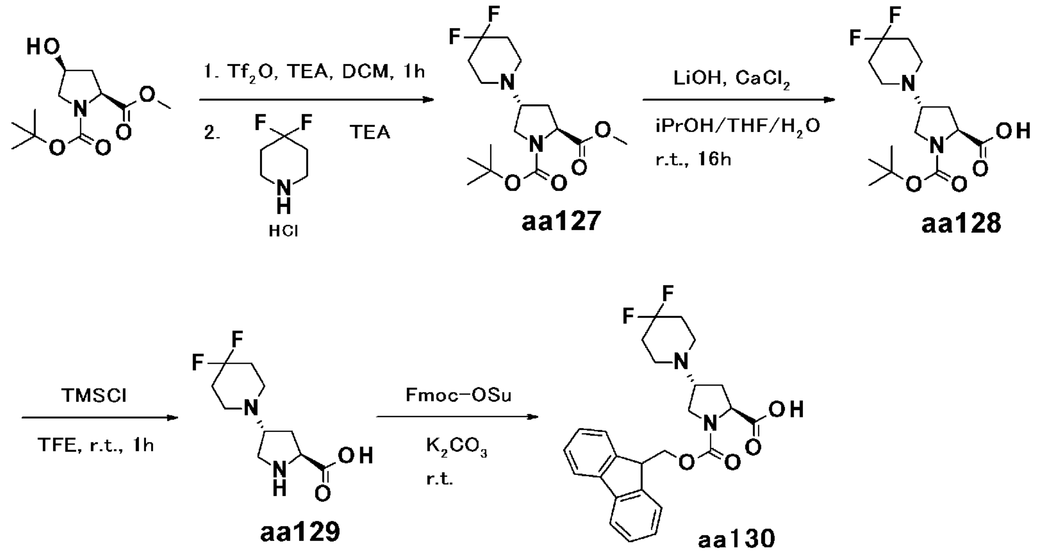


Synthesis of 1-(tert-butyl)2-methyl (2S,4R)-4-(4,4-difluoropiperidin-1-yl)pyrrolidine-1,2-dicarboxylate (Compound aa127, Boc-Pro(pip-4-F2)-OMe)

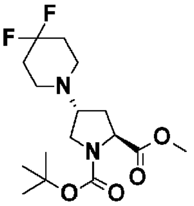

1-tert-Butyl 2-methyl (2S,4S)-4-hydroxypyrrolidine-1,2-dicarboxylate (Boc-cisHyp-OMe) (30 g, 122.3 mmol) and triethylamine (27.2 mL, 195.7 mmol) were dissolved in dichloromethane (300 mL), and trifluoromethanesulfonic anhydride ($Tf_2O$) (24.8 mL, 146.8 mmol) were added dropwise under cooling at -40°C. After stirring for one hour, a solution of triethylamine (70 mL, 489.2 mmol) and 4,4-difluoropiperidine hydrochloride (38.4 g, 317.01 mmol) in dichloromethane (100 mL) was added and the mixture was further stirred for 3 h. The reaction solution was then poured into a saturated aqueous sodium bicarbonate solution and extracted with t-butyl methyl ether three times. The organic layers were washed with brine, dried over anhydrous sodium sulfate, filtered, and then concentrated under reduced pressure to afford 1-(tert-butyl)2-methyl(2S,4R)-4-(4,4-difluoropiperidin-1-yl)pyrrolidine-1,2-dicarboxylate (Compound aa127, Boc-Pro(pip-4-F2)-OMe) (52 g).
LCMS (ESI) m/z = 349 (M+H)+
Retention time: 0.62 min (analytical condition SMD method35)

Synthesis of (2S,4R)-1-tert-butoxycarbonyl-4-(4,4-difluoro-1-piperidyl)pyrrolidine-2-carboxylic acid (Compound aa128, Boc-Pro(pip-4-F2)-OH)

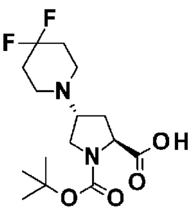

Lithium hydroxide (8.25 g, 344.47 mmol) and calcium chloride (143 g, 1.30 mol) were added to 2-propanol

(iPrOH) (900 mL) and water (300 mL) and the mixture was stirred, after which a solution of 1-(tert-butyl)2-methyl (2S,4R)-4-(4,4-difluoropiperidin-1-yl)pyrrolidine-1,2-dicarboxylate (Compound aa127, Boc-Pro(pip-4-F2)-OMe) (52 g, 149.26 mmol) in tetrahydrofuran (THF) (300 mL) was added and the mixture was stirred at room temperature for 16 h. The organic solvent was removed by concentration under reduced pressure, and the resulting aqueous layer was washed with t-butyl methyl ether. The aqueous layer was then adjusted to pH 5-6 by adding a 1 N aqueous hydrochloric acid solution and extracted with ethyl acetate three times. The organic layers were dried over anhydrous sodium sulfate, filtered, and then concentrated under reduced pressure to afford (2S,4R)-1-tert-butoxycarbonyl-4-(4,4-difluoro-1-piperidyl)pyrrolidine-2-carboxylic acid (Compound aa128, Boc-Pro(pip-4-F2)-OH) (48 g).
LCMS (ESI) m/z = 335 $(M+H)^+$
Retention time: 0.88 min (analytical condition SMD method 13)

Synthesis of (2S,4R)-4-(4,4-difluoro-1-piperidyl)pyrrolidine-2-carboxylic acid (Compound aa129, H-Pro(pip-4-F2)-OH)

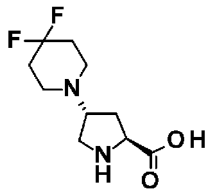

(2S,4R)-1-tert-Butoxycarbonyl-4-(4,4-difluoro-1-piperidyl)pyrrolidine-2-carboxylic acid (Compound aa128, Boc-Pro(pip-4-F2)-OH) (48 g, 143.28 mmol) was dissolved in 2,2,2-trifluoroethanol (TFE) (900 mL), chlorotrimethylsilane (TMSCl) (60 g, 573.12 mmol) was added, and the mixture was stirred at room temperature for 1 h. The reaction solution was then concentrated under reduced pressure to afford (2S,4R)-4-(4,4-difluoro-1-piperidyl)pyrrolidine-2-carboxylic acid (Compound aa129, H-Pro(pip-4-F2)-OH) (37.3 g).
LCMS (ESI) m/z = 235 (M+H)+
Retention time: 0.13 min (analytical condition SMD method 36)

Synthesis of (2S,4R)-4-(4,4-difluoro-1-piperidyl)-1-(9H-fluoren-9-ylmethoxycarbonyl)pyrrolidine-2-carboxylic acid (Compound aa130, Fmoc-Pro(pip-4-F2)-OH)

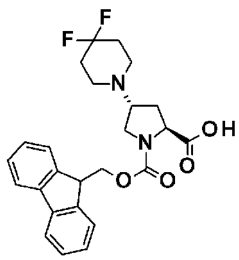

(2S,4R)-4-(4,4-Difluoro-1-piperidyl)pyrrolidine-2-carboxylic acid (Compound aa129, H-Pro(pip-4-F2)-OH) (37.3 g, 159.23 mmol), N-(9-fluorenylmethoxycarbonyloxy)succinimide (Fmoc-OSu) (56.4 g, 167.19 mmol), and potassium carbonate (44 g, 318.46 mmol) were added to water (350 mL) and 1,4-dioxane (260 mL), and the mixture was stirred at room temperature for 16 h. The reaction solution was then washed with a 25% solution of t-butyl methyl ether in hexane, and the aqueous layer was adjusted to pH 1-2 with a 1 N aqueous hydrochloric acid solution and extracted with t-butyl methyl ether three times. The organic layers were washed with brine, then dried over anhydrous sodium sulfate, and filtered, and the filtrate was then concentrated under reduced pressure. The resulting residue was purified by reverse phase column chromatography (5 to 65% water/acetonitrile) to afford (2S,4R)-4-(4,4-difluoro-1-piperidyl)-1-(9H-fluoren-9-ylmethoxycarbonyl)pyrrolidine-2-carboxylic acid (Compound aa130, Fmoc-Pro(pip-4-F2)-OH) (4.5 g, 8% over four steps).
LCMS (ESI) m/z = 457 (M+H)+
Retention time: 2.83 min (analytical condition SMD method 37)

Compound aa133 (Fmoc-cisPro(pip-4-F2)-OH) was synthesized according to the following scheme.

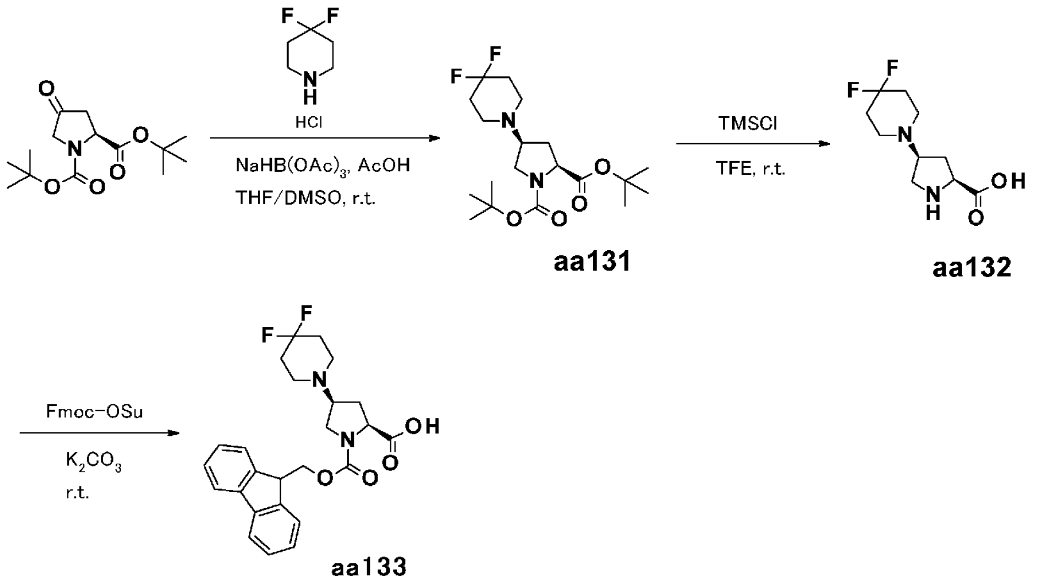


Synthesis of di-tert-butyl (2S,4S)-4-(4,4-difluoro-1-piperidyl)pyrrolidine-1,2-dicarboxylate (Compound aa131, Boc-cis-Pro(pip-4-F2)-OtBu)

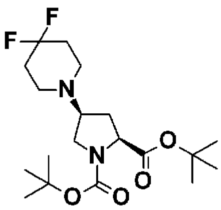

Di-tert-butyl (2S)-4-oxopyrrolidine-1,2-dicarboxylate (20 g, 70.09 mmol) was dissolved in tetrahydrofuran (400 mL) and dimethyl sulfoxide (120 mL), and 4,4-difluoropiperidine hydrochloride (11 g, 90.81 mmol) and acetic acid (8 mL) were added. After stirring at room temperature for 1 h, sodium triacetoxyborohydride ($NaBH(OAc)_3$) (29.8 g, 140.6 mmol) was added and the mixture was stirred for 6 h. The reaction solution was poured into a saturated aqueous sodium bicarbonate solution under ice-cooling and then extracted with ethyl acetate three times. The organic layers were washed with brine, then dried over anhydrous sodium sulfate, filtered, and then concentrated under reduced pressure to afford di-tert-butyl (2S,4S)-4-(4,4-difluoro-1-piperidyl)pyrrolidine-1,2-dicarboxylate (Compound aa131, Boc-cisPro(pip-4-F2)-OtBu) (50 g).
LCMS (ESI) m/z = 391 (M+H)+
Retention time: 0.75 min (analytical condition SMD method 38)

Synthesis of (2S,4S)-4-(4,4-difluoro-1-piperidyl)pyrrolidine-2-carboxylic acid (Compound aa132, H-cisPro(pip-4-F2)-OH)

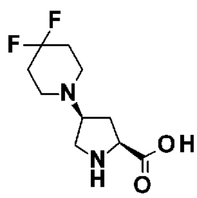

Di-tert-butyl (2S,4S)-4-(4,4-difluoro-1-piperidyl)pyrrolidine-1,2-dicarboxylate (Compound aa131, Boc-cis-Pro(pip-4-F2)-OtBu) (50 g, 128.70 mmol) was dissolved in 2,2,2-trifluoroethanol (TFE) (1000 mL), chlorotrimethylsilane (TMSCl) (80 mL, 643.5 mmol) was added, and the mixture was stirred for 1 h. The reaction solution was then concentrated under reduced pressure to afford (2S,4S)-4-(4,4-difluoro-1-piperidyl)pyrrolidine-2-carboxylic acid (Compound aa132, H-cisPro(pip-4-F2)-OH) (45 g).
LCMS (ESI) m/z = 235 (M+H)+
Retention time: 0.33 min (analytical condition SMD method39)

Synthesis of (2S,4S)-4-(4,4-difluoro-1-piperidyl)-1-(9H-fluoren-9-ylmethoxycarbonyl)pyrrolidine-2-carboxylic acid (Compound aa133, Fmoc-cisPro(pip-4-F2)-OH)

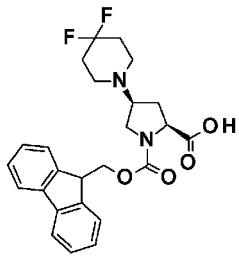

(2S,4S)-4-(4,4-Difluoro-1-piperidyl)pyrrolidine-2-carboxylic acid (Compound aa132, H-cisPro(pip-4-F2)-OH) (45 g, 192.10 mmol), N-(9-fluorenylmethoxycarbonyloxy)succinimide (Fmoc-OSu) (67.4 g, 199.80 mmol), and potassium carbonate (52.4 g, 379.13 mmol) were added to 1,4-dioxane (300 mL) and water (400 mL), and the mixture was stirred at room temperature for 16 h. The reaction solution was then washed with a 25% solution of t-butyl methyl ether in hexane, and the aqueous layer was adjusted to pH 1-2 by adding a 1 N aqueous hydrochloric acid solution. The aqueous layer was extracted with t-butyl methyl ether three times, the organic layers were washed with brine, then dried over anhydrous sodium sulfate, filtered, and concentrated under reduced pressure. The resulting residue was purified by reverse phase column chromatography (5 to 65% water/acetonitrile) to afford (2S,4S)-4-(4,4-difluoro-1-piperidyl)-1-(9H-fluoren-9-ylmethoxycarbonyl)pyrrolidine-2-carboxylic acid (Compound aa133, Fmoc-cisPro(pip-4-F2)-OH) (5.5 g, 17% over four steps).
LCMS (ESI) m/z = 457 (M+H)+
Retention time: 1.45 min (analytical condition SMD method 34)

Synthesis of (2S,3R)-2-amino-3-hydroxy-N-((1R,2R)-2-hydroxy-1,2-diphenylethyl)-N-methylheptanamide (Compound aa134)

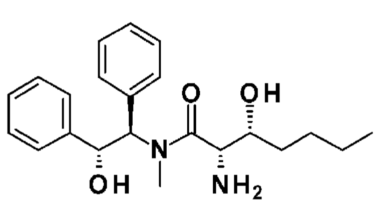

Lithium chloride (165 mg, 3.9 mmol) was heated with a heat gun for one minute under reduced pressure, and then brought back to room temperature. 2-amino-N-((1R,2R)-2-hydroxy-1,2-diphenylethyl)-N-methylacetamide (185 mg, 0.65 mmol) synthesized based on a known article (Angew. Chem. Int. Ed., 2014, 53, 4642) was added under a nitrogen atmosphere, 3.25 mL of tetrahydrofuran was added, and the mixture was stirred. The reaction solution was cooled at -78°C, a 1 M solution of lithium bis(trimethylsilyl)amide in tetrahydrofuran (1.25 mL, 1.25 mmol) was added dropwise, the mixture was stirred for 5 min, and the reaction solution was then further stirred at 4°C for 25 min. The reaction solution was cooled again to -78°C, a 1 M solution of pentanal in tetrahydrofuran (0.5 mL, 0.5 mmol) was added dropwise, and the mixture was stirred for 2.5 h. 50 μL of a 50% saturated aqueous ammonium chloride solution was added and the mixture was brought to room temperature. To the resulting reaction solution was added 10 mL of a 50% saturated

aqueous ammonium chloride solution, the mixture was extracted with 15 mL of ethyl acetate twice. The resulting organic layers were dried over anhydrous sodium sulfate, then filtered, and concentrated under reduced pressure. The resulting residue was purified by reverse phase column chromatography (10 mM aqueous ammonium acetate solution/methanol) to afford (2S,3R)-2-amino-3-hydroxy-N-((1R,2R)-2-hydroxy-1,2-diphenylethyl)-N-methylheptanamide (Compound aa134) (107.6 mg, 58%).
LCMS (ESI) m/z = 371 (M+H)+
Retention time: 0.53 min (analytical condition SQDFA05)

Synthesis of sodium (2S,3R)-2-amino-3-hydroxyheptanoate (Compound aa135)

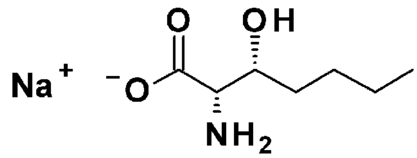

To a solution of (2S,3R)-2-amino-3-hydroxy-N-((1R,2R)-2-hydroxy-1,2-diphenylethyl)-N-methylheptanamide (Compound aa134) obtained by the above synthesis method (3.1 g, 8.37 mmol) in methanol and tetrahydrofuran (1/1, 34 mL) was added 8.4 mL of a 1 N aqueous sodium hydroxide solution, and the mixture was stirred at room temperature for 7 h. The reaction solution was concentrated under reduced pressure, water (40 mL) was added to the resulting residue, and the mixture was washed with 40 mL of dichloromethane three times. The resulting organic layer was extracted with water once, after which the combined aqueous layers were lyophilized to afford (2S,3R)-2-amino-3-hydroxyheptanoic acid sodium salt (Compound aa135) (1.437 g, 94%).
LCMS (ESI) m/z = 160 (M-H)-
Retention time: 0.28 min (analytical condition SQDAA05)

Synthesis of (2S,3R)-2-((((9H-yl)methoxy)carbonyl)amino)-3-hydroxyheptanoic acid (Compound aa136, Fmoc-Ahp(2)(3-R-OH)-OH)

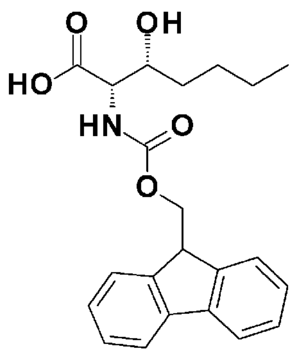

To a solution of N-(9-fluorenylmethoxycarbonyloxy)succinimide (Fmoc-OSu) (2.081 g, 6.17 mmol) in water and 1,4-dioxane (1/1, 31 mL) was added cesium carbonate (4.02 g, 12.34 mmol), followed by stirring. (2S,3R)-2-amino-3-hydroxyheptanoic acid sodium salt (Compound aa135) obtained by the above synthesis method (1.13 g, 6.17 mmol) was added and the mixture was stirred for 45 min. The reaction solution was washed with diethyl ether (10 mL) twice, and the resulting aqueous layer was adjusted to pH 2 with a 5 N aqueous hydrochloric acid solution. The resulting aqueous layer was extracted with dichloromethane (10 mL) three times, and the combined organic layers were dried over anhydrous sodium sulfate, filtered, and concentrated under reduced pressure to afford a crude product of (2S,3R)-2-((((9H-fluoren-9-yl)methoxy)carbonyl)amino)-3-hydroxyheptanoic acid (Compound aa136, Fmoc-Ahp(2)(3-R-OH)-OH) (2.46 g).
LCMS (ESI) m/z = 384 (M+H)+
Retention time: 0.82 min (analytical condition SQDFA05)

Synthesis of (2S,3R)-2-((((9H-fluoren-9-yl)methoxy)carbonyl)amino)-3-((tetrahydro-2H-pyran-2-yl)oxy)heptanoic acid (Compound aa137, Fmoc-Ahp(2)(3-R-OTHP)-OH)

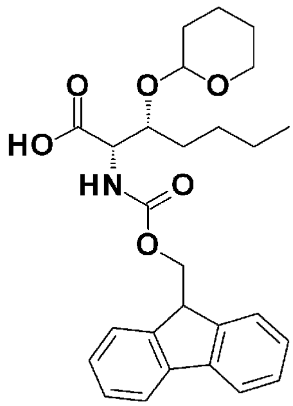

A solution of (2S,3R)-2-((((9H-fluoren-9-yl)methoxy)carbonyl)amino)-3-hydroxyheptanoic acid (Compound aa136, Fmoc-Ahp(2)(3-R-OH)-OH) (2.4 g, 6.26 mmol) and pyridinium p-toluenesulfonate (79 mg, 0.313 mmol) in toluene (12.5 mL) was concentrated under reduced pressure and azeotropically dehydrated. The resulting residue was dissolved in tetrahydrofuran (12.5 mL), dihydropyran (4.01 mL, 43.8 mmol) was added, and the mixture was stirred at 50°C for 2 h. The reaction solution was brought to room temperature, ethyl acetate (12 mL) was added to the reaction solution, and the organic layer was washed with brine (12 mL). The resulting aqueous layer was extracted with ethyl acetate (12 mL), the combined organic layers were washed with brine twice, and the resulting organic layer were dried over anhydrous sodium sulfate, filtered, and concentrated under reduced pressure. The resulting residue was dissolved in tetrahydrofuran (25 mL), phosphate buffer adjusted to pH 8 (25 mL) was added, and the mixture was stirred at 50°C for 4 h. The reaction solution was extracted with ethyl acetate twice at room temperature, and the combined organic layers were washed with brine twice, dried over anhydrous sodium sulfate, filtered, and concentrated under reduced pressure. The resulting residue was purified by reverse phase column chromatography (0.1% aqueous formic acid solution/0.1% formic acid-acetonitrile solution) to afford (2S,3R)-2-((((9H-fluoren-9-yl)methoxy)carbonyl)amino)-3-((tetrahydro-2H-pyran-2-yl)oxy)heptanoic acid (Compound aa137, Fmoc-Ahp(2)(3-R-OTHP)-OH) (2.15 g, 74%).
LCMS (ESI) m/z = 466 (M-H)-
Retention time: 1.00 min (analytical condition SQDFA05)

Synthesis of methyl N-((benzyloxy)carbonyl)-O-(2-(benzyloxy)ethyl)-L-serinate (Compound aa138, Cbz-Ser(EtO-Bn)-OMe)

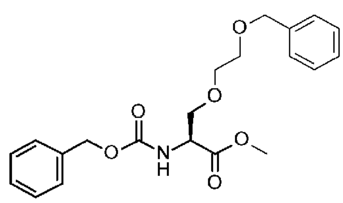

1-Benzyl 2-methyl (S)-aziridine-1,2-dicarboxylate (Cbz-Azy-OMe) (25.0 g, 106 mmol) and 2-(benzyloxy)ethan-1-ol (23.8 g, 156 mmol) were dissolved in dichloromethane (100 mL) under a nitrogen atmosphere. After cooling to 0°C, boron trifluoride-diethyl ether complex (2.00 mL, 15.9 mmol) was added and the mixture was stirred at 0°C for 1 h. Water was added to the reaction solution, the mixture was extracted with dichloromethane twice, and the organic layers were then washed with an aqueous sodium carbonate solution and brine. The organic layers were dried over anhydrous sodium sulfate, the solvent was evaporated under reduced pressure, and the residue was further dried using a pump. The resulting residue was purified by reverse phase column chromatography (water/acetonitrile) to afford methyl N-((benzyloxy)carbonyl)-O-(2-(benzyloxy)ethyl)-L-serinate (Compound aa138, Cbz-Ser(EtOBn)-OMe) (30.0 g, 73%).
LCMS (ESI) m/z = 388 (M+H)+

Retention time: 1.13 min (analytical condition SMD method 9)

Synthesis of N-((benzyloxy)carbonyl)-O-(2-(benzyloxy)ethyl)-L-serine (Compound aa139, Cbz-Ser(EtOBn)-OH)

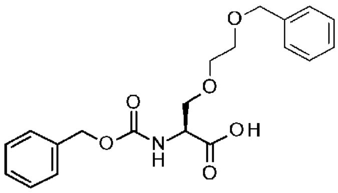

Lithium hydroxide monohydrate (13.9 g, 331 mmol) and calcium chloride (129 g, 1.24 mol) were dissolved in water (321 mL) under a nitrogen atmosphere. A solution of methyl N-((benzyloxy)carbonyl)-O-(2-(benzyloxy)ethyl)-L-serinate (Compound aa138, Cbz-Ser(EtOBn)-OMe) (30.0 g, 77.4 mmol) in 2-propanol/tetrahydrofuran (1.28 L/321 mL) was added thereto at room temperature, and the mixture was stirred for 3 h. A 2 M aqueous hydrochloric acid solution was added until pH 2, the organic layer was removed, and the aqueous layer was extracted with ethyl acetate three times. The resulting organic layers were combined and dried over anhydrous sodium sulfate, the solvent was evaporated under reduced pressure, and the residue was further dried using a pump to afford N-((benzyloxy)carbonyl)-O-(2-(benzyloxy)ethyl)-L-serine (Compound aa139, Cbz-Ser(EtOBn)-OH) (30.0 g) as a crude product. This was used in the next step without purification.

Synthesis of O-(2-hydroxyethyl)-L-serine (Compound aa140, H-Ser(EtOH)-OH)

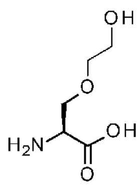

N-((Benzyloxy)carbonyl)-O-(2-(benzyloxy)ethyl)-L-serine (Compound aa139, Cbz-Ser(EtOBn)-OH) (34.0 g, 91.1 mmol) and palladium on carbon (6.80 g, 20% w/w) were dissolved in methanol (500 mL) under a hydrogen atmosphere, and the reaction solution was stirred at room temperature for 16 h. The reaction solution was filtered, the solvent was then evaporated under reduced pressure, and the residue was further dried using a pump to afford O-(2-hydroxyethyl)-L-serine (Compound aa140, H-Ser(EtOH)-OH) (10.7 g) as a crude product. This was used in the next step without purification.

Synthesis of N-(((9H-fluoren-9-yl)methoxy)carbonyl)-O-(2-hydroxyethyl)-L-serine (Compound aa141, Fmoc-Ser(EtOH)-OH)

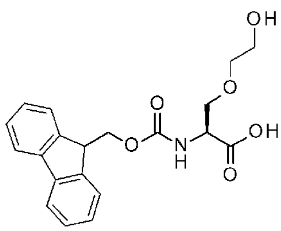

O-(2-Hydroxyethyl)-L-serine (Compound aa140, H-Ser(EtOH)-OH) (5.00 g, 33.5 mmol), N-(9-fluorenylmethoxycarbonyloxy)succinimide (Fmoc-OSu) (12.4 g, 36.9 mmol), and sodium carbonate (10.6 g, 100 mmol) were dissolved

in water/1,4-dioxane (136 mL/56.0 mL) under a nitrogen atmosphere, and the reaction solution was stirred at room temperature for 3 h. After washing the reaction solution with t-butyl methyl ether three times, a 2 M aqueous hydrochloric acid solution was added to the aqueous layer until pH 2, and the aqueous layer was extracted with ethyl acetate three times. The resulting organic layers were combined, washed with brine, and then dried over anhydrous sodium sulfate, the solvent was evaporated under reduced pressure, and the residue was further dried using a pump. The resulting residue was purified by reverse phase column chromatography (0.1% aqueous formic acid solution/0.1 % formic acid-acetonitrile solution) to afford N-(((9H-fluoren-9-yl)methoxy)carbonyl)-O-(2-hydroxyethyl)-L-serine (Compound aa141, Fmoc-Ser(EtOH)-OH) (12.0 g, 96%).
LCMS (ESI) m/z = 372 (M+H)+
Retention time: 1.31 min (analytical condition SMD method 33)

Synthesis of N-(((9H-fluoren-9-yl)methoxy)carbonyl)-O-(2-((tetrahydro-2H-pyran-2-yl)oxy)ethyl)-L-serine (Compound aa142, Fmoc-Ser(EtOTHP)-OH)

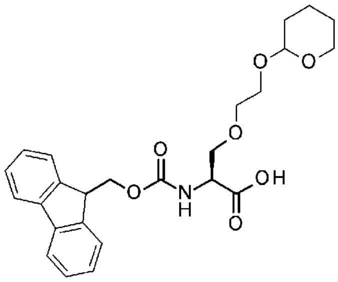

To a solution of N-(((9H-fluoren-9-yl)methoxy)carbonyl)-O-(2-hydroxyethyl)-L-serine (Compound aa141, Fmoc-Ser(EtOH)-OH) (1.6 g, 4.31 mmol) and 3,4-dihydro-2H-pyran (2.728 mL, 30.3 mmol) in tetrahydrofuran (8.616 mL) was added pyridinium p-toluenesulfonate (PPTS) (54.1 mg, 0.215 mmol) under a nitrogen atmosphere, and the mixture was stirred at 50°C for 2 h. The mixture was cooled, and ethyl acetate was then added. The organic layer was then washed with brine three times and dried over anhydrous sodium sulfate, and the solvent was evaporated under reduced pressure. The resulting residue was dissolved in tetrahydrofuran (10 mL), followed by addition of 0.05 M phosphate buffer (100 mL) (prepared by mixing a 1 M aqueous sodium dihydrogenphosphate ($NaH_2PO_4$) solution (94.3 mL) with a 1 M aqueous disodium hydrogenphosphate ($Na_2HPO_4$) solution (5.7 mL)). This mixture was stirred at room temperature for 10 minutes and then extracted with t-butyl methyl ether, the organic layer was washed with brine and dried over anhydrous sodium sulfate, and the solvent was evaporated under reduced pressure. The resulting residue was purified by reverse phase column chromatography (water/acetonitrile) to afford N-(((9H-fluoren-9-yl)methoxy)carbonyl)-O-(2-((tetrahydro-2H-pyran-2-yl)oxy)ethyl)-L-serine (Compound aa142, Fmoc-Ser(EtOTHP)-OH) (1.75 g, 96%).
LCMS (ESI) m/z = 456 (M+H)+
Retention time: 0.81 min (analytical condition SQDFA05)

Synthesis of benzyl (S)-4-((2-(benzyloxy)ethoxy)methyl)-5-oxooxazolidine-3-carboxylate (Compound aa143)

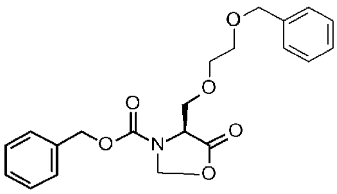

N-((Benzyloxy)carbonyl)-O-(2-(benzyloxy)ethyl)-L-serine (Compound aa139, Cbz-Ser(EtOBn)-OH) (30.0 g, 80.3 mmol), paraformaldehyde (7.20 g), and p-toluenesulfonic acid (0.83 g, 4.82 mmol) were dissolved in toluene (300 mL), and the reaction solution was stirred at 110°C for 16 h. After cooling to room temperature, the reaction solution was washed with an aqueous sodium bicarbonate solution, the organic layers were combined and dried over anhydrous

sodium sulfate, the solvent was evaporated under reduced pressure, and the residue was further dried using a pump to afford benzyl (S)-4-((2-(benzyloxy)ethoxy)methyl)-5-oxooxazolidine-3-carboxylate (Compound aa143) (27.0 g) as a crude product. This was used in the next step without purification.

Synthesis of N-((benzyloxy)carbonyl)-O-(2-(benzyloxy)ethyl)-N-methyl-L-serine (Compound aa144, Cbz-MeSer(EtO-Bn)-OH)

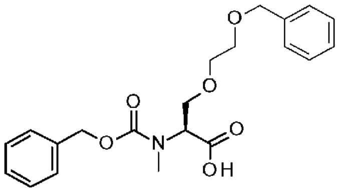

To a solution of benzyl (S)-4-((2-(benzyloxy)ethoxy)methyl)-5-oxooxazolidine-3-carboxylate (Compound aa143) (27.0 g, 70.1 mmol) in dichloromethane (450 mL) were added triethylsilane (24.4 g, 210 mmol) and trifluoroacetic acid (450 mL) under a nitrogen atmosphere, and the mixture was stirred at room temperature for 48 h. The solvent was evaporated from the reaction solution under reduced pressure, and the residue was further dried using a pump and then re-dissolved in an aqueous potassium carbonate solution. The reaction solution was washed with diethyl ether, concentrated hydrochloric acid was added until pH 2, and the mixture was then extracted with ethyl acetate twice. The resulting organic layers were combined and dried over anhydrous sodium sulfate, the solvent was evaporated under reduced pressure, and the residue was further dried using a pump to afford N-((benzyloxy)carbonyl)-O-(2-(benzyloxy)ethyl)-N-methyl-L-serine (Compound aa144, Cbz-MeSer(EtOBn)-OH) (24.0 g) as a crude product. This was used in the next step without purification.

Synthesis of O-(2-hydroxyethyl)-N-methyl-L-serine (Compound aa145, H-MeSer(EtOH)-OH)

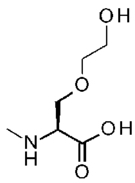

N-((Benzyloxy)carbonyl)-O-(2-(benzyloxy)ethyl)-N-methyl-L-serine (Compound aa144, Cbz-MeSer(EtOBn)-OH) (24.0 g, 62.0 mmol) and palladium on carbon (2.40 g, 10% w/w) were dissolved in methanol (400 mL) under a hydrogen atmosphere, and the reaction solution was stirred at room temperature for 16 h. The reaction solution was filtered, the solvent was then evaporated under reduced pressure, and the residue was further dried using a pump to afford O-(2-hydroxyethyl)-N-methyl-L-serine (Compound aa145, H-MeSer(EtOH)-OH) (12.0 g) as a crude product. This was used in the next step without purification.

Synthesis of N-(((9H-fluoren-9-yl)methoxy)carbonyl)-O-(2-hydroxyethyl)-N-methyl-L-serine (Compound aa146, Fmoc-MeSer(EtOH)-OH)

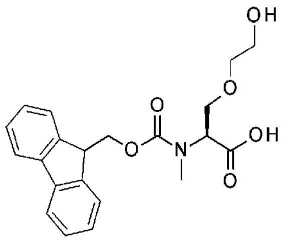

O-(2-Hydroxyethyl)-N-methyl-L-serine (Compound aa145, H-MeSer(EtOH)-OH) (12 g, 73.54 mmol), N-(9-fluorenylmethoxycarbonyloxy)succinimide (Fmoc-OSu) (27.3 g, 81.0 mmol), and sodium carbonate (27.3 g, 258 mmol) were dissolved in water/1,4-dioxane (320 mL/160 mL) under a nitrogen atmosphere, and the reaction solution was then stirred at room temperature for 3 h. After washing the reaction solution with t-butyl methyl ether three times, a 2 M aqueous hydrochloric acid solution was added to the aqueous layer until pH 2, and the aqueous layer was extracted with ethyl acetate three times. The resulting organic layers were combined, washed with brine, and then dried over anhydrous sodium sulfate, the solvent was evaporated under reduced pressure, and the residue was further dried using a pump to afford N-(((9H-fluoren-9-yl)methoxy)carbonyl)-O-(2-hydroxyethyl)-N-methyl-L-serine (Compound aa146, Fmoc-MeSer(EtOH)-OH) (20.0 g) as a crude product. This was used in the next step without purification.

Synthesis of N-(((9H-fluoren-9-yl)methoxy)carbonyl)-N-methyl-O-(2-((tetrahydro-2H-pyran-2-yl)oxy)ethyl)-L-serine (Compound aa147, Fmoc-MeSer(EtOTHP)-OH)

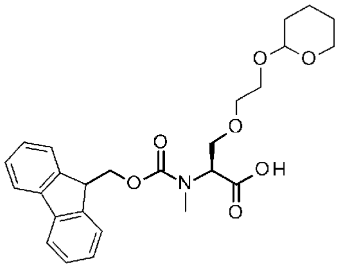

To a solution of N-(((9H-fluoren-9-yl)methoxy)carbonyl)-O-(2-hydroxyethyl)-N-methyl-L-serine (Compound aa146, Fmoc-MeSer(EtOH)-OH) (18.0 g, 46.7 mmol) in tetrahydrofuran (100 mL) were added pyridinium p-toluenesulfonate (0.59 g, 2.34 mmol) and 3,4-dihydro-2H-pyran (27.5 g, 327 mmol) under a nitrogen atmosphere, and the mixture was stirred at 50°C for 3 h. The mixture was cooled to 25°C and ethyl acetate was added. The organic layer was then washed with brine and dried over anhydrous sodium sulfate, and the solvent was evaporated under reduced pressure. 25.0 g of the resulting residue (31 g) was dissolved in tetrahydrofuran (200 mL), followed by addition of 1.0 M phosphate buffer adjusted to pH 6.8 (200 mL). This mixture was stirred at 50°C for 3 h. After cooling to 25°C, ethyl acetate was added, and the organic layer and the aqueous layer were separated. The aqueous layer was extracted with ethyl acetate, after which all the resulting organic layers were combined, washed with brine, and then dried over anhydrous sodium sulfate. The solvent was evaporated under reduced pressure, and the residue was further dried using a pump to afford N-(((9H-fluoren-9-yl)methoxy)carbonyl)-N-methyl-O-(2-((tetrahydro-2H-pyran-2-yl)oxy)ethyl)-L-serine (Compound aa147, Fmoc-MeSer(EtOTHP)-OH) (20.0 g).
LCMS (ESI) m/z = 487 $(M+NH_4)+$
Retention time: 0.68 min (analytical condition SMD method 11)

Synthesis of (4S)-4-methyl-2-phenyl-1,3-dioxolane (Compound aa148)

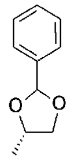

To a solution of (S)-propane-1,2-diol (20.0 g, 263 mmol) in toluene (200 mL) were added benzaldehyde (29.3 g, 276 mmol) and p-toluenesulfonic acid (0.45 g, 2.61 mmol) under a nitrogen atmosphere, and the mixture was stirred at 130°C for 16 h. The reaction solution was concentrated and the residue was purified by normal phase column chromatography (hexane/ethyl acetate) to afford (4S)-4-methyl-2-phenyl-1,3-dioxolane (Compound aa148) (31.5 g, 73%).
LCMS (ESI) m/z = 165 (M+H)+
Retention time: 0.89 min (analytical condition SMD method 12)

Synthesis of (2S)-2-(benzyloxy)propan-1-ol (Compound aa149)

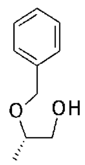

To a solution of (4S)-4-methyl-2-phenyl-1,3-dioxolane (Compound aa148) (32 g, 194.88 mmol) in dichloromethane (1.00 L) was added a 1 M solution of diisobutylaluminum hydride in hexane (390 mL, 390 mmol) at -50°C under a nitrogen atmosphere, and the mixture was stirred at the same temperature for 15 min and then stirred at room temperature for 2 h. The reaction was quenched by adding a saturated aqueous ammonium chloride solution, and the mixture was then extracted with dichloromethane twice. The combined organic layers were washed with brine and dried over anhydrous sodium sulfate, the solvent was evaporated under reduced pressure, and the residue was further dried using a pump to afford (2S)-2-(benzyloxy)propan-1-ol (Compound aa149) (35.5 g) as a crude product. This was used in the next step without purification.

Synthesis of methyl N-((benzyloxy)carbonyl)-O-((S)-2-(benzyloxy)propyl)-L-serinate (Compound aa150, Cbz-Ser(S-2-PrOBn)-OMe)

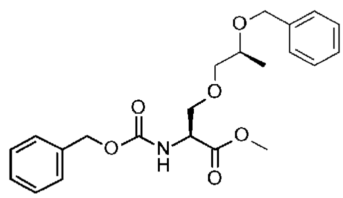

1-Benzyl 2-methyl (S)-aziridine-1,2-dicarboxylate (Cbz-Azy-OMe) (25.0 g, 106 mmol) and (2S)-2-(benzyloxy)propan-1-ol (Compound aa149) (26.5 g, 159 mmol) were dissolved in dichloromethane (188 mL) under a nitrogen atmosphere. After cooling to 0°C, boron trifluoride-diethyl ether complex (2.75 mL) was added and the mixture was stirred at 0°C for 1 hour and 30 minutes. Water was added to the reaction solution, the mixture was extracted with dichloromethane, and the organic layer was then washed with a saturated aqueous sodium carbonate solution and brine. The organic layers were dried over anhydrous sodium sulfate, the solvent was evaporated under reduced pressure, and the residue was further dried using a pump to afford methyl N-((benzyloxy)carbonyl)-O-((S)-2-(benzyloxy)propyl)-L-serinate (Compound aa150, Cbz-Ser(S-2-PrOH)-OMe) (Compound aa150, Cbz-Ser(S-2-PrOBn)-OMe) (30.0 g) as a crude product. This was used in the next step without purification.

Synthesis of N-((benzyloxy)carbonyl)-O-((S)-2-(benzyloxy)propyl)-L-serine (Compound aa151, Cbz-Ser(S-2-PrOBn)-OH)

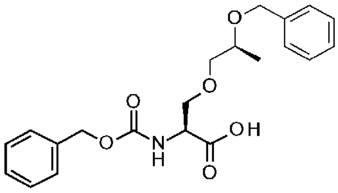

Lithium hydroxide monohydrate (10.5 g) and calcium chloride (103 g) were dissolved in water (300 mL) under a nitrogen atmosphere. A solution of methyl N-((benzyloxy)carbonyl)-O-((S)-2-(benzyloxy)propyl)-L-serinate (Compound aa150, Cbz-Ser(S-2-PrOH)-OMe) (25 g, 62.3 mmol) in 2-propanol/tetrahydrofuran (1.20 L/300 mL) was added thereto at room temperature, and the mixture was stirred for 5 h. A 2 M aqueous hydrochloric acid solution was added until pH 2, the organic layer was removed, and the aqueous layer was extracted with ethyl acetate three times. The resulting organic layers were combined and dried over anhydrous sodium sulfate, the solvent was evaporated under reduced pressure, and the residue was further dried using a pump to afford N-((benzyloxy)carbonyl)-O-((S)-2-(benzyloxy)propyl)-L-serine (Compound aa151, Cbz-Ser(S-2-PrOBn)-OH) (25.0 g) as a crude product. This was used in the next step without purification.

Synthesis of O-((S)-2-hydroxypropyl)-L-serine (Compound aa152, H-Ser(S-2-PrOH)-OH)

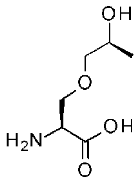

N-((Benzyloxy)carbonyl)-O-((S)-2-(benzyloxy)propyl)-L-serine (Compound aa151, Cbz-Ser(S-2-PrOBn)-OH) (3.00 g, 7.74 mmol) and palladium on carbon (0.30 g, 10% w/w) were dissolved in methanol (50.0 mL) under a hydrogen atmosphere, and the mixture was stirred at room temperature for 16 h. After filtering the reaction solution, the solvent was evaporated under reduced pressure and the residue was further dried using a pump to afford O-((S)-2-hydroxypropyl)-L-serine (Compound aa152, H-Ser(S-2-PrOH)-OH) (0.78 g) as a crude product. This was used in the next step without purification.

Synthesis of N-(((9H-fluoren-9-yl)methoxy)carbonyl)-O-((S)-2-hydroxypropyl)-L-serine (Compound aa153, Fmoc-Ser(S-2-PrOH)-OH)

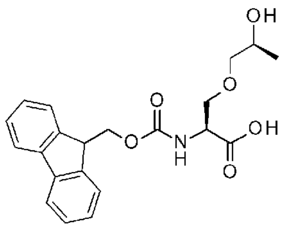

O-((S)-2-Hydroxypropyl)-L-serine (Compound aa152, H-Ser(S-2-PrOH)-OH) (0.78 g, 4.78 mmol), N-(9-fluorenylmethoxycarbonyloxy)succinimide (Fmoc-OSu) (1.61 g, 4.78 mmol), and N-ethyl-isopropylpropan-2-amine (DIPEA) (0.93 mg, 7.17 mmol) were dissolved in water/1,4-dioxane (8.00 mL/22.0 mL) under a nitrogen atmosphere, and the

mixture was stirred at room temperature for 30 min. The reaction solution was washed with hexane (18.0 mL)/t-butyl methyl ether (6.00 mL) twice, a 2 M aqueous hydrochloric acid solution was then added to the aqueous layer until pH 2, and the aqueous layer was extracted with ethyl acetate (20.0 mL) twice. The resulting organic layers were combined, washed with brine, and then dried over anhydrous sodium sulfate, the solvent was evaporated under reduced pressure, and the residue was further dried using a pump. The resulting residue was purified by reverse phase column chromatography (0.1% aqueous formic acid solution/0.1% formic acid-acetonitrile solution) to afford N-(((9H-fluoren-9-yl)methoxy)carbonyl)-O-((S)-2-hydroxypropyl)-L-serine (Compound aa153, Fmoc-Ser(S-2-PrOH)-OH) (1.62 g, 88%).
LCMS (ESI) m/z = 386 (M+H)+
Retention time: 0.68 min (analytical condition SQDFA05)

Synthesis of N-(((9H-fluoren-9-yl)methoxy)carbonyl)-O-((2S)-2-((tetrahydro-2H-pyran-2-yl)oxy)propyl)-L-serine (Compound aa154, Fmoc-Ser(S-2-PrOTHP)-OH)

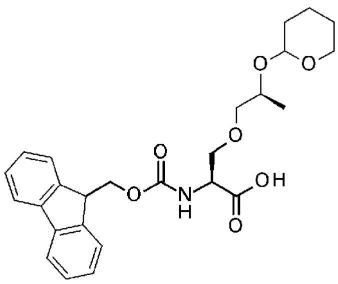

To a solution of N-(((9H-fluoren-9-yl)methoxy)carbonyl)-O-((S)-2-hydroxypropyl)-L-serine (Compound aa153, Fmoc-Ser(S-2-PrOH)-OH) (83 mg, 0.22 mmol) in tetrahydrofuran (1.00 mL) were added pyridinium p-toluenesulfonate (2.71 mg, 0.01 mmol) and 3,4-dihydro-2H-pyran (0.13 g, 1.51 mmol) under a nitrogen atmosphere, and the mixture was stirred at 50°C for 2 h. The mixture was cooled to 25°C and ethyl acetate was added. The organic layer was then washed with brine and dried over anhydrous sodium sulfate, and the solvent was evaporated under reduced pressure. The resulting residue was dissolved in tetrahydrofuran (1.00 mL), followed by addition of 1.0 M phosphate buffer adjusted to pH 6.8 (1.00 mL). This mixture was stirred at 50°C for 3 h. After cooling to 25°C, ethyl acetate (175 mL) was added and the organic layer and the aqueous layer were separated. The aqueous layer was extracted with ethyl acetate (175 mL), after which all the resulting organic layers were combined, washed with brine, and then dried over anhydrous sodium sulfate. The solvent was evaporated under reduced pressure, and the residue was further dried using a pump to afford N-(((9H-fluoren-9-yl)methoxy)carbonyl)-O-((2S)-2-((tetrahydro-2H-pyran-2-yl)oxy)propyl)-L-serine (Compound aa154, Fmoc-Ser(S-2-PrOTHP)-OH) (88.0 mg, 87%).
LCMS (ESI) m/z = 468 (M-H)-
Retention time: 0.86 min, 0.87 min (analytical condition SQDFA05)

Synthesis of benzyl (S)-4-(((S)-2-(benzyloxy)propoxy)methyl)-5-oxooxazolidine-3-carboxylate (Compound aa155)

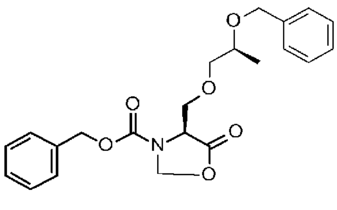

N-((Benzyloxy)carbonyl)-O-((S)-2-(benzyloxy)propyl)-L-serine (Compound aa151, Cbz-Ser(S-2-PrOBn)-OH) (25.0 g, 64.53 mmol), paraformaldehyde (5.80 g), and p-toluenesulfonic acid (0.67 g, 3.89 mmol) were dissolved in toluene (250 mL), and the reaction solution was stirred at 110°C for 16 h. After cooling to room temperature, the reaction solution was washed with an aqueous sodium bicarbonate solution, the organic layers were combined and dried over anhydrous sodium sulfate, the solvent was evaporated under reduced pressure, and the residue was further dried using a pump to afford benzyl (S)-4-(((S)-2-(benzyloxy)propoxy)methyl)-5-oxooxazolidine-3-carboxylate (Compound aa155)

(18.6 g) as a crude product. This was used in the next step without purification.

Synthesis of N-((benzyloxy)carbonyl)-O-((S)-2-(benzyloxy)propyl)-N-methyl-L-serine (Compound aa156, Cbz-MeSer(S-2-PrOBn)-OH)

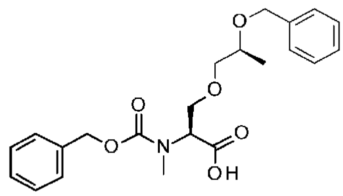

To a solution of benzyl (S)-4-(((S)-2-(benzyloxy)propoxy)methyl)-5-oxooxazolidine-3-carboxylate (Compound aa155) (18.6 g, 46.6 mmol) in dichloromethane (296 mL) were added triethylsilane (16.2 g, 139 mmol) and trifluoroacetic acid (296 mL) under a nitrogen atmosphere, and the mixture was stirred at room temperature for 48 h. The solvent was evaporated from the reaction solution under reduced pressure, and the residue was further dried using a pump and then re-dissolved in an aqueous potassium carbonate solution. The reaction solution was washed with diethyl ether, concentrated hydrochloric acid was added until pH 2, and the mixture was then extracted with ethyl acetate three times. The resulting organic layers were combined and dried over anhydrous sodium sulfate, the solvent was evaporated under reduced pressure, and the residue was further dried using a pump to afford N-((benzyloxy)carbonyl)-O-((S)-2-(benzyloxy)propyl)-N-methyl-L-serine (Compound aa156, Cbz-MeSer(S-2-PrOBn)-OH) (9.4 g) as a crude product. This was used in the next step without purification.

Synthesis of O-((S)-2-hydroxypropyl)-N-methyl-L-serine (Compound aa157, H-MeSer(S-2-PrOH)-OH)

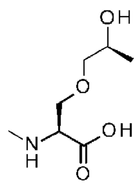

N-((Benzyloxy)carbonyl)-O-((S)-2-(benzyloxy)propyl)-N-methyl-L-serine (Compound aa156, Cbz-MeSer(S-2-PrOBn)-OH) (9.40 g, 23.4 mmol) and palladium on carbon (1.80 g, 10% w/w) were dissolved in methanol (185 mL) under a hydrogen atmosphere, and the reaction solution was stirred at room temperature for 16 h. The reaction solution was filtered, the solvent was then evaporated under reduced pressure, and the residue was further dried using a pump to afford O-((S)-2-hydroxypropyl)-N-methyl-L-serine (Compound aa157, H-MeSer(S-2-PrOH)-OH) (3.40 g) as a crude product. This was used in the next step without purification.

Synthesis of N-(((9H-fluoren-9-yl)methoxy)carbonyl)-O-((S)-2-hydroxypropyl)-N-methyl-L-serine (Compound aa158, Fmoc-MeSer(S-2-PrOH)-OH)

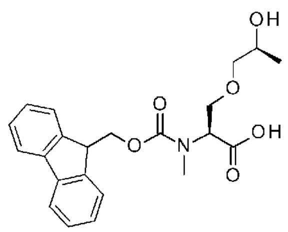

O-((S)-2-Hydroxypropyl)-N-methyl-L-serine (Compound aa157, H-MeSer(S-2-PrOH)-OH) (3.40 g, 19.2 mmol), N-(9-fluorenylmethoxycarbonyloxy)succinimide (Fmoc-OSu) (7.12 g, 22.1 mmol), and sodium carbonate (7.13 g, 67.3 mmol) were dissolved in water/1,4-dioxane (80.0 mL/40.0 mL) under a nitrogen atmosphere, and the reaction solution was stirred at room temperature for 3 h. After washing the reaction solution with t-butyl methyl ether three times, a 2 M aqueous hydrochloric acid solution was added to the aqueous layer until pH 2, and the aqueous layer was extracted with ethyl acetate three times. The resulting organic layers were combined, washed with brine, and then dried over anhydrous sodium sulfate, the solvent was evaporated under reduced pressure, and the residue was further dried using a pump to afford N-(((9H-fluoren-9-yl)methoxy)carbonyl)-O-((S)-2-hydroxypropyl)-N-methyl-L-serine (Compound aa158, Fmoc-MeSer(S-2-PrOH)-OH) (8.00 g) as a crude product. This was used in the next step without purification.

Synthesis of N-(((9H-fluoren-9-yl)methoxy)carbonyl)-N-methyl-O-((2S)-2-((tetrahydro-2H-pyran-2-yl)oxy)propyl)-L-serine (Compound aa159, Fmoc-MeSer(S-2-PrOTHP)-OH)

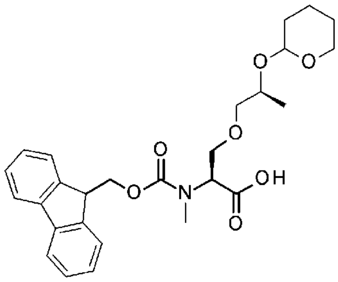

To a solution of N-(((9H-fluoren-9-yl)methoxy)carbonyl)-O-((S)-2-hydroxypropyl)-N-methyl-L-serine (Compound aa158, Fmoc-MeSer(S-2-PrOH)-OH) (80.0 g, 20.0 mmol) in tetrahydrofuran (40.0 mL) were added pyridinium p-toluenesulfonate (0.26 g, 1.02 mmol) and 3,4-dihydro-2H-pyran (11.8 g, 140 mmol) under a nitrogen atmosphere, and the mixture was stirred at 50°C for 2 h. The mixture was cooled to 25°C and ethyl acetate was added. The organic layer was then washed with brine and dried over anhydrous sodium sulfate, and the solvent was evaporated under reduced pressure. The resulting residue (9.30 g) was dissolved in tetrahydrofuran (50.0 mL), followed by addition of 1.0 M phosphate buffer adjusted to pH 6.8 (50.0 mL). This mixture was stirred at 50°C for 3 h. After cooling to 25°C, ethyl acetate was added and the organic layer and the aqueous layer were separated. The aqueous layer was extracted with ethyl acetate, after which all the resulting organic layers were combined, washed with brine, and then dried over anhydrous sodium sulfate. The solvent was evaporated under reduced pressure, and the residue was further dried using a pump. The resulting residue was purified by reverse phase column chromatography (0.5% aqueous ammonium bicarbonate solution/acetonitrile) to afford N-(((9H-fluoren-9-yl)methoxy)carbonyl)-N-methyl-O-((2S)-2-((tetrahydro-2H-pyran-2-yl)oxy)propyl)-L-serine (Compound aa159, Fmoc-MeSer(S-2-PrOTHP)-OH) (7.00 g, 72%).
LCMS (ESI) m/z = 501 $(M+NH_4)+$
Retention time: 0.82 min (analytical condition SMD method 10)

Synthesis of (4R)-4-methyl-2-phenyl-1,3-dioxolane (Compound aa160)

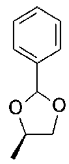

To a solution of (R)-propane-1,2-diol (20 g, 262.83 mmol) in toluene (200 mL) were added benzaldehyde (29.3 g, 276 mmol) and p-toluenesulfonic acid (0.45 g, 2.61 mmol) under a nitrogen atmosphere, and the mixture was stirred at 130°C for 16 h. The reaction solution was concentrated and the residue was purified by normal phase column chromatography (hexane/ethyl acetate) to afford (4R)-4-methyl-2-phenyl-1,3-dioxolane (Compound aa160) (31.5 g, 73%).
LCMS (ESI) m/z = 163 (M-H)-

Retention time: 7.21 min (analytical condition GC01)

Synthesis of (2R)-2-(benzyloxy)propan-1-ol (Compound aa161)

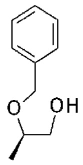

To a solution of (4R)-4-methyl-2-phenyl-1,3-dioxolane (Compound aa160) (32.0 g, 195 mmol) in dichloromethane (1.00 L) was added a 1 M solution of diisobutylaluminum hydride in hexane (390 mL, 390 mmol) at -50°C under a nitrogen atmosphere, and the mixture was stirred at the same temperature for 15 min and then stirred at room temperature for 2 h. The reaction was quenched by adding a saturated aqueous ammonium chloride solution, and the mixture was then extracted with dichloromethane twice. The combined organic layers were washed with brine and dried over anhydrous sodium sulfate, the solvent was evaporated under reduced pressure, and the residue was further dried using a pump to afford (2R)-2-(benzyloxy)propan-1-ol (Compound aa161) (35.5 g) as a crude product. This was used in the next step without purification.

Synthesis of methyl N-((benzyloxy)carbonyl)-O-((R)-2-(benzyloxy)propyl)-L-serinate (Compound aa162, Cbz-Ser(R-2-PrOBn)-OMe)

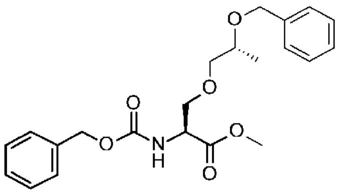

1-Benzyl 2-methyl (S)-aziridine-1,2-dicarboxylate (Cbz-Azy-OMe) (25 g, 106.28 mmol) and (2R)-2-(benzyloxy)propan-1-ol (Compound aa161) (26.5 g, 159 mmol) were dissolved in dichloromethane (188 mL) under a nitrogen atmosphere. After cooling to 0°C, boron trifluoride-diethyl ether complex (2.75 mL) was added and the mixture was stirred at 0°C for 1 hour and 30 minutes. Water was added to the reaction solution, the mixture was extracted with dichloromethane, and the organic layer was then washed with a saturated aqueous sodium carbonate solution and brine. The organic layers were dried over anhydrous sodium sulfate, the solvent was evaporated under reduced pressure, and the residue was further dried using a pump to afford methyl N-((benzyloxy)carbonyl)-O-((R)-2-(benzyloxy)propyl)-L-serinate (Compound aa162, Cbz-Ser(R-2-PrOBn)-OMe) (50.0 g) as a crude product. This was used in the next step without purification.

Synthesis of N-((benzyloxy)carbonyl)-O-((R)-2-(benzyloxy)propyl)-L-serine (Compound aa163, Cbz-Ser(R-2-PrOBn)-OH)

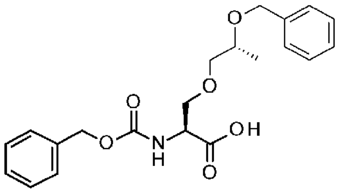

Lithium hydroxide monohydrate (10.5 g) and calcium chloride (103 g) were dissolved in water (300 mL) under a nitrogen atmosphere. A solution of methyl N-((benzyloxy)carbonyl)-O-((R)-2-(benzyloxy)propyl)-L-serinate (Compound aa162, Cbz-Ser(R-2-PrOBn)-OMe) (25.0 g, 62.3 mmol) in 2-propanol/tetrahydrofuran (1.50 L/150 mL) was added thereto at room temperature, and the mixture was stirred for 5 h. A 2 M aqueous hydrochloric acid solution was added until pH 2, the organic layer was removed, and the aqueous layer was extracted with ethyl acetate three times. The resulting organic layers were combined and dried over anhydrous sodium sulfate, the solvent was evaporated under reduced pressure, and the residue was further dried using a pump to afford N-((benzyloxy)carbonyl)-O-((R)-2-(benzyloxy)propyl)-L-serine (Compound aa163, Cbz-Ser(R-2-PrOBn)-OH) (25.0g) as a crude product. This was used in the next step without purification.

Synthesis of O-((R)-2-hydroxypropyl)-L-serine (Compound aa164, H-Ser(R-2-PrOH)-OH)

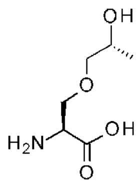

N-((Benzyloxy)carbonyl)-O-((R)-2-(benzyloxy)propyl)-L-serine (Compound aa163, Cbz-Ser(R-2-PrOBn)-OH) (2.87 g, 7.41 mmol) and palladium on carbon (570 mg, 20% w/w) were dissolved in methanol (50 mL) under a hydrogen atmosphere, and the mixture was stirred at room temperature for 16 h. The reaction solution was filtered, the solvent was then evaporated under reduced pressure, and the resulting residue was recrystallized with ethyl acetate at 60°C to afford O-((R)-2-hydroxypropyl)-L-serine (Compound aa164, H-Ser(R-2-PrOH)-OH) (900 mg, 74%).

Synthesis of N-(((9H-fluoren-9-yl)methoxy)carbonyl)-O-((R)-2-hydroxypropyl)-L-serine (Compound aa165, Fmoc-Ser(R-2-PrOH)-OH)

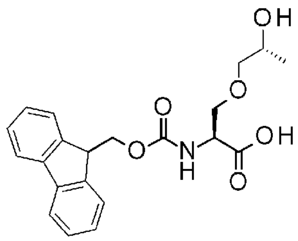

O-((R)-2-Hydroxypropyl)-L-serine (Compound aa164, H-Ser(R-2-PrOH)-OH) synthesized by the method described above (5.0 g, 30.64 mmol) and sodium bicarbonate (7.71 g, 91.8 mmol) were dissolved in water/1,4-dioxane (127 mL/52.9 mL) under a nitrogen atmosphere, N-(9-fluorenylmethoxycarbonyloxy)succinimide (Fmoc-OSu) (10.85 g, 32.2 mmol) was added at room temperature, and the mixture was stirred for 4 h. Water was added to the reaction solution, and the mixture was washed with t-butyl methyl ether. Concentrated hydrochloric acid was then added to the aqueous layer until pH 3, and the aqueous layer was extracted with t-butyl methyl ether twice. The resulting organic layers were washed with brine and then dried over anhydrous sodium sulfate, and the solvent was evaporated under reduced pressure. The resulting residue was purified by reverse phase column chromatography (water/acetonitrile) to afford N-(((9H-fluoren-9-yl)methoxy)carbonyl)-O-((R)-2-hydroxypropyl)-L-serine (Compound aa165, Fmoc-Ser(R-2-PrOH)-OH) (8 g, 68%).
LCMS (ESI) m/z = 386 (M+H)+
Retention time: 2.08 min (analytical condition SMD method 49)

Synthesis of N-(((9H-fluoren-9-yl)methoxy)carbonyl)-O-((2R)-2-((tetrahydro-2H-pyran-2-yl)oxy)propyl)-L-serine (Compound aa166, Fmoc-Ser(R-2-PrOTHP)-OH)

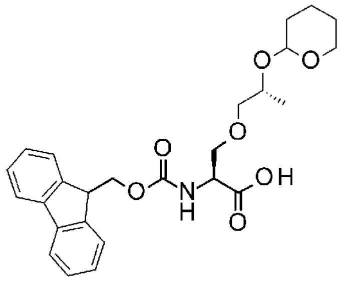

To a solution of N-(((9H-fluoren-9-yl)methoxy)carbonyl)-O-((R)-2-hydroxypropyl)-L-serine (Compound aa165, Fmoc-Ser(R-2-PrOH)-OH) (4.4 g, 11.4 mmol) in tetrahydrofuran (50 mL) were added pyridinium p-toluenesulfonate (143 mg, 5.7 mmol) and 3,4-dihydro-2H-pyran (6.7 g, 79.8 mmol) under a nitrogen atmosphere, and the mixture was stirred at 50°C for 3 h. The mixture was cooled at room temperature, t-butyl methyl ether was added, and the mixture was then washed with brine. The organic layer was dried over anhydrous sodium sulfate and filtered, and the resulting solvent was evaporated under reduced pressure to give a crude product (8 g). To the resulting crude product (800 mg) was added a mixture of tetrahydrofuran (10 mL) and 1.0 M phosphate buffer adjusted to pH 6.8 (10 mL), followed by stirring at 50°C for 3 h. After cooling the reaction solution in room temperature, t-butyl methyl ether was added and the reaction solution was washed with brine and filtered. The resulting solution was dried over anhydrous sodium sulfate, and the solvent was evaporated under reduced pressure. The resulting residue was purified by reverse phase column chromatography (0.5% aqueous ammonium bicarbonate solution/acetonitrile), the resulting fractions were combined, and the acetonitrile was evaporated under reduced pressure. To the resulting aqueous layer was added a 1.0 M phosphate buffer adjusted to pH 6.8. The aqueous layer was extracted with t-butyl methyl ether three times and the solvent was evaporated under reduced pressure to afford N-(((9H-fluoren-9-yl)methoxy)carbonyl)-O-((2R)-2-((tetrahydro-2H-pyran-2-yl)oxy)propyl)-L-serine (Compound aa166, Fmoc-Ser(R-2-PrOTHP)-OH) (400 mg, 65% over two steps).
LCMS (ESI) m/z = 468 (M-H)-
Retention time: 0.85 min, 0.87 min (analytical condition SQDFA05)

Synthesis of benzyl (S)-4-(((R)-2-(benzyloxy)propoxy)methyl)-5-oxooxazolidine-3-carboxylate (Compound aa167)

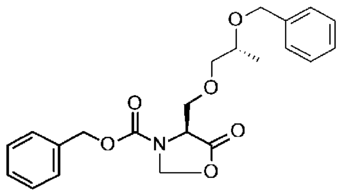

N-((Benzyloxy)carbonyl)-O-((R)-2-(benzyloxy)propyl)-L-serine (Compound aa163, Cbz-Ser(R-2-PrOBn)-OH) (25.0 g, 64.5 mmol), paraformaldehyde (5.80 g), and p-toluenesulfonic acid (0.67 g, 3.89 mmol) were dissolved in toluene (250 mL), and the reaction solution was stirred at 110°C for 16 h. After cooling to room temperature, the reaction solution was washed with an aqueous sodium bicarbonate solution, the organic layers were combined and dried over anhydrous sodium sulfate, the solvent was evaporated under reduced pressure, and the residue was further dried using a pump to afford benzyl (S)-4-(((R)-2-(benzyloxy)propoxy)methyl)-5-oxooxazolidine-3-carboxylate (Compound aa167) (18.6 g) as a crude product. This was used in the next step without purification.

Synthesis of N-((benzyloxy)carbonyl)-O-((R)-2-(benzyloxy)propyl)-N-methyl-L-serine (Compound aa168, Cbz-MeSer(R-2-PrOBn)-OH)

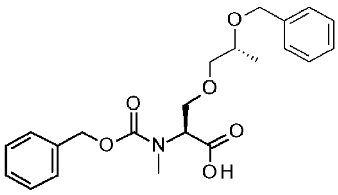

To a solution of benzyl (S)-4-(((R)-2-(benzyloxy)propoxy)methyl)-5-oxooxazolidine-3-carboxylate (Compound aa167) (18.6 g, 46.6 mmol) in dichloromethane (296 mL) were added triethylsilane (16.2 g, 139 mmol) and trifluoroacetic acid (296 mL) under a nitrogen atmosphere, and the mixture was stirred at room temperature for 48 h. The solvent was evaporated from the reaction solution under reduced pressure, and the residue was further dried using a pump and then re-dissolved in an aqueous potassium carbonate solution. The reaction solution was washed with diethyl ether, concentrated hydrochloric acid was added until pH 2, and the mixture was then extracted with ethyl acetate three times. The resulting organic layers were combined and dried over anhydrous sodium sulfate, the solvent was evaporated under reduced pressure, and the residue was further dried using a pump to afford N-((benzyloxy)carbonyl)-O-((R)-2-(benzyloxy)propyl)-N-methyl-L-serine (Compound aa168, Cbz-MeSer(R-2-PrOBn)-OH) (9.40 g) as a crude product. This was used in the next step without purification.

Synthesis of O-((R)-2-hydroxypropyl)-N-methyl-L-serine (Compound aa169, H-Ser(R-2-PrOH)-OH)

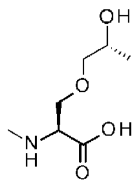

N-((Benzyloxy)carbonyl)-O-((R)-2-(benzyloxy)propyl)-N-methyl-L-serine (Compound aa168, Cbz-MeSer(R-2-PrOBn)-OH) (9.40 g, 23.4 mmol) and palladium on carbon (1.80 g, 10% w/w) were dissolved in methanol (185 mL) under a hydrogen atmosphere, and the reaction solution was stirred at room temperature for 16 h. The reaction solution was filtered, the solvent was then evaporated under reduced pressure, and the residue was further dried using a pump to afford O-((R)-2-hydroxypropyl)-N-methyl-L-serine (Compound aa169, H-Ser(R-2-PrOH)-OH) (3.40 g) as a crude product. This was used in the next step without purification.

Synthesis of N-(((9H-fluoren-9-yl)methoxy)carbonyl)-O-((R)-2-hydroxypropyl)-N-methyl-L-serine (Compound aa170, Fmoc-MeSer(R-2-PrOH)-OH)

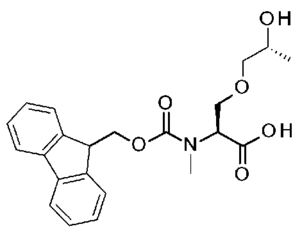

O-((R)-2-Hydroxypropyl)-N-methyl-L-serine (Compound aa169, H-Ser(R-2-PrOH)-OH) (3.40 g, 19.2 mmol), N-(9-fluorenylmethoxycarbonyloxy)succinimide (Fmoc-OSu) (7.12 g, 22.1 mmol), and sodium carbonate (7.13 g, 67.3 mmol) were dissolved in water/1,4-dioxane (80.0 mL/40.0 mL) under a nitrogen atmosphere, and the reaction solution was stirred at room temperature for 3 h. After washing the reaction solution with t-butyl methyl ether three times, a 2 M aqueous hydrochloric acid solution was added to the aqueous layer until pH 2, and the aqueous layer was extracted with ethyl acetate three times. The resulting organic layers were combined, washed with brine, and then dried over anhydrous sodium sulfate, the solvent was evaporated under reduced pressure, and the residue was further dried using

a pump to afford N-(((9H-fluoren-9-yl)methoxy)carbonyl)-O-((R)-2-hydroxypropyl)-N-methyl-L-serine (Compound aa170, Fmoc-MeSer(R-2-PrOH)-OH) (8.00 g) as a crude product. This was used in the next step without purification.

Synthesis of N-(((9H-fluoren-9-yl)methoxy)carbonyl)-N-methyl-O-((2R)-2-((tetrahydro-2H-pyran-2-yl)oxy)propyl)-L-serine (Compound aa171, Fmoc-MeSer(R-2-PrOTHP)-OH)

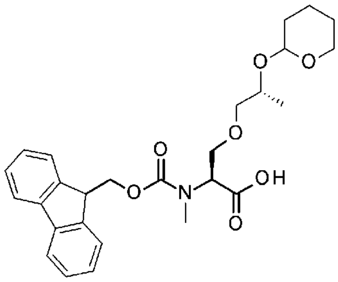

To a solution of N-(((9H-fluoren-9-yl)methoxy)carbonyl)-O-((R)-2-hydroxypropyl)-N-methyl-L-serine (Compound aa170, Fmoc-MeSer(R-2-PrOH)-OH) (8.00 g, 20.0 mmol) in tetrahydrofuran (40.0 mL) were added pyridinium p-toluenesulfonate (0.26 g, 1.02 mmol) and 3,4-dihydro-2H-pyran (11.8 g, 139 mmol) under a nitrogen atmosphere, and the mixture was stirred at 50°C for 2 h. The mixture was cooled to 25°C and ethyl acetate was added. The organic layer was then washed with brine and dried over anhydrous sodium sulfate, and the solvent was evaporated under reduced pressure. The resulting residue (9.3 g) was dissolved in tetrahydrofuran (100 mL), followed by addition of 1.0 M phosphate buffer adjusted to pH 6.8 (100 mL). This mixture was stirred at 50°C for 3 h. After cooling to 25°C, ethyl acetate was added and the organic layer and the aqueous layer were separated. The aqueous layer was extracted with ethyl acetate, after which all the resulting organic layers were combined, washed with brine, and then dried over anhydrous sodium sulfate. The solvent was evaporated under reduced pressure, and the residue was further dried using a pump to afford N-(((9H-fluoren-9-yl)methoxy)carbonyl)-N-methyl-O-((2R)-2-((tetrahydro-2H-pyran-2-yl)oxy)propyl)-L-serine (Compound aa171, Fmoc-MeSer(R-2-PrOTHP)-OH) (8.50 g).
LCMS (ESI) m/z = 501 $(M+NH_4)+$
Retention time: 0.82 min (analytical condition SMD method 10)

N-(((9H-Fluoren-9-yl)methoxy)carbonyl)-O-(2-methyl-2-((tetrahydro-2H-pyran-2-yl)oxy)propyl)-L-serine (Compound aa174, Fmoc-Ser(tBuOTHP)-OH) was synthesized according to the following scheme.

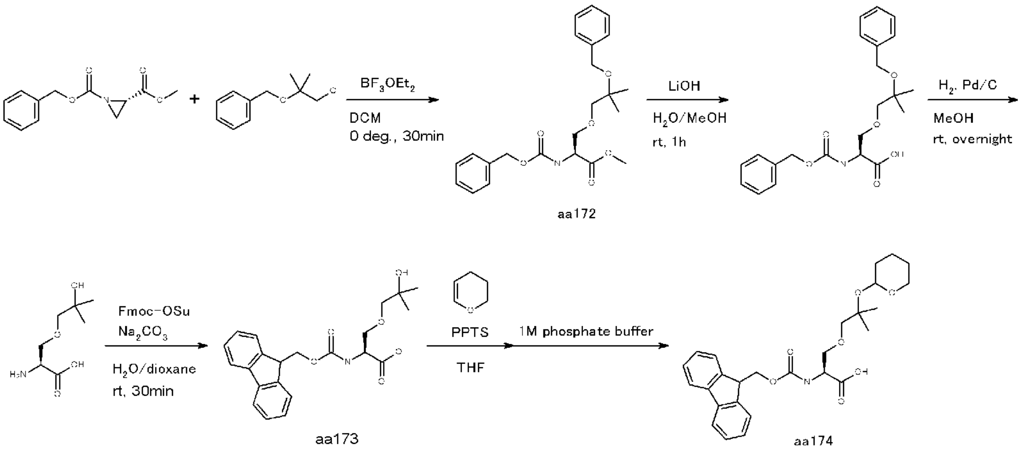


Synthesis of methyl O-(2-(benzyloxy)-2-methylpropyl)-N-((benzyloxy)carbonyl)-L-serinate (Compound aa172, Cbz-Ser(tBuOBn)-OMe)

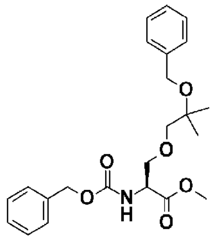

1-Benzyl 2-methyl (S)-aziridine-1,2-dicarboxylate (Cbz-Azy-OMe) (2.0 g, 8.50 mmol) and 2-benzyloxy-2-methylpropan-1-ol (2.30 g, 12.75 mmol) were dissolved in dichloromethane (7.5 mL), and boron trifluoride-diethyl ether complex ($BF_3 \cdot OEt_2$) (0.160 mL, 1.28 mmol) was added dropwise over 5 min under ice-cooling. After stirring for 30 min under ice-cooling, water (2.0 mL) was added, the reaction was quenched by stirring for 10 min, and a saturated aqueous sodium bicarbonate solution was added. The aqueous layer was extracted with dichloromethane, and the organic layer was dried over anhydrous magnesium sulfate. After filtration, the organic solvent was removed by concentration under reduced pressure, and the resulting residue was purified by normal phase column chromatography (hexane/ethyl acetate = 100/0 -> 75/25) to afford methyl O-(2-(benzyloxy)-2-methylpropyl)-N-((benzyloxy)carbonyl)-L-serinate (Compound aa172, Cbz-Ser(tBuOBn)-OMe) (2.36 g, 67%).
LCMS (ESI) m/z = 416 (M+H)+
Retention time: 0.93 min (analytical condition SQDFA05)

Synthesis of N-(((9H-fluoren-9-yl)methoxy)carbonyl)-O-(2-hydroxy-2-methylpropyl)-L-serine (Compound aa173, Fmoc-Ser(tBuOH)-OH)

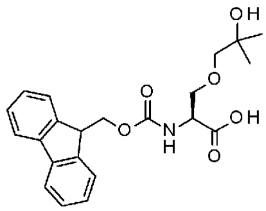

Methyl O-(2-(benzyloxy)-2-methylpropyl)-N-((benzyloxy)carbonyl)-L-serinate (Compound aa172, Cbz-Ser(tBuOBn)-OMe) (2.36 g, 5.68 mmol) was dissolved in methanol (8.0 mL). An aqueous solution of Lithium hydroxide monohydrate (0.477 g, 11.36 mmol) dissolved in water (8.0 mL) was added thereto and the mixture was stirred at room temperature for 1 h. Thereafter, a 2 M aqueous hydrochloric acid solution (8.52 mL, 17.04 mmol) was added. The aqueous layer was extracted with ethyl acetate three times, and the organic layers were dried over anhydrous magnesium sulfate. After filtration, the ethyl acetate was removed by concentration under reduced pressure to afford O-(2-(benzyloxy)-2-methylpropyl)-N-((benzyloxy)carbonyl)-L-serine (Cbz-Ser(tBuOBn)-OH).

O-(2-(benzyloxy)-2-methylpropyl)-N-((benzyloxy)carbonyl)-L-serine obtained above (Cbz-Ser(tBuOBn)-OH) was dissolved in methanol (45 mL), Pd/C (456 mg) was added, and the mixture was stirred at room temperature overnight under a hydrogen atmosphere. The Pd/C was removed by filtration through celite and the methanol was then removed by concentration under reduced pressure to afford O-(2-hydroxy-2-methylpropyl)-L-serine (H-Ser(tBuOH)-OH) (1.05 g) quantitatively.

The resulting O-(2-hydroxy-2-methylpropyl)-L-serine (H-Ser(tBuOH)-OH) (1.59 g, 8.97 mL) and sodium carbonate (2.85 g, 26.9 mmol) were dissolved in water (36 mL) and 1,4-dioxane (15 mL), and N-(9-fluorenylmethoxycarbonyloxy)succinimide (Fmoc-OSu) (3.18 g, 9.42 mmol) was slowly added. After stirring at room temperature for 30 min, water (40 mL) was added to the reaction solution, and the mixture was washed with t-butyl methyl ether (60 mL) three times. Thereafter, the aqueous layer was adjusted to pH 1 by adding a 2 M aqueous hydrochloric acid solution (26.9 mL, 53.8 mmol) to the aqueous layer, and was then extracted with t-butyl methyl ether (60 mL) three times. The organic layers were dried over anhydrous magnesium sulfate and filtered, after which the t-butyl methyl ether was removed by concentration under reduced pressure. The resulting residue was purified by reverse phase column chromatography (0.1% aqueous formic acid solution/0.1 formic acid-acetonitrile solution) to afford N-(((9H-fluoren-9-yl)methoxy)carbonyl)-O-(2-hydroxy-2-methylpropyl)-L-serine (Compound aa173, Fmoc-Ser(tBuOH)-OH) (2.62 g, 73%).
LCMS (ESI) m/z = 400 (M+H)+
Retention time: 0.71 min (analytical condition SQDFA05)

Synthesis of N-(((9H-fluoren-9-yl)methoxy)carbonyl)-O-(2-methyl-2-((tetrahydro-2H-pyran-2-yl)oxy)propyl)-L-serine (Compound aa174, Fmoc-Ser(tBuOTHP)-OH)

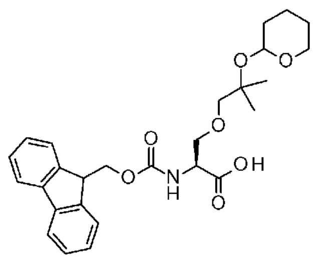

N-(((9H-Fluoren-9-yl)methoxy)carbonyl)-O-(2-hydroxy-2-methylpropyl)-L-serine (Compound aa173, Fmoc-Ser(tBuOH)-OH) (1.2 g, 3.00 mmol) and 3,4-dihydro-2H-pyran (1.90 mL, 21.03 mmol) were dissolved in tetrahydrofuran (6.0 mL), pyridinium p-toluenesulfonate (PPTS) (0.038 g, 0.150 mmol) was added, and the mixture was stirred at 50°C for 2 h. The reaction solution was then diluted with ethyl acetate (15 mL) and washed with brine (15 mL) three times. The organic layers were dried over anhydrous sodium sulfate and then filtered, and the solvent was removed by concentration under reduced pressure. The resulting residue was dissolved in tetrahydrofuran (24.0 mL), 1.0 M phosphate buffer adjusted to pH 6.8 (24.0 mL) was added, and the mixture was stirred at 50°C for 3 h. The reaction solution was diluted with ethyl acetate (45 mL) and then washed with brine (45 mL) three times, and the organic layers were dried over anhydrous sodium sulfate. After filtration, concentration under reduced pressure gave a residue, which was then purified by reverse phase column chromatography (10 mM aqueous ammonium acetate solution/methanol) to afford N-(((9H-fluoren-9-yl)methoxy)carbonyl)-O-(2-methyl-2-((tetrahydro-2H-pyran-2-yl)oxy)propyl)-L-serine (Compound aa174, Fmoc-Ser(tBuOTHP)-OH) (1.05 g, 72%).
LCMS (ESI) m/z = 484 (M+H)+
Retention time: 0.91 min (analytical condition SQDFA05)

N-(((9H-Fluoren-9-yl)methoxy)carbonyl)-N-methyl-O-(2-methyl-2-((tetrahydro-2H-pyran-2-yl)oxy)propyl)-L-serine (Compound aa175, Fmoc-MeSer(tBuOTHP)-OH) was synthesized according to the following scheme.

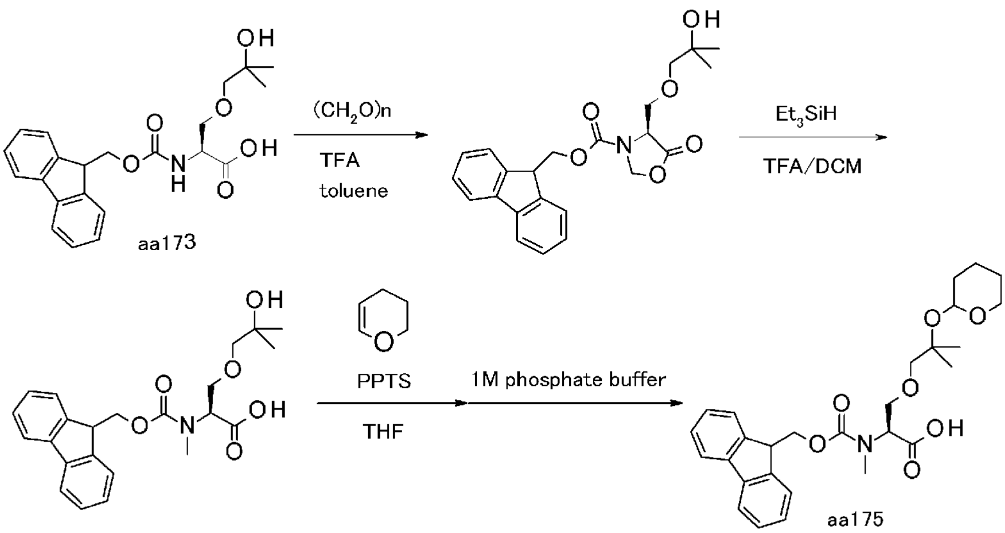


Synthesis of N-(((9H-fluoren-9-yl)methoxy)carbonyl)-N-methyl-O-(2-methyl-2-((tetrahydro-2H-pyran-2-yl)oxy)propyl)-L-serine (Compound aa175, Fmoc-MeSer(tBuOTHP)-OH)

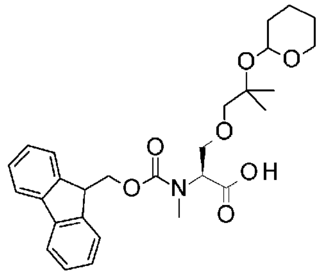

N-(((9H-Fluoren-9-yl)methoxy)carbonyl)-O-(2-hydroxy-2-methylpropyl)-L-serine (Compound aa173, Fmoc-Ser(tBuOH)-OH) (12 g, 30.04 mmol) and paraformaldehyde (2.75 g, 91.67 mmol) were dissolved in trifluoroacetic acid (31.4 g, 277.78 mmol) and toluene (60 mL), and the reaction solution was stirred at room temperature for 4 h. The reaction solution was concentrated under reduced pressure, after which the resulting residue was dissolved in dichloromethane, washed with a saturated aqueous sodium bicarbonate solution and brine, and then dried over anhydrous sodium sulfate. After filtration, the dichloromethane was removed by concentration under reduced pressure to afford (9H-fluoren-9-yl)methyl (S)-4-((2-hydroxy-2-methylpropoxy)methyl)-5-oxooxazolidine-3-carboxylate (12 g, 97%).

The obtained (9H-fluoren-9-yl)methyl (S)-4-((2-hydroxy-2-methylpropoxy)methyl)-5-oxooxazolidine-3-carboxylate (1 g, 2.43 mmol) was dissolved in trifluoroacetic acid/dichloromethane (13 mL/13 mL), and triethylsilane ($Et_3SiH$) (832 mg, 7.16 mmol) was added dropwise. After stirring at room temperature for 16 h, the reaction solution was concentrated under reduced pressure. The resulting residue was dissolved in an aqueous potassium carbonate solution and washed with t-butyl methyl ether, after which the aqueous layer was adjusted to pH 2 with an aqueous hydrochloric acid solution (1 M). The aqueous layer was extracted with t-butyl methyl ether twice, and the organic layers were washed with water twice and with brine twice and dried over anhydrous sodium sulfate. After filtration, concentration under reduced pressure gave a residue, which was then purified by reverse phase column chromatography (water/acetonitrile = 100/0 -> 50/50) to afford N-(((9H-fluoren-9-yl)methoxy)carbonyl)-O-(2-hydroxy-2-methylpropyl)-N-methyl-L-serine (Fmoc-MeSer(tBuOH)-OH) (700 mg, 70%).

The obtained N-(((9H-fluoren-9-yl)methoxy)carbonyl)-O-(2-hydroxy-2-methylpropyl)-N-methyl-L-serine (Fmoc-MeSer(tBuOH)-OH) (6 g, 14.51 mmol) and pyridinium p-toluenesulfonate (PPTS) (182 mg, 0.73 mmol) were dissolved in tetrahydrofuran (48 mL), and 3,4-dihydro-2H-pyran (8.5 g, 101.05 mmol) was added dropwise at room temperature under a nitrogen atmosphere. The reaction solution was stirred at 50°C for 5 h, and the reaction solution was then extracted by adding ethyl acetate. The organic layers were dried over anhydrous sodium sulfate, filtered, and then concentrated under reduced pressure to afford N-(((9H-fluoren-9-yl)methoxy)carbonyl)-N-methyl-O-(2-methyl-2-((tetrahydro-2H-pyran-2-yl)oxy)propyl)-L-serine and tetrahydro-2H-pyran-2-yl N-(((9H-fluoren-9-yl)methoxy)carbonyl)-N-methyl-O-(2-methyl-2-((tetrahydro-2H-pyran-2-yl)oxy)propyl)-L-serinate as a mixture.

The obtained mixture (50 g) was dissolved in phosphate buffer (1 M, pH = 6.8, 1000 mL) and tetrahydrofuran (1000 mL), and the reaction solution was stirred at 50°C for 4 h. The reaction solution was then extracted with ethyl acetate. The organic layers were washed with brine, then dried over anhydrous sodium sulfate, filtered, and then concentrated under reduced pressure. The resulting residue was purified by reverse phase column chromatography (0 to 40% 0.5% aqueous ammonium bicarbonate solution/acetonitrile) to afford N-(((9H-fluoren-9-yl)methoxy)carbonyl)-N-methyl-O-(2-methyl-2-((tetrahydro-2H-pyran-2-yl)oxy)propyl)-L-serine (Compound aa175, Fmoc-MeSer(tBuOTHP)-OH) (20 g).
LCMS (ESI) m/z = 498 $(M+H)^+$
Retention time: 0.94 min (analytical condition SMD method 20)

Synthesis of 4,4-dimethyl-2-phenyl-1,3-dioxane (Compound aa176)

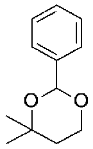

To a solution of 3-methylbutane-1,3-diol (40.0 g, 384 mmol) in chloroform (400 mL) were added dimethoxymethylbenzene (87.6 g, 576 mmol) and p-toluenesulfonic acid (3.59 g, 18.9 mmol) under a nitrogen atmosphere, and the mixture was stirred at 0°C for 1.5 h. The reaction solution was concentrated and the residue was purified by column

chromatography (hexane/ethyl acetate) to afford 4,4-dimethyl-2-phenyl-1,3-dioxane (Compound aa176) (70.0 g, 95%).

Synthesis of 3-(benzyloxy)-3-methylbutan-1-ol (Compound aa177)

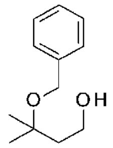

To a solution of 4,4-dimethyl-2-phenyl-1,3-dioxane (Compound aa176) obtained by the above method (5.00 g, 26.0 mmol) in dichloromethane (130 mL) was added a 1 M solution of diisobutylaluminum hydride in hexane (156 mL, 156 mmol) at -50°C under a nitrogen atmosphere, and the mixture was stirred at the same temperature for 15 min and then stirred at room temperature for 1 h. The reaction was quenched by adding methanol, and the mixture was then extracted with dichloromethane twice. The combined organic layers were washed with brine and dried over anhydrous sodium sulfate, the solvent was evaporated under reduced pressure, and the residue was purified by column chromatography (hexane/ethyl acetate) to afford 3-(benzyloxy)-3-methylbutan-1-ol (Compound aa177) (2.00 g, 40%).

Synthesis of methyl O-(3-(benzyloxy)-3-methylbutyl)-N-((benzyloxy)carbonyl)-L-serinate (Compound aa178, Cbz-Ser(2-Me-BuOBn)-OMe)

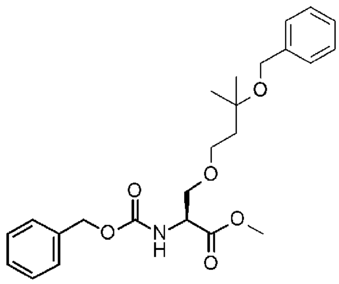

1-Benzyl 2-methyl (S)-aziridine-1,2-dicarboxylate (25 g, 106 mmol) and 3-(benzyloxy)-3-methylbutan-1-ol (Compound aa177) (31.0 g, 159 mmol) were dissolved in dichloromethane (113 mL) under a nitrogen atmosphere and the reaction solution was cooled to 0°C, after which boron trifluoride-diethyl ether complex (2.26 g, 15.9 mmol) was added and the mixture was stirred at 0°C for 3 h. Water was added to the reaction solution, the mixture was extracted with dichloromethane, and the organic layer was then washed with a saturated aqueous sodium carbonate solution and brine. The organic layer was dried over anhydrous sodium sulfate, the

solvent was evaporated under reduced pressure, and the residue was purified by column chromatography (hexane/ethyl acetate) to afford methyl O-(3-(benzyloxy)-3-methylbutyl)-N-((benzyloxy)carbonyl)-L-serinate (Compound aa178, Cbz-Ser(2-Me-BuOBn)-OMe) (28.0 g, 55%).
LCMS (ESI) m/z = 452 (M+Na)+
Retention time: 1.46 min (analytical condition SMD method 13)

Synthesis of O-(3-(benzyloxy)-3-methylbutyl)-N-((benzyloxy)carbonyl)-L-serine (Compound aa179, Cbz-Ser(2-Me-BuOBn)-OH)

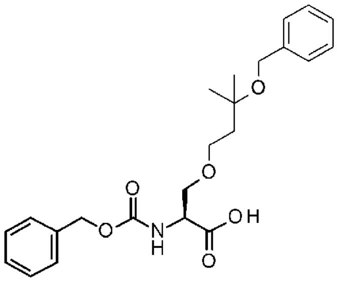

Lithium hydroxide monohydrate (11.2 g) and calcium chloride (110 g) were dissolved in water (278 mL) under a nitrogen atmosphere. A solution of methyl O-(3-(benzyloxy)-3-methylbutyl)-N-((benzyloxy)carbonyl)-L-serinate (Compound aa178, Cbz-Ser(2-Me-BuOBn)-OMe) (28.7 g, 68.4 mmol) in 2-propanol/tetrahydrofuran (278 mL/1115 mL) was added thereto at room temperature, and the mixture was stirred for 5 h. A 2 M aqueous hydrochloric acid solution was added until pH 2, the organic layer was removed, and the aqueous layer was extracted with ethyl acetate three times. The resulting organic layers were combined and dried over anhydrous sodium sulfate, the solvent was evaporated under reduced pressure, and the residue was further dried using a pump to afford O-(3-(benzyloxy)-3-methylbutyl)-N-((benzyloxy)carbonyl)-L-serine (Compound aa179, Cbz-Ser(2-Me-BuOBn)-OH) (28.0 g) as a crude product. This was used in the next step without purification.

Synthesis of O-(3-hydroxy-3-methylbutyl)-L-serine (Compound aa180, H-Ser(2-Me-BuOH)-OH)

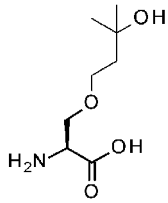

O-(3-(Benzyloxy)-3-methylbutyl)-N-((benzyloxy)carbonyl)-L-serine (Compound aa179, Cbz-Ser(2-Me-BuOBn)-OH) (Compound aa179) (28.0 g, 67.4 mmol) and palladium on carbon (6.00 g, 20% w/w) were dissolved in methanol (500 mL) under a hydrogen atmosphere, and the reaction solution was stirred at room temperature for 16 h. The reaction solution was filtered, the solvent was then evaporated under reduced pressure, and the residue was further dried using a pump, followed by recrystallization from methanol/dichloromethane (1/5) to afford O-(3-hydroxy-3-methylbutyl)-L-serine (Compound aa180, H-Ser(2-Me-BuOH)-OH) (7.00 g, 54%).

Synthesis of N-(((9H-fluoren-9-yl)methoxy)carbonyl)-O-(3-hydroxy-3-methylbutyl)-L-serine (Compound aa181, Fmoc-Ser(2-Me-BuOH)-OH)

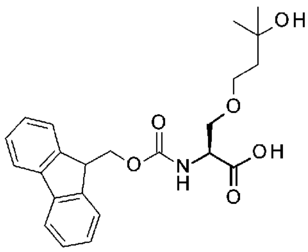

O-(3-Hydroxy-3-methylbutyl)-L-serine (Compound aa180, H-Ser(2-Me-BuOH)-OH) (2.80 g, 14.6 mmol) and sodium carbonate (4.66 g, 44.0 mmol) were dissolved in 1,4-dioxane (24.5 mL)/water (58.8 mL) under a nitrogen atmosphere, after which N-(9-fluorenylmethoxycarbonyloxy)succinimide (Fmoc-OSu) (5.18 g, 15.4 mmol) was added and

the mixture was stirred at room temperature for 3 h. After washing the reaction solution with t-butyl methyl ether three times, a 2 M aqueous hydrochloric acid solution was added to the aqueous layer until pH 2, and the aqueous layer was extracted with ethyl acetate three times. The resulting organic layers were combined, washed with brine, and then dried over anhydrous sodium sulfate, the solvent was evaporated under reduced pressure, and the residue was further dried using a pump to afford N-(((9H-fluoren-9-yl)methoxy)carbonyl)-O-(3-hydroxy-3-methylbutyl)-L-serine (Compound aa181, Fmoc-Ser(2-Me-BuOH)-OH) (5.80 g) as a crude product. This was used in the next step without purification.

Synthesis of N-(((9H-fluoren-9-yl)methoxy)carbonyl)-O-(3-methyl-3-((tetrahydro-2H-pyran-2-yl)oxy)butyl)-L-serine (Compound aa182, Fmoc-Ser(2-Me-BuOTHP)-OH)

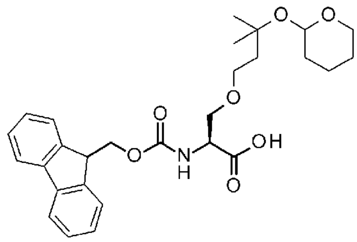

To a solution of N-(((9H-fluoren-9-yl)methoxy)carbonyl)-O-(3-hydroxy-3-methylbutyl)-L-serine (Compound aa181, Fmoc-Ser(2-Me-BuOH)-OH) (6.80 g, 16.5 mmol) in tetrahydrofuran (35.0 mL) were added pyridinium p-toluenesulfonate (0.20 g, 0.82 mmol) and 3,4-dihydro-2H-pyran (10.3 mL) under a nitrogen atmosphere, and the mixture was stirred at 50°C for 5 h. The mixture was cooled to 25°C and ethyl acetate was added. The organic layer was then washed with brine and dried over anhydrous sodium sulfate, and the solvent was evaporated under reduced pressure. 0.75 g of the resulting residue (11.0 g) was dissolved in tetrahydrofuran (20.0 mL), followed by addition of 1.0 M phosphate buffer adjusted to pH 6.8 (20.0 mL). This mixture was stirred at 50°C for 4 h. After cooling to 25°C, ethyl acetate was added and the organic layer and the aqueous layer were separated. The aqueous layer was extracted with ethyl acetate, after which all the resulting organic layers were combined, washed with brine, and then dried over anhydrous sodium sulfate. The solvent was evaporated under reduced pressure, and the resulting residue was purified by reverse phase column chromatography (0.5% aqueous ammonium bicarbonate solution/acetonitrile) to afford N-(((9H-fluoren-9-yl)methoxy)carbonyl)-O-(3-methyl-3-((tetrahydro-2H-pyran-2-yl)oxy)butyl)-L-serine (Compound aa182, Fmoc-Ser(2-Me-BuOTHP)-OH) (0.60 g, 80%).
LCMS (ESI) m/z = 498 (M+H)+
Retention time: 0.72 min (analytical condition SMD method 11)

Synthesis of (9H-fluoren-9-yl)methyl (S)-4-((3-hydroxy-3-methylbutoxy)methyl)-5-oxooxazolidine-3-carboxylate (Compound aa183)

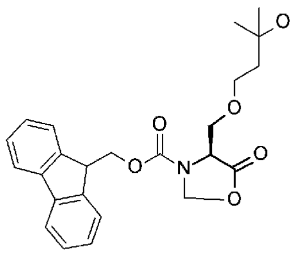

N-(((9H-Fluoren-9-yl)methoxy)carbonyl)-O-(3-hydroxy-3-methylbutyl)-L-serine (Compound aa181, Fmoc-Ser(2-Me-BuOH)-OH) (0.20 g, 0.48 mmol), paraformaldehyde (0.06 g), and trifluoroacetic acid (0.64 g, 5.62 mmol) were dissolved in toluene (10.0 mL), and the reaction solution was stirred at room temperature for 16 h. The solvent was evaporated from the reaction solution under reduced pressure, and the resulting residue was purified by reverse phase column chromatography (water/acetonitrile) to afford (9H-fluoren-9-yl)methyl (S)-4-((3-hydroxy-3-methylbutoxy)methyl)-5-oxooxazolidine-3-carboxylate (Compound aa183) (0.16 g, 80%).

LCMS (ESI) m/z = 448 (M+Na)+
Retention time: 2.47 min (analytical condition SMD method 14)

Synthesis of N-(((9H-fluoren-9-yl)methoxy)carbonyl)-O-(3-hydroxy-3-methylbutyl)-N-methyl-L-serine (Compound aa184, Fmoc-MeSer(2-Me-BuOH)-OH)

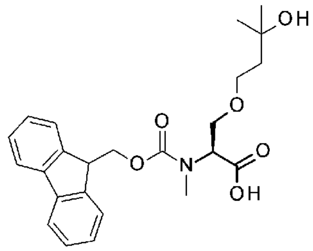

To a solution of (9H-fluoren-9-yl)methyl (S)-4-((3-hydroxy-3-methylbutoxy)methyl)-5-oxooxazolidine-3-carboxylate (Compound aa183) (1.00 g, 2.35 mmol) and aluminum chloride (0.63 g, 4.70 mmol) in dichloromethane (50.0 mL) was slowly added triethylsilane (0.75 mL) dropwise at 0°C under a nitrogen atmosphere, and the mixture was stirred at room temperature for 2 h. The reaction solution was diluted with dichloromethane, washed with a 2 M aqueous hydrochloric acid solution and brine, and the organic layer was then dried over anhydrous sodium sulfate, the solvent was evaporated under reduced pressure, and the resulting residue was purified by reverse phase column chromatography (water/acetonitrile) to afford N-(((9H-fluoren-9-yl)methoxy)carbonyl)-O-(3-hydroxy-3-methylbutyl)-N-methyl-L-serine (Compound aa184, Fmoc-MeSer(2-Me-BuOH)-OH) (0.30 g, 30%).
LCMS (ESI) m/z = 450 (M+Na)+
Retention time: 2.12 min (analytical condition SMD method 15)

Synthesis of (9H-fluoren-9-yl)methyl(S)-4-((6-(dimethylamino)pyridin-3-yl)methyl)-5-oxooxazolidine-3-carboxylate (Compound aa185)

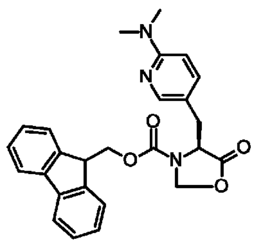

To a solution of (S)-2-((((9H-fluoren-9-yl)methoxy)carbonyl)amino)-3-(6-(dimethylamino)pyridin-3-yl)propanoic acid (Compound aa88, Fmoc-Ala(3-Pyr-4-NMe2)-OH) (500 mg, 0.1.159 mmol) and paraformaldehyde (104 mg, 3.48 mmol) in toluene (3.5 mL) was added trifluoroacetic acid (0.803 mL, 10.43 mmol) at room temperature under a nitrogen atmosphere, and the mixture was stirred overnight. The reaction solution was concentrated, and the resulting residue was purified by reverse phase column chromatography (0.1% aqueous formic acid solution/0.1% formic acid-acetonitrile solution) to afford (9H-fluoren-9-yl)methyl (S)-4-((6-(dimethylamino)pyridin-3-yl)methyl)-5-oxooxazolidine-3-carboxylate (Compound aa185) (517 mg) quantitatively.
LCMS (ESI) m/z = 444 (M+H)+
Retention time: 0.59 min (analytical condition SQDFA05)

Synthesis of (S)-2-((((9H-fluoren-9-yl)methoxy)carbonyl)(methyl)amino)-3-(6-(dimethylamino)pyridin-3-yl)propanoic acid (Compound aa186, Fmoc-MeAla(3-Pyr-4-NMe2)-OH)

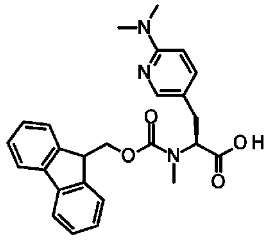

To a solution of (9H-fluoren-9-yl)methyl (S)-4-((6-(dimethylamino)pyridin-3-yl)methyl)-5-oxooxazolidine-3-carboxylate (Compound aa185) (496 mg) in dichloroethane (DCE) (4.0 mL) were added triethylsilane (1.608 mL, 10.07 mmol) and trifluoroacetic acid (2.326 mL, 30.2 mmol) at room temperature under a nitrogen atmosphere, and the reaction solution was stirred at 60°C overnight. The reaction solution was cooled at room temperature and then concentrated under reduced pressure. The resulting residue was purified by reverse phase column chromatography (0.1% aqueous formic acid solution/0.1 % formic acid-acetonitrile solution) to afford (S)-2-((((9H-fluoren-9-yl)methoxy)carbonyl)(methyl)amino)-3-(6-(dimethylamino)pyridin-3-yl)propanoic acid (Compound aa186, Fmoc-MeAla(3-Pyr-4-NMe2)-OH) (181 mg, 36%).
LCMS (ESI) m/z = 446 (M+H)+
Retention time: 0.54 min (analytical condition SQDFA05)

Synthesis of (S)-2-((((9H-fluoren-9-yl)methoxy)carbonyl)(methyl)amino)-3-(4-(trifluoromethyl)phenyl)propanoic acid (Compound aa187, Fmoc-MePhe(4-CF3)-OH)

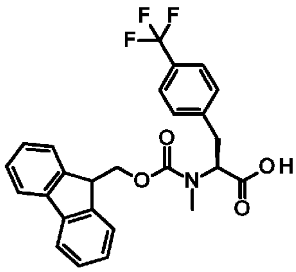

To a solution of commercially available (S)-2-((((9H-fluoren-9-yl)methoxy)carbonyl)amino)-3-(4-(trifluoromethyl)phenyl)propanoic acid (Fmoc-Phe(4-CF3)-OH) (10 g, 21.96 mmol) in dichloromethane (DCM) (110 mL) were added paraformaldehyde (1.978 g, 65.9 mmol), anhydrous magnesium sulfate (6.61 g, 52.9 mmol), and boron trifluoride-diethyl ether complex ($BF_3$·OEt) (3.34 mL, 26.3 mmol) at room temperature under a nitrogen atmosphere, and the mixture was stirred for 2 h. 50% brine was added to the reaction solution, followed by extraction with dichloromethane (DCM). The resulting organic layer was washed with brine, after which the organic layer was separated using a phase separator (purchased from Biotage). The resulting organic solvent was evaporated under reduced pressure to afford a crude product (9H-fluoren-9-yl)methyl (S)-5-oxo-4-(4-(trifluoromethyl)benzyl)oxazolidine-3-carboxylate.

To a solution of the above crude product (9H-fluoren-9-yl)methyl (S)-5-oxo-4-(4-(trifluoromethyl)benzyl)oxazolidine-3-carboxylate in dichloromethane (DCM) (110 mL) were added triethylsilane (10.49 mL, 65.9 mmol), boron trifluoride-diethyl ether complex ($BF_3$·OEt) (8.35 mL, 65.9 mmol), and water (0.396 mL) at room temperature under a nitrogen atmosphere, and the mixture was stirred overnight. 50% brine was added to the reaction solution, the mixture was stirred at room temperature for 15 min, and the organic layer was then separated using a phase separator (purchased from Biotage). The resulting organic solvent was evaporated under reduced pressure, and the resulting residue was purified by reverse phase column chromatography (0.1% aqueous formic acid solution/0.1% formic acid-acetonitrile solution) to afford (S)-2-((((9H-fluoren-9-yl)methoxy)carbonyl)(methyl)amino)-3-(4-(trifluoromethyl)phenyl)propanoic acid (Compound aa187, Fmoc-MePhe(4-CF3)-OH) (7.7 g, 75% over two steps).
LCMS (ESI) m/z = 470 (M+H)+
Retention time: 0.95 min (analytical condition SQDFA05)

Synthesis of (S)-2-((((9H-fluoren-9-yl)methoxy)carbonyl)(methyl)amino)-3-(4-(difluoromethyl)phenyl)propanoic acid (Compound aa188, Fmoc-MePhe(4-CHF2)-OH)

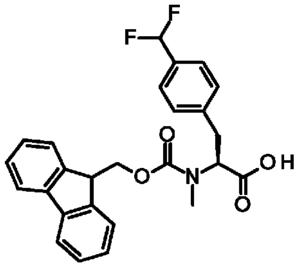

To a solution of commercially available (S)-2-((((9H-fluoren-9-yl)methoxy)carbonyl)amino)-3-(4-(difluoromethyl)phenyl)propanoic acid (Fmoc-Phe(4-CHF2)-OH) (2.0 g, 4.57 mmol) and paraformaldehyde (412 mg, 13.72 mmol) in toluene (15 mL) was added trifluoroacetic acid (3.17 mL, 41.1 mmol) at room temperature under a nitrogen atmosphere, and the mixture was stirred overnight. Water was added to the reaction solution, followed by extraction with dichloromethane (DCM). The resulting organic layer was washed with a saturated aqueous sodium bicarbonate solution and brine and then dried over anhydrous magnesium sulfate. After filtration, the organic solvent was evaporated under reduced pressure to afford a crude product (9H-fluoren-9-yl)methyl (S)-4-(4-(difluoromethyl)benzyl)-5-oxooxazolidine-3-carboxylate (2.8 g).

To a solution of the above crude product (9H-fluoren-9-yl)methyl (S)-4-(4-(difluoromethyl)benzyl)-5-oxooxazolidine-3-carboxylate (2.8 g) in dichloroethane (DCE) (25 mL) were added triethylsilane (6.57 mL, 41.1 mmol) and trifluoroacetic acid (9.51 mL, 123 mmol) at room temperature under a nitrogen atmosphere, and the reaction solution was stirred at 60°C overnight. The reaction solution was cooled at room temperature, water was then added, and the mixture was concentrated under reduced pressure. The resulting residue was diluted with dimethyl sulfoxide (DMSO) and purified by reverse phase column chromatography (0.1% aqueous formic acid solution/0.1% formic acid-acetonitrile solution) to afford (S)-2-((((9H-fluoren-9-yl)methoxy)carbonyl)(methyl)amino)-3-(4-(difluoromethyl)phenyl)propanoic acid (Compound aa188, Fmoc-MePhe(4-CHF2)-OH) (1.38 g, 67% over two steps).
LCMS (ESI) m/z = 452 (M+H)+
Retention time: 0.88 min (analytical condition SQDFA05)

Synthesis of (S)-2-((((9H-fluoren-9-yl)methoxy)carbonyl)(methyl)amino)-3-(4-(difluoromethoxy)phenyl)propanoic acid (Compound aa189, Fmoc-MePhe(4-OCHF2)-OH)

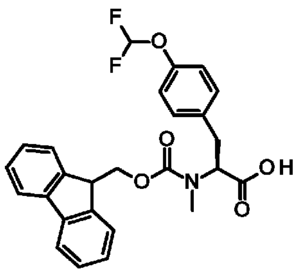

To a solution of commercially available (S)-2-((((9H-fluoren-9-yl)methoxy)carbonyl)amino)-3-(4-(difluoromethoxy)phenyl)propanoic acid (Fmoc-Phe(4-OCHF2)-OH) (1.0 g, 2.205 mmol) and paraformaldehyde (199 mg, 6.62 mmol) in toluene (11 mL) was added trifluoroacetic acid (1.529 mL, 19.85 mmol) at room temperature under a nitrogen atmosphere, and the mixture was stirred overnight. Water was added to the reaction solution, followed by extraction with dichloromethane (DCM). The resulting organic layer was washed with a saturated aqueous sodium bicarbonate solution and brine and then dried over anhydrous magnesium sulfate. After filtration, the organic solvent was evaporated under reduced pressure to afford a crude product (9H-fluoren-9-yl)methyl (S)-4-(4-(difluoromethoxy)benzyl)-5-oxooxazolidine-3-carboxylate.

To a solution of the above crude product (9H-fluoren-9-yl)methyl (S)-4-(4-(difluoromethyl)benzyl)-5-oxooxazolidine-3-carboxylate in dichloroethane (DCE) (15 mL) were added triethylsilane (1.057 mL, 6.62 mmol) and trifluoroacetic acid (1.529 mL, 19.85 mmol) at room temperature under a nitrogen atmosphere, and the reaction solution was stirred at 60°C overnight. The reaction solution was cooled at room temperature, and the solvent was then evaporated under reduced pressure. The resulting residue was diluted with dimethyl sulfoxide (DMSO) and purified by reverse phase column chromatography (0.1% aqueous formic acid solution/0.1% formic acid-acetonitrile solution) to afford (S)-2-((((9H-fluoren-9-yl)methoxy)carbonyl)(methyl)amino)-3-(4-(difluoromethoxy)phenyl)propanoic acid (Compound aa189, Fmoc-MePhe(4-OCHF2)-OH) (696 mg, 68% over two steps).
LCMS (ESI) m/z = 468 (M+H)+
Retention time: 0.87 min (analytical condition SQDFA05)

Synthesis of N-(((9H-fluoren-9-yl)methoxy)carbonyl)-O-(4-chlorophenyl)-L-serine (Compound aa190, Fmoc-Ser(Ph-4-Cl)-OH)

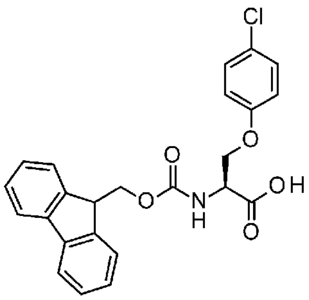

Commercially available methyl trityl-L-serinate (Trt-Ser-OMe) (10.0 g, 27.7 mmol), triphenylphosphine ($PPh_3$) (7.98 g, 30.4 mmol), and 4-chlorophenol (4.09 mL, 41.5 mmol) were mixed with toluene (23 mL), and diisopropyl azodicarboxylate (DIAD) (40% solution in toluene, 1.9 mol/L, 16.02 mL, 30.4 mmol) was added dropwise under ice-cooling. After the dropwise addition was completed, the reaction solution was stirred at 0°C for 5 min, warmed to room temperature, and further stirred for 1 h. The reaction solution was then concentrated under reduced pressure, ethanol/water (8/2, 16.6 mL) was added to the resulting residue, and the mixture was allowed to stand in a freezer for two days. The resulting solid was filtered, washed with ethanol/water (8/2, 20 mL), and dried under reduced pressure to afford methyl O-(4-chlorophenyl)-N-trityl-L-serinate (Trt-Ser(Ph-4-Cl)-OMe) (7.29 g, 56%).

To the above methyl O-(4-chlorophenyl)-N-trityl-L-serinate (Trt-Ser(Ph-4-Cl)-OMe) was added a 4 N hydrochloric acid/1,4-dioxane solution (53.2 mL, 213 mmol), and the mixture was stirred at room temperature for 5 min. The reaction solution was concentrated under reduced pressure, hexane was added to the resulting solid, the mixture was filtered, and the solid was further washed with hexane. The resulting solid was dried under reduced pressure to afford methyl O-(4-chlorophenyl)-L-serinate (H-Ser(Ph-4-Cl)-OMe) as a hydrochloride (4.15 g).

The above methyl O-(4-chlorophenyl)-L-serinate (H-Ser(Ph-4-Cl)-OMe) hydrochloride (300 mg, 1.127 mmol) was dissolved in water (1.67 mL), a solution of lithium hydroxide monohydrate (95 mg, 2.255 mmol) in water/methanol (668 μL/668 μL) was added dropwise over 5 min under ice-cooling, and the mixture was stirred at 0°C for 1 h. To the reaction solution was further added a solution of lithium hydroxide monohydrate (9.5 mg, 0.2255 mmol) in water/methanol (66.8 μL/66.8 μL) at 0°C, and the mixture was stirred for 20 min. To the reaction solution were added 1,4-dioxane (2.337 mL), sodium carbonate (239 mg, 2.255 mmol), water (2.4 mL), and N-(9-fluorenylmethoxycarbonyloxy)succinimide (Fmoc-OSu) (380 mg, 1.127 mmol), and the mixture was stirred at 0°C for 5 min and then stirred at room temperature for 1 h. A 2 N aqueous hydrochloric acid solution was added to the reaction solution at 0°C until pH = 2, and the mixture was then allowed to stand in a freezer. The resulting solid was washed with hexane, water, and hexane to afford N-(((9H-fluoren-9-yl)methoxy)carbonyl)-O-(4-chlorophenyl)-L-serine (Compound aa190, Fmoc-Ser(Ph-4-Cl)-OH) (526.2 mg) quantitatively.
LCMS (ESI) m/z = 438 (M+H)+
Retention time: 0.91 min (analytical condition SQDFA05)

Synthesis of N-(((9H-fluoren-9-yl)methoxy)carbonyl)-O-(2-morpholinoethyl)-L-serine (Compound aa191, Fmoc-Ser(Et-2-Mor)-OH)

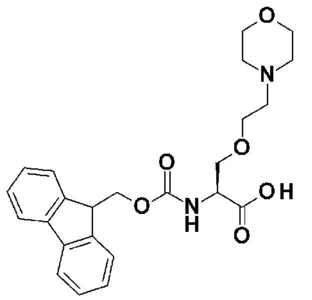

To a solution of commercially available (tert-butoxycarbonyl)-L-serine (Boc-Ser-OH) (20 g, 97.46 mmol) in dimethylformamide (DMF) (100 mL) was added sodium hydride (7.718 g, 321.62 mmol, 60% oil dispersion) at room temperature under a nitrogen atmosphere, and the mixture was stirred for 1 h. The reaction solution was cooled to 0°C, 4-(2-chloroethyl)morpholine (16.040 g, 107.21 mmol) was added dropwise, and the mixture was stirred at room temperature for 16 h. To the reaction solution was added a 50% aqueous formic acid solution at 0°C, the mixture was filtered, and the resulting filtrate was then purified by reverse phase column chromatography (water/acetonitrile) to afford N-(tert-butoxycarbonyl)-O-(2-morpholinoethyl)-L-serine (Boc-Ser(Et-2-Mor)-OH) (5.6 g, 18%).

To N-(tert-butoxycarbonyl)-O-(2-morpholinoethyl)-L-serine (Boc-Ser(Et-2-Mor)-OH) (5.6 g, 17.59 mmol) was added a 4 N hydrochloric acid/1,4-dioxane solution (28.0 mL, 921.53 mmol) at room temperature, and the mixture was stirred for 1 h. The solvent was evaporated under reduced pressure, the resulting residue was washed with dichloromethane, and water (10 mL) was then added to prepare a solution. The resulting solution was adjusted to pH 7 with potassium carbonate, and water (100 mL) and 1,4-dioxane (150 mL) were added, after which potassium carbonate (4.465 g, 32.07 mmol) and N-(9-fluorenylmethoxycarbonyloxy)succinimide (Fmoc-OSu) (5.68 g, 16.84 mmol) were added at room temperature and the mixture was stirred for 16 h. The reaction solution was washed with diethyl ether, and the aqueous layer was adjusted to pH 1 with concentrated hydrochloric acid and extracted with dichloromethane three times. The resulting organic layers were washed with water, dried over anhydrous sodium sulfate, and filtered. The filtrate was concentrated under reduced pressure, and the resulting residue was purified by reverse phase column chromatography (0.1% aqueous hydrochloric acid solution/0.1% hydrochloric acid-acetonitrile solution) to afford N-(((9H-fluoren-9-yl)methoxy)carbonyl)-O-(2-morpholinoethyl)-L-serine (Compound aa191, Fmoc-Ser(Et-2-Mor)-OH) hydrochloride (3.2 g, 48% over two steps).
LCMS (ESI) m/z = 441 (M+H)+
Retention time: 0.50 min (analytical condition SQDFA05)

Synthesis of N-(((9H-fluoren-9-yl)methoxy)carbonyl)-N-butylglycine (Compound aa192, Fmoc-nBuGly-OH)

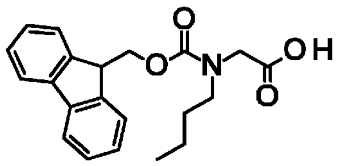

To a solution of sodium hydride (26 g, 1.08 mol) in tetrahydrofuran (THF) (500 mL) was added tert-butyl (tert-butoxycarbonyl)glycinate (Boc-Gly-OtBu) (100 g, 432.36 mmol) in small portions at room temperature. After stirring for 30 min, a solution of 1-iodobutane (239 g, 1.30 mol) in dimethylformamide (DMF) (50 mL) was added dropwise. The reaction solution was stirred at room temperature for 16 h, followed by addition of water. After removing the tetrahydrofuran (THF) under reduced pressure, the reaction solution was extracted with ethyl acetate three times. The resulting organic solvent was dried over anhydrous sodium sulfate, and the solvent was evaporated under reduced pressure. The resulting residue was purified by flash column chromatography (petroleum ether/ethyl acetate) to afford tert-butyl N-(tert-butoxycarbonyl)-N-butylglycinate (Boc-nBuGly-OtBu) (130 g) quantitatively.

To a solution of tert-butyl N-(tert-butoxycarbonyl)-N-butylglycinate (Boc-nBuGly-OtBu) obtained by the above method (260 g, 904.68 mmol) in 1,4-dioxane (1000 mL) was added concentrated hydrochloric acid (1000 mL) dropwise at 0°C. The reaction solution was warmed to room temperature and then stirred at room temperature for 16 h. The reaction solution was concentrated to afford butylglycine (H-nBuGly-OH) hydrochloride (200 g) as a crude product.

A mixture of the crude product butylglycine (H-nBuGly-OH) (60 g), potassium carbonate (188.9 g, 1.37 mol),

and water/1,4-dioxane (1:1) (3000 mL) was stirred at room temperature for 30 min, and N-(9-fluorenylmethoxycarbonyloxy)succinimide (Fmoc-OSu) (183.2 g, 543.09 mmol) was then added in small portions at room temperature. The reaction solution was stirred at room temperature for 16 h and then washed with ether three times. A 5 M aqueous hydrochloric acid solution was added to the resulting aqueous layer until pH = 3, and the aqueous layer was extracted with ethyl acetate three times. The organic solvent was dried over anhydrous sodium sulfate and the solvent was evaporated under reduced pressure. The resulting crude product was washed with ether to afford N-(((9H-fluoren-9-yl)methoxy)carbonyl)-N-butylglycine (Compound aa192, Fmoc-nBuGly-OH) (121 g).
LCMS (ESI) m/z = 354 (M+H)+
Retention time: 0.87 min (analytical condition SQDFA05)

Synthesis of N-(((9H-fluoren-9-yl)methoxy)carbonyl)-N-isopentylglycine (Compound aa193, Fmoc-iPenGly-OH)

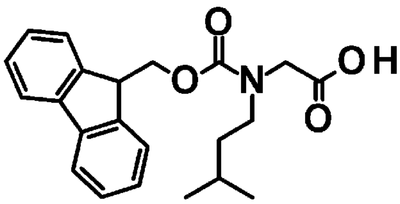

To a solution of 3-methylbutan-1-amine (11.92 mL, 103 mmol) in tetrahydrofuran (100 mL) was added a solution of tert-butyl 2-bromoacetate (10 g, 51.3 mmol) in tetrahydrofuran (100 mL) dropwise at 0°C, and the reaction solution was stirred at 0°C for 10 min and then stirred at room temperature for 1 h. Water (165 mL) was added to the reaction solution at 0°C, after which sodium carbonate (11.95 g, 113 mmol) and (9H-fluoren-9-yl)methyl carbonochloridate (Fmoc-Cl) (29.2 g, 113 mmol) were added in 15 small portions and the mixture was stirred at room temperature for 1 h. To the reaction solution was added a saturated aqueous ammonium chloride solution at 0°C, and the mixture was extracted with ethyl acetate and then washed with water and brine. The resulting organic layer was dried over anhydrous sodium sulfate and the solvent was evaporated under reduced pressure. To the resulting residue was added hexane, and the mixture was filtered. The filtrate was concentrated and the resulting residue was purified by normal phase column chromatography (hexane/ethyl acetate) to afford tert-butyl N-(((9H-fluoren-9-yl)methoxy)carbonyl)-N-isopentylglycinate (Fmoc-iPenGly-OtBu) (15.34 g, 71%).

To a solution of the above tert-butyl N-(((9H-fluoren-9-yl)methoxy)carbonyl)-N-isopentylglycinate (Fmoc-iPenGly-OtBu) (12.34 g, 29.1 mmol) in dichloromethane (133.4 mL) was added trifluoroacetic acid (66.7 mL) dropwise at room temperature, and the mixture was then stirred for 1 h. Toluene (100 mL) was added to the reaction solution, and the solvent was evaporated using a rotary evaporator. This operation was repeated three times. To the resulting residue was then added hexane (300 mL), and the mixture was stored in a freezer. The resulting solid was filtered while washing with hexane to afford N-(((9H-fluoren-9-yl)methoxy)carbonyl)-N-isopentylglycine (Compound aa193, Fmoc-iPenGly-OH) (11.83 g, 89%).
LCMS (ESI) m/z = 368 (M+H)+
Retention time: 0.92 min (analytical condition SQDFA05)

Synthesis of (S)-2-((((9H-fluoren-9-yl)methoxy)carbonyl)amino)-4-(5-oxo-4,5-dihydro-1,2,4-oxadiazol-3-yl)butanoic acid (Compound aa194, Fmoc-Abu(5-Oxo-Odz)-OH)

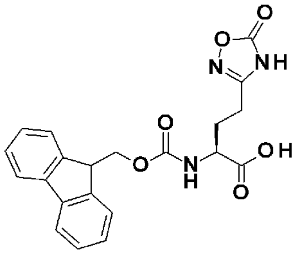

To a solution of commercially available (tert-butoxycarbonyl)-L-glutamine (Boc-Gln-OH) (50 g, 203.03 mmol) in pyridine (350 mL) was added N,N'-dicyclohexylcarbodiimide (DCC) (46.1 g, 223.43 mmol) at room temperature under a nitrogen atmosphere, and the mixture was stirred for 2 h. The reaction solution was filtered and the filtrate was concentrated. To the resulting residue was added dichloromethane and concentrated hydrochloric acid while adjusting it to pH = 3, and the mixture was extracted with dichloromethane twice. The resulting organic layers were dried over anhydrous sodium sulfate and filtered, and the solvent was evaporated under reduced pressure. The resulting residue was purified by normal phase column chromatography (petroleum ether/ethyl acetate) to afford (S)-2-((tert-butoxycarbonyl)amino)-4-cyanobutanoic acid (45.5 g, 98%).

To a solution of (S)-2-((tert-butoxycarbonyl)amino)-4-cyanobutanoic acid obtained as described above (50 g, 219.06 mmol) in ethanol (500 mL) were added hydroxylamine hydrochloride (32 g, 460.50 mmol) and triethylamine (83 mL) at room temperature, and the mixture was stirred at 80°C for 2 h. The reaction solution was concentrated under reduced pressure to afford (S)-2-((tert-butoxycarbonyl)amino)-5-(hydroxyamino)-5-iminopentanoic acid (107 g) as a crude product.

To a solution of the above crude product (S)-2-((tert-butoxycarbonyl)amino)-5-(hydroxyamino)-5-iminopentanoic acid (44 g, 168.40 mmol) in 1,4-dioxane (500 mL) were added 1,1'-carbonyldiimidazole (CDI) (39 g, 240.52 mmol) and 1,8-diazabicyclo[5.4.0]-7-undecene (DBU) (55 g, 361.27 mmol) at room temperature under a nitrogen atmosphere, and the mixture was stirred at 110°C for 4 h. The reaction solution was cooled at room temperature, then adjusted to pH = 2 with concentrated hydrochloric acid, and extracted with dichloromethane twice. The resulting organic layers were dried over anhydrous sodium sulfate and filtered, and the solvent was evaporated under reduced pressure to afford (S)-2-((tert-butoxycarbonyl)amino)-4-(5-oxo-4,5-dihydro-1,2,4-oxadiazol-3-yl)butanoic acid (Boc-Abu(5-Oxo-Odz)-OH) (11 g) as a crude product.

To a solution of (S)-2-((tert-butoxycarbonyl)amino)-4-(5-oxo-4,5-dihydro-1,2,4-oxadiazol-3-yl)butanoic acid (Boc-Abu(5-Oxo-Odz)-OH) obtained as described above (13.5 g, 46.99 mmol) in 1,4-dioxane (10 mL) was added a 4 N hydrochloric acid/1,4-dioxane solution (140 mL) at room temperature under a nitrogen atmosphere, and the mixture was stirred for 16 h. The reaction solution was concentrated under reduced pressure to afford (S)-2-amino-4-(5-oxo-4,5-dihydro-1,2,4-oxadiazol-3-yl)butanoic acid (H-Abu(5-Oxo-Odz)-OH) (8.8 g) as a crude product.

To a solution of the resulting crude product (S)-2-amino-4-(5-oxo-4,5-dihydro-1,2,4-oxadiazol-3-yl)butanoic acid (H-Abu(5-Oxo-Odz)-OH) (8.8 g, 47.02 mmol) and potassium carbonate (13 g, 94.06 mmol) in water/1,4-dioxane (100 mL/100 mL) was added N-(9-fluorenylmethoxycarbonyloxy)succinimide (Fmoc-OSu) (14.3 g, 42.4 mmol) at room temperature under a nitrogen atmosphere, and the mixture was stirred for 3 h. The reaction solution was washed with t-butyl methyl ether/hexane (1/3), and the aqueous layer was adjusted to pH 2 with concentrated hydrochloric acid and extracted with dichloromethane twice. The resulting organic layers were dried over anhydrous sodium sulfate and filtered. The filtrate was concentrated under reduced pressure, and the resulting residue was purified by reverse phase column chromatography (water/acetonitrile) to afford (S)-2-((((9H-fluoren-9-yl)methoxy)carbonyl)amino)-4-(5-oxo-4,5-dihydro-1,2,4-oxadiazol-3-yl)butanoic acid (Compound aa194, Fmoc-Abu(5-Oxo-Odz)-OH) (2.67 g).
LCMS (ESI) m/z = 410 (M+H)+
Retention time: 0.66 min (analytical condition SQDFA05)

Compound aa196 (Fmoc-MeAbu(5-Oxo-Odz)-OH) was synthesized according to the following scheme.

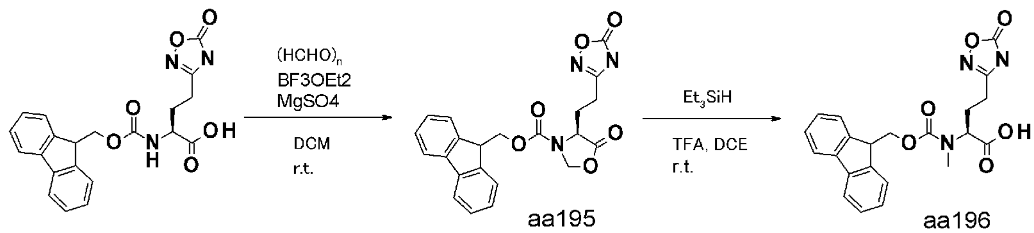


aa195 aa196

<u>Synthesis of 9H-fluoren-9-ylmethyl (4S)-5-oxo-4-[2-(5-oxo-4H-1,2,4-oxadiazol-3-yl)ethyl]oxazolidine-3-carboxylate (Compound aa195)</u>

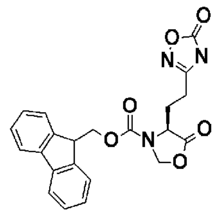

(S)-2-((((9H-Fluoren-9-yl)methoxy)carbonyl)amino)-4-(5-oxo-4,5-dihydro-1,2,4-oxadiazol-3-yl)butanoic acid (Compound aa194, Fmoc-Abu(5-Oxo-Odz)-OH) (93 mg, 0.227 mmol), paraformaldehyde (34.1 mg, 1.136 mmol), and anhydrous magnesium sulfate (68.4 mg, 0.568 mmol) were suspended in dichloromethane (2.27 mL), boron trifluoride-diethyl ether complex ($BF_3 \cdot OEt_2$) (28.8 μL, 0.227 mmol) was added at room temperature, and the mixture was stirred for 23 h. The solid was then removed by filtration, and the filtrate was concentrated under reduced pressure. The resulting residue was purified by reverse phase column chromatography (0.1% aqueous formic acid solution/0.1% formic acid-acetonitrile solution) to afford 9H-fluoren-9-ylmethyl (4S)-5-oxo-4-[2-(5-oxo-4H-1,2,4-oxadiazol-3-yl)ethyl]oxazolidine-3-carboxylate (Compound aa195) (26.0 mg, 27%).
LCMS (ESI) m/z = 422 (M+H)+
Retention time: 0.73 min (analytical condition SQDFA05)

Synthesis of (2S)-2-[9H-fluoren-9-ylmethoxycarbonyl(methyl)amino]-4-(5-oxo-4H-1,2,4-oxadiazol-3-yl)butanoic acid (Compound aa196, Fmoc-MeAbu(5-Oxo-Odz)-OH)

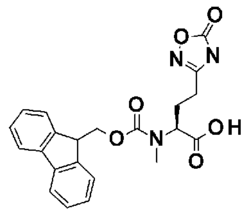

9H-Fluoren-9-ylmethyl (4S)-5-oxo-4-[2-(5-oxo-4H-1,2,4-oxadiazol-3-yl)ethyl]oxazolidine-3-carboxylate (Compound aa195) (23 mg, 0.055 mmol) and triethylsilane (78 μL, 0.491 mmol) were dissolved in trifluoroacetic acid (63.1 μL) and dichloroethane (182 μL), and the reaction solution was stirred at room temperature for 3 h. The reaction solution was concentrated under reduced pressure, and the resulting residue was purified by reverse phase column chromatography (0.1% aqueous formic acid solution/0.1% formic acid-acetonitrile solution) to afford (2S)-2-[9H-fluoren-9-ylmethoxycarbonyl(methyl)amino]-4-(5-oxo-4H-1,2,4-oxadiazol-3-yl)butanoic acid (Compound aa196, Fmoc-MeAbu(5-Oxo-Odz)-OH) (22.1 mg, 96%).
LCMS (ESI) m/z = 424 (M+H)+
Retention time: 0.67 min (analytical condition SQDFA05)

Synthesis of (S)-2-((((9H-fluoren-9-yl)methoxy)carbonyl)(methyl)amino)-3-(pyridin-3-yl)propanoic acid (Compound aa197, Fmoc-MeAla(3-Pyr)-OH)

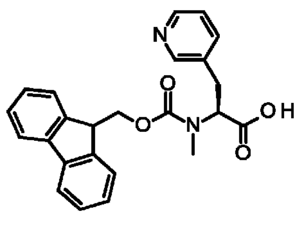

In a screw-capped reaction vessel, a solution of commercially available (S)-2-((((9H-fluoren-9-yl)methoxy)carbonyl)amino)-3-(pyridin-3-yl)propanoic acid (Fmoc-Ala(3-Pyr)-OH) (4.0 g, 10.30 mmol) and paraformaldehyde (1.855 g, 61.8 mmol) in acetic acid (16 mL) was stirred at 90°C for 5 h. The reaction solution was cooled at room temperature,

and the solvent was then evaporated under reduced pressure. To the resulting residue was added ethyl acetate, followed by washing with a saturated aqueous sodium bicarbonate solution and brine. The organic layer was dried over anhydrous magnesium sulfate and filtered, and then the organic solvent was evaporated under reduced pressure to afford a crude product (S)-(9H-fluoren-9-yl)methyl 5-oxo-4-(pyridin-3-ylmethyl)oxazolidine-3-carboxylate (3.17 g).

In a screw-capped reaction vessel, triethylsilane (13.45 mL, 84 mmol) and trifluoroacetic acid (19.46 mL, 253 mmol) were added to a solution of the above crude product (S)-(9H-fluoren-9-yl)methyl 5-oxo-4-(pyridin-3-ylmethyl)oxazolidine-3-carboxylate (2.81 g) in dichloroethane (DCE) (28.5 mL) at room temperature under a nitrogen atmosphere, and the reaction solution was stirred at 85°C for 2 h. The reaction solution was cooled at room temperature, the solvent was then evaporated under reduced pressure, and the resulting residue was purified by reverse phase column chromatography (water/acetonitrile) to afford (S)-2-((((9H-fluoren-9-yl)methoxy)carbonyl)(methyl)amino)-3-(pyridin-3-yl)propanoic acid (Compound aa197, Fmoc-MeAla(3-Pyr)-OH) (2.68 g, 95%).
LCMS (ESI) m/z = 403 (M+H)+
Retention time: 0.54 min (analytical condition SQDFA05)

Synthesis of N-(((9H-fluoren-9-yl)methoxy)carbonyl)-O-(2-(dimethylamino)ethyl)-L-serine (Compound aa198, Fmoc-Ser(Et-2-NMe2)-OH)

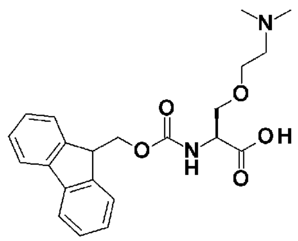

To commercially available trityl-L-serine (Trt-Ser-OH) triethylamine salt (5 g, 11.15 mmol) was added dimethylformamide (DMF) (40 mL) under a nitrogen atmosphere, after which sodium tert-pentoxide (7.36 g, 66.9 mmol) was added at room temperature and the mixture was stirred for 30 min. To the reaction solution was added 2-chloro-N,N-dimethylethan-1-amine hydrochloride (4.01 g, 27.9 mmol) at room temperature, and the mixture was stirred overnight. To the reaction solution was added formic acid (6.41 mL, 167 mmol), and purification by reverse phase column chromatography (10 mM aqueous ammonium acetate solution/methanol) gave O-(2-(dimethylamino)ethyl)-N-trityl-L-serine (Trt-Ser(Et-2-NMe2)-OH) (4.1 g).

To the resulting O-(2-(dimethylamino)ethyl)-N-trityl-L-serine (Trt-Ser(Et-2-NMe2)-OH) (4.1 g, 9.80 mmol) was added dichloromethane (10 mL), after which a 4 N hydrochloric acid/1,4-dioxane solution (40 mL, 160 mmol) and water (4 mL) were added and the mixture was stirred at room temperature for 3 h. Water (80 mL) was added to the reaction solution, which was then washed with hexane twice. To the resulting aqueous layer were added sodium carbonate (26.0 g, 245 mmol), 1,4-dioxane (120 mL), and N-(9-fluorenylmethoxycarbonyloxy)succinimide (Fmoc-OSu) (3.97 g, 11.76 mmol) at 0°C, and the mixture was stirred at room temperature for 2 h. Formic acid (11.28 mL, 294 mmol) was added to the reaction solution, and the 1,4-dioxane was evaporated under reduced pressure. The resulting aqueous layer was purified by reverse phase column chromatography (0.1% aqueous formic acid solution/ 0.1% formic acid-acetonitrile solution) to afford N-(((9H-fluoren-9-yl)methoxy)carbonyl)-O-(2-(dimethylamino)ethyl)-L-serine (Compound aa198, Fmoc-Ser(Et-2-NMe2)-OH) formate (3.07 g, 69% over three steps). To the resulting N-(((9H-fluoren-9-yl)methoxy)carbonyl)-O-(2-(dimethylamino)ethyl)-L-serine (Compound aa198, Fmoc-Ser(Et-2-NMe2)-OH) formate (3.0 g, 7.53 mmol) were added dichloromethane (4 mL) and a 4 N hydrochloric acid/1,4-dioxane solution (9.411 mL, 37.6 mmol), and the mixture was stirred at room temperature for 20 min. The reaction solution was concentrated under reduced pressure to afford N-(((9H-fluoren-9-yl)methoxy)carbonyl)-O-(2-(dimethylamino)ethyl)-L-serine (Compound aa198, Fmoc-Ser(Et-2-NMe2)-OH) hydrochloride (2.7 g, 82%).
LCMS (ESI) m/z = 399 (M+H)+
Retention time: 0.50 min (analytical condition SQDFA05)

Synthesis of N2-(((9H-fluoren-9-yl)methoxy)carbonyl)-N5-(methylsulfonyl)-L-glutamine (Compound aa199, Fmoc-Gln(Ms)-OH)

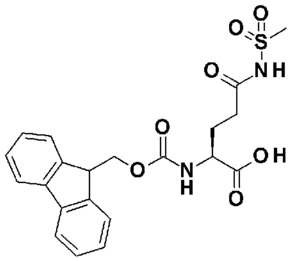

To a solution of commercially available (S)-4-((((9H-fluoren-9-yl)methoxy)carbonyl)amino)-5-(tert-butoxy)-5-oxopentanoic acid (Fmoc-Glu-OtBu) (20 g, 47 mmol), methanesulfonamide (20 g, 210 mmol), and 4-dimethylaminopyridine (DMAP) (1.3 g, 10.6 mmol) in dichloromethane (360 mL) was added 1-(3-dimethylaminopropyl)-3-ethylcarbodiimide hydrochloride (WSC·HCl) (9.64 g, 50.3 mmol) at room temperature under a nitrogen atmosphere, and the mixture was stirred for 16 h. The reaction solution was concentrated under reduced pressure, ethyl acetate was added, and the reaction solution was washed with a 0.1% aqueous hydrochloric acid solution three times and with water once, then dried over anhydrous sodium sulfate, and filtered. The resulting solution was concentrated under reduced pressure, and the residue was purified by normal phase column chromatography (petroleum ether/ethyl acetate) to afford tert-butyl N2-(((9H-fluoren-9-yl)methoxy)carbonyl)-N5-(methylsulfonyl)-L-glutaminate (Fmoc-Gln(Ms)-OtBu) (9.3 g, 39%).

To a solution of tert-butyl N2-(((9H-fluoren-9-yl)methoxy)carbonyl)-N5-(methylsulfonyl)-L-glutaminate (Fmoc-Gln(Ms)-OtBu) obtained as described above (5.5 g, 10.9) in 2,2,2-trifluoroethanol (TFE) (100 mL) was added chlorotrimethylsilane (TMSCl) (3.57 g, 32.861 mmol) at 0°C under a nitrogen atmosphere, and the mixture was stirred for 1 h. The reaction solution was concentrated under reduced pressure, t-butyl methyl ether was added to the resulting residue, and the mixture was concentrated again. This operation was further repeated twice, and recrystallization from acetonitrile/dichloromethane gave N2-(((9H-fluoren-9-yl)methoxy)carbonyl)-N5-(methylsulfonyl)-L-glutamine (Compound aa199, Fmoc-Gln(Ms)-OH) (3.8178 g, 77%) as a white solid.
LCMS (ESI) m/z = 447 (M+H)+
Retention time: 0.65 min (analytical condition SQDFA05)

Synthesis of methyl N-(((9H-fluoren-9-yl)methoxy)carbonyl)-O-(3,3,3-trifluoro-2-hydroxypropyl)-L-serinate (Compound aa200, Fmoc-Ser(1-CF3-EtOH)-OMe)

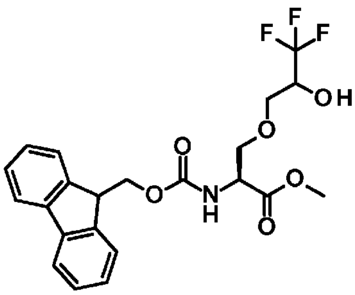

To a solution of separately synthesized 1-((9H-fluoren-9-yl)methyl) 2-methyl (S)-aziridine-1,2-dicarboxylate (Compound aa204, Fmoc-Azy-OMe) (0.20 g, 0.62 mmol) and 3,3,3-trifluoropropane-1,2-diol (0.24 g, 1.9 mmol) in dichloromethane (3.0 mL) was added boron trifluoride-diethyl ether complex ($BF_3 \cdot OEt_2$) (7.8 μL, 0.062 mmol) at 0°C under a nitrogen atmosphere, and the mixture was stirred for 30 min. Moreover, to a solution of 1-((9H-fluoren-9-yl)methyl) 2-methyl (S)-aziridine-1,2-dicarboxylate (Compound aa204, Fmoc-Azy-OMe) (5.0 g, 15.46 mmol) and 3,3,3-trifluoropropane-1,2-diol (6.03 g, 46.4 mmol) in dichloromethane (77.0 mL) was added boron trifluoride-diethyl ether complex ($BF_3 \cdot OEt_2$) (0.19 mL, 1.55 mmol) at 0°C under a nitrogen atmosphere, and the mixture was stirred for 30 min. Furthermore, to a solution of 1-((9H-fluoren-9-yl)methyl) 2-methyl (S)-aziridine-1,2-dicarboxylate (Compound aa204, Fmoc-Azy-OMe) (3.6 g, 11.13 mmol) and 3,3,3-trifluoropropane-1,2-diol (4.34 g, 33.4 mmol) in dichloromethane (55.7 mL) was added boron trifluoride-diethyl ether complex ($BF_3 \cdot OEt_2$) (0.14 mL, 1.11 mmol) at 0°C under a nitrogen atmosphere, and the mixture was stirred for 30 min.

A saturated aqueous sodium bicarbonate solution was added to each of the above three reaction solutions, after which all the reaction solutions were combined, most of the organic solvent was evaporated under reduced pressure,

and the remaining solution was extracted by adding ethyl acetate (300 mL). The organic layer was washed with a brine solution, and the organic solvent was then evaporated under reduced pressure. The resulting residue was purified by normal phase silica gel column chromatography (hexane/ethyl acetate) to afford methyl N-(((9H-fluoren-9-yl)methoxy)carbonyl)-O-(3,3,3-trifluoro-2-hydroxypropyl)-L-serinate (Compound aa200, Fmoc-Ser(1-CF3-EtOH)-OMe) (6.57 g, 53%, purity: 95%) and the same compound (3.1 g, 25%, purity: 87%).
LCMS (ESI) m/z = 454 (M+H)+
Retention time: 1.18 min (analytical condition SMD method 45)

Synthesis of N-(((9H-fluoren-9-yl)methoxy)carbonyl)-O-(3,3,3-trifluoro-2-hydroxypropyl)-L-serine (Compound aa201, Fmoc-Ser(1-CF3-EtOH)-OH)

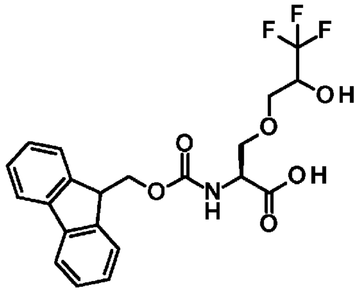

Calcium chloride (2.06 g, 18.5 mmol) was dissolved in water (5.2 mL), lithium hydroxide monohydrate (207 mg, 4.94 mmol) was added, and the mixture was stirred at room temperature for 10 min. To the solution was added a solution of methyl N-(((9H-fluoren-9-yl)methoxy)carbonyl)-O-(3,3,3-trifluoro-2-hydroxypropyl)-L-serinate (Compound aa200, Fmoc-Ser(1-CF3-EtOH)-OMe) (560 mg, 1.24 mmol) in tetrahydrofuran (THF) (5.15 mL) and isopropanol (20.6 mL), and the mixture was stirred at room temperature for 2 h. The reaction was completed by adding a 1 N aqueous hydrochloric acid solution. Further, the same reaction was performed using 0.15 g, 1.11 g, 0.48 g, and 0.96 g of Fmoc-Ser(1-CF3-EtOH)-OMe, respectively. All the reaction solutions were collected and most of the solvent was evaporated under reduced pressure. The resulting solution was extracted by adding ethyl acetate and water. The solvent was evaporated from the organic layer under reduced pressure, and the resulting residue was then purified by reverse phase column chromatography (0.1% aqueous formic acid solution/0.1% formic acid-acetonitrile solution) to afford N-(((9H-fluoren-9-yl)methoxy)carbonyl)-O-(3,3,3-trifluoro-2-hydroxypropyl)-L-serine (Compound aa201, Fmoc-Ser(1-CF3-EtOH)-OH) (3.10 g, 98%).
LCMS (ESI) m/z = 462 (M+Na)+
Retention time: 2.24 min (analytical condition SMD method 46)

Synthesis of N-(((9H-fluoren-9-yl)methoxy)carbonyl)-N-methyl-O-(3,3,3-trifluoro-2-hydroxypropyl)-L-serine (Compound aa202, Fmoc-MeSer(1-CF3-EtOH)-OH)

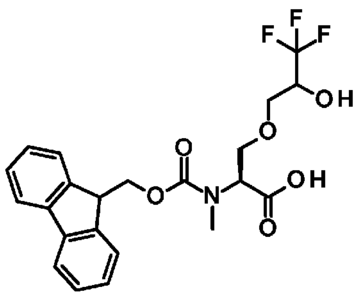

To a solution of N-(((9H-fluoren-9-yl)methoxy)carbonyl)-O-(3,3,3-trifluoro-2-hydroxypropyl)-L-serine (Compound aa201, Fmoc-Ser(1-CF3-EtOH)-OH) (1.84 g, 4.19 mmol) in dichloromethane (20.9 mL) were added paraformaldehyde (151 mg, 5.03 mmol), anhydrous magnesium sulfate (1.26 g, 10.5 mmol), and boron trifluoride-diethyl ether

complex ($BF_3 \cdot OEt_2$) (526 μL, 4.19 mmol) under a nitrogen atmosphere, and the mixture was stirred at room temperature for 3 h. The reaction solution was filtered to remove the insoluble matter, and was then washed with dichloromethane (10 mL).

To the filtered reaction solution were added triethylsilane (2.0 mL, 12.6 mmol) and boron trifluoride-diethyl ether complex ($BF_3 \cdot OEt_2$) (1.58 mL, 12.6 mmol), and the mixture was stirred at room temperature for 30 min. The reaction solution was diluted with brine and extracted with dichloromethane. The resulting organic layer was concentrated under reduced pressure, and the resulting residue was then purified by reverse phase column chromatography (0.1% aqueous formic acid solution/0.1% formic acid-acetonitrile solution) to afford N-(((9H-fluoren-9-yl)methoxy)carbonyl)-N-methyl-O-(3,3,3-trifluoro-2-hydroxypropyl)-L-serine (Compound aa202, Fmoc-MeSer(1-CF3-EtOH)-OH) (640 mg, 34%).
LCMS (ESI) m/z = 452 (M-H)-
Retention time: 2.33 min (analytical condition SMD method 47)

Synthesis of N-(((9H-fluoren-9-yl)methoxy)carbonyl)-O-(3,3,3-trifluoro-2-((tetrahydro-2H-pyran-2-yl)oxy)propyl)-L-serine (Compound aa203, Fmoc-Ser(1-CF3-EtOTHP)-OH)

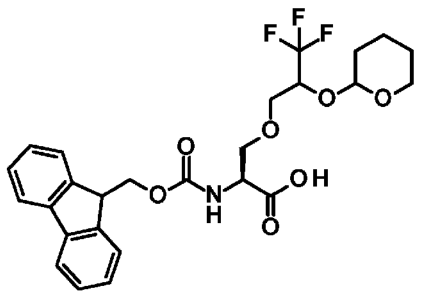

To a solution of N-(((9H-fluoren-9-yl)methoxy)carbonyl)-O-(3,3,3-trifluoro-2-hydroxypropyl)-L-serine (Compound aa201, Fmoc-Ser(1-CF3-EtOH)-OH) (2.52 g, 5.74 mmol) in dichloromethane (19.12 mL) were added 3,4-dihydro-2H-pyran (3.92 mL, 43.0 mmol) and pyridinium p-toluenesulfonate (0.144 g, 0.574 mmol), and the mixture was stirred at 40°C overnight. Water was then added to the reaction solution, the mixture was extracted with dichloromethane, and the organic layer was washed with brine. The organic layer was dried over anhydrous sodium sulfate and then filtered, and the solvent was removed by concentration under reduced pressure. The resulting residue was dissolved in tetrahydrofuran (29 mL), a 1 M aqueous phosphoric acid solution (pH = 8, 29 mL) was added, and the mixture was stirred at 50°C for 3 h. The reaction solution was diluted with water and extracted with ethyl acetate twice. The organic layers were combined, washed with brine, dried over anhydrous sodium sulfate, and then filtered, and the solvent was removed by concentration under reduced pressure. The resulting residue was dissolved in dichloromethane (60 mL) and heptane (60 mL), and the dichloromethane was removed by concentration under reduced pressure to precipitate an oily crude product. The heptane solution was removed by decantation. This operation was repeated twice. The resulting crude product was dissolved in ethyl acetate and washed with a 0.05 M aqueous phosphoric acid solution twice and with brine once. The organic layer was dried over anhydrous sodium sulfate, filtered, and then concentrated under reduced pressure to afford N-(((9H-fluoren-9-yl)methoxy)carbonyl)-O-(3,3,3-trifluoro-2-((tetrahydro-2H-pyran-2-yl)oxy)propyl)-L-serine (Compound aa203, Fmoc-Ser(1-CF3-EtOTHP)-OH) (2.89 g, 96%).
LCMS (ESI) m/z = 522 (M-H)-
Retention time: 1.00 min (analytical condition SQDAA05-2)

Synthesis of 1-((9H-fluoren-9-yl)methyl)2-methyl(S)-aziridine-1,2-dicarboxylate (Compound aa204, Fmoc-Azy-OMe)

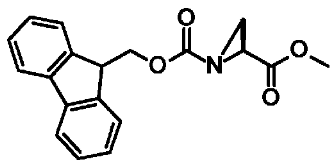

To a solution of commercially available methyl (S)-1-tritylaziridine-2-carboxylate (Trt-Azy-OMe) (50 g, 145.60 mmol) in chloroform/methanol (145 mL/145 mL) was added trifluoroacetic acid (33 mL) dropwise at 0°C under a nitrogen atmosphere, and the reaction solution was stirred for 7 h. To the reaction solution was added a solution of N,N-diisopropylethylamine (DIPEA) (127 mL) and (9H-fluoren-9-yl)methyl carbonochloridate (Fmoc-Cl) (36 g, 139.16 mmol) in 1,4-dioxane (145 mL) dropwise at 0°C, and the reaction solution was stirred at 0°C for 1 hour and 30 minutes. The solvent was evaporated from the reaction solution under reduced pressure, and the reaction solution was diluted with ethyl acetate and then washed with water, with a saturated aqueous ammonium chloride solution twice, and with brine twice. The resulting organic layer was dried over anhydrous sodium sulfate and filtered, and then the solvent was evaporated under reduced pressure. The resulting residue was purified by normal phase column chromatography (petroleum ether/ethyl acetate) to afford 1-((9H-fluoren-9-yl)methyl) 2-methyl (S)-aziridine-1,2-dicarboxylate (Compound aa204, Fmoc-Azy-OMe) (40 g, 85% over two steps).
LCMS (ESI) m/z = 324 (M+H)+
Retention time: 0.86 min (analytical condition SQDFA05)

Synthesis of methyl (S)-2-(1,3-dioxoisoindolin-2-yl)-6-oxohexanoate (Compound aa205)

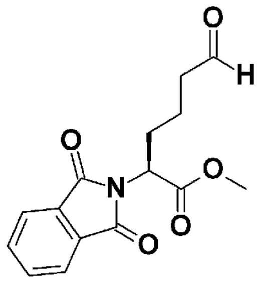

To a solution of commercially available (S)-2-((tert-butoxycarbonyl)amino)-6-hydroxyhexanoic acid (Boc-Nle(6-OH)-OH) (10 g, 40.4 mmol) in toluene/methanol (90 mL/60 mL) was added a 2 M (trimethylsilyl)diazomethane/hexane solution (24.26 mL, 48.5 mmol) dropwise at room temperature under a nitrogen atmosphere, and the mixture was stirred overnight. The reaction solution was concentrated under reduced pressure using a rotary evaporator and the resulting residue was dissolved by adding a toluene/methanol solution (90 mL/60 mL), after which a 2 M (trimethylsilyl)diazomethane/hexane solution (24.26 mL, 48.5 mmol) was added dropwise at room temperature and the mixture was stirred for 6 h. The reaction solution was concentrated under reduced pressure using a rotary evaporator to afford methyl (S)-2-((tert-butoxycarbonyl)amino)-6-hydroxyhexanoate (Boc-Nle(6-OH)-OMe) (13 g) as a crude product.

To the resulting crude product methyl (S)-2-((tert-butoxycarbonyl)amino)-6-hydroxyhexanoate (Boc-Nle(6-OH)-OMe) (13 g) was added a 4 N hydrochloric acid/1,4-dioxane solution (50 mL, 200 mmol) at room temperature, and the mixture was stirred for 6 h. The reaction solution was concentrated under reduced pressure using a rotary evaporator to afford methyl (S)-2-amino-6-hydroxyhexanoate hydrochloride (H-Nle(6-OH)-OMe) (9.2 g) as a crude product.

To a solution of the resulting crude product methyl (S)-2-amino-6-hydroxyhexanoate hydrochloride (H-Nle(6-OH)-OMe) (9.2 g) in acetonitrile (100 mL) were added ethyl 1,3-dioxoisoindoline-2-carboxylate (9.74 g, 44.4 mmol) and N,N-diisopropylethylamine (DIPEA) (15.52 mL, 89 mmol) at room temperature, and the mixture was stirred overnight. The reaction solution was concentrated under reduced pressure using a rotary evaporator, and the resulting residue was purified by normal phase column chromatography (dichloromethane/methanol) to afford methyl (S)-2-(1,3-dioxoisoindolin-2-yl)-6-hydroxyhexanoate (14.6 g).

To a solution of the above methyl (S)-2-(1,3-dioxoisoindolin-2-yl)-6-hydroxyhexanoate (7.08 g, 24.3 mmol) in dichloromethane (100 mL) was added Dess-Martin periodinane (CAS #87413-09-0, 11.34 g, 26.7 mmol) at 0°C, and the mixture was stirred at room temperature for 3 h. The reaction solution was diluted with dichloromethane and washed with a solution of saturated aqueous sodium bicarbonate solution/water (1/1), saturated sodium thiosulfate, and brine. The organic layer was dried over anhydrous sodium sulfate, filtered, and then concentrated under reduced pressure. The resulting residue was purified by normal phase column chromatography (hexane/ethyl acetate) to afford methyl (S)-2-(1,3-dioxoisoindolin-2-yl)-6-oxohexanoate (Compound aa205) (3.6 g, 51% over four steps).
LCMS (ESI) m/z = 290 (M+H)+
Retention time: 0.70 min (analytical condition SQDAA05)

Synthesis of methyl (2S)-2-(1,3-dioxoisoindolin-2-yl)-7,7 ,7-trifluoro-6-hydroxyheptanoate (Compound aa206)

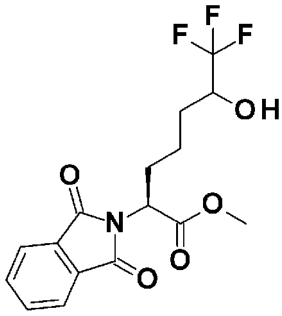

To a solution of methyl (S)-2-(1,3-dioxoisoindolin-2-yl)-6-oxohexanoate (Compound aa205) (3.62 g, 12.51 mmol) in tetrahydrofuran (50 mL) were added (trifluoromethyl)trimethylsilane (1.390 mL, 9.39 mmol) and a 1 M tetrabutylammonium fluoride (TBAF)/tetrahydrofuran (THF) solution (0.626 mL, 0.626 mmol) at 0°C under a nitrogen atmosphere, and the mixture was stirred at 0°C for 10 min. To the reaction solution were added (trifluoromethyl)trimethylsilane (1.390 mL, 9.39 mmol) and a 1 M tetrabutylammonium fluoride (TBAF)/tetrahydrofuran (THF) solution (0.626 mL, 0.626 mmol) at 0°C, and the mixture was stirred for 30 min. To the reaction solution were further added (trifluoromethyl)trimethylsilane (1.390 mL, 9.39 mmol) and a 1 M tetrabutylammonium fluoride (TBAF)/tetrahydrofuran (THF) solution (0.626 mL, 0.626 mmol) at 0°C, and the mixture was stirred for 2 hours and 30 minutes. To the reaction solution was added a 1 N aqueous hydrochloric acid solution (37.5 mL) at 0°C, and the mixture was stirred at room temperature for 20 min and then extracted with ethyl acetate. The organic layer was washed with brine, dried over anhydrous sodium sulfate, filtered, and concentrated under reduced pressure. The resulting residue was purified by reverse phase column chromatography (10 mM aqueous ammonium acetate solution/methanol) to afford methyl (2S)-2-(1,3-dioxoisoindolin-2-yl)-7,7,7-trifluoro-6-hydroxyheptanoate (Compound aa206) (1.8 g, 40%).
LCMS (ESI) m/z = 358 (M-H)-
Retention time: 0.81 min (analytical condition SQDAA05)

Synthesis of Methyl (2S)-2-((((9H-fluoren-9-yl)methoxy)carbonyl)amino)-7,7,7-trifluoro-6-hydroxyheptanoate (Compound aa207, Fmoc-Hnl(7-F3-6-OH)-OMe)

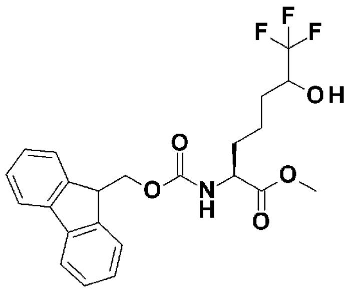

To a solution of methyl (2S)-2-(1,3-dioxoisoindolin-2-yl)-7,7,7-trifluoro-6-hydroxyheptanoate (Compound aa206) obtained by the method described above (3.1 g, 8.63 mmol) in methanol (30 mL) were added hydrazine monohydrate (1.258 mL, 25.9 mmol) and acetic acid (1.482 mL, 25.9 mmol) at room temperature, and the mixture was stirred overnight. The reaction solution was concentrated under reduced pressure using a rotary evaporator to remove the methanol, and the resulting solution was diluted with dimethyl sulfoxide and then purified by reverse phase column chromatography (10 mM aqueous ammonium acetate solution/methanol) to afford methyl (2S)-2-amino-7,7,7-trifluoro-6-hydroxyheptanoate (H-Hnl(7-F3-6-OH)-OMe) (2 g).

To the above methyl (2S)-2-amino-7,7,7-trifluoro-6-hydroxyheptanoate (H-Hnl(7-F3-6-OH)-OMe) (2 g, 8.73 mmol) were added water (25 mL), sodium carbonate (2.93 g, 34.9 mmol), tetrahydrofuran (50 mL), and 9-fluorenylmethyl N-succinimidyl carbonate (Fmoc-OSu) (3.53 g, 10.47 mmol) at room temperature, and the reaction solution was stirred

for 2 h. The reaction solution was concentrated under reduced pressure using a rotary evaporator to remove the tetrahydrofuran, ethyl acetate and 1 N aqueous hydrochloric acid solution were added, and the mixture was extracted with ethyl acetate twice. The organic layers were washed with brine, dried over anhydrous magnesium sulfate, then filtered, and concentrated under reduced pressure. The resulting residue was purified by reverse phase column chromatography (0.1% aqueous formic acid solution/0.1% formic acid-acetonitrile solution) and normal phase column chromatography (dichloromethane/methanol) and then further purified by reverse phase column chromatography (10 mM aqueous ammonium acetate solution/methanol). The resulting fractions were collected, the methanol was evaporated under reduced pressure, and the fractions were extracted with ethyl acetate twice and washed with a saturated potassium bisulfate solution and brine. The resulting organic solvent was evaporated under reduced pressure to afford methyl (2S)-2-((((9H-fluoren-9-yl)methoxy)carbonyl)amino)-7,7,7-trifluoro-6-hydroxyheptanoate (Compound aa207, Fmoc-Hnl(7-F3-6-OH)-OMe) (1.4 g, 36% over two steps).
LCMS (ESI) m/z = 452 (M+H)+
Retention time: 0.86 min (analytical condition SQDFA05)

Synthesis of (2S)-2-((((9H-fluoren-9-yl)methoxy)carbonyl)amino)-7,7,7-trifluoro-6-hydroxyheptanoic acid (Compound aa208, Fmoc-Hnl(7-F3-6-OH)-OH)

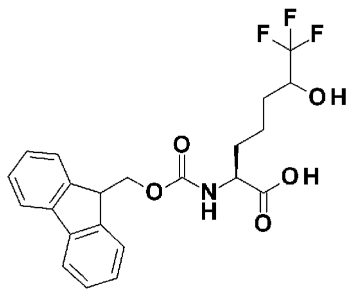

To a solution of calcium chloride (5.16 g, 46.5 mmol) in water (7.00 mL) was added lithium hydroxide monohydrate (0.521 g, 12.40 mmol) at room temperature, and the mixture was stirred for 5 min. A solution of methyl (2S)-2-((((9H-fluoren-9-yl)methoxy)carbonyl)amino)-7,7,7-trifluoro-6-hydroxyheptanoate (Compound aa207, Fmoc-Hnl(7-F3-6-OH)-OMe) (1.4 g, 3.10 mmol) in isopropanol/tetrahydrofuran (28 mL/7 mL) was added dropwise at room temperature, and the reaction solution was stirred overnight. To the reaction solution was added a 1 N aqueous hydrochloric acid solution, and the reaction solution was extracted with t-butyl methyl ether twice, washed with brine, dried over anhydrous magnesium sulfate, then filtered, and concentrated under reduced pressure. The resulting residue was purified by reverse phase column chromatography (0.1% aqueous formic acid solution/0.1% formic acid-acetonitrile solution) to afford (2S)-2-((((9H-fluoren-9-yl)methoxy)carbonyl)amino)-7,7,7-trifluoro-6-hydroxyheptanoic acid (Compound aa208, Fmoc-Hnl(7-F3-6-OH)-OH) (950 mg, 70%).
LCMS (ESI) m/z = 438.6 (M+H)+
Retention time: 0.76 min (analytical condition SQDFA05)

1-3. Synthesis of resins used for peptide synthesis by a peptide synthesizer

Resins used for peptide synthesis by a peptide synthesizer were synthesized as described below. 2-Chlorotrityl chloride resin (100-200 mesh, 1% DVB) was purchased from Watanabe Chemical Industries and Chem-Impex.

Synthesis of (S)-3-((((9H-fluoren-9-yl)methoxy)carbonyl)amino)-4-oxo-4-(piperidin-1-yl)butanoic acid-2-chlorotrityl resin (Compound pd01, Fmoc-Asp(O-Trt(2-Cl)-resin)-pip)

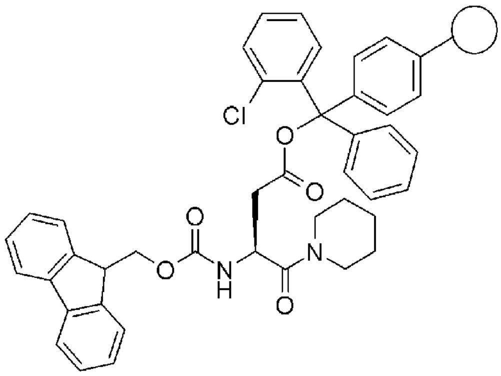

(S)-3-((((9H-Fluoren-9-yl)methoxy)carbonyl)amino)-4-oxo-4-(piperidin-1-yl)butanoic acid-2-chlorotrityl resin (Compound pd01, Fmoc-Asp(O-Trt(2-Cl)-resin)-pip) was synthesized by the method described in WO 2013/100132.

In the present specification, when a polymer or resin is attached to a compound, the polymer or resin site may be represented with "○." In order to specify the point of reaction in the resin site, the chemical structure of the reaction site may be represented as a structure connected to "○." The above structure shows a manner in which the 2-chlorotrityl group on the resin is attached to the side chain carboxylic acid of Asp through an ester bond in Fmoc-Asp(O-Trt(2-Cl)-resin)-pip (Compound pd01).

Synthesis of (S)-3-((((9H-fluoren-9-yl)methoxy)carbonyl)(methyl)amino)-4-oxo-4-(pipendin-1-yl)butanoic acid-2-chlorotrityl resin (Compound pd07, Fmoc-MeAsp(O-Trt(2-Cl)-Resin)-pip)

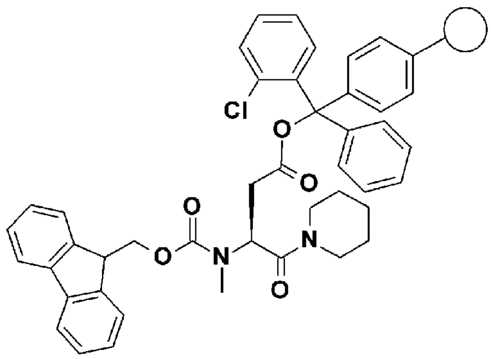

(S)-3-((((9H-Fluoren-9-yl)methoxy)carbonyl)(methyl)amino)-4-oxo-4-(piperidin-1-yl)butanoic acid-2-chlorotrityl resin (Compound pd07, Fmoc-MeAsp(O-Trt(2-Cl)-Resin)-pip) was synthesized by the following route.

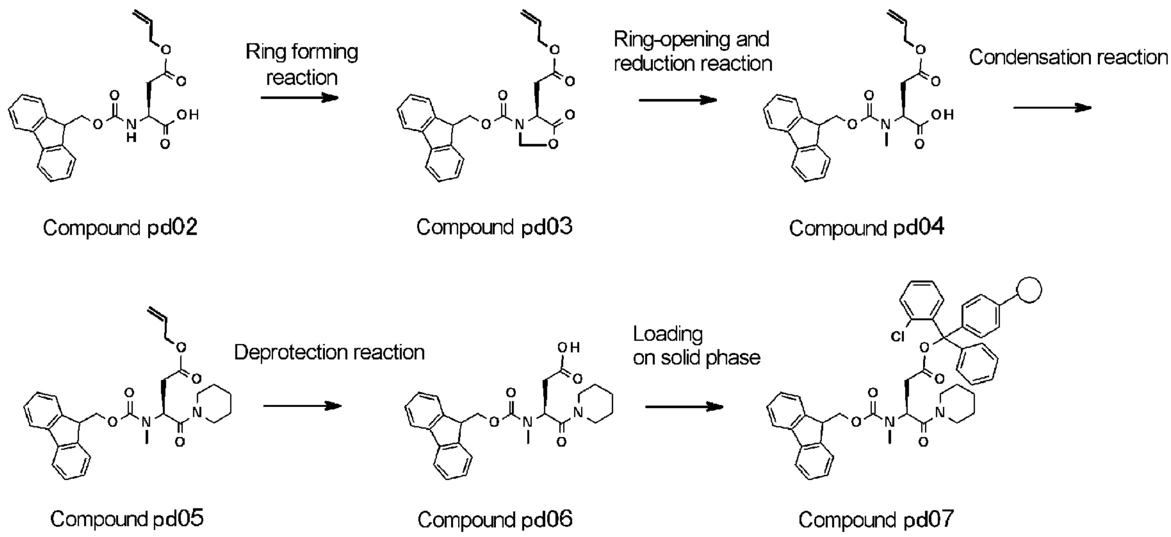


Compound pd02 Compound pd03 Compound pd04

Compound pd05 Compound pd06 Compound pd07

Synthesis of (S)-2-((((9H-fluoren-9-yl)methoxy)carbonyl)(methyl)amino)-4-(allyloxy)-4-oxobutanoic acid (Compound pd04, Fmoc-MeAsp(OAl)-OH)

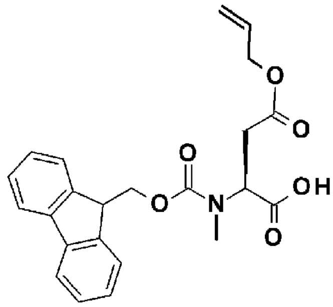

To a solution of (S)-2-((((9H-fluoren-9-yl)methoxy)carbonyl)amino)-4-(allyloxy)-4-oxobutanoic acid (Compound pd02, Fmoc-Asp(OAl)-OH) (10.0 g, 25.3 mmol) in toluene (100 mL) were added paraformaldehyde (1.52 g) and tosylic acid (TsOH, 260 mg, 1.51 mmol) at room temperature under a nitrogen atmosphere, and the mixture was stirred at 110°C for 16 h. The reaction solution was then cooled to room temperature and washed with a saturated aqueous sodium bicarbonate solution twice. The resulting organic layers were dried over anhydrous sodium sulfate, filtered, and concentrated under reduced pressure to afford (9H-fluoren-9-yl)methyl (S)-4-(2-(allyloxy)-2-oxoethyl)-5-oxooxazolidine-3-carboxylate (Compound pd03, 8.0 g) as a crude product.

A solution of (9H-fluoren-9-yl)methyl (S)-4-(2-(allyloxy)-2-oxoethyl)-5-oxooxazolidine-3-carboxylate obtained in the previous step (Compound pd03, 5.0 g, 12.3 mmol) and triethylsilane ($Et_3SiH$, 4.3 g, 37.0 mmol) in dichloromethane/trifluoroacetic acid (TFA) = 1/1 (80 mL/80 mL) was stirred at room temperature for two days under a nitrogen atmosphere, after which the reaction solution was concentrated under reduced pressure. To the resulting residue was added an aqueous potassium carbonate ($K_2CO_3$) solution. After washing with petroleum ether three times, the reaction solution was adjusted to pH 3 with hydrochloric acid and extracted with ethyl acetate three times. The organic layers were combined, dried over anhydrous sodium sulfate, filtered, and concentrated under reduced pressure. The resulting residue was purified by reverse phase silica gel column chromatography (0.1% aqueous formic acid solution/0.1% formic acid-acetonitrile) to afford (S)-2-((((9H-fluoren-9-yl)methoxy)carbonyl)(methyl)amino)-4-(allyloxy)-4-oxobutanoic acid (Compound pd04, Fmoc-MeAsp(OAl)-OH) (3.0 g, 46%, two steps).
LCMS (ESI) m/z = 410 (M+H)+
Retention time: 0.84 min (analytical condition SQDFA05)

Synthesis of allyl (S)-3-((((9H-fluoren-9-yl)methoxy)carbonyl)(methyl)amino)-4-oxo-4-(piperidin-1-yl)butanoate (Compound pd05, Fmoc-MeAsp(OAl)-pip)

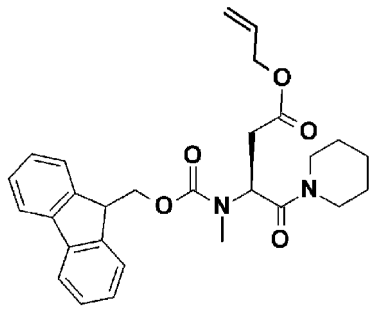

To a solution of 1-ethyl-3-(3-dimethylaminopropyl)carbodiimide hydrochloride (WSCI·HCl) (1.6.5 g, 86 mmol) in DMF (143 mL) was added 1-hydroxybenzotriazole (HOBt) (10.6 g, 79 mmo) at 0°C under a nitrogen atmosphere. Subsequently, a mixed solution of (S)-2-((((9H-fluoren-9-yl)methoxy)carbonyl)(methyl)amino)-4-(allyloxy)-4-oxobutanoic acid (Compound pd04, Fmoc-MeAsp(OAl)-OH) (29.3 g, 71.6 mmol) in DMF/DCM = 1/1 (117 mL) was added dropwise at 0°C, and the mixture was stirred for 30 min. Piperidine (8.49 mL, 86 mmol) was then added dropwise at 0°C and the mixture was stirred for 30 min. After the progress of the reaction was confirmed by LC-MS, ethyl acetate was added to the reaction solution, and the solution was warmed to room temperature. The resulting organic layer was washed with a 2 M aqueous hydrochloric acid solution twice, with a 5% aqueous sodium bicarbonate solution twice, and with brine twice, and then dried over magnesium sulfate. The resulting mixture was filtered and concentrated under reduced pressure to afford allyl (S)-3-((((9H-fluoren-9-yl)methoxy)carbonyl)(methyl)amino)-4-oxo-4-(piperidin-1-yl)butanoate (Compound pd05, Fmoc-MeAsp(OAl)-pip) (33.7 g, 99%).
LCMS (ESI) m/z = 477 (M+H)+
Retention time: 1.32 min (analytical condition SMD method 6)

Synthesis of (S)-3-((((9H-fluoren-9-yl)methoxy)carbonyl)(methyl)amino)-4-oxo-4-(piperidin-1-vl)butanoic acid (Compound pd06, Fmoc-MeAsp-pip)

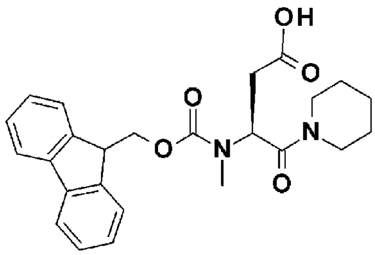

Dichloromethane (132 mL) was added to allyl (S)-3-((((9H-fluoren-9-yl)methoxy)carbonyl)(methyl)amino)-4-oxo-4-(piperidin-1-yl)butanoate (Compound pd05, Fmoc-MeAsp(OAl)-pip) (31.4 g, 65.9 mmol), sodium 4-methylbenzenesulfinate (11.2 g, 62.6 mmol), and tetrakistriphenylphosphine palladium (Pd(PPh3)4) (761 mg, 0.659 mmol) under a nitrogen atmosphere, and the mixture was stirred at room temperature for 1.5 h. To the reaction solution was then added a 5% aqueous sodium bicarbonate solution, and the reaction solution was washed with t-butyl methyl ether (TBME) twice. The resulting aqueous layer was adjusted to an acidic pH with a 6 M aqueous hydrochloric acid solution and then extracted with ethyl acetate. The resulting organic layer was washed with 50% saline twice and dried over anhydrous magnesium sulfate, after which the resulting mixture was filtered and concentrated under reduced pressure. The resulting residue was purified by reverse phase silica gel column chromatography (0.1% aqueous formic acid solution/0.1% formic acid-acetonitrile) and further purified by normal phase silica gel column chromatography ($CO_2H$ silica gel, hexane/ethyl acetate) to afford (S)-3-((((9H-fluoren-9-yl)methoxy)carbonyl)(methyl)amino)-4-oxo-4-(piperidin-1-yl)butanoic acid (Compound pd06, Fmoc-MeAsp-pip) (17.1 g, 60%).
LCMS (ESI) m/z = 437 (M+H)+
Retention time: 1.10 min (analytical condition SMD method 6)

Synthesis of (S)-3-((((9H-fluoren-9-yl)methoxy)carbonyl)(methyl)amino)-4-oxo-4-(piperidin-1-vl)butanoic acid-2-chlorotrityl resin (Compound pd07, Fmoc-MeAsp(O-Trt(2-Cl)-resin)-pip)

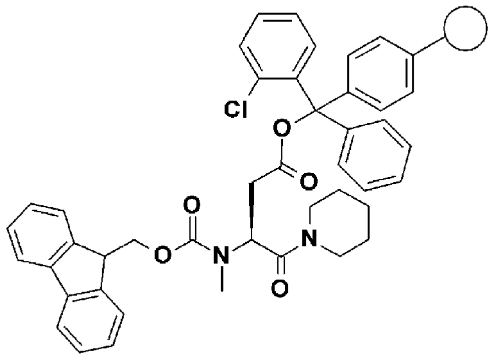

In a reaction vessel with a filter was placed 2-chlorotrityl chloride resin (1.60 mmol/g, 100-200 mesh, 1% DVB, purchased from Watanabe Chemical Industries, 25 g, 40.0 mmol) and dehydrated dichloromethane (400 mL), and the vessel was shaken at room temperature for 10 min. The dichloromethane was removed by applying nitrogen pressure, after which dehydrated methanol (6.48 mL) and diisopropylethylamine (DIPEA) (16.7 mL) were added to (S)-3-((((9H-fluoren-9-yl)methoxy)carbonyl)(methyl)amino)-4-oxo-4-(piperidin-1-yl)butanoic acid (Compound pd06, Fmoc-MeAsp-pip) (8.37 g, 20.0 mmol) and dehydrated dichloromethane (400 mL), the resulting mixture was added to the reaction vessel, and the vessel was shaken for 30 min. The reaction solution was removed by applying nitrogen pressure, after which dehydrated methanol (50.0 mL) and diisopropylethylamine (DIPEA) (16.7 mL) were added to dehydrated dichloromethane (400 mL), the resulting mixture was added to the reaction vessel, and the vessel was shaken for 1 hour and 30 minutes. The reaction solution was removed by applying nitrogen pressure, after which dichloromethane was placed in the vessel, followed by shaking for 5 min. The reaction solution was removed by applying nitrogen pressure. This washing of the resin with dichloromethane was repeated twice, and the resulting resin was dried under reduced pressure overnight to afford (S)-3-((((9H-fluoren-9-yl)methoxy)carbonyl)(methyl)amino)-4-oxo-4-(piperidin-1-yl)butanoic acid-2-chlorotrityl resin (Compound pd07, Fmoc-MeAsp(O-Trt(2-Cl)-resin)-pip) (29.9 g).

The resulting (S)-3-((((9H-fluoren-9-yl)methoxy)carbonyl)(methyl)amino)-4-oxo-4-(piperidin-1-yl)butanoic acid-2-chlorotrityl resin (Compound pd07, Fmoc-MeAsp(O-Trt(2-Cl)-resin)-pip) (10.7 mg) was placed in a reaction vessel, DMF (200 $\mu$L) and piperidine (200 $\mu$L) were added, and the vessel was shaken at room temperature for 1 h. To the reaction mixture was then added DMF (1.6 mL), 400 $\mu$L of the mixture was taken out, its absorbance (301.2 nm) was measured (using Shimadzu, UV-1600PC (cell length: 1.0 cm)), and the loading amount of (S)-3-((((9H-fluoren-9-yl)methoxy)carbonyl)(methyl)amino)-4-oxo-4-(piperidin-1-yl)butanoic acid-2-chlorotrityl resin (Compound pd07, Fmoc-MeAsp(O-Trt(2-Cl)-resin)-pip) was calculated to be 0.416 mmol/g.

Another lot similarly synthesized with a different loading amount was also used for peptide synthesis.

Synthesis of (S)-3-((((9H-fluoren-9-yl)methoxy)carbonyl)amino)-4-(methyl((S)-1-oxo-1-(((S)-1-oxo-1-(piperidin-1-yl)propan-2-yl)amino)-3-phenylpropan-2-yl)amino)-4-oxobutanoic acid-2-chlorotrityl resin (Compound pd08, Fmoc-Asp(O-Trt(2-Cl)-resin)-MePhe-Ala-pip)

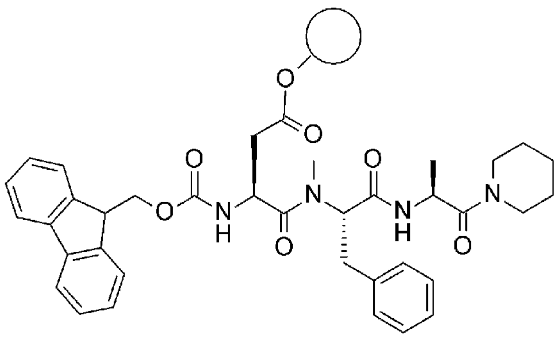

(S)-3-((((9H-Fluoren-9-yl)methoxy)carbonyl)amino)-4-(methyl((S)-1-oxo-1-(((S)-1-oxo-1-(piperidin-1-yl)propan-2-yl)amino)-3-phenylpropan-2-yl)amino)-4-oxobutanoic acid-2-chlorotrityl resin (Compound pd08, Fmoc-Asp(O-Trt(2-Cl)-resin)-MePhe-Ala-pip) was synthesized by the method described in the document (Document: International Publication No. WO 2013/100132A1).

Synthesis of (S)-3-((((9H-fluoren-9-yl)methoxy)carbonyl)amino)-4-(methyl((S)-1-oxo-1-(((S)-1-oxo-3-phenyl-1-(piperidin-1-yl)propan-2-yl)amino)-3-phenylpropan-2-yl)amino)-4-oxobutanoic acid-2-chlorotrityl resin (Compound pd09, Fmoc-Asp(O-Trt(2-Cl)-resin)-MePhe-Phe-pip)

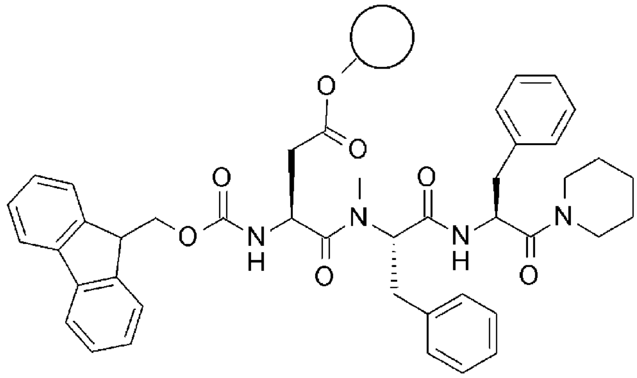

(S)-3-((((9H-Fluoren-9-yl)methoxy)carbonyl)amino)-4-(methyl((S)-1-oxo-1-(((S)-1-oxo-3-phenyl-1-(piperidin-1-yl)propan-2-yl)amino)-3-phenylpropan-2-yl)amino)-4-oxobutanoic acid-2-chlorotrityl resin (Compound pd09, Fmoc-Asp(O-Trt(2-Cl)-resin)-MePhe-Phe-pip) was synthesized by a similar procedure as for Compound pd08 using the method described in the document (Document: International Publication No. WO 2013/100132A1).

Synthesis of (S)-3-((((9H-fluoren-9-yl)methoxy)carbonyl)amino)-4-(methyl((S)-1-oxo-1-(((S)-1-oxo-1-(((S)-1-oxo-1-(piperidin-1-yl)propan-2-yl)amino)-3-phenylpropan-2-yl)amino)-3-phenylpropan-2-yl)amino)-4-oxobutanoic acid-2-chlorotrityl resin (Compound pd10, Fmoc-Asp(O-Trt(2-Cl)-resin)-MePhe-MePhe-Ala-pip)

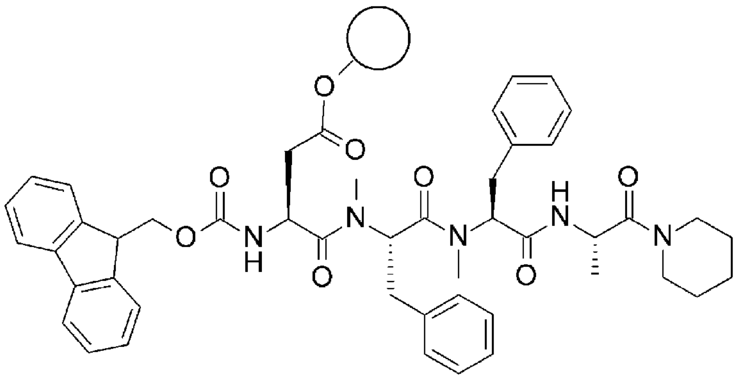

(S)-3-((((9H-Fluoren-9-yl)methoxy)carbonyl)amino)-4-(methyl((S)-1-oxo-1-(((S)-1-oxo-1-(((S)-1-oxo-1-(piperidin-1-yl)propan-2-yl)amino)-3-phenylpropan-2-yl)amino)-3-phenylpropan-2-yl)amino)-4-oxobutanoic acid-2-chlorotrityl resin (Compound pd10, Fmoc-Asp(O-Trt(2-Cl)-resin)-MePhe-MePhe-Ala-pip) was synthesized by the method described in WO 2013/100132.

1-4. Chemical synthesis of peptides

Unless otherwise stated, peptide compounds pd50 to pd70, pd100 to pd247, and pd300 to pd504 were synthesized by the basic route described at the beginning of Example 1 according to the following method.

1) Solid-phase synthesis of peptides by an automated synthesizer

Peptides were synthesized by the Fmoc method described in WO 2013/100132 using a peptide synthesizer (Multipep RS; manufactured by Intavis). The manual attached to the synthesizer was followed for the detailed operational procedure.

In the synthesizer was placed 2-chlorotrityl resin (100 mg per column) to which was attached the side chain carboxylic acid site of aspartic acid with the N-terminus protected by Fmoc; various Fmoc amino acids (0.6 mol/L; prepared as a hydrochloride by the following method and then used as a 0.5 mol/L solution in the case of Fmoc-MeHis(Trt)-OH (Compound aa05)); a solution of 1-hydroxy-7-azabenzotriazole (HOAt) or oxyma (0.375 mol/L) in NMP; and a solution (10% v/v) of diisopropylcarbodiimide (DIC) in N,N-dimethylformamide (DMF). Fmoc-Thr(THP)-OH (Compound aa01) and Fmoc-MeSer(THP)-OH (Compound aa06) were allowed to coexist with oxyma in the NMP solution, to which molecular sieves 4A1/8 (Wako Pure Chemical Industries) or molecular sieves 4A1/16 (Wako Pure Chemical Industries) were further added and placed in the synthesizer. When an amino acid salt such as hydrochloride (e.g., Fmoc-MeHis(Trt)-OH (Compound aa05) hydrochloride) was used, a DIPEA/DMF = 3/1 solution was placed, and 0.96 equivalent of DIPEA relative to the amino acid used for peptide elongation was separately added as the above DMF solution.

Fmoc-MeHis(Trt)-OH (Compound aa05) hydrochloride was prepared by the following method.

To Fmoc-MeHis(Trt)-OH (Compound aa05) (1.166 g) was added DCM (10.5 mL), after which a solution obtained by diluting 4 N HCl/1,4-dioxane (0.88 mL) with DCM (5.3 mL) was added dropwise at 0°C. The reaction solution was then stirred at 0°C for 5 min and concentrated under reduced pressure using a rotary evaporator. The resulting residue was dried under reduced pressure using an oil pump to afford Fmoc-MeHis(Trt)-OH (Compound aa05) as a hydrochloride. NMP (2.21 mL) was then added to the compound to prepare a 0.5 mol/L solution.

Synthesis was performed using a solution (2% v/v) of diazabicycloundecene (DBU) in DMF as an Fmoc deprotection solution. One cycle consisting of washing of the resin with DMF, Fmoc deprotection, and Fmoc amino acid condensation reaction was repeated to elongate a peptide on the resin surface. After completion of the peptide elongation, the Fmoc group at the N-terminus of the resin was removed on the peptide synthesizer, and the resin was then washed with DMF.

2) Cleavage of the elongated peptide from the resin

The resin was swollen again according to the method described in WO 2013/100132 by adding DCM to the linear peptide loaded on the solid phase obtained by the above method, and 2,2,2-trifluoroethanol (TFE)/DCM (1/1, v/v, 2 mL) was then added to the resin, followed by shaking at room temperature for 2 h. The solution in the tube was then filtered using a synthesis column to remove the resin, and the remaining resin was further washed with 2,2,2-trifluoroethanol (TFE)/DCM (1/1, v/v, 1 mL) twice. All the resulting cleaving solutions were combined and concentrated under reduced pressure.

3) Method for cyclizing the cleaved peptide

After the cleavage, concentration under reduced pressure gave a residue, which was then dissolved in DMF/DCM (1/1, v/v, 8 mL). A 0.5 M O-(7-aza-1H-benzotriazol-1-yl)-N,N,N,N-tetramethyluronium hexafluorophosphate (HATU)/DMF solution (1.5 volume equivalents relative to the number of moles (the loading amount (mmol/g) multiplied by the amount of the resin used (usually 0.10 g)) on the resin used) and DIPEA (1.8 equivalents relative to the number of moles on the resin used) were added, and the tube was shaken at room temperature for 2 h. The solvent was then evaporated under reduced pressure. Generation of the intended cyclic peptide was confirmed by LCMS measurement.

4) 5) Deprotection of the protecting group for the side chain functional group possessed by the cyclic peptide

When the sequence contained Tyr(3-F,tBu), 2 mL of the prepared 0.1 M tetramethylammonium hydrogen sulfate/1,1,1,3,3,3-hexafluoroisopropyl alcohol (HFIP) solution (2% triisopropylsilane (TIPS)) was added to dissolve the residue, and the resulting solution was then allowed to stand at room temperature or 30°C for 24 h. When the sequence did not contain Tyr(3-F), 4 mL of the prepared 0.05 M tetramethylammonium hydrogen sulfate/1,1,1,3,3,3-hexafluoroisopropyl alcohol (HFIP) solution (2% triisopropylsilane (TIPS)) was added to dissolve the residue, and the resulting solution was then allowed to stand at room temperature for 4 h. In both cases, after allowing to stand for a certain period of time, diisopropylethylamine (DIPEA) (70 μL) was added and the solvent was evaporated under reduced pressure.

The solvent was evaporated under reduced pressure, DMF or DMSO was then added, the insoluble matter

was removed by filtration through a filter, and the residue was then purified by preparative HPLC.

The 0.1 M tetramethylammonium hydrogen sulfate/HFIP solution (2% TIPS) was prepared by dissolving 68.5 mg of tetrabutylammonium hydrogen sulfate in 4 mL of a solution taken out from a mixed solution of HFIP (11.66 mL), TIPS (0.24 mL), and DCE (0.10 mL). The 0.05 M tetramethylammonium hydrogen sulfate/HFIP solution (2% TIPS) was prepared by dissolving 34.3 mg of tetrabutylammonium hydrogen sulfate in 4 mL of a solution taken out from a mixed solution of HFIP (11.66 mL), TIPS (0.24 mL), and DCE (0.10 mL). Other fluoroalcohols such as 2,2,2-trifluoroethanol (TFE) may also be used instead of HFIP for these solutions (a 0.1 M tetramethylammonium hydrogen sulfate/HFIP solution (2% TIPS) and a 0.05 M tetramethylammonium hydrogen sulfate/HFIP solution (2% TIPS)).

Synthesis of three-residue peptides used for pKa measurement

Peptides were elongated and cleaved from the resin by a similar procedure as in the chemical synthesis of peptide compounds described in Example 1, after which the C-terminal carboxylic acid was condensed with piperidine to synthesize pd30 to pd36. Fmoc-MeGly-Trt(2-Cl)-resin (Compound pd11) used for peptide synthesis was synthesized as follows. ZMeGly (Cbz-MeGly-OH, CAS #39608-31-6) was purchased from Tokyo Chemical Industry.

Synthesis of N-(((9H-fluoren-9-yl)methoxy)carbonyl)-N-methylglycine-2-chlorotrityl resin (Compound pd11, Fmoc-MeGly-Trt(2-Cl)-resin)

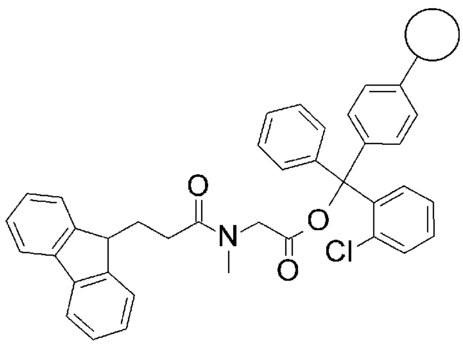

In a reaction vessel with a filter was placed 2-chlorotrityl chloride resin (1.58 mmol/g, 100-200 mesh, 1% DVB, purchased from Watanabe Chemical Industries, 10 g, 15.8 mmol) and dehydrated dichloromethane, and the vessel was shaken at room temperature for 1 h. The dichloromethane was removed by applying nitrogen pressure, after which a solution of commercially available N-(((9H-fluoren-9-yl)methoxy)carbonyl)-N-methylglycine (Fmoc-MeGly-OH) (3.54 g, 11.39 mmol) and diisopropylethylamine (DIPEA) (5.29 mL, 30.4 mmol) in dehydrated dichloromethane (130 mL) was added to the reaction vessel, and the vessel was shaken for 30 min. The reaction solution was removed by applying nitrogen pressure, after which dehydrated methanol (5.76 mL) and diisopropylethylamine (DIPEA) (5.29 mL, 30.4 mmol) were added to dehydrated dichloromethane (130 mL), the resulting mixture was added to the reaction vessel, and the vessel was shaken for 1 h. The reaction solution was removed by applying nitrogen pressure, after which dichloromethane was placed in the vessel, followed by shaking for 5 min. The reaction solution was removed by applying nitrogen pressure. The resulting resin was dried under reduced pressure overnight to afford N-(((9H-fluoren-9-yl)methoxy)carbonyl)-N-methylglycine-2-chlorotrityl resin (Compound pd11, Fmoc-MeGly-Trt(2-Cl)-resin) (13.2 g).

The loading amount of the resulting resin was calculated using the method described in the document (Letters in Peptie Science, 2002, 9, 203). N-(((9H-Fluoren-9-yl)methoxy)carbonyl)-N-methylglycine-2-chlorotrityl resin (Compound pd11, Fmoc-MeGly-Trt(2-Cl)-resin) (14.4 mg) was placed in a reaction vessel, DMF (2 mL) was added, and the vessel was shaken for 1 h. DBU (0.04 mL) was added to the reaction solution, the vessel was shaken for 30 min, DMF (10 mL) was then added, and 1 mL was taken out and further diluted with DMF so that the amount of the solution was 12.5 mL. The absorbance (294 nm) of the resulting solution was measured (using Shimadzu, UV-1600PC (cell length: 1.0 cm)), and the loading amount of N-(((9H-fluoren-9-yl)methoxy)carbonyl)-N-methylglycine-2-chlorotrityl resin (Compound pd11, Fmoc-MeGly-Trt(2-Cl)-resin) was calculated to be 0.789 mmol/g.

Analytical information on peptide compounds measured for pKa

[Table 5]

| Compound ID | **3** | **2** | **1** | **C-term** | Analytical condition | **LCMS(ESI) m/z** | | Retention time (min) |
|---|---|---|---|---|---|---|---|---|
| pd30 | ZMeGly | Ser(Et-2-NMe2) | MeGly | pip | SQDFA05 | 520 | (M+H)+ | 0.45 |
| pd31 | ZMeGly | Ser(Et-2-Mor) | MeGly | pip | SQDFA05 | 562 | (M+H)+ | 0.45 |
| pd32 | ZMeGly | MeAbu(Mor) | MeGly | pip | SQDFA05 | 546 | (M+H)+ | 0.47 |
| pd33 | ZMeGly | MeAbu(pip-4-F2) | MeGly | pip | SQDFA05 | 580 | (M+H)+ | 0.50 |
| pd34 | ZMeGly | MeAbu(pip-3-F2) | MeGly | pip | SQDFA05 | 580 | (M+H)+ | 0.50 |
| pd35 | ZMeGly | MeAbu(5-Oxo-Odz) | MeGly | pip | SQDFA05 | 545 | (M+H)+ | 0.61 |
| pd36 | ZMeGly | Gln(Ms) | MeGly | pip | SQDFA05 | 568 | (M+H)+ | 0.58 |

Structural information on peptide compounds measured for pKa

[Table 6]

| ID | Structure |
|---|---|
| pd30 | 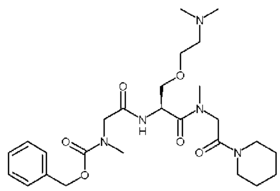 |
| pd31 | 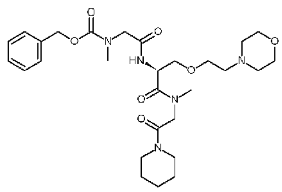 |
| pd32 | 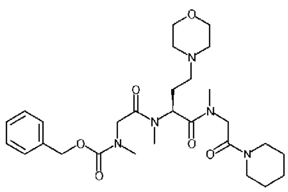 |
| pd33 | 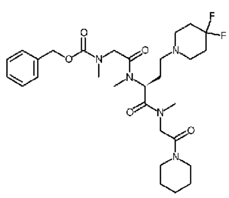 |

(continued)

| ID | Structure |
|---|---|
| pd34 | 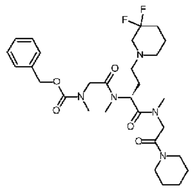 |
| pd35 | 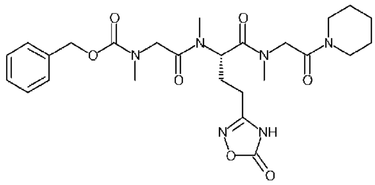 |
| pd36 | 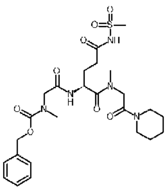 |

[Example 2] Ribosomal synthesis of peptide compounds

2-1. Synthesis of aminoacylated pCpAs

Aminoacylated pCpAs for use in ribosomal translational synthesis (Compounds pc05, pc09, pc14, pc19, pc23, pc25, pc28, pc31, pc35, pc38, pc43, pc44, pc50, pc51, pc52, pc53, pc56, pc59, pc62, pc66, pc70, pc73, pc77, pc82, pc86, pc90, pc93, pc97, pc100, pc104, pc106, pc109, pc113, pc117, pc120, pc121, pc125, pc129, pc132, pc135, pc138, pc140, pc143, pc146, pc150, pc153, pc157, pc160, pc164, pc168, pc171, pc175, pc179, pc183, pc187, pc190, pc194, pc199, pc203, pc205, pc207, pc210, pc212, pc215, pc217, pc220, pc222, pc224, pc228, pc230, pc232, pc233, pc234, pc237, pc238, pc239, pc240, pc243, pc245, and pc248) were synthesized according to the following scheme.

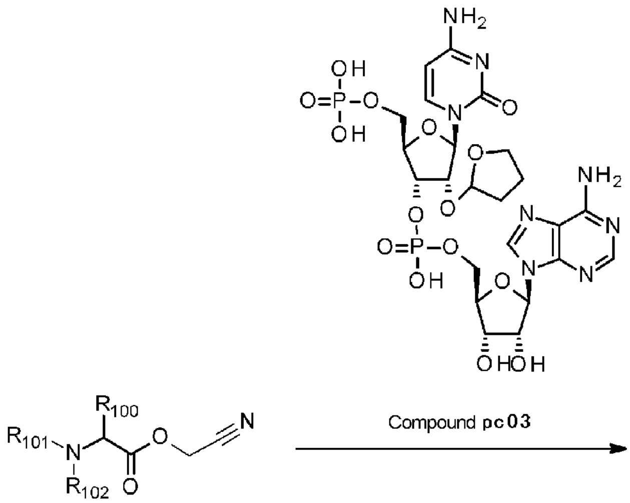


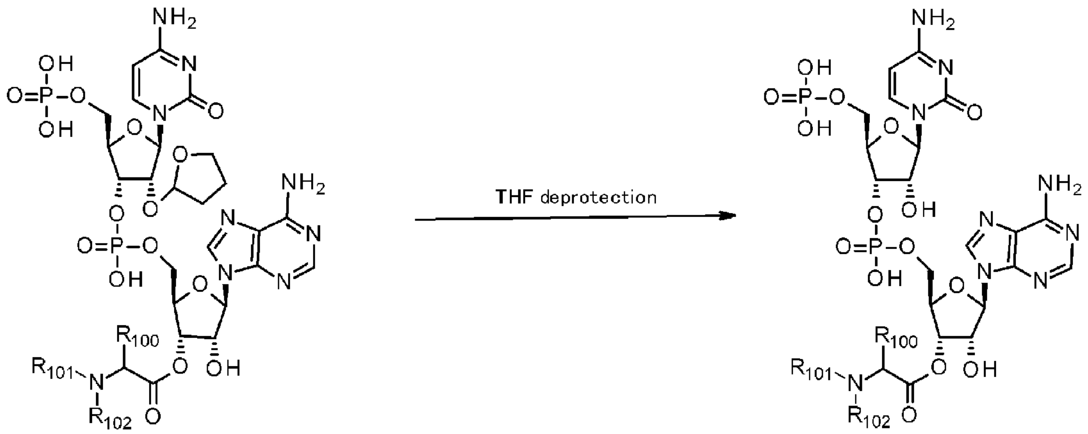


In pCpA amino acids for use in ribosomal synthesis, the ester moiety is present in an equilibrium state described below. Although only either one structure is depicted in the present specification, two equilibrium states can be distinguished and observed depending on the analysis conditions.

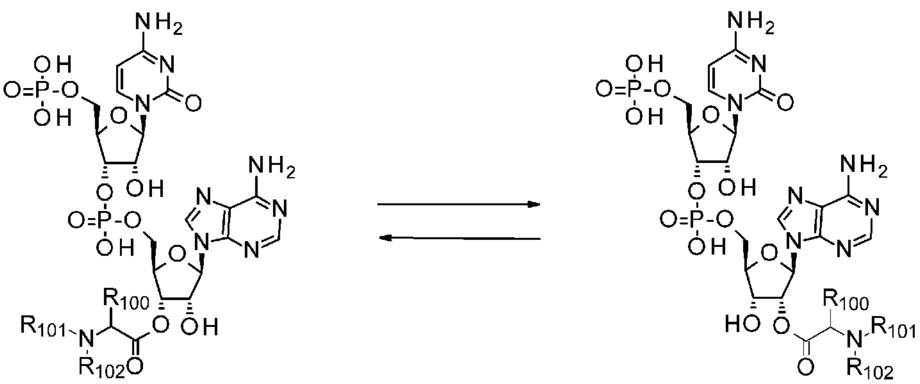

Synthesis of (R)-2-((((4-azidobenzyl)oxy)carbonyl)amino)-3-(tert-butyldisulfanyl)propanoic acid (Compound pc01, Acbz-Cys(StBu)-OH)

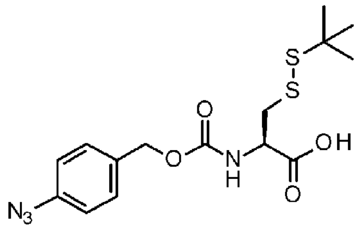

To a mixture of S-tert-butylmercapto-L-cysteine (H-Cys(StBu)-OH) (126 mg, 0.60 mmol) and 4-azidobenzyl (4-nitrophenyl) carbonate synthesized by the method described in the document (Bioconjugate Chem. 2008, 19, 714) (207 mg, 0.66 mmol) was added DMF (0.6 mL) at room temperature under a nitrogen atmosphere. The mixture was cooled in an ice bath, and triethylamine (251 µL, 1.80 mmol) was then added. The reaction mixture was stirred at 25°C for 12 h and then purified by reverse phase silica gel column chromatography (0.1% aqueous formic acid solution/0.1% formic acid-acetonitrile solution) to afford (R)-2-((((4-azidobenzyl)oxy)carbonyl)amino)-3-(tert-butyldisulfanyl)propanoic acid (Compound pc01, Acbz-Cys(StBu)-OH) (220.1 mg, 95%).
LCMS (ESI) m/z = 383 $(M-H)^-$
Retention time: 0.84 min (analytical condition SQDFA05)

Synthesis of (R)-cyanomethyl 2-((((4-azidobenzyl)oxy)carbonyl)amino)-3-(tert-butyldisulfanyl)propanoate (Compound pc02, Acbz-Cys(StBu)-$OCH_2CN$)

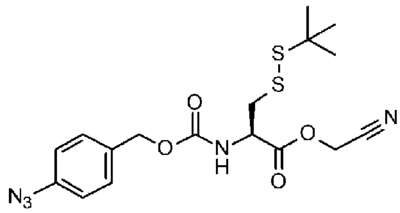

(R)-2-((((4-Azidobenzyl)oxy)carbonyl)amino)-3-(tert-butyldisulfanyl)propanoic acid (Compound pc01, Acbz-Cys(StBu)-OH) (1.15 g, 3.00 mmol) and N-ethylisopropylpropan-2-amine (DIPEA) (0.576 mL, 3.30 mmol) were dissolved in acetonitrile (6.0 ml) under a nitrogen atmosphere, 2-bromoacetonitrile (0.627 mL, 9.00 mmol) was added, and the mixture was stirred at room temperature for 5 h. The reaction solution was concentrated and the residue was purified by column chromatography (ethyl acetate:hexane = 1:9 -> 1:1) to afford (R)-cyanomethyl 2-((((4-azidobenzyl)oxy)carbonyl)amino)-3-(tert-butyldisulfanyl)propanoate (Compound pc02, Acbz-Cys(StBu)-$OCH_2CN$) (1.21 g, 95%).
LCMS (ESI) m/z = 422 $(M-H)^-$
Retention time: 0.90 min (analytical condition SQDFA05)

Synthesis of (2R)-(2R,3S,4R,5R)-2-((((((2R,3R,4R,5R)-5-(4-amino-2-oxopyrimidin-1(2H)-yl)-2-((phosphonooxy)methyl)-4-((tetrahydrofuran-2-yl)oxy)tetrahydrofuran-3-yl)oxy)(hydroxy)phosphoryl)oxy)methyl)-5-(6-amino-9H-purin-9-yl)-4-hydroxytetrahydrofuran-3-yl 2-((((4-azidobenzyl)oxy)carbonyl)amino)-3-(tert-butyldisulfanyl)propanoate (Compound pc04)

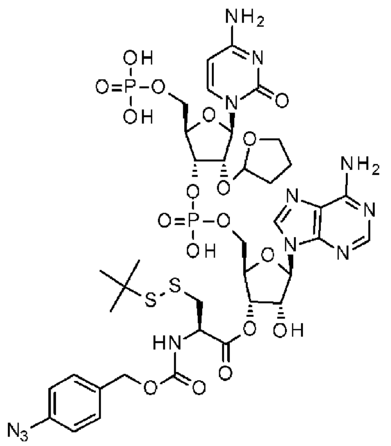

((2R,3R,4R,5R)-5-(4-Amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) synthesized by the method described in the document (Helv. Chim. Acta, 90, 297-310) (120.4 mg, 0.167 mmol) was dissolved in buffer A (60 ml), a solution of (R)-cyanomethyl 2-((((4-azidobenzyl)oxy)carbonyl)amino)-3-(tert-butyldisulfanyl)propanoate (Compound pc02, Acbz-Cys(StBu)-$OCH_2CN$) (212 mg, 0.500 mmol) in acetonitrile (2.5 ml) was added in three divided portions (at the start of the reaction, 5 min after the start of the reaction, and 30 min after the start of the reaction; 0.83 mL each; three times in total), and the mixture was stirred at room temperature for 70 min. The reaction solution was lyophilized, and the resulting residue was purified by reverse phase silica gel column chromatography (0.1% aqueous formic acid solution/0.1% formic acid-acetonitrile) to afford a mixture of the title compound (Compound pc04) and N,N,N-trimethylhexadecan-1-aminium chloride (566 mg).
LCMS (ESI) m/z = 1087 $(M-H)^-$
Retention time: 0.62 min (analytical condition SQDFA05)

Buffer A was prepared as follows. Specifically, acetic acid was added to a solution of N,N,N-trimethylhexadecan-1-aminium chloride (6.40 g, 20 mmol) and imidazole (6.81 g, 100 mmol) in water to give buffer A, pH = 8 of 20 mM N,N,N-trimethylhexadecan-1-aminium and 100 mM imidazole (1 L).

Synthesis of (2R)-(2R,3S,4R,5R)-2-((((((2R,3S,4R,5R)-5-(4-amino-2-oxopyrimidin-1(2H)-yl-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl 2-((((4-azidobenzyl)oxy)carbonyl)amino)-3-(tert-butyldisulfanyl)propanoate (Compound pc05, Acbz-Cys(StBu)-pCpA)

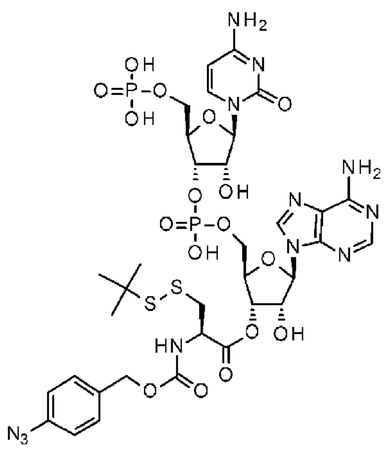

To the mixture of (2R)-(2R,3S,4R,5R)-2-((((((2R,3R,4R,5R)-5-(4-amino-2-oxopyrimidin-1(2H)-yl)-2-((phosphonooxy)methyl)-4-((tetrahydrofuran-2-yl)oxy)tetrahydrofuran-3-yl)oxy)(hydroxy)phosphoryl)oxy)methyl)-5-(6-amino-9H-purin-9-yl)-4-hydroxytetrahydrofuran-3-yl 2-((((4-azidobenzyl)oxy)carbonyl)amino)-3-(tert-butyldisulfanyl)propanoate

(Compound pc04) and N,N,N-trimethylhexadecan-1-aminium chloride obtained in the previous step (270 mg) was added a 80% aqueous acetic acid solution (5 mL), and the mixture was stirred at room temperature for 2 h. The reaction solution was lyophilized, and the resulting residue was purified by reverse phase silica gel column chromatography (0.05% aqueous trifluoroacetic acid solution/0.05% trifluoroacetic acid-acetonitrile) to afford the title compound (Compound pc05, Acbz-Cys(StBu)-pCpA) (17.8 mg, 21%, two steps).
LCMS (ESI) m/z = 1019 $(M+H)^+$
Retention time: 0.54 min (analytical condition SQDFA05)

Synthesis of (S)-2-((((4-azidobenzyl)oxy)carbonyl)amino)-3-(tert-butyldisulfanyl)propanoic acid (Compound pc06, Acbz-D-Cys(StBu)-OH)

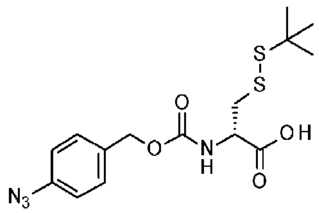

To a mixture of S-tert-butylmercapto-D-cysteine (H-D-Cys(StBu)-OH) (400 mg, 1.91 mmol) and 4-azidobenzyl (4-nitrophenyl) carbonate (661 mg, 2.10 mmol) was added DMF (1.91 mL) at room temperature under a nitrogen atmosphere. The mixture was cooled in an ice bath, and triethylamine (799 μL, 5.73 mmol) was then added. The reaction mixture was stirred at 25°C for 2 h and then purified by reverse phase silica gel column chromatography (0.1% aqueous formic acid solution/0.1% formic acid-acetonitrile solution) to afford (S)-2-((((4-azidobenzyl)oxy)carbonyl)amino)-3-(tert-butyldisulfanyl)propanoic acid (Compound pc06, Acbz-D-Cys(StBu)-OH) (658.2 mg, 90%).
LCMS (ESI) m/z = 383 $(M-H)^-$
Retention time: 0.81 min (analytical condition SQDFA05)

Synthesis of (S)-cyanomethyl 2-((((4-azidobenzyl)oxy)carbonyl)amino)-3-(tert-butyldisulfanyl)propanoate (Compound pc07, Acbz-D-Cys(StBu)-$OCH_2CN$)

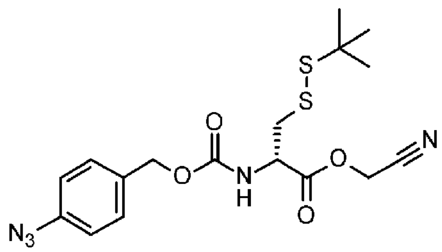

(S)-2-((((4-Azidobenzyl)oxy)carbonyl)amino)-3-(tert-butyldisulfanyl)propanoic acid (Compound pc06, Acbz-D-Cys(StBu)-OH) (0.658 g, 1.71 mmol) and N-ethyl-isopropylpropan-2-amine (DIPEA) (0.329 mL, 1.88 mmol) were dissolved in acetonitrile (3.42 ml) under a nitrogen atmosphere, 2-bromoacetonitrile (0.358 mL, 5.13 mmol) was added, and the mixture was stirred at room temperature for 5 h. The reaction solution was concentrated and the residue was purified by column chromatography (ethyl acetate:hexane = 1:9 -> 1:1) to afford (S)-cyanomethyl 2-((((4-azidobenzyl)oxy)carbonyl)amino)-3-(tert-butyldisulfanyl)propanoate (Compound pc07, Acbz-D-Cys(StBu)-$OCH_2CN$) (0.715 g, 99%).
LCMS (ESI) m/z = 422 $(M-H)^-$
Retention time: 0.90 min (analytical condition SQDFA05)

Synthesis of (2S)-(2R,3S,4R,5R)-2-((((((2R,3R,4R,5R)-5-(4-amino-2-oxopyrimidin-1(2H)-yl)-2-((phosphonooxy)methyl)-4-((tetrahydrofuran-2-yl)oxy)tetrahydrofuran-3-yl)oxy)(hydroxy)phosphoryl)oxy)methyl)-5-(6-amino-9H-purin-9-yl)-4-hydroxytetrahydrofuran-3-yl 2-((((4-azidobenzyl)oxy)carbonyl)amino)-3-(tert-butyldisulfanyl)propanoate (Compound pc08)

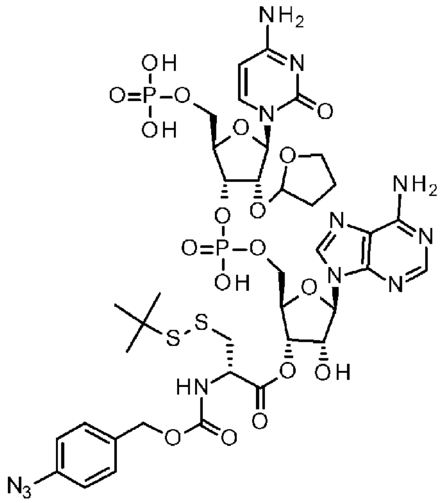

((2R,3R,4R,5R)-5-(4-Amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (60.2 mg, 0.083 mmol) was dissolved in buffer A (40 ml), a solution of (S)-cyanomethyl 2-((((4-azidobenzyl)oxy)carbonyl)amino)-3-(tert-butyldisulfanyl)propanoate (Compound pc07, Acbz-D-Cys(StBu)-$OCH_2CN$) (106 mg, 0.250 mmol) in acetonitrile (1.26 ml) was added in three divided portions (at the start of the reaction, 5 min after the start of the reaction, and 30 min after the start of the reaction; 0.42 mL each; three times in total), and the mixture was stirred at room temperature for 70 min. The reaction solution was lyophilized, and the resulting residue was purified by reverse phase silica gel column chromatography (0.1% aqueous formic acid solution/0.1% formic acid-acetonitrile) to afford a mixture of the title compound (Compound pc08) and N,N,N-trimethylhexadecan-1-aminium chloride (242.5 mg).
LCMS (ESI) m/z = 1087 $(M-H)^-$
Retention time: 0.59 min (analytical condition SQDFA05)

Synthesis of (2S)-(2R,3S,4R,5R)-2-((((((2R,3S,4R,5R)-5-(4-amino-2-oxopyrimidin-1(2H)-yl-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl 2-((((4-azidobenzyl)oxy)carbonyl)amino)-3-(tert-butyldisulfanyl)propanoate (Compound pc09, Acbz-D-Cys(StBu)-pCpA)

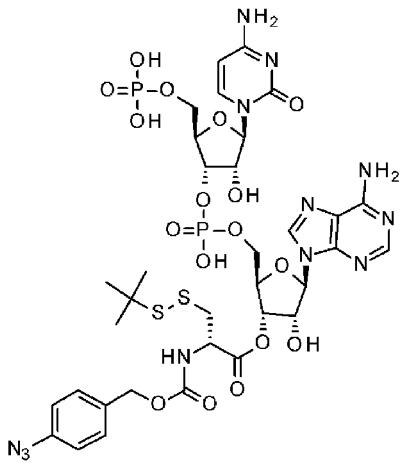

To the mixture of (2S)-(2R,3S,4R,5R)-2-((((((2R,3R,4R,5R)-5-(4-amino-2-oxopyrimidin-1(2H)-yl)-2-((phosphonooxy)methyl)-4-((tetrahydrofuran-2-yl)oxy)tetrahydrofuran-3-yl)oxy)(hydroxy)phosphoryl)oxy)methyl)-5-(6-amino-9H-purin-9-yl)-4-hydroxytetrahydrofuran-3-yl 2-((((4-azidobenzyl)oxy)carbonyl)amino)-3-(tert-butyldisulfanyl)propanoate (Compound pc08) and N,N,N-trimethylhexadecan-1-aminium chloride obtained in the previous step (242.5 mg) was added a 80% aqueous acetic acid solution (5 mL), and the mixture was stirred at room temperature for 2 h. The reaction solution was lyophilized, and the resulting residue was purified by reverse phase silica gel column chromatography (0.05% aqueous trifluoroacetic acid solution/0.05% trifluoroacetic acid-acetonitrile) to afford the title compound (Compound pc09, Acbz-D-Cys(StBu)-pCpA) (24.7 mg, 29%, two steps).
LCMS (ESI) m/z = 1019 $(M+H)^+$
Retention time: 0.55 min (analytical condition SQDFA05)

Synthesis of (R)-3-(tert-butyldisulfanyl)-2-(methylamino)propanoic acid (Compound pc10, H-MeCys(StBu)-OH)

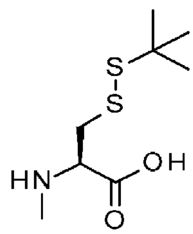

To a solution of (R)-2-((((9H-fluoren-9-yl)methoxy)carbonyl)(methyl)amino)-3-(tert-butyldisulfanyl)propanoic acid (Fmoc-MeCys(StBu)-OH) (3.00 g, 6.73 mmol) in N,N-dimethylformamide (DMF) (13.4 ml) was added 1,8-diazabicyclo[5.4.0]-7-undecene (DBU) (1.12 ml, 7.41 mmol), and the mixture was stirred at room temperature for 30 min. The reaction solution was purified by reverse phase silica gel column chromatography (0.1% aqueous formic acid solution/0.1% formic acid-acetonitrile) to afford (R)-3-(tert-butyldisulfanyl)-2-(methylamino)propanoic acid (Compound pc10, H-MeCys(StBu)-OH) (1.46 g, 97%).
LCMS (ESI) m/z = 224 $(M+H)^+$
Retention time: 0.34 min (analytical condition SQDFA05)

Synthesis of (R)-2-((((4-azidobenzyl)oxy)carbonyl)(methyl)amino)-3-(tert-butyldisulfanyl)propanoic acid (Compound pc11, Acbz-MeCys(StBu)-OH)

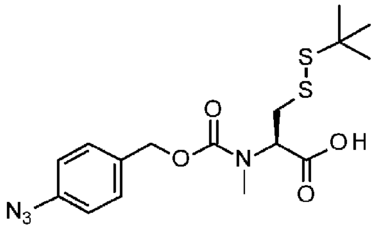

To a mixture of (R)-3-(tert-butyldisulfanyl)-2-(methylamino)propanoic acid (Compound pc10, H-MeCys(St-Bu)-OH) (700 mg, 3.13 mmol) and 4-azidobenzyl (4-nitrophenyl) carbonate (1.034 g, 3.29 mmol) was added N,N-dimethylformamide (DMF) (3.13 mL) at room temperature under a nitrogen atmosphere. The mixture was cooled in an ice bath, and triethylamine (1.31 mL, 9.40 mmol) was then added. The reaction mixture was stirred at 25°C for four days and then purified by reverse phase silica gel column chromatography (0.1% aqueous formic acid solution/0.1% formic acid-acetonitrile solution) to afford (R)-2-((((4-azidobenzyl)oxy)carbonyl)(methyl)amino)-3-(tert-butyldisulfanyl)propanoic acid (Compound pc11, Acbz-MeCys(StBu)-OH) (1.15 g, 92%).
LCMS (ESI) m/z = 397 $(M-H)^-$
Retention time: 0.87 min (analytical condition SQDFA05)

Synthesis of (R)-cyanomethyl 2-((((4-azidobenzyl)oxy)carbonyl)(methyl)amino)-3-(tert-butyldisulfanyl)propanoate (Compound pc12, Acbz-MeCys(StBu)-$OCH_2CN$)

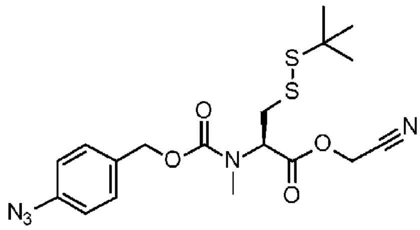

(R)-2-((((4-Azidobenzyl)oxy)carbonyl)(methyl)amino)-3-(tert-butyldisulfanyl)propanoic acid (Compound pc11, Acbz-MeCys(StBu)-OH) (1.14 g, 2.86 mmol) and N-ethyl-isopropylpropan-2-amine (DIPEA) (0.49 mL, 2.80 mmol) were dissolved in acetonitrile (5.72 ml) under a nitrogen atmosphere, 2-bromoacetonitrile (0.598 mL, 8.58 mmol) was added, and the mixture was stirred at room temperature for 7 h. The reaction solution was concentrated and the residue was purified by column chromatography (ethyl acetate:hexane = 1:9 -> 1:1) to afford (R)-cyanomethyl 2-((((4-azidobenzyl)oxy)carbonyl)(methyl)amino)-3-(tert-butyldisulfanyl)propanoate (Compound pc12, Acbz-MeCys(StBu)-$OCH_2CN$) (1.17 g, 93%).
LCMS (ESI) m/z = 436 $(M-H)^-$
Retention time: 0.96 min (analytical condition SQDFA05)

Synthesis of (2R)-(2R,3S,4R,5R)-2-((((((2R,3R,4R,5R)-5-(4-amino-2-oxopyrimidin-1(2H)-yl)-2-((phosphonooxy)methyl)-4-((tetrahydrofuran-2-yl)oxy)tetrahydrofuran-3-yl)oxy)(hydroxy)phosphoryl)oxy)methyl)-5-(6-amino-9H-purin-9-yl)-4-hydroxytetrahydrofuran-3-yl 2-((((4-azidobenzyl)oxy)carbonyl)(methyl)amino)-3-(tert-butyldisulfanyl)propanoate (Compound pc13)

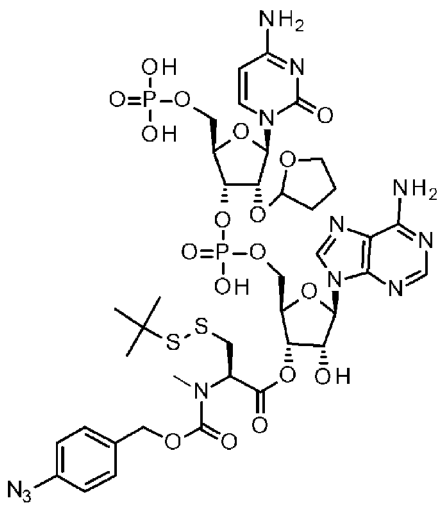

((2R,3R,4R,5R)-5-(4-Amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (60.2 mg, 0.083 mmol) was dissolved in buffer A (40 ml), a solution of (R)-cyanomethyl 2-((((4-azidobenzyl)oxy)carbonyl)(methyl)amino)-3-(tert-butyldisulfanyl)propanoate (Compound pc12, Acbz-MeCys(StBu)-$OCH_2CN$) (109 mg, 0.250 mmol) in acetonitrile (1.26 ml) was added in three divided portions (at the start of the reaction, 5 min after the start of the reaction, and 30 min after the start of the reaction; 0.42 mL each; three times in total), and the mixture was stirred at room temperature for 120 min. The reaction solution was lyophilized, and the resulting residue was purified by reverse phase silica gel column chromatography (0.1% aqueous formic acid solution/0.1% formic acid-acetonitrile) to afford a mixture of the title compound (Compound pc13) and N,N,N-trimethylhexadecan-1-aminium chloride (90 mg).
LCMS (ESI) m/z = 1101 $(M-H)^-$
Retention time: 0.69 min (analytical condition SQDFA05)

Synthesis of (2R)-(2R,3S,4R,5R)-2-((((((2R,3S,4R,5R)-5-(4-amino-2-oxopyrimidin-1(2H)-yl-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl 2-((((4-azidobenzyl)oxy)carbonyl)methyl)amino)-3-(tert-butyldisulfanyl)propanoate (Compound pc14, Acbz-MeCys(StBu)-pCpA)

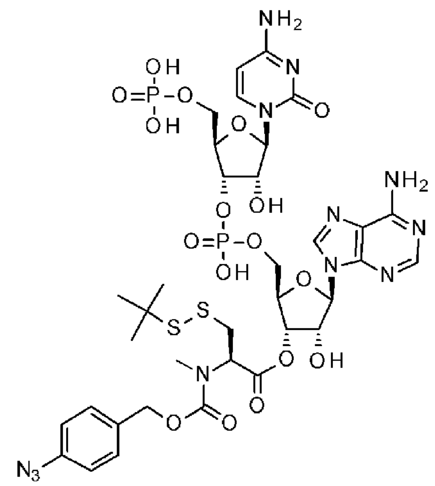

To the mixture of (2R)-(2R,3S,4R,5R)-2-((((((2R,3R,4R,5R)-5-(4-amino-2-oxopyrimidin-1(2H)-yl)-2-((phosphonooxy)methyl)-4-((tetrahydrofuran-2-yl)oxy)tetrahydrofuran-3-yl)oxy)(hydroxy)phosphoryl)oxy)methyl)-5-(6-amino-9H-purin-9-yl)-4-hydroxytetrahydrofuran-3-yl 2-((((4-azidobenzyl)oxy)carbonyl)(methyl)amino)-3-(tert-butyldisulfanyl)propanoate (Compound pc13) and N,N,N-trimethylhexadecan-1-aminium chloride obtained in the previous step (90 mg) was added a 80% aqueous acetic acid solution (5 mL), and the mixture was stirred at room temperature for 150 min. The reaction solution was lyophilized, and the resulting residue was purified by reverse phase silica gel column chromatography (0.05% aqueous trifluoroacetic acid solution/0.05% trifluoroacetic acid-acetonitrile) to afford the title compound (Compound pc14, Acbz-MeCys(StBu)-pCpA) (26.7 mg, 31%, two steps).
LCMS (ESI) m/z = 1033 $(M+H)^+$
Retention time: 0.65 min (analytical condition SQDFA05)

Synthesis of (S)-3-(tert-butyldisulfanyl)-2-(methylamino)propanoic acid (Compound pc15, H-D-MeCys(StBu)-OH)

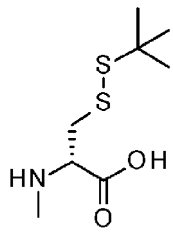

To a solution of (S)-2-((((9H-fluoren-9-yl)methoxy)carbonyl)(methyl)amino)-3-(tert-butyldisulfanyl)propanoic acid (Fmoc-D-MeCys(StBu)-OH) (0.5 g, 1.12 mmol) in N,N-dimethylformamide (DMF) (2.24 ml) was added 1,8-diazabicyclo[5.4.0]-7-undecene (DBU) (0.186 ml, 1.234 mmol), and the mixture was stirred at room temperature for 90 min. The reaction solution was purified by reverse phase silica gel column chromatography (0.1% aqueous formic acid solution/0.1% formic acid-acetonitrile) to afford (S)-3-(tert-butyldisulfanyl)-2-(methylamino)propanoic acid (Compound pc15, H-D-MeCys(StBu)-OH) (0.22 g, 88%).
LCMS (ESI) m/z = 224 $(M+H)^+$
Retention time: 0.38 min (analytical condition SQDFA05)

Synthesis of (S)-2-((((4-azidobenzyl)oxy)carbonyl)(methyl)amino)-3-(tert-butyldisulfanyl)propanoic acid (Compound pc16, Acbz-D-MeCys(StBu)-OH)

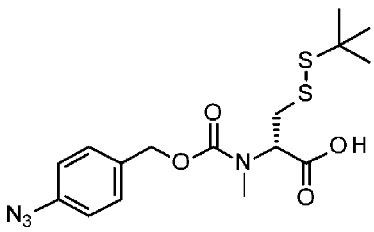

To a mixture of (S)-3-(tert-butyldisulfanyl)-2-(methylamino)propanoic acid (Compound pc15, H-D-MeCys(StBu)-OH) (200 mg, 0.90 mmol) and 4-azidobenzyl (4-nitrophenyl) carbonate (295 mg, 0.94 mmol) was added N,N-dimethylformamide (DMF) (0.90 mL) at room temperature under a nitrogen atmosphere. The mixture was cooled in an ice bath, and triethylamine (374 μL, 2.69 mmol) was then added. The reaction mixture was stirred at 40°C for 2 h and then purified by reverse phase silica gel column chromatography (0.1% aqueous formic acid solution/0.1% formic acid-acetonitrile solution) to afford (S)-2-((((4-azidobenzyl)oxy)carbonyl)(methyl)amino)-3-(tert-butyldisulfanyl)propanoic acid (Compound pc16, Acbz-D-MeCys(StBu)-OH) (350 mg, 98%).
LCMS (ESI) m/z = 397 $(M-H)^-$
Retention time: 0.90 min (analytical condition SQDFA05)

Synthesis of (S)-cyanomethyl 2-((((4-azidobenzyl)oxy)carbonyl)(methyl)amino)-3-(tert-butyldisulfanyl)propanoate (Compound pc17, Acbz-D-MeCys(StBu)-$OCH_2CN$)

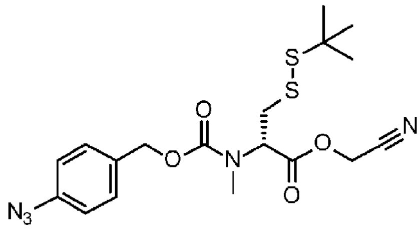

(S)-2-((((4-Azidobenzyl)oxy)carbonyl)(methyl)amino)-3-(tert-butyldisulfanyl)propanoic acid (Compound pc16, Acbz-D-MeCys(StBu)-OH) (350 mg, 0.88 mmol) and N-ethyl-isopropylpropan-2-amine (DIPEA) (0.15 mL, 0.86 mmol) were dissolved in acetonitrile (1.75 ml) under a nitrogen atmosphere, 2-bromoacetonitrile (0.18 mL, 2.63 mmol) was added, and the mixture was stirred at room temperature for 2 h. The reaction solution was concentrated to afford a crude product (R)-cyanomethyl 2-((((4-azidobenzyl)oxy)carbonyl)(methyl)amino)-3-(tert-butyldisulfanyl)propanoate (Compound pc17, Acbz-D-MeCys(StBu)-$OCH_2CN$) (1.17 g). The resulting crude product (R)-cyanomethyl 2-((((4-azidobenzyl)oxy)carbonyl)(methyl)amino)-3-(tert-butyldisulfanyl)propanoate (Compound pc17, Acbz-D-MeCys(StBu)-$OCH_2CN$) was dissolved in acetonitrile (4.4 ml) and used as such in the next step.
LCMS (ESI) m/z = 436 $(M-H)^-$
Retention time: 0.96 min (analytical condition SQDFA05)

Synthesis of (2S)-(2R,3S,4R,5R)-2-((((((2R,3R,4R,5R)-5-(4-amino-2-oxopyrimidin-1(2H)-yl)-2-((phosphonooxy)methyl)-4-((tetrahydrofuran-2-yl)oxy)tetrahydrofuran-3-yl)oxy)(hydroxy)phosphoryl)oxy)methyl)-5-(6-amino-9H-purin-9-yl)-4-hydroxytetrahydrofuran-3-yl 2-((((4-azidobenzyl)oxy)carbonyl)(methyl)amino)-3-(tert-butyldisulfanyl)propanoate (Compound pc18)

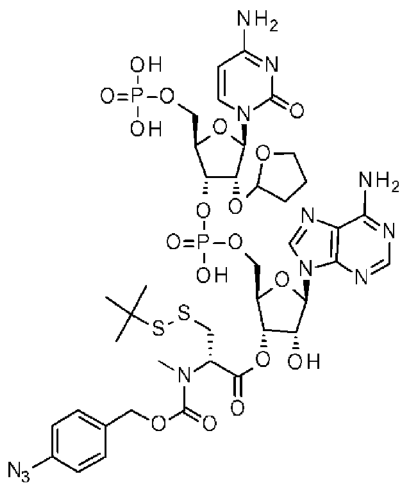

((2R,3R,4R,5R)-5-(4-Amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (85.0 mg, 0.118 mmol) was dissolved in buffer A (56.4 mL), a 0.2 M solution of (R)-cyanomethyl 2-((((4-azidobenzyl)oxy)carbonyl)(methyl)amino)-3-(tert-butyldisulfanyl)propanoate (Compound pc17, Acbz-D-MeCys(StBu)-$OCH_2CN$) in acetonitrile (1.77 ml, 0.353 mmol) was added in three divided portions (at the start of the reaction, 10 min after the start of the reaction, and 30 min after the start of the reaction; 0.59 mL each; three times in total), and the mixture was stirred at room temperature for 120 min. The reaction solution was lyophilized, and the resulting residue was purified by reverse phase silica gel column chromatography (0.1% aqueous formic acid solution/0.1% formic acid-acetonitrile) to afford a mixture of the title compound (Compound pc18) and N,N,N-trimethylhexadecan-1-aminium chloride (105.2 mg).
LCMS (ESI) m/z = 1101 $(M-H)^-$
Retention time: 0.69 min (analytical condition SQDFA05)

Synthesis of (2S)-(2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl 2-((((4-azidobenzyl)oxy)carbonyl)(methyl)amino)-3-(tert-butyldisulfanyl)propanoate (Compound pc19, Acbz-D-MeCys(StBu)-pCpA)

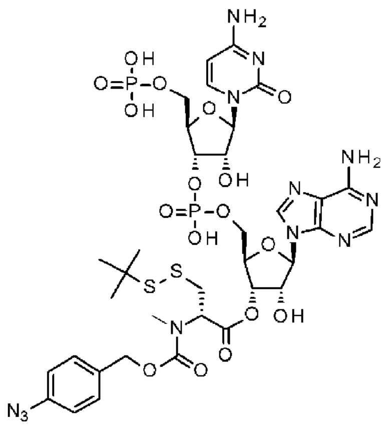

To the mixture of (2S)-(2R,3S,4R,5R)-2-((((((2R,3R,4R,5R)-5-(4-amino-2-oxopyrimidin-1(2H)-yl)-2-((phosphonooxy)methyl)-4-((tetrahydrofuran-2-yl)oxy)tetrahydrofuran-3-yl)oxy)(hydroxy)phosphoryl)oxy)methyl)-5-(6-amino-9H-purin-9-yl)-4-hydroxytetrahydrofuran-3-yl 2-((((4-azidobenzyl)oxy)carbonyl)(methyl)amino)-3-(tert-butyldisulfanyl)propanoate (Compound pc18) and N,N,N-trimethylhexadecan-1-aminium chloride obtained in the previous step (100 mg) was added a 80% aqueous acetic acid solution (5 mL), and the mixture was stirred at room temperature for 80 min. The reaction solution was lyophilized, and the resulting residue was purified by reverse phase silica gel column chromatography (0.05% aqueous trifluoroacetic acid solution/0.05% trifluoroacetic acid-acetonitrile) to afford the title compound (Compound pc19, Acbz-D-MeCys(StBu)-pCpA) (39.8 mg, 43%, two steps).
LCMS (ESI) m/z = 1033 $(M+H)^+$
Retention time: 0.62 min (analytical condition SQDFA05)

Synthesis of (2S)-2-(N-methylpent-4-enamido)-3-(1,3-thiazol-4-yl)propanoic acid (Compound pc20, Pen-MeAla(4-Thz)-OH)

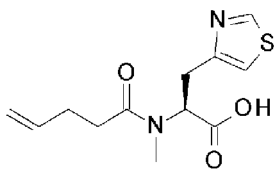

To (S)-2-((((9H-fluoren-9-yl)methoxy)carbonyl)(methyl)amino)-3-(thiazol-4-yl)propanoic acid (Fmoc-MeAla(4-Thz)-OH) synthesized by the method described in WO 2013/100132 (4.20 g, 10.28 mmol) was added a 20% solution of piperidine in DMF (100 mL), and the mixture was stirred at room temperature for 2 h. The reaction solution was concentrated under reduced pressure, diethyl ether was added to the concentrated residue, the mixture was filtered, and the solid on the filter paper was washed with diethyl ether to afford a crude product (2S)-2-(methylamino)-3-(1,3-thiazol-4-yl)propanoic acid.

The obtained (2S)-2-(methylamino)-3-(1,3-thiazol-4-yl)propanoic acid was dissolved in 1,4-dioxane (40 ml)/water (40 ml), after which 2,5-dioxopyrrolidin-1-yl pent-4-enoate synthesized by the method described in the document (Organic Letters, 2011, 13, 4906) (3.200 g, 16.23 mmol) and sodium bicarbonate (1.80 g, 21.43 mmol) were added and the reaction solution was stirred at 30°C for 12 h. After the reaction was completed, water was added to the reaction solution, the mixture was washed with ethyl acetate, and a 1.0 M aqueous sodium bisulfate solution was added until the acidity of the aqueous layer was pH = 2. The resulting mixture was extracted with ethyl acetate, and the organic layer was washed with brine. The organic layer was dried over anhydrous sodium sulfate, filtered, and then concentrated

under reduced pressure, and the resulting residue was purified by normal phase silica gel column chromatography (dichloromethane/methanol) to afford (2S)-2-(N-methylpent-4-enamido)-3-(1,3-thiazol-4-yl)propanoic acid (Compound pc20, Pen-MeAla(4-Thz)-OH) (0.60 g, 22%).
LCMS (ESI) m/z = 269 $(M+H)^+$
Retention time: 1.32 min (analytical condition SMD method 1)

Synthesis of (S)-cyanomethyl 2-(N-methylpent-4-enamido)-3-(thiazol-4-yl)propanoate (Compound pc21, Pen-MeAla(4-Thz)-$OCH_2CN$)

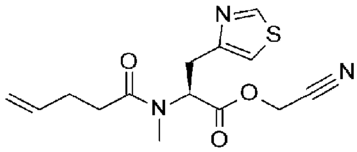

(2S)-2-(N-Methylpent-4-enamido)-3-(1,3-thiazol-4-yl)propanoic acid (Compound pc20, Pen-MeAla(4-Thz)-OH) (1.50 g, 2.80 mmol) and N-ethyl-isopropylpropan-2-amine (DIPEA) (680 mg, 5.26 mmol) were dissolved in dichloromethane (80 ml) under a nitrogen atmosphere, 2-bromoacetonitrile (1.25 g, 10.42 mmol) was added, and the mixture was stirred at room temperature overnight. The reaction solution was concentrated and the resulting residue was purified by normal phase silica gel column chromatography (petroleum ether/ethyl acetate) to afford (S)-cyanomethyl 2-(N-methylpent-4-enamido)-3-(thiazol-4-yl)propanoate (Compound pc21, Pen-MeAla(4-Thz)-$OCH_2CN$) (0.23 g, 27%).
LCMS (ESI) m/z = 308 $(M+H)^+$
Retention time: 1.61 min (analytical condition SMD method 1)

Synthesis of (2S)-(2R,3S,4R,5R)-2-((((((2R,3R,4R,5R)-5-(4-amino-2-oxopyrimidin-1(2H)-yl)-2-((phosphonooxy)methyl)-4-((tetrahydrofuran-2-yl)oxy)tetrahydrofuran-3-yl)oxy)(hydroxy)phosphoryl)oxy)methyl)-5-(6-amino-9H-purin-9-yl)-4-hydroxytetrahydrofuran-3-yl 2-(N-methylpent-4-enamido)-3-(thiazol-4-yl)propanoate (Compound pc22)

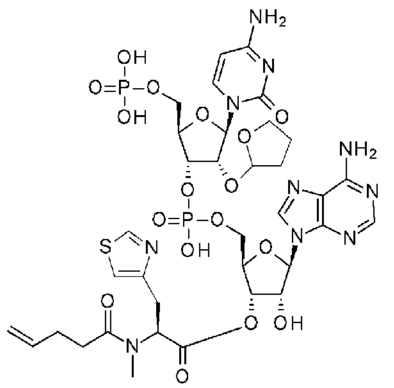

((2R,3R,4R,5R)-5-(4-Amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (300 mg, 0.415 mmol) was dissolved in buffer A (120 ml), a solution of (S)-cyanomethyl 2-(N-methylpent-4-enamido)-3-(thiazol-4-yl)propanoate (Compound pc21, Pen-MeAla(4-Thz)-$OCH_2CN$) (311 mg, 1.013 mmol) in acetonitrile (3.6 ml) was added and the mixture was stirred at room temperature for 4 h. The reaction solution was lyophilized and the resulting residue was purified by reverse phase silica gel column chromatography (10 mM aqueous ammonium acetate/methanol) to afford a mixture of the title compound (Compound pc22) and N,N,N-trimethylhexadecan-1-aminium chloride (300 mg).
LCMS (ESI) m/z = 971 $(M-H)^-$
Retention time: 0.59 min (analytical condition SQDAA05)

Synthesis of (2S)-(2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl 2-(N-methylpent-4-enamido)-3-(thiazol-4-yl)propanoate (Compound pc23, Pen-MeAla(4-Thz)-pCpA)

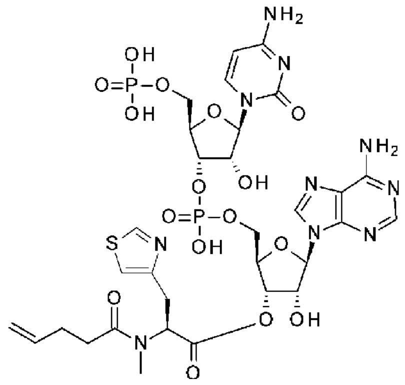

To the mixture of (2S)-(2R,3S,4R,5R)-2-((((((2R,3R,4R,5R)-5-(4-amino-2-oxopyrimidin-1(2H)-yl)-2-((phosphonooxy)methyl)-4-((tetrahydrofuran-2-yl)oxy)tetrahydrofuran-3-yl)oxy)(hydroxy)phosphoryl)oxy)methyl)-5-(6-amino-9H-purin-9-yl)-4-hydroxytetrahydrofuran-3-yl 2-(N-methylpent-4-enamido)-3-(thiazol-4-yl)propanoate (Compound pc22) and N,N,N-trimethylhexadecan-1-aminium chloride obtained in the previous step (300 mg) was added a 80% aqueous acetic acid solution (6 mL), and the mixture was stirred at room temperature for 7 h. The reaction solution was lyophilized, and the resulting residue was purified by reverse phase silica gel column chromatography (0.05% aqueous trifluoroacetic acid solution/0.05% trifluoroacetic acid-acetonitrile) to afford the title compound (Compound pc23, Pen-MeAla(4-Thz)-pCpA) (46 mg, 17%, two steps).
LCMS (ESI) m/z = 903 $(M+H)^+$
Retention time: 0.37 min (analytical condition SQDFA05)

Synthesis of cyanomethyl 2-(N-methylpent-4-enamido)acetate (Compound pc24, Pen-MeGly-$OCH_2CN$)

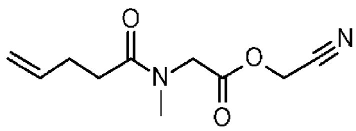

N-Methylglycine (1.5 g, 16.8 mmol) and N-ethyl-isopropylpropan-2-amine (DIPEA) (7.35 ml, 42.1 mmol) were added to dichloromethane (33.7 ml). The mixture was then cooled to 0°C, after which 2,5-dioxopyrrolidin-1-yl pent-4-enoate (1.95 ml, 17.7 mmol) was added and the reaction solution was stirred at 25°C for three days. After the reaction was completed, N-ethyl-isopropylpropan-2-amine (DIPEA) (2.94 ml, 16.8 mmol) and 2-bromoacetonitrile (2.35 ml, 33.7 mmol) were added to the reaction mixture, which was then stirred at 25°C for 4 h. The reaction solution was diluted with dichloromethane, after which a saturated aqueous ammonium chloride solution was added and the organic layer was washed with brine. The organic layer was then dried over anhydrous sodium sulfate, filtered, and then concentrated under reduced pressure, and the resulting residue was purified by normal phase silica gel column chromatography (hexane/ethyl acetate) to afford cyanomethyl 2-(N-methylpent-4-enamido)acetate (Compound pc24, Pen-MeGly-$OCH_2CN$) (1.1 g, 31%).
LCMS (ESI) m/z = 211 $(M+H)^+$
Retention time: 0.72 min (analytical condition SMD method 3)

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl N-methyl-N-(pent-4-enoyl)glycinate (Compound pc25, Pen-MeGly-pCpA)

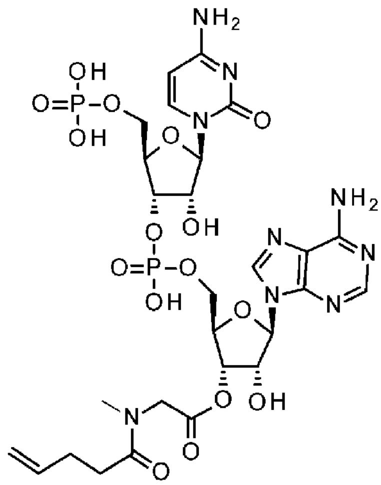

((2R,3R,4R,5R)-5-(4-Amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (250 mg, 0.35 mmol) was dissolved in buffer A (100 ml), a solution of cyanomethyl 2-(N-methylpent-4-enamido)acetate (Compound pc24, Pen-MeGly-$OCH_2CN$) (290 mg, 1.38 mmol) in acetonitrile (1.5 ml) was added, and the mixture was stirred at room temperature for 4 h. A 80% aqueous acetic acid solution (15 ml) was further added to the reaction solution, and the mixture was stirred at room temperature for 4 h.

The reaction solution was purified by reverse phase silica gel column chromatography (0.05% aqueous trifluoroacetic acid solution/0.05% trifluoroacetic acid-acetonitrile) to afford the title compound (Compound pc25, Pen-MeGly-pCpA) (37.6 mg, 13%).
LCMS (ESI) m/z = 806 $(M+H)^+$
Retention time: 0.60 min (analytical condition SMD method 2)

Synthesis of cyanomethyl (S)-2-(pent-4-enamido)-2-phenylacetate (Compound pc26, Pen-Phg-$OCH_2CN$)

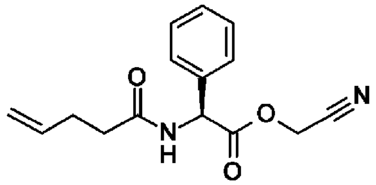

To (S)-2-((tert-butoxycarbonyl)amino)-2-phenylacetic acid (Boc-Phg-OH, 5.0 g, 19.9 mmol) was added dichloromethane (50.0 ml), after which the reaction solution was bubbled with hydrochloric acid gas and stirred at 25°C for 3 h. The precipitated solid was filtered to give (S)-2-amino-2-phenylacetic acid (H-Phg-OH) (3.7 g) as a mixture.

To the resulting mixture (S)-2-amino-2-phenylacetic acid (H-Phg-OH) (3.7 g) were added water (20 mL) and sodium bicarbonate (5.0 g, 59.5 mmol). After stirring, a solution of 2,5-dioxopyrrolidin-1-yl pent-4-enoate (4.0 g, 20.3 mmol) in 1,4-dioxane (20.0 mL) was added. The reaction solution was stirred at 25°C for 3 h and then washed with ethyl acetate three times. The aqueous layer was then adjusted to pH 2 with a 1 M aqueous hydrochloric acid solution and extracted with ethyl acetate three times. The resulting organic layers were combined, washed with brine twice, dried over anhydrous sodium sulfate, filtered, and concentrated under reduced pressure. The resulting residue was purified by normal phase silica gel column chromatography (dichloromethane/methanol) to afford (S)-2-(pent-4-enamido)-2-phenylacetic acid (Pen-Phg-OH) (3.1 g, 66%, two steps).

The above (S)-2-(pent-4-enamido)-2-phenylacetic acid (Pen-Phg-OH) (1.5 g, 6.43 mmol) and N-ethyl-isopropylpropan-2-amine (DIPEA) (2.0 g, 15.5 mmol) were dissolved in dichloromethane (20 ml) under a nitrogen atmosphere, 2-bromoacetonitrile (4.00 g, 33.4 mmol) was added, and the mixture was stirred at 25°C for 2 hours and 30 minutes. The reaction solution was concentrated and the resulting residue was purified by normal phase silica gel column chromatography (petroleum ether/ethyl acetate) to afford cyanomethyl (S)-2-(pent-4-enamido)-2-phenylacetate (Compound pc26, Pen-Phg-$OCH_2CN$) (0.692 g, 40%).
LCMS (ESI) m/z = 273 $(M+H)^+$
Retention time: 0.67 min (analytical condition SQDFA05)

Synthesis of (2S)-(2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl 2-(pent-4-enamido)-2-phenylacetate (Compound pc28, Pen-Phg-pCpA)

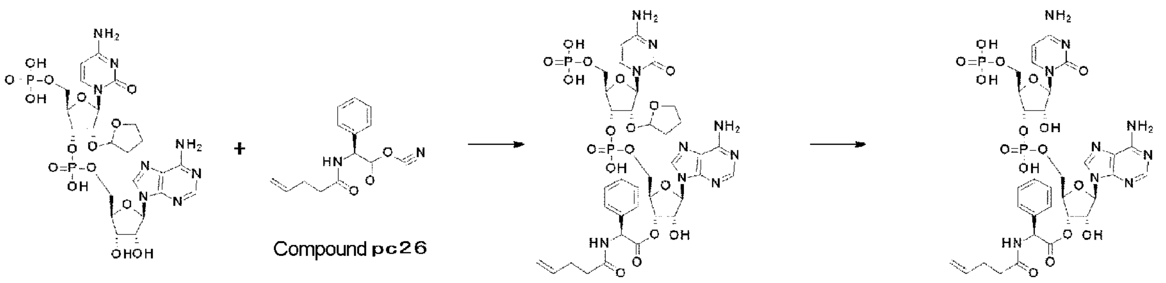

Compound pc03

Compound pc26

Compound pc27

Compound pc28

((2R,3R,4R,5R)-5-(4-Amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (300 mg, 0.415 mmol) was dissolved in buffer A (120 ml), a solution of cyanomethyl (S)-2-(pent-4-enamido)-2-phenylacetate (Compound pc26, Pen-Phg-$OCH_2CN$) (276 mg, 1.01 mmol) in acetonitrile (3.6 ml) was added, and the mixture was stirred at 25°C for 4 h. The reaction solution was lyophilized, and the resulting residue was purified by reverse phase silica gel column chromatography (10 mM aqueous ammonium acetate solution/methanol) and then purified again by reverse phase silica gel column chromatography (0.1% aqueous formic acid solution/0.1% formic acid-acetonitrile) to afford a mixture of (2S)-(2R,3S,4R,5R)-2-((((((2R,3R,4R,5R)-5-(4-amino-2-oxopyrimidin-1(2H)-yl)-2-((phosphonooxy)methyl)-4-((tetrahydrofuran-2-yl)oxy)tetrahydrofuran-3-yl)oxy)(hydroxy)phosphoryl)oxy)methyl)-5-(6-amino-9H-purin-9-yl)-4-hydroxytetrahydrofuran-3-yl 2-(pent-4-enamido)-2-phenylacetate (Compound pc27) and N,N,N-trimethylhexadecan-1-aminium chloride.

To the mixture of Compound pc27 and N,N,N-trimethylhexadecan-1-aminium chloride obtained in the previous step (70 mg) was added a 80% aqueous acetic acid solution (1.4 mL), and the mixture was stirred at 25°C for 7 h. The reaction solution was purified by reverse phase silica gel column chromatography (0.05% aqueous trifluoroacetic acid solution/0.05% trifluoroacetic acid-acetonitrile) to afford the title compound (Compound pc28, Pen-Phg-pCpA) (34 mg, 9%, two steps).
LCMS (ESI) m/z = 868 $(M+H)^+$
Retention time: 0.39 min (analytical condition SQDFA05)

Synthesis of (S)-3-(bis(4-methoxyphenyl)(phenyl)methoxy)-2-(N-methylpent-4-enamido)propanoic acid (Compound pc29, Pen-MeSer(DMT)-OH)

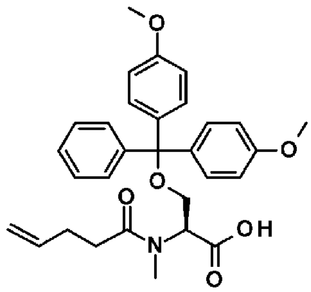

To (S)-2-((((9H-fluoren-9-yl)methoxy)carbonyl)(methyl)amino)-3-(bis(4-methoxyphenyl)(phenyl)methoxy)propanoic acid (Fmoc-MeSer(DMT)-OH) synthesized by the method described in WO 2013/100132 (3.0 g, 4.66 mmol) were added DMF (9.32 mL) and 1,8-diazabicyclo[5.4.0]-7-undecene (DBU) (0.843 mL, 5.59 mmol) at 0°C, and the reaction solution was stirred at 25°C for 1 h. To the reaction solution were then added DBU (1.76 mL, 11.7 mmol) and pent-4-enoyl chloride (1.03 mL, 9.32 mmol) at 0°C, and the reaction solution was stirred at 0°C for 30 min. The reaction solution was purified by reverse phase silica gel column chromatography (10 mM aqueous ammonium acetate solution/methanol) to afford (S)-3-(bis(4-methoxyphenyl)(phenyl)methoxy)-2-(N-methylpent-4-enamido)propanoic acid (Compound pc29, Pen-MeSer(DMT)-OH) (1.50 g, 64%, two steps).
LCMS (ESI) m/z = 502 $(M-H)^-$
Retention time: 0.91 min (analytical condition SQDFA05)

Synthesis of (S)-cyanomethyl 3-(bis(4-methoxyphenyl)(phenyl)methoxy)-2-(N-methylpent-4-enamido)propanoate (Compound pc30, Pen-MeSer(DMT)-$OCH_2CN$)

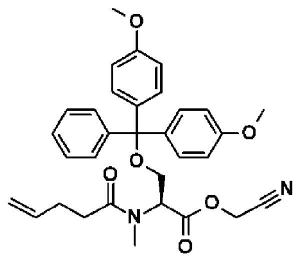

(S)-3-(Bis(4-methoxyphenyl)(phenyl)methoxy)-2-(N-methylpent-4-enamido)propanoic acid (Compound pc29, Pen-MeSer(DMT)-OH) (1.50 g, 2.98 mmol) and N-ethyl-isopropylpropan-2-amine (DIPEA) (2.60 mL, 14.9 mmol) were dissolved in DMF (6.0 ml) under a nitrogen atmosphere, 2-bromoacetonitrile (0.623 mL, 8.94 mmol) was added, and the mixture was stirred at 25°C for 30 min. To the reaction solution was added a saturated aqueous ammonium chloride solution, and the mixture was extracted with ethyl acetate. The resulting organic layer was washed with water, dried over magnesium sulfate, and filtered. The organic layer was concentrated under reduced pressure, and the resulting residue was then purified by normal phase silica gel column chromatography (hexane/ethyl acetate) to afford (S)-cyanomethyl 3-(bis(4-methoxyphenyl)(phenyl)methoxy)-2-(N-methylpent-4-enamido)propanoate (Compound pc30, Pen-MeSer(DMT)-$OCH_2CN$) (1.30 g, 80%).
LCMS (ESI) m/z = 543 $(M+H)^+$
Retention time: 0.57 min (analytical condition FA50)

Synthesis of (2S)-(2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl 3-hydroxy-2-(N-methylpent-4-enamido)propanoate (Compound pc31, Pen-MeSer-pCpA)

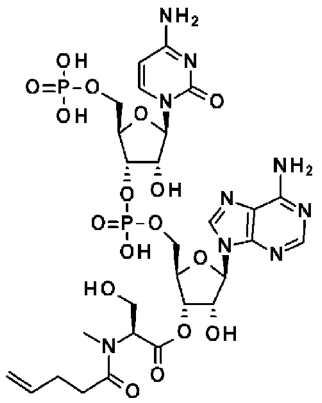

((2R,3R,4R,5R)-5-(4-Amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (300 mg, 0.415 mmol) was dissolved in buffer A (120 ml), a solution of (S)-cyanomethyl 3-(bis(4-methoxyphenyl)(phenyl)methoxy)-2-(N-methylpent-4-enamido)propanoate (Compound pc30, Pen-MeSer(DMT)-$OCH_2CN$) (549 mg, 1.013 mmol) in acetonitrile (3.6 ml) was added, and the mixture was stirred at 25°C for 3 h. The reaction solution was lyophilized, a solvent amount of a 80% aqueous acetic acid solution was added to the resulting crude product, and the mixture was stirred at 25°C for 7 h. The reaction solution was purified by reverse phase silica gel column chromatography (0.05% aqueous trifluoroacetic acid solution/0.05% trifluoroacetic acid-acetonitrile) to afford the title compound (Compound pc31, Pen--MeSer-pCpA) (7.0 mg, 2%, two steps).
LCMS (ESI) m/z = 836 $(M+H)^+$
Retention time: 0.28 min (analytical condition SQDFA05)

Synthesis of (S)-4-(methylsulfonyl)-2-(pent-4-enamido)butanoic acid (Compound pc32, Pen-Met(O2)-OH)

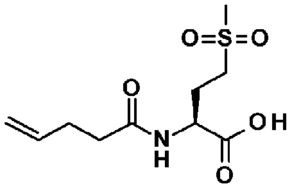

To (S)-2-((((9H-fluoren-9-yl)methoxy)carbonyl)amino)-4-(methylsulfonyl)butanoic acid (Fmoc-Met(O2)-OH) (5.00 g, 12.4 mmol) was added a 20% solution of piperidine in DMF (80.0 mL), and the reaction solution was then stirred at 25°C for 3 h. To the reaction solution was then added diethyl ether (50 mL), the mixture was filtered, and the resulting solid was washed with diethyl ether and hexane to give a crude product (S)-2-amino-4-(methylsulfonyl)butanoic acid (H--Met(O2)-OH) (2.88 g).

To a mixed solvent of water (40 mL) and 1,4-dioxane (40 mL) were added the resulting crude product (S)-2-amino-4-(methylsulfonyl)butanoic acid (H--Met(O2)-OH) (2.88 g), 2,5-dioxopyrrolidin-1-yl pent-4-enoate (3.76 g, 19.1 mmol), and sodium bicarbonate (2.68 g, 31.9 mmol), and the reaction solution was then stirred at 25°C for 4 h. The reaction solution was washed with ethyl acetate twice, after which the aqueous layer was adjusted to pH 2 with a 1 M aqueous hydrochloric acid solution and extracted with dichloromethane four times. The resulting organic layers were combined, washed with brine, dried over anhydrous sodium sulfate, filtered, and concentrated under reduced pressure. The resulting residue was purified by normal phase silica gel column chromatography (dichloromethane/methanol) to afford (S)-4-(methylsulfonyl)-2-(pent-4-enamido)butanoic acid (Pen-Met(O2)-OH) (2.4 g, 74%, two steps).
LCMS (ESI) m/z = 264 $(M+H)^+$
Retention time: 1.00 min (analytical condition SMD method 4)

Synthesis of (S)-cyanomethyl 4-(methylsulfonyl)-2-(pent-4-enamido)butanoate (Compound pc33, Pen-Met(O2)-$OCH_2CN$)

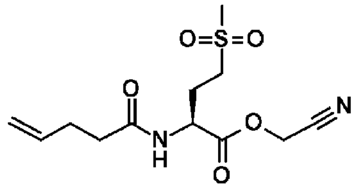

(S)-4-(Methylsulfonyl)-2-(pent-4-enamido)butanoic acid (Pen-Met(O2)-OH) (1.80 g, 6.84 mmol) and N-ethyl-isopropylpropan-2-amine (DIPEA) (3.00 g, 23.2 mmol) were dissolved in dichloromethane (30 ml) under a nitrogen atmosphere, 2-bromoacetonitrile (3.28 g, 27.4 mmol) was added, and the mixture was stirred at 25°C for 16 h. The reaction solution was concentrated and the resulting residue was purified by normal phase silica gel column chromatography (petroleum ether/ethyl acetate) to afford (S)-cyanomethyl 4-(methylsulfonyl)-2-(pent-4-enamido)butanoate (Compound pc33, Pen-Met(O2)-$OCH_2CN$) (1.5 g, 73%).
LCMS (ESI) m/z = 303 $(M+H)^+$
Retention time: 0.50 min (analytical condition SQDAA05)

Synthesis of (2S)-(2R,3S,4R,5R)-2-((((((2R,3R,4R,5R)-5-(4-amino-2-oxopyrimidin-1(2H)-yl)-2-((phosphonooxy)methyl)-4-((tetrahydrofuran-2-yl)oxy)tetrahydrofuran-3-yl)oxy)(hydroxy)phosphoryl)oxy)methyl)-5-(6-amino-9H-purin-9-yl)-4-hydroxytetrahydrofuran-3-yl 4-(methylsulfonyl)-2-(pent-4-enamido)butanoate (Compound pc34)

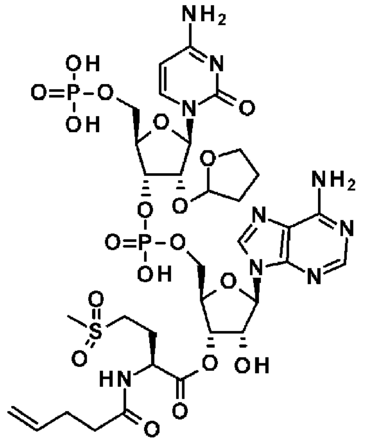

To buffer A (120 ml) were added a solution of ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (300 mg, 0.415 mmol) in water (3.6 mL) and a solution of (S)-cyanomethyl 4-(methylsulfonyl)-2-(pent-4-enamido)butanoate (Compound pc33, Pen-Met(O2)-$OCH_2CN$) (502 mg, 1.66 mmol) in acetonitrile (3.6 ml), and the mixture was stirred at room temperature for 2 h. The reaction solution was lyophilized and the resulting residue was reverse phase silica gel column chromatography (10 mM aqueous ammonium acetate/methanol) to afford a mixture of the title compound (Compound pc34) and N,N,N-trimethylhexadecan-1-aminium chloride (136 mg).
LCMS (ESI) m/z = 968.5 $(M+H)^+$
Retention time: 0.44 min (analytical condition SQDAA05)

Synthesis of (2S)-(2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl4-(methylsulfonyl)-2-(pent-4-enamido)butanoate (Compound pc35, Pen-Met(O2)-pCpA)

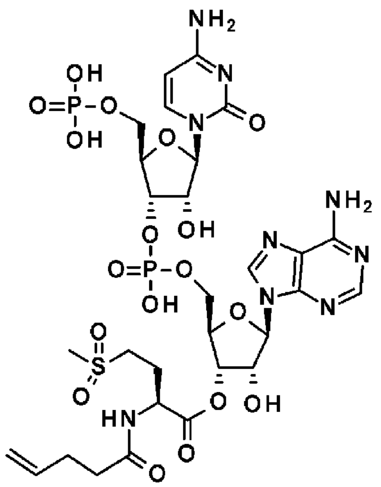

To the mixture of (2S)-(2R,3S,4R,5R)-2-((((((2R,3R,4R,5R)-5-(4-amino-2-oxopyrimidin-1(2H)-yl)-2-((phosphonooxy)methyl)-4-((tetrahydrofuran-2-yl)oxy)tetrahydrofuran-3-yl)oxy)(hydroxy)phosphoryl)oxy)methyl)-5-(6-amino-9H-purin-9-yl)-4-hydroxytetrahydrofuran-3-yl 4-(methylsulfonyl)-2-(pent-4-enamido)butanoate (Compound pc34) and N,N,N-trimethyl hexadecan-1-aminium chloride obtained in the previous step (70 mg) was added a 80% aqueous acetic acid solution (1.0 mL), and the mixture was stirred at room temperature for 2 h. The reaction solution was lyophilized, and the resulting residue was purified by reverse phase silica gel column chromatography (0.05% aqueous trifluoroacetic acid solution/0.05% trifluoroacetic acid-acetonitrile) to afford the title compound (Compound pc35, Pen-Met(O2)-pCpA) (12.7 mg, 7%, two steps).
LCMS (ESI) m/z = 898 $(M+H)^+$
Retention time: 0.29 min (analytical condition SQDFA05)

Synthesis of cyanomethyl (S)-3-(3-chlorophenyl)-2-(pent-4-enamido)propanoate (Compound pc36, Pen-Phe(3-Cl)-$OCH_2CN$)

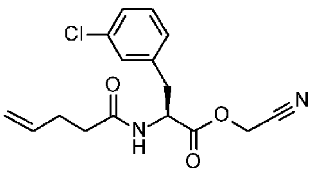

To (S)-2-((((9H-fluoren-9-yl)methoxy)carbonyl)amino)-3-(3-chlorophenyl)propanoic acid (Fmoc-Phe(3-Cl)-OH) (5.00 g, 11.9 mmol) was added a 20% solution of piperidine in DMF (76 mL), and the reaction solution was then stirred at 25°C for 16 h. The reaction solution was concentrated, diethyl ether was then added to the resulting residue, and the mixture was filtered to give a crude product (S)-2-amino-3-(3-chlorophenyl)propanoic acid (H--Phe(3-Cl)-OH) (2.22 g).

To a mixed solvent of water (28 mL) and 1,4-dioxane (28 mL) were added the resulting crude product (S)-2-amino-3-(3-chlorophenyl)propanoic acid (H--Phe(3-Cl)-OH) (2.22 g), 2,5-dioxopyrrolidin-1-yl pent-4-enoate (2.62 g, 13.3 mmol), and sodium bicarbonate (1.86 g, 22.1 mmol), and the reaction solution was then stirred at 25°C for 16 h. The reaction solution was washed with ethyl acetate twice, after which the aqueous layer was adjusted to pH 2 with a 1 M aqueous hydrochloric acid solution and extracted with dichloromethane three times. The resulting organic layers were combined, washed with brine, dried over anhydrous sodium sulfate, filtered, and concentrated under reduced pressure. The resulting residue was purified by normal phase silica gel column chromatography (dichloromethane/methanol) to afford (S)-3-(3-chlorophenyl)-2-(pent-4-enamido)propanoic acid (Pen-Phe(3-Cl)-OH) (1.5 g, 45%, two steps).

(S)-3-(3-Chlorophenyl)-2-(pent-4-enamido)propanoic acid (Pen-Phe(3-Cl)-OH) (1.50 g, 5.32 mmol) and N-ethyl-isopropylpropan-2-amine (DIPEA) (1.37 g, 10.6 mmol) were dissolved in dichloromethane (24 ml) under a nitrogen atmosphere, 2-bromoacetonitrile (2.55 g, 21.3 mmol) was added, and the mixture was stirred at 25°C for 16 h. The reaction solution was concentrated and the resulting residue was purified by normal phase silica gel column chromatography (petroleum ether/ethyl acetate) to afford cyanomethyl (S)-3-(3-chlorophenyl)-2-(pent-4-enamido)propanoate (Compound pc36, Pen-Phe(3-Cl)-$OCH_2CN$) (0.767 g, 45%).

LCMS (ESI) m/z = 321 $(M+H)^+$
Retention time: 0.87 min (analytical condition SQDAA05)

Synthesis of (2S)-(2R,3S,4R,5R)-2-((((((2R,3R,4R,5R)-5-(4-amino-2-oxopyrimidin-1(2H)-yl)-2-((phosphonooxy)methyl)-4-((tetrahydrofuran-2-yl)oxy)tetrahydrofuran-3-yl)oxy)(hydroxy)phosphoryl)oxy)methyl)-5-(6-amino-9H-purin-9-yl)-4-hydroxytetrahydrofuran-3-yl3-(3-chlorophenyl)-2-(pent-4-enamido)propanoate (Compound pc37)

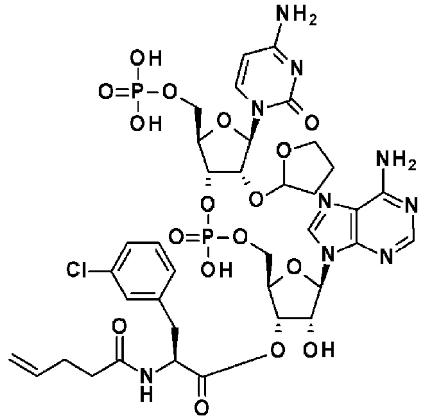

To buffer A (120 ml) were added a solution of ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (200 mg, 0.277 mmol) in water (3.0 mL) and a solution of cyanomethyl (S)-3-(3-chlorophenyl)-2-(pent-4-enamido)propanoate (Compound pc36, Pen-Phe(3-Cl)-$OCH_2CN$) (355 mg, 1.11 mmol) in acetonitrile (3.0 ml), and the mixture was stirred at room temperature for 2 h. The reaction solution was lyophilized and the resulting residue was reverse phase silica gel column chromatography (10 mM aqueous ammonium acetate/methanol) to afford a mixture of the title compound (Compound pc37) and N,N,N-trimethylhexadecan-1-aminium chloride (75 mg).
LCMS (ESI) m/z = 986 $(M+H)^+$
Retention time: 0.77 min (analytical condition SQDAA05)

Synthesis of (2S)-(2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl 3-(3-chlorophenyl)-2-(pent-4-enamido)propanoate (Compound pc38, Pen-Phe(3-Cl)-pCpA)

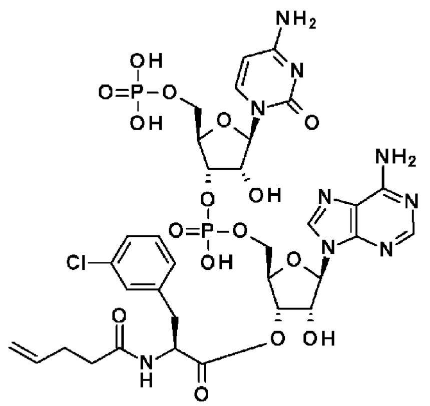

To the mixture of (2S)-(2R,3S,4R,5R)-2-((((((2R,3R,4R,5R)-5-(4-amino-2-oxopyrimidin-1(2H)-yl)-2-((phosphonooxy)methyl)-4-((tetrahydrofuran-2-yl)oxy)tetrahydrofuran-3-yl)oxy)(hydroxy)phosphoryl)oxy)methyl)-5-(6-amino-9H-purin-9-yl)-4-hydroxytetrahydrofuran-3-yl 3-(3-chlorophenyl)-2-(pent-4-enamido)propanoate (Compound pc37) and

N,N,N-trimethylhexadecan-1-aminium chloride obtained in the previous step (40 mg) was added a 80% aqueous acetic acid solution (0.80 mL), and the mixture was stirred at room temperature for 6 h. The reaction solution was purified by reverse phase silica gel column chromatography (10 mM aqueous ammonium acetate solution/methanol) and then purified again by reverse phase silica gel column chromatography (0.05% aqueous trifluoroacetic acid solution/0.05% trifluoroacetic acid-acetonitrile) to afford the title compound (Compound pc38, Pen-Phe(3-Cl)-pCpA) (7.5 mg, 6%, two steps).
LCMS (ESI) m/z = 914 $(M-H)^-$
Retention time: 0.47 min (analytical condition SQDFA05)

Synthesis of (S)-cyanomethyl 3-(4-chlorophenyl)-2-(N-methylpent-4-enamido)propanoate (Compound pc41, Pen-MePhe(4-Cl)-$OCH_2CN$)

The title compound was synthesized according to the following scheme.

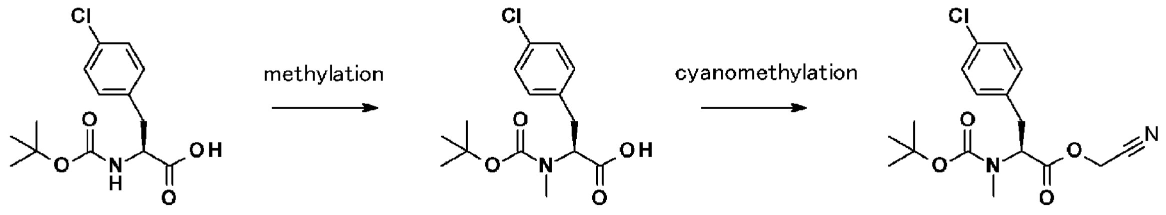


Compound pc39

Compound pc40

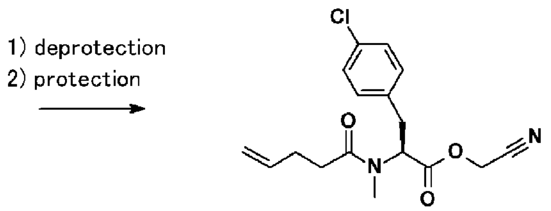


Compound pc41

(S)-2-((tert-Butoxycarbonyl)amino)-3-(4-chlorophenyl)propanoic acid (Boc-Phe(4-Cl)-OH) (4.00 g, 13.3 mmol) was dissolved in tetrahydrofuran (THF) (120 mL), after which sodium hydride (1.60 g, 40.0 mmol, 60% oil dispersion) and methyl iodide (9.48 g, 66.8 mmol) were added at 0°C. The reaction solution was stirred at 30°C for two days in an oil bath, and the reaction was then quenched with ice water (50 mL). The mixed solution was washed with ethyl acetate three times, after which the aqueous layer was adjusted to pH 3-4 with sodium bisulfate and extracted with ethyl acetate three times. The organic layers were combined, dried over anhydrous sodium sulfate, filtered, and concentrated, and the resulting residue was purified by normal phase silica gel column chromatography (dichloromethane/methanol) to afford (S)-2-((tert-butoxycarbonyl)(methyl)amino)-3-(4-chlorophenyl)propanoic acid (Compound pc39, Boc-MePhe(4-Cl)-OH) (2.20 g, 53%).
To a solution of (S)-2-((tert-butoxycarbonyl)(methyl)amino)-3-(4-chlorophenyl)propanoic acid (Compound pc39, Boc-MePhe(4-Cl)-OH) obtained in the previous step (1.50 g, 4.78 mmol) in dichloromethane (80 mL) were added N-ethyl-isopropylpropan-2-amine (DIPEA) (1.24 g, 9.59 mmol) and 2-bromoacetonitrile (2.28 g, 19.0 mmol) under a nitrogen atmosphere, and the mixture was stirred at 25°C for 16 h. The reaction solution was concentrated and the resulting residue was purified by normal phase silica gel column chromatography (petroleum ether/ethyl acetate) to afford cyanomethyl (S)-2-((tert-butoxycarbonyl)(methyl)amino)-3-(4-chlorophenyl)propanoate (Compound pc40, Boc-MePhe(4-Cl)-$OCH_2CN$) (1.45 g, 86%).
Cyanomethyl (S)-2-((tert-butoxycarbonyl)(methyl)amino)-3-(4-chlorophenyl)propanoate obtained in the previous step (Compound pc40, Boc-MePhe(4-Cl)-$OCH_2CN$) (1.30 g, 3.68 mmol) was dissolved in dichloromethane (50 mL), and the reaction solution was bubbled with hydrochloric acid gas and stirred at 20°C for 1 h. The reaction solution was concentrated to afford cyanomethyl (S)-3-(4-chlorophenyl)-2-(methylamino)propanoate (1.06 g) as a mixture.
The mixture of cyanomethyl (S)-3-(4-chlorophenyl)-2-(methylamino)propanoate obtained in the previous step (0.96 g) was dissolved in dichloromethane (50 mL), triethylamine (840 mg, 8.30 mmol) was added at 0°C, and a solution of pent-4-enoyl chloride (472 mg, 3.98 mmol) in dichloromethane (10 mL) was then added dropwise. The reaction solution was stirred at 20°C for 2 h and then concentrated, and the resulting residue was purified by normal phase silica gel

column chromatography (petroleum ether/ethyl acetate) to afford (S)-cyanomethyl 3-(4-chlorophenyl)-2-(N-methylpent-4-enamido)propanoate (Compound pc41, Pen-MePhe(4-Cl)-$OCH_2CN$) (0.918 g, 83%, two steps).
LCMS (ESI) m/z = 335 $(M+H)^+$
Retention time: 2.21 min (analytical condition SMD method 5)

Synthesis of (2S)-(2R,3S,4R,5R)-2-((((((2R,3R,4R,5R)-5-(4-amino-2-oxopyrimidin-1(2H)-yl)-2-((phosphonooxy)methyl)-4-((tetrahydrofuran-2-yl)oxy)tetrahydrofuran-3-yl)oxy)(hydroxy)phosphoryl)oxy)methyl)-5-(6-amino-9H-purin-9-yl)-4-hydroxytetrahydrofuran-3-yl 3-(4-chlorophenyl)-2-(N-methylpent-4-enamido)propanoate (Compound pc42)

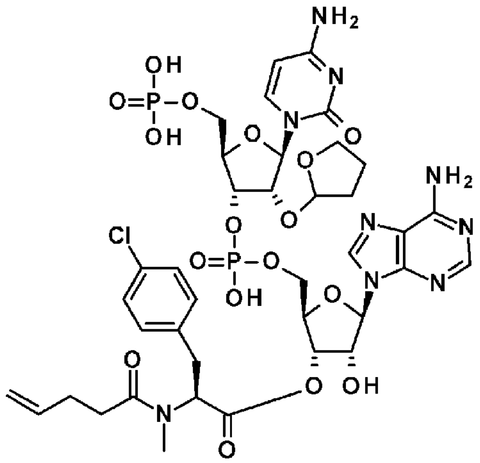

To buffer A (115 ml) were added a solution of ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (200 mg, 0.277 mmol) in water (6.25 mL) and a solution of cyanomethyl (S)-3-(3-chlorophenyl)-2-(pent-4-enamido)propanoate (Compound pc36, Pen-Phe(3-Cl)-$OCH_2CN$) (371 mg, 1.11 mmol) in acetonitrile (3.1 ml), and the mixture was stirred at room temperature for 2 h. The reaction solution was lyophilized, and the resulting residue was purified by reverse phase silica gel column chromatography (0.1% aqueous formic acid solution/0.1% formic acid-acetonitrile solution) to afford a mixture of the title compound (Compound pc42) and N,N,N-trimethylhexadecan-1-aminium chloride (277 mg).
LCMS (ESI) m/z = 1000 $(M+H)^+$
Retention time: 0.58 min (analytical condition SQDFA05)

Synthesis of (2S)-(2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl 3-(4-chlorophenyl)-2-(N-methylpent-4-enamido)propanoate (Compound pc43)

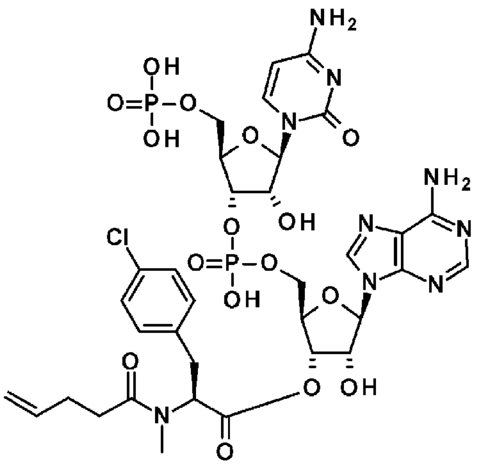

To the mixture of (2S)-(2R,3S,4R,5R)-2-((((((2R,3R,4R,5R)-5-(4-amino-2-oxopyrimidin-1(2H)-yl)-2-((phosphonooxy)methyl)-4-((tetrahydrofuran-2-yl)oxy)tetrahydrofuran-3-yl)oxy)(hydroxy)phosphoryl)oxy)methyl)-5-(6-amino-9H-purin-9-yl)-4-hydroxytetrahydrofuran-3-yl 3-(4-chlorophenyl)-2-(N-methylpent-4-enamido)propanoate (Compound pc42) and N,N,N-trimethylhexadecan-1-aminium chloride obtained in the previous step (277 mg) was added a 80% aqueous acetic acid solution (4.0 mL), and the mixture was stirred at room temperature for 4 h. The reaction solution was purified by reverse phase silica gel column chromatography (0.05% aqueous trifluoroacetic acid solution/0.05% trifluoroacetic acid-acetonitrile) to afford the title compound (Compound pc43, Pen-MePhe(4-Cl)-pCpA) (119 mg, 46%, two steps).
LCMS (ESI) m/z = 928 (M-H)-
Retention time: 0.52 min (analytical condition SQDFA05)

Synthesis of (2S)-(2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl 4-(methylthio)-4-oxo-2-(pent-4-enamido)butanoate (Compound pc44, Pen-Asp(SMe)-pCpA)

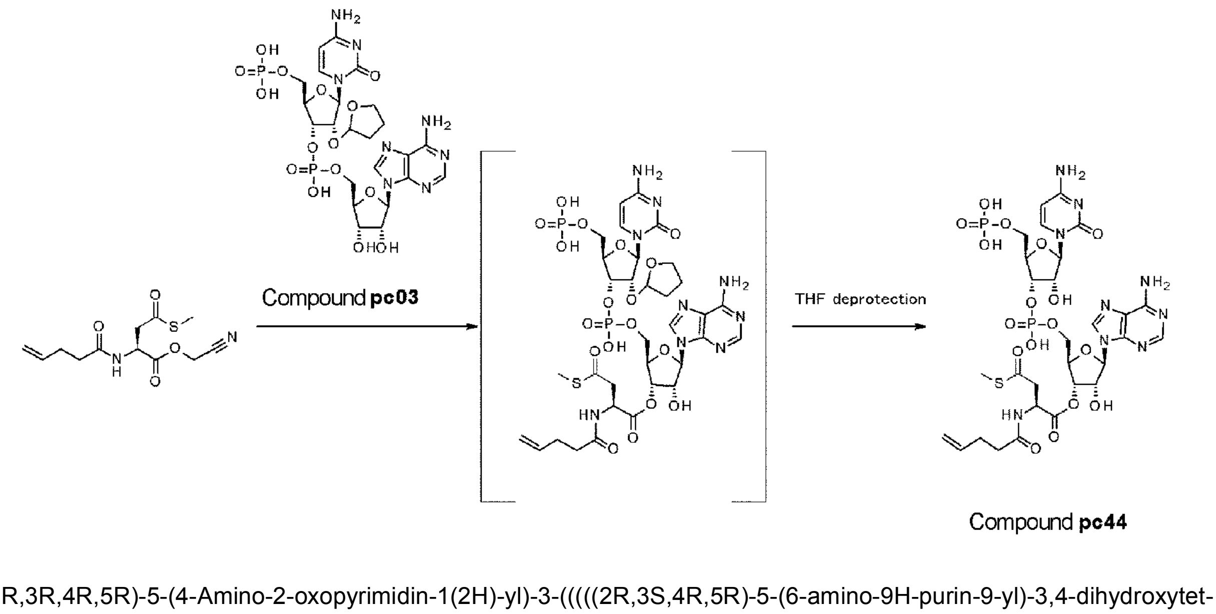


Compound pc44

((2R,3R,4R,5R)-5-(4-Amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (400 mg, 0.55 mmol) was dissolved in buffer A (200 ml), a solution of (S)-cyanomethyl 4-(methylthio)-4-oxo-2-(pent-4-enamido)butanoate synthesized by the method described in the document (WO 2013/100132) (630 mg, 2.22 mmol) in THF (4.0 ml) was added, and the mixture was stirred at room temperature for 1 h. To the reaction solution was further added trifluoroacetic acid (4.6 ml, 60 mmol), the reaction solution was lyophilized,

and the resulting residue was purified by reverse phase silica gel column chromatography (0.05% aqueous trifluoroacetic acid solution/0.05% trifluoroacetic acid-acetonitrile) to afford the title compound (Compound pc44, Pen-Asp(SMe)-pCpA) (20 mg, 4.2%).

LCMS (ESI) m/z = 880.4 $(M+H)^+$

Retention time: 0.38 min (analytical condition SQDFA05)

Compound pc50 (Pen-Ser(nPr)-pCpA) was synthesized according to the following scheme.

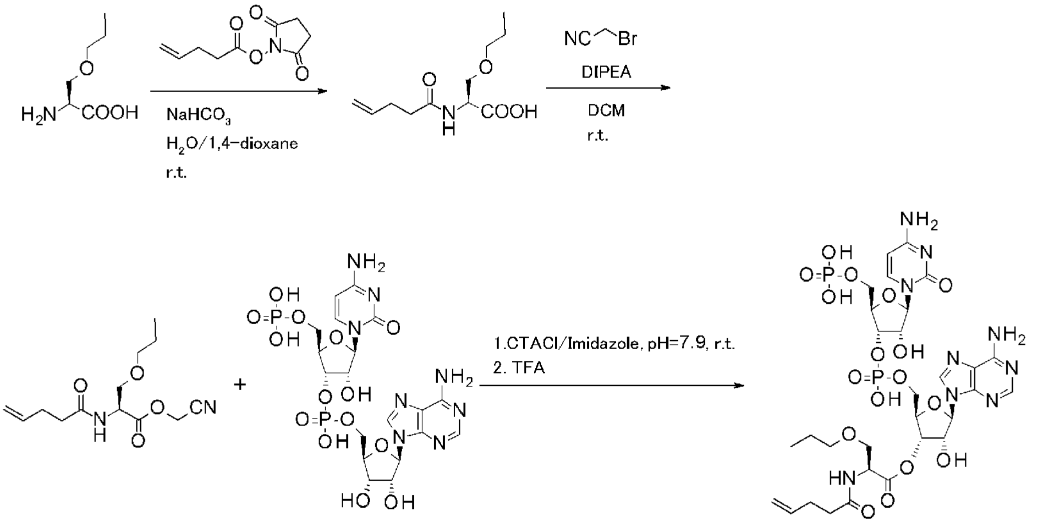


pc50

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl N-(pent-4-enoyl)-O-propyl-L-serinate (Compound pc50, Pen-Ser(nPr)-pCpA)

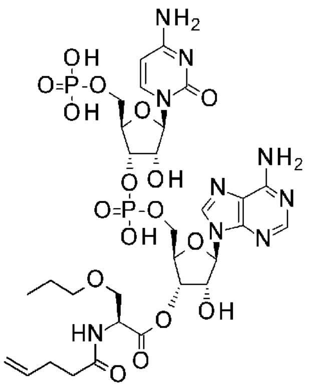

To O-propyl-L-serine (H-Ser(nPr)-OH) (2.5 g, 13.61 mmol) which is an intermediate obtained in the process of synthesizing N-(((9H-fluoren-9-yl)methoxy)carbonyl)-O-propyl-L-serine (Fmoc-Ser(nPr)-OH, Compound aa51), 2,5-dioxopyrrolidin-1-yl pent-4-enoate (6.7 g, 33.98 mmol), and sodium bicarbonate (2.9 g, 34.52 mmol) was added 1,4-dioxane/water (50 mL/50 mL), and the mixture was stirred at room temperature for 16 h. The reaction solution was then washed with diethyl ether, and the aqueous layer was adjusted to pH 5 and then extracted with dichloromethane. The extracted organic layer was purified by normal phase column chromatography (petroleum ether/ethyl acetate) to afford N-(pent-4-enoyl)-O-propyl-L-serine (Pen-Ser(nPr)-OH) (2.3 g, 74%).

The resulting N-(pent-4-enoyl)-O-propyl-L-serine (Pen-Ser(nPr)-OH) (2.3 g, 10.03 mmol), N,N-diisopropylethylamine (DIPEA) (2.6 g, 20.16 mmol), and 2-bromoacetonitrile (4.8 g, 40.02 mmol) were dissolved in dichloromethane (50 mL), and the solution was stirred at room temperature for 16 h. Subsequent purification by normal phase column chromatography (petroleum ether/ethyl acetate) gave cyanomethyl N-(pent-4-enoyl)-O-propyl-L-serinate (Pen-

Ser(nPr)-$OCH_2CN$) (1.8 g, 67%).

((2R,3R,4R,5R)-5-(4-Amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (300 mg, 0.42 mmol) and cyanomethyl N-(pent-4-enoyl)-O-propyl-L-serinate (Pen-Ser(nPr)-$OCH_2CN$) (334 mg, 1.24 mmol) were then added to buffer A (18 mL), and the mixture was stirred at room temperature for 1 h. To the reaction solution was then added trifluoroacetic acid (TFA) (1.7 mL), and the reaction solution was lyophilized. The resulting residue was purified by reverse phase column chromatography (0.05% aqueous trifluoroacetic acid solution/0.05% trifluoroacetic acid-acetonitrile solution) to afford the title compound (Compound pc50, Pen-Ser(nPr)-OH) (61.4 mg, 8%).

LCMS (ESI) m/z = 865 (M+H)+

Retention time: 0.40 min (analytical condition SQDFA05)

Compound pc51 (Pen-MeSer(nPr)-pCpA) was synthesized according to the following scheme.

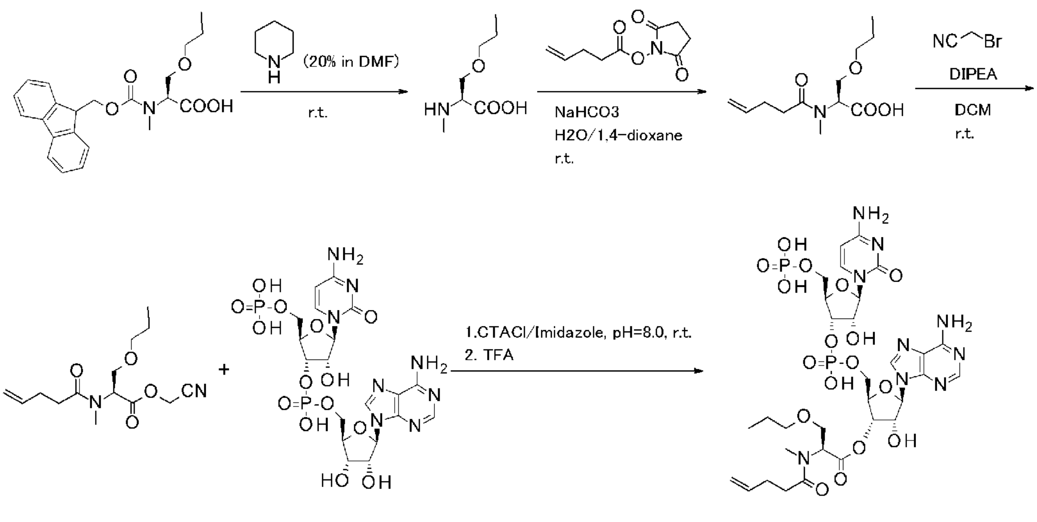


**pc51**

<u>Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl N-methyl-N-(pent-4-enoyl)-O-propyl-L-serinate (Compound pc51, Pen-MeSer(nPr)-pCpA)</u>

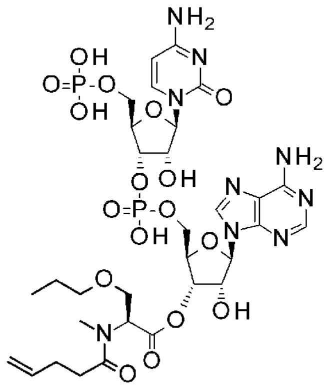

N-(((9H-Fluoren-9-yl)methoxy)carbonyl)-N-methyl-O-propyl-L-serine (Fmoc-MeSer(nPr)-OH, Compound aa52) (10 g, 22.70 mmol) was dissolved in dimethylformamide (DMF) (128 mL), piperidine (32 mL) was added, and the mixture was stirred at room temperature for 3 h. The reaction solution was then diluted with diethyl ether (500 mL), and the precipitated solid was collected to afford N-methyl-O-propyl-L-serine (H-MeSer(nPr)-OH).

To the resulting N-methyl-O-propyl-L-serine (H-MeSer(nPr)-OH), 2,5-dioxopyrrolidin-1-yl pent-4-enoate (7.88 g, 40.0 mmol), and sodium bicarbonate (3.36 g, 40.0 mmol) was added 1,4-dioxane/water (10 mL/10 mL), and the

mixture was stirred at room temperature for 16 h. The reaction solution was then washed with diethyl ether twice, and the aqueous layer was adjusted to pH 1 with an aqueous hydrochloric acid solution (1 M). The aqueous layer was extracted with dichloromethane twice, and the organic layers were dried over anhydrous sodium sulfate, filtered, and then concentrated under reduced pressure. The resulting residue was purified by normal phase column chromatography (petroleum ether/ethyl acetate = 100/0 -> 30/70) to afford N-methyl-N-(pent-4-enoyl)-O-propyl-L-serine (Pen-MeSer(nPr)-OH) (2.7 g, 43%).

The resulting N-methyl-N-(pent-4-enoyl)-O-propyl-L-serine (Pen-MeSer(nPr)-OH) (2.7 g, 11.1 mmol), N,N-diisopropylethylamine (DIPEA) (2.85 g, 22.1 mmol), and 2-bromoacetonitrile (5.30 g, 44.2 mmol) were dissolved in dichloromethane (60 mL), and the mixture was stirred at room temperature for 16 h. The solvent was then removed by concentration under reduced pressure, and the resulting residue was purified by normal phase column chromatography (petroleum ether/ethyl acetate = 100/0 -> 40/60) to afford cyanomethyl N-methyl-N-(pent-4-enoyl)-O-propyl-L-serinate (Pen-MeSer(nPr)-$OCH_2CN$) (2.22 g, 71%).

((2R,3R,4R,5R)-5-(4-Amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (400 mg, 0.56 mmol) was then dissolved in water (3.0 mL) and added to buffer A (100 mL). To this aqueous solution was added a solution of cyanomethyl N-methyl-N-(pent-4-enoyl)-O-propyl-L-serinate (Pen-MeSer(nPr)-$OCH_2CN$) obtained above (625 mg, 2.21 mmol) in tetrahydrofuran (3.0 mL), and the mixture was stirred at room temperature for 1 h. To the reaction solution was then added trifluoroacetic acid (2.3 mL), and the reaction solution was lyophilized. The resulting residue was purified by reverse phase column chromatography (0.05% aqueous trifluoroacetic acid solution/0.05% trifluoroacetic acid-acetonitrile solution) to afford the title compound (pc51, Pen-MeSer(nPr)-pCpA) (69 mg, 14%).

LCMS (ESI) m/z = 878.4 (M+H)+

Retention time: 10.393 min (analytical condition SMD method 21)

LCMS (ESI) m/z = 878.5 (M+H)+

Retention time: 10.910 min (analytical condition SMD method 21)

Compound pc52 (Pen-Ser(iPen)-pCpA) was synthesized according to the following scheme.

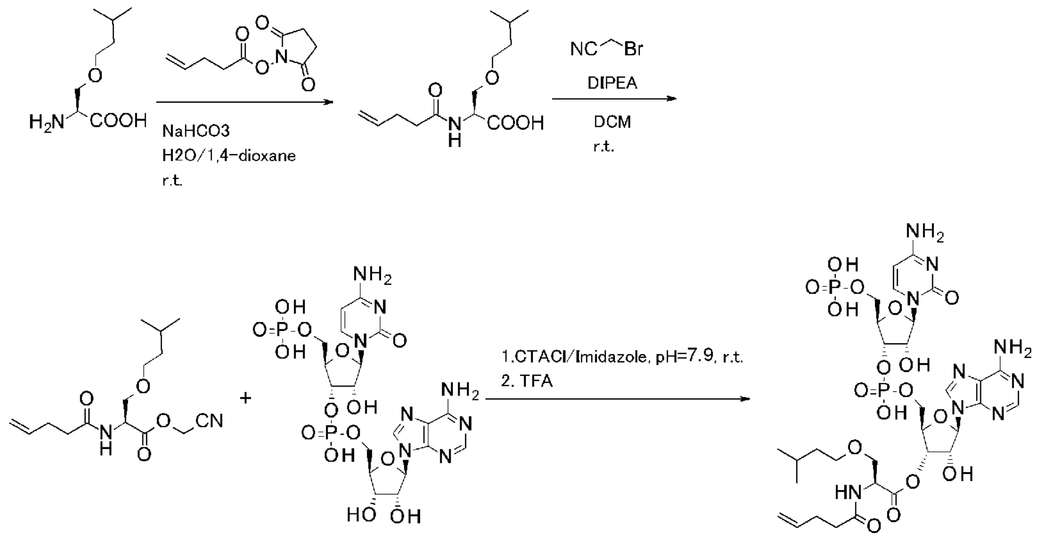


pc52

<u>Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl O-isopentyl-N-(pent-4-enoyl)-L-serinate (Compound pc52, Pen-Ser(iPen)-pCpA)</u>

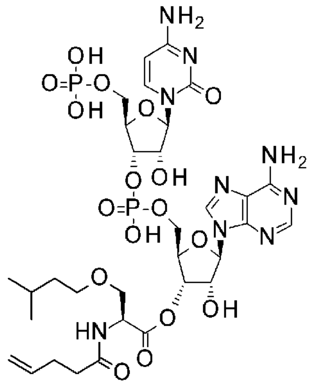

To O-isopentyl-L-serine (H-Ser(iPen)-OH) (2.0 g, 11.43 mmol) which is an intermediate obtained in the process of synthesizing N-(((9H-fluoren-9-yl)methoxy)carbonyl)-O-isopentyl-L-serine (Fmoc-Ser(iPen)-OH, Compound aa54), 2,5-dioxopyrrolidin-1-yl pent-4-enoate (4.5 g, 22.86 mmol), and sodium bicarbonate (1.92 g, 22.86 mmol) was added 1,4-dioxane/water (10 mL/10 mL), and the mixture was stirred at room temperature for 16 h. The reaction solution was then washed with diethyl ether twice, and the aqueous layer was adjusted to pH 2 with an 1 M aqueous hydrochloric acid solution. This aqueous layer was extracted with dichloromethane twice, and the resulting organic layers were dried over anhydrous magnesium sulfate, then filtered, and concentrated under reduced pressure. The resulting residue was purified by normal phase column chromatography (petroleum ether/ethyl acetate = 100/0 -> 30/70) to afford O-isopentyl-N-(pent-4-enoyl)-L-serine (Pen-Ser(iPen)-OH) (2.69 g, 89%).

The resulting O-isopentyl-N-(pent-4-enoyl)-L-serine (Pen-Ser(iPen)-OH) (2.62 g, 10.19 mmol), N,N-diisopropylethylamine (DIPEA) (2.63 g, 20.38 mmol), and 2-bromoacetonitrile (4.89 g, 40.78 mmol) were dissolved in dichloromethane (60 mL), and the mixture was stirred at room temperature for 16 h. Subsequent purification by normal phase column chromatography (petroleum ether/ethyl acetate = 100/0 -> 40/60) gave cyanomethyl O-isopentyl-N-(pent-4-enoyl)-L-serinate (Pen-Ser(iPen)-$OCH_2CN$) (2.22 g, 71%).

((2R,3R,4R,5R)-5-(4-Amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (300 mg, 0.42 mmol) and cyanomethyl O-isopentyl-N-(pent-4-enoyl)-L-serinate (Pen-Ser(iPen)-$OCH_2CN$) (369 mg, 1.25 mmol) were then added to buffer A (75 mL), and the mixture was stirred at room temperature for 1 h. To the reaction solution was then added trifluoroacetic acid (1.7 mL), and the reaction solution was lyophilized. The resulting residue was purified by reverse phase column chromatography (0.05% aqueous trifluoroacetic acid solution/0.05% trifluoroacetic acid-acetonitrile solution) to afford the title compound (Compound pc52, Pen-Ser(iPen)-pCpA) (44.1 mg, 6%).

LCMS (ESI) m/z = 893 (M+H)+

Retention time: 0.44 min (analytical condition SQDFA05)

Compound pc53 (Pen-MeSer(iPen)-pCpA) was synthesized according to the following scheme.

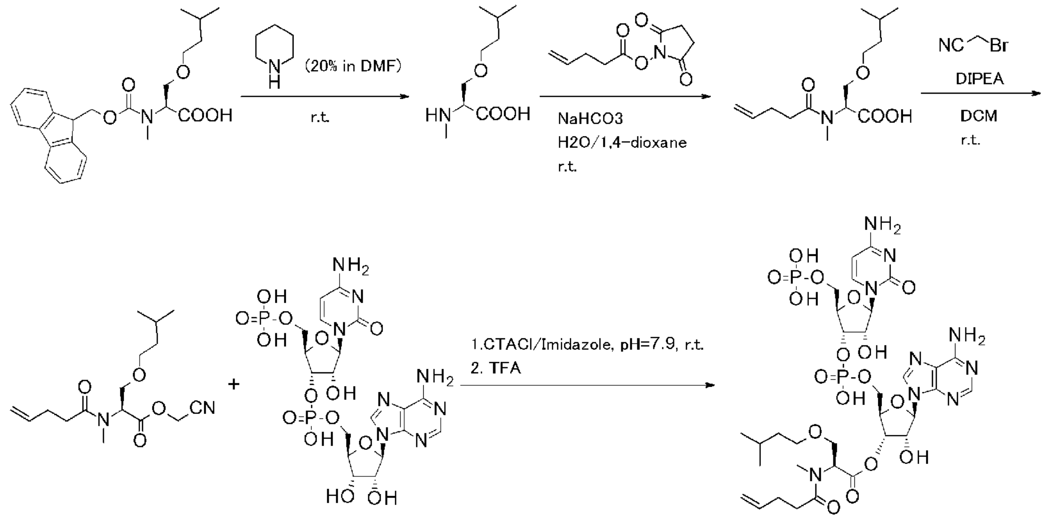


pc53

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl O-isopentyl-N-methyl-N-(pent-4-enoyl)-L-serinate (Compound pc53, Pen-MeSer(iPen)-pCpA)

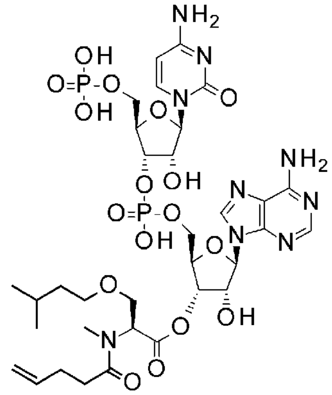

N-(((9H-Fluoren-9-yl)methoxy)carbonyl)-O-isopentyl-N-methyl-L-serine (Fmoc-MeSer(iPen)-OH, Compound aa55) (18 g, 43.74 mmol) was dissolved in dimethylformamide (126 mL), piperidine (54 mL) was added, and the mixture was stirred at room temperature for 3 h. The precipitated solid was then collected to afford O-isopentyl-N-methyl-L-serine (H-MeSer(iPen)-OH).

To the resulting O-isopentyl-N-methyl-L-serine (H-MeSer(iPen)-OH), 2,5-dioxopyrrolidin-1-yl pent-4-enoate (15.6 g, 79.11 mmol), and sodium bicarbonate (4.4 g, 52.37 mmol) was added 1,4-dioxane/water (100 mL/100 mL), and the mixture was stirred at room temperature for 16 h. The reaction solution was then washed with diethyl ether twice, and the aqueous layer was adjusted to pH 1 with an aqueous hydrochloric acid solution. The aqueous layer was extracted with dichloromethane twice, and the organic layers were dried over anhydrous sodium sulfate, filtered, and then concentrated under reduced pressure. The resulting residue was purified by column chromatography (petroleum ether/ethyl acetate = 2/1) to afford O-isopentyl-N-methyl-N-(pent-4-enoyl)-L-serine (Pen-MeSer(iPen)-OH) (5.6 g, 78%).

The resulting O-isopentyl-N-methyl-N-(pent-4-enoyl)-L-serine (Pen-MeSer(iPen)-OH) (4.0 g, 14.74 mmol), N,N-diisopropylethylamine (DIPEA) (3.8 g, 29.4 mmol), and 2-bromoacetonitrile (7.08 g, 59.03 mmol) were dissolved in dichloromethane (80 mL), and the mixture was stirred at room temperature for 16 h. The solvent was then removed by concentration under reduced pressure, and the resulting residue was purified by normal phase column chromatography (petroleum ether/ethyl acetate = 2/1) to afford cyanomethyl O-isopentyl-N-methyl-N-(pent-4-enoyl)-L-serinate (Pen-MeSer(iPen)-$OCH_2CN$) (2 g, 44%).

((2R,3R,4R,5R)-5-(4-Amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (400 mg, 0.55 mmol) was then dissolved in buffer A (100 mL), cyanomethyl O-isopentyl-N-methyl-N-(pent-4-enoyl)-L-serinate (Pen-MeSer(iPen)-$OCH_2CN$) obtained above (686 mg, 2.21 mmol) was added, and the mixture was stirred at room temperature for 2 h. To the reaction solution was then added trifluoroacetic acid (2.3 mL), and the reaction solution was lyophilized. The resulting residue was purified by reverse phase column chromatography (0.05% aqueous trifluoroacetic acid solution/0.05% trifluoroacetic acid-acetonitrile solution) to afford the title compound (Compound pc53, Pen-MeSer(iPen)-pCpA) (59 mg, 6%).
LCMS (ESI) m/z = 907 (M+H)+
Retention time: 0.53 min (analytical condition SQDFA05)

Synthesis of O-ethyl-N-(pent-4-enoyl)-L-homoserine (Compound pc54, Pen-Hse(Et)-OH)

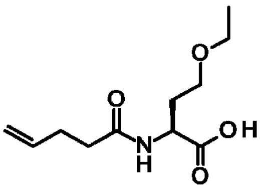

To a solution of commercially available N-(((9H-fluoren-9-yl)methoxy)carbonyl)-O-ethyl-L-homoserine (Fmoc-Hse(Et)-OH, purchased from Watanabe Chemical Industries) (200 mg, 0.541 mmol) in dichloromethane (DCM) (2.0 mL) was added 4-(3-phenylpropyl)piperidine (0.229 mL, 1.083 mmol) at room temperature under a nitrogen atmosphere. The reaction solution was stirred at room temperature for 7 h, after which 4-(3-phenylpropyl)piperidine (0.229 mL, 1.083 mmol) was further added and the mixture was stirred at room temperature for 2 h. Water was added to the reaction solution, followed by washing with hexane. The resulting aqueous layer was purified by reverse phase column chromatography (0.1% aqueous formic acid solution/0.1% formic acid-acetonitrile solution) to afford O-ethyl-L-homoserine (H-Hse(Et)-OH) (78 mg, 98%).

To a solution of O-ethyl-L-homoserine (H-Hse(Et)-OH) (78 mg, 0.530 mmol) in water (2.0 mL) was added sodium carbonate (129 mg, 1.219 mmol) at room temperature under a nitrogen atmosphere, and the reaction solution was stirred until it became clear. To the reaction solution was added a solution of pent-4-enoyl chloride (0.93 mL, 8.39 mmol) in tetrahydrofuran (THF) (2.0 mL) dropwise at room temperature, and the mixture was stirred for 2 h. To the reaction solution were further added pent-4-enoyl chloride (0.93 mL, 8.39 mmol) and sodium carbonate (129 mg, 1.219 mmol) at room temperature, and the mixture was stirred for 13 h. To the reaction solution was added formic acid (0.203 mL), the tetrahydrofuran (THF) was removed by concentration under reduced pressure, and the residue was diluted with water. The resulting solution was purified by reverse phase column chromatography (0.1% aqueous formic acid solution/0.1% formic acid-acetonitrile solution) to afford O-ethyl-N-(pent-4-enoyl)-L-homoserine (Compound pc54, Pen-Hse(Et)-OH) (93 mg, 77%).
LCMS (ESI) m/z = 230 (M+H)+
Retention time: 0.45 min (analytical condition SQDFA05)

Synthesis of cyanomethyl O-ethyl-N-(pent-4-enoyl)-L-homoserinate (Compound pc55, Pen-Hse(Et)-$OCH_2CN$)

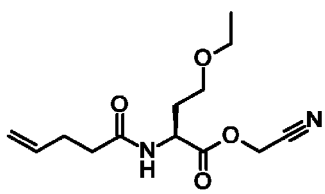

To a solution of O-ethyl-N-(pent-4-enoyl)-L-homoserine (Compound pc54, Pen-Hse(Et)-OH) (93 mg, 0.406 mmol) in acetonitrile (1.0 mL) were added N-ethyl-isopropylpropan-2-amine (DIPEA) (0.213 mL, 1.217 mmol) and 2-bromoacetonitrile (0.170 mL, 2.434 mmol) under a nitrogen atmosphere, and the mixture was stirred at room temperature

for 2 hours and 30 minutes. The reaction solution was concentrated and the resulting residue was purified by normal phase silica gel column chromatography (hexane/ethyl acetate) to afford cyanomethyl O-ethyl-N-(pent-4-enoyl)-L-homoserinate (Compound pc55, Pen-Hse(Et)-$OCH_2CN$) (110 mg) quantitatively.
LCMS (ESI) m/z = 269 (M+H)+
Retention time: 0.56 min (analytical condition SQDFA05)

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl O-ethyl-N-(pent-4-enoyl)-L-homoserinate (Compound pc56, Pen-Hse(Et)-pCpA)

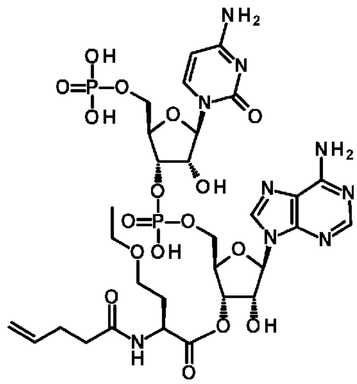

To buffer A (25 mL) were added a solution of ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (50 mg, 0.069 mmol) in water (2.5 mL) and a solution of cyanomethyl O-ethyl-N-(pent-4-enoyl)-L-homoserinate (Compound pc55, Pen-Hse(Et)-$OCH_2CN$) (37.1 mg, 0.138 mmol) in acetonitrile (1.25 mL), and the mixture was stirred at room temperature for 3 h. To the reaction solution was added trifluoroacetic acid (0.5 mL) at 0°C, and the mixture was stirred at 0°C for 1 hour and 30 minutes. The reaction solution was diluted with water and purified by reverse phase column chromatography (0.05% aqueous trifluoroacetic acid solution/0.05% trifluoroacetic acid-acetonitrile solution) to afford the title compound (pc56, Pen-Hse(Et)-pCpA) (14.0 mg, 23% over two steps).
LCMS (ESI) m/z = 865 (M+H)+
Retention time: 0.38 min (analytical condition SQDFA05)

Synthesis of O-ethyl-N-methyl-N-(pent-4-enoyl)-L-homoserine (Compound pc57, Pen-MeHse(Et)-OH)

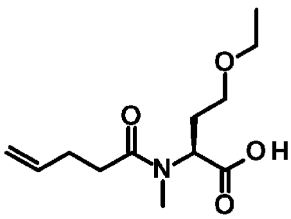

To a solution of N-(((9H-fluoren-9-yl)methoxy)carbonyl)-O-ethyl-N-methyl-L-homoserine (Compound aa106, Fmoc-MeHse(Et)-OH) (101 mg, 0.263 mmol) in dichloromethane (DCM) (1.0 mL) was added 4-(3-phenylpropyl)piperidine (0.112 mL, 0.527 mmol) at room temperature under a nitrogen atmosphere. The reaction solution was stirred at room temperature for 7 h, after which 4-(3-phenylpropyl)piperidine (0.112 mL, 0.527 mmol) was further added and the mixture was stirred at room temperature for 2 h. Water was added to the reaction solution, followed by washing with hexane. The resulting aqueous layer was purified by reverse phase column chromatography (0.1% aqueous formic acid solution/0.1% formic acid-acetonitrile solution) to afford O-ethyl-N-methyl-L-homoserine (H-MeHse(Et)-OH) (31 mg, 73%).
To a solution of O-ethyl-N-methyl-L-homoserine (H-MeHse(Et)-OH) (30 mg, 0.186 mmol) in water (700 μL)

was added sodium carbonate (45.4 mg, 0.428 mmol) at room temperature under a nitrogen atmosphere, and the reaction solution was stirred until it became clear. To the reaction solution was added a solution of pent-4-enoyl chloride (41.2 μL, 0.372 mmol) in tetrahydrofuran (THF) (0.7 mL) dropwise at room temperature, and the mixture was stirred for 2 h. To the reaction solution were further added pent-4-enoyl chloride (41.2 μL, 0.372 mmol) and sodium carbonate (45.4 mg, 0.428 mmol) at room temperature, and the mixture was stirred for 13 h. To the reaction solution was added again pent-4-enoyl chloride (41.2 μL, 0.372 mmol) and sodium carbonate (45.4 mg, 0.428 mmol) at room temperature, and the mixture was stirred for 5 hours and 30 minutes. To the reaction solution was added formic acid (0.107 mL), the tetrahydrofuran (THF) was removed by concentration under reduced pressure, and the residue was diluted with water. The resulting solution was purified by reverse phase column chromatography (0.1% aqueous formic acid solution/0.1% formic acid-acetonitrile solution) to afford O-ethyl-N-methyl-N-(pent-4-enoyl)-L-homoserine (Compound pc57, Pen-MeHse(Et)-OH) (24 mg, 53% over two steps).
LCMS (ESI) m/z = 244 (M+H)+
Retention time: 0.51 min (analytical condition SQDFA05)

Synthesis of cyanomethyl O-ethyl-N-methyl-N-(pent-4-enoyl)-L-homoserinate (Compound pc58, Pen-MeHse(Et)-$OCH_2CN$)

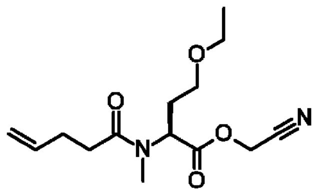

To a solution of O-ethyl-N-methyl-N-(pent-4-enoyl)-L-homoserine (Compound pc57, Pen-MeHse(Et)-OH) (22 mg, 0.090 mmol) in acetonitrile (0.5 mL) were added N-ethyl-isopropylpropan-2-amine (DIPEA) (0.047 mL, 0.271 mmol) and 2-bromoacetonitrile (0.038 mL, 0.543 mmol) under a nitrogen atmosphere, and the mixture was stirred at room temperature for 5 hours and 30 minutes. The reaction solution was concentrated and the resulting residue was purified by normal phase silica gel column chromatography (hexane/ethyl acetate) to afford cyanomethyl O-ethyl-N-methyl-N-(pent-4-enoyl)-L-homoserinate (Compound pc58, Pen-MeHse(Et)-$OCH_2CN$) (26 mg) quantitatively.
LCMS (ESI) m/z = 283 (M+H)+
Retention time: 0.64 min (analytical condition SQDFA05)

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl O-ethyl-N-methyl-N-(pent-4-enoyl)-L-homoserinate (Compound pc59, Pen-MeHse(Et)-pCpA)

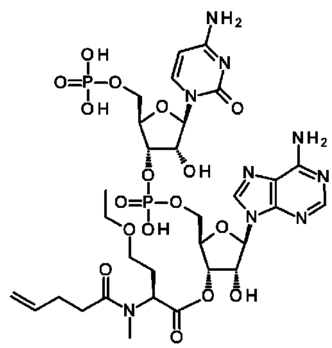

To buffer A (20 mL) were added a solution of ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (33.3 mg, 0.046 mmol) in water (2.0 mL) and a solution of cyanomethyl O-ethyl-N-methyl-N-(pent-4-enoyl)-L-homoserinate (Compound

pc38, Pen-MeHse(Et)-$OCH_2CN$) (26.0 mg, 0.092 mmol) in acetonitrile (1.0 mL), and the mixture was stirred at room temperature for 3 h. To the reaction solution was added trifluoroacetic acid (0.4 mL) at 0°C, and the mixture was stirred at 0°C for 3 h. The reaction solution was diluted with water and purified by reverse phase column chromatography (0.05% aqueous trifluoroacetic acid solution/0.05% trifluoroacetic acid-acetonitrile solution) to afford the title compound (Compound pc59, Pen-MeHse(Et)-pCpA) (10.0 mg, 25% over two steps).
LCMS (ESI) m/z = 879 (M+H)+
Retention time: 0.42 min (analytical condition SQDFA05)

Synthesis of O-methyl-N-(pent-4-enoyl)-L-serine (Compound pc60, Pen-Ser(Me)-OH)

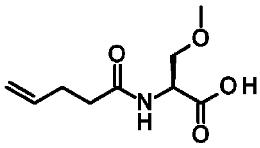

To a solution of commercially available O-methyl-L-serine (H-Ser(Me)-OH, purchased from Watanabe Chemical Industries) (500 mg, 4.20 mmol) in water (4.0 mL) was added sodium carbonate (1.02 g, 9.65 mmol) at room temperature. The solution was stirred until it became clear, after which tetrahydrofuran (THF) (4.0 mL) and pent-4-enoyl chloride (0.93 mL, 8.39 mmol) were added. The reaction solution was stirred at room temperature for 2 hours and 20 minutes, after which pent-4-enoyl chloride (0.93 mL, 8.39 mmol) and sodium carbonate (1.02 g, 9.65 mmol) were added and the mixture was further stirred at room temperature for 2 h. The reaction solution was diluted with water, formic acid (1.61 mL) was added, and the tetrahydrofuran (THF) was removed by concentration under reduced pressure. The resulting solution was purified by reverse phase column chromatography (0.1% aqueous formic acid solution/0.1% formic acid-acetonitrile solution) to afford O-methyl-N-(pent-4-enoyl)-L-serine (Compound pc60, Pen-Ser(Me)-OH) (583 mg, 69%).
LCMS (ESI) m/z = 202 (M+H)+
Retention time: 0.36 min (analytical condition SQDFA05)

Synthesis of cyanomethyl O-methyl-N-(pent-4-enoyl)-L-serinate (Compound pc61, Pen-Ser(Me)-$OCH_2CN$)

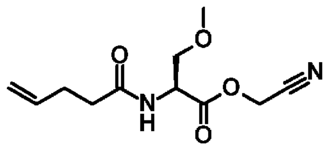

To a solution of O-methyl-N-(pent-4-enoyl)-L-serine (Compound pc60, Pen-Ser(Me)-OH) (300 mg, 1.491 mmol) in acetonitrile (3.0 mL) were added N-ethyl-isopropylpropan-2-amine (DIPEA) (0.781 mL, 4.47 mmol) and 2-bromoacetonitrile (0.623 mL, 8.95 mmol) under a nitrogen atmosphere, and the mixture was stirred at room temperature for 4 h. The reaction solution was concentrated and the resulting residue was purified by normal phase silica gel column chromatography (hexane/ethyl acetate) to afford cyanomethyl O-methyl-N-(pent-4-enoyl)-L-serinate (Compound pc61, Pen-Ser(Me)-$OCH_2CN$) (283 mg, 79%).
LCMS (ESI) m/z = 241 $(M+H)^+$
Retention time: 0.48 min (analytical condition SQDFA05)

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl O-methyl-N-(pent-4-enoyl)-L-serinate (Compound pc62, Pen-Ser(Me)-pCpA)

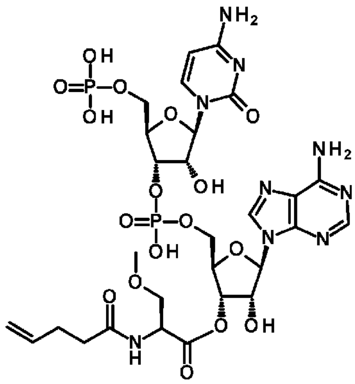

To buffer A (50 mL) were added a solution of ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (100 mg, 0.138 mmol) in water (5.0 mL) and a solution of cyanomethyl O-methyl-N-(pent-4-enoyl)-L-serinate (Compound pc61, Pen-Ser(Me)-$OCH_2CN$) (66.5 mg, 0.277 mmol) in acetonitrile (2.5 mL), and the mixture was stirred at room temperature for 4 h. To the reaction solution was added trifluoroacetic acid (1.0 mL) at 0°C, and the mixture was stirred at 0°C for 1 hour and 30 minutes. The reaction solution was diluted with water and purified by reverse phase column chromatography (0.05% aqueous trifluoroacetic acid solution/0.05% trifluoroacetic acid-acetonitrile solution) to afford the title compound (Compound pc62, Pen-Ser(Me)-pCpA) (14.0 mg, 12% over two steps).
LCMS (ESI) m/z = 837 (M+H)+
Retention time: 0.35 min (analytical condition SQDFA05)

Synthesis of (S)-2-amino-7,7-difluoroheptanoic acid (Compound pc63, H-Hnl(7-F2)-OH)

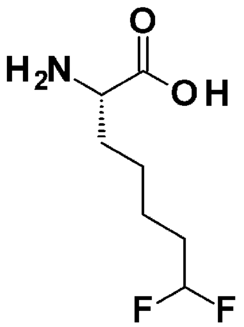

(S)-2-(((9H-Fluoren-9-yl)methoxy)carbonylamino)-7,7-difluoroheptanoic acid (Compound aa58, Fmoc-Hnl(7-F2)-OH) (143 mg, 0.348 mmol) was dissolved in dichloromethane (696 μL), 4-(3-phenylpropyl)piperidine (221 μL, 1.043 mmol) and water (1 mL) were added, and the mixture was stirred at room temperature for 4 h. The resulting reaction solution was directly purified by reverse phase column chromatography (0.1% aqueous formic acid solution/0.1% formic acid-acetonitrile solution) to afford (S)-2-amino-7,7-difluoroheptanoic acid (Compound pc63, H-Hnl(7-F2)-OH) (29.4 mg, 47%).
LCMS (ESI) m/z = 182 (M+H)+
Retention time: 0.24 min (analytical condition SQDFA05)

Synthesis of (S)-7,7-difluoro-2-(pent-4-enamido)heptanoic acid (Compound pc64, Pen-Hnl(7-F2)-OH)

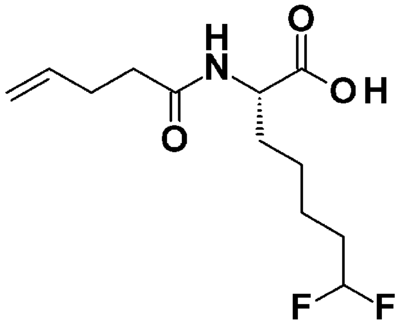

To (S)-2-amino-7,7-difluoroheptanoic acid (Compound pc63, H-Hnl(7-F2)-OH) already described in Examples (14.7 mg, 0.081 mmol) and sodium carbonate (17.2 mg, 0.162 mmol) were added water (243 μL) and 1,4-dioxane (162 μL) at room temperature under a nitrogen atmosphere and the mixture was stirred, after which pent-4-enoyl chloride (18 μL, 0.081 mmol) was added dropwise to the reaction solution at room temperature, and the mixture was stirred for 30 min. To the reaction solution was added a 2 N aqueous hydrochloric acid solution until pH = 2, and the mixture was extracted with ethyl acetate. The resulting organic layer was washed with brine and then concentrated under reduced pressure. The resulting residue was purified by reverse phase column chromatography (0.1% aqueous formic acid solution/0.1% formic acid-acetonitrile solution) to afford (S)-7,7-difluoro-2-(pent-4-enamido)heptanoic acid (Compound pc64, Pen-Hnl(7-F2)-OH) (10 mg, 47%).
LCMS (ESI) m/z = 264 (M+H)+
Retention time: 0.59 min (analytical condition SQDFA05)

Synthesis of cyanomethyl (S)-7,7-difluoro-2-(pent-4-enamido)heptanoate (Compound pc65, Pen-Hnl(7-F2)-$OCH_2CN$)

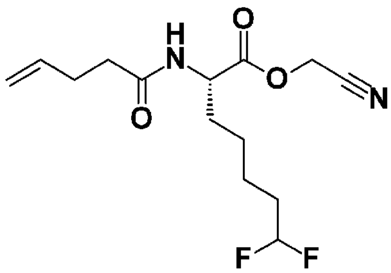

To a solution of (S)-7,7-difluoro-2-(pent-4-enamido)heptanoic acid (Compound pc64, Pen-Hnl(7-F2)-OH) (25.2 mg, 0.096 mmol) in dimethylformamide (DMF) (479 μL) were added 2-bromoacetonitrile (6.67 μL, 0.096 mmol) and N-ethyl-isopropylpropan-2-amine (DIPEA) (25 μL, 0.144 mmol) at 0°C under a nitrogen atmosphere, and the mixture was stirred for 1 h. To the reaction solution was further added 2-bromoacetonitrile (0.667 μL, 0.0096 mmol) at 0°C, and the mixture was stirred for 10 min. To the reaction solution was added a saturated aqueous ammonium chloride solution, and the mixture was extracted with t-butyl methyl ether (TBME). The organic layer was washed with brine, then dried over anhydrous sodium sulfate, filtered, and concentrated under reduced pressure. The resulting crude product was purified by normal phase silica gel chromatography (hexane/ethyl acetate) to afford cyanomethyl (S)-7,7-difluoro-2-(pent-4-enamido)heptanoate (Compound pc65, Pen-Hnl(7-F2)-$OCH_2CN$) (21.2 mg, 73%).
LCMS (ESI) m/z = 303 (M+H)+
Retention time: 0.67 min (analytical condition SQDFA05)

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl (2S)-7,7-difluoro-2-(pent-4-enamido)heptanoate (Compound pc66, Pen-Hnl(7-F2)-pCpA)

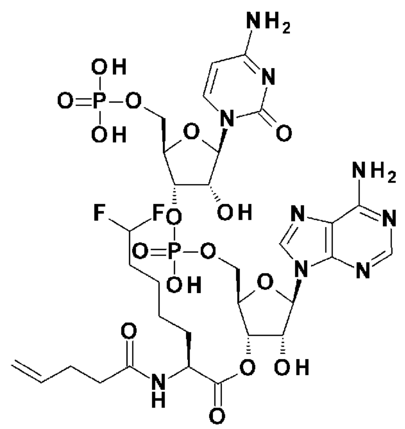

To buffer A (3.6 mL) were added a solution of ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (12.67 mg, 0.18 mmol) in water (0.2 mL) and a solution of the above crude product cyanomethyl (S)-7,7-difluoro-2-(pent-4-enamido)heptanoate (Compound pc65, Pen-Hnl(7-F2)-$OCH_2CN$) (21.2 mg, 0.070 mmol) in acetonitrile (0.1 mL), and the mixture was stirred at room temperature for 2 h. To the reaction solution was added acetic acid (3.6 mL) at room temperature, and the mixture was stirred for 2 h. The reaction solution was diluted with water and purified by reverse phase column chromatography (0.05% aqueous trifluoroacetic acid solution/0.05% trifluoroacetic acid-acetonitrile solution) to afford (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl (2S)-7,7-difluoro-2-(pent-4-enamido)heptanoate (Compound pc66, Pen-Hnl(7-F2)-pCpA) (6.0 mg, 38% over two steps).
LCMS (ESI) m/z = 898 (M+H)+
Retention time: 0.43 min (analytical condition SQDFA05)

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl (2S)-7,7-difluoro-2-(N-methylpent-4-enamido)heptanoate (Compound pc70, Pen-MeHnl(7-F2)-pCpA)

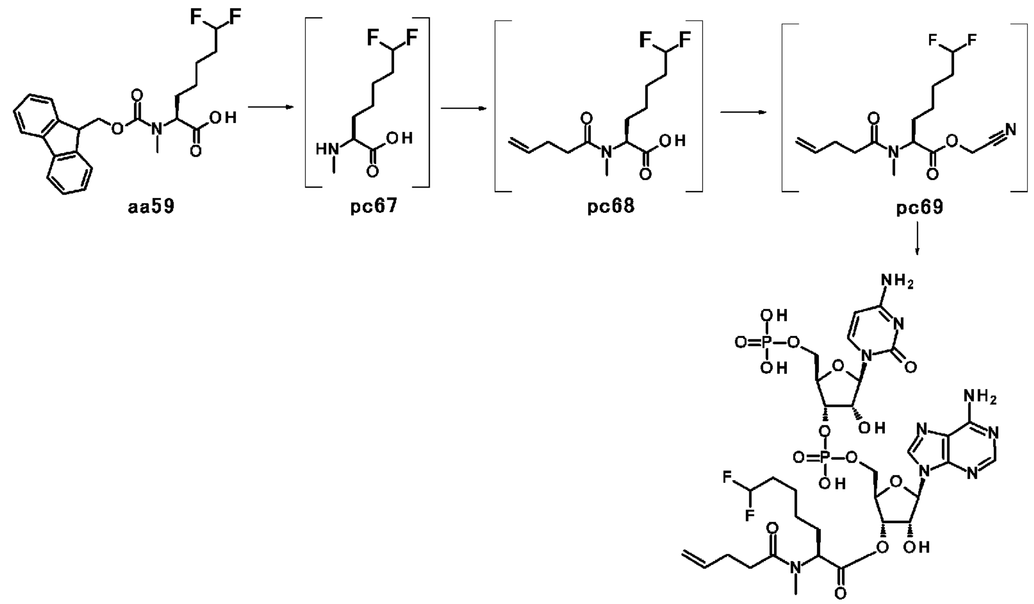

aa59 pc67 pc68 pc69

pc70

To a solution of (S)-2-((((9H-fluoren-9-yl)methoxy)carbonyl)(methyl)amino)-7,7-difluoroheptanoic acid (Compound aa59, Fmoc-MeHnl(7-F2)-OH) (1.17 g, 2.80 mmol) in N,N-dimethylformamide (16 mL) was added piperidine (4

mL, 2.272 mmol) at room temperature under a nitrogen atmosphere, and the mixture was stirred for 16 h. To the reaction solution was added diethyl ether (100 mL), and the mixture was stirred for 30 min. The reaction solution was filtered to afford (2S)-7,7-difluoro-2-(methylamino)heptanoic acid (Compound pc67, H-MeHnl(7-F2)-OH) (441 mg, 81%) as a white solid.

A solution of (2S)-7,7-difluoro-2-(methylamino)heptanoic acid (Compound pc67, H-MeHnl(7-F2)-OH) (412 mg, 2.11 mmol), 2,5-dioxopyrrolidin-1-yl penta-4-enoate (1.24 g, 6.29 mmol), and sodium carbonate (706 mg, 8.40 mmol) in water/1,4-dioxane (1/1, 20 mL) was stirred at room temperature for 16 h under a nitrogen atmosphere. The reaction solution was washed with diethyl ether twice, and the aqueous layer was adjusted to pH 2 with a 1 N aqueous hydrochloric acid solution. The aqueous layer were extracted with dichloromethane twice, and the resulting organic layers were dried over anhydrous sodium sulfate, then filtered, and concentrated under reduced pressure. The resulting residue was purified by normal phase silica gel chromatography (petroleum ether/ethyl acetate) to afford (2S)-7,7-difluoro-2-(N-methylpent-4-enamido)heptanoic acid (Compound pc68, Pen-MeHnl(7-F2)-OH) (300 mg, 51%).

To a solution of (2S)-7,7-difluoro-2-(N-methylpent-4-enamido)heptanoic acid (Compound pc68, Pen-MeHnl(7-F2)-OH) obtained as described above (1.07 g, 3.86 mmol) in dichloromethane (30 mL) were added N-ethyl-isopropylpropan-2-amine (DIPEA) (996 mg, 7.72 mmol) and 2-bromoacetonitrile (1.84 g, 15.34 mmol) under a nitrogen atmosphere, and the mixture was stirred at room temperature for 16 h. The reaction solution was concentrated and the resulting residue was purified by normal phase silica gel column chromatography (petroleum ether/ethyl acetate) to afford a mixture of cyanomethyl (2S)-7,7-difluoro-2-(N-methylpent-4-enamido)heptanoate (Compound pc69, Pen-MeHnl(7-F2)-$OCH_2CN$) (613 mg, 50%).

To buffer A (25 mL) were added a solution of ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (300 mg, 0.42 mmol) in water (75 mL) and a solution of cyanomethyl (2S)-7,7-difluoro-2-(N-methylpent-4-enamido)heptanoate (Compound pc69, Pen-MeHnl(7-F2)-$OCH_2CN$) obtained by the method described above (528 mg, 1.75 mmol) in acetonitrile (3 mL), and the mixture was stirred at room temperature for 2 h. To the reaction solution was added trifluoroacetic acid (1.6 mL), and the reaction solution was lyophilized. The residue was purified by reverse phase column chromatography (0.05% aqueous trifluoroacetic acid solution/0.05% trifluoroacetic acid-acetonitrile solution) to afford the title compound (Compound pc70, Pen-MeHnl(7-F2)-pCpA) (32 mg, 8% over two steps).
LCMS (ESI) m/z = 912 (M+H)+
Retention time: 0.48 min (analytical condition SQDFA05)

<u>Synthesis of N-(pent-4-enoyl)-O-(2,2,3,3-tetrafluoropropyl)-L-serine (Compound pc71, Pen-Ser(F(4)nPr)-OH)</u>

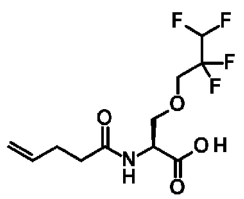

To O-(2,2,3,3-tetrafluoropropyl)-L-serine (H-Ser(F(4)nPr)-OH) already described in Examples (200 mg, 0.913 mmol) and sodium carbonate (193 mg, 1.825 mmol) were added water (2.7 mL) and 1,4-dioxane (1.8 mL) at room temperature under a nitrogen atmosphere and the mixture was stirred, after which pent-4-enoyl chloride (0.202 mL, 1.825 mmol) were added dropwise to the reaction solution at room temperature, and the mixture was stirred for 30 min. To the reaction solution was added a 2 N aqueous hydrochloric acid solution until pH = 2, and the mixture was extracted with ethyl acetate. After washing with brine, the resulting organic layer was concentrated under reduced pressure. The resulting residue was purified by reverse phase column chromatography (0.1% aqueous formic acid solution/0.1% formic acid-acetonitrile solution) to afford N-(pent-4-enoyl)-O-(2,2,3,3-tetrafluoropropyl)-L-serine (Compound pc71, Pen-Ser(F(4)nPr)-OH) (180.9 mg, 66%).
LCMS (ESI) m/z = 302 (M+H)+
Retention time: 0.56 min (analytical condition SQDFA05)

Synthesis of cyanomethyl N-(pent-4-enoyl)-O-(2,2,3,3-tetrafluoropropyl)-L-serinate (Compound pc72, Pen-ser(F(4)nPr)-$OCH_2CN$)

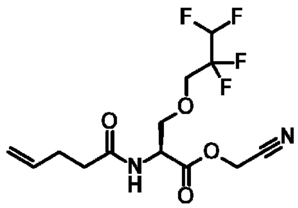

To a solution of N-(pent-4-enoyl)-O-(2,2,3,3-tetrafluoropropyl)-L-serine (Compound pc71, Pen-Ser(F(4)nPr)-OH) (180.9 mg, 0.601 mmol) in dimethylformamide (DMF) (1.5 mL) were added 2-bromoacetonitrile (41.9 μL, 0.601 mmol) and N-ethyl-isopropylpropan-2-amine (DIPEA) (0.157 mL, 0.901 mmol) at 0°C under a nitrogen atmosphere, and the mixture was stirred for 1 h. To the reaction solution were further added 2-bromoacetonitrile (8.38 μL, 0.120 mmol) and N-ethyl-isopropylpropan-2-amine (DIPEA) (0.157 mL, 0.901 mmol) at 0°C, and the mixture was stirred for 1 hour and 30 minutes. To the reaction solution was added a saturated aqueous ammonium chloride solution, and the mixture was extracted with t-butyl methyl ether (TBME). The organic layer was washed with brine, then dried over anhydrous sodium sulfate, filtered, and concentrated under reduced pressure to give a crude product cyanomethyl N-(pent-4-enoyl)-O-(2,2,3,3-tetrafluoropropyl)-L-serinate (Compound pc72, Pen-Ser(F(4)nPr)-$OCH_2CN$) (150 mg).
LCMS (ESI) m/z = 341 (M+H)+
Retention time: 0.67 min (analytical condition SQDFA05)

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl N-(pent-4-enoyl)-O-(2,2,3,3-tetrafluoropropyl)-L-serinate (Compound pc73, Pen-Ser(F(4)nPr)-pCpA)

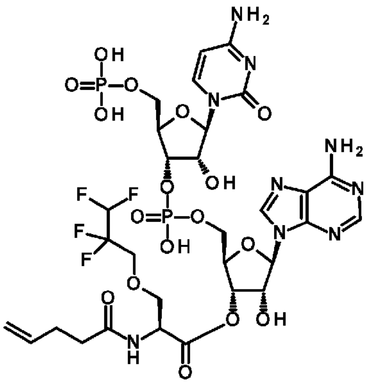

To buffer A (23 mL) were added a solution of ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (80.0 mg, 0.110 mmol) in water (1.2 mL) and a solution of the above crude product cyanomethyl N-(pent-4-enoyl)-O-(2,2,3,3-tetrafluoropropyl)-L-serinate (Compound pc72, Pen-Ser(F(4)nPr)-$OCH_2CN$) (150 mg) in acetonitrile (0.616 mL), and the mixture was stirred at room temperature for 2 h. To the reaction solution was added acetic acid (23.0 mL) at room temperature, and the mixture was stirred for 4 hours and 30 minutes. The reaction solution was diluted with water and purified by reverse phase column chromatography (0.05% aqueous trifluoroacetic acid solution/0.05% trifluoroacetic acid-acetonitrile solution) to afford the title compound (Compound pc73, Pen-Ser(F(4)nPr)-pCpA) (16.3 mg, 16% over two steps).
LCMS (ESI) m/z = 936 (M+H)+
Retention time: 0.43 min (analytical condition SQDFA05)

Synthesis of methyl N-methyl-N-((2-nitrophenyl)sulfonyl)-O-(2,2,3,3-tetrafluoropropyl)-L-serinate (Compound pc74, Ns-MeSer(F(4)nPr)-OMe)

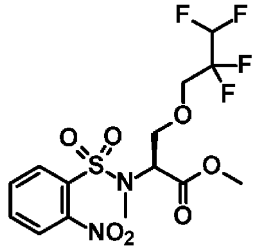

To a solution of methyl N-((2-nitrophenyl)sulfonyl)-O-(2,2,3,3-tetrafluoropropyl)-L-serinate (Compound aa70, Ns-Ser(F(4)nPr)-OMe) already described in Examples (1.0 g, 2.391 mmol) and triphenylphosphine ($PPh_3$) (1.25 g, 4.78 mmol) in dichloromethane (DCM) (40 mL) were added dehydrated methanol (MeOH) (0.145 mL, 3.95 mmol) and a 40% diethyl azodicarboxylate (DEAD)-toluene solution (2.17 mL, purchased from Tokyo Chemical Industry) dropwise at 0°C under a nitrogen atmosphere, and the mixture was then stirred at room temperature for 1 h. To the reaction solution was added formic acid (0.917 mL), and concentration under reduced pressure gave a residue which was then diluted with dimethyl sulfoxide (DMSO) and purified by reverse phase column chromatography (0.1% aqueous formic acid solution/0.1% formic acid-acetonitrile solution) to afford methyl N-methyl-N-((2-nitrophenyl)sulfonyl)-O-(2,2,3,3-tetrafluoropropyl)-L-serinate (Compound pc74, Ns-MeSer(F(4)nPr)-OMe) (625 mg, 61%).
LCMS (ESI) m/z = 433 (M+H)+
Retention time: 0.80 min (analytical condition SQDFA05)

Synthesis of N-methyl-N-(pent-4-enoyl)-O-(2,2,3,3-tetrafluoropropyl)-L-serine (Compound pc75, Pen-Me-Ser(F(4)nPr)-OH)

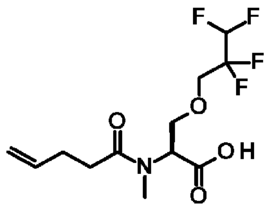

To a solution of methyl N-methyl-N-((2-nitrophenyl)sulfonyl)-O-(2,2,3,3-tetrafluoropropyl)-L-serinate (Compound pc74, Ns-MeSer(F(4)nPr)-OMe) (100 mg, 0.231 mmol) in dichloroethane (DCE) (0.771 mL) was added trimethyltin hydroxide (84 mg, 0.463 mmol) at room temperature under a nitrogen atmosphere, and the mixture was stirred at 60°C for 20 h. The reaction solution was diluted with ethyl acetate, washed with a 1 N aqueous hydrochloric acid solution and brine, then dried over anhydrous magnesium sulfate, filtered, and concentrated under reduced pressure. The resulting residue was purified by reverse phase column chromatography (0.1% aqueous formic acid solution/0.1% formic acid-acetonitrile solution) to afford N-methyl-N-((2-nitrophenyl)sulfonyl)-O-(2,2,3,3-tetrafluoropropyl)-L-serine (Ns-Me-Ser(F(4)nPr)-OH) (88 mg) as a mixture.

To a solution of the above mixture N-methyl-N-((2-nitrophenyl)sulfonyl)-O-(2,2,3,3-tetrafluoropropyl)-L-serine (Ns-MeSer(F(4)nPr)-OH) (44 mg) in acetonitrile (0.5 mL) were added potassium carbonate (72.7 mg, 0.526 mmol) and thiophenol (PhSH) (32.3 μL, 0.316 mmol) at room temperature, and the mixture was stirred for 2 hours and 30 minutes. To the reaction solution were added formic acid (40.3 μL) and t-butyl methyl ether (TBME)/hexane = 1/4, and the mixture was extracted with water. The resulting aqueous layer was purified by reverse phase column chromatography (0.1% aqueous formic acid solution/0.1% formic acid-acetonitrile solution) to afford N-methyl-O-(2,2,3,3-tetrafluoropropyl)-L-serine (H-MeSer(F(4)nPr)-OH) (15 mg, 55% over two steps).

To a solution of the above N-methyl-O-(2,2,3,3-tetrafluoropropyl)-L-serine (H-MeSer(F(4)nPr)-OH) (12 mg, 0.051 mmol) in water (650 μL) was added sodium carbonate (12.6 mg, 0.118 mmol) at room temperature, and the reaction solution was stirred until it became clear, after which tetrahydrofuran (THF) (650 μL) and pent-4-enoyl chloride (11.4 μL, 0.103 mmol) were added dropwise at room temperature and the mixture was stirred for 5 hours and 30 minutes.

To the reaction solution were further added pent-4-enoyl chloride (11.4 μL, 0.103 mmol) and sodium carbonate (12.6 mg, 0.118 mmol) at room temperature, and the mixture was stirred for 13 hours and 30 minutes. To the reaction solution was added formic acid (19.7 μL), and the tetrahydrofuran (THF) was removed under reduced pressure using a rotary evaporator. The resulting aqueous layer was diluted with dimethyl sulfoxide (DMSO) and then purified by reverse phase column chromatography (0.1% aqueous formic acid solution/0.1% formic acid-acetonitrile solution) to afford N-methyl-N-(pent-4-enoyl)-O-(2,2,3,3-tetrafluoropropyl)-L-serine (Compound pc75, Pen-MeSer(F(4)nPr)-OH) (15 mg, 92%).
LCMS (ESI) m/z = 316 (M+H)+
Retention time: 0.63 min (analytical condition SQDFA05)

Synthesis of cyanomethyl N-methyl-N-(pent-4-enoyl)-O-(2,2,3,3-tetrafluoropropyl)-L-serinate (Compound pc76, Pen-MeSer(F(4)nPr)-$OCH_2CN$)

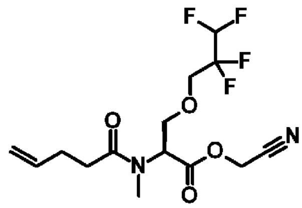

To a solution of N-methyl-N-(pent-4-enoyl)-O-(2,2,3,3-tetrafluoropropyl)-L-serine (Compound pc75, Pen-MeSer(F(4)nPr)-OH) (53 mg, 0.168 mmol) in acetonitrile (0.5 mL) were added N-ethyl-isopropylpropan-2-amine (DIPEA) (88 μL, 0.504 mmol) and 2-bromoacetonitrile (70.3 μL, 1.009 mmol) at room temperature under a nitrogen atmosphere, and the mixture was stirred for 1 hour and 30 minutes. The reaction solution was concentrated under reduced pressure and the resulting residue was purified by normal phase silica gel column chromatography (hexane/ethyl acetate) to afford cyanomethyl N-methyl-N-(pent-4-enoyl)-O-(2,2,3,3-tetrafluoropropyl)-L-serinate (Compound pc76, Pen-MeSer(F(4)nPr)-$OCH_2CN$) (57 mg, 96%).
LCMS (ESI) m/z = 355 (M+H)+
Retention time: 0.74 min (analytical condition SQDFA05)

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl N-methyl-N-(pent-4-enoyl)-O-(2,2,3,3-tetrafluoropropyl)-L-serinate (Compound pc77, Pen-MeSer(F(4)nPr)-pCpA)

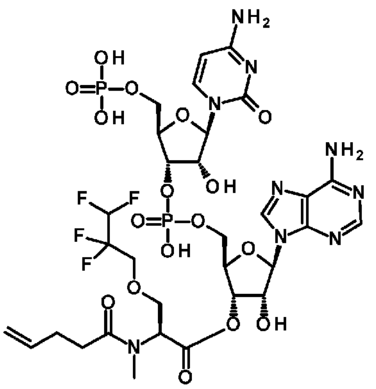

To buffer A (16 mL) were added a solution of ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (30.0 mg, 0.042 mmol) in water (1.2 mL) and a solution of cyanomethyl N-methyl-N-(pent-4-enoyl)-O-(2,2,3,3-tetrafluoropropyl)-L-serinate (Compound pc76, Pen-MeSer(F(4)nPr)-$OCH_2CN$) (29.4 mg, 0.083 mmol) in acetonitrile (0.6 mL), and the mixture

was stirred at room temperature for 2 hours and 30 minutes. To the reaction solution was added trifluoroacetic acid (TFA) (320 μL) at 0°C, and the mixture was stirred for 30 min. The reaction solution was diluted with water and purified by reverse phase column chromatography (0.05% aqueous trifluoroacetic acid solution/0.05% trifluoroacetic acid-acetonitrile solution) to afford the title compound (Compound pc77, Pen-MeSer(F(4)nPr)-pCpA) (10 mg, 25% over two steps).
LCMS (ESI) m/z = 948 (M-H)-
Retention time: 0.47 min (analytical condition SQDFA05)

Synthesis of (2S)-2-amino-6-((tetrahydro-2H-pyran-2-yl)oxy)hexanoic acid (Compound pc78, H-Nle(6-OTHP)-OH)

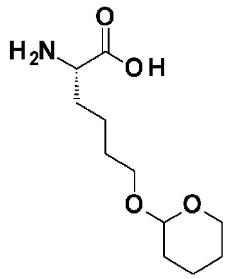

(2S)-2-((((9H-Fluoren-9-yl)methoxy)carbonyl)amino)-6-((tetrahydro-2H-pyran-2-yl)oxy)hexanoic acid (Compound aa107, Fmoc-Nle(6-OTHP)-OH) (150 mg, 0.331 mmol) was dissolved in N,N-dimethylformamide (1.65 mL), after which piperidine (84 mg, 0.992 mmol) was added and the mixture was stirred at room temperature for 3 h. The resulting reaction solution was directly purified by reverse phase column chromatography (10 mM aqueous ammonium acetate solution/methanol) to afford (2S)-2-amino-6-((tetrahydro-2H-pyran-2-yl)oxy)hexanoic acid (Compound pc78, H-Nle(6-OH)-OH) (51 mg, 67%).
LCMS (ESI) m/z = 230 (M-H)-
Retention time: 0.45 min (analytical condition SQDAA05)

Synthesis of (2S)-2-(pent-4-enamido)-6-((tetrahydro-2H-pyran-2-yl)oxy)hexanoic acid (Compound pc79, Pen-Nle(6-OTHP)-OH)

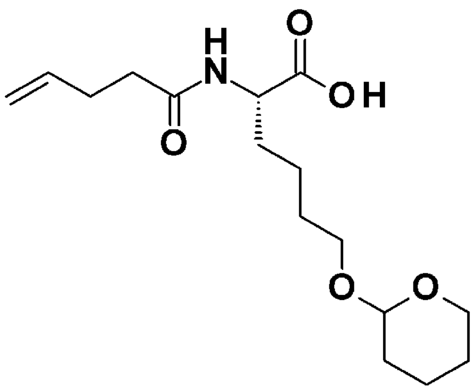

To (2S)-2-amino-6-((tetrahydro-2H-pyran-2-yl)oxy)hexanoic acid (Compound pc78, H-Nle(6-OH)-OH) (51 mg, 0.221 mmol) and sodium carbonate (25.7 mg, 0.243 mmol) were added water (1.1 mL) and tetrahydrofuran (1.1 mL) at room temperature under a nitrogen atmosphere and the mixture was stirred, after which pent-4-enoyl chloride (36.5 μL, 0.331 mmol) was added dropwise to the reaction solution and the mixture was stirred at room temperature for 48 h. To the reaction solution was added a 20% aqueous potassium bisulfate solution, and the mixture was extracted with ethyl acetate. The resulting organic layer was concentrated under reduced pressure to afford (2S)-2-(pent-4-enamido)-6-((tetrahydro-2H-pyran-2-yl)oxy)hexanoic acid (Compound pc79, Pen-Nle(6-OTHP)-OH) (70 mg) as a crude product.
LCMS (ESI) m/z = 312 (M-H)-
Retention time: 0.71 min (analytical condition SQDAA05)

Synthesis of cyanomethyl (2S)-2-(pent-4-enamido)-6-((tetrahydro-2H-pyran-2-yl)oxy)hexanoate (Compound pc80, Pen-Nle(6-OTHP)-$OCH_2CN$)

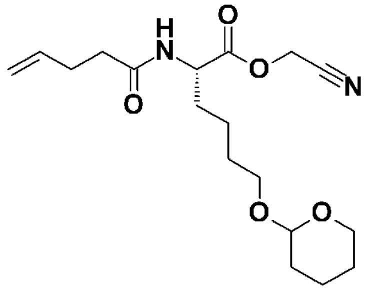

To a solution of (2S)-2-(pent-4-enamido)-6-((tetrahydro-2H-pyran-2-yl)oxy)hexanoic acid (Compound pc79, Pen-Nle(6-OTHP)-OH) (70 mg, 0.223 mmol) in dimethylformamide (DMF) (1.12 mL) were added 2-bromoacetonitrile (31.2 μL, 0.447 mmol) and N-ethyl-isopropylpropan-2-amine (DIPEA) (62.3 μL, 0.447 mmol) at 0°C under a nitrogen atmosphere, and the mixture was stirred for 2 h. To the reaction solution was added water, and extraction with ethyl acetate, drying over anhydrous sodium sulfate, filtration, and concentration under reduced pressure gave a crude product, which was then purified by normal phase silica gel chromatography (hexane/ethyl acetate) to afford cyanomethyl (2S)-2-(pent-4-enamido)-6-((tetrahydro-2H-pyran-2-yl)oxy)hexanoate (Compound pc80, Pen-Nle(6-OTHP)-$OCH_2CN$) (43 mg, 55%).
LCMS (ESI) m/z = 375 (M+Na)+
Retention time: 0.83 min (analytical condition SQDAA05)

Synthesis of (2S,3S,4R,5R)-2-((((((2R,3R,4R,5R)-5-(4-amino-2-oxopyrimidin-1(2H)-yl)-2-((phosphonooxy)methyl)-4-((tetrahydrofuran-2-yl)oxy)tetrahydrofuran-3-yl)oxy)(hydroxy)phosphoryl)oxy)methyl)-5-(6-amino-9H-purin-9-yl)-4-hydroxytetrahydrofuran-3-yl 2-(pent-4-enamido)-6-((tetrahydro-2H-pyran-2-yl)oxy)hexanoate (Compound pc81, Pen-Nle(6-OTHP)-DCpA (THF))

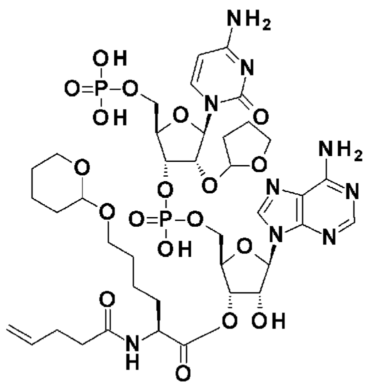

To buffer A (13.88 mL) were added a solution of ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (22.04 mg, 0.122 mmol) in tetrahydrofuran (458 μL) and water (915 μL), and (2S)-cyanomethyl 2-(pent-4-enamido)-6-((tetrahydro-2H-pyran-2-yl)oxy)hexanoate (Compound pc80, Pen-Nle(6-OTHP)-$OCH_2CN$) (43 mg, 0.122 mmol), and the mixture was stirred at room temperature for 2 h. The solvent was evaporated by lyophilization, and the resulting residue was simply purified by reverse phase column chromatography (0.1% aqueous formic acid solution/0.1% formic acid-acetonitrile solution) to afford the title compound (Compound pc81, Pen-Nle(6-OTHP)-pCpA(THF)) (40 mg).
LCMS (ESI) m/z = 1016 (M-H)-
Retention time: 0.61 min (analytical condition SQDFA05)

Synthesis of (2S,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl 6-hydroxy-2-(pent-4-enamido)hexanoate (Compound pc82, Pen-Nle(6-OH)-pCpA)

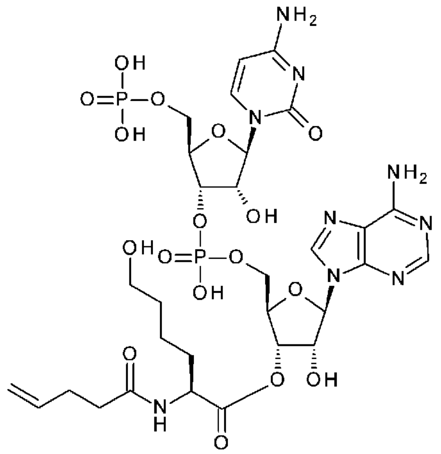

(2S,3S,4R,5R)-2-((((((2R,3R,4R,5R)-5-(4-Amino-2-oxopyrimidin-1(2H)-yl)-2-((phosphonooxy)methyl)-4-((tetrahydrofuran-2-yl)oxy)tetrahydrofuran-3-yl)oxy)(hydroxy)phosphoryl)oxy)methyl)-5-(6-amino-9H-purin-9-yl)-4-hydroxytetrahydrofuran-3-yl 2-(pent-4-enamido)-6-((tetrahydro-2H-pyran-2-yl)oxy)hexanoate (Compound pc81, Pen-Nle(6-OTHP)-pCpA(THF)) (32 mg, 0.031 mmol) was dissolved in a 80% aqueous acetic acid solution (1.05 mL), and the mixture was stirred for 1 h. Trifluoroacetic acid (4.81 μL, 0.063 mmol) was then added and the mixture was stirred for 1 h. The reaction solution was diluted with water and purified by reverse phase column chromatography (0.05% aqueous trifluoroacetic acid solution/0.05% trifluoroacetic acid-acetonitrile solution) to afford the title compound (Compound pc82, Pen-Nle(6-OH)-pCpA) (10.0 mg, 37%).
LCMS (ESI) m/z = 864 (M+H)+
Retention time: 0.33 min (analytical condition SQDFA05)

Synthesis of O-(2-((tetrahydro-2H-pyran-2-yl)oxy)ethyl)-L-serine (Compound pc83, H-Ser(EtOTHP)-OH)

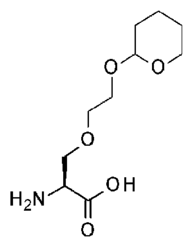

To a solution of N-(((9H-fluoren-9-yl)methoxy)carbonyl)-O-(2-((tetrahydro-2H-pyran-2-yl)oxy)ethyl)-L-serine (Compound aa142, Fmoc-Ser(EtOTHP)-OH) (0.50 g, 1.10 mmol) in N,N-dimethylformamide (6.40 mL) was added piperidine (1.60 mL) under a nitrogen atmosphere, and the mixture was stirred at room temperature for 3 h. To the reaction solution was added diethyl ether/hexane (20.0 mL/20.0 mL), and the precipitated solid was collected to afford O-(2-((tetrahydro-2H-pyran-2-yl)oxy)ethyl)-L-serine (Compound pc83, H-Ser(EtOTHP)-OH) (0.10 g) as a crude product. This was used in the next step without purification.

Synthesis of N-(pent-4-enoyl)-O-(2-((tetrahydro-2H-pyran-2-yl)oxy)ethyl)-L-serine (Compound pc84, Pen-Ser(EtOTHP)-OH)

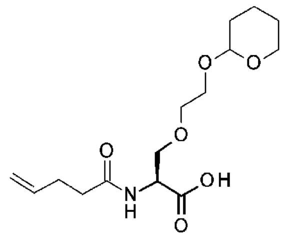

O-(2-((Tetrahydro-2H-pyran-2-yl)oxy)ethyl)-L-serine (Compound pc83, H-Ser(EtOTHP)-OH) (0.20 g, 0.86 mmol) was dissolved in 1,4-dioxane (4.00 mL)/water (4.00 mL) under a nitrogen atmosphere, after which 2,5-dioxopyrrolidin-1-yl pent-4-enoate synthesized by the method described in the document (Organic Letters, 2011, 13, 4906) (0.34 g, 1.86 mmol) and sodium bicarbonate (0.15 g, 1.79 mmol) were added and the mixture was stirred at room temperature for 16 h. After the reaction was completed, the reaction solution was washed with ethyl acetate three times and the aqueous layer was lyophilized to afford N-(pent-4-enoyl)-O-(2-((tetrahydro-2H-pyran-2-yl)oxy)ethyl)-L-serine (Compound pc84, Pen-Ser(EtOTHP)-OH) (0.76 g) as a crude product. This was used in the next step without purification.

Synthesis of cyanomethyl N-(pent-4-enoyl)-O-(2-((tetrahydro-2H-pyran-2-yl)oxy)ethyl)-L-serinate (Compound pc85, Pen-Ser(EtOTHP)-$OCH_2CN$)

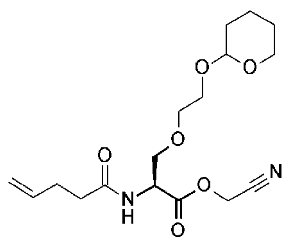

N-(Pent-4-enoyl)-O-(2-((tetrahydro-2H-pyran-2-yl)oxy)ethyl)-L-serine (Compound pc84, Pen-Ser(EtOTHP)-OH) (0.16 g, 0.49 mmol), bromoacetonitrile (0.24 g, 1.97 mmol), and N-ethyl-isopropylpropan-2-amine (DIPEA) (0.13 mg, 0.98 mmol) were dissolved in dichloromethane (10.0 mL)/N,N-dimethylformamide (1.00 mL) under a nitrogen atmosphere, and the mixture was stirred at 25°C for 16 h. The reaction solution was concentrated and the residue was purified by normal phase column chromatography (hexane/ethyl acetate) to afford cyanomethyl N-(pent-4-enoyl)-O-(2-((tetrahydro-2H-pyran-2-yl)oxy)ethyl)-L-serinate (Compound pc85, Pen-Ser(EtOTHP)-$OCH_2CN$) (0.08 g, 16%).

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl O-(2-hydroxyethyl)-N-(pent-4-enoyl)-L-serinate (Compound pc86, Pen-Ser(EtOH)-pCpA)

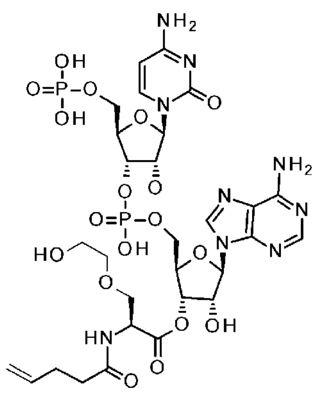

To buffer A (100 mL) were added ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (400 mg, 0.55 mmol) and cyanomethyl N-(pent-4-enoyl)-O-(2-((tetrahydro-2H-pyran-2-yl)oxy)ethyl)-L-serinate (Compound pc85, Pen-Ser(EtOTHP)-$OCH_2CN$) (779 mg, 2.20 mmol), and the mixture was stirred at room temperature for 1 h. To the reaction solution was added trifluoroacetic acid (2.3 mL), and the reaction solution was then purified by reverse phase column chromatography (0.05% aqueous trifluoroacetic acid solution/0.05% trifluoroacetic acid-acetonitrile solution) to afford the title compound (Compound pc86, 20.0 mg, 2%).
LCMS (ESI) m/z = 866 (M+H)+
Retention time: 0.30 min (analytical condition FA05)

Synthesis of N-methyl-O-(2-((tetrahydro-2H-pyran-2-yl)oxy)ethyl)-L-serine (Compound pc87, H-MeSer(EtOTHP)-OH)

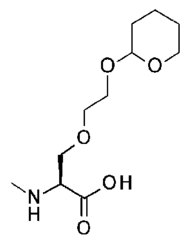

To a solution of N-(((9H-fluoren-9-yl)methoxy)carbonyl)-N-methyl-O-(2-((tetrahydro-2H-pyran-2-yl)oxy)ethyl)-L-serine (Compound aa147, Fmoc-MeSer(EtOTHP)-OH) (10.0 g, 21.3 mmol) in N,N-dimethylformamide (80.0 mL) was added piperidine (30.0 mL) under a nitrogen atmosphere, and the mixture was stirred at room temperature for 3 h. To the reaction solution was added diethyl ether (500 mL), and the precipitated solid was collected to afford N-methyl-O-(2-((tetrahydro-2H-pyran-2-yl)oxy)ethyl)-L-serine (Compound pc87, H-MeSer(EtOTHP)-OH) (3.20 g) as a crude product. This was used in the next step without purification.

Synthesis of N-methyl-N-(pent-4-enoyl)-O-(2-((tetrahydro-2H-pyran-2-yl)oxy)ethyl)-L-serine (Compound pc88, Pen-MeSer(EtOTHP)-OH)

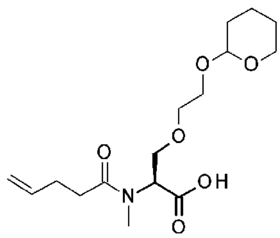

N-Methyl-O-(2-((tetrahydro-2H-pyran-2-yl)oxy)ethyl)-L-serine (Compound pc87, H-MeSer(EtOTHP)-OH) (3.00 g, 12.1 mmol) was dissolved in 1,4-dioxane (60.0 mL)/water (60.0 mL) under a nitrogen atmosphere, after which 2,5-dioxopyrrolidin-1-yl pent-4-enoate synthesized by the method described in the document (Organic Letters, 2011, 13, 4906) (5.98 g, 30.3 mmol) and sodium bicarbonate (2.04 g, 24.3 mmol) were added and the mixture was stirred at room temperature for 16 h. After the reaction was completed, the reaction solution was washed with ethyl acetate three times and the aqueous layer was lyophilized to afford N-methyl-N-(pent-4-enoyl)-O-(2-((tetrahydro-2H-pyran-2-yl)oxy)ethyl)-L-serine (Compound pc88, Pen-MeSer(EtOTHP)-OH) (5.00 g) as a crude product. This was used in the next step without purification.

Synthesis of cyanomethyl N-methyl-N-(pent-4-enoyl)-O-(2-((tetrahydro-2H-pyran-2-yl)oxy)ethyl)-L-serinate (Compound pc89, Pen-MeSer(EtOTHP)-$OCH_2CN$)

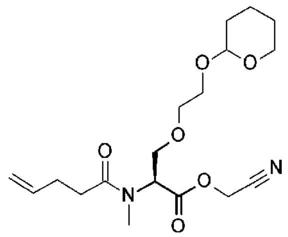

N-Methyl-N-(pent-4-enoyl)-O-(2-((tetrahydro-2H-pyran-2-yl)oxy)ethyl)-L-serine (Compound pc88, Pen-Me-Ser(EtOTHP)-OH) (4.00 g, 12.1 mmol), bromoacetonitrile (5.80 g, 48.4 mmol), and N-ethyl-isopropylpropan-2-amine (DIPEA) (3.10 g, 24.0 mmol) were dissolved in dichloromethane (100 mL)/N,N-dimethylformamide (10.0 mL) under a nitrogen atmosphere, and the mixture was stirred at 25°C for 48 h. The reaction solution was concentrated and the residue was purified by normal phase column chromatography (hexane/ethyl acetate) to afford cyanomethyl N-methyl-N-(pent-4-enoyl)-O-(2-((tetrahydro-2H-pyran-2-yl)oxy)ethyl)-L-serinate (Compound pc89, Pen-Me-Ser(EtOTHP)-$OCH_2CN$) (1.20 g, 27%).

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl O-(2-hydroxyethyl)-N-methyl-N-(pent-4-enoyl)-L-serinate (Compound pc90, Pen-MeSer(EtOH)-pCpA)

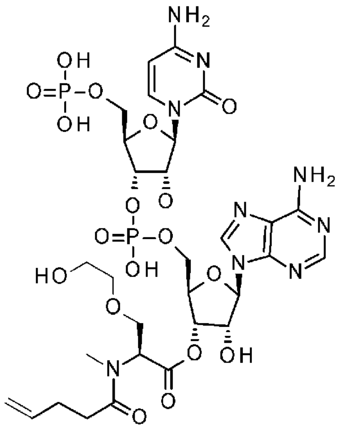

To buffer A (100 mL) were added ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (400 mg, 0.55 mmol) and cyanomethyl N-methyl-N-(pent-4-enoyl)-O-(2-((tetrahydro-2H-pyran-2-yl)oxy)ethyl)-L-serinate (Compound pc89, Pen-Me-Ser(EtOTHP)-$OCH_2CN$) (815 mg, 2.21 mmol), and the mixture was stirred at room temperature for 2 h. To the reaction solution was added trifluoroacetic acid (2.30 mL), and the reaction solution was then purified by reverse phase column chromatography (0.05% aqueous trifluoroacetic acid solution/0.05% trifluoroacetic acid-acetonitrile solution) to afford the title compound (Compound pc90, Pen-MeSer(EtOH)-pCpA) (33.7 mg, 3%).
LCMS (ESI) m/z = 881 (M+H)+
Retention time: 0.35 min (analytical condition FA05)

Synthesis of O-((S)-2-hydroxypropyl)-N-(pent-4-enoyl)-L-serine (Compound pc91, Pen-Ser(S-2-PrOH)-OH)

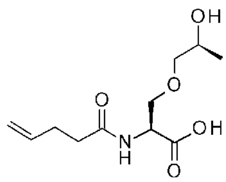

O-((S)-2-Hydroxypropyl)-L-serine (Compound aa152, H-Ser(S-2-PrOH)-OH) (0.12 g, 0.74 mmol) was dissolved in 1,4-dioxane (0.74 mL)/water (0.74 mL) under a nitrogen atmosphere, after which pent-4-enoyl chloride (0.13 g, 1.10 mmol) and N-ethyl-isopropylpropan-2-amine (DIPEA) (0.19 g, 1.47 mmol) were added and the mixture was stirred at room temperature for 30 min. The reaction solution was purified by reverse phase column chromatography (0.1% aqueous formic acid solution/0.1% formic acid-acetonitrile solution) to afford O-((S)-2-hydroxypropyl)-N-(pent-4-enoyl)-L-serine (Compound pc91, Pen-Ser(S-2-PrOH)-OH) (0.12 g, 67%).
LCMS (ESI) m/z = 246 (M+H)+
Retention time: 0.41 min (analytical condition FA05)

Synthesis of cyanomethyl O-((S)-2-hydroxypropyl)-N-(pent-4-enoyl)-L-serinate (Compound pc92, Pen-ser(S-2-PrOH)-$OCH_2CN$)

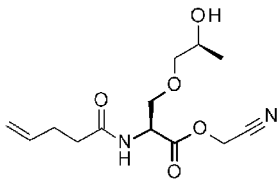

O-((S)-2-Hydroxypropyl)-N-(pent-4-enoyl)-L-serine (Compound pc91, Pen-Ser(S-2-PrOH)-OH) (0.11 g, 0.90 mmol), bromoacetonitrile (0.11 g, 0.90 mmol), and N-ethyl-isopropylpropan-2-amine (DIPEA) (0.17 g, 1.35 mmol) were dissolved in N,N-dimethylformamide (1.00 mL) under a nitrogen atmosphere, and the mixture was stirred at room temperature for 1 h. The reaction solution was purified by reverse phase column chromatography (0.1% aqueous formic acid solution/0.1% formic acid-acetonitrile solution) to afford cyanomethyl O-((S)-2-hydroxypropyl)-N-(pent-4-enoyl)-L-serinate (Compound pc92, Pen-Ser(S-2-PrOH)-$OCH_2CN$) (0.12 g, 96%).
LCMS (ESI) m/z = 285 (M+H)+
Retention time: 0.46 min (analytical condition FA05)

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl O-((S)-2-hydroxypropyl)-N-(pent-4-enoyl)-L-serinate (Compound pc93, Pen-Ser(S-2-PrOH)-pCpA)

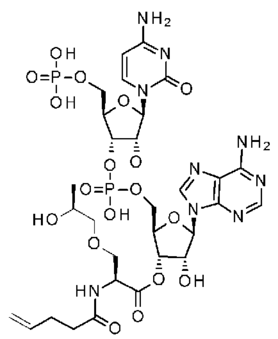

To buffer A (30.0 mL) were added ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (76.0 mg, 0.11 mmol) and cyanomethyl

O-((S)-2-hydroxypropyl)-N-(pent-4-enoyl)-L-serinate (Compound pc92, Pen-Ser(S-2-PrOH)-$OCH_2CN$) (120 mg, 0.44 mmol), and the mixture was stirred at room temperature for 3 hours and 30 minutes. After cooling to 0°C, acetic acid (6.00 mL) was added to the reaction solution, and the mixture was stirred at room temperature for 2 h. To the reaction solution was added water (30.0 mL), and the reaction solution was purified by reverse phase column chromatography (0.05% aqueous trifluoroacetic acid solution/0.05% trifluoroacetic acid-acetonitrile solution) to afford the title compound (Compound pc93, Pen-Ser(S-2-PrOH)-pCpA) (11.0 mg, 12%).
LCMS (ESI) m/z = 880 (M+H)+
Retention time: 0.32 min (analytical condition FA05)

Synthesis of N-methyl-O-((2S)-2-((tetrahydro-2H-pyran-2-yl)oxy)propyl)-L-serine (Compound pc94, H-MeSer(S-2-PrO-THP)-OH)

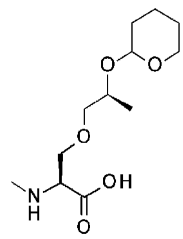

To a solution of N-(((9H-fluoren-9-yl)methoxy)carbonyl)-N-methyl-O-((2S)-2-((tetrahydro-2H-pyran-2-yl)oxy)propyl)-L-serine (Compound aa159, Fmoc-MeSer(S-2-PrOTHP)-OH) (7.00 g, 14.5 mmol) in N,N-dimethylformamide (50.0 mL) was added piperidine (20.0 mL) under a nitrogen atmosphere, and the mixture was stirred at room temperature for 3 h. To the reaction solution was added hexane (1.50 L), and the precipitated solid was collected to afford N-methyl-O-((2S)-2-((tetrahydro-2H-pyran-2-yl)oxy)propyl)-L-serine (Compound pc94, H-MeSer(S-2-PrOTHP)-OH) (2.40 g) as a crude product. This was used in the next step without purification.

Synthesis of N-methyl-N-(pent-4-enoyl)-O-((2S)-2-((tetrahydro-2H-pyran-2-yl)oxy)propyl)-L-serine (Compound pc95, Pen-MeSer(S-2-PrOTHP)-OH)

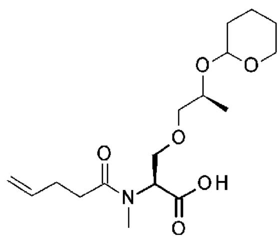

N-Methyl-O-((2S)-2-((tetrahydro-2H-pyran-2-yl)oxy)propyl)-L-serine (Compound pc94, H-MeSer(S-2-PrOTHP)-OH) (2.50 g, 9.57 mmol) was dissolved in 1,4-dioxane (50.0 mL)/water (50.0 mL) under a nitrogen atmosphere, after which 2,5-dioxopyrrolidin-1-yl pent-4-enoate synthesized by the method described in the document (Organic Letters, 2011, 13, 4906) (4.70 g, 23.8 mmol) and sodium bicarbonate (1.60 g, 19.1 mmol) were added and the mixture was stirred at room temperature for 16 h. After the reaction was completed, the reaction solution was washed with ethyl acetate three times and the aqueous layer was lyophilized to afford N-methyl-N-(pent-4-enoyl)-O-((2S)-2-((tetrahydro-2H-pyran-2-yl)oxy)propyl)-L-serine (Compound pc95, Pen-MeSer(S-2-PrOTHP)-OH) (5.10 g) as a crude product. This was used in the next step without purification.

Synthesis of cyanomethyl N-methyl-N-(pent-4-enoyl)-O-((2S)-2-((tetrahydro-2H-pyran-2-yl)oxy)propyl)-L-serinate (Compound pc96, Pen-MeSer(S-2-PrOTHP)-$OCH_2CN$)

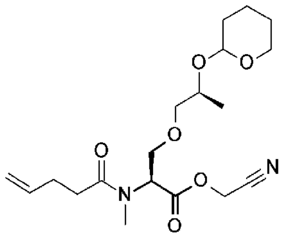

N-Methyl-N-(pent-4-enoyl)-O-((2S)-2-((tetrahydro-2H-pyran-2-yl)oxy)propyl)-L-serine (Compound pc95, Pen-MeSer(S-2-PrOTHP)-OH) (3.10 g, 9.03 mmol), bromoacetonitrile (4.30 g, 35.9 mmol), and N-ethyl-isopropylpropan-2-amine (DIPEA) (2.30 g, 17.8 mmol) were dissolved in dichloromethane (100 mL)/N,N-dimethylformamide (10.0 mL) under a nitrogen atmosphere, and the mixture was stirred at 25°C for 16 h. The reaction solution was concentrated and the residue was purified by normal phase column chromatography (hexane/ethyl acetate) to afford cyanomethyl N-methyl-N-(pent-4-enoyl)-O-((2S)-2-((tetrahydro-2H-pyran-2-yl)oxy)propyl)-L-serinate (Compound pc96, Pen-MeSer(S-2-PrOTHP)-$OCH_2CN$) (1.01 g, 29%).

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl O-((S)-2-hydroxypropyl)-N-methyl-N-(pent-4-enoyl)-L-serinate (Compound pc97, Pen-MeSer(S-2-PrOH)-pCpA)

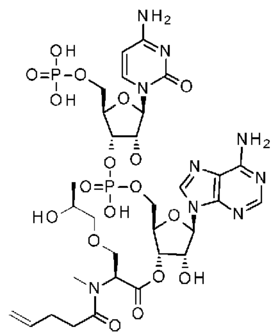

To buffer A (100 mL) were added ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (400 mg, 0.55 mmol) and cyanomethyl N-methyl-N-(pent-4-enoyl)-O-((2S)-2-((tetrahydro-2H-pyran-2-yl)oxy)propyl)-L-serinate (Compound pc96, Pen-MeSer(S-2-PrOTHP)-$OCH_2CN$) (900 mg, 2.21 mmol), and the mixture was stirred at room temperature for 2 h. To the reaction solution was added trifluoroacetic acid (2.3 mL), and the reaction solution was then purified by reverse phase column chromatography (0.05% aqueous trifluoroacetic acid solution/0.05% trifluoroacetic acid-acetonitrile solution) to afford the title compound (Compound pc97, Pen-MeSer(S-2-PrOH)-pCpA) (79.8 mg, 16%). LCMS (ESI) m/z = 894 (M+H)+
Retention time: 0.35 min (analytical condition FA05)

Synthesis of O-((R)-2-hydroxypropyl)-N-(pent-4-enoyl)-L-serine (Compound pc98, Pen-Ser(R-2-PrOH)-OH)

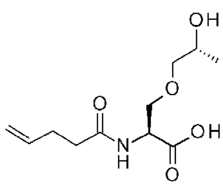

O-((R)-2-Hydroxypropyl)-L-serine (Compound aa164, H-Ser(R-2-PrOH)-OH) (0.18 g, 1.10 mmol) was dissolved

in 1,4-dioxane (0.55 mL)/water (1.66 mL) under a nitrogen atmosphere, after which pent-4-enoyl chloride (0.20 g, 1.66 mmol) and sodium carbonate (0.23 g, 2.21 mmol) were added and the mixture was stirred at room temperature for 30 min. The reaction solution was purified by reverse phase column chromatography (0.1% aqueous formic acid solution/0.1% formic acid-acetonitrile solution) to afford O-((R)-2-hydroxypropyl)-N-(pent-4-enoyl)-L-serine (Compound pc98, Pen-Ser(R-2-PrOH)-OH) (0.11 g, 41%).
LCMS (ESI) m/z = 246 (M+H)+
Retention time: 0.41 min (analytical condition FA05)

Synthesis of cyanomethyl O-((R)-2-hydroxypropyl)-N-(pent-4-enoyl)-L-serinate (Compound pc99, Pen-Ser(R-2-PrOH)-$OCH_2CN$)

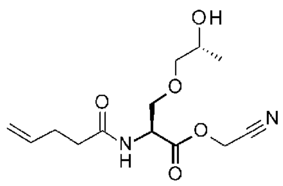

O-((R)-2-Hydroxypropyl)-N-(pent-4-enoyl)-L-serine (Compound pc98, Pen-Ser(R-2-PrOH)-OH) (0.11 g, 0.45 mmol), bromoacetonitrile (0.11 g, 0.90 mmol), and N-ethyl-isopropylpropan-2-amine (DIPEA) (0.17 g, 1.35 mmol) were dissolved in N,N-dimethylformamide (1.00 mL) under a nitrogen atmosphere, and the mixture was stirred at room temperature for 1 h. The reaction solution was purified by reverse phase column chromatography (0.1% aqueous formic acid solution/0.1% formic acid-acetonitrile solution) to afford cyanomethyl O-((R)-2-hydroxypropyl)-N-(pent-4-enoyl)-L-serinate (Compound pc99, Pen-Ser(R-2-PrOH)-$OCH_2CN$) (0.11 g, 88%).
LCMS (ESI) m/z = 285 (M+H)+
Retention time: 0.46 min (analytical condition FA05)

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl O-((R)-2-hydroxypropyl)-N-(pent-4-enoyl)-L-serinate (Compound pc100, Pen-Ser(R-2-PrOH)-pCpA)

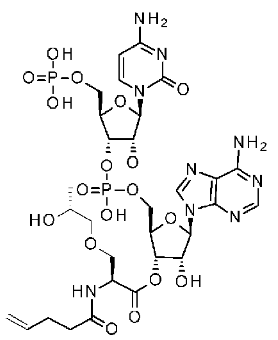

To buffer A (30.0 mL) were added ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (23.0 mg, 0.03 mmol) and cyanomethyl O-((R)-2-hydroxypropyl)-N-(pent-4-enoyl)-L-serinate (Compound pc99, Pen-Ser(R-2-PrOH)-$OCH_2CN$) (36.0 mg, 0.13 mmol), and the mixture was stirred at room temperature for 3 hours and 30 minutes. After cooling to 0°C, acetic acid (6.00 mL) was added to the reaction solution, and the mixture was stirred at room temperature for 2 h. To the reaction solution was added water (30.0 mL), and the reaction solution was purified by reverse phase column chromatography (0.05% aqueous trifluoroacetic acid solution/0.05% trifluoroacetic acid-acetonitrile solution) to afford the title compound (Compound pc100, Pen-Ser(R-2-PrOH)-pCpA) (9.80 mg, 35%).
LCMS (ESI) m/z = 880 (M+H)+
Retention time: 0.33 min (analytical condition FA05)

Synthesis of N-methyl-O-((2R)-2-((tetrahydro-2H-pyran-2-yl)oxy)propyl)-L-serine (Compound pc101, H-MeSer(R-2-PrOTHP)-OH)

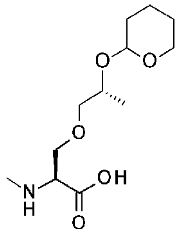

To a solution of N-(((9H-fluoren-9-yl)methoxy)carbonyl)-N-methyl-O-((2R)-2-((tetrahydro-2H-pyran-2-yl)oxy)propyl)-L-serine (Compound aa171, Fmoc-MeSer(R-2-PrOTHP)-OH) (7.00 g, 14.5 mmol) in N,N-dimethylformamide (50.0 mL) was added piperidine (20.0 mL) under a nitrogen atmosphere, and the mixture was stirred at room temperature for 3 h. To the reaction solution was added hexane (1.500 L), and the precipitated solid was collected to afford N-methyl-O-((2R)-2-((tetrahydro-2H-pyran-2-yl)oxy)propyl)-L-serine (Compound pc101, H-MeSer(R-2-PrOTHP)-OH) (2.40 g) as a crude product. This was used in the next step without purification.

Synthesis of N-methyl-N-(pent-4-enoyl)-O-((2R)-2-((tetrahydro-2H-pyran-2-yl)oxy)propyl)-L-serine (Compound pc102, Pen-MeSer(R-2-PrOTHP)-OH)

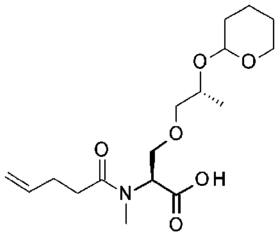

N-Methyl-O-((2R)-2-((tetrahydro-2H-pyran-2-yl)oxy)propyl)-L-serine (Compound pc101, H-MeSer(R-2-PrOTHP)-OH) (2.50 g, 9.57 mmol) was dissolved in 1,4-dioxane (50.0 mL)/water (50.0 mL) under a nitrogen atmosphere, after which 2,5-dioxopyrrolidin-1-yl pent-4-enoate synthesized by the method described in the document (Organic Letters, 2011, 13, 4906) (4.70 g, 23.8 mmol) and sodium bicarbonate (1.60 g, 19.1 mmol) were added and the mixture was stirred at room temperature for 16 h. After the reaction was completed, the reaction solution was washed with ethyl acetate three times and the aqueous layer was lyophilized to afford N-methyl-N-(pent-4-enoyl)-O-((2R)-2-((tetrahydro-2H-pyran-2-yl)oxy)propyl)-L-serine (Compound pc102, Pen-MeSer(R-2-PrOTHP)-OH) (5.10 g) as a crude product. This was used in the next step without purification.

Synthesis of cyanomethyl N-methyl-N-(pent-4-enoyl)-O-((2R)-2-((tetrahydro-2H-pyran-2-yl)oxy)propyl)-L-serinate (Compound pc103, Pen-MeSer(R-2-PrOTHP)-$OCH_2CN$)

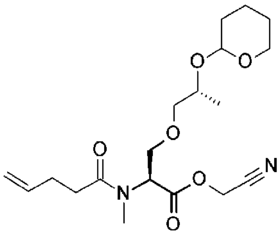

N-Methyl-N-(pent-4-enoyl)-O-((2R)-2-((tetrahydro-2H-pyran-2-yl)oxy)propyl)-L-serine (Compound pc102, Pen-MeSer(R-2-PrOTHP)-OH) (3.10 g, 9.03 mmol), bromoacetonitrile (4.30 g, 35.9 mmol), and N-ethyl-isopropylpropan-2-amine (DIPEA) (2.30 g, 17.8 mmol) were dissolved in dichloromethane (100 mL)/N,N-dimethylformamide (10.0 mL) under a nitrogen atmosphere, and the mixture was stirred at 25°C for 16 h. The reaction solution was concentrated and the residue was purified by normal phase column chromatography (hexane/ethyl acetate) to afford cyanomethyl N-methyl-N-(pent-4-enoyl)-O-((2R)-2-((tetrahydro-2H-pyran-2-yl)oxy)propyl)-L-serinate (Compound pc103, Pen-MeSer(R-2-PrOTHP)-$OCH_2CN$) (1.01 g, 29%).

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl O-((R)-2-hydroxypropyl)-N-methyl-N-(pent-4-enoyl)-L-serinate (Compound pc104, Pen-MeSer(R-2-PrOH)-pCpA)

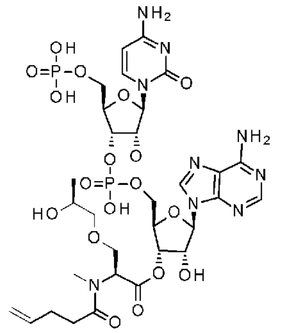

To buffer A (100 mL) were added ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (400 mg, 0.55 mmol) and cyanomethyl N-methyl-N-(pent-4-enoyl)-O-((2R)-2-((tetrahydro-2H-pyran-2-yl)oxy)propyl)-L-serinate (Compound pc103, Pen-MeSer(R-2-PrOTHP)-$OCH_2CN$) (846 mg, 2.21 mmol), and the mixture was stirred at room temperature for 2 h. To the reaction solution was added trifluoroacetic acid (2.30 mL), and the reaction solution was then purified by reverse phase column chromatography (0.05% aqueous trifluoroacetic acid solution/0.05% trifluoroacetic acid-acetonitrile solution) to afford the title compound (Compound pc104, Pen-MeSer(R-2-PrOH)-pCpA) (69.5 mg, 16%).
LCMS (ESI) m/z = 894 (M+H)+
Retention time: 0.35 min (analytical condition FA05)

Compound pc106 was synthesized according to the following scheme.

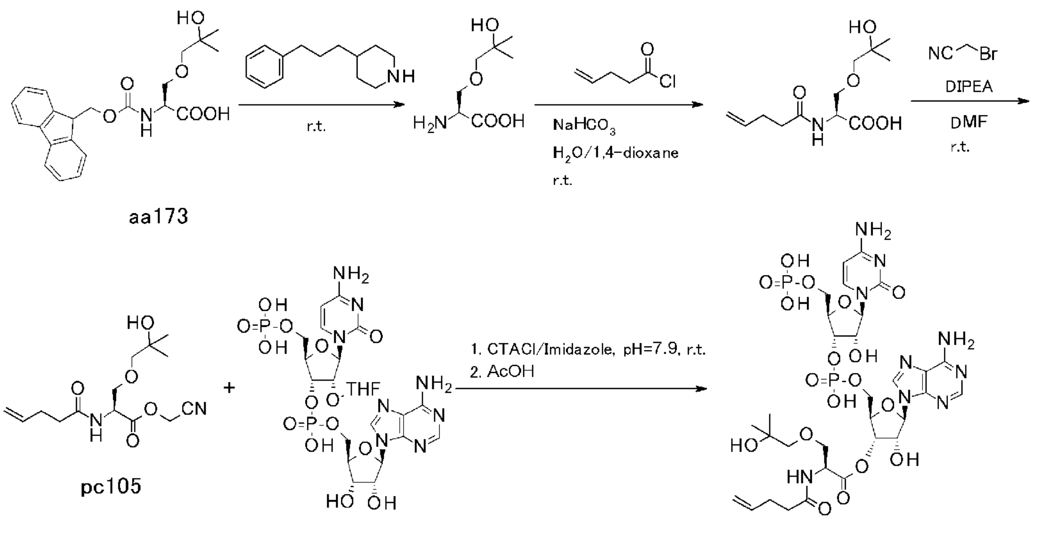


aa173

pc105

pc106

Synthesis of cyanomethyl O-(2-hydroxy-2-methylpropyl)-N-(pent-4-enoyl)-L-serinate (Compound pc105, Pen-Ser(tBuOH)-$OCH_2CN$)

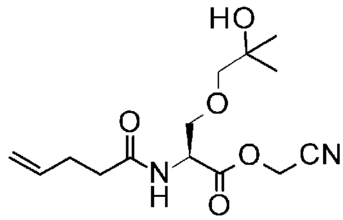

N-(((9H-Fluoren-9-yl)methoxy)carbonyl)-O-(2-hydroxy-2-methylpropyl)-L-serine (Compound aa173, Fmoc-Ser(tBuOH)-OH) (200 mg, 0.501 mmol) was dissolved in dichloromethane (1.0 mL), 4-(3-phenylpropyl)piperidine (318 μL, 1.502 mmol) was added, and the mixture was stirred at room temperature for 1 min. To the reaction solution was then added water (1 mL), and the mixture was stirred at room temperature for 1 h. The aqueous layer was then purified by reverse phase column chromatography (water/acetonitrile = 95/5) to afford O-(2-hydroxy-2-methylpropyl)-L-serine (H-Ser(tBuOH)-OH) (72 mg, 81%).

The obtained O-(2-hydroxy-2-methylpropyl)-L-serine (H-Ser(tBuOH)-OH) (69 mg, 0.389 mmol) and sodium bicarbonate (124 mg, 1.168 mmol) were dissolved in 1,4-dioxane/water (260 μL/519 μL), pent-4-enoyl chloride (86 μL, 0.779 mmol) was added, and the mixture was stirred at room temperature for 30 min. To the aqueous layer was then added an aqueous hydrochloric acid solution (2 M) to adjust it to pH 2, and the aqueous layer was extracted with ethyl acetate. The organic layer was washed with brine, then dried over anhydrous sodium sulfate, filtered, and then concentrated under reduced pressure to afford O-(2-hydroxy-2-methylpropyl)-N-(pent-4-enoyl)-L-serine (Pen-Ser(tBuOH)-OH) as a crude product.

The resulting crude product O-(2-hydroxy-2-methylpropyl)-N-(pent-4-enoyl)-L-serine (Pen-Ser(tBuOH)-OH) and N,N-diisopropylethylamine (DIPEA) (151 mg, 1.167 mmol) were dissolved in dimethylformamide (DMF) (973 μL), 2-bromoacetonitrile (52.4 μL, 0.778 mmol) was added, and the mixture was stirred at room temperature for 1 h. Ethyl acetate was then added to the reaction solution, which was then washed with water three times. The organic layer was dried over anhydrous sodium sulfate and then filtered, the solvent was removed by concentration under reduced pressure, and the resulting residue was purified by reverse phase column chromatography (water/acetonitrile) to afford cyanomethyl O-(2-hydroxy-2-methylpropyl)-N-(pent-4-enoyl)-L-serinate (Compound pc105, Pen-Ser(tBuOH)-$OCH_2CN$) (36 mg, 31%).
LCMS (ESI) m/z = 299 (M+H)+
Retention time: 0.51 min (analytical condition SQDFA05)

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hvdroxytetrahydrofuran-3-yl O-(2-hydroxy-2-methylpropyl)-N-(pent-4-enoyl)-L-serinate (Compound pc106, Pen-Ser(tBuOH)-pCpA)

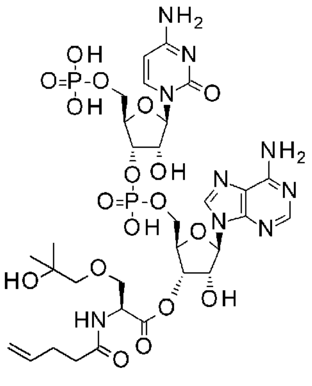

((2R,3R,4R,5R)-5-(4-Amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (21.8 mg, 0.030 mmol) was dissolved in water (0.4 mL), and this was added to buffer A (6 mL). To this aqueous solution was added a solution of cyanomethyl O-(2-hydroxy-2-methylpropyl)-N-(pent-4-enoyl)-L-serinate (Compound pc105, Pen-Ser(tBuOH)-$OCH_2CN$) (36 mg, 0.121 mmol) in acetonitrile (0.2 mL) dropwise, and the mixture was stirred at room temperature for 3 h. To the reaction solution was then added acetic acid (6 mL), and this reaction solution was stirred at room temperature for 3 h. Water (30 mL) was then added, and the mixture was purified by reverse phase column chromatography (0.05% aqueous trifluoroacetic acid solution/0.05% trifluoroacetic acid-acetonitrile solution) to afford the title compound (Compound pc106, Pen-Ser(tBuOH)-pCpA) (10.1 mg, 38%).
LCMS (ESI) m/z = 894 (M+H)+
Retention time: 0.35 min (analytical condition SQDFA05)

Compound pc109 (Pen-MeSer(tBuOH)-pCpA) was synthesized according to the following scheme.

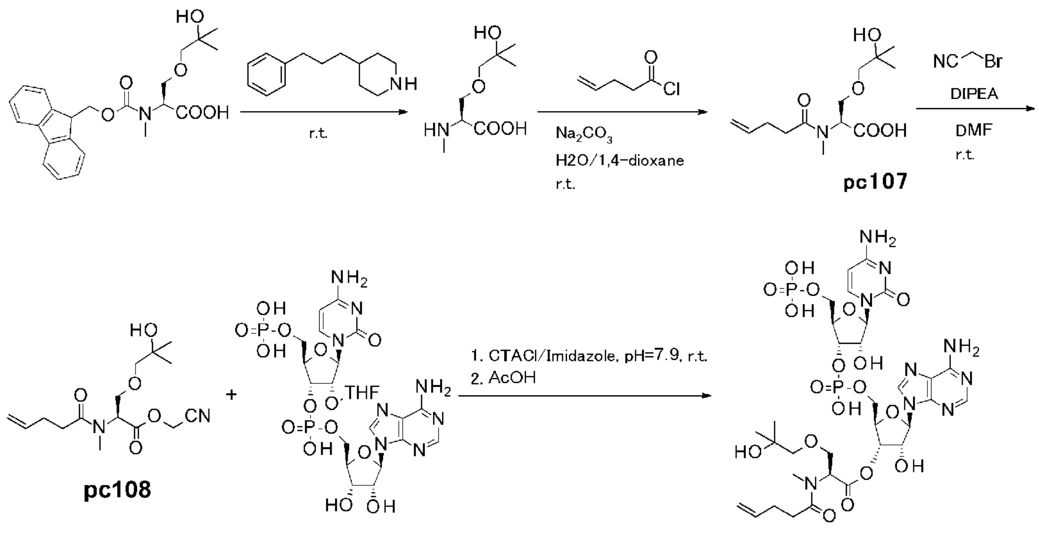

pc107

pc108

pc109

Synthesis of O-(2-hydroxy-2-methylpropyl)-N-methyl-N-(pent-4-enoyl)-L-serine (Compound pc107, Pen-MeSer(tBuOH)-OH)

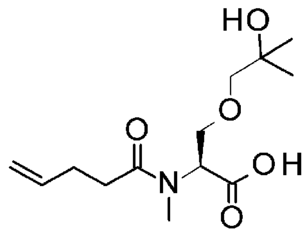

N-(((9H-Fluoren-9-yl)methoxy)carbonyl)-O-(2-hydroxy-2-methylpropyl)-N-methyl-L-serine (Fmoc-MeSer(tBuOH)-OH) (416 mg, 1.006 mmol) which is an intermediate resulting from the process of synthesizing N-(((9H-fluoren-9-yl)methoxy)carbonyl)-N-methyl-O-(2-methyl-2-((tetrahydro-2H-pyran-2-yl)oxy)propyl)-L-serine (Compound aa175, Fmoc-MeSer(tBuOTHP)-OH) was dissolved in dichloromethane (5.0 mL), 4-(3-phenylpropyl)piperidine (426 μL, 2.012 mmol) was added, and the mixture was stirred at room temperature for 9 h. The reaction solution was then diluted with dichloromethane and extracted with water. The aqueous layer was washed with dichloromethane and then purified by reverse phase column chromatography (0.1% aqueous formic acid solution/0.1% formic acid-acetonitrile solution) to afford O-(2-hydroxy-2-methylpropyl)-N-methyl-L-serine (H-MeSer(tBuOH)-OH) (137 mg, 71%).

The obtained O-(2-hydroxy-2-methylpropyl)-N-methyl-L-serine (H-MeSer(tBuOH)-OH) (110 mg, 0.575 mmol) was dissolved in water (2.0 mL), sodium carbonate (140 mg, 1.323 mmol) was added and stirred until it dissolved. A solution of pent-4-enoyl chloride (127 μL, 1.150 mmol) in tetrahydrofuran (2.0 mL) was then added and the mixture was stirred at room temperature for 260 min. Pent-4-enoyl chloride (63.5 μL, 0.575 mmol) and sodium carbonate (73 mg,

0.690 mmol) were then further added and the mixture was stirred at room temperature for 2 h. Pent-4-enoyl chloride (63.5 μL, 0.575 mmol) and sodium carbonate (73 mg, 0.690 mmol) were further added and the mixture was stirred at room temperature overnight. To the reaction solution was then added formic acid (0.221 mL, 5.75 mmol), and the tetrahydrofuran was removed by concentration under reduced pressure. The resulting residue was diluted with dimethyl sulfoxide and purified by reverse phase column chromatography (0.1% aqueous formic acid solution/0.1% formic acid-acetonitrile solution) to afford O-(2-hydroxy-2-methylpropyl)-N-methyl-N-(pent-4-enoyl)-L-serine (Compound pc107, Pen-MeSer(tBuOH)-OH) (144 mg, 78%).
LCMS (ESI) m/z = 274 (M+H)+
Retention time: 0.46 min (analytical condition SQDFA05)

Synthesis of cyanomethyl O-(2-hydroxy-2-methylpropyl)-N-methyl-N-(pent-4-enoyl)-L-serinate (Compound pc108, Pen-Set(tBuOH)-$OCH_2CN$)

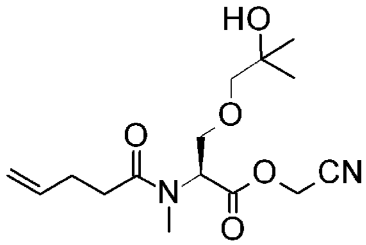

O-(2-Hydroxy-2-methylpropyl)-N-methyl-N-(pent-4-enoyl)-L-serine (Compound pc107, Pen-Me-Ser(tBuOH)-OH) (120 mg, 0.439 mmol) was dissolved in acetonitrile (1 mL), N,N-diisopropylethylamine (DIPEA) (230 μL, 1.317 mmol) and 2-bromoacetonitrile (183 μL, 2.63 mmol) were added, and the mixture was stirred at room temperature for 110 min. 2-Bromoacetonitrile (91.5 μL, 1.32 mmol) and N,N-diisopropylethylamine (DIPEA) (115 μL, 0.659 mmol) were then further added, the mixture was stirred at room temperature for 30 min, and the reaction solution was then concentrated under reduced pressure. The residue obtained in this manner and the residue obtained by the same operation using O-(2-hydroxy-2-methylpropyl)-N-methyl-N-(pent-4-enoyl)-L-serine (Compound pc107, Pen-Me-Ser(tBuOH)-OH) (20 mg, 0.073 mmol) were combined and purified by normal phase column chromatography (hexane/ethyl acetate) to afford cyanomethyl O-(2-hydroxy-2-methylpropyl)-N-methyl-N-(pent-4-enoyl)-L-serinate (Compound pc108, Pen-Set(tBuOH)-$OCH_2CN$) (137 mg).
LCMS (ESI) m/z = 313 (M+H)+
Retention time: 0.58 min (analytical condition SQDFA05)

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl O-(2-hydroxy-2-methylpropyl)-N-methyl-N-(pent-4-enoyl)-L-serinate (Compound pc109, Pen-MeSer(tBuOH)-pCpA)

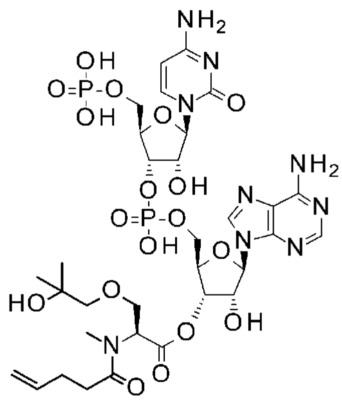

To buffer A (21 mL) were added a solution of ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (46.3 mg, 0.064 mmol) in water (1.4 mL) and a solution of cyanomethyl O-(2-hydroxy-2-methylpropyl)-N-methyl-N-(pent-4-enoyl)-L-serinate (Compound pc108, Pen-Set(tBuOH)-$OCH_2CN$) (80 mg, 0.256 mmol) in acetonitrile (0.7 mL), and the mixture was stirred at room temperature for 2 h. The reaction solution was then lyophilized and the resulting residue was dissolved in a 80% aqueous acetic acid solution (4.0 mL), and the solution was stirred at room temperature for 8 h. The reaction solution was then diluted with water and purified by reverse phase column chromatography (0.05% aqueous trifluoroacetic acid solution/0.05% trifluoroacetic acid-acetonitrile solution) to afford the title compound (Compound pc109, Pen-Me-Ser(tBuOH)-pCpA) (20 mg, 34%).
LCMS (ESI) m/z = 908 (M+H)+
Retention time: 0.38 min (analytical condition SQDFA05)

Synthesis of O-(3-methyl-3-((tetrahydro-2H-pyran-2-yl)oxy)butyl)-L-serine (Compound pc110, H-Ser(2-Me-BuOTHP)-OH)

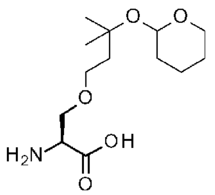

To a solution of N-(((9H-fluoren-9-yl)methoxy)carbonyl)-O-(3-methyl-3-((tetrahydro-2H-pyran-2-yl)oxy)butyl)-L-serine (Compound aa182, Fmoc-Ser(2-Me-BuOTHP)-OH) (9.50 g, 1.10 mmol) in N,N-dimethylformamide (77.0 mL) was added piperidine (22.0 mL) under a nitrogen atmosphere, and the mixture was stirred at room temperature for 3 h. To the reaction solution was added diethyl ether (500 mL), and the precipitated solid was collected to afford O-(3-methyl-3-((tetrahydro-2H-pyran-2-yl)oxy)butyl)-L-serine (Compound pc110, H-Ser(2-Me-BuOTHP)-OH) (2.90 g) as a crude product. This was used in the next step without purification.

Synthesis of O-(3-methyl-3-((tetrahydro-2H-pyran-2-yl)oxy)butyl)-N-(pent-4-enoyl)-L-serine (Compound pc111, Pen-Ser(2-Me-BuOTHP)-OH)

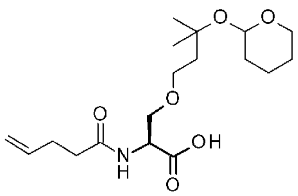

O-(3-methyl-3-((tetrahydro-2H-pyran-2-yl)oxy)butyl)-L-serine (Compound pc110, H-Ser(2-Me-BuOTHP)-OH) (0.24 g, 0.86 mmol) was dissolved in 1,4-dioxane (4.00 mL)/water (4.00 mL) under a nitrogen atmosphere, after which 2,5-dioxopyrrolidin-1-yl pent-4-enoate synthesized by the method described in the document (Organic Letters, 2011, 13, 4906) (0.34 g, 1.72 mmol) and sodium bicarbonate (0.14 g, 1.71 mmol) were added and the mixture was stirred at room temperature for 5 h. After the reaction was completed, the reaction solution was washed with diethyl ether twice, a 1 M aqueous hydrochloric acid solution was added to the aqueous layer until pH 2, and the aqueous layer was extracted with dichloromethane twice. The resulting organic layers were combined, washed with saturated brine, and then dried over anhydrous sodium sulfate, the solvent was evaporated under reduced pressure, and the resulting residue was purified by normal phase column chromatography (hexane/ethyl acetate) to afford O-(3-methyl-3-((tetrahydro-2H-pyran-2-yl)oxy)butyl)-N-(pent-4-enoyl)-L-serine (Compound pc111, Pen-Ser(2-Me-BuOTHP)-OH) (0.30 g, 98%).

Synthesis of cyanomethyl O-(3-methyl-3-((tetrahydro-2H-pyran-2-yl)oxy)butyl)-N-(pent-4-enoyl)-L-serinate (Compound pc112, Pen-Ser(2-Me-BuOTHP)-$OCH_2CN$)

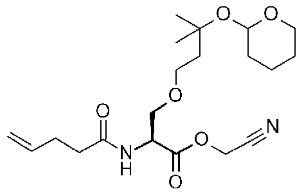

O-(3-Methyl-3-((tetrahydro-2H-pyran-2-yl)oxy)butyl)-N-(pent-4-enoyl)-L-serine (Compound pc111, Pen-Ser(2-Me-BuOTHP)-OH) (3.76 g, 10.5 mmol), bromoacetonitrile (5.06 g, 42.2 mmol), and N-ethyl-isopropylpropan-2-amine (DIPEA) (2.71 g, 21.0 mmol) were dissolved in N,N-dimethylformamide (10.0 mL)/dichloromethane (100 mL) under a nitrogen atmosphere, and the mixture was stirred at room temperature for two days. The reaction solution was concentrated under reduced pressure, and the resulting residue was purified by normal phase column chromatography (hexane/ethyl acetate) to afford cyanomethyl O-(3-methyl-3-((tetrahydro-2H-pyran-2-yl)oxy)butyl)-N-(pent-4-enoyl)-L-serinate (Compound pc112, Pen-Ser(2-Me-BuOTHP)-$OCH_2CN$) (3.00 g, 72%).

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl O-(3-hydroxy-3-methylbutyl)-N-(pent-4-enoyl)-L-serinate (Compound pc113, Pen-Ser(2-Me-BuOH)-pCpA)

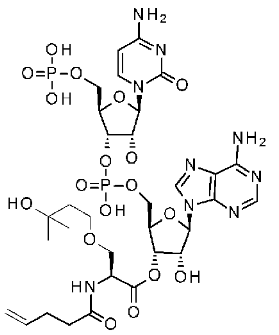

To buffer A (125 mL) were added ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (400 mg, 1.01 mmol) and cyanomethyl O-(3-methyl-3-((tetrahydro-2H-pyran-2-yl)oxy)butyl)-N-(pent-4-enoyl)-L-serinate (Compound pc112, Pen-Ser(2-Me-BuOTHP)-$OCH_2CN$) (877 mg, 1.21 mmol), and the mixture was stirred at room temperature for 2 h. To the reaction solution was added trifluoroacetic acid (2.80 mL), and the mixture was lyophilized. The resulting residue was then purified by reverse phase column chromatography (0.05% aqueous trifluoroacetic acid solution/0.05% trifluoroacetic acid-acetonitrile solution) to afford the title compound (Compound pc113, Pen-Ser(2-Me-BuOH)-pCpA) (54.0 mg, 5%).
LCMS (ESI) m/z = 909 (M+H)+
Retention time: 0.36 min (analytical condition FA05)

Synthesis of O-(3-hydroxy-3-methylbutyl)-N-methyl-L-serine (Compound pc114, H-MeSer(2-Me-BuOH)-OH)

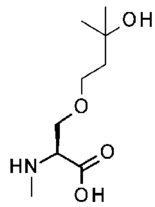

To a solution of N-(((9H-fluoren-9-yl)methoxy)carbonyl)-O-(3-hydroxy-3-methylbutyl)-N-methyl-L-serine (Compound aa184, Fmoc-MeSer(2-Me-BuOH)-OH) (0.15 g, 0.35 mmol) in N,N-dimethylformamide (1.50 mL) was added piperidine (0.37 mL) under a nitrogen atmosphere, and the mixture was stirred at room temperature for 2 h. To the reaction solution was added hexane, and the precipitated solid was collected to afford O-(3-hydroxy-3-methylbutyl)-N-methyl-L-serine (Compound pc114, H-MeSer(2-Me-BuOH)-OH) (0.05 g) as a crude product. This was used in the next step without purification.

Synthesis of O-(3-hydroxy-3-methylbutyl)-N-methyl-N-(pent-4-enoyl)-L-serine (Compound pc115, Pen-MeSer(2-Me-BuOH)-OH)

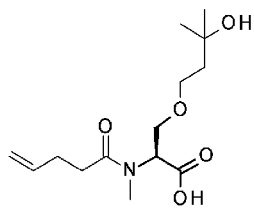

O-(3-Hydroxy-3-methylbutyl)-N-methyl-L-serine (Compound pc114, H-MeSer(2-Me-BuOH)-OH) (0.07 g, 0.34 mmol) was dissolved in 1,4-dioxane (1.00 mL)/water (1.00 mL) under a nitrogen atmosphere, after which 2,5-dioxopyrrolidin-1-yl pent-4-enoate synthesized by the method described in the document (Organic Letters, 2011, 13, 4906) (0.13 g, 0.67 mmol) and sodium bicarbonate (0.06 g, 0.68 mmol) were added and the mixture was stirred at room temperature for 7 h. After the reaction was completed, the reaction solution was washed with diethyl ether three times, a 2 M aqueous hydrochloric acid solution was then added until pH 2, and extraction with t-butyl methyl ether (TBME) was carried out three times. The resulting organic layers were combined, washed with saturated brine, and then dried over anhydrous sodium sulfate, the solvent was evaporated under reduced pressure, and the residue was further dried using a pump to afford O-(3-hydroxy-3-methylbutyl)-N-methyl-N-(pent-4-enoyl)-L-serine (Compound pc115, Pen-MeSer(2-Me-BuOH)-OH) (0.10 g) as a crude product. This was used in the next step without purification.

Synthesis of cyanomethyl O-(3-hydroxy-3-methylbutyl)-N-methyl-N-(pent-4-enoyl)-L-serinate (Compound pc116, Pen-MeSer(2-Me-BuOH)-$OCH_2CN$)

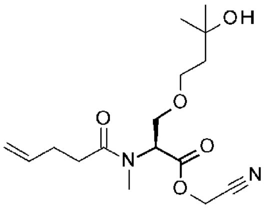

O-(3-Hydroxy-3-methylbutyl)-N-methyl-N-(pent-4-enoyl)-L-serine (Compound pc115, Pen-MeSer(2-Me-BuOH)-OH) (0.50 g, 1.74 mmol), bromoacetonitrile (0.83 g, 6.91 mmol), and N-ethyl-isopropylpropan-2-amine (DIPEA) (0.45 g, 3.48 mmol) were dissolved in dichloromethane (15.0 mL) under a nitrogen atmosphere, and the mixture was stirred at 25°C for 16 h. The reaction solution was concentrated and the residue was purified by normal phase column chromatography (hexane/ethyl acetate) to afford cyanomethyl O-(3-hydroxy-3-methylbutyl)-N-methyl-N-(pent-4-enoyl)-L-serinate (Compound pc116, Pen-MeSer(2-Me-BuOH)-$OCH_2CN$) (0.36 g, 66%).

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl O-(3-hydroxy-3-methylbutyl)-N-methyl-N-(pent-4-enoyl)-L-serinate (Compound pc117, Pen-MeSer(2-Me-BuOH)-pCpA)

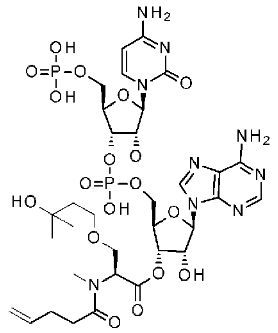

To buffer A (100 mL) were added ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (400 mg, 0.55 mmol) and cyanomethyl O-(3-hydroxy-3-methylbutyl)-N-methyl-N-(pent-4-enoyl)-L-serinate (Compound pc116, Pen-MeSer(2-Me-BuOH)-$OCH_2CN$) (360 mg, 1.10 mmol), and the mixture was stirred at room temperature for 1 h. To the reaction solution was added trifluoroacetic acid (2.80 mL), and the reaction solution was then lyophilized. The resulting residue was purified by reverse phase column chromatography (0.05% aqueous trifluoroacetic acid solution/0.05% trifluoroacetic acid-acetonitrile solution) to afford the title compound (Compound pc117, Pen-MeSer(2-Me-BuOH)-pCpA) (86.0 mg, 8%).
LCMS (ESI) m/z = 923 (M+H)+
Retention time: 0.39 min (analytical condition FA05)

Synthesis of N5,N5-dimethyl-L-glutamine (Compound pc118, H-Gln(Me2)-OH)

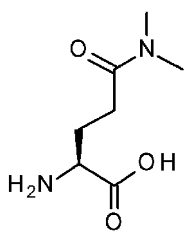

N2-(((9H-Fluoren-9-yl)methoxy)carbonyl)-N5,N5-dimethyl-L-glutamine (Compound aa94, Fmoc-Gln(Me2)-OH) (22.5 g, 56.76 mmol) and piperidine (45 mL) were dissolved in dimethylformamide (DMF) (180 mL), and the mixture was stirred at room temperature for 16 h. To the reaction solution was added diethyl ether, the mixture was stirred for 30 min, and the precipitate was collected by filtration to afford N5,N5-dimethyl-L-glutamine (Compound pc118, H-Gln(Me2)-OH) (5.92 g, 60%).
LCMS (ESI) m/z = 175 (M+H)+
Retention time: 0.35 min (analytical condition SMD method 33)

Synthesis of N5,N5-dimethyl-N2-(pent-4-enoyl)-L-glutamine (Compound pc119, Pen-Gln(Me2)-OH)

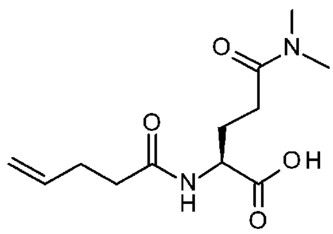

N5,N5-Dimethyl-L-glutamine (Compound pc118, H-Gln(Me2)-OH) (5.92 g, 34.0 mmol) was dissolved in a mixture of water (80 mL) and 1,4-dioxane (80 mL), sodium bicarbonate (0.89 g, 10.6 mmol) and 2,5-dioxopyrrolidin-1-yl pent-4-enoate (1.25 g, 6.34 mmol) were added, and the mixture was stirred at room temperature for 16 h. The reaction solution was washed with ethyl acetate three times, and the aqueous layer was adjusted to pH 2 with an aqueous hydrochloric acid solution (1 mol/L). The aqueous layer was extracted with dichloromethane three times, the organic layers were washed with saturated brine and dried over anhydrous sodium sulfate, and the solvent was then evaporated under reduced pressure. The resulting crude product was purified by normal phase silica gel column chromatography (petroleum ether/ethyl acetate) to afford N5,N5-dimethyl-N2-(pent-4-enoyl)-L-glutamine (Compound pc119, Pen-Gln(Me2)-OH) (3.14 g, 36%).
LCMS (ESI) m/z = 257 (M+H)+
Retention time: 1.03 min (analytical condition SMD method 33)

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl N5,N5-dimethyl-N2-(pent-4-enoyl)-L-glutaminate (Compound pc120, Pen-Gln(Me2)-pCpA)

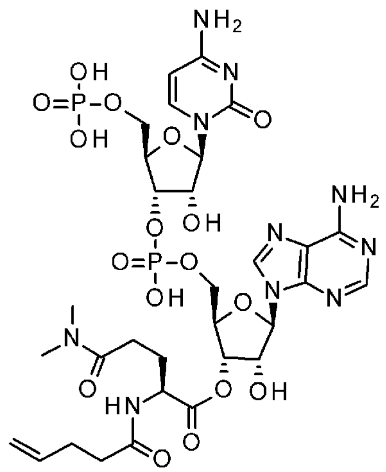

N5,N5-Dimethyl-N2-(pent-4-enoyl)-L-glutamine (Compound pc118, Pen-Gln(Me2)-OH) (3.14 g) and 2-bromoacetonitrile (6.23 g, 58.8 mmol) were dissolved in dichloromethane (DCM) (85 mL) under a nitrogen atmosphere, N-ethyl-isopropylpropan-2-amine (DIPEA) (3.35 g, 25.9 mmol) was added, and the mixture was stirred at room temperature. After 16 hours, the solvent was evaporated from the reaction solution under reduced pressure. The residue was purified by column chromatography (petroleum ether/ethyl acetate) to afford cyanomethyl N5,N5-dimethyl-N2-(pent-4-enoyl)-L-glutaminate (Pent-Gln(Me2)-$OCH_2CN$) (2.63 g, 73%).

To buffer A (75 mL) were added the above cyanomethyl N5,N5-dimethyl-N2-(pent-4-enoyl)-L-glutaminate (Pent-Gln(Me2)-$OCH_2CN$) (490 mg, 1.66 mmol) and ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (300 mg, 0.42 mmol), and the mixture was stirred at room temperature for 5 h. To the reaction solution was added trifluoroacetic acid (TFA) (1.7 mL), the reaction solution was lyophilized, and the resulting crude product was purified by reverse phase column chromatography (0.05% aqueous trifluoroacetic acid solution/0.05% trifluoroacetic acid-acetonitrile solution) to afford the title compound (Compound pc120, Pen-Gln(Me2)-pCpA) (31 mg, 4%).
LCMS (ESI) m/z = 891 (M+H)+

Retention time: 0.33 min (analytical condition SQDFA05)

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl N2,N5,N5-trimethyl-N2-(pent-4-enoyl)-L-glutaminate (Compound pc121, Pen-MeGln(Me2)-pCpA)

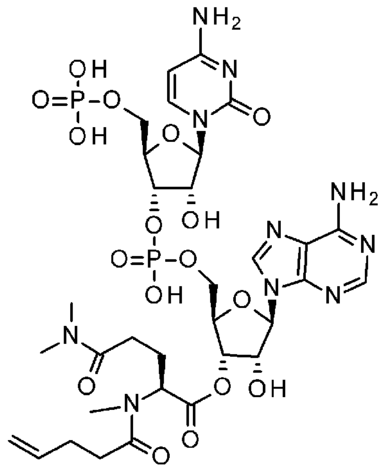

To a solution of N2-(((9H-fluoren-9-yl)methoxy)carbonyl)-N2,N5,N5-trimethyl-L-glutamine (Compound aa96, Fmoc-MeGln(Me2)-OH) (12.0 g, 31.3 mmol) in N,N-dimethylformamide (48.0 mL) was added piperidine (18.0 mL) at room temperature, and the mixture was stirred. After 16 hours, diethyl ether (100 mL) and hexane (100 mL) were added to the reaction solution, and the mixture was stirred for 30 min. The precipitate was collected by filtration to afford N2,N5,N5-trimethyl-L-glutamine (H-MeGln(Me2)-OH) (1.63 g, 32%).

The above N2,N5,N5-trimethyl-L-glutamine (H-MeGln(Me2)-OH) (1.63 g, 8.66 mmol) was dissolved in a mixed solution of water (68 mL) and 1,4-dioxane (68 mL), sodium bicarbonate (1.51 g, 18.0 mmol) and 2,5-dioxopyrrolidin-1-yl pent-4-enoate (2.05 g, 10.4 mmol) were added, and the mixture was stirred at 25°C for 16 h. The reaction solution was washed with diethyl ether three times, and the aqueous layer was adjusted to pH = 4 with an aqueous hydrochloric acid solution (1 mol/L). The aqueous layer was extracted with dichloromethane (100 mL) twice, the organic layers were dried over anhydrous sodium sulfate, and the solvent was evaporated under reduced pressure. The resulting crude product was purified by normal phase silica gel column chromatography (petroleum ether/ethyl acetate) to afford N2,N5,N5-trimethyl-N2-(pent-4-enoyl)-L-glutamine (Pent-MeGln(Me2)-OH) (400 mg, 17%).

After additional synthesis was performed according to the above procedure, the following reaction was carried out.

The resulting N2,N5,N5-trimethyl-N2-(pent-4-enoyl)-L-glutamine (Pent-MeGln(Me2)-OH) (0.76 g, 2.81 mmol), 2-bromoacetonitrile (1.34 g, 11.2 mmol), and N-ethyl-isopropylpropan-2-amine (DIPEA) (727 mg, 5.63 mmol) were dissolved in dichloromethane (DCM) (20 mL), and the mixture was stirred at 25°C. After 16 hours, the solvent was evaporated from the reaction solution under reduced pressure. The residue was purified by normal phase silica gel column chromatography (petroleum ether/ethyl acetate) to afford cyanomethyl N2,N5,N5-trimethyl-N2-(pent-4-enoyl)-L-glutaminate (Pen-MeGln(Me2)-$OCH_2CN$) (520 mg, 60%).

To buffer A (75 mL) were added a solution of the above cyanomethyl N2,N5,N5-trimethyl-N2-(pent-4-enoyl)-L-glutaminate (Pen-MeGln(Me2)-$OCH_2CN$) (433 mg, 1.40 mmol) in acetonitrile (2 mL), and ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (250 mg, 0.35 mmol), and the mixture was stirred at room temperature for 1 h. To the reaction solution was added trifluoroacetic acid (TFA) (1.70 mL), the reaction solution was lyophilized, and the resulting residue was purified by reverse phase column chromatography (0.05% aqueous trifluoroacetic acid solution/0.05% trifluoroacetic acid-acetonitrile solution) to afford the title compound (Compound pc121, Pen-MeGln(Me2)-pCpA) (55 mg).
LCMS (ESI) m/z = 905 (M+H)+
Retention time: 0.35 min (analytical condition SQDFA05)

Synthesis of O-(2-(tert-butylamino)-2-oxoethyl)-L-serine (Compound pc122, H-Ser(NtBu-Aca)-OH)

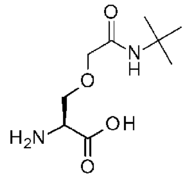

To a solution of N-(((9H-fluoren-9-yl)methoxy)carbonyl)-O-(2-(tert-butylamino)-2-oxoethyl)-L-serine (Compound aa104, Fmoc-Ser(NtBu-Aca)-OH) (10.0 g, 22.7 mmol) in N,N-dimethylformamide (128 mL) was added piperidine (32.0 mL) under a nitrogen atmosphere, and the mixture was stirred at room temperature for 3 h. To the reaction solution was added diethyl ether (500 ml), and the precipitated solid was collected to afford O-(2-(tert-butylamino)-2-oxoethyl)-L-serine (Compound pc122, H-Ser(NtBu-Aca)-OH) (3.10 g) as a crude product. This was used in the next step without purification.

Synthesis of O-(2-(tert-butylamino)-2-oxoethyl)-N-(pent-4-enoyl)-L-serine (Compound pc123, Pen-Ser(NtBu-Aca)-OH)

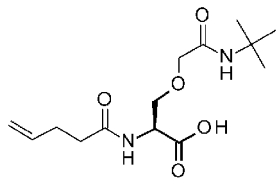

O-(2-(tert-Butylamino)-2-oxoethyl)-L-serine (Compound pc122, H-Ser(NtBu-Aca)-OH) (0.12 g, 0.55 mmol) was dissolved in 1,4-dioxane (2.50 mL)/water (2.50 mL) under a nitrogen atmosphere, after which 2,5-dioxopyrrolidin-1-yl pent-4-enoate synthesized by the method described in the document (Organic Letters, 2011, 13, 4906) (0.22 g, 1.10 mmol) and sodium bicarbonate (0.09 g, 1.10 mmol) were added and the mixture was stirred at room temperature for 16 h. After the reaction was completed, the reaction solution was washed with diethyl ether twice, a 1 M aqueous hydrochloric acid solution was added to the aqueous layer until pH 2, and the aqueous layer was extracted with dichloromethane twice. The resulting organic layers were combined, washed with saturated brine, and then dried over anhydrous sodium sulfate, the solvent was evaporated under reduced pressure, and the resulting residue was purified by normal phase column chromatography (hexane/ethyl acetate) to afford O-(2-(tert-butylamino)-2-oxoethyl)-N-(pent-4-enoyl)-L-serine (Compound pc123, Pen-Ser(NtBu-Aca)-OH) (0.10 g, 61%).

Synthesis of cyanomethyl O-(2-(tert-butylamino)-2-oxoethyl)-N-(pent-4-enoyl)-L-serinate (Compound pc124, Pen-Ser(NtBu-Aca)-$OCH_2CN$)

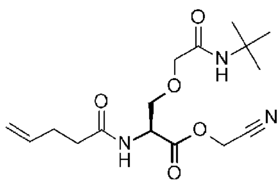

O-(2-(tert-Butylamino)-2-oxoethyl)-N-(pent-4-enoyl)-L-serine (Compound pc123, Pen-Ser(NtBu-Aca)-OH) (3.10 g, 10.3 mmol), bromoacetonitrile (4.97 g, 41.8 mmol), and N-ethyl-isopropylpropan-2-amine (DIPEA) (2.67 g, 20.6 mmol) were dissolved in dichloromethane (60.0 mL) under a nitrogen atmosphere, and the mixture was stirred at 25°C for 16 h. The reaction solution was concentrated under reduced pressure, and the resulting residue was purified by normal phase column chromatography (hexane/ethyl acetate) to afford cyanomethyl O-(2-(tert-butylamino)-2-oxoethyl)-N-(pent-4-enoyl)-L-serinate (Compound pc124, Pen-Ser(NtBu-Aca)-$OCH_2CN$) (1.80 g, 51%).

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl O-(2-(tert-butylamino)-2-oxoethyl)-N-(pent-4-enoyl)-L-serinate (Compound pc125, Pen-Ser(NtBu-Aca)-pCpA)

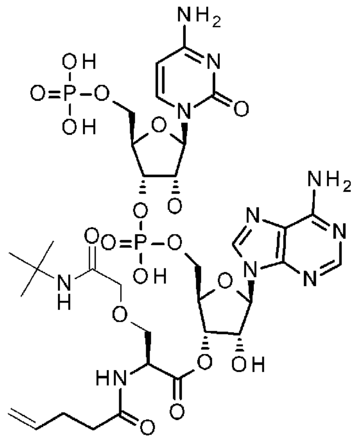

To buffer A (125 mL) were added ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (500 mg, 0.69 mmol) and cyanomethyl O-(2-(tert-butylamino)-2-oxoethyl)-N-(pent-4-enoyl)-L-serinate (Compound pc124, Pen-Ser(NtBu-Aca)-$OCH_2CN$) (939 mg, 2.77 mmol), and the mixture was stirred at room temperature for 1 h. To the reaction solution was added trifluoroacetic acid (2.80 mL), and the mixture was lyophilized. The resulting residue was then purified by reverse phase column chromatography (0.05% aqueous trifluoroacetic acid solution/0.05% trifluoroacetic acid-acetonitrile solution) to afford the title compound (Compound pc125, Pen-Ser(NtBu-Aca)-pCpA) (48.0 mg, 7%).
LCMS (ESI) m/z = 935 (M+H)+
Retention time: 0.40 min (analytical condition FA05)

Synthesis of O-(2-(tert-butylamino)-2-oxoethyl)-N-methyl-L-serine (Compound pc126, H-MeSer(NtBu-Aca)-OH)

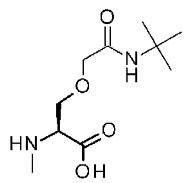

To a solution of N-(((9H-fluoren-9-yl)methoxy)carbonyl)-O-(2-(tert-butylamino)-2-oxoethyl)-N-methyl-L-serine (Compound aa105, Fmoc-MeSer(NtBu-Aca)-OH) (13.6 g, 29.9 mmol) in N,N-dimethylformamide (150 mL) was added piperidine (44.0 mL) under a nitrogen atmosphere, and the mixture was stirred at room temperature for 3 h. To the reaction solution was added hexane (500 mL)/diethyl ether (500 mL), the mixture was stirred at room temperature for 30 min, and the precipitated solid was collected to afford O-(2-(tert-butylamino)-2-oxoethyl)-N-methyl-L-serine (Compound pc126, H-MeSer(NtBu-Aca)-OH) (4.10 g) as a crude product. This was used in the next step without purification.

Synthesis of O-(2-(tert-butylamino)-2-oxoethyl)-N-methyl-N-(pent-4-enoyl)-L-serine (Compound pc127, Pen-MeSer(NtBu-Aca)-OH)

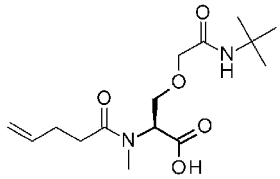

O-(2-(tert-Butylamino)-2-oxoethyl)-N-methyl-L-serine (Compound pc126, H-MeSer(NtBu-Aca)-OH) (4.50 g, 19.4 mmol) was dissolved in 1,4-dioxane (90.0 mL)/water (90.0 mL) under a nitrogen atmosphere, after which 2,5-dioxopyrrolidin-1-yl pent-4-enoate synthesized by the method described in the document (Organic Letters, 2011, 13, 4906) (9.50 g, 48.2 mmol) and sodium bicarbonate (3.30 g, 39.3 mmol) were added and the mixture was stirred at 25°C for 16 h. After the reaction was completed, the reaction solution was washed with diethyl ether three times, a 1 M aqueous hydrochloric acid solution was then added until pH 2, and the aqueous layer was extracted with dichloromethane twice. The resulting organic layers were combined, washed with saturated brine, and then dried over anhydrous sodium sulfate, the solvent was evaporated under reduced pressure, and the resulting residue was purified by normal phase column chromatography (diethyl ether/ethyl acetate) to afford O-(2-(tert-butylamino)-2-oxoethyl)-N-methyl-N-(pent-4-enoyl)-L-serine (Compound pc127, Pen-MeSer(NtBu-Aca)-OH) (2.50 g, 41%).

Synthesis of cyanomethyl O-(2-(tert-butylamino)-2-oxoethyl)-N-methyl-N-(pent-4-enoyl)-L-serinate (Compound pc128, Pen-MeSer(NtBu-Aca)-$OCH_2CN$)

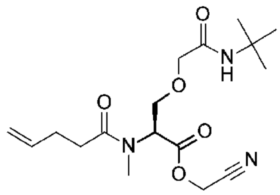

O-(2-(tert-Butylamino)-2-oxoethyl)-N-methyl-N-(pent-4-enoyl)-L-serine (Compound pc127, Pen-MeSer(NtBu-Aca)-OH) (2.30 g, 7.32 mmol), bromoacetonitrile (3.52 g, 29.4 mmol), and N-ethyl-isopropylpropan-2-amine (DIPEA) (1.89 g, 14.6 mmol) were dissolved in dichloromethane (50.0 mL) under a nitrogen atmosphere, and the mixture was stirred at 25°C for 16 h. The reaction solution was concentrated and the residue was purified by normal phase column chromatography (diethyl ether/ethyl acetate) to afford cyanomethyl O-(2-(tert-butylamino)-2-oxoethyl)-N-methyl-N-(pent-4-enoyl)-L-serinate (Compound pc128, Pen-MeSer(NtBu-Aca)-$OCH_2CN$) (1.10 g, 43%).

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(4-amino-7H-pyrrolo[2,3-d]pyrimidin-7-yl)-4-hydroxytetrahydrofuran-3-yl O-(2-(tert-butylamino)-2-oxoethyl)-N-methyl-N-(pent-4-enoyl)-L-serinate (Compound pc129, Pen-MeSer(NtBu-Aca)-pCpA)

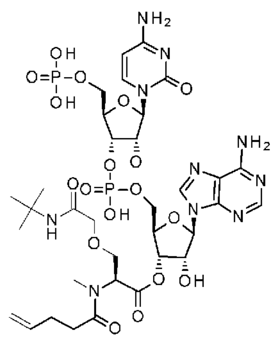

To buffer A (75.0 mL) were added ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (300 mg, 0.42 mmol) and cyanomethyl O-(2-(tert-butylamino)-2-oxoethyl)-N-methyl-N-(pent-4-enoyl)-L-serinate (Compound pc128, Pen-MeSer(NtBu-Aca)-$OCH_2CN$) (586 mg, 1.66 mmol), and the mixture was stirred at room temperature for 2 h. To the reaction solution was added trifluoroacetic acid (1.7 mL), and the reaction solution was then lyophilized. The resulting residue was purified by reverse phase column chromatography (0.05% aqueous trifluoroacetic acid solution/0.05% trifluoroacetic acid-acetonitrile solution) to afford the title compound (Compound pc129, Pen-MeSer(NtBu-Aca)-pCpA) (51.0 mg, 6%).
LCMS (ESI) m/z = 949 (M+H)+
Retention time: 0.43 min (analytical condition FA05)

Synthesis of (S)-2-(pent-4-enamido)-4-phenylbutanoic acid (Compound pc130, Pen-Hph-OH)

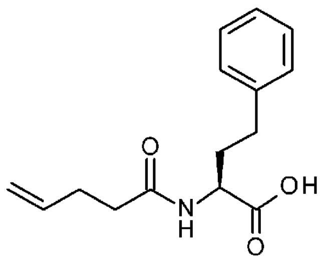

Commercially available (S)-2-amino-4-phenylbutanoic acid (H-Hph-OH, CAS No. 943-73-7) (1.00 g, 5.58 mmol), sodium bicarbonate (0.94 g, 11.2 mmol), and 2,5-dioxopyrrolidin-1-yl pent-4-enoate (1.32 g, 6.69 mmol) were dissolved in a mixed solution of water (20 mL) and 1,4-dioxane (20 mL), and the mixture was stirred at room temperature for 16 h. The reaction solution was washed with ethyl acetate (20 mL) twice, and the aqueous layer was adjusted to pH 2 with an aqueous hydrochloric acid solution (1 mol/L). The aqueous layer was extracted with dichloromethane (60 mL) three times, the organic layers were dried over anhydrous sodium sulfate, and the solvent was evaporated under reduced pressure. The residue was purified by normal phase column chromatography (dichloromethane/methanol) to afford (S)-2-(pent-4-enamido)-4-phenylbutanoic acid (Compound pc130, Pen-Hph-OH) (1.1 g, 75%).
LCMS (ESI) m/z = 262 (M+H)+
Retention time: 1.56 min (analytical condition ELSD1)

Synthesis of cyanomethyl (S)-2-(pent-4-enamido)-4-phenylbutanoate (Compound pc131, Pen-Hph-$OCH_2CN$)

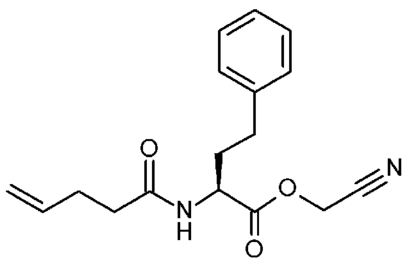

(S)-2-(Pent-4-enamido)-4-phenylbutanoic acid (Compound pc130, Pen-Hph-OH) (1.1 g, 4.21 mmol), 2-bromoacetonitrile (2.00 g, 16.7 mmol), and N-ethyl-isopropylpropan-2-amine (DIPEA) (1.1 g) were dissolved in dichloromethane (DCM) (20 mL) under a nitrogen atmosphere, and the mixture was stirred at 25°C. After 16 hours, the solvent was evaporated from the reaction solution under reduced pressure to afford cyanomethyl (S)-2-(pent-4-enamido)-4-phenylbutanoate (Compound pc131, Pen-Hph-$OCH_2CN$) (0.95 g, 75%).
LCMS (ESI) m/z = 301 (M+H)+
Retention time: 1.86 min (analytical condition ELSD2)

Synthesis of (2R,3S,4R,5R)-2-((((((2R,3S,4R,5R)-5-(4-amino-2-oxopvrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl (2S)-2-(pent-4-enamido)-4-phenylbutanoate (Compound pc132, Pen-Hph-pCpA)

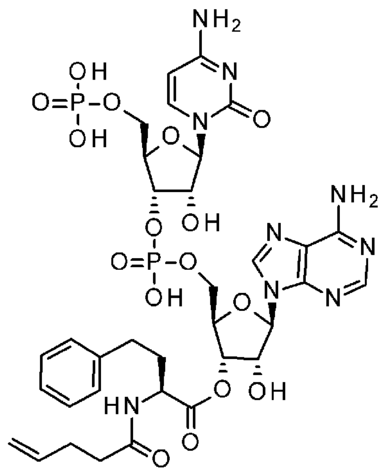

To buffer A (75 mL) were added a solution of ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (300 mg, 0.42 mmol) in water (3 mL) and a solution of cyanomethyl (S)-2-(pent-4-enamido)-4-phenylbutanoate (Compound pc131, Pen-Hph-$OCH_2CN$) (504 mg, 1.68 mmol) in acetonitrile (2.25 mL), and the mixture was stirred at room temperature for 1 h. The reaction solution was lyophilized, a 80% aqueous acetic acid solution (10 mL) was added, and the mixture was stirred for 3 h. The mixture was purified by reverse phase column chromatography (0.05% aqueous trifluoroacetic acid solution/0.05% trifluoroacetic acid-acetonitrile solution) to afford the title compound (Compound pc132, Pen-Hph-pCpA) (37 mg, 10%).
LCMS (ESI) m/z = 894 (M-H)-
Retention time: 0.47 min (analytical condition SQDFA05)

Compound pc135 (Pen-MeHph-pCpA) was synthesized according to the following scheme.

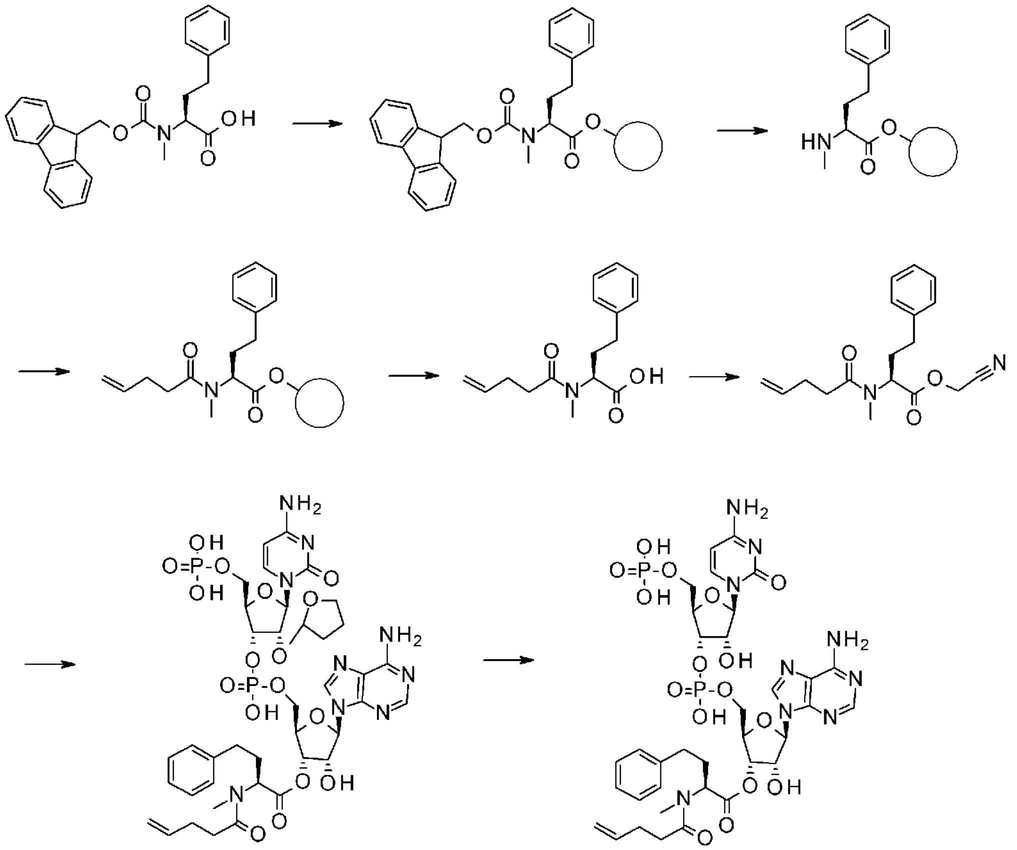

Synthesis of (S)-2-(N-methylpent-4-enamido)-4-phenylbutanoic acid (Compound pc133, Pen-MeHph-OH)

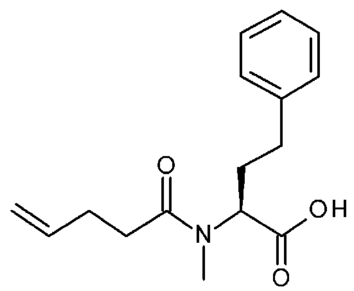

In a reaction vessel with a filter were placed 2-chlorotrityl chloride resin (1.60 mmol/g, 100-200 mesh, 1% DVB, purchased from Watanabe Chemical Industries, 1.68 g, 2.69 mmol) and dehydrated dichloromethane (DCM) (15 mL), and the vessel was shaken at room temperature for 10 min. After removing the dichloromethane (DCM), a solution of (S)-2-((((9H-fluoren-9-yl)methoxy)carbonyl)(methyl)amino)-4-phenylbutanoic acid (Compound aa11, Fmoc-MeHph-OH) (1.12 g, 2.68 mmol) and diisopropylethylamine (DIPEA) (1.40 mL, 8.05 mmol) in dehydrated dichloromethane (DCM) (25 mL) was added to the reaction vessel, and the vessel was shaken for 30 min. To the reaction solution was added dehydrated methanol (2.17 mL, 53.7 mmol), and the vessel was shaken for 1 h. The reaction solution was removed, the resin was then suspended in dichloromethane (DCM), and the solution was removed. This operation was repeated four times. Further, the resin was suspended in N,N-dimethylformamide (DMF), and the solution was removed. This operation was repeated four times. The resulting resin was used as it is in the next reaction.

To the above resin was added N,N-dimethylformamide (DMF) (15.0 mL) containing 2% (v/v) 1,8-diazabicyclo[5.4.0]-7-undecene (DBU), and the vessel was shaken at room temperature for 15 min. The reaction solution was removed, the resin was then suspended in N,N-dimethylformamide (DMF) (20.0 mL), and the solution was removed. This operation was repeated five times. The resulting resin was used as it is in the next reaction.

To the above resin was added a solution of pent-4-enoic acid (1.09 mL, 10.7 mmol), 1-hydroxy-7-azabenzotriazole (HOAt) (1.09 g, 8.04 mmol), and N,N'-diisopropylcarbodiimide (DIC) (2.09 mL, 13.4 mmol) in N,N-dimethylformamide (DMF) (15 mL), and the vessel was shaken at room temperature for 20 h.

The reaction solution was removed, the resin was then suspended in N,N-dimethylformamide (DMF), and the reaction solution was removed. This operation was repeated four times. Further, the resin was suspended in dichloromethane (DCM), and the reaction solution was removed. This operation was repeated four times. The resulting resin was used as it is in the next reaction.

The above resin was suspended in 2,2,2-trifluoroethanol (TFE)/dichloromethane (DCM) (1/1, 50 mL), and the vessel was shaken at room temperature for 3 h. The solvent was evaporated from the filtrate under reduced pressure, and the resulting crude product was purified by reverse phase column chromatography (0.1% aqueous formic acid solution/0.1% formic acid-acetonitrile solution) to afford (S)-2-(N-methylpent-4-enamido)-4-phenylbutanoic acid (Compound pc133, Pen-MeHph-OH) (295 mg, 40%, four steps).
LCMS (ESI) m/z = 276 (M+H)+
Retention time: 0.71 min (analytical condition SQDFA05)

Synthesis of cyanomethyl (S)-2-(N-methylpent-4-enamido)-4-phenylbutanoate (Compound pc134, Pen-MeHph-$OCH_2CN$)

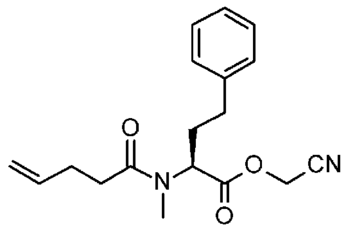

(S)-2-(N-Methylpent-4-enamido)-4-phenylbutanoic acid (Compound pc133, Pen-MeHph-OH) (295 mg, 1.07 mmol) and 2-bromoacetonitrile (257 mg, 2.15 mmol) were dissolved in N,N-dimethylformamide (DMF) (2.68 mL) under a nitrogen atmosphere, N-ethyl-isopropylpropan-2-amine (DIPEA) (416 mg, 3.22 mmol) was added, and the mixture was stirred at room temperature. After 10 minutes, 2-bromoacetonitrile (38.6 mg, 0.322 mmol) was added, and the mixture was stirred for 10 minutes. A saturated aqueous ammonium chloride solution was added to the reaction solution, and the mixture was extracted with t-butyl methyl ether (MTBE). The organic layer was washed with saturated brine and dried over anhydrous sodium sulfate. The solvent was evaporated from the filtrate under reduced pressure to afford cyanomethyl (S)-2-(N-methylpent-4-enamido)-4-phenylbutanoate (Compound pc134, Pen-MeHph-$OCH_2CN$) (440 mg) as a crude product.
LCMS (ESI) m/z = 315 (M+H)+
Retention time: 0.81 min (analytical condition SQDFA05)

Synthesis of (2S)-(2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl 2-(N-methylpent-4-enamido)-4-phenylbutanoate (Compound pc135, Pen-MeHph-pCpA)

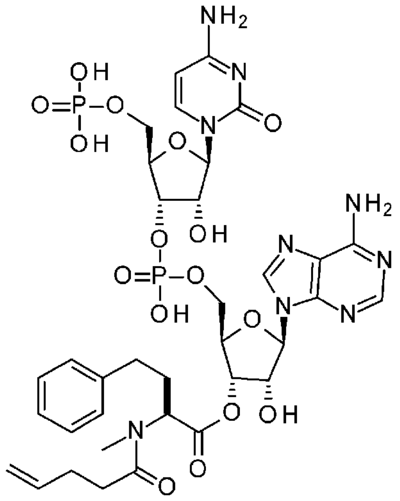

To buffer A (27.6 mL) were added a solution of ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (100 mg, 0.138 mmol) in water (1.50 mL) and a solution of cyanomethyl (S)-2-(N-methylpent-4-enamido)-4-phenylbutanoate (Compound pc134, Pen-MeHph-$OCH_2CN$) (174 mg, 0.554 mmol) in acetonitrile (0.770 mL), and the mixture was stirred at a temperature between 37°C and 40°C for 1 h. To the reaction solution was added acetic acid (30.0 mL), and the mixture was stirred at room temperature for 110 min. The reaction solution was lyophilized, and the resulting crude product was purified by reverse phase column chromatography (0.01% aqueous trifluoroacetic acid solution/0.01% trifluoroacetic acid-acetonitrile solution) to afford the title compound (Compound pc135, Pen-MeHph-pCpA) (48.6 mg, 39%).
LCMS (ESI) m/z = 911 (M+H)+
Retention time: 0.52 min (analytical condition SQDFA05)

Compound pc138 (Pen-Ser(Ph-2-Cl)-pCpA) was synthesized according to the following scheme.

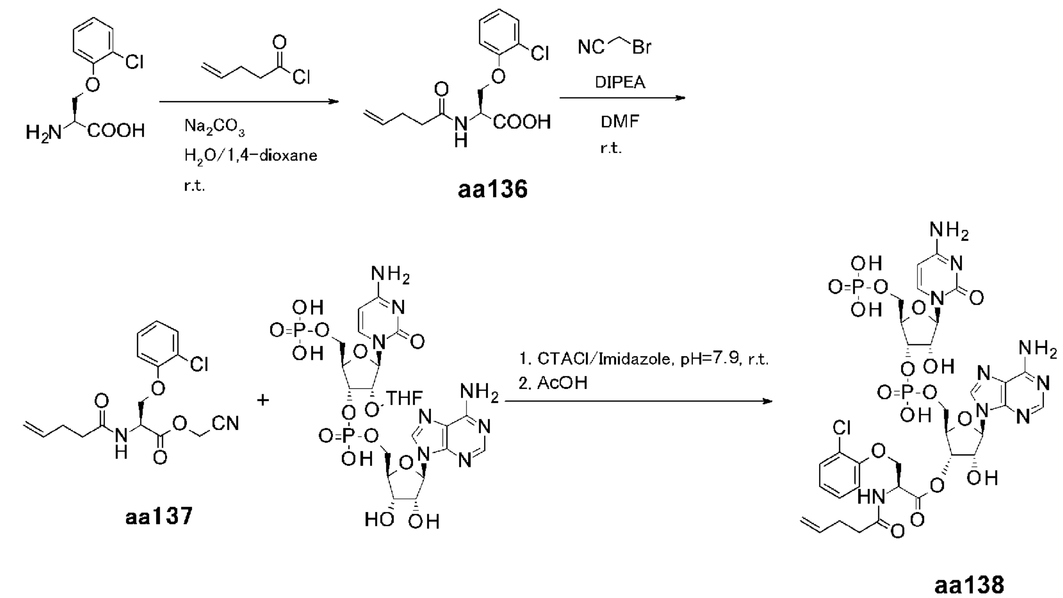


aa136

aa137

aa138

Synthesis of O-(2-chlorophenyl)-N-(pent-4-enoyl)-L-serine (Compound pc136, Pen-Ser(Ph-2-Cl)-OH)

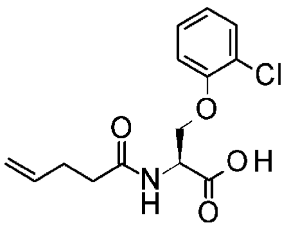

O-(2-Chlorophenyl)-L-serine (Compound aa63, H-Ser(Ph-2-Cl)-OH) (124 mg, 0.575 mmol) and sodium carbonate (76 mg, 0.719 mmol) were dissolved in tetrahydrofuran/water (1.5 mL/1.5 mL), pent-4-enoyl chloride (76 mg, 0.719 mmol) was added, and the mixture was stirred at room temperature for 10 min. Pent-4-enoyl chloride (7.5 mg, 0.0633 mmol) and sodium carbonate (7.6 mg, 0.0719 mmol) were then further added and the mixture was stirred at room temperature. To the reaction solution was then added a 5 N aqueous hydrochloric acid solution (0.322 mL, 1.6 mmol), and the mixture was extracted with ethyl acetate. The ethyl acetate was removed by concentration under reduced pressure to afford O-(2-chlorophenyl)-N-(pent-4-enoyl)-L-serine (Compound pc136, Pen-Ser(Ph-2-Cl)-OH) (148 mg, 86%).
LCMS (ESI) m/z = 298 (M+H)+
Retention time: 0.66 min (analytical condition SQDFA05)

Synthesis of cyanomethyl O-(2-chlorophenyl)-N-(pent-4-enoyl)-L-serinate (Compound pc137, Pen-Ser(Ph-2-Cl)-$OCH_2CN$)

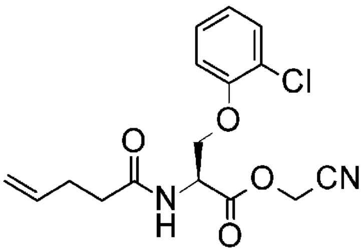

O-(2-Chlorophenyl)-N-(pent-4-enoyl)-L-serine (Compound pc136, Pen-Ser(Ph-2-Cl)-OH) (148 mg, 0.497 mmol) was dissolved in dimethylformamide (5 mL), N,N-diisopropylethylamine (DIPEA) (174 μL, 0.994 mmol) and 2-bromoacetonitrile (132 μL, 1.988 mmol) were added, and the mixture was stirred at room temperature. Water was then added to the reaction solution, and the mixture was extracted with ethyl acetate. The organic layer was dried over anhydrous sodium sulfate, filtered, and then concentrated under reduced pressure. The resulting residue was purified by normal phase column chromatography (hexane/ethyl acetate) to afford cyanomethyl O-(2-chlorophenyl)-N-(pent-4-enoyl)-L-serinate (Compound pc137, Pen-Ser(Ph-2-Cl)-$OCH_2CN$) (100.1 mg, 60%).
LCMS (ESI) m/z = 337 (M+H)+
Retention time: 0.76 min (analytical condition SQDFA05)

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl O-(2-chlorophenyl)-N-(pent-4-enoyl)-L-serinate (Compound pc138, Pen-Ser(Ph-2-Cl)-pCpA)

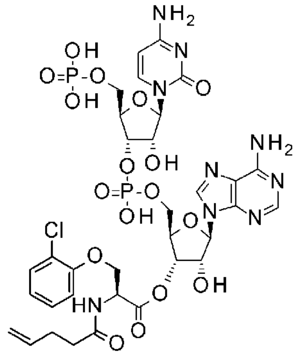

To buffer A (6.23 mL) was added a solution of ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (40.5 mg, 0.056 mmol) in water (1.0 mL), and a solution of cyanomethyl O-(2-chlorophenyl)-N-(pent-4-enoyl)-L-serinate (Compound pc137, Pen-Ser(Ph-2-Cl)-$OCH_2CN$) (75.6 mg, 0.224 mmol) in acetonitrile (0.3 mL) was added, and the mixture was stirred at room temperature for 35 min. To the reaction solution was then added acetic acid (0.257 mL, 4.49 mmol), and the mixture was purified by reverse phase column chromatography (0.1% aqueous formic acid solution/0.1% formic acid-acetonitrile solution). The fractions were lyophilized, after which the resulting solid was dissolved in water/acetic acid (2.4 mL/0.6 mL) and the mixture was stirred at room temperature for 2 h. The reaction solution was then diluted with water and purified by reverse phase column chromatography (0.05% aqueous trifluoroacetic acid solution/0.05% trifluoroacetic acid-acetonitrile solution) to afford the title compound (Compound pc138, Pen-Ser(Ph-2-Cl)-pCpA) (18.7 mg, 36%).
LCMS (ESI) m/z = 932 (M+H)+
Retention time: 0.45 min (analytical condition SQDFA05)

Synthesis of cyanomethyl O-(2-chlorophenyl)-N-methyl-N-(pent-4-enoyl)-L-serinate (Compound pc139, Pen-MeSer(Ph-2-Cl)-$OCH_2CN$)

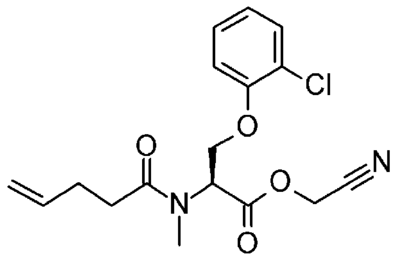

To a solution of N-(((9H-fluoren-9-yl)methoxy)carbonyl)-O-(2-chlorophenyl)-N-methyl-L-serine (Compound aa65, Fmoc-MeSer(Ph-2-Cl)-OH) (15 g, 33.2 mmol) in N,N-dimethylformamide (80 mL) was added piperidine (34 mL) at room temperature, and the mixture was stirred. After 16 hours, diethyl ether (500 mL) and hexane (1000 mL) were added and the precipitate was collected by filtration to afford O-(2-chlorophenyl)-N-methyl-L-serine (H-MeSer(Ph-2-Cl)-OH) (6.5 g, 85%) as a crude product.

The above O-(2-chlorophenyl)-N-methyl-L-serine (H-MeSer(Ph-2-Cl)-OH) was dissolved in a mixture of water (90 mL) and 1,4-dioxane (90 mL), sodium bicarbonate (4.97 g, 59.2 mmol) and 2,5-dioxopyrrolidin-1-yl pent-4-enoate (11.6 g, 58.8 mmol) were added, and the mixture was stirred at room temperature for 16 h. The reaction solution was washed with diethyl ether twice, and the aqueous layer was adjusted to pH = 2 with an aqueous hydrochloric acid solution (1 mol/L). The aqueous layer was extracted with dichloromethane twice, the organic layers were dried over anhydrous sodium sulfate, and the solvent was then evaporated under reduced pressure. The residue was purified by normal phase silica gel column chromatography (petroleum ether/ethyl acetate) to afford O-(2-chlorophenyl)-N-methyl-N-(pent-4-enoyl)-L-serine (Pen-MeSer(Ph-2-Cl)-OH) (7.4 g, 77%).

To a solution of the above compound O-(2-chlorophenyl)-N-methyl-N-(pent-4-enoyl)-L-serine (Pen-MeSer(Ph-2-Cl)-OH) (1.00 g) in dichloromethane (DCM) (30 mL) were added N,N-diisopropylethylamine (DIPEA) (0.830 g, 6.42

mmol) and 2-bromoacetonitrile (1.54 g, 12.8 mmol), and the mixture was stirred at 25°C. After 36 hours, the solvent was evaporated from the reaction solution under reduced pressure. The residue was purified by normal phase silica gel column chromatography (petroleum ether/ethyl acetate) to afford cyanomethyl O-(2-chlorophenyl)-N-methyl-N-(pent-4-enoyl)-L-serinate (Compound pc139, Pen-MeSer(Ph-2-Cl)-$OCH_2CN$) (500 mg).
LCMS (ESI) m/z = 351 (M+H)+
Retention time: 1.11 min (analytical condition SMD method 9)

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl O-(2-chlorophenyl)-N-methyl-N-(pent-4-enoyl)-L-serinate (Compound pc140, Pen-MeSer(Ph-2-Cl)-pCpA)

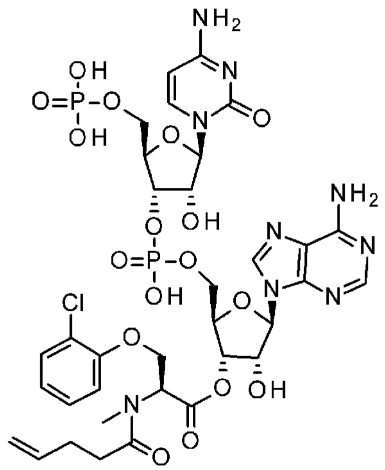

To buffer A (20 mL) were added a solution of ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (40.0 mg, 0.055 mmol) in water (2.0 mL) and a solution of cyanomethyl O-(2-chlorophenyl)-N-methyl-N-(pent-4-enoyl)-L-serinate (Compound pc139, Pen-MeSer(Ph-2-Cl)-$OCH_2CN$) (38.8 mg, 0.111 mmol) in acetonitrile (1.00 mL), and the mixture was stirred at room temperature for 1 h. The reaction solution was ice-cooled, trifluoroacetic acid (TFA) (1 mL) was added, and the mixture was stirred for 90 min. The reaction solution was diluted with water and purified by reverse phase column chromatography (0.05% aqueous trifluoroacetic acid solution/0.05% trifluoroacetic acid-acetonitrile solution) to afford the title compound (Compound pc140, Pen-MeSer(Ph-2-Cl)-pCpA) (4.20 mg, 8%).
LCMS (ESI) m/z = 944 (M-H)-
Retention time: 0.52 min (analytical condition SQDFA05)

Synthesis of O-(3-chlorophenyl)-N-(pent-4-enoyl)-L-serine (Compound pc141, Pen-Ser(Ph-3-Cl)-OH)

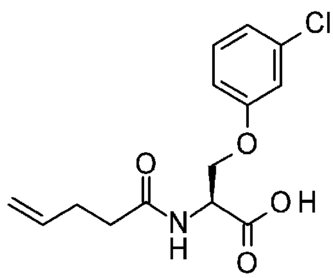

To a mixed solution of (S)-2-amino-3-(3-chlorophenoxy)propanoic acid (Compound aa21, H-Ser(Ph-3-Cl)-OH) (200 mg, 0.927 mmol) and sodium carbonate (123 mg, 1.16 mmol) in tetrahydrofuran (THF) (2.5 mL) and water (2.5 mL) was added 4-pentenoyl chloride (CAS No. 39716-58-0) (121 mg, 1.02 mmol) at room temperature, and the mixture

was stirred for 1 h. Sodium carbonate (59 mg) and 4-pentenoyl chloride (0.0514 mL) were then added, and the mixture was stirred for 1 h. A 5 mol/L aqueous hydrochloric acid solution (1 mL) was added, followed by extraction with ethyl acetate. The solvent was evaporated from the organic layer under reduced pressure to afford O-(3-chlorophenyl)-N-(pent-4-enoyl)-L-serine (Compound pc141, Pen-Ser(Ph-3-Cl)-OH) (250 mg) as a crude product.
LCMS (ESI) m/z = 298 (M+H)+
Retention time: 0.69 min (analytical condition SQDFA05)

Synthesis of cyanomethyl O-(3-chlorophenyl)-N-(pent-4-enoyl)-L-serinate (Compound pc142, Pen-Ser(Ph-3-Cl)-$OCH_2CN$)

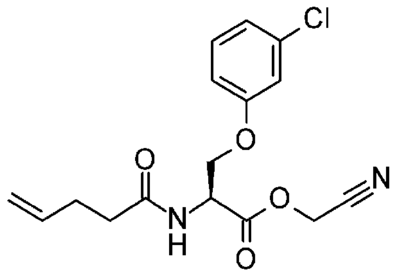

To a solution of O-(3-chlorophenyl)-N-(pent-4-enoyl)-L-serine (Compound pc141, Pen-Ser(Ph-3-Cl)-OH) (221 mg) in dimethylformamide (DMF) (7.4 mL) were added N,N-diisopropylethylamine (DIPEA) (0.259 mL, 1.49 mmol) and bromoacetonitrile (0.198 mL, 2.97 mmol) at room temperature, and the mixture was stirred for 30 min. Water was added to the reaction solution and the mixture was extracted with ethyl acetate. The resulting organic layer was dried over anhydrous sodium sulfate and the solvent was then evaporated under reduced pressure. The resulting crude product was purified by normal phase silica gel column chromatography (hexane/ethyl acetate) to afford cyanomethyl O-(3-chlorophenyl)-N-(pent-4-enoyl)-L-serinate (Compound pc142, Pen-Ser(Ph-3-Cl)-$OCH_2CN$) (257 mg) quantitatively.
LCMS (ESI) m/z = 337 (M+H)+
Retention time: 0.80 min (analytical condition SQDFA05)

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl O-(3-chlorophenyl)-N-(pent-4-enoyl)-L-serinate (Compound pc143, Pen-Ser(Ph-3-Cl)-pCpA)

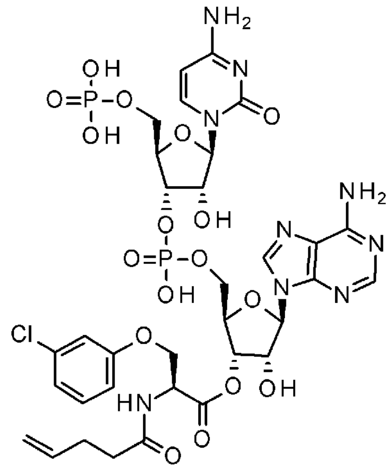

To buffer A (30 mL) were added a solution of ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (53.6 mg, 0.074 mmol) in water (1.5 mL) and a solution of cyanomethyl O-(3-chlorophenyl)-N-(pent-4-enoyl)-L-serinate (Compound

pc142, Pen-Ser(Ph-3-Cl)-$OCH_2CN$) (100 mg, 0.297 mmol) in acetonitrile (0.75 mL), and the mixture was stirred at room temperature for 40 min. To the reaction solution was added acetic acid (0.34 mL, 5.94 mmol), and the mixture was purified by reverse phase column chromatography to afford (2R,3S,4R,5R)-2-((((((2R,3R,4R,5R)-5-(4-amino-2-oxopyrimidin-1(2H)-yl)-2-((phosphonooxy)methyl)-4-((tetrahydrofuran-2-yl)oxy)tetrahydrofuran-3-yl)oxy)(hydroxy)phosphoryl)oxy)methyl)-5-(6-amino-9H-purin-9-yl)-4-hydroxytetrahydrofuran-3-yl O-(3-chlorophenyl)-N-(pent-4-enoyl)-L-serinate (Pen-Ser(Ph-3-Cl)-pCpA (THF)).

To the resulting (2R,3S,4R,5R)-2-((((((2R,3R,4R,5R)-5-(4-amino-2-oxopyrimidin-1(2H)-yl)-2-((phosphonooxy)methyl)-4-((tetrahydrofuran-2-yl)oxy)tetrahydrofuran-3-yl)oxy)(hydroxy)phosphoryl)oxy)methyl)-5-(6-amino-9H-purin-9-yl)-4-hydroxytetrahydrofuran-3-yl O-(3-chlorophenyl)-N-(pent-4-enoyl)-L-serinate (Pen-Ser(Ph-3-Cl)-pCpA(THF)) was added a mixed solution of acetic acid (4 mL) and water (1 mL), and the mixture was stirred at room temperature for 140 min. Purification by reverse phase column chromatography (0.05% aqueous trifluoroacetic acid solution/0.05% trifluoroacetic acid-acetonitrile solution) gave the title compound (Compound pc143, Pen-Ser(Ph-3-Cl)-pCpA) (15.6 mg).
LCMS (ESI) m/z = 932 (M+H)+
Retention time: 0.88 min (analytical condition SMD method 25)

Synthesis of (S)-4-(2-chlorophenyl)-2-(pent-4-enamido)butanoic acid (Compound pc144, Pen-Hph(2-Cl)-OH)

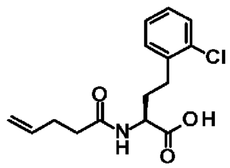

Commercially available (S)-2-amino-4-(2-chlorophenyl)butanoic acid (H-Hph(2-Cl)-OH) (200 mg, 0.936 mmol) was dissolved in water (650 μL), after which sodium carbonate (228 mg, 2.153 mmol) was added at room temperature and the mixture was stirred until it was a clear solution. To the reaction solution were added tetrahydrofuran (THF) (650 μL) and pent-4-enoyl chloride (207 μL, 1.872 mmol) at room temperature and the mixture was stirred for 7.5 hours, after which pent-4-enoyl chloride (207 μL, 1.872 mmol) and sodium carbonate (228 mg, 2.153 mmol) were added and the mixture was stirred overnight. After the disappearance of the raw material was confirmed by LC-MS, the reaction solution was diluted with water, formic acid (359 μL) was added, and the tetrahydrofuran (THF) was removed under reduced pressure by a rotary evaporator. To the resulting mixture was added dimethyl sulfoxide (DMSO), and the mixture was purified by reverse phase column chromatography (0.1% aqueous formic acid solution/0.1% formic acid-acetonitrile solution) to afford (S)-4-(2-chlorophenyl)-2-(pent-4-enamido)butanoic acid (Compound pc144, Pen-Hph(2-Cl)-OH) (223 mg, 81%).
LCMS (ESI) m/z = 296 (M+H)+
Retention time: 0.69 min (analytical condition SQDFA05)

Synthesis of cyanomethyl (S)-4-(2-chlorophenyl)-2-(pent-4-enamido)butanoate (Compound pc145, Pen-Hph(2-Cl)-$OCH_2CN$)

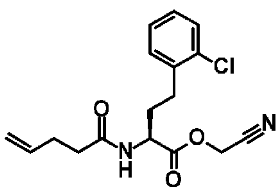

(S)-4-(2-Chlorophenyl)-2-(pent-4-enamido)butanoic acid (Compound pc144, Pen-Hph(2-Cl)-OH) (223 mg, 0.754 mmol) was dissolved in acetonitrile (1.5 mL) under a nitrogen atmosphere, N-ethyl-isopropylpropan-2-amine (DIPEA) (395 μL, 2.262 mmol) and 2-bromoacetonitrile (545 mg, 4.52 mmol) were added, and the mixture was stirred at room temperature for 4 h. The reaction solution was concentrated and the resulting residue was purified by normal

phase silica gel column chromatography (hexane/ethyl acetate) to afford cyanomethyl (S)-4-(2-chlorophenyl)-2-(pent-4-enamido)butanoate (Compound pc145, Pen-Hph(2-Cl)-$OCH_2CN$) (245 mg, 97%).
LCMS (ESI) m/z = 335 (M+H)+
Retention time: 0.78 min (analytical condition SQDFA05)

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl (2S)-4-(2-chlorophenyl)-2-(pent-4-enamido)butanoate (Compound pc146, Pen-Hph(2-Cl)-pCpA)

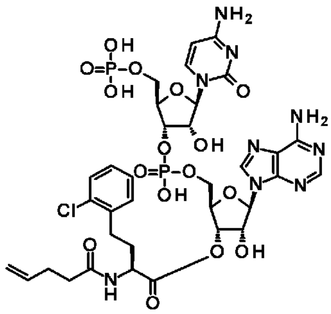

To buffer A (18 mL) were added a solution of ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (40 mg, 0.055 mmol) in water (1.8 mL) and a solution of cyanomethyl (S)-4-(2-chlorophenyl)-2-(pent-4-enamido)butanoate (Compound pc145, Pen-Hph(2-Cl)-$OCH_2CN$) (37.1 mg, 0.111 mmol) in acetonitrile (0.9 mL), and the mixture was stirred at room temperature for 30 min. To the reaction solution was added trifluoroacetic acid (900 μL) at 0°C and the mixture was stirred for 30 min, after which the reaction solution was diluted with water and purified by reverse phase column chromatography (0.05% aqueous trifluoroacetic acid solution/0.05% trifluoroacetic acid-acetonitrile solution) to afford the title compound (Compound pc146, Pen-Hph(2-Cl)-pCpA) (17 mg, 33% over two steps).
LCMS (ESI) m/z = 931 (M+H)+
Retention time: 0.60 min (analytical condition SQDFA05)
Compound pc150 (Pen-MeHph(2-Cl)-pCpA) was synthesized according to the following scheme.

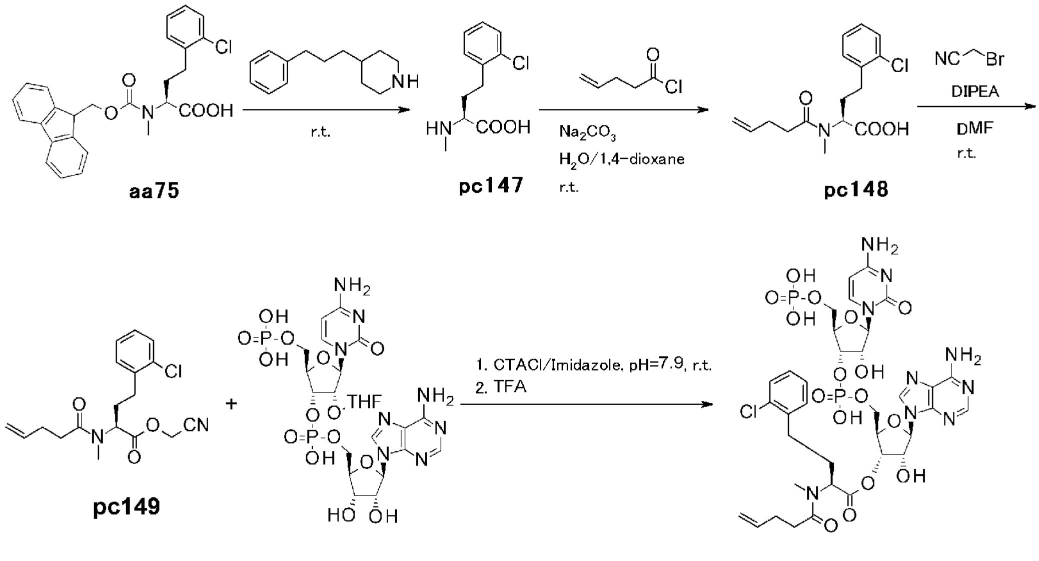

aa75 pc147 pc148 pc149

pc150

Synthesis of (2S)-4-(2-chlorophenyl)-2-(methylamino)butanoic acid (Compound pc147, H-MeHph(2-Cl)-OH)

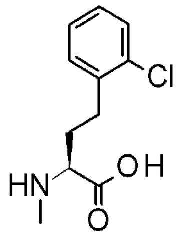

(S)-2-((((9H-Fluoren-9-yl)methoxy)carbonyl)(methyl)amino)-4-(2-chlorophenyl)butanoic acid (Compound aa75, Fmoc-MeHph(2-Cl)-OH) (274.3 mg, 0.610 mmol) was dissolved in dichloromethane (1.74 mL), 4-(3-phenylpropyl)piperidine (258 μL, 1.219 mmol) was added, and the mixture was stirred at room temperature for 5 h. Water was then added to the reaction solution, and the dichloromethane was removed by concentration under reduced pressure. The resulting aqueous layer was purified by reverse phase column chromatography (0.1% aqueous formic acid solution/0.1% formic acid-acetonitrile solution) to afford (2S)-4-(2-chlorophenyl)-2-(methylamino)butanoic acid (Compound pc147, H-MeHph(2-Cl)-OH) (152.6 mg) quantitatively.
LCMS (ESI) m/z = 228 (M+H)+
Retention time: 0.38 min (analytical condition SQDFA05)

Synthesis of (2S)-4-(2-chlorophenyl)-2-[methyl(pent-4-enoyl)amino]butanoic acid (Compound pc148, Pen-MeHph(2-Cl)-OH)

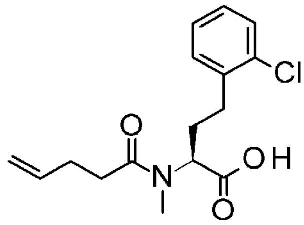

(2S)-4-(2-Chlorophenyl)-2-(methylamino)butanoic acid (Compound pc147, H-MeHph(2-Cl)-OH) (152.6 mg, 0.670 mmol) was dissolved in tetrahydrofuran/water (0.96 mL/0.96 mL), and sodium carbonate (234 mg, 2.212 mmol) was added. Pent-4-enoyl chloride (149 μL, 1.340 mmol) was then added and the mixture was stirred at room temperature for 30 min. Pent-4-enoyl chloride (149 μL, 1.340 mmol) and sodium carbonate (234 mg, 2.212 mmol) were then further added and the mixture was stirred at room temperature for 90 min. The reaction was quenched by adding formic acid (257 μL, 6.70 mmol), the tetrahydrofuran was removed by concentration under reduced pressure, and the resulting residue was purified by reverse phase column chromatography (0.1% aqueous formic acid solution/0.1% formic acid-acetonitrile solution) to afford (2S)-4-(2-chlorophenyl)-2-[methyl(pent-4-enoyl)amino]butanoic acid (Compound pc148, Pen-MeHph(2-Cl)-OH) (64.3 mg, 31%).
LCMS (ESI) m/z = 310 (M+H)+
Retention time: 0.75 min (analytical condition SQDFA05)

Synthesis of cyanomethyl (2S)-4-(2-chlorophenyl)-2-[methyl(pent-4-enoyl)amino]butanoate (Compound pc149, Pen-MeHph(2-Cl)-$OCH_2CN$)

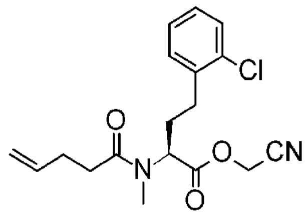

(2S)-4-(2-Chlorophenyl)-2-[methyl(pent-4-enoyl)amino]butanoic acid (Compound pc148, Pen-MeHph(2-Cl)-OH) (61.8 mg, 0.199 mmol) and 2-bromoacetonitrile (27.8 μL, 0.339 mmol) were dissolved in dimethylformamide (665 μL), N,N-diisopropylethylamine (DIPEA) (105 μL, 0.598 mmol) was added, and the mixture was stirred at room temperature. A saturated aqueous ammonium chloride solution was then added to the reaction solution, and the mixture was extracted with t-butyl methyl ether (MTBE). The organic layer was washed with saturated brine, then dried over anhydrous sodium sulfate, filtered, and concentrated under reduced pressure to afford cyanomethyl (2S)-4-(2-chlorophenyl)-2-[methyl(pent-4-enoyl)amino]butanoate (Compound pc149, Pen-MeHph(2-Cl)-$OCH_2CN$) (66.3 mg, 95%).
LCMS (ESI) m/z = 349 (M+H)+
Retention time: 0.86 min (analytical condition SQDFA05)

Synthesis of [(2R,3S,4R,5R)-2-[[[(2R,3S,4R,5R)-5-(4-amino-2-oxo-pyrimidin-1-yl)-4-hydroxy-2-(phosphonooxymethyl)tetrahydrofuran-3-yl]oxy-hydroxy-phosphoryl]oxymethyl]-5-(6-aminopurin-9-yl)-4-hydroxy-tetrahydrofuran-3-yl](2S)-4-(2-chlorophenyl)-2-[methyl(pent-4-enoyl)amino]butanoate (Compound pc150, Pen-MeHph(2-Cl)-pCpA)

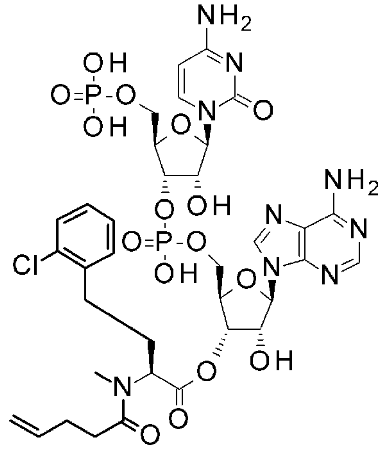

((2R,3R,4R,5R)-5-(4-Amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (68.3 mg, 0.095 mmol) was dissolved in water (2.9 mL), and this was added to buffer A (29 mL). To the aqueous solution was added a solution of cyanomethyl (2S)-4-(2-chlorophenyl)-2-[methyl(pent-4-enoyl)amino]butanoate (Compound pc149, Pen-MeHph(2-Cl)-$OCH_2CN$) (66.3 mg, 0.190 mmol) in acetonitrile (1.45 mL), and the mixture was stirred at room temperature for 90 min. To the reaction solution was then added trifluoroacetic acid (1.45 mL, 18.82 mmol) at 0°C, and the mixture was stirred for 30 min. The reaction solution was then diluted with water and purified by reverse phase column chromatography (0.05% aqueous trifluoroacetic acid solution/0.05% trifluoroacetic acid-acetonitrile solution) to afford the title compound (Compound pc150, Pen-MeHph(2-Cl)-pCpA) (43.2 mg, 48%).
LCMS (ESI) m/z = 945 (M+H)+
Retention time: 0.54 min (analytical condition SQDFA05)

Synthesis of (S)-4-(3-chlorophenyl)-2-(pent-4-enamido)butanoic acid (Compound pc151, Pen-Hph(3-Cl)-OH)

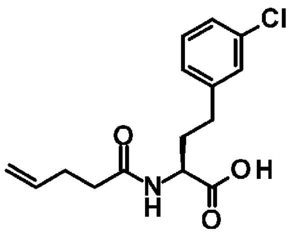

Commercially available (S)-2-amino-4-(3-chlorophenyl)butanoic acid (H-Hph(3-Cl)-OH) (200 mg, 0.936 mmol) was dissolved in water (650 μL), after which sodium carbonate (228 mg, 2.153 mmol) was added at room temperature and the mixture was stirred until it was a clear solution. To the reaction solution were added tetrahydrofuran (THF) (650 μL) and pent-4-enoyl chloride (207 μL, 1.872 mmol) at room temperature, and the mixture was stirred for 6 h. After the disappearance of the raw material was confirmed by LC-MS, formic acid (359 μL) was added to the reaction solution, and the tetrahydrofuran (THF) was removed under reduced pressure by a rotary evaporator. To the resulting mixture was added dimethyl sulfoxide (DMSO), and the mixture was purified by reverse phase column chromatography (0.1% aqueous formic acid solution/0.1% formic acid-acetonitrile solution) to afford (S)-4-(3-chlorophenyl)-2-(pent-4-enamido)butanoic acid (Compound pc151, Pen-Hph(3-Cl)-OH) (215 mg, 78%).
LCMS (ESI) m/z = 296 (M+H)+
Retention time: 0.70 min (analytical condition SQDFA05)

Synthesis of cyanomethyl (S)-4-(3-chlorophenyl)-2-(pent-4-enamido)butanoate (Compound pc152, Pen-Hph(3-Cl)-$OCH_2CN$)

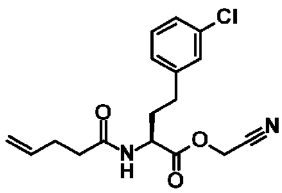

(S)-4-(3-Chlorophenyl)-2-(pent-4-enamido)butanoic acid (Compound pc151, Pen-Hph(3-Cl)-OH) (215 mg, 0.727 mmol) was dissolved in acetonitrile (1.5 mL) under a nitrogen atmosphere, N,N-diisopropylethylamine (DIPEA) (381 μL, 2.181 mmol) and 2-bromoacetonitrile (543 mg, 4.52 mmol) were added, and the mixture was stirred at room temperature for 4 h. The reaction solution was concentrated and the resulting residue was purified by normal phase silica gel column chromatography (hexane/ethyl acetate) to afford cyanomethyl (S)-4-(3-chlorophenyl)-2-(pent-4-enamido)butanoate (Compound pc152, Pen-Hph(3-Cl)-$OCH_2CN$) (237 mg, 97%).
LCMS (ESI) m/z = 335 (M+H)+
Retention time: 0.80 min (analytical condition SQDFA05)

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl (2S)-4-(3-chlorophenyl)-2-(pent-4-enamido)butanoate (Compound pc153, Pen-Hph(3-Cl)-pCpA)

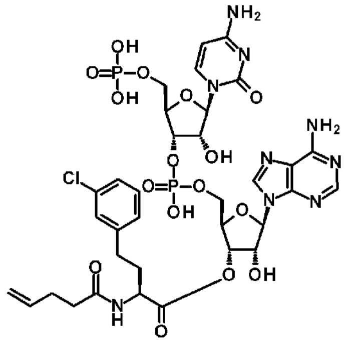

To buffer A (18 mL) were added a solution of ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (40 mg, 0.055 mmol) in water (1.8 mL) and a solution of cyanomethyl (S)-4-(3-chlorophenyl)-2-(pent-4-enamido)butanoate (Compound pc152, Pen-Hph(3-Cl)-$OCH_2CN$) (37.1 mg, 0.111 mmol) in acetonitrile (0.9 mL), and the mixture was stirred at room temperature for 30 min. To the reaction solution was added trifluoroacetic acid (900 μL) at 0°C and the mixture was stirred for 30 min, after which the reaction solution was diluted with water and purified by reverse phase column chromatography (0.05% aqueous trifluoroacetic acid solution/0.05% trifluoroacetic acid-acetonitrile solution) to afford the title compound (Compound pc153, Pen-Hph(3-Cl)-pCpA) (11 mg, 21% over two steps).
LCMS (ESI) m/z = 931 (M+H)+
Retention time: 0.62 min (analytical condition SQDFA05)

Compound pc157 (Pen-MeHph(3-Cl)-pCpA) was synthesized according to the following scheme.

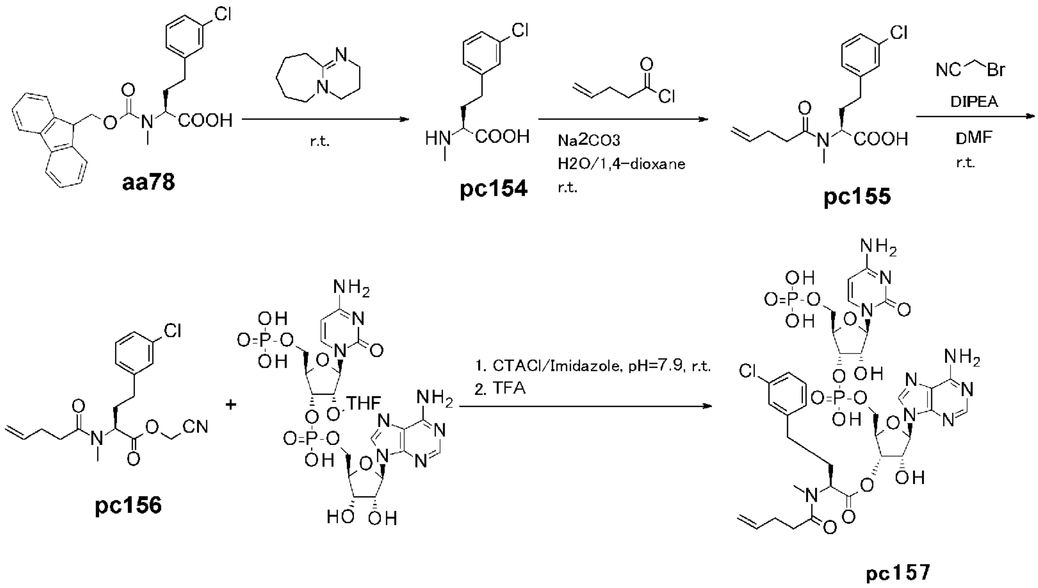

aa78 pc154 pc155 pc156 pc157

Synthesis of (2S)-4-(3-chlorophenyl)-2-(methylamino)butanoic acid (Compound pc154, H-MeHph(3-Cl)-OH)

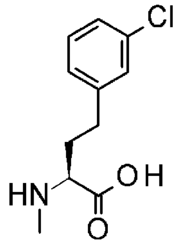

(S)-2-((((9H-Fluoren-9-yl)methoxy)carbonyl)(methyl)amino)-4-(3-chlorophenyl)butanoic acid (Compound aa78, Fmoc-MeHph(3-Cl)-OH) (261.7 mg, 0.582 mmol) was dissolved in dimethylformamide (1.66 mL), 1,8-diazabicyclo[5.4.0]-7-undecene (DBU) (52.6 μL, 0.349 mmol) was added, and the mixture was stirred at room temperature for 2 h. To the reaction solution was then added a 0.1% aqueous formic acid solution, and the mixture was purified by reverse phase column chromatography (0.1% aqueous formic acid solution/0.1% formic acid-acetonitrile solution) to afford (2S)-4-(3-chlorophenyl)-2-(methylamino)butanoic acid (Compound pc154, H-MeHph(3-Cl)-OH) (50.5 mg, 38%).
LCMS (ESI) m/z = 228 (M+H)+
Retention time: 0.41 min (analytical condition SQDFA05)

Synthesis of (2S)-4-(3-chlorophenyl)-2-[methyl(pent-4-enoyl)amino]butanoic acid (Compound pc155, Pen-MeHph(3-Cl)-OH)

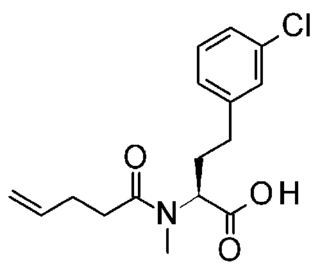

(2S)-4-(3-Chlorophenyl)-2-(methylamino)butanoic acid (Compound pc154, H-MeHph(3-Cl)-OH) (50.5 mg, 0.222 mmol) was dissolved in tetrahydrofuran/water (0.32 mL/0.32 mL), and sodium carbonate (78 mg, 0.732 mmol) was added. Pent-4-enoyl chloride (49.2 μL, 0.444 mmol) was then added and the mixture was stirred at room temperature. Pent-4-enoyl chloride (49.2 μL, 0.444 mmol) and sodium carbonate (78 mg, 0.732 mmol) were then further added and the mixture was stirred at room temperature for 90 min. The reaction was quenched by adding formic acid (85 μL, 2.218 mmol), the tetrahydrofuran was removed by concentration under reduced pressure, and the resulting residue was purified by reverse phase column chromatography (0.1% aqueous formic acid solution/0.1% formic acid-acetonitrile solution) to afford (2S)-4-(3-chlorophenyl)-2-[methyl(pent-4-enoyl)amino]butanoic acid (Compound pc155, Pen-MeHph(3-Cl)-OH) (39.0 mg, 57%).
LCMS (ESI) m/z = 310 (M+H)+
Retention time: 0.76 min (analytical condition SQDFA05)

Synthesis of cyanomethyl (2S)-4-(3-chlorophenyl)-2-[methyl(pent-4-enoyl)amino]butanoate (Compound pc156, Pen-MeHph(3-Cl)-$OCH_2CN$)

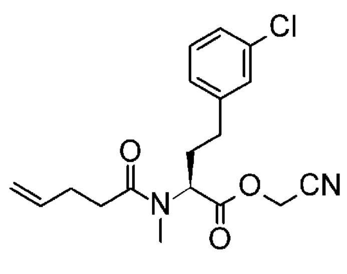

(2S)-4-(3-Chlorophenyl)-2-[methyl(pent-4-enoyl)amino]butanoic acid (Compound pc155, Pen-MeHph(3-Cl)-OH) (37.5 mg, 0.121 mmol) and 2-bromoacetonitrile (16.9 μL, 0.242 mmol) were dissolved in dimethylformamide (403 μL), N,N-diisopropylethylamine (DIPEA) (63.4 μL, 0.363 mmol) was added, and the mixture was stirred at room temperature. A saturated aqueous ammonium chloride solution was then added to the reaction solution, and the mixture was extracted with t-butyl methyl ether. The organic layer was washed with saturated brine, then dried over anhydrous sodium sulfate, filtered, and concentrated under reduced pressure to afford cyanomethyl (2S)-4-(3-chlorophenyl)-2-[methyl(pent-4-enoyl)amino]butanoate (Compound pc156, Pen-MeHph(3-Cl)-$OCH_2CN$) (31.5 mg, 75%).
LCMS (ESI) m/z = 349 (M+H)+

Retention time: 0.86 min (analytical condition SQDFA05)

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl (2S)-4-(3-chlorophenyl)-2-(N-methylpent-4-enamido)butanoate (Compound pc157, Pen-MeHph(3-Cl)-pCpA)

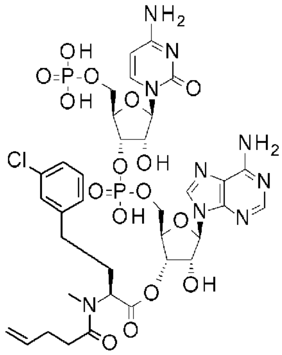

((2R,3R,4R,5R)-5-(4-Amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (32.6 mg, 0.045 mmol) was dissolved in water (1.4 mL), and this was added to buffer A (14 mL). To the aqueous solution was added a solution of cyanomethyl (2S)-4-(3-chlorophenyl)-2-[methyl(pent-4-enoyl)amino]butanoate (Compound pc156, Pen-MeHph(3-Cl)-$OCH_2CN$) (31.5 mg, 0.090 mmol) in acetonitrile (0.7 mL), and the mixture was stirred at room temperature for 30 min. To the reaction solution was then added trifluoroacetic acid (0.7 mL, 9.09 mmol) at 0°C, and the mixture was stirred for 30 min. The reaction solution was then diluted with water (16 mL) and purified by reverse phase column chromatography (0.05% aqueous trifluoroacetic acid solution/0.05% trifluoroacetic acid-acetonitrile solution) to afford the title compound (Compound pc157, Pen-MeHph(3-Cl)-pCpA) (18.5 mg, 43%).
LCMS (ESI) m/z = 945 (M+H)+
Retention time: 0.55 min (analytical condition SQDFA05)

Synthesis of (S)-4-(4-chlorophenyl)-2-(pent-4-enamido)butanoic acid (Compound pc158, Pen-Hph(4-Cl)-OH)

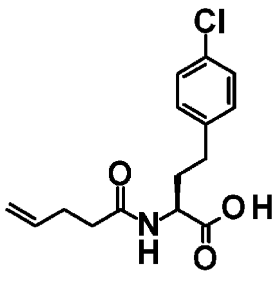

Commercially available (S)-2-amino-4-(4-chlorophenyl)butanoic acid (H-Hph(4-Cl)-OH) (200 mg, 0.936 mmol) was dissolved in water (650 μL), after which sodium carbonate (228 mg, 2.153 mmol) was added at room temperature and the mixture was stirred at room temperature until it was a clear solution. To the reaction solution were added tetrahydrofuran (THF) (650 μL) and pent-4-enoyl chloride (207 μL, 1.872 mmol) at room temperature, and the mixture was stirred at room temperature for 6 h. After the disappearance of the raw material was confirmed by LC-MS, formic acid (359 μL) was added to the reaction solution, and the tetrahydrofuran (THF) was removed under reduced pressure by a rotary evaporator. To the resulting mixture was added dimethyl sulfoxide (DMSO), and the mixture was purified by reverse phase column chromatography (0.1% aqueous formic acid solution/0.1% formic acid-acetonitrile solution) to afford (S)-4-(4-chlorophenyl)-2-(pent-4-enamido)butanoic acid (Compound pc158, Pen-Hph(4-Cl)-OH) (130 mg, 47%).

LCMS (ESI) m/z = 296 (M+H)+
Retention time: 0.70 min (analytical condition SQDFA05)

Synthesis of cyanomethyl (S)-4-(4-chlorophenyl)-2-(pent-4-enamido)butanoate (Compound pc159, Pen-Hph(4-Cl)-$OCH_2CN$)

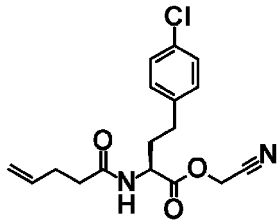

(S)-4-(4-Chlorophenyl)-2-(pent-4-enamido)butanoic acid (Compound pc158, Pen-Hph(4-Cl)-OH) (130 mg, 0.440 mmol) was dissolved in acetonitrile (1.0 mL) under a nitrogen atmosphere, N,N-diisopropylethylamine (DIPEA) (230 μL, 1.319 mmol) and 2-bromoacetonitrile (316 mg, 2.64 mmol) were added, and the mixture was stirred at room temperature for 4 h. The reaction solution was concentrated and the resulting residue was purified by normal phase silica gel column chromatography (hexane/ethyl acetate) to afford cyanomethyl (S)-4-(4-chlorophenyl)-2-(pent-4-enamido)butanoate (Compound pc159, Pen-Hph(4-Cl)-$OCH_2CN$) (139 mg, 94%).
LCMS (ESI) m/z = 335 (M+H)+
Retention time: 0.80 min (analytical condition SQDFA05)

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl (2S)-4-(4-chlorophenyl)-2-(pent-4-enamido)butanoate (Compound pc160, Pen-Hph(4-Cl)-pCpA)

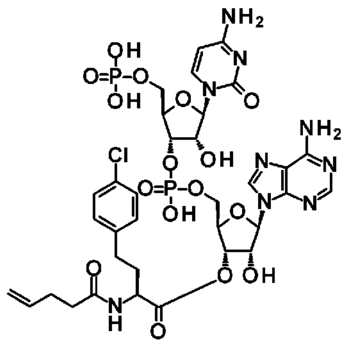

To buffer A (18 mL) were added a solution of ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (40 mg, 0.055 mmol) in water (1.8 mL) and a solution of cyanomethyl (S)-4-(4-chlorophenyl)-2-(pent-4-enamido)butanoate (Compound pc159, Pen-Hph(4-Cl)-$OCH_2CN$) (37.1 mg, 0.111 mmol) in acetonitrile (0.9 mL), and the mixture was stirred at room temperature for 6 h. To the reaction solution was added trifluoroacetic acid (900 μL) at 0°C and the mixture was stirred for 40 min, after which the reaction solution was diluted with water and purified by reverse phase column chromatography (0.05% aqueous trifluoroacetic acid solution/0.05% trifluoroacetic acid-acetonitrile solution) to afford the title compound (Compound pc160, Pen-Hph(4-Cl)-pCpA) (12 mg, 23% over two steps).
LCMS (ESI) m/z = 931 (M+H)+
Retention time: 0.62 min (analytical condition SQDFA05)

Compound pc164 (Pen-MeHph(4-Cl)-pCpA) was synthesized according to the following scheme.

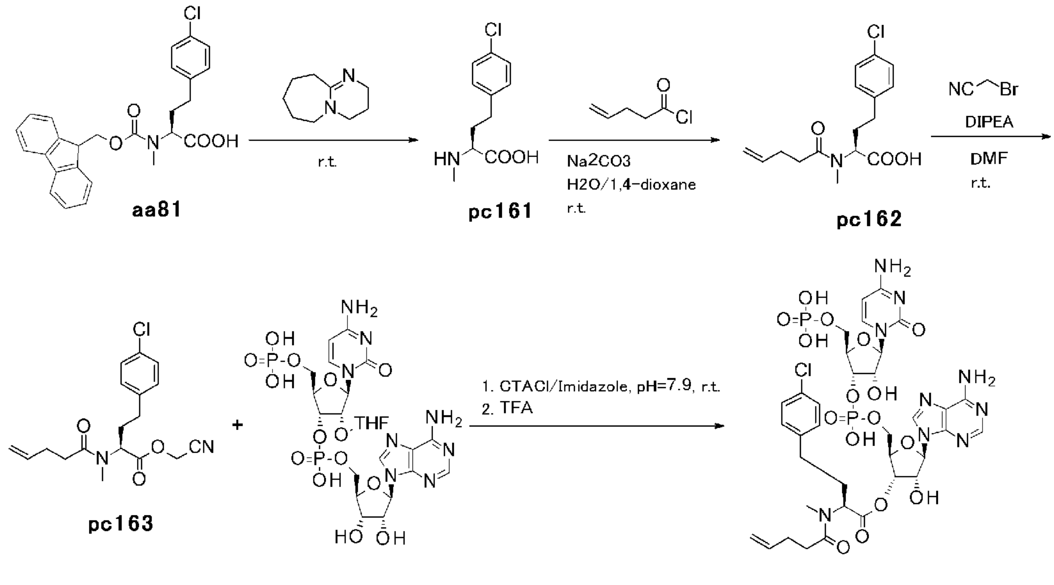


aa81 pc161 pc162 pc163 pc164

Synthesis of (2S)-4-(4-chlorophenyl)-2-(methylamino)butanoic acid (Compound pc161, H-MeHph(4-Cl)-OH)

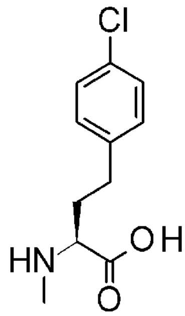

(S)-2-((((9H-Fluoren-9-yl)methoxy)carbonyl)(methyl)amino)-4-(4-chlorophenyl)butanoic acid (Compound aa81, Fmoc-MeHph(4-Cl)-OH) (180.5 mg, 0.401 mmol) was dissolved in dimethylformamide (1.15 mL), 1,8-diazabicyclo[5.4.0]-7-undecene (DBU) (36.3 μL, 0.241 mmol) was added, and the mixture was stirred at room temperature for 2 h. To the reaction solution was then added a 0.1% aqueous formic acid solution, and the mixture was purified by reverse phase column chromatography (0.1% aqueous formic acid solution/0.1% formic acid-acetonitrile solution) to afford (2S)-4-(4-chlorophenyl)-2-(methylamino)butanoic acid (Compound pc161, H-MeHph(4-Cl)-OH) (56.5 mg, 62%).
LCMS (ESI) m/z = 228 (M+H)+
Retention time: 0.42 min (analytical condition SQDFA05)

Synthesis of (2S)-4-(4-chlorophenyl)-2-[methyl(pent-4-enoyl)amino]butanoic acid (Compound pc162, Pen-MeHph(4-Cl)-OH)

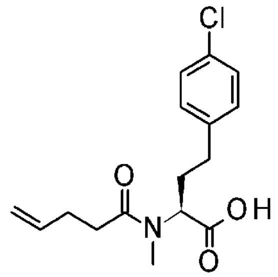

(2S)-4-(4-Chlorophenyl)-2-(methylamino)butanoic acid (Compound pc161, H-MeHph(4-Cl)-OH) (56.5 mg, 0.248 mmol) was dissolved in tetrahydrofuran/water (0.35 mL/0.35 mL), and sodium carbonate (87 mg, 0.819 mmol) was added. Pent-4-enoyl chloride (55.0 μL, 0.496 mmol) was then added and the mixture was stirred at room temperature. Pent-4-enoyl chloride (55.0 μL, 0.496 mmol) and sodium carbonate (87 mg, 0.819 mmol) were then further added and the mixture was stirred at room temperature for 90 min. The reaction was quenched by adding formic acid (95 μL, 2.481 mmol), the tetrahydrofuran was removed by concentration under reduced pressure, and the resulting residue was purified by reverse phase column chromatography (0.1% aqueous formic acid solution/0.1% formic acid-acetonitrile solution) to afford (2S)-4-(4-chlorophenyl)-2-[methyl(pent-4-enoyl)amino]butanoic acid (Compound pc162, Pen-MeHph(4-Cl)-OH) (55.1 mg, 72%).
LCMS (ESI) m/z = 310 (M+H)+
Retention time: 0.76 min (analytical condition SQDFA05)

Synthesis of cyanomethyl (2S)-4-(4-chlorophenyl)-2-[methyl(pent-4-enoyl)amino]butanoate (Compound pc163, Pen-MeHph(4-Cl)-$OCH_2CN$)

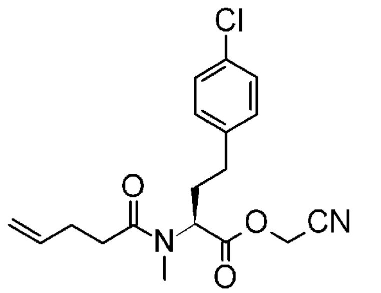

(2S)-4-(4-Chlorophenyl)-2-[methyl(pent-4-enoyl)amino]butanoic acid (Compound pc162, Pen-MeHph(4-Cl)-OH) (53.9 mg, 0.174 mmol) and 2-bromoacetonitrile (24.27 μL, 0.348 mmol) were dissolved in dimethylformamide (580 μL), N,N-diisopropylethylamine (DIPEA) (91 μL, 0.522 mmol) was added, and the mixture was stirred at room temperature. A saturated aqueous ammonium chloride solution was then added to the reaction solution, and the mixture was extracted with t-butyl methyl ether. The organic layer was washed with saturated brine, then dried over anhydrous sodium sulfate, filtered, and concentrated under reduced pressure to afford cyanomethyl (2S)-4-(4-chlorophenyl)-2-[methyl(pent-4-enoyl)amino]butanoate (Compound pc163, Pen-MeHph(4-Cl)-$OCH_2CN$) (50.7 mg, 84%).
LCMS (ESI) m/z = 349 (M+H)+
Retention time: 0.86 min (analytical condition SQDFA05)

Synthesis of [(2R,3S,4R,5R)-2-[[[(2R,3S,4R,5R)-5-(4-amino-2-oxo-pyrimidin-1-yl)-4-hydroxy-2-(phosphonooxymethyl)tetrahydrofuran-3-yl]oxy-hydroxy-phosphoryl]oxymethyl]-5-(6-aminopurin-9-yl)-4-hydroxy-tetrahydrofuran-3-yl](2S)-4-(4-chlorophenyl)-2-[methyl(pent-4-enoyl)aminolbutanoate (Compound pc164, Pen-MeHph(4-Cl)-pCpA)

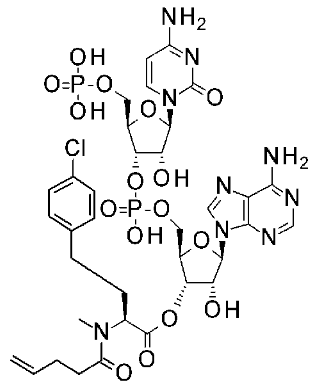

((2R,3R,4R,5R)-5-(4-Amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (52.5 mg, 0.073 mmol) was dissolved in water (2.2 mL), and this was added to buffer A (22 mL). To the aqueous solution was added a solution of cyanomethyl (2S)-4-(4-chlorophenyl)-2-[methyl(pent-4-enoyl)amino]butanoate (Compound pc163, Pen-MeHph(4-Cl)-$OCH_2CN$) (50.7 mg, 0.145 mmol) in acetonitrile (1.1 mL), and the mixture was stirred at room temperature for 30 min. To the reaction solution was then added trifluoroacetic acid (1.1 mL, 14.28 mmol) at 0°C, and the mixture was stirred for 30 min. The reaction solution was then diluted with water (25 mL) and purified by reverse phase column chromatography (0.05% aqueous trifluoroacetic acid solution/0.05% trifluoroacetic acid-acetonitrile solution) to afford the title compound (Compound pc164, Pen-MeHph(4-Cl)-pCpA) (31.3 mg, 46%).
LCMS (ESI) m/z = 945 (M+H)+
Retention time: 0.54 min (analytical condition SQDFA05)

<u>Synthesis of (S)-2-((tert-butoxycarbonyl)amino)-3-((5-fluoropyridin-3-yl)methoxy)propanoic acid (Compound pc165, Boc-Ser(3-F-5-Me-Pyr)-OH)</u>

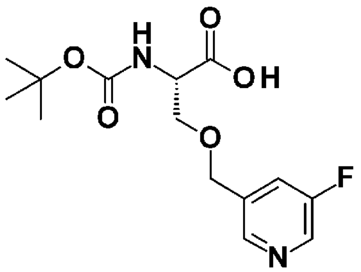

To a solution of sodium hydride (731 mg, 18.27 mmol, 60% oil dispersion) in N,N-dimethylformamide (10 mL) was added a solution of commercially purchased (S)-2-((tert-butoxycarbonyl)amino)-3-hydroxypropanoic acid (Boc-Ser-OH) (1.136 g, 5.54 mmol) in N,N-dimethylformamide (10 mL) dropwise at 0°C under a nitrogen atmosphere, and the mixture was stirred for 1 h. To the reaction solution was added a solution of 3-(bromomethyl)-5-fluoropyridine hydrobromide (Compound aa15) (1.5 g, 5.54 mmol) in N,N-dimethylformamide (10 mL) dropwise, and the mixture was stirred at room temperature for 16 h. A saturated aqueous ammonium chloride solution was added to the reaction solution, and the mixture was extracted with ethyl acetate twice. The organic layers were washed with water and with saturated brine twice, after which the resulting organic layers were dried over anhydrous sodium sulfate, filtered, and concentrated under reduced pressure. The resulting residue was purified by reverse phase column chromatography (0.1% aqueous formic acid solution/0.1% formic acid-acetonitrile solution) to afford (S)-2-((tert-butoxycarbonyl)amino)-3-((5-fluoropyridin-3-yl)methoxy)propanoic acid (Compound pc165, Boc-Ser(3-F,5-Me-Pyr)-OH) (500 mg).
LCMS (ESI) m/z = 313 (M-H)-
Retention time: 0.63 min (analytical condition SQDFA05)

Synthesis of (S)-3-((5-fluoropyridin-3-yl)methoxy)-2-(pent-4-enamido)propanoic acid (Compound pc166, Pen-Ser(3-F-5-Me-Pyr)-OH)

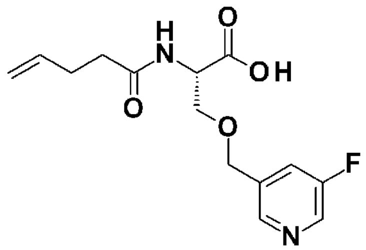

To a solution of (S)-2-((tert-butoxycarbonyl)amino)-3-((5-fluoropyridin-3-yl)methoxy)propanoic acid (Compound pc165, Boc-Ser(3-F-5-Me-Pyr)-OH) (195 mg, 0.620 mmol) in 1,4-dioxane (2.2 mL) was added a 4 N hydrochloric acid/1,4-dioxane solution (4 mL) at room temperature, and the mixture was stirred at room temperature for 2 h. To the reaction solution was further added a 4 N hydrochloric acid/1,4-dioxane solution (2 mL), and the mixture was stirred for 30 min and concentrated under reduced pressure. The resulting residue and sodium carbonate (255 mg, 2.40 mol) were dissolved in water (5.01 mL) and 1,4-dioxane (3.0 mL), pent-4-enoyl chloride (146 μL, 1.32 mmol) was added at room temperature, and the mixture was stirred for 45 min. Pent-4-enoyl chloride (45 μL, 0.41 mmol) was further added at room temperature, and the mixture was stirred for 45 min. To the reaction solution was added a 1 N aqueous hydrochloric acid solution (1.60 mL), and the mixture was concentrated under reduced pressure. The resulting residue was diluted with water and then purified by reverse phase column chromatography (0.1% aqueous formic acid solution/0.1% formic acid-acetonitrile solution) to afford (S)-3-((5-fluoropyridin-3-yl)methoxy)-2-(pent-4-enamido)propanoic acid (Compound pc166, Pen-Ser(3-F,5-Me-Pyr)-OH) (95.4 mg).
LCMS (ESI) m/z = 297 (M+H)+
Retention time: 0.59 min (analytical condition SQDAA05)

Synthesis of (S)-cyanomethyl 3-((5-fluoropyridin-3-yl)methoxy)-2-(pent-4-enamido)propanoate (Compound pc167, Pen-Ser(3-F-5-Me-Pyr)-$OCH_2CN$)

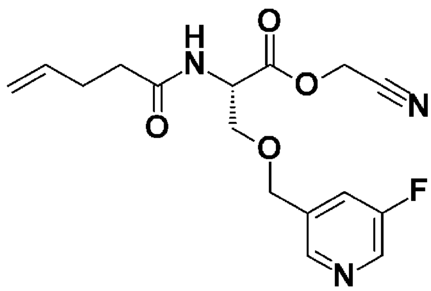

To a solution of (S)-3-((5-fluoropyridin-3-yl)methoxy)-2-(pent-4-enamido)propanoic acid (Compound pc166, Pen-Ser(3-F-5-Me-Pyr)-OH) (85 mg, 0.287 mmol) in dimethylformamide (DMF) (2.87 mL) were added 2-bromoacetonitrile (20.0 μL, 0.0287 mmol) and N,N-diisopropylethylamine (DIPEA) (75 μL, 0.43 mmol) at room temperature under a nitrogen atmosphere, and the mixture was stirred for 4 h. To the reaction solution was added a saturated aqueous ammonium chloride solution, and the mixture was extracted with ethyl acetate and washed with water three times and with saturated brine, after which the resulting organic layer was dried over anhydrous sodium sulfate, filtered, and concentrated under reduced pressure to give a crude product (S)-cyanomethyl 3-((5-fluoropyridin-3-yl)methoxy)-2-(pent-4-enamido)propanoate (Compound pc167, Pen-Ser(3-F,5-Me-Pyr)-$OCH_2CN$) (68.4 mg, 71%).
LCMS (ESI) m/z = 336 (M+H)+
Retention time: 0.72 min (analytical condition SQDAA05)

Synthesis of (2S)-(2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl 3-((5-fluoropyridin-3-yl)methoxy)-2-(pent-4-enamido)propanoate (Compound pc168, Pen-Ser(3-F-5-Me-Pyr)-pCpA)

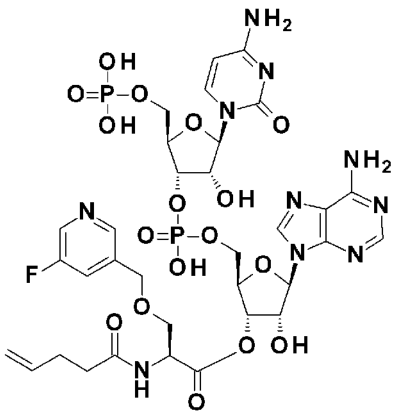

To buffer A (14 mL) were added a solution of ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (48.8 mg, 0.068 mmol) in water (0.9 mL) and a solution of the above crude product (S)-cyanomethyl 3-((5-fluoropyridin-3-yl)methoxy)-2-(pent-4-enamido)propanoate (Compound pc167, Pen-Ser(3-F,5-Me-Pyr)-$OCH_2CN$) (68.0 mg, 0.203 mmol) in acetonitrile (0.55 mL), and the mixture was stirred at room temperature for 90 min. To the reaction solution was added acetic acid (14 mL) at room temperature, and the mixture was stirred for 90 min. The reaction solution was diluted with water (80 mL) and purified by reverse phase column chromatography (0.05% aqueous trifluoroacetic acid solution/0.05% trifluoroacetic acid-acetonitrile solution) to afford the title compound (Compound pc168, Pen-Ser(3-F-5-Me-Pyr)-pCpA) (20.9 mg, 33% over two steps).
LCMS (ESI) m/z = 931 (M+H)+
Retention time: 0.37 min (analytical condition SQDFA05)

Synthesis of (2S)-3-[(5-fluoropyridin-3-yl)methoxy]-2-(N-methylpent-4-enamido)propanoic acid (Compound pc169, Pen-MeSer(3-F-5-Me-Pyr)-OH)

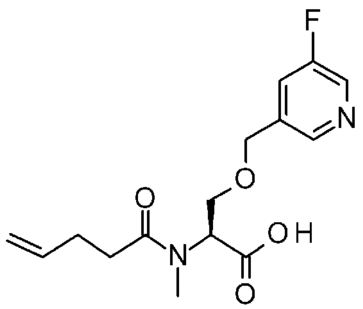

To a solution of N-(((9H-fluoren-9-yl)methoxy)carbonyl)-O-((5-fluoropyridin-3-yl)methyl)-N-methyl-L-serine (Compound aa67, Fmoc-MeSer(3-F-5-Me-Pyr)-OH) (7.00 g, 15.5 mmol) in N,N-dimethylformamide (DMF) (95 mL) was added piperidine (24 mL) at room temperature, and the mixture was stirred. After 16 hours, diethyl ether (100 mL) was added and the mixture was stirred for 30 min. The precipitate was collected by filtration to afford (2S)-3-[(5-fluoropyridin-3-yl)methoxy]-2-(methylamino)propanoic acid (H-MeSer(3-F-5-Me-Pyr)-OH) (2.75 g) as a crude product.

The above (2S)-3-[(5-fluoropyridin-3-yl)methoxy]-2-(methylamino)propanoic acid (H-MeSer(3-F-5-Me-Pyr)-OH) was dissolved in a mixture of water (50 mL) and 1,4-dioxane (50 mL), sodium bicarbonate (2.03 g, 24.2 mmol)

and 2,5-dioxopyrrolidin-1-yl pent-4-enoate (4.75 g, 24.1 mmol) were added, and the mixture was stirred at room temperature for 16 h. The reaction solution was washed with diethyl ether twice, and the aqueous layer was adjusted to pH = 2 with an aqueous hydrochloric acid solution (1 mol/L). Extraction with dichloromethane was carried out twice, drying was carried out over anhydrous sodium sulfate, and the solvent was then evaporated under reduced pressure. The resulting crude product was purified by normal phase silica gel column chromatography (petroleum ether/ethyl acetate) to afford (2S)-3-[(5-fluoropyridin-3-yl)methoxy]-2-(N-methylpent-4-enamido)propanoic acid (Compound pc169, Pen-Me-Ser(3-F-5-Me-Pyr)-OH) (1.66 g, 44%).
LCMS (ESI) m/z = 311 (M+H)+
Retention time: 1.14 min (analytical condition SMD method 33)

Synthesis of cyanomethyl (2S)-3-[(5-fluoropyridin-3-yl)methoxy]-2-(N-methylpent-4-enamido)propanoate (Compound pc170, Pen-MeSer(3-F-5-Me-Pyr)-$OCH_2CN$)

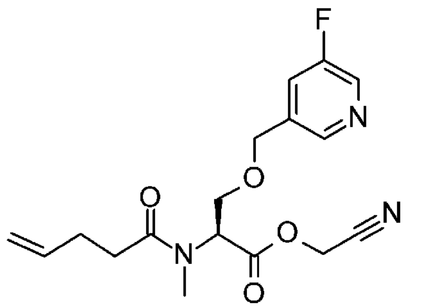

(2S)-3-[(5-Fluoropyridin-3-yl)methoxy]-2-(N-methylpent-4-enamido)propanoic acid (Compound pc169, Pen-MeSer(3-F-5-Me-Pyr)-OH) (1.65 g, 5.32 mmol), 2-bromoacetonitrile (2.55 g, 21.3 mmol), and N,N-diisopropylethylamine (DIPEA) (1.37 g, 10.6 mmol) were dissolved in dichloromethane (DCM) (100 mL), and the mixture was stirred at 25°C. After 16 hours, the solvent was evaporated from the reaction solution under reduced pressure. The residue was purified by normal phase silica gel column chromatography (petroleum ether/ethyl acetate) to afford cyanomethyl (2S)-3-[(5-fluoropyridin-3-yl)methoxy]-2-(N-methylpent-4-enamido)propanoate (Compound pc170, Pen-MeSer(3-F-5-Me-Pyr)-$OCH_2CN$) (1.00 g, 54%).
LCMS (ESI) m/z = 350 (M+H)+
Retention time: 0.90 min (analytical condition SMD method 9)

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl O-((5-fluoropyridin-3-yl)methyl)-N-methyl-N-(pent-4-enoyl)-L-serinate (Compound pc171, Pen-MeSer(3-F-5-Me-Pyr)-pCpA)

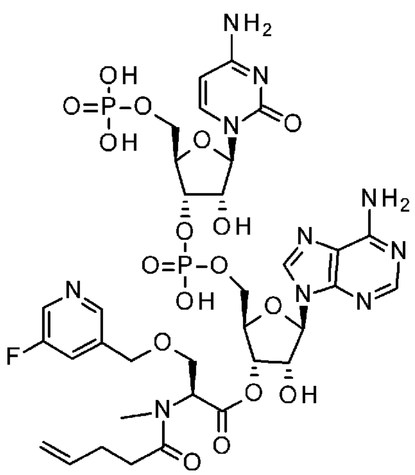

To buffer A (75 mL) were added ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (300 mg, 0.42 mmol) and cyanomethyl (2S)-3-[(5-fluoropyridin-3-yl)methoxy]-2-(N-methylpent-4-enamido)propanoate (Compound pc170, Pen-MeSer(3-F-5-Me-Pyr)-$OCH_2CN$) (580 mg, 1.66 mmol), and the mixture was stirred at room temperature for 2 h.

To the reaction solution was added trifluoroacetic acid (TFA) (1.6 mL), the reaction solution was lyophilized, and the residue was purified by reverse phase column chromatography (0.05% aqueous trifluoroacetic acid solution/0.05% trifluoroacetic acid-acetonitrile solution) to afford the title compound (Compound pc171, Pen-MeSer(3-F-5-Me-Pyr)-pCpA) (60 mg, 8%).
LCMS (ESI) m/z = 945 (M+H)+
Retention time: 0.42 min (analytical condition SQDFA05)

Synthesis of (S)-2-amino-5-phenylpentanoic acid (Compound pc172, H-Phe{#(CH2)2}-OH)

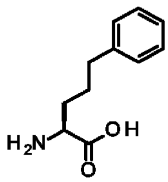

To a solution of commercially available (S)-2-((((9H-fluoren-9-yl)methoxy)carbonyl)amino)-5-phenylpentanoic acid (Fmoc-Phe{#(CH2)2}-OH, purchased from Watanabe Chemical Industries) (500 mg, 1.203 mmol) in dichloromethane (DCM) (1.2 mL) was added 4-(3-phenylpropyl)piperidine (0.510 mL, 2.407 mmol) at room temperature under a nitrogen atmosphere. The reaction solution was stirred at room temperature for 23 hours, then diluted with dichloromethane (DCM), and extracted with water. The resulting aqueous layer was concentrated and lyophilized, dimethyl sulfoxide (DMSO) and formic acid were added to the resulting residue, which was then purified by reverse phase column chromatography (0.1% aqueous formic acid solution/0.1% formic acid-acetonitrile solution) twice to afford (S)-2-amino-5-phenylpentanoic acid (Compound pc172, H-Phe{#(CH2)2}-OH) (80 mg, 34%).
LCMS (ESI) m/z = 194 (M+H)+
Retention time: 0.37 min (analytical condition SQDFA05)

Synthesis of (S)-2-(pent-4-enamido)-5-phenylpentanoic acid (Compound pc173, Pen-Phe{#(CH2)2}-OH)

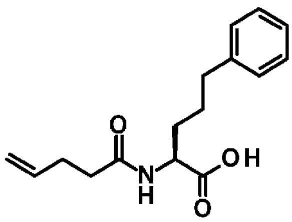

To a solution of (S)-2-amino-5-phenylpentanoic acid (Compound pc172, H-Phe{#(CH2)2}-OH) (80 mg, 0.414 mmol) in water (2.0 mL) was added sodium carbonate (101 mg, 0.952 mmol) at room temperature under a nitrogen atmosphere, and the reaction solution was stirred until it became clear. To the reaction solution was added a solution of pent-4-enoyl chloride (0.092 mL, 0.828 mmol) in tetrahydrofuran (THF) (2.0 mL) dropwise at room temperature, and the mixture was stirred for 8 h. To the reaction solution were further added pent-4-enoyl chloride (0.092 mL, 0.828 mmol) and sodium carbonate (101 mg, 0.952 mmol) at room temperature, and the mixture was stirred for 15 h. To the reaction solution was added formic acid (0.159 mL), the tetrahydrofuran (THF) was removed by concentration under reduced pressure, and the residue was then diluted with dimethyl sulfoxide (DMSO). The resulting solution was purified by reverse phase column chromatography (0.1% aqueous formic acid solution/0.1% formic acid-acetonitrile solution) to afford (S)-2-(pent-4-enamido)-5-phenylpentanoic acid (Compound pc173, Pen-Phe{#(CH2)2}-OH) (67 mg, 59%).
LCMS (ESI) m/z = 276 (M+H)+

Retention time: 0.67 min (analytical condition SQDFA05)

Synthesis of cyanomethyl (S)-2-(pent-4-enamido)-5-phenylpentanoate (Compound pc174, Pen-Phe{#(CH2)2}-$OCH_2CN$)

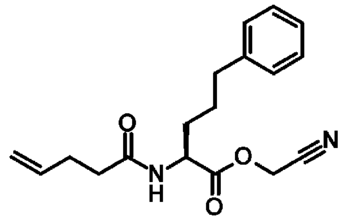

To a solution of (S)-2-(pent-4-enamido)-5-phenylpentanoic acid (Compound pc173, Pen-Phe{#(CH2)2}-OH) (67 mg, 0.243 mmol) in acetonitrile (0.5 mL) were added N,N-diisopropylethylamine (DIPEA) (0.127 mL, 0.730 mmol) and 2-bromoacetonitrile (0.102 mL, 1.460 mmol) under a nitrogen atmosphere, and the mixture was stirred at room temperature for 3 h. The reaction solution was concentrated and the resulting residue was purified by normal phase silica gel column chromatography (hexane/ethyl acetate) to afford cyanomethyl (S)-2-(pent-4-enamido)-5-phenylpentanoate (Compound pc174, Pen-Phe{#(CH2)2}-$OCH_2CN$) (67 mg, 88%).
LCMS (ESI) m/z = 315 (M+H)+
Retention time: 0.77 min (analytical condition SQDFA05)

Synthesis of (2R,3S,4R,5R)-2-((((((2R,3R,4R,5R)-5-(4-amino-2-oxopyrimidin-1(2H)-yl)-2-((phosphonooxy)methyl)-4-((tetrahydrofuran-2-yl)oxy)tetrahydrofuran-3-yl)oxy)(hydroxy)phosphoryl)oxy)methyl)-5-(6-amino-9H-purin-9-yl)-4-hydroxytetrahydrofuran-3-yl (2S)-2-(pent-4-enamido)-5-phenylpentanoate (pc175, Pen-Phe{#(CH2)2}-pCpA)

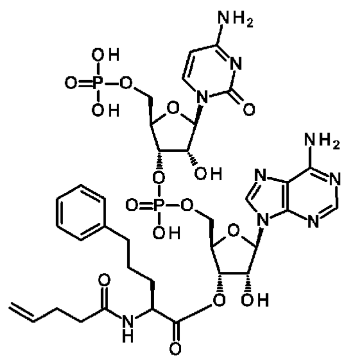

To buffer A (12 mL) were added a solution of ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (19.0 mg, 0.026 mmol) in water (1.2 mL) and a solution of cyanomethyl (S)-2-(pent-4-enamido)-5-phenylpentanoate (Compound pc174, Pen-Phe{#(CH2)2}-$OCH_2CN$) (33 mg, 0.105 mmol) in acetonitrile (0.6 mL), and the mixture was stirred at room temperature for 4 hours and 30 minutes. The reaction solution was lyophilized and a 80% aqueous acetic acid solution (3.0 mL) was added to the resulting residue at room temperature, and the mixture was stirred for 5 h. The reaction solution was diluted with water and purified by reverse phase column chromatography (0.05% aqueous trifluoroacetic acid solution/0.05% trifluoroacetic acid-acetonitrile solution) to afford the title compound (Compound pc175, Pen-Phe{#(CH2)2}-pCpA) (8.5 mg, 36% over two steps).
LCMS (ESI) m/z = 910 (M+H)+
Retention time: 0.49 min (analytical condition SQDFA05)

Synthesis of (S)-2-(methylamino)-5-phenylpentanoic acid (Compound pc176, H-MePhe{#(CH2)2}-OH)

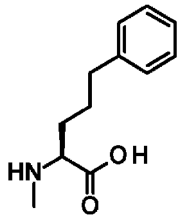

To a solution of (S)-2-((((9H-fluoren-9-yl)methoxy)carbonyl)(methyl)amino)-5-phenylpentanoic acid (Compound aa82, Fmoc-MePhe{#(CH2)2}-OH) (488 mg, 1.136 mmol) in dichloromethane (DCM) (2.2 mL) was added 4-(3-phenylpropyl)piperidine (0.481 mL, 2.272 mmol) at room temperature under a nitrogen atmosphere, and the mixture was stirred for 20 h. To the reaction solution was added dichloromethane (DCM), the mixture was filtered, and the resulting solid was then washed with dichloromethane (DCM) and hexane to afford (S)-2-(methylamino)-5-phenylpentanoic acid (Compound pc176, H-MePhe{#(CH2)2}-OH) (213 mg, 90%) as a white solid.
LCMS (ESI) m/z = 208 (M+H)+
Retention time: 0.38 min (analytical condition SQDFA05)

Synthesis of (S)-2-(N-methylpent-4-enamido)-5-phenylpentanoic acid (Compound pc177, Pen-MePhe{#(CH2)2}-OH)

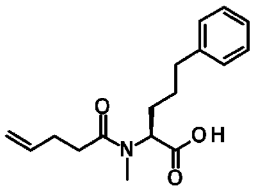

To a solution of (S)-2-(methylamino)-5-phenylpentanoic acid (Compound pc176, H-MePhe{#(CH2)2}-OH) (213 mg, 1.028 mmol) in water (5.0 mL) was added sodium carbonate (251 mg, 2.364 mmol) at room temperature under a nitrogen atmosphere, and the reaction solution was stirred until it became clear. To the reaction solution was added a solution of pent-4-enoyl chloride (0.228 mL, 2.055 mmol) in tetrahydrofuran (THF) (5.0 mL) dropwise at room temperature, and the mixture was stirred for 4 hours and 20 minutes. To the reaction solution were further added pent-4-enoyl chloride (0.228 mL, 2.055 mmol) and sodium carbonate (251 mg, 2.364 mmol) at room temperature, and the mixture was stirred for 40 min. To the reaction solution was added formic acid (0.394 mL), the tetrahydrofuran (THF) was removed by concentration under reduced pressure, and the residue was then diluted with dimethyl sulfoxide (DMSO). The resulting solution was purified by reverse phase column chromatography (0.1% aqueous formic acid solution/0.1% formic acid-acetonitrile solution) to afford (S)-2-(N-methylpent-4-enamido)-5-phenylpentanoic acid (Compound pc177, Pen-MePhe{#(CH2)2}-OH) (250 mg, 84%).
LCMS (ESI) m/z = 290 (M+H)+
Retention time: 0.74 min (analytical condition SQDFA05)

Synthesis of cyanomethyl (S)-2-(N-methylpent-4-enamido)-5-phenylpentanoate (Compound pc178, Pen-MePhe{#(CH2)2}-$OCH_2CN$)

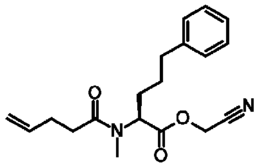

To a solution of (S)-2-(N-methylpent-4-enamido)-5-phenylpentanoic acid (Compound pc177, Pen-MePhe{#(CH2)2}-OH) (250 mg, 0.864 mmol) in acetonitrile (2.0 mL) were added N,N-diisopropylethylamine (DIPEA) (0.453 mL, 2.59 mmol) and 2-bromoacetonitrile (0.361 mL, 5.18 mmol) under a nitrogen atmosphere, and the mixture was stirred at room temperature for 5 h. The reaction solution was concentrated and the resulting residue was purified by normal phase silica gel column chromatography (hexane/ethyl acetate) to afford cyanomethyl (S)-2-(N-methylpent-4-enamido)-5-phenylpentanoate (Compound pc178, Pen-MePhe{#(CH2)2}-$OCH_2CN$) (252 mg, 89%).
LCMS (ESI) m/z = 329 (M+H)+
Retention time: 0.84 min (analytical condition SQDFA05)

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl (2S)-2-(N-methylpent-4-enamido)-5-phenylpentanoate (Compound pc179, Pen-MePhe{#(CH2)2}-pCpA)

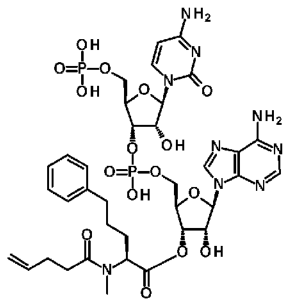

To buffer A (25 mL) were added a solution of ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (50.0 mg, 0.069 mmol) in water (2.5 mL) and a solution of cyanomethyl (S)-2-(N-methylpent-4-enamido)-5-phenylpentanoate (Compound pc178, Pen-MePhe{#(CH2)2}-$OCH_2CN$) (91 mg, 0.277 mmol) in acetonitrile (1.5 mL), and the mixture was stirred at room temperature for 3 h. The reaction solution was lyophilized and a 80% aqueous acetic acid solution (8.0 mL) was added to the resulting residue at room temperature, and the mixture was stirred for 7 hours and 30 minutes. The reaction solution was diluted with water and purified by reverse phase column chromatography (0.05% aqueous trifluoroacetic acid solution/0.05% trifluoroacetic acid-acetonitrile solution) to afford the title compound (Compound pc179, Pen-MePhe{#(CH2)2}-pCpA) (30 mg, 47% over two steps).
LCMS (ESI) m/z = 924 (M+H)+
Retention time: 0.53 min (analytical condition SQDFA05)

Synthesis of O-benzyl-L-serine (Compound pc180, H-Ser(Bn)-OH)

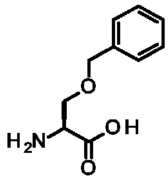

To a solution of commercially available N-(((9H-fluoren-9-yl)methoxy)carbonyl)-O-benzyl-L-serine (Fmoc-Ser(Bn)-OH, purchased from Watanabe Chemical Industries) (490 mg, 1.174 mmol) in dichloromethane (DCM) (2.4 mL) was added 4-(3-phenylpropyl)piperidine (0.497 mL, 2.348 mmol) at room temperature under a nitrogen atmosphere, and the mixture was stirred for 18 h. The reaction solution was diluted with dichloromethane (DCM) and filtered, after which the resulting solid was washed with dichloromethane (DCM) and hexane to afford O-benzyl-L-serine (Compound pc180, H-Ser(Bn)-OH) (211 mg, 92%).
LCMS (ESI) m/z = 196 (M+H)+
Retention time: 0.31 min (analytical condition SQDFA05)

Synthesis of O-benzyl-N-(pent-4-enoyl)-L-serine (Compound pc181, Pen-Ser(Bn)-OH)

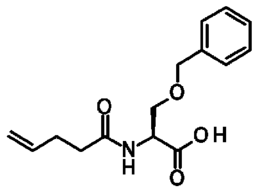

To a solution of O-benzyl-L-serine (Compound pc180, H-Ser(Bn)-OH) (211 mg, 1.081 mmol) in water (5.0 mL) was added sodium carbonate (263 mg, 2.486 mmol) at room temperature under a nitrogen atmosphere, and the reaction solution was stirred until it became clear. To the reaction solution was added a solution of pent-4-enoyl chloride (0.240 mL, 2.162 mmol) in tetrahydrofuran (THF) (5.0 mL) dropwise at room temperature, and the mixture was stirred for 8 h. To the reaction solution were further added pent-4-enoyl chloride (0.240 mL, 2.162 mmol) and sodium carbonate (263 mg, 2.486 mmol) at room temperature, and the mixture was stirred for 15 h. To the reaction solution was added formic acid (0.415 mL), the tetrahydrofuran (THF) was removed by concentration under reduced pressure, and the residue was then diluted with dimethyl sulfoxide (DMSO). The resulting solution was purified by reverse phase column chromatography (0.1% aqueous formic acid solution/0.1% formic acid-acetonitrile solution) to afford O-benzyl-N-(pent-4-enoyl)-L-serine (Compound pc181, Pen-Ser(Bn)-OH) (237 mg, 79%).
LCMS (ESI) m/z = 278 (M+H)+
Retention time: 0.60 min (analytical condition SQDFA05)

Synthesis of cyanomethyl O-benzyl-N-(pent-4-enoyl)-L-serinate (Compound pc182, Pen-Ser(Bn)-$OCH_2CN$)

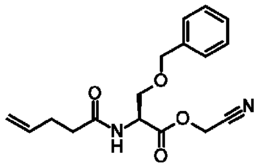

To a solution of O-benzyl-N-(pent-4-enoyl)-L-serine (Compound pc181, Pen-Ser(Bn)-OH) (237 mg, 0.855 mmol) in acetonitrile (2.0 mL) were added N,N-diisopropylethylamine (DIPEA) (0.448 mL, 2.56 mmol) and 2-bromoacetonitrile (0.357 mL, 5.13 mmol) under a nitrogen atmosphere, and the mixture was stirred at room temperature for 3 h. The reaction solution was concentrated and the resulting residue was purified by normal phase silica gel column chromatography (hexane/ethyl acetate) to afford cyanomethyl O-benzyl-N-(pent-4-enoyl)-L-serinate (Compound pc182, Pen-Ser(Bn)-$OCH_2CN$) (252 mg, 93%).
LCMS (ESI) m/z = 317 (M+H)+
Retention time: 0.72 min (analytical condition SQDFA05)

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl O-benzyl-N-(pent-4-enoyl)-L-serinate (Compound pc183, Pen-Ser(Bn)-pCpA)

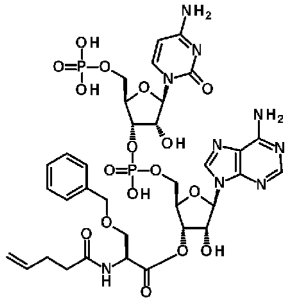

To buffer A (22 mL) were added a solution of ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (45.7 mg, 0.063 mmol) in water (2.2 mL) and a solution of cyanomethyl O-benzyl-N-(pent-4-enoyl)-L-serinate (Compound pc182, Pen-Ser(Bn)-$OCH_2CN$) (80 mg, 0.253 mmol) in acetonitrile (1.1 mL), and the mixture was stirred at room temperature for 4 hours and 30 minutes. The reaction solution was lyophilized and a 80% aqueous acetic acid solution (5.0 mL) was added to the resulting residue at room temperature, and the mixture was stirred for 5 h. The reaction solution was diluted with water and purified by reverse phase column chromatography (0.05% aqueous trifluoroacetic acid solution/0.05% trifluoroacetic acid-acetonitrile solution) to afford the title compound (Compound pc183, Pen-Ser(Bn)-pCpA) (19.5 mg, 34% over two steps).
LCMS (ESI) m/z = 912 (M+H)+
Retention time: 0.63 min (analytical condition SMD method 24)

Synthesis of O-benzyl-N-methyl-L-serine (Compound pc184, H-MeSer(Bn)-OH)

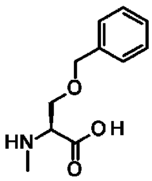

To a solution of N-(((9H-fluoren-9-yl)methoxy)carbonyl)-O-benzyl-N-methyl-L-serine (Compound aa83, Fmoc-MeSer(Bn)-OH) (425 mg, 0.985 mmol) in dichloromethane (DCM) (2.0 mL) was added 4-(3-phenylpropyl)piperidine (0.417 mL, 1.970 mmol) at room temperature under a nitrogen atmosphere, and the mixture was stirred for 20 h. To the reaction solution was added dichloromethane (DCM), the mixture was filtered, and the resulting solid was then washed with dichloromethane (DCM) and hexane to afford O-benzyl-N-methyl-L-serine (Compound pc184, H-MeSer(Bn)-OH) (183 mg, 89%) as a white solid.
LCMS (ESI) m/z = 210 (M+H)+
Retention time: 0.33 min (analytical condition SQDFA05)

Synthesis of O-benzyl-N-methyl-N-(pent-4-enoyl)-L-serine (Compound pc185, Pen-MeSer(Bn)-OH)

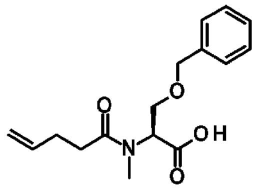

To a solution of O-benzyl-N-methyl-L-serine (Compound pc184, H-MeSer(Bn)-OH) (183 mg, 0.875 mmol) in water (5.0 mL) was added sodium carbonate (213 mg, 2.012 mmol) at room temperature under a nitrogen atmosphere,

and the reaction solution was stirred until it became clear. To the reaction solution was added a solution of pent-4-enoyl chloride (0.194 mL, 1.749 mmol) in tetrahydrofuran (THF) (5.0 mL) dropwise at room temperature, and the mixture was stirred for 8 hours and 30 minutes. To the reaction solution were further added pent-4-enoyl chloride (0.194 mL, 1.749 mmol) and sodium carbonate (213 mg, 2.012 mmol) at room temperature, and the mixture was stirred for 1 hour and 40 minutes. To the reaction solution was added formic acid (0.335 mL), the tetrahydrofuran (THF) was removed by concentration under reduced pressure, and the residue was then diluted with dimethyl sulfoxide (DMSO). The resulting solution was purified by reverse phase column chromatography (0.1% aqueous formic acid solution/0.1% formic acid-acetonitrile solution) to afford O-benzyl-N-methyl-N-(pent-4-enoyl)-L-serine (Compound pc185, Pen-MeSer(Bn)-OH) (229 mg, 90%).
LCMS (ESI) m/z = 292 (M+H)+
Retention time: 0.66 min (analytical condition SQDFA05)

Synthesis of cyanomethyl O-benzyl-N-methyl-N-(pent-4-enoyl)-L-serinate (Compound pc186, Pen-MeSer(Bn)-$OCH_2CN$)

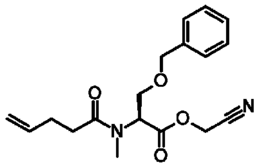

To a solution of O-benzyl-N-methyl-N-(pent-4-enoyl)-L-serine (Compound pc185, Pen-MeSer(Bn)-OH) (229 mg, 0.786 mmol) in acetonitrile (2.0 mL) were added N,N-diisopropylethylamine (DIPEA) (0.412 mL, 2.358 mmol) and 2-bromoacetonitrile (0.329 mL, 4.72 mmol) under a nitrogen atmosphere, and the mixture was stirred at room temperature for 5 h. The reaction solution was concentrated and the resulting residue was purified by normal phase silica gel column chromatography (hexane/ethyl acetate) to afford cyanomethyl O-benzyl-N-methyl-N-(pent-4-enoyl)-L-serinate (Compound pc186, Pen-MeSer(Bn)-$OCH_2CN$) (252 mg, 97%).
LCMS (ESI) m/z = 331 (M+H)+
Retention time: 0.78 min (analytical condition SQDFA05)

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl O-benzyl-N-methyl-N-(pent-4-enoyl)-L-serinate (Compound pc187, Pen-MeSer(Bn)-pCpA)

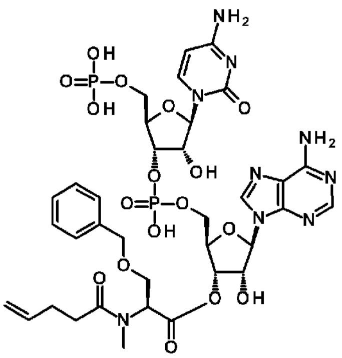

To buffer A (25 mL) were added a solution of ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (50.0 mg, 0.069 mmol) in water (2.5 mL) and a solution of cyanomethyl O-benzyl-N-methyl-N-(pent-4-enoyl)-L-serinate (Compound pc186, Pen-MeSer(Bn)-$OCH_2CN$) (91 mg, 0.277 mmol) in acetonitrile (1.5 mL), and the mixture was stirred at room temperature for 3 h. The reaction solution was lyophilized and a 80% aqueous acetic acid solution (8.0 mL) was added

to the resulting residue at room temperature, and the mixture was stirred for 7 hours and 30 minutes. The reaction solution was diluted with water and purified by reverse phase column chromatography (0.05% aqueous trifluoroacetic acid solution/0.05% trifluoroacetic acid-acetonitrile solution) to afford the title compound (Compound pc187, Pen-Me-Ser(Bn)-pCpA) (26.3 mg, 41% over two steps).
LCMS (ESI) m/z = 926 (M+H)+
Retention time: 0.48 min (analytical condition SQDFA05)

Synthesis of (2S,4R)-4-ethoxy-1-(pent-4-enoyl)pyrrolidine-2-carboxylic acid (Compound pc188, Pen-Hyp(Et)-OH)

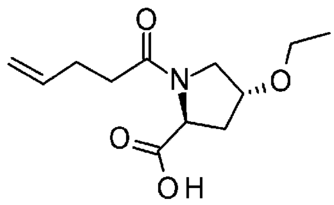

A solution of (2S,4R)-1-[(tert-butoxy)carbonyl]-4-ethoxypyrrolidine-2-carboxylic acid (Boc-Hyp(Et)-OH, CAS No. 146060-18-6) (5 g, 19.28 mmol) in dichloromethane (DCM) (50 mL) was stirred at a temperature of 20°C while bubbling with hydrogen chloride gas. After 4 hours, the solvent was evaporated from the reaction solution under reduced pressure to afford (2S,4R)-4-ethoxypyrrolidine-2-carboxylic acid hydrochloride (H-Hyp(Et)-OH·HCl) (3.8 g) as a crude product.

To a solution of the above crude product (2S,4R)-4-ethoxypyrrolidine-2-carboxylic acid hydrochloride (H-Hyp(Et)-OH·HCl) (2.1 g, 10.7 mmol) in water (20 mL) were added sodium bicarbonate (2.50 g, 29.8 mmol) and a solution of 2,5-dioxopyrrolidin-1-yl pent-4-enoate (2.30 g, 11.7 mmol) in 1,4-dioxane (20 mL), and the mixture was stirred at 20°C for 3 h. The pH was adjusted to 2 with an aqueous hydrochloric acid solution (1 mol/L), followed by extraction with ethyl acetate. The organic layer was washed with brine and then dried over anhydrous sodium sulfate, and the solvent was evaporated under reduced pressure to afford (2S,4R)-4-ethoxy-1-(pent-4-enoyl)pyrrolidine-2-carboxylic acid (Pen-Hyp(Et)-OH) (Compound pc188, 2.1 g) as a crude product.
LCMS (ESI) m/z = 242 (M+H)+
Retention time: 1.40 min (analytical condition SMD method 5)

Synthesis of cyanomethyl (2S,4R)-4-ethoxy-1-(pent-4-enoyl)pyrrolidine-2-carboxylate (Compound pc189, Pen-Hyp(Et)-$OCH_2CN$)

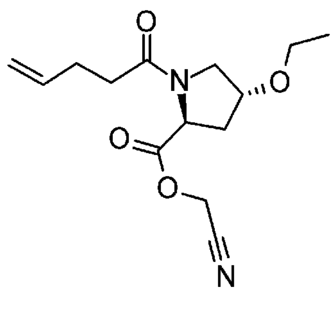

To a solution of (2S,4R)-4-ethoxy-1-(pent-4-enoyl)pyrrolidine-2-carboxylic acid (Pen-Hyp(Et)-OH) (Compound pc188, 1.50 g, 6.22 mmol) in dichloromethane (DCM) (20 mL) were added N,N-diisopropylethylamine (DIPEA) (2.00 g, 15.5 mmol) and 2-bromoacetonitrile (4.00 g, 33.4 mmol), and the mixture was stirred at 20°C. After 2 hours, the solvent was evaporated from the reaction solution under reduced pressure. The residue was purified by normal phase silica gel column chromatography (petroleum ether/ethyl acetate) to afford cyanomethyl (2S,4R)-4-ethoxy-1-(pent-4-enoyl)pyrrolidine-2-carboxylate (Compound pc189, Pen-Hyp(Et)-$OCH_2CN$) (662 mg, 38%).
LCMS (ESI) m/z = 281 (M+H)+
Retention time: 1.82 min (analytical condition ELSD2)

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl (2S,4R)-4-ethoxy-1-(pent-4-enoyl)pyrrolidine-2-carboxylate (Compound pc190, Pen-Hyp(Et)-pCpA)

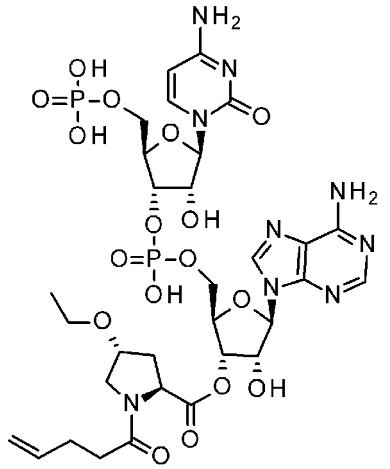

To buffer A (100 mL) were added a solution of ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (250 mg, 0.346 mmol) in water (3.00 mL) and a solution of cyanomethyl (2S,4R)-4-ethoxy-1-(pent-4-enoyl)pyrrolidine-2-carboxylate (Compound pc189, Pen-Hyp(Et)-$OCH_2CN$) (388 mg, 1.38 mmol) in acetonitrile (1.5 mL), and the mixture was stirred at room temperature for 2 h. The reaction solution was lyophilized and a 80% aqueous acetic acid solution (0.6 mL) was added to the resulting residue, and the mixture was stirred for 6 h. The reaction solution was purified by reverse phase column chromatography (0.05% aqueous trifluoroacetic acid solution/0.05% trifluoroacetic acid-acetonitrile solution) to afford the title compound (Compound pc190, Pen-Hyp(Et)-pCpA) (40.8 mg, 13%).
LCMS (ESI) m/z = 874 (M-H)-
Retention time: 0.39 min (analytical condition SQDFA05)

Synthesis of (S)-2-amino-4-(4,4-difluoropiperidin-1-yl)butanoic acid (Compound pc191, H-Abu(pip-4-F2)-OH)

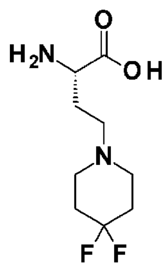

(S)-2-((((9H-Fluoren-9-yl)methoxy)carbonyl)amino)-4-(4,4-difluoropiperidin-1-yl)butanoic acid (Compound aa109, Fmoc-Abu(pip-4-F2)-OH) (300 mg, 0.675 mmol) was dissolved in dichloromethane (1.5 mL), after which 4-(3-phenylpropyl)piperidine (286 μL, 1.35 mmol) was added and the mixture was stirred at room temperature for 3 h. The resulting reaction solution was directly purified by reverse phase column chromatography (0.1% aqueous formic acid solution/0.1% formic acid-acetonitrile solution) to afford (S)-2-amino-4-(4,4-difluoropiperidin-1-yl)butanoic acid (Compound pc191, H-Abu(pip-4-F2)-OH) (150 mg) quantitatively.
LCMS (ESI) m/z = 223 (M+H)+
Retention time: 0.16 min (analytical condition SQDFA05)

Synthesis of (S)-4-(4,4-difluoropiperidin-1-yl)-2-((((4-(2-(4-fluorophenyl)acetamido)benzyl)oxy)carbonyl)amino)butanoic acid (Compound pc192, F-Pnaz-Abu(pip-4-F2)-OH)

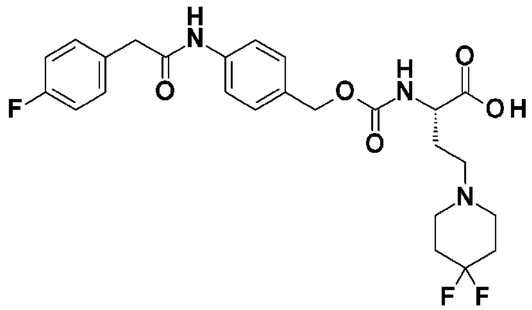

To (S)-2-amino-4-(4,4-difluoropiperidin-1-yl)butanoic acid (Compound pc191, H-Abu(pip-4-F2)-OH) (80 mg, 0.36 mmol) dissolved in dimethyl sulfoxide (3 mL) was added separately prepared 4-(2-(4-fluorophenyl)acetamido)benzyl (4-nitrophenyl) carbonate (Compound pc196) (153 mg, 0.36 mmol), and the mixture was stirred at room temperature for 18 h. The resulting reaction solution was directly purified by reverse phase column chromatography (0.1% aqueous formic acid solution/0.1% formic acid-acetonitrile solution) to afford (S)-4-(4,4-difluoropiperidin-1-yl)-2-((((4-(2-(4-fluorophenyl)acetamido)benzyl)oxy)carbonyl)amino)butanoic acid (Compound pc192, F-Pnaz-Abu(pip-4-F2)-OH) (90 mg, 49%).
LCMS (ESI) m/z = 508 (M+H)+
Retention time: 0.52 min (analytical condition SQDFA05)

Synthesis of cyanomethyl (S)-4-(4,4-difluoropiperidin-1-yl)-2-((((4-(2-(4-fluorophenyl)acetamido)benzyl)oxy)carbonyl)amino)butanoate (Compound pc193, F-Pnaz-Abu(pip-4-F2)-$OCH_2CN$)

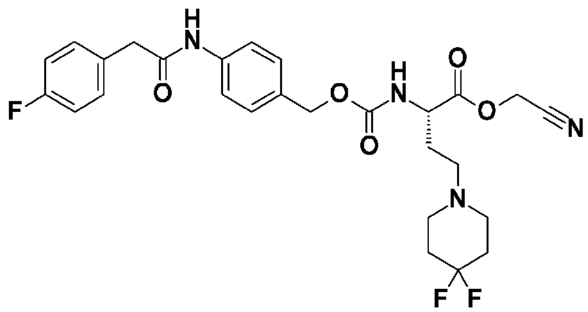

(S)-4-(4,4-Difluoropiperidin-1-yl)-2-((((4-(2-(4-fluorophenyl)acetamido)benzyl)oxy)carbonyl)amino)butanoic acid (Compound pc192, F-Pnaz-Abu(pip-4-F2)-OH) (30 mg, 0.059 mmol) was dissolved in acetonitrile (500 μL) under a nitrogen atmosphere, 2-bromoacetonitrile (7.97 μL, 0.118 mmol) and N,N-diisopropylethylamine (DIPEA) (31 μL, 0.177 mmol) were added thereto at 0°C, and the mixture was stirred for 3 h. The reaction solution was concentrated under reduced pressure, and the resulting crude product was purified by normal phase silica gel chromatography (hexane/ethyl acetate) to afford cyanomethyl (S)-4-(4,4-difluoropiperidin-1-yl)-2-((((4-(2-(4-fluorophenyl)acetamido)benzyl)oxy)carbonyl)amino)butanoate (Compound pc193, F-Pnaz-Abu(pip-4-F2)-$OCH_2CN$) (18.3 mg, 57%).
LCMS (ESI) m/z = 547 $(M+H)^+$
Retention time: 0.56 min (analytical condition SQDFA05)

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl (2S)-4-(4,4-difluoropiperidin-1-yl)-2-((((4-(2-(4-fluorophenyl)acetamido)benzyl)oxy)carbonyl)amino)butanoate (Compound pc194, F-Pnaz-Abu(pip-4-F2)-pCpA)

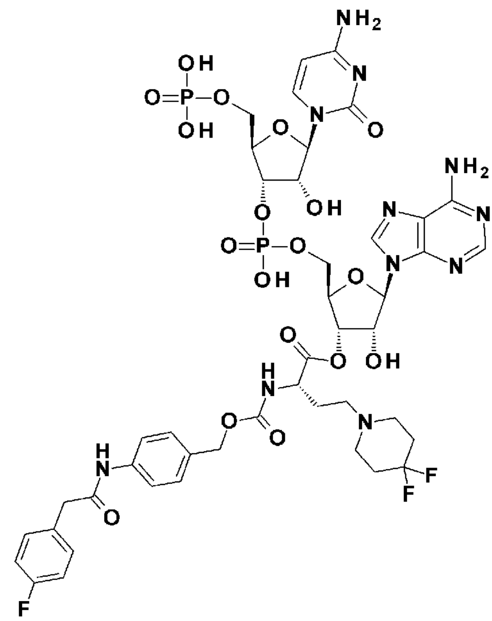

To buffer A (8.34 mL) were added a solution of ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (9.91 mg, 0.014 mmol) in water (0.2 mL) and a solution of cyanomethyl (S)-4-(4,4-difluoropiperidin-1-yl)-2-((((4-(2-(4-fluorophenyl)acetamido)benzyl)oxy)carbonyl)amino)butanoate (Compound pc193, F-Pnaz-Abu(pip-4-F2)-$OCH_2CN$) (15 mg, 0.027 mmol) in acetonitrile (417 μL), and the mixture was stirred at room temperature for 2 h. To the reaction solution was added trifluoroacetic acid (416 μL) at room temperature, and the mixture was stirred for 30 min. The reaction solution was diluted with water and purified by reverse phase column chromatography (0.05% aqueous trifluoroacetic acid solution/0.05% trifluoroacetic acid-acetonitrile solution) to afford the title compound (Compound pc194, F-Pnaz-Abu(pip-4-F2)-pCpA) (3.41 mg, 22%).
LCMS (ESI) m/z = 1143 (M+H)+
Retention time: 0.47 min (analytical condition SQDFA05)

Synthesis of 2-(4-fluorophenyl)-N-(4-(hydroxymethyl)phenyl)acetamide (Compound pc195)

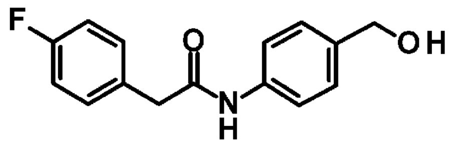

To a mixture of 2-(4-fluorophenyl)acetic acid (300 mg, 1.95 mmol), 4-aminophenylmethanol (264 mg, 2.14 mmol), and DMT-MM (646 mg, 2.34 mmol) was added tetrahydrofuran (9.7 mL) under a nitrogen atmosphere, and the mixture was then stirred at 40°C for 2 h. The reaction solution was purified by reverse phase column chromatography (0.1% aqueous formic acid solution/0.1% formic acid-acetonitrile solution) to afford 2-(4-fluorophenyl)-N-(4-(hydroxymethyl)phenyl)acetamide (Compound pc195) (400.0 mg, 79.0%).
LCMS (ESI) m/z = 260 (M+H)+
Retention time: 0.55 min (analytical condition SQDFA05)

Synthesis of 4-(2-(4-fluorophenyl)acetamido)benzyl (4-nitrophenyl) carbonate (Compound pc196)

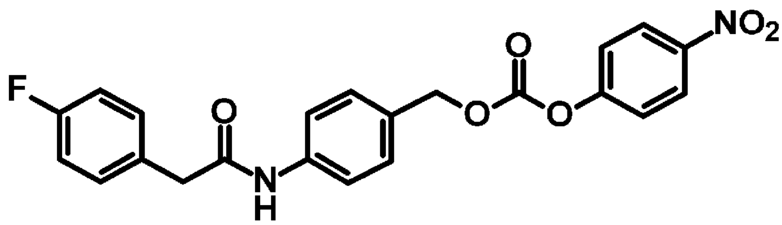

2-(4-Fluorophenyl)-N-(4-(hydroxymethyl)phenyl)acetamide (Compound pc195) (300 mg, 1.16 mmol) was dissolved in tetrahydrofuran (1.00 mL) under a nitrogen atmosphere, pyridine (0.187 mL, 2.31 mmol) was added, and the reaction solution was cooled to 0°C. To the reaction solution was slowly added a solution of 4-nitrophenyl chloroformate (233 mg, 1.16 mmol) in tetrahydrofuran (1.13 mL) dropwise at 0°C, and the mixture was then stirred at room temperature for 2 h. The reaction solution was concentrated, and the resulting residue was washed with hexane-ethyl acetate 3:1 to afford 4-(2-(4-fluorophenyl)acetamido)benzyl (4-nitrophenyl) carbonate (Compound pc196) (330.0 mg, 67%).
LCMS (ESI) m/z = 425 (M+H)+
Retention time: 0.84 min (analytical condition SQDFA05)

Synthesis of (2S)-4-(4,4-difluoropiperidin-1-yl)-2-(N-methylpent-4-enamido)butanoic acid (Compound pc197, Pen-MeAbu(pip-4-F2)-OH)

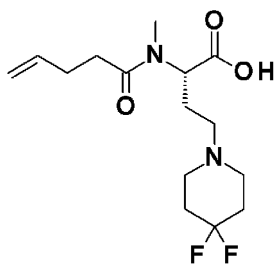

(S)-2-((((9H-Fluoren-9-yl)methoxy)carbonyl)(methyl)amino)-4-(4,4-difluoropiperidin-1-yl)butanoic acid (Compound aa110, Fmoc-MeAbu(pip-4-F2)-OH) (1.4 g, 3.05 mmol) was dissolved in N,N-dimethylformamide (7 mL), piperidine (2.8 mL) was added, and the mixture was stirred at room temperature for 2 h. To the resulting reaction solution was added diethyl ether (14 mL), and the mixture was stirred at room temperature for 30 min and then filtered. A solution of the resulting solid, 2,5-dioxopyrrolidin-1-yl pent-4-enoate (1.4 g, 7.10 mmol), and sodium carbonate (590 mg, 7.02 mmol) in water/1,4-dioxane (1/1, 20 mL) was stirred at room temperature for 16 h. The reaction solution was washed with diethyl ether three times, and the aqueous layer was adjusted to pH 4 with a 5 N aqueous hydrochloric acid solution and then concentrated by lyophilization. The resulting residue was purified by normal phase silica gel chromatography (dichloromethane/methanol) to afford (2S)-4-(4,4-difluoropiperidin-1-yl)-2-(N-methylpent-4-enamido)butanoic acid (Compound pc197, Pen-MeAbu(pip-4-F2)-OH) (600 mg, 62% over two steps).
LCMS (ESI) m/z = 319 (M+H)+
Retention time: 0.137 min (analytical condition SMD method 16)

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl (2S)-4-(4,4-difluoropiperidin-1-yl)-2-(N-methylpent-4-enamido)butanoate (Compound pc199, Pen-MeAbu(pip-4-F2)-pCpA)

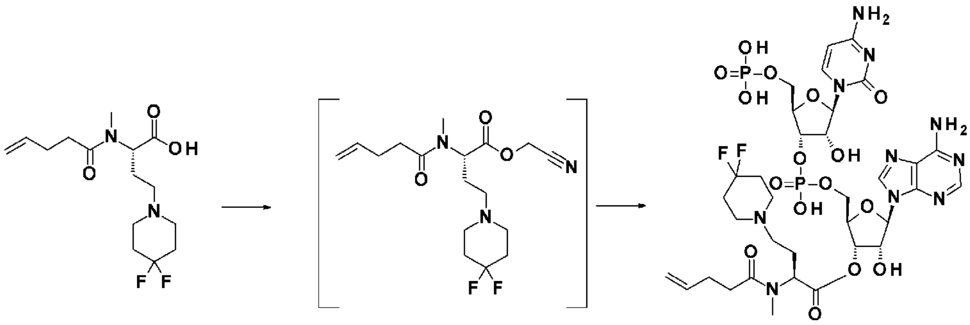

pc197 pc198 pc199

To a solution of (2S)-4-(4,4-difluoropiperidin-1-yl)-2-(N-methylpent-4-enamido)butanoic acid (Compound pc197, Pen-MeAbu(pip-4-F2)-OH) (600 mg, 1.88 mmol) in dichloromethane (20 mL) were added N,N-diisopropylethylamine (DIPEA) (730 mg, 5.65 mmol) and 2-bromoacetonitrile (898 mg, 7.49 mmol) under a nitrogen atmosphere, and the mixture was stirred at room temperature for 16 h. The reaction solution was concentrated and the resulting residue was purified by normal phase silica gel column chromatography (petroleum ether/ethyl acetate) to afford cyanomethyl (2S)-4-(4,4-difluoropiperidin-1-yl)-2-(N-methylpent-4-enamido)butanoate (Compound pc198, Pen-MeAbu(pip-4-F2)-$OCH_2CN$) (280 mg, 42%).

To buffer A (75 mL) were added ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (300 mg, 0.42 mmol) and a solution of cyanomethyl (2S)-4-(4,4-difluoropiperidin-1-yl)-2-(N-methylpent-4-enamido)butanoate (Compound pc198, Pen-MeAbu(pip-4-F2)-$OCH_2CN$) (300 mg, 0.84 mmol) in acetonitrile (2 mL), and the mixture was stirred at room temperature for 90 min. To the reaction solution was added trifluoroacetic acid (1.6 mL), and the reaction solution was lyophilized. The resulting residue was purified by reverse phase column chromatography (0.05% aqueous trifluoroacetic acid solution/0.05% trifluoroacetic acid-acetonitrile solution) to afford the title compound (Compound pc199, Pen-MeAbu(pip-4-F2)-pCpA) (21 mg, 3% over two steps).

LCMS (ESI) m/z = 477 (M+2H)2+

Retention time: 0.749 min (analytical condition SMD method 29)

<u>Synthesis of (2S)-4-(3,3-difluoropiperidin-1-yl)-2-(N-methylpent-4-enamido)butanoic acid (Compound pc201, Pen-Abu(pip-3-F2)-OH)</u>

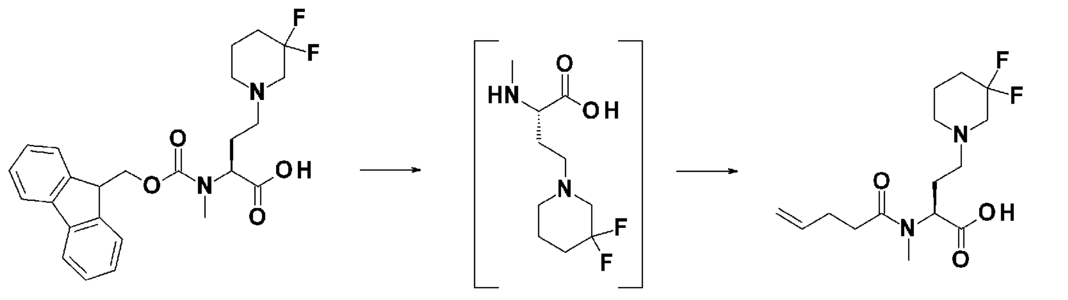

aa116 pc200 pc201

To a solution of (S)-2-((((9H-fluoren-9-yl)methoxy)carbonyl)(methyl)amino)-4-(3,3-difluoropiperidin-1-yl)butanoic acid (Compound aa116, Fmoc-MeAbu(pip-3-F2)-OH) (2 g, 4.36 mmol) in N,N-dimethylformamide (10 mL) was added piperidine (4 mL) at room temperature under a nitrogen atmosphere, and the mixture was stirred for 2 h. To the reaction solution was added diethyl ether (100 mL), the mixture was stirred for 30 min, and the resulting precipitate was filtered to afford (2S)-4-(3,3-difluoropiperidin-1-yl)-2-(methylamino)butanoic acid (Compound pc200, H-MeAbu(pip-3-F2)-OH) (979 mg, 95%).

A solution of (2S)-4-(3,3-difluoropiperidin-1-yl)-2-(methylamino)butanoic acid (Compound pc200, H-MeAbu(pip-3-F2)-OH) (1 g, 4.23 mmol), 2,5-dioxopyrrolidin-1-yl pent-4-enoate (2.1 g, 10.70 mmol), and sodium bicarbonate (1.43 g, 17.02 mmol) in water/1,4-dioxane (1/1, 40 mL) was stirred at room temperature for 16 h under a nitrogen

atmosphere. The reaction solution was washed with diethyl ether three times, and the aqueous layer was adjusted to pH 4 with a 5 N aqueous hydrochloric acid solution and lyophilized. The resulting residue was purified by normal phase silica gel chromatography (dichloromethane/methanol) to afford (2S)-4-(3,3-difluoropiperidin-1-yl)-2-(N-methylpent-4-enamido)butanoic acid (Compound pc201, Pen-MeAbu(pip-3-F2)-OH) (0.5 g).
LCMS (ESI) m/z = 319 (M+H)+
Retention time: 0.334 min (analytical condition SMD method 16)

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl (2S)-4-(3,3-difluoropiperidin-1-yl)-2-(N-methylpent-4-enamido)butanoate (Compound pc203, Pen-MeAbu(pip-3-F2)-pCpA)

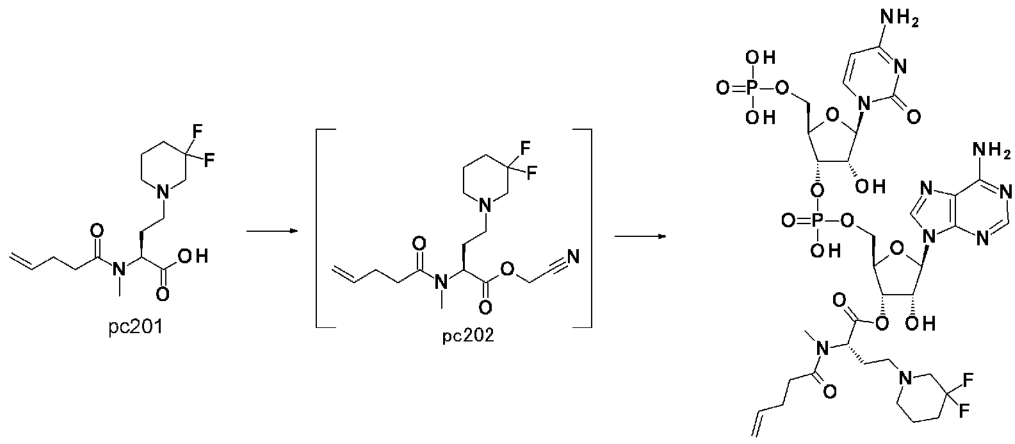
pc201 pc202 pc203

To a solution of (2S)-4-(3,3-difluoropiperidin-1-yl)-2-(N-methylpent-4-enamido)butanoic acid (Compound pc201, Pen-MeAbu(pip-3-F2)-OH) (500 mg, 1.57 mmol) in dichloromethane (15 mL) were added N,N-diisopropylethylamine (DIPEA) (610 mg) and 2-bromoacetonitrile (750 mg, 6.25 mmol) under a nitrogen atmosphere, and the mixture was stirred at room temperature for 16 h. The reaction solution was concentrated and the resulting residue was purified by normal phase silica gel column chromatography (petroleum ether/ethyl acetate) to afford cyanomethyl (2S)-4-(3,3-difluoropiperidin-l-yl)-2-(N-methylpent-4-enamido)butanoate (Compound pc202, Pen-MeAbu(pip-3-F2)-$OCH_2CN$) (380 mg).

To buffer A (75 mL) were added ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (350 mg, 0.48 mmol) and a solution of cyanomethyl (2S)-4-(3,3-difluoropiperidin-1-yl)-2-(N-methylpent-4-enamido)butanoate (Compound pc202, Pen-MeAbu(pip-3-F2)-$OCH_2CN$) (350 mg, 0.98 mmol) in acetonitrile (2 mL), and the mixture was stirred at room temperature for 2 h. To the reaction solution was added trifluoroacetic acid (1.6 mL) at room temperature, and the mixture was stirred for 30 min. The reaction solution was lyophilized, and the resulting residue was purified by reverse phase column chromatography (0.05% aqueous trifluoroacetic acid solution/0.05% trifluoroacetic acid-acetonitrile solution) to afford the title compound (Compound pc203, Pen-MeAbu(pip-3-F2)-pCpA) (25 mg, 3%).
LCMS (ESI) m/z = 477 (M+2H)2+, 953 (M+H)+
Retention time: 0.887 min, 0.993 min (analytical condition SMD method 34)

Synthesis of (S)-3-(6-(dimethylamino)pyridin-3-yl)-2-((((4-(2-(4-fluorophenyl)acetamido)benzyl)oxy)carbonyl)amino)propanoic acid (Compound pc204, F-Pnaz-Ala(3-Pyr-4-NMe2)-OH)

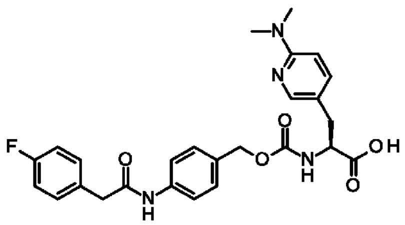

To a solution of (S)-2-((((9H-fluoren-9-yl)methoxy)carbonyl)amino)-3-(6-(dimethylamino)pyridin-3-yl)propanoic acid (Compound aa88, Fmoc-Ala(3-Pyr-4-NMe2)-OH) (500 mg, 1.159 mmol) in dichloromethane (5.0 mL) was added 4-(3-phenylpropyl)piperidine (0.491 mL, 2.318 mmol) at room temperature under a nitrogen atmosphere, and the mixture was stirred overnight. To the reaction solution were added t-butyl methyl ether/hexane = 1/4 and water, followed by washing with t-butyl methyl ether/hexane = 1/4. The resulting aqueous layer was purified by reverse phase column chromatography (0.1% aqueous formic acid solution/0.1% formic acid-acetonitrile solution) to afford (S)-2-amino-3-(6-(dimethylamino)pyridin-3-yl)propanoic acid (H-Ala(3-Pyr-4-NMe2)-OH) (260 mg) quantitatively.

To a solution of the above (S)-2-amino-3-(6-(dimethylamino)pyridin-3-yl)propanoic acid (H-Ala(3-Pyr-4-NMe2)-OH) (100 mg) in dimethylformamide (DMF) (500 μL) were added separately prepared 4-(2-(4-fluorophenyl)acetamido)benzyl (4-nitrophenyl) carbonate (Compound pc196) (243 mg, 0.573 mmol) and triethylamine ($Et_3N$) (153 μL, 1.099 mmol) at room temperature, and the mixture was stirred at 35°C for 5 h. To the reaction solution were added water and dimethyl sulfoxide (DMSO), and the mixture was purified by reverse phase chromatography (0.1% aqueous formic acid solution/0.1% formic acid-acetonitrile solution) and then purified again by reverse phase chromatography (10 mM aqueous ammonium acetate solution/methanol) to afford (S)-3-(6-(dimethylamino)pyridin-3-yl)-2-((((4-(2-(4-fluorophenyl)acetamido)benzyl)oxy)carbonyl)amino)propanoic acid (Compound pc204, F-Pnaz-Ala(3-Pyr-4-NMe2)-OH) (55 mg, 24% over two steps).

LCMS (ESI) m/z = 495 (M+H)+

Retention time: 0.74 min (analytical condition SQDAA05)

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl (2S)-3-(6-(dimethylamino)pyridin-3-yl)-2-((((4-(2-(4-fluorophenyl)acetamido)benzyl)oxy)carbonyl)amino)propanoate (Compound pc205, F-Pnaz-Ala(3-Pyr-4-NMe2)-pCpA)

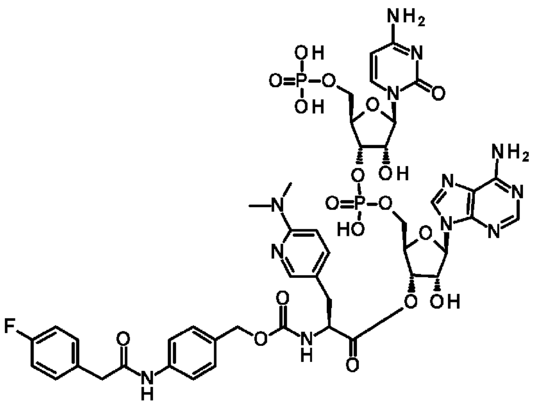

To a solution of (S)-3-(6-(dimethylamino)pyridin-3-yl)-2-((((4-(2-(4-fluorophenyl)acetamido)benzyl)oxy)carbonyl)amino)propanoic acid (Compound pc204, F-Pnaz-Ala(3-Pyr-4-NMe2)-OH) (20 mg, 0.040 mmol) in acetonitrile (0.4 mL) were added N,N-diisopropylethylamine (DIPEA) (21.19 μL, 0.121 mmol) and 2-bromoacetonitrile (16.9 μL, 0.243 mmol) under a nitrogen atmosphere, and the mixture was stirred at room temperature for 3 hours and 30 minutes. The reaction solution was concentrated to afford cyanomethyl (S)-3-(6-(dimethylamino)pyridin-3-yl)-2-((((4-(2-(4-fluorophenyl)acetamido)benzyl)oxy)carbonyl)amino)propanoate (F-Pnaz-Ala(3-Pyr-4-NMe2)-$OCH_2CN$) as a crude product.

To buffer A (20 mL) were added a solution of ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (14.9 mg, 0.021

mmol) in water (2.0 mL) and a solution of the above crude product cyanomethyl (S)-3-(6-(dimethylamino)pyridin-3-yl)-2-((((4-(2-(4-fluorophenyl)acetamido)benzyl)oxy)carbonyl)amino)propanoate (F-Pnaz-Ala(3-Pyr-4-NMe2)-$OCH_2CN$) in acetonitrile (1.0 mL), and the mixture was stirred at room temperature for 2 hours and 30 minutes. To the reaction solution was added trifluoroacetic acid (TFA) (0.4 mL) at 0°C and the mixture was stirred at 0°C for 40 min, after which the reaction solution was diluted with water and purified by reverse phase column chromatography (0.05% aqueous trifluoroacetic acid solution/0.05% trifluoroacetic acid-acetonitrile solution) to afford the title compound (Compound pc205, F-Pnaz-Ala(3-Pyr-4-NMe2)-pCpA) (6.7 mg, 29%).
LCMS (ESI) m/z = 1129 $(M+H)^+$
Retention time: 0.43 min (analytical condition SQDFA05)

Synthesis of (S)-3-(6-(dimethylamino)pyridin-3-yl)-2-((((4-(2-(4-fluorophenyl)acetamido)benzyl)oxy)carbonyl)(methyl)amino)propanoic acid (Compound pc206, F-Pnaz-MeAla(3-Pyr-4-NMe2)-OH)

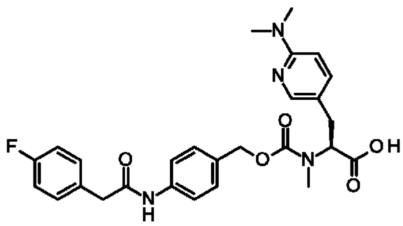

To a solution of (S)-2-((((9H-fluoren-9-yl)methoxy)carbonyl)(methyl)amino)-3-(6-(dimethylamino)pyridin-3-yl)propanoic acid (Compound aa186, Fmoc-MeAla(3-Pyr-4-NMe2)-OH) (181 mg, 0.406 mmol) in dichloromethane (1.8 mL) was added 4-(3-phenylpropyl)piperidine (0.172 mL, 0.813 mmol) at room temperature under a nitrogen atmosphere, and the mixture was stirred overnight. To the reaction solution were added t-butyl methyl ether/hexane = 1/4 and water, followed by washing with t-butyl methyl ether/hexane = 1/4. The resulting aqueous layer was purified by reverse phase column chromatography (0.1% aqueous formic acid solution/0.1% formic acid-acetonitrile solution) to afford (S)-3-(6-(dimethylamino)pyridin-3-yl)-2-(methylamino)propanoic acid (H-MeAla(3-Pyr-4-NMe2)-OH) (47 mg, 52%).

To a solution of the above (S)-3-(6-(dimethylamino)pyridin-3-yl)-2-(methylamino)propanoic acid (H-MeAla(3-Pyr-4-NMe2)-OH) (33 mg) in dimethylformamide (DMF) (200 μL) were added separately prepared 4-(2-(4-fluorophenyl)acetamido)benzyl (4-nitrophenyl) carbonate (Compound pc196) (75 mg, 0.177 mmol) and triethylamine ($Et_3N$) (47.4 μL, 0.340 mmol) at room temperature, and the mixture was stirred at 35°C for 7 h and then stirred at 55°C overnight. To the reaction solution was added formic acid (28.3 μL), after which the mixture was purified by reverse phase chromatography (0.1% aqueous formic acid solution/0.1% formic acid-acetonitrile solution) and then purified again by reverse phase chromatography (10 mM aqueous ammonium acetate solution/methanol) to afford (S)-3-(6-(dimethylamino)pyridin-3-yl)-2-((((4-(2-(4-fluorophenyl)acetamido)benzyl)oxy)carbonyl)(methyl)amino)propanoic acid (Compound pc206, F-Pnaz-MeAla(3-Pyr-4-NMe2)-OH) (19 mg, 25%).
LCMS (ESI) m/z = 509 (M+H)+
Retention time: 0.75 min (analytical condition SQDAA05)

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl (2S)-3-(6-(dimethylamino)pyridin-3-yl)-2-((((4-(2-(4-fluorophenyl)acetamido)benzyl)oxy)carbonyl)(methyl)amino)propanoate (Compound pc207, F-Pnaz-MeAla(3-Pyr-4-NMe2)-pCpA)

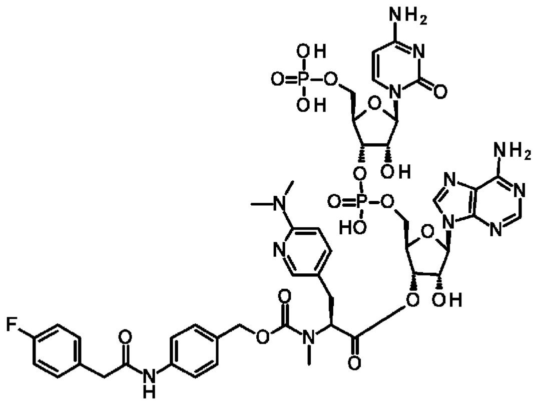

To a solution of (S)-3-(6-(dimethylamino)pyridin-3-yl)-2-((((4-(2-(4-fluorophenyl)acetamido)benzyl)oxy)carbonyl)(methyl)amino)propanoic acid (Compound pc206, F-Pnaz-MeAla(3-Pyr-4-NMe2)-OH) (19 mg, 0.037 mmol) in acetonitrile (0.4 mL) were added N,N-diisopropylethylamine (DIPEA) (19.58 μL, 0.112 mmol) and 2-bromoacetonitrile (15.61 μL, 0.224 mmol) under a nitrogen atmosphere, and the mixture was stirred at room temperature for 5 hours and 30 minutes. The reaction solution was concentrated to afford cyanomethyl (S)-3-(6-(dimethylamino)pyridin-3-yl)-2-((((4-(2-(4-fluorophenyl)acetamido)benzyl)oxy)carbonyl)(methyl)amino)propanoate (F-Pnaz-MeAla(3-Pyr-4-NMe2)-$OCH_2CN$) as a crude product.

To buffer A (20 mL) were added a solution of ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (13.52 mg, 0.019 mmol) in water (2.0 mL) and a solution of the above crude product cyanomethyl (S)-3-(6-(dimethylamino)pyridin-3-yl)-2-((((4-(2-(4-fluorophenyl)acetamido)benzyl)oxy)carbonyl)(methyl)amino)propanoate (F-Pnaz-MeAla(3-Pyr-4-NMe2)-$OCH_2CN$) in acetonitrile (1.0 mL), and the mixture was stirred at room temperature for 1 hour and 30 minutes. To the reaction solution was added trifluoroacetic acid (TFA) (0.4 mL) at 0°C and the mixture was stirred at 0°C for 30 min, after which the reaction solution was diluted with water and purified by reverse phase column chromatography (0.05% aqueous trifluoroacetic acid solution/0.05% trifluoroacetic acid-acetonitrile solution) to afford the title compound (Compound pc207, F-Pnaz-MeAla(3-Pyr-4-NMe2)-pCpA) (3.0 mg, 14%).
LCMS (ESI) m/z = 1141.5 (M-H)-
Retention time: 0.45 min (analytical condition SQDFA05)

Synthesis of tert-butyl N-(tert-butoxycarbonyl)-N-pentylglycinate (Compound pc208, Boc-nPenGly-OtBu)

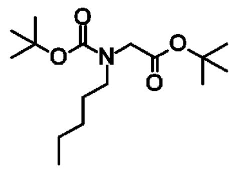

To a solution of sodium hydride (5.2 g, 216.67 mmol, 60% oil dispersion) in tetrahydrofuran (THF) (100 mL) was added tert-butyl (tert-butoxycarbonyl)glycinate (Boc-Gly-OtBu) (20 g, 86.47 mmol) at 0°C under a nitrogen atmosphere, after which the reaction solution was stirred at room temperature for 30 min and a solution of 1-iodopentane (51.4 g, 259.54 mmol) in dimethylformamide (10 mL) was added dropwise. The reaction solution was stirred at room temperature for 16 h, water was then added, and the mixture was extracted with ethyl acetate three times. The resulting organic layers were dried over anhydrous sodium sulfate, the solvent was evaporated under reduced pressure, and the residue was further dried using a pump to afford tert-butyl N-(tert-butoxycarbonyl)-N-pentylglycinate (Compound pc208, Boc-nPenGly-OtBu) (6.3 g) as a crude product.
LCMS (ESI) m/z = 302 (M+H)+
Retention time: 1.37 min (analytical condition SMD method 9)

Synthesis of cyanomethyl N-(pent-4-enoyl)-N-pentylglycinate (Compound pc209, Pen-nPenGly-$OCH_2CN$)

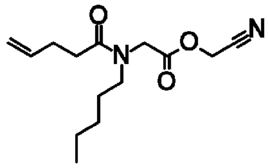

The crude product tert-butyl N-(tert-butoxycarbonyl)-N-pentylglycinate (Compound pc208, Boc-nPenGly-OtBu) obtained as described above (10.2 g, 33.84 mmol) was dissolved in 1,4-dioxane (37.5 mL), concentrated hydrochloric acid (37.5 mL) was added at 0°C, and the mixture was stirred at room temperature for 16 h. The solvent was evaporated under reduced pressure from the reaction solution, and the residue was dried using a pump to afford pentylglycine (H-nPenGly-OH) hydrochloride (4.9 g) as a crude product.

The crude product pentylglycine (H-nPenGly-OH) hydrochloride obtained as described above (6.2 g, 42.7 mmol) was dissolved in 1,4-dioxane (71 mL)/water (71 mL), after which 2,5-dioxopyrrolidin-1-yl pent-4-enoate synthesized by the method described in the document (Organic Letters, 2011, 13, 4906) (20 g, 101.43 mmol) and sodium bicarbonate (5.7 g, 85.4 mmol) were added and the reaction solution was stirred at 35°C for 16 h. After the reaction was completed, the reaction solution was washed with diethyl ether twice, and a 1.0 M aqueous hydrochloric acid solution was added to the aqueous layer until pH = 2. The resulting mixture was extracted with dichloromethane twice, and the organic layers were dried over anhydrous sodium sulfate, filtered, and then concentrated under reduced pressure. The resulting residue was purified by normal phase silica gel column chromatography (petroleum ether/ethyl acetate) to afford N-(pent-4-enoyl)-N-pentylglycine (Pen-nPenGly-OH) (1.8 g, 7.92 mmol).

A solution of N-(pent-4-enoyl)-N-pentylglycine (Pen-nPenGly-OH) (1.8 g, 7.92 mmol), 2-bromoacetonitrile (3.8 g, 31.68 mmol), and N,N-diisopropylethylamine (DIPEA) (2.0 g, 15.84 mmol) in dichloromethane (36 mL) was stirred at room temperature for 16 h under a nitrogen atmosphere. The reaction solution was concentrated and the resulting residue was purified by normal phase silica gel column chromatography (petroleum ether/ethyl acetate) to afford cyanomethyl N-(pent-4-enoyl)-N-pentylglycinate (Compound pc209, Pen-nPenGly-$OCH_2CN$) (1.6 g).
LCMS (ESI) m/z = 267 (M+H)+
Retention time: 1.05 min (analytical condition SMD method 9)

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl N-(pent-4-enoyl)-N-pentylglycinate (Compound pc210, Pen-nPenGly-pCpA)

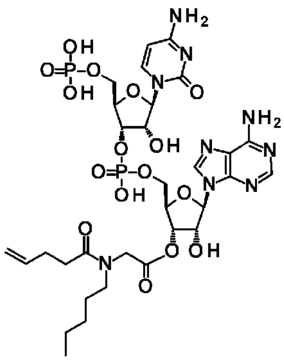

To buffer A (75 mL) were added ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (300 mg, 0.42 mmol) and a solution of cyanomethyl N-(pent-4-enoyl)-N-pentylglycinate (Compound pc209, Pen-nPenGly-$OCH_2CN$) (448 mg, 1.68 mmol) in acetonitrile (3.0 mL), and the mixture was stirred at room temperature for 2 h. To the reaction solution was added trifluoroacetic acid (1.7 mL), the reaction solution was lyophilized as such, and the resulting residue was purified by reverse

phase column chromatography (0.05% aqueous trifluoroacetic acid solution/0.05% trifluoroacetic acid-acetonitrile solution) to afford the title compound (Compound pc210, Pen-nPenGly-pCpA) (40 mg, 6%).
LCMS (ESI) m/z = 862 (M+H)+
Retention time: 0.48 min (analytical condition SQDFA05)

Synthesis of N-hexyl-N-(pent-4-enoyl)glycine (Compound pc211, Pen-nHexGly-OH)

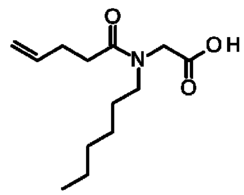

To a solution of sodium hydride (6.0 g, 250 mmol, 60% oil dispersion) in tetrahydrofuran (THF) (150 mL) was added tert-butyl (tert-butoxycarbonyl)glycinate (Boc-Gly-OtBu) (25 g, 108.09 mmol) at 0°C under a nitrogen atmosphere, after which the reaction solution was stirred at room temperature for 30 min and a solution of 1-iodohexane (68.8 g, 324.42 mmol) in dimethylformamide (15 mL) was added dropwise. The reaction solution was stirred at room temperature for 16 h, water was then added, and the mixture was extracted with ethyl acetate three times. The resulting organic layers were dried over anhydrous sodium sulfate, the solvent was evaporated under reduced pressure, and the residue was further dried using a pump to afford tert-butyl N-(tert-butoxycarbonyl)-N-hexylglycinate (Boc-nHexGly-OtBu) (8.3 g) as a crude product.

The crude product tert-butyl N-(tert-butoxycarbonyl)-N-hexylglycinate (Boc-nHexGly-OtBu) (11.5 g) was dissolved in 1,4-dioxane (40.4 mL), concentrated hydrochloric acid (40.4 mL) was added at 0°C, and the mixture was stirred at room temperature for 16 h. The solvent was evaporated from the reaction solution under reduced pressure, and the residue was further dried using a pump to afford hexylglycine (H-nHexGly-OH) hydrochloride (5.8 g) as a crude product.

The crude product hexylglycine (H-nHexGly-OH) hydrochloride (7.1 g) was dissolved in 1,4-dioxane (76 mL)/water (76 mL), after which 2,5-dioxopyrrolidin-1-yl pent-4-enoate synthesized by the method described in the literature (Organic Letters, 2011, 13, 4906) (21.6 g, 109.54 mmol) and sodium bicarbonate (6.1 g, 72.61 mmol) were added and the reaction solution was stirred at 35°C for 16 h. After the reaction was completed, the reaction solution was washed with diethyl ether twice, and a 1.0 M aqueous hydrochloric acid solution was added to the aqueous layer until the acidity became pH = 2. The resulting mixture was extracted with dichloromethane twice, and the organic layers were dried over anhydrous sodium sulfate, filtered, and then concentrated under reduced pressure. The resulting residue was purified by normal phase silica gel column chromatography (petroleum ether/ethyl acetate) to afford N-hexyl-N-(pent-4-enoyl)glycine (Compound pc211, Pen-nHexGly-OH) (4.0 g, 16.6 mmol).
LCMS (ESI) m/z = 242 (M+H)+
Retention time: 0.98 min (analytical condition SMD method9)

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl N-hexyl-N-(pent-4-enoyl)glycinate (Compound pc212, Pen-nHexGly-pCpA)

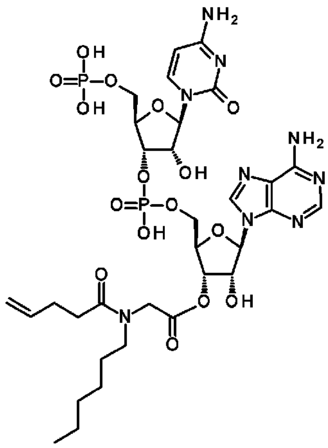

A solution of N-hexyl-N-(pent-4-enoyl)glycine (Compound pc211, Pen-nHexGly-OH) (4.0 g, 16.6 mmol), 2-bromoacetonitrile (8.0 g, 66.7 mmol), and N,N-diisopropylethylamine (DIPEA) (4.3 g, 33.3 mmol) in dichloromethane (70 mL) was stirred at room temperature for 16 h under a nitrogen atmosphere. The reaction solution was concentrated and the resulting residue was purified by normal phase silica gel column chromatography (petroleum ether/ethyl acetate) to afford cyanomethyl N-hexyl-N-(pent-4-enoyl)glycinate (Pen-nHexGly-$OCH_2CN$) (1.2 g, 26%).

To buffer A (75 mL) were added ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (300 mg, 0.42 mmol) and cyanomethyl N-hexyl-N-(pent-4-enoyl)glycinate (Pen-nHexGly-$OCH_2CN$) (465 mg, 1.66 mmol) in acetonitrile (3.0 mL), and the mixture was stirred at room temperature for 2 h. To the reaction solution was added trifluoroacetic acid (1.7 mL), the reaction solution was lyophilized as such, and the resulting residue was purified by reverse phase column chromatography (0.05% aqueous trifluoroacetic acid solution/0.05% trifluoroacetic acid-acetonitrile solution) to afford the title compound (Compound pc212, Pen-nHexGly-pCpA) (53 mg, 7%).
LCMS (ESI) m/z = 876 (M+H)+
Retention time: 0.52 min (analytical condition SQDFA05)

Synthesis of N-(pent-4-enoyl)-N-phenethylglycine (Compound pc213, Pen-(PhEt) NGly-OH)

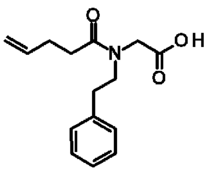

To a solution of 2-phenylethan-1-amine (6.0 g, 49.51 mmol) in water (80 mL) was added a solution of 2-bromoacetic acid (2.3 g, 16.55 mmol) in water (20 mL) at 0°C, and a 8 N aqueous sodium hydroxide solution (8.2 mL) was then added dropwise at 0°C. The reaction solution was stirred at room temperature for 16 h and then washed with ethyl acetate three times. The resulting aqueous layer was concentrated to about 60 mL under reduced pressure and neutralized to pH = 7 with concentrated hydrochloric acid to give an aqueous phenethylglycine (H-(PhEt)NGly-OH) solution as a mixture.

To the aqueous phenethylglycine (H-(PhEt)NGly-OH) solution as a mixture was added 1,4-dioxane (60 mL), after which 2,5-dioxopyrrolidin-1-yl pent-4-enoate synthesized by the method described in the document (Organic Letters, 2011, 13, 4906) (6.6 g, 33.47 mmol) and sodium bicarbonate (2.8 g, 33.33 mmol) and the reaction solution was stirred at 25°C for 16 h. The reaction solution was washed with diethyl ether three times, and a 1.0 M aqueous hydrochloric acid solution was added to the aqueous layer until the acidity became pH = 4. The resulting mixture was extracted with dichloromethane twice, and the organic layers were dried over anhydrous sodium sulfate, filtered, and then concentrated under reduced pressure. The resulting residue was purified by normal phase silica gel column chromatography (petroleum ether/ethyl acetate) to afford N-(pent-4-enoyl)-N-phenethylglycine (Compound pc213, Pen-(PhEt)NGly-OH) (1.4 g, 32% over two steps).

LCMS (ESI) m/z = 262 (M+H)+
Retention time: 0.90 min (analytical condition SMD method 16)

Synthesis of cyanomethyl N-(pent-4-enoyl)-N-phenethylglycinate (Compound pc214, Pen-(PhEt)NGly-$OCH_2CN$)

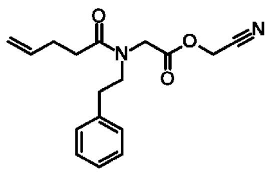

A solution of N-(pent-4-enoyl)-N-phenethylglycine (Compound pc213, Pen-(PhEt)NGly-OH) (1.5 g, 5.74 mmol), 2-bromoacetonitrile (2.74 g, 22.84 mmol), and N,N-diisopropylethylamine (DIPEA) (1.48 g, 11.45 mmol) in dichloromethane (45 mL) was stirred at 25°C for 16 h under a nitrogen atmosphere. The reaction solution was concentrated and the resulting residue was purified by normal phase silica gel column chromatography (petroleum ether/ethyl acetate) to afford cyanomethyl N-(pent-4-enoyl)-N-phenethylglycinate (Compound pc214, Pen-(PhEt)NGly-$OCH_2CN$) (1.1 g, 64%).
LCMS (ESI) m/z = 301 (M+H)+
Retention time: 1.03 min (analytical condition SMD method 16)

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl N-(pent-4-enoyl)-N-phenethylglycinate (Compound pc215, Pen-(PhEt) NGly-pCpA)

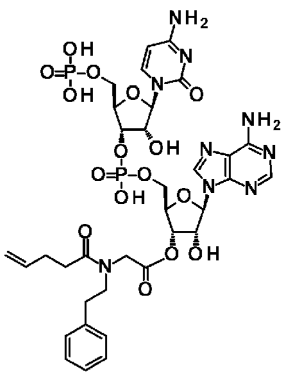

To buffer A (75 mL) were added ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (300 mg, 0.42 mmol) and a solution of cyanomethyl N-(pent-4-enoyl)-N-phenethylglycinate (Compound pc214, Pen-(PhEt)NGly-$OCH_2CN$) (373 mg, 1.24 mmol) in acetonitrile (2.0 mL), and the mixture was stirred at room temperature for 2 h. To the reaction solution was added trifluoroacetic acid (1.6 mL), the reaction solution was lyophilized as such, and the resulting residue was purified by reverse phase column chromatography (0.05% aqueous trifluoroacetic acid solution/0.05% trifluoroacetic acid-acetonitrile solution) to afford the title compound (Compound pc215, Pen-(PhEt)NGly-pCpA) (57.5 mg, 8%).
LCMS (ESI) m/z = 897 (M+H)+
Retention time: 0.50 min (analytical condition SQDFA05)

Synthesis of cyanomethyl N-(2-ethoxyethyl)-N-(pent-4-enoyl)glycinate (Compound pc216, Pen-(EtOEt)NGly-$OCH_2CN$)

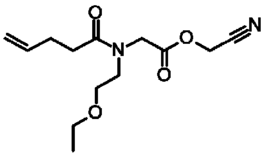

To a solution of 2-ethoxyethan-1-amine (9.6 g, 107.87 mmol) in water (160 mL) was added a solution of 2-bromoacetic acid (5.0 g, 35.97 mmol) in water (40 mL) at 0°C, and a 8 N aqueous sodium hydroxide solution (17.8 mL) was then added dropwise at 0°C. The reaction solution was stirred at room temperature for 16 h and then washed with ethyl acetate three times. The resulting aqueous layer was concentrated to about 200 mL under reduced pressure and neutralized to pH = 7 with concentrated hydrochloric acid to give an aqueous (2-ethoxyethyl)glycine (H-(EtOEt)NGly-OH) solution as a mixture.

To the aqueous (2-ethoxyethyl)glycine (H-(EtOEt)NGly-OH) solution as a mixture was added 1,4-dioxane (200 mL), after which 2,5-dioxopyrrolidin-1-yl pent-4-enoate synthesized by the method described in the document (Organic Letters, 2011, 13, 4906) (14.0 g, 71.36 mmol) and sodium bicarbonate (6.0 g, 71.42 mmol) and the reaction solution was stirred at 25°C for 16 h. The reaction solution was washed with diethyl ether three times, and a 1.0 M aqueous hydrochloric acid solution was added to the aqueous layer until the acidity became pH = 4. The resulting mixture was extracted with dichloromethane twice, and the organic layers were dried over anhydrous sodium sulfate, filtered, and then concentrated under reduced pressure. The resulting residue was purified by normal phase silica gel column chromatography (petroleum ether/ethyl acetate) to afford N-(2-ethoxyethyl)-N-(pent-4-enoyl)glycine (Pen-(EtOEt)NGly-OH) (3.1 g, 38% over two steps).

A solution of N-(2-ethoxyethyl)-N-(pent-4-enoyl)glycine (Pen-(EtOEt)NGly-OH) (3.1 g, 13.52 mmol), 2-bromoacetonitrile (6.65 g, 55.44 mmol), and N,N-diisopropylethylamine (DIPEA) (3.61 g, 27.93 mmol) in dichloromethane (60 mL) was stirred at 25°C for 16 h. The reaction solution was concentrated and the resulting residue was purified by normal phase silica gel column chromatography (petroleum ether/ethyl acetate) to afford cyanomethyl N-(2-ethoxyethyl)-N-(pent-4-enoyl)glycinate (Compound pc216, Pen-(EtOEt)NGly-$OCH_2CN$) (2.7 g, 74%).
LCMS (ESI) m/z = 269 (M+H)+
Retention time: 0.87 min (analytical condition SMD method 16)

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl N-(2-ethoxyethyl)-N-(pent-4-enoyl)glycinate (Compound pc217, Pen-(EtOEt)NGly-pCpA)

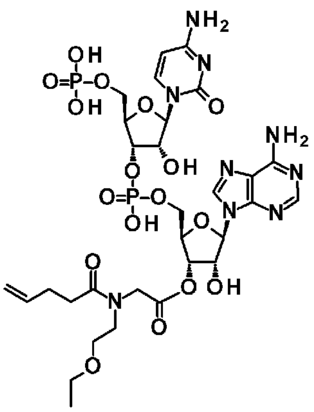

To buffer A (75 mL) were added ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (300 mg, 0.42 mmol) and a solution of cyanomethyl N-(2-ethoxyethyl)-N-(pent-4-enoyl)glycinate (Compound pc216, Pen-(EtOEt)NGly-$OCH_2CN$) (343 mg, 1.28 mmol) in acetonitrile (2.0 mL), and the mixture was stirred at room temperature for 1 h. To the reaction solution was added trifluoroacetic acid (1.6 mL), the reaction solution was lyophilized as such, and the resulting residue was purified by reverse phase column chromatography (0.05% aqueous trifluoroacetic acid solution/0.05% trifluoroacetic acid-acetonitrile solution) to afford the title compound (Compound pc217, Pen-(EtOEt) NGly-pCpA) (50.0 mg, 7%).

LCMS (ESI) m/z = 865 (M+H)+
Retention time: 0.40 min (analytical condition SQDFA05)

Synthesis of N-(pent-4-enoyl)-N-(2-phenoxyethyl)glycine (Compound pc218, Pen-(PhOEt)NGly-OH)

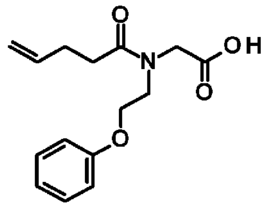

To a solution of 2-phenoxyethan-1-amine (14.8 g, 108.03 mmol) in water (160 mL) was added a solution of 2-bromoacetic acid (5.0 g, 35.97 mmol) in water (40 mL) at 0°C, and a 8 N aqueous sodium hydroxide solution (17.8 mL) was then added dropwise at 0°C. The reaction solution was stirred at room temperature for 16 h and then washed with ethyl acetate three times. The resulting aqueous layer was concentrated to about 100 mL under reduced pressure and neutralized to pH = 7 with concentrated hydrochloric acid to give an aqueous (2-phenoxyethyl)glycine (H-(PhOEt)NGly-OH) solution as a mixture.

To the aqueous (2-phenoxyethyl)glycine (H-(PhOEt)NGly-OH) solution as a mixture was added 1,4-dioxane (100 mL), after which 2,5-dioxopyrrolidin-1-yl pent-4-enoate synthesized by the method described in the document (Organic Letters, 2011, 13, 4906) (14.0 g, 71.36 mmol) and sodium bicarbonate (6.0 g, 71.42 mmol) and the reaction solution was stirred at 25°C for 16 h. The reaction solution was washed with diethyl ether three times, and a 5 N aqueous hydrochloric acid solution was added to the aqueous layer until the acidity became pH = 4. The resulting mixture was extracted with dichloromethane twice, and the organic layers were dried over anhydrous sodium sulfate, filtered, and then concentrated under reduced pressure. The resulting residue was purified by normal phase silica gel column chromatography (petroleum ether/ethyl acetate) to afford N-(pent-4-enoyl)-N-(2-phenoxyethyl)glycine (Compound pc218, Pen-(PhOEt)NGly-OH) (2.2 g, 22% over two steps).
LCMS (ESI) m/z = 278 (M+H)+
Retention time: 0.91 min (analytical condition SMD method 16)

Synthesis of cyanomethyl N-(pent-4-enoyl)-N-(2-phenoxyethyl)glycinate (Compound pc219, Pen-(PhOEt )NG1y-$OCH_2CN$)

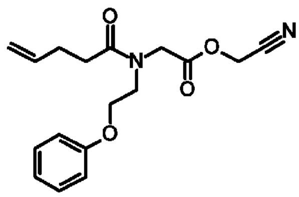

A solution of N-(pent-4-enoyl)-N-(2-phenoxyethyl)glycine (Compound pc218, Pen-(PhOEt)NGly-OH) (2.2 g, 7.93 mmol), 2-bromoacetonitrile (3.78 g, 31.51 mmol), and N,N-diisopropylethylamine (DIPEA) (2.04 g, 15.78 mmol) in dichloromethane (60 mL) was stirred at 25°C for 16 h under a nitrogen atmosphere. The reaction solution was concentrated and the resulting residue was purified by normal phase silica gel column chromatography (petroleum ether/ethyl acetate) to afford cyanomethyl N-(pent-4-enoyl)-N-(2-phenoxyethyl)glycinate (Compound pc219, Pen-(PhOEt)NGly-$OCH_2CN$) (2.0 g, 80%).
LCMS (ESI) m/z = 317 (M+H)+
Retention time: 1.02 min (analytical condition SMD method 16)

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl N-(pent-4-enoyl)-N-(2-phenoxyethyl)glycinate (Compound pc220, Pen-(PhOEt)NGly-pCpA)

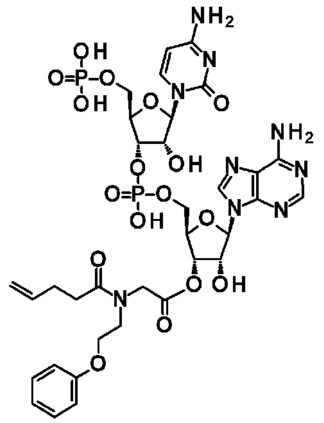

To buffer A (75 mL) were added ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (300 mg, 0.42 mmol) and a solution of cyanomethyl N-(pent-4-enoyl)-N-(2-phenoxyethyl)glycinate (Compound pc219, Pen-(PhOEt)NGly-$OCH_2CN$) (393 mg, 1.24 mmol) in acetonitrile (2.0 mL), and the mixture was stirred at room temperature for 1 h. To the reaction solution was added trifluoroacetic acid (1.6 mL), the reaction solution was lyophilized as such, and the resulting residue was purified by reverse phase column chromatography (0.05% aqueous trifluoroacetic acid solution/0.05% trifluoroacetic acid-acetonitrile solution) to afford the title compound (Compound pc220, Pen-(PhOEt)NGly-pCpA) (53.3 mg, 7%).
LCMS (ESI) m/z = 913 (M+H)+
Retention time: 0.49 min (analytical condition SQDFA05)

Compound pc222 (F-Pnaz-Pro(pip-4-F2)-pCpA) was synthesized according to the following scheme.

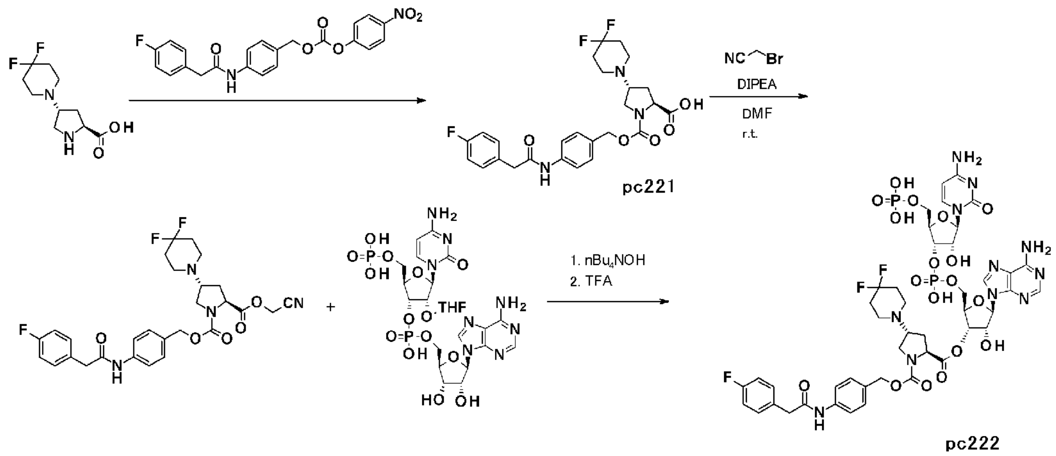


pc221

pc222

Synthesis of (2S,4R)-4-(4,4-difluoro-1-piperidyl)-1-[[4-[[2-(4-fluorophenyl)acetyl]amino]phenyl]methoxycarbonyl]pyrrolidine-2-carboxylic acid (Compound pc221, F-Pnaz-Pro(pip-4-F2)-OH)

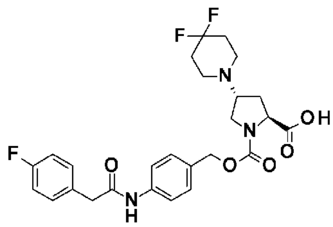

(2S,4R)-4-(4,4-Difluoro-1-piperidyl)pyrrolidine-2-carboxylic acid (Compound aa129, H-Pro(pip-4-F2)-OH) (135 mg, 0.576 mmol) was dissolved in dimethyl sulfoxide (2 mL), triethylamine (0.161 mL, 1.153 mmol) and 4-(2-(4-fluorophenyl)acetamido)benzyl (4-nitrophenyl) carbonate (Compound pc196) (245 mg, 0.576 mmol) were added, and the mixture was stirred at room temperature for 5 h. To the reaction solution was added a 0.1% aqueous ammonium acetate solution (2 mL), and the reaction solution was then purified by reverse phase column chromatography (10 mM aqueous ammonium acetate solution/methanol) to afford (2S,4R)-4-(4,4-difluoro-1-piperidyl)-1-[[4-[[2-(4-fluorophenyl)acetyl]amino]phenyl]methoxycarbonyl]pyrrolidine-2-carboxylic acid (Compound pc221, F-Pnaz-Pro(pip-4-F2)-OH) (143 mg, 48%).
LCMS (ESI) m/z = 520 (M+H)+
Retention time: 0.77 min (analytical condition SQDAA05)

Synthesis of 2-((2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl) 1-(4-(2-(4-fluorophenyl)acetamido)benzyl) (2S,4R)-4-(4,4-difluoropiperidin-1-yl)pyrrolidine-1,2-dicarboxylate (Compound pc222, F-Pnaz-Pro(pip-4-F2)-pCpA)

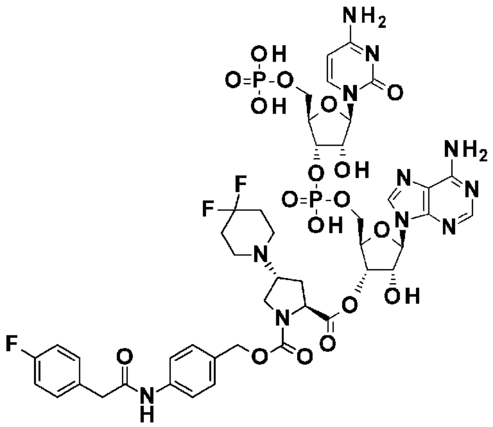

((2R,3R,4R,5R)-5-(4-Amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (400 mg, 0.554 mmol) was dissolved in water (2.0 mL), tetrabutylammonium hydroxide (40% aqueous solution, 0.9 g, 1.387 mmol) was added, and the mixture was stirred. The water was removed by lyophilization to afford ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate tetrabutylammonium salt (0.723 g).

(2S,4R)-4-(4,4-Difluoro-1-piperidyl)-1-[[4-[[2-(4-fluorophenyl)acetyl]amino]phenyl]methoxycarbonyl]pyrrolidine-2-carboxylic acid (Compound pc221, F-Pnaz-Pro(pip-4-F2)-OH) (80 mg, 0.154 mmol) and 2-bromoacetonitrile (10.2 μL, 0.146 mmol) were then dissolved in dimethylformamide (0.6 mL), N,N-diisopropylethylamine (DIPEA) (0.054 mL, 0.308 mmol) was added, and the mixture was stirred at room temperature for 17 h. To the reaction solution was added previously prepared ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate tetrabutylammonium salt (125 mg), and the mixture was stirred at 50°C for 46 h. After cooling to room temperature, formic acid (0.030 mL, 0.770 mmol) was added to the reaction solution, and the mixture was purified by reverse phase column chromatography (0.1% aqueous formic acid solution/0.1% formic acid-acetonitrile solution). The fractions were lyophilized, the resulting residue was dissolved in a 5% aqueous trifluoroacetic acid solution,

and the solution was stirred at room temperature for 30 min. The reaction solution was then diluted by adding water and purified by reverse phase column chromatography (0.05% aqueous trifluoroacetic acid solution/0.05% trifluoroacetic acid-acetonitrile solution). The fractions were lyophilized to afford the title compound (Compound pc222, F-Pnaz-Pro(pip-4-F2)-pCpA) (7.7 mg, 9%).

LCMS (ESI) m/z = 1155 (M+H)+

Retention time: 0.44 min (analytical condition SQDFA05)

Compound pc224 (F-Pnaz-cisPro(pip-4-F2)-OH) was synthesized according to the following scheme.

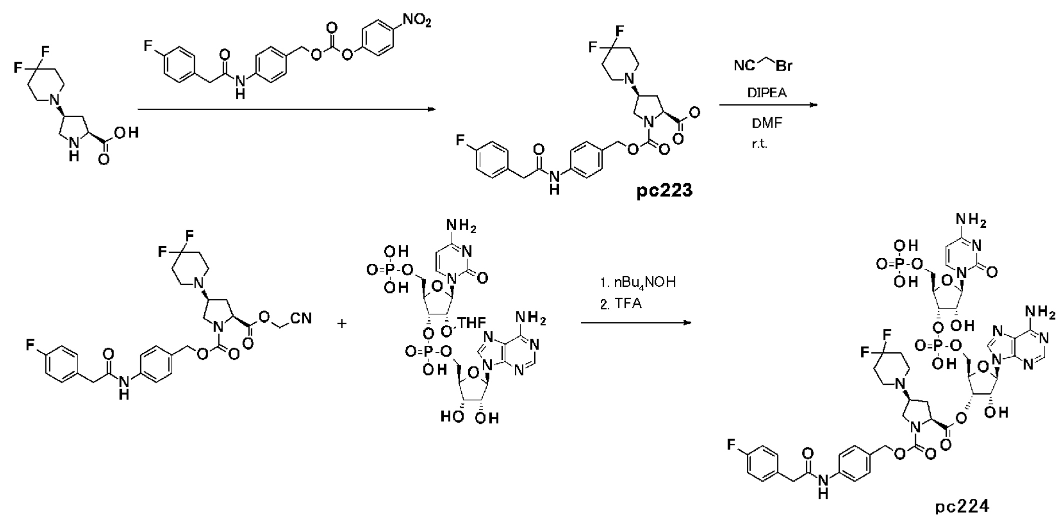


pc223

pc224

<u>Synthesis of (2S,4S)-4-(4,4-difluoro-1-piperidyl)-1-[[4-[[2-(4-fluorophenyl)acetyl]amino]phenyl]methoxycarbonyl]pyrrolidine-2-carboxylic acid (Compound pc223, F-Pnaz-cisPro(pip-4-F2)-OH)</u>

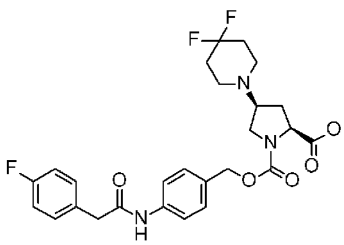

(2S,4S)-4-(4,4-Difluoropiperidin-1-yl)pyrrolidine-2-carboxylic acid (Compound aa132, H-cisPro(pip-4-F2)-OH) (90 mg, 0.384 mmol) was dissolved in dimethyl sulfoxide (1.5 mL), triethylamine (0.107 mL, 0.768 mmol) and 4-(2-(4-fluorophenyl)acetamido)benzyl (4-nitrophenyl) carbonate (Compound pc196) (163 mg, 0.384 mmol) were added, and the mixture was stirred at room temperature for 5 h. To the reaction solution was added a 0.1% aqueous ammonium acetate solution (2 mL), and the reaction solution was then purified by reverse phase column chromatography (10 mM aqueous ammonium acetate solution/methanol) to afford (2S,4S)-4-(4,4-difluoro-1-piperidyl)-1-[[4-[[2-(4-fluorophenyl)acetyl]amino]phenyl]methoxycarbonyl]pyrrolidine-2-carboxylic acid (Compound pc223, F-Pnaz-cisPro(pip-4-F2)-OH) (161 mg, 81%).

LCMS (ESI) m/z = 520 (M+H)+

Retention time: 0.80 min (analytical condition SQDAA05)

<u>Synthesis of 2-((2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl)1-(4-(2-(4-fluorophenyl)acetamido)benzyl) (2S,4S)-4-(4,4-difluoropiperidin-1-yl)pyrrolidine-1,2-dicarboxylate (Compound pc224, F-Pnaz-cisPro(pip-4-F2)-pCpA)</u>

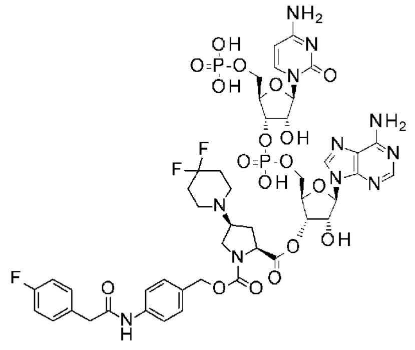

((2R,3R,4R,5R)-5-(4-Amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (400 mg, 0.554 mmol) was dissolved in water (2.0 mL), tetrabutylammonium hydroxide (40% aqueous solution, 0.9 g, 1.387 mmol) was added, and the mixture was stirred. The water was removed by lyophilization to afford ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate tetrabutylammonium salt (0.723 g).

(2S,4S)-4-(4,4-Difluoro-1-piperidyl)-1-[[4-[[2-(4-fluorophenyl)acetyl]amino]phenyl]methoxycarbonyl]pyrrolidine-2-carboxylic acid (Compound pc223, F-Pnaz-cisPro(pip-4-F2)-OH) (100 mg, 0.192 mmol) and 2-bromoacetonitrile (0.013 mL, 0.183 mmol) were then dissolved in dimethylformamide (0.6 mL), N,N-diisopropylethylamine (DIPEA) (0.067 mL, 0.385 mmol) was added, and the mixture was stirred at room temperature for 17 h. To the reaction solution was added previously prepared ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate tetrabutylammonium salt (155 mg), and the mixture was stirred at 50°C for 46 h. After cooling to room temperature, formic acid (0.037 mL, 0.960 mmol) was added to the reaction solution, and the mixture was purified by reverse phase column chromatography (0.1% aqueous formic acid solution/0.1% formic acid-acetonitrile solution). The fractions were lyophilized, the resulting residue was dissolved in a 5% aqueous trifluoroacetic acid solution, and the solution was stirred at room temperature for 30 min. The reaction solution was then diluted by adding water and purified by reverse phase column chromatography (0.05% aqueous trifluoroacetic acid solution/0.05% trifluoroacetic acid-acetonitrile solution). The fractions were lyophilized to afford the title compound (Compound pc224, F-Pnaz-cisPro(pip-4-F2)-pCpA) (7.2 mg, 7%).
LCMS (ESI) m/z = 1155 (M+H)+
Retention time: 0.46 min (analytical condition SQDFA05)

Compound pc228 (F-Pnaz-Abu(Mor)-pCpA) was synthesized according to the following scheme.

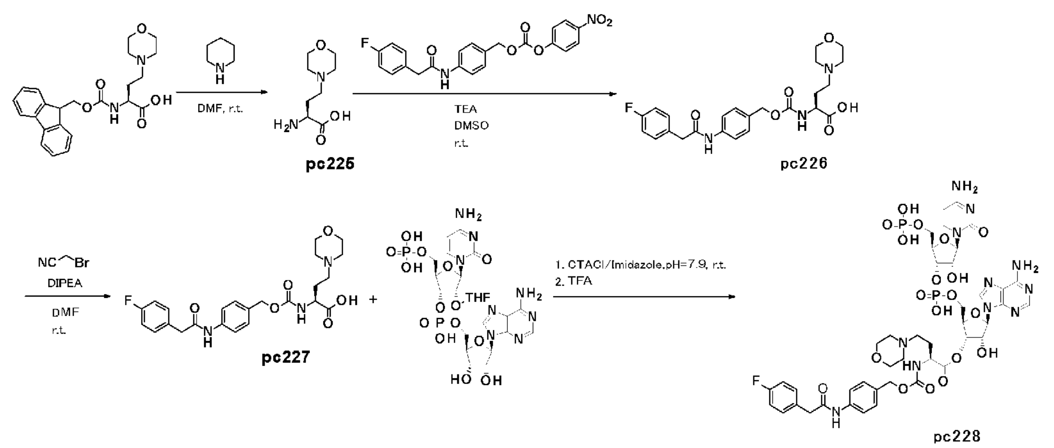


<u>Synthesis of (2S)-2-amino-4-morpholino-butanoic acid (Compound pc225, H-Abu(Mor)-OH)</u>

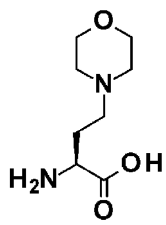

(S)-2-((((9H-Fluoren-9-yl)methoxy)carbonyl)amino)-4-morpholinobutanoic acid (Compound aa123, Fmoc-Abu(Mor)-OH) (12.5 g, 30.45 mmol) was dissolved in dimethylformamide (30 mL), piperidine (8 mL) was added, and the mixture was stirred for 2 h. The reaction solution was then diluted with diethyl ether, and the precipitated solid was collected to afford (2S)-2-amino-4-morpholino-butanoic acid (Compound pc225, H-Abu(Mor)-OH) (4 g, 70%).
LCMS (ESI) m/z = 189 (M+H)+
Retention time: 0.238 min (analytical condition SMD method39)

Synthesis of (2S)-2-[[4-[[2-(4-fluorophenyl)acetyl]amino]phenyl]methoxycarbonylamino]-4-morpholino-butanoic acid (Compound pc226, F-Pnaz-Abu(Mor)-OH)

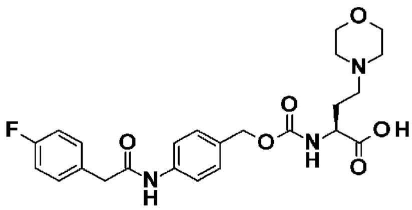

(2S)-2-Amino-4-morpholino-butanoic acid (Compound pc225, H-Abu(Mor)-OH) (640 mg, 3.40 mmol), 4-(2-(4-fluorophenyl)acetamido)benzyl (4-nitrophenyl) carbonate (Compound pc196) (1.3 g, 3.06 mmol), and triethylamine (0.85 mL, 6.12 mmol) were dissolved in dimethyl sulfoxide (12 mL), and the mixture was stirred at 25°C for 16 h under a nitrogen atmosphere. The reaction solution was then purified by reverse phase column chromatography (0.1% aqueous formic acid solution/0.1% formic acid-acetonitrile solution) to afford (2S)-2-[[4-[[2-(4-fluorophenyl)acetyl]amino]phenyl]methoxycarbonylamino]-4-morpholino-butanoic acid (Compound pc226, F-Pnaz-Abu(Mor)-OH) (1.0 g, 69%).
LCMS (ESI) m/z = 474 (M+H)+
Retention time: 0.74 min (analytical condition SMD method 43)

Synthesis of cyanomethyl (2S)-2-[[4-[[2-(4-fluorophenyl)acetyl]amino]phenyl]methoxycarbonylamino]-4-morpholino-butanoate (Compound pc227, F-Pnaz-Abu(Mor)-$OCH_2CN$)

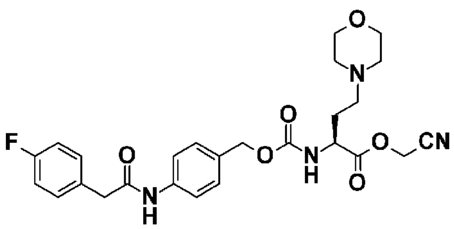

(2S)-2-[[4-[[2-(4-Fluorophenyl)acetyl]amino]phenyl]methoxycarbonylamino]-4-morpholino-butanoic acid (Compound pc226, F-Pnaz-Abu(Mor)-OH) (1 g, 2.11 mmol), N,N-diisopropylethylamine (DIPEA) (540 mg, 4.18 mmol), and 2-bromoacetonitrile (1 g, 8.34 mmol) were dissolved in dichloromethane (30 mL), and the solution was stirred at 25°C for 48 h under a nitrogen atmosphere. The reaction solution was then concentrated under reduced pressure, and the resulting residue was purified by normal phase column chromatography (dichloromethane/acetone) to afford cyanomethyl (2S)-2-[[4-[[2-(4-fluorophenyl)acetyl]amino]phenyl]methoxycarbonylamino]-4-morpholino-butanoate (Compound pc227, F-Pnaz-Abu(Mor)-$OCH_2CN$) (505 mg, 47%).
LCMS (ESI) m/z = 513 (M+H)+
Retention time: 0.66 min (analytical condition SMD method 44)

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl (2S)-2-((((4-(2-(4-fluorophenyl)acetamido)benzyl)oxy)carbonyl)amino)-4-morpholinobutanoate (Compound pc228, F-Pnaz-Abu(Mor)-pCpA)

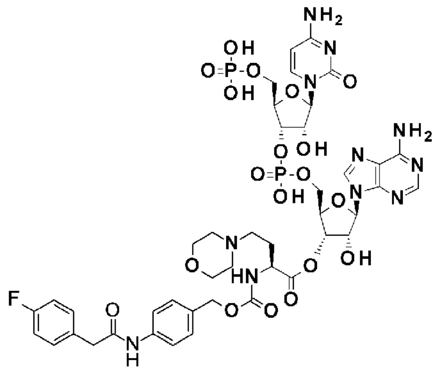

((2R,3R,4R,5R)-5-(4-Amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (400 mg, 0.554 mmol) was dissolved in buffer A (100 mL), and a solution of cyanomethyl (2S)-2-[[4-[[2-(4-fluorophenyl)acetyl]amino]phenyl]methoxycarbonylamino]-4-morpholino-butanoate (Compound pc227, F-Pnaz-Abu(Mor)-$OCH_2CN$) (150 mg, 0.29 mmol) in acetonitrile (5.0 mL) was added over 15 min. After stirring at room temperature for 1 h, trifluoroacetic acid (2.3 mL) was added and the reaction solution was lyophilized. The resulting residue was purified by reverse phase column chromatography (0.05% aqueous trifluoroacetic acid solution/0.05% trifluoroacetic acid-acetonitrile solution) to afford the title compound (Compound pc228, F-Pnaz-Abu(Mor)-pCpA) (33.2 mg, 10%).
LCMS (ESI) m/z = 1109 (M+H)+
Retention time: 1.12 min (analytical condition SMD method 34)

Synthesis of N-(2-(4,4-difluoropiperidin-1-yl)ethyl)-N-(((4-(2-(4-fluorophenyl)acetamido)benzyl)oxy)carbonyl)glycine (Compound pc229, F-Pnaz-(2-(pip-4-F2)-Et)Glv-OH)

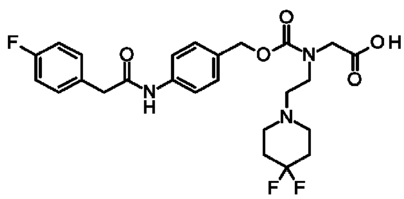

To a solution of N-(((9H-fluoren-9-yl)methoxy)carbonyl)-N-(2-(4,4-difluoropiperidin-1-yl)ethyl)glycine (Compound aa126, Fmoc-(2-(pip-4-F2)-Et)Gly-OH) (1.081 mg, 2.433 mmol) in dichloromethane (DCM) was added 4-(3-phenylpropyl)piperidine (2.061 mL, 9.73 mmol) at room temperature, and the mixture was stirred for 30 min. To the reaction solution was added water, and the mixture was purified by reverse phase chromatography (0.1% aqueous formic acid solution/0.1% formic acid-acetonitrile solution) to afford (2-(4,4-difluoropiperidin-1-yl)ethyl)glycine (H-(2-(pip-4-F2)-Et)Gly-OH) (585.6 mg).

To the above (2-(4,4-difluoropiperidin-1-yl)ethyl)glycine (H-(2-(pip-4-F2)-Et)Gly-OH) (150 mg) were added dimethyl sulfoxide (DMSO) (3.0 mL) and triethylamine ($Et_3N$) (400 μL), after which separately prepared 4-(2-(4-fluorophenyl)acetamido)benzyl (4-nitrophenyl) carbonate (Compound pc 196) (356 mg, 0.829 mmol) was added at room

temperature and the mixture was stirred for 10 min. The reaction solution was diluted with dimethyl sulfoxide (DMSO) and purified by reverse phase chromatography (10 mM aqueous ammonium acetate solution/methanol) to afford N-(2-(4,4-difluoropiperidin-1-yl)ethyl)-N-(((4-(2-(4-fluorophenyl)acetamido)benzyl)oxy)carbonyl)glycine (Compound pc229, F-Pnaz-(2-(pip-4-F2)-Et)Gly-OH) (253.5 mg, 80% over two steps).
LCMS (ESI) m/z = 508 (M+H)+
Retention time: 0.75 min (analytical condition SQDAA05)

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl N-(2-(4,4-difluoropiperidin-1-yl)ethyl)-N-(((4-(2-(4-fluorophenyl)acetamido)benzyl)oxy)carbonyl)glycinate (Compound pc230,F-Pnaz-(2-(pip-4-F2)-Et)Gly-pCpA)

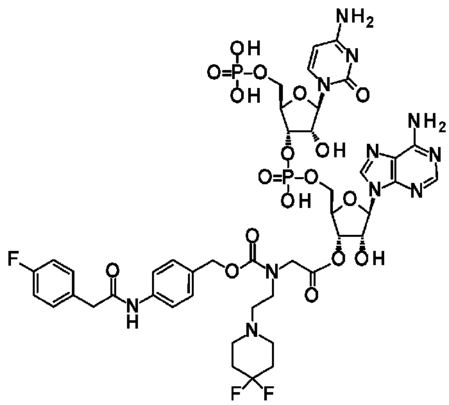

A solution of N-(2-(4,4-difluoropiperidin-1-yl)ethyl)-N-(((4-(2-(4-fluorophenyl)acetamido)benzyl)oxy)carbonyl)glycine (Compound pc229, F-Pnaz-(2-(pip-4-F2)-Et)Gly-OH) (48 mg, 0.094 mmol), 2-bromoacetonitrile (13 μL, 0.188 mmol), and N,N-diisopropylethylamine (DIPEA) (48 μL, 0.282 mmol) in acetonitrile (0.797 mL) was stirred at room temperature for 30 min under a nitrogen atmosphere. The reaction solution was concentrated to afford a mixture of cyanomethyl N-(2-(4,4-difluoropiperidin-1-yl)ethyl)-N-(((4-(2-(4-fluorophenyl)acetamido)benzyl)oxy)carbonyl)glycinate (F-Pnaz-(2-(pip-4-F2)-Et)Gly-$OCH_2CN$) which was used as such in the next reaction.

To buffer A (30 mL) were added ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (47.5 mg, 0.066 mmol) and a solution of the above mixture of cyanomethyl N-(2-(4,4-difluoropiperidin-1-yl)ethyl)-N-(((4-(2-(4-fluorophenyl)acetamido)benzyl)oxy)carbonyl)glycinate (F-Pnaz-(2-(pip-4-F2)-Et)Gly-$OCH_2CN$) (25.7 mg, 0.047 mmol) in acetonitrile (3.0 mL), and the mixture was stirred at room temperature for 3 h. To the reaction solution was added trifluoroacetic acid (TFA) (1.7 mL) at 0°C, and the mixture was stirred for 15 min. The reaction solution was purified by reverse phase column chromatography (0.05% aqueous trifluoroacetic acid solution/0.05% trifluoroacetic acid-acetonitrile solution) to afford the title compound (Compound pc230, F-Pnaz-(2-(pip-4-F2)-Et)Gly-pCpA) (11.9 mg).
LCMS (ESI) m/z = 1143 (M+H)+
Retention time: 0.46 min (analytical condition SQDFA05)

Synthesis of (S)-3-(4-(difluoromethyl)phenyl)-2-((((4-(2-(4-fluorophenyl)acetamido)benzyl)oxy)carbonyl)amino)propanoic acid (Compound pc231, F-Pnaz-Phe(4-CHF2)-OH)

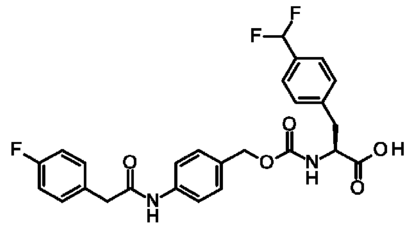

To a solution of commercially available (S)-2-((((9H-fluoren-9-yl)methoxy)carbonyl)amino)-3-(4-(difluoromethyl)phenyl)propanoic acid (Fmoc-Phe(4-CHF2)-OH) (300 mg, 0.686 mmol) in dichloromethane (3.0 mL) was added 4-(3-phenylpropyl)piperidine (0.291 mL, 1.372 mmol) at room temperature under a nitrogen atmosphere, and the mixture was stirred overnight. To the reaction solution were added t-butyl methyl ether/hexane = 1/4 and water, followed by washing with t-butyl methyl ether/hexane = 1/4. The resulting aqueous layer was purified by reverse phase column chromatography (0.1% aqueous formic acid solution/0.1% formic acid-acetonitrile solution) to afford (S)-2-amino-3-(4-(difluoromethyl)phenyl)propanoic acid (H-Phe(4-CHF2)-OH) (128 mg, 87%).

To a solution of the above (S)-2-amino-3-(4-(difluoromethyl)phenyl)propanoic acid (H-Phe(4-CHF2)-OH) (128 mg) in dimethylformamide (DMF) (2.0 mL) were added separately prepared 4-(2-(4-fluorophenyl)acetamido)benzyl (4-nitrophenyl) carbonate (Compound pc196) (303 mg, 0.714 mmol) and triethylamine ($Et_3N$) (191 μL, 1.368 mmol) at room temperature, and the mixture was stirred at 40°C for 2 h. To the reaction solution were added water and dimethyl sulfoxide (DMSO) at room temperature, and the mixture was purified by reverse phase chromatography (0.1% aqueous formic acid solution/0.1% formic acid-acetonitrile solution) to afford (S)-3-(4-(difluoromethyl)phenyl)-2-((((4-(2-(4-fluorophenyl)acetamido)benzyl)oxy)carbonyl)amino)propanoic acid (Compound pc231, F-Pnaz-Phe(4-CHF2)-OH) (262 mg, 88%).

LCMS (ESI) m/z = 501 (M+H)+

Retention time: 0.72 min (analytical condition SQDFA05)

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl (2S)-3-(4-(difluoromethyl)phenyl)-2-((((4-(2-(4-fluorophenyl)acetamido)benzyl)oxy)carbonyl)amino)propanoate (Compound pc232, F-Pnaz-Phe(4-CHF2)-pCpA)

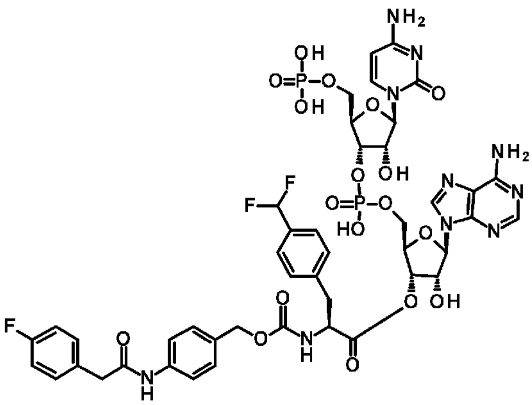

To a solution of (S)-3-(4-(difluoromethyl)phenyl)-2-((((4-(2-(4-fluorophenyl)acetamido)benzyl)oxy)carbonyl)amino)propanoic acid (Compound pc231, F-Pnaz-Phe(4-CHF2)-OH) (35 mg, 0.070 mmol) in acetonitrile (0.8 mL) were added N,N-diisopropylethylamine (DIPEA) (36.6 μL, 0.210 mmol) and 2-bromoacetonitrile (29.2 μL, 0.420 mmol) under a nitrogen atmosphere, and the mixture was stirred at room temperature overnight. The reaction solution was concentrated to afford cyanomethyl (S)-3-(4-(difluoromethyl)phenyl)-2-((((4-(2-(4-fluorophenyl)acetamido)benzyl)oxy)carbonyl)amino)propanoate (F-Pnaz-Phe(4-CHF2)-$OCH_2CN$) as a crude product.

To buffer A (20 mL) were added a solution of ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (25 mg, 0.035 mmol) in water (2.0 mL) and a solution of the above crude product cyanomethyl (S)-3-(4-(difluoromethyl)phenyl)-2-((((4-(2-(4-fluorophenyl)acetamido)benzyl)oxy)carbonyl)amino)propanoate (F-Pnaz-Phe(4-CHF2)-$OCH_2CN$) in acetonitrile (1.0 mL), and the mixture was stirred at room temperature for 1 hour and 20 minutes. To the reaction solution was added trifluoroacetic acid (TFA) (0.4 mL) at 0°C, and the reaction solution was stirred at 0°C for 50 minutes and then further stirred at room temperature for 1 hour and 10 minutes. The reaction solution was diluted with water and purified by reverse phase column chromatography (0.05% aqueous trifluoroacetic acid solution/0.05% trifluoroacetic acid-acetonitrile solution) to afford the title compound (Compound pc232, F-Pnaz-Phe(4-CHF2)-pCpA) (11 mg, 28%).
LCMS (ESI) m/z = 1135 (M+H)+
Retention time: 0.54 min (analytical condition SQDFA05)

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl (2S)-3-(3-chlorophenyl)-2-(N-methylpent-4-enamido)propanoate (Compound pc233, Pen-MePhe(3-Cl)-pCpA)

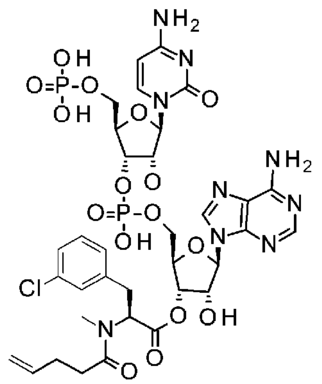

To a reaction vessel equipped with a filter were placed 2-chlorotrityl resin (15 g, 1.60 mmol/g) and dichloromethane, the vessel was shaken for 10 min, and the resin was allowed to swell. After removing the dichloromethane, a solution of (S)-2-((((9H-fluoren-9-yl)methoxy)carbonyl)(methyl)amino)-3-(3-chlorophenyl)propanoic acid (Fmoc-MePhe(3-Cl)-OH) synthesized by the method described in WO 2013/100132 (5.0 g, 11.47 mmol) and N-ethyl-isopropylpropan-2-amine (DIPEA) (7.4 g, 57.34 mmol) in dichloromethane (50 mL) was added and the vessel was shaken at room temperature for 3 h, after which the dichloromethane solution was removed and the resin was washed with dichloromethane three times. A solution of piperidine (20 mL) in dimethylformamide (80 mL) was added and the vessel was shaken at room temperature for 3 h, after which the solution was removed and the resin was washed with dimethylformamide three times. A solution of 4-pentenoic acid (2.29 g, 22.94 mmol), 1-hydroxy-7-azabenzotriazole (HOAt) (3.12 g, 22.94 mmol), and N,N'-diisopropylcarbodiimide (DIC) (3.18 g, 25.23 mmol) in dimethylformamide (80 mL) was added and the vessel was shaken at room temperature for 3 h. The solution was removed, the resin was washed with dimethylformamide four times and with dichloromethane four times, a 2% trifluoroacetic acid/dichloromethane solution (80 mL) was added, the vessel was shaken at room temperature for 30 min, and the solution was filtered to achieve cleavage from the resin. This operation was repeated three times. The resulting filtrate was concentrated, and the residue was then purified by reverse phase column chromatography (water/acetonitrile) to afford (S)-3-(3-chlorophenyl)-2-(N-methylpent-4-enamido)propanoic acid (Pen-MePhe(3-Cl)-OH) (1.6 g, 47%).

A solution of (S)-3-(3-chlorophenyl)-2-(N-methylpent-4-enamido)propanoic acid (Pen-MePhe(3-Cl)-OH) (1.0 g, 3.38 mmol), N-ethyl-isopropylpropan-2-amine (DIPEA) (0.87 g, 6.76 mmol), and 2-bromoacetonitrile (1.62 g, 13.51 mmol) in dichloromethane (100 mL) was stirred at room temperature for 16 h under a nitrogen atmosphere, after which the solvent was evaporated by an evaporator and the resulting residue was purified by normal phase column chromatography (petroleum ether/ethyl acetate) to afford cyanomethyl (S)-3-(3-chlorophenyl)-2-(N-methylpent-4-enamido)propanoate (Pen-MePhe(3-Cl)-$OCH_2CN$) (0.7 g, 72%).

To buffer A (86.4 mL) were added a solution of ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (150 mg, 0.208 mmol) in water (4.69 mL) and a solution of cyanomethyl (S)-3-(3-chlorophenyl)-2-(N-methylpent-4-enamido)propanoate (Pen-MePhe(3-Cl)-$OCH_2CN$) in acetonitrile (2.3 mL), and the reaction solution was stirred at room temperature for 2 h and then lyophilized. The resulting residue was purified by reverse phase column chromatography (0.1% aqueous formic

acid solution/0.1% formic acid-acetonitrile solution), after which a 80% aqueous acetic acid solution (3.0 mL) was added to the resulting mixture which was then stirred at room temperature for 4 h. The reaction solution was diluted with water and purified by reverse phase silica gel column chromatography (0.05% aqueous trifluoroacetic acid solution/0.05% trifluoroacetic acid-acetonitrile solution) to afford the title compound (Compound pc233, Pen-MePhe(3-Cl)-pCpA) (45.1 mg, 23%).
LCMS (ESI) m/z = 930 (M+H)+
Retention time: 0.51 min (analytical condition SQDFA05)

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl (2S)-2-(pent-4-enamido)-3-(4-(trifluoromethyl)phenyl)propanoate (Compound pc234, Pen-Phe(4-CF3)-pCpA)

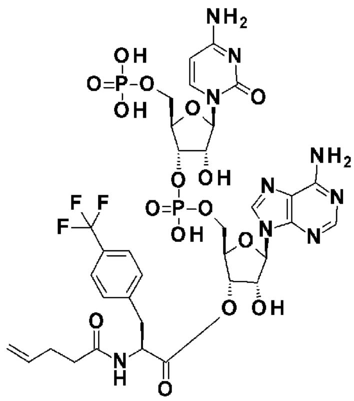

To a reaction vessel equipped with a filter were placed 2-chlorotrityl resin (15 g, 1.60 mmol/g) and dichloromethane, the vessel was shaken for 10 min, and the resin was allowed to swell. After removing the dichloromethane, a solution of commercially available (S)-2-((((9H-fluoren-9-yl)methoxy)carbonyl)amino)-3-(4-(trifluoromethyl)phenyl)propanoic acid (Fmoc-Phe(4-CF3)-OH) (5.0 g, 10.98 mmol) and N-ethyl-isopropylpropan-2-amine (DIPEA) (7.09 g, 54.84 mmol) in dichloromethane (100 mL) was added and the vessel was shaken at room temperature for 3 h, after which the dichloromethane solution was removed and the resin was washed with dichloromethane four times. A 20% piperidine/dimethylformamide solution (80 mL) was added and the vessel was shaken at room temperature for 3 h, after which the solution was removed and the resin was washed with dimethylformamide four times. A solution of 4-pentenoic acid (2.52 g, 25.17 mmol), 1-hydroxy-7-azabenzotriazole (HOAt) (3.43 g, 25.20 mmol), and N,N'-diisopropylcarbodiimide (DIC) (3.49 g, 27.65 mmol) in dimethylformamide (80 mL) was added and the vessel was shaken at room temperature for 3 h, after which the solution was removed and the resin was washed with dimethylformamide four times and with dichloromethane four times. A 2% trifluoroacetic acid/dichloromethane solution (80 mL) was added, the vessel was shaken at room temperature for 30 min, and the solution was filtered to achieve cleavage from the resin. This operation was repeated three times. The resulting filtrate was concentrated to give (S)-2-(pent-4-enamido)-3-(4-(trifluoromethyl)phenyl)propanoic acid (Pen-Phe(4-CF3)-OH) (1.6 g) as a crude product.

A solution of (S)-2-(pent-4-enamido)-3-(4-(trifluoromethyl)phenyl)propanoic acid (Pen-Phe(4-CF3)-OH) (2.60 g, 8.25 mmol), N-ethyl-isopropylpropan-2-amine (DIPEA) (3.61 g, 0.03 mol), and 2-bromoacetonitrile (4.00 g, 33.35 mmol) in dichloromethane (40 mL) was stirred at room temperature for 16 h under a nitrogen atmosphere, after which the solvent was evaporated by an evaporator and the resulting residue was purified by normal phase column chromatography (petroleum ether/ethyl acetate) to afford cyanomethyl (S)-2-(pent-4-enamido)-3-(4-(trifluoromethyl)phenyl)propanoate (Pen-Phe(4-CF3)-$OCH_2CN$) (1.6 g, 55%).

To buffer A (86.4 mL) were added a solution of ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (150 mg, 0.208 mmol) in water (4.69 mL) and a solution of cyanomethyl (S)-2-(pent-4-enamido)-3-(4-(trifluoromethyl)phenyl)propanoate (Pen-Phe(4-CF3)-$OCH_2CN$) in acetonitrile (2.3 mL), and the reaction solution was stirred at room temperature for 1 hour and 30 minutes and then lyophilized. The resulting residue was purified by reverse phase column chromatography (0.1% aqueous formic acid solution/0.1% formic acid-acetonitrile solution), after which a 80% aqueous acetic acid solution (3.0 mL) was added to the resulting mixture which was then stirred at room temperature for 5 h. The reaction solution was

diluted with water and purified by reverse phase silica gel column chromatography (0.05% aqueous trifluoroacetic acid solution/0.05% trifluoroacetic acid-acetonitrile solution) to afford the title compound (Compound pc234, Pen-Phe(4-CF3)-pCpA) (58.9 mg, 30%).
LCMS (ESI) m/z = 950 (M+H)+
Retention time: 0.50 min (analytical condition SQDFA05)

Synthesis of N-(((4-(2-(4-fluorophenyl)acetamido)benzyl)oxy)carbonyl)-O-(2-morpholinoethyl)-L-serine (Compound pc235, F-Pnaz-Ser(Et-2-Mor)-OH)

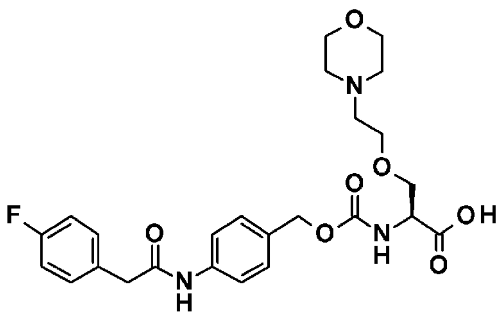

To a solution of O-(2-morpholinoethyl)-L-serine hydrochloride (1.0 g, 3.94 mmol) which is a synthetic intermediate of N-(((9H-fluoren-9-yl)methoxy)carbonyl)-O-(2-morpholinoethyl)-L-serine (Compound aa191, Fmoc-Ser(Et-2-Mor)-OH) and separately prepared 4-(2-(4-fluorophenyl)acetamido)benzyl (4-nitrophenyl) carbonate (Compound pc196) (1.39 g, 3.28 mmol) in dimethyl sulfoxide (6.0 mL) was added triethylamine ($Et_3N$) (1.66 g, 16.4 mmol) at room temperature under a nitrogen atmosphere, and the mixture was stirred for 16 h. The reaction solution was purified by reverse phase chromatography (0.05% aqueous trifluoroacetic acid solution/0.05% trifluoroacetic acid-acetonitrile solution) to afford N-(((4-(2-(4-fluorophenyl)acetamido)benzyl)oxy)carbonyl)-O-(2-morpholinoethyl)-L-serine (Compound pc235, F-Pnaz-Ser(Et-2-Mor)-OH) (1.7 g, 85%).
LCMS (ESI) m/z = 504 (M+H)+
Retention time: 0.98 min (analytical condition SMD method 13)

Synthesis of cyanomethyl N-(((4-(2-(4-fluorophenyl)acetamido)benzyl)oxy)carbonyl)-O-(2-morpholinoethyl)-L-serinate (Compound pc236, F-Pnaz-Ser(Et-2-Mor)-$OCH_2CN$)

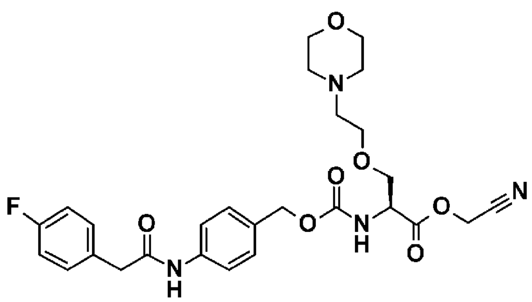

A solution of N-(((4-(2-(4-fluorophenyl)acetamido)benzyl)oxy)carbonyl)-O-(2-morpholinoethyl)-L-serine (Compound pc235, F-Pnaz-Ser(Et-2-Mor)-OH) (500 mg, 0.993 mmol), N,N-diisopropylethylamine (DIPEA) (256.68 mg, 1.986 mmol), 2-bromoacetonitrile (476.44 mg, 3.972 mmol), and a catalytic amount of dimethylformamide in dichloromethane (10 mL) was stirred at room temperature for 4 h under a nitrogen atmosphere. The reaction solution was concentrated and the resulting residue was purified by reverse phase column chromatography (water/acetonitrile) to afford cyanomethyl N-(((4-(2-(4-fluorophenyl)acetamido)benzyl)oxy)carbonyl)-O-(2-morpholinoethyl)-L-serinate (Compound pc236, F-Pnaz-Ser(Et-2-Mor)-$OCH_2CN$) (140 mg, 25%).
LCMS (ESI) m/z = 543 (M+H)+
Retention time: 1.02 min (analytical condition SMD method 13)

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl N-(((4-(2-(4-fluorophenyl)acetamido)benzyl)oxy)carbonyl)-O-(2-morpholinoethyl)-L-serinate (Compound pc237, F-Pnaz-Ser(Et-2-Mor)-pCpA)

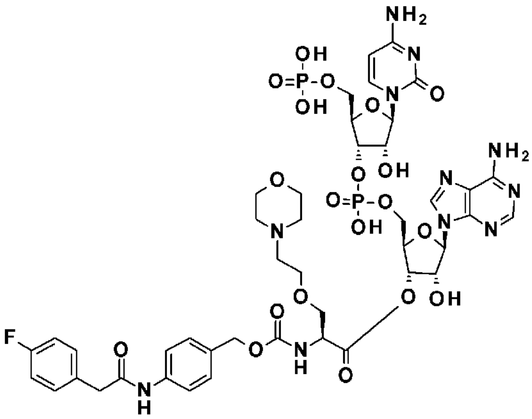

To buffer A (100 mL) was added a solution of ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (250 mg, 0.3463 mmol) in water (2.0 mL), after which a solution of cyanomethyl N-(((4-(2-(4-fluorophenyl)acetamido)benzyl)oxy)carbonyl)-O-(2-morpholinoethyl)-L-serinate (Compound pc236, F-Pnaz-Ser(Et-2-Mor)-$OCH_2CN$) (120 mg, 0.2078 mmol) in acetonitrile (4.0 mL) was added using a syringe pump over 15 min. The reaction solution was stirred at room temperature for 2 h, trifluoroacetic acid (TFA) (1.84 mL) was then added, and the mixture was purified by reverse phase column chromatography (0.05% aqueous trifluoroacetic acid solution/0.05% trifluoroacetic acid-acetonitrile solution) to afford the title compound (Compound pc237, F-Pnaz-Ser(Et-2-Mor)-pCpA) (35 mg, 9%).
LCMS (ESI) m/z = 1136 (M-H)-
Retention time: 0.40 min (analytical condition SQDFA05)

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl (2S)-1-(pent-4-enoyl)piperidine-2-carboxylate (Compound pc238, Pen-Pic(2)-pCpA)

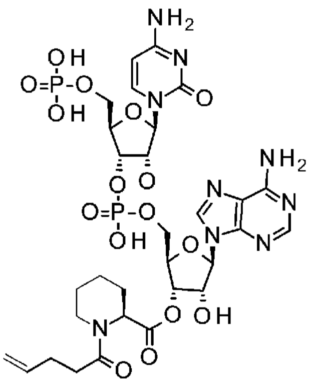

A solution of commercially available (S)-piperidine-2-carboxylic acid (H-Pic(2)-OH) (5.0 g, 38.71 mmol), 2,5-dioxopyrrolidin-1-yl pent-4-enoate synthesized by the method described in the document (Organic Letters, 2011, 13, 4906) (23.0 g, 116.28 mmol), and sodium bicarbonate (6.5 g, 77.52 mmol) in 1,4-dioxane/water (65 mL/65 mL) was stirred at 25°C for 16 h. The reaction solution was washed with petroleum ether/ethyl acetate = 1/1, and a 1 N aqueous

hydrochloric acid solution was added to the aqueous layer until the acidity became pH = 3. The resulting mixture was extracted with ethyl acetate, and the organic layer was washed with brine, dried over anhydrous sodium sulfate, filtered, and then concentrated under reduced pressure. The resulting residue was purified by normal phase silica gel column chromatography (dichloromethane/methanol) to afford (S)-1-(pent-4-enoyl)piperidine-2-carboxylic acid (Pen-Pic(2)-OH) (5.182 g).

A solution of the above (S)-1-(pent-4-enoyl)piperidine-2-carboxylic acid (Pen-Pic(2)-OH) (5.182 g, 49.15 mmol), N-ethyl-isopropylpropan-2-amine (DIPEA) (6.34 g, 0.03 mol), and 2-bromoacetonitrile (11.79 g, 98.29 mmol) in dichloromethane (112 mL) was stirred at 25°C for 16 h, after which the solvent was evaporated by an evaporator and the resulting residue was purified by normal phase column chromatography (petroleum ether/ethyl acetate) to afford cyanomethyl (S)-1-(pent-4-enoyl)piperidine-2-carboxylate (Pen-Pic(2)-$OCH_2CN$) (4.018 g).

To buffer A (50 mL) were added a solution of ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (150 mg, 0.208 mmol) in water (1.0 mL) and a solution of cyanomethyl (S)-1-(pent-4-enoyl)piperidine-2-carboxylate (Pen-Pic(2)-$OCH_2CN$) (207 mg, 0.83 mmol) in acetonitrile (1.0 mL), and the mixture was stirred at room temperature for 7 h, after which trifluoroacetic acid (1.15 mL) was added and the reaction solution was lyophilized. Water was added to the resulting residue which was then washed with ethyl acetate twice, and the aqueous layer was lyophilized. The resulting residue was purified by preparative HPCL to afford the title compound (Compound pc238, Pen-Pic(2)-pCpA) (17 mg, 10%).
LCMS (ESI) m/z = 844 (M-H)-
Retention time: 0.39 min (analytical condition SQDFA05)

<u>Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl (((4-(2-(4-fluorophenyl)acetamido)benzyl)oxy)carbonyl)-L-phenylalaninate (Compound pc239, F-Pnaz-Phe-pCpA)</u>

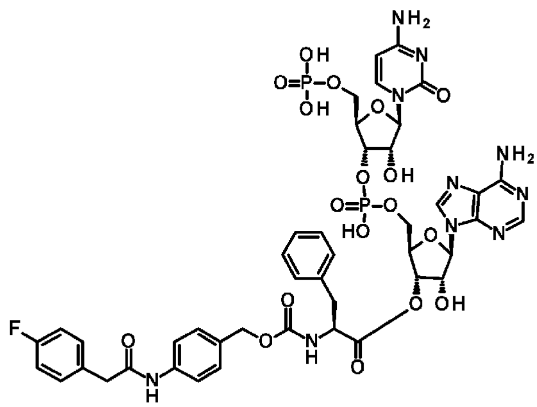

To a solution of commercially available L-phenylalanine (H-Phe-OH) (40 mg, 0.242 mmol) and separately prepared 4-(2-(4-fluorophenyl)acetamido)benzyl (4-nitrophenyl) carbonate (Compound pc196) (123 mg, 0.291 mmol) in dimethyl sulfoxide (0.25 mL) was added triethylamine (33.8 μL, 0.242 mmol), and the mixture was stirred at room temperature overnight. The reaction solution was purified by reverse phase column chromatography (0.1% aqueous formic acid solution/0.1% formic acid-acetonitrile solution) to afford (((4-(2-(4-fluorophenyl)acetamido)benzyl)oxy)carbonyl)-L-phenylalanine (F-Pnaz-Phe-OH) (104.8 mg, 96%).

To a solution of the above (((4-(2-(4-fluorophenyl)acetamido)benzyl)oxy)carbonyl)-L-phenylalanine (F-Pnaz-Phe-OH) (30 mg, 0.067 mmol) and 2-bromoacetonitrile (9.82 μL, 0.147 mmol) in acetonitrile (0.3 mL) was added N-ethyl-isopropylpropan-2-amine (DIPEA) (18.61 μL, 0.107 mol) at 0°C, and the mixture was stirred for 5 min and then stirred at room temperature overnight. The solvent was evaporated by an evaporator to afford cyanomethyl (((4-(2-(4-fluorophenyl)acetamido)benzyl)oxy)carbonyl)-L-phenylalaninate (F-Pnaz-Phe-$OCH_2CN$) as a crude product.

To buffer A (40 mL) were added ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (97 mg, 0.134 mmol) and a solution of the above crude product cyanomethyl (((4-(2-(4-fluorophenyl)acetamido)benzyl)oxy)carbonyl)-L-phenylalaninate (F-Pnaz-Phe-$OCH_2CN$) in acetonitrile (2.0 mL), and the mixture was stirred at room temperature for 1 hour and 30 minutes, after

which trifluoroacetic acid (2.0 mL) was added at 0°C and the mixture was stirred at room temperature for 30 minutes. The reaction solution was purified by reverse phase column chromatography (0.05% aqueous trifluoroacetic acid solution/0.05% trifluoroacetic acid-acetonitrile solution) to afford the title compound (Compound pc239, F-Pnaz-Phe-pCpA) (22.7 mg, 31%).
LCMS (ESI) m/z = 1083 (M-H)-
Retention time: 0.52 min (analytical condition SQDFA05)

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl N-(((4-(2-(4-fluorophenyl)acetamido)benzyl)oxy)carbonyl)-N-methyl-L-phenylalaninate (Compound pc240, F-Pnaz-MePhe-pCpA)

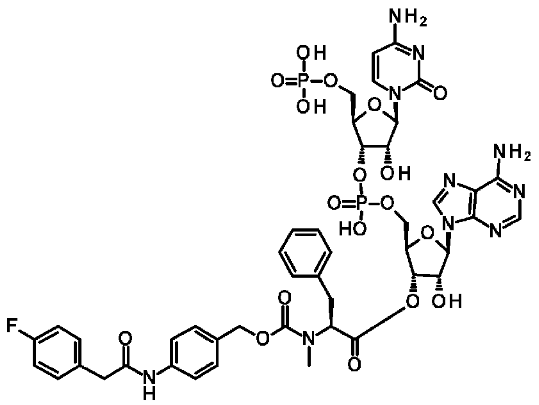

To a solution of commercially available methyl-L-phenylalanine (H-MePhe-OH) (40 mg, 0.223 mmol) and separately prepared 4-(2-(4-fluorophenyl)acetamido)benzyl (4-nitrophenyl) carbonate (Compound pc196) (114 mg, 0.268 mmol) in dimethyl sulfoxide (0.25 mL) was added triethylamine (31.1 μL, 0.223 mmol), and the mixture was stirred at room temperature overnight. The reaction solution was purified by reverse phase column chromatography (0.1% aqueous formic acid solution/0.1% formic acid-acetonitrile solution) to afford N-(((4-(2-(4-fluorophenyl)acetamido)benzyl)oxy)carbonyl)-N-methyl-L-phenylalanine (F-Pnaz-MePhe-OH) (57.1 mg, 55%).
To a solution of the above N-(((4-(2-(4-fluorophenyl)acetamido)benzyl)oxy)carbonyl)-N-methyl-L-phenylalanine (F-Pnaz-MePhe-OH) (30 mg, 0.065 mmol) and 2-bromoacetonitrile (6.06 μL, 0.090 mmol) in acetonitrile (0.3 mL) was added N-ethyl-isopropylpropan-2-amine (DIPEA) (18.05 μL, 0.103 mol) at 0°C, and the mixture was stirred for 5 min and then stirred at room temperature for 2 h. To the reaction solution was further added 2-bromoacetonitrile (6.06 μL, 0.090 mmol), and the mixture was stirred at room temperature overnight. The solvent was evaporated by an evaporator to afford cyanomethyl N-(((4-(2-(4-fluorophenyl)acetamido)benzyl)oxy)carbonyl)-N-methyl-L-phenylalaninate (F-Pnaz-MePhe-$OCH_2CN$) as a crude product.
To buffer A (40 mL) were added ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (94 mg, 0.130 mmol) and a solution of the above crude product cyanomethyl N-(((4-(2-(4-fluorophenyl)acetamido)benzyl)oxy)carbonyl)-N-methyl-L-phenylalaninate (F-Pnaz-MePhe-$OCH_2CN$) in acetonitrile (2.0 mL), and the mixture was stirred at room temperature for 2 hours and 30 minutes, after which trifluoroacetic acid (2.0 mL) was added at 0°C and the mixture was stirred at room temperature for 30 minutes. The reaction solution was purified by reverse phase column chromatography (0.05% aqueous trifluoroacetic acid solution/0.05% trifluoroacetic acid-acetonitrile solution) to afford the title compound (Compound pc240, F-Pnaz-MePhe-pCpA) (21.54 mg, 30%).
LCMS (ESI) m/z = 1097 (M-H)-
Retention time: 0.54 min (analytical condition SQDFA05)

Synthesis of (S)-2-(pent-4-enamido)-3-(pyridin-3-yl)propanoic acid (Compound pc241, Pen-Ala(3-Pyr)-OH)

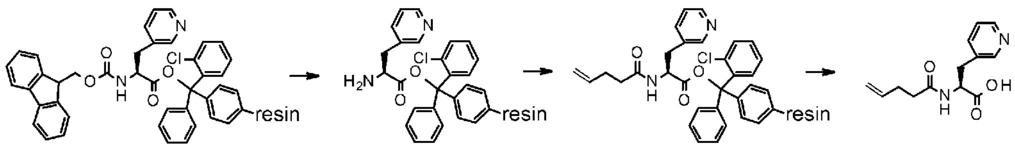

**pc241+++** **pc241++** **pc241+** **pc241**

2-Chlorotrityl resin (1.60 mmol/g, 3.21 g) was added to dichloromethane (20 mL), the vessel was shaken for 10 min, and the resin was allowed to swell. After removing the dichloromethane, a solution of commercially available (S)-2-((((9H-fluoren-9-yl)methoxy)carbonyl)amino)-3-(pyridin-3-yl)propanoic acid (Fmoc-Ala(3-Pyr)-OH) (2.00 g, 5.15 mmol) and N-ethyl-isopropylpropan-2-amine (DIPEA) (1.33 g, 10.3 mmol) in dichloromethane (33 mL), and the vessel was shaken at room temperature for 30 min, after which methanol (2.78 mL) was added and the vessel was further shaken for 1 h. After removing the dichloromethane solution, the resin was washed with dichloromethane (15 mL) twice to afford Compound pc241+++. To the obtained Compound pc241++ was added a 2% 1,8-diazabicyclo[5.4.0]-7-undecene (DBU)/dimethylformamide solution (20 mL), and the vessel was shaken at room temperature for 15 min, after which the solution was removed and the resin was washed with dimethylformamide (20 mL) five times to afford Compound pc241++ (2.35 g, 5.14 mmol). To the obtained Compound pc241++ was added a solution of 4-pentenoic acid (2.06 g, 20.6 mmol), 1-hydroxy-7-azabenzotriazole (HOAt) (2.10 g, 15.4 mmol), and N,N'-diisopropylcarbodiimide (DIC) (3.24 g, 25.7 mmol) in dimethylformamide (20 mL), and the vessel was shaken at room temperature overnight. The solution was removed and the resin was washed with dimethylformamide (20 mL) four times and with dichloromethane (20 mL) four times to afford Compound pc241+ (2.76 g, 5.12 mmol). To the obtained Compound pc241+ was added dichloromethane (30 mL), the vessel was shaken for 15 min, and the resin was allowed to swell. After removing the dichloromethane, 2,2,2-trifluoroethanol (TFE) (50 mL)/dichloromethane (50 mL) was added, the vessel was shaken at room temperature for 2.5 hours, and Compound pc241 was cleaved from the resin. The cleaving solution TFE/DCM (100 mL) was removed, and the resin was further washed with TFE (30 mL)/DCM (30 mL) twice. This washing solution (60 mL in total) and the above cleaving solution (100 mL) were combined and evaporated by an evaporator, and the resulting residue was purified by reverse phase column chromatography (0.1% aqueous formic acid solution/0.1% formic acid-acetonitrile solution) to afford (S)-2-(pent-4-enamido)-3-(pyridin-3-yl)propanoic acid (Compound pc241, Pen-Ala(3-Pyr)-OH) (564 mg, 44%).
LCMS (ESI) m/z = 247 (M-H)-
Retention time: 0.33 min (analytical condition SQDFA05)

Synthesis of cyanomethyl (S)-2-(pent-4-enamido)-3-(pyridin-3-yl)propanoate (Compound pc242, Pen-Ala(3-Pyr)-$OCH_2CN$)

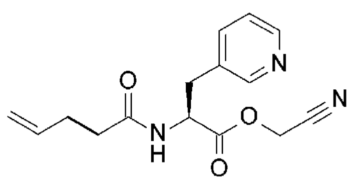

(S)-2-(Pent-4-enamido)-3-(pyridin-3-yl)propanoic acid (Compound pc241, Pen-Ala(3-Pyr)-OH) (100 mg, 0.40 mmol) and N-ethyl-isopropylpropan-2-amine (DIPEA) (106 μL, 0.60 mmol) were dissolved in dimethylformamide (1.00 mL) under a nitrogen atmosphere, 2-bromoacetonitrile (28.1 μL, 0.40 mmol) was added at room temperature, and the mixture was stirred at room temperature for 30 min, after which N-ethyl-isopropylpropan-2-amine (DIPEA) (35 μL, 0.20mmol) and 2-bromoacetonitrile (8.43 μL, 0.12 mmol) were added again. After stirring at room temperature for 10 min, a saturated aqueous ammonium chloride solution was added and the mixture was extracted with t-butyl methyl ether. The organic layer was washed with brine and dried over anhydrous sodium sulfate, and the solvent was then evaporated by an evaporator to give a crude product cyanomethyl (S)-2-(pent-4-enamido)-3-(pyridin-3-yl)propanoate (Compound pc242, Pen-Ala(3-Pyr)-$OCH_2CN$). The obtained crude product was dissolved in acetonitrile (0.57 mL) and used as such in the next step.
LCMS (ESI) m/z = 288 (M+H)+
Retention time: 0.40 min (analytical condition SQDFA05)

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl (2S)-2-(pent-4-enamido)-3-(pyridin-3-yl)propanoate (Compound pc243, Pen-Ala(3-Pyr)-pCpA)

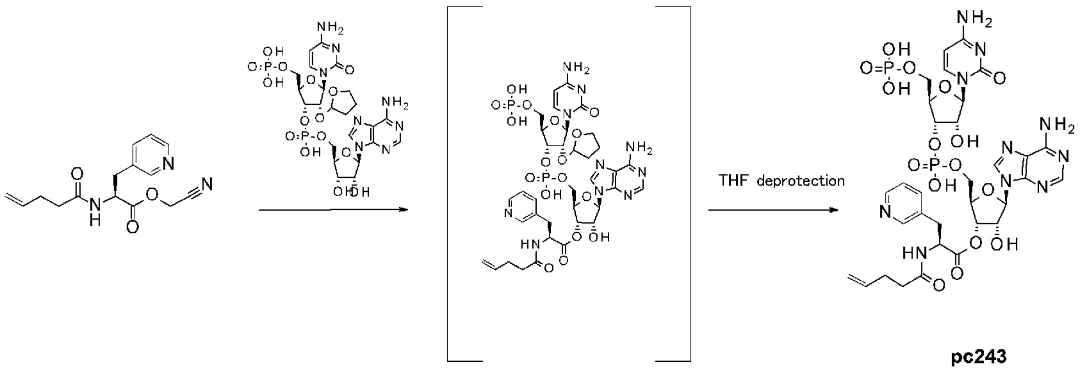


pc243

((2R,3R,4R,5R)-5-(4-Amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (59.2 mg, 0.082 mmol) was dissolved in buffer A (17 mL), a solution of cyanomethyl (S)-2-(pent-4-enamido)-3-(pyridin-3-yl)propanoate (Compound pc242, Pen-Ala(3-Pyr)-$OCH_2CN$) in acetonitrile (0.57 mL) was added, and the mixture was stirred at room temperature for 60 min. To the reaction solution was added acetic acid (17 mL) and the mixture was stirred for 1 h, after which the reaction solution was purified by reverse phase column chromatography (0.05% aqueous trifluoroacetic acid solution/0.05% trifluoroacetic acid-acetonitrile solution) to afford the title compound (Compound pc243, Pen-Ala(3-Pyr)-pCpA) (14.8 mg, 20.5%).
LCMS (ESI) m/z = 896 (M+H)+
Retention time: 0.39, 0.41 min (analytical condition SMD method 6)

Synthesis of (S)-2-(N-methylpent-4-enamido)-3-(pyridin-3-yl)propanoic acid (Compound pc244, Pen-MeAla(3-Pyr)-OH)

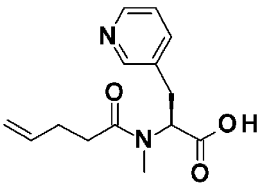

To a reaction vessel equipped with a filter were placed 2-chlorotrityl resin (1.31 g, 1.60 mmol/g) and dichloromethane (15 mL), the vessel was shaken for 10 min, and the resin was allowed to swell. After removing the dichloromethane, a solution of (S)-2-((((9H-fluoren-9-yl)methoxy)carbonyl)(methyl)amino)-3-(pyridin-3-yl)propanoic acid (Compound aa197, Fmoc-MeAla(3-Pyr)-OH) (846.6 mg, 2.104 mmol) and N-ethyl-isopropylpropan-2-amine (DIPEA) (816 mg, 6.311 mmol) in dichloromethane (25 mL) was added and the vessel was shaken at room temperature for 30 min, after which the unreacted part of the resin was capped with methanol (1.704 mL). Filtration was performed, and the resin was washed with dichloromethane four times. The resin was washed with dimethylformamide, a 2% 1,8-diazabicyclo[5.4.0]-7-undecene (DBU)/dimethylformamide solution (15 mL) was then added, and the vessel was shaken at room temperature for 15 min, after which the solution was removed and the resin was washed with dimethylformamide (20 mL) five times. A solution of 4-pentenoic acid (0.841 g, 8.40 mmol), 1-hydroxy-7-azabenzotriazole (HOAt) (0.858 g, 6.30 mmol), and N,N'-diisopropylcarbodiimide (DIC) (1.325 g, 10.50 mmol) in dimethylformamide (15 mL) was added and the vessel was shaken at room temperature overnight, after which the solution was removed and the resin was washed with dimethylformamide (15 mL) four times and with dichloromethane (15 mL) four times. A 2,2,2-trifluoroethanol (TFE)/dichloromethane solution (1/1, 50 mL) was added, the vessel was shaken at room temperature for 2 hours and 30 minutes, the solution was filtered to achieve cleavage from the resin, and the resin was then washed with a 2,2,2-trifluoroethanol (TFE)/dichloromethane solution (1/1, 30 mL) twice. The resulting filtrate was concentrated, and the residue was purified by reverse phase column chromatography (0.1% aqueous formic acid solution/0.1% formic acid-acetonitrile solution) to afford (S)-2-(N-methylpent-4-enamido)-3-(pyridin-3-yl)propanoic acid (Compound pc244, Pen-MeAla(3-Pyr)-OH) (196.2 mg, 36%).
LCMS (ESI) m/z = 263 (M+H)+
Retention time: 0.39 min (analytical condition SQDFA05)

Synthesis of (2S)-(2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl 2-(N-methylpent-4-enamido)-3-(pyridin-3-yl)propanoate (Compound pc245, Pen-MeAla(3-Pyr)-pCpA)

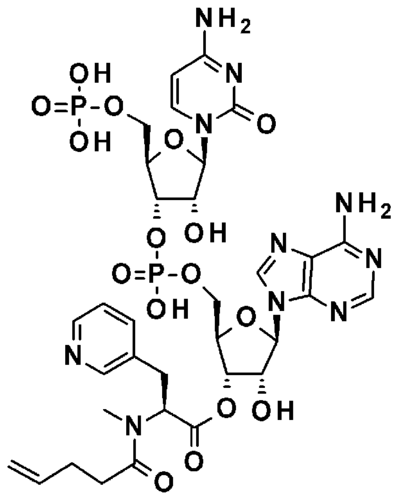

To a solution of (S)-2-(N-methylpent-4-enamido)-3-(pyridin-3-yl)propanoic acid (Compound pc244, Pen-MeAla(3-Pyr)-OH) (196.2 mg, 0.748 mmol) and 2-bromoacetonitrile (52.2 μL, 0.748 mmol) in dimethylformamide (1.87 mL) was added N-ethyl-isopropylpropan-2-amine (DIPEA) (196 μL, 1.122 mol) at room temperature under a nitrogen atmosphere, and the mixture was stirred for 30 min. To the reaction solution were further added 2-bromoacetonitrile (10.43 μL, 0.150 mmol) and N-ethyl-isopropylpropan-2-amine (DIPEA) (40 μL, 0.229 mmol), and the mixture was stirred at room temperature for 15 min. To the reaction solution was added a saturated aqueous ammonium chloride solution, and the mixture was extracted with ethyl acetate, after which the organic layer was washed with brine, dried over anhydrous sodium sulfate, and filtered. The resulting organic layer was concentrated under reduced pressure to afford cyanomethyl (S)-2-(N-methylpent-4-enamido)-3-(pyridin-3-yl)propanoate (Pen-MeAla(3-Pyr)-$OCH_2CN$) as a crude product.

To buffer A (37.14 mL) were added a solution of ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (134 mg, 0.186 mmol) in water (2.0 mL) and a solution of the above crude product cyanomethyl (S)-2-(N-methylpent-4-enamido)-3-(pyridin-3-yl)propanoate (Pen-MeAla(3-Pyr)-$OCH_2CN$) in acetonitrile (1.0 mL) dropwise and the mixture was stirred at room temperature for 1 hour, after which acetic acid (37.1 mL) was added to the reaction solution, and the mixture was further stirred at room temperature for 2 hours and 30 minutes. The reaction solution was diluted with water and purified by reverse phase silica gel column chromatography (0.05% aqueous trifluoroacetic acid solution/0.05% trifluoroacetic acid-acetonitrile solution) to afford the title compound (Compound pc245, Pen-MeAla(3-Pyr)-pCpA) (42 mg, 25%).
LCMS (ESI) m/z = 895 (M-H)-
Retention time: 0.41 min (analytical condition SMD method 6)

Synthesis of methyl N-((benzyloxy)carbonyl)-O-(3,3,3-trifluoro-2-hydroxypropyl)-L-serinate (Compound pc246, Cbz-Ser(1-CF3-EtOH)-OMe)

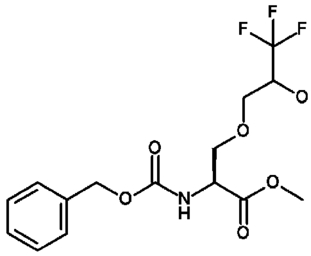

To a solution of 3,3,3-trifluoropropane-1,2-diol (500 mg, 3.85 mmol) and 1-benzyl 2-methyl (S)-aziridine-1,2-dicarboxylate (Cbz-Azy-OMe) (603 mg, 2.56 mmol) in dichloromethane (7 mL) was added boron trifluoride-diethyl ether complex ($BF_3 \cdot OEt_2$) (54.7 mg, 0.39 mmol) at 0°C under a nitrogen atmosphere, and the reaction solution was then stirred at 0°C for 30 min. The reaction solution was diluted with dichloromethane, washed with a saturated aqueous sodium bicarbonate solution, water, and brine, then dried over anhydrous sodium sulfate, and filtered, and the solvent was then evaporated under reduced pressure. The resulting residue was purified by reverse phase column chromatography (water/acetonitrile) to afford methyl N-((benzyloxy)carbonyl)-O-(3,3,3-trifluoro-2-hydroxypropyl)-L-serinate (Compound pc246, Cbz-Ser(1-CF3-EtOH)-OMe) (250 mg, 27%).
LCMS (ESI) m/z = 366 (M+H)+
Retention time: 1.13 min (analytical condition SMD method 39)

Synthesis of N-(((4-(2-(4-fluorophenyl)acetamido)benzyl)oxy)carbonyl)-O-(3,3,3-trifluoro-2-hydroxypropyl)-L-serine (Compound pc247, F-Pnaz-Ser(1-CF3-EtOH)-OH)

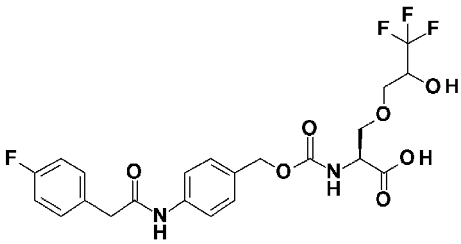

To a solution of calcium chloride (911 mg, 8.21 mmol) and lithium hydroxide (57 mg, 2.19 mmol) in water (2.3 mL) was added a solution of isopropanol (9 mL) and methyl N-((benzyloxy)carbonyl)-O-(3,3,3-trifluoro-2-hydroxypropyl)-L-serinate (Compound pc246, Cbz-Ser(1-CF3-EtOH)-OMe) (200 mg, 0.55 mmol) in tetrahydrofuran (2.3 mL), and the reaction solution was stirred at room temperature for 2 h. To the reaction solution was added a 1 M aqueous hydrochloric acid solution, and the mixture was extracted with ethyl acetate twice. The organic layers were washed with brine, dried over anhydrous sodium sulfate, and then filtered, and the solvent was evaporated under reduced pressure to afford N-((benzyloxy)carbonyl)-O-(3,3,3-trifluoro-2-hydroxypropyl)-L-serine (Cbz-Ser(1-CF3-EtOH)-OH) (346 mg) as a crude product.

A mixture of the resulting crude product N-((benzyloxy)carbonyl)-O-(3,3,3-trifluoro-2-hydroxypropyl)-L-serine (Cbz-Ser(1-CF3-EtOH)-OH) (300 mg, 0.85 mmol) and 10% Pd/C (150 mg) in methanol (7 mL) was stirred at room temperature for 16 h under a hydrogen atmosphere and then filtered, and the solvent was evaporated from the resulting solution under reduced pressure to afford O-(3,3,3-trifluoro-2-hydroxypropyl)-L-serine (H-Ser(1-CF3-EtOH)-OH) (49 mg) as a crude product.

A solution of the crude product O-(3,3,3-trifluoro-2-hydroxypropyl)-L-serine (H-Ser(1-CF3-EtOH)-OH) obtained by the above method (500 mg, 2.30 mmol), 4-(2-(4-fluorophenyl)acetamido)benzyl (4-nitrophenyl) carbonate (Compound pc196) (814 mg, 1.92 mmol), and triethylamine (388 mg, 3.84 mmol) in dimethyl sulfoxide (2.5 mL) was stirred at room temperature for 16 h under a nitrogen atmosphere. The reaction solution was purified by reverse phase column chromatography (0.1% aqueous formic acid solution/0.1% formic acid-acetonitrile solution) to afford N-(((4-(2-(4-fluorophenyl)acetamido)benzyl)oxy)carbonyl)-O-(3,3,3-trifluoro-2-hydroxypropyl)-L-serine (Compound pc247, F-Pnaz-Ser(1-CF3-EtOH)-OH) (726 mg, 75%).
LCMS (ESI) m/z = 503 (M+H)+
Retention time: 2.05 min (analytical condition SMD method 48)

Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl N-(((4-(2-(4-fluorophenyl)acetamido)benzyl)oxy)carbonyl)-O-(3,3,3-trifluoro-2-hydroxypropyl)-L-serinate (Compound pc248, F-Pnaz-Ser(1-CF3-EtOH)-pCpA)

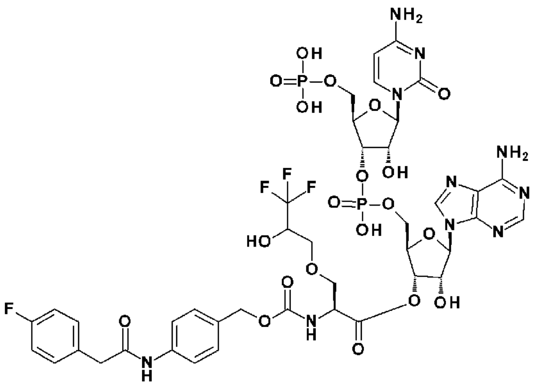

A solution of N-(((4-(2-(4-fluorophenyl)acetamido)benzyl)oxy)carbonyl)-O-(3,3,3-trifluoro-2-hydroxypropyl)-L-serine (Compound pc247, F-Pnaz-Ser(1-CF3-EtOH)-OH) (500 mg, 0.995 mmol), 2-bromoacetonitrile (477.49 mg, 3.981 mmol), and N,N-diisopropylethylamine (DIPEA) (385.86 mg, 2.986 mmol) in dichloromethane (20 mL) was stirred at room temperature for 16 h under a nitrogen atmosphere. The reaction solution was concentrated under reduced pressure, and the resulting crude product was purified by reverse phase column chromatography (water/acetonitrile) to afford cyanomethyl N-(((4-(2-(4-fluorophenyl)acetamido)benzyl)oxy)carbonyl)-O-(3,3,3-trifluoro-2-hydroxypropyl)-L-serinate (F-Pnaz-Ser(1-CF3-EtOH)-$OCH_2CN$) (150 mg, 28%).

To buffer A (100 mL) was added ((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc03) (400 mg, 0.554 mmol), after which a solution of cyanomethyl N-(((4-(2-(4-fluorophenyl)acetamido)benzyl)oxy)carbonyl)-O-(3,3,3-trifluoro-2-hydroxypropyl)-L-serinate (F-Pnaz-Ser(1-CF3-EtOH)-$OCH_2CN$) (149.88 mg, 0.277 mmol) in acetonitrile (0.5 mL) was added using a syringe pump over 15 min or more. The reaction solution was stirred at room temperature for 30 min, and trifluoroacetic acid (2.3 mL) was then added. The reaction solution was lyophilized, and the resulting residue was purified by reverse phase column chromatography (0.05% aqueous trifluoroacetic acid solution/0.05% trifluoroacetic acid-acetonitrile solution) to afford the title compound (Compound pc248, F-Pnaz-Ser(1-CF3-EtOH)-pCpA) (46.1 mg, 7%).
LCMS (ESI) m/z = 1137 (M+H)+
Retention time: 0.48 min (analytical condition SQDFA05)

2-1-2. Preparation of amino acids for use in the translational synthesis by ARS

H-Phe(4-OCHF2)-OH, an amino acid for use in the translational synthesis by ARS, was purchased from ASIBA PHARMTECH, Inc. H-Gln(Me)-OH (Compound aa30) was synthesized as follows.

Synthesis of N5-methyl-L-glutamine (Compound aa30, H-Gln(Me)-OH)

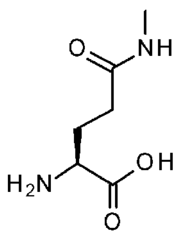

N2-(((9H-Fluoren-9-yl)methoxy)carbonyl)-N5-methyl-L-glutamine (Compound aa98, Fmoc-Gln(Me)-OH) (210 mg, 0.55 mmol) was dissolved in dichloromethane (DCM) (1.50 mL), 4-(3-phenylpropyl)piperidine (0.233 mL, 1.10 mmol) was added at room temperature, and the mixture was stirred for 4 hours and 30 minutes. Water was added to the reaction solution, and the mixture was purified by reverse phase column chromatography to afford N5-methyl-L-glutamine (Compound aa30, H-Gln(Me)-OH) (78 mg, 89%).
LCMS (ESI) m/z = 161 (M+H)+
Retention time: 0.15 min (analytical condition SQDFA05)

2-2. Synthesis of acylated tRNAs

Acylated tRNAs used for panning were prepared by the procedure described in the patent document (WO 2013/100132). The base sequence of the tRNA (CA deficient) used is provided in Table 7. Each elongator aminoacylated tRNAs provided in Table 8 was prepared. Hereafter, translation solutions were prepared at a final concentration of 20 μM when used for translation.

[Table 7]

tRNA Glu UAG (-CA) RNA base sequence for CUA codon

GUCCCCUUCGUCUAGAGGCCCAGGACACCGCCCUuagACGGCGGUAACAGGGGUUCGAAUCCCCUAGGGGAC
GC （SEQ ID NO： 1 ）

[Table 8]

| Amino acid | tRNA body | Elongator aminoacylated tRNA* |
|---|---|---|
| MeGly | tRNA Glu UAG (-CA) | MeGly-tRNA Glu UAG (-CA) |
| MeAla(4-Thz) | tRNA Glu UAG (-CA) | MeAla(4-Thz)-tRNA Glu UAG (-CA) |
| MePhe(4-Cl) | tRNA Glu UAG (-CA) | MePhe(4-Cl)-tRNA Glu UAG (-CA) |
| Phe(3-Cl) | tRNA Glu UAG (-CA) | Phe(3-Cl)-tRNA Glu UAG (-CA) |
| Met(02) | tRNA Glu UAG (-CA) | Met(O2)-tRNA Glu UAG (-CA) |
| Phg | tRNA Glu UAG (-CA) | Phg-tRNA Glu UAG (-CA) |
| MeSer | tRNA Glu UAG (-CA) | MeSer-tRNA Glu UAG (-CA) |
| Asp(SMe) | tRNA Glu UAG (-CA) | Asp(SMe)-tRNA Glu UAG (-CA) |
| Ser(Me) | tRNA Glu UAG (-CA) | Ser(Me)-tRNA Glu UAG (-CA) |
| Ser(nPr) | tRNA Glu UAG (-CA) | Ser(nPr)-tRNA Glu UAG (-CA) |
| MeSer(nPr) | tRNA Glu UAG (-CA) | MeSer(nPr)-tRNA Glu UAG (-CA) |
| Ser(iPen) | tRNA Glu UAG (-CA) | Ser(iPen)-tRNA Glu UAG (-CA) |
| MeSer(iPen) | tRNA Glu UAG (-CA) | MeSer(iPen)-tRNA Glu UAG (-CA) |
| Hse(Et) | tRNA Glu UAG (-CA) | Hse(Et)-tRNA Glu UAG (-CA) |
| MeHse(Et) | tRNA Glu UAG (-CA) | MeHse(Et)-tRNA Glu UAG (-CA) |
| Hnl(7-F2) | tRNA Glu UAG (-CA) | Hnl(7-F2)-tRNA Glu UAG (-CA) |
| MeHnl(7-F2) | tRNA Glu UAG (-CA) | MeHnl(7-F2)-tRNA Glu UAG (-CA) |
| Ser(F(4)nPr) | tRNA Glu UAG (-CA) | Ser(F(4)nPr)-tRNA Glu UAG (-CA) |
| MeSer(F(4)nPr) | tRNA Glu UAG (-CA) | MeSer(F(4)nPr)-tRNA Glu UAG (-CA) |
| Nle(6-OH) | tRNA Glu UAG (-CA) | Nle(6-OH)-tRNA Glu UAG (-CA) |
| Ser(EtOH) | tRNA Glu UAG (-CA) | Ser(EtOH)-tRNA Glu UAG (-CA) |
| MeSer(EtOH) | tRNA Glu UAG (-CA) | MeSer(EtOH)-tRNA Glu UAG (-CA) |
| Ser(S-2-PrOH) | tRNA Glu UAG (-CA) | Ser(S-2-PrOH)-tRNA Glu UAG (-CA) |
| MeSer(S-2-PrOH) | tRNA Glu UAG (-CA) | MeSer(S-2-PrOH)-tRNA Glu UAG (-CA) |
| Ser(R-2-PrOH) | tRNA Glu UAG (-CA) | Ser(R-2-PrOH)-tRNA Glu UAG (-CA) |
| MeSer(R-2-PrOH) | tRNA Glu UAG (-CA) | MeSer(R-2-PrOH)-tRNA Glu UAG (-CA) |
| Ser(tBuOH) | tRNA Glu UAG (-CA) | Ser(tBuOH)-tRNA Glu UAG (-CA) |
| MeSer(tBuOH) | tRNA Glu UAG (-CA) | MeSer(tBuOH)-tRNA Glu UAG (-CA) |
| Ser(2-Me-BuOH) | tRNA Glu UAG (-CA) | Ser(2-Me-BuOH)-tRNA Glu UAG (-CA) |
| MeSer(2-Me-BuOH) | tRNA Glu UAG (-CA) | MeSer(2-Me-BuOH)-tRNA Glu UAG (-CA) |
| Gln(Me) | tRNA Glu UAG (-CA) | Gln(Me)-tRNA Glu UAG (-CA) |
| Gln(Me2) | tRNA Glu UAG (-CA) | Gln(Me2)-tRNA Glu UAG (-CA) |
| MeGln(Me2) | tRNA Glu UAG (-CA) | MeGln(Me2)-tRNA Glu UAG (-CA) |

(continued)

| Amino acid | tRNA body | Elongator aminoacylated tRNA* |
|---|---|---|
| Ser(NtBu-Aca) | tRNA Glu UAG (-CA) | Ser(NtBu-Aca)-tRNA Glu UAG (-CA) |
| MeSer(NtBu-Aca) | tRNA Glu UAG (-CA) | MeSer(NtBu-Aca)-tRNA Glu UAG (-CA) |
| Hph | tRNA Glu UAG (-CA) | Hph-tRNA Glu UAG (-CA) |
| MeHph | tRNA Glu UAG (-CA) | MeHph-tRNA Glu UAG (-CA) |
| Ser(Ph-2-Cl) | tRNA Glu UAG (-CA) | Ser(Ph-2-Cl)-tRNA Glu UAG (-CA) |
| MeSer(Ph-2-Cl) | tRNA Glu UAG (-CA) | MeSer(Ph-2-Cl)-tRNA Glu UAG (-CA) |
| Ser(Ph-3-Cl) | tRNA Glu UAG (-CA) | Ser(Ph-3-Cl)-tRNA Glu UAG (-CA) |
| Hph(2-Cl) | tRNA Glu UAG (-CA) | Hph(2-Cl)-tRNA Glu UAG (-CA) |
| MeHph(2-Cl) | tRNA Glu UAG (-CA) | MeHph(2-Cl)-tRNA Glu UAG (-CA) |
| Hph(3-Cl) | tRNA Glu UAG (-CA) | Hph(3-Cl)-tRNA Glu UAG (-CA) |
| MeHph(3-Cl) | tRNA Glu UAG (-CA) | MeHph(3-Cl)-tRNA Glu UAG (-CA) |
| Hph(4-Cl) | tRNA Glu UAG (-CA) | Hph(4-Cl)-tRNA Glu UAG (-CA) |
| MeHph(4-Cl) | tRNA Glu UAG (-CA) | MeHph(4-Cl)-tRNA Glu UAG (-CA) |
| Ser(3-F-5-Me-Pyr) | tRNA Glu UAG (-CA) | Ser(3-F-5-Me-Pyr)-tRNA Glu UAG (-CA) |
| MeSer(3-F-5-Me-Pyr) | tRNA Glu UAG (-CA) | MeSer(3-F-5-Me-Pyr)-tRNA Glu UAG (-CA) |
| Phe{#(CH2)2} | tRNA Glu UAG (-CA) | Phe{#(CH2)2}-tRNA Glu UAG (-CA) |
| MePhe{#(CH2)2} | tRNA Glu UAG (-CA) | MePhe{#(CH2)2}-tRNA Glu UAG (-CA) |
| Ser(Bn) | tRNA Glu UAG (-CA) | Ser(Bn)-tRNA Glu UAG (-CA) |
| MeSer(Bn) | tRNA Glu UAG (-CA) | MeSer(Bn)-tRNA Glu UAG (-CA) |
| Hyp(Et) | tRNA Glu UAG (-CA) | Hyp(Et)-tRNA Glu UAG (-CA) |
| Abu(pip-4-F2) | tRNA Glu UAG (-CA) | Abu(pip-4-F2)-tRNA Glu UAG (-CA) |
| MeAbu(pip-4-F2) | tRNA Glu UAG (-CA) | MeAbu(pip-4-F2)-tRNA Glu UAG (-CA) |
| MeAbu(pip-3-F2) | tRNA Glu UAG (-CA) | MeAbu(pip-3-F2)-tRNA Glu UAG (-CA) |
| Ala(3-Pyr-4-NMe2) | tRNA Glu UAG (-CA) | Ala(3-Pyr-4-NMe2)-tRNA Glu UAG (-CA) |
| MeAla(3-Pyr-4-NMe2) | tRNA Glu UAG (-CA) | MeAla(3-Pyr-4-NMe2)-tRNA Glu UAG (-CA) |
| nPenGly | tRNA Glu UAG (-CA) | nPenGly-tRNA Glu UAG (-CA) |
| nHexGly | tRNA Glu UAG (-CA) | nHexGly-tRNA Glu UAG (-CA) |
| (PhEt)NGly | tRNA Glu UAG (-CA) | (PhEt)NGly-tRNA Glu UAG (-CA) |
| (EtOEt)NGly | tRNA Glu UAG (-CA) | (EtOEt)NGly-tRNA Glu UAG (-CA) |
| (PhOEt)NGly | tRNA Glu UAG (-CA) | (PhOEt)NGly-tRNA Glu UAG (-CA) |
| Pro(pip-4-F2) | tRNA Glu UAG (-CA) | Pro(pip-4-F2)-tRNA Glu UAG (-CA) |
| cisPro(pip-4-F2) | tRNA Glu UAG (-CA) | cisPro(pip-4-F2)-tRNA Glu UAG (-CA) |
| MeNva(5-F2) | tRNA Glu UAG (-CA) | MeNva(5-F2)-tRNA Glu UAG (-CA) |

(continued)

| Amino acid | tRNA body | Elongator aminoacylated tRNA* |
|---|---|---|
| MePhe(3-Cl) | tRNA Glu UAG (-CA) | MePhe(3-Cl)-tRNA Glu UAG (-CA) |
| Abu(Mor) | tRNA Glu UAG (-CA) | Abu(Mor)-tRNA Glu UAG (-CA) |
| Phe(4-CF3) | tRNA Glu UAG (-CA) | Phe(4-CF3)-tRNA Glu UAG (-CA) |
| 2-(pip-4-F2)-EtGly | tRNA Glu UAG (-CA) | 2-(pip-4-F2)-EtGly-tRNA Glu UAG (-CA) |
| Phe(4-CHF2) | tRNA Glu UAG (-CA) | Phe(4-CHF2)-tRNA Glu UAG (-CA) |
| Leu | tRNA Glu UAG (-CA) | Leu-tRNA Glu UAG (-CA) |
| MeVal | tRNA Glu UAG (-CA) | MeVal-tRNA Glu UAG (-CA) |
| Ser(Et-2-Mor) | tRNA Glu UAG (-CA) | Ser(Et-2-Mor)-tRNA Glu UAG (-CA) |
| Ser(1-CF3-EtOH) | tRNA Glu UAG (-CA) | Ser(1-CF3-EtOH)-tRNA Glu UAG (-CA) |

*tRNA Glu UAG RNA base sequence for CUA codon:

GUCCCCUUCGUCUAGAGGCCCAGGACACCGCCCUuagACGGCGGUAACAGGGGUUC GAAUCCCCUAGGGGACGCCA (SEQ ID NO: 2)

2-3. Ribosomal synthesis of peptide compounds

Peptide compounds containing desired unnatural amino acids were ribosomally synthesized by adding tRNAs aminoacylated by various amino acids to a cell-free translation system and initiating translation. The translation system used was PURE system, a reconstituted cell-free protein synthesis system derived from prokaryotes. Specifically, the ribosomal synthesis was performed by adding template mRNA (SEQ ID NO: 12) and 0.25 mM each of natural amino acids encoded by each template mRNAs to a translation solution (1 mM GTP, 1 mM ATP, 20 mM creatine phosphate, 50 mM HEPES-KOH pH 7.6, 100 mM potassium acetate, 10 mM magnesium acetate, 2 mM spermidine, 1 mM dithiothreitol, 1.5 mg/ml E. coli MRE600 (RNase negative)-derived tRNA (Roche), 0.1 mM 10-HCO-H4 folate, 0.6 μM methionyl-tRNA transformylase, 0.26 μM EF-G, 0.24 μM RF2, 0.17 μM RF3, 0.5 μM RRF, 4 μg/ml creatine kinase, 3 μg/ml myokinase, 2 units/ml inorganic pyrophosphatase, 1.1 μg/ml nucleoside diphosphate kinase, 2.7 μM IF1, 0.4 μM IF2, 1.5 μM IF3, 40 μM EF-Tu, 28 μM EF-Ts, 1.2 μM ribosome, 0.73 μM AlaRS, 0.03 μM ArgRS, 0.38 μM AsnRS, 0.13 μM AspRS, 0.02 μM CysRS, 0.06 μM GlnRS, 0.23 μM GluRS, 0.09 μM GlyRS, 0.02 μM HisRS, 0.4 μM IleRS, 0.04 μM LeuRS, 0.11 μM LysRS, 0.03 μM MetRS, 0.68 μM PheRS, 0.16 μM ProRS, 0.04 μM SerRS, 0.09 μM ThrRS, 0.03 μM TrpRS, 0.02 μM TyrRS, and 0.02 μM ValRS), adding an aminoacylated tRNA to the translation reaction mixture, and allowing the mixture to stand at 37°C for 30 min to 1 h. The translation products were identified by measuring the MALDI-MS spectra using α-cyano-4-hydroxycinnamic acid as a matrix.

2-4. Translational synthesis of peptide compounds containing unnatural amino acids

To 1 μM template mRNA (SEQ ID NO: 12) were added 0.25 mM each of 19 protein-constituent amino acids excluding Leu (Ala, Arg, Asn, Asp, Cys, Gln, Glu, Gly, His, Ile, Lys, Met, Phe, Pro, Ser, Thr, Trp, Tyr, and Val), the above-described transcription translation solution containing 20 μM acylated tRNA, and when translating an F-Pnaz protected unnatural amino acid, 0.5 μM E. coli-derived penicillin G amidase (PGA), and the mixture was incubated at 37°C for 60 min. When Phe(4-OCHF2) was evaluated, aminoacyl-tRNA was not added, and Phe(4-OCHF2) was added at 1 mM instead of amino acid Phe. The obtained translation reaction products were purified by SEP C-TIP (Nikkyo Technos) and analyzed by MALDI-MS. As a result, mass spectra (MS) indicating the molecular weights of peptide compounds containing unnatural amino acids were observed as main products (Figs. 3 to 6). Template mRNAs and expected translated peptide compounds (SEQ ID NO: 11), and the molecular weights thereof (calculated) are described in Table 9 below (separately translated).

[Table 9]

| Amino acid | Translated peptide compound | Molecular weight m/z: [M+H]+ |
|---|---|---|
| MeGly | MKAGPGFM-MeGly-KSGSGS | 1440.64 |
| MeAla(4-Thz) | MKAGPGFM-MeAla(4-Thz)-KSGSGS | 1537.65 |
| MePhe(4-Cl) | MKAGPGFM-MePhe(4-Cl)-KSGSGS | 1564.66 |
| Phe(3-Cl) | MKAGPGFM-Phe(3-Cl)-KSGSGS | 1550.64 |
| Met(02) | MKAGPGFM-Met(O2)-KSGSGS | 1532.54 |
| Phg | MKAGPGFM-Phg-KSGSGS | 1502.66 |
| MeSer | MKAGPGFM-MeSer-KSGSGS | 1470.65 |
| Phe(4-CF3) | MKAGPGFM-Phe(4-CF3)-KSGSGS | 1584.67 |
| Ser(Me) | MKAGPGFM-Ser(Me)-KSGSGS | 1470.65 |
| Ser(nPr) | MKAGPGFM-Ser(nPr)-KSGSGS | 1498.68 |
| MeSer(nPr) | MKAGPGFM-MeSer(nPr)-KSGSGS | 1512.70 |
| Ser(iPen) | MKAGPGFM-Ser(iPen)-KSGSGS | 1526.71 |
| MeSer(iPen) | MKAGPGFM-MeSer(iPen)-KSGSGS | 1540.73 |
| Hse(Et) | MKAGPGFM-Hse(Et)-KSGSGS | 1498.68 |
| MeHse(Et) | MKAGPGFM-MeHse(Et)-KSGSGS | 1512.70 |
| Hnl(7-F2) | MKAGPGFM-Hnl(7-F2)-KSGSGS | 1532.68 |
| MeHnl(7-F2) | MKAGPGFM-MeHnl(7-F2)-KSGSGS | 1546.70 |
| Ser(F(4)nPr) | MKAGPGFM-Ser(F(4)nPr)-KSGSGS | 1570.64 |
| MeSer(F(4)nPr) | MKAGPGFM-MeSer(F(4)nPr)-KSGSGS | 1584.66 |
| Nle(6-OH) | MKAGPGFM-Nle(6-OH)-KSGSGS | 1498.68 |
| Ser(EtOH) | MKAGPGFM-Ser(EtOH)-KSGSGS | 1500.66 |
| MeSer(EtOH) | MKAGPGFM-MeSer(EtOH)-KSGSGS | 1514.67 |

(continued)

| Amino acid | Translated peptide compound | Molecular weight m/z: [M+H]+ |
|---|---|---|
| Ser(S-2-PrOH) | MKAGPGFM-Ser(S-2-PrOH)-KSGSGS | 1514.68 |
| MeSer(S-2-PrOH) | MKAGPGFM-MeSer(S-2-PrOH)-KSGSGS | 1528.69 |
| Ser(R-2-PrOH) | MKAGPGFM-Ser(R-2-PrOH)-KSGSGS | 1514.68 |
| MeSer(R-2-PrOH) | MKAGPGFM-MeSer(R-2-PrOH)-KSGSGS | 1528.69 |
| Ser(tBuOH) | MKAGPGFM-Ser(tBuOH)-KSGSGS | 1528.69 |
| MeSer(tBuOH) | MKAGPGFM-MeSer(tBuOH)-KSGSGS | 1542.71 |
| Ser(2-Me-BuOH) | MKAGPGFM-Ser(2-Me-BuOH)-KSGSGS | 1542.71 |
| MeSer(2-Me-BuOH) | MKAGPGFM-MeSer(2-Me-BuOH)-KSGSGS | 1556.72 |
| Gln(Me) | MKAGPGFM-Gln(Me)-KSGSGS | 1511.68 |
| Gln(Me2) | MKAGPGFM-Gln(Me2)-KSGSGS | 1525.69 |
| MeGln(Me2) | MKAGPGFM-MeGln(Me2)-KSGSGS | 1539.71 |
| Ser(NtBu-Aca) | MKAGPGFM-Ser(NtBu-Aca)-KSGSGS | 1569.72 |
| MeSer(NtBu-Aca) | MKAGPGFM-MeSer(NtBu-Aca)-KSGSGS | 1583.74 |
| Hph | MKAGPGFM-Hph-KSGSGS | 1530.69 |
| MeHph | MKAGPGFM-MeHph-KSGSGS | 1544.70 |
| Ser(Ph-2-Cl) | MKAGPGFM-Ser(Ph-2-Cl)-KSGSGS | 1566.63 |
| MeSer(Ph-2-Cl) | MKAGPGFM-MeSer(Ph-2-Cl)-KSGSGS | 1580.64 |
| Ser(Ph-3-Cl) | MKAGPGFM-Ser(Ph-3-Cl)-KSGSGS | 1566.63 |
| Hph(2-Cl) | MKAGPGFM-Hph(2-Cl)-KSGSGS | 1564.65 |
| MeHph(2-Cl) | MKAGPGFM-MeHph(2-Cl)-KSGSGS | 1578.66 |
| Hph(3-Cl) | MKAGPGFM-Hph(3-Cl)-KSGSGS | 1564.65 |
| MeHph(3-Cl) | MKAGPGFM-MeHph(3-Cl)-KSGSGS | 1578.66 |
| Hph(4-Cl) | MKAGPGFM-Hph(4-Cl)-KSGSGS | 1564.65 |
| MeHph(4-Cl) | MKAGPGFM-MeHph(4-Cl)-KSGSGS | 1578.66 |
| Ser(3-F-5-Me-Pyr) | MKAGPGFM-Ser(3-F-5-Me-Pyr)-KSGSGS | 1565.67 |
| MeSer(3-F-5-Me-Pyr) | MKAGPGFM-MeSer(3-F-5-Me-Pyr)-KSGSGS | 1579.68 |
| Phe{#(CH2)2} | MKAGPGFM-Phe{#(CH2)2}-KSGSGS | 1544.70 |
| MePhe{#(CH2)2} | MKAGPGFM-MePhe{#(CH2)2}-KSGSGS | 1558.72 |
| Ser(Bn) | MKAGPGFM-Ser(Bn)-KSGSGS | 1546.68 |
| MeSer(Bn) | MKAGPGFM-MeSer(Bn)-KSGSGS | 1560.70 |
| Hyp(Et) | MKAGPGFM-Hyp(Et)-KSGSGS | 1510.68 |
| Abu(pip-4-F2) | MKAGPGFM-Abu(pip-4-F2)-KSGSGS | 1573.71 |
| MeAbu(pip-4-F2) | MKAGPGFM-MeAbu(pip-4-F2)-KSGSGS | 1587.73 |
| MeAbu(pip-3-F2) | MKAGPGFM-MeAbu(pip-3-F2)-KSGSGS | 1587.73 |
| Ala(3-Pyr-4-NMe2) | MKAGPGFM-Ala(3-Pyr-4-NMe2)-KSGSGS | 1560.71 |
| MeAla(3-Pyr-4-NMe2) | MKAGPGFM-MeAla(3-Pyr-4-NMe2)-KSGSGS | 1574.72 |
| nPenGly | MKAGPGFM-nPenGly-KSGSGS | 1496.70 |
| nHexGly | MKAGPGFM-nHexGly-KSGSGS | 1510.72 |
| (PhEt)NGly | MKAGPGFM-(PhEt)NGly-KSGSGS | 1530.69 |
| (EtOEt)NGly | MKAGPGFM-(EtOEt)NGly-KSGSGS | 1498.68 |
| (PhOEt)NGly | MKAGPGFM-(PhOEt)NGly-KSGSGS | 1546.68 |
| Pro(pip-4-F2) | MKAGPGFM-Pro(pip-4-F2)-KSGSGS | 1585.71 |
| cisPro(pip-4-F2) | MKAGPGFM-cisPro(pip-4-F2)-KSGSGS | 1585.71 |
| MeNva(5-F2) | MKAGPGFM-MeNva(5-F2)-KSGSGS | 1518.67 |
| MePhe(3-Cl) | MKAGPGFM-MePhe(3-Cl)-KSGSGS | 1564.65 |
| Abu(Mor) | MKAGPGFM-Abu(Mor)-KSGSGS | 1539.71 |
| Phe(4-CF3) | MKAGPGFM-Phe(4-CF3)-KSGSGS | 1584.66 |
| 2-(pip-4-F2)-EtGly | MKAGPGFM-2-(pip-4-F2)-EtGly-KSGSGS | 1573.71 |
| Phe(4-CHF2) | MKAGPGFM-Phe(4-CHF2)-KSGSGS | 1566.67 |

(continued)

| Amino acid | Translated peptide compound | Molecular weight m/z: [M+H]+ |
|---|---|---|
| Phe(4-OCHF2) | MKAGPG-Phe(4-OCHF2)-M-Phe(4-OCHF2)-KSGSGS | 1648.66 |
| Leu | MKAGPGFM-Leu-KSGSGS | 1482.69 |
| MeVal | MKAGPGFM-MeVal-KSGSGS | 1482.69 |
| Ser(Et-2-Mor) | MKAGPGFM-Ser(Et-2-Mor)-KSGSGS | 1569.72 |
| Ser(1-CF3-EtOH) | MKAGPGFM-Ser(1-CF3-EtOH)-KSGSGS | 1568.65 |

[Example 3] Establishment of an improved method of measuring membrane permeability using Caco-2 cells

(1) Cell culture

Caco-2 cells (CACO-2 Lot No. 028; Riken BRC Cell Bank) were seeded onto a 24-well Transwell membrane (Corning HTS Transwell, pore size 0.4 μm, polycarbonate membrane) at a density of 1.0 x $10^5$ cells/well and cultured in DMEM medium containing 10% FBS, penicillin-streptomycin-glutamine (100x), an L-glutamine/sodium chloride solution, and a non-essential amino acid (Neaa) in an incubator maintained at a temperature of 37°C and 5% $CO_2$. The medium was replaced every two to three days, and Caco-2 cells were subjected to membrane permeability measurement on Days 21 to 23 after the seeding.

(2) Preincubation

To perform long-time incubation as an improved method of measuring membrane permeability, the effect of incubation time on cells was examined by assessing transepithelial electrical resistance (TEER) using various buffers.

In accordance with a conventional method of measuring membrane permeability (Artursson, P., 2001. Adv Drug Deliv Rev 46, 27-43; Mason, A.K., 2013. Drug Metab Dispos 41, 1347-1366; Polli, J.W., 2001. J Pharmacol Exp Ther 299, 620-628; Sun, H. 2008. Expert Opin Drug Metab Toxicol 4, 395-411), fasted state simulated intestinal and stomach fluid (FaSSIF) (with 1% DMSO) was added to the apical side and Hanks' balanced salt solution (HBSS) (with 4% BSA) was added to the basal side, and the cells were incubated at 37°C. The results showed that the TEER was decreased by 30% or more after incubation for three hours. This demonstrated that cells cannot be incubated for more than three hours by the conventional method, and compounds with a long lag time cannot be evaluated by the conventional method (Fig. 7).

Next, to perform long-time incubation, the effect on cells provided by incubation time (0, 2, 4, 6, 8, 24 hr) in the medium (DMEM) was evaluated by measuring the TEER at each time. The results showed that the TEER hardly decreased for 24 hours when DMEM + FaSSIF (1% DMSO, 2% dimethylacetamide (DMA)) were added to the apical side and DMEM to the basal side. Consequently, it was found that cells can be incubated for 24 hours under the condition where DMEM + FaSSIF (1% DMSO, 2% DMA) are added to the apical side and DMEM is added to the basal side (Fig. 8).

(3) Membrane permeability evaluation after preincubation

Tests for membrane permeability from the apical side to the basal side (A to B tests) were performed after the preincubation established in (2) above, in accordance with the conventional method using cyclosporin (Compound A), which is a cyclic peptide, and other cyclic peptides (Compounds B to H) as test substances. The preincubation was carried out after culturing Caco-2 cells for three weeks according to (1) above. Specifically, the cells were preincubated for 24 hours according to (2) above, under the conditions where DMEM + FaSSIF (1% DMSO, 2% DMA) + test substance were added to the apical side and DMEM was added to the basal side. Following the preincubation, A to B tests were initiated by removing the preincubation solution on the apical and basal sides by suction and adding FaSSIF/HBSS (with 1% DMSO, 2% DMA) (pH 6.0) containing the test substance to the apical side and HBSS (4% BSA) (pH 7.4) to the basal side. Each well was shaken at 220 rpm while being warmed to maintain at 37°C. 30, 60, 90, and 120 minutes after the initiation, the samples on the basal side were collected and the permeation amount was measured by LC/MS. The membrane permeability coefficient ($P_{app}$) was calculated from the permeation amount.

The absorption factor (Fa) was calculated by assessing the plasma concentration and fecal excretion rate of cyclosporin, which is a cyclic peptide, and other cyclic peptides in mice, after intravenous and oral administration. Compounds were intravenously and orally administered to male mice (C57BL/6J, six-week-old, manufactured by CLEA Japan), blood was collected over time from the dorsal pedal vein until 24 hours after the administration using a hematocrit tube treated with heparin as an anticoagulant, and feces were collected until 72 hours after the administration. Measurement was performed using an LC/MS/MS instrument (API3200, manufactured by AB SCIEX). The absorption factor

(Fa) was calculated from the obtained plasma and fecal drug concentrations (Qingqing X., et al. 2016, Xenobiotica. 46, 913-921).

The conventional method was found to underestimate membrane permeability coefficients, because for most of the test substances, the membrane permeability coefficients calculated by the improved method (a method involving long-time preincubation) were larger than those calculated by the conventional method. In particular, the membrane permeability coefficient of Compound H was smaller than 1.0 x $10^{-8}$ cm/sec according to the conventional method and too small to be evaluated, while the membrane permeability coefficient was 3 x $10^{-7}$ cm/sec according to the improved method. Thus, compounds having a small membrane permeability coefficient showed significant variance in the values. It was also confirmed that the correlation between the Fa and the membrane permeability coefficient of a compound according to the improved method (Preincubation (+)) tends to be closer than that according to the conventional method (Preincubation (-)) (Table 10, Fig. 9, and Fig. 10). Therefore, it was shown that the absorption factor of a test substance can be estimated by measuring membrane permeability according to the improved method described above. It was assumed that "membrane permeability coefficient ($P_{app}$) ≥ 1.0 x $10^{-6}$ cm/sec" can be used as a criterion for selecting a peptide compound having high membrane permeability (for example, a peptide compound which has a membrane permeability sufficient to develop the compound as an oral drug), because compounds having a membrane permeability coefficient of 1.0 x $10^{-6}$ cm/sec or more as measured by the improved method have Fa of 0.3 or more.

[Table 10]

| | $P_{app}$ (cm/sec) | | Fa |
|---|---|---|---|
| | Preincubati on (-) | Preincubation(+) | |
| Compound A | 8. 75E-06 | 1. 10E-05 | 0.898 |
| Compound B | 1. 75E-06 | 4. 17E-06 | 0. 628 |
| Compound C | 6. 62E-06 | 9. 67E-06 | 0. 935 |
| Compound D | 8. 31E-07 | 2. 91E-06 | 0. 350 |
| Compound E | 5. 33E-07 | 9. 50E-07 | 0. 592 |
| Compound F | 5. 38E-08 | 1. 08E-06 | 0. 492 |
| Compound G | 1. 88E-07 | 1. 71E-06 | 0. 335 |
| Compound H | N.D. | 3. 23E-07 | 0. 169 |
| N. D. = Not detected | | | |

In the following Examples, membrane permeability was measured in accordance with the improved method established as described above. Specifically, Caco-2 cells were cultured on a 96-well Transwell for three weeks, after which DMEM + FaSSIF (1% DMSO) + compound were added to the apical side and DMEM was added to the basal side, and the cells were preincubated at 5% $CO_2$, 37°C, and 80 rpm for 20 to 24 hours. Following the preincubation, measurement of membrane permeability was initiated by removing the preincubation solution on the apical and basal sides by suction, washing the membrane, and adding FaSSIF/HBSS buffer (1% DMSO) (pH 6.0) containing the compound to the apical side and HBSS buffer (4% BSA) (pH 7.4) to the basal side. Each well was shaken at 5% $CO_2$, 37°C, and 80 rpm, and 180 minutes after the initiation, the sample on the basal side was collected and the permeation amount was measured by LC/MS. The membrane permeability coefficient ($P_{app}$) was calculated from the permeation amount. Herein, the terms "Caco-2 (cm/sec)" in the columns in the tables and "$P_{app}$ (cm/sec)" are used in the same meaning.

[Example 4] Panning of target-binding peptide compounds (in vitro selection)

4-1. Synthesis of acylated tRNAs

Acylated tRNAs used for panning were prepared by the procedure described in the patent document (WO 2013/100132). The base sequence of the tRNA (CA deficient) used is provided in Table 11. The elongator aminoacylated tRNA mixtures provided in Table 12 were prepared. Hereafter, translation solutions were prepared at a final concentration shown in Table 12 when used for translation. For Acbz-protected pCpA amino acids, TCEP was added at a final concentration of 42 mM and the amino acids were deprotected at room temperature for 45 min instead of deprotection with iodine, after which phenol extraction and later operations were carried out. For Pnaz-protected pCpA amino acids, phenol extraction and later operations were carried out without performing deprotection. In the preparation of initiator aminoacylated tRNAs, phenol extraction and later operations were carried out without performing deprotection after ligation reaction. Three types of acylated tRNAs shown in Table 13 were separately prepared, and any one of them was added to the translation solution at a final concentration shown in the table, when used for translation.

[Table 11]

| RNA name | RNA SEQ ID NO | RNA sequence |
|---|---|---|
| tRNAGluCUA(CA deficient) | T - 1 (SEQ ID NO : 3) | GUCCCCUUCGUCUAGAGGCCCAGGACACCGCCCUcuaACGGCGGUAACAGGGGUUCGAAUCCCCUAGGGGACGC |
| tRNAGluCAA(CA deficient) | T - 2 (SEQ ID NO: 4) | GUCCCCUUCGUCUAGAGGCCCAGGACACCGCCCUcaaACGGCGGUAACAGGGGUUCGAAUCCCCUAGGGGACGC |
| tRNAGluUAG(CA deficient) | T - 3 (SEQ ID NO: 5) | GUCCCCUUCGUCUAGAGGCCCAGGACACCGCCCUuagACGGCGGUAACAGGGGUUCGAAUCCCCUAGGGGACGC |
| tRNAGluCUG(CA deficient) | T - 4 (SEQ ID NO : 6) | GUCCCCUUCGUCUAGAGGCCCAGGACACCGCCCUcugACGGCGGUAACAGGGGUUCGAAUCCCCUAGGGGACGC |
| tRNAGluCCG(CA deficient) | T - 5 (SEQ ID NO: 7) | GUCCCCUUCGUCUAGAGGCCCAGGACACCGCCCUccgACGGCGGUAACAGGGGUUCGAAUCCCCUAGGGGACGC |
| tRNAGluCCU(CA deficient) | T - 6 (SEQ ID NO: 8) | GUCCCCUUCGUCUAGAGGCCCAGGACACCGCCCUccuACGGCGGUAACAGGGGUUCGAAUCCCCUAGGGGACGC |
| tRNAGluUUC(CA deficient) | T - 7 (SEQ ID NO: 9) | GUCCCCUUCGUCUAGAGGCCCAGGACACCGCCCUuucACGGCGGUAACAGGGGUUCGAAUCCCCUAGGGGACGC |
| tRNAfMetCAU(CA deficient) | T - 10 (SEQ ID NO: 10) | GGCGGGGUGGAGCAGCCUGGUAGCUCGUCGGGCUcauAACCCGAAGAUCGUCGGUUCAAAUCCGGCCCCCGCAAC |
| tRNAGluAAA(CA deficient) | SEQ ID NO: 13 | GUCCCCUUCGUCUAGAGGCCCAGGACACCGCCCUaaaACGGCGGUAACAGGGGUUCGAAUCCCCUAGGGGACGC |
| tRNAGluAGA(CA deficient) | SEQ ID NO: 14 | GUCCCCUUCGUCUAGAGGCCCAGGACACCGCCCUagaACGGCGGUAACAGGGGUUCGAAUCCCCUAGGGGACGC |
| tRNAGluAUA(CA deficient) | SEQ ID NO: 15 | GUCCCCUUCGUCUAGAGGCCCAGGACACCGCCCUauaACGGCGGUAACAGGGGUUCGAAUCCCCUAGGGGACGC |
| tRNAGluACA(CA deficient) | SEQ ID NO : 16 | GUCCCCUUCGUCUAGAGGCCCAGGACACCGCCCUacaACGGCGGUAACAGGGGUUCGAAUCCCCUAGGGGACGC |
| tRNAGluAAG(CA deficient) | SEQ ID NO : 17 | GUCCCCUUCGUCUAGAGGCCCAGGACACCGCCCUaagACGGCGGUAACAGGGGUUCGAAUCCCCUAGGGGACGC |
| tRNAGluAUG(CA deficient) | SEQ ID NO : 18 | GUCCCCUUCGUCUAGAGGCCCAGGACACCGCCCUaugACGGCGGUAACAGGGGUUCGAAUCCCCUAGGGGACGC |
| tRNAGluACG(CA deficient) | SEQ ID NO : 19 | GUCCCCUUCGUCUAGAGGCCCAGGACACCGCCCUacgACGGCGGUAACAGGGGUUCGAAUCCCCUAGGGGACGC |
| tRNAGluAAU (CA deficient) | SEQ ID NO: 20 | GUCCCCUUCGUCUAGAGGCCCAGGACACCGCCCUaauACGGCGGUAACAGGGGUUCGAAUCCCCUAGGGGACGC |
| tRNAGluAUU(CA deficient) | SEQ ID NO: 21 | GUCCCCUUCGUCUAGAGGCCCAGGACACCGCCCUauuACGGCGGUAACAGGGGUUCGAAUCCCCUAGGGGACGC |

(continued)

| RNA name | RNA SEQ ID NO | RNA sequence |
|---|---|---|
| tRNAGluACU(CA deficient) | SEQ ID NO : 22 | GUCCCCUUCGUCUAGAGGCCCAGGACACCGCCCUacuACGGCGGUAACAGGGGUUCGAAUCCCCUAGGGGACGC |
| tRNAGluAUC(CA deficient) | SEQ ID NO : 23 | GUCCCCUUCGUCUAGAGGCCCAGGACACCGCCCUaucACGGCGGUAACAGGGGUUCGAAUCCCCUAGGGGACGC |

[Table 12]

| Elongator aminoacylated tRNA mixture | Contained aminoacylated tRNA name | Final concentration when used for translation [μM] |
|---|---|---|
| Panning involving long side chain amino acids having low membrane permeability | Asp(sMe)-tRNAGluCUA | 20 |
| | Phe(3-Cl)-tRNAGluCAA | 20 |
| | MeGly-tRNAGluUAG | 20 |
| | MeAla(4-Thz)-tRNAGluCUG | 20 |
| | MePhe(4-Cl)-tRNAGluCCG | 20 |
| | Met(O2)-tRNAGluCCU | 20 |
| | Phg-tRNAGluUUC | 20 |
| Panning to evaluate long side chain amino acids having high membrane permeability | Asp(SMe)-tRNAGluCTA | 10 |
| | MePhe-tRNAGluAAA | 10 |
| | Phe-tRNAGluAUA | 10 |
| | Ser(3-F-5-Me-Pyr)-tRNAGluAGA | 10 |
| | Pic(2)-tRNAGluACA | 10 |
| | MeHph-tRNAGluAAG | 10 |
| | Ser(Me)-tRNAGluAUG | 10 |
| | MeAla(3-pyr)-tRNAGluACG | 10 |
| | Ser(Ph-2-Cl)-tRNAGluAAU | 10 |
| | Ala(3-Pyr)-tRNAGluAUU | 10 |
| | MeSer(3-F-5-Me-Pyr)-tRNAGluACU | 10 |
| | MeGly-tRNAGluAUC | 10 |

[Table 13]

| Acylated tRNA name | Final concentration when used for translation [ μM] ] |
|---|---|
| Acbz-Cys(StBu)-tRNAfMetCAU | 25 |
| Acbz-MeCys(StBu)-tRNAfMetCAU | 25 |
| Acbz-D-MeCys(StBu)-tRNAfMetCAU | 25 |

4-2. Randomized double-stranded DNA libraries encoding peptide compound libraries

DNA libraries were constructed by the procedure described in the patent document (WO 2013/100132). For panning involving long side chain amino acids having low membrane permeability, a library in which any of the codons TTT, TTG, CTA, ATT, GTT, CCG, ACT, GCT, TAC, CAT, CAG, TGG, CGG, AGT, AGG, and GGT randomly and repeatedly appears nine times was prepared for iSP, and a library in which any of the codons CTT, CTA, ATT, ATG, GTT, ACT, TAC, CAG, TGG, AGT, GGT, TTT, TTG, CCG, GCT, TAG, CAT, AAC, GAA, CGG, and AGG repeatedly appears nine times was prepared for iRT. For panning to evaluate long side chain amino acids having high membrane permeability, a library in which a random domain triplet repeatedly appears eight to nine times was prepared.

4-3. Preparation of biotinylated target proteins

GTPase KRas (KRAS), dual specificity mitogen-activated protein kinase kinase 1 (MEK1), mitogen-activated protein kinase 3 (ERK1), and interleukin 6 receptor (IL-6R) were used as target proteins used for panning. KRAS, ERK1, and IL-6R were biotinylated using the methods of non-patent documents BMC biotechnology, 2008, 8, 41 and Protein Science, 1999, 8, 921-929. IL-6R was prepared according to a non-patent document J Biochem. 1990; 108(4):673-6. ERK1 was prepared according to the method of Biochem Biophys Res Commun. 2008 Dec 26; 377(4):1123-7. As MEK1, a biotinylated active form (Product Number: 07-441-20N) was purchased from Carna Biosciences. As KRAS, one expressed by E. coli and purified was used.

4-4. Translation solutions used for panning

The translation solution used for panning involving long side chain amino acids having low membrane permeability is constituted by the following composition: 1 mM GTP, 1 mM ATP, 20 mM creatine phosphate, 50 mM HEPES-KOH pH 7.6, 100 mM potassium acetate, 10 mM magnesium acetate, 2 mM spermidine, 1 mM dithiothreitol, 0.5 mg/ml E. coli MRE600 (RNase negative)-derived tRNA (Roche), 4 μg/ml creatine kinase, 3 μg/ml myokinase, 2 units/ml inorganic pyrophosphatase, 1.1 μg/ml nucleoside diphosphate kinase, 0.26 μM EF-G, 2.7 μM IF1, 0.4 μM IF2, 1.5 μM IF3, 40 μM EF-Tu, 44 μM EF-Ts, 1.2 μM ribosome, 2.73 μM AlaRS, 0.09 μM GlyRS, 0.4 μM IleRS, 0.5 μM mutant PheRS05 (WO 2016/148044), 0.16 μM ProRS, 1 μM mutant SerRS37 (WO 2016/148044), 0.09 μM ThrRS, 0.01 μM TrpRS, 0.02 μM TyrRS, 1 μM mutant ValRS13 (WO 2016/148044), 0.11 μM LysRS, 0.33 μM HisRS, 3 μM in vitro transcribed E. coli tRNAAlalB, 3 μM purified E. coli tRNA His QUG (purified according to the procedure of Nucleic Acids Res. 2010 Apr; 38(6):e89), 250 μM glycine, 250 μM isoleucine, 250 μM proline, 250 μM threonine, 250 μM tryptophan, 250 μM lysine, 5 mM N-methylvaline, 5 mM N-methylserine, 5 mM N-methylalanine, 5 mM N-methylphenylalanine, 250 μM 3-fluorotyrosine, 5 mM N-methylhistidine, and the elongator aminoacylated tRNA mixture shown in Table 12 (for panning involving long side chain amino acids having low membrane permeability). The translation solution was prepared by further adding any one of the initiator aminoacylated tRNAs (Table 13) when using the initiation suppression method (iSP), or by further adding 250 μM cysteine and 0.02 μM CysRS when using the initiation read-through method (iRT). The translation solution for panning to evaluate long side chain amino acids having high membrane permeability is constituted by the following composition: 1 mM GTP, 1 mM ATP, 20 mM creatine phosphate, 50 mM HEPES-KOH pH 7.6, 100 mM potassium acetate, 10 mM magnesium acetate, 2 mM spermidine, 1 mM dithiothreitol, 1.0 mg/ml E. coli MRE600 (RNase negative)-derived tRNA (Roche), 4 μg/ml creatine kinase, 3 μg/ml myokinase, 2 units/ml inorganic pyrophosphatase, 1.1 μg/ml nucleoside diphosphate kinase, 0.26 μM EF-G, 2.7 μM IF1, 0.4 μM IF2, 1.5 μM IF3, 40 μM EF-Tu, 46 μM EF-Ts, 1.2 μM ribosome, 5.46 μM AlaRS, 0.09 μM GlyRS, 0.5 μM mutant PheRS05 (WO2016/148044), 0.16 μM ProRS, 0.09 μM ThrRS, 1 μM mutant ValRS13 (WO 2016/148044), 3 μM in vitro transcribed E. coli tRNAAlalB, 250 μM glycine, 250 μM proline, 250 μM threonine, 5 mM N-methylvaline, 5 mM N-methylalanine, 5 mM N-methylphenylalanine, the elongator aminoacylated tRNA mixture shown in Table 12 (for panning to evaluate long side chain amino acids having high membrane permeability), and Acbz-MeCys(StBu)-tRNAfMetCAU, an initiator aminoacylated tRNA (Table 13).

4-5. Implementation of panning

Panning was implemented in accordance with WO 2013/100132 using the above-described double-stranded DNA libraries and translation solutions. For panning to evaluate long side chain amino acids having high membrane permeability, a TEV protease recognition sequence was introduced between the target protein and biotin, and the protein was eluted by TEV protease. A peptide library was allowed to interact with the biotinylated protein and then the protein was collected by streptavidin-immobilized magnetic beads, and the beads were then washed, after which a TEV eluate (50 mM Tris-HCl pH 8.0, 0.5 mM EDTA, 1 mM DTT, 0.1 U/μLAcTEV protease (Thermo Fisher Scientific, Product No. 12575015)) was added to the beads and allowed to react at 30°C for 10 min. After the reaction, the supernatant was collected and subjected to PCR. The N-terminal amino acids and targets used for each panning are listed in Table 14 below.

[Table 14]

| | Panning ID | iRT/iSP | N-term. | Target |
|---|---|---|---|---|
| Panning involving long side chain amino acids having low membrane permeability | 1 | iRT | Cys | MEK1 |
| | 2 | iRT | Cys | KRAS |
| | 3 | iRT | Cys | ERK1 |
| | 4 | iSP | MeCys | KRAS |
| | 5 | iSP | D-MeCys | KRAS |
| | 6 | iSP | Cys | IL-6R |
| | 7 | iSP | MeCys | IL-6R |
| | 8 | iSP | D-MeCys | IL-6R |
| | 9 | iSP | Cys | IL-6R |
| | 10 | iSP | Cys | KRAS |
| Panning to evaluate long side chain amino acids having high membrane permeability | 11 | iSP | MeCys | ERK1 |
| | 12 | iSP | MeCys | IL-6R |

4-6. Analysis of enriched sequences

Base sequences in the DNA pool for each round of panning were analyzed, and a phylogeny was constructed by UPGMA. The similarity matrix used assigned "1" to a case where amino acids match each other, and "0" to a case where amino acids do not match each other. Clusters were divided with the pruning line set at 0.2 from the leaves. Further, sequences that had the appearance frequency of more than 0.05% in at least one round, and that increased the appearance frequency 10-fold or more by adding a target compared to when the pool of the preceding round was divided and panning was performed without adding a target, were extracted. The appearance frequency of each amino acid group (amino acids having a long side chain, aromatic amino acids not having a long side chain, or non-aromatic amino acids not having a long side chain) in a random domain was calculated for each cluster. The obtained amino acid appearance frequencies for all clusters were summed and divided by the number of clusters. The resulting value was the appearance frequency of each amino acid group in that panning. Amino acids were categorized into the three "amino acid categories" in Table 15 below. The appearance frequencies for each panning are summarized in Table 16. The analysis results demonstrated that the appearance frequencies of long side chain amino acids tend to be higher irrespective of the presence of the target. From this it was assumed that hit rates for cyclic peptide compounds capable of specifically binding to target molecules can be improved, that is, screening efficiency for such compounds can be improved, by using a library including cyclic peptide compounds having a long side chain when screening for the cyclic peptide compounds capable of specifically binding to the target molecules.

[Table 15]

| Amino acid category table | | | |
|---|---|---|---|
| | Amino acid having a long side chain (Long) | Amino acid not having a long side chain (Short) | Amino acid excluded from comparison of side chain length (Excluded) |
| Panning involving long side chain amino acids having low membrane permeability | Phe(3-Cl)<br>MePhe(4-Cl)<br>Tyr(3-F)<br>Trp | Phg<br>MeHis<br>MePhe<br>MeAla(4-Thz) | Thr<br>MeSer<br>Met(O2)<br>Pro<br>MeAla<br>MeVal<br>Ile<br>MeGly<br>Gly |
| Panning to evaluate long side chain amino acids having high membrane permeability | MeHph<br>Ser(Ph-2-Cl)<br>MeSer(3-F-5-Me-Pyr)<br>Ser(3-F-5-Me-Pyr) | MePhe<br>Phe<br>MeAla(3-pyr)<br>Ala(3-Pyr) | Pic(2)<br>Ser(Me)<br>MeGly<br>Thr<br>MeAla<br>Gly<br>Pro |

[Table 16]

| **Panning ID** | **Target** | **Long Obs/ Calc[%]** | **Short Obs/ Calc[%]** | **Excluded Obs/ Calc[%]** | **The number of clusters** | **The number of clones** |
|---|---|---|---|---|---|---|
| 1 | MEK | 155% | 50% | 98% | 4 | 76 |
| 2 | KRAS | 115% | 110% | 89% | 26 | 87 |
| 3 | ERK | 142% | 125% | 70% | 18 | 98 |
| 4 | KRAS | 172% | 120% | 61% | 9 | 29 |
| 5 | KRAS | 200% | 37% | 77% | 4 | 7 |

(continued)

| Panning ID | Target | Long Obs/ Calc[%] | Short Obs/ Calc[%] | Excluded Obs/ Calc[%] | The number of clusters | The number of clones |
|---|---|---|---|---|---|---|
| 6 | IL-6R | 192% | 32% | 82% | 5 | 37 |
| 7 | IL-6R | 187% | 37% | 82% | 7 | 41 |
| 8 | IL-6R | 178% | 48% | 83% | 8 | 53 |
| 9 | IL-6R | 194% | 47% | 76% | 14 | 83 |
| 10 | KRAS | 148% | 73% | 88% | 14 | 35 |
| 11 | ERK1 | 178% | 33% | 92% | 9 | 36 |
| 12 | IL-6R | 160% | 53% | 94% | 16 | 43 |

[Example 5] Membrane permeability tests for cyclic peptide compounds having a long side chain

As described above, it was found that cyclic peptide compounds having a long side chain such as a Trp, Tyr(3-F), Phe(3-Cl), or MePhe(4-Cl) side chain are enriched by panning as hit compounds capable of specifically binding to target molecules. Next, membrane permeability of cyclic peptide compounds having such a side chain was evaluated by the improved method described above.

5-1. Membrane permeability tests for cyclic peptide compounds having a Trp side chain

Cyclic peptide compounds having a Trp side chain and cyclic peptide compounds not having the same side chain were synthesized and membrane permeability tests were performed by the improved method. Since MeTrp contains a Trp side chain, cyclic peptide compounds containing MeTrp as a constituent amino acid are also included in cyclic peptide compounds having a Trp side chain. As a result, cyclic peptide compounds not having a Trp side chain were able to achieve $P_{app} \geq 1.0 \times 10^{-6}$ cm/sec by increasing the ClogP (Fig. 11). On the other hand, it was difficult to increase membrane permeability of cyclic peptide compounds having one or more Trp side chains to $P_{app} \geq 1.0 \times 10^{-6}$ cm/sec even by increasing the ClogP (Fig. 12). For example, sequences of cyclic peptide compounds having various Trp side chain positions and N-alkyl patterns and having a ClogP/total aa in the range of 1.06 ≤ ClogP/total aa ≤ 1.43 are provided in Table 17. It was difficult for such compounds to achieve membrane permeability of $P_{app} \geq 1.0 \times 10^{-6}$ cm/sec.

The ClogP is a computed distribution coefficient calculated by a computer and was calculated using DayLight Version 4.95 of DayLight Chemical Information Systems, Inc. Structural and analytical data of the peptide sequences (pd50 to pd70, pd100 to pd247, and pd300 to pd504) evaluated for membrane permeability are summarized in Tables 26 and 27 below.

The columns of Table 17 are described taking pd100 as an example. The intersection unit is defined as the first amino acid. In pd100, the first amino acid is Asp. The amino acid adjacent to the intersection unit (Asp) and constituting the cyclic portion is defined as the second amino acid (which is Thr in pd100), and subsequent amino acids ranging from the third and fourth amino acids to the N-terminal amino acid (▲ unit) are defined in the same manner (in pd100, the third amino acid is MeAla, the fourth amino acid is MeGly, and the N-terminal amino acid (▲ unit) is the 11th amino acid D-Val because the cyclic portion is composed of 11 residues). The C-term column shows a functional group condensed with the carboxylic acid site of the C-terminal amino acid, where pip represents piperidine and pyrro represents pyrrolidine. "None" in the C-term column means that the C-terminal amino acid is present as carboxylic acid (pd100 has a structure where the C-terminal amino acid is the intersection unit Asp, the carboxylic acid site of which is condensed with pip (piperidine)). Table 17 shows that pd100 is a peptide in which the ▲ unit and the intersection unit are cyclized by an amide bond. Cyclic peptide compounds described in the text are all expressed in the same manner unless otherwise stated. The first and eleventh amino acid residues form a cyclic portion in all compounds described in Table 17.

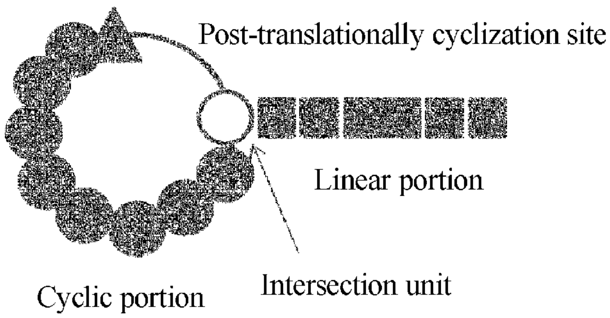
Post-translationally cyclization site
Linear portion
Intersection unit
Cyclic portion

**Scheme A-1**

[Table 17]

Membrane permeability of cyclic peptide compounds having a Trp side chain

| Compound ID | 11 | 10 | 9 | 8 | 7 | 6 | 5 | 4 | 3 | 2 | 1 | C-term | cLogP/ total AA | Caco-2 (cm/sec) |
|---|---|---|---|---|---|---|---|---|---|---|---|---|---|---|
| pd100 | D-Val | MeAla | MeLeu | Leu | MePhe | Trp | MeLeu | MeGly | MeAla | Thr | Asp | pip | 1.06 | 1.1E-07 |
| pd101 | MeAla | Leu | MeLeu | MeLeu | Ala | MeLeu | MeIle | Trp | MeGly | MeAla | Asp | pip | 1.23 | 1.3E-07 |
| pd102 | MeAla | Leu | MeLeu | MeTrp | Val | MeLeu | MeIle | Thr | MeGly | MeLeu | Asp | pip | 1.25 | 1.1E-06 |
| pd103 | MeGly | MeLeu | Ile | MeLeu | Trp | MeGly | MeLeu | MeLeu | Ser(tBu) | MeAla | Asp | pip | 1.26 | 9.3E-08 |
| pd104 | MeAla | MeLeu | MeLeu | Ala | MeLeu | MeTrp | MePhe | Thr | MeAla | MeLeu | Asp | pip | 1.27 | 2.5E-08 |
| pd105 | MeAla | MeLeu | MeLeu | Ala | MeLeu | MeLeu | MeTrp | Thr | MeAla | MeLeu | Asp | pip | 1.27 | 2.8E-07 |
| pd106 | D-Val | MeTrp | Leu | MeLeu | Thr | MeGly | MeLeu | Ser(tBu) | MeLeu | MeLeu | Asp | pip | 1.28 | 3.6E-07 |
| pd107 | g-MeAbu | MeLeu | Ile | MePhe | MeTrp | Ser(tBu) | MeLeu | Thr | MeLeu | MeLeu | Asp | pip | 1.30 | 1.7E-07 |
| pd108 | D-Ala | MeLeu | Ile | MeLeu | Trp | MeGly | MeLeu | Ser(tBu) | MeLeu | MeVal | Asp | pip | 1.34 | 8.7E-07 |
| pd109 | MeAla | MeLeu | MeLeu | Val | MeLeu | MeTrp | MePhe | Thr | MeAla | MeLeu | Asp | pip | 1.36 | 9.2E-09 |
| pd110 | g-MeAbu | MeLeu | Ile | MeLeu | MeLeu | Ser(tBu) | MeLeu | Trp | MeAla | MeLeu | Asp | pip | 1.36 | 2.9E-07 |
| pd111 | MeLeu | Leu | MeLeu | MeTrp | Leu | MePhe | MeIle | Thr | MeGly | MeLeu | Asp | pip | 1.43 | 1.6E-07 |

As demonstrated above, it was difficult to improve membrane permeability of cyclic peptide compounds having a Trp side chain to a level of $P_{app} \geq 1.0 \times 10^{-6}$cm/sec even by increasing the ClogP/total aa by modification.

5-2. Membrane permeability tests for cyclic peptide compounds having a Tyr(3-F) or Tyr side chain

Cyclic peptide compounds having a Tyr(3-F) or Tyr side chain were synthesized and membrane permeability tests were performed by the improved method. As a result, cyclic peptide compounds having a Tyr(3-F) or Tyr side chain showed the same tendency as in cyclic peptide compounds having a Trp side chain. Sequences of cyclic peptide compounds having a ClogP/total aa in the range of 1.06 ≤ ClogP/total aa ≤ 1.29 are provided in Table 18. As in the case of Trp, it was difficult for such compounds to achieve $P_{app} \geq 1.0 \times 10^{-6}$ cm/sec.

[Table 18]

Membrane permeability of cyclic peptide compounds having a Tyr(3-F) or Tyr side chain

| Compound ID | 11 | 10 | 9 | 8 | 7 | 6 | 5 | 4 | 3 | 2 | 1 | C-term | cLogP/ totalAA | Caco-2 (cm/sec) |
|---|---|---|---|---|---|---|---|---|---|---|---|---|---|---|
| pd112 | D-Ala | MePhe | Ile | MeLeu | Tyr | MeGly | MeAla | Ser(tBu) | MeAla | MeLeu | Asp | pip | 1.06 | 1.1E-07 |
| pd113 | D-Val | MeAla | MeLeu | Leu | MePhe | Tyr(3-F) | MeLeu | MeAla | MeAla | Thr | Asp | pip | 1.07 | 2.9E-08 |
| pd114 | g-MeAbu | MeLeu | Tyr(3-F) | MeLeu | MePhe | Ser(tBu) | MeLeu | Thr | MeGly | MeLeu | Asp | pip | 1.08 | 1.7E-07 |
| pd115 | MeGly | MePhe | Val | MeLeu | Tyr | MeGly | MeAla | MeLeu | Ser(tBu) | MeVal | Asp | pip | 1.10 | 2.2E-07 |
| pd116 | MeAla | Leu | MeAla | MePhe | Val | MeAla | MeIle | Tyr | MeGly | MeLeu | Asp | pip | 1.12 | 1.4E-07 |
| pd117 | D-Ala | MeLeu | Ile | MeAla | Tyr | MeGly | MeLeu | Ser(tBu) | MePhe | MeVal | Asp | pip | 1.15 | 5.6E-07 |
| pd118 | D-Leu | MePhe | Tyr(3-F) | MeLeu | Thr | MeGly | MeLeu | Ser(tBu) | MeLeu | MeAla | Asp | pip | 1.15 | 2.0E-07 |
| pd119 | g-MeAbu | MeLeu | Ile | MePhe | MeLeu | Tyr(3-F) | MeLeu | Thr | MeAla | MeLeu | Asp | pip | 1.17 | 1.6E-07 |
| pd120 | MeAla | Leu | MeLeu | MePhe | Ala | MeAla | MeLeu | Tyr(3-F) | MeGly | MeLeu | Asp | pip | 1.18 | 2.3E-07 |
| pd121 | D-Val | MeAla | Val | MeLeu | Tyr | MeGly | MeLeu | Ser(tBu) | MePhe | MeVal | Asp | pip | 1.18 | 1.0E-06 |
| pd122 | MeAla | Tyr(3-F) | MeLeu | MeLeu | Val | MePhe | MeLeu | Thr | MeGly | MeLeu | Asp | pip | 1.21 | 4.6E-07 |
| pd123 | MeGly | MeLeu | Tyr(3-F) | MeLeu | Thr | MeGly | MePhe | MeLeu | Leu | MeLeu | Asp | pip | 1.21 | 4.4E-07 |
| pd124 | D-Ala | MeLeu | Ile | MeLeu | Tyr(3-F) | MeGly | MeLeu | Ser(tBu) | MePhe | MeAla | Asp | pip | 1.22 | 3.2E-07 |
| pd125 | g-MeAbu | MeVal | Ile | MeLeu | MePhe | Ser(tBu) | MeLeu | Tyr | MeAla | MeLeu | Asp | pip | 1.25 | 4.5E-07 |
| pd126 | MeAla | Val | MeLeu | MeLeu | Ala | MePhe | MeIle | Tyr | MeGly | MeLeu | Asp | pip | 1.25 | 4.2E-07 |
| pd127 | D-Val | MePhe | Leu | MeLeu | Tyr | MeGly | MeLeu | Ser(tBu) | MeLeu | MeAla | Asp | pip | 1.28 | 1.3E-07 |
| pd128 | D-Val | MePhe | Leu | MeLeu | Thr | MeGly | MeLeu | Tyr(3-F) | MeLeu | MeLeu | Asp | pip | 1.29 | 1.7E-07 |
| pd129 | g-MeAbu | MeLeu | Ile | MePhe | MeLeu | Ser(tBu) | MeLeu | Tyr | MeAla | MeLeu | Asp | pip | 1.29 | 1.6E-07 |

5-3. Membrane permeability tests for cyclic peptide compounds having a Phe(3-Cl), MePhe(4-Cl), or Phe(4-$CF_3$) side chain

Cyclic peptide compounds having a Phe(3-Cl), MePhe(4-Cl), or Phe(4-$CF_3$) side chain were synthesized and membrane permeability tests were performed by the improved method. As a result, many of the cyclic peptide compounds having such a side chain achieved $P_{app} \geq 1.0 \times 10^{-6}$ cm/sec while having a ClogP/total aa in the range of ClogP/total aa ≥ 1.10. Fig. 13 provides examples of Phe(4-$CF_3$). Cyclic peptide compounds having a Phe(4-$CF_3$) side chain were able to achieve $P_{app} \geq 1.0 \times 10^{-6}$ cm/sec while having a ClogP/total aa in the range of ClogP/total aa ≥ 1.10 (Fig. 13).
Table 19 provides the sequences of a part of cyclic peptide compounds having a Phe(4-$CF_3$) side chain which achieved $P_{app} \geq 1.0 \times 10^{-6}$ cm/sec while having a ClogP/total aa in the range of 1.10 ≤ ClogP/total aa ≤ 1.50.

[Table 19]

| Compound ID | 11 | 10 | 9 | 8 | 7 | 6 | 5 | 4 | 3 | 2 | 1 | C-term | cLogP/ totalAA | Caco-2 (cm/sec) |
|---|---|---|---|---|---|---|---|---|---|---|---|---|---|---|
| pd130 | D-Ala | MePhe | Phe(4-$CF_3$) | MeLeu | Thr | MeGly | MeLeu | Ser (tBu) | MeVal | MeAla | Asp | pip | 1.10 | 1.1E-06 |
| pd131 | MeAla | Phe(4-$CF_3$) | MeLeu | MePhe | Ala | MeLeu | MeIle | Thr | MeGly | MeAla | Asp | pip | 1.11 | 1.1 E-06 |
| pd132 | MeGly | MeAla | Ile | MeLeu | Thr | MeGly | MePhe | MeLeu | Phe(4-CF3) | MeVal | Asp | pip | 1.15 | 3.1 E-06 |
| pd133 | D-MeAla | MeLeu | Phe(4-CF3) | MeLeu | Thr | MeAla | MeVal | Ile | Pro | Leu | Asp | pip | 1.17 | 2.3E-06 |
| pd134 | D-Val | MeVal | Phe(4-CF3) | MeLeu | Thr | MeGly | MeLeu | Ser (tBu) | MePhe | MeAla | Asp | pip | 1.18 | 2.6E-06 |
| pd135 | | | | MeLeu | MeAla | MePhe | Thr | MeLeu | Phe(4-CF3) | Pro | Asp | pip | 1.21 | 1.3E-06 |
| pd136 | | | | MeLeu | Thr | Pro | MeAla | Phe(4-CF3) | MeLeu | MeLeu | Asp | pip | 1-21 | 1-6E-06 |
| pd137 | D-Ala | MeLeu | Ser (tBu) | MeLeu | Pic(2) | MeAla | Thr | MePhe | Phe(4-CF3) | MeLeu | Asp | pip | 1.23 | 2.2E-06 |
| pd138 | D-Val | MeLeu | Phe(4-CF3) | MeLeu | Thr | MeGly | MeLeu | Ser (tBu) | MePhe | MeAla | Asp | pip | 1.23 | 2.6E-06 |
| pd139 | | | | Pro | MeLeu | Phe(4-$CF_3$) | Thr | MeLeu | Leu | Leu | Asp | pip | 1.23 | 1.9E-06 |
| pd140 | MeGly | MePhe | Phe(4-CF3) | MeLeu | Thr | MeGly | MeLeu | MeLeu | Ser (tBu) | MeVal | Asp | pip | 1.23 | 2.5E-06 |
| pd141 | D-Ala | MeLeu | Pic(2) | MeLeu | Pic(2) | MeAla | Thr | MePhe | Phe(4-CF3) | MeLeu | Asp | pip | 1.24 | 2.1 E-06 |
| pd142 | MeAla | Leu | MeLeu | MeLeu | Phe(4-CF3) | MeAla | MeIle | Thr | MeGly | MeLeu | Asp | pip | 1.25 | 2.9 E-06 |
| pd143 | | | | Ser (tBu) | MeLeu | EtGly | Thr | MeLeu | MeLeu | Phe(4-$CF_3$) | Asp | pip | 1.29 | 1.1 E-06 |

(continued)

| Compound ID | 11 | 10 | 9 | 8 | 7 | 6 | 5 | 4 | 3 | 2 | 1 | C-term | cLogP/ totalAA | Caco-2 (cm/sec) |
|---|---|---|---|---|---|---|---|---|---|---|---|---|---|---|
| pd144 | | | Ala | MeLeu | Leu | Ile | MeLeu | Phe(4-$CF_3$) | Thr | MeLeu | Asp | pip | 1.29 | 2.1E-06 |
| pd145 | | | Val | MeLeu | Leu | MeAla | Phe(4-CF3) | Thr | MePhe | MeLeu | Asp | pip | 1.30 | 1.2E-06 |
| pd146 | | | Val | MeLeu | Leu | MeAla | MeLeu | Phe(4-CF3) | Thr | MeLeu | Asp | pip | 1.30 | 1.7E-06 |
| pd147 | | | | MeLeu | Phe(4-$CF_3$) | MeLeu | MeLeu | Ser (tBu) | nPrGly | Thr | Asp | pip | 1.33 | 1.9E-06 |
| pd148 | | D-Val | Phe(4-$CF_3$) | MeLeu | MeLeu | Thr | MeGly | MeLeu | MeLeu | Leu | Asp | pip | 1.34 | 1.1E-06 |
| pd149 | | | | D-Ala | MeLeu | Leu | Phe(4-CF3) | MeLeu | Thr | MeLeu | MeAsp | pip | 1.35 | 1.7E-06 |
| pd150 | | | | MeLeu | MeGly | Phe | Phe(4-CF3) | MeLeu | MeLeu | Thr | MeAsp | pip | 1.35 | 1.7E-06 |
| pd151 | | | | Leu | MeLeu | MeLeu | Thr | MeGly | MeLeu | Phe(4-CF3) | MeAsp | pip | 1.36 | 3.2E-06 |
| pd152 | | | MeLeu | Phe(4-CF3) | MeLeu | MeIle | Thr | nPrGly | MeLeu | Ser (tBu) | Asp | pip | 1.43 | 1.2E-06 |
| pd153 | | | Leu | MeAla | MeLeu | Thr | MeLeu | Phe(4-CF3) | MeLeu | MeLeu | Asp | pip | 1.43 | 1.4E-06 |

Cyclic peptide compouns having a Phe(3-Cl) side chain showed the same tendency as in cyclic peptide compounds having a Phe(4-$CF_3$) side chain. Table 20 provides the sequences of a part of cyclic peptide compounds which achieved $P_{app} \geq 1.0 \times 10^{-6}$ cm/sec while having a ClogP/total aa in the range of 1.17 ≤ ClogP/total aa ≤ 1.31.

[Table 20]

| Compound ID | 11 | 10 | 9 | 8 | 7 | 6 | 5 | **4** | 3 | 2 | 1 | C-term | cLogP/ total AA | Caco-2 (cm/sec) |
|---|---|---|---|---|---|---|---|---|---|---|---|---|---|---|
| pd154 | Ala | MePhe (3-Cl) | Val | MeVal | Thr | MeGly | MeLeu | Ser (tBu) | MePhe | MeLeu | Asp | pip | 1.17 | 1.4E-06 |
| pd155 | D-Pro | MePhe (3-Cl) | Val | MeVal | Thr | MeGly | MeLeu | Ser (tBu) | MePhe | MeLeu | Asp | pip | 1.20 | 2.1E-06 |
| pd156 | Pro | MePhe (3-Cl) | Val | MeVal | Thr | MeGly | MeLeu | Ser (tBu) | MePhe | MeLeu | Asp | pip | 1.20 | 2.9E-06 |
| pd157 | D-Ala | MePhe (3-Cl) | Leu | MeLeu | Thr | MeGly | MeVal | Ser (tBu) | MePhe | MeLeu | Asp | pip | 1.21 | 2.2E-06 |
| pd158 | MeGly | MePhe | Ile | MeLeu | Thr | MeGly | MePhe (3-Cl) | MeLeu | Ser (tBu) | MeVal | Asp | pip | 1.22 | 1.4E-06 |
| pd159 | D-Ala | MePhe (3-Cl) | Val | MeVal | Thr | MeGly | MeLeu | Ser (tBu) | MePhe | MeLeu | MeAsp | pip | 1.23 | 1.8E-06 |
| pd160 | Ala | MePhe (3-Cl) | Val | MeVal | Thr | MeGly | MeLeu | Ser (tBu) | MePhe | MeLeu | MeAsp | pip | 1.23 | 3.4E-06 |
| pd161 | MeAla | Leu | MeLeu | MePhe | Ala | MePhe (3-Cl) | MeLeu | Thr | MeGly | MeLeu | Asp | pip | 1.23 | 1.4E-06 |
| pd162 | Pro | Phe | Ser (tBu) | MeLeu | Thr | MeGly | MeVal | Phe | MePhe (3-Cl) | MeLeu | MeAsp | pip | 1.25 | 1.2E-06 |
| pd163 | D-Pro | Phe | Ser (tBu) | MeLeu | Thr | MeGly | MeVal | Phe | MePhe (3-Cl) | MeLeu | MeAsp | pip | 1.25 | 1.7E-06 |
| pd164 | D-Val | MeVal | Abu | MeLeu | Ala(3-Pyr) | MeGly | MeLeu | Ser (tBu) | MePhe (3-Cl) | MeLeu | Asp | pip | 1.26 | 1.6E-06 |
| pd165 | D-Pro | MePhe (3-Cl) | Val | MeVal | Thr | MeGly | MeLeu | Ser (tBu) | MePhe | MeLeu | MeAsp | pip | 1.26 | 2.8E-06 |
| pd166 | Pro | MePhe (3-Cl) | Val | MeVal | Thr | MeGly | MeLeu | Ser (tBu) | MePhe | MeLeu | MeAsp | pip | 1.26 | 3.2E-06 |
| pd167 | D-MeAla | Phe | Ser (tBu) | MeLeu | Thr | MeGly | MeVal | Phe | MePhe (3-Cl) | MeLeu | MeAsp | pip | 1.27 | 1.7E-06 |

(continued)

| Compound ID | 11 | 10 | 9 | 8 | 7 | 6 | 5 | **4** | 3 | 2 | 1 | C-term | cLogP/ total AA | Caco-2 (cm/sec) |
|---|---|---|---|---|---|---|---|---|---|---|---|---|---|---|
| pd168 | D-MeAla | MePhe (3-Cl) | Val | MeVal | Thr | MeGly | MeLeu | Ser (tBu) | MePhe | MeLeu | MeAsp | pip | 1.28 | 2.3E-06 |
| pd169 | MeAla | MePhe (3-Cl) | Val | MeVal | Thr | MeGly | MeLeu | Ser (tBu) | MePhe | MeLeu | MeAsp | pip | 1.28 | 3.8E-06 |
| pd170 | g-MeAbu | MeLeu | Ile | MeLeu | MePhe (3-Cl) | Ser (tBu) | MeLeu | Ala(4-Pyr) | MeAla | MeLeu | Asp | pip | 1.28 | 1.6E-06 |
| pd171 | D-Val | MeVal | Leu | MePhe (3-Cl) | Ser (tBu) | MeLeu | Thr | MeAla | MeLeu | Leu | Asp | pip | 1.29 | 1.2E-06 |
| pd172 | D-Leu | MeLeu | Leu | MeLeu | Thr | MeGly | MeLeu | Ala(4-Pyr) | MePhe (3-Cl) | MeLeu | Asp | pip | 1.31 | 1.1E-06 |

Cyclic peptide compounds having a MePhe(4-Cl) side chain showed the same tendency as in cyclic peptide compounds having a Phe(3-Cl) or Phe(4-$CF_3$) side chain. Table 21 provides the sequences of a part of cyclic peptide compounds which achieved $P_{app} \geq 1.0 \times 10^{-6}$ cm/sec while having a ClogP/total aa in the range of $1.22 \leq$ ClogP/total aa $\leq 1.31$.

[Table 2]

| Compound ID | 11 | 10 | 9 | 8 | 7 | 6 | 5 | 4 | 3 | 2 | 1 | C-term | cLogP/ total AA | Caco-2 (cm/sec) |
|---|---|---|---|---|---|---|---|---|---|---|---|---|---|---|
| pd173 | MeAla | Leu | MeLeu | MePhe | Ala | MePhe (4-Cl) | MeLeu | Thr | MeGly | MeLeu | Asp | pip | 1.23 | 1.6E-06 |
| pd174 | MeAla | Val | MeLeu | MePhe (4-Cl) | Leu | MePhe | MeLeu | Thr | MeGly | MeLeu | Asp | pip | 1.31 | 1.3E-06 |
| pd175 | MeGly | MePhe | Ile | MeLeu | Thr | MeGly | MePhe (4-Cl) | MeLeu | Ser (tBu) | MeVal | Asp | pip | 1.22 | 1.1E-06 |
| pd176 | MeGly | MePhe (4-Cl) | Ile | MeLeu | Thr | MeGly | MePhe | MeLeu | Ser (tBu) | MeVal | Asp | pip | 1.22 | 1.2E-06 |

As described above, it was difficult to obtain cyclic peptide compounds having a Trp, Tyr, or Tyr(3-F) side chain which met the criterion of $P_{app} \geq 1.0 \times 10^{-6}$ cm/sec even by increasing the ClogP/total aa by modification. On the other hand, many cyclic peptide compounds having other long side chains which met the criterion of $P_{app} \geq 1.0 \times 10^{-6}$ cm/sec could be obtained by increasing the ClogP/total aa by modification.

For example, cyclic peptides containing amino acids having a long side chain (Ser(iPen), MeSer(iPen), Hph, MeHph, Ser(nPr), Hnl(7-F2), Ser(Ph-2-Cl), Ser(Ph-3-Cl), Ser(3-F-5-Me-Pyr), Ser(F(4)nPr), Hph(2-Cl), Hph(3-Cl), Hph(4-Cl), Phe{#(CH2)2}, Ser(Bn), Hyp(Et), MeAbu(pip-3-F2), MeAbu(pip-4-F2), Ala(3-Pyr-4-NMe2), nPenGly, nHexGly, (Ph-Et)NGly, (EtOEt)NGly, (PhOEt)Gly, Ser(S-2-PrOH), Ser(tBuOH), Ser(2-Me-2-BuOH), Gln(Me2), Ser(NtBu-Aca), Me-Ser(NtBu-Aca), Hse(Et), Nle(6-OH), MeAbu(Mor), 2-((pip-4-F2)-Et)Gly, Pro(pip-4-F2), cisPro(pip-4-F2), Gln(Me), Ahp(2)(3-R-OH), Lys(Ac), Phe(4-CHF2), Phe(4-OCHF2), Ser(Et-2-Mor), Abu(5-Oxo-Odz), Ser(EtOH), Ser(R-2-PrOH), Hnl(7-F3-6-OH), and Ser(1-CF3-EtOH)) were evaluated for membrane permeability to find that they were able to achieve Caco-2 $\geq 1.0 \times 10^{-6}$ cm/sec while having a ClogP/total AA in the range of $1.13 \leq$ ClogP/total aa $\leq 1.35$. A part of sequence data are provided below.

[Table 22]

| Compound ID | 11 | 10 | 9 | 8 | 7 | 6 | 5 | 4 | 3 | 2 | 1 | C-term | cLogP/ total AA | Caco-2 |
|---|---|---|---|---|---|---|---|---|---|---|---|---|---|---|
| pd300 | D-Ala | MePhe | Leu | MeLeu | Thr | MeGly | MeLeu | Ser(iPen) | MePhe | MeLeu | Asp | pip | 1.26 | 1.9E-06 |
| pd301 | D-Ala | MePhe | Ser(iPen) | MeLeu | Thr | MeGly | MeLeu | Ser(tBu) | MePhe | MeLeu | Asp | pip | 1.21 | 1.7E-06 |
| pd302 | g-MeAbu | MeLeu | Ile | MePhe | MePhe | Ser(iPen) | MeLeu | Thr | MeAla | MeLeu | Asp | pip | 1.23 | 1.4E-06 |
| pd303 | g-MeAbu | MeLeu | Ser(iPen) | MePhe | MePhe | Ser(tBu) | MeLeu | Thr | MeAla | MeLeu | Asp | pip | 1.18 | 1.0E-06 |
| pd304 | MeAla | Ser(iPen) | MeLeu | MePhe | Val | MePhe | MeLeu | Thr | MeGly | MeLeu | Asp | pip | 1.27 | 2.2E-06 |
| pd305 | MeGly | MePhe | Val | MeLeu | Thr | MeGly | MePhe | MeLeu | Ser(iPen) | MeLeu | Asp | pip | 1.22 | 2.3E-06 |
| pd306 | D-Val | MePhe | Leu | MeAbu | Thr | MeGly | MeSer (iPen) | Leu | MePhe | MeLeu | Asp | pip | 1.26 | 1.2E-06 |
| pd307 | MeAla | Leu | MeSer (iPen) | MePhe | Abu | MePhe | MeLeu | Thr | MeGly | MeLeu | Asp | pip | 1.24 | 2.9E-06 |
| pd308 | MeAla | Leu | MeLeu | MePhe | Leu | MePhe | MeSer (iPen) | Thr | MeGly | MeLeu | Asp | pip | 1.32 | 2.1 E-06 |
| pd309 | D-Ala | MeLeu | Ser(tBu) | MeAla | MeLeu | MeAla | Thr | MePhe | Hph | MeLeu | Asp | pip | 1.16 | 1.1 E-06 |
| pd310 | MeAla | MePhe | Hph | MeLeu | Thr | MeGly | MeLeu | MePhe | Ser(tBu) | MeAla | Asp | pip | 1.16 | 1.6E-06 |
| pd311 | MeVal | Ala | MePhe | MeLeu | MeGly | Thr | MeVal | Hph | MeLeu | MePhe | Asp | pip | 1.25 | 2.6E-06 |
| pd312 | D-Val | MePhe | Leu | MeLeu | Thr | MeGly | MeLeu | Hph | MeLeu | MeAla | Asp | pip | 1.25 | 1.5E-06 |
| pd313 | D-Val | MeAla | Val | MeLeu | Thr | MeGly | MeLeu | Hph | MePhe | MeLeu | Asp | pip | 1.20 | 2.5E-06 |
| pd314 | D-Leu | MePhe | Hph | MeLeu | Thr | MeGly | MeLeu | Ser(tBu) | MeLeu | MeAla | Asp | pip | 1.24 | 1.4E-06 |
| pd315 | D-Val | MeLeu | Hph | MeLeu | Thr | MeGly | MeLeu | Ser(tBu) | MePhe | MeLeu | Asp | pip | 1.33 | 1.7E-06 |
| pd316 | g-MeAbu | MeLeu | Ile | MePhe | MeLeu | Hph | MeLeu | Thr | MeAla | MeLeu | Asp | pip | 1.26 | 1.4E-06 |
| pd317 | g-MeAbu | MeLeu | Hph | MeLeu | MePhe | Ser(tBu) | MeLeu | Thr | MeGly | MeLeu | Asp | pip | 1.16 | 1.8E-06 |
| pd318 | MeAla | Val | MeLeu | MePhe | Ala | MeAla | MeLeu | Hph | MeGly | MeLeu | Asp | pip | 1.22 | 2.5E-06 |
| pd319 | MeGly | MeVal | Hph | MeLeu | Thr | MeGly | MePhe | MeLeu | Val | MeLeu | Asp | pip | 1.20 | 2.2E-06 |
| pd320 | D-Val | MeHph | Leu | MeAbu | Thr | MeGly | MeLeu | Leu | MePhe | MeLeu | Asp | pip | 1.29 | 1.7E-06 |
| pd321 | D-Ala | MePhe | Val | MeLeu | Thr | MeGly | MeLeu | Leu | MeHph | MeLeu | Asp | pip | 1.25 | 1.3E-06 |

(continued)

| Compound ID | 11 | 10 | 9 | 8 | 7 | 6 | 5 | 4 | 3 | 2 | 1 | C-term | cLogP/ total AA | Caco-2 |
|---|---|---|---|---|---|---|---|---|---|---|---|---|---|---|
| pd322 | MeAla | Leu | MeLeu | MeHph | Abu | MePhe | MeLeu | Thr | MeGly | MeLeu | Asp | pip | 1.26 | 3.1 E-06 |
| pd323 | MeAla | Leu | MeLeu | MePhe | Leu | MeHph | MeLeu | Thr | MeGly | MeLeu | Asp | pip | 1.35 | 2.3E-06 |
| pd324 | MeGly | MePhe | Ile | MeLeu | Thr | MeGly | MeHph | MeLeu | Ser(tBu) | MeLeu | Asp | pip | 1.25 | 3.3E-06 |
| pd325 | D-Val | MePhe | Leu | MeLeu | Thr | MeGly | MeLeu | Ser(nPr) | MePhe | MeLeu | Asp | pip | 1.26 | 2.2E-06 |
| pd326 | D-Val | MePhe | Val | MeLeu | Ser(nPr) | MeGly | MeLeu | Ser(tBu) | MePhe | MeAla | Asp | pip | 1.22 | 3.3E-06 |
| pd327 | D-Val | MePhe | Ser(nPr) | MeLeu | Thr | MeGly | MeLeu | Ser(tBu) | MePhe | MeLeu | Asp | pip | 1.21 | 3.1E-06 |
| pd328 | g-MeAbu | MeLeu | Val | MePhe | MePhe | Ser(nPr) | MeLeu | Thr | MeLeu | MeLeu | Asp | pip | 1.23 | 2.3E-06 |
| pd329 | MeAla | Leu | MeLeu | MePhe | Ser(nPr) | MePhe | MeLeu | Thr | MeGly | MeLeu | Asp | pip | 1.23 | 4.2E-06 |
| pd330 | MeGly | MePhe | Ile | MeLeu | Thr | MeGly | MePhe | MeLeu | Ser(nPr) | MeLeu | Asp | pip | 1.18 | 1.8E-06 |
| pd331 | D-Val | MePhe | Hnl(7-F2) | MeLeu | Thr | MeGly | MeLeu | Ser(tBu) | MePhe | MeVal | Asp | pip | 1.21 | 2.1 E-06 |
| pd332 | D-Val | MePhe | Leu | MeLeu | Thr | MeGly | MeLeu | Hnl(7-F2) | MePhe | MeVal | Asp | pip | 1.27 | 1.5E-06 |
| pd333 | g-MeAbu | MeLeu | Ile | MePhe | MePhe | Hnl(7-F2) | MeLeu | Thr | MeAla | MeLeu | Asp | pip | 1.20 | 1.3E-06 |
| pd334 | MeAla | Hnl(7-F2) | MeLeu | MePhe | Val | MePhe | MeIle | Thr | MeGly | MeLeu | Asp | pip | 1.23 | 2.4E-06 |
| pd335 | MeAla | Leu | MeLeu | MePhe | Hnl(7-F2) | MePhe | MeIle | Thr | MeGly | MeLeu | Asp | pip | 1.28 | 2.0E-06 |
| pd336 | nPrGly | MePhe | Hnl(7-F2) | MeLeu | Thr | MeGly | MePhe | MeLeu | Ser(tBu) | MeVal | Asp | pip | 1.25 | 2.5E-06 |
| pd337 | MeGly | MePhe | Ile | MeLeu | Thr | MeGly | MePhe | MeLeu | Hnl(7-F2) | MeLeu | Asp | pip | 1.23 | 2.0E-06 |
| pd338 | MeGly | MePhe | Hnl(7 -F2) | MeLeu | Thr | MeGly | MePhe | MeLeu | Ser(iPen) | MeLeu | Asp | pip | 1.25 | 1.8E-06 |
| pd339 | D-Val | MeLeu | Ser(Ph-2-Cl) | MeAla | Thr | MeGly | MeLeu | Ser(tBu) | MePhe | MeVal | Asp | pip | 1.18 | 2.6E-06 |
| pd340 | D-Ala | MePhe | Ser(Ph-2-Cl) | MeLeu | Thr | MeGly | MeLeu | Ser(tBu) | MeAla | MeLeu | Asp | pip | 1.14 | 1.2E-06 |
| pd341 | MeAla | Leu | MeLeu | MeAla | Ser(Ph-2-Cl) | MePhe | MeIle | Thr | MeGly | MeLeu | Asp | pip | 1.24 | 1.4E-06 |
| pd342 | D-Val | MePhe | Ser(Ph-3-Cl) | MeAla | Thr | MeGly | MeLeu | Ser(tBu) | MePhe | MeVal | Asp | pip | 1.19 | 1.9E-06 |

(continued)

| Compound ID | 11 | 10 | 9 | 8 | 7 | 6 | 5 | 4 | 3 | 2 | 1 | C-term | cLogP/ total AA | Caco-2 |
|---|---|---|---|---|---|---|---|---|---|---|---|---|---|---|
| pd343 | D-Ala | MePhe | Ser(Ph-3-Cl) | MeVal | Thr | MeGly | MeLeu | Ser(tBu) | MePhe | MeVal | Asp | pip | 1.19 | 2.2E-06 |
| pd344 | MeAla | Ser(Ph-3-Cl) | MeLeu | MePhe | Ala | MePhe | MeIle | Thr | MeGly | MeLeu | Asp | pip | 1.26 | 1.3E-06 |
| pd345 | MeAla | Val | MeLeu | MePhe | Ser(Ph-3-Cl) | MePhe | MeIle | Thr | MeGly | MeAla | Asp | pip | 1.21 | 1.8E-06 |
| pd346 | D-Val | MePhe | Ser(3-F-5-Me-Pyr) | MeLeu | Thr | MeGly | MeLeu | Ser(tBu) | MePhe | MeVal | Asp | pip | 1.13 | 2.4E-06 |
| pd347 | MeVal | Ser(3-F-5-Me-Pyr) | MeLeu | MePhe | Leu | MePhe | MeIle | Thr | MeGly | MeLeu | Asp | pip | 1.28 | 1.1E-06 |
| pd348 | MeAla | Leu | MeLeu | MePhe | Ser(F(4) nPr) | MePhe | MeIle | Thr | MeGly | MeLeu | Asp | pip | 1.23 | 1.1 E-06 |
| pd349 | nPrGly | MePhe | Ser(F(4) nPr) | MeLeu | Thr | MeGly | MePhe | MeLeu | Ser(tBu) | MeLeu | Asp | pip | 1.25 | 1.8E-06 |
| pd350 | D-Val | MeAla | Hph(2-Cl) | MeLeu | Thr | MeGly | MeLeu | Ser(tBu) | MePhe | MeVal | Asp | pip | 1.21 | 1.7E-06 |
| pd351 | D-Val | MeAla | Hph(2-Cl) | MeLeu | Thr | MeGly | MeLeu | Leu | MePhe | MeVal | Asp | pip | 1.26 | 1.9E-06 |
| pd352 | MeAla | Hph(2-Cl) | MeLeu | MeLeu | Ala | MePhe | MeIle | Thr | MeGly | MeLeu | Asp | pip | 128 | 1.4E-06 |
| pd353 | MeAla | Hph(2-Cl) | MeLeu | MeLeu | Ala | MePhe | MeVal | Thr | MeGly | MeLeu | Asp | pip | 1.23 | 1.4E-06 |
| pd354 | MeGly | MePhe | Ile | MeLeu | Thr | MeGly | MeLeu | MeLeu | Hph(2-Cl) | MeVal | Asp | pip | 1.32 | 1.5E-06 |
| pd355 | D-Val | MeAla | Hph(3-Cl) | MeLeu | Thr | MeGly | MeLeu | Leu | MePhe | MeVal | Asp | pip | 1.26 | 2.3E-06 |
| pd356 | MeAla | Hph(3-Cl) | MeLeu | MeLeu | Ala | MePhe | MeVal | Thr | MeGly | MeLeu | Asp | pip | 1.23 | 1.2E-06 |
| pd357 | MeGly | MeLeu | Hph(3-Cl) | MeLeu | Thr | MeGly | MePhe | MeLeu | Ser(tBu) | MeVal | Asp | pip | 1.27 | 1.3E-06 |
| pd358 | MeGly | MePhe | Ile | MeLeu | Thr | MeGly | MeLeu | MeLeu | Hph(3-Cl) | MeVal | Asp | pip | 1.32 | 1.1E-06 |
| pd359 | D-Val | MeAla | Hph(4-Cl) | MeLeu | Thr | MeGly | MeLeu | Ser(tBu) | MePhe | MeVal | Asp | pip | 1.21 | 1.1 E-06 |
| pd360 | D-Val | MeAla | Hph(4-Cl) | MeLeu | Thr | MeGly | MeLeu | Leu | MePhe | MeVal | Asp | pip | 1.26 | 1.6E-06 |
| pd361 | MeAla | Hph(4-Cl) | MeLeu | MeLeu | Ala | MePhe | MeVal | Thr | MeGly | MeLeu | Asp | pip | 1.23 | 1.1 E-06 |

(continued)

| Compound ID | 11 | 10 | 9 | 8 | 7 | 6 | 5 | 4 | 3 | 2 | 1 | C-term | cLogP/ total AA | Caco-2 |
|---|---|---|---|---|---|---|---|---|---|---|---|---|---|---|
| pd362 | MeGly | MePhe | Ile | MeLeu | Thr | MeGly | MeLeu | MeLeu | Hph(4-Cl) | MeVal | Asp | pip | 1.32 | 1.0E-06 |
| pd363 | D-Val | MePhe | Phe{# (CH2)2} | MeLeu | Thr | MeGly | MeLeu | Ser(tBu) | MePhe | MeAla | Asp | pip | 1.24 | 1.1 E-06 |
| pd364 | D-Ala | MePhe | Val | MeLeu | Thr | MeGly | MeLeu | Phe{# (CH2)2} | MePhe | MeVal | Asp | pip | 1.24 | 1.0E-06 |
| pd365 | MeAla | Phe{# (CH2)2} | MeLeu | MePhe | Ala | MePhe | MeVal | Thr | MeGly | MeLeu | Asp | pip | 1.21 | 1.5E-06 |
| pd366 | MeGly | MePhe | Phe{# (CH2)2} | MeLeu | Thr | MeGly | MePhe | MeLeu | Ser(tBu) | MeIle | Asp | pip | 1.29 | 1.8E-06 |
| pd367 | MeGly | MePhe | Ile | MeLeu | Thr | MeGly | MePhe | MeLeu | Phe{# (CH2)2} | MeAla | Asp | pip | 1.21 | 1.5E-06 |
| pd368 | MeAla | Leu | MeLeu | MePhe | Ser(Bn) | MePhe | MeAla | Thr | MeGly | MeLeu | Asp | pip | 1.18 | 2.0E-06 |
| pd369 | MeGly | MePhe | Ser(Bn) | MeLeu | Thr | MeGly | MePhe | MeLeu | Ser(tBu) | MeLeu | Asp | pip | 1.21 | 3.0E-06 |
| pd370 | MeGly | MePhe | Ile | MeLeu | Thr | MeGly | MePhe | MeLeu | Ser(Bn) | MeIle | Asp | pip | 1.26 | 2.5E-06 |
| pd371 | D-MeAla | MeLeu | Leu | MeLeu | Thr | Hyp(Et) | MeLeu | Ile | MeLeu | Leu | Asp | pip | 1.29 | 3.4E-06 |
| pd372 | D-Leu | Pro | Ser(tBu) | MeLeu | Hyp(Et) | MeLeu | Thr | MePhe | Phe | MeLeu | Asp | pip | 1.23 | 1.4E-06 |
| pd373 | Ala | MeVal | Ser(tBu) | MeLeu | Hyp(Et) | MeLeu | Thr | MePhe | Phe | MeLeu | Asp | pip | 1.26 | 2.0E-06 |
| pd374 | Val | Gly | MeLeu | MeLeu | MeLeu | MePhe | Thr | MePhe | MeLeu | Hyp(Et) | Asp | pip | 1.26 | 2.5E-06 |
| pd375 | D-Abu | MeLeu | Ser(tBu) | MeLeu | Leu | MeLeu | Thr | Hyp(Et) | Phe | MeLeu | Asp | pip | 1.24 | 2.9E-06 |
| pd376 | Pro | MeVal | Ser(tBu) | MeLeu | Hyp(Et) | MeVal | Thr | MePhe | Phe | MeLeu | Asp | pip | 1.25 | 2.4E-06 |
| pd377 | D-MeAla | MeLeu | Hyp(Et) | MeLeu | Thr | MeLeu | MeVal | Phe(4-CF3) | Pic(2) | Leu | Asp | pip | 1.28 | 1.4E-06 |
| pd378 | D-Leu | Hyp(Et) | Ser(tBu) | MeLeu | MeLeu | Pro | Thr | MePhe | Phe | MeLeu | Asp | pip | 1.23 | 1.2E-06 |
| pd379 | D-Leu | Pro | Ser(tBu) | MeLeu | MeLeu | Hyp(Et) | Thr | MePhe | Phe | MeLeu | Asp | pip | 1.23 | 1.7E-06 |
| pd380 | D-Val | MeNva | Ser(tBu) | MeLeu | MeLeu | Leu | Thr | Hyp(Et) | Phe | MeLeu | Asp | pip | 1.24 | 3.8E-06 |
| pd381 | D-MeLeu | MeNva | Leu | MeLeu | Thr | Hyp(Et) | MeLeu | Ile | MeLeu | Pro | Asp | pip | 1.30 | 5.6E-06 |

(continued)

| Compound ID | 11 | 10 | 9 | 8 | 7 | 6 | 5 | 4 | 3 | 2 | 1 | C-term | cLogP/ total AA | Caco-2 |
|---|---|---|---|---|---|---|---|---|---|---|---|---|---|---|
| pd382 | D-MeLeu | MeNva | Leu | MeLeu | Thr | Pro | MeLeu | Ile | MeLeu | Hyp(Et) | Asp | pip | 1.30 | 4.6E-06 |
| pd383 | D-Val | MeIle | Hyp(Et) | Pic(2) | Ser(tBu) | MeLeu | Thr | MeLeu | MeLeu | MeLeu | Asp | pip | 1.30 | 2.8E-06 |
| pd384 | D-Leu | MeIle | Hyp(Et) | MeLeu | Ser(tBu) | Pro | Thr | MeLeu | MeLeu | MeLeu | Asp | pip | 1.30 | 3.3E-06 |
| pd385 | D-Leu | MeIle | Pro | MeLeu | Ser(tBu) | Hyp(Et) | Thr | MeLeu | MeLeu | MeLeu | Asp | pip | 1.30 | 3.4E-06 |
| pd386 | D-Ala | MePhe | Leu | MeAbu(pip-3-F2) | Thr | nBuGly | MeLeu | Ser(tBu) | MePhe | MeVal | Asp | pip | 1.26 | 2.3E-06 |
| pd387 | D-Val | MePhe | Leu | MeLeu | Thr | MeGly | MeAbu(pip-3-F2) | Ser(tBu) | MePhe | MeVal | Asp | pip | 1.19 | 3.5E-06 |
| pd388 | D-Ala | MePhe | Leu | MeLeu | Thr | nBuGly | MeVal | Leu | MePhe | MeAbu (pip-3-F2) | Asp | pip | 1.31 | 1.9E-06 |
| pd389 | D-Val | MeAbu (pip-3-F2) | Leu | MePhe | Thr | MeGly | MeLeu | Leu | MePhe(3-Cl) | MeAla | Asp | pip | 1.22 | 1.1E-06 |
| pd390 | g-MeAbu | MeLeu | Leu | MePhe | MePhe | Leu | MeLeu | Thr | MeAbu(pip-3-F2) | MeAla | Asp | pip | 1.17 | 2.0E-06 |
| pd391 | MeAla | Leu | MeLeu | MePhe | Leu | MePhe | MeAbu(pip-3-F2) | Thr | MeGly | MeVal | Asp | pip | 1.21 | 2.4E-06 |
| pd392 | MeAla | Leu | MeLeu | MePhe | Leu | MePhe | MeAla | Thr | nBuGly | MeAbu (pip-3-F2) | Asp | pip | 1.27 | 3.5E-06 |
| pd393 | MeGly | MePhe | Leu | MeLeu | Thr | MeGly | MePhe | MeAbu (pip-3-F2) | Ser(iPen) | MeLeu | Asp | pip | 1.22 | 2.8E-06 |
| pd394 | D-Val | MePhe | Leu | MeAbu(pip-4-F2) | Thr | nBuGly | MeLeu | Leu | MePhe | MeAla | Asp | pip | 1.22 | 1.8E-06 |
| pd395 | nBuGly | MePhe | Leu | MeLeu | Thr | MeGly | MePhe | MeAbu (pip-4-F2) | Leu | MeVal | Asp | pip | 1.24 | 1.7E-06 |
| pd396 | D-Ala | MeLeu | Ala(3-Pyr-4-NMe2) | MeLeu | Thr | iPenGly | MeLeu | Ser(tBu) | MePhe | MeVal | Asp | pip | 1.27 | 1.5E-06 |
| pd397 | g-MeAbu | MeLeu | Leu | MeLeu | MePhe | Ala(3-Pyr-4-NMe2) | MeLeu | Thr | MeLeu | MeLeu | Asp | pip | 1.28 | 1.2E-06 |

(continued)

| Compound ID | 11 | 10 | 9 | 8 | 7 | 6 | 5 | 4 | 3 | 2 | 1 | C-term | cLogP/ total AA | Caco-2 |
|---|---|---|---|---|---|---|---|---|---|---|---|---|---|---|
| pd398 | MeAla | Ala(3-Pyr-4-NMe2) | MeLeu | MePhe | Leu | MeAla | MeLeu | Thr | nBuGly | MeLeu | Asp | pip | 1.25 | 2.7E-06 |
| pd399 | MeVal | Leu | MeLeu | MePhe(3-Cl) | Ala(3-Pyr-4-NMe2) | MeAla | MeLeu | Thr | nBuGly | MeAla | Asp | pip | 1.26 | 1.5E-Q6 |
| pd400 | MeAla | Ala(3-Pyr-4-NMe2) | MeAla | MeAla | Leu | MePhe(3-Cl) | MeLeu | Thr | nBuGly | MeLeu | Asp | pip | 1.18 | 2.3E-06 |
| pd401 | MeAla | Leu | MeLeu | MeSer(nPr) | Ala(3-Pyr-4-NMe2) | MePhe(3-Cl) | MeAla | Thr | iPenGly | MeVal | Asp | pip | 1.23 | 2.9E-06 |
| pd402 | nBuGly | MeLeu | Ala(3-Pyr-4-NMe2) | MeLeu | Thr | MeGly | MePhe | MeLeu | Ser(iPen) | MeAla | Asp | pip | 1.22 | 1.0E-06 |
| pd403 | MeVal | Leu | MeAbu | MePhe | Ala | MePhe | MeAbu | Thr | nPenGly | MeLeu | Asp | pip | 1.28 | 9.0E-06 |
| pd404 | MeGly | MePhe | Val | MeAla | Thr | nPenGly | MePhe | MeLeu | Ser(tBu) | MeLeu | Asp | pip | 1.22 | 1.3E-06 |
| pd405 | nPenGly | MePhe | Leu | MeAbu | Thr | MeGly | MePhe | MeAbu | Ser(tBu) | MeLeu | Asp | pip | 1.24 | 2.2E-06 |
| pd406 | D-Leu | MePhe | Ile | MeAbu | Thr | MeGly | nPenGly | Ser(tBu) | MePhe | MeLeu | Asp | pip | 1.27 | 1.1E-06 |
| pd407 | D-Ala | MePhe | Val | nPenGly | Thr | MeAla | MeLeu | Ser(tBu) | MePhe | MeLeu | Asp | pip | 1.22 | 3.0E-06 |
| pd408 | MeAbu | Leu | MeLeu | MePhe | Abu | MePhe | nPenGly | Thr | MeGly | MeLeu | Asp | pip | 1.29 | 1.8E-06 |
| pd409 | D-Ala | MePhe | Leu | MeAbu | Thr | (PhEt)NGly | MeLeu | Ser(tBu) | MeLeu | MeAbu | Asp | pip | 1.22 | 1.4E-06 |
| pd410 | MeVal | Leu | MeAbu | MePhe | Ala | MeLeu | MeAbu | Thr | (PhEt)NGly | MeLeu | Asp | pip | 1.28 | 6.0E-06 |
| pd411 | MeGly | MeLeu | Ile | MeAbu | Thr | (PhEt)NGly | MePhe | MeAbu | Ser(tBu) | MeLeu | Asp | pip | 1.23 | 3.3E-06 |
| pd412 | (PhEt)NGly | MeLeu | Leu | MeAbu | Thr | MeGly | MePhe | MeAbu | Ser(tBu) | MeLeu | Asp | pip | 1.23 | 1.5E-06 |
| pd413 | D-Leu | MeLeu | Ile | MeAbu | Thr | MeGly | (PhEt)NGly | Ser(tBu) | MePhe | MeLeu | Asp | pip | 1.26 | 1.5E-06 |
| pd414 | D-Ala | MeLeu | Ile | (PhEt)NGly | Thr | MeAbu | MeLeu | Ser(tBu) | MePhe | MeAbu | Asp | pip | 1.22 | 1.6E-06 |
| pd415 | MeAbu | Leu | MeLeu | MePhe | Abu | MeLeu | (PhEt)NGly | Thr | MeGly | MeLeu | Asp | pip | 1.28 | 3.3E-06 |
| pd416 | D-Leu | MePhe | Leu | MeAbu | Thr | (EtOEt) NGly | MeLeu | Ser(tBu) | MePhe | MeAbu | Asp | pip | 1.23 | 3.1E-06 |

(continued)

| Compound ID | **11** | **10** | **9** | **8** | **7** | **6** | **5** | **4** | **3** | **2** | **1** | **C-term** | **cLogP/ total AA** | **Caco-2** |
|---|---|---|---|---|---|---|---|---|---|---|---|---|---|---|
| pd417 | MeVal | Leu | MeAbu | MePhe | Leu | MePhe | MeAbu | Thr | (EtOEt) NGly | MeLeu | Asp | pip | 1.29 | 1.0E-05 |
| pd418 | MeGly | MePhe | Val | MeLeu | Thr | (EtOEt) NGly | MePhe | MeLeu | Ser(tBu) | MeLeu | Asp | pip | 1.23 | 1.5E-06 |
| pd419 | MeAbu | Leu | MeLeu | MePhe | Abu | MePhe | (EtOEt) NGly | Thr | nBuGly | MeLeu | Asp | pip | 1.31 | 2.3E-06 |
| pd420 | MeVal | Leu | MeAbu | MePhe | Ala | MeLeu | MeAbu | Thr | (PhOEt) NGly | MeLeu | Asp | pip | 1.29 | 4.5E-06 |
| pd421 | (PhOEt) NGly | MeLeu | Leu | MeAbu | Thr | MeGly | MePhe | MeAbu | Ser(tBu) | MeLeu | Asp | pip | 1.24 | 1.3E-06 |
| pd422 | D-Leu | MePhe | Leu | MeAbu | Thr | nBuGly | MeAla | Ser(tBu) | MeLeu | (PhOEt) NGly | Asp | pip | 1.29 | 1.0E-06 |
| pd423 | D-Ala | MeLeu | Ile | (PhOEt) NGly | Thr | MeAbu | MeLeu | Ser(tBu) | MePhe | MeAbu | Asp | pip | 1.23 | 1.0E-06 |
| pd424 | MeAbu | Leu | MeLeu | MePhe | Abu | MeLeu | (PhOEt) NGly | Thr | MeGly | MeLeu | Asp | pip | 1.29 | 1.2E-06 |
| pd425 | MeAla | Ser(S-2-PrOH) | MeLeu | MePhe(3-Cl) | Leu | MePhe | MeIle | Thr | nPrGly | MeLeu | Asp | pip | 1.28 | 1.2E-06 |
| pd426 | D-Val | MePhe | Ser (tBuOH) | MeLeu | Thr | nPrGly | MeLeu | Ser(iPen) | MePhe | MeVal | Asp | pip | 1.26 | 1.4E-06 |
| pd427 | D-Val | MePhe | Leu | MeLeu | Thr | MeGly | MeLeu | Ser (tBuOH) | MePhe(3-Cl) | MeLeu | Asp | pip | 1.25 | 1.3E-06 |
| pd428 | MeAla | Ser (tBuOH) | MeLeu | MePhe | Leu | MePhe | MeIle | Thr | nPrGly | MeLeu | Asp | pip | 1.25 | 2.8E-06 |
| pd429 | MeAla | Leu | MeLeu | MePhe | Ser (tBuOH) | MePhe | MeIle | Thr | nPrGly | MeLeu | Asp | pip | 1.25 | 3.3E-06 |
| pd430 | MeGly | MePhe | Ser (tBuOH) | MeLeu | Thr | nPrGly | MePhe(3-Cl) | MeLeu | Ser(tBu) | MeLeu | Asp | pip | 1.22 | 1.3E-06 |
| pd431 | nPrGly | MePhe | Ile | MeLeu | Thr | nPrGly | MePhe | MeLeu | Ser(tBuOH) | MeVal | Asp | pip | 1.26 | 1.1E-06 |

(continued)

| Compound ID | **11** | **10** | **9** | **8** | **7** | **6** | **5** | **4** | **3** | **2** | **1** | **C-term** | **cLogP/ total AA** | **Caco-2** |
|---|---|---|---|---|---|---|---|---|---|---|---|---|---|---|
| pd432 | D-Val | MePhe | Leu | MeLeu | Thr | nPrGly | MeLeu | Ser (tBuOH) | MePhe | MeLeu | Asp | pip | 1.29 | 1.5E-06 |
| pd433 | D-Val | MePhe | Ser(2-Me-2-BuOH) | MeLeu | Thr | MeGly | MeLeu | Ser(iPen) | MePhe | MeLeu | Asp | pip | 1.23 | 1.4E-06 |
| pd434 | D-Val | MePhe | Leu | MeVal | Thr | MeGly | MeLeu | Ser(2-Me-2-BuOH) | MePhe(3-Cl) | MeLeu | Asp | pip | 1.23 | 1.7E-06 |
| pd435 | g-MeAbu | MeVal | Ile | MePhe | MePhe(3-Cl) | Ser(2-Me-2-BuOH) | MeLeu | Thr | MeLeu | MeLeu | Asp | pip | 1.24 | 1.4E-06 |
| pd436 | MeAla | Ser(2-Me-2-BuOH) | MeLeu | MePhe | Leu | MeHph | MeLeu | Thr | MeGly | MeLeu | Asp | pip | 1.23 | 1.3E-06 |
| pd437 | MeAla | Leu | MeLeu | MePhe | Ser(2-Me-2-BuOH) | MeHph | MeLeu | Thr | MeGly | MeLeu | Asp | pip | 1.23 | 1.3E-06 |
| pd438 | D-Val | MeHph | Leu | MeLeu | Thr | MeGly | MeLeu | Ser(2-Me-2-BuOH) | MePhe | MeLeu | Asp | pip | 1.26 | 1.0E-06 |
| pd439 | MeLeu | Leu | MeLeu | MePhe | Leu | MePhe | MeLeu | Gln(Me2) | MeGly | MeLeu | Asp | pip | 1.35 | 1.0E-06 |
| pd440 | MeAla | MePhe | Leu | MeLeu | Gln(Me2) | MeAla | MeLeu | Ser(tBu) | MeLeu | MeLeu | Asp | pip | 1.22 | 4.0E-06 |
| pd441 | D-Leu | MePhe | Leu | MeLeu | Gln(Me2) | MeGly | MeLeu | Ser(tBu) | MePhe | MeLeu | Asp | pip | 1.25 | 1.7E-06 |
| pd442 | D-Leu | MePhe | Ser(NtBu-Aca) | MeLeu | Thr | nPrGly | MeLeu | Leu | MePhe | MeAla | Asp | pip | 1.22 | 1.1E-06 |
| pd443 | nBuGly | MePhe | Ile | MeLeu | Thr | nPrGly | MePhe | MeLeu | Ser(NtBu-Aca) | MeAla | Asp | pip | 1.25 | 1.0E-06 |
| pd444 | D-Leu | MePhe | Leu | MeLeu | Thr | MeGly | MeLeu | Hse(Et) | MePhe | MeLeu | Asp | pip | 1.21 | 1.1E-06 |
| pd445 | MeGly | MePhe | Hse(Et) | MeLeu | Thr | nBuGly | MePhe | MeLeu | Leu | MeLeu | Asp | pip | 1.24 | 1.1E-06 |
| pd446 | g-MeAbu | MeLeu | Val | MePhe | MePhe | Ser(tBu) | MeLeu | Nle(6-OH) | MeLeu | MeLeu | Asp | pip | 1.27 | 1.3E-06 |
| pd447 | g-MeAbu | MeVal | Val | MePhe | MePhe | Ser(tBu) | MeLeu | Nle(6-OH) | MeLeu | MeLeu | Asp | pip | 1.22 | 1.1E-06 |
| pd448 | MeAla | Val | MeLeu | MePhe | Val | MePhe | MeLeu | Nle(6-OH) | MeGly | MeLeu | Asp | pip | 1.22 | 1.3E-06 |
| pd449 | D-Leu | MePhe | Leu | MeLeu | Nle(6-OH) | MeGly | MeLeu | Ser(tBu) | MePhe | MeVal | Asp | pip | 1.30 | 2.0E-06 |

(continued)

| Compound ID | **11** | **10** | **9** | **8** | **7** | **6** | **5** | **4** | **3** | **2** | **1** | **C-term** | **cLogP/ total AA** | **Caco-2** |
|---|---|---|---|---|---|---|---|---|---|---|---|---|---|---|
| pd450 | D-Val | MePhe | Leu | MeLeu | Nle(6-OH) | MeGly | MeLeu | Ser(tBu) | MePhe | MeVal | Asp | pip | 1.25 | 1.8E-06 |
| pd451 | MeAla | Leu | MeLeu | MePhe | Leu | MePhe | MeLeu | Nle(6-OH) | MeGly | MeLeu | Asp | pip | 1.32 | 4.1E-06 |
| pd452 | D-Leu | MePhe | Leu | MeAbu (Mor) | Thr | nBuGly | MeLeu | Ser(tBu) | MePhe | MeVal | Asp | pip | 1.25 | 1.3E-06 |
| pd453 | MeAla | Leu | MeLeu | MePhe | Leu | MePhe | MeAbu (Mor) | Thr | nBuGly | MeVal | Asp | pip | 1.22 | 2.3E-06 |
| pd454 | MeAla | Leu | MeLeu | MePhe | Leu | MePhe | MeLeu | Thr | nBuGly | MeAbu (Mor) | Asp | pip | 1.27 | 1.2E-06 |
| pd455 | MeGly | MePhe | Val | MeLeu | Thr | (2-(pip-4-F2)-Et)Gly | MePhe | MeLeu | Ser(tBu) | MeLeu | Asp | pip | 1.23 | 1.1E-06 |
| pd456 | MeAla | Leu | MeLeu | MePhe | Leu | MePhe | (2-(pip-4-F2)-Et)Gly | Thr | MeGly | MeLeu | Asp | pip | 1.20 | 5.0E-06 |
| pd457 | D-Ala | Pro(4-pip-4-F2) | Ser(tBu) | MeLeu | MeLeu | MeLeu | Thr | MePhe | Phe | MeLeu | Asp | pip | 1.24 | 1.1E-06 |
| pd458 | Pro(4-pip-4-F2) | MePhe(3-Cl) | Val | MeVal | Thr | MeGly | MeLeu | Ser(tBu) | MePhe | MeLeu | Asp | pip | 1.22 | 1.2E-06 |
| pd459 | D-Abu | MeLeu | Ser(tBu) | MeLeu | Leu | MeLeu | Thr | Pro(4-pip-4-F2) | Phe | MeLeu | Asp | pip | 1.24 | 1.6E-06 |
| pd460 | Val | Gly | MeLeu | MeLeu | MeLeu | MePhe | Thr | MePhe | MeLeu | Pro(4-pip-4-F2) | Asp | pip | 1.26 | 1.2E-06 |
| pd461 | cisPro(4-pip-4-F2) | MePhe(3-Cl) | Val | MeVal | Thr | MeGly | MeLeu | Ser(tBu) | MePhe | MeLeu | Asp | pip | 1.22 | 1.4E-06 |
| pd462 | D-Abu | MeLeu | Ser(tBu) | MeLeu | Leu | MeLeu | Thr | cisPro(4-pip-4-F2) | Phe | MeLeu | Asp | pip | 1.24 | 1.1E-06 |
| pd463 | MeLeu | Leu | MeLeu | MePhe | Phe | MeVal | MeLeu | Gln(Me) | MeGly | MeLeu | Asp | pip | 1.28 | 1.2E-06 |
| pd464 | D-Leu | MePhe | Leu | MeLeu | Gln(Me) | MeGly | MeLeu | Ser(tBu) | MePhe | MeLeu | Asp | pip | 1.23 | 1.1E-06 |
| pd465 | MeLeu | Leu | MeLeu | MeAla(4-Thz) | Leu | MePhe | MeIle | Gln(Me) | nBuGly | MeLeu | Asp | pip | 1.33 | 1.1E-06 |

(continued)

| Compound ID | **11** | **10** | **9** | **8** | **7** | **6** | **5** | **4** | **3** | **2** | **1** | **C-term** | **cLogP/ total AA** | **Caco-2** |
|---|---|---|---|---|---|---|---|---|---|---|---|---|---|---|
| pd466 | D-Ala | MePhe | Leu | MeLeu | Ahp(2)(3-R-OH) | MeGly | MeLeu | Ser(tBu) | MePhe | MeAla | Asp | pip | 1.21 | 1.1E-06 |
| pd467 | D-Val | MePhe | Ala | MeLeu | Ahp(2)(3-R-OH) | MeGly | MeLeu | Ser(tBu) | MePhe | MeAla | Asp | pip | 1.16 | 1.4E-06 |
| pd468 | g-MeAbu | MeLeu | Ile | MePhe | MePhe | Ahp(2)(3-R-OH) | MeLeu | Thr | MeAla | MeLeu | Asp | pip | 1.17 | 1.0E-06 |
| pd469 | MeAla | Val | MeLeu | MePhe | Ala | MePhe | MeLeu | Ahp(2)(3-R-OH) | MeGly | MeLeu | Asp | pip | 1.26 | 2.5E-06 |
| pd470 | D-Val | MePhe | Leu | MeLeu | Lys(Ac) | MeGly | MeLeu | Ser(tBu) | MePhe | MeVal | Asp | pip | 1.23 | 2.1E-06 |
| pd471 | D-Leu | MePhe | Leu | MeLeu | Lys(Ac) | MeGly | MeLeu | Ser(tBu) | MePhe | MeVal | Asp | pip | 1.27 | 2.1E-06 |
| pd472 | D-Val | MeVal | Phe(4-CHF2) | MeLeu | Thr | MeGly | MeLeu | Ser(tBu) | MePhe | MeLeu | Asp | pip | 1.25 | 1.1E-06 |
| pd473 | MeAla | Phe(4-CHF2) | MeLeu | MePhe | Leu | MeLeu | MeIle | Thr | MeGly | MeAbu | Asp | pip | 1.23 | 4.5E-06 |
| pd474 | MeAla | Leu | MeLeu | MeLeu | Phe(4-CHF2) | MeLeu | MeVal | Thr | MeGly | MeLeu | Asp | pip | 1.27 | 4.5E-06 |
| pd475 | MeGly | MeLeu | Ile | MeLeu | Thr | MeGly | MePhe | MeLeu | Phe(4-CHF2) | MeVal | Asp | pip | 1.22 | 4.2E-06 |
| pd476 | D-Val | MeVal | Phe(4-OCHF2) | MeLeu | Thr | MeGly | MeLeu | Ser(tBu) | MePhe | MeLeu | Asp | pip | 1.27 | 1.1 E-06 |
| pd477 | D-Leu | MePhe | Val | MeLeu | Thr | MeGly | MeAla | Phe(4-OCHF2) | MeLeu | MeLeu | Asp | pip | 1.23 | 2.2E-06 |
| pd478 | gMeAbu | MeLeu | Phe(4-OCHF2) | MeLeu | MePhe | Ser(tBu) | MeLeu | Thr | MeAla | MeLeu | Asp | pip | 1.20 | 1.3E-06 |
| pd479 | MeAla | Phe(4-OCHF2) | MeLeu | MePhe | Leu | MeLeu | MeIle | Thr | nBuGly | MeAla | Asp | pip | 1.35 | 1.0E-06 |
| pd480 | MeAla | Leu | MeLeu | MeLeu | Phe(4-OCHF2) | MeAla | MeIle | Thr | MeGly | MeLeu | Asp | pip | 1.20 | 5.0E-06 |

(continued)

| Compound ID | 11 | 10 | 9 | 8 | 7 | 6 | 5 | 4 | 3 | 2 | 1 | C-term | cLogP/ total AA | Caco-2 |
|---|---|---|---|---|---|---|---|---|---|---|---|---|---|---|
| pd481 | MeGly | MeLeu | Ile | MeLeu | Thr | MeGly | MePhe | MeLeu | Phe(4-OCHF2) | MeVal | Asp | pip | 1.24 | 3.8E-06 |
| pd482 | MeAbu | Leu | MeLeu | MePhe | Ser( Et-2-Mor) | MeAbu | MeLeu | Thr | nBuGly | MeLeu | Asp | pip | 1.29 | 1.1 E-06 |
| pd483 | MeAbu | Ser(Et-2-Mor) | MeLeu | MeAbu | Val | MePhe | MeLeu | Thr | nBuGly | MeLeu | Asp | pip | 1.24 | 1.9E-06 |
| pd484 | MeGly | MeLeu | Ser(Et-2-Mor) | MeLeu | Thr | nBuGly | MePhe | MeLeu | Val | MeLeu | Asp | pip | 1.23 | 1.8E-06 |
| pd485 | gMeAbu | MeLeu | Ile | MePhe | MeAbu | Ser(tBu) | MeLeu | Ser(Et-2-Mor) | MeLeu | MeLeu | Asp | pip | 1.28 | 1.2E-06 |
| pd486 | MeGly | MeAbu | Val | MeLeu | Ser(Et-2-Mor) | nBuGly | MePhe | MeLeu | Leu | MeLeu | Asp | pip | 1.34 | 1.1E-06 |
| pd487 | MeAbu | Leu | MeLeu | MePhe | Abu(5-Oxo-Odz) | MeLeu | MeLeu | Thr | nBuGly | MeLeu | Asp | pip | 1.26 | 2.2E-06 |
| pd488 | MeGly | MeLeu | Val | MeLeu | Abu(5-Oxo-Odz) | nBuGly | MePhe | MeLeu | Leu | MeLeu | Asp | pip | 1.32 | 1.6E-06 |
| pd489 | MeAla | Ser(R-2-PrOH) | MeLeu | MePhe(3-Cl) | Leu | MePhe | MeIle | Thr | nBuGly | MeLeu | Asp | pip | 1.33 | 1.1E-06 |
| pd490 | MeGly | MePhe(3-Cl) | Ser(R-2-PrOH) | MeLeu | Thr | nBuGly | MePhe | MeLeu | Ser(tBu) | MeLeu | Asp | pip | 1.23 | 1.0E-06 |
| pd491 | MeVal | Ser(R-2-PrOH) | MeLeu | MePhe(3-Cl) | Leu | MePhe | MeIle | Thr | MeGly | MeLeu | Asp | pip | 1.26 | 1.1E-06 |
| pd492 | D-Val | MePhe | Ser(R-2-PrOH) | MeLeu | Val | MeGly | MeLeu | Ser(tBu) | MePhe | MeLeu | Asp | pip | 1.24 | 2.5E-06 |
| pd493 | gMeAbu | MeLeu | Val | MePhe | MePhe | Ser(tBu) | MeLeu | Ser(R-2-PrOH) | MeLeu | MeLeu | Asp | pip | 1.25 | 1.3E-06 |
| pd494 | MeAla | Leu | MeLeu | MePhe | Leu | MePhe | MeIle | Ser(R-2-PrOH) | MeGly | MeLeu | Asp | pip | 1.30 | 5.2E-06 |

(continued)

| Compound ID | 11 | 10 | 9 | 8 | 7 | 6 | 5 | 4 | 3 | 2 | 1 | C-term | cLogP/ total AA | Caco-2 |
|---|---|---|---|---|---|---|---|---|---|---|---|---|---|---|
| pd495 | MeAla | Ser(R-2-PrOH) | MeLeu | MePhe | Leu | MePhe | MeIle | Val | MeGly | MeLeu | Asp | pip | 1.26 | 3.7E-06 |
| pd496 | MeGly | MePhe | Val | MeLeu | Ser(R-2-PrOH) | nBuGly | MePhe | MeLeu | Ser(tBu) | MeLeu | Asp | pip | 1.31 | 1.0E-06 |
| pd497 | MeGly | MePhe | Ser(R-2-PrOH) | MeLeu | Ile | MeGly | MePhe | MeLeu | Ser(tBu) | MeLeu | Asp | pip | 1.21 | 3.3E-06 |
| pd498 | gMeAbu | MeLeu | Val | MePhe | MePhe | Ser(tBu) | MeLeu | Ser(EtOH) | MeLeu | MeLeu | Asp | pip | 1.23 | 1.1E-06 |
| pd499 | MeAla | Leu | MeLeu | MePhe | Leu | MePhe | MeIle | Ser(EtOH) | MeGly | MeLeu | Asp | pip | 1.28 | 4.7E-06 |
| pd500 | MeAla | Ser(EtOH) | MeLeu | MePhe | Leu | MePhe | MeIle | Val | MeGly | MeLeu | Asp | pip | 1.23 | 2.2E-06 |
| pd501 | MeGly | MePhe | Val | MeLeu | Ser(EtOH) | nBuGly | MePhe | MeLeu | Ser(tBu) | MeLeu | Asp | pip | 1.28 | 1.1E-06 |
| pd502 | MeGly | MePhe | Hnl(7-F3-6-OH) | MeLeu | Ile | MeGly | MePhe | MeLeu | Ser(tBu) | MeLeu | Asp | pip | 1.27 | 1.0E-06 |
| pd503 | MeAla | Leu | MeAbu | MePhe | Leu | MePhe | MeVal | Ser(1-CF3-EtOH) | MeGly | MeLeu | Asp | pip | 1.23 | 2.0E-06 |
| pd504 | MeGly | MePhe | Ser(1-CF3-EtOH) | MeLeu | Ile | MeGly | MePhe | MeLeu | Ser(tBu) | MeLeu | Asp | pip | 1.26 | 4.1E-06 |

Withou wishing to be bound by any particular theory, the present inventors consider as follows. Based on the above results, it would be difficult to improve membrane permeability of cyclic peptide compounds having a side chain such as Trp, Tyr, or Tyr(3-F) by the hit-to-lead strategy even if they are obtained as hit compounds. On the other hand, other long side chains can contribute to binding to target molecules during panning, and cyclic peptide compounds having such side chains can have high membrane permeability. Therefore, such long side chains would be useful for obtaining cyclic peptide compounds capable of specifically binding to target molecules and having high membrane permeability.

[Example 6] Evaluation of the effect of aromatic ring count (ARC) in a cyclic peptide on membrane permeability

The following data demonstrated that aromatic ring count (ARC), the number of aromatic rings contained in the side chains of the cyclic portion of a cyclic peptide compound is preferably 3 or less to achieve $P_{app} \geq 1.0 \times 10^{-6}$ (Fig. 14). As the ARC was increased, the $P_{app}$, i.e., membrane permeability of a cyclic peptide compound was generally decreased. When the ARC was 4 or more, it was difficult to achieve $P_{app} \geq 1.0 \times 10^{-6}$.

In this analysis, cyclic peptide compounds having a fused-ring structure in the side chains of the cyclic portion and cyclic peptide compounds having a substituted or unsubstituted hydroxyphenyl group were excluded from analysis targets. When ARC = 3, a $P_{app}$ of $1.0 \times 10^{-6}$ or more was observed in sequences having a ClogP/total AA of 0.88 or more. On the other hand, almost no compounds where ARC = 4 had a $P_{app}$ of $1.0 \times 10^{-6}$ or more even when such compounds had a ClogP/total AA (0.88 or more) comparable to those of the above compounds where ARC = 3. This demonstrated that when sequences where ARC = 3 and sequences where ARC = 4 are compared, the sequences where ARC = 3 had higher membrane permeability than the sequences where ARC = 4, although the sequences where ARC = 3 were as lipophilic as the sequences where ARC = 4 (Tables 23-1, 23-2, and 24). The columns of Tables 23-1, 23-2, and 24 are described taking as an example pd198, a peptide containing linear portion 1. As described above, the intersection unit is defined as the first amino acid. In pd198, the first amino acid is Asp. The amino acid adjacent to the intersection unit (Asp) and constituting the cyclic portion is defined as the second amino acid (which is Phe in pd198), and subsequent amino acids ranging from the third and fourth amino acids to the N-terminal amino acid (**▲** unit) are defined in the same manner (in pd198, the third amino acid is MeVal, the fourth amino acid is MeLeu, and the N-terminal amino acid (**▲** unit) is the 10th amino acid g-MeAbu because the cyclic portion is composed of 10 residues). The amino acids of the linear portion 1 are represented by H-1, H-2, and H-3 sequentially from the side of the intersection unit (which is Asp in pd198). In pd198, since H-1 is MePhe and H-2 is Ala, the linear portion 1 represents a moiety in which MePhe is bonded to the C-terminus of the intersection unit Asp and Ala is then bonded to MePhe. The C-term column shows a functional group condensed with the carboxylic acid site of the C-terminal amino acid, where pip represents piperidine and pyrro represents pyrroridine. "None" in the C-term column means that the C-terminal amino acid is present as carboxylic acid (in pd198, the C-terminal amino acid is Ala of H-2, the carboxylic acid site of which is condensed with pip (piperidine)). Tables 23-1, 23-2, and 24 show that pd198 is a peptide in which the ▲ unit and the intersection unit are cyclized by an amide bond. Cyclic peptides described in the text are all expressed in the same manner unless otherwise stated.

[Table 23-1]

Sequences where ARC = 3 having a $P_{app}$ in the range of $P_{app} \geq 1.0 \times 10^{-6}$

| Compound ID | 11 | 10 | 9 | 8 | 7 | 6 | 5 | 4 | 3 | 2 | 1 | H-1 | H-2 | H-3 | C-term | cLogP/ total AA | Caco-2 (cm/sec) |
|---|---|---|---|---|---|---|---|---|---|---|---|---|---|---|---|---|---|
| pd162 | Pro | Phe | Ser(tBu) | MeLeu | Thr | MeGly | MeVal | Phe | MePhe(3-Cl) | MeLeu | MeAsp | | | | pip | 1.25 | 1.2E-06 |
| pd163 | D-Pro | Phe | Ser(tBu) | MeLeu | Thr | MeGly | MeVal | Phe | MePhe(3-Cl) | MeLeu | MeAsp | | | | pip | 1.25 | 1.7E-06 |
| pd167 | D-MeAla | Phe | Ser(tBu) | MeLeu | Thr | MeGly | MeVal | Phe | MePhe(3-Cl) | MeLeu | MeAsp | | | | pip | 1.27 | 1.7E-06 |
| pd177 | MeGly | MePhe | Ser(Bn) | MeLeu | Thr | MeGly | MePhe | MeLeu | Ser(tBu) | MeLeu | Asp | | | | pip | 1.21 | 3.0E-06 |
| pd178 | MeGly | MePhe | Ile | MeLeu | Thr | MeGly | MePhe | MeLeu | Ser(Bn) | MeIle | Asp | | | | pip | 1.26 | 2.5E-06 |
| pd179 | MeAla | Leu | MeLeu | MePhe | Ser(Bn) | MePhe | MeAla | Thr | MeGly | MeLeu | Asp | | | | pip | 1.18 | 2.0E-06 |
| pd180 | D-Val | MePhe | Ser(3-F-5-Me-Pyr) | MeLeu | Thr | MeGly | MeLeu | Ser(tBu) | MePhe | MeVal | Asp | | | | pip | 1.13 | 2.4E-06 |
| pd181 | MeVal | Ser(3-F-5-Me-Pyr) | MeLeu | MePhe | Leu | MePhe | MeIle | Thr | MeGly | MeLeu | Asp | | | | pip | 1.28 | 1.1E-06 |
| pd182 | MeVal | Ala | MePhe | MeLeu | MeGly | Thr | MeVal | Hph | MeLeu | MePhe | Asp | | | | pip | 1.25 | 2.6E-06 |
| pd183 | MeAla | MePhe | Hph | MeLeu | Thr | MeGly | MeLeu | MePhe | Ser(tBu) | MeAla | Asp | | | | pip | 1.16 | 2.1E-06 |
| pd184 | MeAla | MePhe | Hph | MeLeu | Thr | MeGly | MeLeu | MeHph | Ser(tBu) | MeAla | Asp | | | | pip | 1.21 | 1.2E-06 |
| pd185 | D-Ala | MePhe | Ser(Ph-3-Cl) | MeVal | Thr | MeGly | MeLeu | Ser(tBu) | MePhe | MeVal | Asp | | | | pip | 1.19 | 2.2E-06 |
| pd186 | MeAla | Val | MeLeu | MePhe | Ser(Ph-3-Cl) | MePhe | MeIle | Thr | MeGly | MeAla | Asp | | | | pip | 1.21 | 1.8E-06 |
| pd187 | D-Val | MePhe | Ser(Ph-3-Cl) | MeAla | Thr | MeGly | MeLeu | Ser(tBu) | MePhe | MeVal | Asp | | | | pip | 1.19 | 1.9E-06 |
| pd188 | MeAla | Ser(Ph-3-Cl) | MeLeu | MePhe | Ala | MePhe | MeIle | Thr | MeGly | MeLeu | Asp | | | | pip | 1.26 | 1.3E-06 |
| pd189 | MeGly | MePhe | Phe{#(CH2)2} | MeLeu | Thr | MeGly | MePhe | MeLeu | Ser(tBu) | MeIle | Asp | | | | pip | 1.29 | 1.8E-06 |
| pd190 | MeGly | MePhe | Ile | MeLeu | Thr | MeGly | MePhe | MeLeu | Phe{#(CH2)2} | MeAla | Asp | | | | pip | 1.21 | 1.5E-06 |
| pd191 | MeAla | Phe{#(CH2)2} | MeLeu | MePhe | Ala | MePhe | MeVal | Thr | MeGly | MeLeu | Asp | | | | pip | 1.21 | 1.5E-06 |
| pd192 | D-Val | MePhe | Phe{#(CH2)2} | MeLeu | Thr | MeGly | MeLeu | Ser(tBu) | MePhe | MeAla | Asp | | | | pip | 1.24 | 1.1E-06 |
| pd193 | D-Ala | MePhe | Val | MeLeu | Thr | MeGly | MeLeu | Phe{#(CH2)2} | MePhe | MeVal | Asp | | | | pip | 1.24 | 1.0E-06 |
| pd194 | D-Ala | MePhe | Val | MeLeu | Thr | nPrGly | MeLeu | Ser(tBu) | MePhe | MePhe | Asp | | | | pip | 1.25 | 5.3E-06 |
| pd195 | nPrGly | MePhe | Ile | MePhe | Thr | MeGly | MePhe | MeLeu | Ser(tBu) | MeVal | Asp | | | | pip | 1.26 | 5.0E-06 |
| pd196 | Pro | MePhe | Leu | MeLeu | Thr | MeGly | MeVal | Ser(tBu) | MePhe | MePhe | MeAsp | | | | pip | 1.24 | 4.0E-06 |
| pd197 | MeAla | MePhe | Leu | MeLeu | Ala(3-Pyr) | MeGly | MeLeu | Ser(tBu) | MePhe | MeVal | Asp | | | | pip | 1.25 | 3.0E-06 |
| pd198 | | g-MeAbu | Val | MeLeu | Thr | MePhe | Gly | MeLeu | MeVal | Phe | Asp | MePhe | Ala | | pip | 0.88 | 2.7E-06 |
| pd199 | D-MeAla | MePhe | Leu | MeLeu | Thr | MeGly | MeVal | Ser(tBu) | MePhe | MePhe | MeAsp | | | | pip | 1.26 | 2.6E-06 |
| pd200 | D-Pro | MePhe | Leu | MeLeu | Thr | MeGly | MeVal | Ser(tBu) | MePhe | MePhe | MeAsp | | | | pip | 1.24 | 2.6E-06 |

[Table 23-2]

| Compound ID | 11 | 10 | 9 | 8 | 7 | 6 | 5 | 4 | 3 | 2 | 1 | H-1 | H-2 | H-3 | C-term | cLogP/ total AA | Caco-2 (cm/sec) |
|---|---|---|---|---|---|---|---|---|---|---|---|---|---|---|---|---|---|
| pd201 | | D-MeAla | MeAla | MePhe | Thr | MeAla | MeLeu | Ile | MeLeu | MeVal | Asp | MePhe | MePhe | Ala | pip | 1.12 | 2.3E-06 |
| pd202 | MeAla | MePhe | Leu | MeLeu | Thr | MeGly | MeVal | Ser (tBu) | MePhe | MePhe | MeAsp | | | | pip | 1.26 | 2.1 E-06 |
| pd203 | Pro | MePhe | Leu | MeLeu | Thr | MeGly | MeLeu | Ser (tBu) | MePhe | MePhe | Asp | | | | pip | 1.23 | 2.1E-06 |
| pd204 | MeAla | Leu | MeLeu | MePhe | Leu | MePhe | MePhe | Thr | MeGly | MeLeu | Asp | | | | pip | 1.29 | 2.0E-06 |
| pd205 | MeGly | MePhe | Ile | MePhe | Thr | MeGly | MePhe | MeLeu | Ser (tBu) | MeVal | Asp | | | | pip | 1.15 | 1.8E-06 |
| pd206 | D-Pro | MePhe | Leu | MeLeu | Thr | MeGly | MeLeu | Ser (tBu) | MePhe | MePhe | Asp | | | | pip | 1.23 | 1.8E-06 |
| pd207 | | | b-MeAla | MeLeu | MePhe | Leu | MePhe | Thr | MePhe | MeLeu | Asp | | | | pip | 1.28 | 1.7E-06 |
| pd208 | D-Ala | MeLeu | MeAla | MePhe | Ser (tBu) | MePhe | Leu | MeVal | Thr | MePhe | MeAsp | | | | pip | 1.26 | 1.7E-06 |
| pd209 | Ala | MePhe | Leu | MeLeu | Thr | MeGly | MeLeu | Ser (tBu) | MePhe | MePhe | MeAsp | | | | pip | 1.26 | 1.7E-06 |
| pd210 | D-Val | MePhe | Val | MePhe | Thr | MeGly | MeLeu | Ser (tBu) | MePhe | MeLeu | Asp | | | | pip | 1.23 | 1.6E-06 |
| pd211 | | | MeAla | MeLeu | Leu | MePhe | Thr | MePhe | MePhe | MeLeu | Asp | | | | pip | 1.39 | 1.4E-06 |
| pd212 | | | MeAla | Thr | MeAla | MeLeu | Val | MePhe | Phe | MeLeu | Asp | MePhe | Ala | | pip | 1.03 | 1.4E-06 |
| pd213 | MeAla | MeLeu | MeAla | MePhe | Ser (tBu) | MePhe | Leu | MeVal | Thr | MePhe | MeAsp | | | | pip | 1.31 | 1.3E-06 |
| pd214 | Pro | MeLeu | MeAla | MePhe | Ser (tBu) | MePhe | Leu | MeLeu | Thr | MePhe | Asp | | | | pip | 1.28 | 1.2E-06 |
| pd215 | D-Val | MePhe | Leu | MeLeu | Thr | MeGly | MeLeu | Ser (tBu) | MePhe | MePhe | Asp | | | | pip | 1.27 | 1.2E-06 |
| pd216 | g-MeAbu | MeLeu | Val | MePhe | MePhe | Ser (tBu) | MeLeu | Thr | MePhe | MeLeu | Asp | | | | pip | 1.25 | 1.2E-06 |

(continued)

| Compound ID | **11** | **10** | **9** | **8** | **7** | **6** | **5** | **4** | **3** | **2** | **1** | **H-1** | **H-2** | **H-3** | **C-term** | **cLogP/ total AA** | **Caco-2** (cm/sec) |
|---|---|---|---|---|---|---|---|---|---|---|---|---|---|---|---|---|---|
| pd217 | Ala | MePhe | Leu | MeLeu | Thr | MeGly | MeLeu | Ser (tBu) | MePhe | MePhe | Asp | | | | pip | 1.19 | 1.1E-06 |
| pd218 | | | MeAla | MeLeu | Leu | MeLeu | MePhe | Phe | MeLeu | Thr | Asp | MePhe | Ala | | pip | 1.21 | 1.1E-06 |
| pd219 | D-Pro | MeLeu | MeAla | MePhe | Ser (tBu) | MePhe | Leu | MeVal | Thr | MePhe | MeAsp | | | | pip | 1.29 | 1.1E-06 |
| pd220 | D-Ala | MeLeu | MeAla | MePhe | Ser (tBu) | MePhe | Leu | MeLeu | Thr | MePhe | Asp | | | | pip | 1.24 | 1.1E-06 |
| pd221 | D-Val | MePhe | Leu | MeLeu | Thr | MeGly | MeLeu | Val | MePhe | MePhe | Asp | | | | pip | 1.28 | 1.1E-06 |
| pd222 | D-MeAla | Ile | MeLeu | MeLeu | Phe | MeVal | MeAla | Thr | MeGly | MePhe | Asp | MePhe | Ala | | pip | 1.06 | 1.1E-06 |
| pd223 | | | D-MeAla | MeLeu | MePhe | Leu | MePhe | Thr | MePhe | MeLeu | Asp | | | | pip | 1.39 | 1.1E-06 |
| pd224 | D-Ala | MePhe | Leu | MeLeu | Thr | MeGly | MeLeu | Ser (tBu) | MePhe | MePhe | MeAsp | | | | pip | 1.26 | 1.0E-06 |
| pd225 | D-Leu | MePhe | Leu | MePhe | Thr | MeGly | MeLeu | Ile | MePhe | MeLeu | Asp | | | | pip | 1.37 | 1.0E-06 |

[Table 24]

Sequences where ARC = 4 having a $P_{app}$ in the range of $P_{app} < 1.0 \times 10^{-6}$

| Compound ID | 11 | 10 | 9 | 8 | 7 | 6 | 5 | 4 | 3 | 2 | 1 | H-1 | H-2 | H-3 | C-term | cLogP/ total AA | Caco-2 (cm/sec) |
|---|---|---|---|---|---|---|---|---|---|---|---|---|---|---|---|---|---|
| pd226 | MeAla | MePhe | Leu | MeLeu | Thr | MeAla | MeAla(4-Thz) | Phe | MePhe | MeLeu | Asp | | | | pip | 1.19 | 6.8E-07 |
| pd227 | MePhe | Leu | MeLeu | MePhe | Leu | MePhe | MePhe | Thr | MeGly | MeLeu | Asp | | | | pip | 1.42 | 6.1E-07 |
| pd228 | MeLeu | Leu | MeLeu | MePhe | Phe | MePhe | MeAla(4-Thz) | Thr | MeGly | MeLeu | Asp | | | | pip | 1.27 | 5.8E-07 |
| pd229 | MeVal | MePhe | Leu | MeLeu | MeAla | MeGly | MePhe | Phe | MePhe | Thr | Asp | | | | pip | 1.24 | 4.1E-07 |
| pd230 | MePhe | Phe | Thr | MePhe | Leu | MePhe | MeLeu | MeGly | MeLeu | MeAla | Asp | | | | pip | 1.29 | 2.5E-07 |
| pd231 | MeAla | MePhe | Leu | MeLeu | Phe | MeGly | MePhe | Thr | MePhe | Leu | Asp | | | | pip | 1.24 | 2.1E-07 |
| pd232 | MeAla | MeLeu | MePhe | Ala | MeLeu | MePhe | MePhe | Thr | MeAla | MePhe | Asp | | | | pip | 1.26 | 1.9E-07 |
| pd233 | MeAla | MePhe | MeLeu | Val | MeLeu | MePhe | MePhe | Thr | MeAla | MePhe | Asp | | | | pip | 1.35 | 1.7E-07 |
| pd234 | D-Ala | MeLeu | Leu | MePhe | Ser(tBu) | MePhe | Thr | MeAla | MePhe | MePhe | Asp | | | | pip | 1.24 | 1.5E-07 |
| pd235 | | Ala | Phe | MeLeu | Ala(4-Thz) | MePhe | Gly | MeLeu | MeLeu | Leu | Asp | MePhe | Ala | | pip | 1.06 | 1.0E-07 |
| pd236 | MeAla | Leu | MeLeu | Phe | Phe | MePhe | MeAla(4-Thz) | Thr | MeGly | MeLeu | Asp | | | | pip | 1.08 | 9.6E-08 |
| pd237 | Ala | MePhe | MeAla | MePhe | MeLeu | Phe | MeLeu | Ser(tBu) | Thr | MePhe | Asp | | | | pip | 1.23 | 8.9E-08 |
| pd238 | MeAla | MePhe | Leu | Thr | MeLeu | MeAla | MeAla(4-Thz) | Phe | MePhe | MeLeu | Asp | | | | pip | 1.19 | 8.8E-08 |
| pd239 | | b-MeAla | MePhe | MeLeu | MeAla | MePhe | Ile | MeLeu | MeLeu | Thr | Asp | MePhe | MePhe | Ala | pip | 1.18 | 6.5E-08 |
| pd240 | | MeAla | MePhe | Ile | MeAla | MeLeu | MeLeu | Thr | MePhe | MeLeu | Asp | MePhe | MePhe | Ala | pip | 1.27 | 6.0E-08 |
| pd241 | | | D-Val | Ala | Thr | MeAla | Phe | MeLeu | MePhe | MeLeu | Asp | MePhe | Phe | | pip | 1.11 | 5.8E-08 |
| pd242 | | D-Ala | Thr | MePhe | Ile | MeAla | MeLeu | MePhe | MeLeu | MeLeu | Asp | MePhe | MePhe | Ala | pip | 1.22 | 5.8E-08 |
| pd243 | D-Leu | MeLeu | MePhe | MePhe | Ser(tBu) | MePhe | Leu | MeLeu | Thr | MePhe | Asp | | | | pip | 1.50 | 5.7E-08 |
| pd244 | Ala | Phe | MeLeu | MePhe | Leu | Thr | MeLeu | Phe | MeGly | MePhe | Asp | | | | pip | 1.18 | 4.5E-08 |
| pd245 | MeLeu | Thr | Leu | MePhe | MePhe | MeGly | Phe | MeAla | Phe | Ser(tBu) | Asp | | | | pip | 1.11 | 1.9E-08 |
| pd246 | D-Ala | MePhe | MeGly | Leu | Thr | Phe | MeLeu | Ser(tBu) | MePhe | MePhe | Asp | | | | pip | 1.13 | 1.4E-08 |
| pd247 | | | Ala | Thr | MeAla | MePhe | Val | MePhe | MeLeu | Gly | Asp | MePhe | MePhe | Ala | pip | 0.88 | 8.7E-08 |

Examples of amino acids having basic and acidic side chains which had difficulty in achieving Papp ≥ 1.0 x $10^{-6}$ cm/sec in terms of membrane permeability

Sequences containing Ser(Et-2-NMe2), an amino acid having an observed basic pKa of 9.1 and a calculated basic pKa of 8.9, had difficulty in achieving Papp ≥ 1.0 x $10^{-6}$ cm/sec in terms of membrane permeability while having a ClogP/total aa in the range of 1.21 ≤ ClogP/total aa ≤ 1.36 (pd50 to pd58). On the other hand, as described herein, sequences containing MeAbu(pip-4-F2), MeAbu(Mor), Ser(Et-2-Mor), or MeAbu(pip-3-F2) were able to achieve Papp ≥ 1.0 x $10^{-6}$ cm/sec in terms of membrane permeability.

Meanwhile, sequences containing Gln(Ms), an amino acid having an observed pKa of 3.8 and a calculated pKa of 5.3, had difficulty in achieving Papp ≥ 1.0 x $10^{-6}$ cm/sec in terms of membrane permeability while having a ClogP/total aa in the range of 1.20 ≤ ClogP/total aa ≤ 1.33 (pd59 to pd70). On the other hand, as described herein, sequences containing Abu(5-Oxo-Odz) were able to achieve Papp ≥ 1.0 x $10^{-6}$ cm/sec in terms of membrane permeability.

Membrane permeability of cyclic petide compounds containing Ser(Et-2-NMe2) or Gln(Ms)

[Table 25]

| Compound ID | 11 | 10 | 9 | 8 | 7 | 6 | 5 | 4 | 3 | 2 | 1 | C-term | cLogP/ total AA | Caco-2 |
|---|---|---|---|---|---|---|---|---|---|---|---|---|---|---|
| pd50 | D-Val | MePhe | Ser(Et-2-NMe2) | MeLeu | Thr | nPrGly | MeLeu | Ser(tBu) | MePhe | MeVal | Asp | pip | 1.21 | 1.5E-07 |
| pd51 | D-Leu | MePhe | Leu | MeLeu | Thr | nBuGly | MeLeu | Ser(Et-2-NMe2) | MePhe | MeVal | Asp | pip | 1.36 | 7.0E-08 |
| pd52 | D-Val | MePhe | Leu | MeLeu | Thr | nPrGly | MeLeu | Ser(Et-2-NMe2) | MePhe | MeVal | Asp | pip | 1.26 | 1.2E-07 |
| pd53 | D-Val | MePhe | Ser(Et-2-NMe2) | MeLeu | Thr | nBuGly | MeLeu | Ser(tBu) | MePhe | MeVal | Asp | pip | 1.26 | 1.4E-07 |
| pd54 | gMeAbu | MeLeu | Leu | MePhe | MePhe | Ser(Et-2-NMe2) | MeLeu | Thr | MeLeu | MeLeu | Asp | pip | 1.22 | 7.3E-08 |
| pd55 | gMeAbu | MeLeu | Ser(Et-2-NMe2) | MePhe | MePhe | Leu | MeLeu | Thr | MeLeu | MeLeu | Asp | pip | 1.22 | 1.0E-07 |
| pd56 | MeAla | Ser(Et-2-NMe2) | MeLeu | MePhe | Leu | MePhe | MeLeu | Thr | nBuGly | MeVal | Asp | pip | 1.28 | 1.2E-07 |
| pd57 | MeAla | Leu | MeLeu | MePhe | Ser(Et-2-NMe2) | MePhe | MeLeu | Thr | nPrGly | MeVal | Asp | pip | 1.23 | 1.2E-07 |
| pd58 | nBuGly | MePhe | Ser(Et-2-NMe2) | MeLeu | Thr | MeGly | MePhe | MeAbu | Ser (iPen) | MeLeu | Asp | pip | 1.22 | 2.1 E-08 |
| pd59 | D-Leu | MePhe (3-Cl) | Gln(Ms) | MeLeu | Thr | nBuGly | MeLeu | Leu | MePhe | MeVal | Asp | pip | 1.23 | <8.8E-09 |
| pd60 | D-Leu | MePhe (3-Cl) | Leu | MeLeu | Thr | nBuGly | MeLeu | Gln(Ms) | MePhe (3-Cl) | MeVal | Asp | pip | 1.29 | 1.0E-08 |
| pd61 | D-Leu | MePhe (3-Cl) | Leu | MeAbu | Thr | nBuGly | MeLeu | Gln(Ms) | MePhe (3-Cl) | MeVal | Asp | pip | 1.21 | <9.4E-08 |
| pd62 | MeLeu | Gln(Ms) | MeLeu | MePhe | Leu | MePhe | MeLeu | Thr | nBuGly | MeVal | Asp | pip | 1.22 | <1.3E-07 |
| pd63 | MeAbu | Gln(Ms) | MeLeu | MePhe (3-Cl) | Leu | MePhe | MeLeu | Thr | nBuGly | MeVal | Asp | pip | 1.20 | <1.0E-07 |

(continued)

| Compound ID | **11** | **10** | **9** | **8** | **7** | **6** | **5** | **4** | **3** | **2** | **1** | **C-term** | **cLogP/ total AA** | **Caco-2** |
|---|---|---|---|---|---|---|---|---|---|---|---|---|---|---|
| pd64 | MeLeu | Leu | MeAbu | MePhe | Gln(Ms) | MePhe (3-Cl) | MeLeu | Thr | nBuGly | MeLeu | Asp | pip | 1.25 | 1.5E-07 |
| pd65 | nBuGly | MePhe | Gln(Ms) | MeLeu | Thr | nBuGly | MePhe | MeLeu | Leu | MeLeu | Asp | pip | 1.24 | 6.0E-08 |
| pd66 | nBuGly | MePhe (3-Cl) | Gln(Ms) | MeLeu | Thr | nBuGly | MePhe | MeLeu | Leu | MeLeu | Asp | pip | 1.30 | 9.0E-08 |
| pd67 | D-Val | MePhe | Gln(Ms) | MeLeu | Leu | nBuGly | MeLeu | Ser(tBu) | MePhe | MeVal | Asp | pip | 1.26 | 6.0E-08 |
| pd68 | gMeAbu | MeLeu | Leu | MePhe | MePhe | Gln(Ms) | MeLeu | Leu | MeLeu | MeLeu | Asp | pip | 1.22 | 1.0E-07 |
| pd69 | MeAbu | Leu | MeLeu | MePhe | Gln(Ms) | MePhe | MeLeu | Leu | nBuGly | MeVal | Asp | pip | 1.33 | <1.4E-07 |
| pd70 | nBuGly | MePhe | Gln(Ms) | MeLeu | Leu | MeGly | MePhe | MeLeu | Leu | MeLeu | Asp | pip | 1.28 | 1.7E-07 |

Structural data of a part of cyclic peptides evaluated for membrane permeability

[Table 26]

| ID | structure |
|---|---|
| pd50 | 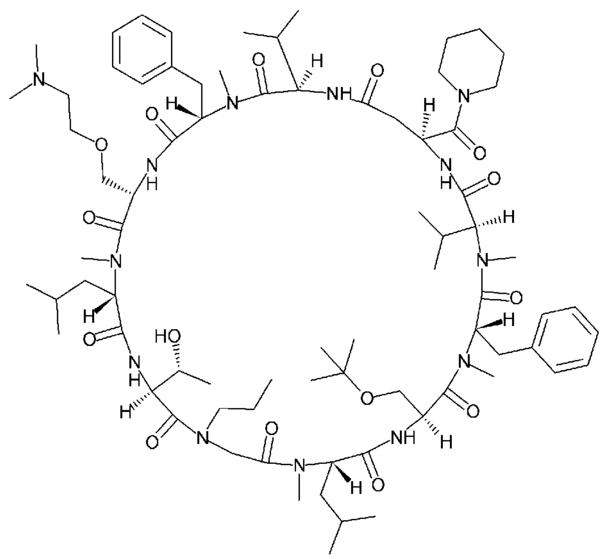 |
| pd51 | 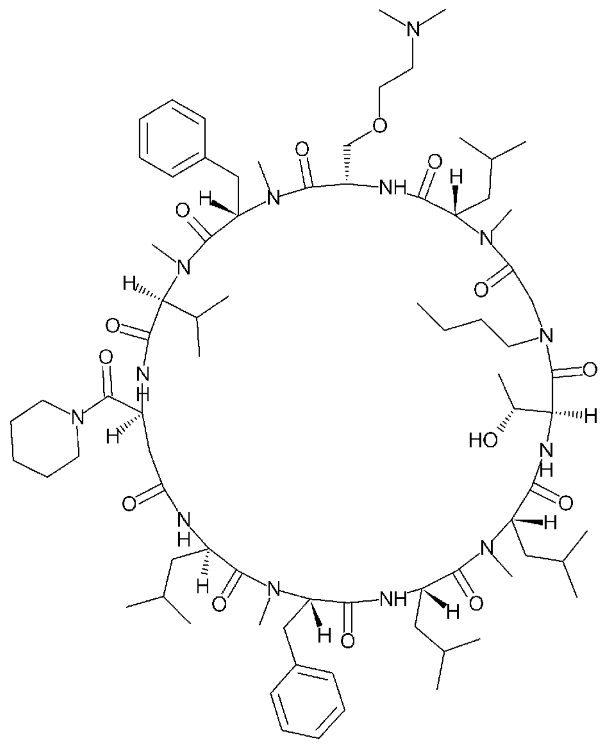 |

| ID | structure |
|---|---|
| pd52 | 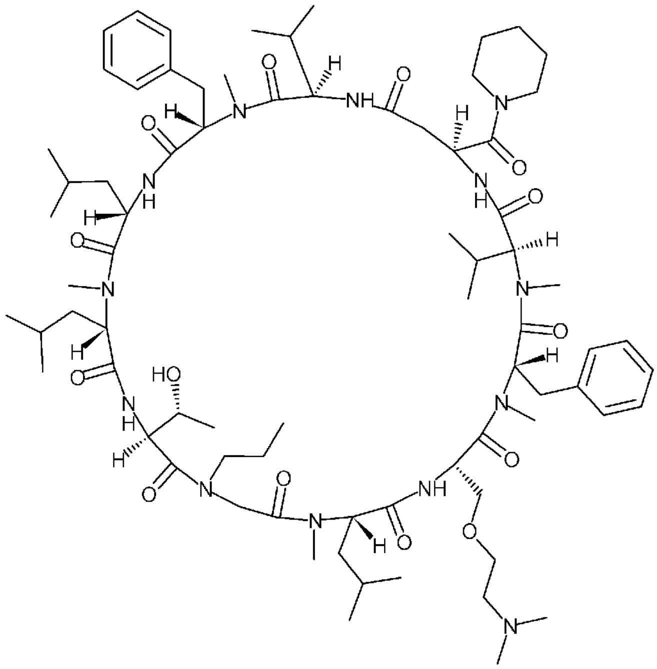 |
| pd53 | 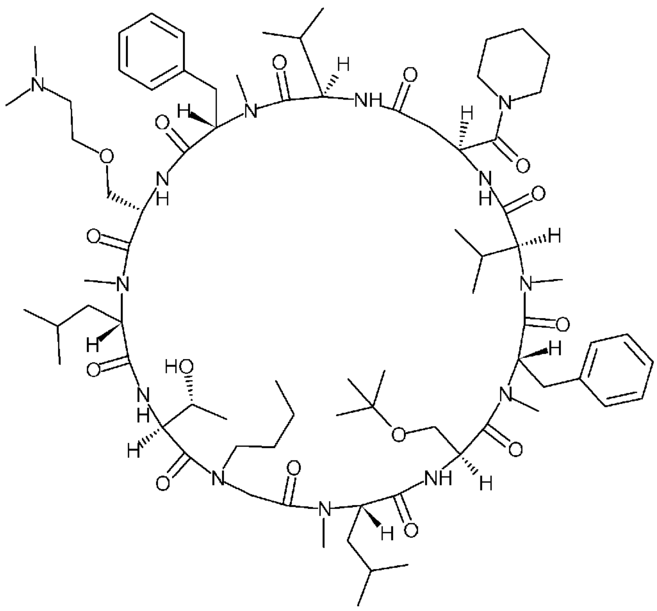 |

| ID | structure |
|---|---|
| pd54 | 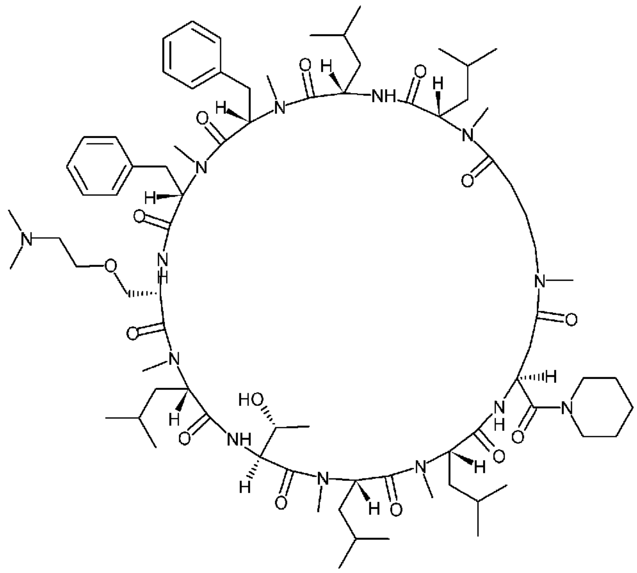 |
| pd55 | 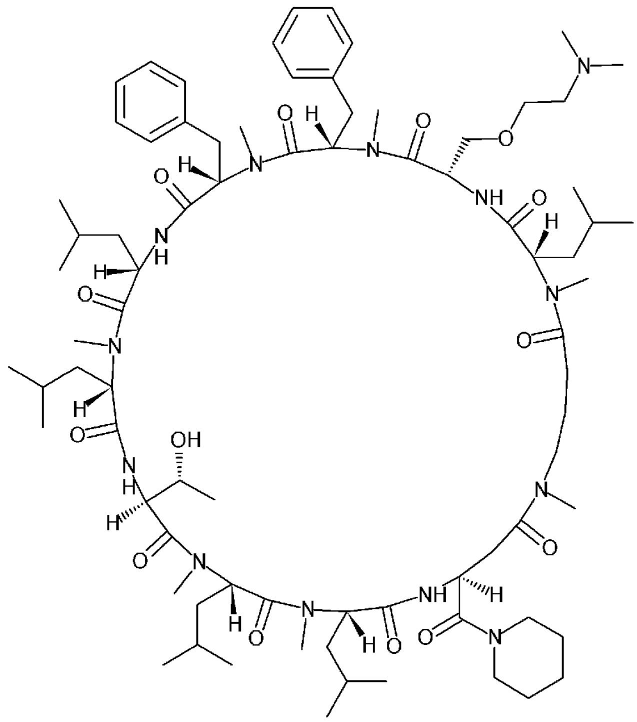 |

| ID | structure |
|---|---|
| pd56 | 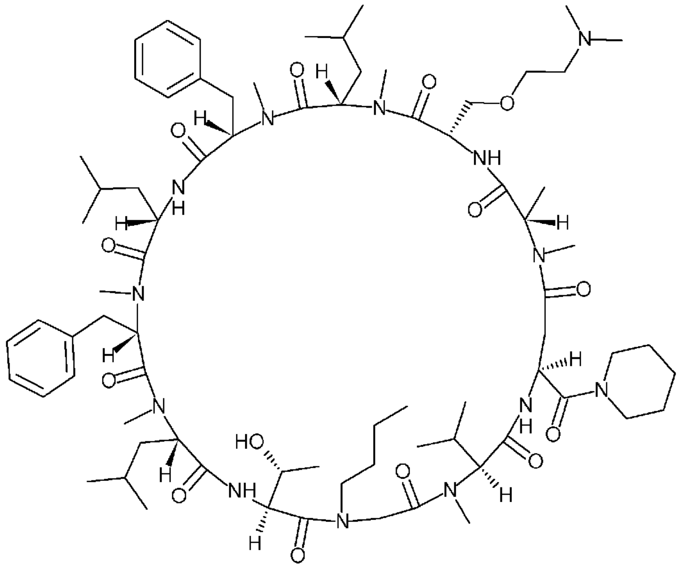 |
| pd57 | 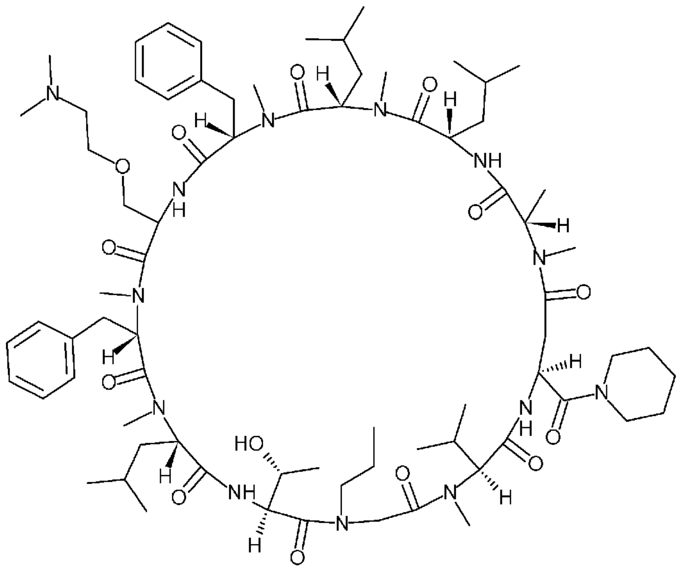 |

| ID | structure |
|---|---|
| pd58 | 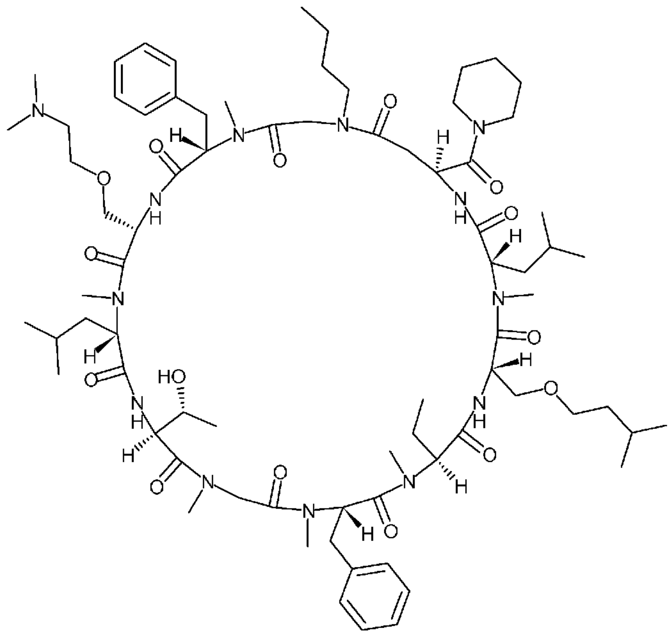 |
| pd59 | 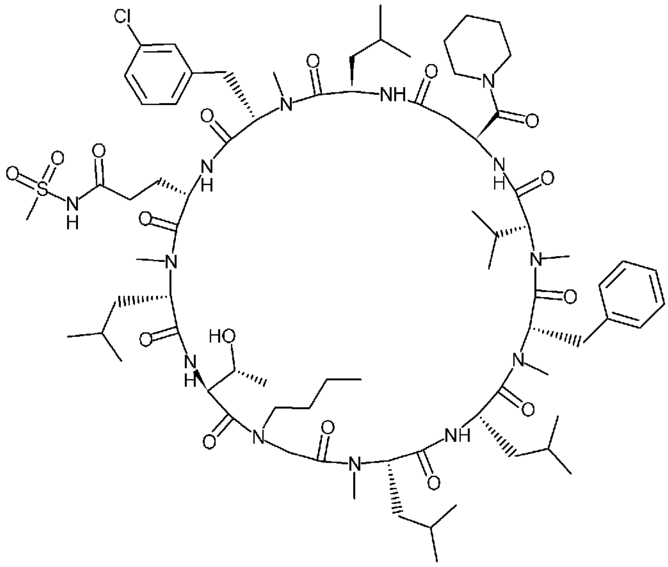 |

| ID | structure |
|---|---|
| pd60 | 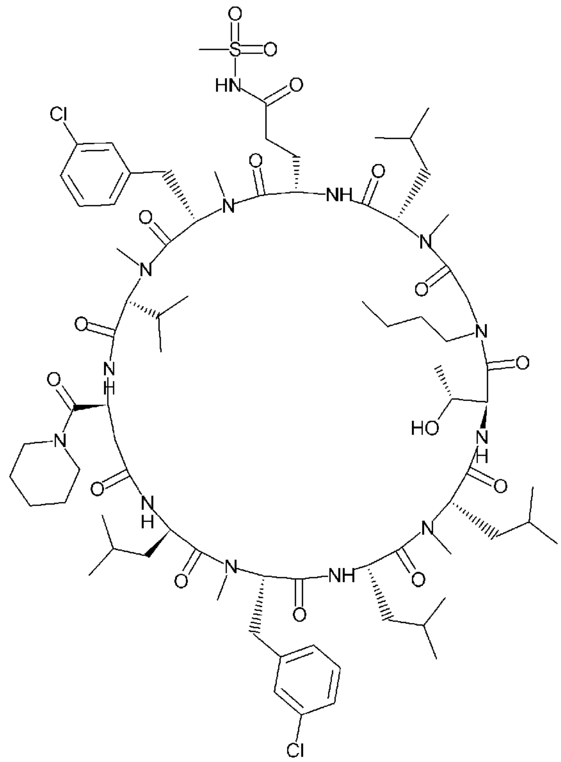 |
| pd61 | 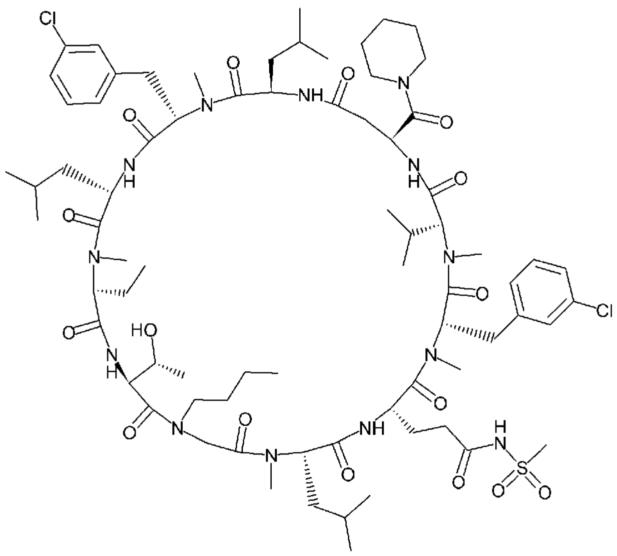 |

| ID | structure |
|---|---|
| pd62 | 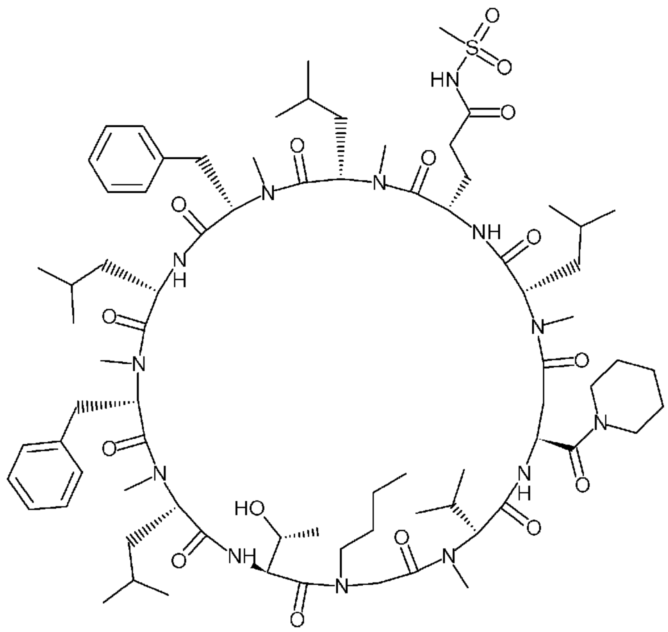 |
| pd63 | 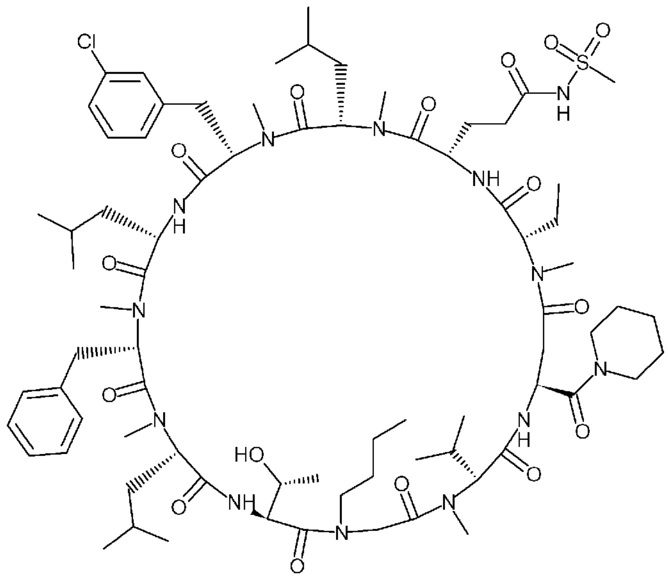 |

| ID | structure |
|---|---|
| pd64 | 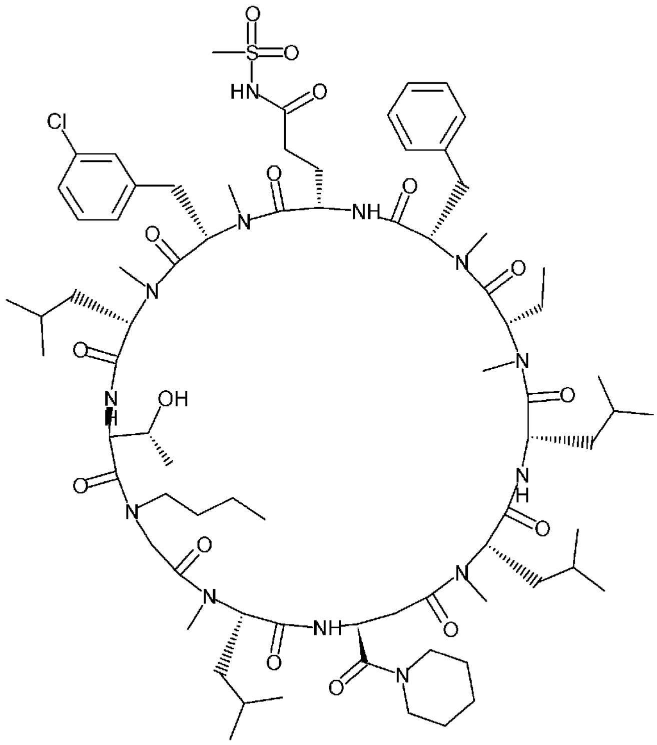 |
| pd65 | 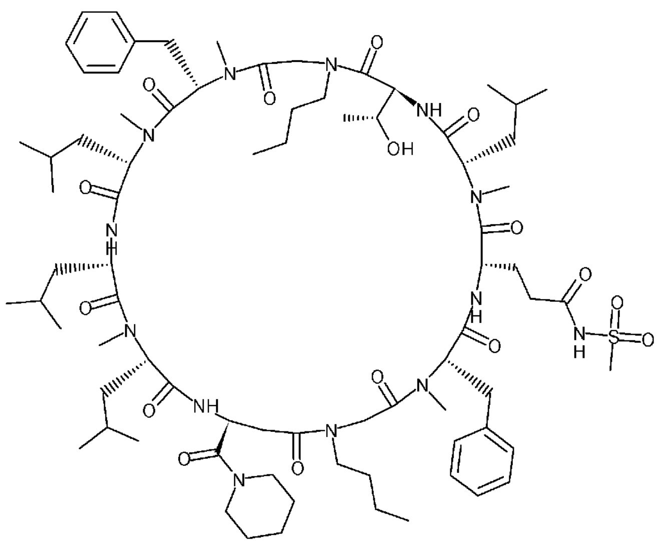 |

| ID | structure |
| --- | --- |
| pd66 | 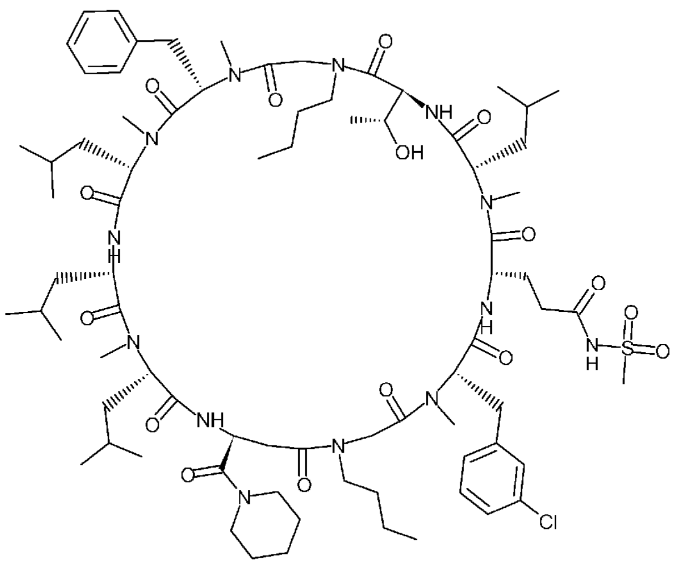 |
| pd67 | 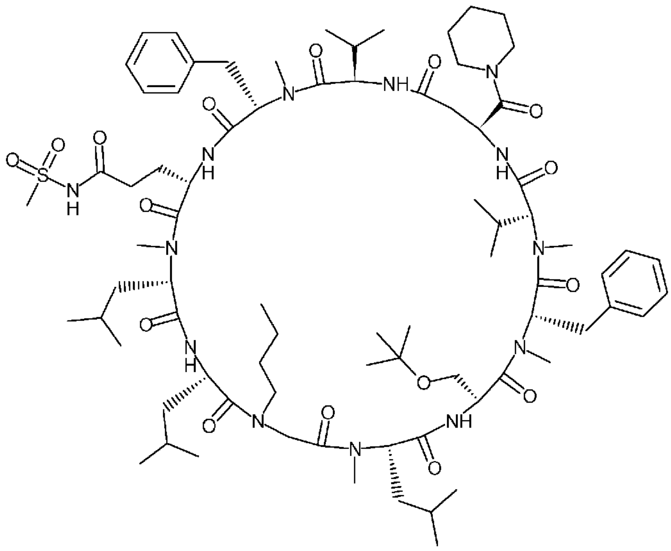 |

| ID | structure |
|---|---|
| pd68 | 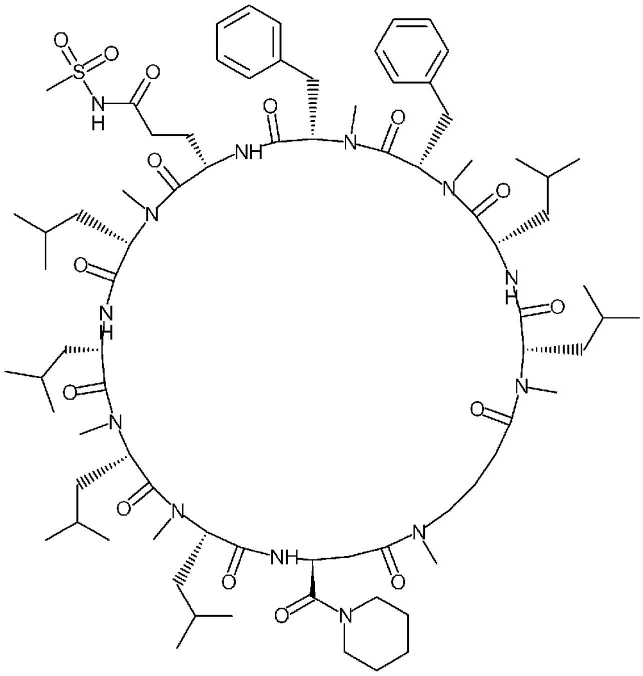 |
| pd69 | 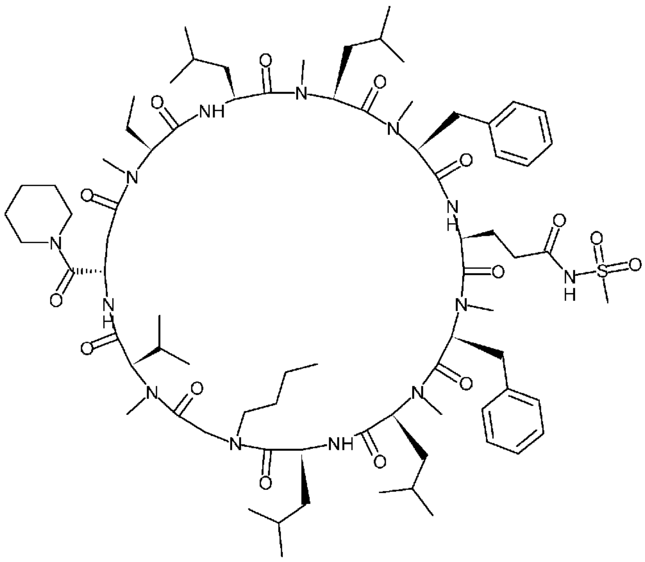 |

| ID | structure |
|---|---|
| pd70 | 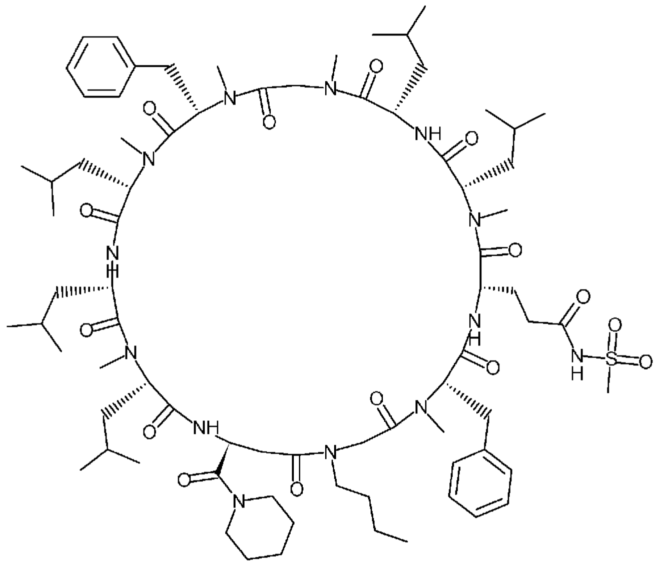 |

| ID | structure |
|---|---|
| pd100 | 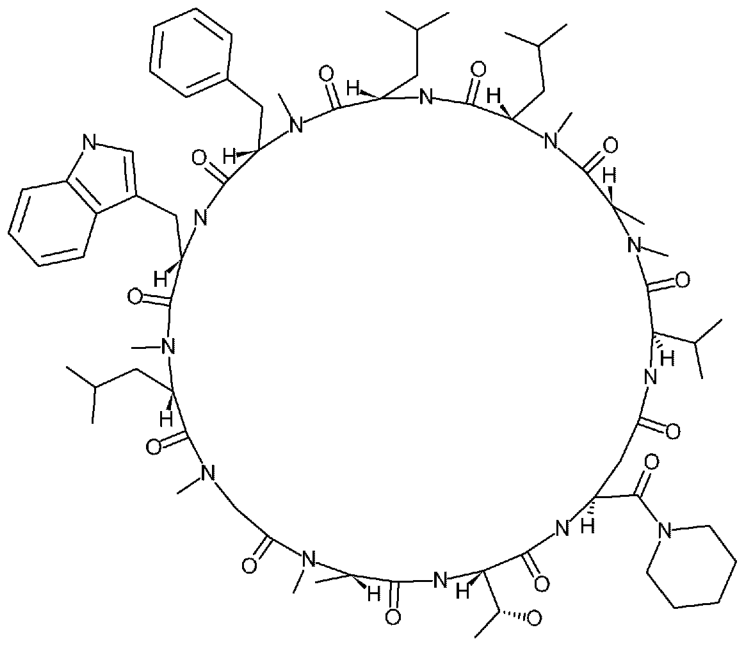 |
| pd101 | 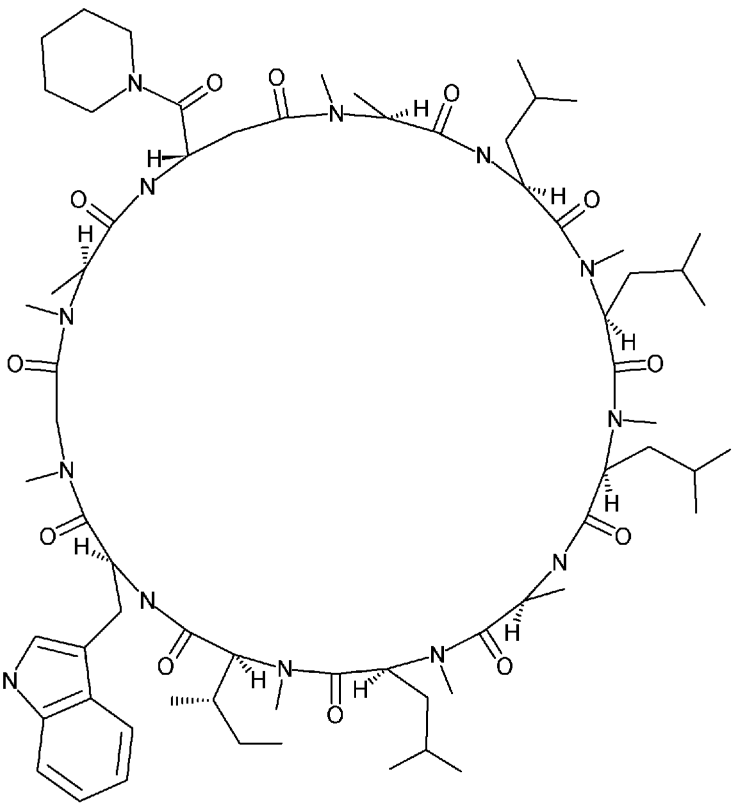 |

| ID | structure |
|---|---|
| pd102 | 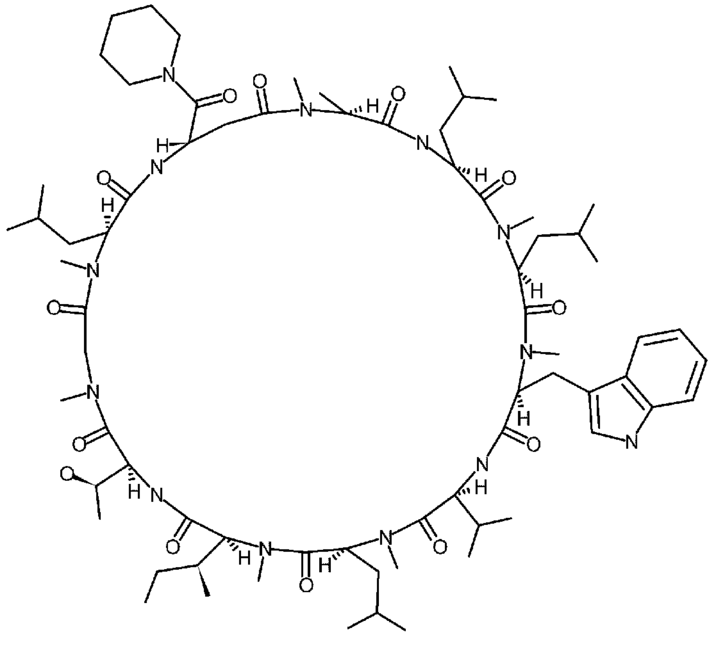 |
| pd103 | 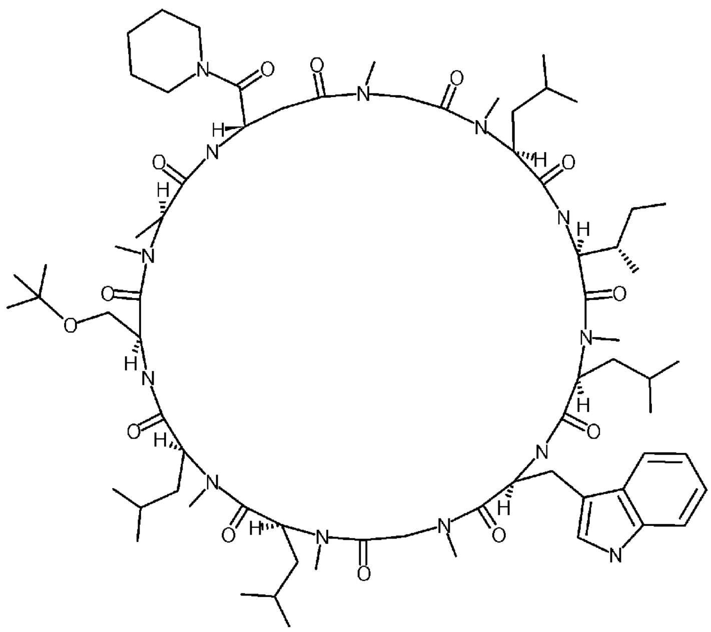 |

| ID | structure |
|---|---|
| pd104 | 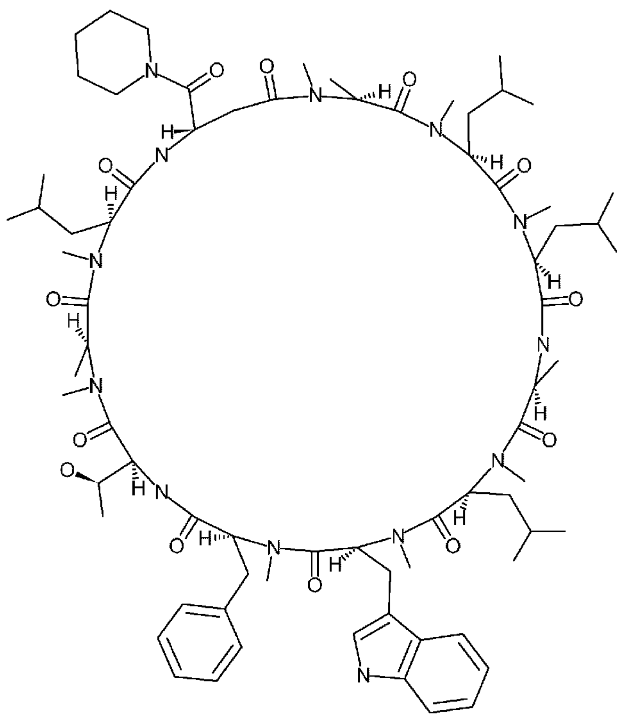 |
| pd105 | 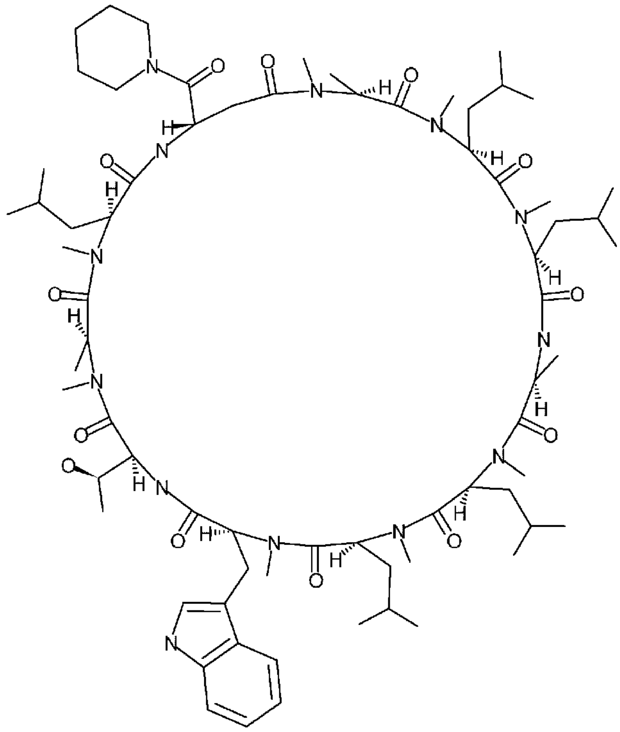 |

| ID | structure |
|---|---|
| pd106 | 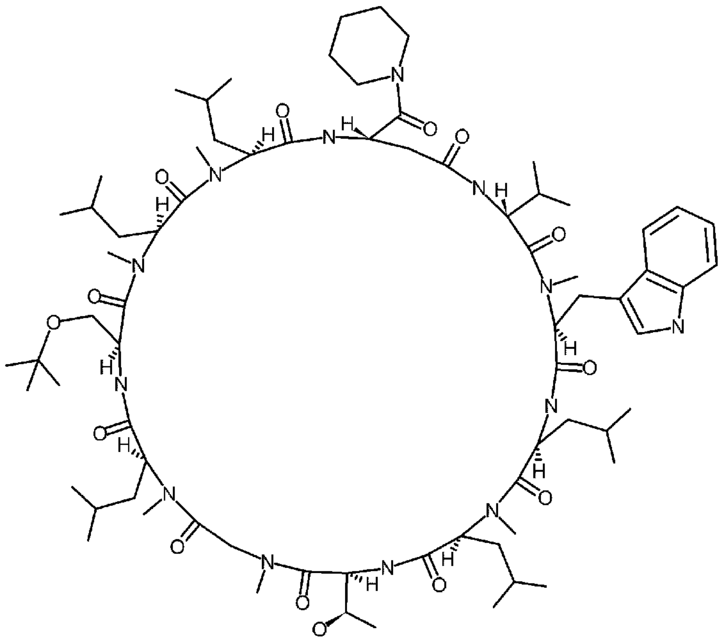 |
| pd107 | 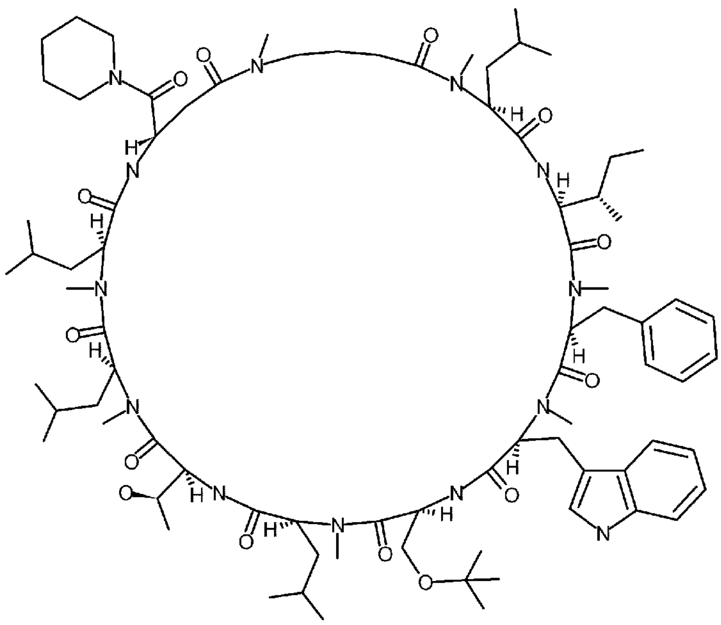 |

| ID | structure |
| --- | --- |
| pd108 | 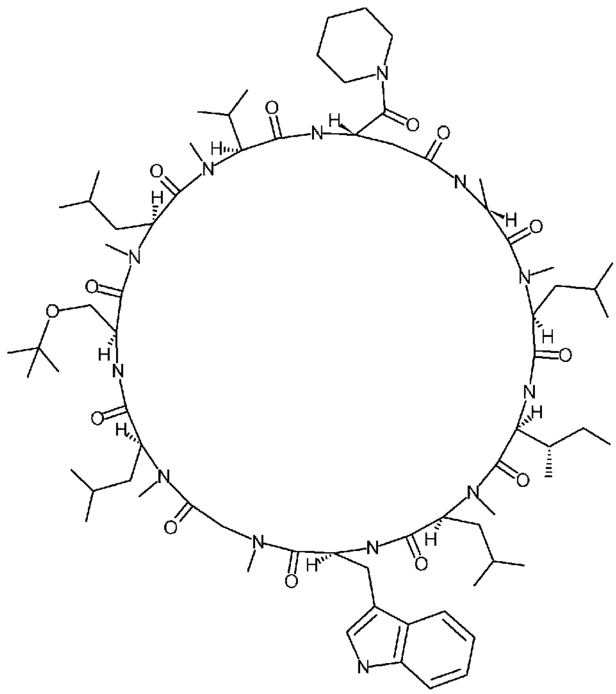 |
| pd109 | 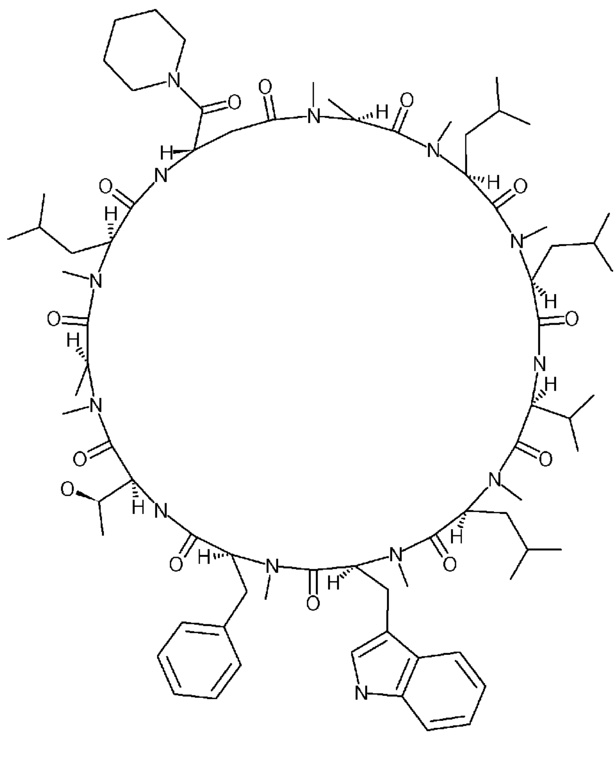 |

| ID | structure |
|---|---|
| pd110 | 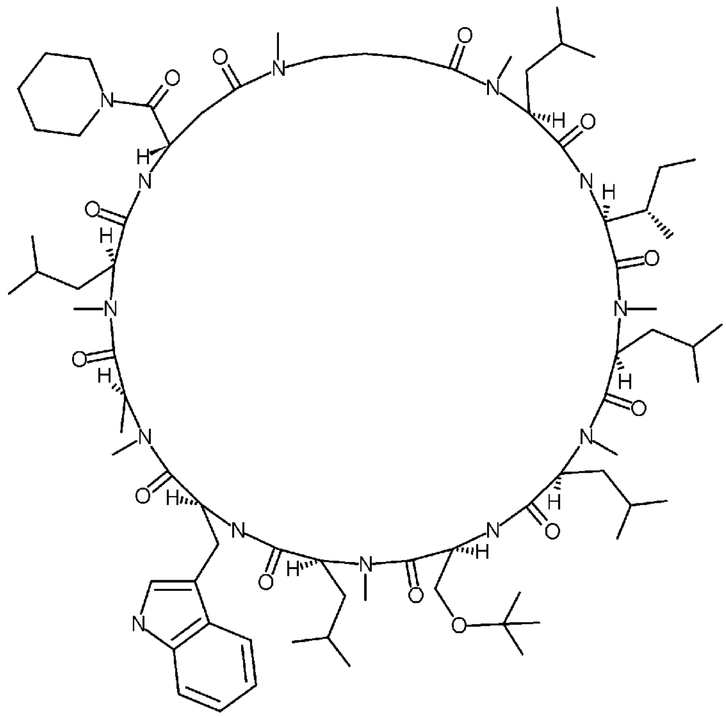 |
| pd111 | 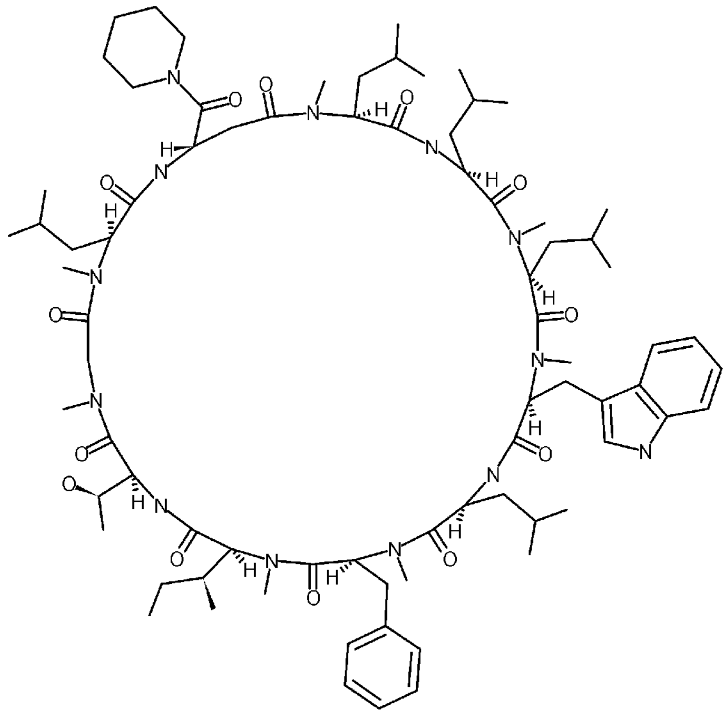 |

| ID | structure |
|---|---|
| pd112 | 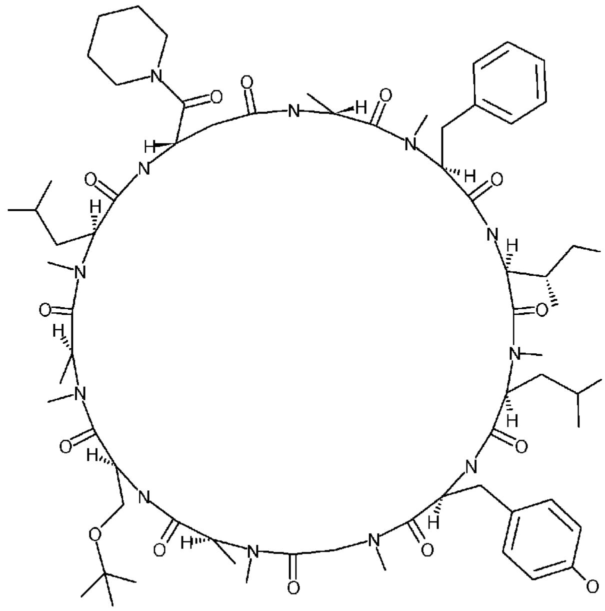 |
| pd113 | 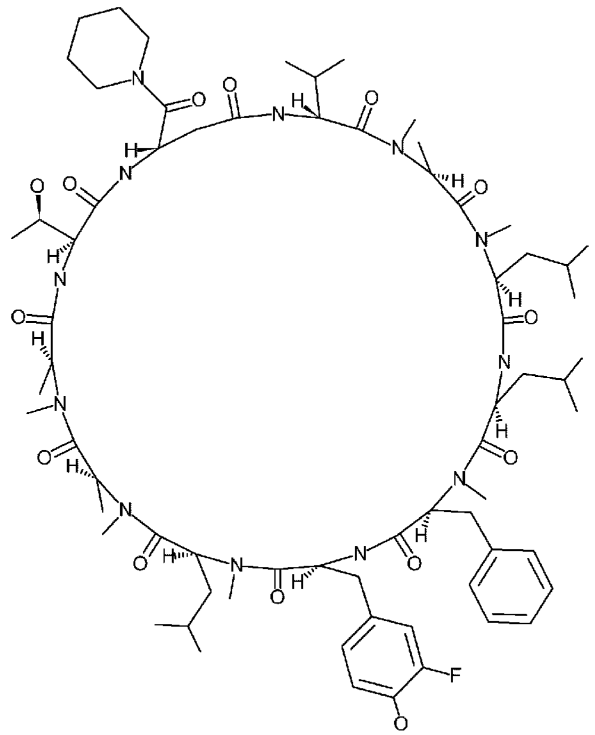 |

| ID | structure |
|---|---|
| pd114 | 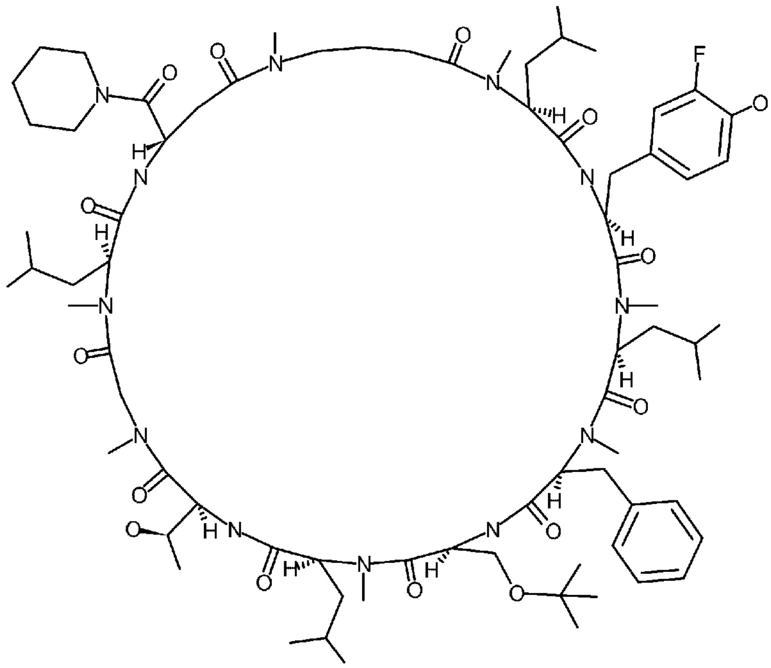 |
| pd115 | 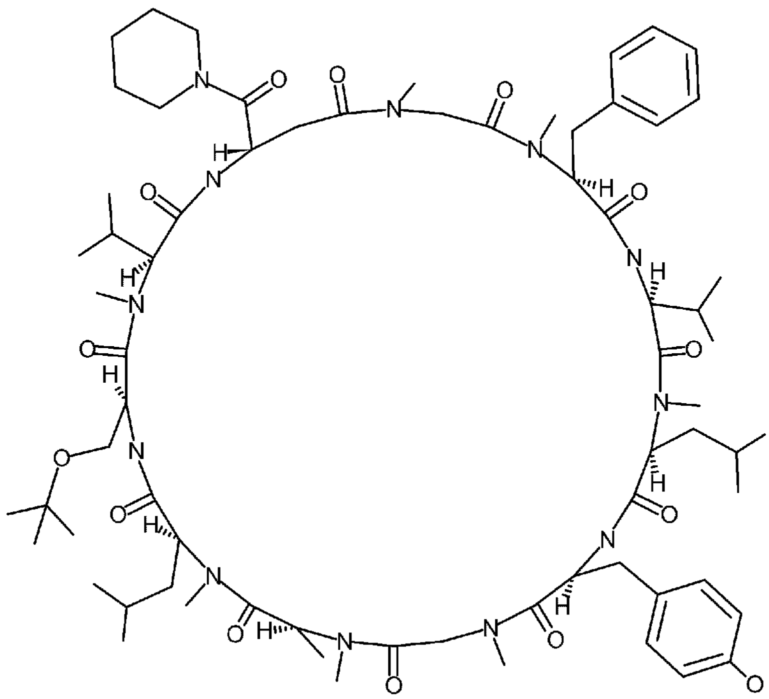 |

| ID | structure |
|---|---|
| pd116 | 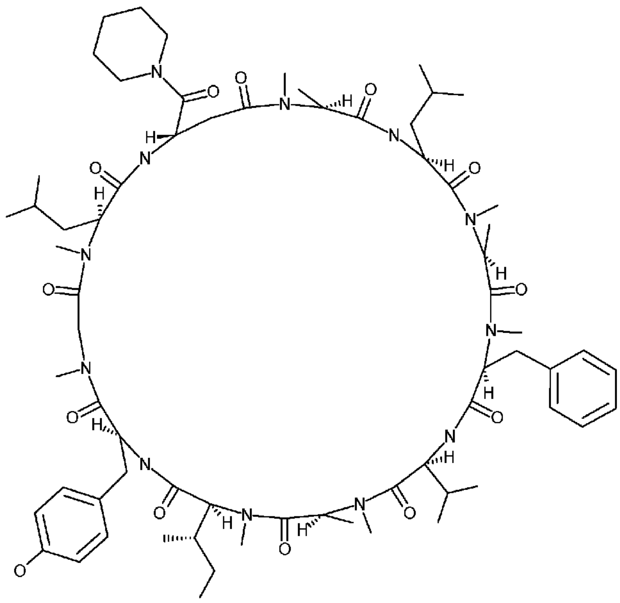 |
| pd117 | 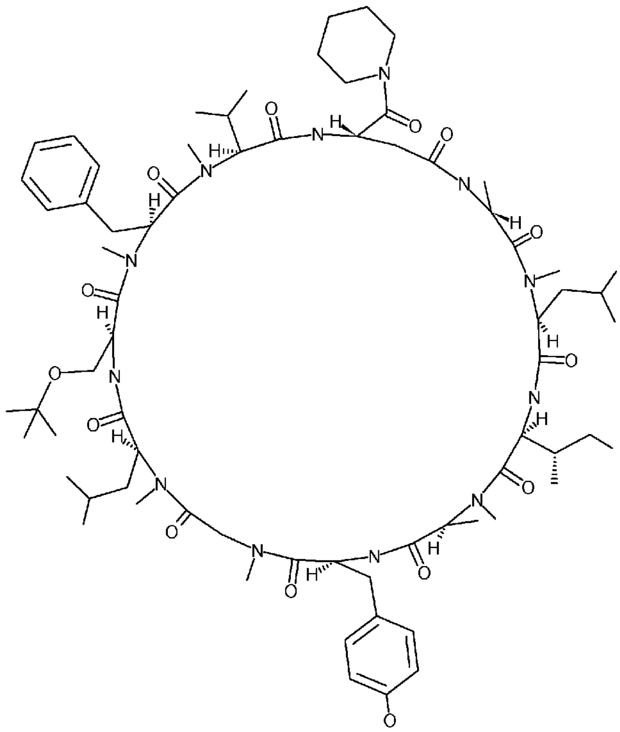 |

| ID | structure |
|---|---|
| pd118 | 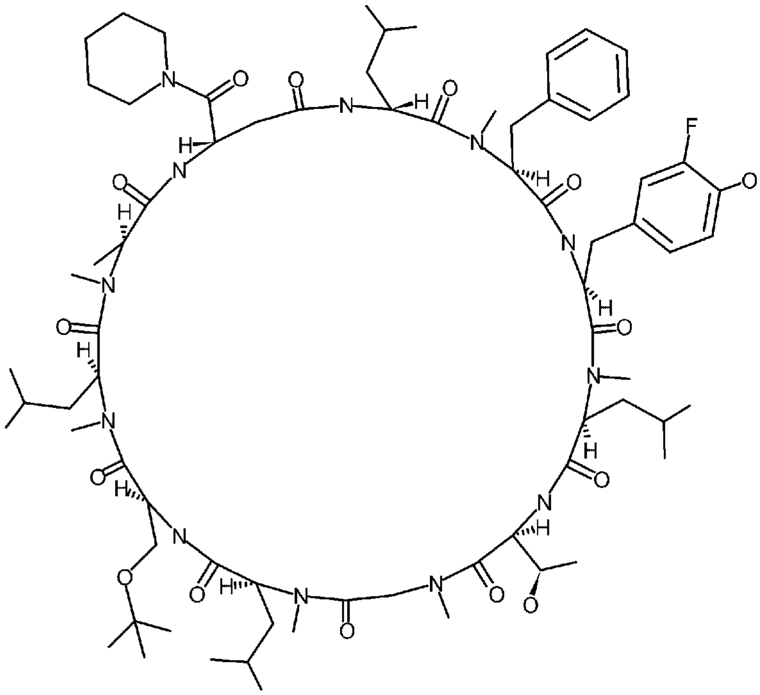 |
| pd119 | 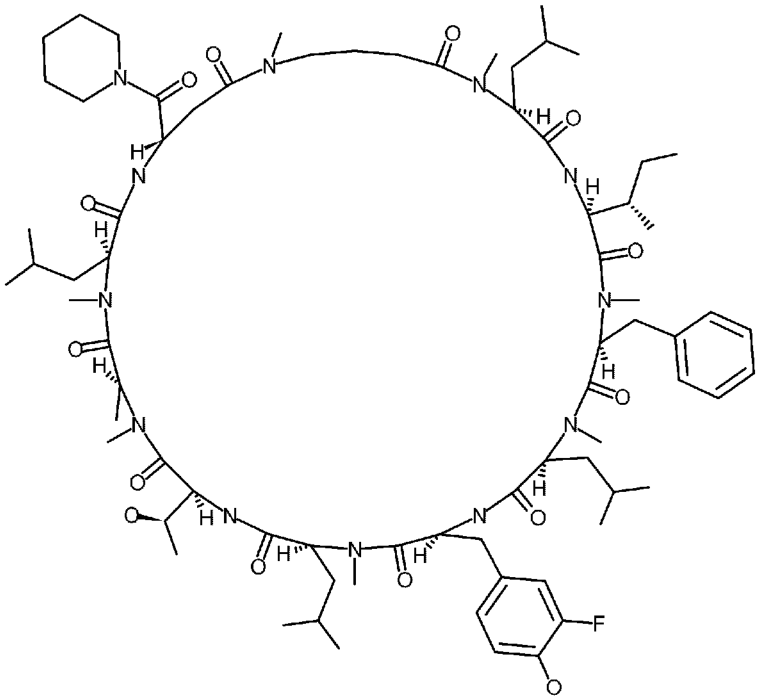 |

| ID | structure |
|---|---|
| pd120 | 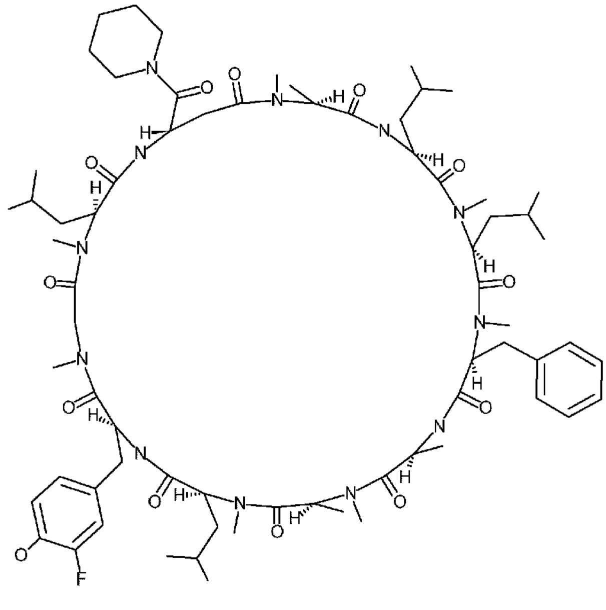 |
| pd121 | 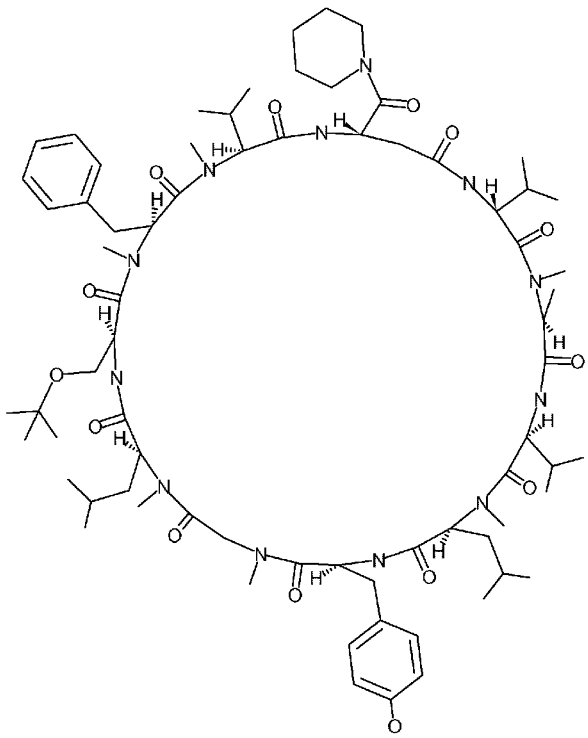 |

| ID | structure |
|---|---|
| pd122 | 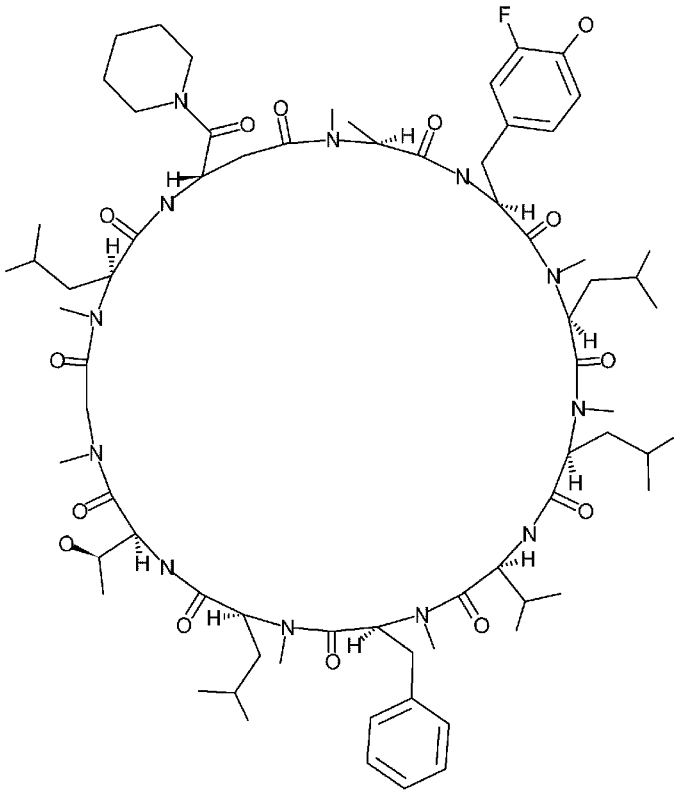 |
| pd123 | 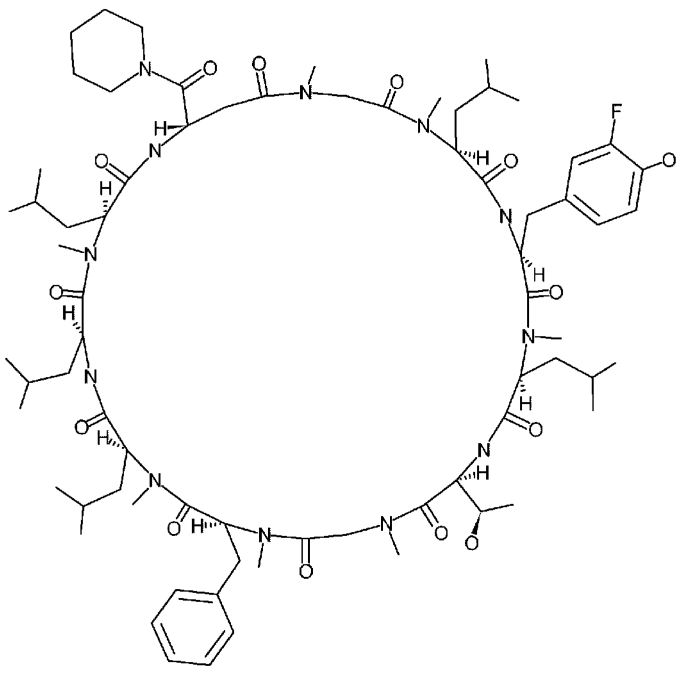 |

| ID | structure |
|---|---|
| pd124 | 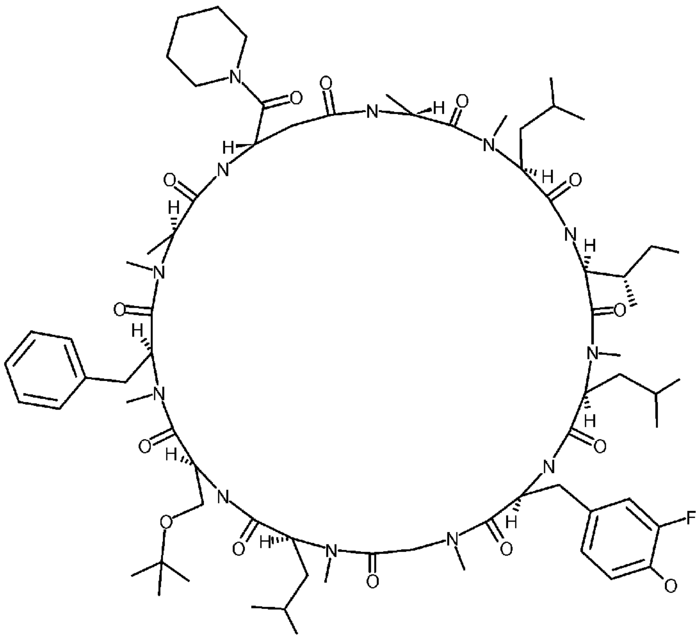 |
| pd125 | 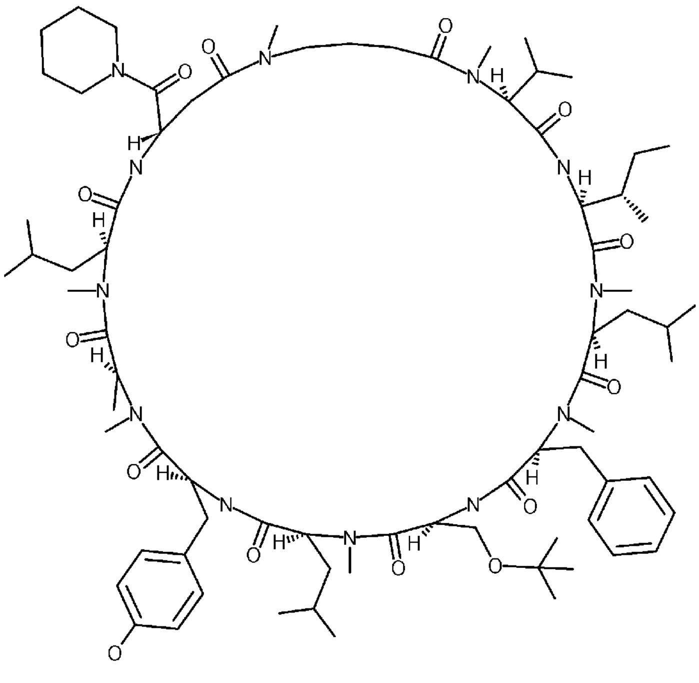 |

| ID | structure |
|---|---|
| pd126 | 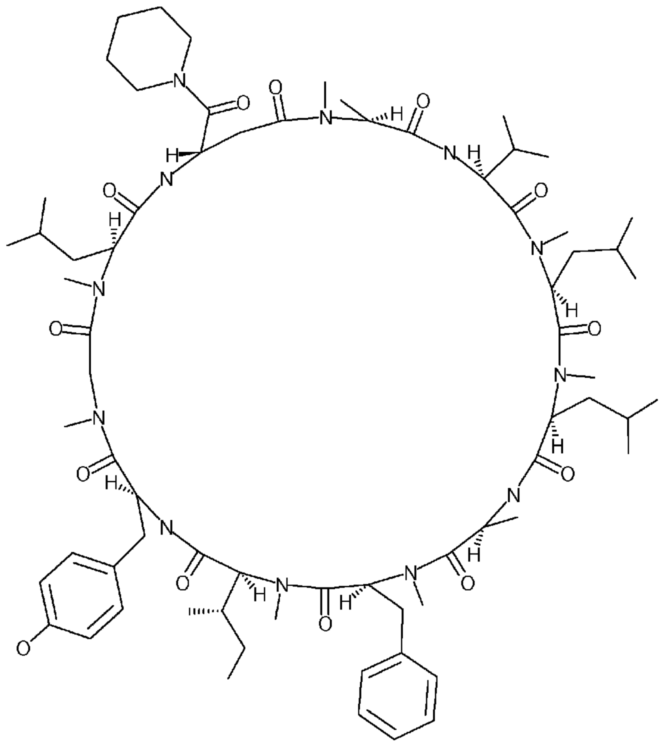 |
| pd127 | 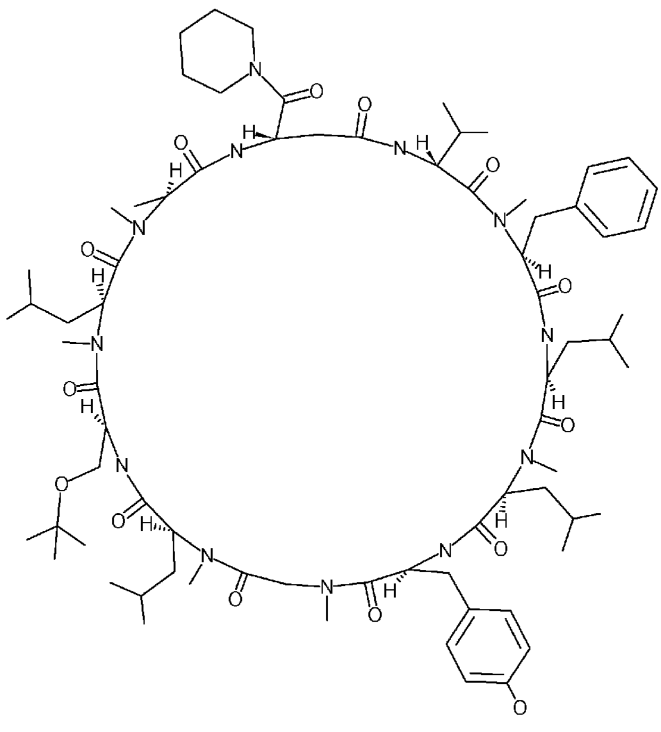 |

| ID | structure |
|---|---|
| pd128 | 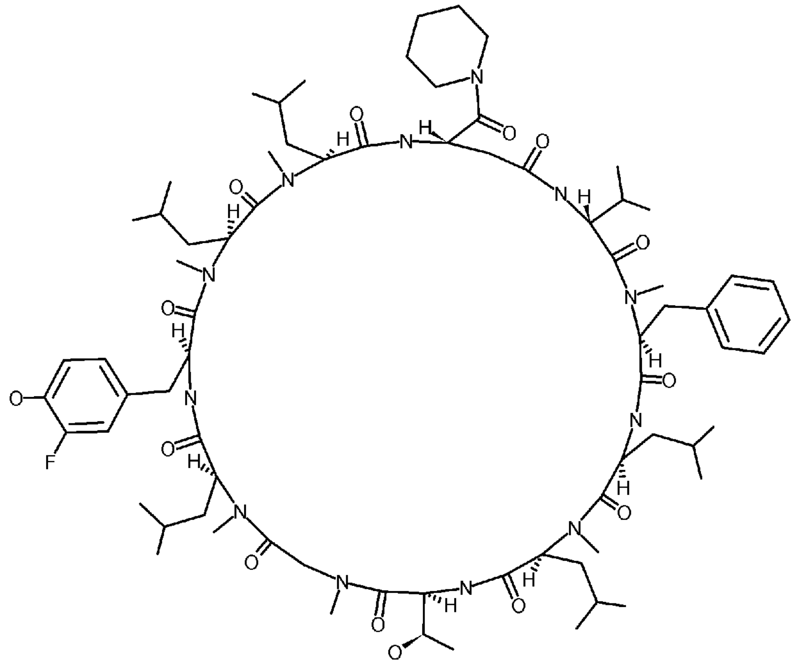 |
| pd129 | 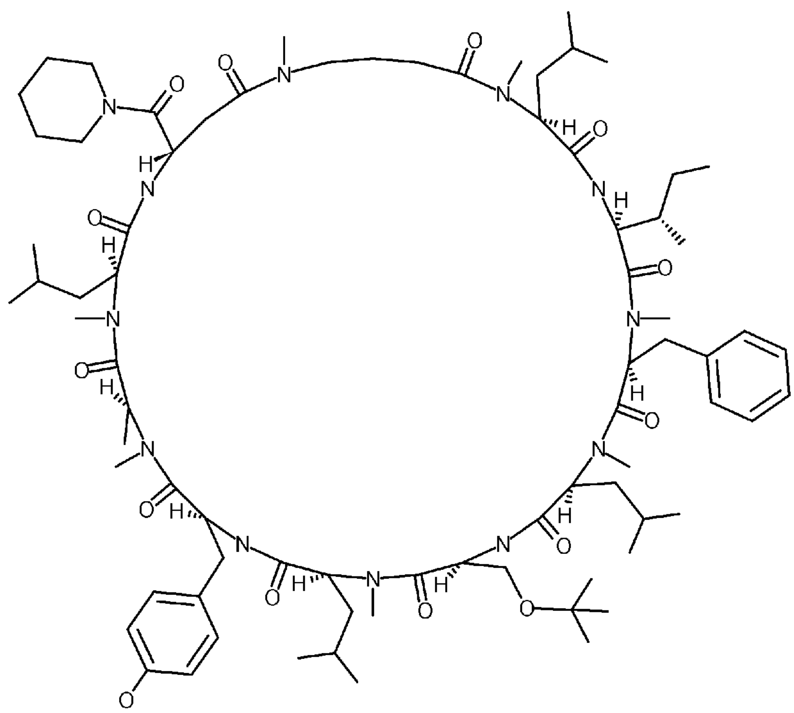 |

| ID | structure |
|---|---|
| pd130 | 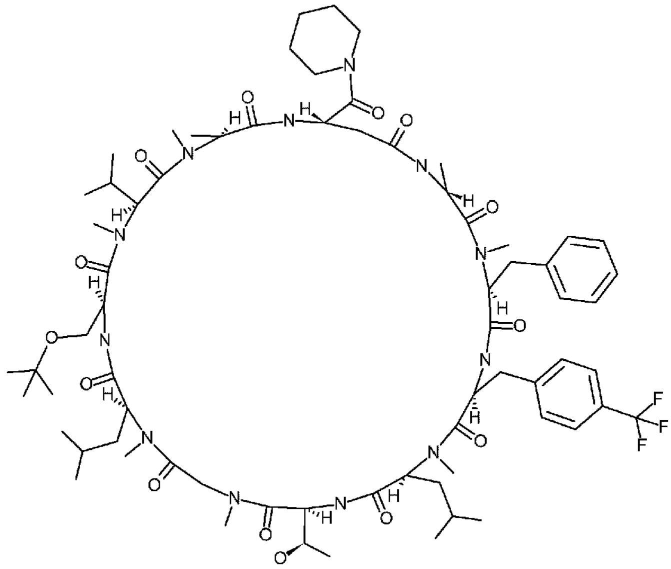 |
| pd131 | 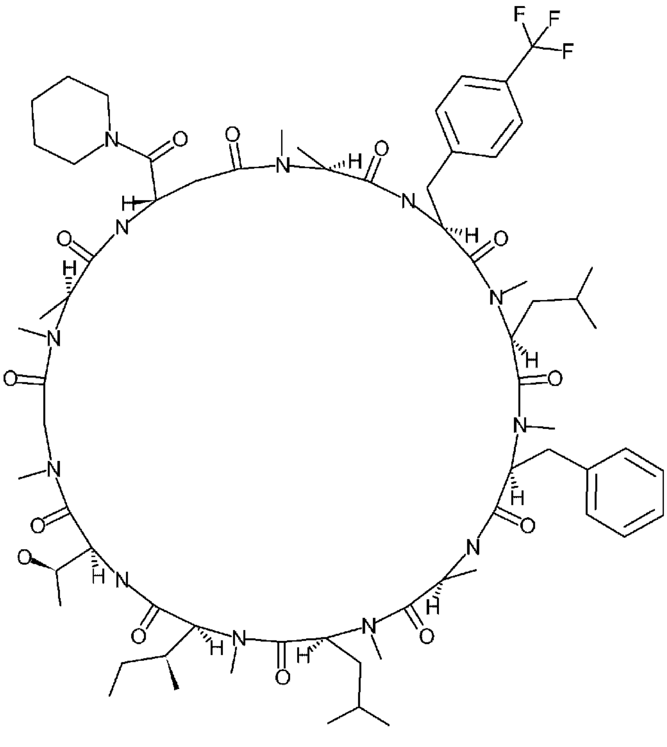 |

| ID | structure |
|---|---|
| pd132 | 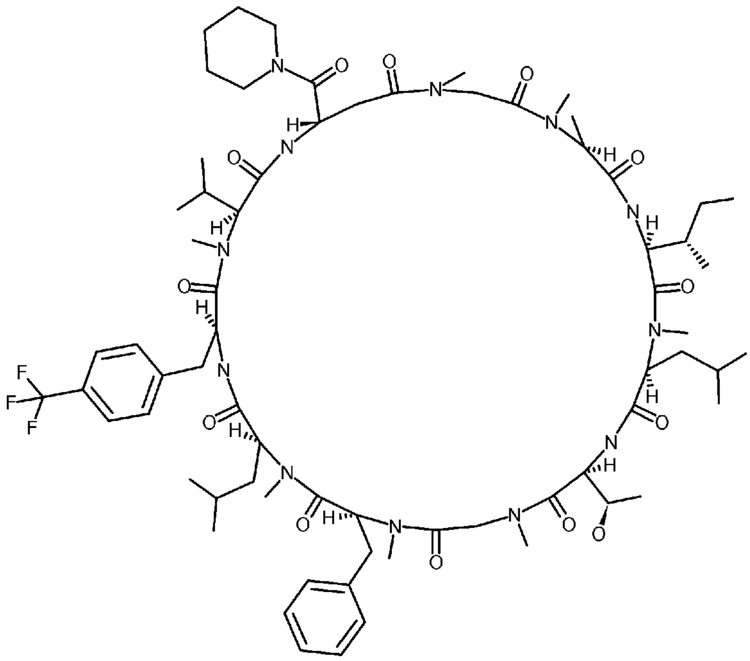 |
| pd133 | 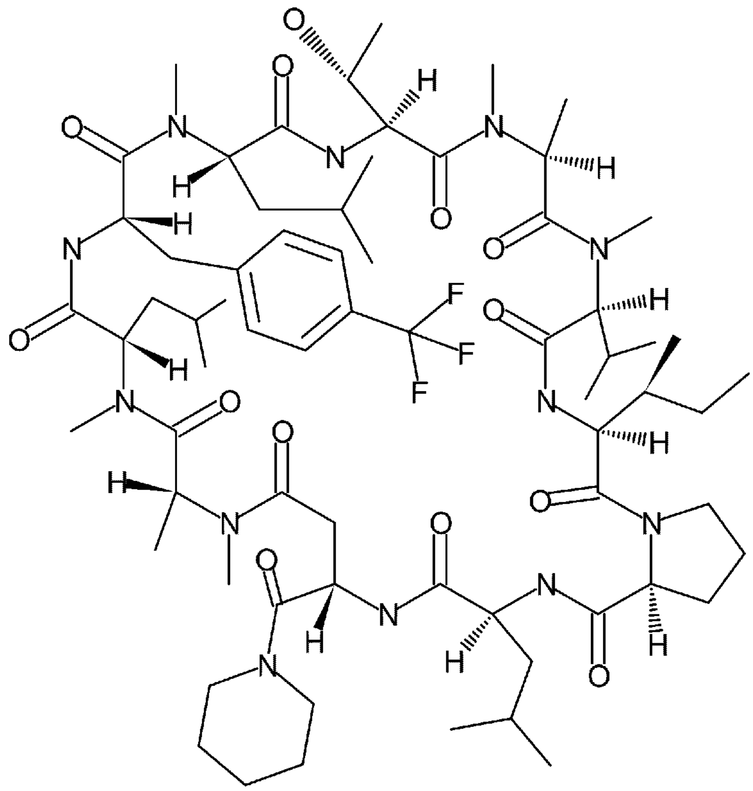 |

| ID | structure |
|---|---|
| pd134 | 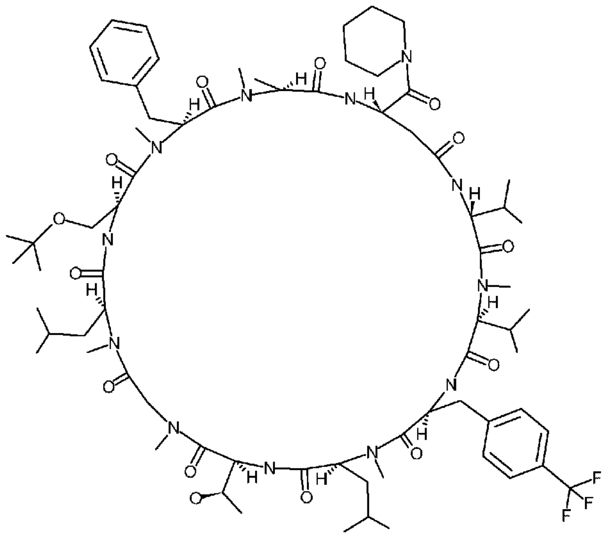 |
| pd135 | 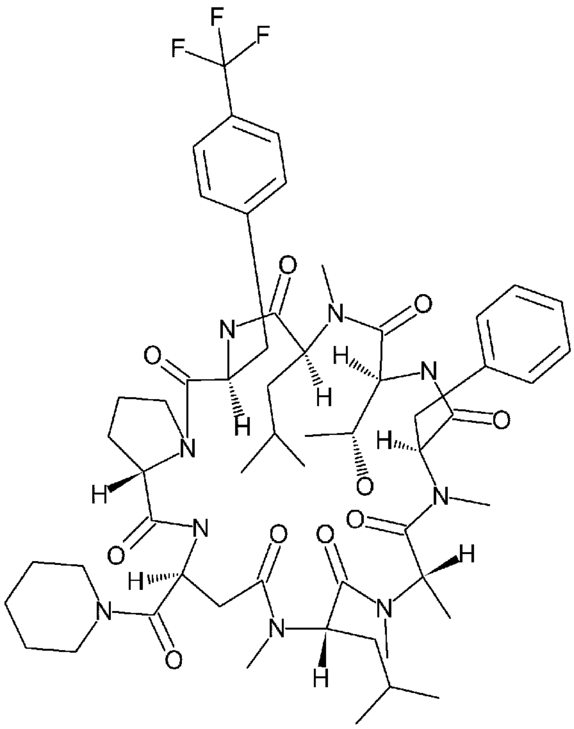 |

| ID | structure |
|---|---|
| pd136 | 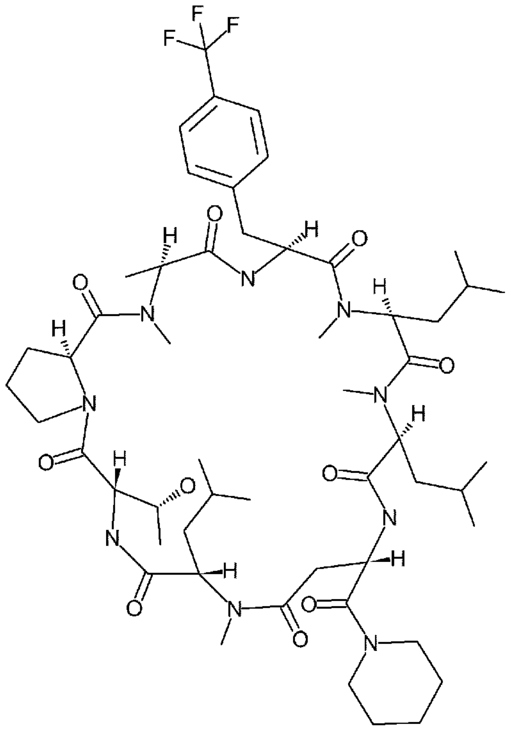 |
| pd137 | 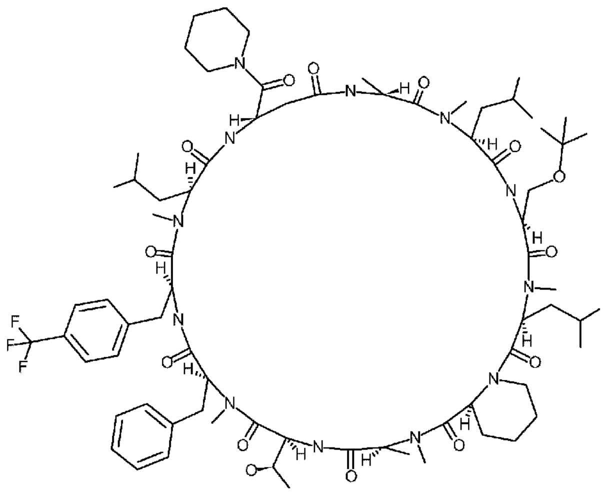 |

| ID | structure |
|---|---|
| pd138 | 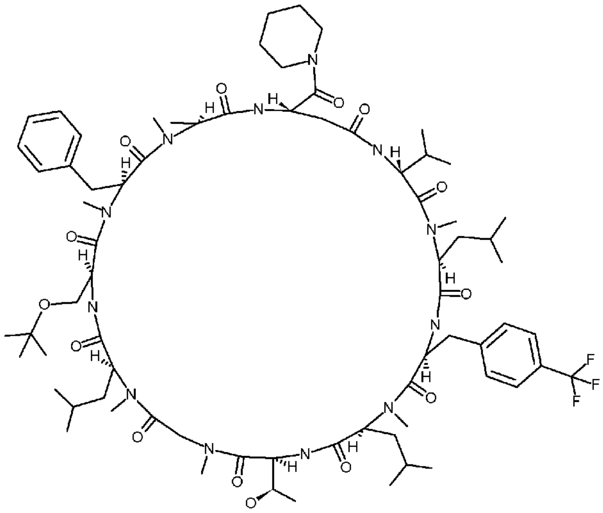 |
| pd139 | 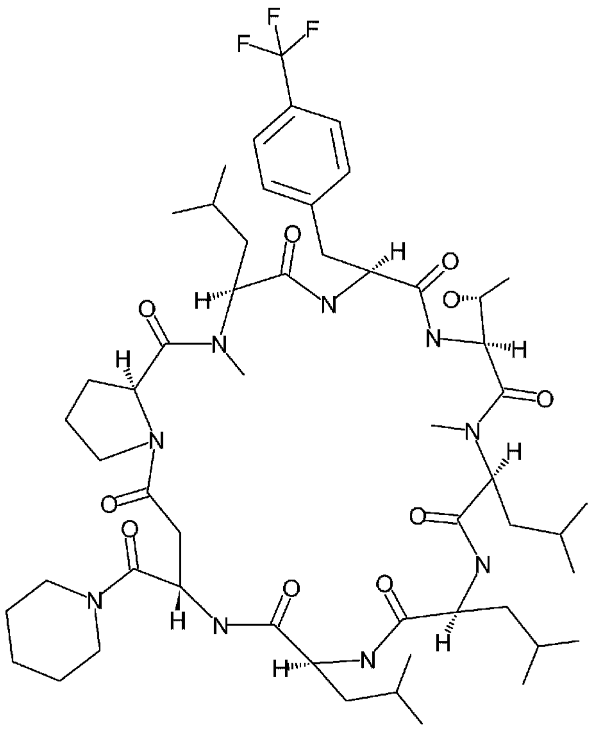 |

| ID | structure |
| --- | --- |
| pd140 | 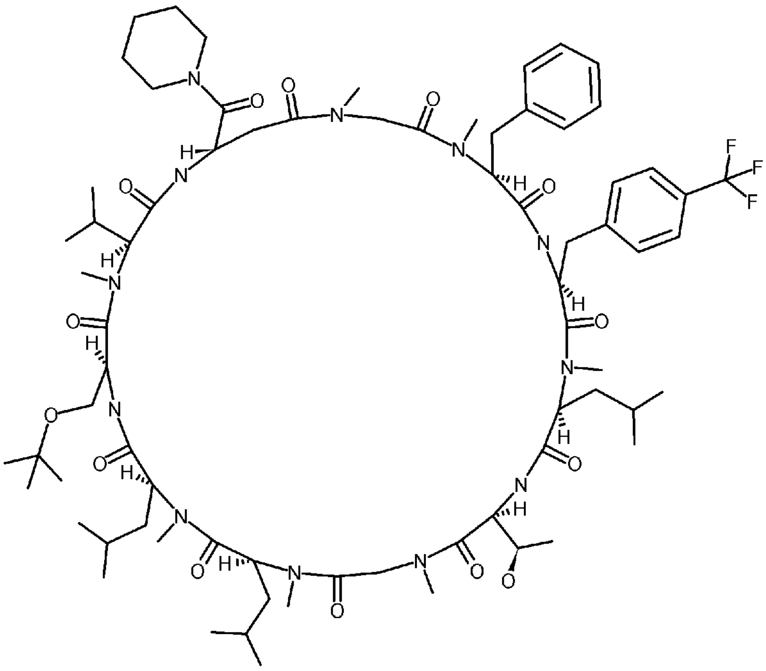 |
| pd141 | 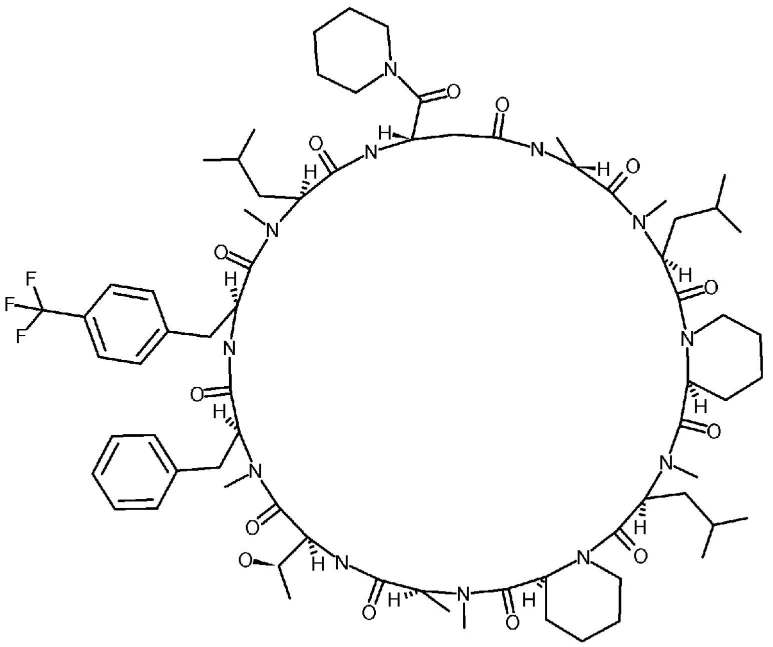 |

| ID | structure |
|---|---|
| pd142 | 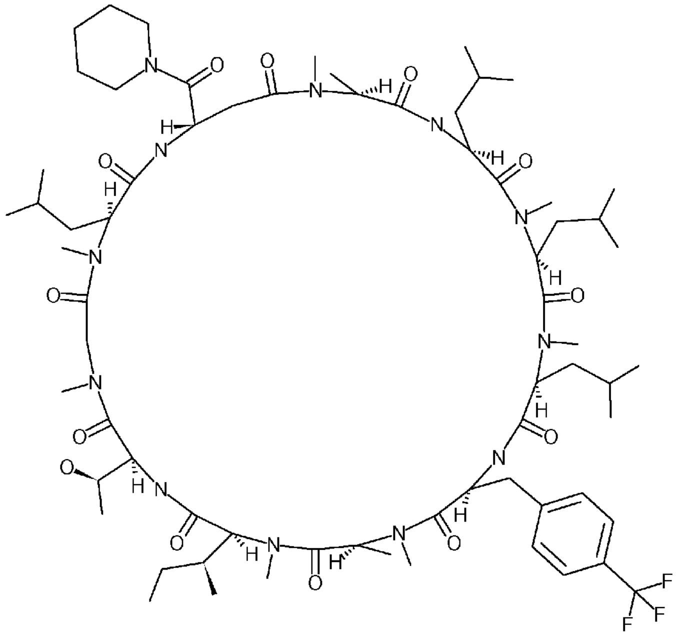 |
| pd143 | 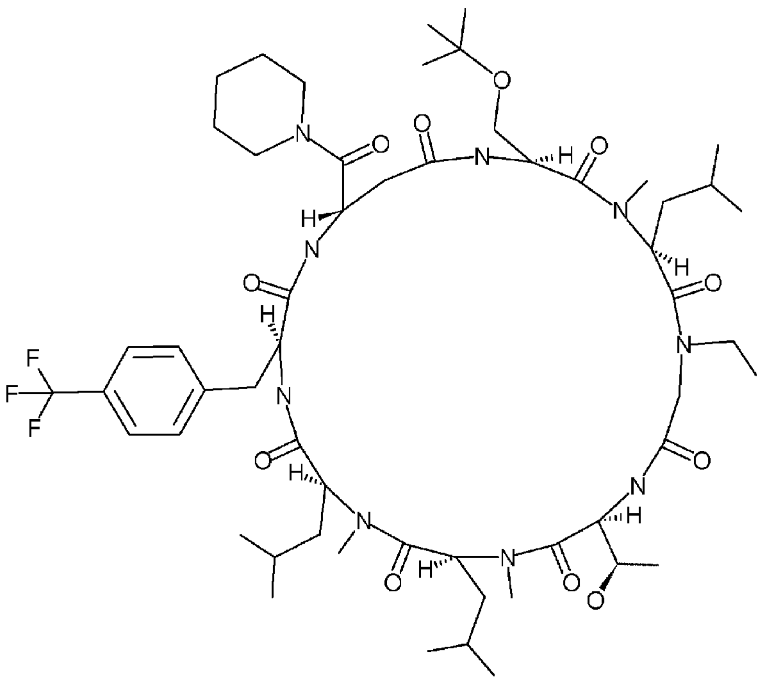 |

| ID | structure |
|---|---|
| pd144 | 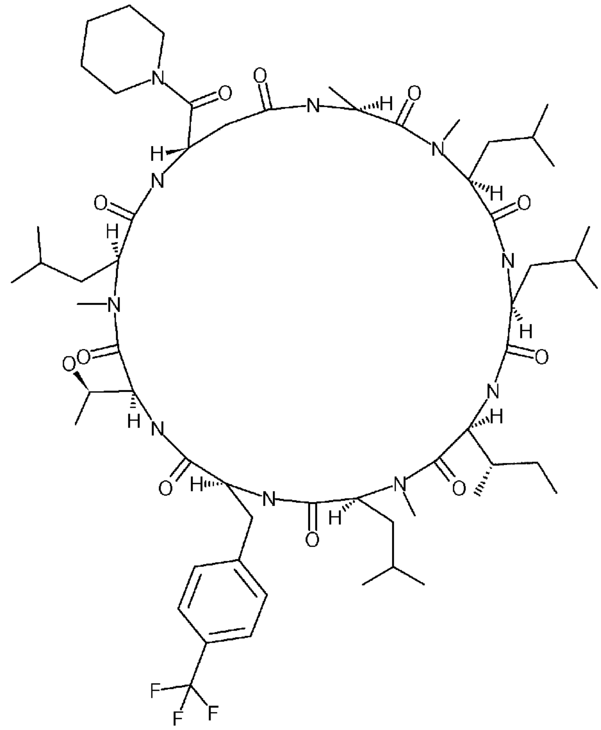 |
| pd145 | 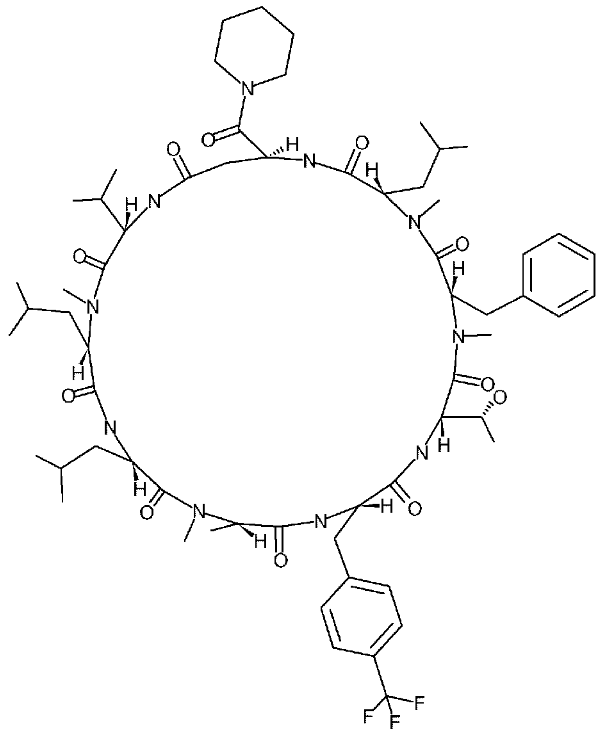 |

| ID | structure |
|---|---|
| pd146 | 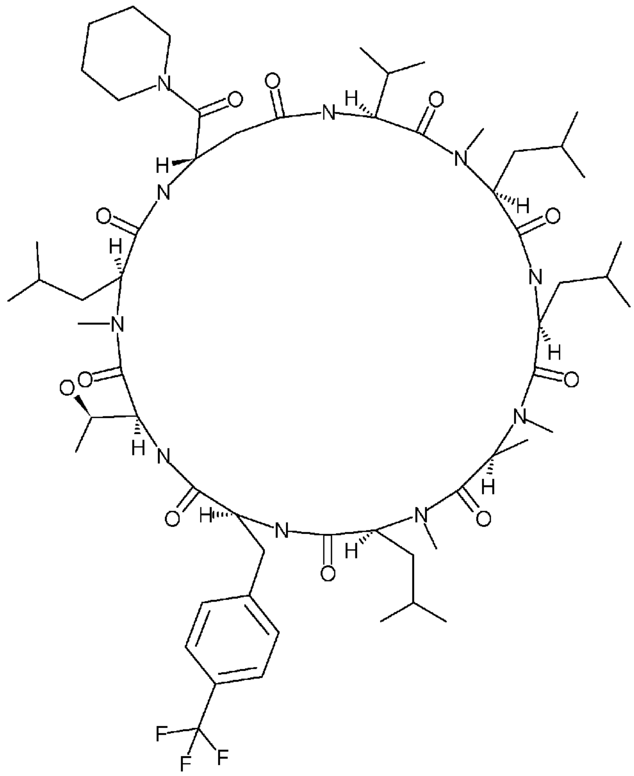 |
| pd147 | 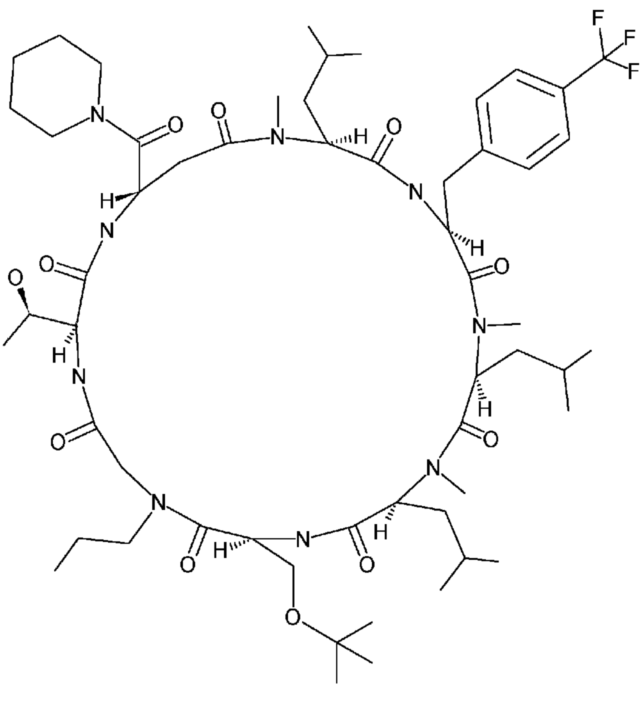 |

| ID | structure |
|---|---|
| pd148 | 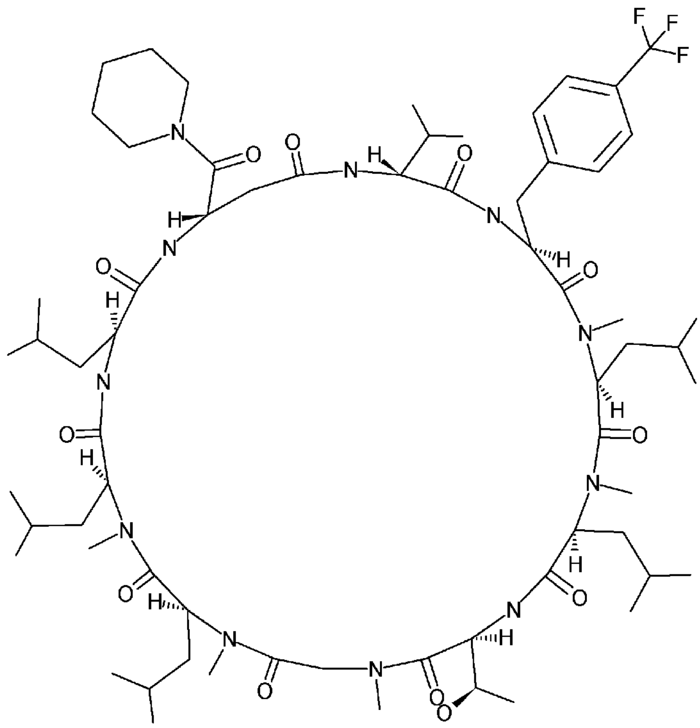 |
| pd149 | 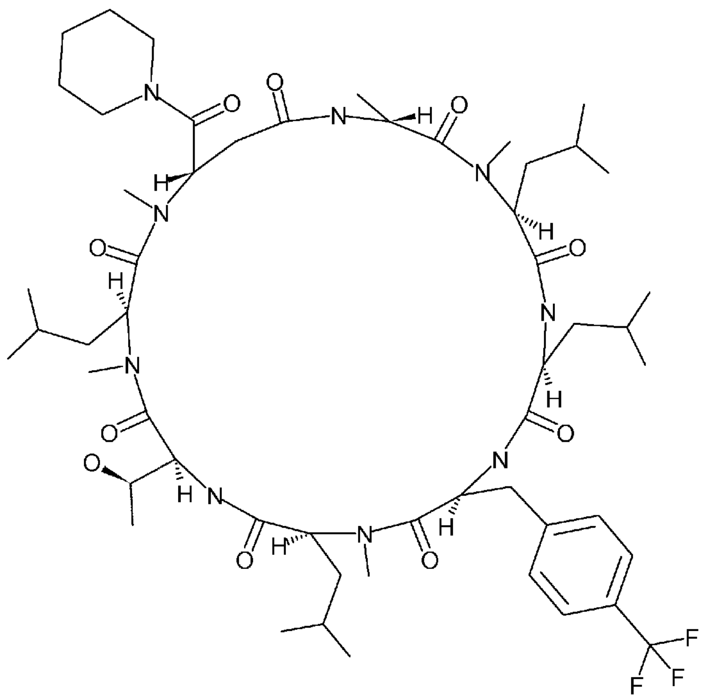 |

| ID | structure |
|---|---|
| pd150 | 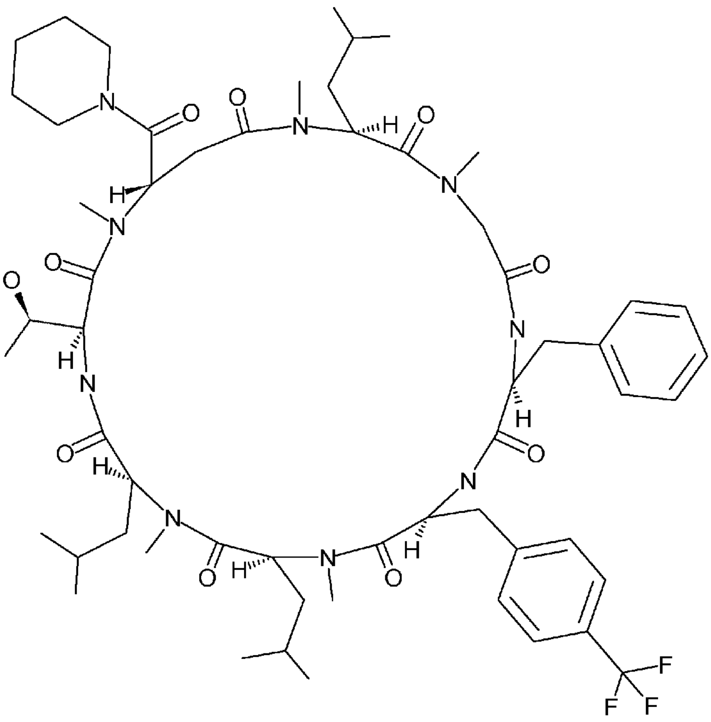 |
| pd151 | 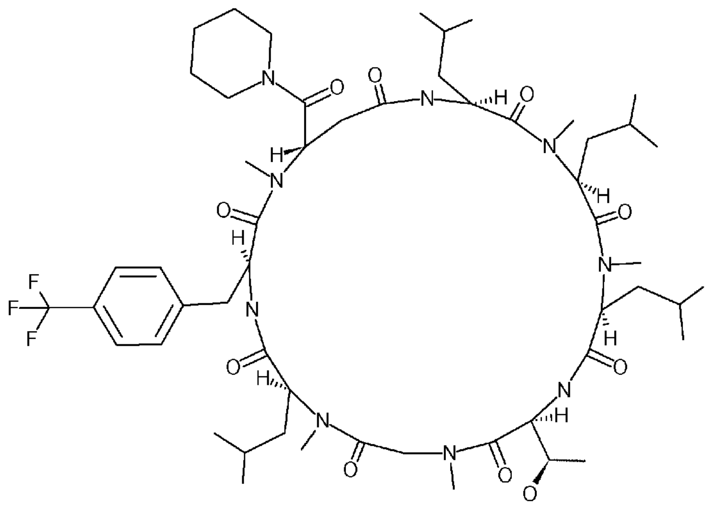 |

| ID | structure |
|---|---|
| pd152 | 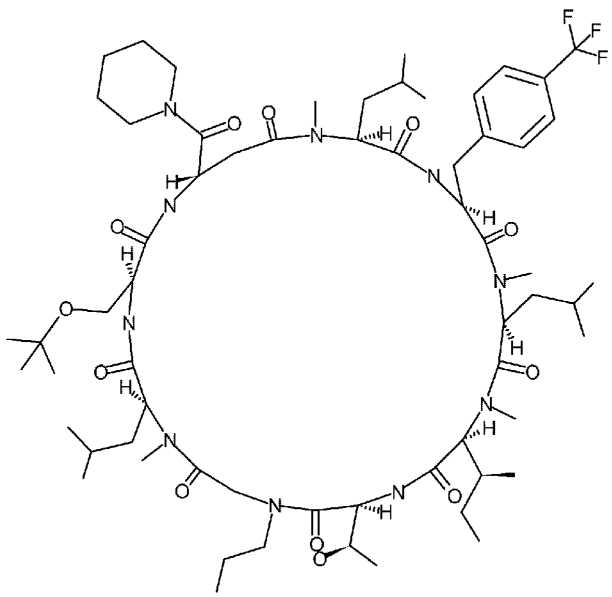 |
| pd153 | 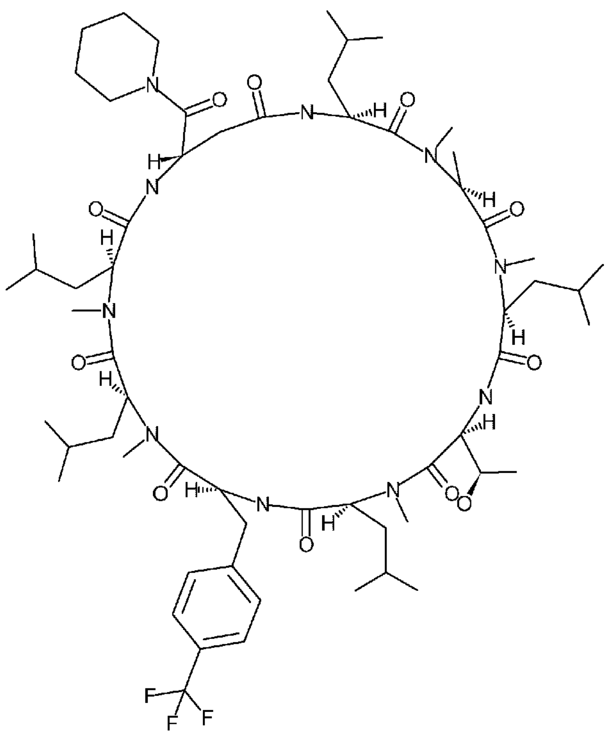 |

| ID | structure |
|---|---|
| pd154 | 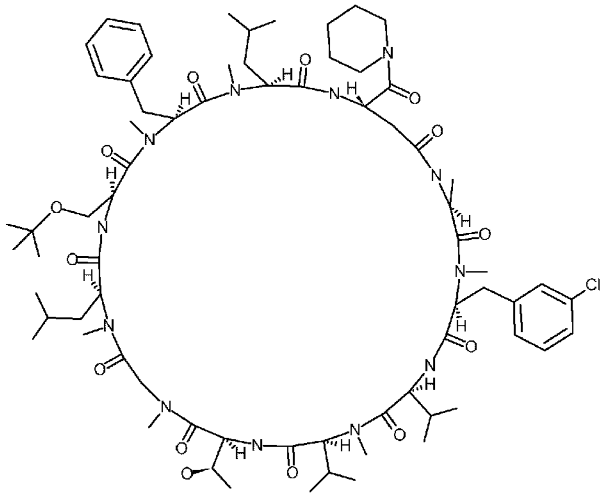 |
| pd155 | 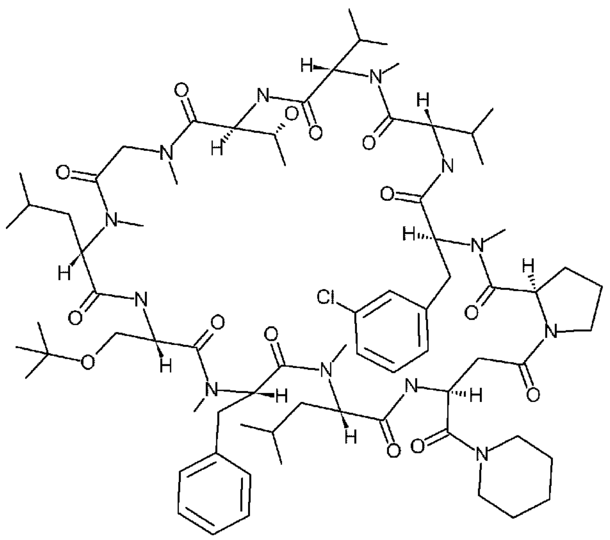 |

| ID | structure |
|---|---|
| pd156 | 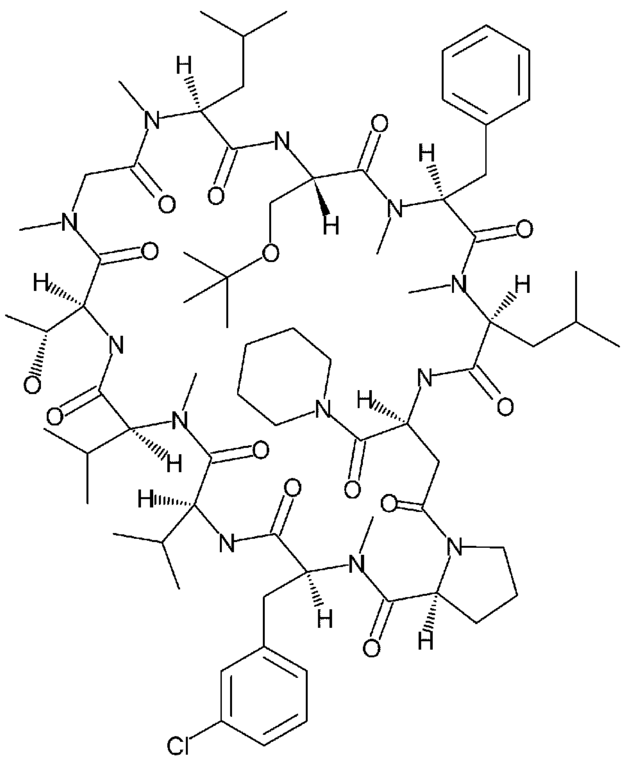 |
| pd157 | 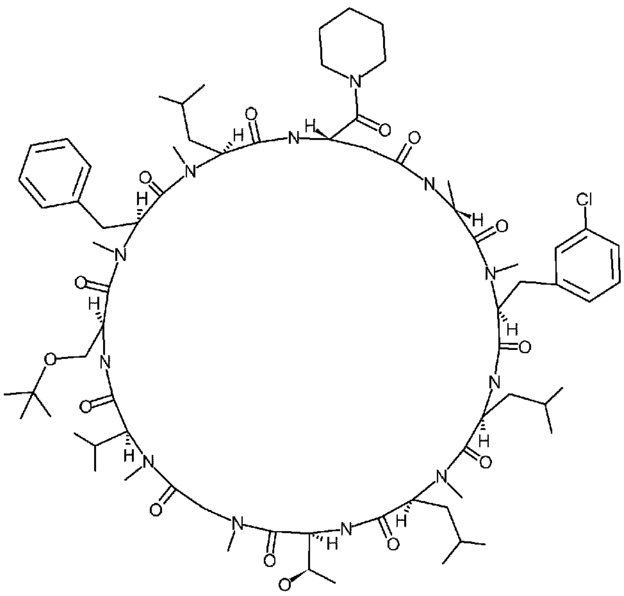 |

| ID | structure |
|---|---|
| pd158 | 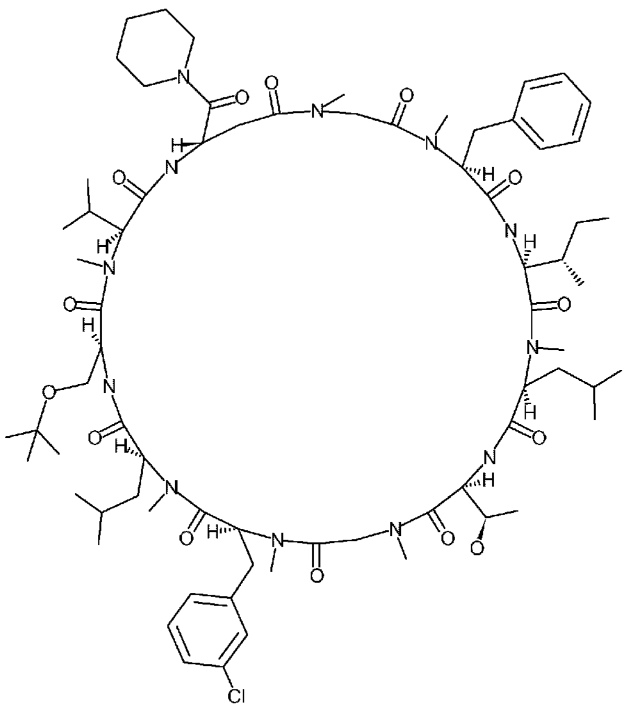 |
| pd159 | 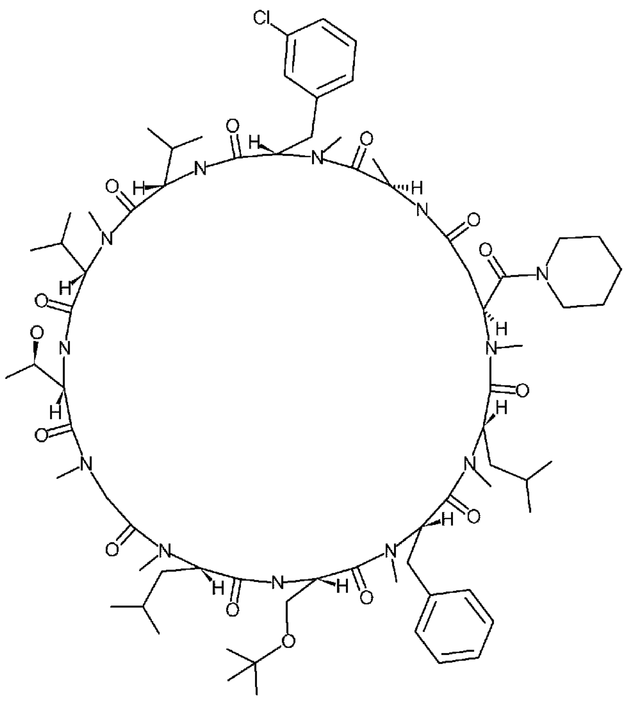 |

| ID | structure |
|---|---|
| pd160 | 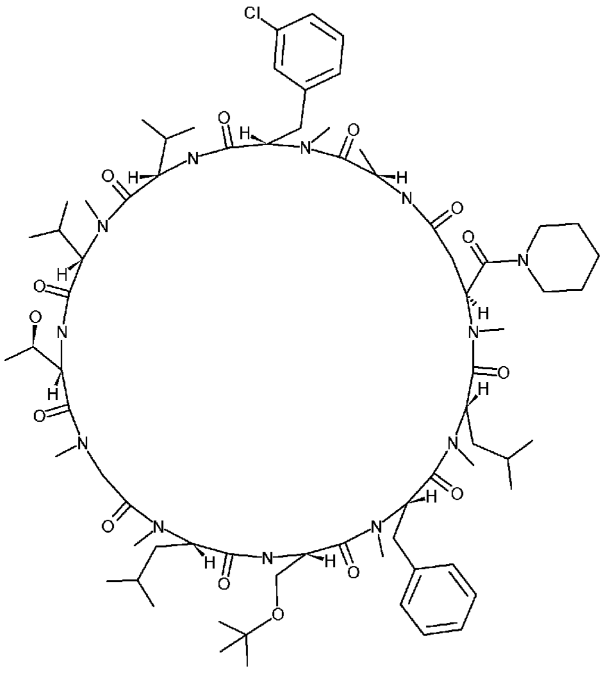 |
| pd161 | 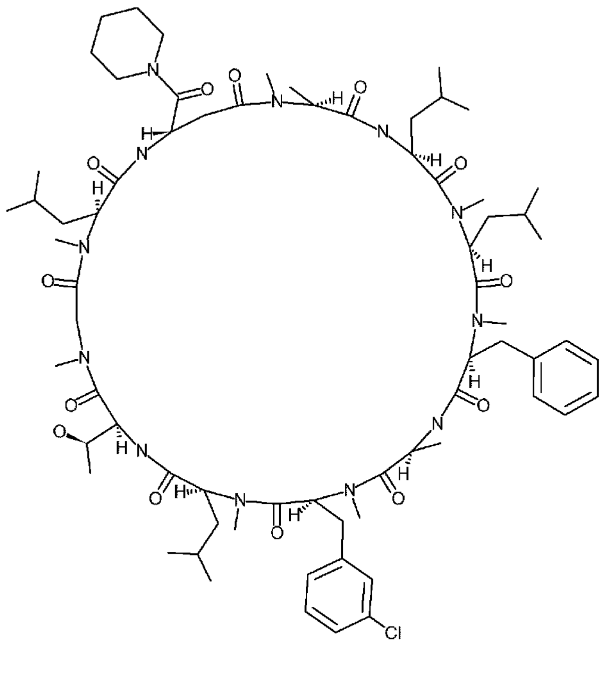 |

| ID | structure |
|---|---|
| pd162 | 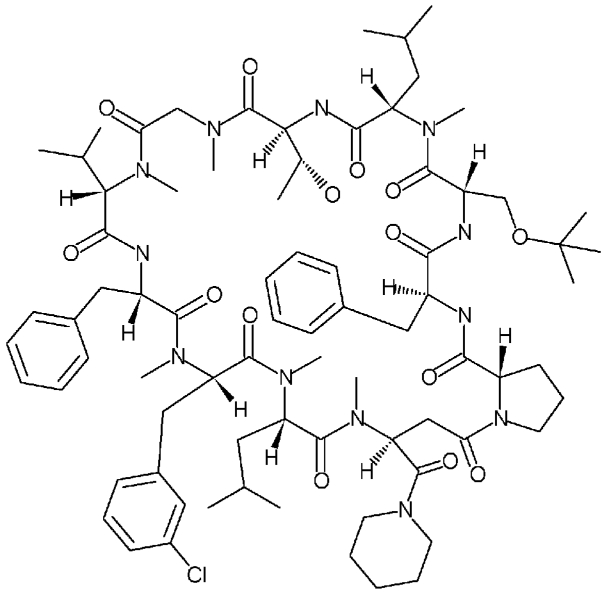 |
| pd163 | 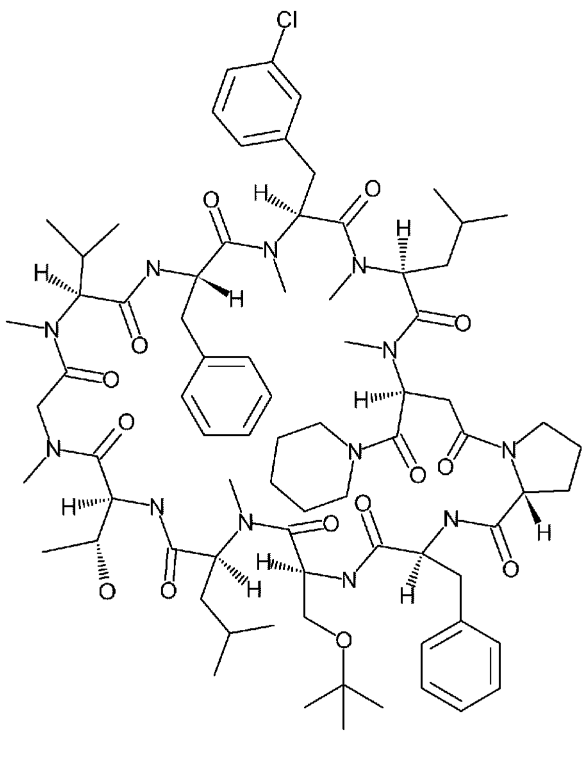 |

| ID | structure |
|---|---|
| pd164 | 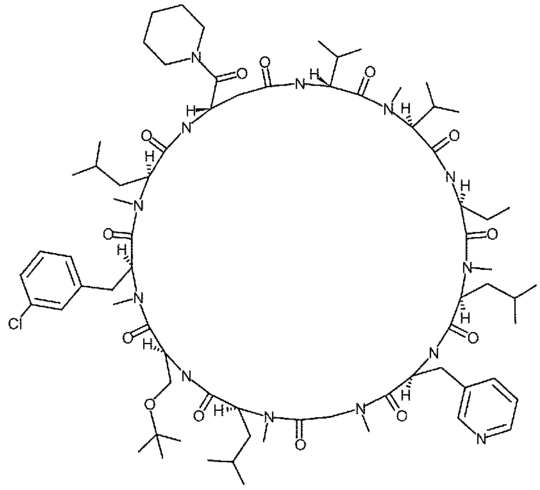 |
| pd165 | 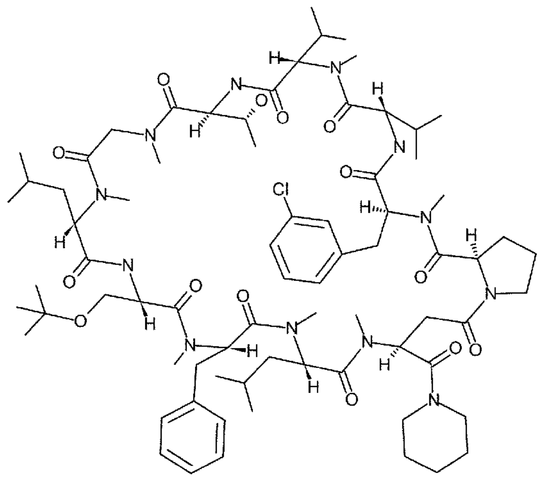 |

| ID | structure |
|---|---|
| pd166 | 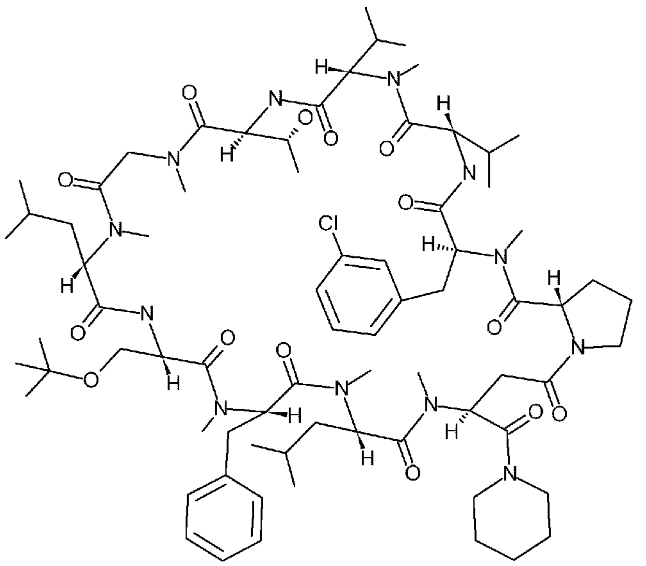 |
| pd167 | 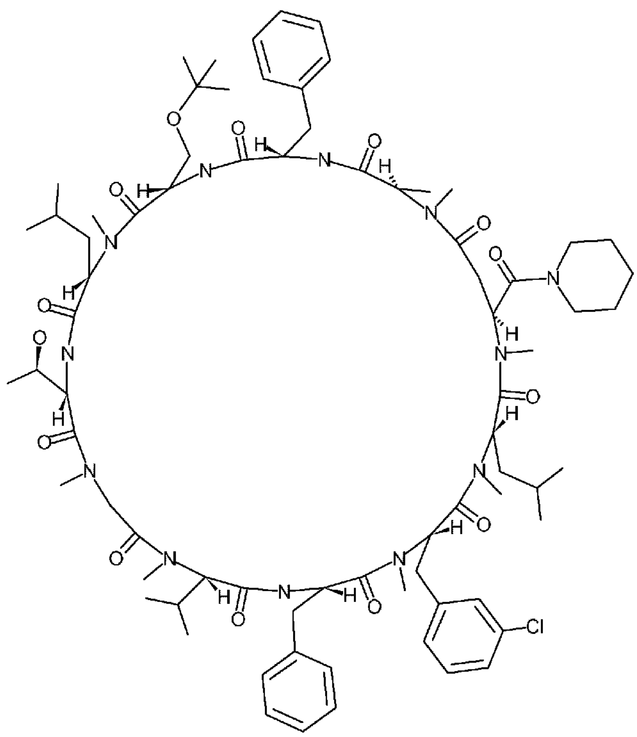 |

| ID | structure |
|---|---|
| pd168 | 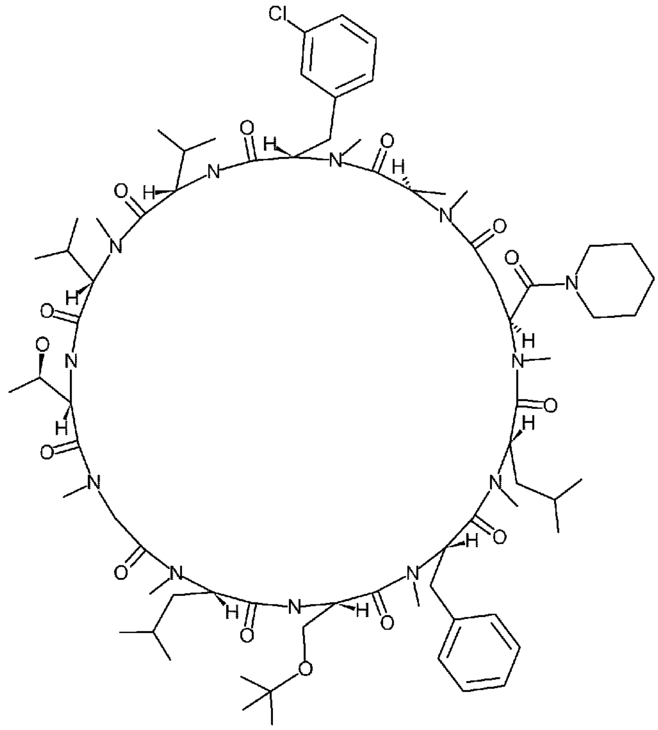 |
| pd169 | 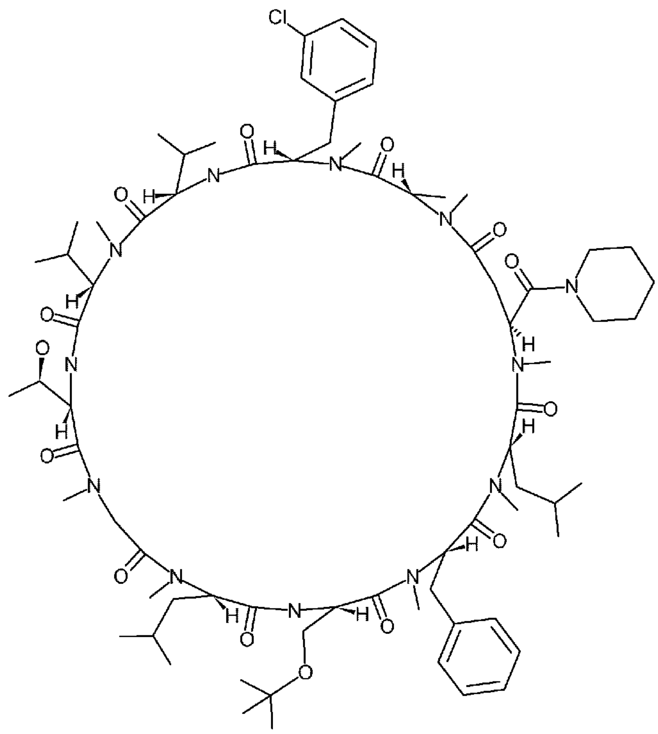 |

| ID | structure |
|---|---|
| pd170 | 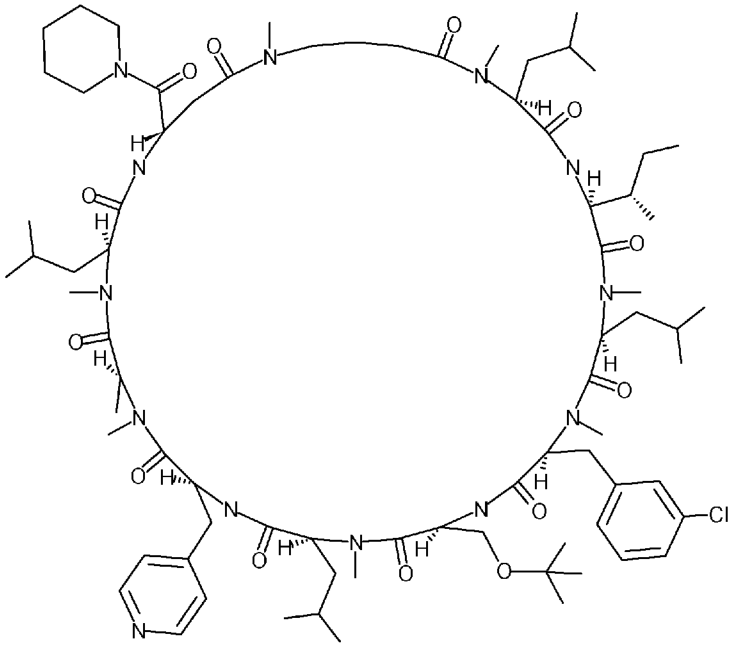 |
| pd171 | 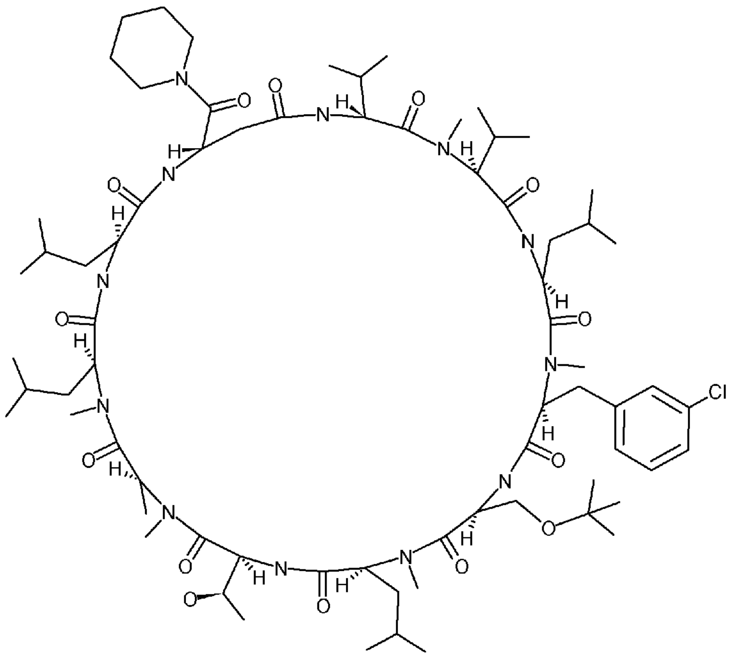 |

| ID | structure |
|---|---|
| pd172 | 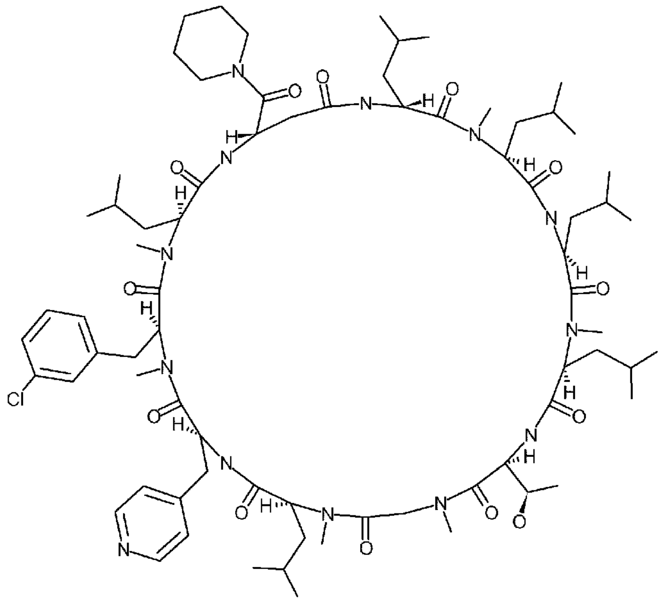 |
| pd173 | 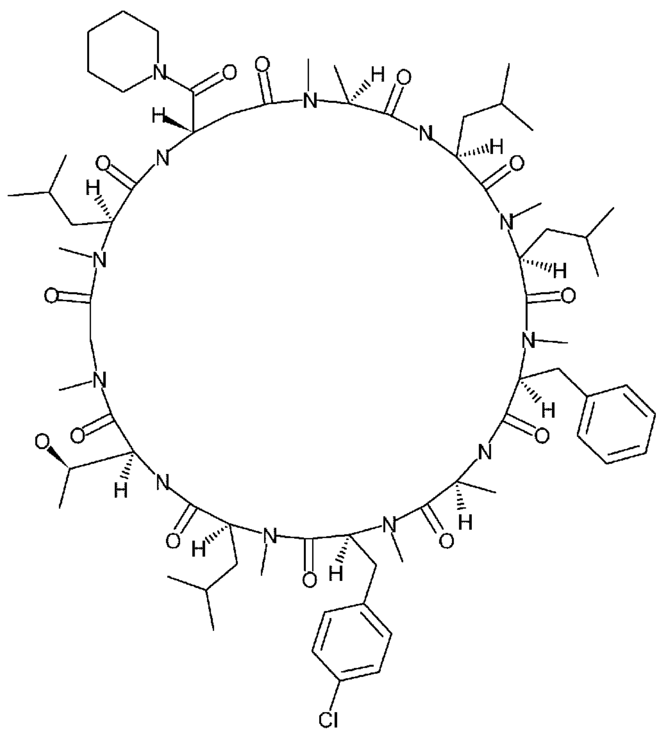 |

| ID | structure |
| --- | --- |
| pd174 | 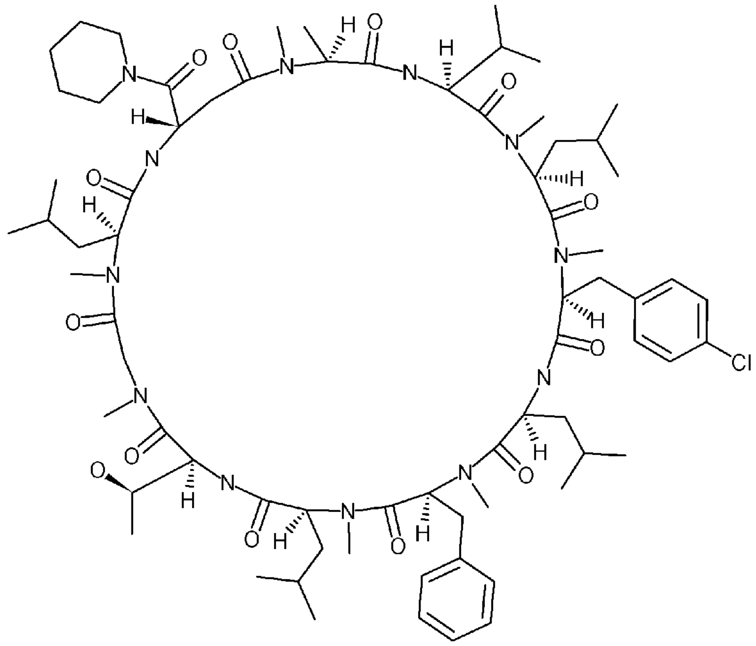 |
| pd175 | 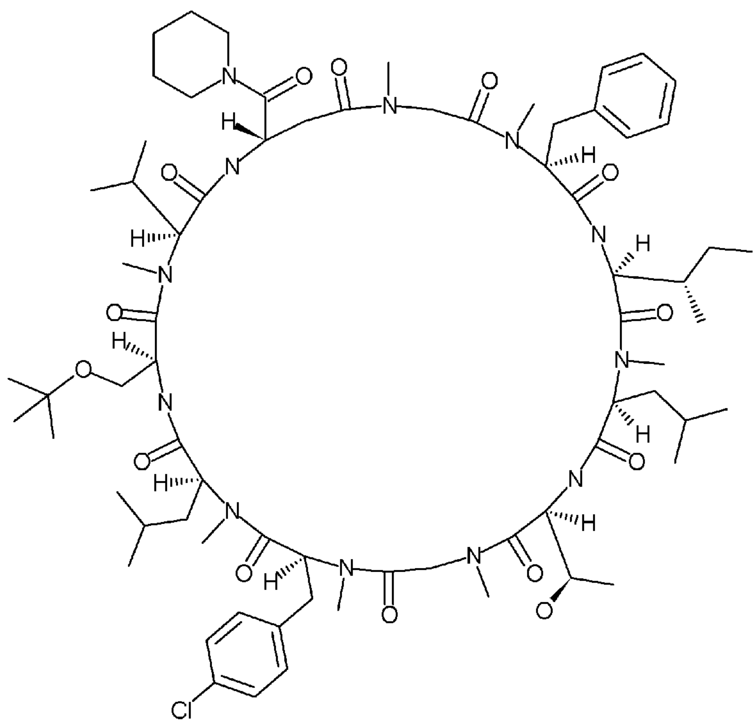 |

| ID | structure |
| --- | --- |
| pd176 | 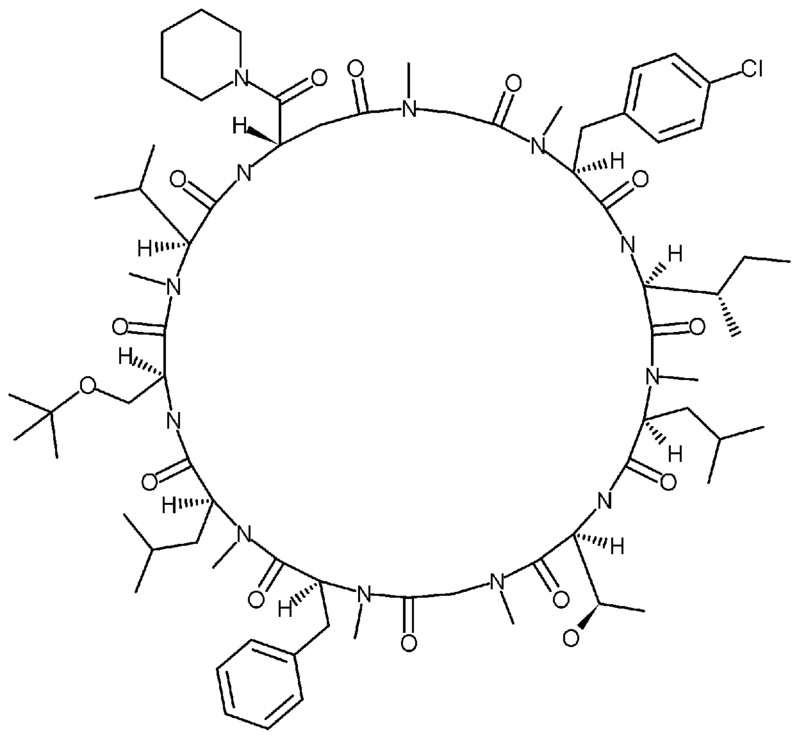 |

| ID | structure |
|---|---|
| pd177 | 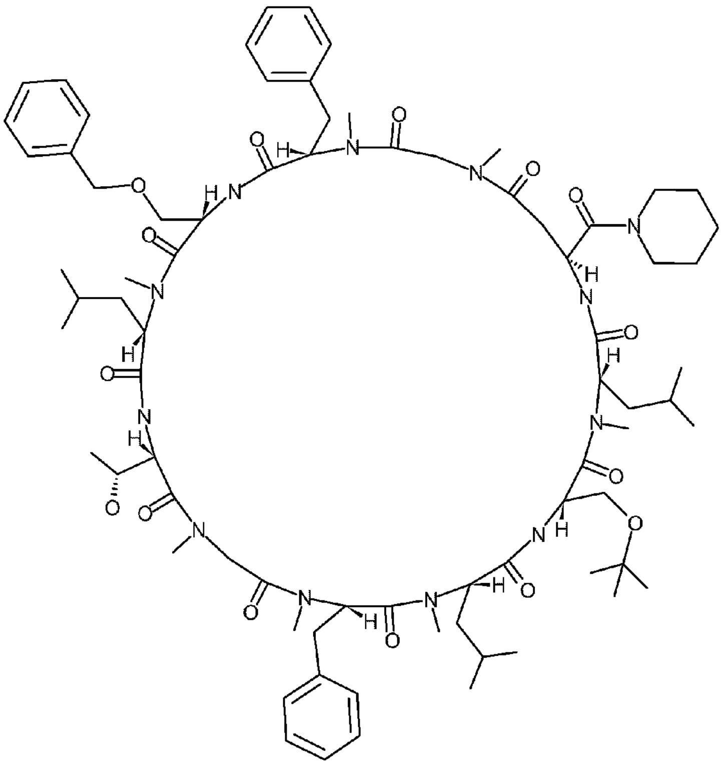 |
| pd178 | 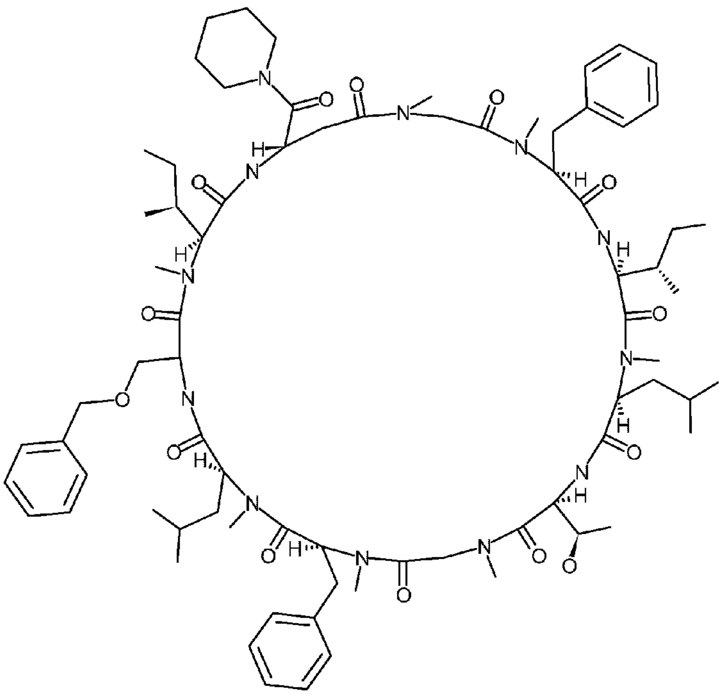 |

| ID | structure |
|---|---|
| pd179 | 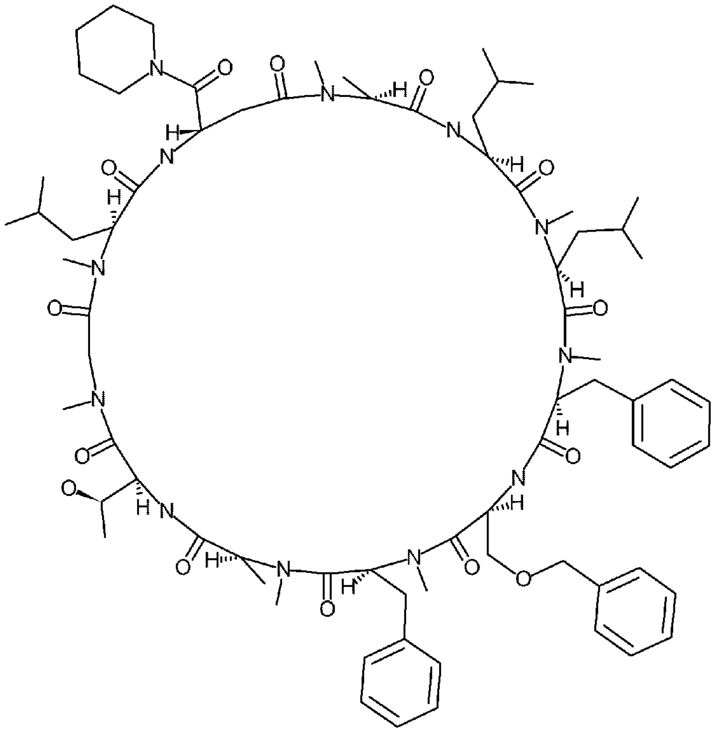 |
| pd180 | 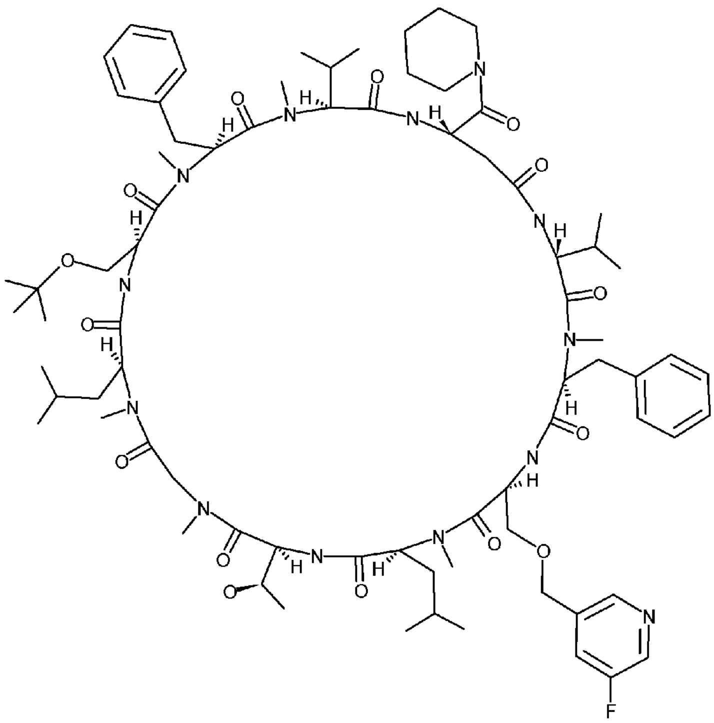 |

| ID | structure |
|---|---|
| pd181 | 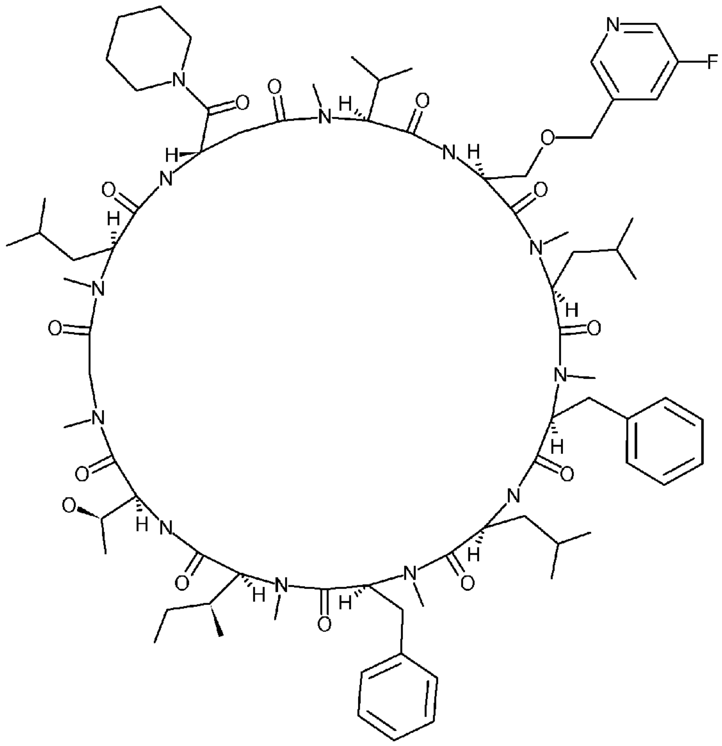 |
| pd182 | 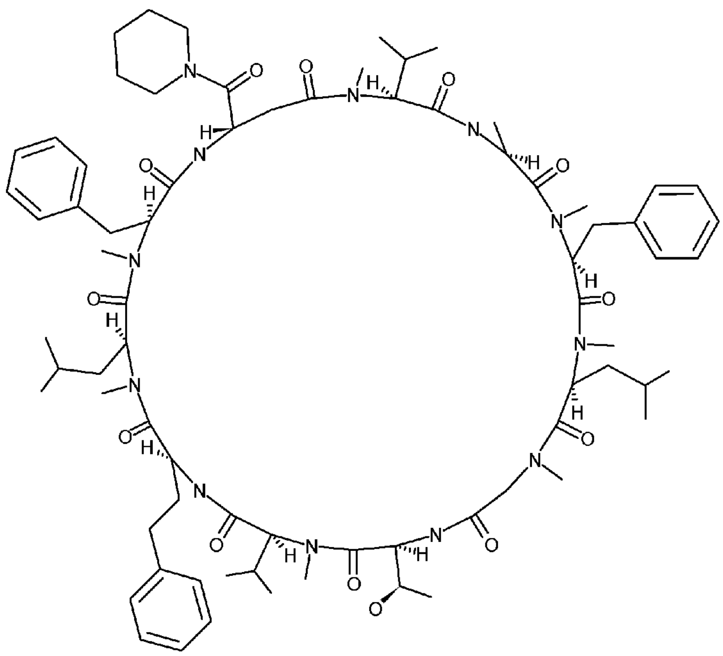 |

| ID | structure |
|---|---|
| pd183 | 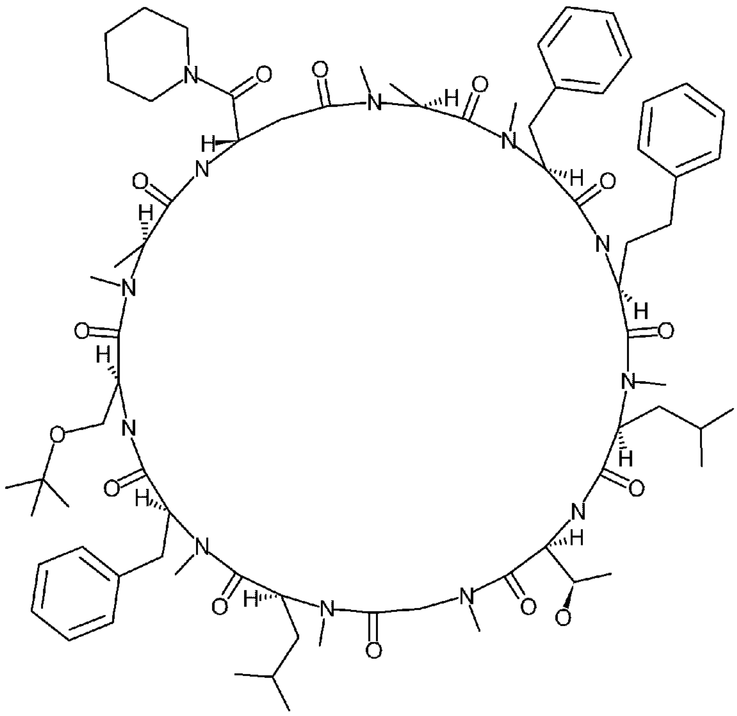 |
| pd184 | 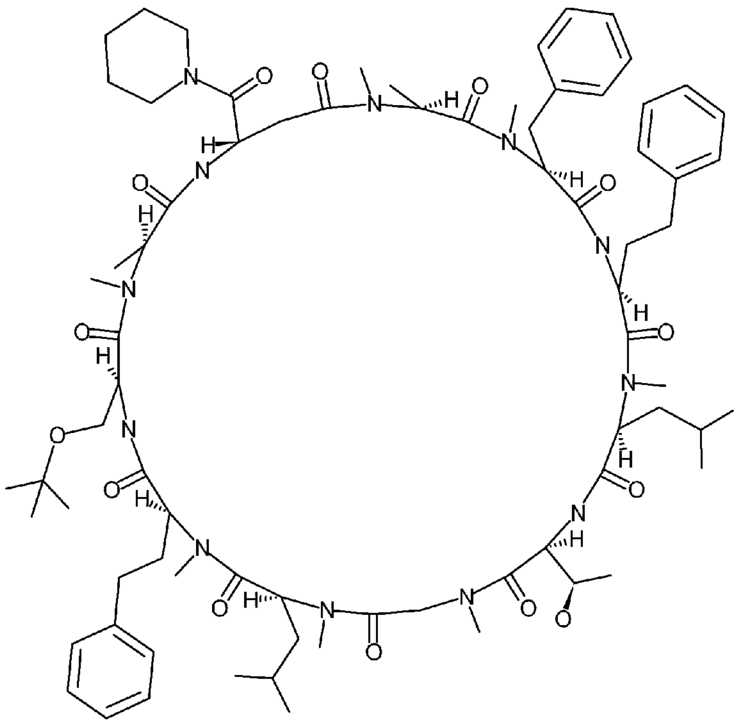 |

| ID | structure |
|---|---|
| pd185 | 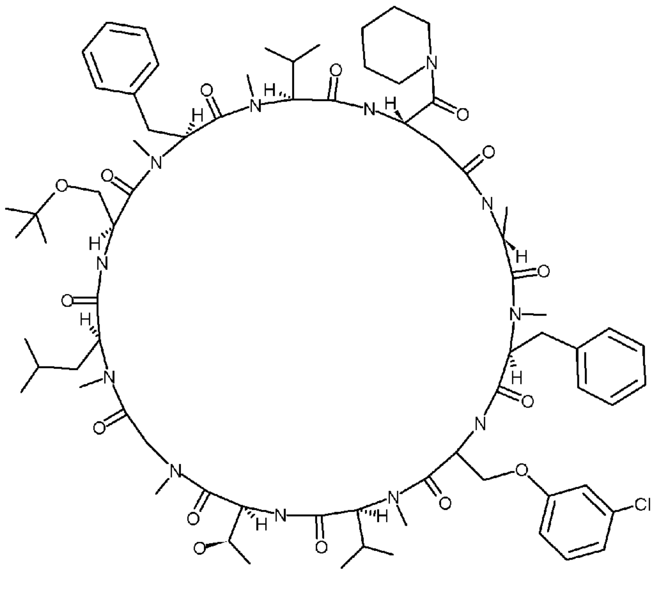 |
| pd186 | 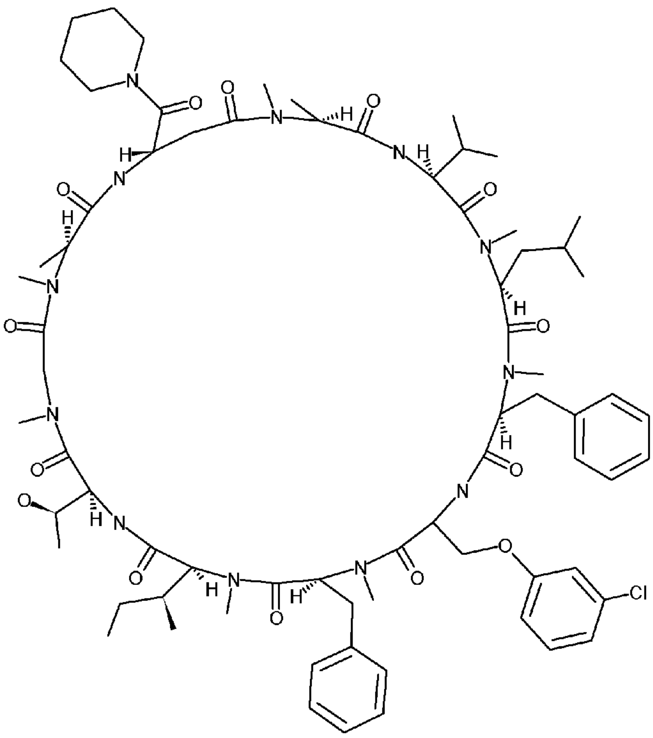 |

| ID | structure |
|---|---|
| pd187 | 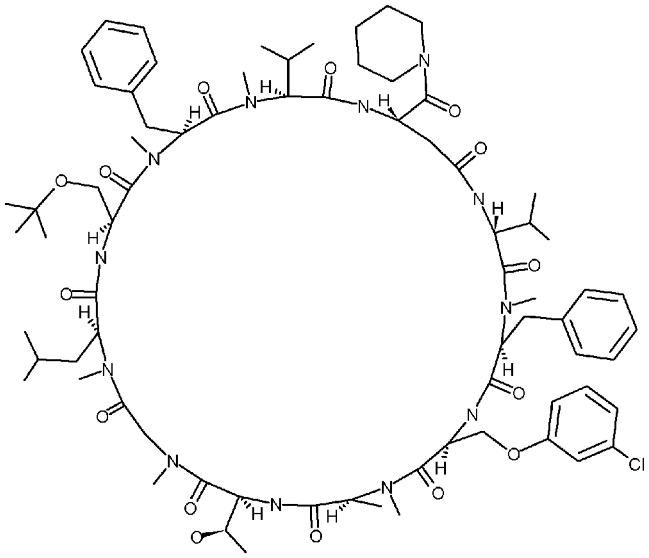 |
| pd188 | 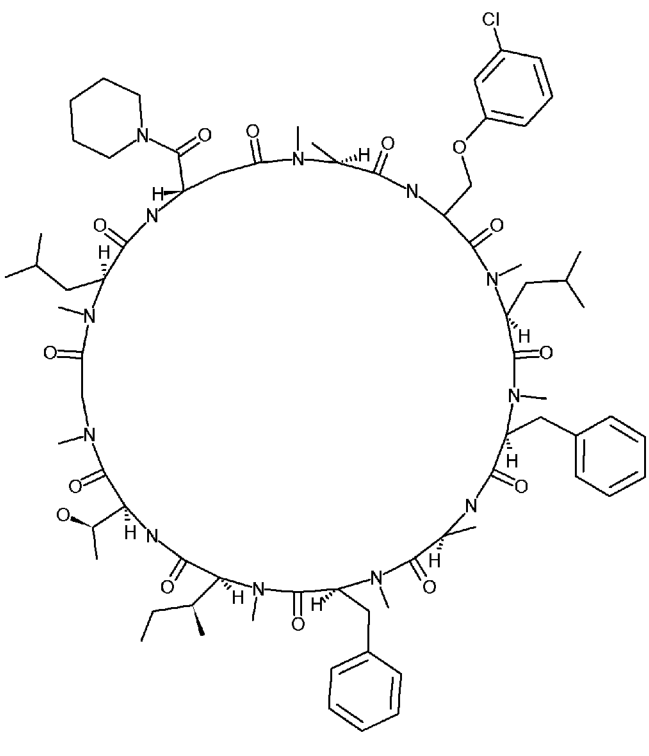 |

| ID | structure |
|---|---|
| pd189 | 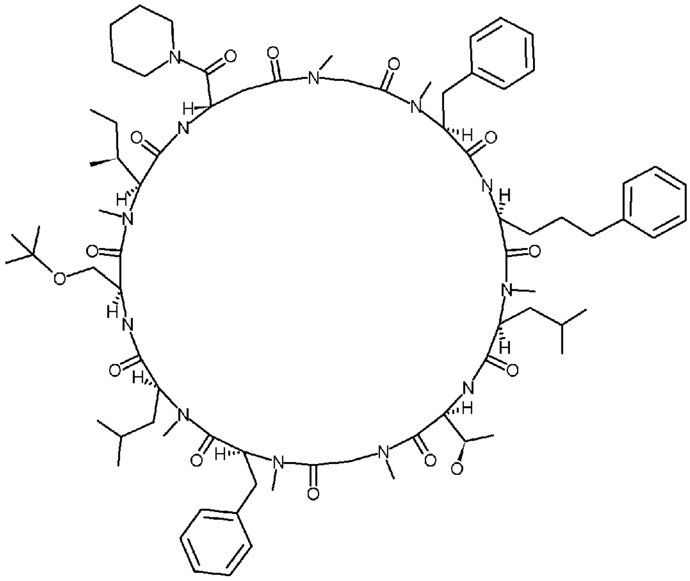 |
| pd190 | 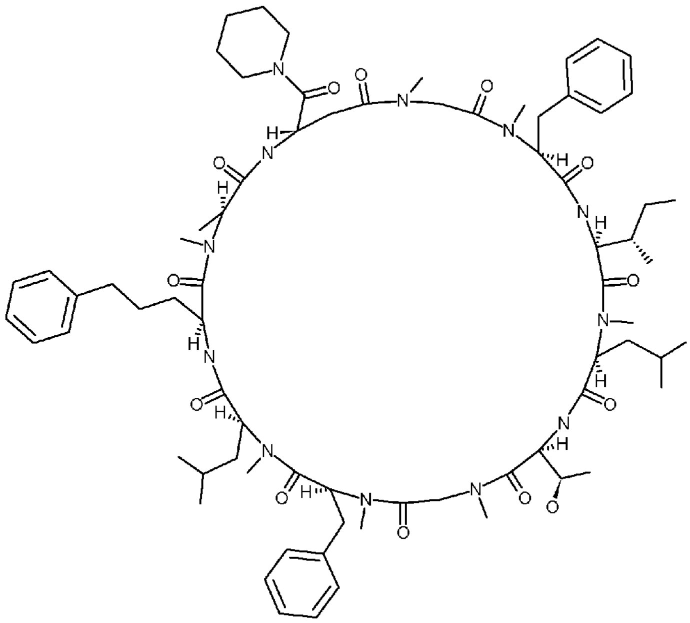 |

| ID | structure |
|---|---|
| pd191 | 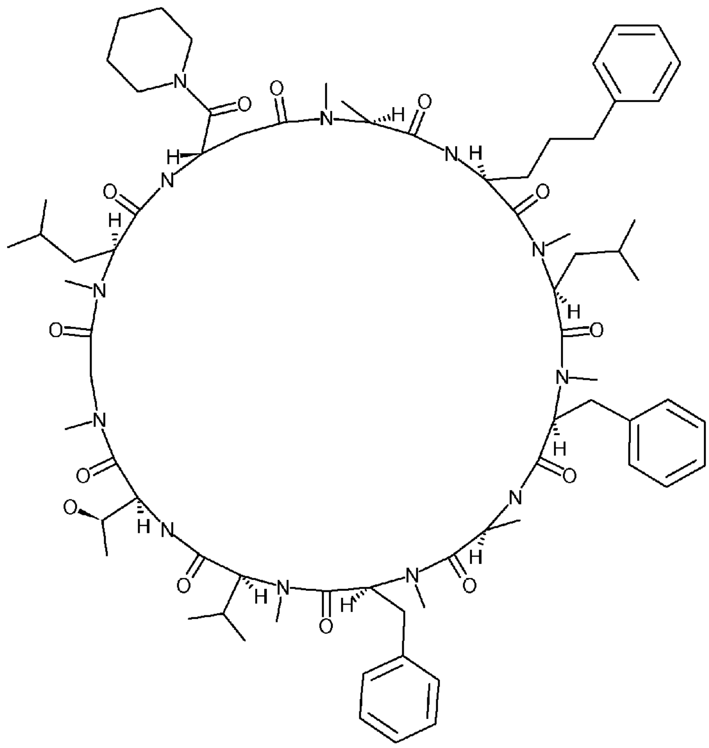 |
| pd192 | 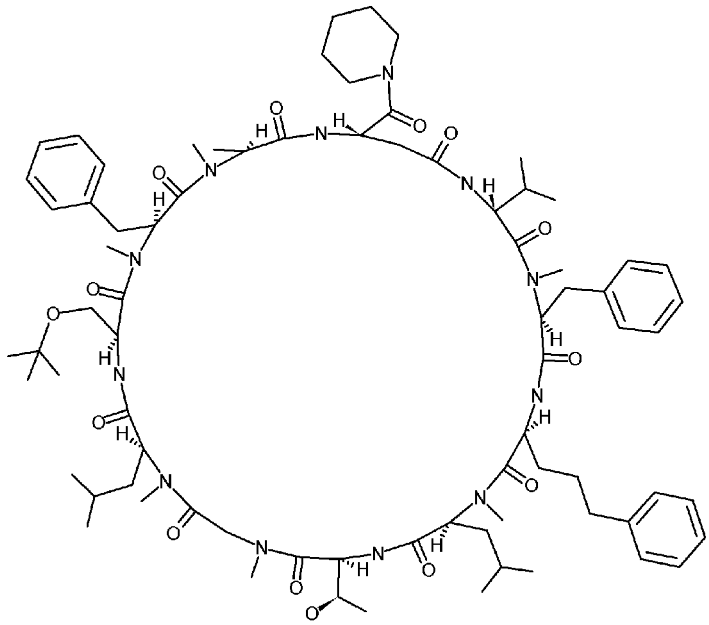 |

| ID | structure |
|---|---|
| pd193 | 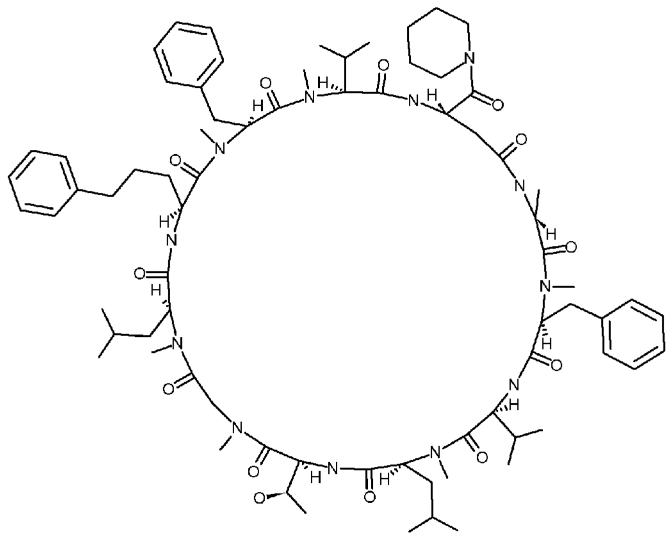 |
| pd194 | 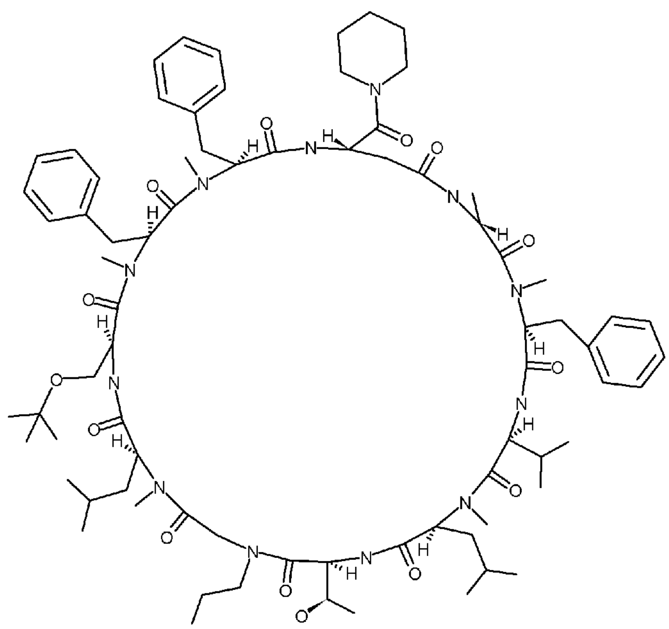 |

| ID | structure |
|---|---|
| pd195 | 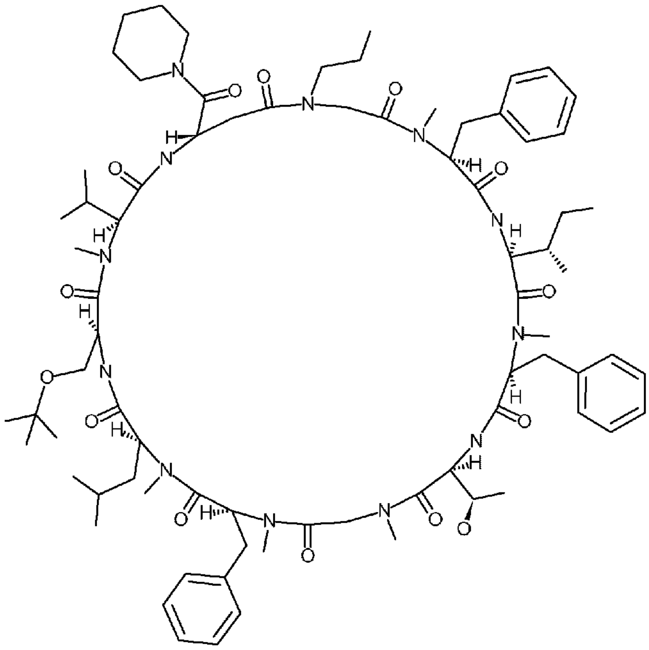 |
| pd196 | 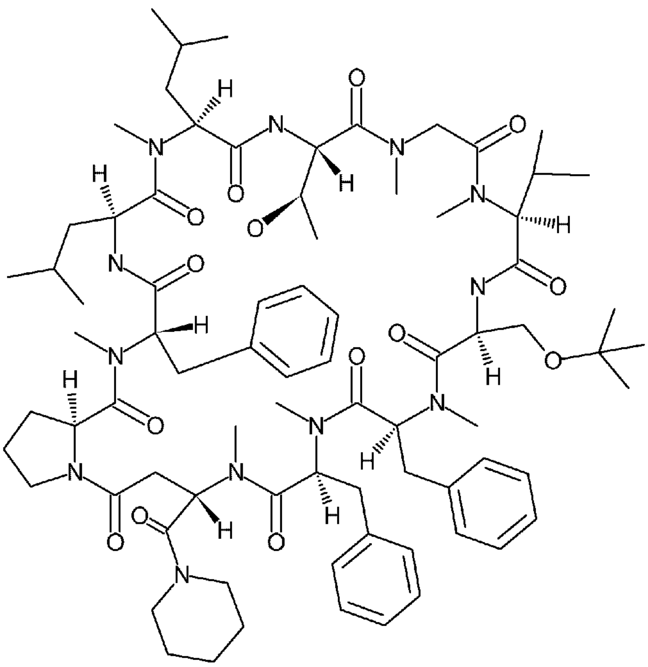 |

| ID | structure |
|---|---|
| pd197 | 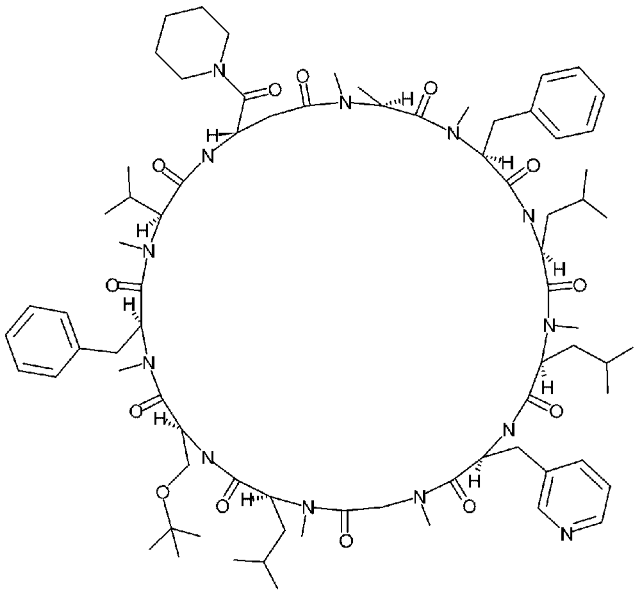 |
| pd198 | 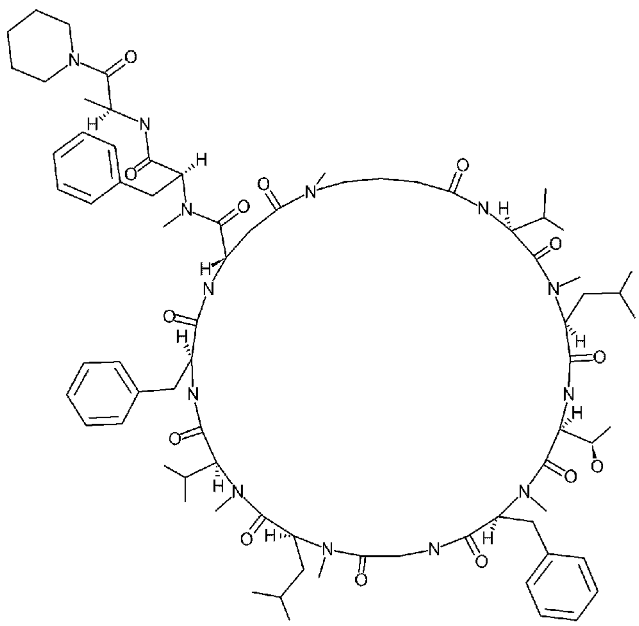 |

| ID | structure |
| --- | --- |
| pd199 | 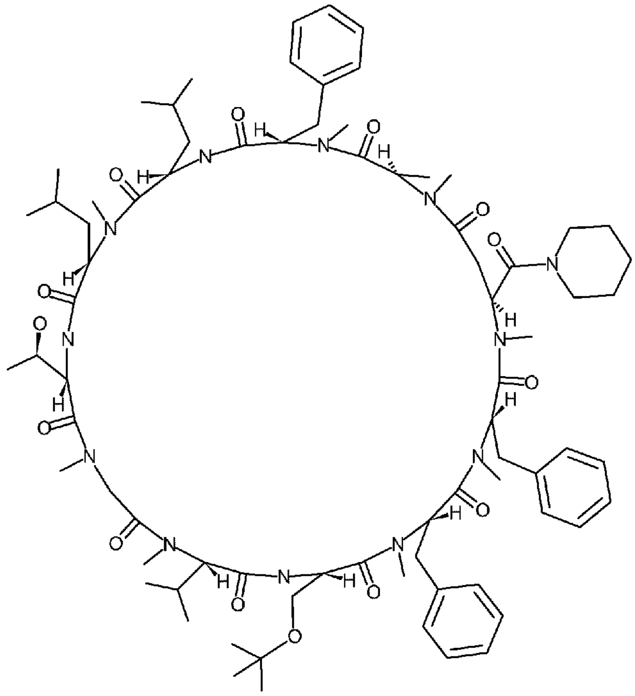 |
| pd200 | 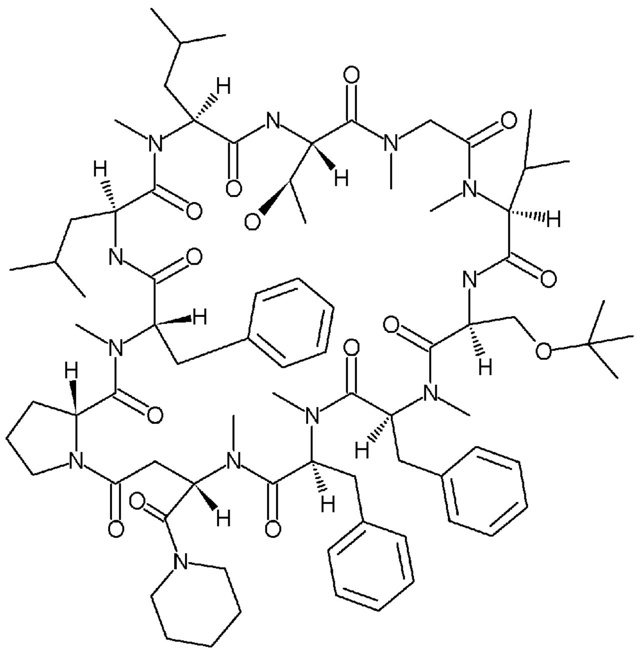 |

| ID | structure |
|---|---|
| pd201 | 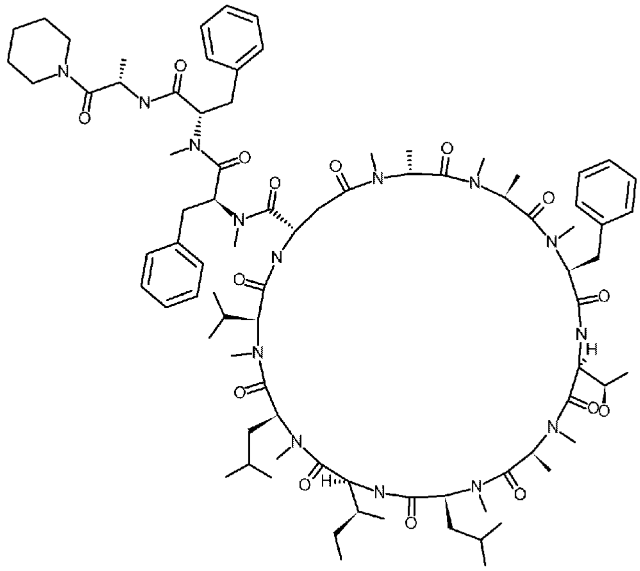 |
| pd202 | 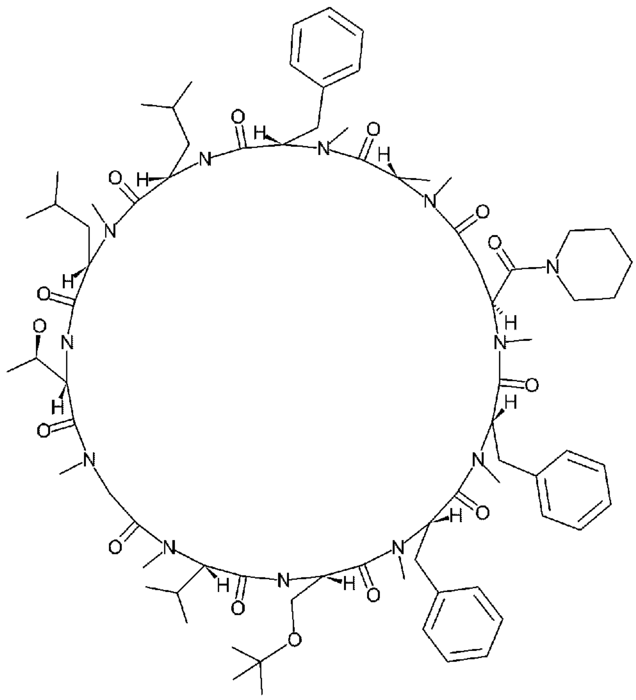 |

| ID | structure |
|---|---|
| pd203 | 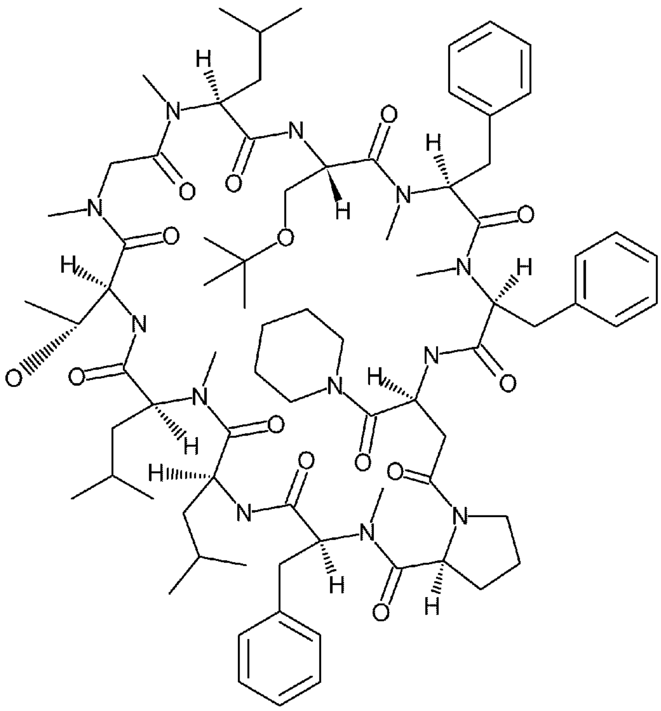 |
| pd204 | 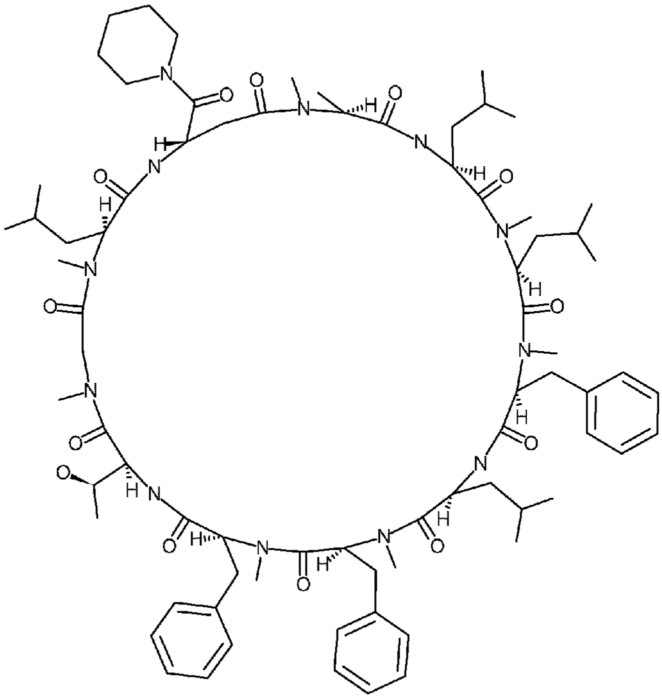 |

| ID | structure |
|---|---|
| pd205 | 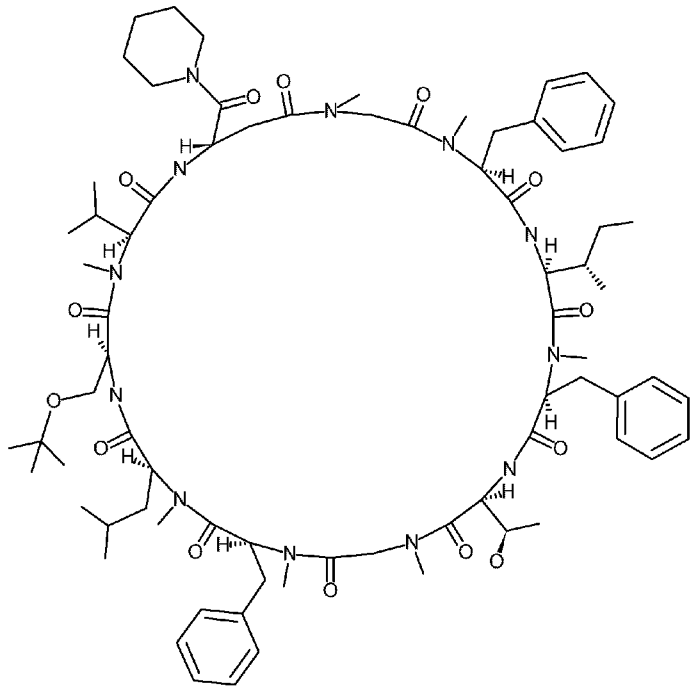 |
| pd206 | 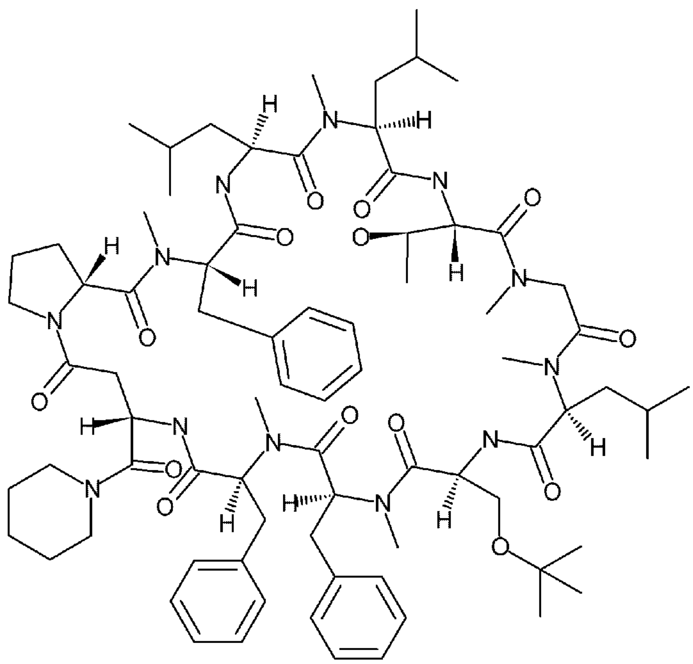 |

| ID | structure |
|---|---|
| pd207 | 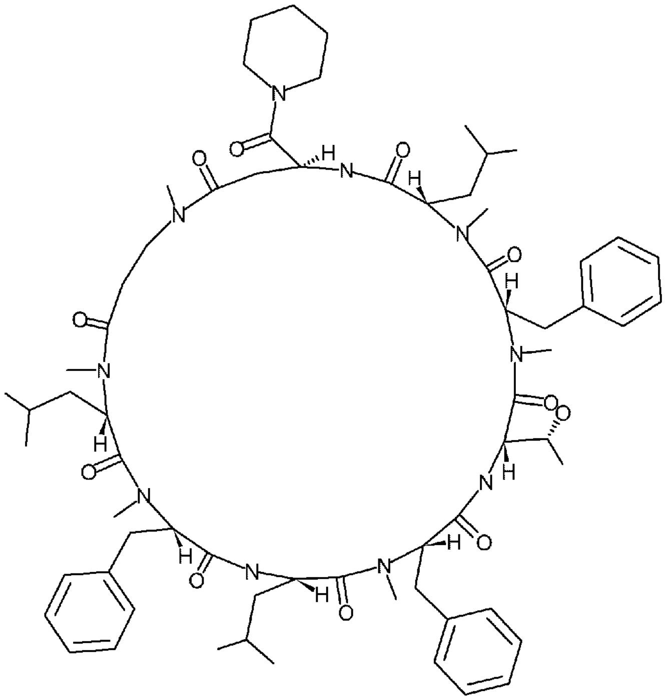 |
| pd208 | 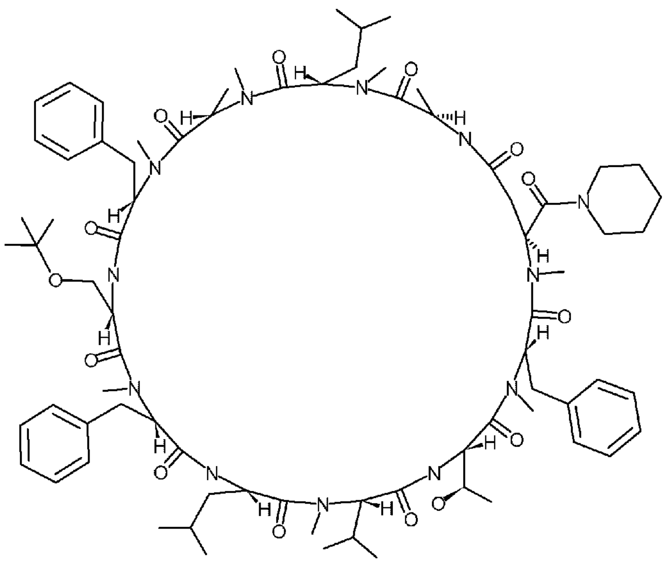 |

| ID | structure |
| --- | --- |
| pd209 | 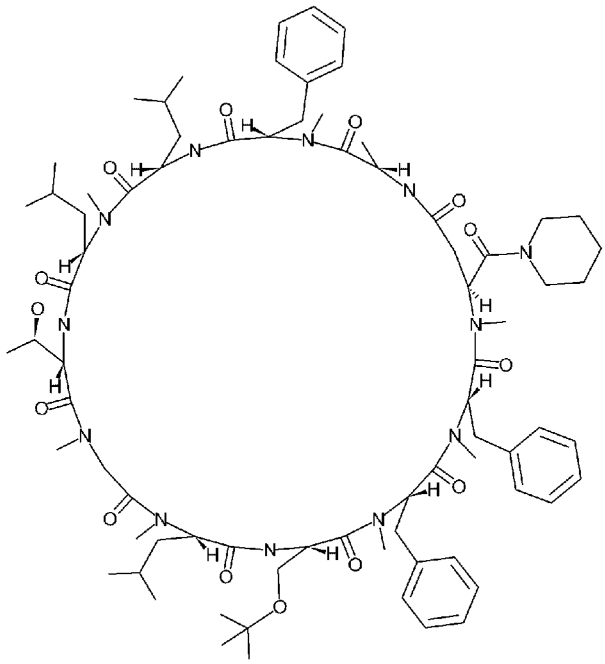 |
| pd210 | 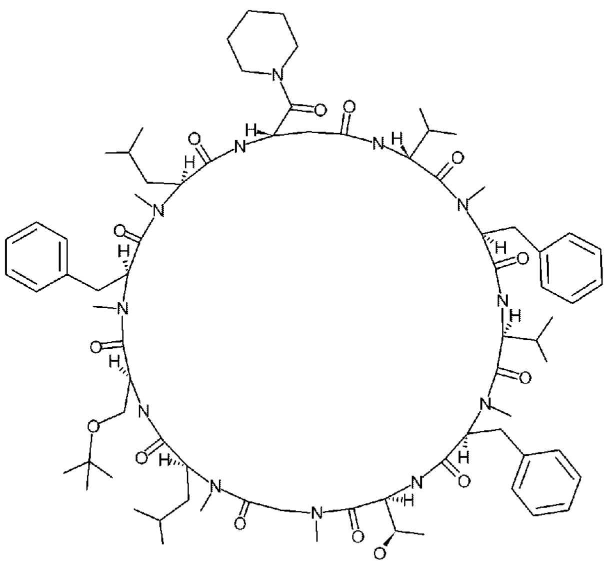 |

| ID | structure |
|---|---|
| pd211 | 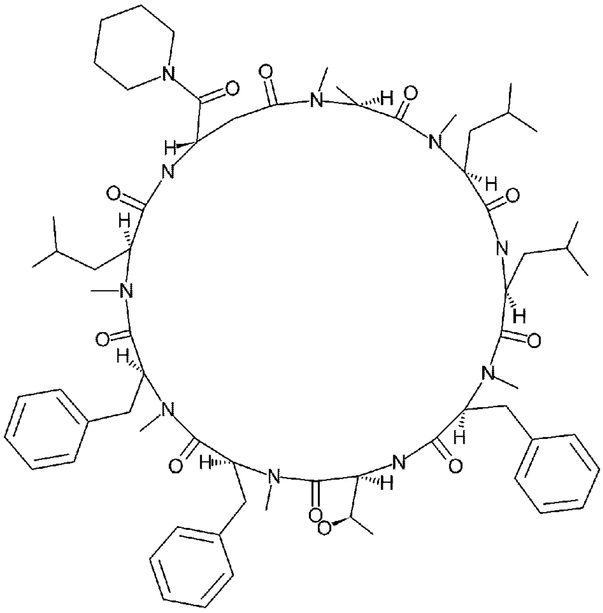 |
| pd212 | 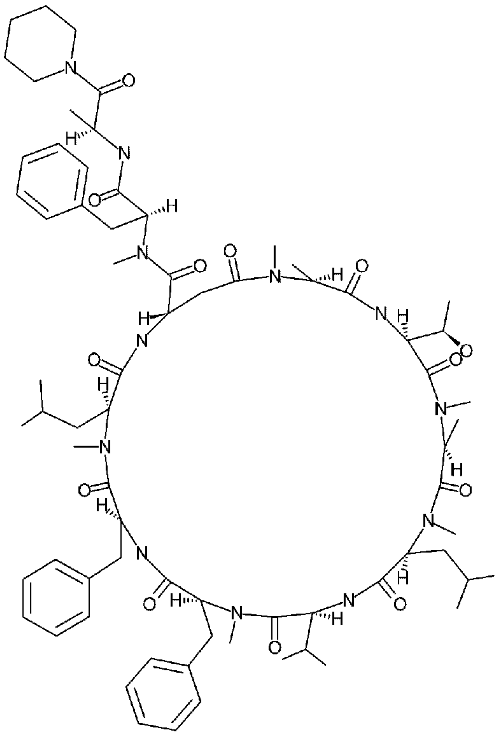 |

| ID | structure |
|---|---|
| pd213 | 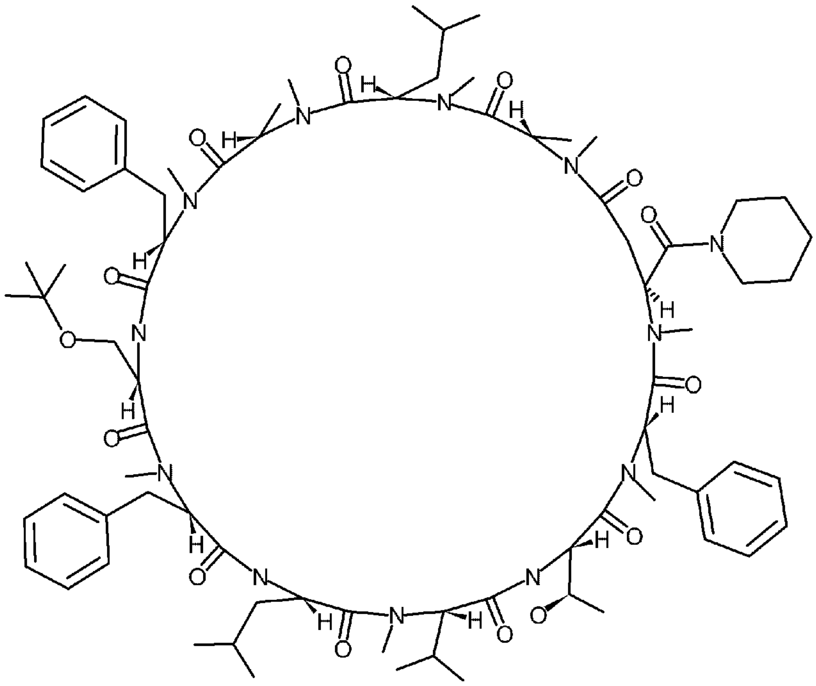 |
| pd214 | 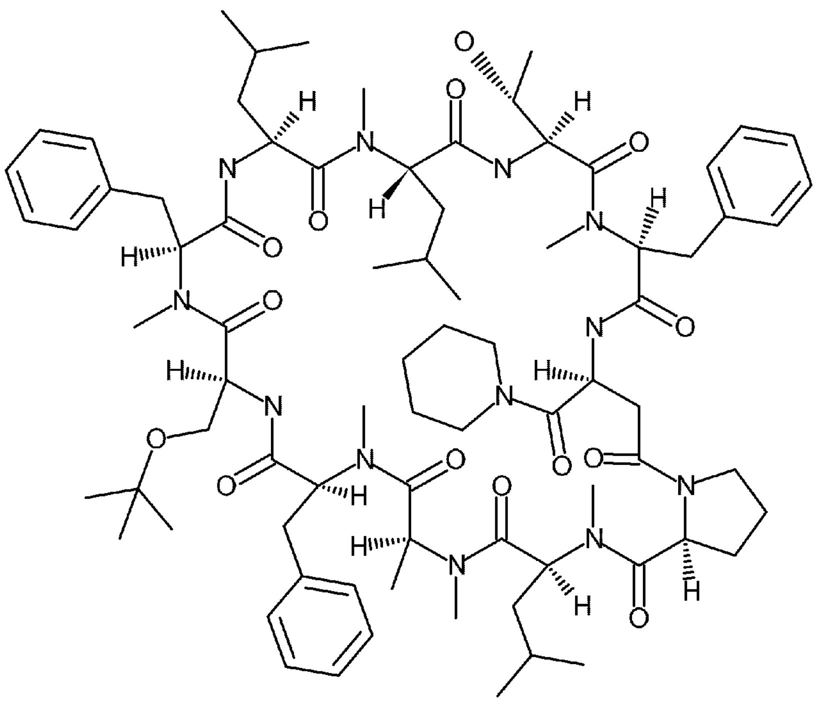 |

| ID | structure |
|---|---|
| pd215 | 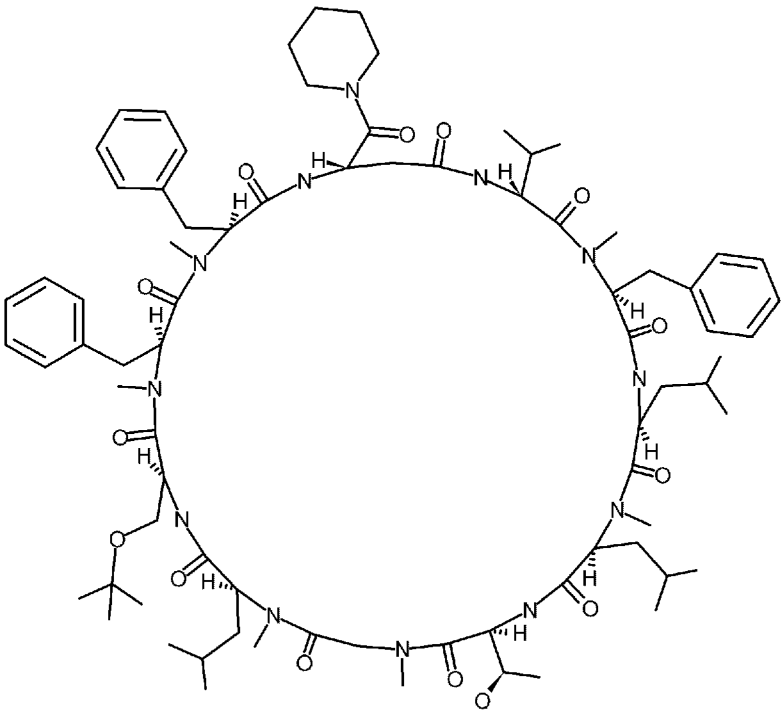 |
| pd216 | 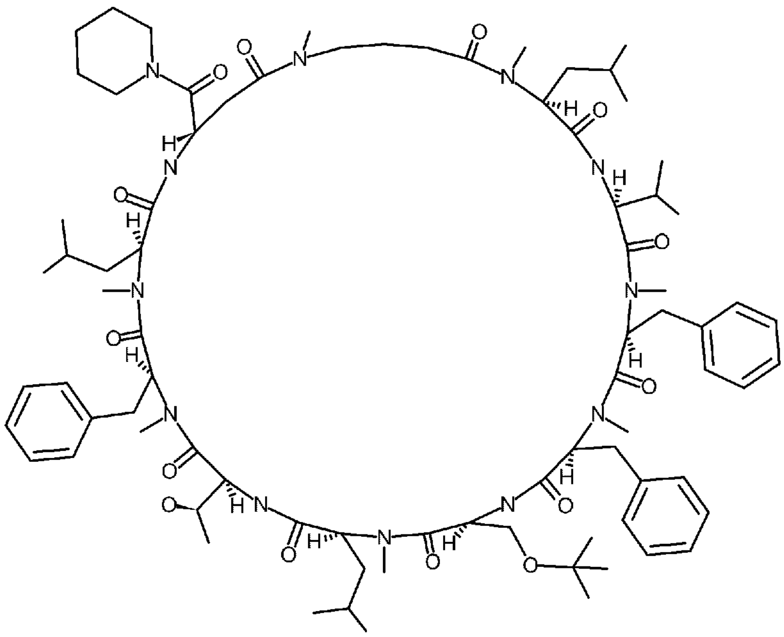 |

| ID | structure |
|---|---|
| pd217 | 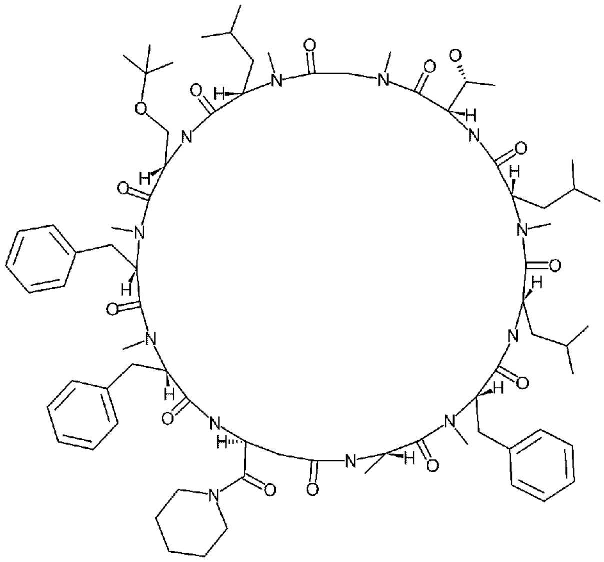 |
| pd218 | 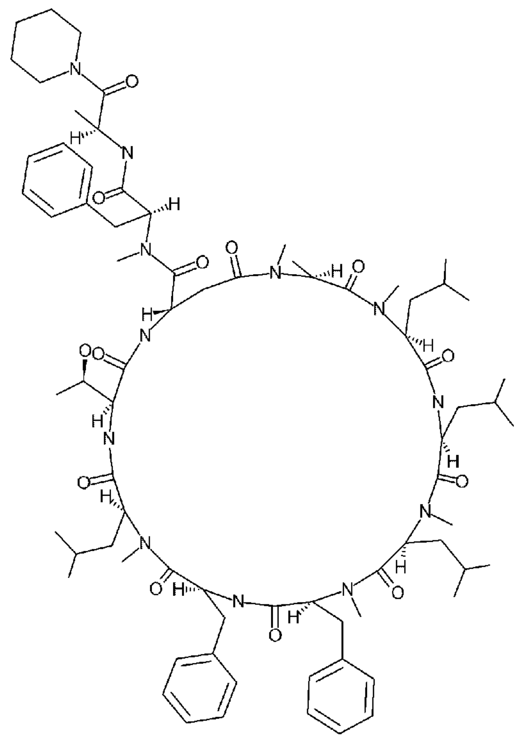 |

| ID | structure |
|---|---|
| pd219 | 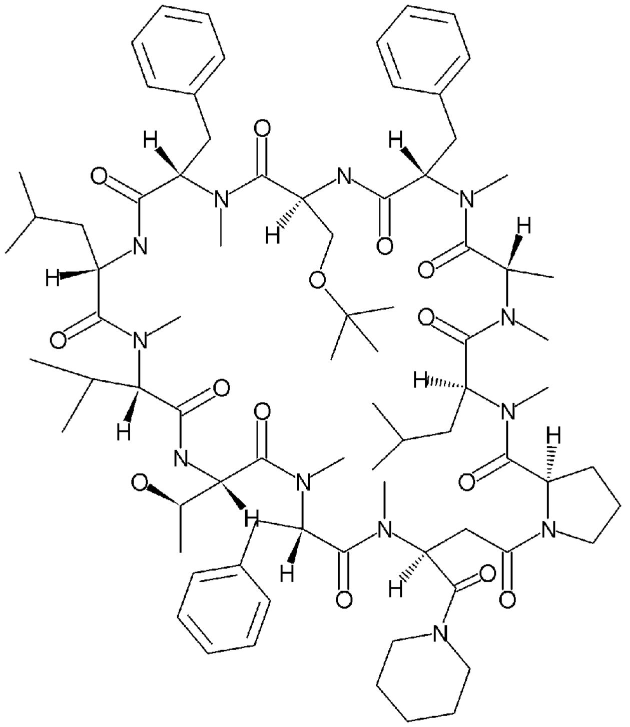 |
| pd220 | 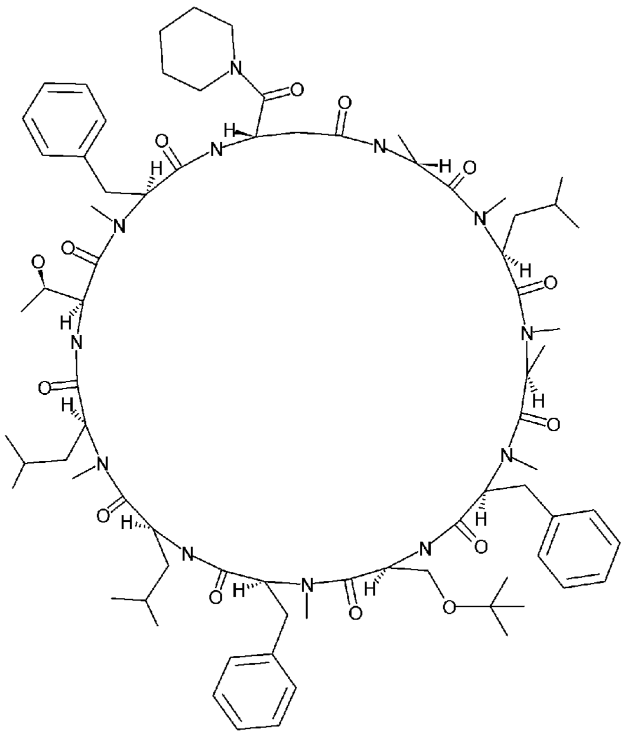 |

| ID | structure |
|---|---|
| pd221 | 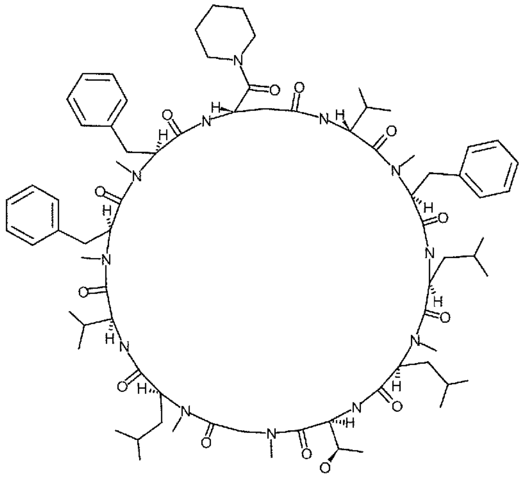 |
| pd222 | 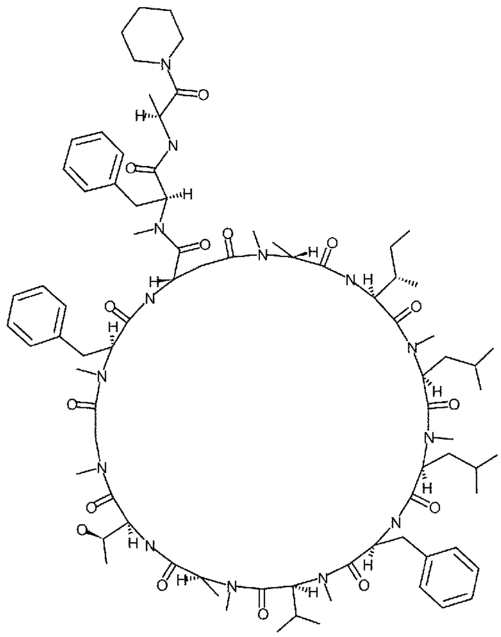 |

| ID | structure |
|---|---|
| pd223 | 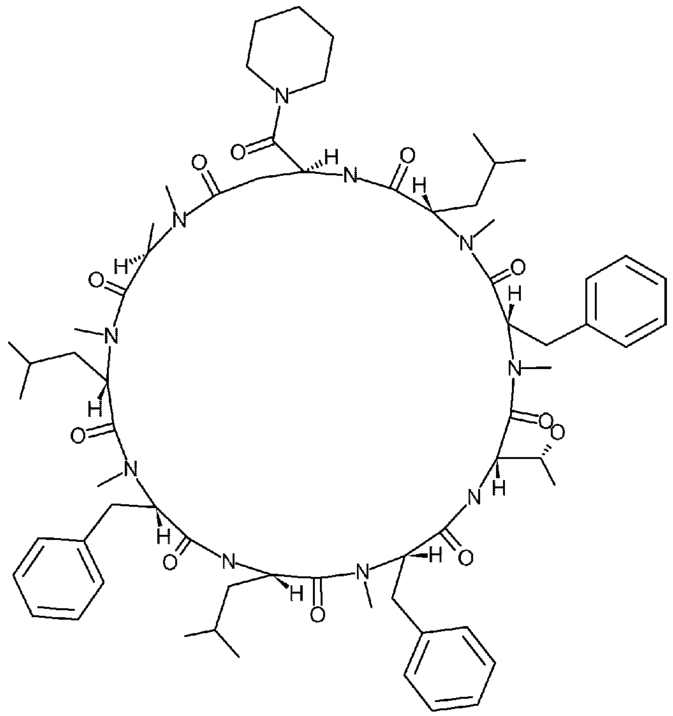 |
| pd224 | 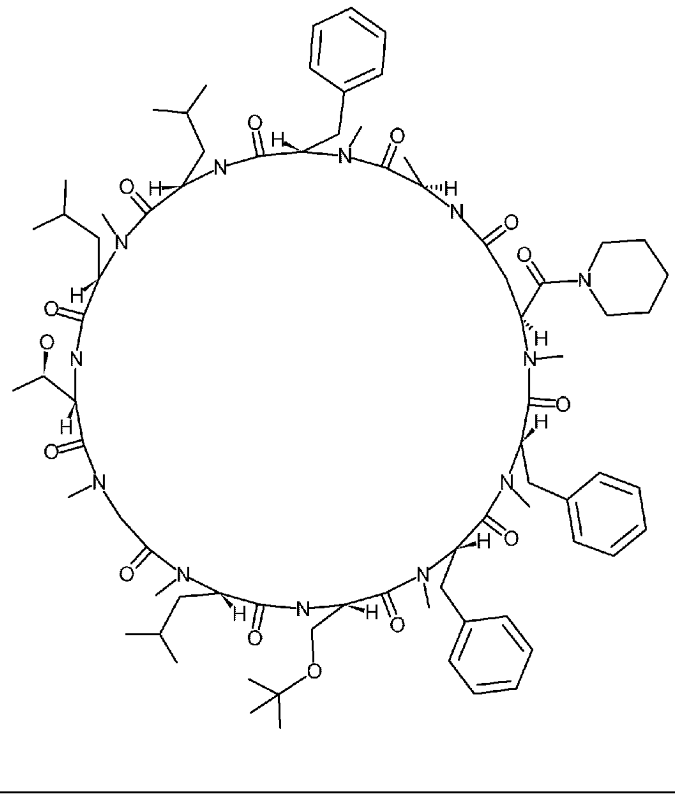 |

| ID | structure |
|---|---|
| pd225 | 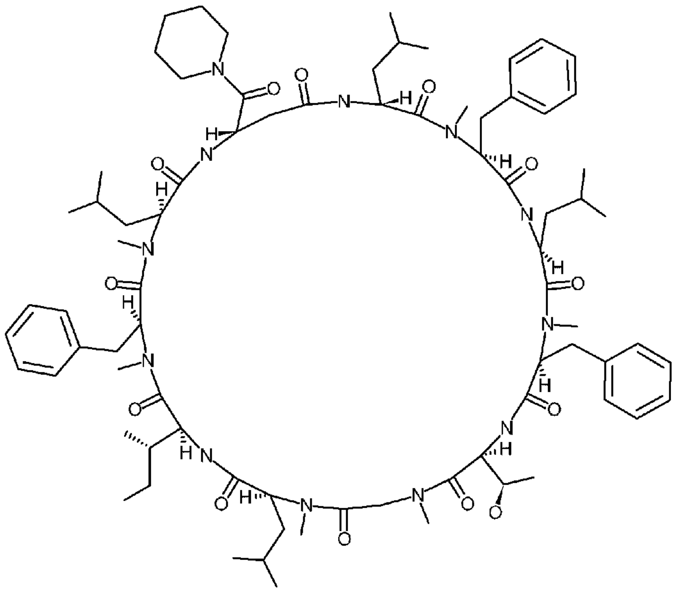 |
| pd226 | 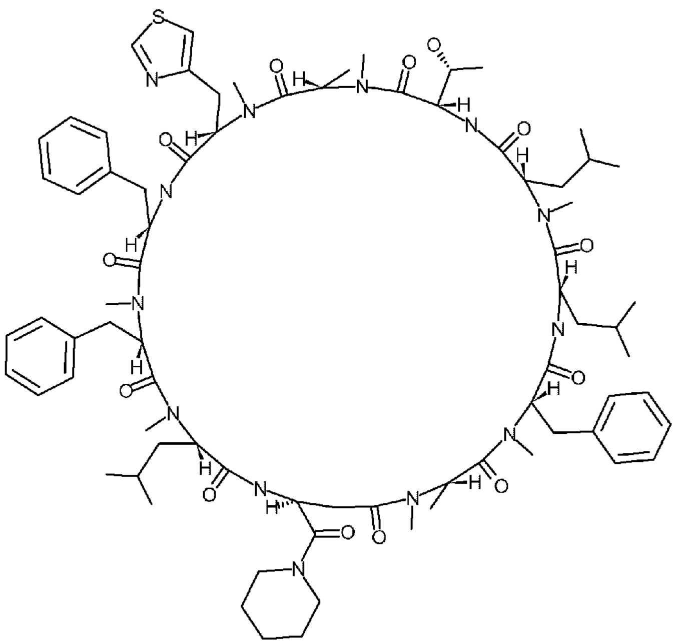 |

| ID | structure |
|---|---|
| pd227 | 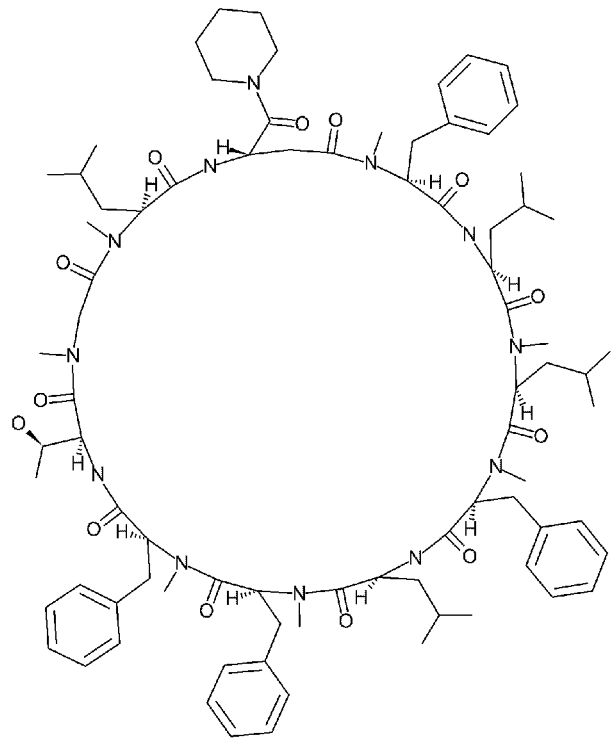 |
| pd228 | 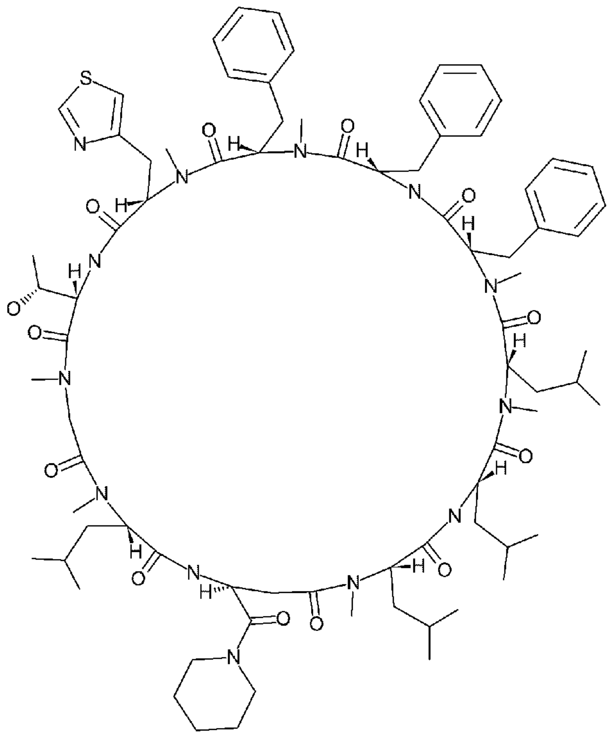 |

| ID | structure |
|---|---|
| pd229 | 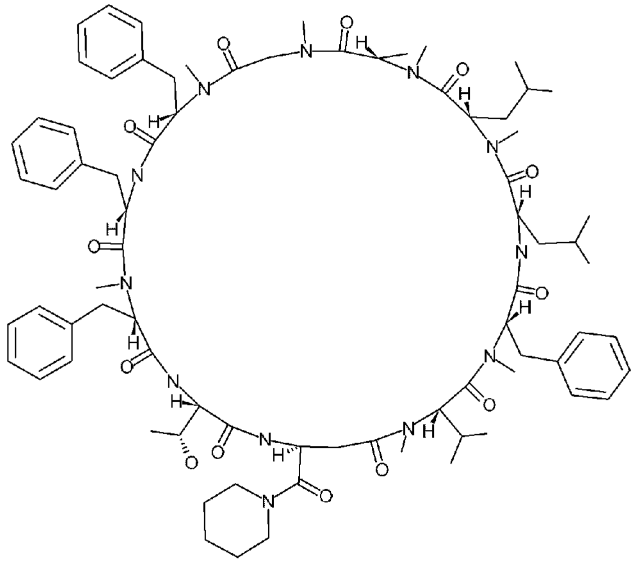 |
| pd230 | 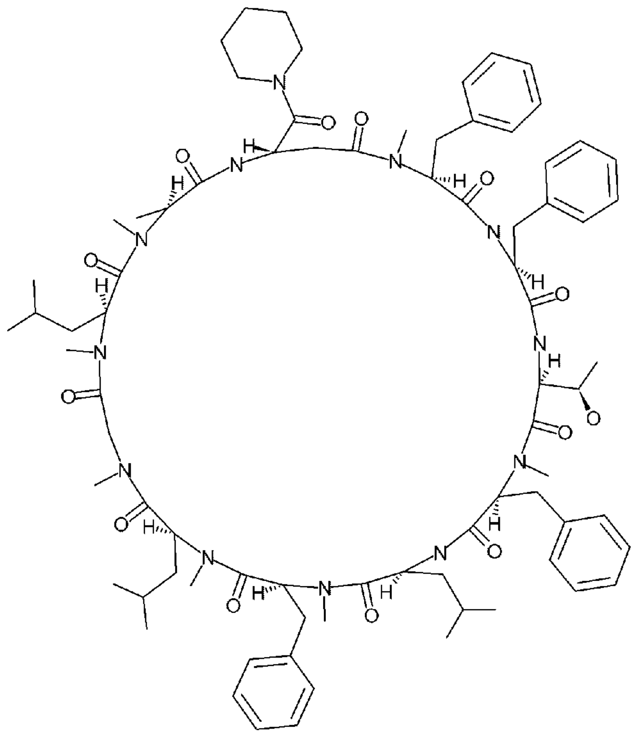 |

| ID | structure |
|---|---|
| pd231 | 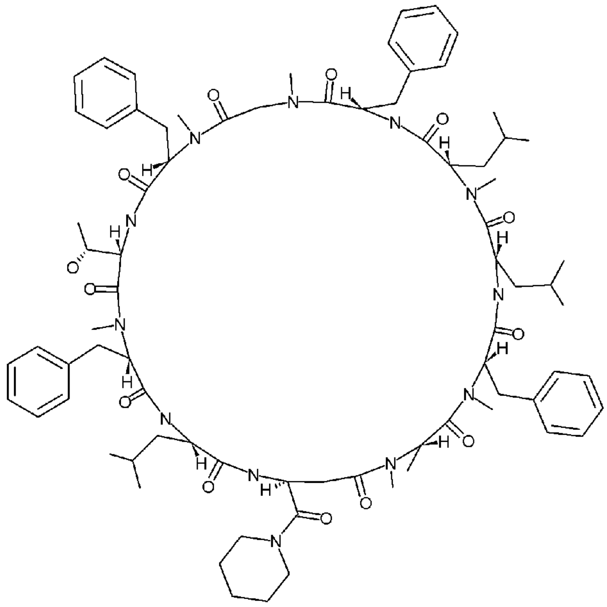 |
| pd232 | 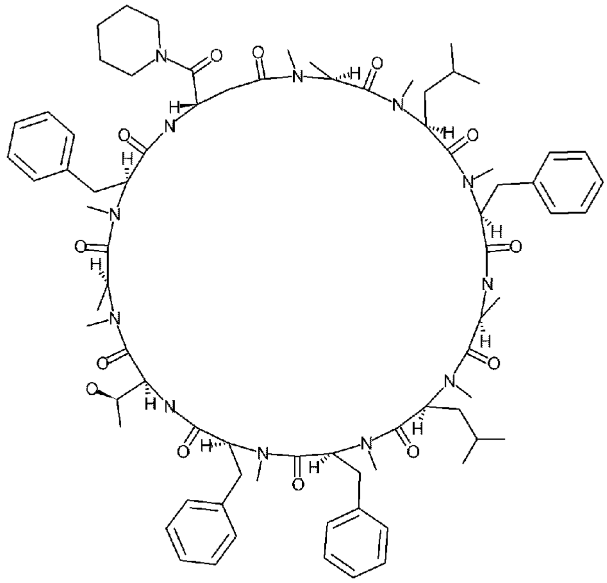 |

| ID | structure |
| --- | --- |
| pd233 | 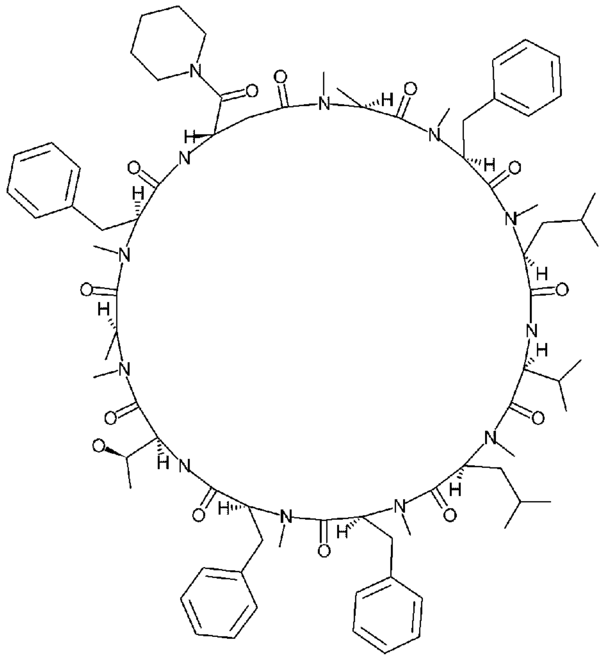 |
| pd234 | 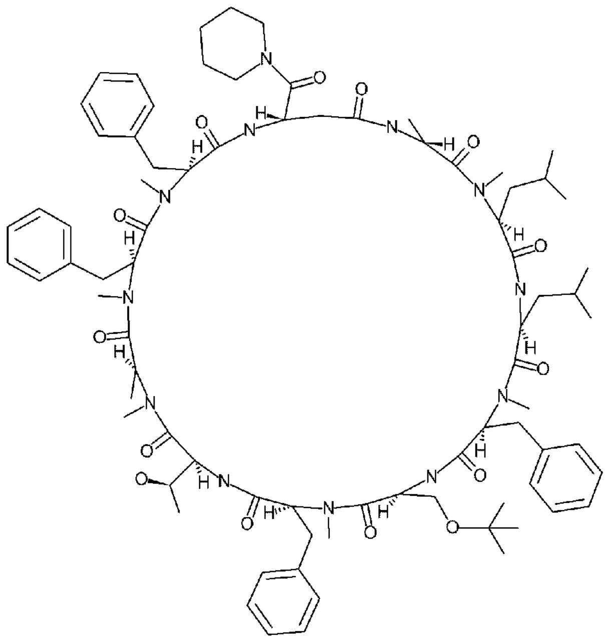 |

| ID | structure |
| --- | --- |
| pd235 | 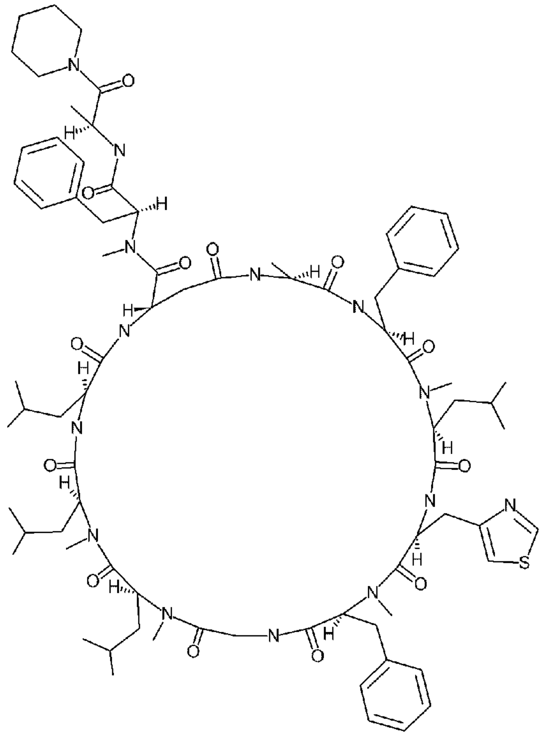 |
| pd236 | 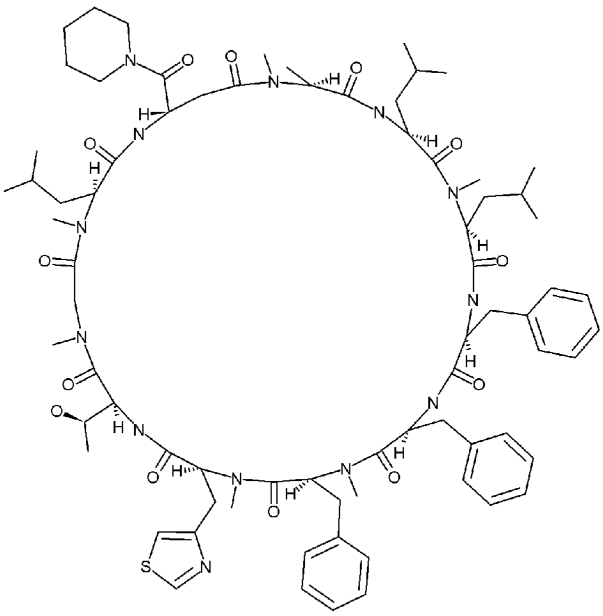 |

| ID | structure |
|---|---|
| pd237 | 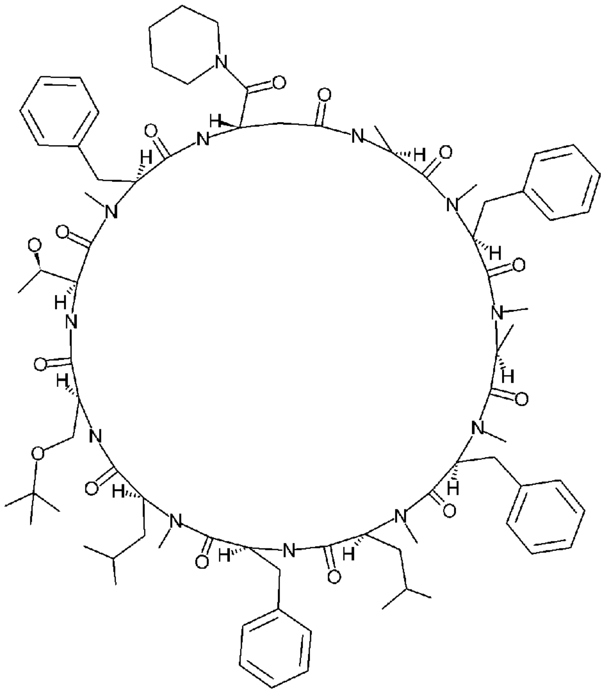 |
| pd238 | 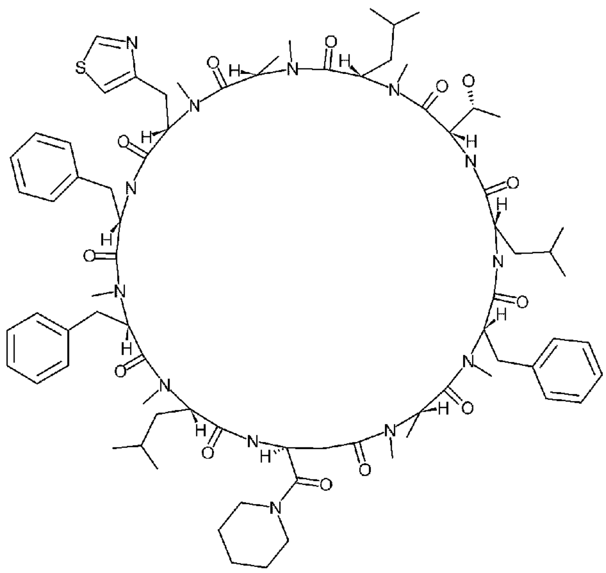 |

| ID | structure |
|---|---|
| pd239 | 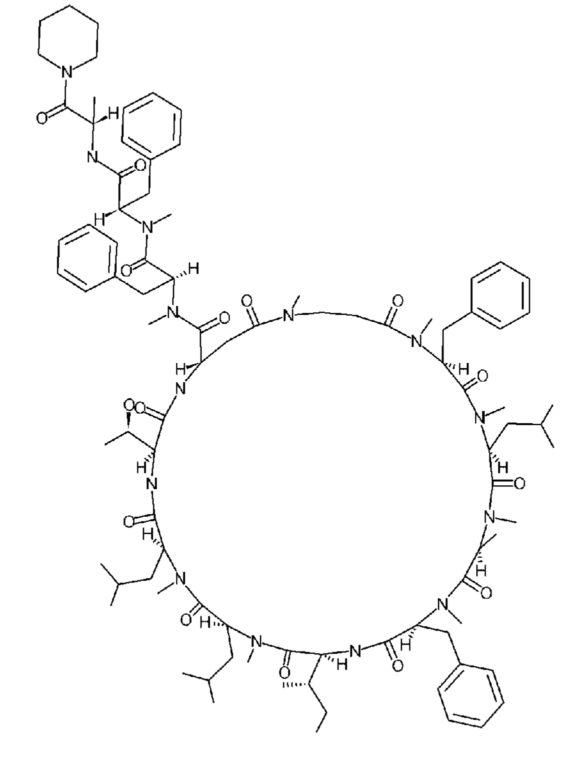 |
| pd240 | 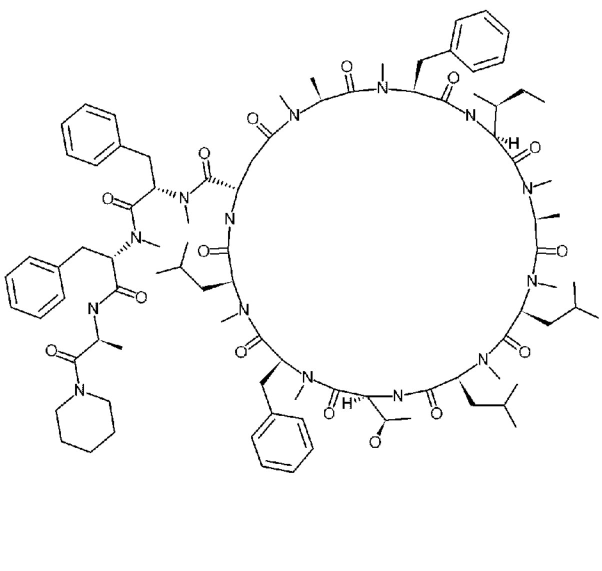 |

| ID | structure |
|---|---|
| pd241 | 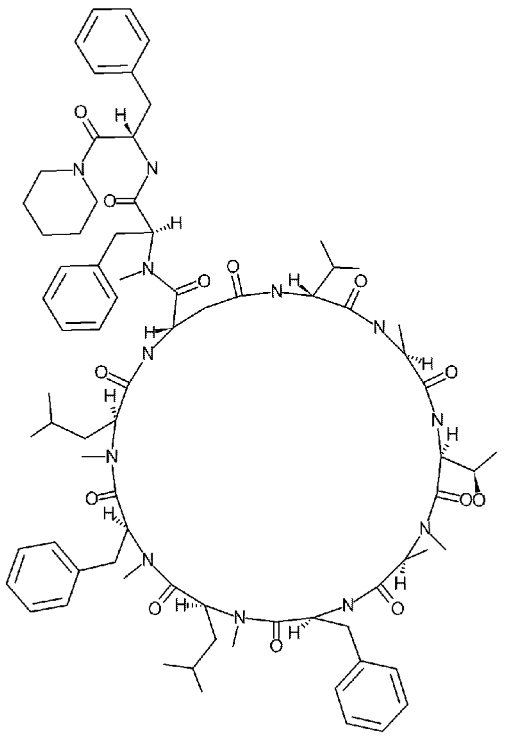 |
| pd242 | 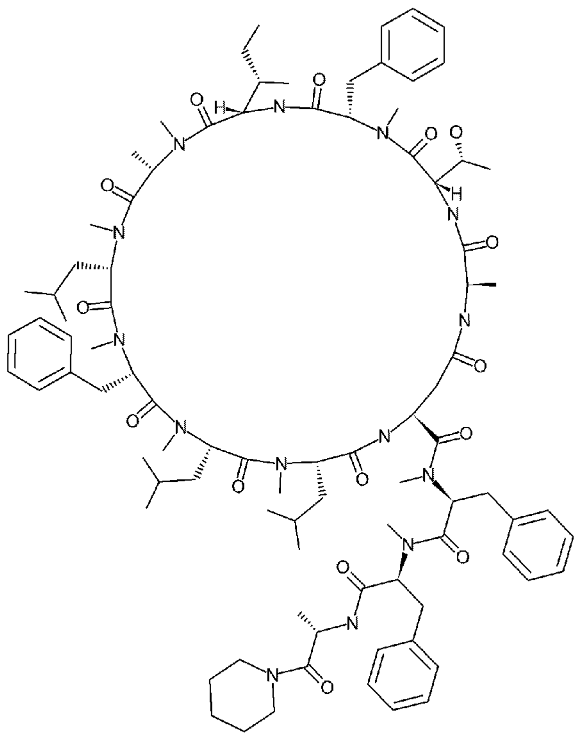 |

| ID | structure |
|---|---|
| pd243 | 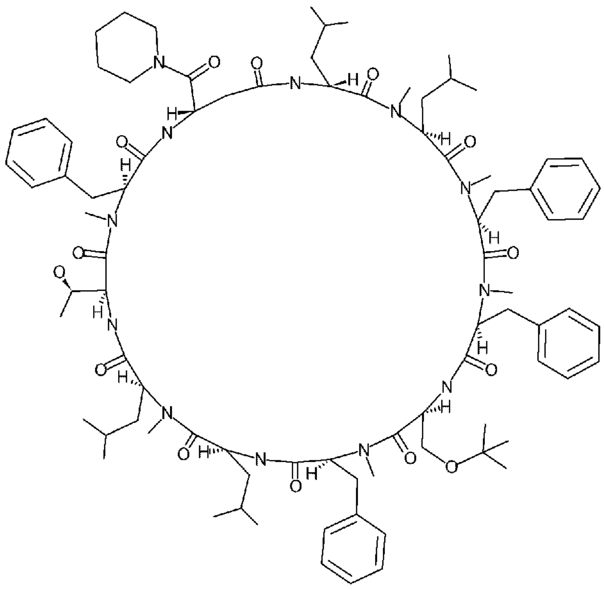 |
| pd244 | 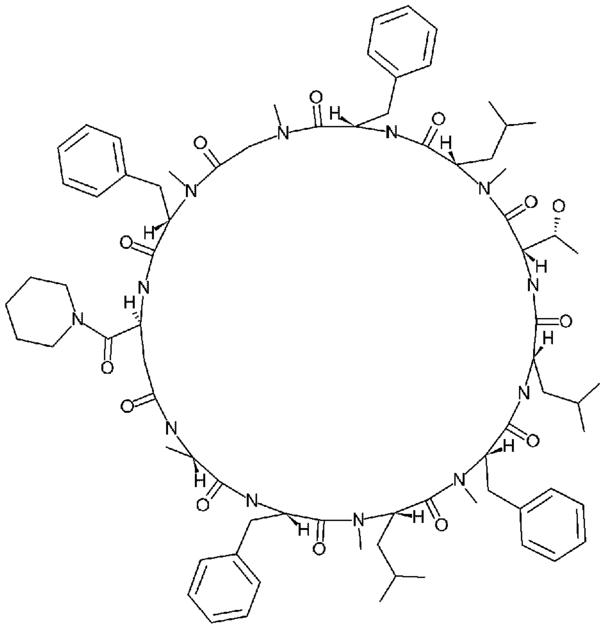 |

| ID | structure |
|---|---|
| pd245 | 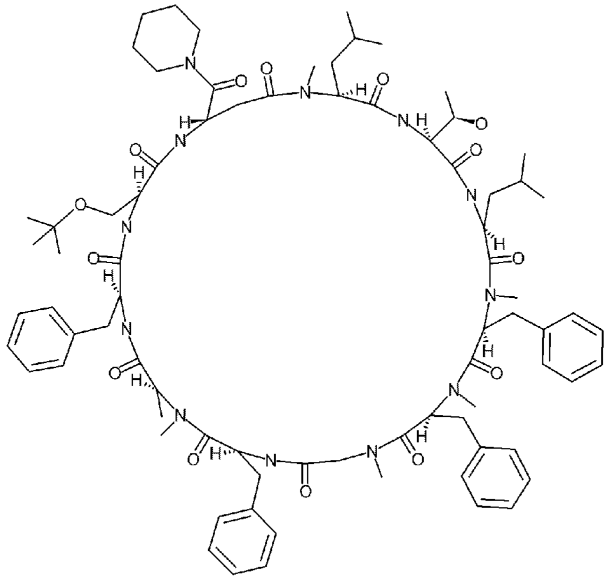 |
| pd246 | 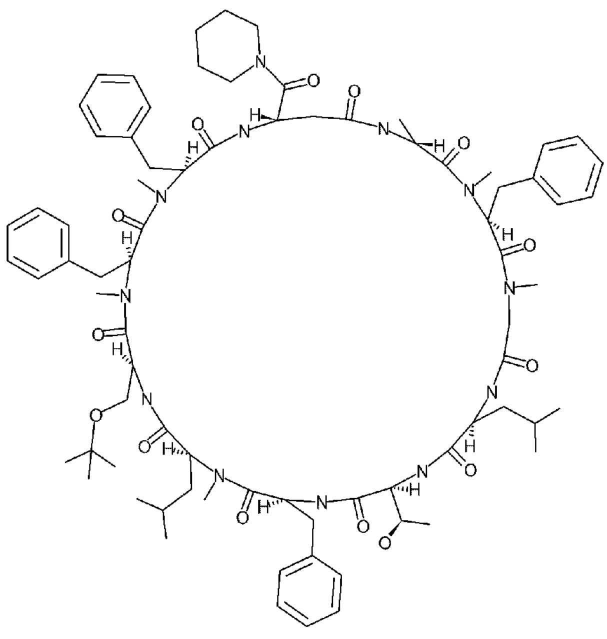 |

| ID | structure |
| --- | --- |
| pd247 | 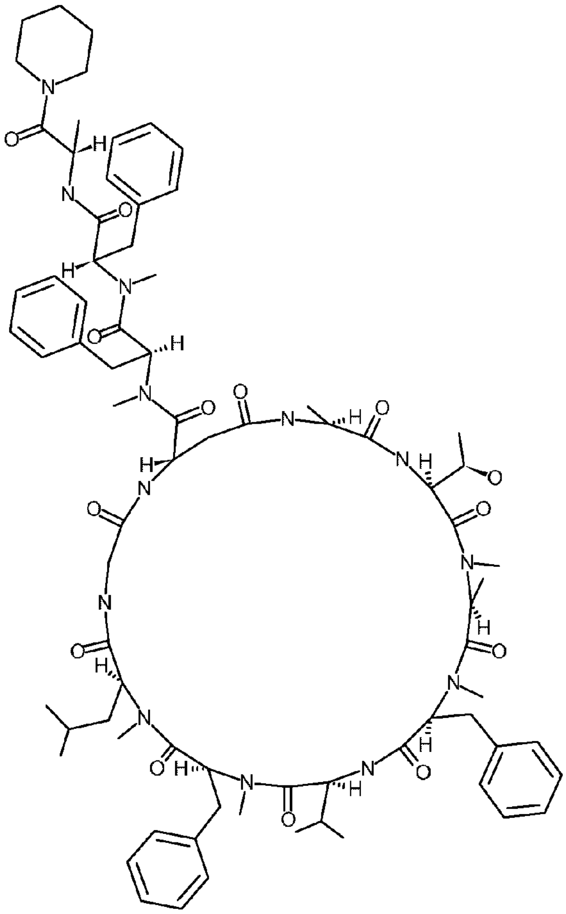 |

| ID | structure |
|---|---|
| pd300 | 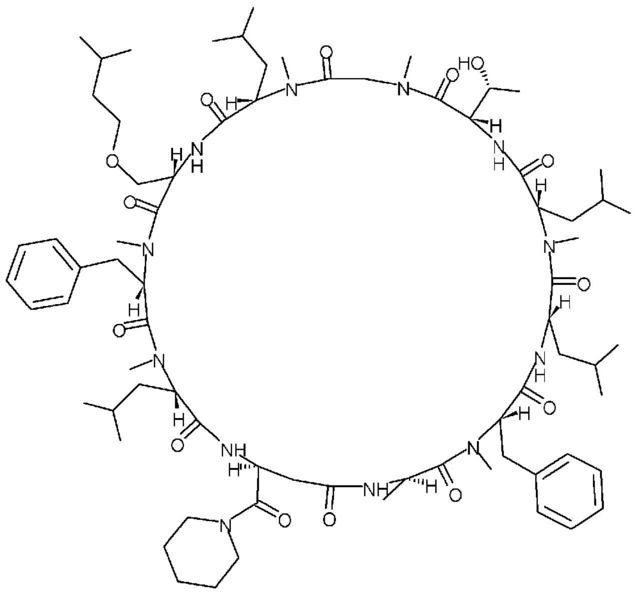 |
| pd301 | 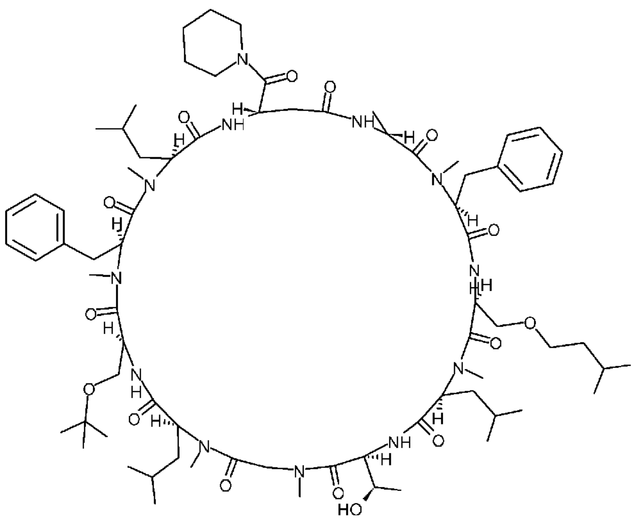 |

| ID | structure |
|---|---|
| pd302 | 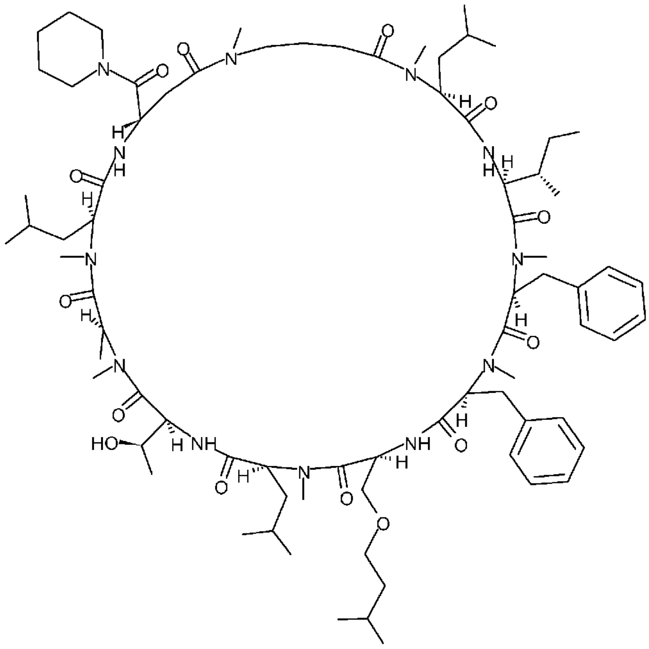 |
| pd303 | 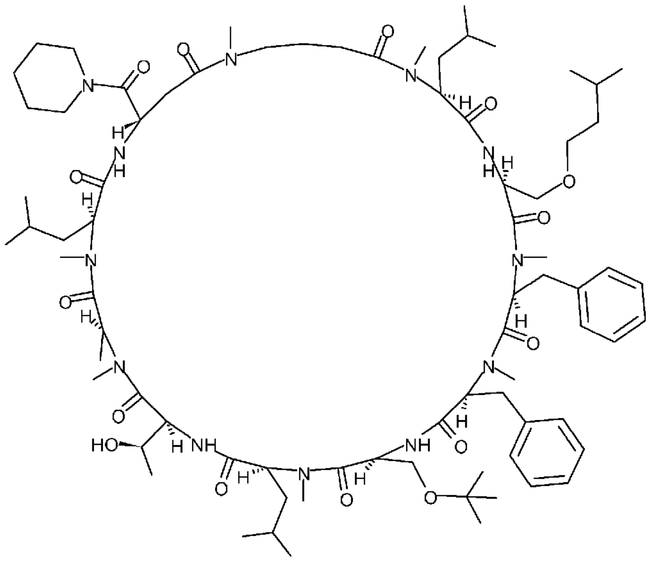 |

| ID | structure |
|---|---|
| pd304 | 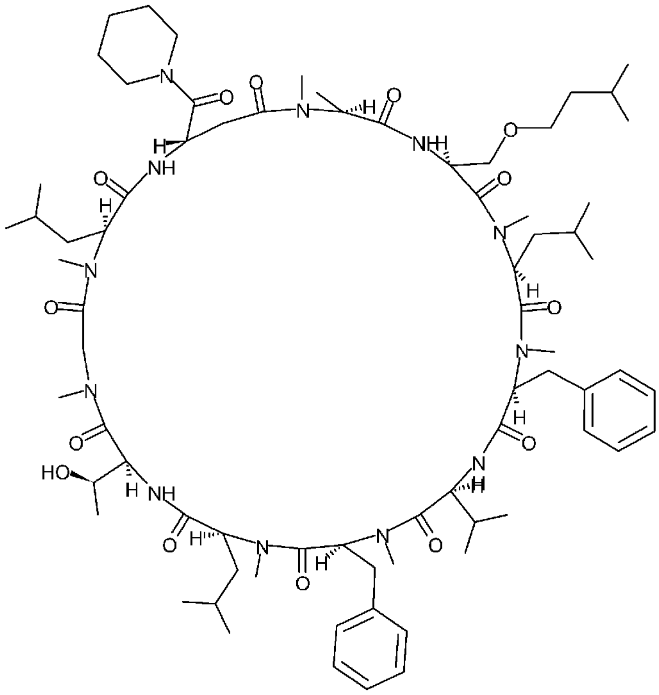 |
| pd305 | 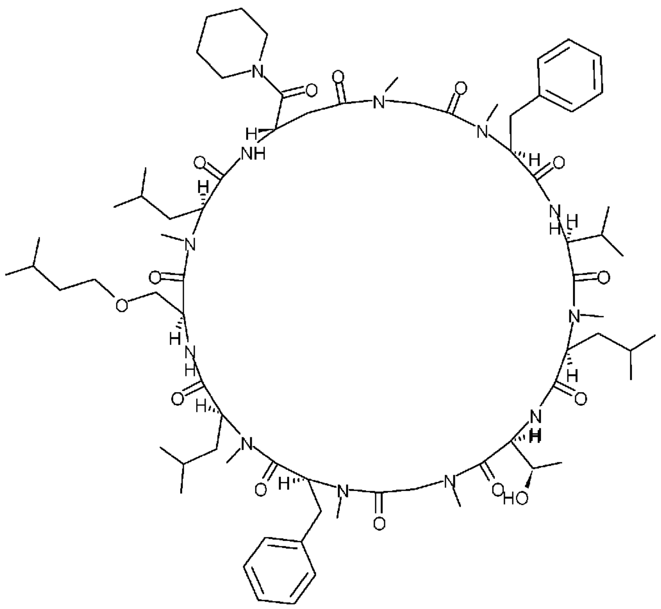 |

| ID | structure |
|---|---|
| pd306 | 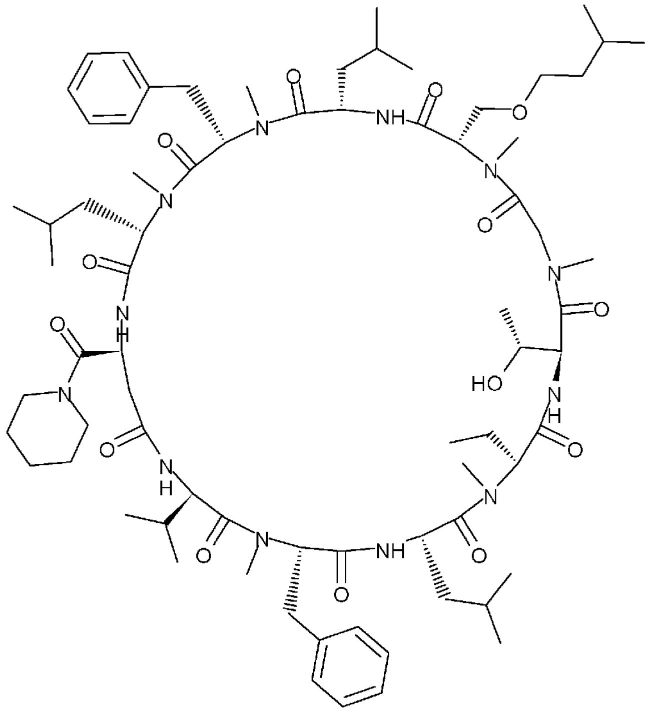 |
| pd307 | 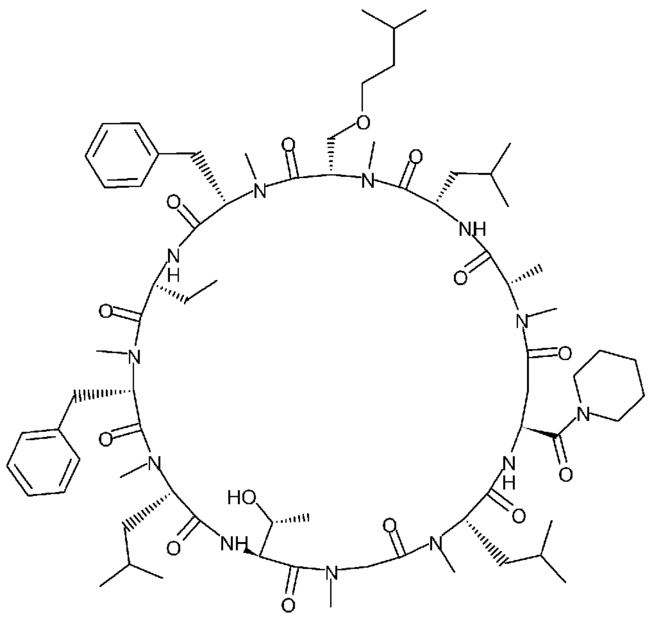 |

| ID | structure |
|---|---|
| pd308 | 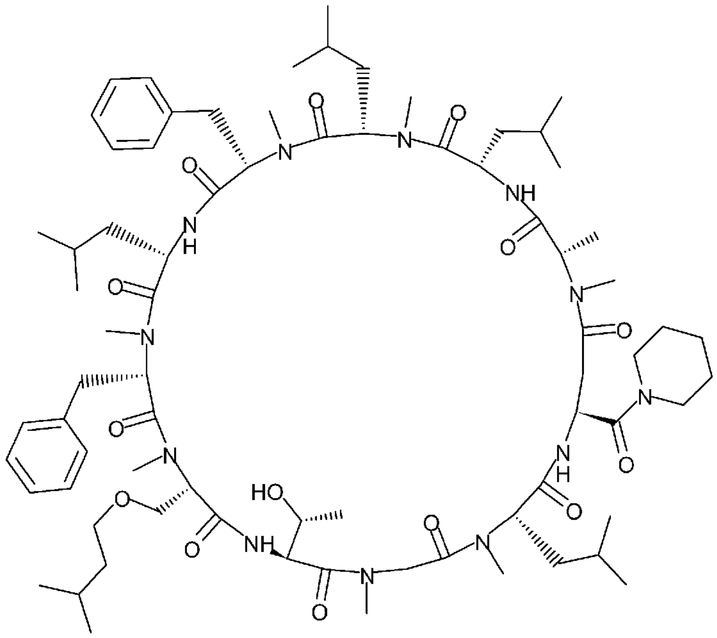 |
| pd309 | 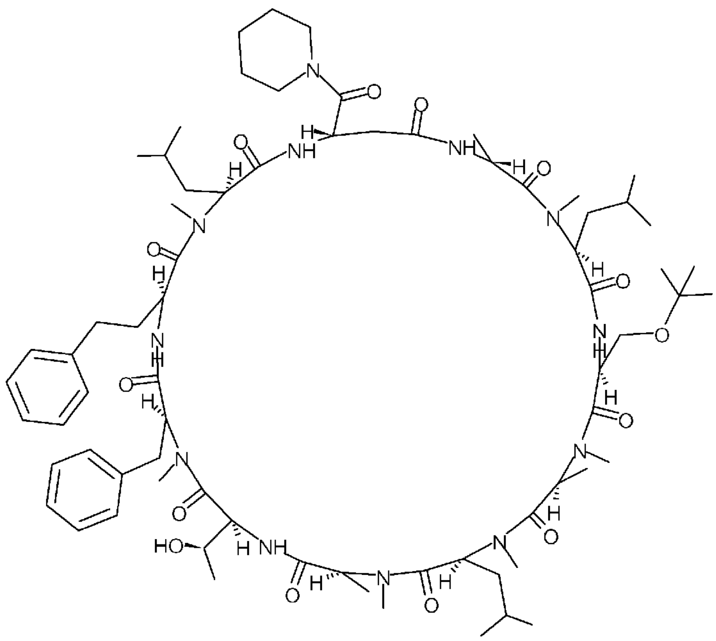 |

| ID | structure |
|---|---|
| pd310 | 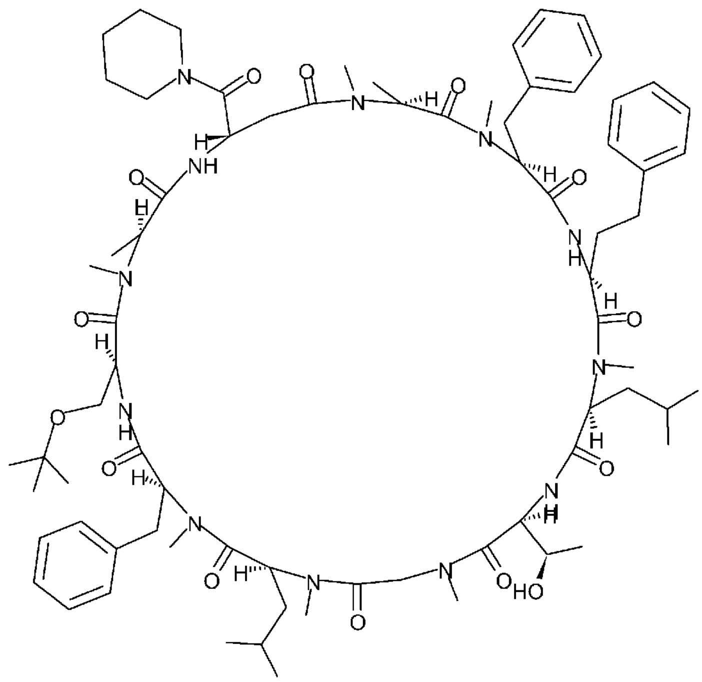 |
| pd311 | 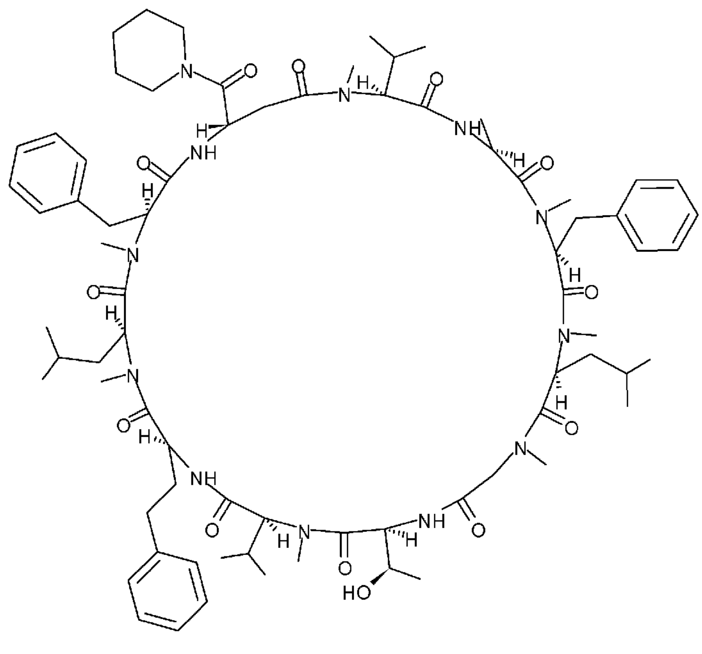 |

| ID | structure |
|---|---|
| pd312 | 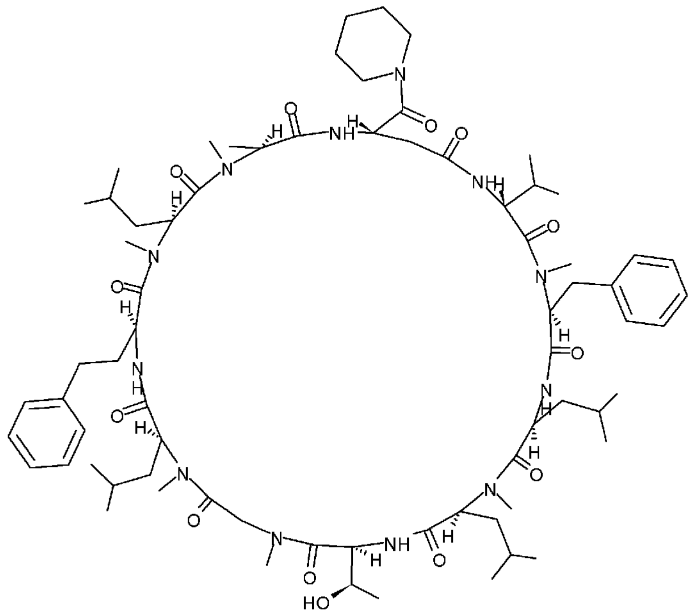 |
| pd313 | 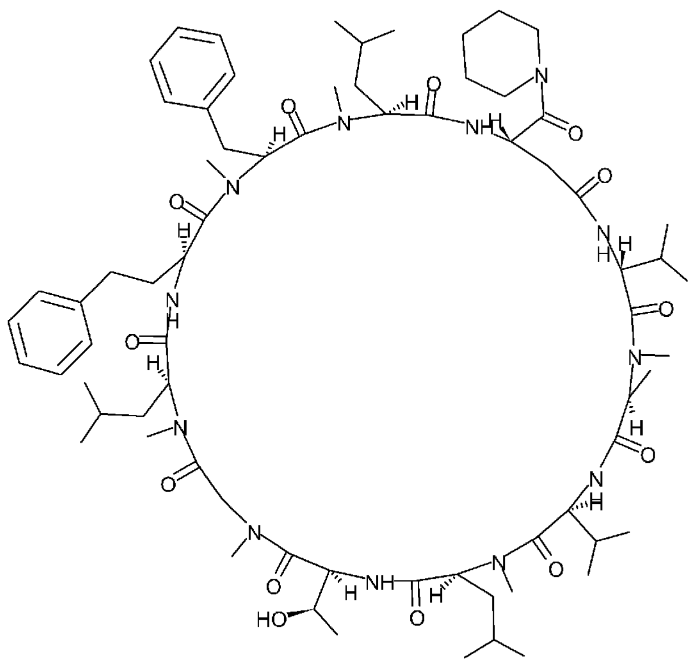 |

| ID | structure |
|---|---|
| pd314 | 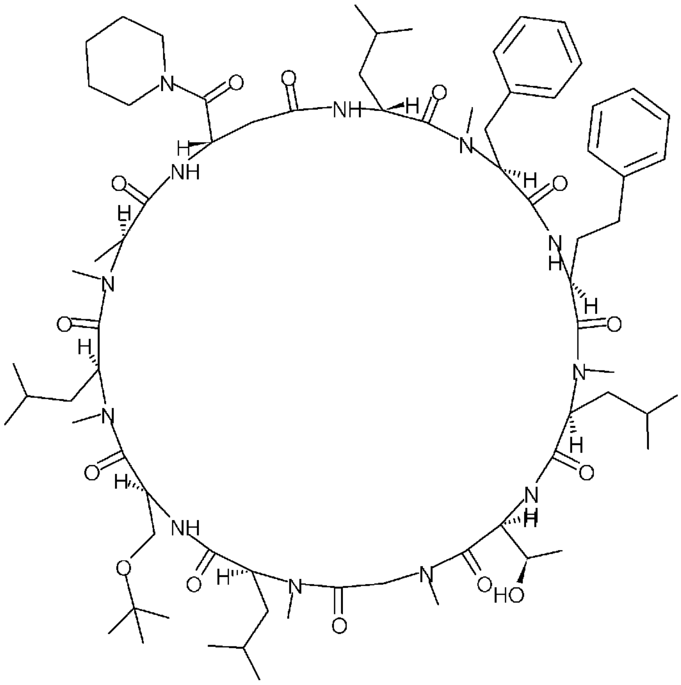 |
| pd315 | 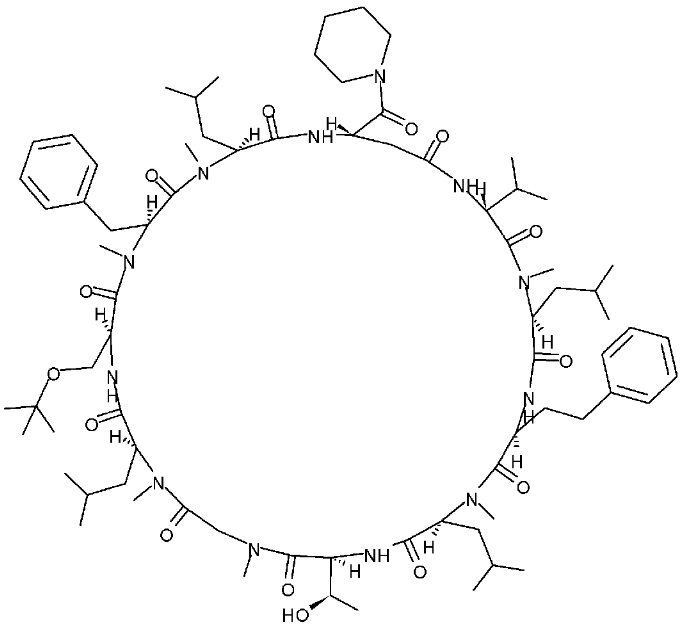 |

| ID | structure |
|---|---|
| pd316 | 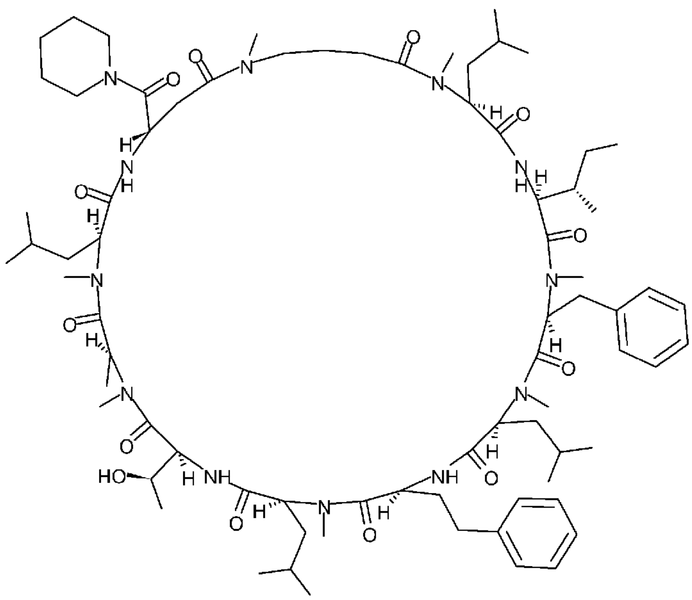 |
| pd317 | 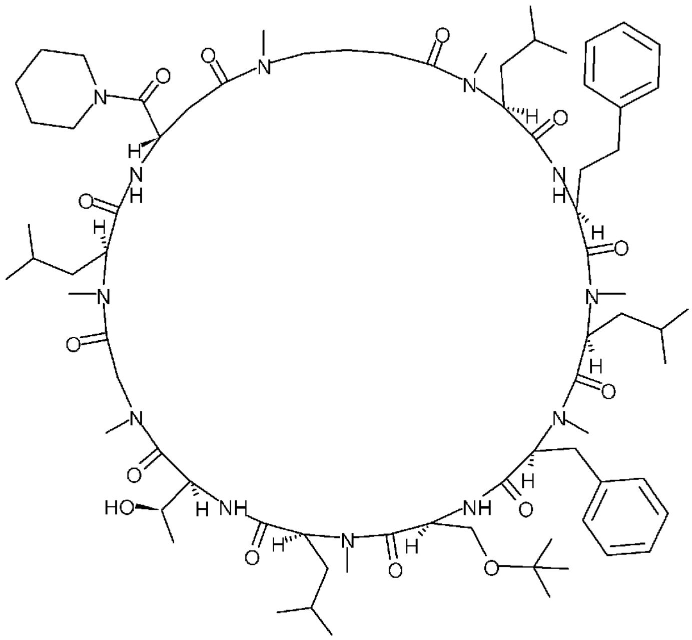 |

| ID | structure |
|---|---|
| pd318 | 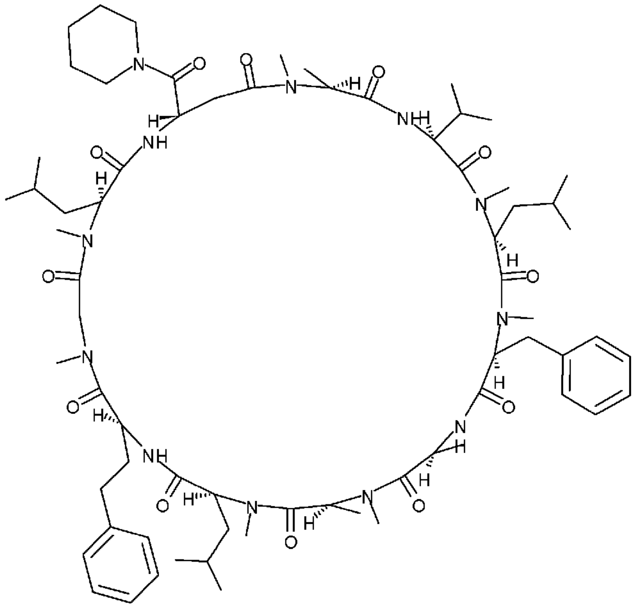 |
| pd319 | 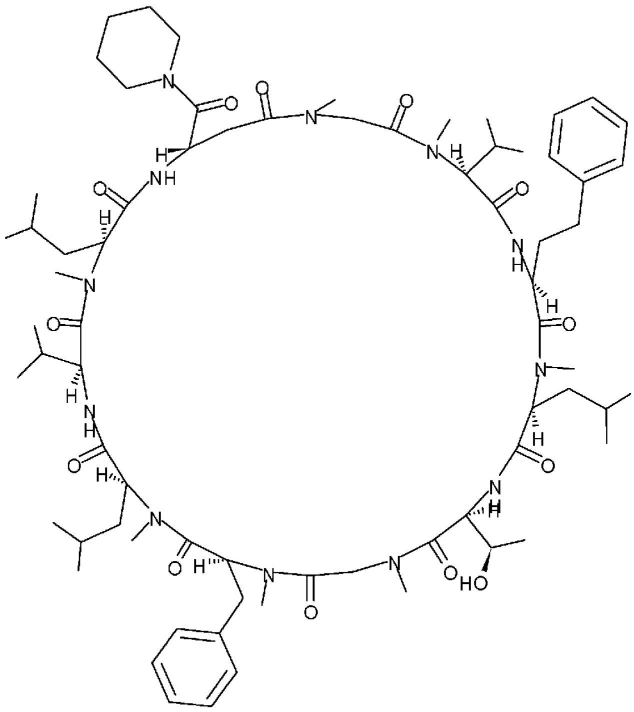 |

| ID | structure |
|---|---|
| pd320 | 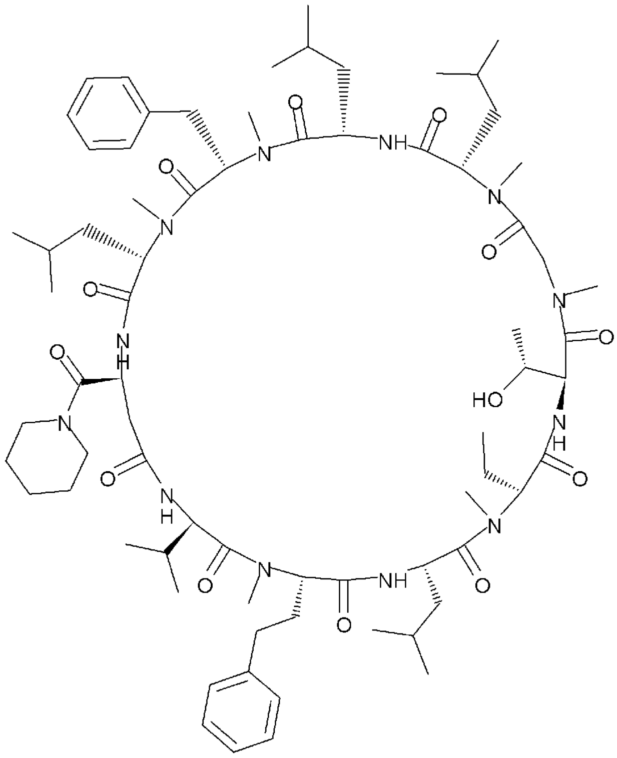 |
| pd321 | 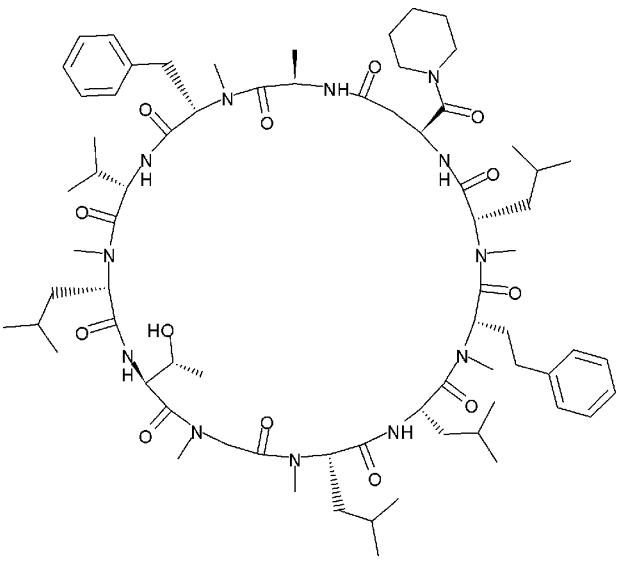 |

| ID | structure |
|---|---|
| pd322 | 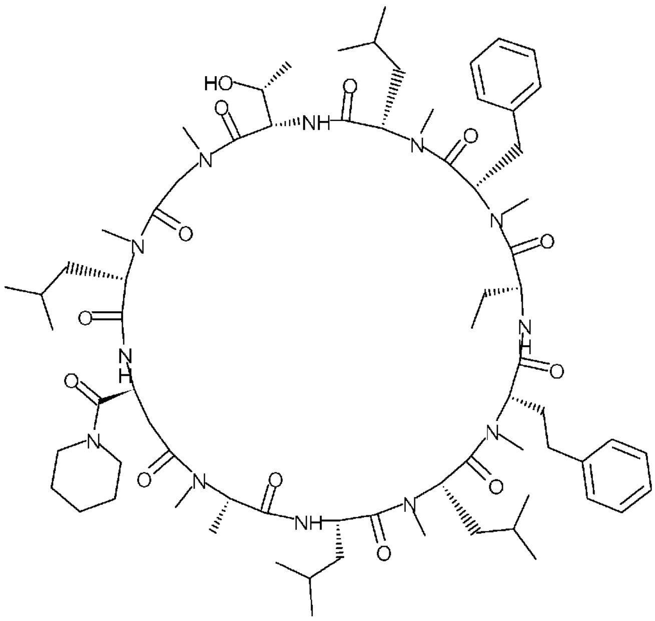 |
| pd323 | 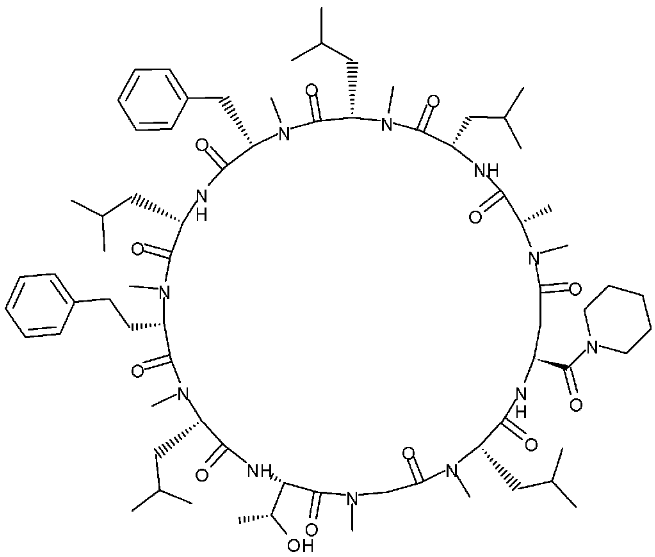 |

| ID | structure |
|---|---|
| pd324 | 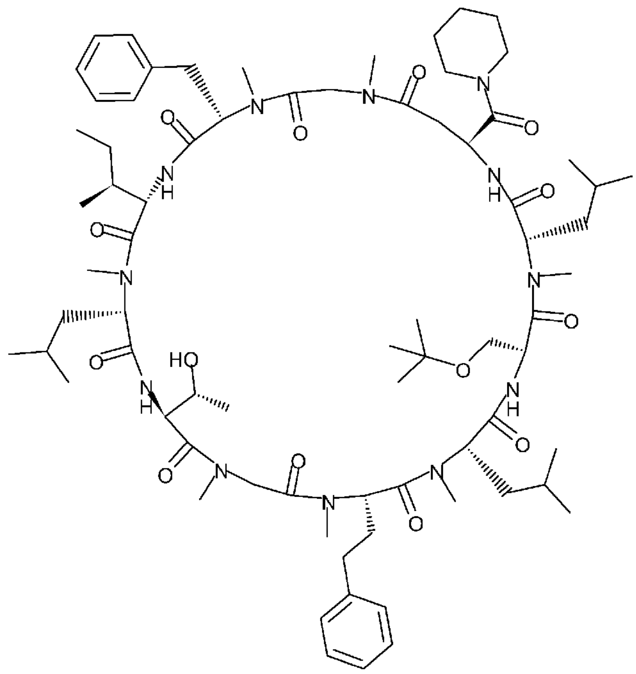 |
| pd325 | 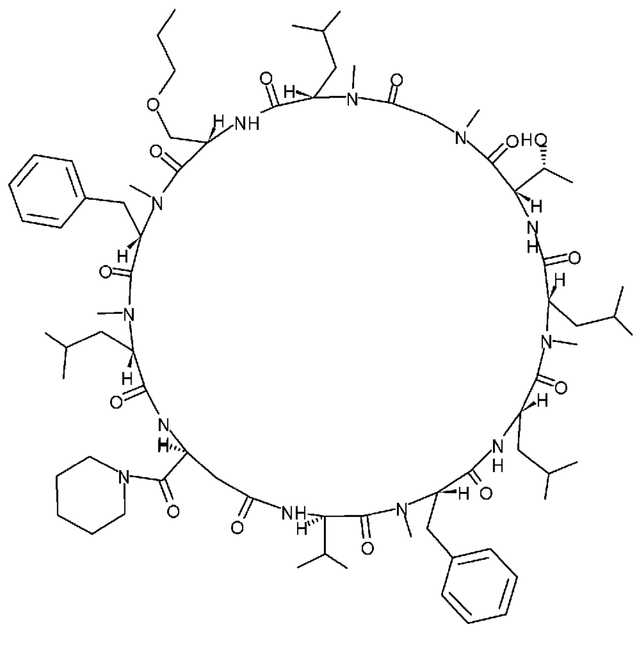 |

| ID | structure |
| --- | --- |
| pd326 | 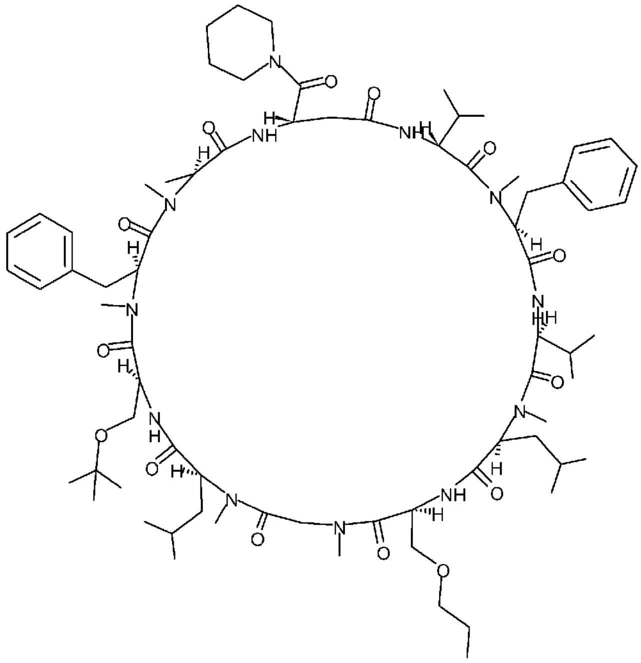 |
| pd327 | 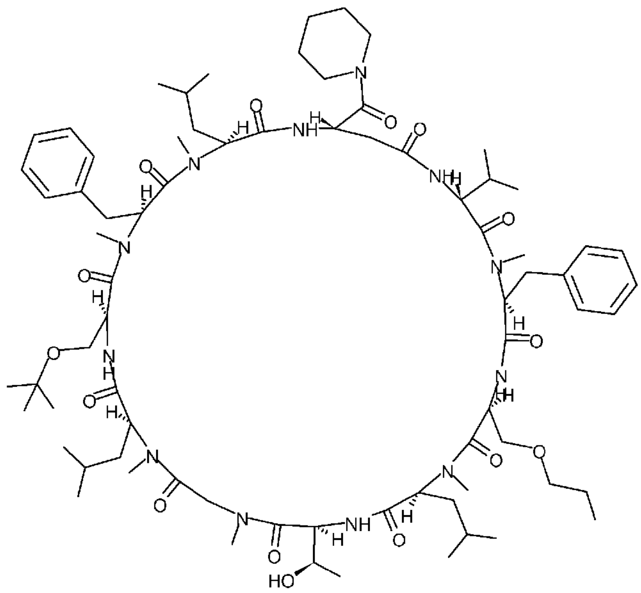 |

| ID | structure |
|---|---|
| pd328 | 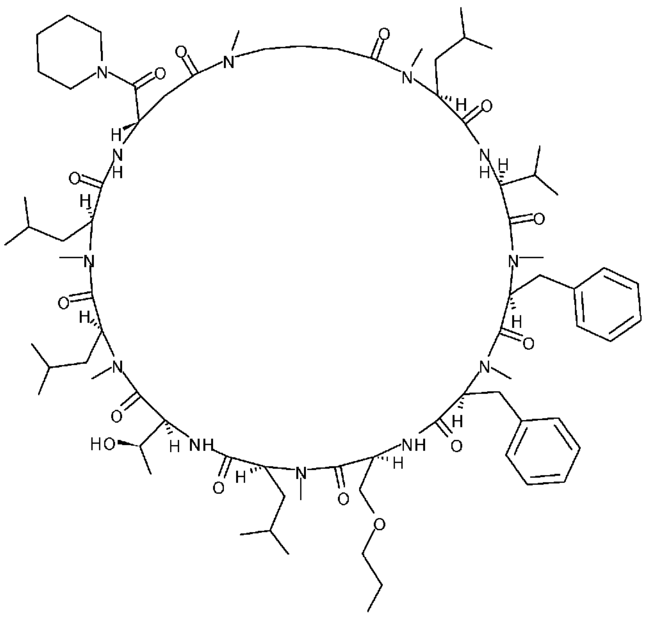 |
| pd329 | 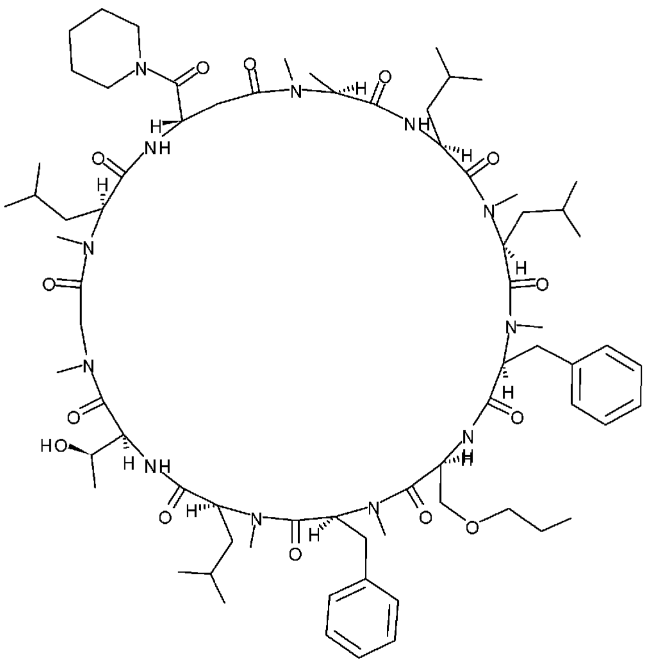 |

| ID | structure |
|---|---|
| pd330 | 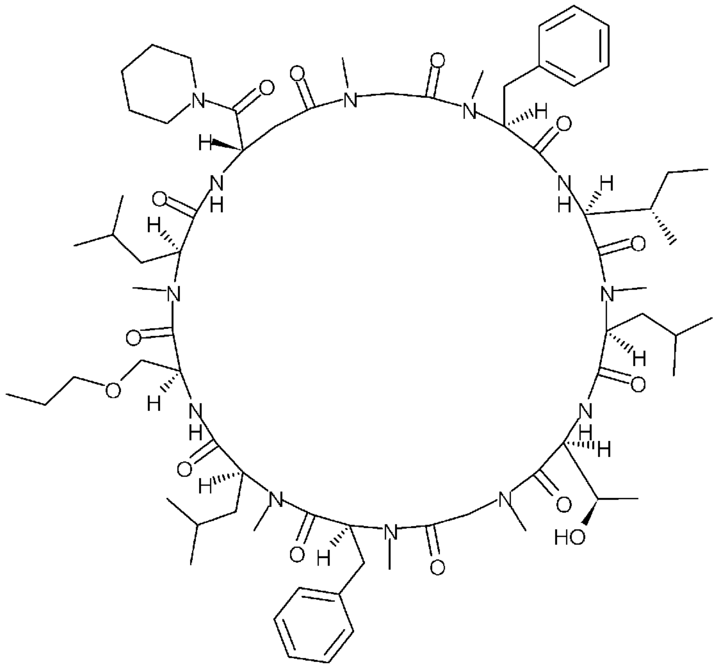 |
| pd331 | 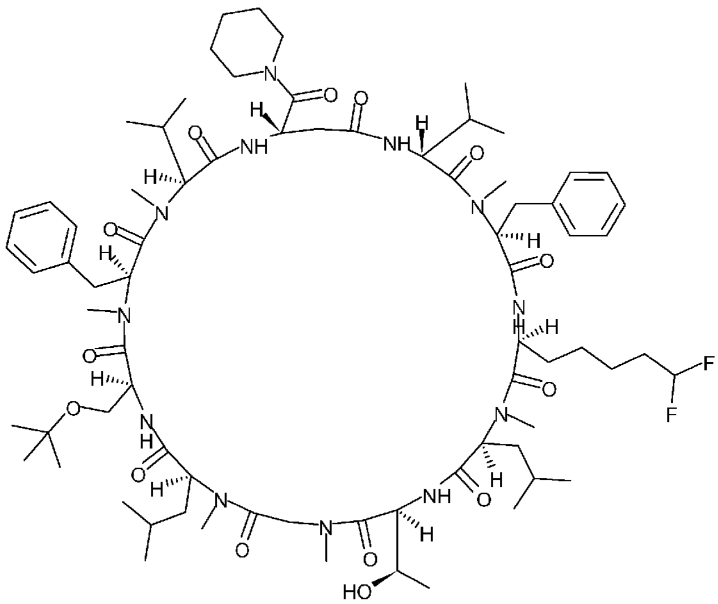 |

| ID | structure |
|---|---|
| pd332 | 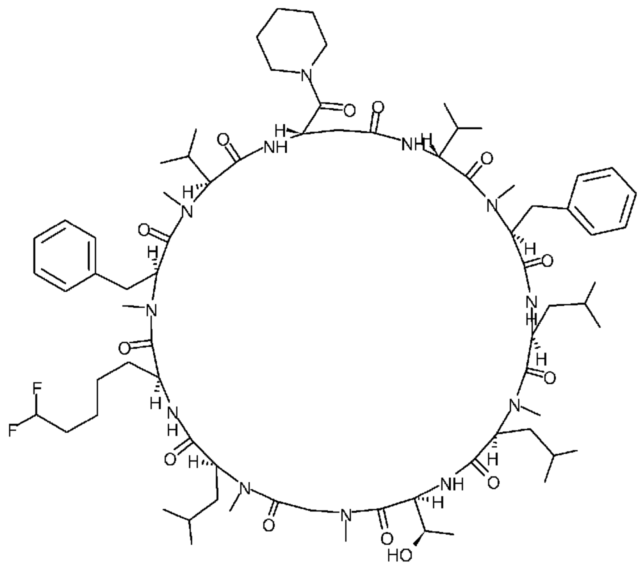 |
| pd333 | 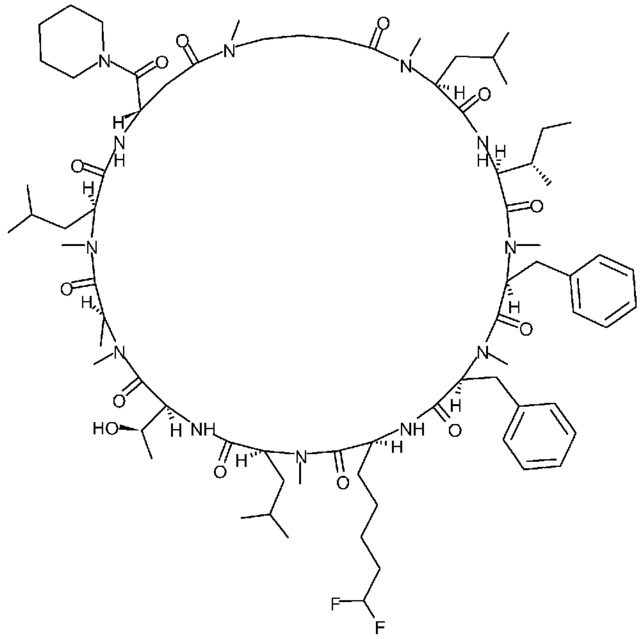 |

| ID | structure |
|---|---|
| pd334 | 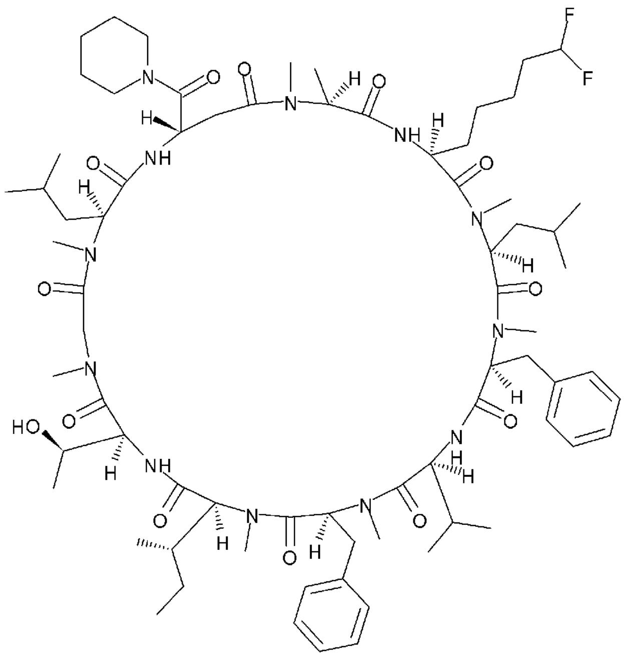 |
| pd335 | 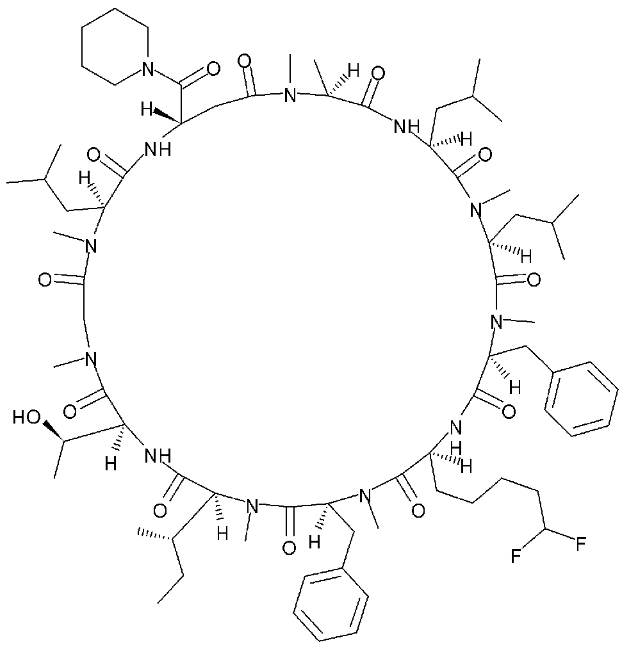 |

| ID | structure |
|---|---|
| pd336 | 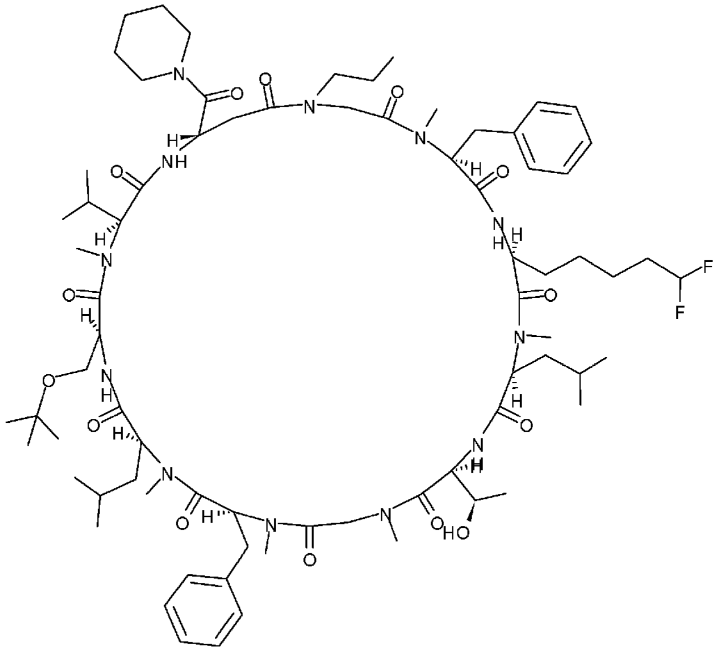 |
| pd337 | 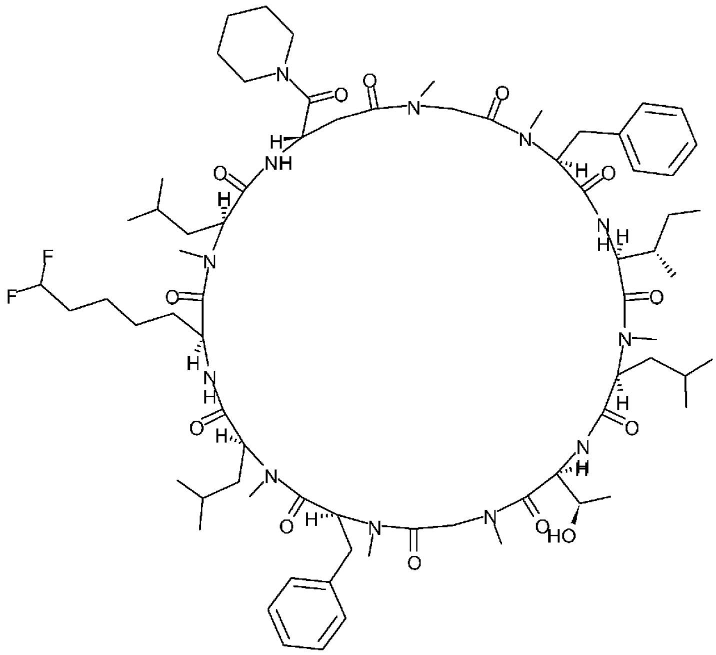 |

| ID | structure |
|---|---|
| pd338 | 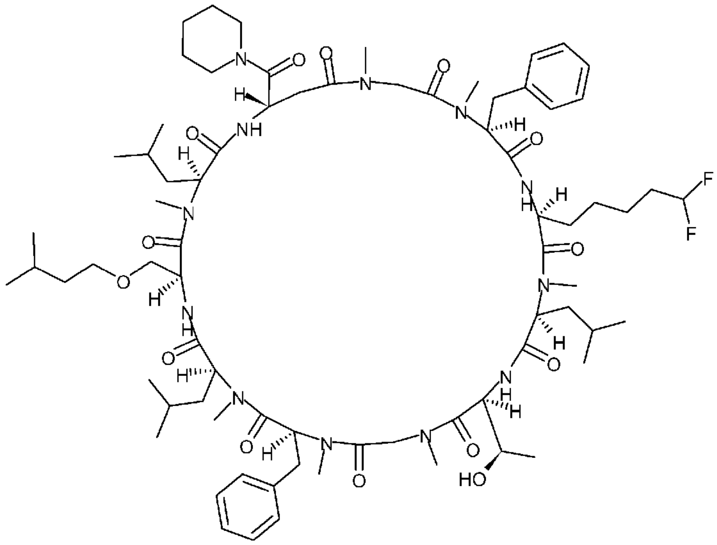 |
| pd339 | 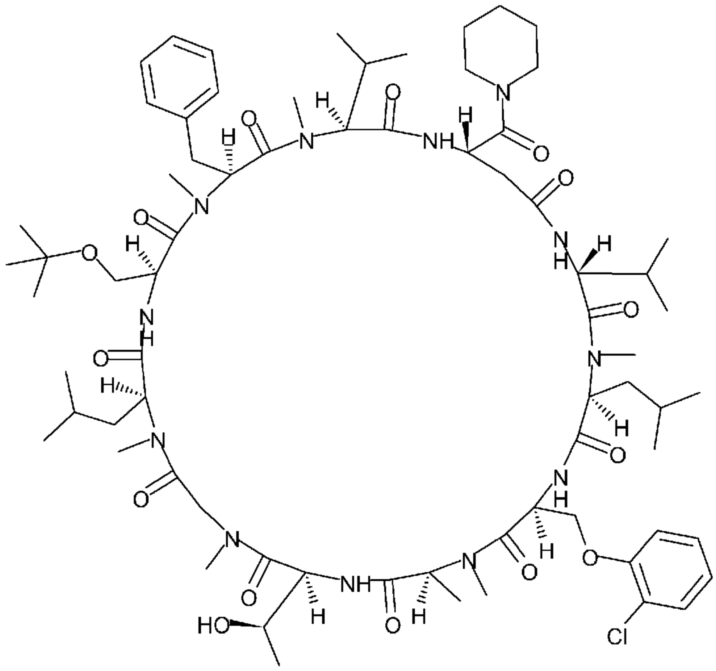 |

| ID | structure |
|---|---|
| pd340 | 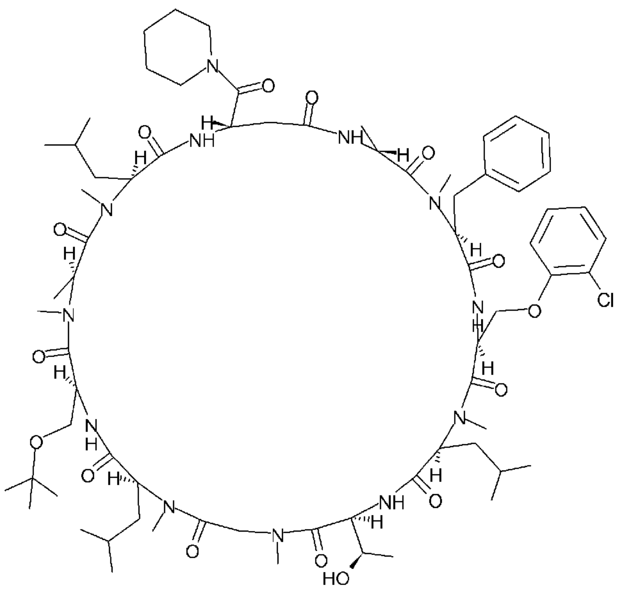 |
| pd341 | 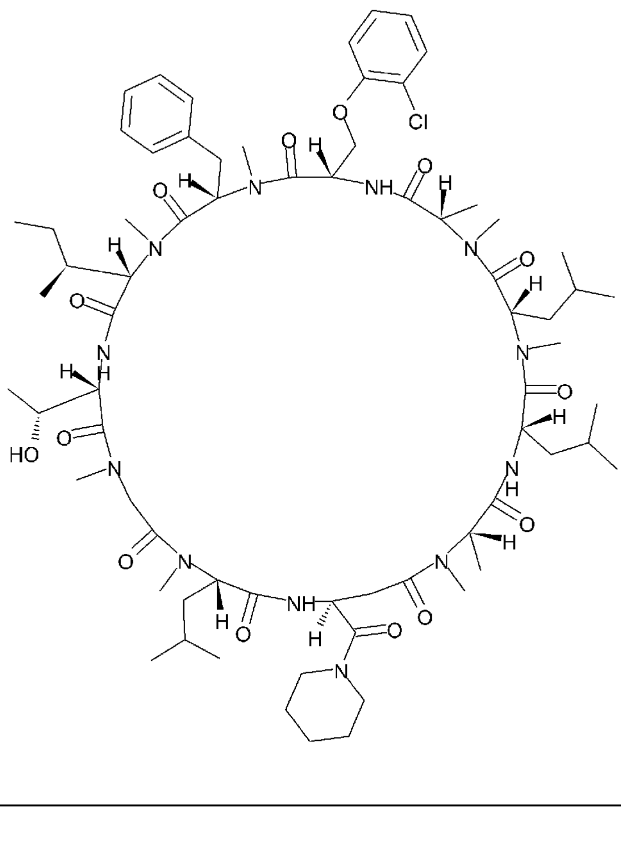 |

| ID | structure |
|---|---|
| pd342 | 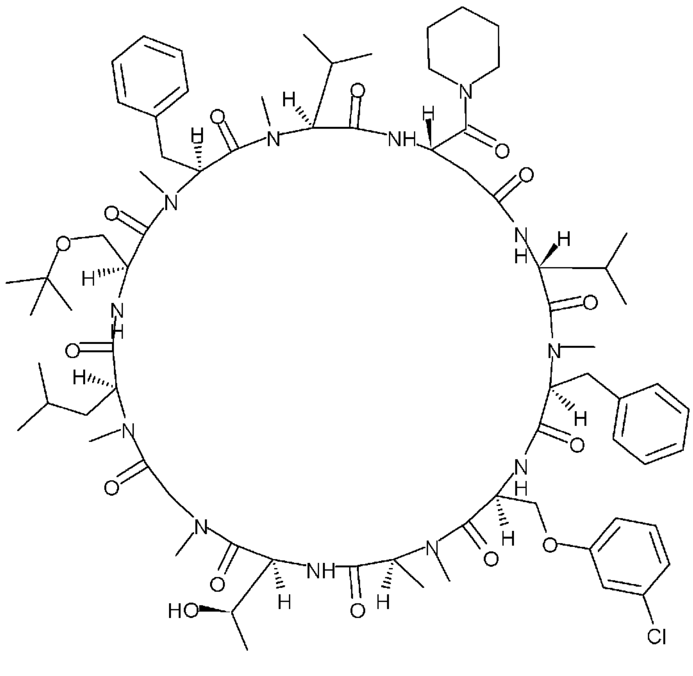 |
| pd343 | 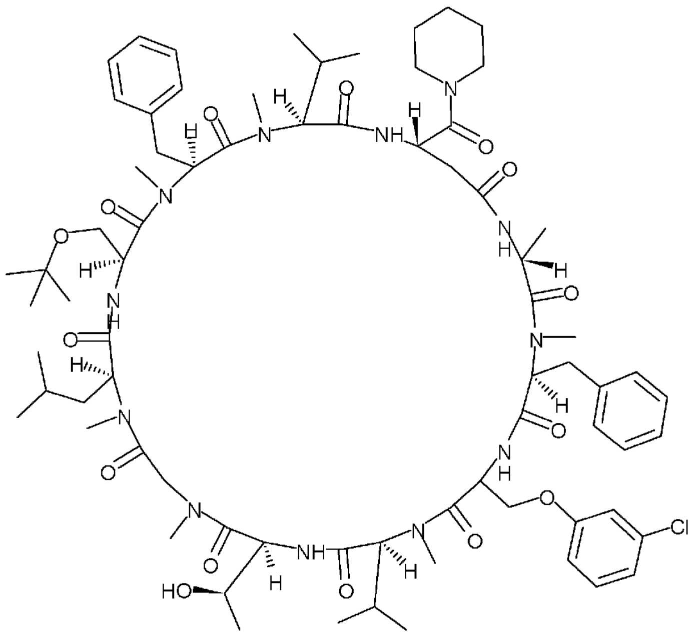 |

| ID | structure |
|---|---|
| pd344 | 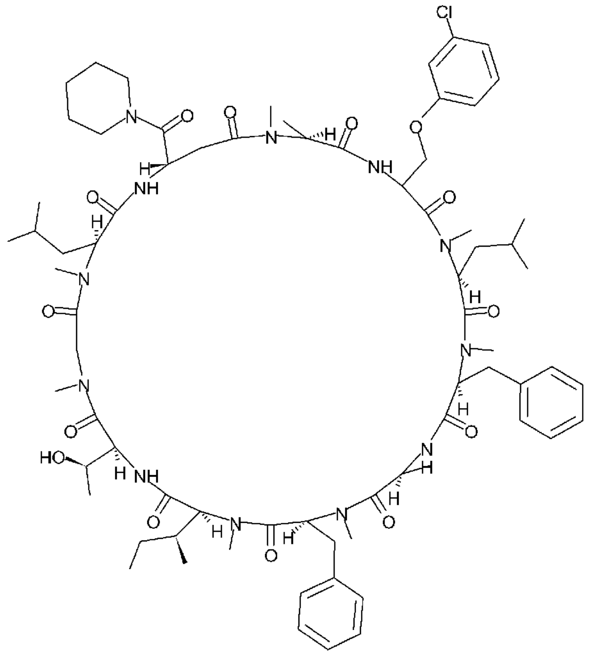 |
| pd345 | 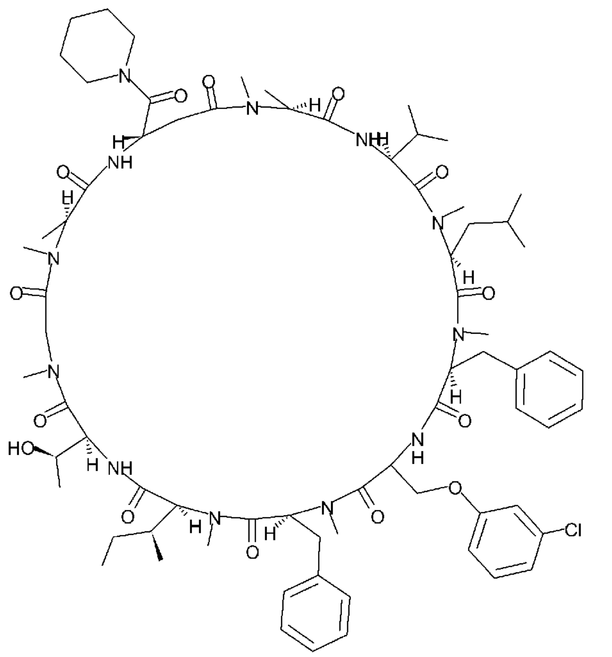 |

| ID | structure |
|---|---|
| pd346 | 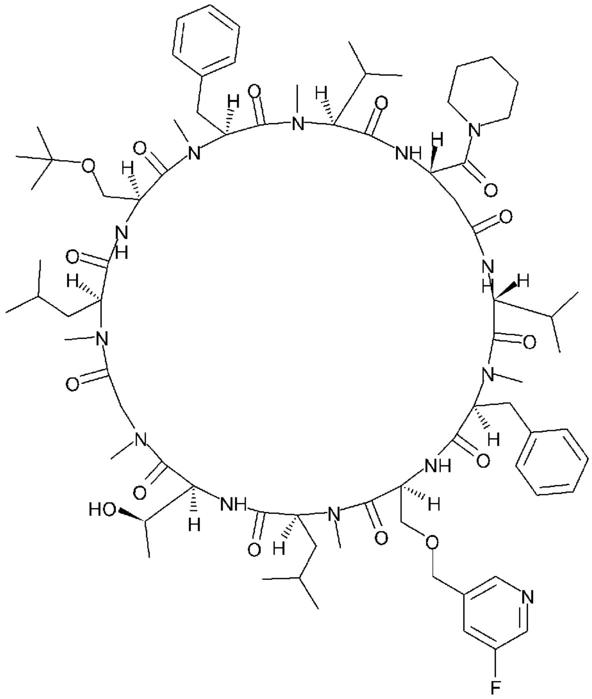 |
| pd347 | 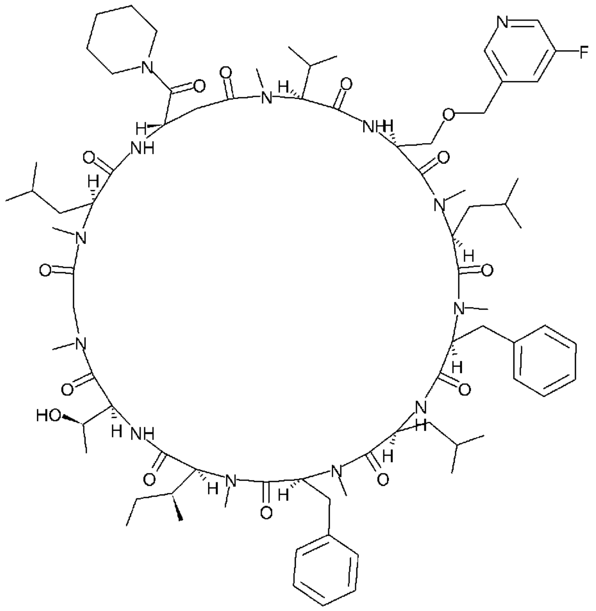 |

| ID | Structure |
|---|---|
| pd348 | 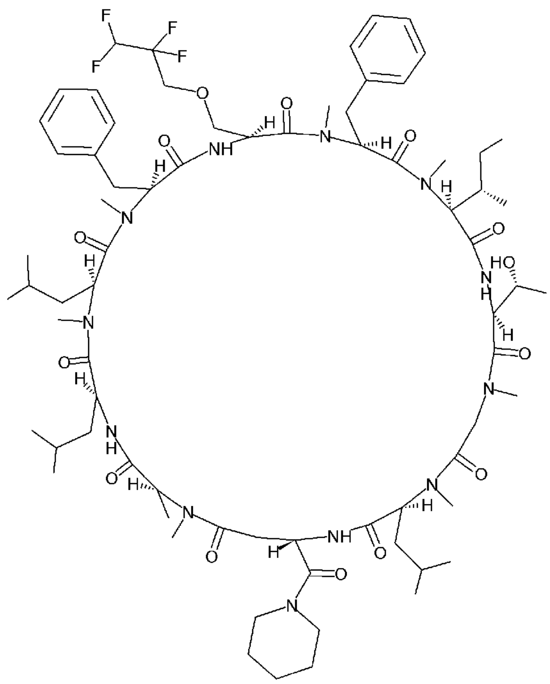 |
| pd349 | 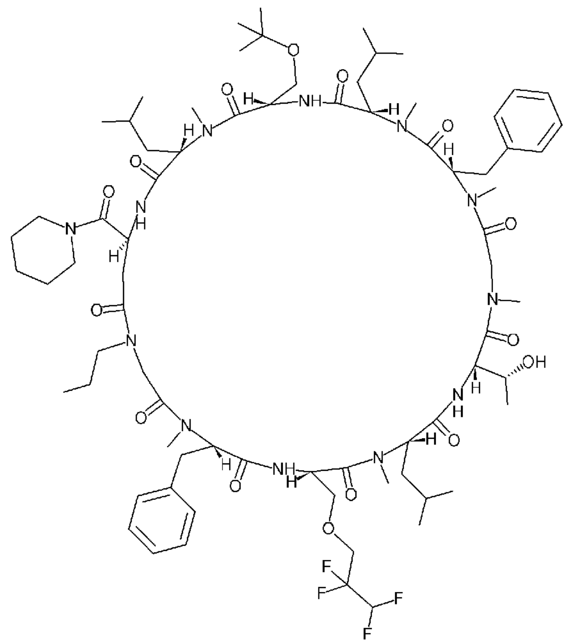 |

| ID | Structure |
|---|---|
| pd350 | 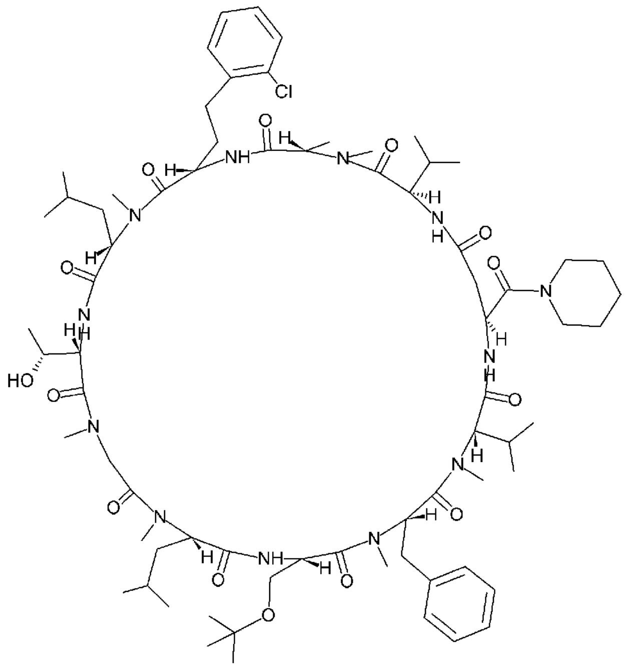 |
| pd351 | 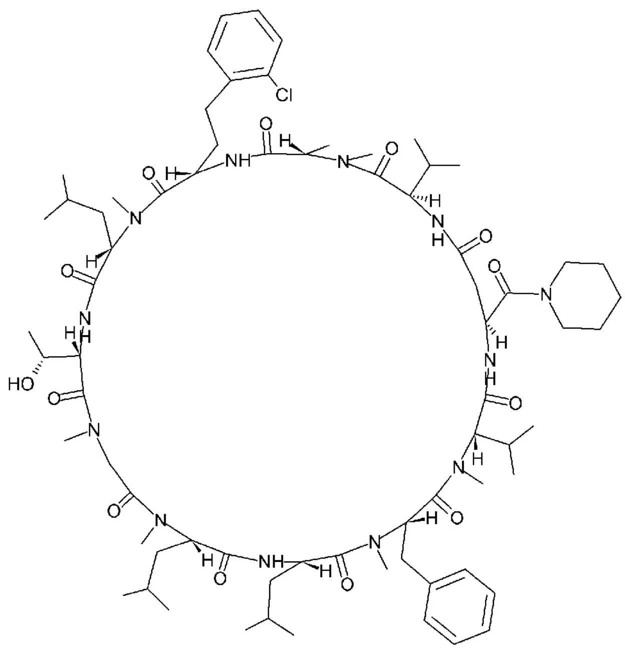 |

| ID | Structure |
|---|---|
| pd352 | 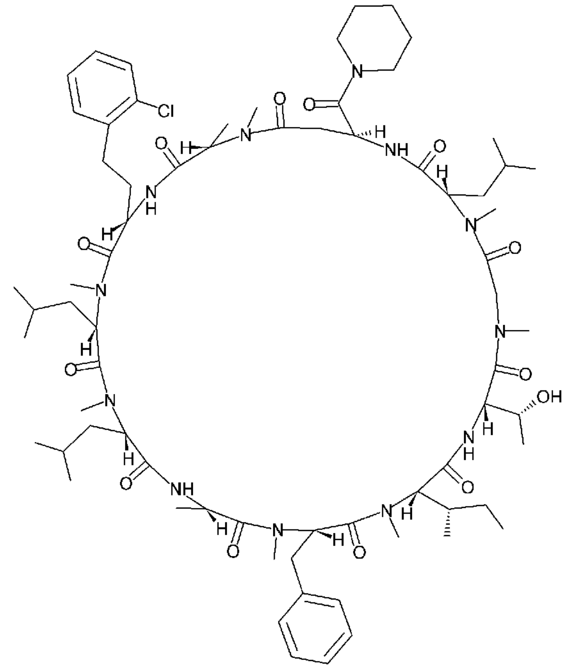 |
| pd353 | 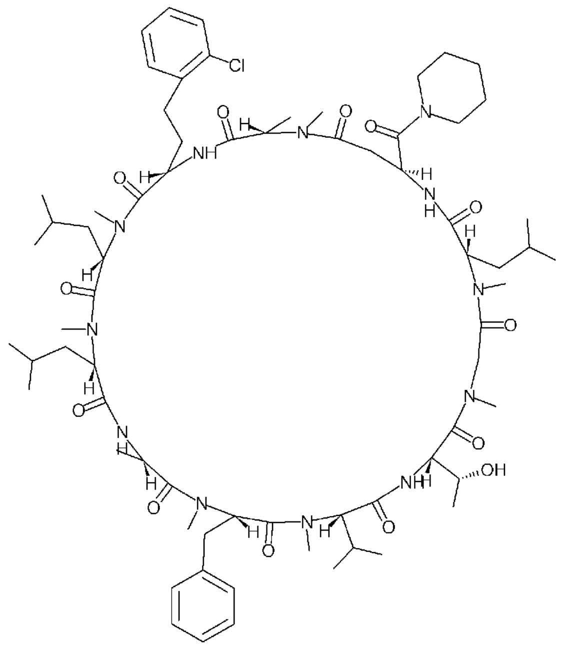 |

| ID | Structure |
|---|---|
| pd354 | 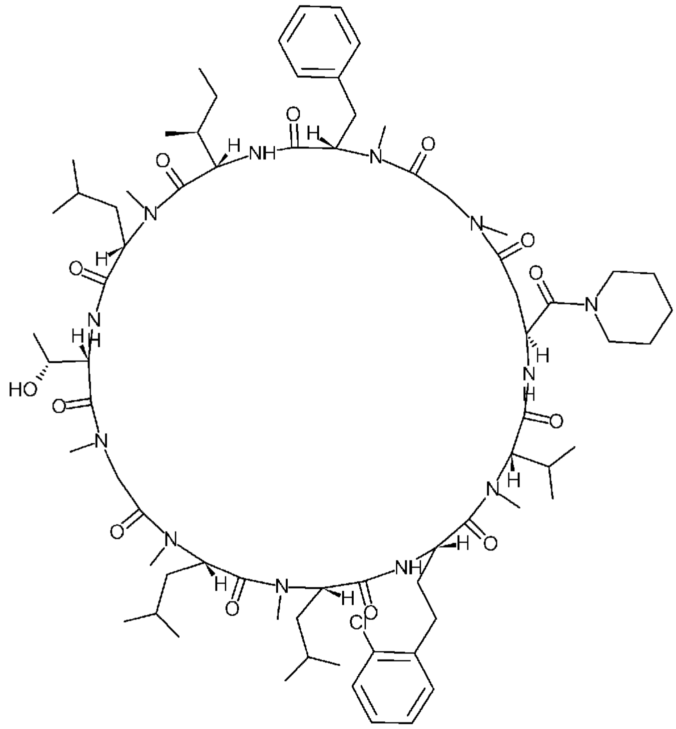 |
| pd355 | 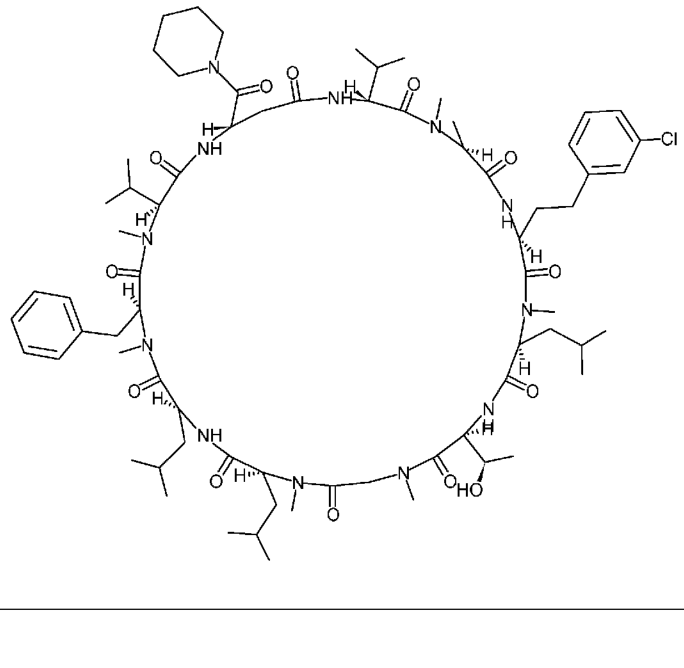 |

| ID | Structure |
|---|---|
| pd356 | 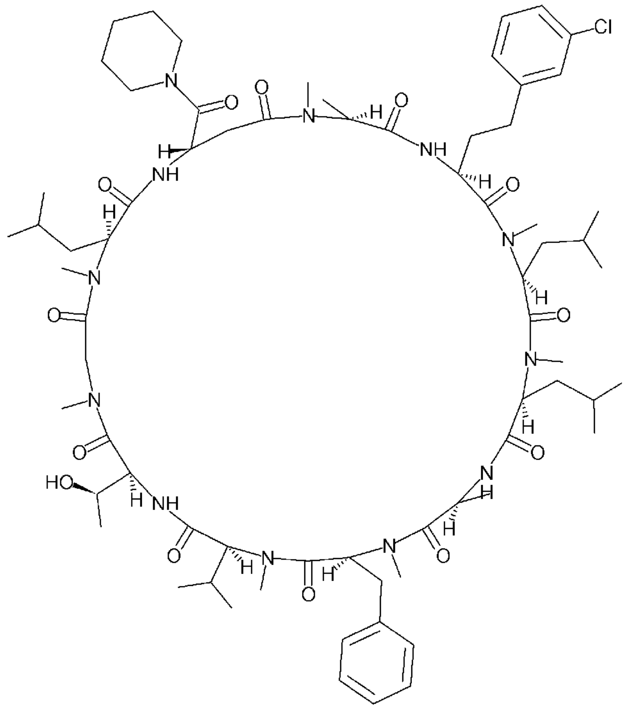 |
| pd357 | 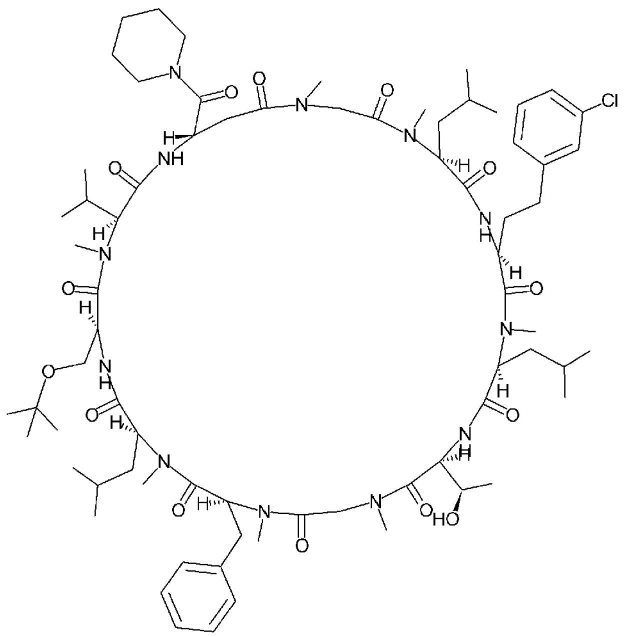 |

| ID | Structure |
| --- | --- |
| pd358 | 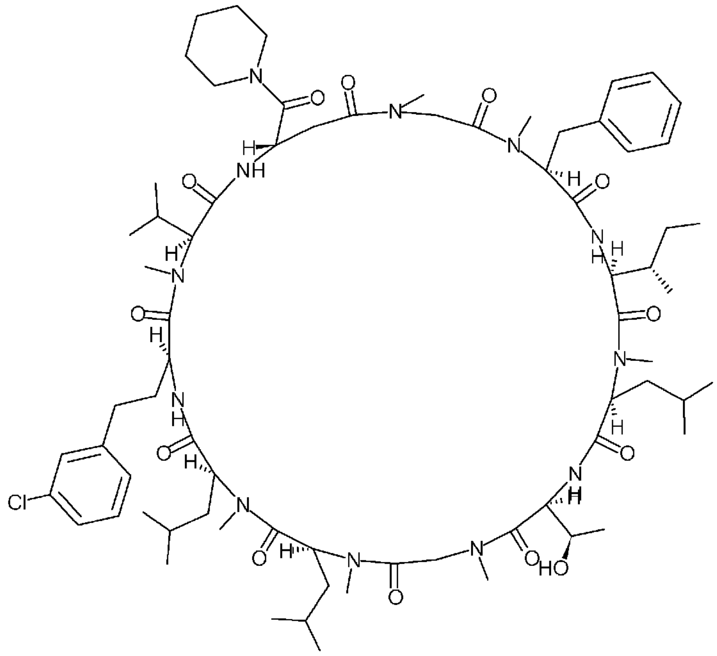 |
| pd359 | 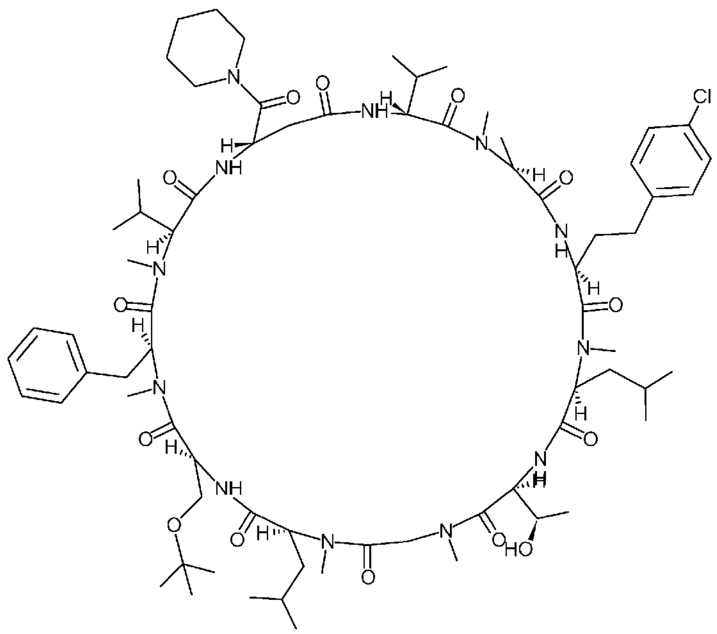 |

| ID | Structure |
|---|---|
| pd360 | 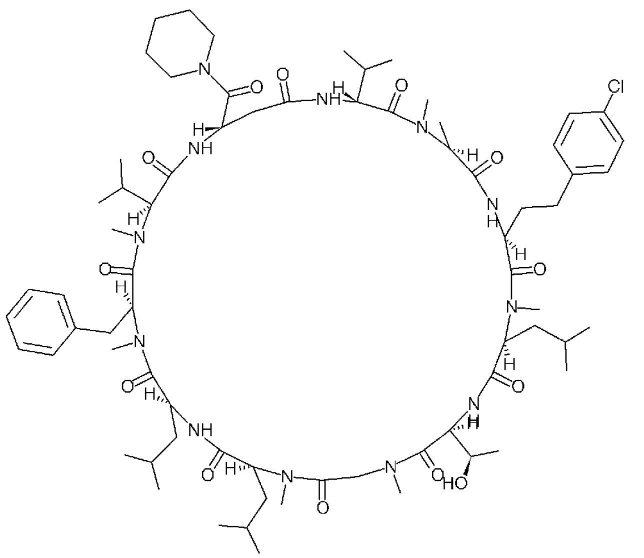 |
| pd361 | 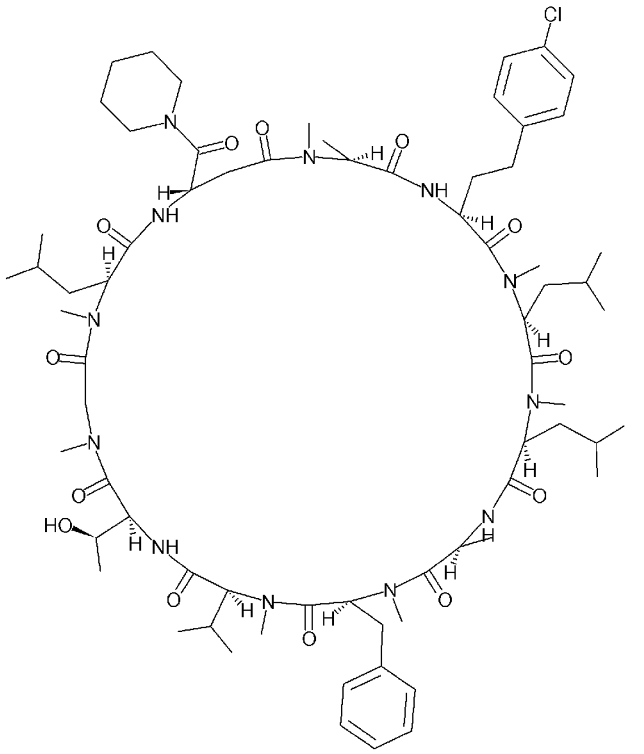 |

| ID | Structure |
|---|---|
| pd362 | 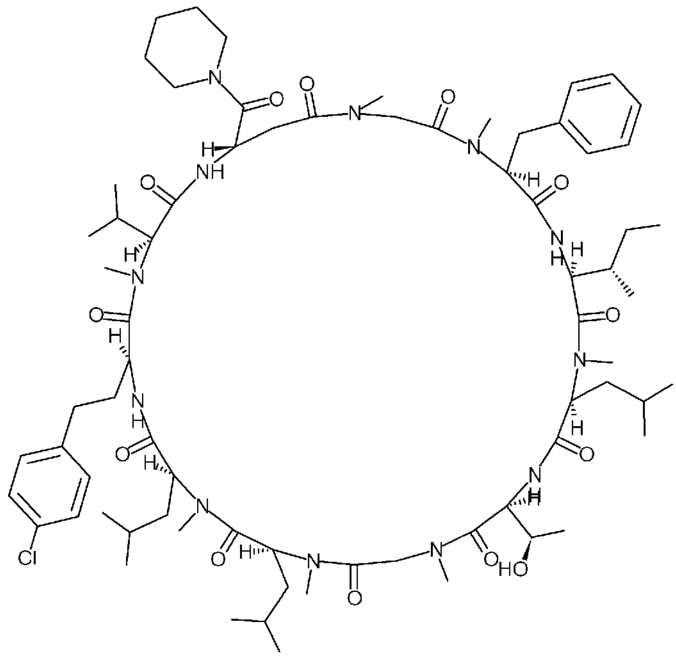 |
| pd363 | 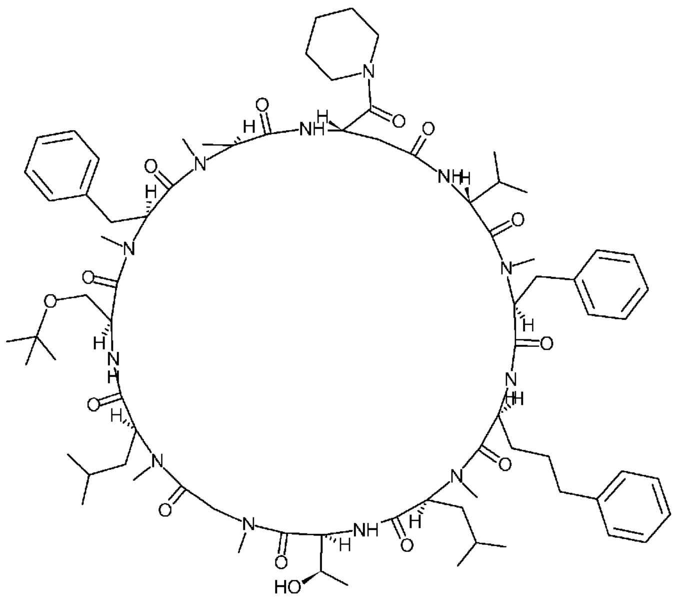 |

| ID | Structure |
|---|---|
| pd364 | 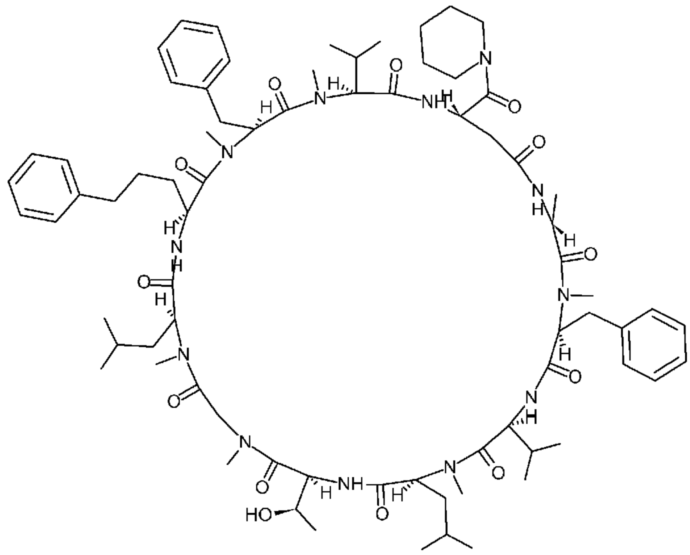 |
| pd365 | 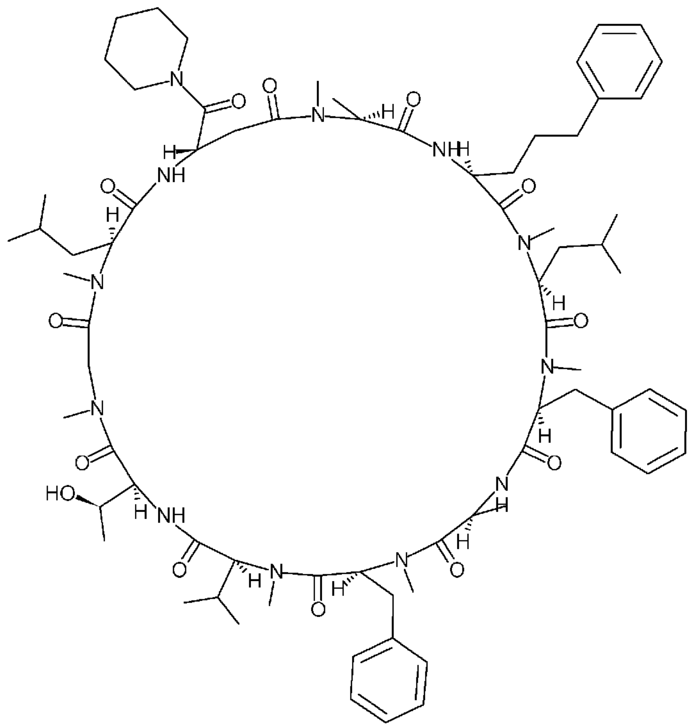 |

| ID | Structure |
|---|---|
| pd366 | 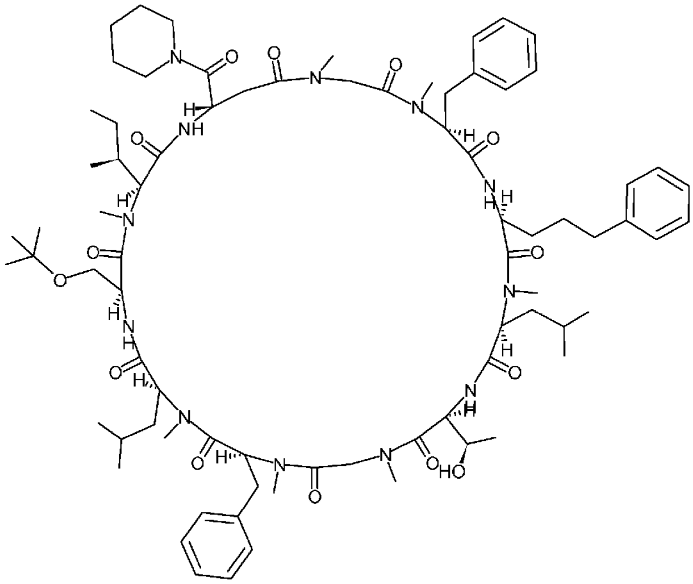 |
| pd367 | 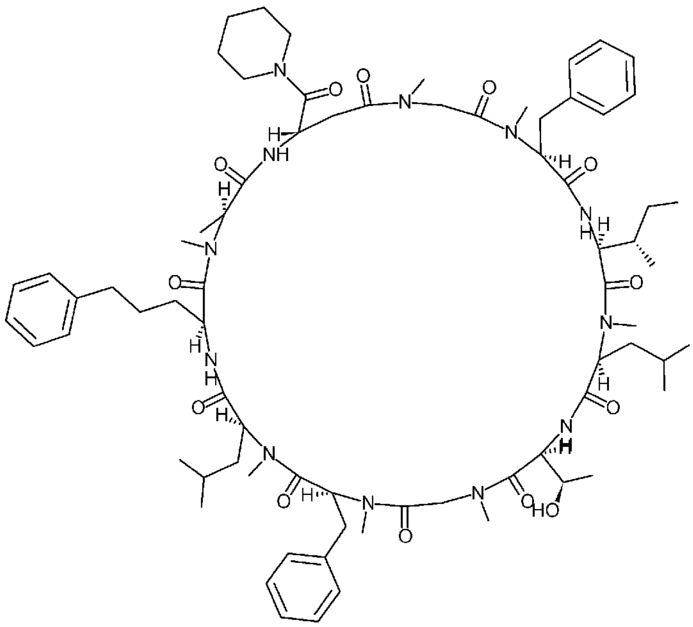 |

| ID | Structure |
|---|---|
| pd368 | 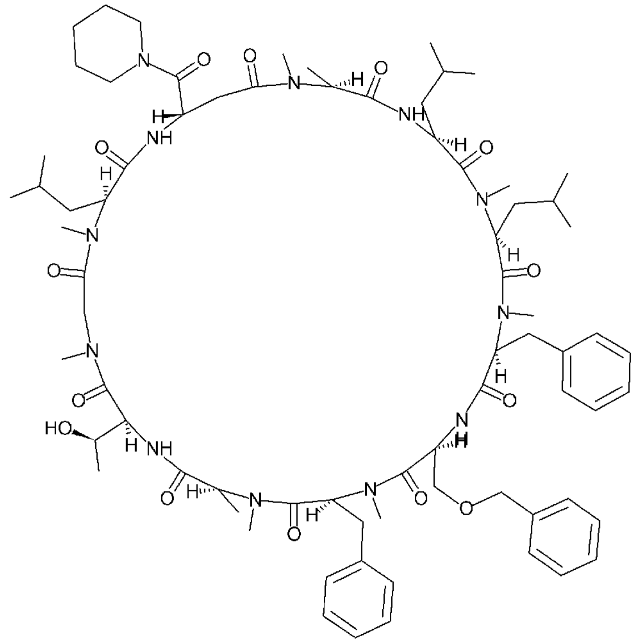 |
| pd369 | 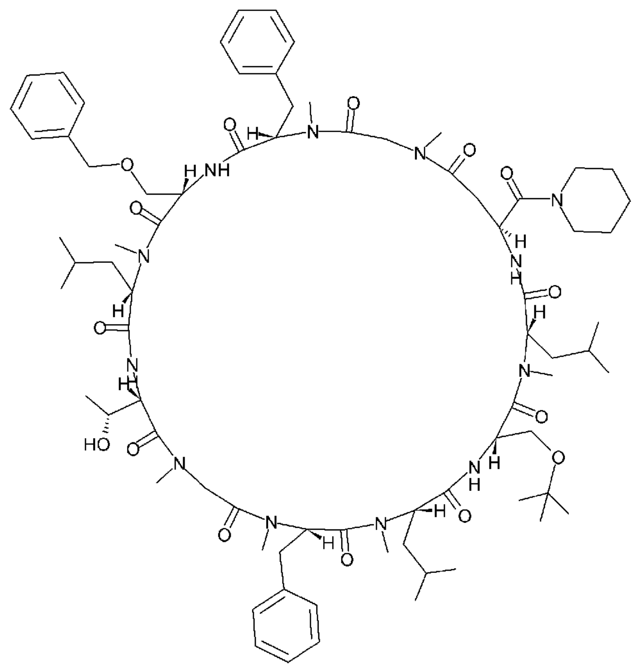 |

| ID | Structure |
|---|---|
| pd370 | 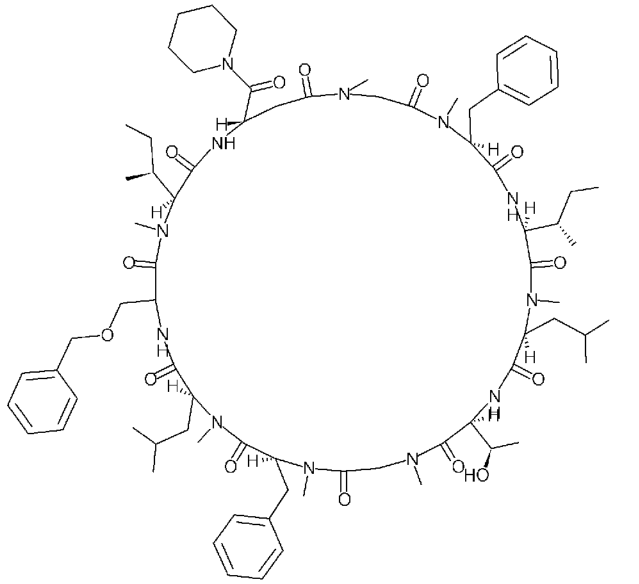 |
| pd371 | 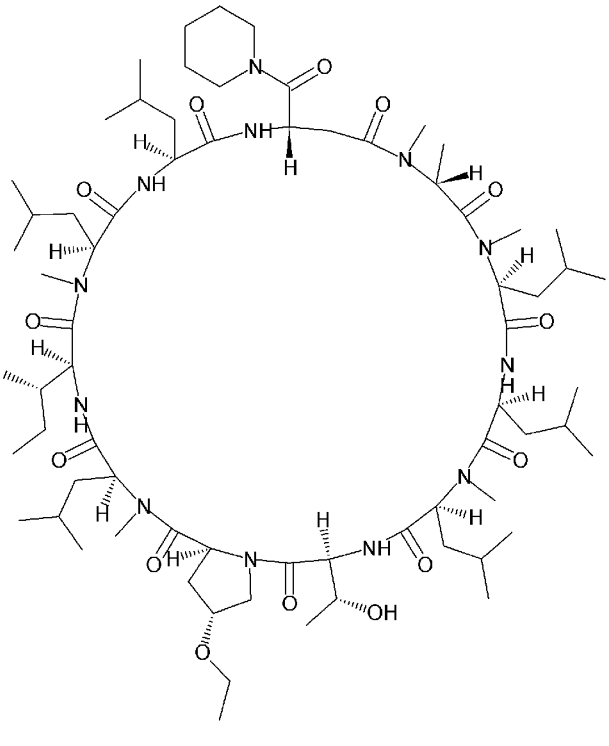 |

| ID | Structure |
|---|---|
| pd372 | 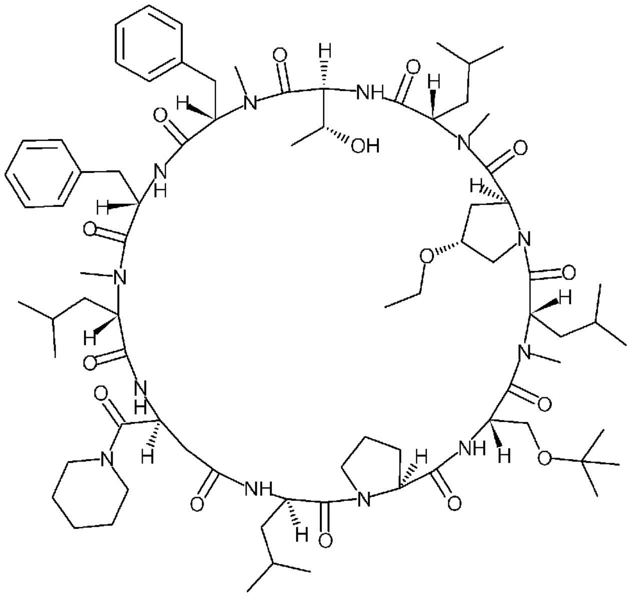 |
| pd373 | 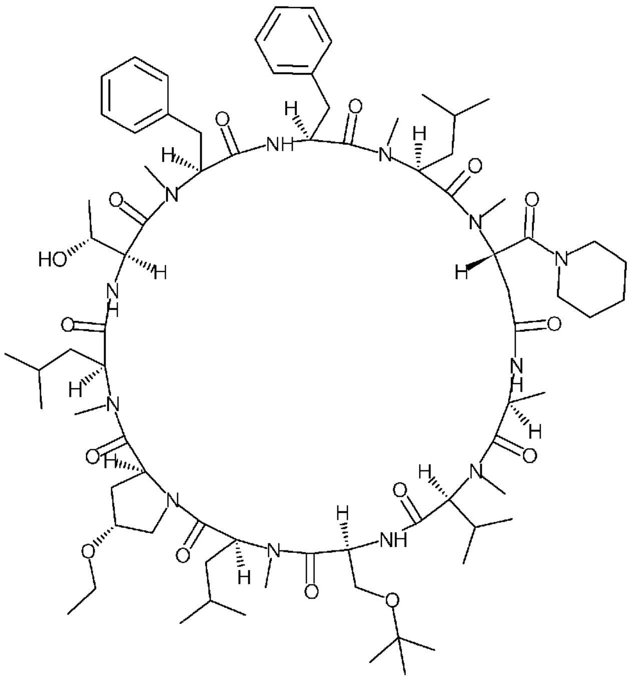 |

| ID | Structure |
|---|---|
| pd374 | 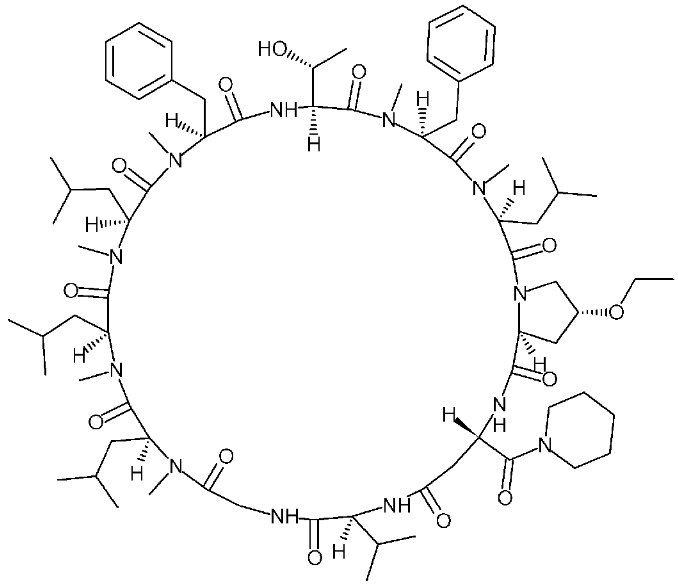 |
| pd375 | 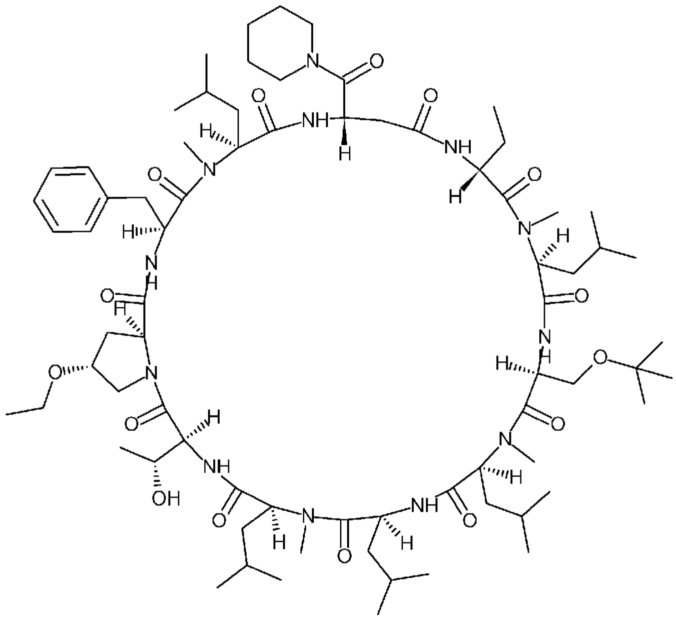 |

| ID | Structure |
|---|---|
| pd376 | 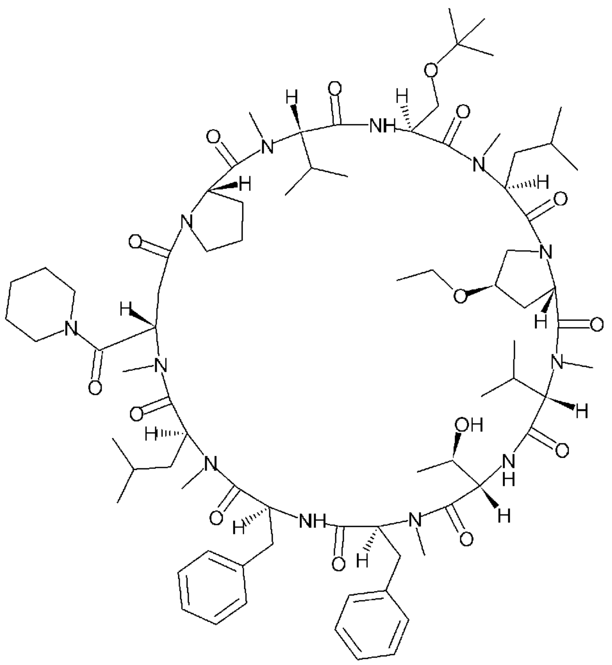 |
| pd377 | 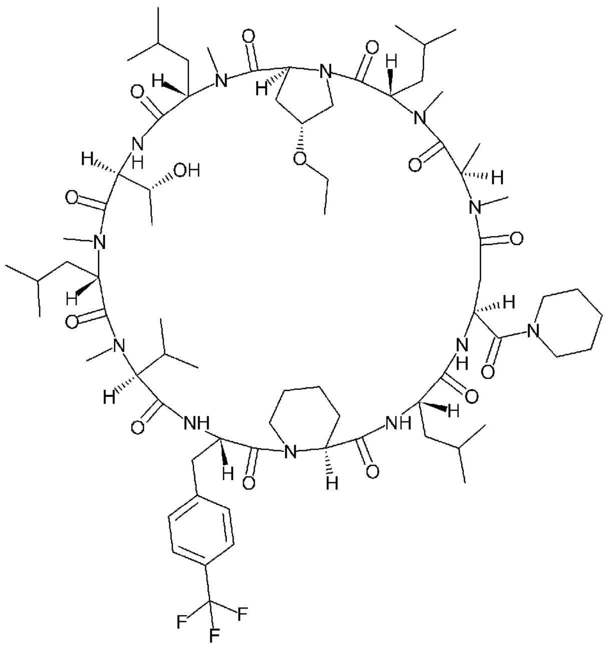 |

| ID | Structure |
|---|---|
| pd378 | 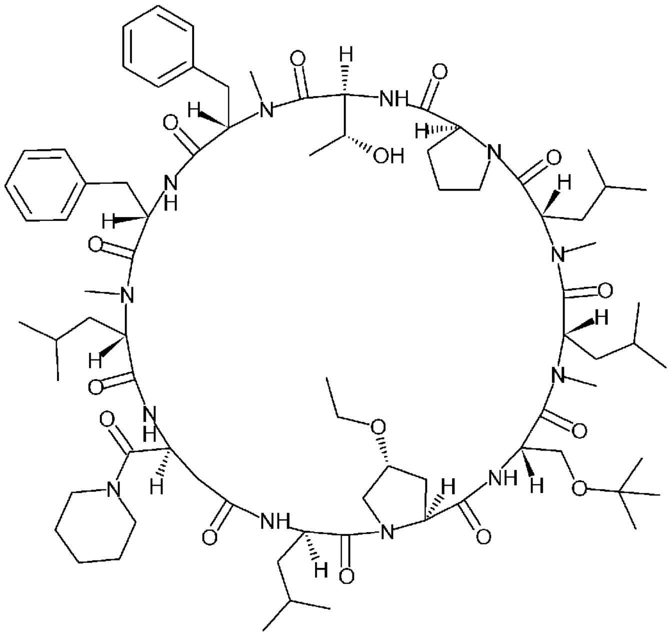 |
| pd379 | 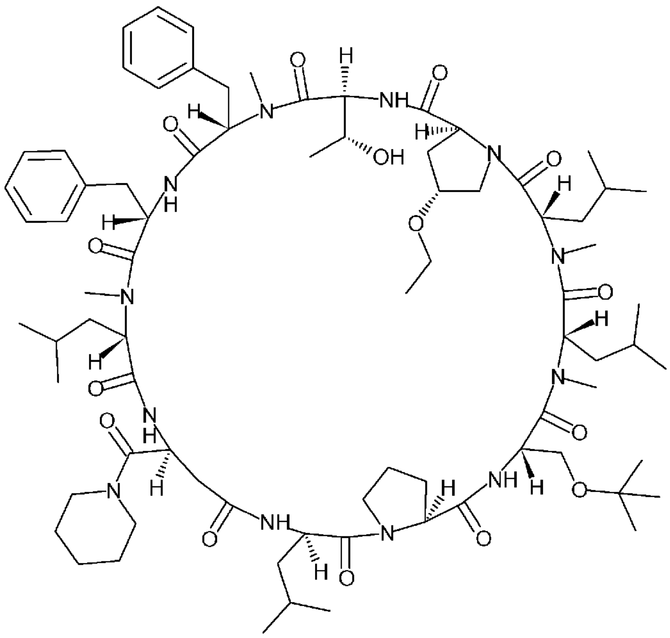 |

| ID | Structure |
|---|---|
| pd380 | 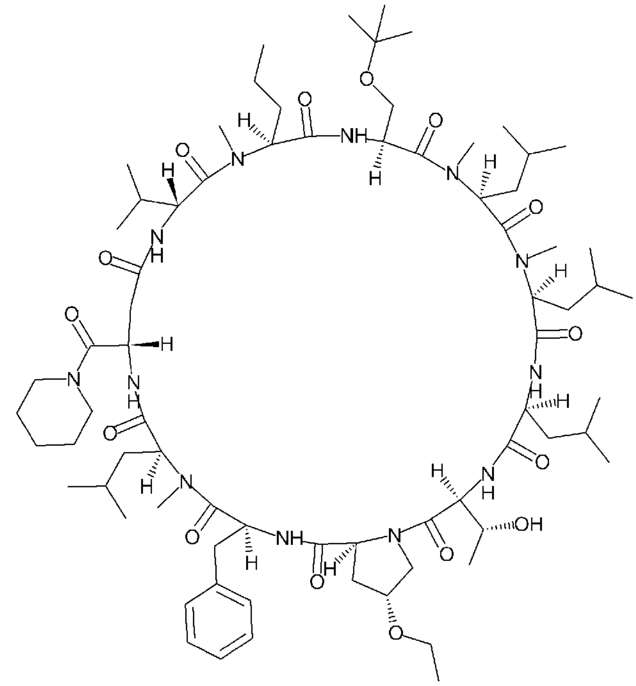 |
| pd381 | 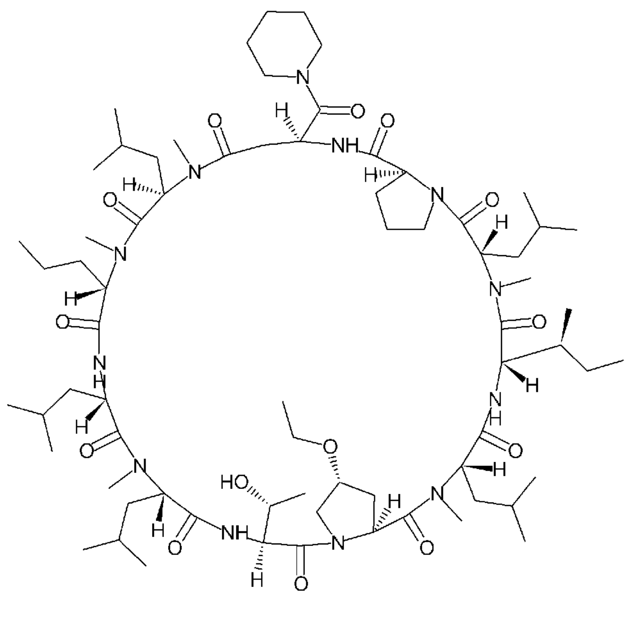 |

| ID | Structure |
|---|---|
| pd382 | 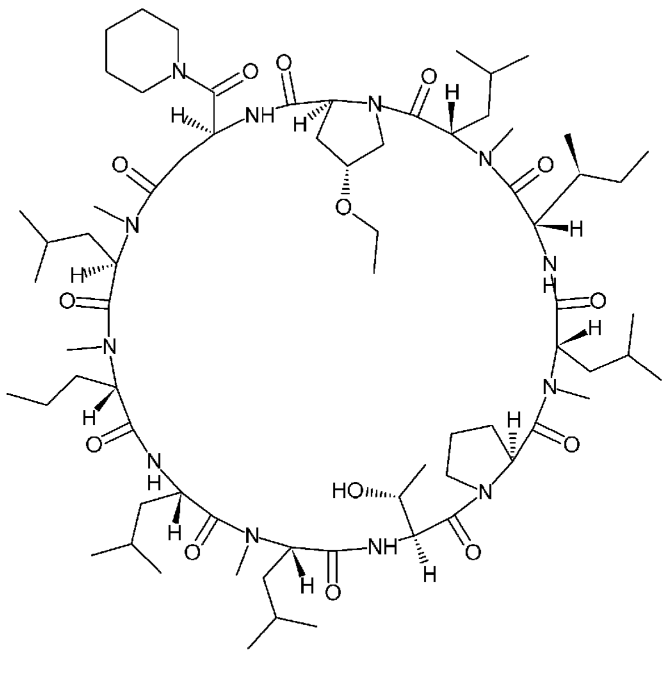 |
| pd383 | 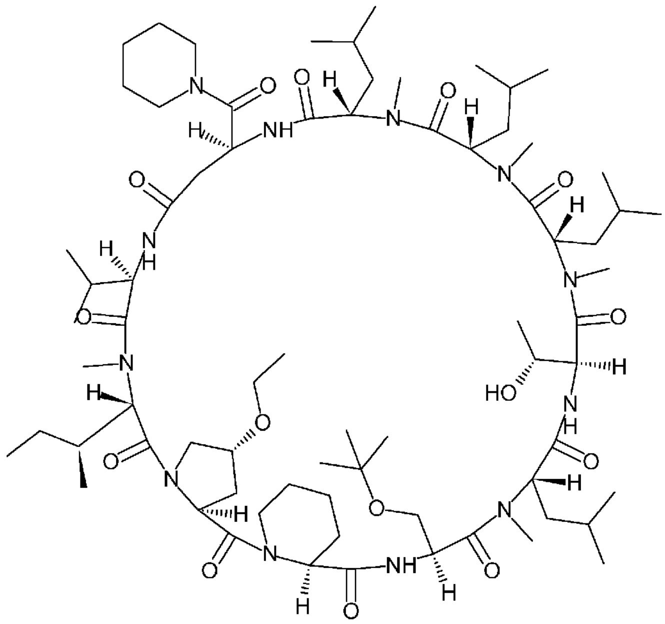 |

| ID | Structure |
|---|---|
| pd384 | 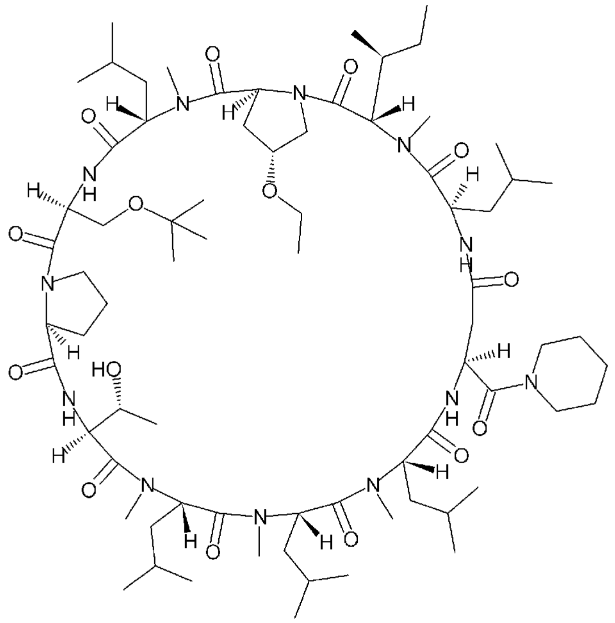 |
| pd385 | 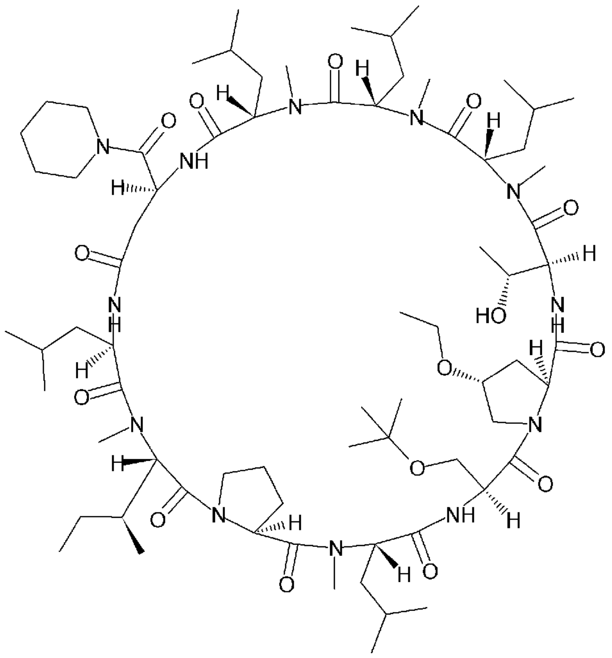 |

| ID | Structure |
|---|---|
| pd386 | 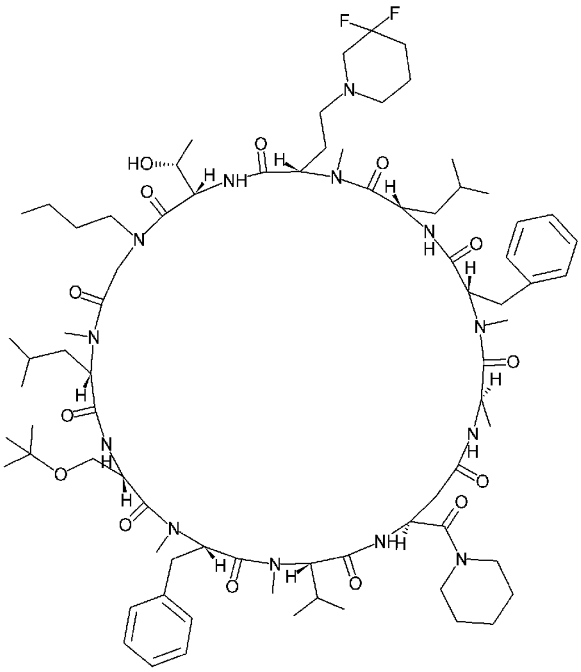 |
| pd387 | 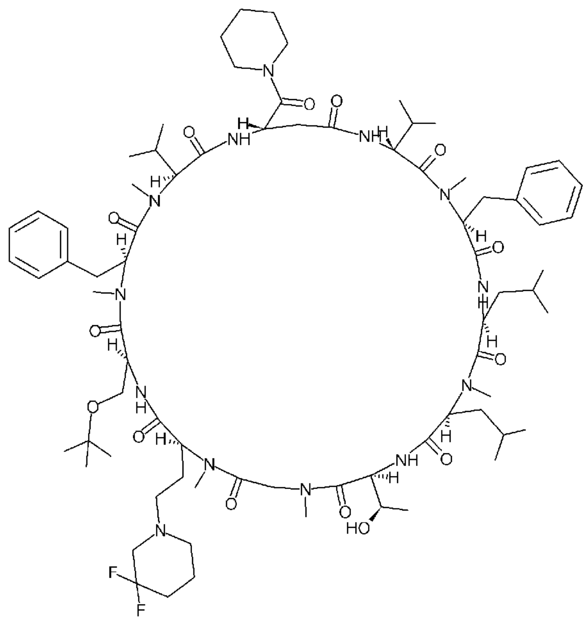 |

| ID | Structure |
|---|---|
| pd388 | 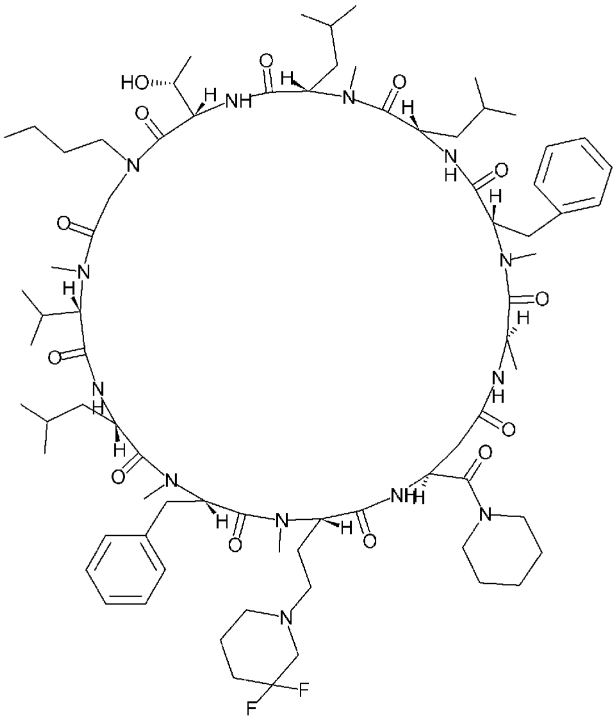 |
| pd389 | 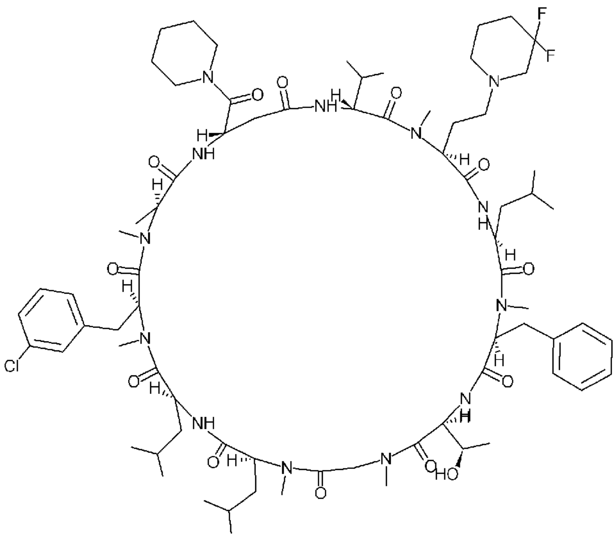 |

| ID | Structure |
|---|---|
| pd390 | 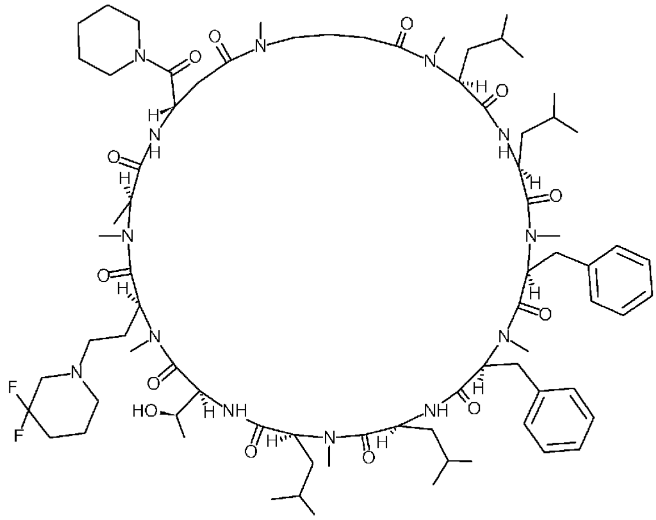 |
| pd391 | 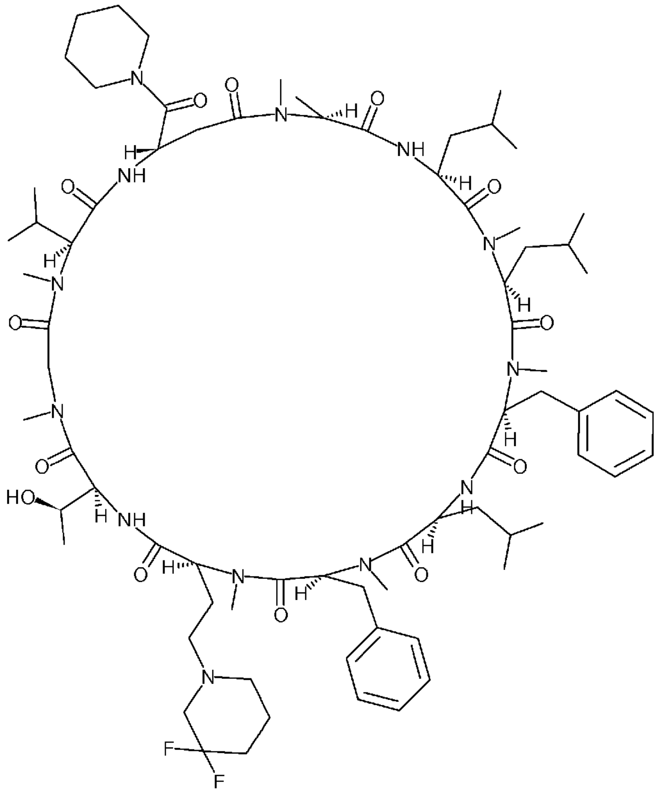 |

| ID | Structure |
|---|---|
| pd392 | 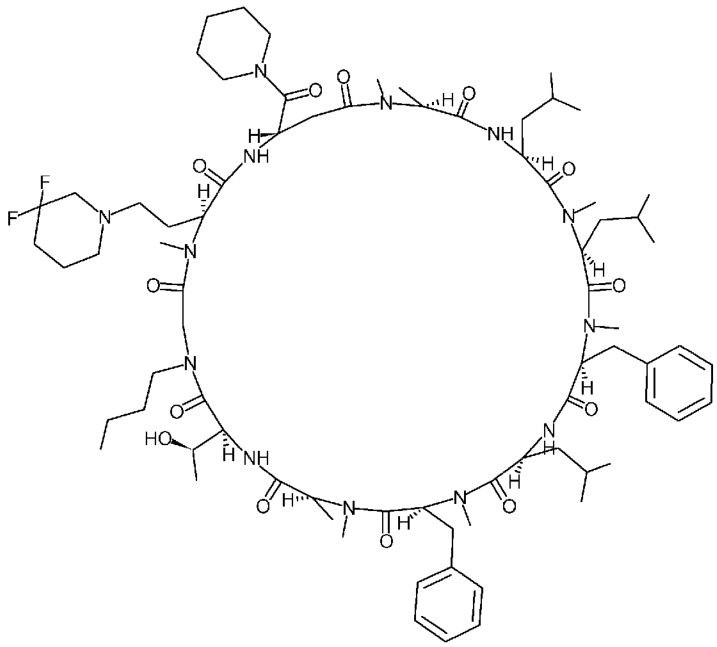 |
| pd393 | 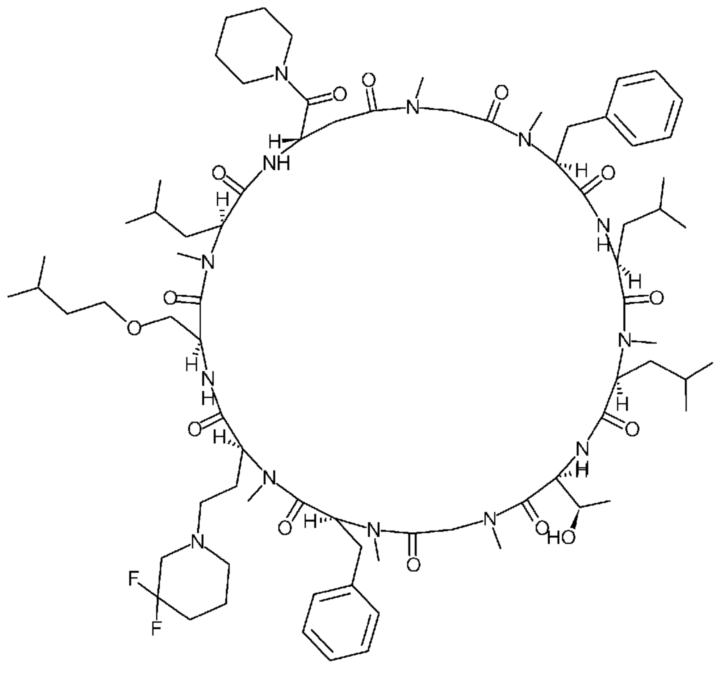 |

| ID | Structure |
|---|---|
| pd394 | 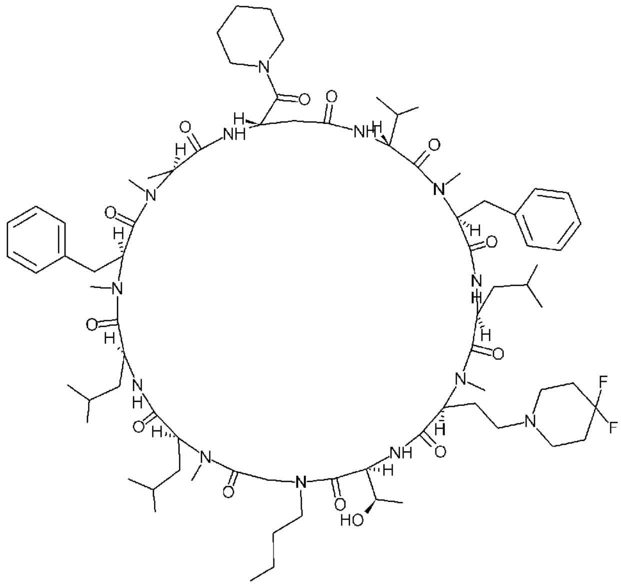 |
| pd395 | 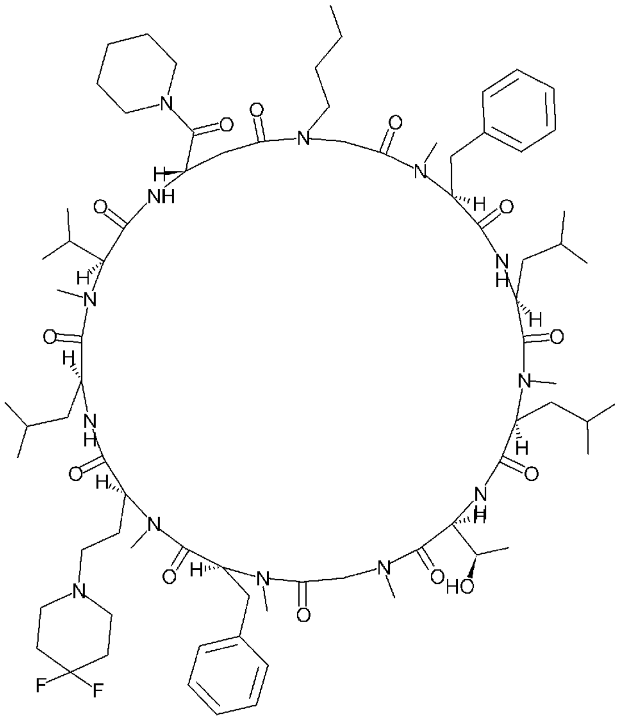 |

| ID | Structure |
|---|---|
| pd396 | 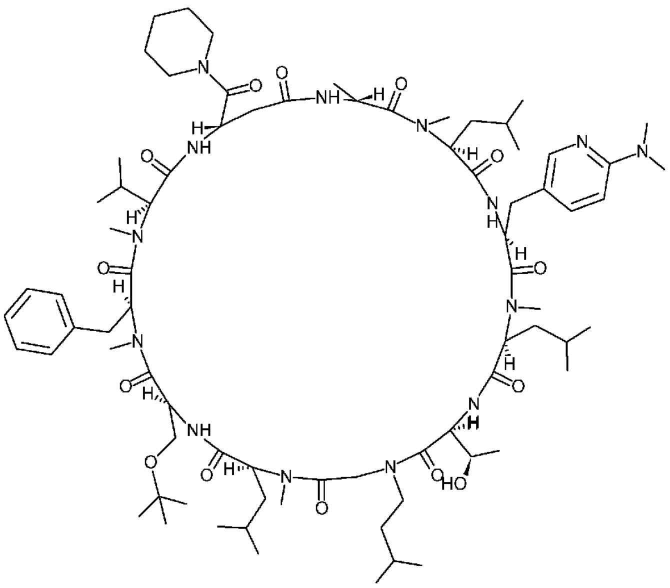 |
| pd397 | 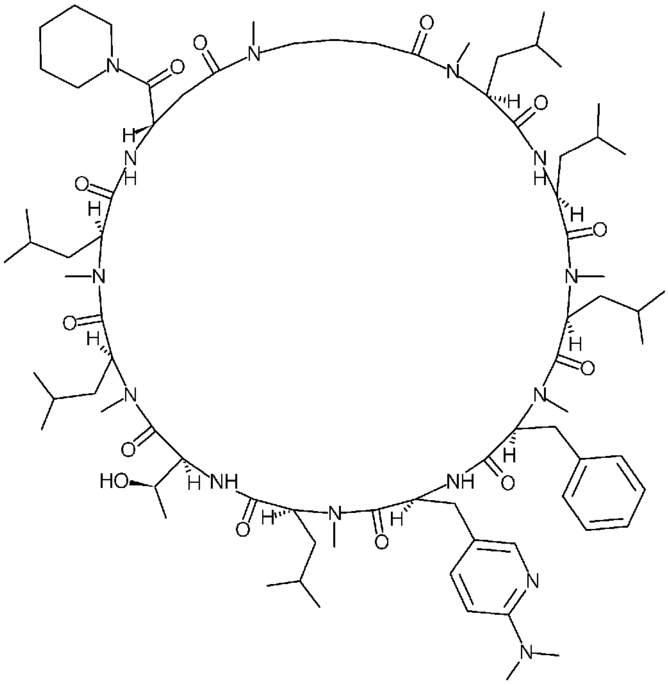 |

| ID | Structure |
|---|---|
| pd398 | 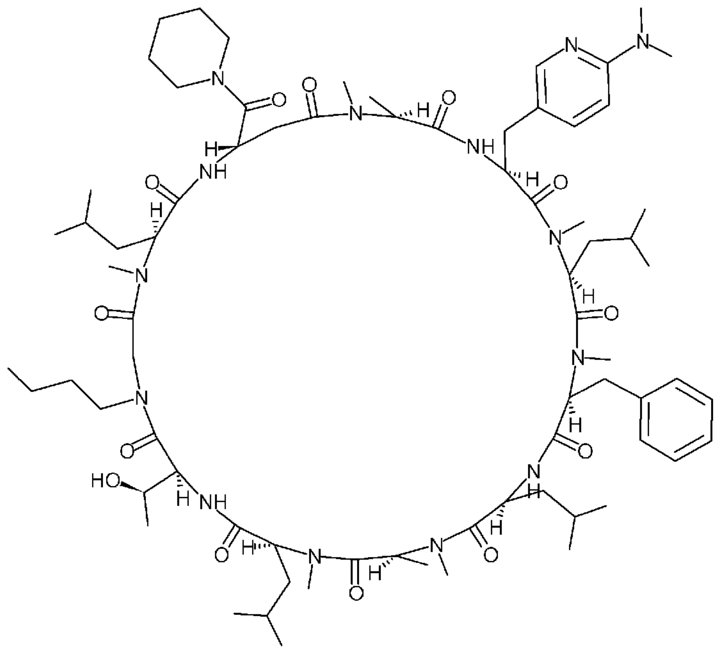 |
| pd399 | 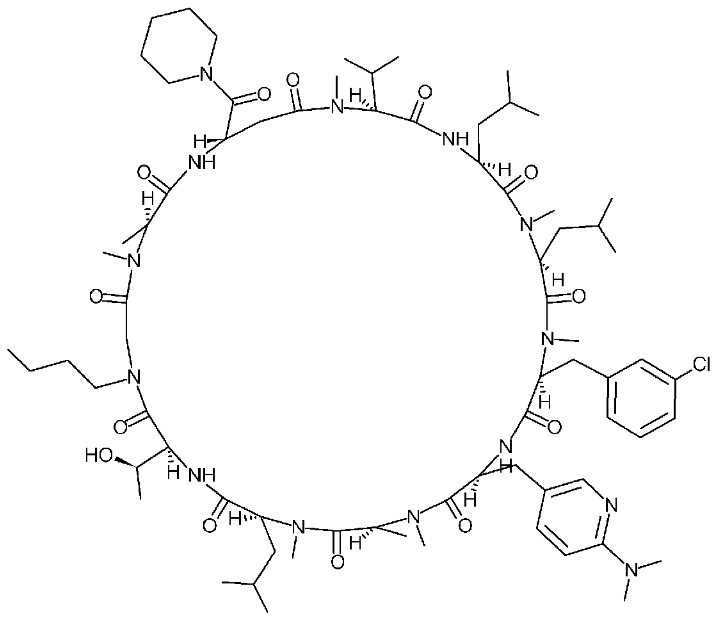 |

| ID | Structure |
|---|---|
| pd400 | 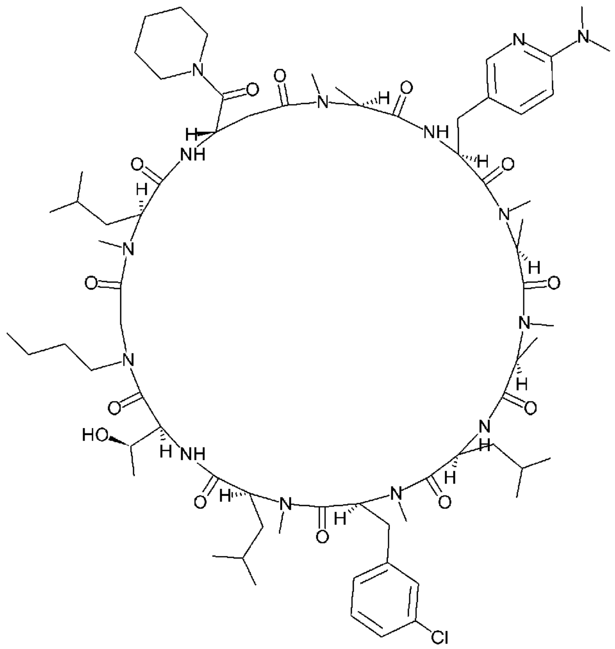 |
| pd401 | 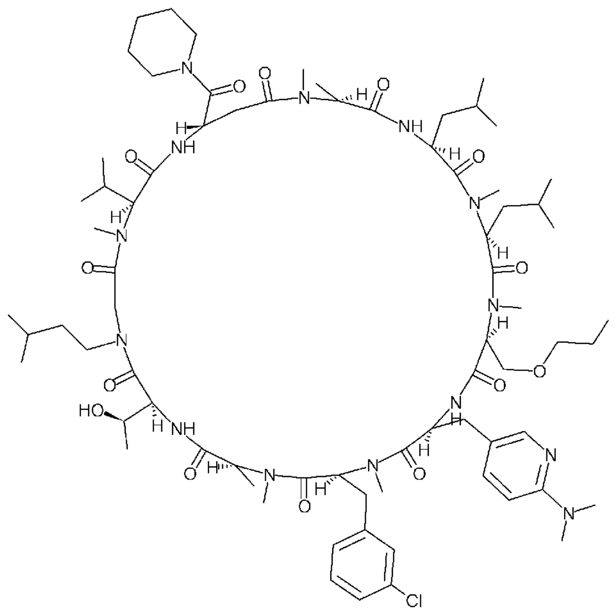 |

| ID | Structure |
|---|---|
| pd402 | 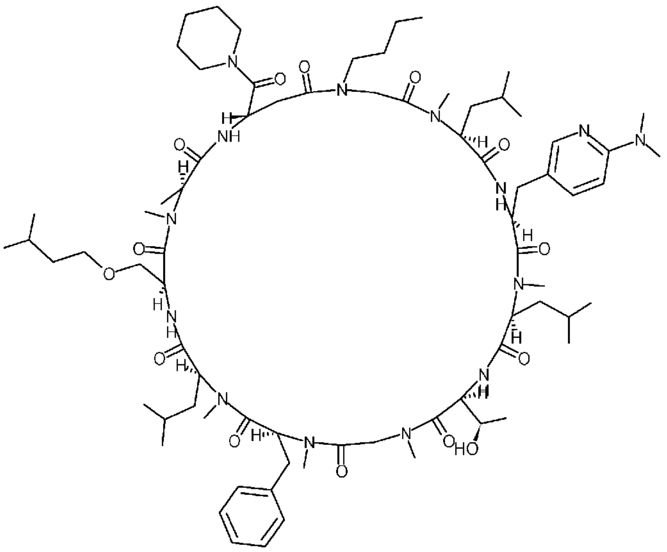 |
| pd403 | 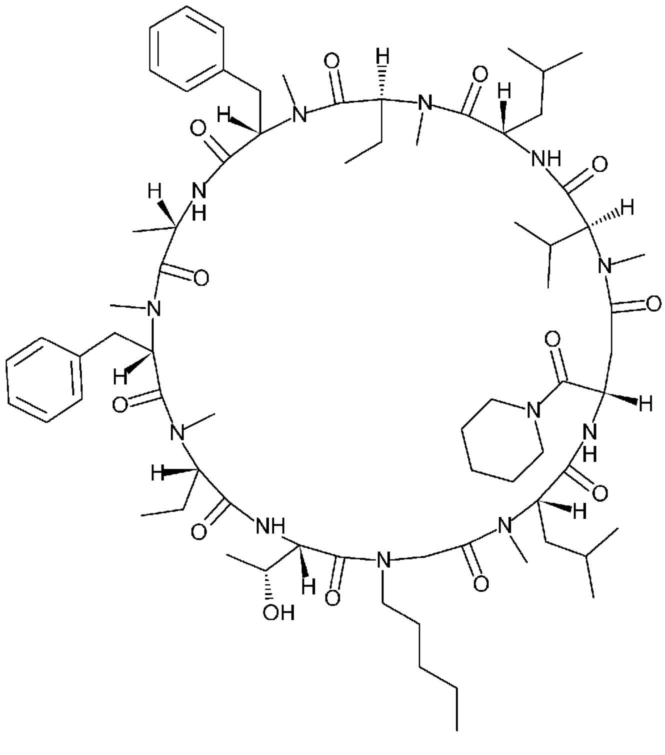 |

| ID | Structure |
|---|---|
| pd404 | 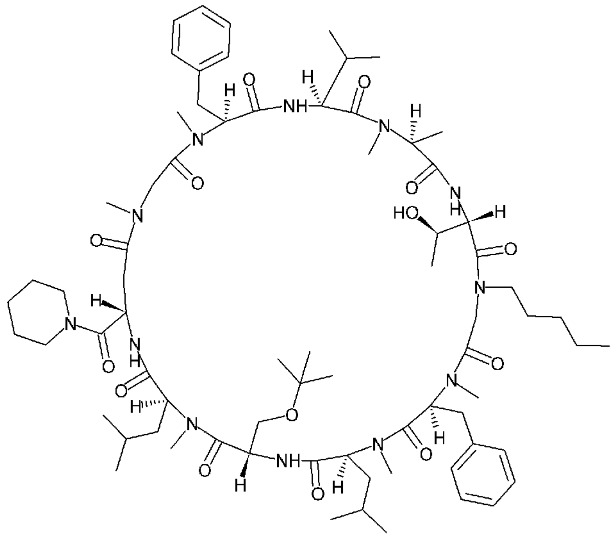 |
| pd405 | 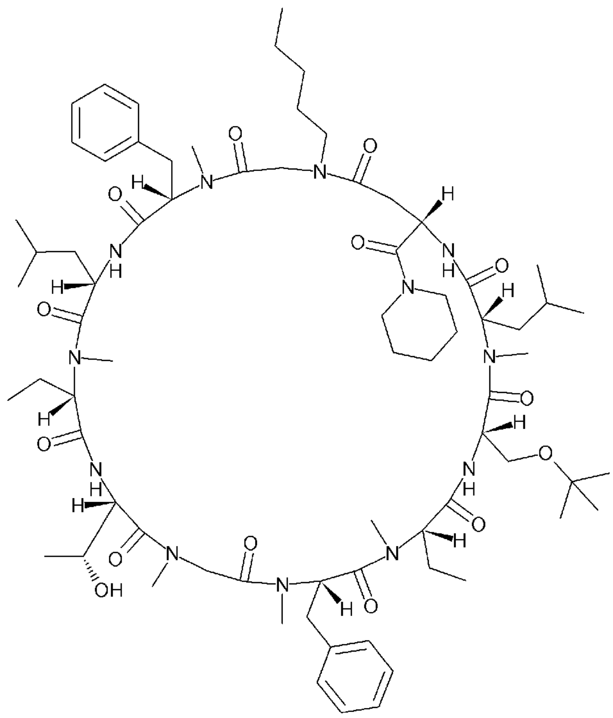 |

| ID | Structure |
|---|---|
| pd406 | 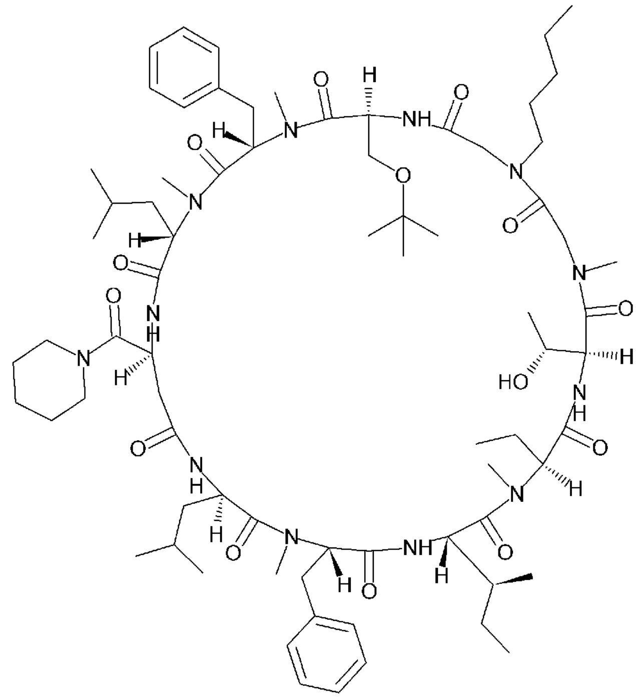 |
| pd407 | 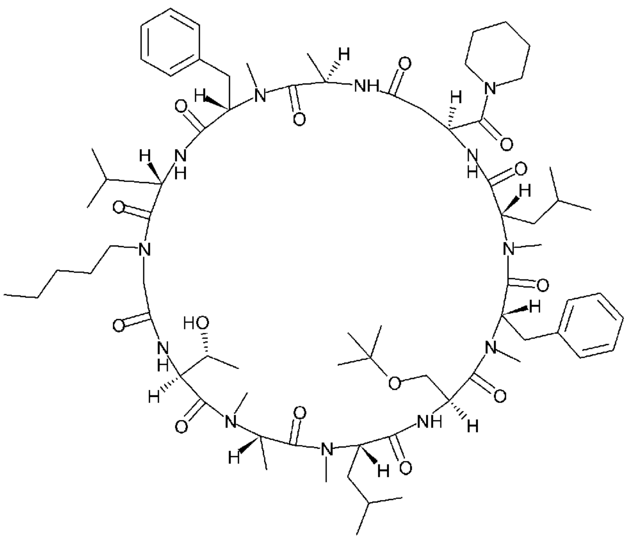 |

| ID | Structure |
|---|---|
| pd408 | 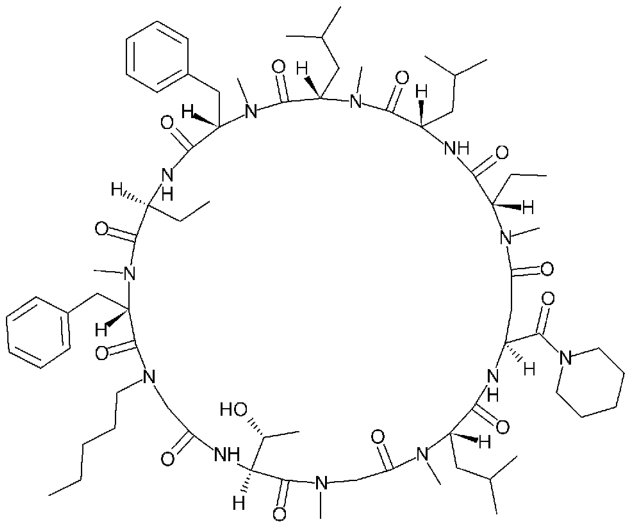 |
| pd409 | 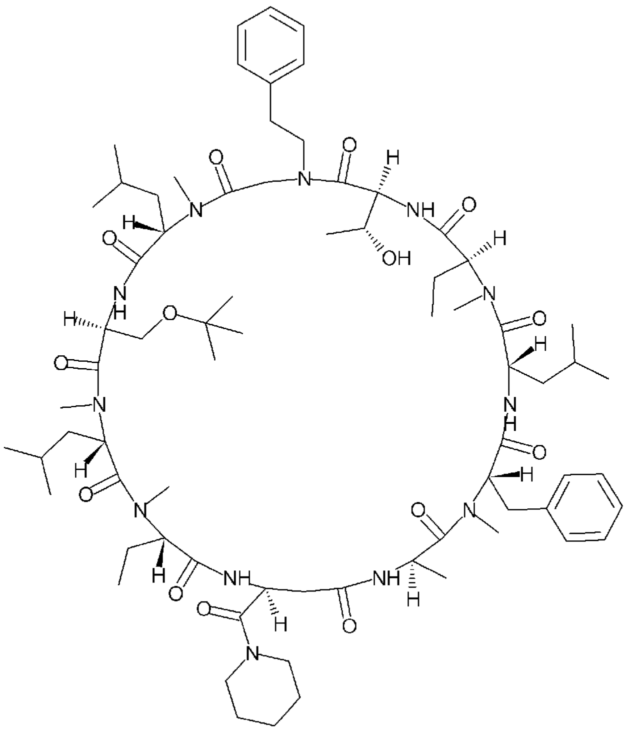 |

| ID | Structure |
| --- | --- |
| pd410 | 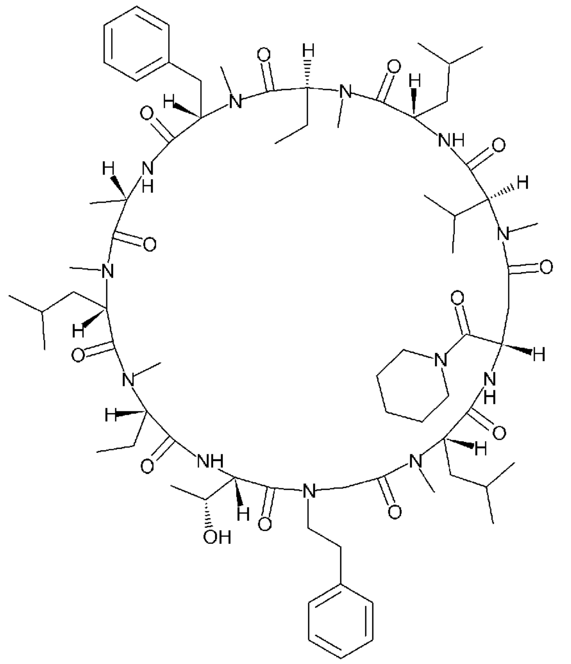 |
| pd411 | 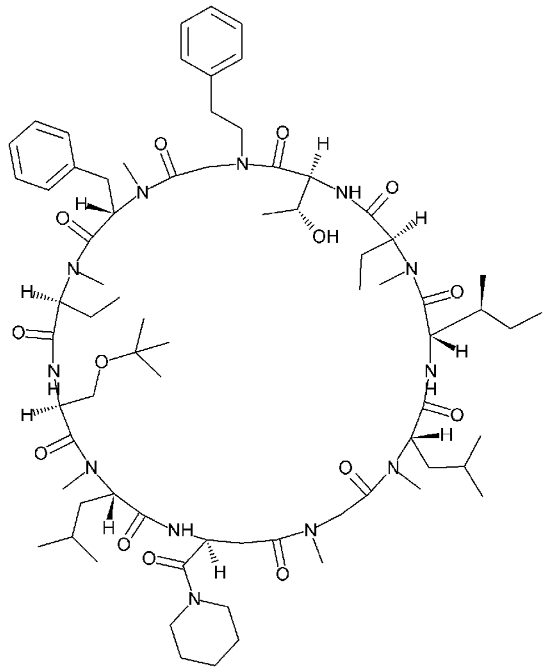 |

| ID | Structure |
|---|---|
| pd412 | 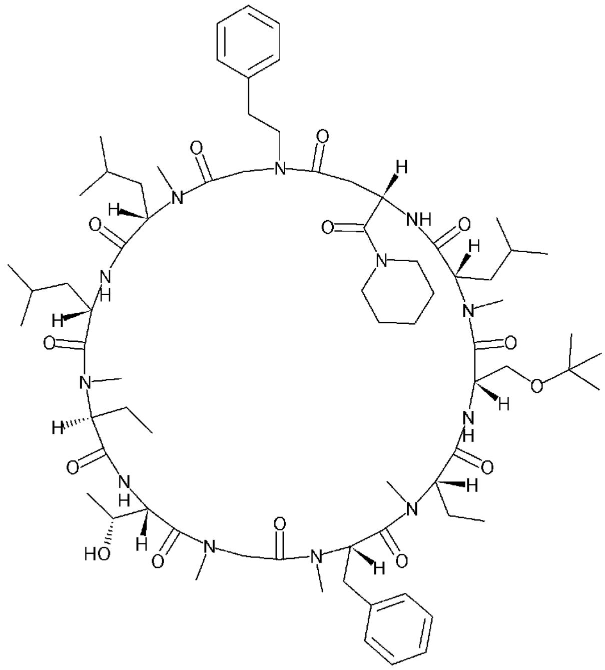 |
| pd413 | 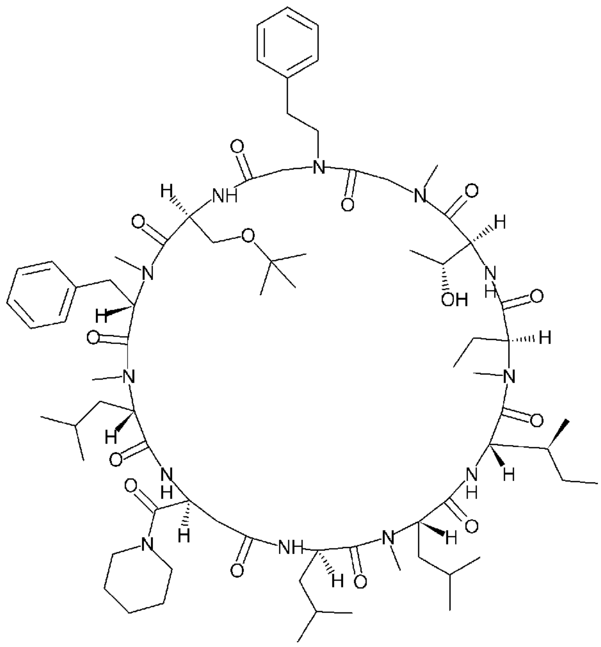 |

| ID | Structure |
|---|---|
| pd414 | 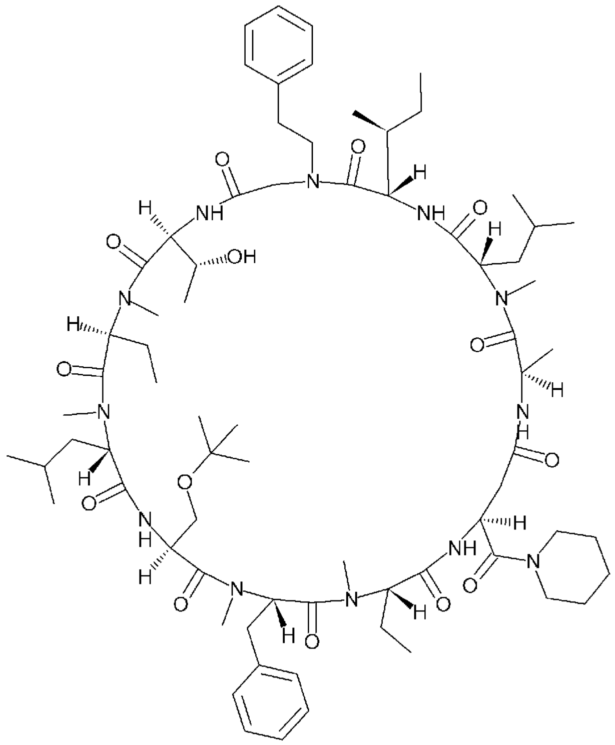 |
| pd415 | 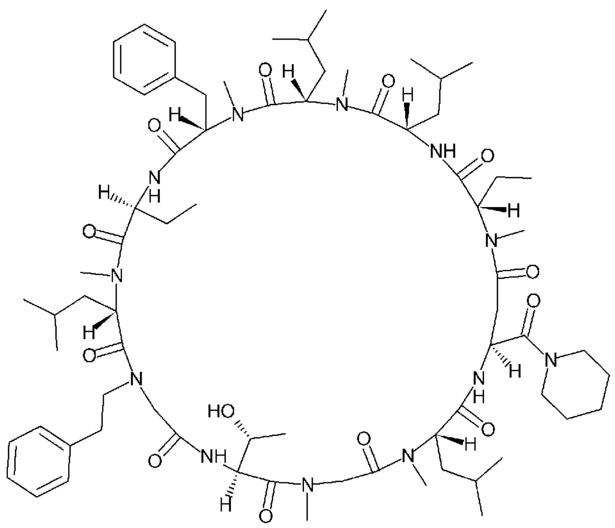 |

| ID | Structure |
|---|---|
| pd416 | 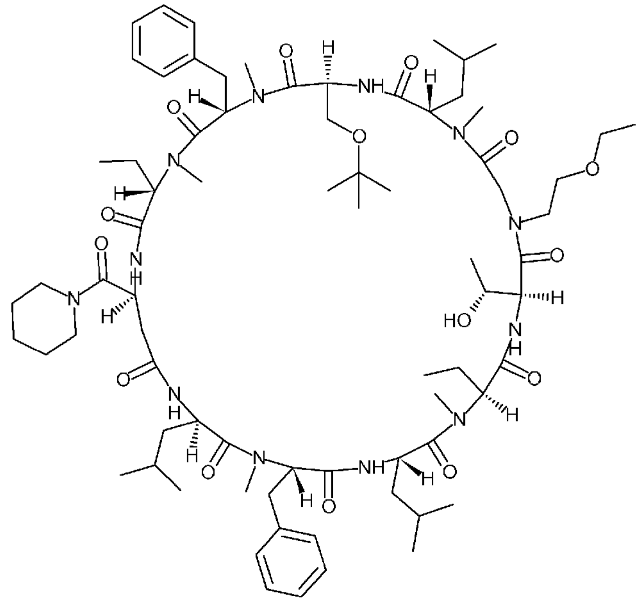 |
| pd417 | 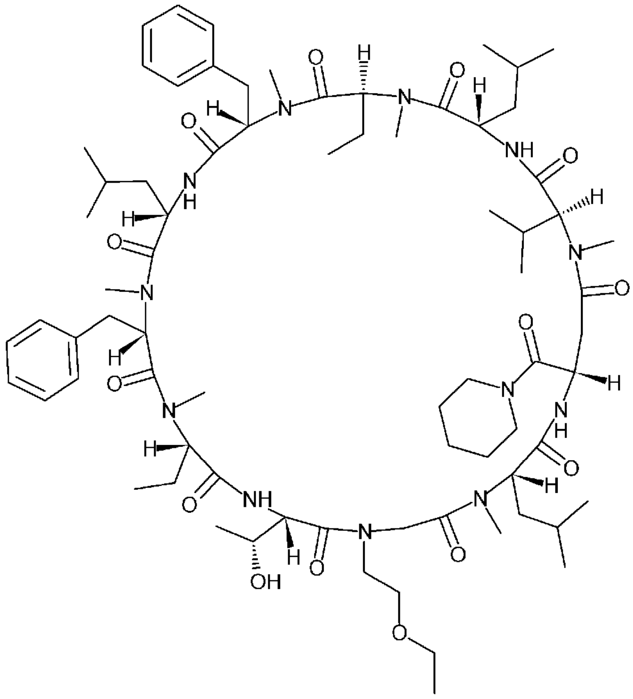 |

| ID | Structure |
|---|---|
| pd418 | 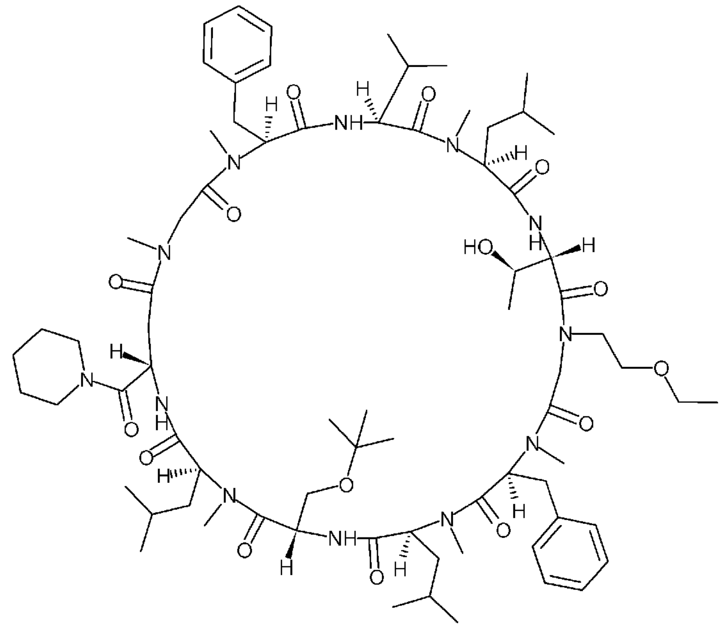 |
| pd419 | 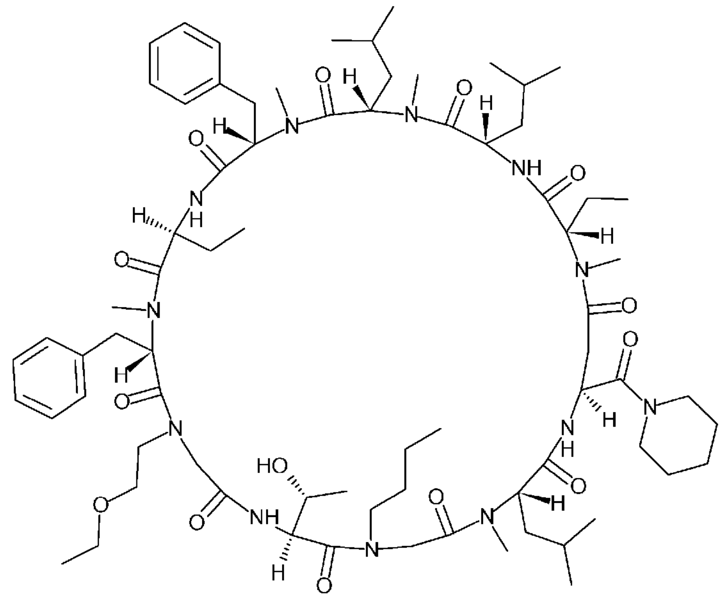 |

| ID | Structure |
| --- | --- |
| pd420 | 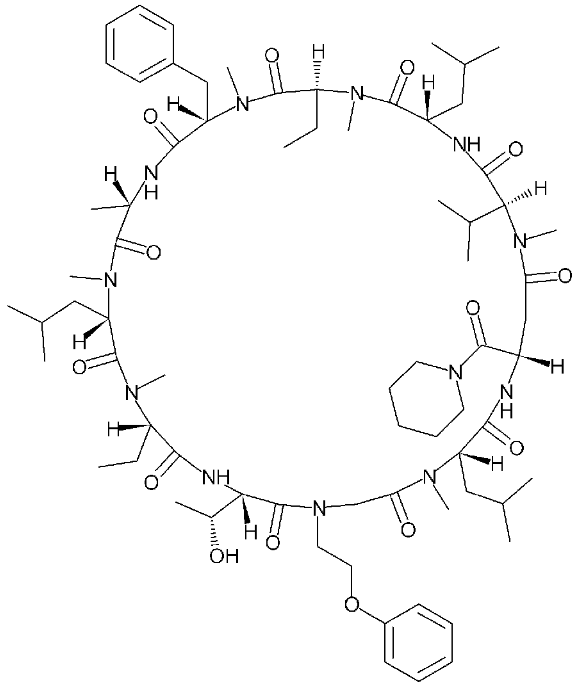 |
| pd421 | 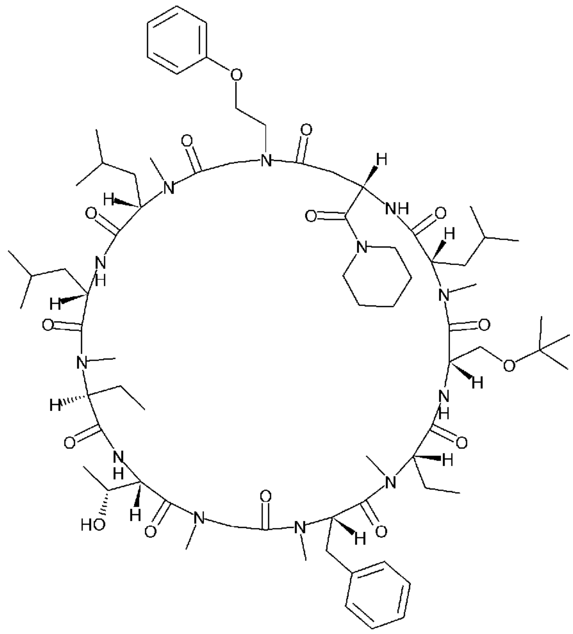 |

| ID | Structure |
|---|---|
| pd422 | 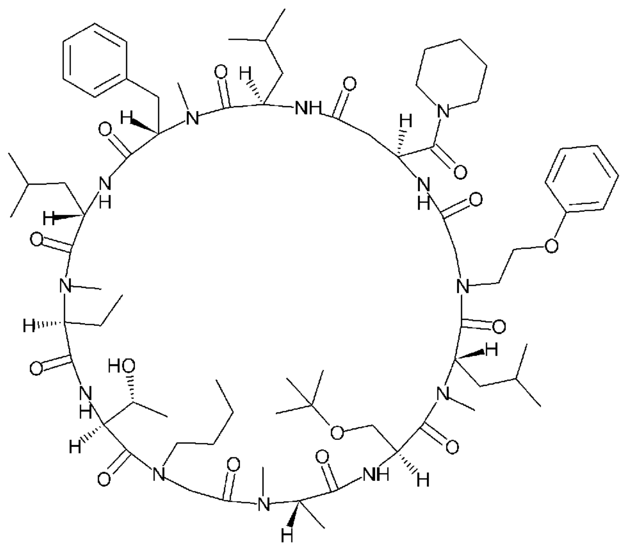 |
| pd423 | 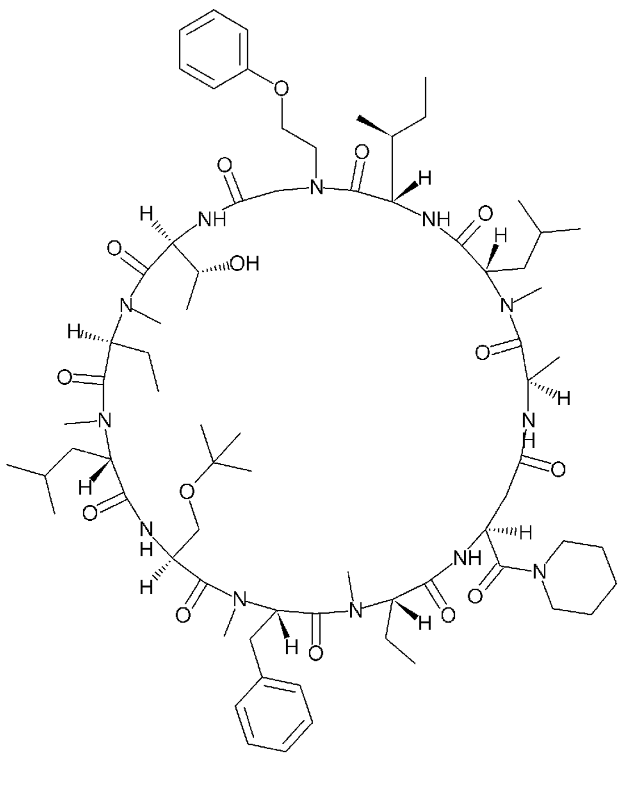 |

| ID | Structure |
|---|---|
| pd424 | 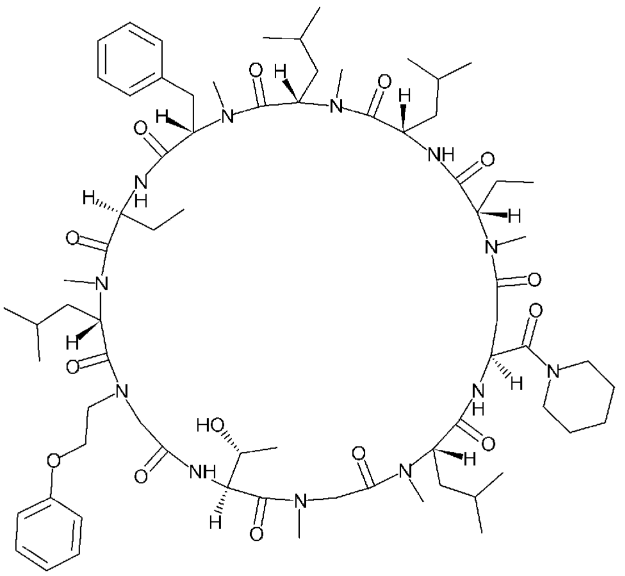 |
| pd425 | 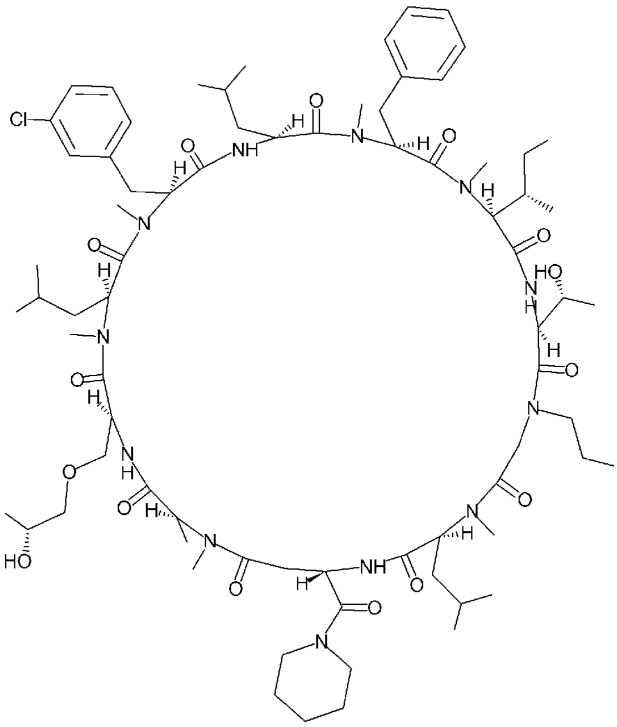 |

| ID | Structure |
|---|---|
| pd426 | 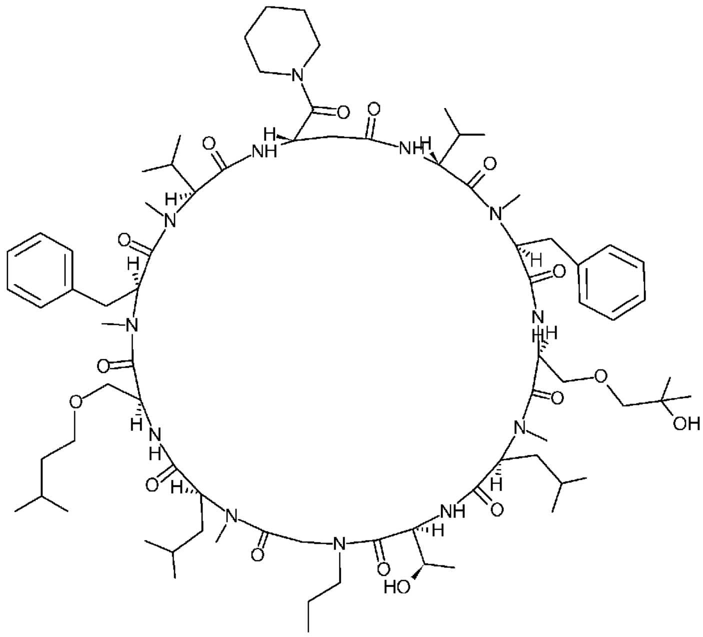 |
| pd427 | 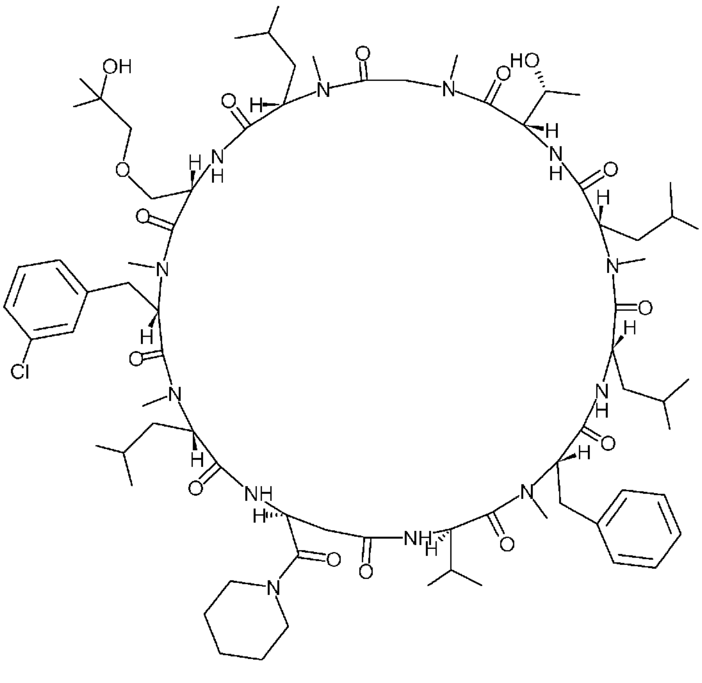 |

| ID | Structure |
|---|---|
| pd428 | 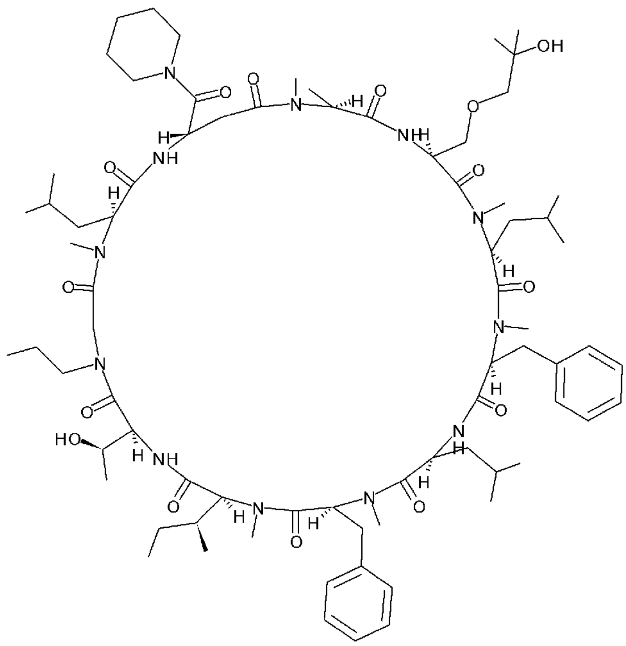 |
| pd429 | 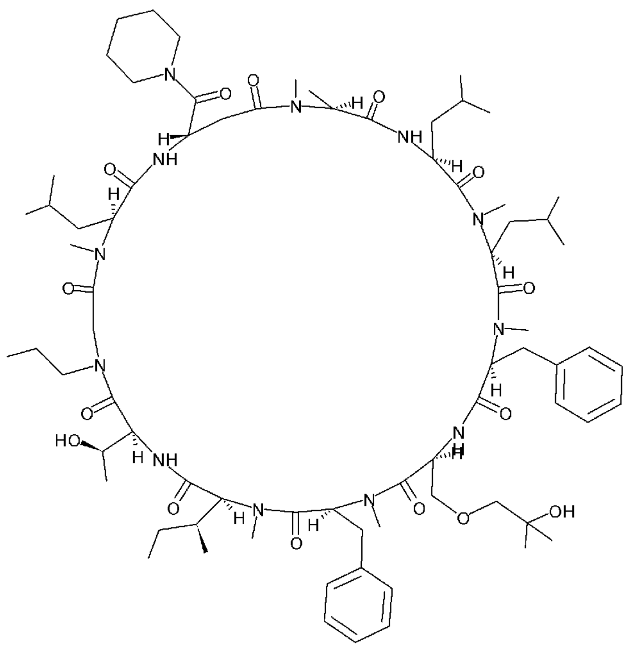 |

| ID | Structure |
|---|---|
| pd430 | 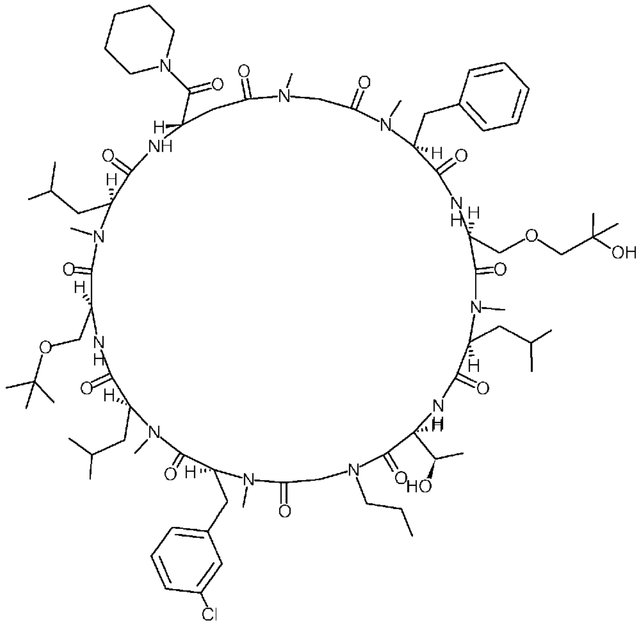 |
| pd431 | 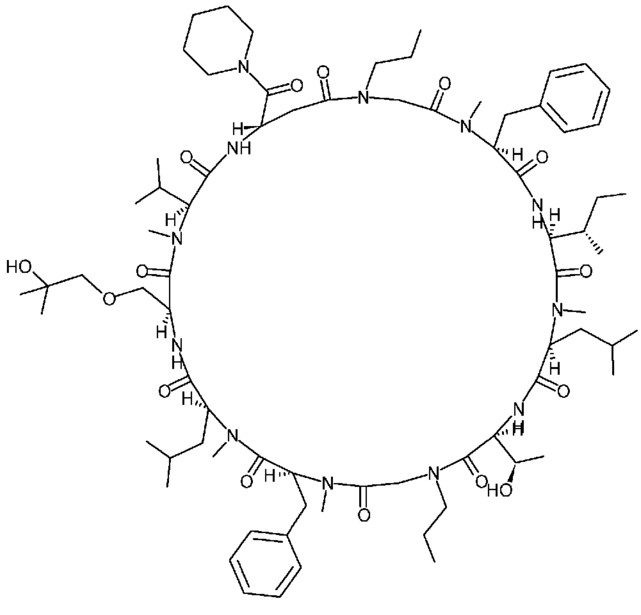 |

| ID | Structure |
|---|---|
| pd432 | 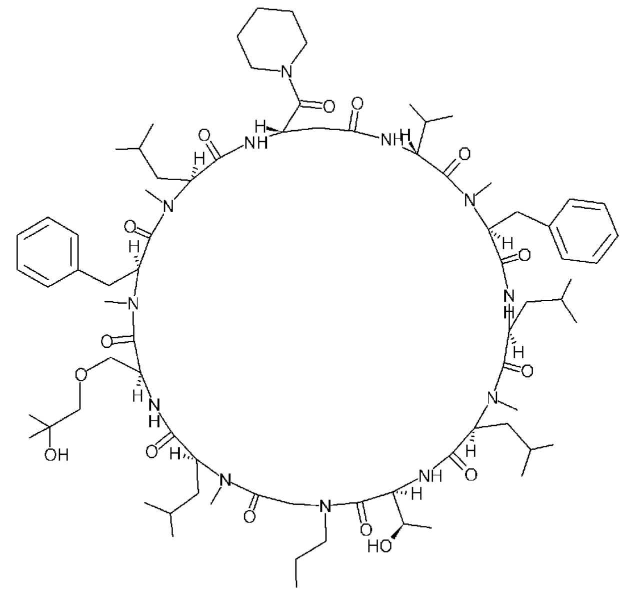 |
| pd433 | 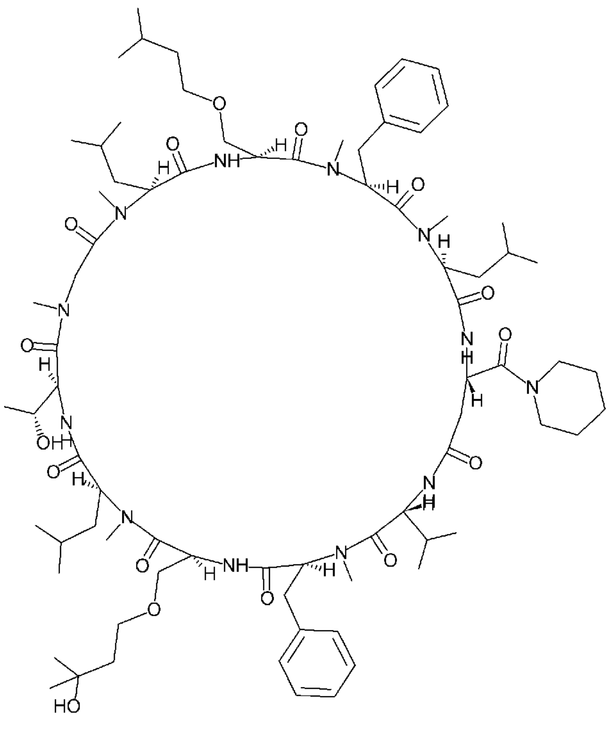 |

| ID | Structure |
|---|---|
| pd434 | 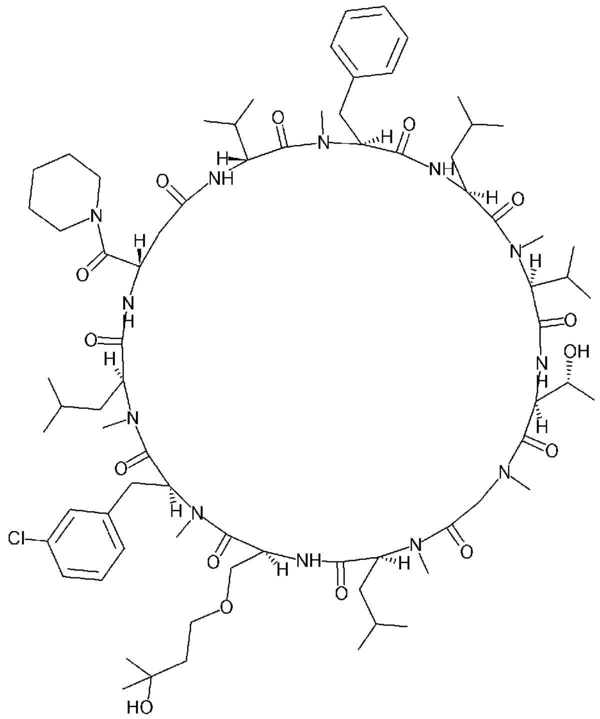 |
| pd435 | 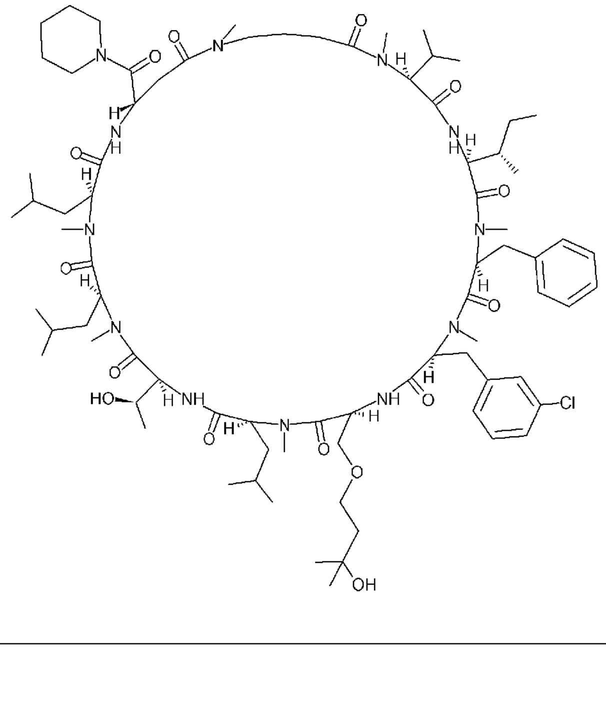 |

| ID | Structure |
|---|---|
| pd436 | 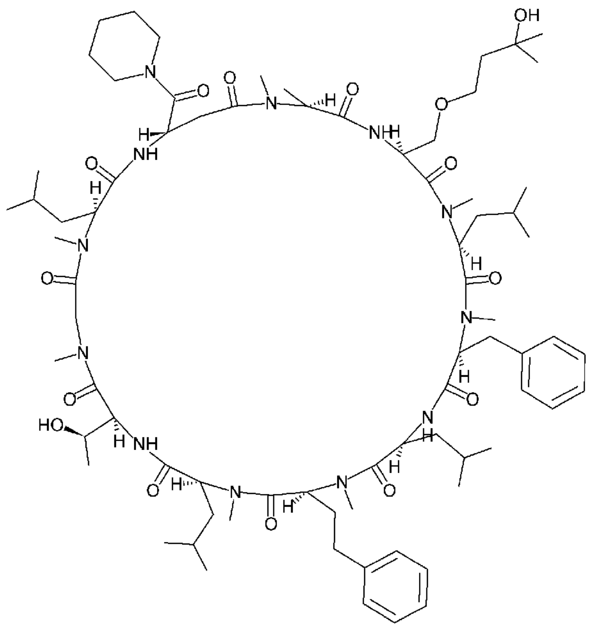 |
| pd437 | 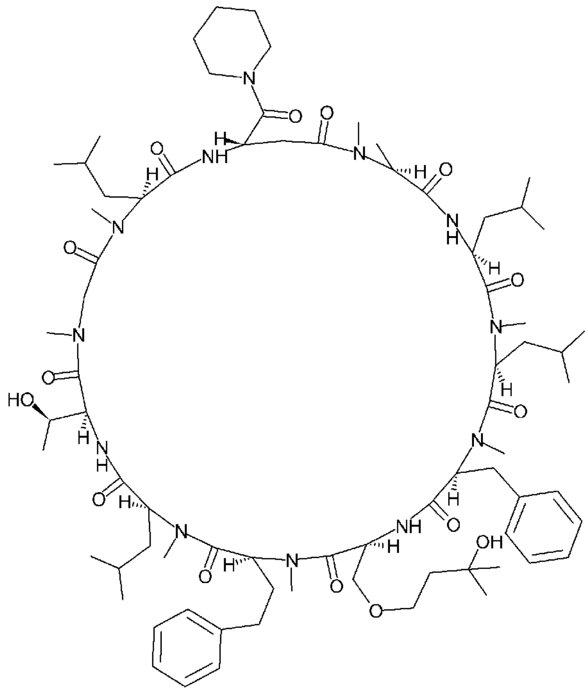 |

| ID | Structure |
|---|---|
| pd438 | 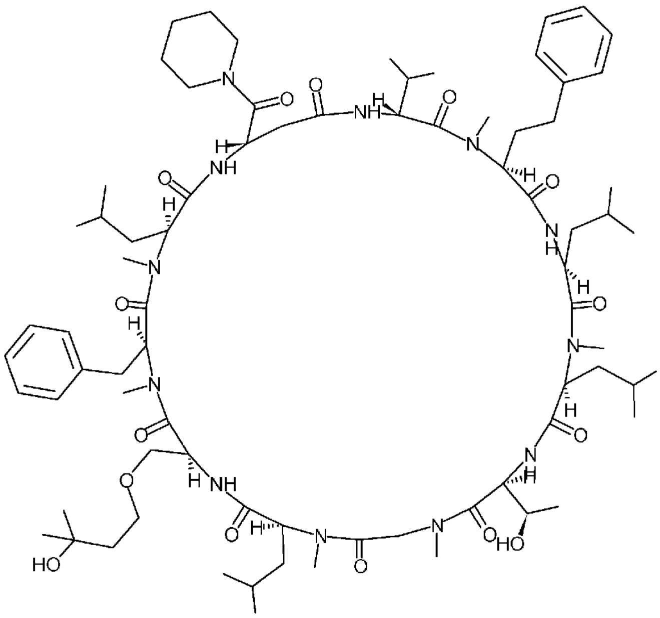 |
| pd439 | 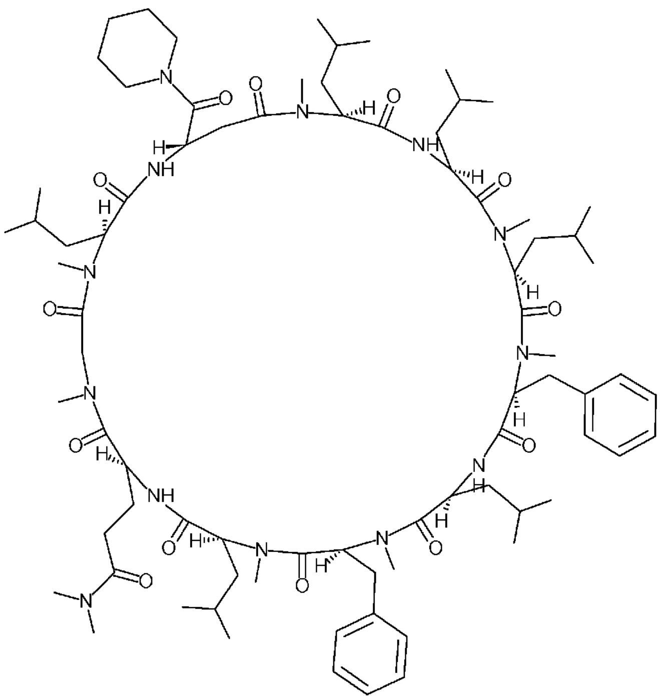 |

| ID | Structure |
|---|---|
| pd440 | 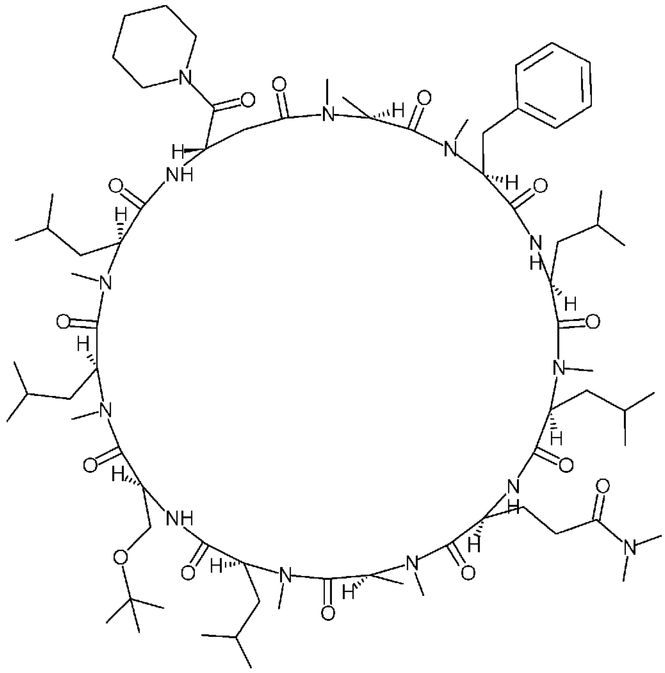 |
| pd441 | 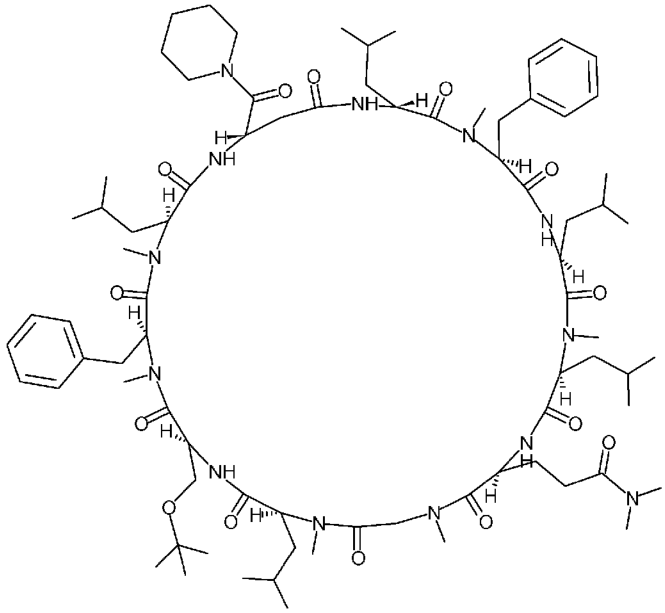 |

| ID | Structure |
|---|---|
| pd442 | 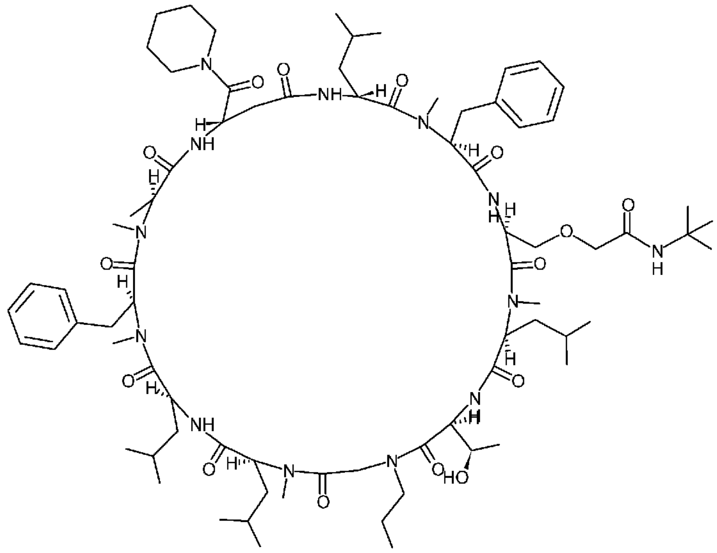 |
| pd443 | 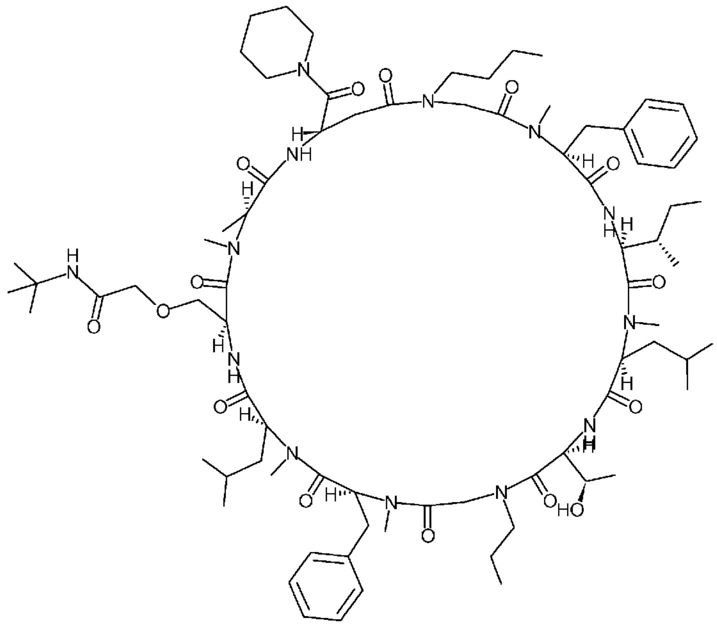 |

| ID | Structure |
|---|---|
| pd444 | 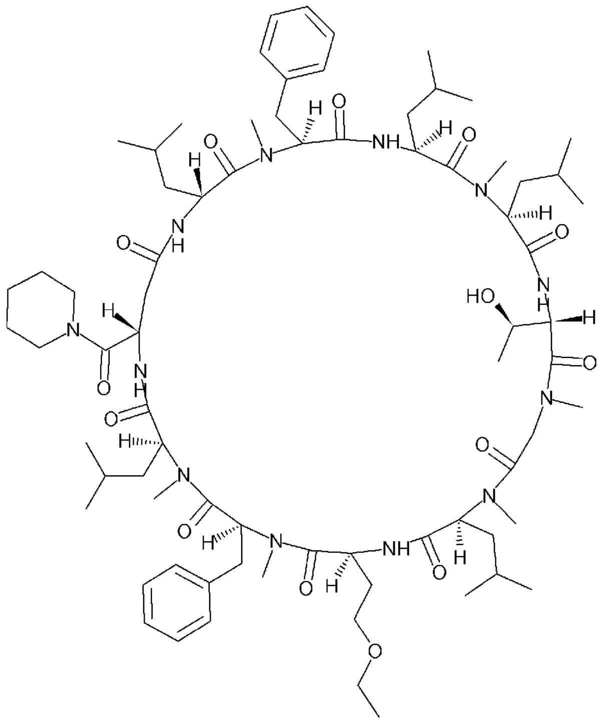 |
| pd445 | 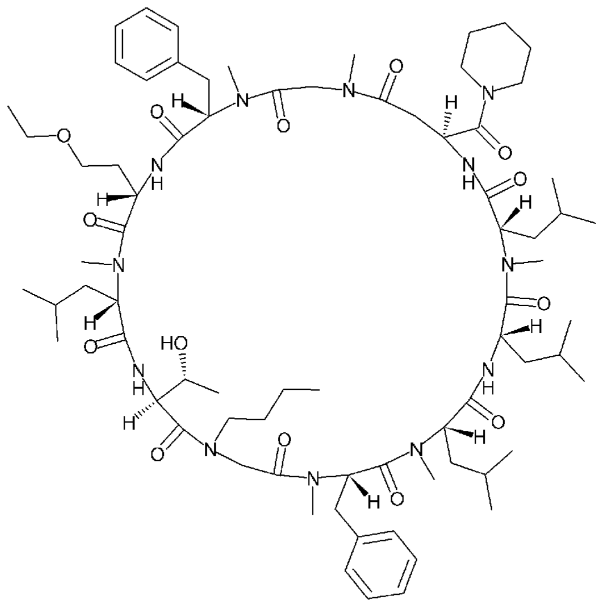 |

| ID | Structure |
| --- | --- |
| pd446 | 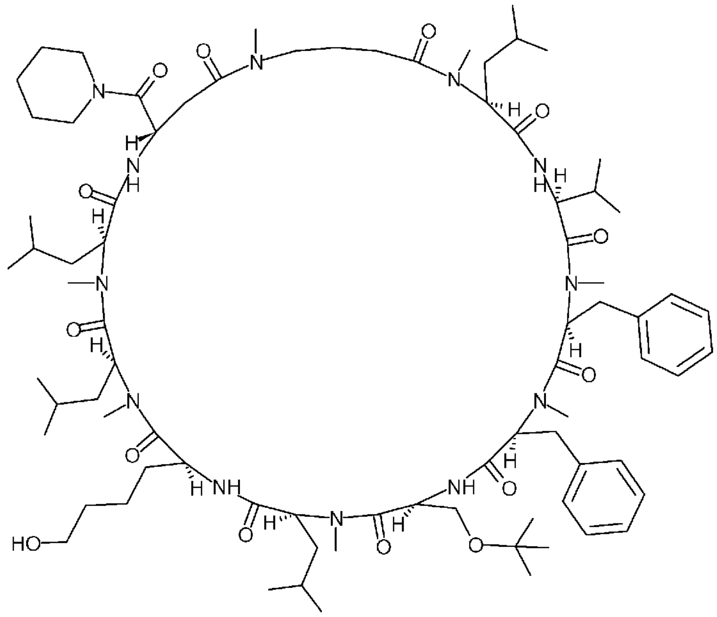 |
| pd447 | 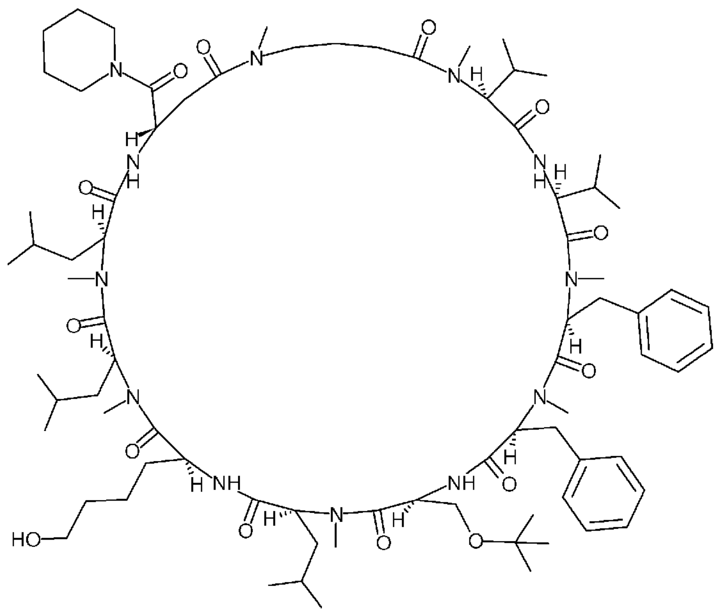 |

| ID | Structure |
|---|---|
| pd448 | 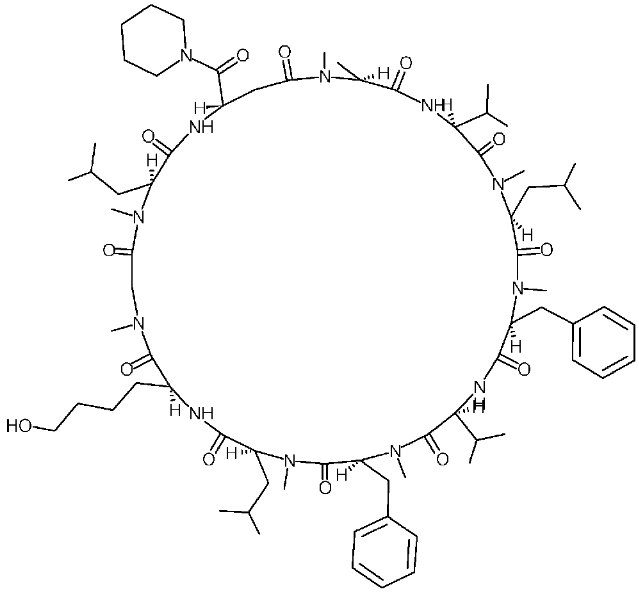 |
| pd449 | 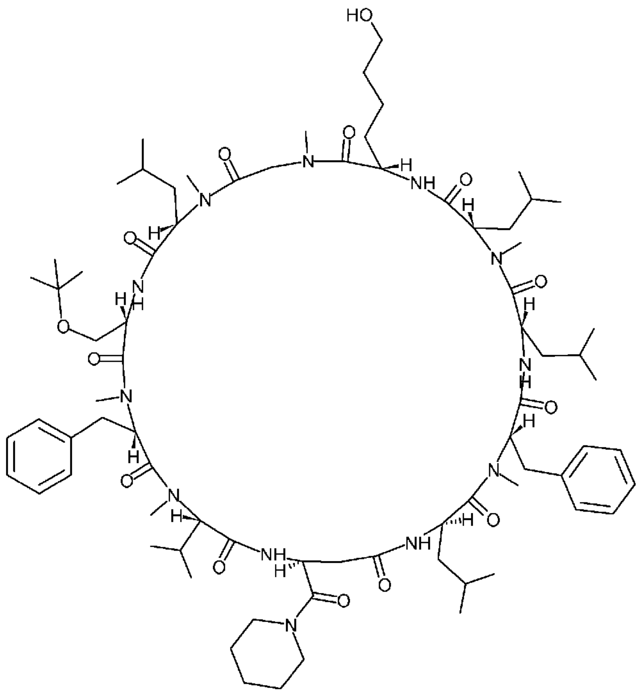 |

| ID | Structure |
|---|---|
| pd450 | 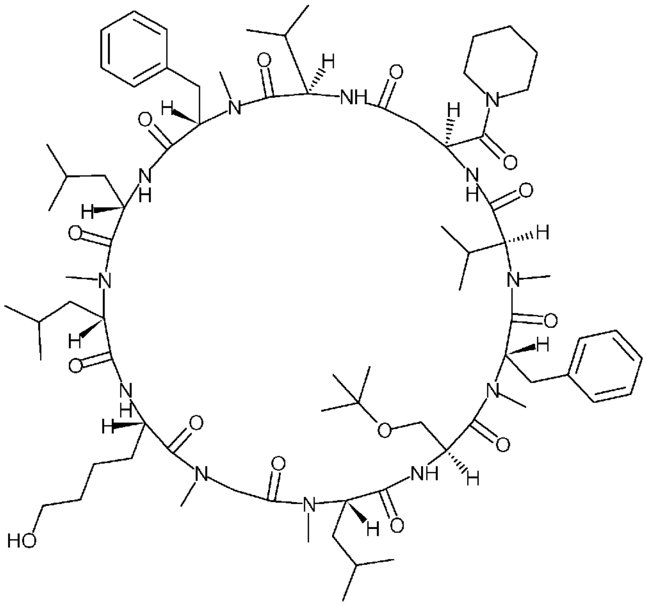 |
| pd451 | 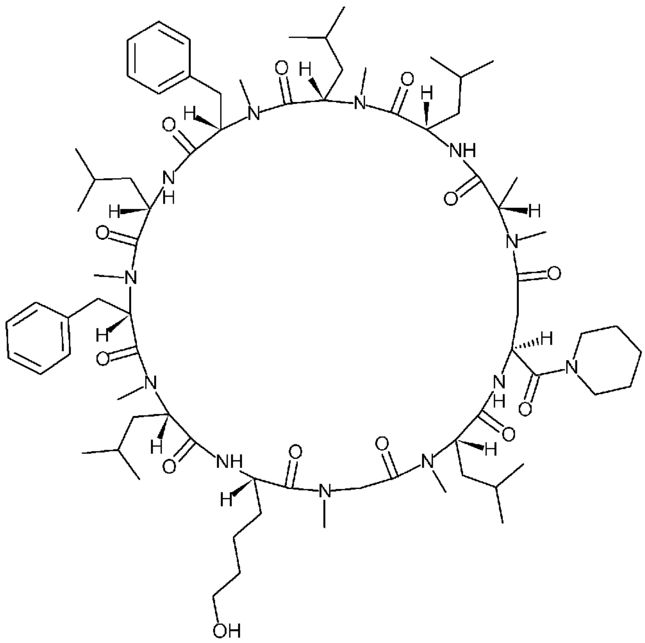 |

| ID | Structure |
|---|---|
| pd452 | 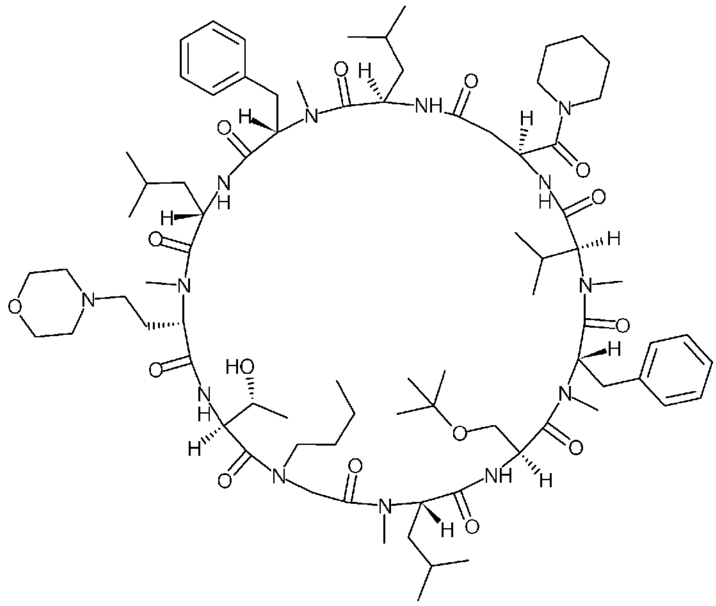 |
| pd453 | 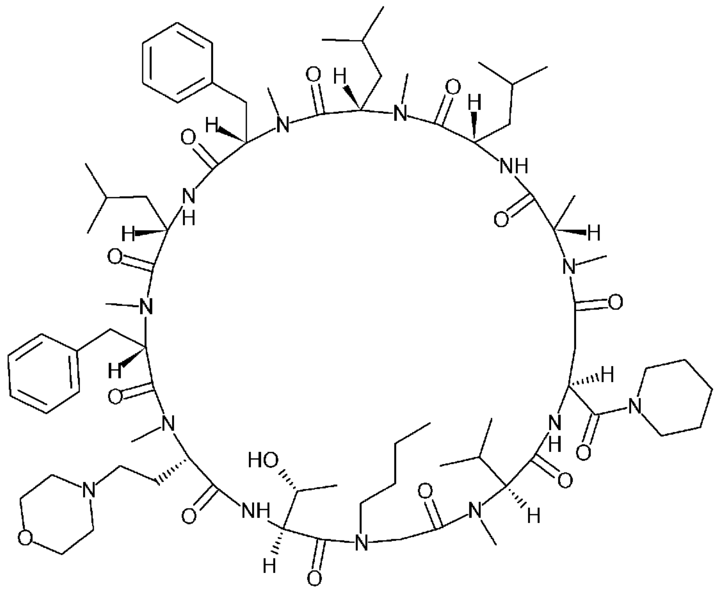 |

| ID | Structure |
|---|---|
| pd454 | 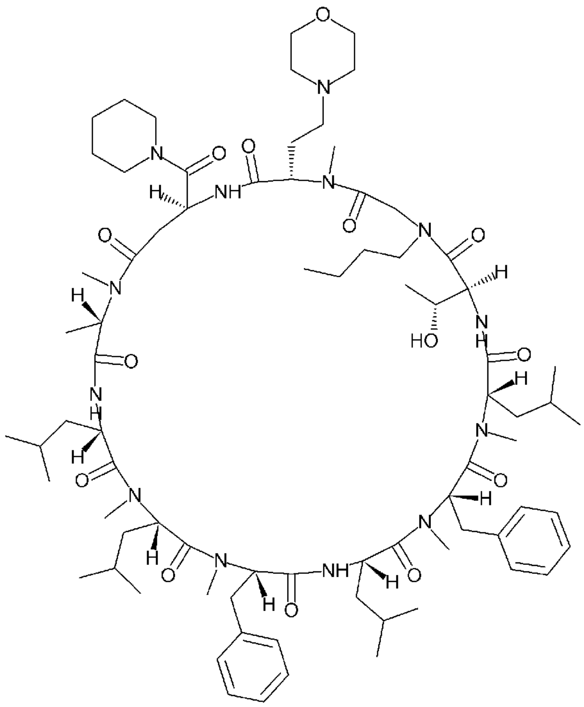 |
| pd455 | 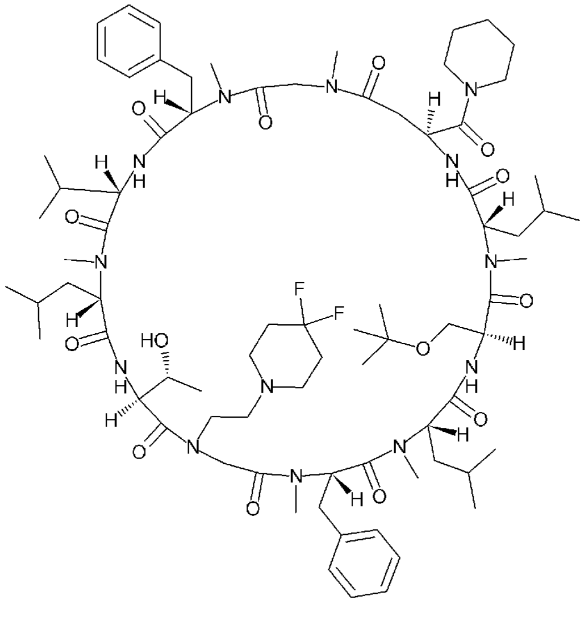 |

| ID | Structure |
| --- | --- |
| pd456 | 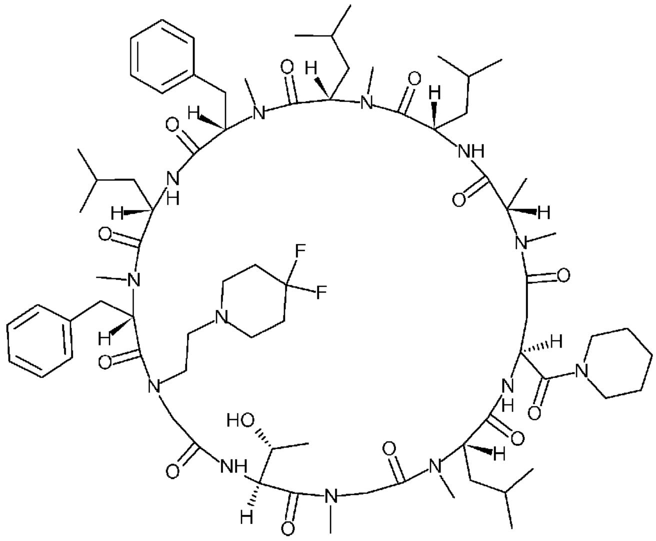 |
| pd457 | 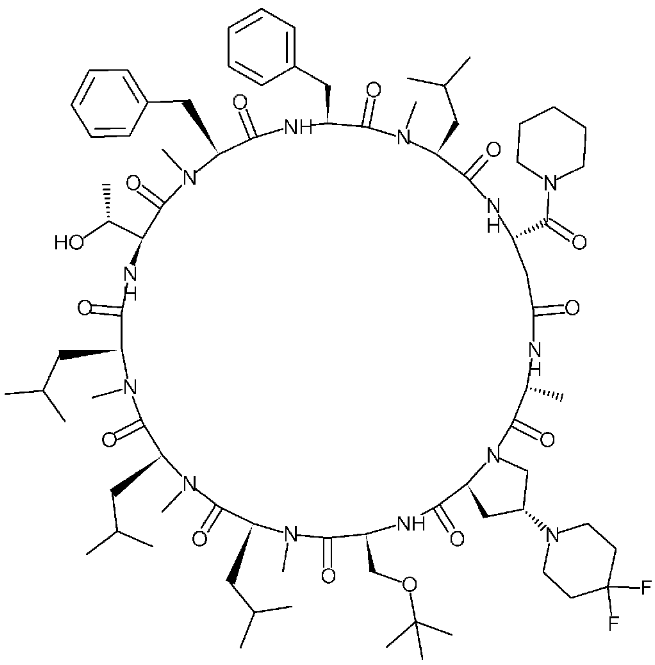 |

| ID | Structure |
|---|---|
| pd458 | 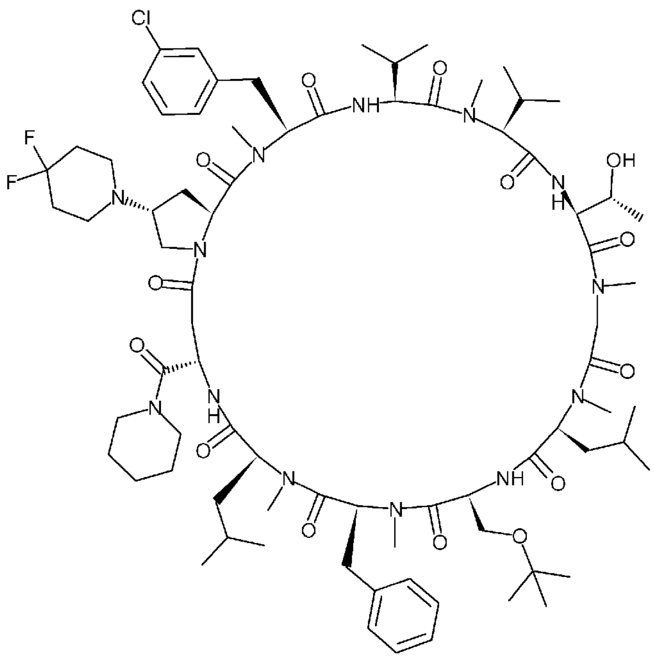 |
| pd459 | 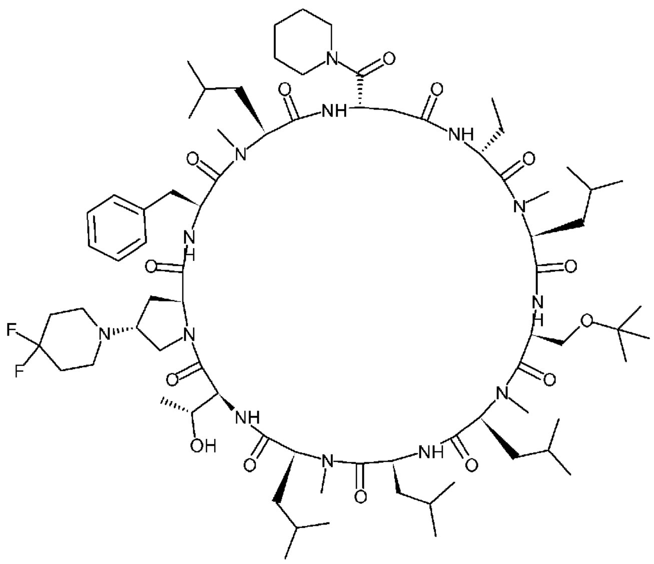 |

| ID | Structure |
|---|---|
| pd460 | 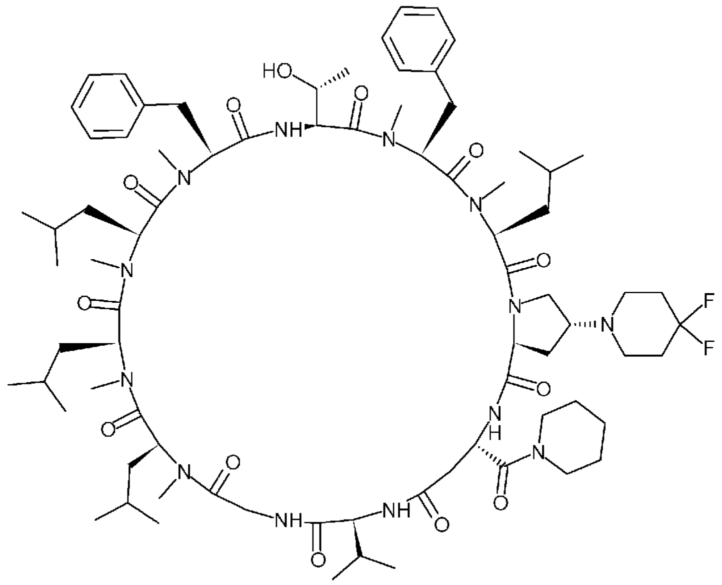 |
| pd461 | 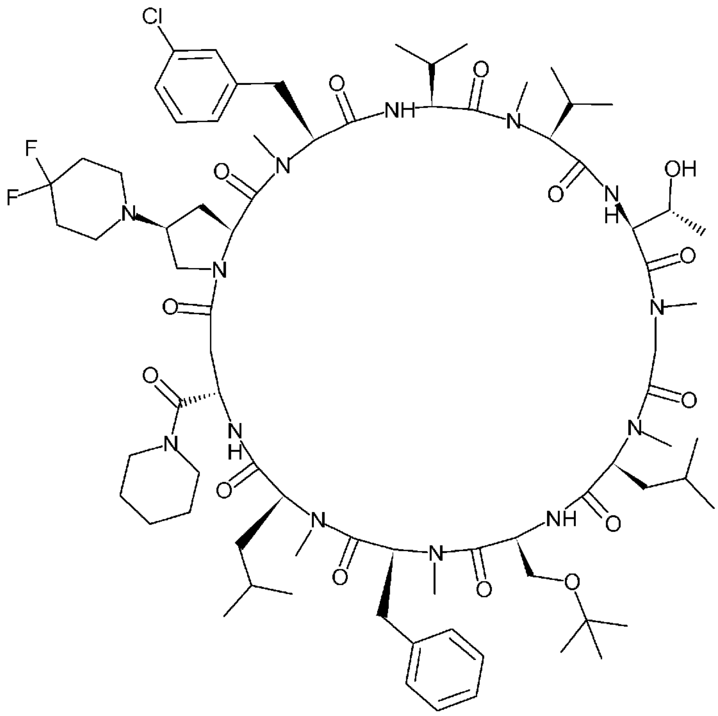 |

| ID | Structure |
| --- | --- |
| pd462 | 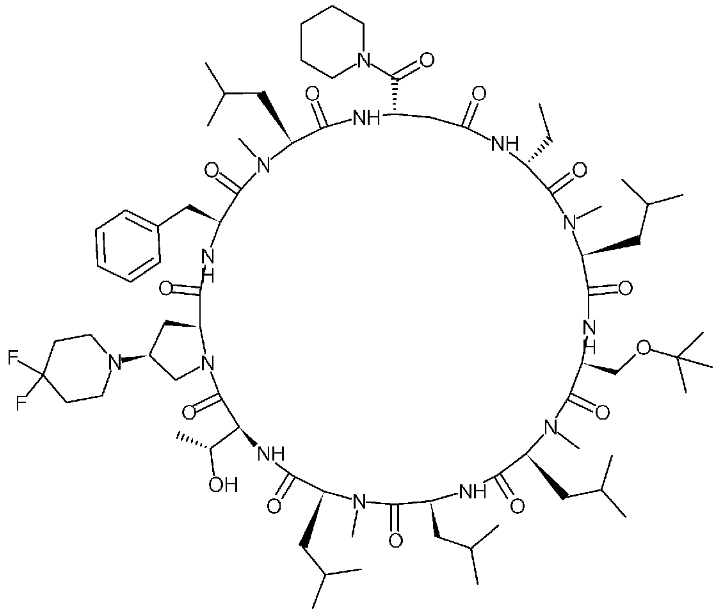 |
| pd463 | 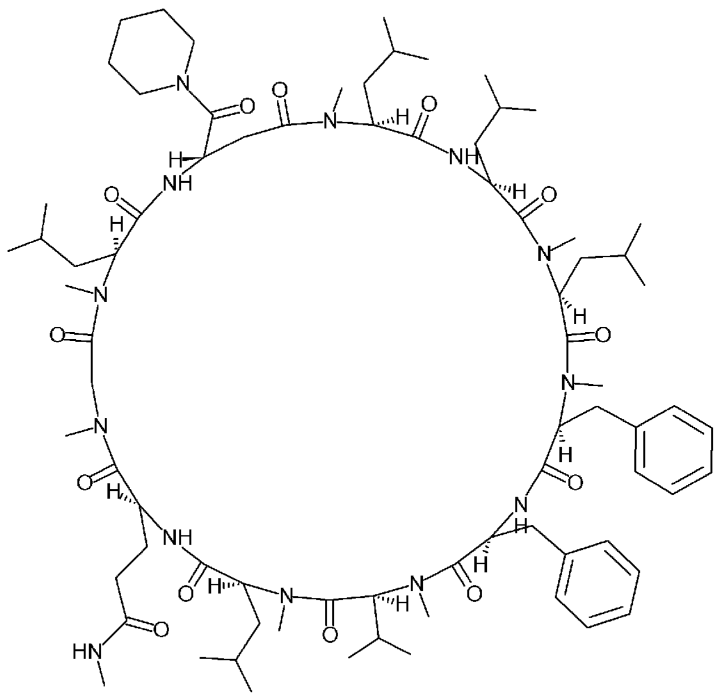 |

| ID | Structure |
|---|---|
| pd464 | 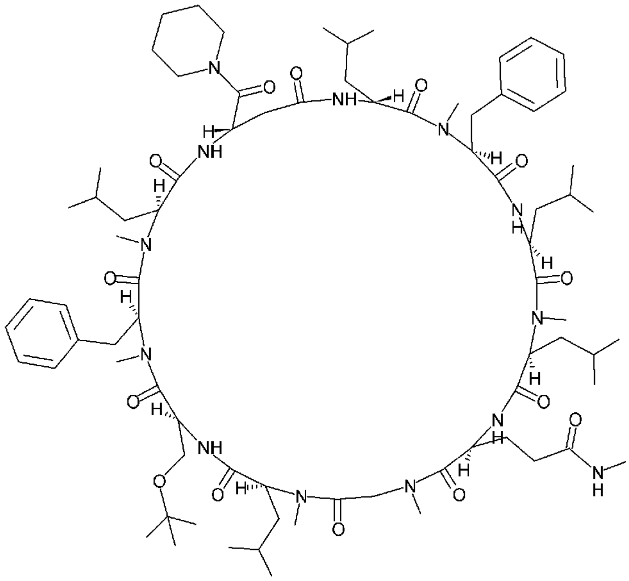 |
| pd465 | 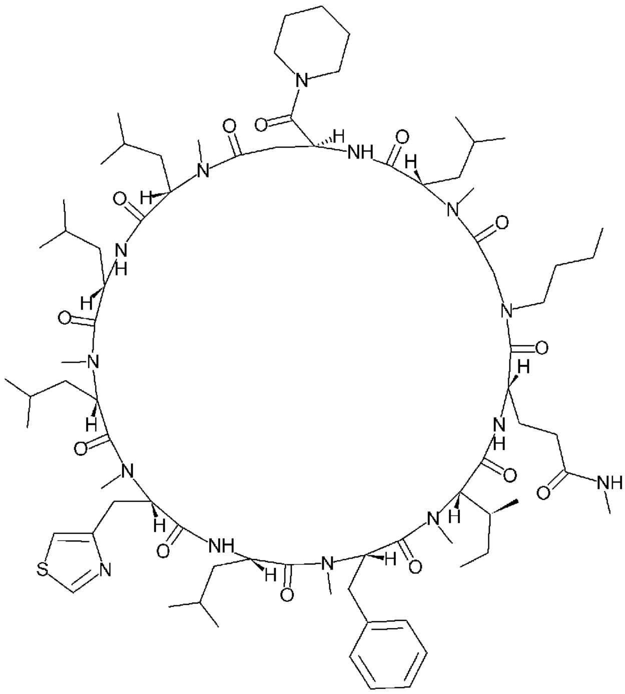 |

| ID | Structure |
|---|---|
| pd466 | 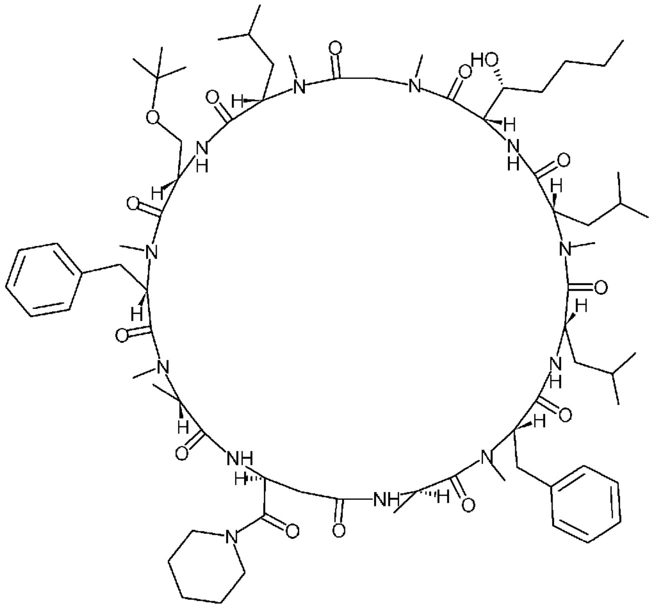 |
| pd467 | 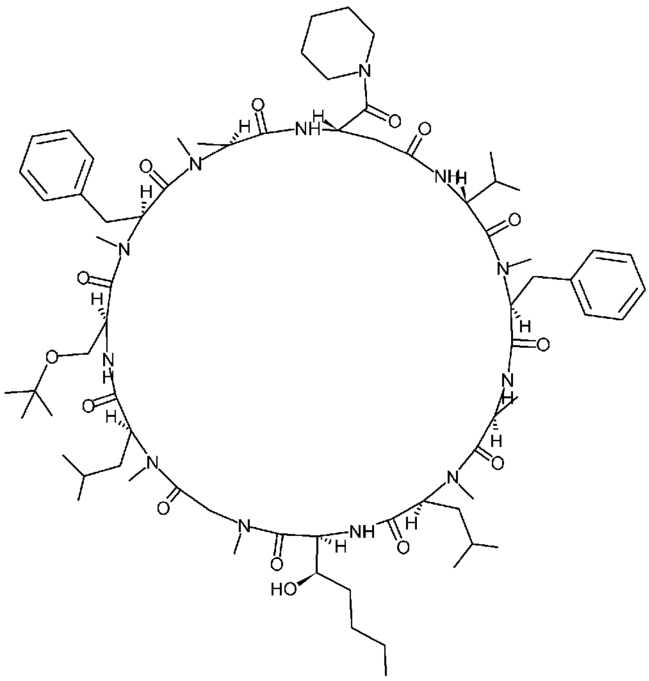 |

| ID | Structure |
|---|---|
| pd468 | 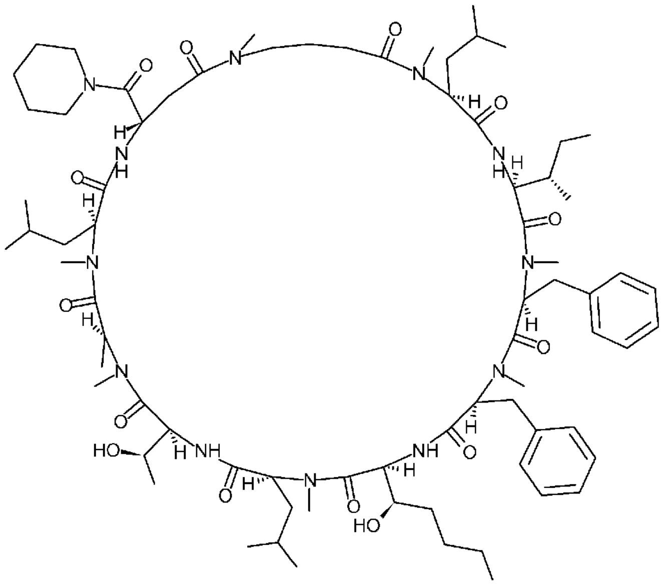 |
| pd469 | 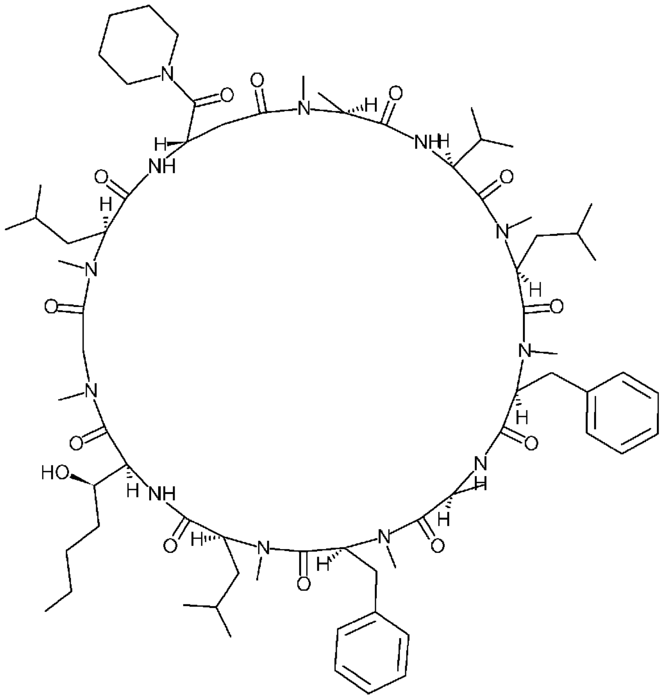 |

| ID | Structure |
|---|---|
| pd470 | 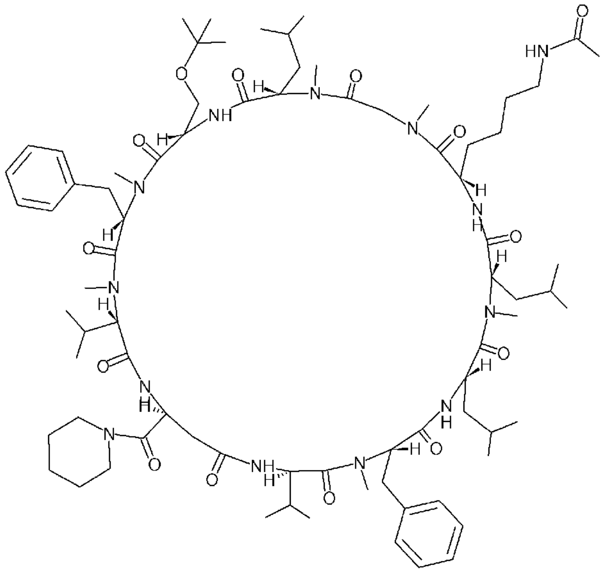 |
| pd471 | 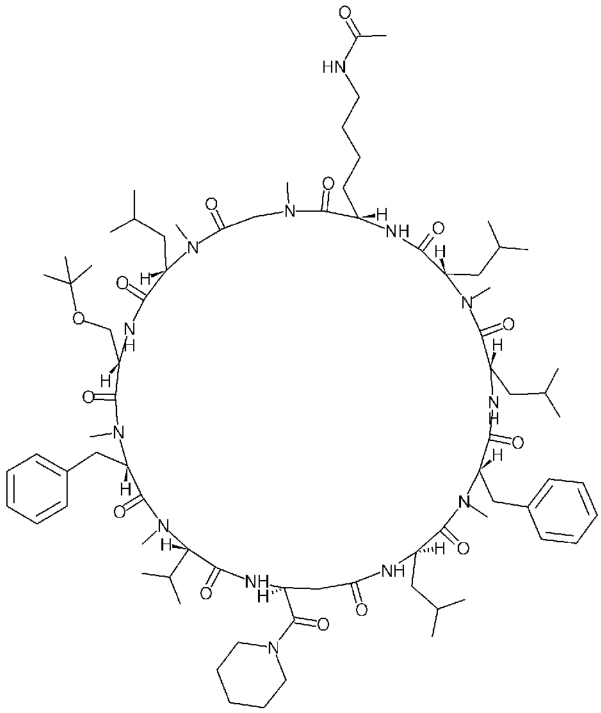 |

| ID | Structure |
|---|---|
| pd472 | 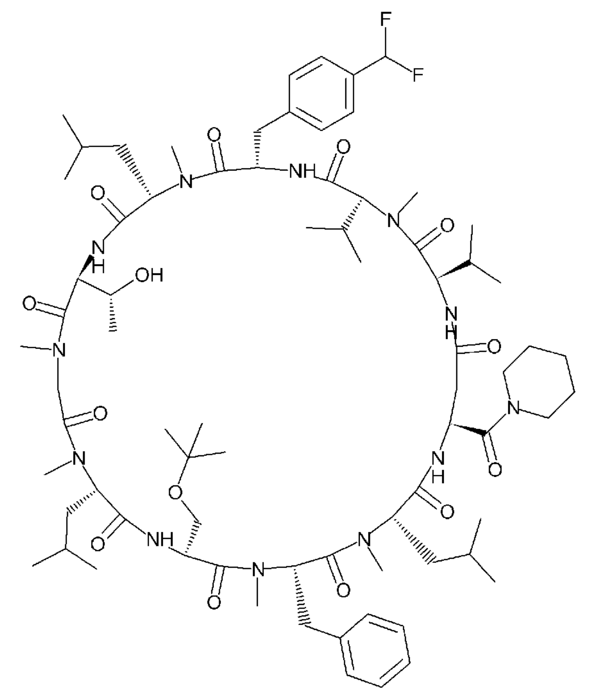 |
| pd473 | 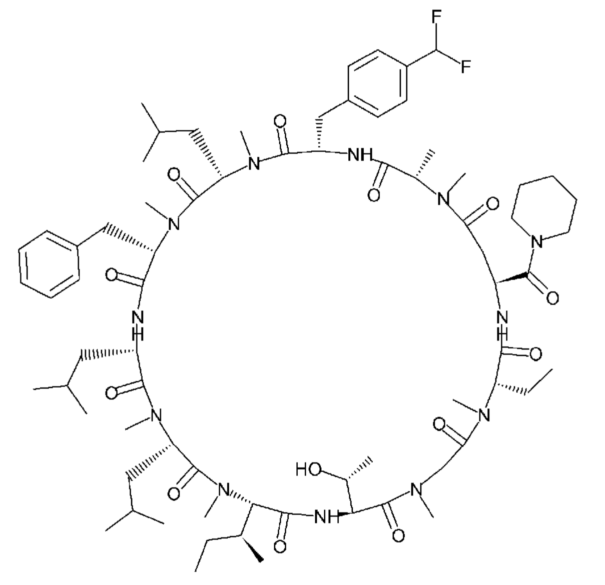 |

| ID | Structure |
|---|---|
| pd474 | 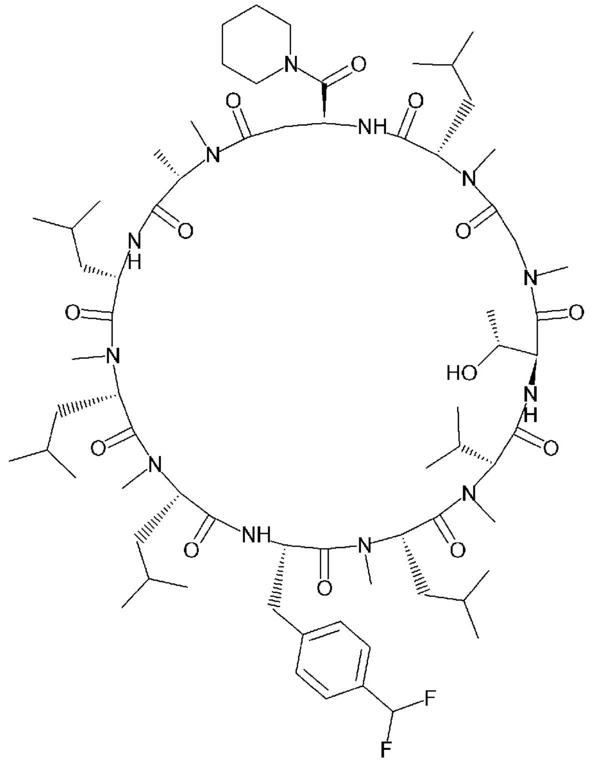 |
| pd475 | 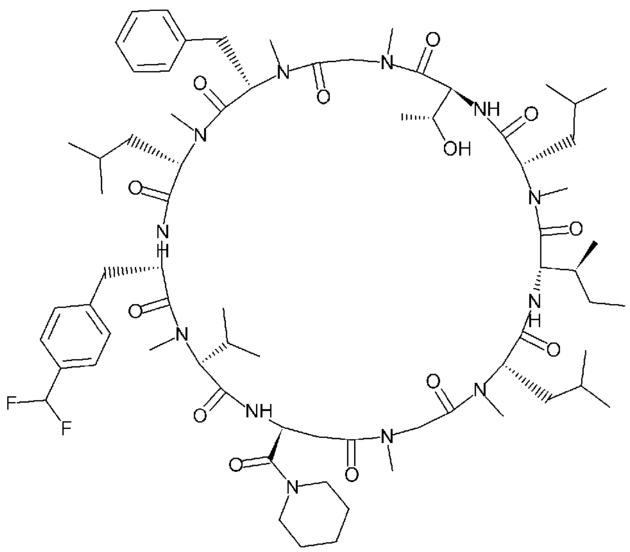 |

| ID | Structure |
|---|---|
| pd476 | 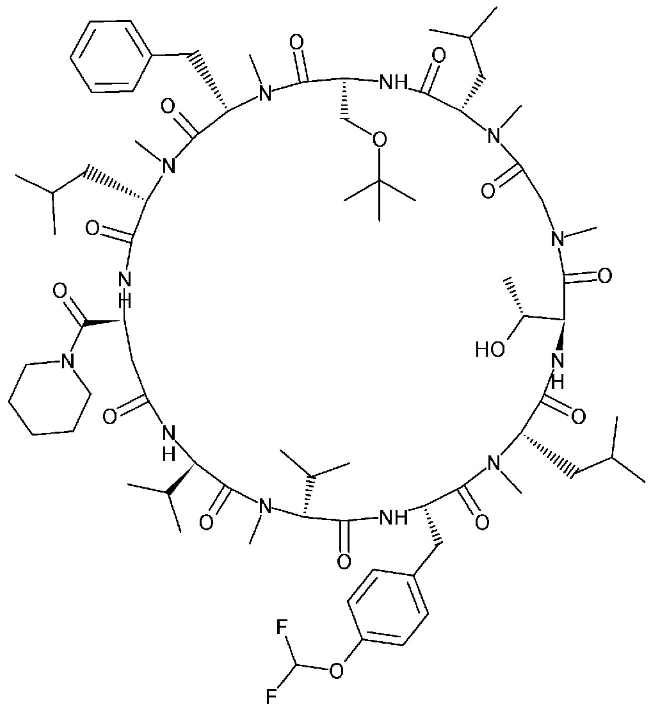 |
| pd477 | 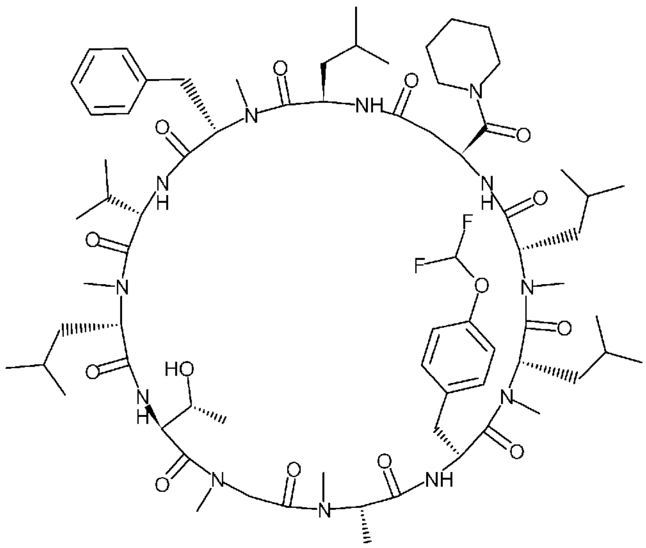 |

| ID | Structure |
|---|---|
| pd478 | 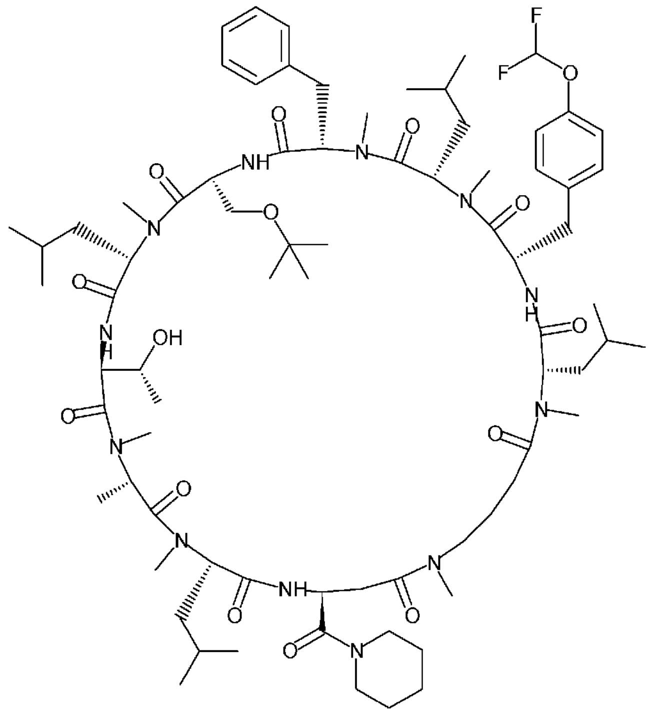 |
| pd479 | 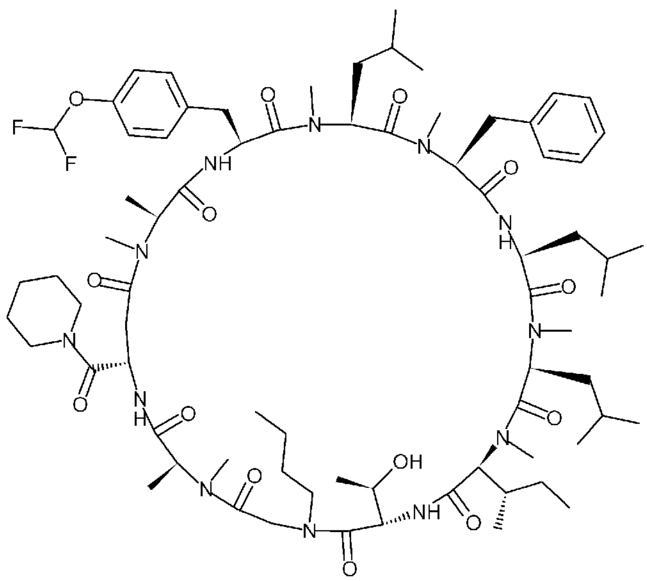 |

| ID | Structure |
|---|---|
| pd480 | 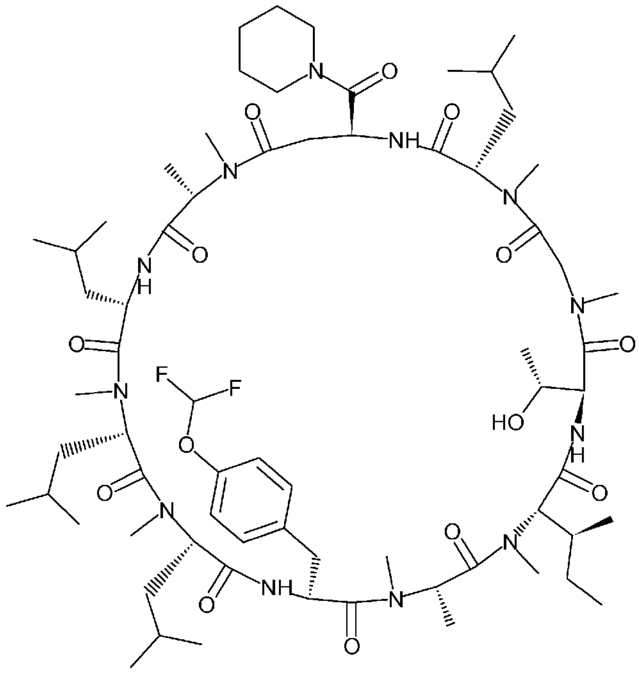 |
| pd481 | 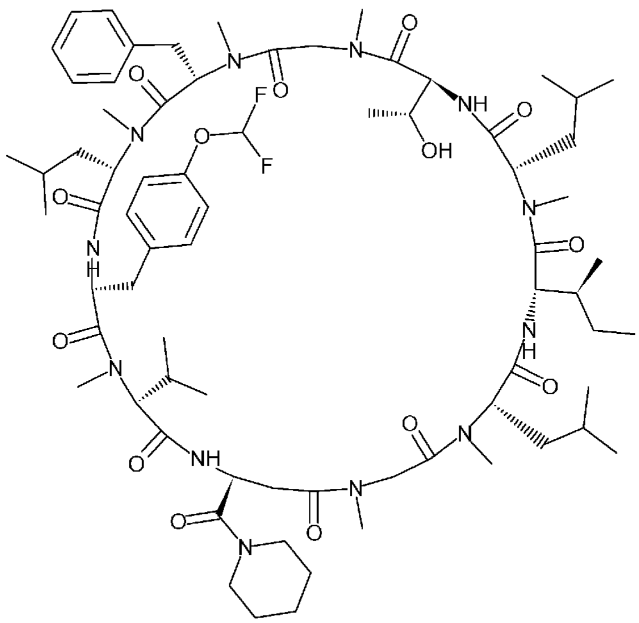 |

| ID | Structure |
|---|---|
| pd482 | 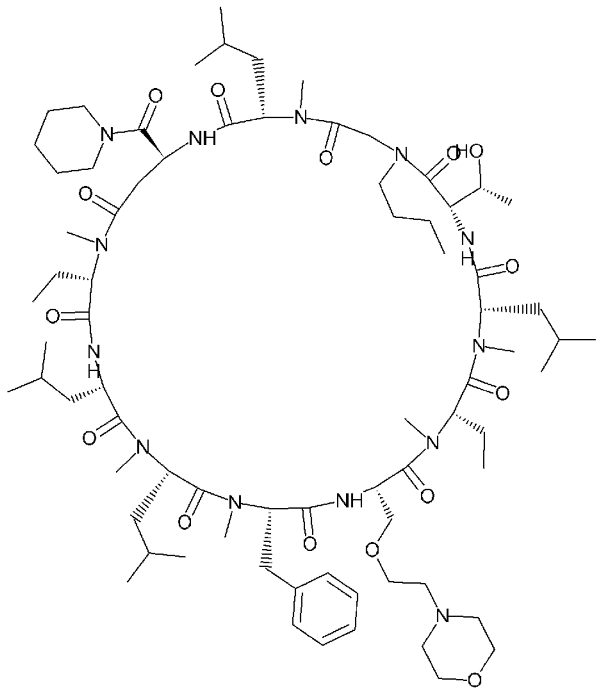 |
| pd483 | 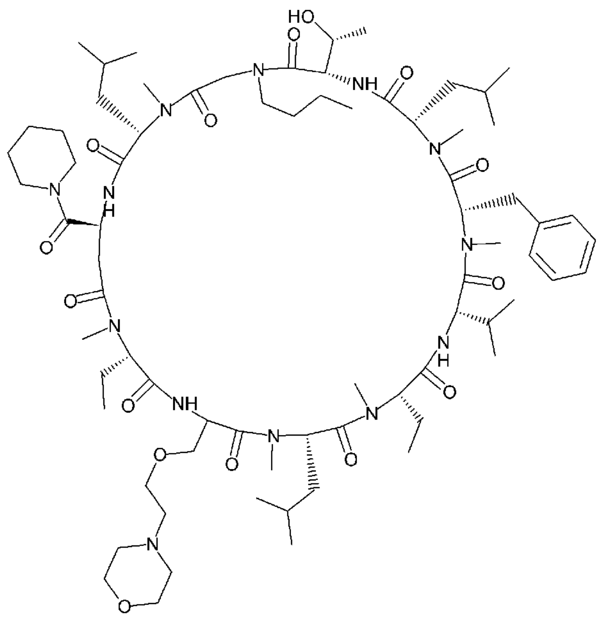 |

| ID | Structure |
|---|---|
| pd484 | 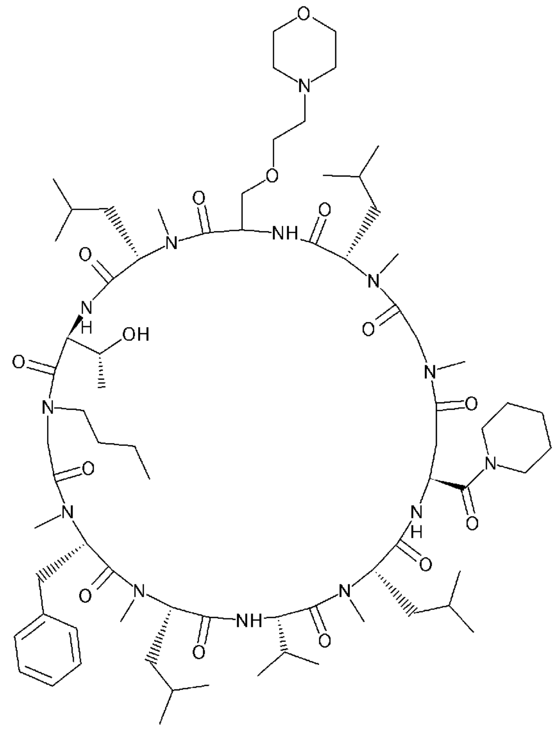 |
| pd485 | 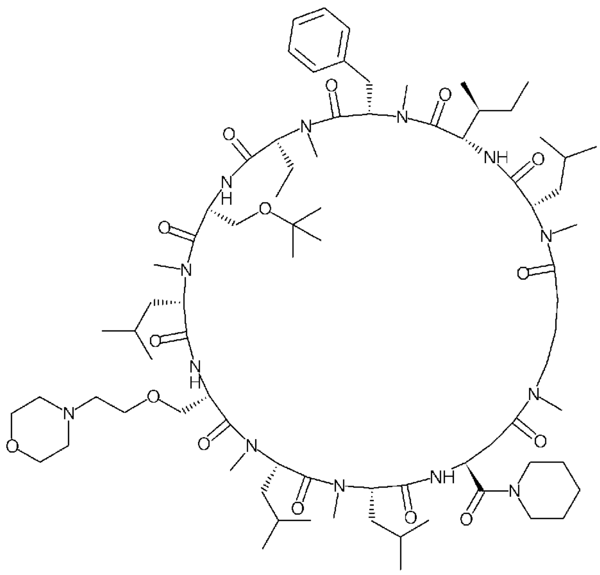 |

| ID | Structure |
|---|---|
| pd486 | 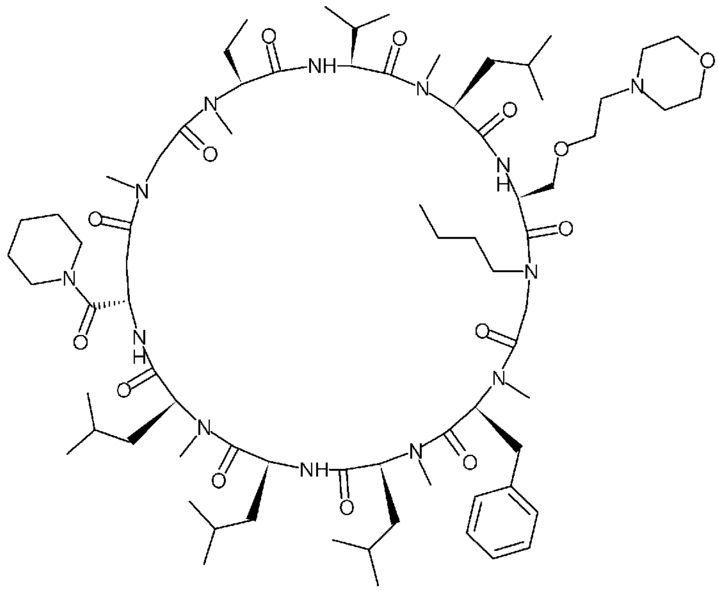 |
| pd487 | 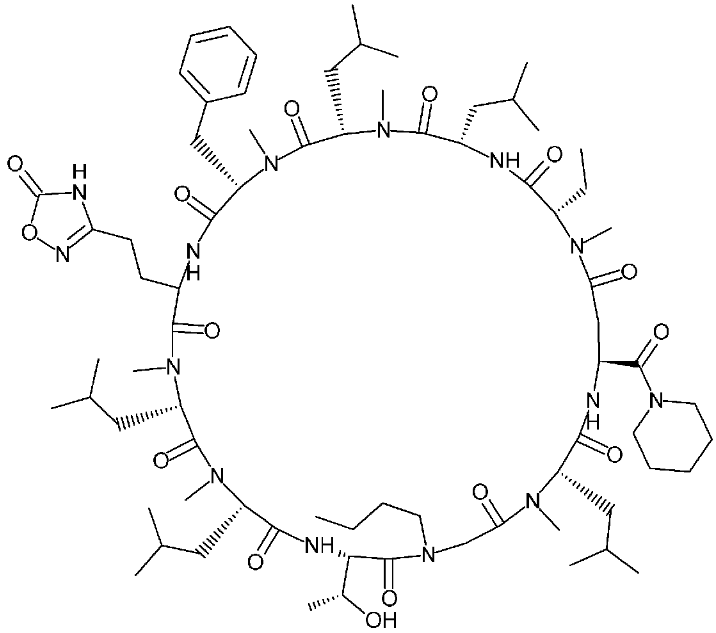 |

| ID | structure |
|---|---|
| **pd488** | 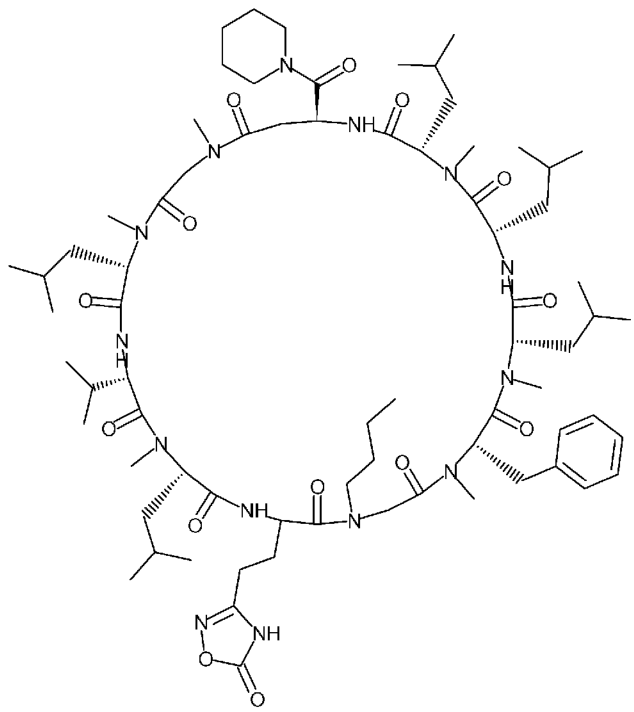 |
| **pd489** | 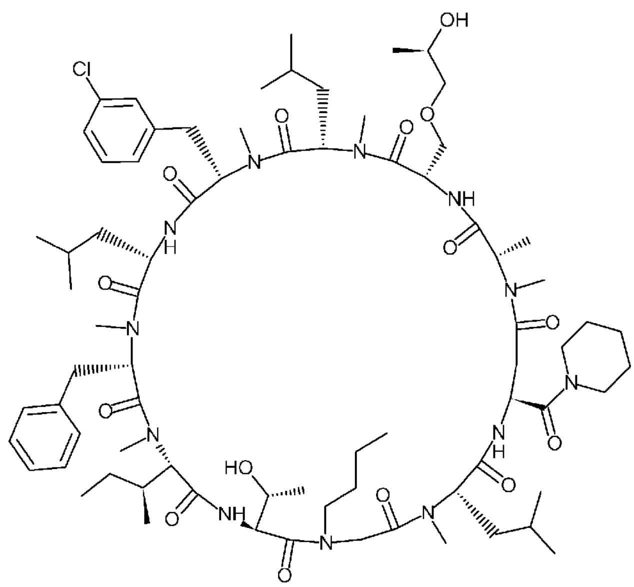 |

| ID | structure |
|---|---|
| pd490 | 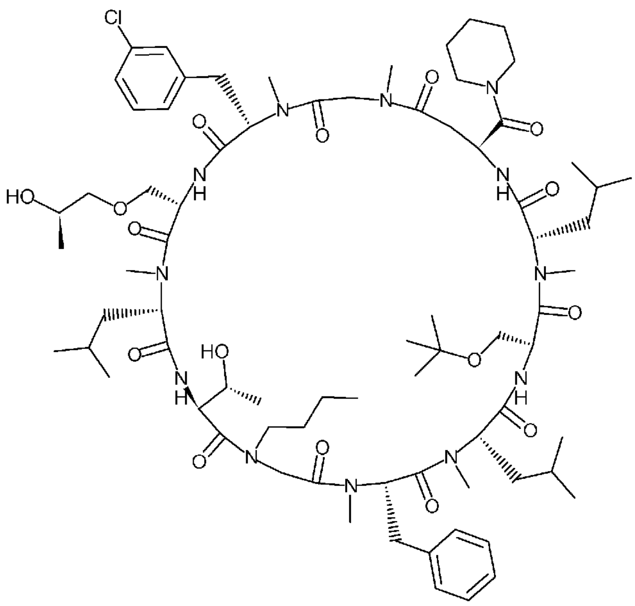 |
| pd491 | 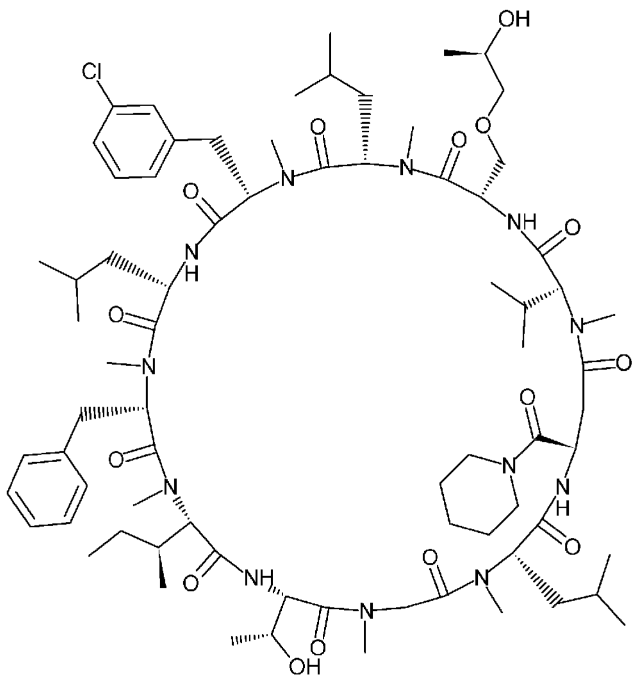 |

| ID | structure |
|---|---|
| pd492 | 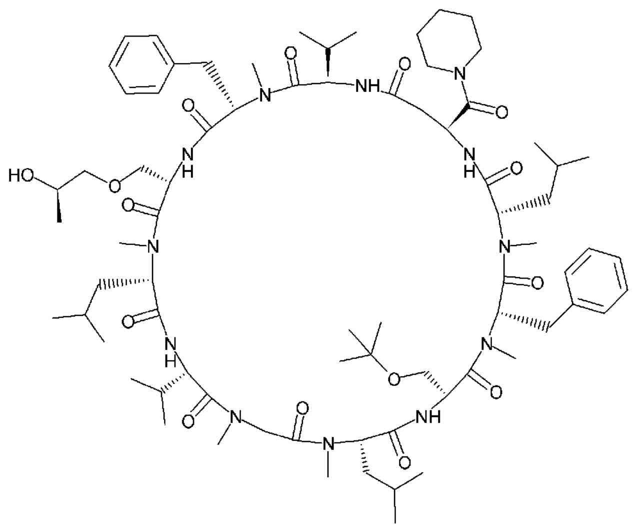 |
| pd493 | 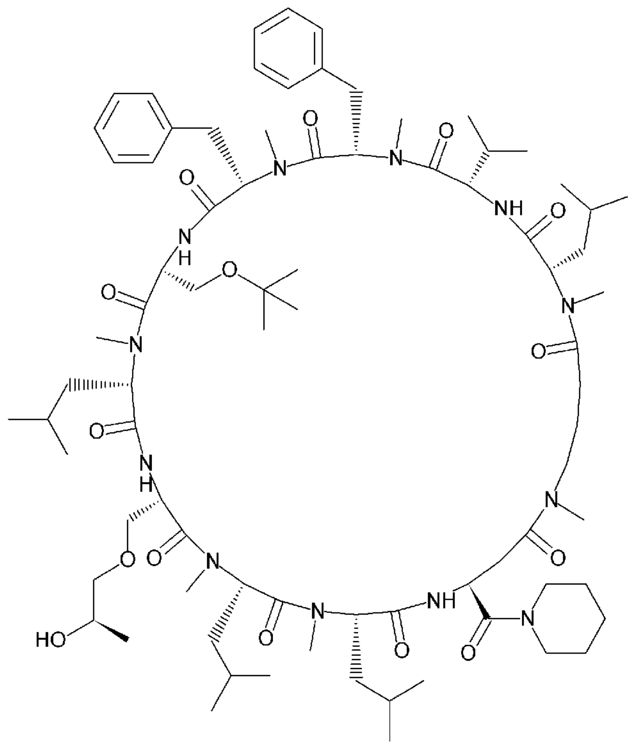 |

| ID | structure |
|---|---|
| pd494 | 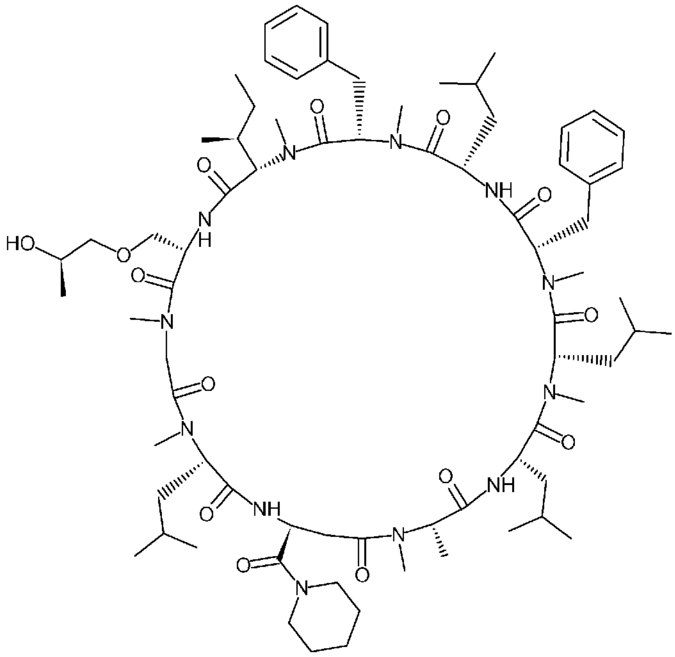 |
| pd495 | 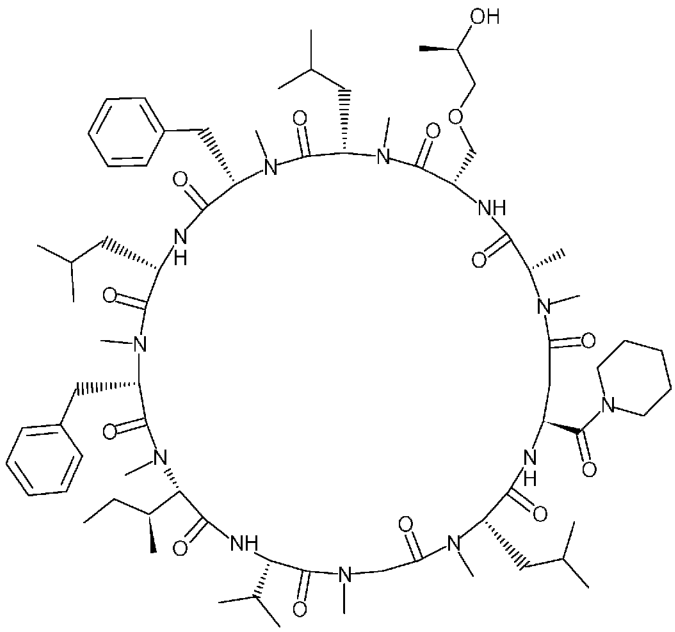 |

| ID | structure |
|---|---|
| pd496 | 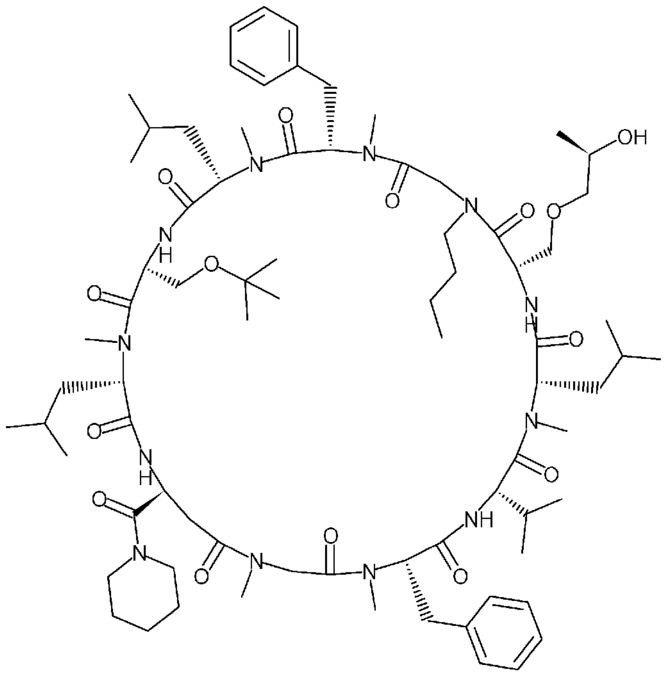 |
| pd497 | 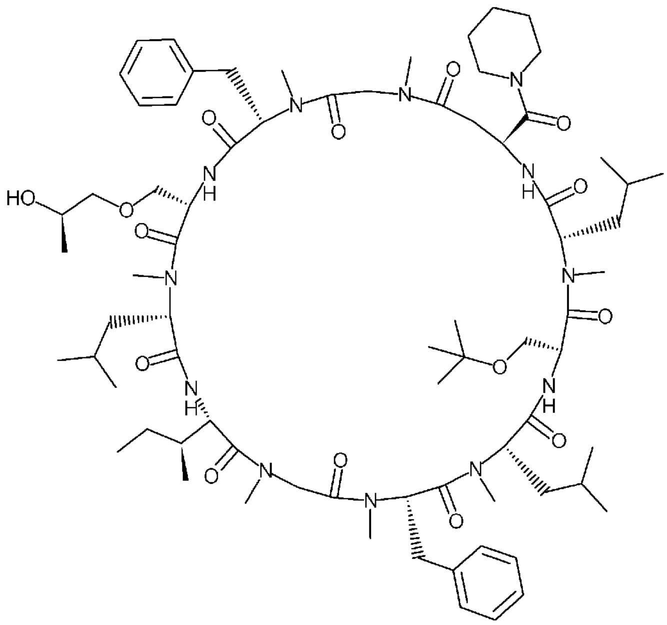 |

| ID | structure |
|---|---|
| pd498 | 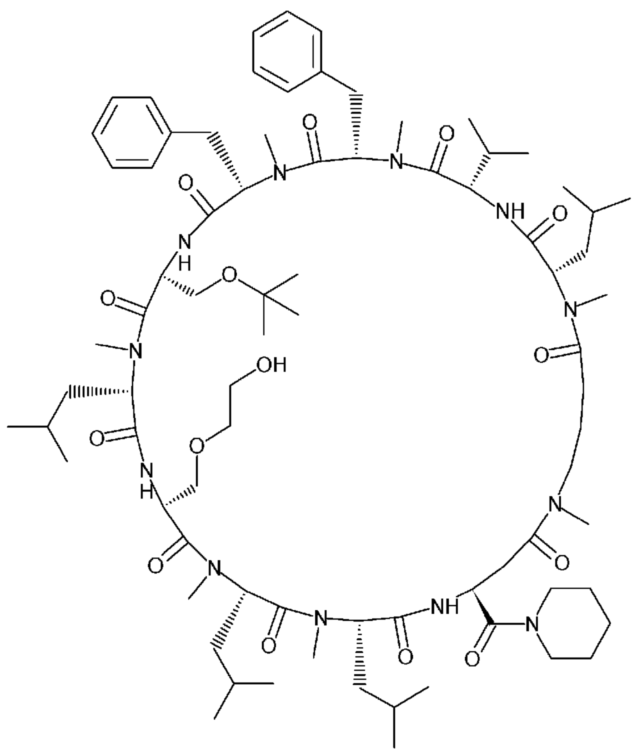 |
| pd499 | 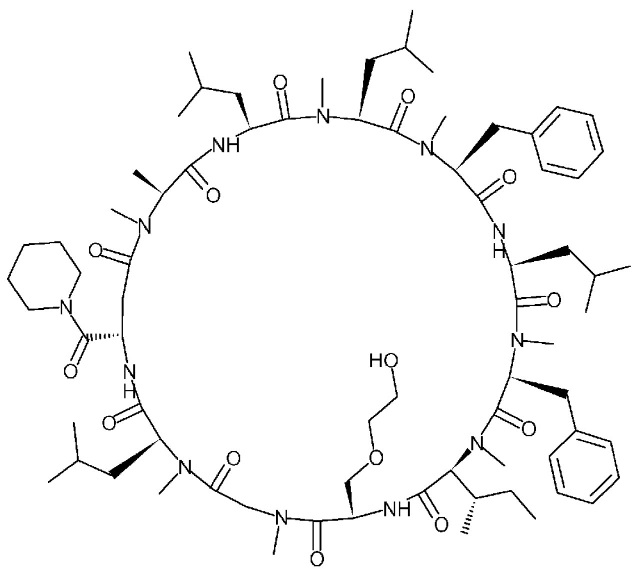 |

| ID | structure |
|---|---|
| pd500 | 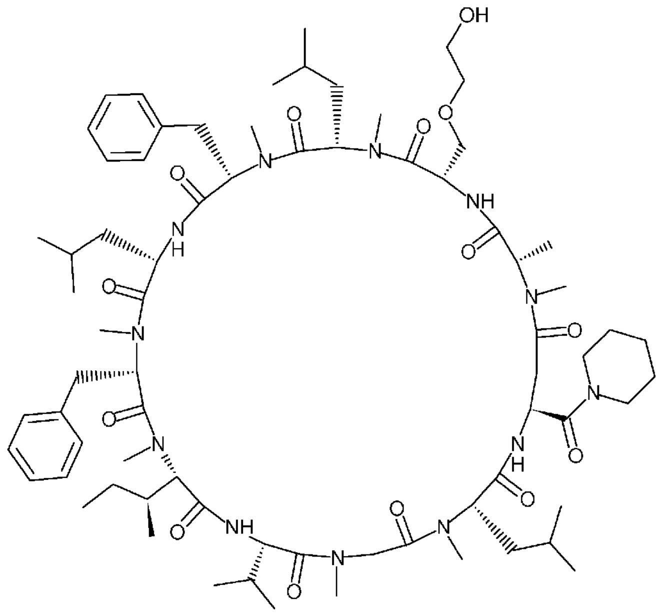 |
| pd501 | 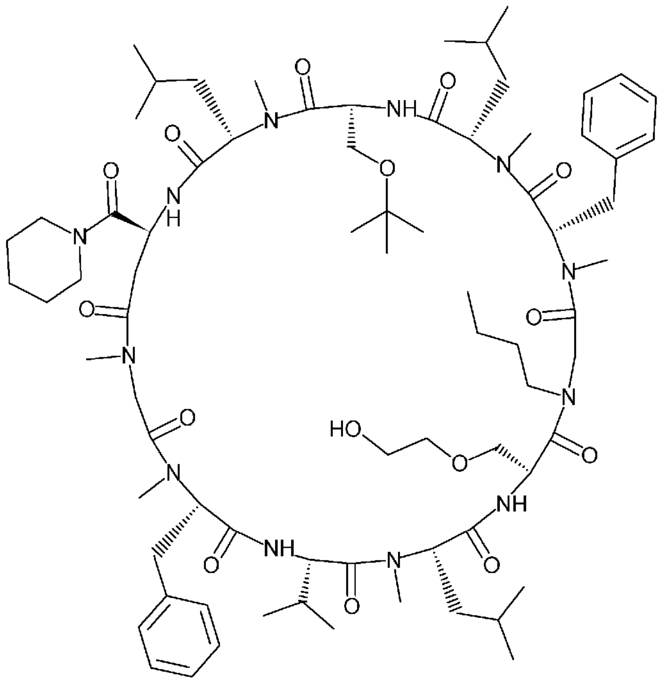 |

| ID | structure |
|---|---|
| pd502 | 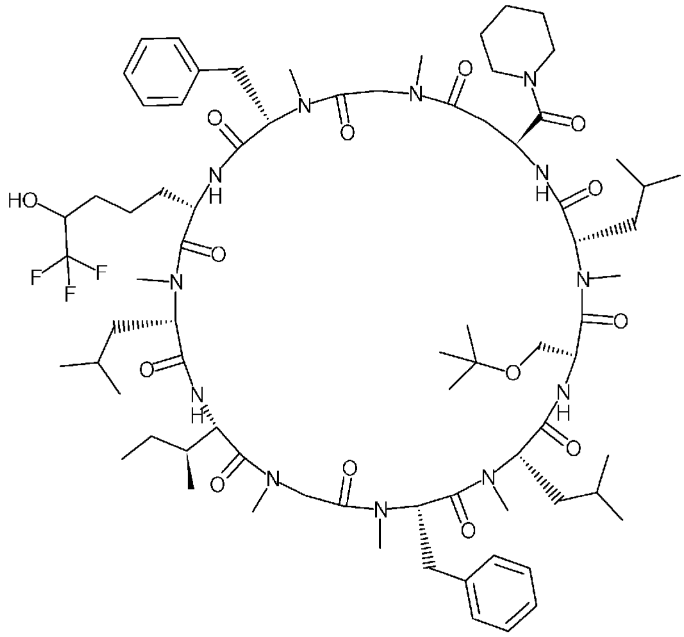 |
| pd503 | 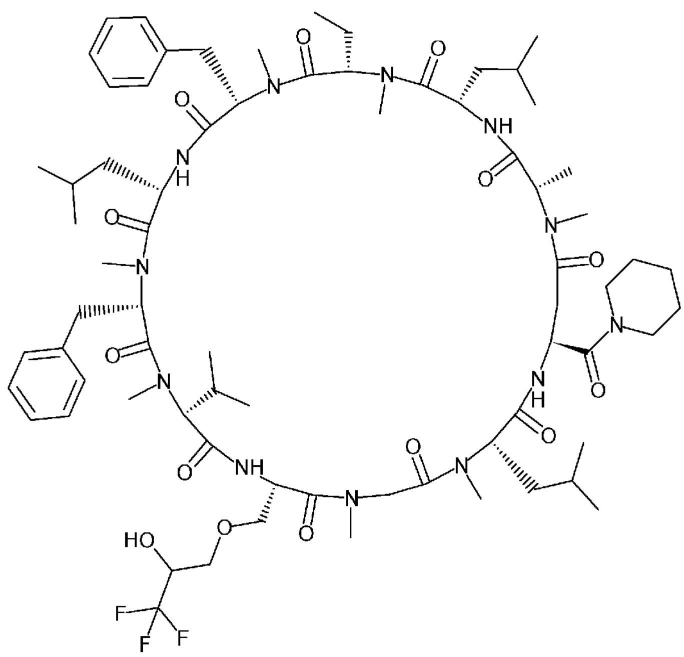 |

| ID | structure |
|---|---|
| pd504 | 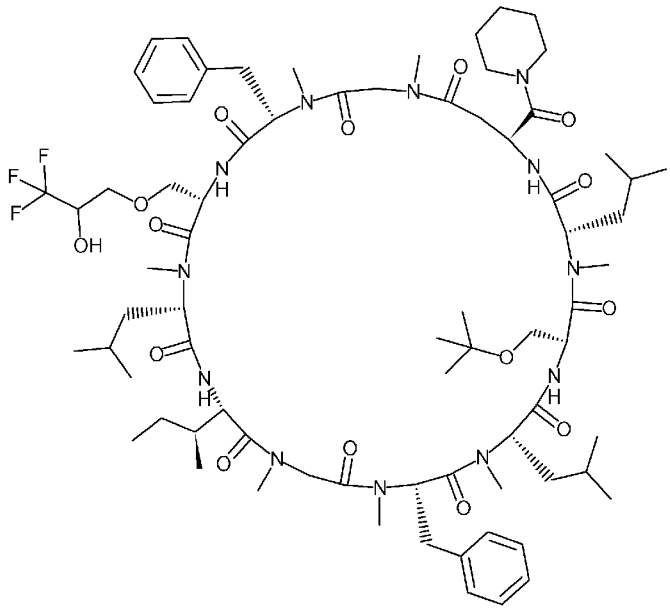 |

Analytical data of a part of cyclic peptides evaluated for membrane permeability

[Table 27]

| Compound ID | Analytical condition | LCMS(ESI) m/z | | Retention time (min) |
|---|---|---|---|---|
| pd50 | SQDFA05 | 1473 | (M+H)+ | 0.84 |
| pd51 | SQDFA05 | 1471 | (M+H)+ | 0.84 |
| pd52 | SQDFA05 | 1443 | (M+H)+ | 0.79 |
| pd53 | SQDFA05 | 1487 | (M+H)+ | 0.85 |
| pd54 | SQDFA05 | 1485 | (M+H)+ | 0.74, 0.82 |
| pd55 | SQDFA05 | 1485 | (M+H)+ | 0.79,0.85 |
| pd56 | SQDFA05 | 1443 | (M+H)+ | 0.77, 0.82 |
| pd57 | SQDFA05 | 1429 | (M+H)+ | 0.71 |
| pd58 | SQDFA05 | 1459 | (M+H)+ | 0.81 |
| pd59 | SQDAA50 | 1553 | (M+H)+ | 0.80 |
| pd60 | SQDAA50 | 1587 | (M+H)+ | 0.79 |
| pd61 | SQDAA50 | 1559 | (M+H)+ | 0.77 |
| pd62 | SQDAA50 | 1533 | (M+H)+ | 0.76 |
| pd63 | SQDAA50 | 1539 | (M+H)+ | 0.76 |
| pd64 | SQDAA50 | 1553 | (M+H)+ | 0.75 |
| pd65 | SQDAA50 | 1533 | (M+H)+ | 0.76 |
| pd66 | SQDAA50 | 1567 | (M+H)+ | 0.77 |
| pd67 | SQDAA50 | 1547 | (M+H)+ | 0.80 |
| pd68 | SQDAA50 | 1545 | (M+H)+ | 0.73 |

(continued)

| Compound ID | Analytical condition | LCMS(ESI) m/z | | Retention time (min) |
|---|---|---|---|---|
| pd69 | SQDAA50 | 1517 | (M+H)+ | 0.75 |
| pd70 | SQDAA50 | 1503 | (M+H)+ | 0.74 |
| pd100 | SQDFA05 | 1339.5 | (M+H)+ | 0.91 |
| pd101 | SQDFA05 | 1303 | (M+H)+ | 1.02 |
| pd102 | SQDFA05 | 1361 | (M+H)+ | 1.00 |
| pd103 | SQDFA05 | 1361 | (M+H)+ | 1.12 |
| pd104 | SQDFA05 | 1395 | (M+H)+ | 1.03 |
| pd105 | SQDFA05 | 1361 | (M+H)+ | 1.01 |
| pd106 | SQDFA05 | 1419 | (M+H)+ | 1.11 |
| pd107 | SQDFA05 | 1509 | (M+H)+ | 1.12 |
| pd108 | SQDFA05 | 1389 | (M+H)+ | 1.11 |
| pd109 | SQDFA05 | 1423 | (M+H)+ | 1.06 |
| pd110 | SQDFA05 | 1445 | (M+H)+ | 1.17 |
| pd111 | SQDFA05 | 1451 | (M+H)+ | 1.13 |
| pd112 | SQDFA05 | 1330 | (M+H)+ | 0.89 |
| pd113 | SQDFA05 | 1348 | (M+H)+ | 0.84 |
| pd114 | SQDFA05 | 1448 | (M+H)+ | 1.02 |
| pd115 | SQDFA05 | 1344 | (M+H)+ | 0.89 |
| pd116 | SQDFA05 | 1300 | (M+H)+ | 0.90 |
| pd117 | SQDFA05 | 1358 | (M+H)+ | 0.96 |
| pd118 | SQDFA05 | 1420 | (M+H)+ | 1.00 |
| pd119 | SQDFA05 | 1432 | (M+H)+ | 0.98 |
| pd120 | SQDFA05 | 1332 | (M+H)+ | 0.94 |
| pd121 | SQDFA05 | 1372 | (M+H)+ | 1.00 |
| pd122 | SQDFA05 | 1390 | (M+H)+ | 1.01 |
| pd123 | SQDFA05 | 1390 | (M+H)+ | 0.97 |
| pd124 | SQDFA05 | 1390 | (M+H)+ | 0.98 |
| pd125 | SQDFA05 | 1442 | (M+H)+ | 1.07 |
| pd126 | SQDFA05 | 1342 | (M+H)+ | 1.01 |
| pd127 | SQDFA05 | 1400 | (M+H)+ | 1.06 |
| pd128 | SQDFA05 | 1418 | (M+H)+ | 1.05 |
| pd129 | SQDFA05 | 1456 | (M+H)+ | 1.06 |
| pd130 | SQDFA05 | 1398 | (M+H)+ | 1.05 |
| pd131 | SQDFA05 | 1354 | (M+H)+ | 0.98 |
| pd132 | SQDFA05 | 1368 | (M+H)+ | 1.01 |
| pd133 | SQDFA05 | 1360 | (M+H)+ | 1.09 |
| pd134 | SQDFA05 | 1426 | (M+H)+ | 1.09 |
| pd135 | SQDFA05 | 1097 | (M+H)+ | 0.95 |

(continued)

| Compound ID | Analytical condition | LCMS(ESI) m/z | | Retention time (min) |
|---|---|---|---|---|
| pd136 | SQDFA05 | 1063 | (M+H)+ | 0.97 |
| pd137 | SQDFA05 | 1452 | (M+H)+ | 1.19 |
| pd138 | SQDFA05 | 1440 | (M+H)+ | 1.13 |
| pd139 | SQDFA05 | 1077 | (M+H)+ | 1.03 |
| pd140 | SQDFA05 | 1440 | (M+H)+ | 1.10 |
| pd141 | SQDFA05 | 1420 | (M+H)+ | 1.06 |
| pd142 | SQDFA05 | 1362 | (M+H)+ | 1.11 |
| pd143 | SQDFA05 | 1109 | (M+H)+ | 1.06 |
| pd144 | SQDFA05 | 1178 | (M+H)+ | 1.06 |
| pd145 | SQDFA05 | 1212 | (M+H)+ | 1.05 |
| pd146 | SQDFA05 | 1178 | (M+H)+ | 1.09 |
| pd147 | SQDFA05 | 1123 | (M+H)+ | 1.06 |
| pd148 | SQDFA05 | 1291 | (M+H)+ | 1.05 |
| pd149 | SQDFA05 | 1079 | (M+H)+ | 0.97 |
| pd150 | SQDFA05 | 1113 | (M+H)+ | 0.98 |
| pd151 | SQDFA05 | 1079 | (M+H)+ | 1.03 |
| pd152 | SQDFA05 | 1250 | (M+H)+ | 1.14 |
| pd153 | SQDFA05 | 1206 | (M+H)+ | 1.18 |
| pd154 | SQDFA05 | 1392 | (M+H)+ | 1.07 |
| pd155 | SQDFA05 | 1418 | (M+H)+ | 1.13 |
| pd156 | SQDFA05 | 1418 | (M+H)+ | 1.10 |
| pd157 | SQDFA50 | 1406 | (M+H)+ | 0.79 |
| pd158 | SQDFA50 | 1406 | (M+H)+ | 0.72 |
| pd159 | SQDFA05 | 1406 | (M+H)+ | 1.16 |
| pd160 | SQDFA05 | 1406 | (M+H)+ | 1.15 |
| pd161 | SQDFA50 | 1362 | (M+H)+ | 0.67 |
| pd162 | SQDFA05 | 1466 | (M+H)+ | 1.11 |
| pd163 | SQDFA05 | 1466 | (M+H)+ | 1.13 |
| pd164 | SQDFA05 | 1419 | (M+H)+ | 1.00 |
| pd165 | SQDFA05 | 1432 | (M+H)+ | 1.22 |
| pd166 | SQDFA05 | 1432 | (M+H)+ | 1.19 |
| pd167 | SQDFA05 | 1454 | (M+H)+ | 1.12 |
| pd168 | SQDFA05 | 1420 | (M+H)+ | 1.17 |
| pd169 | SQDFA05 | 1420 | (M+H)+ | 1.16 |
| pd170 | SQDFA05 | 1475 | (M+H)+ | 0.98 |
| pd171 | SQDFA05 | 1400 | (M+H)+ | 1.15 |
| pd172 | SQDFA05 | 1433 | (M+H)+ | 0.99 |
| pd173 | SQDAA50 | 1362 | (M+H)+ | 1.21 |

(continued)

| Compound ID | Analytical condition | LCMS(ESI) m/z | | Retention time (min) |
|---|---|---|---|---|
| pd174 | SQDAA50 | 1390 | (M+H)+ | 1.18 |
| pd175 | SQDAA50 | 1406 | (M+H)+ | 1.18 |
| pd176 | SQDAA50 | 1406 | (M+H)+ | 1.18 |
| pd177 | SQDFA05 | 1450 | (M+H)+ | 1.09 |
| pd178 | SQDFA05 | 1420 | (M+H)+ | 1.09 |
| pd179 | SQDFA05 | 1392 | (M+H)+ | 1.05 |
| pd180 | SQDFA05 | 1483 | (M+H)+ | 1.13 |
| pd181 | SQDFA05 | 1481 | (M+H)+ | 1.17 |
| pd182 | SQDFA05 | 1390 | (M+H)+ | 1.08 |
| pd183 | SQDFA05 | 1406 | (M+H)+ | 1.09 |
| pd184 | SQDFA05 | 1420 | (M+H)+ | 1.10 |
| pd185 | SQDFA05 | 1442 | (M+H)+ | 1.12 |
| pd186 | SQDFA05 | 1398 | (M+H)+ | 1.06 |
| pd187 | SQDFA05 | 1442 | (M+H)+ | 1.12 |
| pd188 | SQDFA05 | 1412 | (M+H)+ | 1.09 |
| pd189 | SQDFA05 | 1448 | (M+H)+ | 1.13 |
| pd190 | SQDFA05 | 1376 | (M+H)+ | 1.05 |
| pd191 | SQDFA05 | 1376 | (M+H)+ | 1.05 |
| pd192 | SQDFA05 | 1434 | (M+H)+ | 1.13 |
| pd193 | SQDFA05 | 1390 | (M+H)+ | 1.08 |
| pd194 | SQDFA50 | 1434 | (M+H)+ | 0.82 |
| pd195 | SQDFA50 | 1434 | (M+H)+ | 0.78 |
| pd196 | SQDFA05 | 1446 | (M+H)+ | 1.15 |
| pd197 | SQDFA05 | 1433 | (M+H)+ | 0.93 |
| pd198 | SQDFA05 | 1447 | (M+H)+ | 1.03 |
| pd199 | SQDFA05 | 1434 | (M+H)+ | 1.13 |
| pd200 | SQDFA05 | 1446 | (M+H)+ | 1.15 |
| pd201 | SQDAA50 | 1574 | (M+H)+ | 0.90 |
| pd202 | SQDFA05 | 1434 | (M+H)+ | 1.12 |
| pd203 | SQDFA05 | 1446 | (M+H)+ | 1.13 |
| pd204 | SQDFA05 | 1404 | (M+H)+ | 1.10 |
| pd205 | SQDFA05 | 1406 | (M+H)+ | 1.03 |
| pd206 | SQDFA05 | 1446 | (M+H)+ | 1.12 |
| pd207 | SQDFA05 | 1220 | (M+H)+ | 0.82 |
| pd208 | SQDFA05 | 1434 | (M+H)+ | 1.12 |
| pd209 | SQDFA05 | 1434 | (M+H)+ | 1.16 |
| pd210 | SQDFA05 | 1434 | (M+H)+ | 1.13 |
| pd211 | SQDFA05 | 1220 | (M+H)+ | 1.05 |

(continued)

| Compound ID | Analytical condition | LCMS(ESI) m/z | | Retention time (min) |
|---|---|---|---|---|
| pd212 | SQDFA05 | 1348 | (M+H)+ | 1.04 |
| pd213 | SQDFA05 | 1448 | (M+H)+ | 1.12 |
| pd214 | SQDFA05 | 1460 | (M+H)+ | 1.13 |
| pd215 | SQDFA05 | 1448 | (M+H)+ | 1.15 |
| pd216 | SQDFA05 | 1490 | (M+H)+ | 1.16 |
| pd217 | SQDFA05 | 1420 | (M+H)+ | 1.09 |
| pd218 | SQDFA05 | 1404 | (M+H)+ | 1.14 |
| pd219 | SQDFA05 | 1460 | (M+H)+ | 1.06 |
| pd220 | SQDFA05 | 1434 | (M+H)+ | 1.15 |
| pd221 | SQDFA05 | 1404 | (M+H)+ | 1.10 |
| pd222 | SQDFA05 | 1544 | (M-H)- | 1.06 |
| pd223 | SQDFA05 | 1220 | (M+H)+ | 1.02 |
| pd224 | SQDFA05 | 1434 | (M+H)+ | 1.16 |
| pd225 | SQDFA05 | 1432 | (M+H)+ | 1.16 |
| pd226 | SQDFA05 | 1459 | (M+H)+ | 1.06 |
| pd227 | SQDFA05 | 1480 | (M+H)+ | 1.19 |
| pd228 | SQDFA05 | 1487 | (M+H)+ | 1.14 |
| pd229 | SQDFA05 | 1424 | (M+H)+ | 1.10 |
| pd230 | SQDFA05 | 1438 | (M+H)+ | 1.13 |
| pd231 | SQDFA05 | 1424 | (M+H)+ | 1.08 |
| pd232 | SQDFA05 | 1424 | (M+H)+ | 1.07 |
| pd233 | SQDFA05 | 1452 | (M+H)+ | 1.13 |
| pd234 | SQDFA05 | 1468 | (M+H)+ | 1.11 |
| pd235 | SQDFA05 | 1500 | (M+H)+ | 1.11 |
| pd236 | SQDFA05 | 1431 | (M+H)+ | 0.99 |
| pd237 | SQDFA05 | 1468 | (M+H)+ | 1.14 |
| pd238 | SQDFA05 | 1459 | (M+H)+ | 1.02 |
| pd239 | SQDFA05 | 1664 | (M+H)+ | 1.11 |
| pd240 | SQDAA50 | 1664 | (M+H)+ | 0.95 |
| pd241 | SQDFA50 | 1410 | (M+H)+ | 0.76 |
| pd242 | SQDAA50 | 1650 | (M+H)+ | 0.94 |
| pd243 | SQDAA50 | 1552 | (M+H)+ | 0.94 |
| pd244 | SQDFA05 | 1410 | (M+H)+ | 1.03 |
| pd245 | SQDFA05 | 1440 | (M+H)+ | 0.99 |
| pd246 | SQDFA05 | 1440 | (M+H)+ | 1.05 |
| pd247 | SQDFA05 | 1439 | (M+H)+ | 0.93 |
| pd300 | SQDFA05 | 1400 | (M+H)+ | 1.16 |
| pd301 | SQDFA05 | 1430 | (M+H)+ | 1.16 |

(continued)

| Compound ID | Analytical condition | LCMS(ESI) m/z | | Retention time (min) |
|---|---|---|---|---|
| pd302 | SQDFA05 | 1442 | (M+H)+ | 1.14 |
| pd303 | SQDFA05 | 1472 | (M+H)+ | 1.15 |
| pd304 | SQDFA05 | 1400 | (M+H)+ | 1.14 |
| pd305 | SQDFA05 | 1386 | (M+H)+ | 1.09 |
| pd306 | SQDFA05 | 1400 | (M+H)+ | 1.17 |
| pd307 | SQDFA05 | 1386 | (M+H)+ | 1.13 |
| pd308 | SQDFA05 | 1414 | (M+H)+ | 1.21 |
| pd309 | SQDFA05 | 1372 | (M+H)+ | 1.10 |
| pd310 | SQDFA05 | 1406 | (M+H)+ | 1.09 |
| pd311 | SQDFA05 | 1390 | (M+H)+ | 1.08 |
| pd312 | SQDFA05 | 1356 | (M+H)+ | 1.11 |
| pd313 | SQDFA05 | 1342 | (M+H)+ | 1.06 |
| pd314 | SQDFA05 | 1400 | (M+H)+ | 1.14 |
| pd315 | SQDFA05 | 1428 | (M+H)+ | 1.21 |
| pd316 | SQDFA05 | 1412 | (M+H)+ | 1.13 |
| pd317 | SQDFA05 | 1428 | (M+H)+ | 1.12 |
| pd318 | SQDFA05 | 1298 | (M+H)+ | 1.04 |
| pd319 | SQDFA05 | 1342 | (M+H)+ | 1.02 |
| pd320 | SQDFA05 | 1370 | (M+H)+ | 1.13 |
| pd321 | SQDFA05 | 1356 | (M+H)+ | 1.09 |
| pd322 | SQDFA05 | 1356 | (M+H)+ | 1.08 |
| pd323 | SQDFA05 | 1384 | (M+H)+ | 1.14 |
| pd324 | SQDFA05 | 1400 | (M+H)+ | 1.10 |
| pd325 | SQDFA05 | 1400 | (M+H)+ | 1.16 |
| pd326 | SQDFA05 | 1386 | (M+H)+ | 1.13 |
| pd327 | SQDFA05 | 1430 | (M+H)+ | 1.17 |
| pd328 | SQDFA05 | 1442 | (M+H)+ | 1.15 |
| pd329 | SQDFA05 | 1386 | (M+H)+ | 1.10 |
| pd330 | SQDFA05 | 1372 | (M+H)+ | 1.05 |
| pd331 | SQDFA05 | 1450 | (M+H)+ | 0.76 |
| pd332 | SQDFA05 | 1420 | (M+H)+ | 0.73 |
| pd333 | SQDFA05 | 1448 | (M+H)+ | 0.68 |
| pd334 | SQDFA05 | 1406 | (M+H)+ | 0.72 |
| pd335 | SQDFA05 | 1420 | (M+H)+ | 0.72 |
| pd336 | SQDFA05 | 1450 | (M+H)+ | 0.72 |
| pd337 | SQDFA05 | 1406 | (M+H)+ | 0.66 |
| pd338 | SQDFA05 | 1450 | (M+H)+ | 0.73 |
| pd339 | SQDFA05 | 1408 | (M+H)+ | 1.11 |

(continued)

| Compound ID | Analytical condition | LCMS(ESI) m/z | | Retention time (min) |
|---|---|---|---|---|
| pd340 | SQDFA05 | 1394 | (M+H)+ | 1.08 |
| pd341 | SQDFA05 | 1378 | (M+H)+ | 1.06 |
| pd342 | SQDFA05 | 1442 | (M+H)+ | 1.12 |
| pd343 | SQDFA05 | 1442 | (M+H)+ | 1.12 |
| pd344 | SQDFA05 | 1412 | (M+H)+ | 1.09 |
| pd345 | SQDFA05 | 1398 | (M+H)+ | 1.06 |
| pd346 | SQDFA05 | 1483 | (M+H)+ | 1.13 |
| pd347 | SQDFA05 | 1481 | (M+H)+ | 1.17 |
| pd348 | SQDFA05 | 1456 | (M-H)- | 1.04, 1.11 |
| pd349 | SQDFA05 | 1500 | (M-H)- | 1.11 |
| pd350 | SQDFA05 | 1406 | (M+H)+ | 1.17 |
| pd351 | SQDFA05 | 1376 | (M+H)+ | 1.17 |
| pd352 | SQDFA05 | 1376 | (M+H)+ | 1.13 |
| pd353 | SQDFA05 | 1362 | (M+H)+ | 1.11 |
| pd354 | SQDFA05 | 1390 | (M+H)+ | 1.17 |
| pd355 | SQDFA05 | 1376 | (M+H)+ | 1.18 |
| pd356 | SQDFA05 | 1362 | (M+H)+ | 1.12 |
| pd357 | SQDFA05 | 1420 | (M+H)+ | 1.18 |
| pd358 | SQDFA05 | 1390 | (M+H)+ | 1.18 |
| pd359 | SQDFA05 | 1406 | (M+H)+ | 1.18 |
| pd360 | SQDFA05 | 1376 | (M+H)+ | 1.18 |
| pd361 | SQDFA05 | 1362 | (M+H)+ | 1.12 |
| pd362 | SQDFA05 | 1390 | (M+H)+ | 1.18 |
| pd363 | SQDFA05 | 1434 | (M+H)+ | 1.13 |
| pd364 | SQDFA05 | 1390 | (M+H)+ | 1.08 |
| pd365 | SQDFA05 | 1376 | (M+H)+ | 1.05 |
| pd366 | SQDFA05 | 1448 | (M+H)+ | 1.13 |
| pd367 | SQDFA05 | 1376 | (M+H)+ | 1.05 |
| pd368 | SQDFA05 | 1392 | (M+H)+ | 1.05 |
| pd369 | SQDFA05 | 1450 | (M+H)+ | 1.09 |
| pd370 | SQDFA05 | 1420 | (M+H)+ | 1.09 |
| pd371 | SQDAA50 | 1358 | (M+H)+ | 0.86 |
| pd372 | SQDAA50 | 1468 | (M+H)+ | 0.88 |
| pd373 | SQDAA50 | 1456 | (M+H)+ | 0.88 |
| pd374 | SQDAA50 | 1412 | (M+H)+ | 0.89 |
| pd375 | SQDAA50 | 1422 | (M+H)+ | 0.90 |
| pd376 | SQDAA50 | 1468 | (M+H)+ | 0.86 |
| pd377 | SQDAA50 | 1444 | (M+H)+ | 0.88 |

(continued)

| Compound ID | Analytical condition | LCMS(ESI) m/z | | Retention time (min) |
|---|---|---|---|---|
| pd378 | SQDAA50 | 1468 | (M+H)+ | 0.87 |
| pd379 | SQDAA50 | 1468 | (M+H)+ | 0.87 |
| pd380 | SQDAA50 | 1422 | (M+H)+ | 0.88 |
| pd381 | SQDAA50 | 1370 | (M+H)+ | 0.85 |
| pd382 | SQDAA50 | 1370 | (M+H)+ | 0.85 |
| pd383 | SQDAA50 | 1414 | (M+H)+ | 0.90 |
| pd384 | SQDAA50 | 1414 | (M+H)+ | 0.89 |
| pd385 | SQDAA50 | 1414 | (M+H)+ | 0.88 |
| pd386 | SQDFA05 | 1505 | (M+H)+ | 0.84 |
| pd387 | SQDFA05 | 1491 | (M+H)+ | 0.84 |
| pd388 | SQDFA05 | 1475 | (M+H)+ | 0.87 |
| pd389 | SQDFA05 | 1467 | (M+H)+ | 0.80 |
| pd390 | SQDFA05 | 1489 | (M+H)+ | 0.82 |
| pd391 | SQDFA05 | 1447 | (M+H)+ | 0.81 |
| pd392 | SQDFA05 | 1461 | (M+H)+ | 0.85 |
| pd393 | SQDFA05 | 1491 | (M+H)+ | 0.84 |
| pd394 | SQDFA05 | 1475 | (M+H)+ | 0.80 |
| pd395 | SQDFA05 | 1475 | (M+H)+ | 0.79 |
| pd396 | SQDFA05 | 1472 | (M+H)+ | 0.85 |
| pd397 | SQDFA05 | 1484 | (M+H)+ | 0.88 |
| pd398 | SQDFA05 | 1414 | (M+H)+ | 0.82 |
| pd399 | SQDFA05 | 1434 | (M+H)+ | 0.77 |
| pd400 | SQDFA05 | 1406 | (M+H)+ | 0.76 |
| pd401 | SQDFA05 | 1464 | (M+H)+ | 0.82 |
| pd402 | SQDFA05 | 1444 | (M+H)+ | 0.83 |
| pd403 | SQDFA05 | 1356 | (M+H)+ | 1.11 |
| pd404 | SQDFA05 | 1386 | (M+H)+ | 1.16 |
| pd405 | SQDFA05 | 1386 | (M+H)+ | 1.11 |
| pd406 | SQDFA05 | 1400 | (M+H)+ | 1.14 |
| pd407 | SQDFA05 | 1386 | (M+H)+ | 1.11 |
| pd408 | SQDFA05 | 1356 | (M+H)+ | 1.13 |
| pd409 | SQDFA05 | 1386 | (M+H)+ | 1.12 |
| pd410 | SQDFA05 | 1356 | (M+H)+ | 1.09 |
| pd411 | SQDFA05 | 1386 | (M+H)+ | 1.17 |
| pd412 | SQDFA05 | 1386 | (M+H)+ | 1.13 |
| pd413 | SQDFA05 | 1400 | (M+H)+ | 1.12 |
| pd414 | SQDFA05 | 1386 | (M+H)+ | 1.10 |
| pd415 | SQDFA05 | 1356 | (M+H)+ | 1.13 |

(continued)

| Compound ID | Analytical condition | LCMS(ESI) m/z | | Retention time (min) |
|---|---|---|---|---|
| pd416 | SQDFA05 | 1430 | (M+H)+ | 1.16 |
| pd417 | SQDFA05 | 1400 | (M+H)+ | 1.13 |
| pd418 | SQDFA05 | 1430 | (M+H)+ | 1.11 |
| pd419 | SQDFA05 | 1400 | (M+H)+ | 1.19 |
| pd420 | SQDFA05 | 1372 | (M+H)+ | 1.09 |
| pd421 | SQDFA05 | 1402 | (M+H)+ | 1.12 |
| pd422 | SQDFA05 | 1416 | (M+H)+ | 1.16 |
| pd423 | SQDFA05 | 1402 | (M+H)+ | 1.08 |
| pd424 | SQDFA05 | 1372 | (M+H)+ | 1.14 |
| pd425 | SQDFA05 | 1462 | (M-H)- | 1.12 |
| pd426 | SQDFA50 | 1488 | (M+H)+ | 0.85 |
| pd427 | SQDFA50 | 1464 | (M+H)+ | 0.71 |
| pd428 | SQDFA50 | 1444 | (M+H)+ | 0.72 |
| pd429 | SQDFA50 | 1444 | (M+H)+ | 0.69, 0.79 |
| pd430 | SQDFA50 | 1494 | (M+H)+ | 0.72 |
| pd431 | SQDFA50 | 1444 | (M+H)+ | 0.75 |
| pd432 | SQDFA50 | 1458 | (M+H)+ | 0.77 |
| pd433 | SQDFA50 | 1488 | (M+H)+ | 0.79 |
| pd434 | SQDFA50 | 1464 | (M+H)+ | 0.68 |
| pd435 | SQDFA50 | 1520 | (M+H)+ | 0.66, 0.77 |
| pd436 | SQDFA50 | 1444 | (M+H)+ | 0.63, 0.67 |
| pd437 | SQDFA50 | 1444 | (M+H)+ | 0.58, 0.64 |
| pd438 | SQDFA50 | 1458 | (M+H)+ | 0.70 |
| pd439 | SQDFA05 | 1465 | (M-H)- | 1.18 |
| pd440 | SQDFA05 | 1433 | (M-H)- | 1.12 |
| pd441 | SQDFA05 | 1481 | (M-H)- | 1.17 |
| pd442 | SQDFA05 | 1471 | (M+H)+ | 1.00 |
| pd443 | SQDFA05 | 1471 | (M+H)+ | 1.03 |
| pd444 | SQDFA05 | 1414 | (M+H)+ | 1.16 |
| pd445 | SQDFA05 | 1414 | (M+H)+ | 1.15 |
| pd446 | SQDFA05 | 1484 | (M+H)+ | 1.11 |
| pd447 | SQDFA05 | 1470 | (M+H)+ | 1.11 |
| pd448 | SQDFA05 | 1370 | (M+H)+ | 1.00 |
| pd449 | SQDFA05 | 1442 | (M+H)+ | 1.14 |
| pd450 | SQDAA50 | 1428 | (M+H)+ | 0.85 |
| pd451 | SQDAA50 | 1398 | (M+H)+ | 0.83 |
| pd452 | SQDFA05 | 1513 | (M+H)+ | 0.83 |
| pd453 | SQDFA05 | 1455 | (M+H)+ | 0.76 |

(continued)

| Compound ID | Analytical condition | LCMS(ESI) m/z | | Retention time (min) |
|---|---|---|---|---|
| pd454 | SQDFA05 | 1469 | (M+H)+ | 0.78 |
| pd455 | SQDFA05 | 1505 | (M+H)+ | 0.80 |
| pd456 | SQDFA05 | 1447 | (M+H)+ | 0.78 |
| pd457 | SQDFA05 | 1531 | (M+H)+ | 1.04 |
| pd458 | SQDFA05 | 1537 | (M+H)+ | 0.99 |
| pd459 | SQDFA05 | 1497 | (M+H)+ | 1.09 |
| pd460 | SQDFA05 | 1487 | (M+H)+ | 0.57 |
| pd461 | SQDFA05 | 1537 | (M+H)+ | 0.57 |
| pd462 | SQDFA05 | 1497 | (M+H)+ | 1.07 |
| pd463 | SQDFA05 | 1437 | (M-H)- | 1.11 |
| pd464 | SQDFA05 | 1467 | (M-H)- | 1.13 |
| pd465 | SQDFA50 | 1502 | (M-H)- | 0.83 |
| pd466 | SQDFA05 | 1386 | (M+H)+ | 1.15 |
| pd467 | SQDFA05 | 1372 | (M+H)+ | 1.11 |
| pd468 | SQDFA05 | 1428 | (M+H)+ | 1.11 |
| pd469 | SQDFA05 | 1356 | (M+H)+ | 1.10 |
| pd470 | SQDFA05 | 1469 | (M+H)+ | 1.07 |
| pd471 | SQDFA50 | 1483 | (M+H)+ | 0.76 |
| pd472 | SQDAA50 | 1450 | (M+H)+ | 0.85 |
| pd473 | SQDAA50 | 1392 | (M+H)+ | 0.81 |
| pd474 | SQDAA50 | 1372 | (M+H)+ | 0.82 |
| pd475 | SQDAA50 | 1392 | (M+H)+ | 0.80 |
| pd476 | SQDAA50 | 1466 | (M+H)+ | 0.85 |
| pd477 | SQDAA50 | 1408 | (M+H)+ | 0.82 |
| pd478 | SQDAA50 | 1494 | (M+H)+ | 0.86 |
| pd479 | SQDAA50 | 1436 | (M+H)+ | 0.84 |
| pd480 | SQDAA50 | 1360 | (M+H)+ | 0.79 |
| pd481 | SQDAA50 | 1408 | (M+H)+ | 0.81 |
| pd482 | SQDAA50 | 1451 | (M+H)+ | 0.83 |
| pd483 | SQDAA50 | 1437 | (M+H)+ | 0.84 |
| pd484 | SQDAA50 | 1437 | (M+H)+ | 0.86 |
| pd485 | SQDAA50 | 1507 | (M+H)+ | 0.89 |
| pd486 | SQDAA50 | 1421 | (M+H)+ | 0.85 |
| pd487 | SQDAA50 | 1448 | (M+H)+ | 0.79 |
| pd488 | SQDAA50 | 1418 | (M+H)+ | 0.76 |
| pd489 | SQDFA05 | 1478 | (M+H)+ | 1.15 |
| pd490 | SQDFA05 | 1494 | (M+H)+ | 1.11 |
| pd491 | SQDFA05 | 1464 | (M+H)+ | 1.12 |

(continued)

| Compound ID | Analytical condition | LCMS(ESI) m/z | | Retention time (min) |
|---|---|---|---|---|
| pd492 | SQDFA05 | 1444 | (M+H)+ | 1.14 |
| pd493 | SQDFA05 | 1500 | (M+H)+ | 1.14 |
| pd494 | SQDFA05 | 1414 | (M+H)+ | 1.08 |
| pd495 | SQDFA05 | 1400 | (M+H)+ | 1.06 |
| pd496 | SQDFA05 | 1458 | (M+H)+ | 1.12 |
| pd497 | SQDFA05 | 1430 | (M+H)+ | 1.06 |
| pd498 | SQDFA05 | 1486 | (M+H)+ | 1.11 |
| pd499 | SQDFA05 | 1400 | (M+H)+ | 1.05 |
| pd500 | SQDFA05 | 1386 | (M+H)+ | 1.04 |
| pd501 | SQDFA05 | 1444 | (M+H)+ | 1.09 |
| pd502 | SQDFA05 | 1482 | (M+H)+ | 1.11, 1.16 |
| pd503 | SQDFA05 | 1426 | (M+H)+ | 1.03, 1.08 |
| pd504 | SQDFA05 | 1484 | (M+H)+ | 1.10 |

A part of amino acids having basic side chains whose basic pKas were calculated by a method described herein are provided in the following table.

[Table 28]

| X | Ser(Et-2-NMe2) | MeAbu(pip-4-F2) | MeAbu(Mor) | Ser(Et-2-Mor) | MeAbu(pip-3-F2) |
|---|---|---|---|---|---|
| Amino acid structure | 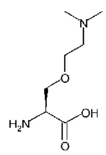 | 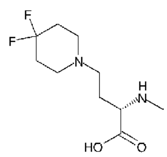 | 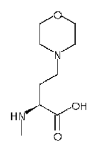 | 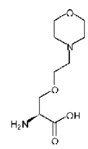 | 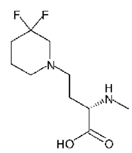 |
| basic pKa | 8.9 | 8.6 | 8.2 | 7.1 | 6.8 |

A part of amino acids having acidic side chains whose pKas were calculated by a method described herein are provided in the following table.

[Table 29]

| X | Abu(5-Oxo-Odz) | Gln(Ms) |
|---|---|---|
| Amino acid structure | 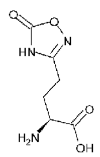 | 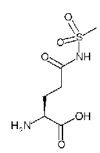 |
| pKa | 8.3 | 5.3 |

Herein, measurement of pKas of amino acid side chains was carried out according to the following procedure.

Instrument used

Sirius T3 (Sirius Analytical Instruments Ltd., Forest Row, East Sussex, RH18 5DW, UK)

[Table 30]

| | |
|---|---|
| pH electrode: | Ag/AgCl, Double junction reference |
| Purge gas : | Argon |
| pH range: | 1.8-12.2 |
| Measurement condition | |
| Set temperature | 25°C |
| Ion concentration | 0.15M |
| Solution for measurement | 0.5M KOH, 0.5M HCl |

(Implementation procedure)

0.15 M KCl was added to about 1 mg of a test substance as a powder or in about 100 uL of a 10 mM DMSO solution. When the test substance was a basic compound, 0.5 M HCl was added until pH 2 and the test substance was then titrated to pH 12 with 0.5 M KOH.

When the test substance was an acidic compound, 0.5 M KOH was added until pH 12 and the test substance was then titrated to pH 2 with 0.5 M HCl.

Titration operations were automatically performed by T3 manufactured by Sirius, and pKas were determined from the resulting titration curves using Sirius T3 Refinement software.

pKas were similarly determined using imipramine HCl, propranolol HCl, and warfarin as reference compounds, and were confirmed to be within the test results obtained in Sirius.

pKas of amino acid side chains were determined by synthesizing a sequence in which the amino acid to be evaluated is introduced into the second residue (the position X in the following table) of a peptide composed of three residues as shown below.

[Table 31]

| 3 | 2 | 1 | C-term |
|---|---|---|---|
| ZMeGly | X | MeGly | pip |

The results are provided below.

Industrial Applicability

[Table 32]

| X | Ser(Et-2-NMe2) | Ser(Et-2-Mor) | MeAbu(Mor) | MeAbu(pip-4-F2) | MeAbu(pip-3-F2) |
|---|---|---|---|---|---|
| Amino acid structure | 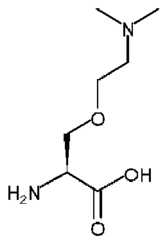 | 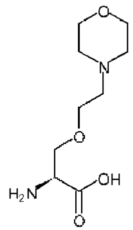 | 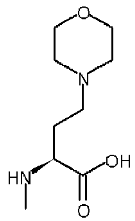 | 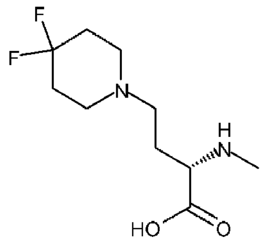 | 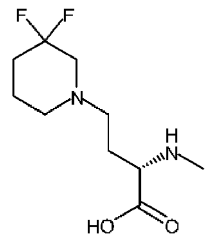 |
| basic pKa | 9.1 | 7.2 | 6.8 | 6.7 | 5.7 |
| X | | | | MeAbu(5-Oxo-Odz) | Gln(Ms) |
| Amino acid structure | | | | 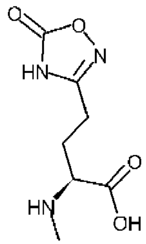 | 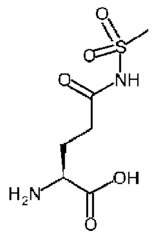 |
| pKa | | | | 5.7 | 3.8 |

Use of an embodiment of the present disclosure allows efficient screening for cyclic peptide compounds capable of specifically binding to target molecules and having high cell membrane permeability. The present disclosure is useful in the field of manufacturing of or screening for cyclic peptide compounds expected to be useful, for example as pharmaceuticals.

## Claims

1. A library of cyclic peptide compounds, wherein the library substantially consists of cyclic peptide compounds which do not have (1) an indole skeleton or (2) a substituted or unsubstituted hydroxyphenyl group in the side chains of the cyclic portion.

2. The library according to claim 1, wherein if the library comprises cyclic peptide compounds having an acidic side chain in the cyclic portion, the cyclic peptide compounds substantially consist of cyclic peptide compounds the acidic side chain of which has a pKa of 3.5 to 10.

3. The library according to claim 1 or 2, wherein if the library comprises cyclic peptide compounds having a basic side chain in the cyclic portion, the cyclic peptide compounds substantially consist of cyclic peptide compounds the basic side chain of which has a basic pKa of 4.0 to 10.

4. The library according to any one of claims 1 to 3, wherein the library comprises cyclic peptide compounds having a long side chain of 5.4 to 13 angstroms in length in the cyclic portion.

5. The library according to any one of claims 1 to 4, wherein the long side chain is a side chain comprising no amide bond or one amide bond in the side chain.

6. The library according to any one of claims 1 to 5, wherein the average of the number of amino acids constituting the cyclic portion of each cyclic peptide compound comprised in the library is 5 to 15.

7. The library according to any one of claims 1 to 6, wherein the average of the number of amino acids constituting the peptide moiety excluding the nucleic acid-linked portion of each cyclic peptide compound comprised in the library is 5 to 20.

8. The library according to any one of claims 1 to 7, wherein the average of the percentage of the number of N-substituted amino acids relative to the number of amino acids comprised in the peptide moiety excluding the nucleic acid-linked portion of each cyclic peptide compound comprised in the library is 30% or higher.

**9.** The library according to any one of claims 4 to 8, wherein the long side chain comprises $R^1$, wherein $R^1$ is:
(a) a phenyl group, or

(b) a 4- to 6-membered heterocyclic group having 1 to 3 heteroatoms independently selected from the group consisting of an oxygen atom and a nitrogen atom in the ring,
wherein (a) and (b) optionally have at least one substituent independently selected from Group A below:

Group A: oxo, a halogen atom, a C1-C4 alkyl group optionally substituted with a halogen atom, and a C1-C4 dialkylamino group (wherein one or two non-adjacent methylene groups in the C1-C4 alkyl group are optionally substituted with an oxygen atom);
or

(c) a group represented by the following general formula (II), (III), or (IV):

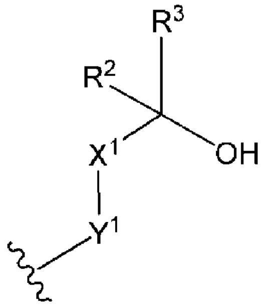
、
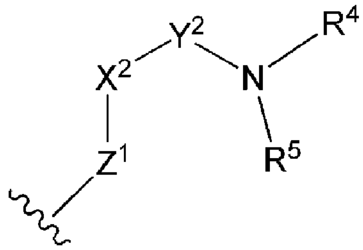
、
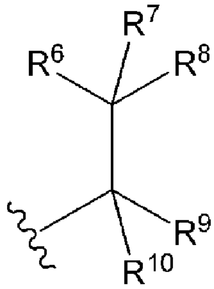

(II) (III) (IV)

wherein

$X^1$ represents a single bond, or a C1-C2 alkylene group optionally having at least one substituent independently selected from Group B below,
$X^2$ represents a single bond, or a C1-C2 alkylene group optionally having at least one substituent independently selected from Group B below,
$Y^1$ represents a single bond, an oxygen atom, a carbonyl group (-CO-), or a sulfonyl group ($-SO_2-$),
$Y^2$ represents a single bond, a C1-C2 alkylene group optionally substituted with a C1-C4 alkyl group, a carbonyl group (-CO-), or a sulfonyl group ($-SO_2-$),
$Z^1$ represents a single bond, a methylene group optionally having at least one substituent independently selected from Group B below, or an oxygen atom,
$R^2$ and $R^3$ each independently represent a hydrogen atom, a C1-C6 alkyl group, a C2-C4 alkenyl group, or a C2-C4 alkynyl group, wherein the C1-C6 alkyl group is optionally substituted with a halogen atom, and one or two non-adjacent methylene groups in the C1-C6 alkyl group are optionally substituted with an oxygen atom,
$R^2$ and $R^3$, or $R^2$ and $X^1$ are optionally joined to form a 4- to 6-membered ring structure, wherein the ring structure is optionally substituted with a C1-C4 alkyl group or a halogen atom, optionally has an oxygen atom in the ring, and is optionally partially unsaturated,
$R^4$ represents a C1-C6 alkyl group, a C2-C6 alkenyl group, a C2-C6 alkynyl group, or a C1-C6 alkanoyl group,
$R^5$ represents a hydrogen atom, a C1-C6 alkyl group, a C2-C6 alkenyl group, or a C2-C6 alkynyl group, wherein one or two non-adjacent methylene groups in the C1-C6 alkyl group of $R^4$ and/or $R^5$ are optionally substituted with an oxygen atom,
or $R^4$ and $R^5$, or $R^5$ and $X^2$ are optionally taken together with the atom(s) to which they are attached to form a 4- to 6-membered ring structure, wherein the ring structure is optionally substituted with a C1-C4 alkyl group or a halogen atom, optionally has an oxygen atom in the ring, and is optionally partially unsaturated,
$R^6$, $R^7$, $R^8$, $R^9$, and $R^{10}$ each independently represent a hydrogen atom, a halogen atom, a C1-C4 alkyl group, a C2-C4 alkenyl group, or a C2-C4 alkynyl group, wherein one or two non-adjacent methylene groups in the C1-C4 alkyl group are optionally substituted with an oxygen atom, and

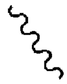

represents the point of attachment;
Group B: a halogen atom, a C1-C4 alkyl group, and a C1-C2 alkoxy group.

**10.** The library according to any one of claims 1 to 9, wherein the average of the number of aromatic rings comprised in the side chains of the cyclic portion of each cyclic peptide compound comprised in the library is 0 to 3.

**11.** A method of screening for a cyclic peptide compound that can specifically bind to a target molecule, the method comprising the steps of:

(i) contacting a cyclic peptide compound comprised in the library according to any one of claims 1 to 10 with the target molecule; and
(ii) selecting a cyclic peptide compound that can specifically bind to the target molecule.

**12.** A cell-free translation system for producing a peptide compound, wherein the cell-free translation system does not substantially comprise (a), (b), or (c) below:

(a) an amino acid having an indole skeleton in the side chain, or a nucleic acid encoding the amino acid;
(b) an amino acid having a fused-ring structure formed by two or more aromatic rings in the side chain, or a nucleic acid encoding the amino acid; and
(c) an amino acid having a substituted or unsubstituted hydroxyphenyl group in the side chain, or a nucleic acid encoding the amino acid.

**13.** A method of producing a library of peptide compounds, the method comprising at least one step selected from the group consisting of (a) and (b) below:

(a) preparing an amino acid pool substantially not comprising an amino acid which has an indole skeleton in the side chain, and synthesizing a peptide compound using a part or all of the amino acids comprised in the pool as constituent amino acids; and
(b) preparing a template pool substantially not comprising a nucleic acid encoding an amino acid which has an indole skeleton in the side chain, and synthesizing a peptide compound from the template pool.

**14.** A method of producing a cyclic peptide compound, the method comprising the steps of:

(i) contacting a cyclic peptide compound comprised in the library according to any one of claims 1 to 10 with a target molecule;
(ii) selecting a cyclic peptide compound capable of binding to the target molecule; and
(iii) producing a cyclic peptide compound based on the amino acid sequence of the cyclic peptide compound selected in (ii).

**15.** A cyclic peptide compound, wherein:

(1) the cyclic peptide compound does not have a methylthio group, a thiol group, an indole skeleton, or a substituted or unsubstituted hydroxyphenyl group in the side chains of the cyclic portion;
(2) if the cyclic peptide compound has an acidic side chain in the cyclic portion, the acidic side chain has a pKa of 3.5 to 10;
(3) if the cyclic peptide compound has a basic side chain in the cyclic portion, the basic side chain has a basic pKa of 4.0 to 10;
(4) the cyclic peptide compound has a long side chain having a side chain length of 6.0 to 13 angstroms in the cyclic portion; and
(5) the long side chain comprises $R^1$, wherein $R^1$ is as defined in claim 9.

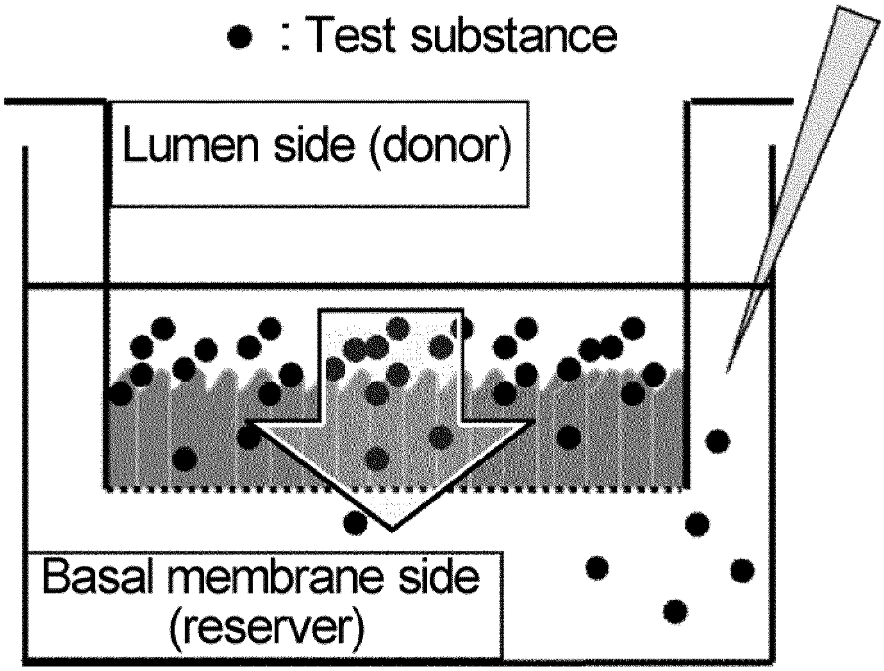
● : Test substance
Lumen side (donor)
Basal membrane side (reserver)

FIG. 1

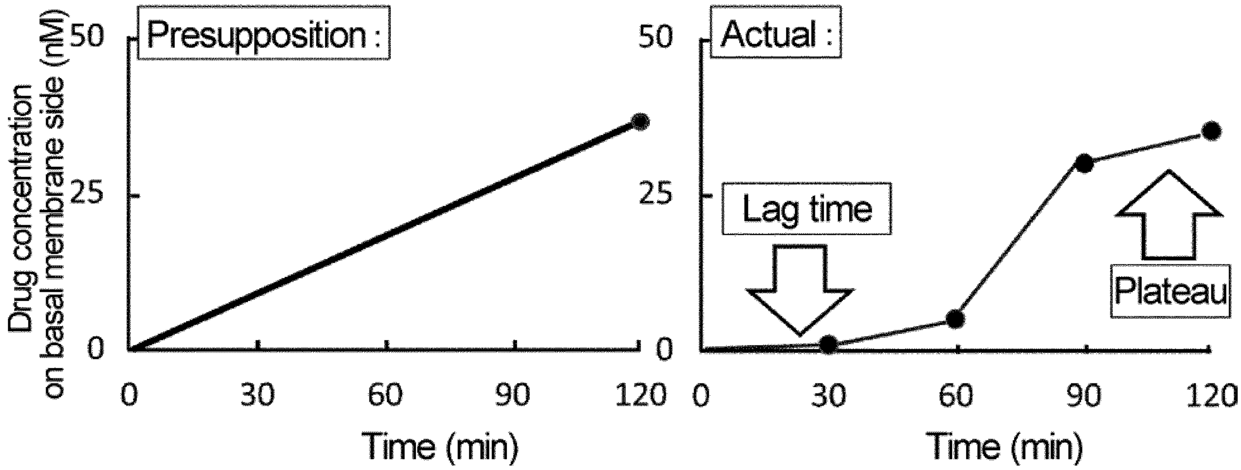
Drug concentration on basal membrane side (nM)
Presupposition :
Actual :
Lag time
Plateau
50
25
0
0
30
60
90
120
Time (min)
Time (min)

FIG. 2

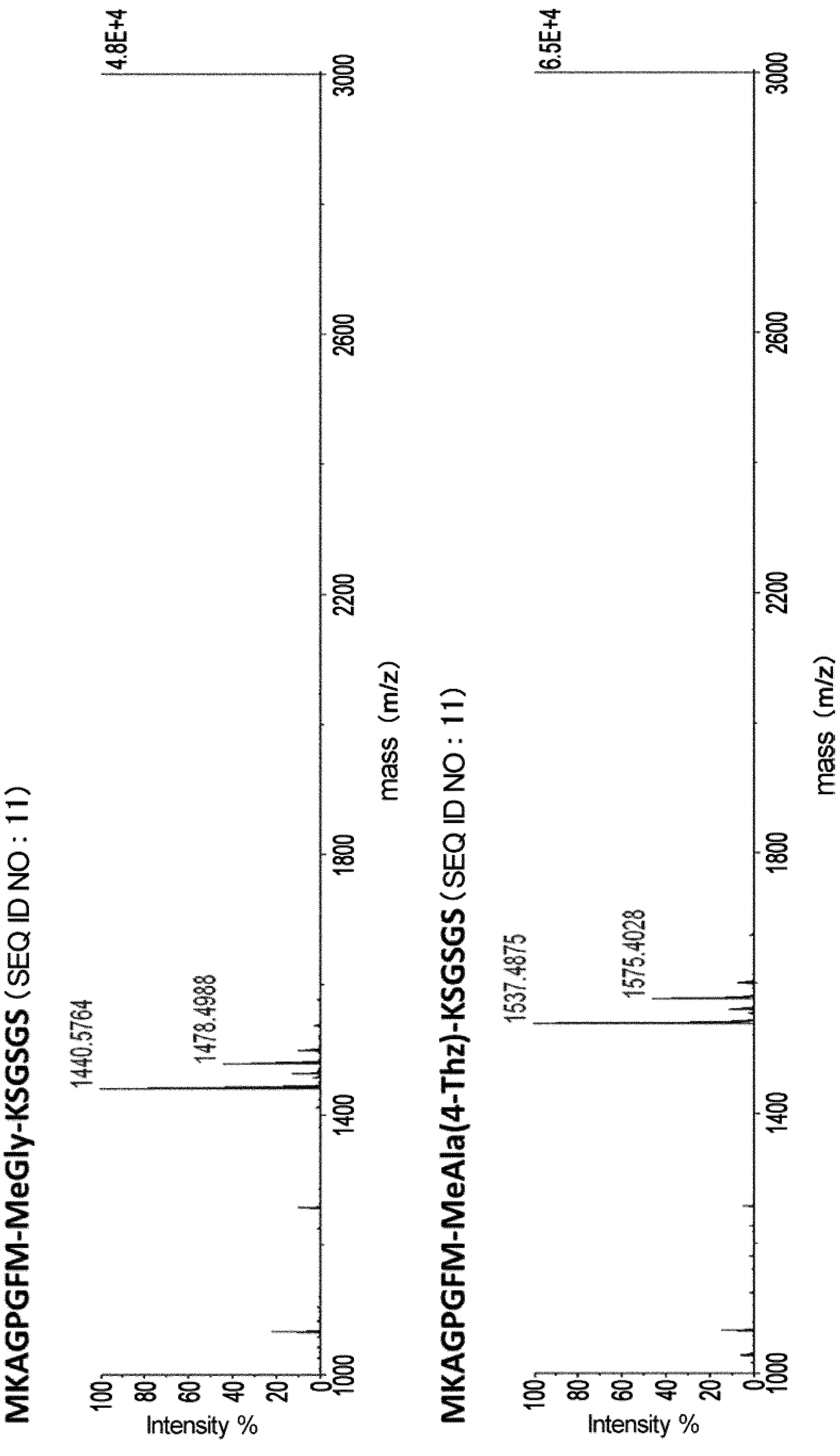

MKAGPGFM-MeGly-KSGSGS (SEQ ID NO : 11)
4.8E+4
1440.5764
1478.4988
Intensity %
100
80
60
40
20
0
1000
1400
1800
2200
2600
3000
mass (m/z)
MKAGPGFM-MeAla(4-Thz)-KSGSGS (SEQ ID NO : 11)
6.5E+4
1537.4875
1575.4028
Intensity %
100
80
60
40
20
0
1000
1400
1800
2200
2600
3000
mass (m/z)


FIG. 3-1

| Amino acid | Translated peptide compound | Molecular weight m/z: [M+H]+ (Observed value) |
|---|---|---|
| MeGly | MKAGPGFM-MeGly-KSGSGS | 1440.46 |
| MeAla(4-Thz) | MKAGPGFM-MeAla(4-Thz)-KSGSGS | 1537.27 |
| MePhe(4-Cl) | MKAGPGFM-MePhe(4-Cl)-KSGSGS | 1564.26 |
| Phe(3-Cl) | MKAGPGFM-Phe(3-Cl)-KSGSGS | 1550.24 |
| Met(O2) | MKAGPGFM-Met(O2)-KSGSGS | 1532.48 |
| Phg | MKAGPGFM-Phg-KSGSGS | 1502.42 |
| MeSer | MKAGPGFM-MeSer-KSGSGS | 1470.31 |
| Phe(4-CF3) | MKAGPGFM-Phe(4-CF3)-KSGSGS | 1584.30 |
| Ser(Me) | MKAGPGFM-Ser(Me)-KSGSGS | 1470.47 |
| Ser(nPr) | MKAGPGFM-Ser(nPr)-KSGSGS | 1498.35 |
| MeSer(nPr) | MKAGPGFM-MeSer(nPr)-KSGSGS | 1512.61 |
| Ser(iPen) | MKAGPGFM-Ser(iPen)-KSGSGS | 1526.39 |
| MeSer(iPen) | MKAGPGFM-MeSer(iPen)-KSGSGS | 1540.58 |
| Hse(Et) | MKAGPGFM-Hse(Et)-KSGSGS | 1498.64 |
| MeHse(Et) | MKAGPGFM-MeHse(Et)-KSGSGS | 1512.64 |
| Hnl(7-F2) | MKAGPGFM-Hnl(7-F2)-KSGSGS | 1532.55 |
| MeHnl(7-F2) | MKAGPGFM-MeHnl(7-F2)-KSGSGS | 1546.79 |
| Ser(F(4)nPr) | MKAGPGFM-Ser(F(4)nPr)-KSGSGS | 1570.51 |
| MeSer(F(4)nPr) | MKAGPGFM-MeSer(F(4)nPr)-KSGSGS | 1584.35 |
| Nle(6-OH) | MKAGPGFM-Nle(6-OH)-KSGSGS | 1498.52 |
| Ser(EtOH) | MKAGPGFM-Ser(EtOH)-KSGSGS | 1500.39 |
| MeSer(EtOH) | MKAGPGFM-MeSer(EtOH)-KSGSGS | 1514.65 |
| Ser(S-2-PrOH) | MKAGPGFM-Ser(S-2-PrOH)-KSGSGS | 1514.57 |
| MeSer(S-2-PrOH) | MKAGPGFM-MeSer(S-2-PrOH)-KSGSGS | 1528.41 |
| Ser(R-2-PrOH) | MKAGPGFM-Ser(R-2-PrOH)-KSGSGS | 1514.58 |
| MeSer(R-2-PrOH) | MKAGPGFM-MeSer(R-2-PrOH)-KSGSGS | 1528.39 |
| Ser(tBuOH) | MKAGPGFM-Ser(tBuOH)-KSGSGS | 1528.61 |
| MeSer(tBuOH) | MKAGPGFM-MeSer(tBuOH)-KSGSGS | 1542.80 |
| Ser(2-Me-BuOH) | MKAGPGFM-Ser(2-Me-BuOH)-KSGSGS | 1542.41 |
| MeSer(2-Me-BuOH) | MKAGPGFM-MeSer(2-Me-BuOH)-KSGSGS | 1556.48 |
| Gln(Me) | MKAGPGFM-Gln(Me)-KSGSGS | 1575.58 |
| Gln(Me2) | MKAGPGFM-Gln(Me2)-KSGSGS | 1525.77 |
| MeGln(Me2) | MKAGPGFM-MeGln(Me2)-KSGSGS | 1539.76 |
| Ser(NtBu-Aca) | MKAGPGFM-Ser(NtBu-Aca)-KSGSGS | 1569.83 |
| MeSer(NtBu-Aca) | MKAGPGFM-MeSer(NtBu-Aca)-KSGSGS | 1583.85 |

FIG. 3-2

| Amino acid | Translated peptide compound | Molecular weight m/z: [M+H]+ (Observed value) |
|---|---|---|
| Hph | MKAGPGFM-Hph-KSGSGS | 1530.53 |
| MeHph | MKAGPGFM-MeHph-KSGSGS | 1544.52 |
| Ser(Ph-2-Cl) | MKAGPGFM-Ser(Ph-2-Cl)-KSGSGS | 1566.54 |
| MeSer(Ph-2-Cl) | MKAGPGFM-MeSer(Ph-2-Cl)-KSGSGS | 1580.33 |
| Ser(Ph-3-Cl) | MKAGPGFM-Ser(Ph-3-Cl)-KSGSGS | 1566.53 |
| Hph(2-Cl) | MKAGPGFM-Hph(2-Cl)-KSGSGS | 1564.83 |
| MeHph(2-Cl) | MKAGPGFM-MeHph(2-Cl)-KSGSGS | 1578.81 |
| Hph(3-Cl) | MKAGPGFM-Hph(3-Cl)-KSGSGS | 1564.81 |
| MeHph(3-Cl) | MKAGPGFM-MeHph(3-Cl)-KSGSGS | 1578.81 |
| Hph(4-Cl) | MKAGPGFM-Hph(4-Cl)-KSGSGS | 1564.81 |
| MeHph(4-Cl) | MKAGPGFM-MeHph(4-Cl)-KSGSGS | 1578.81 |
| Ser(3-F,5-Me-Pyr) | MKAGPGFM-Ser(3-F,5-Me-Pyr)-KSGSGS | 1565.48 |
| MeSer(3-F,5-Me-Pyr) | MKAGPGFM-MeSer(3-F,5-Me-Pyr)-KSGSGS | 1579.40 |
| Phe{#(CH2)2} | MKAGPGFM-Phe{#(CH2)2}-KSGSGS | 1544.82 |
| MePhe{#(CH2)2} | MKAGPGFM-MePhe{#(CH2)2}-KSGSGS | 1558.85 |
| Ser(Bn) | MKAGPGFM-Ser(Bn)-KSGSGS | 1546.79 |
| MeSer(Bn) | MKAGPGFM-MeSer(Bn)-KSGSGS | 1560.82 |
| Hyp(Et) | MKAGPGFM-Hyp(Et)-KSGSGS | 1510.38 |
| Abu(pip-4-F2) | MKAGPGFM-Abu(pip-4-F2)-KSGSGS | 1573.66 |
| MeAbu(pip-4-F2) | MKAGPGFM-MeAbu(pip-4-F2)-KSGSGS | 1587.53 |
| MeAbu(pip-3-F2) | MKAGPGFM-MeAbu(pip-3-F2)-KSGSGS | 1587.53 |
| Ala(3-Pyr-4-NMe2) | MKAGPGFM-Ala(3-Pyr-4-NMe2)-KSGSGS | 1560.48 |
| MeAla(3-Pyr-4-NMe2) | MKAGPGFM-MeAla(3-Pyr-4-NMe2)-KSGSGS | 1574.45 |
| nPenGly | MKAGPGFM-nPenGly-KSGSGS | 1496.39 |
| nHexGly | MKAGPGFM-nHexGly-KSGSGS | 1510.41 |
| (PhEt)NGly | MKAGPGFM-(PhEt)NGly-KSGSGS | 1530.68 |
| (EtOEt)NGly | MKAGPGFM-(EtOEt)NGly-KSGSGS | 1498.68 |
| (PhOEt)NGly | MKAGPGFM-(PhOEt)NGly-KSGSGS | 1546.68 |
| Pro(pip-4-F2) | MKAGPGFM-Pro(pip-4-F2)-KSGSGS | 1586.05 |
| cisPro(pip-4-F2) | MKAGPGFM-cisPro(pip-4-F2)-KSGSGS | 1586.05 |
| MeNva(5-F2) | MKAGPGFM-MeNva(5-F2)-KSGSGS | 1518.32 |
| MePhe(3-Cl) | MKAGPGFM-MePhe(3-Cl)-KSGSGS | 1564.25 |
| Abu(Mor) | MKAGPGFM-Abu(Mor)-KSGSGS | 1539.70 |
| 2-(pip-4-F2)-EtGly | MKAGPGFM-2-(pip-4-F2)-EtGly-KSGSGS | 1574.04 |
| Phe(4-CHF2) | MKAGPGFM-Phe(4-CHF2)-KSGSGS | 1566.74 |
| Phe(4-OCHF2) | MKAGPG-Phe(4-OCHF2)-M-Phe(4-OCHF2)-KSGSGS | 1648.67 |
| Ser(Et-2-Mor) | MKAGPGFM-Ser(Et-2-Mor)-KSGSGS | 1569.68 |
| Ser(1-CF3-EtOH) | MKAGPGFM-Ser(1-CF3-EtOH)-KSGSGS | 1568.60 |

FIG. 3-3

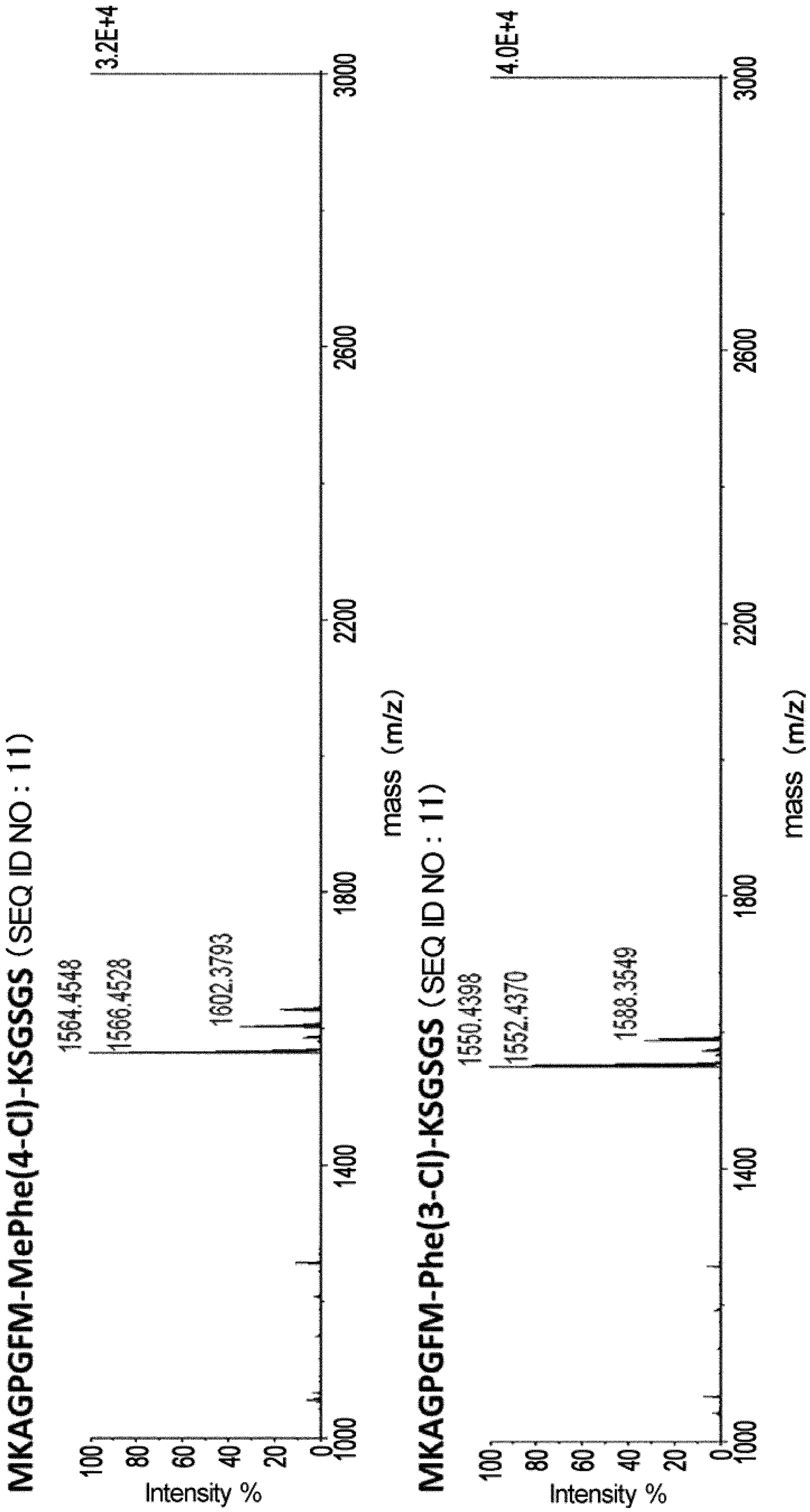
MKAGPGFM-MePhe(4-Cl)-KSGSGS（SEQ ID NO : 11)
3.2E+4
Intensity %
100
80
60
40
20
0
1564.4548
1566.4528
1602.3793
1000
1400
1800
2200
2600
3000
mass（m/z)
MKAGPGFM-Phe(3-Cl)-KSGSGS（SEQ ID NO : 11)
4.0E+4
Intensity %
100
80
60
40
20
0
1550.4398
1552.4370
1588.3549
1000
1400
1800
2200
2600
3000
mass（m/z)

FIG. 4

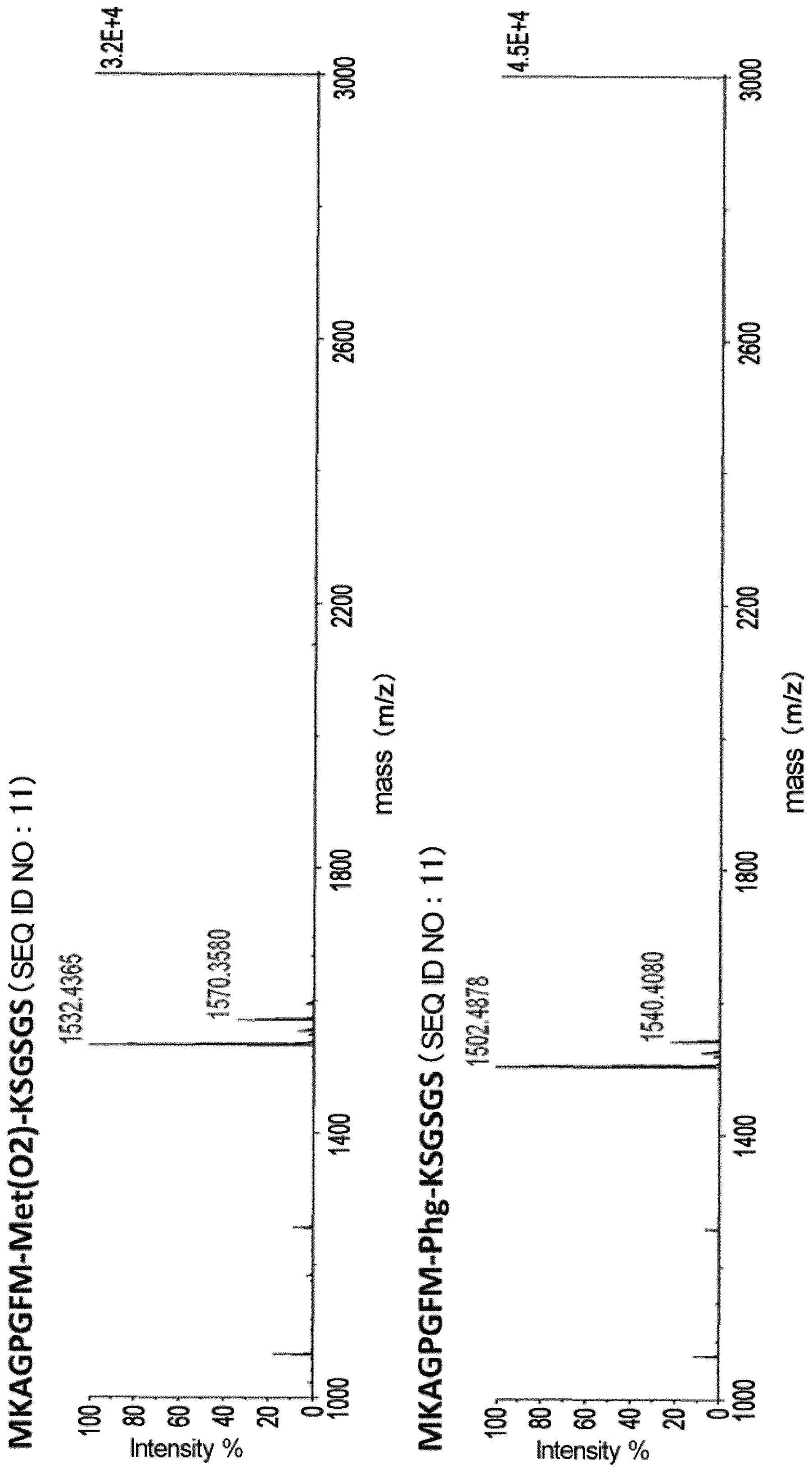
MKAGPGFM-Met(O2)-KSGSGS (SEQ ID NO : 11)
Intensity %
100
80
60
40
20
0
1000
1400
1800
2200
2600
3000
mass (m/z)
1532.4365
1570.3580
3.2E+4
MKAGPGFM-Phg-KSGSGS (SEQ ID NO : 11)
Intensity %
100
80
60
40
20
0
1000
1400
1800
2200
2600
3000
mass (m/z)
1502.4878
1540.4080
4.5E+4

FIG. 5

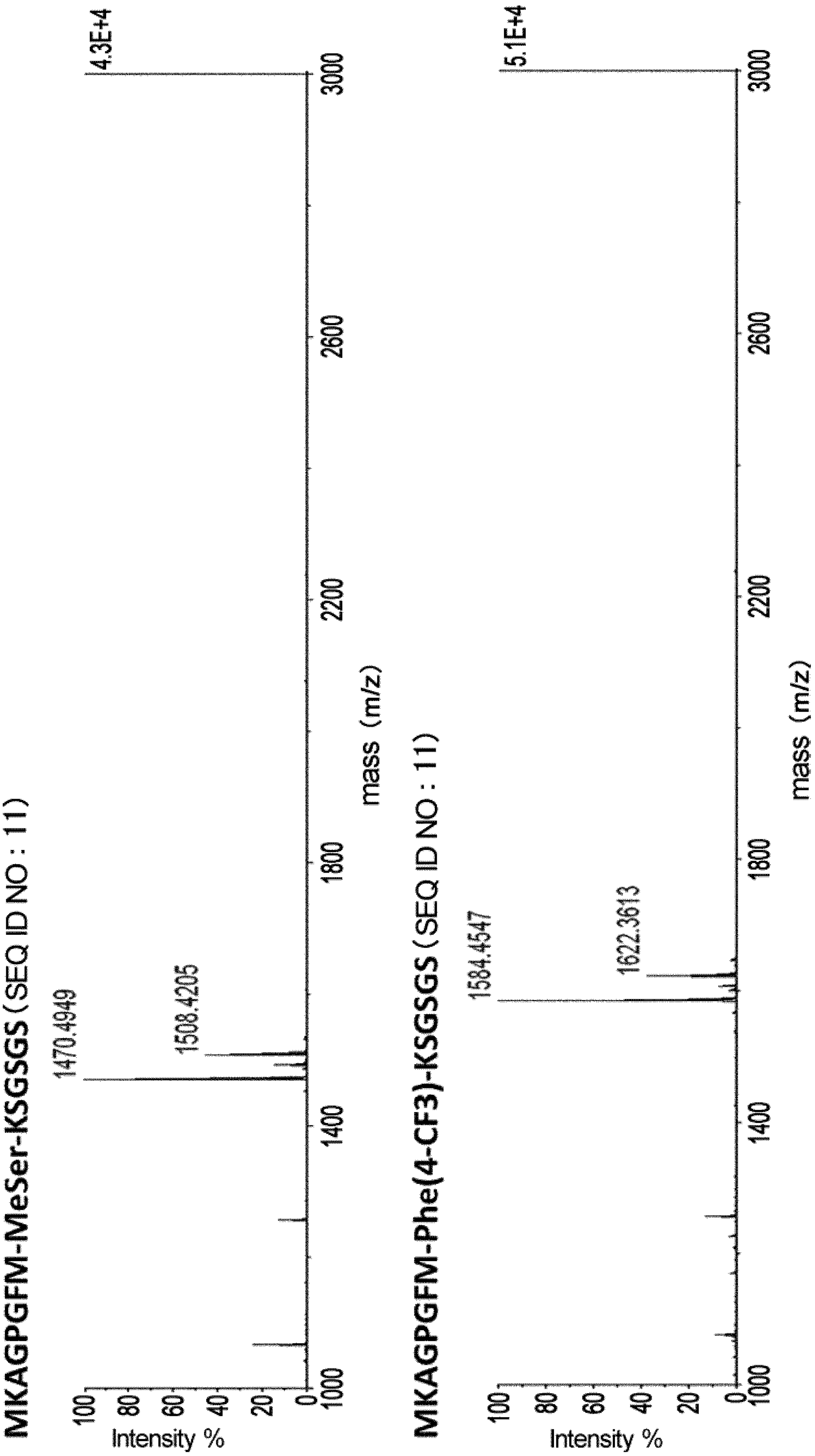
MKAGPGFM-MeSer-KSGSGS (SEQ ID NO : 11)
Intensity %
100
80
60
40
20
0
1470.4949
1508.4205
4.3E+4
1000
1400
1800
2200
2600
3000
mass (m/z)
MKAGPGFM-Phe(4-CF3)-KSGSGS (SEQ ID NO : 11)
Intensity %
100
80
60
40
20
0
1584.4547
1622.3613
5.1E+4
1000
1400
1800
2200
2600
3000
mass (m/z)

FIG. 6

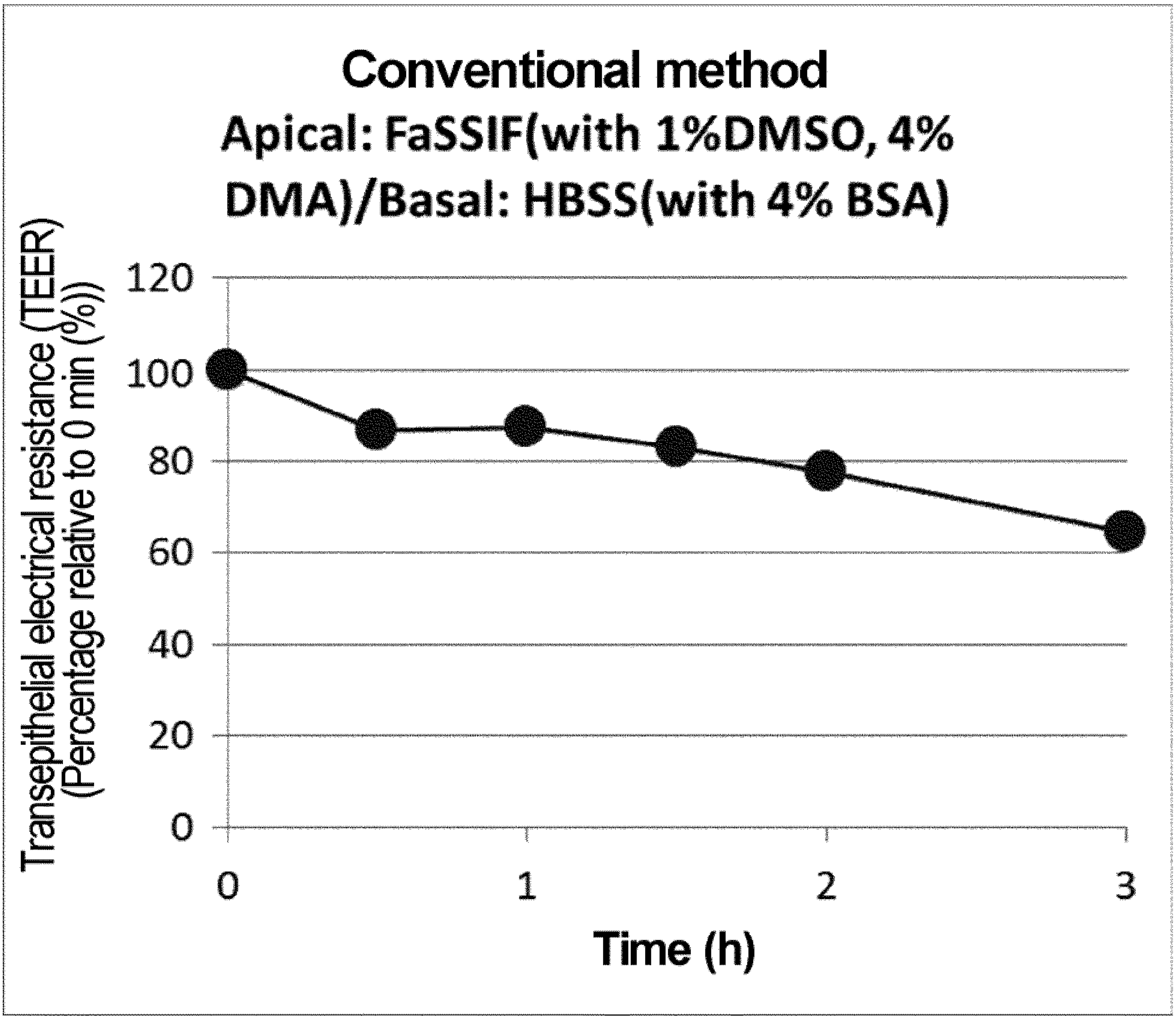
Conventional method
Apical: FaSSIF(with 1%DMSO, 4% DMA)/Basal: HBSS(with 4% BSA)
Transepithelial electrical resistance (TEER) (Percentage relative to 0 min (%))
120
100
80
60
40
20
0
0
1
2
3
Time (h)

FIG. 7

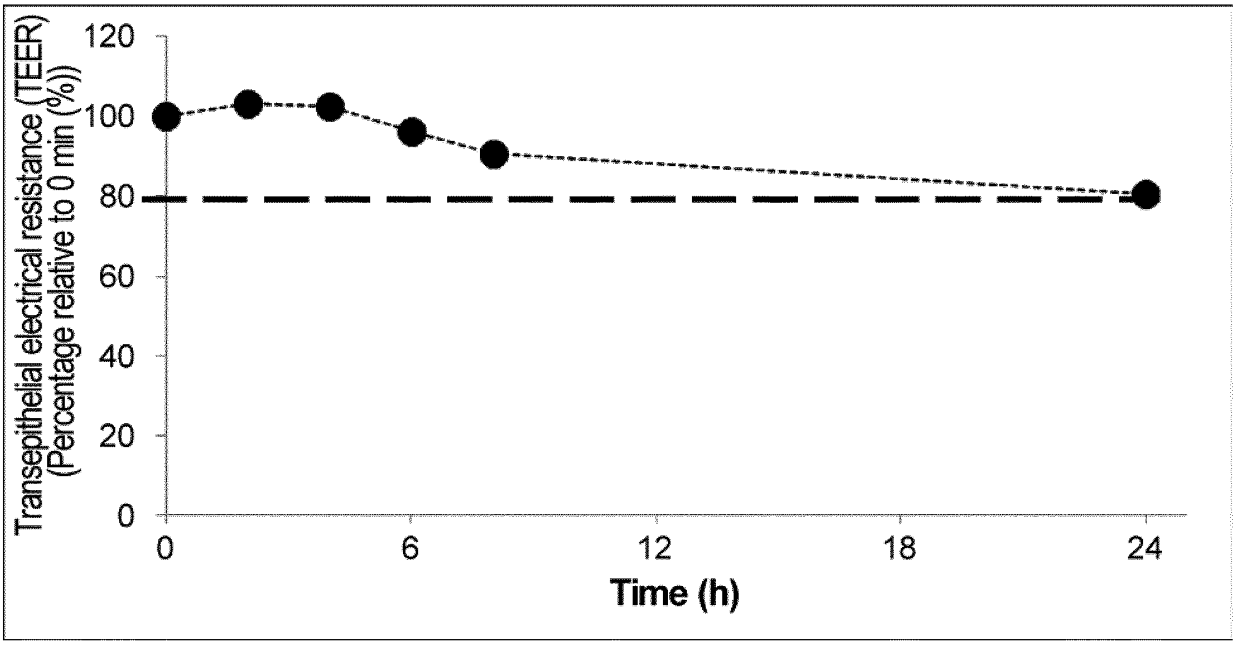
Transepithelial electrical resistance (TEER)
(Percentage relative to 0 min (%))
120
100
80
60
40
20
0
0
6
12
18
24
Time (h)

FIG. 8

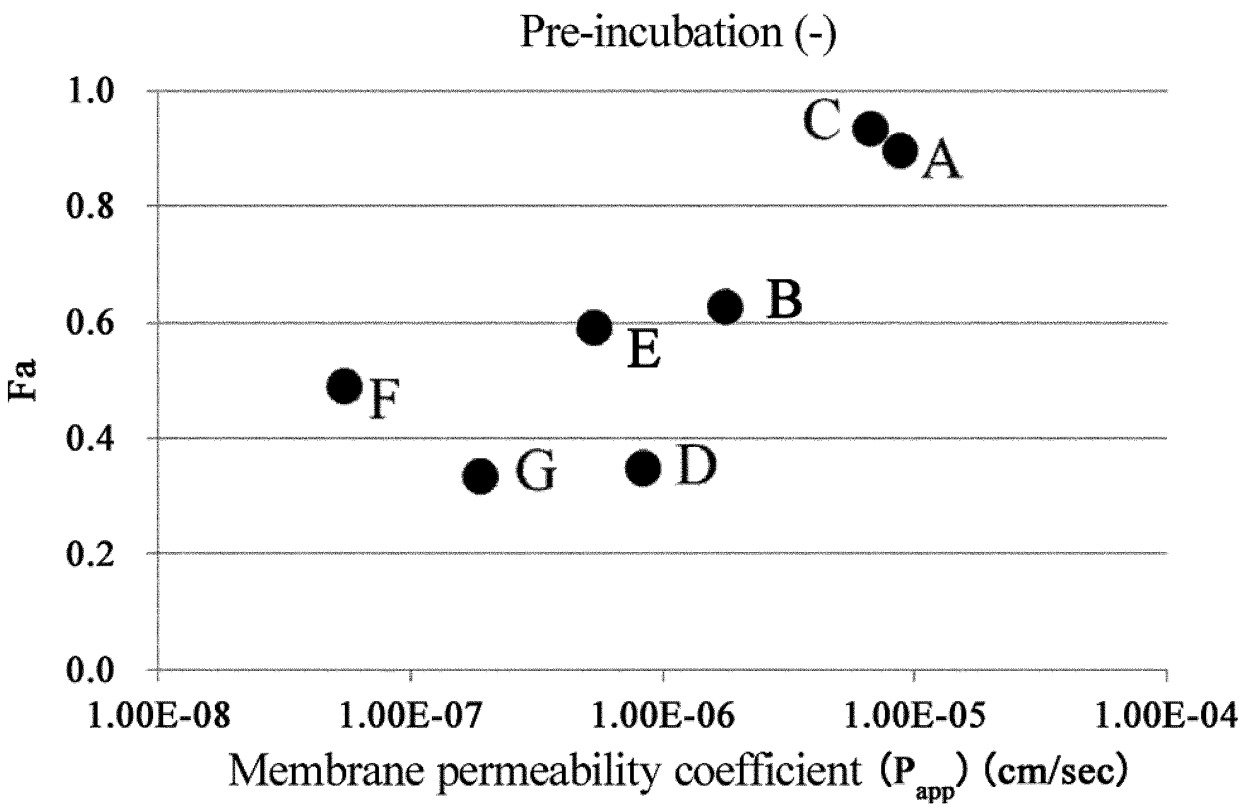
Pre-incubation (-)
1.0
0.8
0.6
0.4
0.2
0.0
Fa
C
A
B
E
F
G
D
1.00E-08
1.00E-07
1.00E-06
1.00E-05
1.00E-04
Membrane permeability coefficient ($P_{app}$) (cm/sec)

FIG. 9

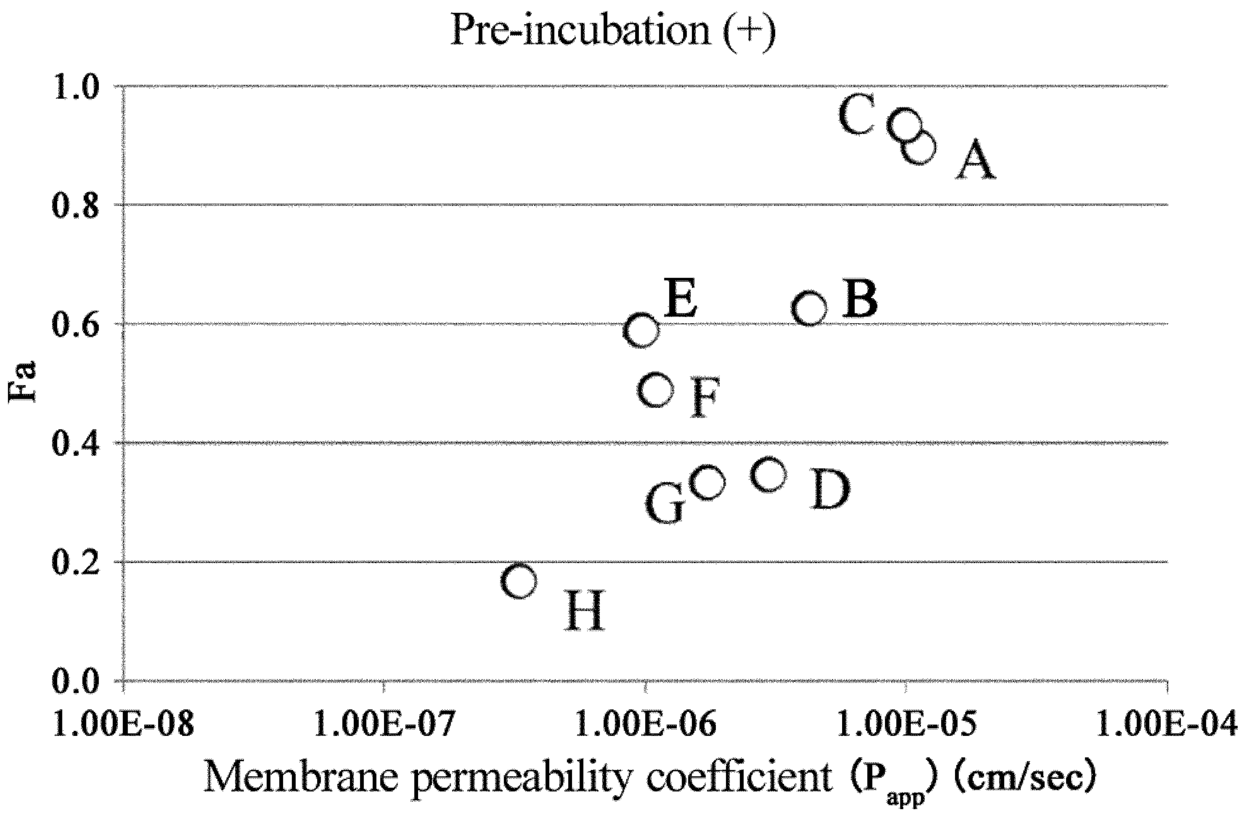
Pre-incubation (+)
1.0
0.8
0.6
0.4
0.2
0.0
Fa
C
A
E
B
F
G
D
H
1.00E-08
1.00E-07
1.00E-06
1.00E-05
1.00E-04
Membrane permeability coefficient ($P_{app}$) (cm/sec)

FIG. 10

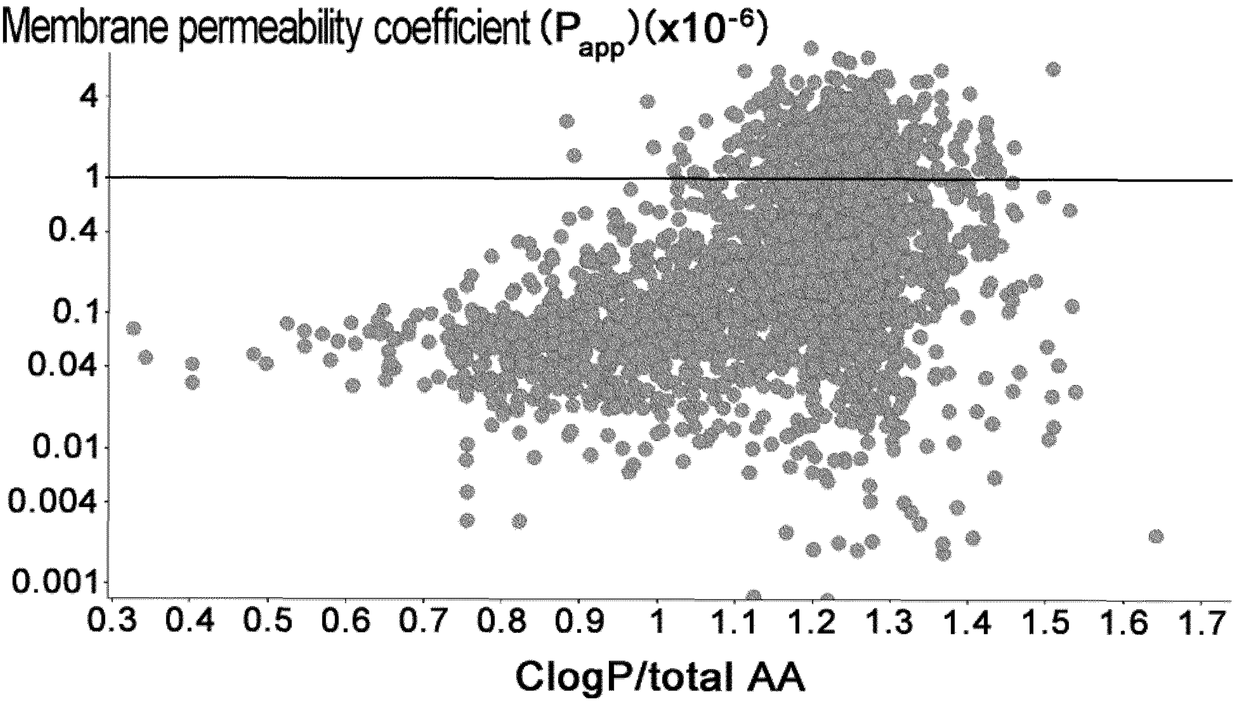
Membrane permeability coefficient ($P_{app}$)($x10^{-6}$)
4
1
0.4
0.1
0.04
0.01
0.004
0.001
0.3 0.4 0.5 0.6 0.7 0.8 0.9 1 1.1 1.2 1.3 1.4 1.5 1.6 1.7
ClogP/total AA

FIG. 11

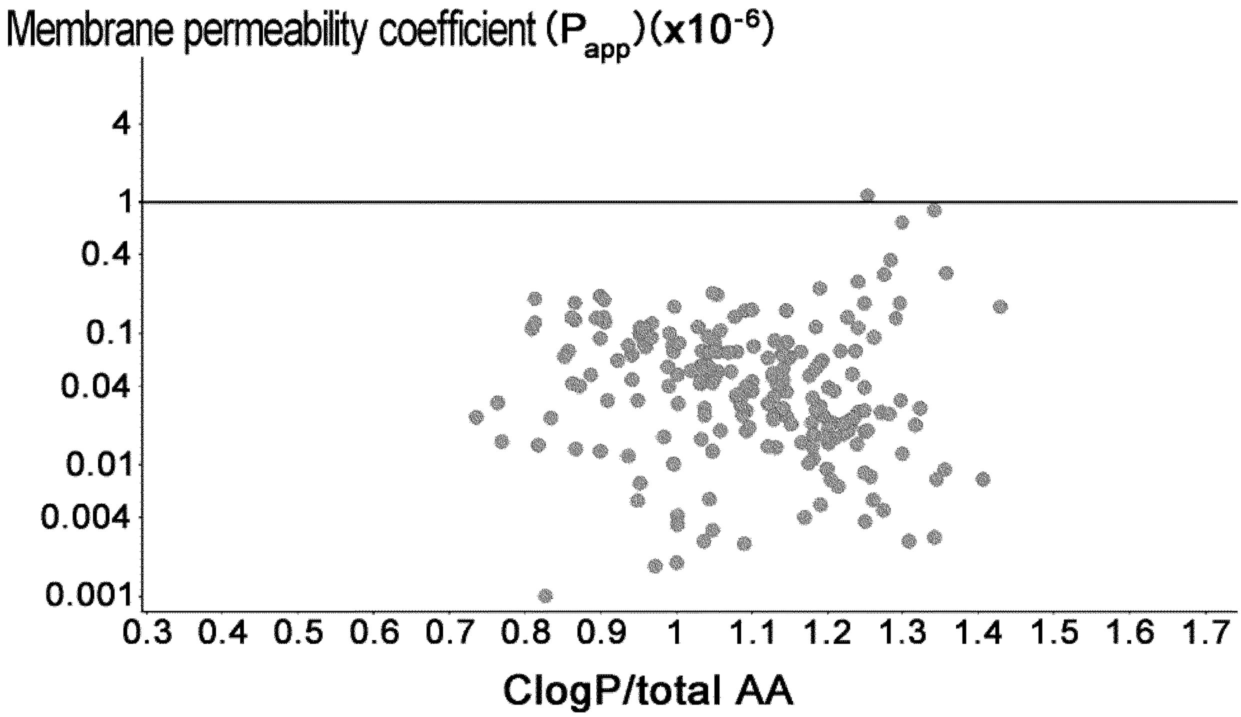
Membrane permeability coefficient ($P_{app}$)(x$10^{-6}$)
4
1
0.4
0.1
0.04
0.01
0.004
0.001
0.3
0.4
0.5
0.6
0.7
0.8
0.9
1
1.1
1.2
1.3
1.4
1.5
1.6
1.7
ClogP/total AA

FIG. 12

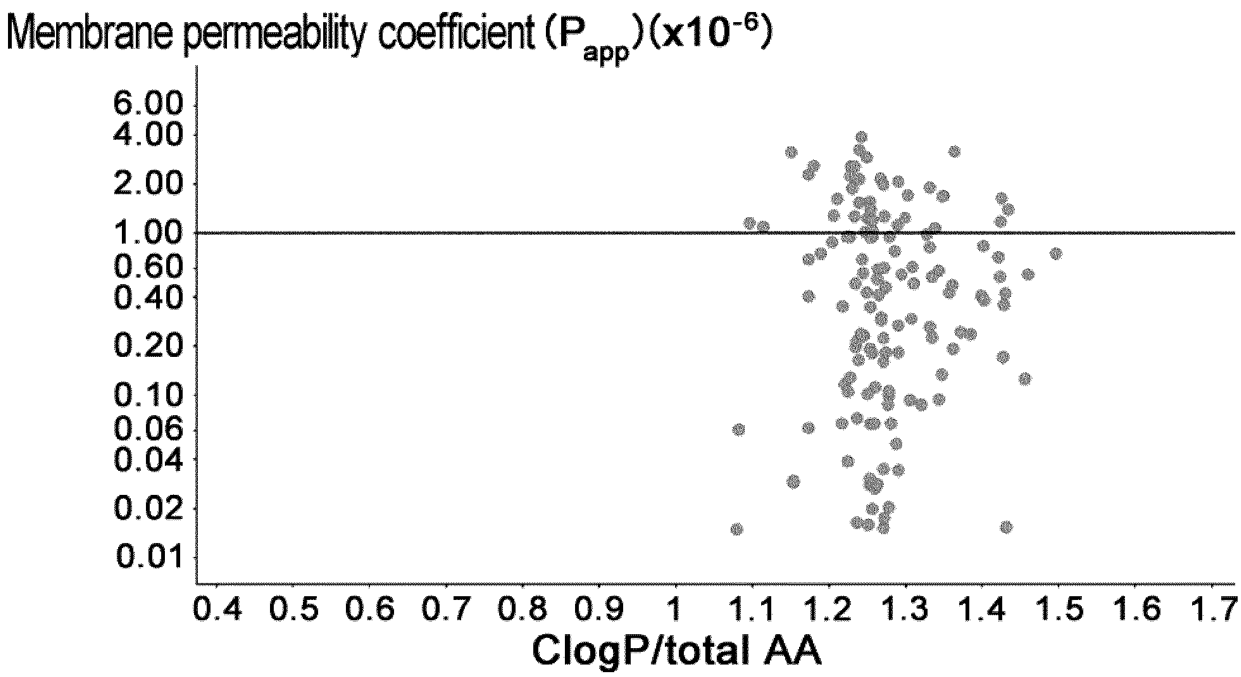
Membrane permeability coefficient (Papp)(x10-6)
6.00
4.00
2.00
1.00
0.60
0.40
0.20
0.10
0.06
0.04
0.02
0.01
0.4 0.5 0.6 0.7 0.8 0.9 1 1.1 1.2 1.3 1.4 1.5 1.6 1.7
ClogP/total AA

FIG. 13

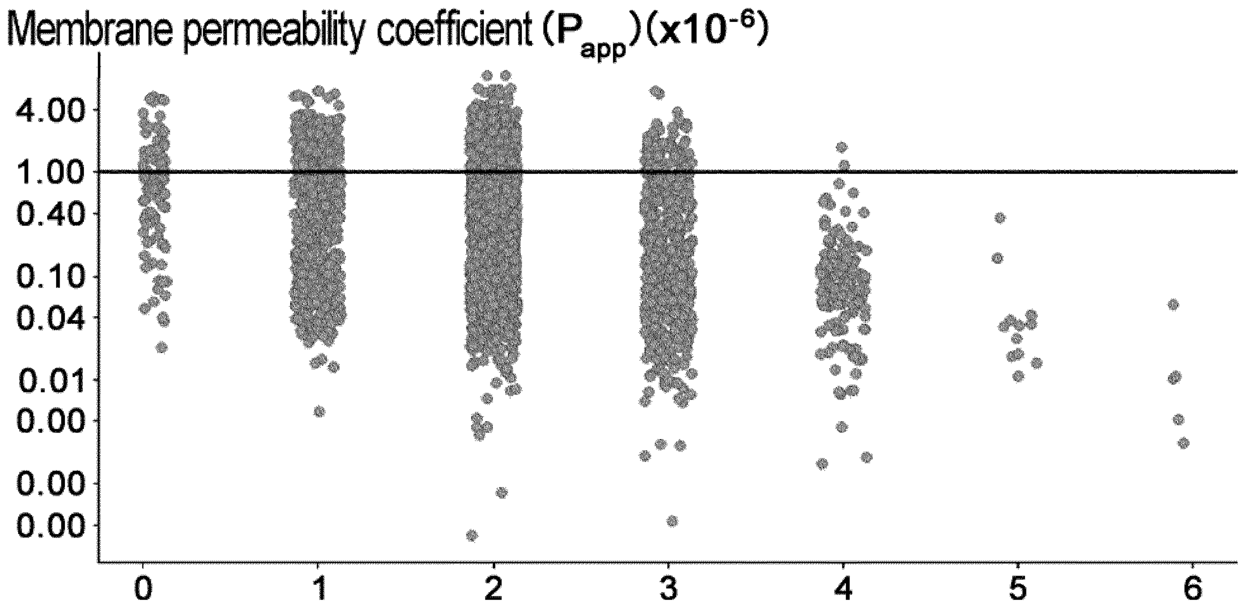
Membrane permeability coefficient ($P_{app}$)(x$10^{-6}$)
4.00
1.00
0.40
0.10
0.04
0.01
0.00
0.00
0.00
0
1
2
3
4
5
6

FIG. 14

**INTERNATIONAL SEARCH REPORT**

International application No. PCT/JP2018/022097

A. CLASSIFICATION OF SUBJECT MATTER
Int.Cl. C40B40/10(2006.01)i, C07K1/13(2006.01)i, C07K2/00(2006.01)i, C12P21/02(2006.01)i, C40B30/04(2006.01)i, G01N33/566(2006.01)i, C07K7/64(2006.01)n, C12N15/09(2006.01)n

According to International Patent Classification (IPC) or to both national classification and IPC

B. FIELDS SEARCHED

Minimum documentation searched (classification system followed by classification symbols)
Int.Cl. C40B40/10, C07K1/13, C07K2/00, C12P21/02, C40B30/04, G01N33/566, C07K7/64, C12N15/09

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
CAplus/REGISTRY (STN), JSTPlus/JMEDPlus/JST7580 (JDreamIII)

C. DOCUMENTS CONSIDERED TO BE RELEVANT

| Category* | Citation of document, with indication, where appropriate, of the relevant passages | Relevant to claim No. |
|---|---|---|
| X<br>Y<br>A | LOW, K. E. et al., "Rational design of calpain inhibitors based on calpastatin peptidomimetics", Journal of Medicinal Chemistry, 2016, vol. 59, pp. 5403-5415, entire description, in particular, page 5404, fig. 2, pp. 5406-5407 | 1-7, 9-11, 13-15<br>12<br>8 |
| X<br>Y<br>A | OVADIA, O. et al., "The effect of multiple N-methylation on intestinal permeability of cyclic hexapeptides", Molecular Pharmaceutics, 2011, vol. 8, pp. 479-487, entire description, in particular, page 480, fig. 1, p. 481, fig. 2 | 1-3, 5-8, 10, 11, 13, 14<br>12<br>4, 9, 15 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 16 August 2018 (16.08.2018) | 28 August 2018 (28.08.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT

International application No.
PCT/JP2018/022097

C (Continuation). DOCUMENTS CONSIDERED TO BE RELEVANT

| Category* | Citation of document, with indication, where appropriate, of the relevant passages | Relevant to claim No. |
|---|---|---|
| X<br><br>Y<br>A | TAKAHASHI, T. et al., "Solid phase library synthesis of cyclic depsipeptides: aurilide and aurilide analogues", Journal of Combinatorial Chemistry, 2003, vol. 5, pp. 414-428, entire description, in particular, pages 415, 417, 419 | 1-3, 5-8, 10, 11, 13, 14<br>12<br>4, 9, 15 |
| X<br>Y<br>A | WO 2013/100132 A1 (CHUGAI PHARMACEUTICAL CO., LTD.) 04 July 2013, claims, pp. 146-150, examples & US 2015/0080549 A1 claims, paragraphs [0451]-[0456], examples & EP 2813512 A1 | 15<br>12<br>1-11, 13, 14 |
| P, X<br>P, Y | WO 2017/136708 A1 (THE JOHNS HOPKINS UNIVERSITY) 10 August 2017, claims, examples (Family: none) | 1-11, 13-15<br>12 |
| A | JP 2017-43615 A (SHIONOGI & CO., LTD.) 02 March 2017 (Family: none) | 1-15 |

Form PCT/ISA/210 (continuation of second sheet) (January 2015)

**INTERNATIONAL SEARCH REPORT**

International application No.
PCT/JP2018/022097

It is recognized that the invention in claim 15 pertains to a cyclic peptide compound which is specified so as to satisfy the conditions (1) to (5), and is a candidate compound that binds to a target molecule and has a cell membrane permeability. Since acidic amino acids and basic amino acids that are natural amino acids are included in the ranges of acidic side chain pKa and basic side chain Basic pKa defined in (2) and (3), the group is not recognized to be a special feature. In addition, although it is specified that the compound has a long side chain having a length of 6.0-13 Å and having a group represented by $R^1$ according to (4) and (5), a halogenated phenyl group, a commonly used non-natural amino acid, and an amino acid having various constitutions are included in those corresponding to the group. In addition, although the amino acid constituting the cyclic peptide compound is merely specified, the entire structure thereof, namely, an amino acid sequence and the like are not specifically specified. Therefore, said peptide compound are considered to include numerous peptide compounds having various structures (constituent amino acid number, amino acid sequence, side chain, and structures of parts other than amino acids).

However, a compound which consists of 11 amino acids, is configured only by a limited number of amino acids, and also uses a specific substance as described in paragraph [0115] with respect to the long side chain, is merely described in the specification, and only some cyclic peptide compounds having a specific structure are disclosed within the meaning of PCT Article 5. To this end, even considering common technical knowledge at the time of filing, it is difficult to understand what kind of compound other than the compound described in the examples or a compound similar thereto can be used as a candidate compound that binds to a target molecule and has cell membrane permeability.

Thus, the invention in claim 15 is beyond the scope disclosed by the specification, and does not comply with the requirements for evidence under PCT Article 6.

In addition, as disclosed in document 4, most compounds in the embodiments of the present application are considered to be known compounds, and thus the technical feature in common with the compounds of claim 15 are considered to be unclear.

Thus, claim 15 does not comply with the requirements for clarity under PCT Article 6.

Accordingly, a meaningful search for all of the cyclic peptide compounds cannot be carried out, and thus a search was carried out for the scope of the example compounds, i.e., for compounds satisfying all of the following conditions of a) to c): a) having phenylalanine or proline substituted with an alkoxy group having 2 or more carbon atoms; b) having leucine; and C) having 1 C(O)-piperidine or $-C(O)-N-CHC(O)-CH_2-Ph$ in the side chain.

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013100132 A
- WO 2012033154 A
- WO 2016148044 A
- WO 2008117833 A
- WO 2012074129 A
- WO 2013100132 A1


### Non-patent literature cited in the description


- **VILLAR et al.** *Nat Chem Biol.,* September 2014, vol. 10 (9), 723-731
- Thermal Measurements and Equilibrium. Experimental Chemistry Lecture. Maruzen Co., Ltd, vol. 5, 460
- **ARTURSSON, P.** *Adv Drug Deliv Rev,* 2001, vol. 46, 27-43
- **MASON, A.K.** *Drug Metab Dispos,* 2013, vol. 41, 1347-1366
- **POLLI, J.W.** *J Pharmacol Exp Ther,* 2001, vol. 299, 620-628
- **SUN, H.** *Expert Opin Drug Metab Toxicol,* 2008, vol. 4, 395-411
- **BHOOPATHY, S. et al.** *Methods Mol Biol,* 2014, vol. 1113, 229-252
- **KNIPP, G.T. et al.** *J Pharm Sci,* 1997, vol. 86, 1105-1110
- **KORZEKWA, K.R. et al.** *Drug Metab Dispos,* 2012, vol. 40, 865-876
- **SUN, H. et al.** *Drug Metab Dispos,* 2008, vol. 36, 102-123
- **OZEKI K. et al.** *Int J Pharm.,* 2015, vol. 495, 963-971
- **P. ARTURSSONAND J. et al.** *Biochem Biophys Res Commun.,* 1991, vol. 175, 880-885
- **ROBERTS, RW. ; SZOSTAK, JW.** RNA-peptide fusions for the in vitro selection of peptides and proteins. *Proc. Natl. Acad. Sci. USA.,* 1997, vol. 94, 12297-302
- **NEMOTO, N ; MIYAMOTO-SATO, E ; HUSIMI, Y ; YANAGAWA, H.** In vitro virus: bonding of mRNA bearing puromycin at the 3'-terminal end to the C-terminal end of its encoded protein on the ribosome in vitro. *FEBS Lett.,* 1997, vol. 414, 405-8
- **YAMAGUCHI, J. ; NAIMUDDIN, M ; BIYANI, M ; SASAKI, T ; MACHIDA, M ; KUBO, T ; FUNATSU, T ; HUSIMI, Y ; NEMOTO, N.** cDNA display: a novel screening method for functional disulfide-rich peptides by solid-phase synthesis and stabilization of mRNA-protein fusions. *Nucleic Acids Res.,* 2009, vol. 37 (16), e108
- **MATTHEAKIS, LC ; BHATT, RR ; DOWER, WJ.** An in vitro polysome display system for identifying ligands from very large peptide libraries. *Proc. Natl. Acad. Sci. USA.,* 1994, vol. 91, 9022-6
- **REIERSEN, H ; LOBERSLI, I ; LOSET, GA ; HVATTUM, E ; SIMONSEN, B ; STACY, JE ; MCGREGOR, D ; FITZGERALD, K ; WELSCHOF, M ; BREKKE, OH.** Covalent antibody display-an in vitro antibody-DNA library selection system. *Nucleic Acids Res.,* 2005, vol. 33, e10
- **ODEGRIP, R ; COOMBER, D ; ELDRIDGE, B ; HEDERER, R ; KUHLMAN, PA ; ULLMAN, C ; FITZGERALD, K ; MCGREGOR, D.** CIS display: In vitro selection of peptides from libraries of protein-DNA complexes. *Proc. Natl. Acad. Sci. USA.,* 2004, vol. 101, 2806-10
- **TAWFIK, DS ; GRIFFITHS, AD.** Man-made cell-like compartments for molecular evolution. *Nat. Biotechnol.,* 1998, vol. 16, 652-6
- Solid-phase Synthesis Handbook. Merck Co, 01 May 2002
- **SHIMIZU, Y. ; INOUE, A. ; TOMARI, Y. ; SUZUKI, T. ; YOKOGAWA, T. ; NISHIKAWA, K. ; UEDA, T.** Cell-free translation reconstituted with purified components. *Nat. Biotechnol.,* 2001, vol. 19, 751-5
- **SHIMIZU, Y. ; UEDA, T.** PURE technology. *Methods Mol. Biol.,* 2010, vol. 607, 11-21
- **JOSEPHSON, K. ; HARTMAN, MC. ; SZOSTAK, JW.** Ribosomal synthesis of unnatural peptides. *J. Am. Chem. Soc.,* 2005, vol. 127, 11727-35
- **KWON, I. et al.** Breaking the degeneracy of the genetic code. *J. Am. Chem. Soc.,* 2003, vol. 125, 7512-3
- **KAWAKAMI, T. et al.** Ribosomal synthesis of polypeptoids and peptoid-peptide hybrids. *J. Am. Chem. Soc.,* 2008, vol. 130, 16861-3
- **KAWAKAMI, T. et al.** Diverse backbone-cyclized peptides via codon reprogramming. *Nat. Chem. Biol.,* 2009, vol. 5, 888-90

- **DEDKOVA LM et al.** Construction of modified ribosomes for incorporation of D-amino acids into proteins. *Biochemistry,* 2006, vol. 45, 15541-51
- **DOI Y et al.** Elongation factor Tu mutants expand amino acid tolerance of protein biosynthesis system. *J Am Chem Soc.,* 2007, vol. 129, 14458-62
- **PARK HS et al.** Expanding the genetic code of Escherichia coli with phosphoserine. *Science,* 2011, vol. 333, 1151-4
- **ANDERSON, JC. ; SCHULTZ, PG.** Adaptation of an orthogonal archaeal leucyl-tRNA and synthetase pair for four-base, amber, and opal suppression. *Biochemistry,* 2003, vol. 42, 9598-608
- **MURAKAMI, H. ; KOUROUKLIS, D. ; SUGA, H.** Using a solid-phase ribozyme aminoacylation system to reprogram the genetic code. *Chem. Biol.,* 2003, vol. 10, 1077-84
- **KIGA, D. ; SAKAMOTO, K. ; KODAMA, K. ; KIGAWA, T. ; MATSUDA, T. ; YABUKI, T. ; SHIROUZU, M. ; HARADA, Y. ; NAKAYAMA, H. ; TAKIO, K.** An engineered Escherichia coli tyrosyl-tRNA synthetase for site-specific incorporation of an unnatural amino acid into proteins in eukaryotic translation and its application in a wheat germ cell-free system. *Proc. Natl. Acad. Sci. U S A.,* 2002, vol. 99, 9715-20
- **CHIN, JW. ; CROPP, TA. ; ANDERSON, JC. ; MUKHERJI, M. ; ZHANG, Z. ; SCHULTZ, PG. ; CHIN, JW.** An expanded eukaryotic genetic code. *Science,* 2003, vol. 301, 964-7
- **HARTMAN, MC. ; JOSEPHSON, K. ; SZOSTAK, JW.** Enzymatic aminoacylation of tRNA with unnatural amino acids. *Proc. Natl. Acad. Sci. U S A.,* 2006, vol. 103, 4356-61
- **SUBTELNY, AO. ; HARTMAN, MC. ; SZOSTAK, JW.** Ribosomal synthesis of N-methyl peptides. *J. Am. Chem. Soc.,* 2008, vol. 130, 6131-6
- **HECKLER, TG. ; CHANG, LH. ; ZAMA, Y. ; NAKA, T. ; CHORGHADE, MS. ; HECHT, SM.** T4 RNA ligase mediated preparation of novel "chemically misacylated" tRNAPheS. *Biochemistry,* 1984, vol. 23, 1468-73
- **MURAKAMI, H. ; BONZAGNI, NJ. ; SUGA, H.** Aminoacyl-tRNA synthesis by a resin-immobilized ribozyme. *J. Am. Chem. Soc.,* 2002, vol. 124, 6834-5
- **HASHIMOTO, N. ; NINOMIYA, K. ; ENDO, T. ; SISIDO, M.** Simple and quick chemical aminoacylation of tRNA in cationic micellar solution under ultrasonic agitation. *Chem. Commun.,* 2005, 4321-3
- **NINOMIYA, K. ; MINOHATA, T. ; NISHIMURA, M. ; SISIDO, M.** In situ chemical aminoacylation with amino acid thioesters linked to a peptide nucleic acid. *J. Am. Chem. Soc.,* 2004, vol. 126, 15984-9
- *CHEMICAL ABSTRACTS,* 146060-18-6
- *CHEMICAL ABSTRACTS,* 121343-82-6
- *Angew. Chem. Int. Ed.,* 2014, vol. 53, 4642
- *CHEMICAL ABSTRACTS,* 87413-09-0
- *CHEMICAL ABSTRACTS,* 39608-31-6
- *Letters in Peptie Science,* 2002, vol. 9, 203
- *Bioconjugate Chem.,* 2008, vol. 19, 714
- *Helv. Chim. Acta,* vol. 90, 297-310
- *Organic Letters,* 2011, vol. 13, 4906
- *CHEMICAL ABSTRACTS,* 943-73-7
- *CHEMICAL ABSTRACTS,* 39716-58-0
- **QINGQING X. et al.** *Xenobiotica,* 2016, vol. 46, 913-921
- *BMC biotechnology,* 2008, vol. 8, 41
- *Protein Science,* 1999, vol. 8, 921-929
- *J Biochem.,* 1990, vol. 108 (4), 673-6
- *Biochem Biophys Res Commun.,* 26 December 2008, vol. 377 (4), 1123-7
- *Nucleic Acids Res.,* April 2010, vol. 38 (6), e89

SEQUENCE LISTING

<110> CHUGAI SEIYAKU KABUSHIKI KAISHA

<120> Cyclic peptide compounds with high membrane permeability and libraries comprising them

<130> C1-A1708P

<150> JP2017-114074
<151> 2017-06-09

<160> 23

<170> PatentIn version 3.5

<210> 1
<211> 74
<212> RNA
<213> Artificial Sequence

<220>
<223> tRNA Glu UAG (-CA)

<400> 1
guccccuucg ucuagaggcc caggacaccg cccuuagacg gcgguaacag ggguucgaau 60

ccccuagggg acgc 74

<210> 2
<211> 76
<212> RNA
<213> Artificial Sequence

<220>
<223> tRNA Glu UAG

<400> 2
guccccuucg ucuagaggcc caggacaccg cccuuagacg gcgguaacag ggguucgaau 60

ccccuagggg acgcca 76

<210> 3
<211> 74
<212> RNA
<213> Artificial Sequence

<220>
<223> T-1: tRNAGluCUA(-CA)

<400> 3
guccccuucg ucuagaggcc caggacaccg cccucuaacg gcgguaacag ggguucgaau 60

ccccuagggg acgc 74

<210> 4
<211> 74
<212> RNA
<213> Artificial Sequence

<220>
<223> T-2: tRNAGluCAA(-CA)

<400> 4
guccccuucg ucuagaggcc caggacaccg cccucaaacg gcgguaacag ggguucgaau 60

ccccuagggg acgc 74

<210> 5
<211> 74
<212> RNA
<213> Artificial Sequence

<220>
<223> T-3: tRNAGluUAG(-CA)

<400> 5
guccccuucg ucuagaggcc caggacaccg cccuuagacg gcgguaacag ggguucgaau 60

ccccuagggg acgc 74

<210> 6
<211> 74
<212> RNA
<213> Artificial Sequence

<220>
<223> T-4: tRNAGluCUG(-CA)

<400> 6
guccccuucg ucuagaggcc caggacaccg cccucugacg gcgguaacag ggguucgaau 60

ccccuagggg acgc 74

<210> 7
<211> 74
<212> RNA
<213> Artificial Sequence

<220>
<223> T-5: tRNAGluCCG(-CA)

<400> 7
guccccuucg ucuagaggcc caggacaccg cccuccgacg gcgguaacag ggguucgaau 60

ccccuagggg acgc 74

<210> 8
<211> 74
<212> RNA
<213> Artificial Sequence

<220>
<223> T-6: tRNAGluCCU(-CA)

<400> 8
guccccuucg ucuagaggcc caggacaccg cccuccuacg gcgguaacag ggguucgaau 60

ccccuagggg acgc 74

<210> 9
<211> 74
<212> RNA
<213> Artificial Sequence

<220>
<223> T-7: tRNAGluUUC(-CA)

<400> 9
guccccuucg ucuagaggcc caggacaccg cccuuucacg gcgguaacag gggucgaau 60

ccccuagggg acgc 74

<210> 10
<211> 75
<212> RNA
<213> Artificial Sequence

<220>
<223> T-10: tRNAfMetCAU(-CA)

<400> 10
ggcgggguggагcagccugg uagcucgucg ggcucauaac ccgaagaucg ucgguucaaa 60

uccggccccc gcaac 75

<210> 11
<211> 15
<212> PRT
<213> Artificial Sequence

<220>
<223> An artificially synthesized peptide sequence

<220>
<221> MISC_FEATURE
<222> (9)..(9)
<223> Xaa = MeGly or MeAla(4-Thz) or MePhe(4-Cl) or Phe(3-Cl) or Met(O2) or Phg or MeSer or Phe(4-CF3)

<400> 11

Met Lys Ala Gly Pro Gly Phe Met Xaa Lys Ser Gly Ser Gly Ser
1               5                   10                  15

<210> 12
<211> 82
<212> RNA
<213> Artificial Sequence

<220>
<223> mRNA

<400> 12
ggguuaacuu uaagaaggag auauacauau gaaggcuggu ccggguuuua ugcuaaagag 60

ugguaguggu aguuaagcuu cg 82

<210> 13
<211> 74
<212> RNA
<213> Artificial Sequence

<220>
<223> An artificially synthesized sequence

<400> 13
guccccuucg ucuagaggcc caggacaccg cccuaaaacg gcgguaacag ggguucgaau 60

ccccuagggg acgc 74

<210> 14
<211> 74
<212> RNA
<213> Artificial Sequence

<220>
<223> An artificially synthesized sequence

<400> 14
guccccuucg ucuagaggcc caggacaccg cccuagaacg gcgguaacag ggguucgaau 60

ccccuagggg acgc 74

<210> 15
<211> 74
<212> RNA
<213> Artificial Sequence

<220>
<223> An artificially synthesized sequence

<400> 15
guccccuucg ucuagaggcc caggacaccg cccuauaacg gcgguaacag ggguucgaau 60

ccccuagggg acgc 74

<210> 16
<211> 74
<212> RNA
<213> Artificial Sequence

<220>
<223> An artificially synthesized sequence

<400> 16
guccccuucg ucuagaggcc caggacaccg cccuacaacg gcgguaacag ggguucgaau 60

ccccuagggg acgc 74

<210> 17
<211> 74
<212> RNA
<213> Artificial Sequence

<220>
<223>  An artificially synthesized sequence

<400>  17
guccccuucg ucuagaggcc caggacaccg cccuaagacg gcgguaacag ggguucgaau    60

ccccuagggg acgc    74

<210>  18
<211>  74
<212>  RNA
<213>  Artificial Sequence

<220>
<223>  An artificially synthesized sequence

<400>  18
guccccuucg ucuagaggcc caggacaccg cccuaugacg gcgguaacag ggguucgaau    60

ccccuagggg acgc    74

<210>  19
<211>  74
<212>  RNA
<213>  Artificial Sequence

<220>
<223>  An artificially synthesized sequence

<400>  19
guccccuucg ucuagaggcc caggacaccg cccuacgacg gcgguaacag ggguucgaau    60

ccccuagggg acgc    74

<210>  20
<211>  74
<212>  RNA
<213>  Artificial Sequence

<220>
<223>  An artificially synthesized sequence

<400>  20
guccccuucg ucuagaggcc caggacaccg cccuaauacg gcgguaacag ggguucgaau    60

ccccuagggg acgc    74

<210>  21
<211>  74
<212>  RNA
<213>  Artificial Sequence

<220>
<223>  An artificially synthesized sequence

<400>  21
guccccuucg ucuagaggcc caggacaccg cccuauuacg gcgguaacag ggguucgaau    60

ccccuagggg acgc    74

```
<210>  22
<211>  74
<212>  RNA
<213>  Artificial Sequence

<220>
<223>  An artificially synthesized sequence

<400>  22
guccccuucg ucuagaggcc caggacaccg cccuacuacg gcgguaacag ggguucgaau        60

ccccuagggg acgc                                                          74


<210>  23
<211>  74
<212>  RNA
<213>  Artificial Sequence

<220>
<223>  An artificially synthesized sequence

<400>  23
guccccuucg ucuagaggcc caggacaccg cccuaucacg gcgguaacag ggguucgaau        60

ccccuagggg acgc                                                          74
```